(19) Europäisches Patentamt
European Patent Office
Office européen des brevets

(11) **EP 4 700 047 A1**

(12) **EUROPEAN PATENT APPLICATION**
published in accordance with Art. 153(4) EPC

(43) Date of publication:
**25.02.2026 Bulletin 2026/09**

(21) Application number: 24793078.7

(22) Date of filing: **19.04.2024**

(51) International Patent Classification (IPC):
*C07K 16/46* (2006.01)    *C07K 16/32* (2006.01)
*A61P 35/00* (2006.01)    *A61K 39/00* (2006.01)

(86) International application number:
**PCT/KR2024/005350**

(87) International publication number:
**WO 2024/219894 (24.10.2024 Gazette 2024/43)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:  **21.04.2023   KR 20230052859**

(71) Applicant: **Centenaire Biosciences, Inc.
Seongnam-si, Gyeonggi-do 13453 (KR)**

(72) Inventors:
• **PARK, Hyunkyu**
  **Yongin-si, Gyeonggi-do 16836 (KR)**
• **BAE, Ji Seon**
  **Suwon-si, Gyeonggi-do 16528 (KR)**
• **LEE, Deokjae**
  **Yongin-si, Gyeonggi-do 16978 (KR)**
• **CHOI, Eun Shik**
  **Yongin-si, Gyeonggi-do 16939 (KR)**
• **YANG, Gi-Hyeok**
  **Seoul 06544 (KR)**

(74) Representative: **ABG Intellectual Property Law,
S.L.
Avenida de Burgos, 16D
Edificio Euromor
28036 Madrid (ES)**

Remarks:
The complete document including Reference
Table(s) and the Sequence Listing(s) can be
downloaded from the EPO website

(54) **ANTIGEN-BINDING PROTEIN CONTAINING TWO FC DOMAINS AND USE THEREOF**

(57)    The present invention provides a fusion protein comprising one antigen binding site and two Fc domains and having a novel antibody structure. Despite having a molecular weight similar to that of human IgG, such a novel antibody structure allows up to four times more Fc domains to be present on cell surface antigens compared to natural human antibodies. As a result, the fusion protein exhibits increased affinity for Fcγ receptors and enhanced effector functions. Therefore, the fusion protein with this novel antibody structure can be utilized as a new antibody platform.

EP 4 700 047 A1

## Description

### Technical Field

The present invention relates to a novel antibody format having an antigen binding site that specifically binds to a cancer surface antigen, and two Fc domains.

### Background Art

Antibody-based therapeutic agents and Fc fusion proteins are a group of clinically important drugs for patients with cancer, immune diseases, infectious diseases, and inflammatory diseases. ADCC (antibody-dependent cell-mediated cytotoxicity), ADCP (antibody-dependent cellular phagocytosis), and CDC (complement-dependent cytotoxicity), which are induced by the interaction between the antibody Fc domain and the innate immune system, play an important role in alleviating or treating symptoms of the disease.

Attempts are being made to maintain the bivalency of the antibody and to improve the effector function by increasing the number of Fc domains (Claudio Sustmann et al., MAbs. 2019; Dennis R Goulet et al., Proteins, 2020). Although these platforms confirmed improvements in binding affinity to Fcγ receptors and ADCC, it is difficult to obtain homogeneous antibodies due to the complexity of production and purification. Attempts to improve the effector function by connecting the Fc domains of antibodies in tandem or constituting a large number of Fc domains are also currently underway (U.S. Patent Publication US 2020/0040084 A1). In this case, there is a disadvantage that permeability to the tissue may be significantly lowered due to the increase in the size or molecular weight of the antibody. In addition, multimeric Fc domains linked in tandem may not effectively induce an increase in effector function because they have a heterogeneous geometric structure from the large amount of Fc domains provided by IgG antibodies bound to cell surface antigens.

### Detailed Description of Invention

### Technical Problem

Accordingly, the present inventors studied to improve the function of the antibody and at the same time to solve the problems of the existing antibody format designed to comprise multiple Fc domains in tandem as described above. As a result, the present inventors developed a novel engineered antibody format that enables Fc domains to be present on a cell surface antigen a maximum of four times compared to a natural human antibody, even though it has a molecular weight (approximately 150 kDa) similar to that of natural human immunoglobulin G (IgG).

### Solution to Problem

In one aspect of the present invention, there is provided a fusion protein comprising an antigen binding site, a first Fc domain or a variant thereof linked to a first linking position of the antigen binding site, and a second Fc domain or a variant thereof linked to a second linking position of the antigen binding site.

In another aspect of the present invention, there is provided a fusion protein comprising two antigen binding sites linked in tandem (tandem two antigen-binding regions), a first Fc domain or a variant thereof linked to a first linking position of the tandem 2 antigen binding sites, and a second Fc domain or a variant thereof linked to a second linking position of the tandem 2 antigen binding sites. According to one embodiment, each of the two antigen binding sites constituting the tandem 2 antigen binding sites may be a sequence comprising a CDR sequence or a variable region that is each capable of binding to different epitopes of the same antigen or to different antigens, or a sequence consisting of a variable region.

In the fusion proteins described in the present disclosure, according to one embodiment, the antigen binding site may be a sequence comprising a CDR sequence or a variable region of an antibody, or a sequence consisting of a variable region. Therefore, the antigen binding site may comprise a first peptide consisting of or comprising a light chain CDR sequence or a light chain variable region of an antibody, and a second peptide consisting of or comprising a heavy chain CDR sequence or a heavy chain variable region of an antibody. The first Fc domain and the second Fc domain may each be a dimer consisting of two peptide sequences. The first peptide of the antigen binding site may bind to a first Fc domain or a variant thereof, and the second peptide of the antigen binding site may bind to a second Fc domain or a variant thereof.

In the fusion proteins described in the present disclosure, according to another embodiment, the first Fc domain and the second Fc domain may be linked to each other through a covalent bond, non-covalent bond, or linker, or may not be linked to each other. In a preferred embodiment, the first Fc domain and the second Fc domain may not be linked to each other.

According to embodiments of the present disclosure, the Fc domain may be an Fc domain of a wild-type (WT) immunoglobulin, and may comprise modifications, for example amino acid substitutions, for modulating the reactivity of the Fc domain to Fcγ receptors (FcγRs) or ADCC, or minimizing the formation of undesirable multimers of the Fc domain. The Fc domain comprises CH2 and CH3 regions, and may comprise a CH4 region and/or a hinge region, and it should be interpreted that the Fc domain comprises Fc domain fragments that exhibit the function of the Fc domain.

According to embodiments of the present disclosure, an antigen binding site and an Fc domain or a variant thereof may be joined either directly or through a linker. For example, an antigen binding site and an Fc domain or a variant thereof may be connected with or without a linker between the N-terminus and the C-terminus, between the N-terminus and the N-terminus, or between the C-terminus and the C-terminus of each peptide molecule.

According to embodiments of the present disclosure, when an antigen binding site and an Fc domain or a variant thereof is joined through a linker, the linker can be a commonly used peptide linker. For example, the linker may be a peptide consisting of about 1 to about 70 amino acid residues, about 2 to about 60 amino acid residues, about 2 to about 50 amino acid residues, about 2 to about 40 amino acid residues, about 2 to about 30 amino acid residues, about 3 to about 50 amino acid residues, about 3 to about 40 amino acid residues, about 3 to about 30 amino acid residues, about 2 to about 28 amino acid residues, about 2 to about 26 amino acid residues, about 2 to about 24 amino acid residues, about 2 to about 22 amino acid residues, about 2 to about 20 amino acid residues, about 2 to about 18 amino acid residues, about 2 to about 16 amino acid residues, about 2 to about 14 amino acid residues, about 2 to about 12 amino acid residues, or about 2 to about 10 amino acid residues. The connection between a first Fc domain and an antigen binding site, the connection between a second Fc domain and an antigen binding site, or both may be achieved through a linker.

In another aspect of the present invention, there is provided a pharmaceutical composition for preventing or treating cancer, comprising the fusion protein as an active ingredient.

In another aspect of the present invention, there is provided a polynucleotide encoding the fusion protein, and a vector comprising the polynucleotide, and a transformed cell into which the vector has been introduced.

In another aspect of the present invention, there is provided a method for treating or preventing cancer, comprising administering the fusion protein to a subject.

In another aspect of the present invention, there is provided a use of the fusion protein for the treatment of cancer.

In another aspect of the present invention, there is provided a use of the fusion protein for use in the manufacture of a medicament for treating cancer.

### Effects of Invention

Unlike wild type (WT) antibodies, the fusion protein having the novel antibody format of the present invention comprises one or two antigen binding sites and two Fc domains. The two Fc domains are not directly linked to each other, but the two Fc domains are each independently linked to two different polypeptide chains constituting the antigen binding site. Even though this novel antibody format has a size and molecular weight similar to that of human IgG, it can enable Fc domains to be present on a cell surface antigen a maximum of four times compared to a natural human antibody. Due to these properties, the fusion protein having the novel antibody format has improved affinity (avidity) for Fcγ receptors and can induce improved effector functions. Therefore, the fusion protein having the novel antibody format can be utilized for various purposes by replacing conventional antibodies.

### Brief Description of Drawings

Figure 1a is a schematic diagram of natural human immunoglobulin (IgG).
Figure 1b is a schematic diagram of a novel engineered antibody format.
Figure 1c is a schematic diagram showing a cancer cell with its tumor antigens bound to the antigen-specific human immunoglobulins (IgG) in a monovalent manner.
Figure 1d is a schematic diagram showing a cancer cell with its tumor antigens bound to the antigen-specific human immunoglobulin (IgG) in a monovalent or bivalent manner.
Figure 1e is a schematic diagram showing a cancer cell with its tumor antigens bound to the antigen-specific human immunoglobulins (IgG) in a bivalent manner.
Figure 1f is a schematic diagram showing a cancer cell with its tumor antigens bound to the antigen-specific novel engineered antibody.
Figure 2a is a schematic diagram of a novel monovalent antibody format (WT) with two Fc domains.
Figure 2b is a schematic diagram of an antibody (M1) in which VH Q105C and VL A43C amino acid substitutions are introduced in a novel monovalent antibody format with two Fc domains.
Figure 2c is a schematic diagram of an antibody (M2) in which CH1 F122C and CL S121C amino acid substitutions are introduced in a novel monovalent antibody format with two Fc domains.

Figure 2d is a schematic diagram of an antibody (M3) in which VH G44C and VL Q100C amino acid substitutions are introduced in a novel monovalent antibody format with two Fc domains.

Figure 3a illustrates sequence information indicating the position of Cys substitution in the VH-CH1 domain of trastuzumab. The WT sequence is SEQ ID NO: 8, the sequence of Mutant 1 is SEQ ID NO: 10, the sequence of Mutant 2 is SEQ ID NO: 12, and the sequence of Mutant 3 is SEQ ID NO: 14.

Figure 3b illustrates sequence information indicating the position of Cys substitution in the VL-CL domain of trastuzumab. The WT sequence is SEQ ID NO: 9, the sequence of Mutant 1 is SEQ ID NO: 11, the sequence of Mutant 2 is SEQ ID NO: 13, and the sequence of Mutant 3 is SEQ ID NO: 15.

Figure 4 illustrates a result obtained by SDS-PAGE analysis of WT, M1, M2, or M3.

Figure 5a illustrates a result obtained by size exclusion chromatography analysis of WT.

Figure 5b illustrates a result obtained by size exclusion chromatography analysis of M1.

Figure 5c illustrates a result obtained by size exclusion chromatography analysis of M2.

Figure 5d illustrates a result obtained by size exclusion chromatography analysis of M3.

Figure 6a is a schematic diagram of an antibody (M3) in which the CL domain and the hinge region are linked with a 15-mer peptide.

Figure 6b is a schematic diagram of an antibody (V1) in which the CL domain and the hinge region are linked with a 10-mer peptide.

Figure 6c is a schematic diagram of an antibody (V2) in which the CL domain and the hinge region are linked with a 5-mer peptide.

Figure 6d is a schematic diagram of an antibody (V3) in which the CL domain and the hinge region are directly linked without a linker.

Figure 7 illustrates a result obtained by SDS-PAGE analysis of M3, V1, V2, or V3.

Figure 8a is a schematic diagram of fragments generated when H01 or P01 is cleaved with papain.

Figure 8b is a schematic diagram of fragments generated by papain cleavage of H01 or P01 when a disulfide bond in the hinge region is abnormally formed.

Figure 8c illustrates a result obtained by SDS-PAGE analysis of the H01 papain cleavage product.

Figure 8d illustrates a result obtained by SDS-PAGE analysis of the P01 papain cleavage product.

Figure 9 illustrates a result obtained by SDS-PAGE analysis of H01wt or H01.

Figure 10a is a schematic diagram of H01Fv1, which has an Fv-(Fc)2 structure.

Figure 10b is a schematic diagram of H01Fv2, which has an Fv-(Fc)2 structure.

Figure 10c is a schematic diagram of H01Fv3, which has an Fv-(Fc)2 structure.

Figure 10d is a schematic diagram of H01Fv4, which has an Fv-(Fc)2 structure.

Figure 10e is a schematic diagram of H01Fv5, which has an Fv-(Fc)2 structure.

Figure 10f is a schematic diagram of H01Fv6, which has an Fv-(Fc)2 structure.

Figure 10g is a schematic diagram of H01Fv7, which has an Fv-(Fc)2 structure.

Figure 10h is a table showing the mutated positions of the Fv-(Fc)2 structure.

Figure 11 illustrates a result obtained by SDS-PAGE analysis of the Fv-(Fc)2 structure after Protein A purification.

Figures 12a to 12g illustrate results obtained by SEC analysis of the Fv-(Fc)2 structure after Protein A purification.

Figure 13a illustrates an analysis of the sensorgram data for the binding of H01Fv1, a purified Fv-(Fc)2 structure, to human HER2.

Figure 13b illustrates an analysis of the sensorgram data for the binding of H01Fv2, a purified Fv-(Fc)2 structure, to human HER2.

Figure 13c illustrates an analysis of the sensorgram data for the binding of H01Fv4, a purified Fv-(Fc)2 structure, to human HER2.

Figure 13d illustrates an analysis of the sensorgram data for the binding of H01Fv5, a purified Fv-(Fc)2 structure, to human HER2.

Figure 13e illustrates an analysis of the sensorgram data for the binding of H01Fv6, a purified Fv-(Fc)2 structure, to human HER2.

Figure 13f illustrates an analysis of the sensorgram data for the binding of H01Fv7, a purified Fv-(Fc)2 structure, to human HER2.

Figure 14 illustrates a differential scanning fluorimetry analysis of the melting temperatures of H01, P01, trastuzumab, and pertuzumab.

Figure 15 is a bio-layer interferometry (BLI) analysis of the sensorgram data showing competitive binding of H01 and P01.

Figure 16a is a schematic diagram showing the binding mode of H01 in combination with P01 to HER2 on HER2-positive cancer cells.

Figure 16b is a schematic diagram showing the binding mode of trastuzumab in combination with pertuzumab to HER2 on HER2-positive cancer cells.

Figure 17a illustrates a flow cytometry analysis of the amount of Fc domain present on the surface of NCI-N87 gastric cancer cell line after treatment with anti-HER2 antibody (50 nM).

Figure 17b illustrates a flow cytometry analysis of the amount of Fc domain present on the surface of BT474 breast cancer cell line after treatment with anti-HER2 antibody (50 nM).

Figure 17c illustrates a flow cytometry analysis of the amount of Fc domain present on the surface of SK-OV3 ovarian cancer cell line after treatment with anti-HER2 antibody (50 nM).

Figure 17d illustrates a flow cytometry analysis of the amount of Fc domain present on the surface of SNU-1 gastric cancer cell line after treatment with anti-HER2 antibody (50 nM).

Figure 17e illustrates a flow cytometry analysis of the amount of Fc domain present on the surface of SNU-5 gastric cancer cell line after treatment with anti-HER2 antibody (50 nM).

Figure 18a illustrates a flow cytometry analysis of the amount of Fc domain present on the surface of NCI-N87 gastric cancer cell line after treatment with anti-HER2 antibody at indicated concentrations (20, 50, and 100 nM).

Figure 18b illustrates a flow cytometry analysis of the amount of Fc domain present on the surface of BT474 breast cancer cell line after treatment with anti-HER2 antibody at indicated concentrations (20, 50, and 100 nM).

Figure 18c illustrates a flow cytometry analysis of the amount of Fc domain present on the surface of SK-OV3 ovarian cancer cell line after treatment with anti-HER2 antibody at indicated concentrations (20, 50, and 100 nM).

Figure 18d illustrates a flow cytometry analysis of the amount of Fc domain present on the surface of SNU-1 gastric cancer cell line after treatment with anti-HER2 antibody at indicated concentrations (20, 50, and 100 nM).

Figure 18e illustrates a flow cytometry analysis of the amount of Fc domain present on the surface of SNU-5 gastric cancer cell line after treatment with anti-HER2 antibody at indicated concentrations (20, 50, and 100 nM).

Figure 19a is a schematic diagram of H01DE4 in which S239D and I332E mutations were introduced in H01.

Figure 19b is a schematic diagram of P01DE4 in which S239D and I332E mutations were introduced in P01.

Figures 20a to 20d are sensorgrams binding profiles of H01, H01DE4, P01, or P01DE4 for human HER2.

Figures 21a to 21h are sensorgrams binding profiles of H01, P01, H01DE4, P01DE4, human IgG1, trastuzumab, pertuzumab, or margetuximab for Fcγ receptor 1.

Figures 22a to 22h are sensorgrams binding profiles of H01, P01, H01DE4, P01DE4, human IgG1, trastuzumab, pertuzumab, or margetuximab for Fcγ receptor 2A (131R isoform).

Figures 23a to 23h are sensorgrams binding profiles of H01, P01, H01DE4, P01DE4, human IgG1, trastuzumab, pertuzumab, or margetuximab for Fcγ receptor 3A (158V isoform).

Figure 24a is a graph showing an antibody concentration in blood over time after H01, P01, trastuzumab, or pertuzumab (10 mg/kg) was administered intravenously to Sprague-Dawley rats.

Figure 24b illustrates the pharmacokinetic parameters (PK parameters) of each antibody after H01, P01, trastuzumab, or pertuzumab (10 mg/kg) was administered intravenously to Sprague-Dawley rats.

Figure 25 is a schematic diagram of HP501, which is a HER2 biparatopic engineered antibody.

Figure 26 is a schematic diagram of HP501 to HP516, which are HER2 biparatopic engineered antibodies.

Figure 27 illustrates a size exclusion chromatography analysis of HP501 to HP516, which are HER2 biparatopic engineered antibodies.

Figure 28a is a bio-layer interferometry analysis of the sensorgram data for the binding of HP503 to HER2.

Figure 28b is a bio-layer interferometry analysis of the sensorgram data for the binding of HP507 to HER2.

Figure 28c is a bio-layer interferometry analysis of the sensorgram data for the binding of HP511 to HER2.

Figure 28d is a bio-layer interferometry analysis of the sensorgram data for the binding of HP515 to HER2.

Figure 29a is a bio-layer interferometry analysis of the sensorgram data for the binding of HP503 for Fcγ receptor 1, Fcγ receptor 2A (131R isoform), or Fcγ receptor 3A (158V isoform).

Figure 29b is a bio-layer interferometry analysis of the sensorgram data for the binding of HP507 for Fcγ receptor 1, Fcγ receptor 2A (131R isoform), or Fcγ receptor 3A (158V isoform).

Figure 29c is a bio-layer interferometry analysis of the sensorgram data for the binding of HP511 for Fcγ receptor 1, Fcγ receptor 2A (131R isoform), or Fcγ receptor 3A (158V isoform).

Figure 29d is a bio-layer interferometry analysis of the sensorgram data for the binding of HP515 for Fcγ receptor 1, Fcγ receptor 2A (131R isoform), or Fcγ receptor 3A (158V isoform).

Figure 30a is a bio-layer interferometry analysis of the sensorgram data for the binding of HP503, HP507, HP511, or HP515 for the neonatal Fc receptor (FcRn).

Figure 30b is a bio-layer interferometry analysis of the sensorgram data for the binding of human IgG1, trastuzumab, pertuzumab, or margetuximab for the neonatal Fc receptor (FcRn).

Figure 31a illustrates the CDC (complement-dependent cytotoxicity) activity of human IgG1, trastuzumab (TRA), trastuzumab + pertuzumab (TRA + PER), H01, or H01 + P01 in the BT474 breast cancer cell line.

Figure 31b illustrates the CDC activity of human IgG1, trastuzumab (TRA), trastuzumab + pertuzumab (TRA + PER), H01, or H01 + P01 in the NCI-N87 gastric cancer cell line.

Figure 31c is a schematic diagram of a monovalent (one-arm) antibody (OA_TRA) having trastuzumab Fab.

Figure 31d is a schematic diagram of a Duomab antibody (Duo_TRA) having trastuzumab Fab.

Figure 31e is a schematic diagram of a tandem Fc antibody (Tandem_Fc_TRA) having trastuzumab Fab.

Figure 31f illustrates the CDC activity of human IgG1, trastuzumab (TRA), H01, a one-arm trastuzumab (OA_TRA), a Duomab trastuzumab (Duo_TRA), or a tandem Fc trastuzumab (Tandem_Fc_TRA) and the CDC activity of either of the antibodies in combination with pertuzumab , in the NCI-N87 gastric cancer cell line.

Figure 31g illustrates the CDC activity of human IgG1, trastuzumab (TRA), H01, a one-arm trastuzumab (OA_TRA), a Duomab trastuzumab (Duo_TRA), or a tandem Fc trastuzumab (Tandem_Fc_TRA) and the CDC activity of either of the antibodies in combination with pertuzumab, in the BT474 breast cancer cell line.

Figure 32a illustrates the ADCC activity of human IgG1, trastuzumab, or H01 in the NCI-N87 gastric cancer cell line.

Figure 32b illustrates the ADCC activity of human IgG1, trastuzumab, or H01 in the MDA-MB-453 breast cancer cell line.

Figure 32c illustrates the ADCC activity of human IgG1, trastuzumab, or H01 in the SNU-601 gastric cancer cell line.

Figure 32d illustrates the ADCC activity of human IgG1, trastuzumab, or H01 in the SNU-5 gastric cancer cell line.

Figure 33a illustrates the antitumor activity of trastuzumab, trastuzumab + pertuzumab, H01, H01 + P01, or HP507 at a high concentration (5 mg/kg each) in a C.B-17 SCID mouse model of tumor xenograft of SNU-5 gastric cancer cell line.

Figure 33b illustrates the antitumor activity of trastuzumab (TRA, 1 mg/kg), pertuzumab (PER, 1 mg/kg), TRA (0.5 mg/kg) + PER (0.5 mg/kg), H01 (1 mg/kg), P01 (1 mg/kg), or H01 (0.5 mg/kg) + P01 (0.5 mg/kg) at a low concentration in a C.B-17 SCID mouse model of tumor xenograft of SNU-5 gastric cancer cell line.

Figure 34 illustrates the antitumor activity of IVIG (intravenous immunoglobulin) (50 mg/kg) alone and in combination with H01, trastuzumab, or trastuzumab + pertuzumab at a high concentration (5 mg/kg each) in a mouse model of tumor xenograft of SNU-601 gastric cancer cell line.

Figure 35 illustrates the antitumor activity of IVIG (50 mg/kg) alone and in combination with H01 or trastuzumab at indicated concentrations (0.2 mg/kg, 0.5 mg/kg) in a mouse model of tumor xenograft of NCI-N87 gastric cancer cell line.

Figure 36 is a vector map for a vector to express human HER2 protein in mammalian cells.

Figure 37 illustrates the flow cytometry quantification of HER2 expression in a CT26 mouse large intestine cancer cell line clone expressing human HER2 (CT26-HER2).

Figure 38 illustrates the stability of human HER2 expression in the CT26-HER2 clones.

Figure 39 illustrates the relative expression of human HER2 in a CT26-HER2 cell line (Clone name: #2-60) compared to human cancer cell lines, and an amount of H01 binding to the surface of CT26-HER2 cells compared to trastuzumab.

Figure 40 illustrates the antitumor activity of trastuzumab or H01 in a syngeneic CT26-HER2 mouse tumor model.

Figure 41a is a schematic diagram of a monovalent engineered mAb (monoclonal antibody) according to one embodiment of the present invention.

Figure 41b is a schematic diagram of a biparatopic engineered mAb according to one embodiment of the present invention.

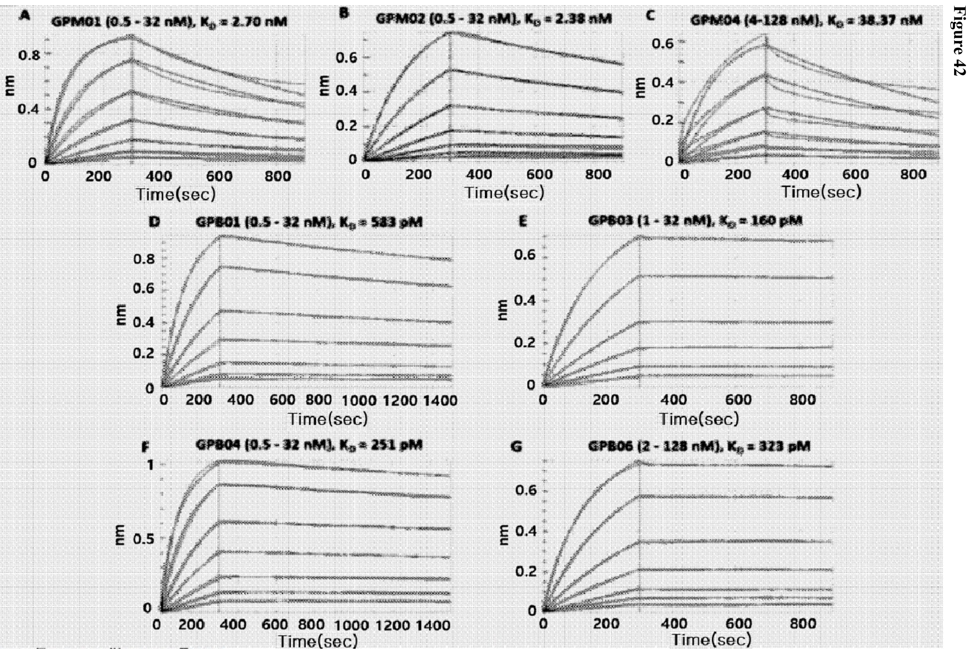

Figure 42 illustrates the bio-layer interferometry analysis of the sensorgram data for the binding of a fusion protein according to one embodiment of the present invention to the target. (A) illustrates the sensorgram data for the binding of GPM01, a monovalent engineered mAb targeting GPC-3, to human GPC-3; and (B) illustrates the sensorgram data for the binding of GPM02, a monovalent engineered mAb targeting GPC-3, to human GPC-3. (C) illustrates the sensorgram data for the binding of GPM04, a monovalent engineered mAb targeting GPC-3, to human GPC-3; and (D) illustrates the sensorgram data for the binding of GPB01, a biparatopic engineered mAb targeting GPC-3, to human GPC-3. (E) illustrates the sensorgram data for the binding of GPB03, a biparatopic engineered mAb targeting GPC-3, to human GPC-3; and (F) illustrates the sensorgram data for the binding of GPB04, a biparatopic engineered mAb targeting GPC-3, to human GPC-3; and (G) illustrates the sensorgram data for the binding of GPB06, a biparatopic engineered mAb targeting GPC-3, to human GPC-3.

Figure 43 illustrates the flow cytometry analysis of the amount of Fc domain present on the surface of HepG2 liver cancer cell line after treatment with GPC-3 antibody (100 nM).

Figure 44 illustrates the SDS-PAGE analysis showing the inhibition of AKT phosphorylation in the PC-3 prostate cancer cell line after treatment with EphA2 antibody (100 nM).

Figure 45 illustrates the flow cytometry analysis of the amount of Fc domain present on the surface of PC-3 prostate cancer cell line after treatment with EphA2 antibody (100 nM).

Figure 46 illustrates the bio-layer interferometry analysis of the sensorgram data for the binding of MEM01 or MEM06, a monovalent engineered mAb targeting MET, to human MET.

Figure 47 illustrates the flow cytometry analysis of the amount of Fc domain present on the surface of MKN45 gastric cancer cell line after treatment with MET antibody at indicated concentrations (20, 50, and 100 nM).

Figure 48 illustrates the flow cytometry analysis of the amount of Fc domain present on the surface of SNU5 gastric cancer cell line after treatment with an antibody at indicated concentrations (50 and 100 nM).

Figure 49 illustrates the bio-layer interferometry analysis of the sensorgram data for the binding of a monovalent engineered mAb targeting EGFR to human EGFR.

Figure 50 illustrates the bio-layer interferometry analysis of the sensorgram data for the binding of a monovalent engineered mAb targeting CD33 (33-1, 33-2, and 33-3) or a biparatopic engineered mAb targeting CD33 (33-4, 33-5, 33-6, and 33-7) to human CD33.

Figure 51 illustrates the bio-layer interferometry analysis of the sensorgram data for the binding of a monovalent engineered mAb targeting CEACAM5 to human CEACAM5.

Figure 52 illustrates the bio-layer interferometry analysis of the sensorgram data for the binding of a fusion protein according to one embodiment of the present invention to the target. (A) illustrates the sensorgram data for the binding of T01, a monovalent engineered mAb targeting TROP2, to human TROP2; and (B) illustrates the sensorgram data for the binding of MSM01, a monovalent engineered mAb targeting mesothelin, to human mesothelin; and (C) illustrates the sensorgram data for the binding of LIM01, a monovalent engineered mAb targeting LIV-1, to human LIV-1.

Figure 53a is a schematic diagram of a novel engineered antibody format.

Figure 53b is a schematic diagram showing a receptor bound to the receptor-specific human immunoglobulins (IgG) in a monovalent manner.

Figure 53c is a schematic diagram showing a receptor bound to the receptor-specific human immunoglobulin (IgG) in a monovalent or bivalent manner.

Figure 53d is a schematic diagram showing a receptor bound to the receptor-specific human immunoglobulins (IgG) in a bivalent manner.

Figure 53e is a schematic diagram showing a receptor bound to the novel engineered antibody format.

Figure 54 is a schematic diagram of an engineered antibody format with two Fc domains according to one embodiment of the present invention.

Figure 55a is a sensorgram binding profile of trastuzumab or H01 for Fcγ receptor 1.

Figure 55b is a sensorgram binding profile of trastuzumab or H01 for Fcγ receptor 2A (131H isoform).

Figure 55c is a sensorgram binding profile of trastuzumab or H01 for Fcγ receptor 3A (158V isoform).

Figure 55d illustrates the equilibrium dissociation constant ($K_D$), association rate constant (Ka), and dissociation rate constant (Kd) values of trastuzumab or H01 for Fcγ receptor 1, Fcγ receptor 2A, and Fcγ receptor 3A.

Figures 56a and 56b illustrate the binding affinity of trastuzumab or H01 to human immune cells.

Figure 57 illustrates the ADCC activity of trastuzumab or H01 in (A) gastric cancer cell line (SNU-5 cells) or (B) breast cancer cell line (ZR-75-1 cells).

Figure 58 is a schematic diagram of an engineered antibody format with two Fc domains according to one embodiment of the present invention.

Figures 59a to 59d are schematic diagrams showing the binding mode of an engineered antibody format with two Fc domains according to one embodiment of the present invention, which simultaneously binds to the cell surface, or to soluble antigens on the cell surface and around the cell.

Figure 60 illustrates results obtained by SDS-PAGE analysis of H01 (A), H04, H05, H06, H07, H08, or H09 under reducing conditions (B) or under non-reducing conditions (C).

Figure 61 illustrates results obtained by SDS-PAGE analysis of (A) H01P, P01H, (B) ME13, ME14, ME15, ME16, or (C) CE05 under non-reducing conditions (NR) or reducing conditions (R).

Figure 62 illustrates results obtained by SDS-PAGE analysis of (A) EAVC-P1, EAVC-P2, EAVC-P3, EAVC-P4, EAVC-P5, EAVC-P6, EAVC-P7, EAVC-P8, or (B) EAVC-C3, EAVC-13, EAVC-L3, EAVC-M3 or EAVC-N3 under non-reducing conditions (NR) or reducing conditions (R).

Figure 63 illustrates results obtained by SDS-PAGE analysis of (A) HAVC01, HAVC02, HAVC03, HAVC05, HAVC06, HAVC07, HAVC08 or (B) HAVC04 under non-reducing conditions (NR) or reducing conditions (R).

Figure 64a illustrates results obtained by size exclusion chromatography analysis of (A) HAVC01, (B) HAVC02, (C) HAVC03, or (D) HAVC04.

Figure 64b illustrates results obtained by size exclusion chromatography analysis of (A) HAVC05, (B) HAVC06, (C) HAVC07, or (D) HAVC08.

## Best Mode for Carrying out the Invention

### Definition of Terms

As used herein, the term "fusion protein with two Fcs" or "antibody with two Fcs" refers to a fusion protein in which two Fc domains are independently joined to two polypeptide chains constituting the antigen binding site. The two polypeptide chains constituting the antigen binding site may be different from each other. For example, one of the two polypeptide chains constituting the antigen binding site may be a sequence comprising or consisting of the light chain CDR sequence or light chain variable region of the antibody, or may be an scFv, and the other may be a sequence comprising or consisting of the heavy chain CDR sequence or heavy chain variable region of the antibody, or may be an scFv. In one embodiment, the fusion protein having the two Fc regions may comprise the sequence of a "humanized" form of a non-human antibody, which is a chimeric antibody comprising a human immunoglobulin comprising native CDRs. In addition, the fusion protein may comprise a "fully human antibody" or a portion of a "human antibody." In addition, in one embodiment, the multispecific fusion protein or antigen binding domain may be a "monoclonal antibody" or a portion thereof.

As used herein, the term "antibody" refers to a substance that specifically binds to an antigen and causes an antigen-antibody reaction. In addition, the antibody is also referred to as immunoglobulin. The antibody may refer to any one selected from IgG, IgE, IgM, IgD, and IgA, and may be a subclass of IgG, such as IgG1, IgG2, IgG3, IgG4, or a subclass of IgA, such as IgA1 or IgA2. In addition, the antibody may be an agonistic antibody or an antagonistic antibody.

As used herein, the term "Fab" or "Fab region" refers to a region of an antibody that binds to an antigen. Conventional IgG generally comprises two Fab regions. Each Fab region typically consists of one variable region and one constant region of each heavy chain and light chain. Specifically, the variable region and the constant region of the heavy chain in the Fab region are the VH and CH1 regions, and the variable region and the constant region of the light chain in the Fab region are the VL and CL regions. VH, CH1, VL, and CL of the Fab region may be arranged in various ways to impart the antigen binding ability according to the present disclosure, including the CrossMab Fab technology in which VH and VL have an arrangement substituted for each other.

As used herein, the term "heavy chain" refers to a polypeptide chain of about 50 kDa to about 70 kDa. Here, the N-terminal portion comprises a variable region of at least about 120 to 130 amino acids, and the C-terminal portion comprises a constant region. The constant region may be one of five types: alpha ($\alpha$), delta ($\delta$), epsilon ($\varepsilon$), gamma ($\gamma$), and mu ($\mu$). Here, $\alpha$, $\delta$, and $\gamma$ comprise about 450 amino acids, and $\mu$ and $\varepsilon$ comprise about 550 amino acids.

As used herein, the term "light chain" refers to a polypeptide chain of about 25 kDa. Here, the N-terminal portion comprises a variable region of at least about 100 to about 110 amino acids, and the C-terminal portion comprises a constant region. There are two types of light chain constant domains: kappa ($\kappa$) or lambda ($\lambda$). In addition, the constant region of the light chain is referred to as "CL". The heavy chain C domains (CH domains) are numbered from N-terminus to C-terminus (e.g., CH1, CH2, CH3, etc.). The CL and a CH1 regions of any of these antibody classes may be used in the present disclosure. In certain embodiments, the CL and CH1 regions provided herein may be of the IgG type (for example, IgG1).

As used herein, the term "Fc" or "Fc region" refers to the C-terminal region of an immunoglobulin heavy chain, including a native Fc region, a recombinant Fc region, and a variant Fc region. Therefore, Fc refers to the last two constant region immunoglobulin domains of IgA, IgD, and IgG, the last three constant region immunoglobulin domains of IgE and IgM, and the hinge present in the N-terminus of these domains. For IgA and IgM, the Fc may comprise a J chain. For IgG, the Fc comprises the immunoglobulin domains Cy2 (CH2) and Cy3 (CH3) and the hinge between Cy1 and Cy2. Although the interface of the Fc region may vary, the human IgG heavy chain Fc region is generally defined as comprising residues C226, P230, or A231 at the C-terminus, where numbering is according to the EU index. As used herein, "Fc polypeptide" or "Fc derived polypeptide" refers to a polypeptide comprising all or part of an Fc region. In one embodiment, a variant Fc region may be in a form in which at least one amino acid, for example, about 1 to about 10 amino acids, or about 1 to about 5 amino acids, are substituted compared to the native sequence Fc region. In addition, the variant Fc region may have at least about 80% homology, at least about 90% homology, or at least about 95% homology to the native sequence Fc region.

As used herein, the term "Fv" or "Fv fragment" or "Fv region" is a polypeptide comprising the VL and VH domains of a single antibody.

As used herein, the term "single chain Fv" or "scFv" refers to an antibody fragment comprising the VH and VL domains of an antibody within a single polypeptide chain.

As used herein, the term "variable region" refers to an antibody region comprising one or more immunoglobulin domains encoded by any one of the VL (including Vkappa (VK) and Vlambda (VL)) and/or VH genes that constitute the light chain (including kappa and lambda) and heavy chain immunoglobulin loci, respectively. The light or heavy chain variable region (VL or VH) consists of a "framework" or "FR" region that includes three hypervariable regions referred to as "complementarity determining regions" or "CDRs".

As used herein, the term "antigen" refers to a structure capable of selectively binding to an antibody. A target antigen may be a polypeptide, carbohydrate, nucleic acid, lipid, hapten, or other naturally occurring compound or synthetic compound. Specifically, an antigen is a polypeptide and may be a protein present on or within a cell.

As used herein, the term "epitope" refers to an antigenic determinant and is a part on an antigen to which an antibody or polypeptide binds. A protein epitope may comprise amino acid residues that are directly involved in binding as well as amino acid residues that are effectively blocked by specific antigen binding antibodies or peptides. It is the simplest form or smallest structural region of a complex antigen molecule that may bind to an antibody or receptor. The epitope may be linear or structural/conformational.

As used herein, the term "Z protein" may be referred to as a "Z domain", "additional protein", or "additional domain", and the "Z protein" is a protein that binds to the antibody of the novel format herein. The "Z protein" may be any one selected from the group consisting of a receptor, a soluble protein, a cytokine, a single-chain Fv fragment (single-chain fragment variable; scFv), an antibody mimetics, a single domain antibody (sdAb), a therapeutic peptide, and a peptide vaccine.

As used herein, the term "vector" refers to a material for transporting or expressing a nucleic acid sequence including a nucleic acid sequence encoding a multispecific fusion protein (for example, an antibody) described herein. Specifically, vectors include expression vectors, plasmids, phage vectors, viral vectors, episomes, and artificial chromosomes.

As used herein, the term "polynucleotide," also referred to as "nucleic acid," refers to a polymer of nucleotides of any length. Specifically, the polynucleotide may be DNA or RNA.

**Antibody comprising two Fcs**

In one aspect of the present invention, there is provided an antibody comprising a plurality of Fc domains, characterized in that the ratio of the antigen binding site and the Fc domain is 1:2 or 2:2. Specifically, the antibody may be a fusion protein comprising an antigen binding site, a first Fc domain or a variant thereof, and a second Fc domain or a variant thereof.

Here, the antigen binding site may consist of two different polypeptide chains. In addition, each polypeptide may be linked to a first Fc domain or a variant thereof and a second Fc domain or a variant thereof.

Here, in one embodiment of the fusion protein, when the antigen binding site comprises Fab, it may be a fusion protein in which the two Fc domains are bound to the C-terminus of the CH1 region of the heavy chain and the C-terminus of the constant region of the light chain, respectively. In addition, the Fc domain and Fab may be linked through a peptide linker.

In addition, in one embodiment of the fusion protein, when the antigen binding site is Fv, it may be a fusion protein in which the two Fc domains are bound to the C-terminus of the variable region of the heavy chain and the C-terminus of the variable region of the light chain, respectively. In addition, the Fc domain and Fv may be linked through a peptide linker.

This novel antibody format or structure has a molecular weight similar to human IgG. In addition, the fusion protein may have an antigen binding affinity equivalent to that of a human IgG-based antibody. However, the antibody format enables Fc domains to be present on a cell surface antigen a maximum of four times compared to a natural human antibody. Due to these characteristics, the fusion protein may have increased affinity for Fcγ receptors, and may have increased effector functions compared to the wild type antibody. Each Fc domain bound to the fusion protein may have a similar level of Fc receptor (Fcγ receptor and FcRn) binding affinity as the Fc domain of an IgG-based antibody, but due to the avidity effect, the apparent binding affinity (apparent affinity) of the fusion protein to the Fc receptor (Fcγ receptor and FcRn) may be significantly increased compared to a human IgG antibody. In addition, the fusion protein has a similar level of thermal stability as that of an IgG-based antibody.

Specifically, the fusion protein may be a fusion protein comprising (a) an antigen binding site consisting of a first polypeptide comprising at least one CDR sequence and a second polypeptide comprising at least one CDR sequence, wherein the first polypeptide and the second polypeptide form a dimer and the antigen binding site is capable of specifically binding to a target antigen, (b) a first Fc domain or a variant thereof that is a dimer consisting of two polypeptide sequences, one of which is joined to the first polypeptide of the antigen binding site, and (c) a second Fc domain or a variant thereof that is a dimer consisting of two polypeptide sequences, one of which is joined to the second polypeptide of the antigen binding site.

Here, the first polypeptide of the antigen binding site may comprise CDR1, CDR2, and CDR3 of an antibody heavy chain, and the second polypeptide of the antigen binding site may comprise CDR1, CDR2, and CDR3 of an antibody light chain. In addition, the first polypeptide of the antigen binding site may further comprise a CH1 region of an antibody heavy chain, and/or the second polypeptide of the antigen binding site may further comprise a constant region of an antibody light chain.

In addition, the Z protein may be bound to a first Fc domain or a variant thereof, and a second Fc domain or a variant thereof. In particular, the Z protein may be bound to the N-terminus of a hinge domain of a first Fc domain or a variant thereof, and a second Fc domain or a variant thereof.

The specific structure of the fusion protein is described in more detail below.

**Antigen binding site**

Here, the antigen binding site is capable of specifically binding to a protein expressed on the cell surface. Specifically, the antigen binding site is capable of specifically binding to a cancer antigen.

In one embodiment, the antigen binding site is capable of specifically binding to any one antigen selected from the group consisting of PD-L1, EGFR, EGFRvIII, BCMA, CD22, CD25, CD30, CD33, CD37, CD38, CD52, CD56, CD123, c-Met (MET), DLL3, DR4, DR5, GD2, nectin-4, RANKL, SLAMF7, Trop-2, LIV-1, claudin 18.2, IL13α2, CD3, HER2, HER3, FGFR2, FGFR3, GPC3, ROR1, Folα, CD20, CD19, CTLA-4, VEGFR, NCAM1, ICAM-1, ICAM-2, CEACAM5, CEACAM6, carcinoembryonic antigen (CEA), CA-125, alphafetoprotein (AFP), MUC-1, MUC-16, PSMA, PSCA, epithelial tumor antigen (ETA), melanoma-associated antigen (MAGE), immature laminin receptor, TAG-72, HPV E6/E7, BING-4, calcium-activated chloride channel 2, cyclin-B1, 9D7, Ep-CAM, EphA2, EphA3, mesothelin, SAP-1, survivin, and virus-derived antigens.

A second antigen binding site is also capable of specifically binding to any one antigen selected from the group consisting of the above antigens. According to one embodiment, the antigen to which a first antigen binding site binds may be different from the antigen to which a second antigen binding site binds. In one embodiment, the first antigen binding site may comprise a sequence that specifically binds to HER2, and the second antigen binding site may comprise a sequence that specifically binds to EGFR. In another embodiment, the first antigen binding site may comprise a sequence that specifically binds to one epitope of an antigen, and the second antigen binding site may comprise a sequence that specifically binds to a different epitope of the same antigen.

**Embodiments of antigen binding sites**

Here, the antigen binding site may comprise a variable region that specifically binds to the antigen. Specifically, the variable region may include the heavy chain variable region and light chain variable region of any one antibody selected from the group consisting of cetuximab, panitumumab, necitumumab, imgatuzumab, depatuxizumab, losatuxizumab, etevritamab, AMG-595, atezolizumab, avelumab, durvalumab, trastuzumab, pertuzumab, onartuzumab, emibetuzumab, telisotuzumab, datopotamab, sacituzumab, rovalpituzumab, tarlatamab, belantamab, ladiratuzumab, codrituzumab, aprutumab, bemarituzumab, vofatamab, ramucirumab, rituximab, obinutuzumab, daratumumab, and 1C1 (Clone name), but is not limited thereto.

As an embodiment of the present invention, it may include an antigen binding site that specifically binds to EGFR. For example, it may comprise a heavy chain variable region comprising H-CDR1 comprising the amino acid sequence of SEQ ID NO: 175, H-CDR2 comprising the amino acid sequence of SEQ ID NO: 176, and H-CDR3 comprising the amino acid sequence of SEQ ID NO: 177 of cetuximab, and may comprise a light chain variable region comprising L-CDR1 comprising the amino acid sequence of SEQ ID NO: 178, L-CDR2 comprising the amino acid sequence of SEQ ID NO: 179, and L-CDR3 comprising the amino acid sequence of SEQ ID NO: 180. As another example, it may comprise a heavy chain variable region comprising H-CDR1 comprising the amino acid sequence of SEQ ID NO: 181, H-CDR2 comprising the amino acid sequence of SEQ ID NO: 182, and H-CDR3 comprising the amino acid sequence of SEQ ID NO: 183 of panitumumab, and may comprise a light chain variable region comprising L-CDR1 comprising the amino acid sequence of SEQ ID NO: 184, L-CDR2 comprising the amino acid sequence of SEQ ID NO: 185, and L-CDR3 comprising the amino acid sequence of SEQ ID NO: 186. As another example, it may comprise a heavy chain variable region comprising H-CDR1 represented by SEQ ID NO: 187, H-CDR2 represented by SEQ ID NO: 188, and H-CDR3 represented by SEQ ID NO: 189 of necitumumab, and may comprise a light chain variable region comprising L-CDR1 comprising the amino acid sequence of SEQ ID NO: 190, L-CDR2 comprising the amino acid sequence of SEQ ID NO: 191, and L-CDR3 comprising the amino acid sequence of SEQ ID NO: 192. In addition, it may comprise a heavy chain variable region comprising H-CDR1 comprising the amino acid sequence of SEQ ID NO: 193, H-CDR2 comprising the amino acid sequence of SEQ ID NO: 194, and H-CDR3 comprising the amino acid sequence of SEQ ID NO: 195 of imgatuzumab, and may comprise a light chain variable region comprising L-CDR1 comprising the amino acid sequence of SEQ ID NO: 196, L-CDR2 comprising the amino acid sequence of SEQ ID NO: 197, and L-CDR3 comprising the amino acid sequence of SEQ ID NO: 198. In addition, it may comprise a heavy chain variable region comprising H-CDR1 comprising the amino acid sequence of SEQ ID NO: 199, H-CDR2 comprising the amino acid sequence of SEQ ID NO: 200, and H-CDR3 comprising the amino acid sequence of SEQ ID NO: 201 of depatuxizumab, and may comprise a light chain variable region comprising L-CDR1 comprising the amino acid sequence of SEQ ID NO: 202, L-CDR2 comprising the amino acid sequence of SEQ ID NO: 203, and L-CDR3 comprising the amino acid sequence of SEQ ID NO: 204. In addition, it may comprise a heavy chain variable region comprising H-CDR1 comprising the amino acid sequence of SEQ ID NO: 199, H-CDR2 comprising the amino acid sequence of SEQ ID NO: 205, and H-CDR3 comprising the amino acid sequence of SEQ ID NO: 206 of losatuxizumab, and may comprise a light chain variable region comprising L-CDR1 comprising the amino acid sequence of SEQ ID NO: 202, L-CDR2 comprising the amino acid sequence of SEQ ID NO: 203, and L-CDR3 comprising the amino acid sequence of SEQ ID NO: 204.

As an embodiment of the present invention, it may include an antigen binding site that specifically binds to EGFRvIII. For example, it may comprise a heavy chain variable region comprising H-CDR1 comprising the amino acid sequence of SEQ ID NO: 207, H-CDR2 comprising the amino acid sequence of SEQ ID NO: 208, and H-CDR3 comprising the amino acid sequence of SEQ ID NO: 209 of etevritamab, and may comprise a light chain variable region comprising L-CDR1 comprising the amino acid sequence of SEQ ID NO: 210, L-CDR2 comprising the amino acid sequence of SEQ ID NO: 211, and L-CDR3 comprising the amino acid sequence of SEQ ID NO: 212. In addition, it may comprise a heavy chain variable region comprising H-CDR1 comprising the amino acid sequence of SEQ ID NO: 213, H-CDR2 comprising the amino acid sequence of SEQ ID NO: 214, and H-CDR3 comprising the amino acid sequence of SEQ ID NO: 215 of AMG-595, and may comprise a light chain variable region comprising L-CDR1 comprising the amino acid sequence of SEQ ID NO: 210, L-CDR2 comprising the amino acid sequence of SEQ ID NO: 216, and L-CDR3 comprising the amino acid sequence of SEQ ID NO: 217.

As an embodiment of the present invention, it may include an antigen binding site that specifically binds to PD-L1. For example, it may comprise a heavy chain variable region comprising H-CDR1 comprising the amino acid sequence of SEQ ID NO: 218, H-CDR2 comprising the amino acid sequence of SEQ ID NO: 219, and H-CDR3 comprising the amino acid sequence of SEQ ID NO: 220 of atezolizumab, and may comprise a light chain variable region comprising L-CDR1 comprising the amino acid sequence of SEQ ID NO: 221, L-CDR2 comprising the amino acid sequence of SEQ ID NO: 222, and L-CDR3 comprising the amino acid sequence of SEQ ID NO: 223. In addition, it may comprise a heavy chain variable region comprising H-CDR1 comprising the amino acid sequence of SEQ ID NO: 224, H-CDR2 comprising the amino acid sequence of SEQ ID NO: 225, and H-CDR3 comprising the amino acid sequence of SEQ ID NO: 226 of avelumab, and may comprise a light chain variable region comprising L-CDR1 comprising the amino acid sequence of SEQ ID NO: 227, L-CDR2 comprising the amino acid sequence of SEQ ID NO: 228, and L-CDR3 comprising the amino acid sequence of SEQ ID NO: 229. In addition, it may comprise a heavy chain variable region comprising H-CDR1 comprising the amino acid sequence of SEQ ID NO: 230, H-CDR2 comprising the amino acid sequence of SEQ ID NO: 231, and H-CDR3 comprising the amino acid sequence of SEQ ID NO: 232 of durvalumab, and may comprise a light chain variable region comprising L-CDR1 comprising the amino acid sequence of SEQ ID NO: 233, L-CDR2 comprising the amino acid sequence of SEQ ID NO: 234, and L-CDR3 comprising the amino acid sequence of SEQ ID NO: 235.

As an embodiment of the present invention, it may include an antigen binding site that specifically binds to HER2. For example, it may comprise a heavy chain variable region comprising H-CDR1 comprising the amino acid sequence of SEQ ID NO: 21, H-CDR2 comprising the amino acid sequence of SEQ ID NO: 22, and H-CDR3 comprising the amino acid sequence of SEQ ID NO: 23 of trastuzumab, and may comprise a light chain variable region comprising L-CDR1 comprising the amino acid sequence of SEQ ID NO: 24, L-CDR2 comprising the amino acid sequence of SEQ ID NO: 25, and L-CDR3 comprising the amino acid sequence of SEQ ID NO: 26. In addition, it may comprise a heavy chain variable region comprising H-CDR1 comprising the amino acid sequence of SEQ ID NO: 33, H-CDR2 comprising the amino acid sequence of SEQ ID NO: 34, and H-CDR3 comprising the amino acid sequence of SEQ ID NO: 35 of pertuzumab, and may comprise a light chain variable region comprising L-CDR1 comprising the amino acid sequence of SEQ ID NO: 36, L-CDR2 comprising the amino acid sequence of SEQ ID NO: 37, and L-CDR3 comprising the amino acid sequence of SEQ ID NO: 38.

As an embodiment of the present invention, it may include an antigen binding site that specifically binds to c-Met. For example, it may comprise a heavy chain variable region comprising H-CDR1 comprising the amino acid sequence of SEQ ID NO: 236, H-CDR2 comprising the amino acid sequence of SEQ ID NO: 237, and H-CDR3 comprising the amino acid sequence of SEQ ID NO: 238 of onartuzumab, and may comprise a light chain variable region comprising L-CDR1 comprising the amino acid sequence of SEQ ID NO: 239, L-CDR2 comprising the amino acid sequence of SEQ ID NO: 240, and L-CDR3 comprising the amino acid sequence of SEQ ID NO: 241. In addition, it may comprise a heavy chain variable region comprising H-CDR1 comprising the amino acid sequence of SEQ ID NO: 242, H-CDR2 comprising the amino acid sequence of SEQ ID NO: 243, and H-CDR3 comprising the amino acid sequence of SEQ ID NO: 244 of emibetuzumab, and may comprise a light chain variable region comprising L-CDR1 comprising the amino acid sequence of SEQ ID NO: 245, L-CDR2 comprising the amino acid sequence of SEQ ID NO: 246, and L-CDR3 comprising the amino acid sequence of SEQ ID NO: 247. In addition, it may comprise a heavy chain variable region comprising H-CDR1 comprising the amino acid sequence of SEQ ID NO: 248, H-CDR2 comprising the amino acid sequence of SEQ ID NO: 249, and H-CDR3 comprising the amino acid sequence of SEQ ID NO: 250 of telisotuzumab, and may comprise a light chain variable region comprising L-CDR1 comprising the amino acid sequence of SEQ ID NO: 251, L-CDR2 comprising the amino acid sequence of SEQ ID NO: 252, and L-CDR3 comprising the amino acid sequence of SEQ ID NO: 253.

As an embodiment of the present invention, it may include an antigen binding site that specifically binds to Trop-2. For example, it may comprise a heavy chain variable region comprising H-CDR1 comprising the amino acid sequence of SEQ ID NO: 254, H-CDR2 comprising the amino acid sequence of SEQ ID NO: 255, and H-CDR3 comprising the amino acid sequence of SEQ ID NO: 256 of datopotamab, and may comprise a light chain variable region comprising L-CDR1 comprising the amino acid sequence of SEQ ID NO: 257, L-CDR2 comprising the amino acid sequence of SEQ ID NO: 258, and L-CDR3 comprising the amino acid sequence of SEQ ID NO: 259. In addition, it may comprise a heavy chain variable region comprising H-CDR1 comprising the amino acid sequence of SEQ ID NO: 260, H-CDR2 comprising the amino acid sequence of SEQ ID NO: 261, and H-CDR3 comprising the amino acid sequence of SEQ ID NO: 262 of sacituzumab, and may comprise a light chain variable region comprising L-CDR1 comprising the amino acid sequence of SEQ ID NO: 263, L-CDR2 comprising the amino acid sequence of SEQ ID NO: 264, and L-CDR3 comprising the amino acid sequence of SEQ ID NO: 265.

As an embodiment of the present invention, it may include an antigen binding site that specifically binds to DLL3.

For example, it may comprise a heavy chain variable region comprising H-CDR1 comprising the amino acid sequence of SEQ ID NO: 266, H-CDR2 comprising the amino acid sequence of SEQ ID NO: 267, and H-CDR3 comprising the amino acid sequence of SEQ ID NO: 268 of rovalpituzumab, and may comprise a light chain variable region comprising L-CDR1 comprising the amino acid sequence of SEQ ID NO: 269, L-CDR2 comprising the amino acid sequence of SEQ ID NO: 270, and L-CDR3 comprising the amino acid sequence of SEQ ID NO: 271. In addition, it may comprise a heavy chain variable region comprising H-CDR1 comprising the amino acid sequence of SEQ ID NO: 272, H-CDR2 comprising the amino acid sequence of SEQ ID NO: 273, and H-CDR3 comprising the amino acid sequence of SEQ ID NO: 274 of tarlatamab, and may comprise a light chain variable region comprising L-CDR1 comprising the amino acid sequence of SEQ ID NO: 275, L-CDR2 comprising the amino acid sequence of SEQ ID NO: 276, and L-CDR3 comprising the amino acid sequence of SEQ ID NO: 277.

As an embodiment of the present invention, it may include an antigen binding site that specifically binds to BCMA. For example, it may comprise a heavy chain variable region comprising H-CDR1 comprising the amino acid sequence of SEQ ID NO: 278, H-CDR2 comprising the amino acid sequence of SEQ ID NO: 279, and H-CDR3 comprising the amino acid sequence of SEQ ID NO: 280 of belantamab, and may comprise a light chain variable region comprising L-CDR1 comprising the amino acid sequence of SEQ ID NO: 281, L-CDR2 comprising the amino acid sequence of SEQ ID NO: 282, and L-CDR3 comprising the amino acid sequence of SEQ ID NO: 283.

As an embodiment of the present invention, it may include an antigen binding site that specifically binds to LIV-1. For example, it may comprise a heavy chain variable region comprising H-CDR1 comprising the amino acid sequence of SEQ ID NO: 284, H-CDR2 comprising the amino acid sequence of SEQ ID NO: 285, and H-CDR3 comprising the amino acid sequence of SEQ ID NO: 286 of ladiratuzumab, and may comprise a light chain variable region comprising L-CDR1 comprising the amino acid sequence of SEQ ID NO: 287, L-CDR2 comprising the amino acid sequence of SEQ ID NO: 288, and L-CDR3 comprising the amino acid sequence of SEQ ID NO: 289.

As an embodiment of the present invention, it may include an antigen binding site that specifically binds to GPC-3. For example, it may comprise a heavy chain variable region comprising H-CDR1 comprising the amino acid sequence of SEQ ID NO: 99, H-CDR2 comprising the amino acid sequence of SEQ ID NO: 100, and H-CDR3 comprising the amino acid sequence of SEQ ID NO: 101 of codrituzumab, and may comprise a light chain variable region comprising L-CDR1 comprising the amino acid sequence of SEQ ID NO: 102, L-CDR2 comprising the amino acid sequence of SEQ ID NO: 103, and L-CDR3 comprising the amino acid sequence of SEQ ID NO: 104.

As an embodiment of the present invention, it may include an antigen binding site that specifically binds to FGFR2. For example, it may comprise a heavy chain variable region comprising H-CDR1 comprising the amino acid sequence of SEQ ID NO: 290, H-CDR2 comprising the amino acid sequence of SEQ ID NO: 291, and H-CDR3 comprising the amino acid sequence of SEQ ID NO: 292 of aprutumab, and may comprise a light chain variable region comprising L-CDR1 comprising the amino acid sequence of SEQ ID NO: 293, L-CDR2 comprising the amino acid sequence of SEQ ID NO: 294, and L-CDR3 comprising the amino acid sequence of SEQ ID NO: 295. In addition, it may comprise a heavy chain variable region comprising H-CDR1 comprising the amino acid sequence of SEQ ID NO: 296, H-CDR2 comprising the amino acid sequence of SEQ ID NO: 297, and H-CDR3 comprising the amino acid sequence of SEQ ID NO: 298 of bemarituzumab, and may comprise a light chain variable region comprising L-CDR1 comprising the amino acid sequence of SEQ ID NO: 299, L-CDR2 comprising the amino acid sequence of SEQ ID NO: 300, and L-CDR3 comprising the amino acid sequence of SEQ ID NO: 301.

As an embodiment of the present invention, it may include an antigen binding site that specifically binds to FGFR3. For example, it may comprise a heavy chain variable region comprising H-CDR1 comprising the amino acid sequence of SEQ ID NO: 302, H-CDR2 comprising the amino acid sequence of SEQ ID NO: 303, and H-CDR3 comprising the amino acid sequence of SEQ ID NO: 304 of vofatamab, and may comprise a light chain variable region comprising L-CDR1 comprising the amino acid sequence of SEQ ID NO: 305, L-CDR2 comprising the amino acid sequence of SEQ ID NO: 306, and L-CDR3 comprising the amino acid sequence of SEQ ID NO: 307.

As an embodiment of the present invention, it may include an antigen binding site that specifically binds to VEGFR2. For example, it may comprise a heavy chain variable region comprising H-CDR1 comprising the amino acid sequence of SEQ ID NO: 308, H-CDR2 comprising the amino acid sequence of SEQ ID NO: 309, and H-CDR3 comprising the amino acid sequence of SEQ ID NO: 310 of ramucirumab, and may comprise a light chain variable region comprising L-CDR1 comprising the amino acid sequence of SEQ ID NO: 311, L-CDR2 comprising the amino acid sequence of SEQ ID NO: 312, and L-CDR3 comprising the amino acid sequence of SEQ ID NO: 313.

As an embodiment of the present invention, it may include an antigen binding site that specifically binds to CD20. For example, it may comprise a heavy chain variable region comprising H-CDR1 comprising the amino acid sequence of SEQ ID NO: 314, H-CDR2 comprising the amino acid sequence of SEQ ID NO: 315, and H-CDR3 comprising the amino acid sequence of SEQ ID NO: 316 of rituximab, and may comprise a light chain variable region comprising L-CDR1 comprising the amino acid sequence of SEQ ID NO: 317, L-CDR2 comprising the amino acid sequence of SEQ ID NO: 318, and L-CDR3 comprising the amino acid sequence of SEQ ID NO: 319. For example, it may comprise a heavy chain variable region comprising H-CDR1 comprising the amino acid sequence of SEQ ID NO: 320, H-CDR2 comprising the amino acid sequence of SEQ ID NO: 321, and H-CDR3 comprising the amino acid sequence of SEQ ID NO: 322 of obinutuzumab, and may comprise a light chain variable region comprising L-CDR1 comprising the amino acid sequence of SEQ ID NO: 323, L-CDR2 comprising the amino acid sequence of SEQ ID NO: 324, and L-CDR3 comprising the amino acid sequence of SEQ ID NO: 325.

As an embodiment of the present invention, it may include an antigen binding site that specifically binds to CD38. For example, it may comprise a heavy chain variable region comprising H-CDR1 comprising the amino acid sequence of SEQ ID NO: 326, H-CDR2 comprising the amino acid sequence of SEQ ID NO: 327, and H-CDR3 comprising the amino acid sequence of SEQ ID NO: 328 of daratumumab, and may comprise a light chain variable region comprising L-CDR1 comprising the amino acid sequence of SEQ ID NO: 329, L-CDR2 comprising the amino acid sequence of SEQ ID NO: 330, and L-CDR3 comprising the amino acid sequence of SEQ ID NO: 331.

As an embodiment of the present invention, it may include an antigen binding site that specifically binds to EphA2. For example, it may comprise a heavy chain variable region comprising H-CDR1 comprising the amino acid sequence of SEQ ID NO: 157, H-CDR2 comprising the amino acid sequence of SEQ ID NO: 158, and H-CDR3 comprising the amino acid sequence of SEQ ID NO: 159 of 1C1, and may comprise a light chain variable region comprising L-CDR1 comprising the amino acid sequence of SEQ ID NO: 160, L-CDR2 comprising the amino acid sequence of SEQ ID NO: 161, and L-CDR3 comprising the amino acid sequence of SEQ ID NO: 162.

Table 1 below shows the CDR sequences of non-limiting exemplary antibodies with anticancer efficacy that may be used in embodiments of the present invention.

[Table 1]

| Antibody | Heavy chain | | | Light chain | | |
|---|---|---|---|---|---|---|
| | CDR | Sequence | SEQ ID NO | CDR | Sequence | SEQ ID NO |
| Cetuximab | VH-CDR1 | NYGVH | 175 | VL-CDR1 | RASQSIGTNIH | 178 |
| | VH-CDR2 | VIWSGGNTDYNTPFTS | 176 | VL-CDR2 | YASESIS | 179 |
| | VH-CDR3 | ALTYYDYEFAY | 177 | VL-CDR3 | QQNNNWPTT | 180 |
| Panitumumab | VH-CDR1 | DYYWT | 181 | VL-CDR1 | QASQDISNYLN | 184 |
| | VH-CDR2 | HIYYSGNTNYNPSLKS | 182 | VL-CDR2 | DASNLET | 185 |
| | VH-CDR3 | DRVTGAFDI | 183 | VL-CDR3 | QHFDHLPLA | 186 |
| Necitumumab | VH-CDR1 | DYYWS | 187 | VL-CDR1 | RASQSVSSYLA | 190 |
| | VH-CDR2 | YIYYSGSTDYNPSLKS | 188 | VL-CDR2 | DASNRAT | 191 |
| | VH-CDR3 | VSIFGVGTFDY | 189 | VL-CDR3 | HQYGSTPLT | 192 |
| Imgatuzumab | VH-CDR1 | DYKIH | 193 | VL-CDR1 | RASQGINNYLN | 196 |
| | VH-CDR2 | YFNPNSGYSTYAQKFQG | 194 | VL-CDR2 | NTNNLQT | 197 |
| | VH-CDR3 | LSPGGYYVMDA | 195 | VL-CDR3 | LQHNSFPT | 198 |
| Depatuxizumab | VH-CDR1 | DFAWN | 199 | VL-CDR1 | HSSQDINSNIG | 202 |
| | VH-CDR2 | YISYSGNTRYQPSLKS | 200 | VL-CDR2 | HGTNLDD | 203 |
| | VH-CDR3 | AGRGFPY | 201 | VL-CDR3 | VQYAQFPWT | 204 |
| Losatuxizumab | VH-CDR1 | DFAWN | 199 | VL-CDR1 | HSSQDINSNIG | 202 |
| | VH-CDR2 | YISYNGNTRYQPSLKS | 205 | VL-CDR2 | HGTNLDD | 203 |
| | VH-CDR3 | ASRGFPY | 206 | VL-CDR3 | VQYAQFPWT | 204 |
| Etevritamab | VH-CDR1 | NYGMH | 207 | VL-CDR1 | RSSQSLVHSDGNTYLS | 210 |
| | VH-CDR2 | VIWYDGSDKYYADSVRG | 208 | VL-CDR2 | RISRRFS | 211 |
| | VH-CDR3 | DGYDILTGNPRDFDY | 209 | VL-CDR3 | MQSTHVPRT | 212 |
| AMG-595 | VH-CDR1 | SYGMH | 213 | VL-CDR1 | RSSQSLVHSDGNTYLS | 210 |
| | VH-CDR2 | VIWYDGSNKYYVDSVKG | 214 | VL-CDR2 | KISNRFS | 216 |
| | VH-CDR3 | DGWQQLAPFDY | 215 | VL-CDR3 | MQATQLPRT | 217 |
| Atezolizumab | VH-CDR1 | DSWIH | 218 | VL-CDR1 | RASQDVSTAVA | 221 |
| | VH-CDR2 | WISPYGGSTYYADSVKG | 219 | VL-CDR2 | SASFLYS | 222 |
| | VH-CDR3 | RHWPGGFDY | 220 | VL-CDR3 | QQYLYHPAT | 223 |
| Avelumab | VH-CDR1 | SSYIM | 224 | VL-CDR1 | TGTSSDVGGYNYVS | 227 |
| | VH-CDR2 | SIYPSGGITFYADTVKG | 225 | VL-CDR2 | DVSNRPS | 228 |
| | VH-CDR3 | IKLGTVTTVDY | 226 | VL-CDR3 | SSYTSSSTRV | 229 |
| Durvalumab | VH-CDR1 | RYWMS | 230 | VL-CDR1 | RASQRVSSSYLA | 233 |
| | VH-CDR2 | NIKQDGSEKYYVDSVKG | 231 | VL-CDR2 | DASSRAT | 234 |
| | VH-CDR3 | EGGWFGELAFDY | 232 | VL-CDR3 | QQYGSLPWT | 235 |
| Trastuzumab | VH-CDR1 | DTYIH | 21 | VL-CDR1 | RASQDVNTAVA | 24 |
| | VH-CDR2 | RIYPTNGYTRYADSVKG | 22 | VL-CDR2 | SASFLYS | 25 |
| | VH-CDR3 | WGGDGFYAMDY | 23 | VL-CDR3 | QQHYTTPPT | 26 |
| Pertuzumab | VH-CDR1 | DYTMD | 33 | VL-CDR1 | KASQDVSIGVA | 36 |
| | VH-CDR2 | DVNPNSGGSIYNQRFKG | 34 | VL-CDR2 | SASYRYT | 37 |
| | VH-CDR3 | NLGPSFYFDYW | 35 | VL-CDR3 | QQYYIYPYT | 38 |
| Onartuzumab | VH-CDR1 | SYWLH | 236 | VL-CDR1 | KSSQSLLYTSSQKNYLA | 239 |
| | VH-CDR2 | MIDPSNSDTRFNPNFKD | 237 | VL-CDR2 | WASTRES | 240 |
| | VII-CDR3 | YRSYVTPLDY | 238 | VL-CDR3 | QQYYAYPWT | 241 |
| Emibetuzumab | VH-CDR1 | DYYMH | 242 | VL-CDR1 | SVSSSVSSIYLH | 245 |
| | VH-CDR2 | RVNPNRRGTTYNQKFEG | 243 | VL-CDR2 | STSNLAS | 246 |

| | | | | | | |
|---|---|---|---|---|---|---|
| | VH-CDR3 | ANWLDY | 244 | VL-CDR3 | QVYSGYPLT | 247 |
| Telisotuzumab | VH-CDR1 | AYTMH | 248 | VL-CDR1 | KSSESVDSYANSFLH | 251 |
| | VH-CDR2 | WIKPNNGLANYAQKFQG | 249 | VL-CDR2 | RASTRES | 252 |
| | VH-CDR3 | SEITTEFDY | 250 | VL-CDR3 | QQSKEDPLT | 253 |
| Datopotamab | VH-CDR1 | TAGMQ | 254 | VL-CDR1 | KASQDVSTAVA | 257 |
| | VH-CDR2 | WINTHSGVPKYAEDFKG | 255 | VL-CDR2 | SASYRYT | 258 |
| | VH-CDR3 | SGFGSSYWYFDV | 256 | VL-CDR3 | QQHYITPLT | 259 |
| Sacituzumab | VH-CDR1 | NYGMN | 260 | VL-CDR1 | KASQDVSIAVA | 263 |
| | VH-CDR2 | WINTYTGEPTYTDDFKG | 261 | VL-CDR2 | SASYRYT | 264 |
| | VH-CDR3 | GGFGSSYWYFDV | 262 | VL-CDR3 | QQHYITPLT | 265 |
| Rovalpituzumab | VH-CDR1 | NYGMN | 266 | VL-CDR1 | KASQSVSNDVV | 269 |
| | VH-CDR2 | WINTYTGEPTYADDFKG | 267 | VL-CDR2 | YASNRYT | 270 |
| | VH-CDR3 | IGDSSPSDY | 268 | VL-CDR3 | QQDYTSPWT | 271 |
| Tarlatamab | VH-CDR1 | SYYWS | 272 | VL-CDR1 | RASQRVNNNYLA | 275 |
| | VH-CDR2 | YVYYSGTTNYNPSLKS | 273 | VL-CDR2 | GASSRAT | 276 |
| | VH-CDR3 | IAVTGFYFDY | 274 | VL-CDR3 | QQYDRSPLT | 277 |
| Belantamab | VH-CDR1 | NYWMH | 278 | VL-CDR1 | SASQDISNYLN | 281 |
| | VH-CDR2 | ATYRGHSDTYYNQKFKG | 279 | VL-CDR2 | YTSNLHS | 282 |
| | VH-CDR3 | GAIYDGYDVLDN | 280 | VL-CDR3 | QQYRKLPWT | 283 |
| Ladiratuzumab | VH-CDR1 | DYYMH | 284 | VL-CDR1 | RSSQSLLHSSGNTYLE | 287 |
| | VH-CDR2 | WIDPENGDTEYGPKFQG | 285 | VL-CDR2 | KISTRFS | 288 |
| | VH-CDR3 | HNAHYGTWFAY | 286 | VL-CDR3 | FQGSHVPYT | 289 |
| Codrituzumab | VH-CDR1 | DYEMH | 99 | VL-CDR1 | RSSQSLVHSNRNTYLH | 102 |
| | VH-CDR2 | ALDPKTGDTAYSQKFKG | 100 | VL-CDR2 | KVSNRFS | 103 |
| | VH-CDR3 | FYSYTY | 101 | VL-CDR3 | SQNTHVPPT | 104 |
| Aprutumab | VH-CDR1 | SYAMS | 290 | VL-CDR1 | SGSSSNIGNNYVS | 293 |
| | VH-CDR2 | AISGSGTSTYYADSVKG | 291 | VL-CDR2 | ENYNRPA | 294 |
| | VH-CDR3 | VRYNWNHGDWFDP | 292 | VL-CDR3 | SSWDDSLNYWV | 295 |
| Bemarituzumab | VH-CDR1 | TYNVH | 296 | VL-CDR1 | KASQGVSNDVA | 299 |
| | VH-CDR2 | SIYPDNGDTSYNQNFKG | 297 | VL-CDR2 | SASYRYT | 300 |
| | VH-CDR3 | GDFAY | 298 | VL-CDR3 | QQHSTTPYT | 301 |
| Vofatamab | VH-CDR1 | STGIS | 302 | VL-CDR1 | RASQDVDTSLA | 305 |
| | VH-CDR2 | RIYPTSGSTNYADSVKG | 303 | VL-CDR2 | SASFLYS | 306 |
| | VH-CDR3 | TYGIYDLYVDYTEYVMDY | 304 | VL-CDR3 | QQSTGHPQT | 307 |
| Ramucirumab | VH-CDR1 | SYSMN | 308 | VL-CDR1 | RASQGIDNWLG | 311 |
| | VH-CDR2 | SISSSSSYIYYADSVKG | 309 | VL-CDR2 | DASNLDT | 312 |
| | VH-CDR3 | VTDAFDI | 310 | VL-CDR3 | QQAKAFPPT | 313 |
| Rituximab | VH-CDR1 | SYNMH | 314 | VL-CDR1 | RSSKSLLHSNGITYLY | 317 |
| | VH-CDR2 | AIYPGNGDTSYNQKFKG | 315 | VL-CDR2 | QMSNLVS | 318 |
| | VH-CDR3 | STYYGGDWYFNV | 316 | VL-CDR3 | AQNLELPYT | 319 |
| Obinutuzumab | VH-CDR1 | YSWIN | 320 | VL-CDR1 | RASQSVSSYLA | 323 |
| | VH-CDR2 | RIFPGDGDTDYNGKFKG | 321 | VL-CDR2 | DASNRAT | 324 |
| | VH-CDR3 | NVFDGYWLVY | 322 | VL-CDR3 | QQRSNWPPT | 325 |
| Daratumumab | VH-CDR1 | SFAMS | 326 | VL-CDR1 | RASQSVSSYLA | 329 |
| | VH-CDR2 | AISGSGGGTYYADSVKG | 327 | VL-CDR2 | DASNRAT | 330 |
| | VH-CDR3 | DKILWFGEPVFDY | 328 | VL-CDR3 | QQRSNWPPT | 331 |
| 1C1 (Clone name) | VH-CDR1 | HYMMA | 157 | VL-CDR1 | RASQSISTWLA | 160 |
| | VH-CDR2 | RIGPSGGPTHYADSVKG | 158 | VL-CDR2 | KASNLHT | 161 |
| | VH-CDR3 | YDSGYDYVAVAGPAEYFQH | 159 | VL-CDR3 | QQYNSYSRT | 162 |

**Antigen**

As antigens to which the antigen binding site described herein may specifically bind, the following non-limiting substances may be exemplified.

"Epidermal growth factor receptor (EGFR)": It is a cell membrane receptor that regulates cell growth, division, survival, and death. In various cancers, the expression of EGFR is increased in tumor tissues. It is known that tumor tissues with the increased EGFR are invasive, metastatic, and highly resistant to anticancer agents. In one embodiment, the substance that inhibits EGFR may be cetuximab, panitumumab, necitumumab, imgatuzumab, depatuxizumab, or losatuxizumab, but is not limited thereto.

"Epidermal growth factor receptor variant 3 (EGFRvIII)": It is a mutation in which exons 2 to 7 of EGFR are deleted. It is mainly reported in glioblastoma multiforme, and most patients with EGFRvIII-positive mutation have a poor prognosis. In one embodiment, the substance that inhibits EGFRvIII may be cetuximab, panitumumab, necitumumab, imgatuzumab, depatuxizumab, losatuxizumab, etevritamab, or AMG-595, but is not limited thereto.

"Programmed cell death-ligand 1 (PD-L1)": It is a protein overexpressed on the surface of cancer cells. It is a major cancer-specific antigen that plays an important role in inducing exhaustion and apoptosis of T cells and acquiring immune tolerance in cancer cells. In one embodiment, PD-L1 targeting anticancer antibody may be atezolizumab, avelumab, or durvalumab, but is not limited thereto.

"HER-2/neu (human epidermal growth factor receptor 2)": It regulates cell proliferation through activation of PI3K/AkT. It is known that it is overexpressed in metastatic breast cancer and ovarian cancer, etc., and induces resistance against anticancer agents. The HER-2/neu targeting anticancer agent may be trastuzumab or pertuzumab, but is not limited thereto.

"c-Met (mesenchymal-epithelial transition factor)": It is a hepatocyte growth factor receptor. Its amplification or mutation is frequently reported in cancer cells, and it is known to promote tumor growth, metastasis, and malignancy. Specifically, the inhibitor of the protein may be onartuzumab, emibetuzumab, or telisotuzumab, but is not limited thereto.

"Trop-2 (tumor-associated calcium signal transducer 2)": It is a cellular glycoprotein related to cancer cell growth and proliferation. It is known to be specifically overexpressed in non-small cell lung cancer and breast cancer. Specifically, the antibody targeting the protein may be datopotamab or sacituzumab, but is not limited thereto.

"DLL3 (delta-like ligand 3)": It is a major cancer target antigen known to be expressed in approximately 85% of small cell lung cancer patients. Specifically, the antibody targeting the protein may be rovalpituzumab or tarlatamab, but is not limited thereto.

"BCMA (B-cell maturation antigen)": It is an important factor in the survival and proliferation of myeloma cells and is a clinically proven target for treatment of multiple myeloma. Specifically, the antibody targeting the protein may be belantamab, but is not limited thereto.

"LIV-1 (zinc transporter ZIP6)": It is a highly cancer-specific antigen overexpressed in breast cancer. Specifically, the antibody targeting the protein may be ladiratuzumab, but is not limited thereto.

"GPC-3 (glypican-3)": It is a highly cancer-specific antigen that is specifically overexpressed in liver cancer. Specifically, the antibody targeting the protein may be codrituzumab, but is not limited thereto.

"FGFR (fibroblast growth factor receptor)": It is a receptor for fibroblast growth factor (FGF), which regulates various biological processes including cell growth, differentiation, and migration. The FGFR gene is easily mutated, and these variants are commonly observed in breast cancer, uterine cancer, ovarian cancer, cervical cancer, and the like. The four FGFR genes are made of seven signaling receptors, of which FGFR2 and FGFR3 are highly cancer-specific antigens. The antibody targeting FGFR2 or FGFR3 may be aprutumab, bemarituzumab, or vofatamab, but is not limited thereto.

"Vascular endothelial growth factor receptor (VEGFR)": It is a cell membrane receptor for a vascular endothelial growth factor that induces angiogenesis. The VEGFR inhibitor inhibits tumor growth and metastasis by inhibiting angiogenesis. In one embodiment, the VEGFR inhibitor may be ramucirumab, but is not limited thereto.

"CD20 (B lymphocyte antigen CD20)": It is a protein expressed on the surface of B cells, which is used as a target protein for the treatment of B cell lymphoma. The CD20 inhibitor may be rituximab or obinutuzumab, but is not limited thereto.

"CD38 (cluster of differentiation 38)": It is a protein that acts as a signal transduction receptor in immune cells and regulates cell proliferation and death. The inhibitor targeting the protein may be daratumumab, but is not limited thereto.

"EphA2 (EPH receptor A2)": It is overexpressed in cancer cells and has a significant impact on the growth and metastasis of cancer cells. The antibody targeting the protein may be 1C1, but is not limited thereto.

The antigen binding site that specifically binds to the antigens exemplified above may include CDR sequences as exemplified below.

**EGFR:**

1) VH region (SEQ ID NO: 334) comprising the amino acid sequences of SEQ ID NO: 175 (VH-CDR1), SEQ ID NO: 176 (VH-CDR2) and SEQ ID NO: 177 (VH-CDR3), and VL region (SEQ ID NO: 335) comprising the amino acid sequences of SEQ ID NO: 178 (VL-CDR1), SEQ ID NO: 179 (VL-CDR2) and SEQ ID NO: 180 (VL-CDR3);

2) VH region (SEQ ID NO: 336) comprising the amino acid sequences of SEQ ID NO: 181 (VH-CDR1), SEQ ID NO: 182 (VH-CDR2) and SEQ ID NO: 183 (VH-CDR3), and VL region (SEQ ID NO: 337) comprising the amino acid sequences of SEQ ID NO: 184 (VL-CDR1), SEQ ID NO: 185 (VL-CDR2) and SEQ ID NO: 186 (VL-CDR3);

3) VH region (SEQ ID NO: 338) comprising the amino acid sequences of SEQ ID NO: 187 (VH-CDR1), SEQ ID NO: 188 (VH-CDR2) and SEQ ID NO: 189 (VH-CDR3), and VL region (SEQ ID NO: 339) comprising the amino acid sequences of SEQ ID NO: 190 (VL-CDR1), SEQ ID NO: 191 (VL-CDR2) and SEQ ID NO: 192 (VL-CDR3);

4) VH region (SEQ ID NO: 340) comprising the amino acid sequences of SEQ ID NO: 193 (VH-CDR1), SEQ ID NO: 194 (VH-CDR2) and SEQ ID NO: 195 (VH-CDR3), and VL region (SEQ ID NO: 341) comprising the amino acid sequences of SEQ ID NO: 196 (VL-CDR1), SEQ ID NO: 197 (VL-CDR2) and SEQ ID NO: 198 (VL-CDR3);

5) VH region (SEQ ID NO: 342) comprising the amino acid sequences of SEQ ID NO: 199 (VH-CDR1), SEQ ID NO: 200 (VH-CDR2) and SEQ ID NO: 201 (VH-CDR3), and VL region (SEQ ID NO: 343) comprising the amino acid sequences of SEQ ID NO: 202 (VL-CDR1), SEQ ID NO: 203 (VL-CDR2) and SEQ ID NO: 204 (VL-CDR3);
6) VH region (SEQ ID NO: 344) comprising the amino acid sequences of SEQ ID NO: 199 (VH-CDR1), SEQ ID NO: 205 (VH-CDR2) and SEQ ID NO: 206 (VH-CDR3), and VL region (SEQ ID NO: 345) comprising the amino acid sequences of SEQ ID NO: 202 (VL-CDR1), SEQ ID NO: 203 (VL-CDR2) and SEQ ID NO: 204 (VL-CDR3);

**EGFRvIII:**

7) VH region (SEQ ID NO: 346) comprising the amino acid sequences of SEQ ID NO: 207 (VH-CDR1), SEQ ID NO: 208 (VH-CDR2) and SEQ ID NO: 209 (VH-CDR3), and VL region (SEQ ID NO: 347) comprising the amino acid sequences of SEQ ID NO: 210 (VL-CDR1), SEQ ID NO: 211 (VL-CDR2) and SEQ ID NO: 212 (VL-CDR3);
8) VH region (SEQ ID NO: 348) comprising the amino acid sequences of SEQ ID NO: 213 (VH-CDR1), SEQ ID NO: 214 (VH-CDR2) and SEQ ID NO: 215 (VH-CDR3), and VL region (SEQ ID NO: 349) comprising the amino acid sequences of SEQ ID NO: 210 (VL-CDR1), SEQ ID NO: 216 (VL-CDR2) and SEQ ID NO: 217 (VL-CDR3);

**PD-L1:**

9) VH region (SEQ ID NO: 350) comprising the amino acid sequences of SEQ ID NO: 218 (VH-CDR1), SEQ ID NO: 219 (VH-CDR2) and SEQ ID NO: 220 (VH-CDR3), and VL region (SEQ ID NO: 351) comprising the amino acid sequences of SEQ ID NO: 221 (VL-CDR1), SEQ ID NO: 222 (VL-CDR2) and SEQ ID NO: 223 (VL-CDR3);
10) VH region (SEQ ID NO: 352) comprising the amino acid sequences of SEQ ID NO: 224 (VH-CDR1), SEQ ID NO: 225 (VH-CDR2) and SEQ ID NO: 226 (VH-CDR3), and VL region (SEQ ID NO: 353) comprising the amino acid sequences of SEQ ID NO: 227 (VL-CDR1), SEQ ID NO: 228 (VL-CDR2) and SEQ ID NO: 229 (VL-CDR3);
11) VH region (SEQ ID NO: 354) comprising the amino acid sequences of SEQ ID NO: 230 (VH-CDR1), SEQ ID NO: 231 (VH-CDR2) and SEQ ID NO: 232 (VH-CDR3), and VL region (SEQ ID NO: 355) comprising the amino acid sequences of SEQ ID NO: 233 (VL-CDR1), SEQ ID NO: 234 (VL-CDR2) and SEQ ID NO: 235 (VL-CDR3);

**HER2:**

12) VH region (SEQ ID NO: 356) comprising the amino acid sequences of SEQ ID NO: 21 (VH-CDR1), SEQ ID NO: 22 (VH-CDR2) and SEQ ID NO: 23 (VH-CDR3), and VL region (SEQ ID NO: 357) comprising the amino acid sequences of SEQ ID NO: 24 (VL-CDR1), SEQ ID NO: 25 (VL-CDR2) and SEQ ID NO: 26 (VL-CDR3);
13) VH region (SEQ ID NO: 27) comprising the amino acid sequences of SEQ ID NO: 33 (VH-CDR1), SEQ ID NO: 34 (VH-CDR2) and SEQ ID NO: 35 (VH-CDR3), and VL region (SEQ ID NO: 28) comprising the amino acid sequences of SEQ ID NO: 36 (VL-CDR1), SEQ ID NO: 37 (VL-CDR2) and SEQ ID NO: 38 (VL-CDR3);

**c-Met:**

14) VH region (SEQ ID NO: 358) comprising the amino acid sequences of SEQ ID NO: 236 (VH-CDR1), SEQ ID NO: 237 (VH-CDR2) and SEQ ID NO: 238 (VH-CDR3), and VL region (SEQ ID NO: 359) comprising the amino acid sequences of SEQ ID NO: 239 (VL-CDR1), SEQ ID NO: 240 (VL-CDR2) and SEQ ID NO: 241 (VL-CDR3);
15) VH region (SEQ ID NO: 360) comprising the amino acid sequences of SEQ ID NO: 242 (VH-CDR1), SEQ ID NO: 243 (VH-CDR2) and SEQ ID NO: 244 (VH-CDR3), and VL region (SEQ ID NO: 361) comprising the amino acid sequences of SEQ ID NO: 245 (VL-CDR1), SEQ ID NO: 246 (VL-CDR2) and SEQ ID NO: 247 (VL-CDR3);
16) VH region (SEQ ID NO: 362) comprising the amino acid sequences of SEQ ID NO: 248 (VH-CDR1), SEQ ID NO: 249 (VH-CDR2) and SEQ ID NO: 250 (VH-CDR3), and VL region (SEQ ID NO: 363) comprising the amino acid sequences of SEQ ID NO: 251 (VL-CDR1), SEQ ID NO: 252 (VL-CDR2) and SEQ ID NO: 253 (VL-CDR3);

**Trop-2:**

17) VH region (SEQ ID NO: 364) comprising the amino acid sequences of SEQ ID NO: 254 (VH-CDR1), SEQ ID NO: 255 (VH-CDR2) and SEQ ID NO: 256 (VH-CDR3), and VL region (SEQ ID NO: 365) comprising the amino acid sequences of SEQ ID NO: 257 (VL-CDR1), SEQ ID NO: 258 (VL-CDR2) and SEQ ID NO: 259 (VL-CDR3);
18) VH region (SEQ ID NO: 366) comprising the amino acid sequences of SEQ ID NO: 260 (VH-CDR1), SEQ ID NO: 261 (VH-CDR2) and SEQ ID NO: 262 (VH-CDR3), and VL region (SEQ ID NO: 367) comprising the amino acid sequences of SEQ ID NO: 263 (VL-CDR1), SEQ ID NO: 264 (VL-CDR2) and SEQ ID NO: 265 (VL-CDR3);

**DLL3:**

19) VH region (SEQ ID NO: 368) comprising the amino acid sequences of SEQ ID NO: 266 (VH-CDR1), SEQ ID NO: 267 (VH-CDR2) and SEQ ID NO: 268 (VH-CDR3), and VL region (SEQ ID NO: 369) comprising the amino acid sequences of SEQ ID NO: 269 (VL-CDR1), SEQ ID NO: 270 (VL-CDR2) and SEQ ID NO: 271 (VL-CDR3);
20) VH region (SEQ ID NO: 370) comprising the amino acid sequences of SEQ ID NO: 272 (VH-CDR1), SEQ ID NO: 273 (VH-CDR2) and SEQ ID NO: 274 (VH-CDR3), and VL region (SEQ ID NO: 371) comprising the amino acid sequences of SEQ ID NO: 275 (VL-CDR1), SEQ ID NO: 276 (VL-CDR2) and SEQ ID NO: 277 (VL-CDR3);

**BCMA:**
21) VH region (SEQ ID NO: 372) comprising the amino acid sequences of SEQ ID NO: 278 (VH-CDR1), SEQ ID NO: 279 (VH-CDR2) and SEQ ID NO: 280 (VH-CDR3), and VL region (SEQ ID NO: 373) comprising the amino acid sequences of SEQ ID NO: 281 (VL-CDR1), SEQ ID NO: 282 (VL-CDR2) and SEQ ID NO: 283 (VL-CDR3);
**LIV-1:**
22) VH region (SEQ ID NO: 374) comprising the amino acid sequences of SEQ ID NO: 284 (VH-CDR1), SEQ ID NO: 285 (VH-CDR2) and SEQ ID NO: 286 (VH-CDR3), and VL region (SEQ ID NO: 375) comprising the amino acid sequences of SEQ ID NO: 287 (VL-CDR1), SEQ ID NO: 288 (VL-CDR2) and SEQ ID NO: 289 (VL-CDR3);
**GPC-3:**
23) VH region (SEQ ID NO: 87) comprising the amino acid sequences of SEQ ID NO: 99 (VH-CDR1), SEQ ID NO: 100 (VH-CDR2) and SEQ ID NO: 101 (VH-CDR3), and VL region (SEQ ID NO: 88) comprising the amino acid sequences of SEQ ID NO: 102 (VL-CDR1), SEQ ID NO: 103 (VL-CDR2) and SEQ ID NO: 104 (VL-CDR3);
**FGFR2:**

24) VH region (SEQ ID NO: 376) comprising the amino acid sequences of SEQ ID NO: 290 (VH-CDR1), SEQ ID NO: 291 (VH-CDR2) and SEQ ID NO: 292 (VH-CDR3), and VL region (SEQ ID NO: 377) comprising the amino acid sequences of SEQ ID NO: 293 (VL-CDR1), SEQ ID NO: 294 (VL-CDR2) and SEQ ID NO: 295 (VL-CDR3);
25) VH region (SEQ ID NO: 378) comprising the amino acid sequences of SEQ ID NO: 296 (VH-CDR1), SEQ ID NO: 297 (VH-CDR2) and SEQ ID NO: 298 (VH-CDR3), and VL region (SEQ ID NO: 379) comprising the amino acid sequences of SEQ ID NO: 299 (VL-CDR1), SEQ ID NO: 300 (VL-CDR2) and SEQ ID NO: 301 (VL-CDR3);

**FGFR3:**
26) VH region (SEQ ID NO: 380) comprising the amino acid sequences of SEQ ID NO: 302 (VH-CDR1), SEQ ID NO: 303 (VH-CDR2) and SEQ ID NO: 304 (VH-CDR3), and VL region (SEQ ID NO: 381) comprising the amino acid sequences of SEQ ID NO: 305 (VL-CDR1), SEQ ID NO: 306 (VL-CDR2) and SEQ ID NO: 307 (VL-CDR3);
**VEGFR2:**
27) VH region (SEQ ID NO: 382) comprising the amino acid sequences of SEQ ID NO: 308 (VH-CDR1), SEQ ID NO: 309 (VH-CDR2) and SEQ ID NO: 310 (VH-CDR3), and VL region (SEQ ID NO: 383) comprising the amino acid sequences of SEQ ID NO: 311 (VL-CDR1), SEQ ID NO: 312 (VL-CDR2) and SEQ ID NO: 313 (VL-CDR3);
**CD20:**

28) VH region (SEQ ID NO: 384) comprising the amino acid sequences of SEQ ID NO: 314 (VH-CDR1), SEQ ID NO: 315 (VH-CDR2) and SEQ ID NO: 316 (VH-CDR3), and VL region (SEQ ID NO: 385) comprising the amino acid sequences of SEQ ID NO: 317 (VL-CDR1), SEQ ID NO: 318 (VL-CDR2) and SEQ ID NO: 319 (VL-CDR3);
29) VH region (SEQ ID NO: 386) comprising the amino acid sequences of SEQ ID NO: 320 (VH-CDR1), SEQ ID NO: 321 (VH-CDR2) and SEQ ID NO: 322 (VH-CDR3), and VL region (SEQ ID NO: 387) comprising the amino acid sequences of SEQ ID NO: 323 (VL-CDR1), SEQ ID NO: 324 (VL-CDR2) and SEQ ID NO: 325 (VL-CDR3);

**CD38:**
30) VH region (SEQ ID NO: 388) comprising the amino acid sequences of SEQ ID NO: 326 (VH-CDR1), SEQ ID NO: 327 (VH-CDR2) and SEQ ID NO: 328 (VH-CDR3), and VL region (SEQ ID NO: 389) comprising the amino acid sequences of SEQ ID NO: 329 (VL-CDR1), SEQ ID NO: 330 (VL-CDR2) and SEQ ID NO: 331 (VL-CDR3);
**EphA2:**
31) VH region (SEQ ID NO: 143) comprising the amino acid sequences of SEQ ID NO: 157 (VH-CDR1), SEQ ID NO: 158 (VH-CDR2) and SEQ ID NO: 159 (VH-CDR3), and VL region (SEQ ID NO: 145) comprising the amino acid sequences of SEQ ID NO: 160 (VL-CDR1), SEQ ID NO: 161 (VL-CDR2) and SEQ ID NO: 162 (VL-CDR3).

Table 2 below shows the polynucleotide sequence and the polypeptide sequence of an exemplary signal sequence for efficient expression of the fusion proteins according to various embodiments. When the above antibodies are expressed in mammalian cells, the amino acid sequence of SEQ ID NO: 333 may be included as the signal sequence, but is not limited thereto.

[Table 2]

| Name | Sequence | SEQ ID NO |
|------|----------|-----------|
| Polynucleotide sequence | ATGACACGCCTCACAGTTCTTGCCTTGCTGGCTGGACTTCTT GCCTCATCCAGGGCA | 332 |
| Polypeptide sequence | MTRLTVLALLAGLLASSRA | 333 |

Table 3 below shows the variable region polypeptide sequences of anticancer antibodies, which are described as antigen binding sites of various fusion proteins described herein. The fusion proteins according to exemplary embodiments may comprise or consist of these variable region polypeptides.

[Table 3]

| Antigen | Antibody name | Variable region | Variable region polypeptide sequence | SEQ ID NO |
|---|---|---|---|---|
| EGFR | Cetuximab | VH | QVQLKQSGPGLVQPSQSLSITCTVSGFSLTNYGVHWVRQSPGKGLEWLGVIWSGGNTDYNTPFTSRLSINKDNSKSQVFFKMNSLQSNDTAIYYCARALTYYDYEFAYWGQGTLVTVSA | 334 |
| | | VL | DILLTQSPVILSVSPGERVSFSCRASQSIGTNIHWYQQRTNGSPRLLIKYASESISGIPSRFSGSGSGTDFTLSINSVESEDIADYYCQQNNNWPTTFGAGTKLELK | 335 |
| | Panitumumab | VH | QVQLQESGPGLVKPSETLSLTCTVSGGSVSSGDYYWTWIRQSPGKGLEWIGHIYYSGNTNYNPSLKSRLTISIDTSKTQFSLKLSSVTAADTAIYYCVRDRVTGAFDIWGQGTMVTVSS | 336 |
| | | VL | DIQMTQSPSSLSASVGDRVTITCQASQDISNYLNWYQQKPGKAPKLLIYDASNLETGVPSRFSGSGSGTDFTFTISSLQPEDIATYFCQHFDHLPLAFGGGTKVEIK | 337 |
| | Necitumumab | VH | QVQLQESGPGLVKPSQTLSLTCTVSGGSISSGDYYWSWIRQPPGKGLEWIGYIYYSGSTDYNPSLKSRVTMSVDTSKNQFSLKVNSVTAADTAVYYCARVSIFGVGTFDYWGQGTLVTVSS | 338 |
| | | VL | EIVMTQSPATLSLSPGERATLSCRASQSVSSYLAWYQQKPGQAPRLLIYDASNRATGIPARFSGSGSGTDFTLTISSLEPEDFAVYYCIRYGSTPLTFGGGTKAEIK | 339 |
| | Imgatuzumab | VH | QVQLVQSGAEVKKPGSSVKVSCKASGFTFTDYKIHWVRQAPGQGLEWMGYFNPNSGYSTYAQKFQGRVTITADKSTSTAYMELSSLRSEDTAVYYCARLSPGGYYVMDAWGQGTTVTVSS | 340 |
| | | VL | DIQMTQSPSSLSASVGDRVTITCRASQGINNYLNWYQQKPGKAPKRLIYNTNNLQTGVPSRFSGSGSGTEFTLTISSLQPEDFATYYCLQHNSFPTFGQGTKLEIK | 341 |
| | Depatuxizumab | VH | QVQLQESGPGLVKPSQTLSLTCTVSGYSISSDFAWNWIRQPPGKGLEWMGYISYSGNTRYQPSLKSRITISRDTSKNQFFLKLNSVTAADTATYYCVTAGRGFPYWGQGTLVTVSS | 342 |
| | | VL | DIQMTQSPSSMSVSVGDRVTITCHSSQDINSNIGWLQQKPGKSFKGLIYHGTNLDDGVPSRFSGSGSGTDYTLTISSLQPEDFATYYCVQYAQFPWTFGGGTKLEIK | 343 |
| | Losatuxizumab | VH | EVQLQESGPGLVKPSQTLSLTCTVSGYSISRDFAWNWIRQPPGKGLEWMGYISYSGNTRYQPSLKSRITISRDTSKNQFFLKLNSVTAADTATYYCVTASRGFPYWGQGTLVTVSS | 344 |
| | | VL | DIQMTQSPSSMSVSVGDRVTITCHSSQDINSNIGWLQQKPGKSFKGLIYHGTNLDDGVPSRFSGSGSGTDYTLTISSLQPEDFATYYCVQYAQFPWTFGGGTKLEIK | 345 |
| EGFRvIII | Elevritamab | VH | QVQLVESGGGVVQSGRSLRLSCAASGFTFRSYGMHWVRQAPGKGLEWVAVIWYDGSDKYYADSVKGRFTISRDNSKNTLYLQMNSLRAEDTAVYYCARDGYDILTGNPRDFDYWGQGTLVTVSS | 346 |
| | | VL | DTVMTQTPLSSHVTLGQPASISCRSSQSLVHSDGNTYLSWLQQRPGQPPRLLIYRISRRFSGVPDRFSGSGAGTDFTLEISRVEAEDVGVYYCMQSTHVPRTFGCGTKVEIK | 347 |
| | AMG-595 | VH | QVQLVESGGGVVQPGRSLRLSCAASGFTPSSYGMHWVRQAPGKGLEWVAVIWYDGSNKYYVDSVKGRFTISRDNSKNTLYLQMNSLRAEDTAVYYCARDGWQQLAPFDYWGQGTLVTVSS | 348 |

| | | | | |
|---|---|---|---|---|
| | | VL | DIVMTQTPLSSPVTLGQPASISCRSSQSLVHSDGNTYLSWLHQRPGQPPRLLIYKISNRFSGVPDRFSGSGAGTAFTLKISRVEAEDVGVYYCMQATQLPRTFGQGTKVEIK | 349 |
| PD-L1 | Atezolizumab | VH | EVQLVESGGGLVQPGGSLRLSCAASGFTFSDSWIHWVRQAPGKGLEWVAWISPYGGSTYYADSVKGRFTISADTSKNTAYLQMNSLRAEDTAVYYCARRHWPGGFDYWGQGTLVTVSS | 350 |
| | | VL | DIQMTQSPSSLSASVGDRVTITCRASQDVSTAVAWYQQKPGKAPKLLIYSASFLYSGVPSRFSGSGSGTDFTLTISSLQPEDFATYYCQQYLYHPATFGQGTKVEIK | 351 |
| | Avelumab | VH | EVQLLESGGGLVQPGGSLRLSCAASGFTFSSYIMMWVRQAPGKGLEWVSSIYPSGGITFYADTVKGRFTISRDNSKNTLYLQMNSLRAEDTAVYYCARIKLGTVTTVDYWGQGTLVTVSS | 352 |
| | | VL | QSALTQPASVSGSPGQSITISCTGTSSDVGGYNYVSWYQQHPGKAPKLMIYDVSNRPSGVSNRFSGSKSGNTASLTISGLQAEDEADYYCSSYTSSSTRVFGTGTKVTVL | 353 |
| | Durvalumab | VH | EVQLVESGGGLVQPGGSLRLSCAASGFTFSRYWMSWVRQAPGKGLEWVANIKQDGSEKYYVDSVKGRFTISRDNAKNSLYLQMNSLRAEDTAVYYCAREGGWFGELAFDYWGQGTLVTVSS | 354 |
| | | VL | EIVLTQSPGTLSLSPGERATLSCRASQRVSSSYLAWYQQKPGQAPRLLIYDASSRATGIPDRFSGSGSGTDFTLTISRLEPEDFAVYYCQQYGSLPWTFGQGTKVEIK | 355 |
| HER2 | Trastuzumab | VH | EVQLVESGGGLVQPGGSLRLSCAASGFNIKDTYIHWVRQAPGKGLEWVARIYPTNGYTRYADSVKGRFTISADTSKNTAYLQMNSLRAEDTAVYYCSRWGGDGFYAMDYWGQGTLVTVSS | 356 |
| | | VL | DIQMTQSPSSLSASVGDRVTITCRASQDVNTAVAWYQQKPGKAPKLLIYSASFLYSGVPSRFSGSRSGTDFTLTISSLQPEDFATYYCQQHYTTPPTFGQGTKVEIK | 357 |
| | Pertuzumab | VH | EVQLVESGGGLVQPGGSLRLSCAASGFTFTDYTMDWVRQAPGKGLEWVADVNPNSGGSIYNQRFKGRFTLSVDRSKNTLYLQMNSLRAEDTAVYYCARNLGPSFYFDYWGQGTLVTVSS | 27 |
| | | VL | DIQMTQSPSSLSASVGDRVTITCKASQDVSIGVAWYQQKPGKAPKLLIYSASYRYTGVPSRFSGSGSGTDFTLTISSLQPEDFATYYCQQYYIYPYTFGQGTKVEIK | 28 |
| c-Met | Onartuzumab | VH | EVQLVESGGGLVQPGGSLRLSCAASGYTFTSYWLHWVRQAPGKGLEWVGMIDPSNSDTRFNPNFKDRFTISADTSKNTAYLQMNSLRAEDTAVYYCATYRSYVTPLDYWGQGTLVTVSS | 358 |
| | | VL | DIQMTQSPSSLSASVGDRVTITCKSSQSLLYTSSQKNYLAWYQQKPGKAPKLLIYWASTRESGVPSRFSGSGSGTDFTLTISSLQPEDFATYYCQQYYAYPWTFGQGTKVEIK | 359 |
| | Emibetuzumab | VH | QVQLVQSGAEVKKPGASVKVSCKASGYTFTDYYMHWVRQAPGQGLEWMGRVNPNRRGTTYNQKFEGRVTMTTDTSTSTAYMELRSLRSDDTAVYYCARANWLDYWGQGTTVTVSS | 360 |
| | | VL | DIQMTQSPSSLSASVGDRVTITCSVSSSVSSIYLHWYQQKPGKAPKLLIYSTSNLASGVPSRFSGSGSGTDFTLTISSLQPEDFATYYCQVYSGYPLTFGGGTKVEIK | 361 |
| | Telisotuzumab | VH | QVQLVQSGAEVKKPGASVKVSCKASGYIFTAYTMHWVRQAPGQGLEWMGWIKPNNGLANYAQKFQGRVTMTRDTSISTAYMELSRLRSDDTAVYYCARSEITTEFDYWGQGTLVTVSS | 362 |
| | | VL | DIVMTQSPDSLAVSLGERATINCKSSESVDSYANSFLHWYQQKPGQPPK | 363 |

21

| | | | | |
|---|---|---|---|---|
| | | | LLIYRASTRESGVPDRFSGSGSGTDFTLTISSLQAEDVAVYYCQQSKED PLTFGGGTKVEIK | |
| TROP-2 | Datopotama b | VH | QVQLVQSGAEVKKPGASVKVSCKASGYTFTTAGMQWVRQAPGQGLEWMG WINTHSGVPKYAEDFKGRVTISADTSTSTAYLQLSSLKSEDTAVYYCAR SGFGSSYWYFDVWGQGTLVTVSS | 364 |
| | | VL | DIQMTQSPSSLSASVGDRVTITCKASQDVSTAVAWYQQKPGKAPKLLIY SASYRYTGVPSRFSGSGSGTDFTLTISSLQPEDFAVYYCQQHYITPLTF GQGTKLEIK | 365 |
| | Sacituzuma b | VH | QVQLQQSGSELKKPGASVKVSCKASGYTFTNYGMNWVKQAPGQGLKWMG WINTYTGEPTYTDDFKGRFAFSLDTSVSTAYLQISSLKADDTAVYFCAR GGFGSSYWYFDVWGQGSLVTVSS | 366 |
| | | VL | DIQLTQSPSSLSASVGDRVSITCKASQDVSIAVAWYQQKPGKAPKLLIY SASYRYTGVPDRFSGSGSGTDFTLTISSLQPEDFAVYYCQQHYITPLTF GAGTKVEIK | 367 |
| DLL3 | Rovalpituz umab | VH | QVQLVQSGAEVKKPGASVKVSCKASGYTFTNYGMNWVRQAPGQGLEWMG WINTYTGEPTYADDFKGRVTMTTDTSTSTAYMELRSLRSDDTAVYYCAR IGDSSPSDYWGQGTLVTVSS | 368 |
| | | VL | EIVMTQSPATLSVSPGERATLSCKASQSVSNDVVWYQQKPGQAPRLLIY YASNRYTGIPARFSGSGSGTEFTLTISSLQSEDFAVYYCQQDYTSPWTF GQGTKLEIK | 369 |
| | Tarlatamab | VH | QVQLQESGPGLVKPSETLSLTCTVSGGSISSYYWSWIRQPPGKCLEWIG YVYYSGTTNYNPSLKSRVTISVDTSKNQFSLKLSSVTAADTAVYYCASI AVTGFYFDYWGQGTLVTVSS | 370 |
| | | VL | EIVLTQSPGTLSLSPGERVTLSCRASQRVNNNYLAWYQQRPGQAPRLLI YGASSRATGIPDRFSGSGSGTDFTLTISRLEPEDFAVYYCQQYDRSPLT FGCGTKLEIK | 371 |
| BCMA | Belantamab | VH | QVQLVQSGAEVKKPGSSVKVSCKASGGTFSNYWMHWVRQAPGQGLEWMG ATYRGHSDTYYNQKFKGRVTITADKSTSTAYMELSSLRSEDTAVYYCAR GAIYDGYDVLDNWGQGTLVTVSS | 372 |
| | | VL | DIQMTQSPSSLSASVGDRVTITCSASQDISNYLNWYQQKPGKAPKLLIY YTSNLHSGVPSRFSGSGSGTDFTLTISSLQPEDFATYYCQQYRKLPWTF GQGTKLEIK | 373 |
| LIV-1 | Ladiratuzu mab | VH | QVQLVQSGAEVKKPGASVKVSCKASGLTIEDYYMHWVRQAPGQGLEWMG WIDPENGDTEYGPKFQGRVTMTRDTSINTAYMELSRLRSDDTAVYYCAV HNAHYGTWFAYWGQGTLVTVSS | 374 |
| | | VL | DVVMTQSPLSLPVTLGQPASISCRSSQSLLHSSGNTYLEWYQQRPGQSP RPLIYKISTRFSGVPDRFSGSGSGTDFTLKISRVEAEDVGVYYCFQGSH VPYTFGGGTKVEIK | 375 |
| GPC-3 | Codrituzum ab | VH | QVQLVQSGAEVKKPGASVKVSCKASGYTFTDYEMHWVRQAPGQGLEWMG ALDPKTGDTAYSQKFKGRVTLTADKSTSTAYMELSSLTSEDTAVYYCTR FYSYTYWGQGTLVTVSS | 87 |
| | | VL | DVVMTQSPLSLPVTPGEPASISCRSSQSLVHSNRNTYLHWYLQKPGQSP QLLIYKVSNRFSGVPDRFSGSGSGTDFTLKISRVEAEDVGVYYCSQNTH VPPTFGQGTKLEIK | 88 |
| FGFR2 | Aprutumab | VH | EVQLLESGGGLVQPGGSLRLSCAASGFTFSSYAMSWVRQAPGKGLEWVS AISGSGTSTYYADSVKGRFTISRDNSKNTLYLQMNSLRAEDTAVYYCAR VRYNWNHGDWFDPWGQGTLVTVSS | 376 |
| | | VL | QSVLTQPPSASGTPGQRVTISCSGSSSNIGNNYVSWYQQLPGTAPKLLI YENYNRPAGVPDRFSGSKSGTSASLAISGLRSEDEADYYCSSWDDSLNY | 377 |

| | | | | |
|---|---|---|---|---|
| | | | WVFGGGTKLTVL | |
| | Bemarituzumab | VH | QVQLVQSGAEVKKPGSSVKVSCKASGYIFTTYNVHWVRQAPGQGLEWIG SIYPDNGDTSYNQNFKGRATITADKSTSTAYMELSSLRSEDTAVYYCAR GDFAYWGQGTLVTVSS | 378 |
| | | VL | DIQMTQSPSSLSASVGDRVTITCKASQGVSNDVAWYQQKPGKAPKLLIY SASYRYTGVPSRFSGSGSGTDFTFTISSLQPEDIATYYCQQHSTTPYTF GQGTKLEIK | 379 |
| FGFR3 | Vofatamab | VH | EVQLVESGGGLVQPGGSLRLSCAASGFTFTSTGISWVRQAPGKGLEWVG RIYPTSGSTNYADSVKGRFTISADTSKNTAYLQMNSLRAEDTAVYYCAR TYGIYDLYVDYTEYVMDYWGQGTLVTVSS | 380 |
| | | VL | DIQMTQSPSSLSASVGDRVTITCRASQDVDTSLAWYKQKPGKAPKLLIY SASFLYSGVPSRFSGSGSGTDFTLTISSLQPEDFATYYCQQSTGHPQTF GQGTKVEIK | 381 |
| VEGFR2 | Ramucirumab | VH | EVQLVQSGGGLVKPGGSLRLSCAASGFTFSSYSMNWVRQAPGKGLEWVS SISSSSSYIYYADSVKGRFTISRDNAKNSLYLQMNSLRAEDTAVYYCAR VTDAFDIWGQGTMVTVSS | 382 |
| | | VL | DIQMTQSPSSVSASIGDRVTITCRASQGIDNWLGWYQQKPGKAPKLLIY DASNLDTGVPSRFSGSGSGTYFTLTISSLQAEDFAVYFCQQAKAFPPTF GGGTKVDIK | 383 |
| CD20 | Rituximab | VH | QVQLQQPGAELVKPGASVKMSCKASGYTFTSYNMHWVKQTPGRGLEWIG AIYPGNGDTSYNQKFKGKATLTADKSSSTAYMQLSSLTSEDSAVYYCAR STYYGGDWYFNVWGAGTTVTVSA | 384 |
| | | VL | QIVLSQSPAILSASPGEKVTMTCRASSSVSYIHWFQQKPGSSPKPWIYA TSNLASGVPVRFSGSGSGTSYSLTISRVEAEDAATYYCQQWTSNPPTFG GGTKLEIK | 385 |
| | Obinutuzumab | VH | QVQLVQSGAEVKKPGSSVKVSCKASGYAFSYSWINWVRQAPGQGLEWMG RIFPGDGDTDYNGKFKGRVTITADKSTSTAYMELSSLRSEDTAVYYCAR NVFDGYWLVYWGQGTLVTVSS | 386 |
| | | VL | DIVMTQTPLSLPVTPGEPASISCRSSKSLLHSNGITYLYWYLQKPGQSP QLLIYQMSNLVSGVPDRFSGSGSGTDFTLKISRVEAEDVGVYYCAQNLE LPYTFGGGTKVEIK | 387 |
| CD38 | Daratumumab | VH | EVQLLESGGGLVQPGGSLRLSCAVSGFTFNSFAMSWVRQAPGKGLEWVS AISGSGGGTYYADSVKGRFTISRDNSKNTLYLQMNSLRAEDTAVYFCAK DKILWFGEPVFDYWGQGTLVTVSS | 388 |
| | | VL | EIVLTQSPATLSLSPGERATLSCRASQSVSSYLAWYQQKPGQAPRLLIY DASNRATGIPARFSGSGSGTDFTLTISSLEPEDFAVYYCQQRSNWPPTF GQGTKVEIK | 389 |
| EphA2 | 1C1 | VH | EVQLLESGGGLVQPGGSLRLSCAASGFTFSHYMMAWVRQAPGKGLEWVS RIGPSGGPTHYADSVKGRFTISRDNSKNTLYLQMNSLRAEDTAVYYCAG YDSGYDYVAVAGPAEYFQHWGQGTLVTVSS | 143 |
| | | VL | DIQMTQSPSSLSASVGDRVTITCRASQSISTWLAWYQQKPGKAPKLLIY KASNLHTGVPSRFSGSGSGTEFSLTISGLQPDDFATYYCQQYNSYSRTF GQGTKVEIK | 145 |

Table 4 below shows the polynucleotide sequences encoding polypeptides of variable regions of anticancer antibodies, which are described as antigen binding sites of various fusion proteins described herein.

[Table 4]

| Antigen | Antibody name | Variable region | Variable region polypeptide sequence | SEQ ID NO |
|---|---|---|---|---|
| EGFR | Cetuximab | VH | CAAGTGCAGCTTAAACAGAGTGGACCAGGGCTCGTTCAGCCTTCTCAAAGT TTGAGCCATAACATGTACCGTAAGTGGTTTTTCACTGACTAACTACGGAGTG CATTGGGTTCGACAGTCCCCTGGAAAGGGGTCTCGAATGGCTTGGAGTTATT TGGAGTGGCGGTAACACTGACTACAACACACCATTCACCTCTCGGCTGTCT ATAAACAAGGATAACTCTAAGTCACAGGTCTTTTTCAAGATGAACAGCCTC CAAAGTAACGACACCGCCATCTATTACTGTGCAAGAGCCTTGACCTACTAC GATTACGAGTTTGACTATTGGGGTCAGGGAACTCTGGTAACAGTCAGTGCT | 390 |
| | | VL | GACATCCTTCTCACTCAGTCCCCCGTAATTCTGAGCCGTGTCTCCCGGCGAG CGCGTCAGTTTCTCATGCAGGGCTAGCCAGAGTATAGGTACTAACATTCAT TGGTATCAACAGCGAACCAACGGCTCACCCCGGCTTGCTTATCAAATATGCC TCTGAGAGTATCTCTGGCATTCCATCCCGTTTTAGCGGTAGTGGTTCAGGA ACAGATTTCACTCTTTCAATTAACTCTGTAGAATCAGAAGACATAGCCGAT TATTATTGTCAGCAAAACAACAATTGGCCTACTACATTTGGAGCTGGTACA AAACTGGAACTCAAG | 391 |
| | Panitumumab | VH | CAGGTACAACTGCAAGAGAGCGGACCTGGGTTGGTAAAGCCAAGTGAGACC CTGTCACTTACTTGCACAGTATCAGGAGGAAGTGTCTCAAGTGGCCGATTAC TATTGGACTTGGATTCGACAAAGCCCCGGAAAAGGATTGGAATGGATCGGC CACATATATTATTCAGGAAATACCAACTATAATCCCAGTCTTAAATCTCGG CTTACCATTAGCATTGACACTTCCAAAAACCCAATTTTCACTTAAACTGAGT TCTGTGACTGCTGCCGACACTGCTATATACTACTGCGTCCGCGACCGGGTC ACTGGGGCTTTTGATATATTGGGGACAAGGAACTATGGTTACTGTATCATCT | 392 |
| | | VL | GATATTCAAATGACCCAATCTCCTTCATCACTCTCAGCATCAGTGGGGGAT AGAGTGACAATAACTTGCCAAGCAAGCCAGGATATATCTAATTATCTCAAC TGGTATCAACAAAAGCCAGGTAAAGCTCCAAAGTTGTTGATTTACCATGCC TCTAATCTCGAGACCGGCGTACCAAGTAGGTTCAGTGGTTCAGGAAGTGGG ACTGATTTCACTTTTACCATTTCTTCTCTCCAGCCCGAAGACATAGCAACT TATTTCTGTCAACACTTCGATCATCTTCCTTTGGCATTTGGTGGCGGCACT AAAGTTGAAATCAAG | 393 |
| | Necitumumab | VH | CAGGTACAACTCCAGGAATCCGGTCCTGGTCTCGTAAAGCCAAGCCAAACA TTGAGTTTGACCTGCACAGTCAGTGGTGGCAGTATTTCCTCAGGCGATTAT TACTGGAGTTGGATACGCCAGCCACCCGGTAAGGGGATTGGAATGGATCGGT TATATATACTACTCTGGATCTACAGACTATAATCCTTCCCTGAAGTCCAGG GTTACCATGAGTGTTGACACAATCCAAAAACCAGTTCTCCTTGAAGGTCAAC TCTGTGACCGCTGCCGATACTGCCGGTTTATTATTGTGCCACGTGTATCTATC TTCGGAGTAGGCACCTTTGACTATTGCGGTCAAGGCACCCTGGTCACCGTG TCATCT | 394 |
| | | VL | GAGATAGTAATGACACAGTCACCTGCTACCCCTTTCCCTCAGCCCTGGCGAA CGCGCCACCTTGTCTTGCAGGGCTAGCCAAAGTGTATCCTCTTATTGGCA TGGTATCAGCAGAAACCAGGGCAAGCCCCACCGGCTCCTGATTTATGATGCC TCCAATCGAGCCACCGGGATTCCTGCCCGATTCAGCGGATCAGGCAGTGGG ACTGATTTTACATTGACCATTAGCTCTCTGGAGCCTGAGGACTTTGCCGTG TATTATTGTCATCAATATGGGAGTACTCCCCTCACATTTGGCCGGCGGGACA AAAGCCAGAAATTAAA | 395 |
| | Imgatuzumab | VH | CAAGTACAGTTGGTACAAAGTGGGGCCGAGGTCAAGAAACCTGGTTCTAGC GTAAAGGTTAGCTGCAAGGCAAGCGGCTTCACCTTCACCGATTACAAAATT | 396 |

| | | | | |
|---|---|---|---|---|
| | | | CATTGGGTCCGTCAAGCCCCCGGTCAGGGGCTTGAATGGATGGGTTATTTC AACCCCAACTCTGGTTATTCTACCTATGCACAGAAGTTCCAGGGCCGCGTC ACTATAACTGCAGACAAGTCCACAAGTACAGCCTATATGGAACTGTCCAGT TTGCGTAGCGAGGATACTGCCGTATATTACTGTGCTAGACTCTCACCCGGC GGATATTACGTTATGGATGCATGGGGCCAGGGCACCACCGTGACCGTGAGC AGC | |
| | | VL | GACATCCAAATGACACAGTCTCCTTCCTCACTCTCCGCTTCTGTAGGAGAT AGAGTGACAATAACATGCCGTGCTTCACAAGGTATCAATAACTACCTCAAT TGGTATCAACAGAAACCTGGTAAAGCTCCAAAGCGCCTTATCTATAATACC AACAATCTCCAGACTGGTGTTCCAAGTAGGTTCTCCGGGAGCGGTAGTGGG ACTGAGTTTACATTGACAATTTCATCATTGCAACCCGAAGATTTTGCCACC TACTATTGCTTGCAGCATAATAGTTTTCCCACTTTCGGTCAGGGAACTAAG CTCGAAATAAAA | 397 |
| | Depatuxiz umab | VH | CAGGTACAACTCCAAGAATCTGGACCTGGGCTGGTAAAACCATCTCAGACC TTGAGTCTCACTTGCACTGTGTCCGGTTACTCTATATCTAGCGATTTTGCA TGGAATTGGATTCGCCAGCCACCCGGTAAGGGACTGGAGTGGATGGGTTAC ATTTCATACAGTGGAAACACACGTTACCAACCCAGTCTCAAGAGCCGGATA ACCATTAGTAGGGACACAAGCAAAAACCAATTTTTCCTCAAACTGAATTCA GTGACTGCTGCCGACACAGCCACTTACTATTGTGTCACAGCCGGTCGGGGG TTCCCTTACTGGGGACAGGGTACTCTCGTTACTGTAAGTTCT | 398 |
| | | VL | GACATACAAATGACTCAATCACCTAGCTCCATGTCTGTTTCCGTTGGCGAC CGCGTAACCATCACCTGTCATTCCTCACAGGATATAAATTCCAATATTGGG TGGCTTCAGCAGAAACCAGGTAAATCATTCAAGGGCCTTATTTATCACGGG ACCAACCTTGATGACGTGTCCCCTCTCGCTTTAGCGGTTCTGGATCAGGT ACAGATTATACCCTGACCATCTCTTCTCTCCAGCCCGAGGATTTCGCTACT TACTACTGTGTGCAATACGCACAGTTCCCCTGGACATTCGGCGGTGGAACT AAACTGGAGATAAAG | 399 |
| | Losatuxiz umab | VH | GAAGTTCAGTTGCAAGAAAGTGGCCCCGGACTTGTTAAACCATCCCAGACC TTGAGCCTGACTTGCACCGTTTCTGGCTATTCTATTTCCCGGGACTTCGCA TGGAACTGGATACGCCAACCTCCCGGCAAGGGCTTGGAGTGGATGGGGTAT ATCTCCTATAACGGCAATACACGCTATCAACCTAGTTTGAAGAGCCGCATA ACCATCAGTAGAGACACAAGTAAAAACCAATTCTTCTTGAAACTGAATAGT GTGACCGCTGCAGACACTGCAACCTATTACTGCGTCACAGCCAGTCGGGGG TTTCCATATTGGGGTCAAGGTACACTTGTAACCGTGTCTTCA | 400 |
| | | VL | GATATACAAATGACCCAGTCCCCTTCCTCCATGTCTGTCAGCGTCGGTGAT AGGGTTACCATAACTTGTCATAGCAGCCAAGACATAAACAGCAATATAGGT TGGCTGCAACAAAAGCCCGGTAAATCTTTCAAGGGGTTGATTTACCACGGA ACTAACCTCGATGATGGTGTGCCCAGTCGCTTTTCAGGTAGTGGGTCTGGA ACAGACTACACCCTGACAATCTCCAGTTTGCAACCAGAAGACTTTGCTACT TATTATTGTGTACAGTACGCTCAATTTCCTTGGACTTTCGGGGGAGGAACC AAACTTGAGATCAAG | 401 |
| EGFRvIII | Etevritam ab | VH | CAAGTCCAACTCGTGGAGTCTGGAGGCGGTGTGGTCCAAAGCGGGAGATCC TTGCGGCTGTCTTGCGCAGCATCAGGGTTTACATTTAGGAACTACGGCATG CACTGGGTTAGACAGGCACCCGGTAAGTGTCTTGAATGGGTAGCAGTAATC TGGTATGATGGTAGCGATAAATACTATGCTGATAGCGTTCGTGGTCGGTTC ACCATTAGTCGGGACAACTCAAAGAACACTTTGTACCTGCAAATGAACTCT CTGAGGGCCGAGGATACAGCTGTATATTACTGTGCTAGGGATGGCTATGAC ATACTCACCGGCAACCCACGGGATTTCGATTACTGGGGGCAAGGGACATTG GTAACAGTTTCATCT | 402 |

| | | | | |
|---|---|---|---|---|
| | | VL | GATACAGTAATGACCCAGACTCCTCTGAGTAGTCATGTTACACTGGGCCAGCCTGCCTCTATCTCCTGTCGTTCAAGTCAGAGTCTGGTGCACTCAGACGGAAACACCTACCTTTCATGGTTGCAACAGCGTCCTGGTCAACCTCCTCGATTGTTGATATACAGAATTAGTCGGCGTTTTTCTGGCGTCCCAGATCGCTTCTCCGGCTCAGGTGCCGGAACAGACTTCACCCTTGAGATCAGTCGAGTGGAGGCTGAGGATGTGGGCGTATATTACTGTATGCAAAGCACTCACGTGCCAAGGACATTCGGGTGTGGAACCAAGGTAGAGATCAAA | 403 |
| | AMG-595 | VH | CAGGTGCAGTTGGTTGAGAGCGGCGGAGGTGTGGTACAGCCTGGAAGGAGTCTGCGACTCTCATGTGCCGCCTCCGGTTTTACATTTTCTTCCTACGGCATGCATTGGGTTCGACAAGCACCAGGCAAGGGATTGGAATGGGTGGCAGTAATTTGGTACGACGGGTCAAATAAATACTATGTTGACAGCGTGAAAGGGCGATTCACTATCAGTCGTGACAATAGTAAAAACACTTTGTATCTGCAAATGAATAGTCTGAGGGCAGAGGACACAGCAGTCTATTACTGTGCTCGTGATGGTTGGCAACAGCTTGCCCCTTTTGACTATTGGGGACAAGGAACTTTGGTAACAGTAAGCAGC | 404 |
| | | VL | GACATTGTTATGACACAAACACCTTTGTCCTCACCCGTAACACTGGGCCAACCTGCCTCCATATCCTGCCGAAGCAGTCAATCACTTGTCCACTCTGATGGGAATACCTACTTGTCATGGCTGCATCAGCGACCAGGTCAGCCTCCAAGGCTTCTCATATATAAAATATCCAATCGTTTTTCAGGTGTTCCCGACCGTTTCTCCGGGTCAGGGGCCGGAACCGCCTTCACTCTCAAAATATCCAGAGTGGAGGCTGAAGACGTAGGTGTATATTATTGTATGCAAGCCACCCAGTTGCCACGCACCTTTGGGCAAGGGACTAAAGTAGAAATAAAG | 405 |
| PD-L1 | Atezolizumab | VH | GAAGTTCAACTGGTGGAGTCTGGAGGGGGGTCTCGTCCAGCCCGGCGGGAGCTTGAGACTGTCTTGTGCTGCATCCGGCTTTACCTTTAGCGATTCCTGGATACACTGGGTAAGACAAGCACCTGGTAAAGGGTTGGAATGGGTGGCATGGATTTCCCCATACGGGGGATCAACCTATTATGCTGATAGCGTGAAGGGCCGGTTTACTATTTCAGCCGACACAAGCAAGAACACCGCCTACCTCCAAATGAATTCTCTGAGGGCCGAGGACACAGCAGTCTATTACTGTGCTAGGAGGCATTGGCCCGGCGGATTCGACTACTGGGGGCAGGGCACTCTGGTCACAGTAAGCTCA | 406 |
| | | VL | GACATCCAAATGACACAGTCTCCAAGCTCTCTCTCCGCAAGCGTGGGCGACCGGGTCACAATAACATGCCGCGCCTCACAAGACGTGTCTACAGCCGTTGCTTGGTATCAGCAAAAGCCCGGAAAAGCTCCCAAACTGCTCATTTATTCAGCCAGCTTTCTCTACAGCGGGGTGCCAAGCCGGTTCAGTGGGAGCGGAAGTGGCACCGACTTTACTCTTACCATCTCCTCTCTGCAACCCGAAGACTTCGCCACCTATTACTGCCAGCAATACCTGTATCATCCAGCTACTTTCGGGCAAGGGACCAAGGTTGAAATCAAA | 407 |
| | Avelumab | VH | GAGGTTCAGCTTCTGGAGTCAGGAGGTGGTCTTGTTCAACCCGGAGGGTCTCTCCGTTTGTCCTGCGCCGCAAGTGGGTTCACATTTTCCAGTTATATCATGATGTGGGTGAGGCAAGCCCCTGGGAAAGGATTGGAGTGGGTCTCATCTATTTATCCATCTGGAGGTATTACATTCTATGCCGACACAGTTAAGGGCAGGTTTACTATAAGCCGCGATAATTCCAAAAACACCCTGTACCTGCAAATGAATTCACTGCGAGCCGAGGACACTGCTGTCTACTATTGCGCCAGAATCAAACTGGGGACAGTAACAACTGTAGACTATTGGGGGCAGGGGACTTTGGTCACTGTATCATCA | 408 |
| | | VL | CAGTCAGCTCTGACACAGCCCGCATCTGTCTCTGGGAGTCCCGGACAGTCAATAACTATATCATGCACAGGGACCTCCTCTGATGTTGGGGGATACAATTACGTGTCCTGGTATCAGCAGCACCCCGGCAAGGCCCCCAAACTCATGATATACGACGTTTCAAATCGTCCTTCAGGCGTGTCTAACAGATTTTCCGGTTCTAAATCAGGCAATACCGCTTCCTTGACTATCTCAGGACTTCAAGCAGAGGATGAAGCAGACTATTACTGCTCAAGCTATACCTCCAGTTCTACTAGAGTGTTCGGT | 409 |

| | | | | |
|---|---|---|---|---|
| | | | ACAGGAACTAAGGTAACAGTGCTG | |
| | Durvalumab | VH | GAGGTTCAGCTCGTGGAGAGTGGGGGCGGACTTGTTCAGCCAGGCGGTTCA TTGCGGTTGTCTTGTGCCGCCTCAGGATTTACATTCTCAAGATACTGGATG AGCTGGGTGAGGCAAGCACCCGGTAAAGGTCTCGAGTGGGTAGCTAATATC AAACAAGATGGGAGCGAGAAGTATTATGTTGACAGCGTGAAGGGTCGCTTT ACCATATCAAGGGATAACGCTAAGAACTCCCTTTATCTTCAGATGAATAGT CTCCGCGCTGAGGACACCGCAGTATATTACTGTGCTAGGGAAGGAGGGTGG TTTGGGGAATTGGCATTCGATTACTGGGGTCAGGGTACTCTCGTTACAGTC AGTTCC | 410 |
| | | VL | GAAATCGTTTTGACACAGAGCCCTGGGACACTGTCCTTGAGCCCAGGAGAA CGCGCAACCCTCTCCTGCCGTGCAAGTCAGCGTGTTTCCTCATCTTACCTT GCTTGGTATCAACAAAAGCCAGGGCAGGCTCCTAGACTGCTTATCTATGAC GCTTCTAGCAGAGCTACTGGGATACCAGATAGGTTTTCCGGGTCTGGTTCA GGCACAGACTTCACCCTCACCATATCTCGACTCGAACCTGAGGATTTTGCA GTGTACTATTGTCAACAATACGGTAGTTTGCCTTGGACCTTTGGACAGGGC ACTAAGGTCGAGATTAAA | 411 |
| HER2 | Trastuzumab | VH | GAAGTGCAGCTGGTCGAAAGTGGCGGTGGACTTGTGCAACCTGGCGGTAGC CTCCGTCTCAGCTGCGCTGCAAGTGGGTTCAACATCAAGGACACTTATATT CATTGGGTCCGACAGGCACCTGGGAAAGGTTTGGAGTGGGTCGCACGGATC TATCCCACTAATGGTTACACAAGATATGCCGATTCAGTAAAAGGCCGGTTT ACAATCAGCGCAGATACTTCAAAAAAACACTGCCTATCTTCAAATGAACTCA CTTCGAGCAGAAGACACAGCCGTCTATTATTGTAGTCGTTGGGGGAGGCGAC GGCTTTTATGCTATGGACTACTGGGGACAAGGAACTCTGGTCACAGTTTCA TCA | 412 |
| | | VL | GATATTCAGATGACTCAGAGTCCTAGTTCCCTCAGCGCCTCCGTAGGCGAC AGAGTTACAATAACTTGCCGAGCAAGCCAAGACGTAAACACTGCAGTCGCC TGGTACCAACAGAAACCAGGCAAAGCTCCAAAACTCTTGATTTACAGTGCT TCCTTCCTTTATAGTGGCGTTCCAAGCCGCTTCAGCGGCAGCCGCTCTGGC ACCGACTTCACTCTCACTATTTCTTCCTTGCAACCTGAAGACTTCGCCACT TATTATTGCCAGCAACACTACACAACACCCCCAACATTCGGACAGGGCACA AAGGTAGAAATAAAA | 413 |
| | Pertuzumab | VH | GAAGTGCAACTGGTGGAGTCTGGTGGTGGATTGGTGCAGCCAGGCGGTTCT CTGCGACTTAGTTGTGCAGCCTCCGGCTTTACCTTCACTGATTATACAATG GACTGGGTTCGGCAGGCACCCGGTAAGGGGCTTGAGTGGGTCGCCGACGTC AATCCTAATTCAGGGGGAAGTATTTATAACCAAAGGTTCAAGGGTCGATTT ACATTGTCCGTAGATCGTAGTAAAAATACCCTCTACCTTCAAATGAACTCC CTGAGGGCAGAGGATACCGCAGTCTACTACTGCGCTCGTAACCTGGGGCCT AGTTTTTATTTCGATTATTGGGGCCAAGGCACATTGGTAACTGTGTCTTCA | 414 |
| | | VL | GATATACAAATGACACAATCTCCTAGTTCATTGAGTGCCTCAGTCGGCGAC CGAGTCACTATAACTTGTAAAGCAAGCCAAGATGTTAGCATTGGCGTAGCT TGGTATCAGCAGAAACCTGGAAAAGCACCAAAACTGCTTATCTACTCCGCT AGTTACCGTTACACCGGAGTTCCCTCAAGGTTTTCTGGCAGCGGAAGTGGG ACTGACTTCACTCTGACTATTTCTTCACTTCAGCCAGAAGACTTCGCTACT TATTACTGTCAGCAGTACTATATCTATCCCTATACATTTGGACAAGGAACC AAAGTTGAGATTAAA | 415 |
| c-Met | Onartuzumab | VH | GAAGTCCAACTGGTAGAATCTGGAGGGGGTCTTGTTCAACCTGGGGGCAGT CTCAGGCTGTCATGTGCAGCAAGTGGATATACATTTACTTCCTATTGGCTC CATTGGGTACGACAAGCACCTGGGAAAGGGCTGGAATGGGTTGGTATGATC GACCCATCAAACTCTGACACCCGCTTTAATCCAAATTTTAAAGACCGCTTT | 416 |

| | | | | |
|---|---|---|---|---|
| | | | ACAATATCCGCAGATACAAGTAAGAACACCGCATATCTCCAGATGAACAGC CTGCGTGCAGAGGATACCGCAGTGTACTACTGTGCAACCTACCGGTCCTAT GTAACCCCTCTCGACTATTGGGGGTCAAGGCACACTTGTCACCGTGAGTAGC | |
| | | VL | GACATACAAATGACCCAATCACCAAGTTCCCTTTCTGCTTCAGTCGGTGAT CGCGTGACAATAACATGCAAATCATCTCAAAGTCTCTTGTACACAAGCAGC CAAAAAAATTATCTTGCTTGGTACCAGCAGAAGCCAGGGAAAGCACCAAAA CTGCTGATCTACTGGGCTTCAACAAGAGAATCCGGGGGTGCCCAGCCGCTTT TCCGGTTCCGGCAGTGGAACTGATTTCACCCTCACTATTTCCTCATTGCAA CCCGAGGACTTCGCAACCTATTACTGTCAACAGTATTACGCCTACCCTTGG ACATTTGGACAAGGAACTAAGGTTGAAATTAAA | 417 |
| | Emibetuzu mab | VH | CAAGTACAGCTCGTCCAATCCGGCGCTGAAGTCAAGAAGCCCGGGAGCTTCC GTTAAAGTTTCCTGCAAAGCCAGCGGCTACACTTTCACTGATTACTATATG CACTGGGTTAGACAAGCACCCGGGCAGGGTCTCGAATGGATGGGTAGGGTT AATCCAAATCGCAGGGGGAACTACTTACAACCAGAAATTTGAGGGCAGGGTT ACCATGACTACCGATACCAGCACATCTACTGCATATATGGAGCTGCGTTCT CTGAGGAGTGATGATACAGCAGTGTACTATTGTGCCCGCGCTAACTGGTTG GACTACTGGGGGCAAGGTACAACTGTCACAGTATCTAGC | 418 |
| | | VL | GACATTCAAATGACACAAAGTCCATCCTCTCTCAGTGCTTCAGTGGGGGAC CGAGTAACCATAACATGCAGTGTCTCAAGTAGCGTGAGTAGCATCTACCTC CATTGGTATCAGCAGAAACCTGGCAAGGCACCCAAACTCCTCATTTATTCC ACAAGTAATCTTGCTTCCGGCGTACCTTCTAGGTTTAGCGGGTCCGGCTCT GGCACCGATTTCACCCTCACTATTAGCTCCTTGCAACCTGAGGACTTTGCT ACTTACTATTGTCAGGTTTATTCCGGTTACCCCCTCACATTCGGTGGAGGA ACCAAGGTAGAGATTAAG | 419 |
| | Telisotuz umab | VH | CAGGTCCAGTTGGTACAGTCAGGGGGCAGAAGTTAAGAAACCAGGCGCTTCT GTAAAGGTTAGTTGCAAGGCAAGCGGGTATATATTCACAGCCTATACCATG CATTGGGTGCGTCAAGCTCCTGGGCAAGGATTGGAGTGGATGGGCTGGATC AAGCCCAACAATGGTCTGGCCAACTACGCACAGAAGTTCCAAGGTCGTGTA ACCATGACCAGGGACACTTCAATAAGCACCGCCTACATGGAATTGAGCAGA CTTCGATCAGATGATACAGCAGTTTACTATTGCGCTAGGAGTGAAATTACC ACAGAGTTTGATTACTGGGGCCAAGGAACTCTGGTGACTGTTTCCAGT | 420 |
| | | VL | GATATCGTTATGACACAGTCCCCCGACAGCCTGGCTGTCAGTCTCGGGGAG AGAGCAACTATAAACTGTAAAAGCAGTGAATCCGTCGATTCATACGCAAAC AGTTTTCTGCATTGGTATCAGCAAAAACCCGGCCAGCCACCCAAACTGCTC ATATATCGGGCTAGTACACGTGAGTCAGGCGTACCAGACCGCTTTAGCGGA TCAGGAAGTGGGACAGACTTTACCTTGACCATTAGCTCACTTCAGGCTGAG GACGTTGCAGTTTACTACTGCCAACAAAGTAAGGAAGACCCACTCACATTC GGCGGAGGAACTAAGGTCGAGATTAAG | 421 |
| TROP-2 | Datopotam ab | VH | CAGGTGCAGTTGGTCCAGTCCGGTGCAGAGGTAAAGAAACCAGGCGCTTCC GTTAAAGTATCCTGTAAAGCTAGCGGTTATACTTTCACTACTGCCGGAATG CAGTGGGTGCGACAGGCTCCTGGTCAGGGTCTCGAATGGATGGGATGGATC AATACTCACTCAGGAGTGCCAAAGTATGCTGAAGATTTCAAGGGGCGTGTT ACTATCTCCGCTGACACATCCACATCTACTGCTTATCTTCAGCTCTCTAGC CTGAAGTCAGAAGATACTGCTGTTTATTATTGTGCAAGGAGCGGGTTTGGA AGTTCCTATTGGTACTTCGACGTTTGGGGACAAGGAACACTGGTCACTGTT TCCAGC | 422 |
| | | VL | GATATACAGATGACCCAATCACCCTCTTCTTTGAGTGCATCAGTAGGTGAT CGTGTTACAATTACCTGTAAAGCTAGTCAAGACGTGTCTACTGCTGTCGCC TGGTATCAACAGAAACCAGGCAAAGCACCAAAACTTTTGATTTATTCTGCT | 423 |

| | | | | |
|---|---|---|---|---|
| | | | TCTTATAGATATACAGGCGTCCCCAGCCGGTTTTCAGGTTCCGGTTCCGGG ACCGACTTTACCCTCACTATCAGCTCTCTTCAGCCTGAGGACTTCGCCGTT TATTATTGCCAGCAGCACTATATCACCCCACTGACTTTTGGGCAAGGGACT AAGCTGGAGATCAAA | |
| | Sacituzum ab | VH | CAAGTACAGCTTCAGCAATCAGGCTCTGAACTCAAGAAACCTGGTGCCAGC GTGAAAGTATCCTGTAAGGCATCCGGGTACACTTTTACAAACTATGGAATG AATTGGGTAAAACAAGCCCCCGGACAAGGGCTTAAATGGATGGGTTGGATA AATACTTACACCGGAGAACCTACATATACAGACGACTTTAAAGGGCGATTC GCTTTCTCCCTTGACACAAGTGTAAGCACAGCCTACCTGCAAATCAGCAGT CTCAAAGCCGACGATACAGCCGTGTACTTTTGCGCCAGGGGTGGCTTCGGC TCCAGCTATTGGTACTTCGATGTCTGGGGACAGGGCAGCCTTGTAACTGTA TCTAGT | 424 |
| | | VL | GATATTCAGTTGACACAGAGCCCTAGCTCACTTTCAGCCTCTGTGGGTGAT AGGGTTAGTATTACCTGTAAGGCATCCCAAGACGTTAGCATTGCCGTCGCC TGGTACCAGCAGAAACCCGGAAAAGCTCCAAAACTGCTCATCTACTCAGCA TCCTATCGTTATACAGGTGTACCTGACAGGTTCTCTGGCTCCGGGAGCGGT ACCGATTTTACTTTGACCATTTCAAGTTTGCAACCAGAAGACTTCGCTGTT TACTACTGCCAGCAGCATTACATTACTCCACTCACATTCGGAGCAGGGACA AAGGTCGAAATCAAA | 425 |
| DLL3 | Rovalpitu zumab | VH | CAAGTCCAGCTTGTTCAAAGTGGGGGCTGAAGTGAAGAAACCAGGGGCTAGT GTTAAAGTGAGCTGTAAGGCATCAGGATACACTTTCACAAACTACGGAATG AATTGGGTTCGCCAAGCACCTGGTCAAGGCTTGGAATGGATGGGTTGGATT AACACATATACAGGTGAGCCAACTTACGCCGATGATTTCAAGGGGCGAGTT ACCATGACTACCGACACCTCAACATCCACTGCATACATGGAGCTTCGCTCA CTCCGAAGCGATGATACTGCAGTTTACTATTGCGCTCGCATCGGTGACTCA TCACCTAGCGACTACTGGGGCCAAGGTACATTGGTAACAGTTTCTTCA | 426 |
| | | VL | GAGATCGTGATGACCCAGAGTCCCGCTACTCTCTCAGTGAGTCCTGGTGAA CGTGCTACACTGTCTTGTAAGGCCAGTCAGTCCGTCTCAAACGATGTCGTT TGGTATCAGCAAAAGCCAGGACAAGCCCCCAGACTCCTGATATACTACGCC AGTAATCGCTATACTGGAATCCCCGCTAGATTCAGTGGGAGTGGAAGCGGA ACTGAATTTACCTTGACTATATCCTCATTGCAAAGCGAAGACTTTGCCGTT TACTATTGTCAACAAGACTACACCTCTCCTTGGACCTTCGGACAAGGTACA AAACTTGAAATCAAG | 427 |
| | Tarlatama b | VH | CAAGTACAGCTCCAAGAAAGTGGCCCCCGGATTGGTGAAGCCATCCGAAACA CTTTCCCTTACCTGCACTGTCTCCGGGGGCTCCATCAGTAGTTATTACTGG AGTTGGATACGCCAACCACCCGGTAAGTGTCTGGAGTGGATAGGTTATGTG TATTACTCAGGCACAACCAATTATAATCCATCCTTGAAAAGCCGGGTAACC ATCTCAGTAGATACCAGCAAAAACCAGTTCTCCCTGAAACTGTCCAGTGTT ACTGCTGCTGATACCGCCGTATATTATTGTGCATCCATTGCAGTGACAGGG TTTTATTTTGACTATTGGGGCCAGGGTACTTTGGTAACCGTATCTTCA | 428 |
| | | VL | GAGATCGTCCTGACCCAAAGCCCCAGGTACTCTTTCCCTCAGCCCAGGCGAA AGGGTCACTCTGTCATGCAGGGCTAGTCAAAGAGTCAACAATAATTACCTC GCATGGTATCAACAAAGACCCGGACAGGCTCCACGCCTGCTCATATATGGA GCAAGTAGCCGAGCTACTGGCATTCCCGATAGATTCAGTGGATCTGGATCT GGGACCGATTTTACTCTGACAATAAGTCGTCTTGAACCTGAAGATTTTGCA GTATACTATTGTCAGCAATATGACAGGAGCCCCCTGACATTCGGGTGCGGT ACTAAGCTGGAAATCAAA | 429 |
| BCMA | Belantama b | VH | CAAGTCCAACTGGTCCAGTCAGGCGCAGAAGTTAAAAAGCCTGGCAGCAGT GTGAAGGTGTCTTGTAAGGCAAGCGGCGGTACATTTAGTAATTATTGGATG | 430 |

| | | | | |
|---|---|---|---|---|
| | | | CACTGGGTACGGCAGGCTCCCGGCCAAGGGCTTGAATGGATGGGCGCCACA TACCGAGGTCATTCAGACACCTATTACAACCAGAAATTCAAGGGGCGCGTG ACCATTACAGCAGATAAATCAACTTCTACAGCCTACATGGAACTCAGCTCC CTCCGGTCAGAGGATACAGCAGTCTACTACTGTGCTCGCGGAGCCATTTAC GATGGGTATGATGTGCTGGATAATTGGGGGCAGGGCACACTCGTGACCGTA AGTAGT | |
| | | VL | GATATACAGATGACCCAGTCACCATCCAGCCTTAGTGCATCCGTCGGGGAT CGGGTGACTATTACTTGCTCCGCTTCTCAAGATATTTCAAACTATCTGAAT TGGTATCAGCAAAAGCCTGGGAAGGCCCCAAAATTGCTGATCTATTACACT TCAAATTTGCACTCAGGGGTTCCCTCTCGCTTCAGCGGAAGCGGAAGCGGT ACTGATTTTACCTTGACTATCTCTAGCCTCCAGCCAGAGGACTTTGCTACC TACTACTGCCAACAGTACAGGAAACTCCCATGGACTTTTGGACAAGGCACC AAGCTCGAAATTAAG | 431 |
| LIV-1 | Ladiratuz umab | VH | CAAGTTCAGTTGGTTCAATCTGGGGCCGAAGTCAAAAAACCTGGCGCTTCA GTTAAAGTTAGCTGCAAAGCAAGCGGTCTCACTATAGAAGACTATTATATG CACTGGGTCAGACAGGCTCCAGGGCAAGGGCTTGAGTGGATGGGGTGGATA GATCCAGAGAATGGGGACACCGAGTATGGACCCAAAATTCCAGGGGCGTGTA ACCATGACCCGAGACACTTCAATAAATACTGCATACATGGAACTCTCCCGG CTCCGGAGCGACGATACAGCCGTGTATTACTGTGCTGTCCACAACGCCCAC TACGGCACATGGTTTGCATATTGGGGGCAGGGAACTCTTGTTACTGTTTCT TCA | 432 |
| | | VL | GACGTAGTGATGACTCAGTCTCCACTGTCCCTGCCAGTGACATTGGGCCAA CCTGCAAGTATTTCATGCAGATCAAGTCAATCTCTCCTGCACAGTAGCGGC AACACATACTTGGAGTGGTATCAACAACGCCCAGGTCAATCACCCAGGCCA CTGATATATAAAATCTCAACTCGATTCAGCGGTGTTCCCGACAGGTTCTCA GGATCTGGCTCCGGCACTGATTTTACCTTGAAGATCTCACGAGTGGAAGCT GAGGATGTGGGAGTATATTACTGTTTCCAAGGTTCACATGTCCCTTATACT TTTGGTGGAGGAACTAAGGTAGAGATCAAG | 433 |
| GPC-3 | Codrituzu mab | VH | CAGGTGCAACTCGTTCAAAGCGGGGCCGAGGTGAAGAAACCAGGGGCCTCA GTTAAGGTGAGTTGCAAGGCAAGTGGATACACTTTCACCGATTATGAAATG CATTGGGTGCGTCAGGCCCCAGGACAAGGACTGGAGTGGATGGGCGCTCTC GATCCTAAGACTGGTGATACTGCTTACTCTCAAAAGTTCAAAGGCCGAGTC ACCTTGACCGCCGACAAGTCCACATCCACTGCATATATGGAATTGTCAAGT CTGACAAGCGAAGATACAGCCGTCTACTACTGCACCCGCTTTTATAGCTAT ACATATTGGGGACAGGGGACCTTGGTTACTGTGTCATCT | 434 |
| | | VL | GACGTGGTAATGACACAATCACCTTTGTCTCTTCCCGTAACCCCCGGTGAA CCAGCCAGCATCTCATGCAGAAGCAGTCAGTCACTGGTACATTCCAACCGT AATACTTATCTTCACTGGTACTTGCAGAAGCCTGGGCAGTCTCCTCAACTT TTGATATATAAAGTGAGCAATCGGTTTAGCGGTGTCCCAGACCGCTTTTCT GGATCTGGAAGTGGAACAGACTTTACTCTGAAAATAAGCAGAGTCGAGGCA GAAGATGTCGGAGTTTACTACTGTAGCCAGAACACACACGTACCCCCCAACC TTTGGACAGGGCACAAAGTTGGAAATCAAG | 435 |
| FGFR2 | Aprutumab | VH | GAGGTACAACTGCTTGAATCTGGAGGAGGGGTTGGTACAACCTGGTGGTTCA CTGCGATTGTCCTGTGCAGCCTCAGGCTTTACTTTCTCATCATATGCCATG TCCTGGGTAAGGCAGGCACCTGGAAAAGGACTCGAATGGGTCTCAGCCATC TCCGGTTCAGGCACATCAACTTACTATGCAGACTCTGTCAAAGGGCGCTTT ACAATATCTAGGGATAATTCAAAAAAATACATTGTACTTGCAGATGAACAGT TTGCGTGCCGAAGATACCGCAGTGTACTATTGCGCTAGGGTTCGATATAAC TGGAACCATGGTGACTGGTTTGACCCTTGGGGCCAAGGCACACTGGTGACA GTGAGTTCC | 436 |

| | | | | |
|---|---|---|---|---|
| | | VL | CAGTCCGTCCTCACACAACCACCTAGTGCCTCTGGTACACCAGGACAACGT GTCACAATTTCCTGCAGCGGGTCAAGTTCAAACATAGGGAATAACTATGTG TCATGGTATCAACAACTTCCTGGTACTGCTCCAAAGCTCCTCATTTATGAA AACTATAACAGGCCCGCAGGTGTCCCAGATCGATTTTCAGGATCAAAGTCC GGTACCTCAGCCAGTTTGGCAATTAGTGGCCTTCGATCCGAAGATGAAGCA GATTACTACTGTTCATCCTGGGACGATTCTCTTAACTATTGGGTATTCGGC GGAGGCACTAAACTCACCGTCCTT | 437 |
| | Bemarituz umab | VH | CAAGTGCAGCTTGTTCAGAGTGGGGCTGAAGTCAAAAAGCCAGGCTCAAGC GTGAAAGTCAGCTGCAAGGCCAGCGGTTACATCTTCACAACTTACAATGTT CACTGGGTCAGACAAGCCCCTGGTCAGGGGCTTGAGTGGATAGGATCAATC TACCCCGATAATGGGGACACCAGCTATAATCAAAACTTCAAAGGACGTGCA ACAATCACAGCCGACAAGTCAACTTCAACAGCCTACATGGAGCTTTCCAGC TTGCGATCCGAAGATACTGCCGTATATTACTGTGCTAGAGGCGACTTCGCT TATTGGGGACAAGGTACTTTGGTGACTGTTTCTTCT | 438 |
| | | VL | GACATTCAAATGACCCAATCCCCCAGTTCCTTGAGTGCCTCCGTCGGAGAT CGAGTTACTATTACATGTAAAGCTAGTCAAGGGGTCAGTAACGACGTTGCT TGGTACCAGCAGAAGCCAGGTAAGGCTCCCAAGCTCCTGATATATAGCGCC TCATACCGCTACACAGGTGTGCCTTCCCGGTTTAGTGGCTCAGGATCAGGG ACAGATTTTACATTCACTATAAGCTCTTTGCAGCCCGAAGACATAGCCACA TATTATTGCCAGCAGCATTCCACTACTCCATACACATTTGGACAGGGAACA AAGCTGGAGATTAAG | 439 |
| FGFR3 | Vofatamab | VH | GAAGTACAGCTTGTCGAGAGCGGTGGCGGGCTTGTACAACCTGGTGGAAGC TTGCGGTTGAGTTGTGCAGCCAGCGGTTTCACATTTACTAGCACTGGAATA TCATGGGTCCGTCAAGCTCCAGGGAAAGGGCTGGAATGGGTTGGCCGAATA TATCCCACCAGTGGCTCTACCAACTACGCAGACTCAGTCAAGGGTCGCTTT ACAATTTCTGCTGACACAAGTAAGAACACCGCATATTTGCAGATGAACTCA CTGCGAGCCGAGGATACCGCCGTTTACTACTGTGCAAGGACATACGGAATT TACGATCTTTACGTTGATTATACAGAGTATGTGATGGATTATTGGGGGCAG GGCACCCTCGTCACTGTCAGTTCT | 440 |
| | | VL | GACATCCAGATGACCCAGAGCCCCTCTTCTTTGTCAGCAAGCGTCGGAGAC CGCGTTACCATTACTTGCCGTGCCTCTCAGGACGTGGACACCAGCCTTGCT TGGTACAAGCAGAAACCAGGAAAAGCCCCCAAGCTGCTCATCTATTCCGCT TCATTTCTCTACAGCGGAGTGCCATCCCGTTTCTCCGGTTCAGGCTCTGGA ACAGACTTCACTCTGACTATAAGCAGTCTTCAACCCGAAGACTTCGCTACA TACTATTGTCAGCAATCAACCGGACACCCACAGACATTCGGCCAGGGCACT AAAGTAGAGATTAAA | 441 |
| VEGFR2 | Ramuc irum ab | VH | GAAGTTCAGCTTGTGCAGAGTGGCGGAGGGCTTGTGAAACCAGGAGGATCA CTGAGGCTCTCCTGTGCAGCATCCGGTTTCACATTCAGCAGCTATAGTATG AACTGGGTGCGTCAGGCTCCAGGGAAGGGACTGGAATGGGTCTCAAGCATT TCCTCCTCCTCTTCATATATATATTACGCCGACAGTGTAAAAGGCCGCTTT ACAATATCTCGGGATAACGCTAAAAATAGCTTGTACCTTCAGATGAATTCA CTGAGGGCTGAGGACACTGCTGTGTACTACTGTGCAAGAGTCACCGACGCT TTTGATATTTGGGGTCAGGGGACAATGGTGACCGTCTCCTCA | 442 |
| | | VL | GATATACAAATGACCCAGTCTCCCAGTTCAGTATCCGCCAGCATAGGTGAC CGCGTGACCATAACATGCCGGGCCAGCCAGGGAATTGATAATTGGTTGGGG TGGTATCAACAAAAGCCCGGAAAAGCTCCCAAGCTCCTTATCTATGATGCT TCTAACTTGGATACAGGTGTACCCAGTCGATTTAGTGGCTCCGGGAGTGGG ACTTATTTCACCCTCACTATATCTTCTCTGCAAGCAGAAGACTTTGCAGTA TATTTCTGTCAACAGGCCAAAGCATTCCCTCCAACCTTCGGTGGGGGGACA AAGGTAGACATTAAA | 443 |

| | | | | |
|---|---|---|---|---|
| CD20 | Rituximab | VH | CAGGTCCAGCTCCAACAACCTGGAGCAGAATTGGTCAAGCCAGGGGCAAGC GTCAAGATGAGCTGCAAGGCAAGCGGCTATACTTTCACCTCCTACAATATG CATTGGGTCAAACAAACTCCAGGTCGTGGGCTTGAGTGGATCGGGGCCATT TACCCAGGCAACGGCGACACCTCATATAACCAAAAGTTTAAGGGAAAAGCC ACTTTGACAGCAGATAAAAGTAGTAGCACTGCATACATGCAACTGTCAAGT CTGACTAGCGAAGATAGTGCCGTATATTATTGCGCTAGGTCCACATATTAC GGCGGTGATTGGTACTTCAATGTTTGGGGAGCCGGGACTACAGTCACCGTA TCCGCT | 444 |
| | | VL | CAAATCGTCCTGTCTCAATCACCAGCAATTCTGAGTGCTAGTCCCGGAGAA AAAGTCACTATGACCTGTAGAGCCTCATCATCTGTTTCCTATATACATTGG TTTCAGCAGAAACCTGGATCTTCTCCCAAGCCCTGGATTTATGCAACCTCT AACCTCGCAAGTGGAGTCCCCGTGCGGTTTTCAGGCAGCGGTTCCGGTACA AGTTATTCCCTGACCATCAGCCGTGTGGAAGCAGAAGACGCCGCCACATAC TACTGCCAACAGTGGACCTCAAATCCTCCCACCTTTGGGGGAGGTACTAAA CTTGAAATAAAA | 445 |
| | Obinutuzu mab | VH | CAAGTGCAACTGGTTCAAAGTGGAGCCGAGGTCAAAAAACCTGGTTCCTCC GTCAAAGTGTCTTGTAAAGCTTCAGGGTACGCATTCTCCTACTCCTGGATA AACTGGGTGCGTCAGGCTCCTGGGCAAGGTCTGGAATGGATGGGCCGGATT TTCCCAGGAGATGGCGACACAGACTACAATGGGAAGTTTAAGGGTCGGGTA ACCATCACCGCTGACAAGAGTACATCTACCGCCTATATGGAGCTTTCTTCA CTTAGGAGTGAGGACACAGCAGTCTACTATTGTGCTCGAAATGTGTTTGAC GGGTATTGGCTGGTGTATTGGGGCCAGGGGTACCCTCGTAACAGTATCATCA | 446 |
| | | VL | GATATTGTTATGACACAAACACCACTGTCCCTCCCTGTTACACCCGGAGAG CCTGCTTCCATAAGTTGTCGATCCTCCAAATCACTTCTCCACTCAAATGGA ATCACTTATCTTTATTGGTATCTTCAGAAGCCAGGACAGTCCCCTCAACTG TTGATTTATCAGATGTCAAATTTGGTGAGTGGGGTGCCAGATAGGTTTTCT GGATCCGGTTCCGGTACTGACTTTACATTGAAAATATCCCGAGTCGAAGCC GAAGACGTGGGCGTGTACTATTGCGCTCAGAACCTTGAGCTGCCTTACACC TTTGGCGGTGGGACTAAAGTGGAAATTAAG | 447 |
| CD38 | Daratumum ab | VH | GAGGTTCAGTTGCTGGAGAGCGGAGGGGGGGCTTGTCCAACCAGGCGGTTCT CTGCGACTTTCTTGTGCAGTGTCTGGGTTTACCTTCAACAGCTTTGCCATG TCCTGGGTGCGCCAAGCACCCGGAAAGGGACTGGAGTGGGTTAGCGCAATC TCTGGGTCAGGGGGGAGGGACTTATTATGCTGACTCTGTTAAGGGTAGATTT ACAATCAGTCGCGATAATAGTAAAAATACACTGTATCTTCAGATGAACTCT CTCAGAGCCGAGGATACAGCCGTGTATTTCTGTGCCAAAGACAAGATCCTT TGGTTCGGAGAGCCTGTTTTCGACTATTGGGGTCAAGGGACATTGGTGACA GTAAGCTCT | 448 |
| | | VL | GAAATTGTTCTCACCCAGAGTCCAGCTACCCTGTCCCTGAGCCCCGGCGAG AGAGCAACCTTGAGTTGCCGAGCCTCTCAATCCGTCTCCTCCTATCTGGCC TGGTACCAACAAAAACCAGGCCAAGCCCCCCGTTTGCTGATATACGACGCC AGTAACCGAGCTACCGGCATACCCGCCCGCTTTAGCGGCTCTGGATCTGGT ACAGATTTCACACTCACTATATCAAGTCTGGAACCTGAAGATTTCGCAGTC TATTATTGCCAACAACGGTCAAATTGGCCCCCTACATTTGGACAAGGGACC AAAGTGGAGATTAAG | 449 |
| EphA2 | 1C1 | VH | GAGGTACAGTTGCTGGAGTCAGGAGGTGGATTGGTCCAACCCGGAGGATCT CTTCGTCTGTCCTGCGCCGCCTCAGGATTTACCTTCTCATTATATGATG GCATGGGTACGTCAGGCTCCAGGCAAAGGTCTGGAATGGGTTAGTCGGATT GGTCCCTCAGGGGGTCCTACCCATTATGCCGATTCTGTAAAGGGCCGTTTT ACCATAAGCAGAGACAACTCTAAGAACACCCTTTACCTTCAGATGAATAGC CTGAGGGCTGAGGATACCGCAGTGTATTACTGCGCAGGCTATGACTCTGGG | 450 |

| | | | | |
|---|---|---|---|---|
| | | | TACGACTATGTCGCCGTAGCAGGACCTGCCGAGTATTTTCAACACTGGGGACAGGGGACCCTTGTCACAGTTTCTAGT | |
| | | VL | GATATTCAAATGACACAAAGCCCAAGTTCCTTGTCCGCCTCAGTTGGTGATCGTGTGACAATAACCTGTCGGGCTTCACAATCCATATCTACATGGCTGGCTTGGTACCAGCAAAAGCCAGGTAAAGCCCCAAAACTCCTGATTTACAAGGCAAGTAACTTGCATACTGGGGTACCCAGCCGTTTCTCTGGGTCAGGCTCTGGGACAGAGTTTAGTCTTACAATTTCTGGTCTGCAACCCGATGACTTCGCTACCTATTACTGTCAACAATATAATAGTTATTCTCGAACATTTGGTCAGGGAACAAAAGTGGAAATCAAA | 451 |

**Fc region or fragment thereof**

Here, the above-described first Fc domain and the second Fc domain may each be an Fc region of an immunoglobulin. The Fc region of an immunoglobulin may be an Fc domain variant as well as a wild type Fc domain. Here, the Fc region may be an Fc region of IgG, IgA, IgE, IgD, or IgM.

As used herein, the term "Fc domain variant" may refer to a form which is different from the wild type Fc domain in terms of glycosylation pattern, or has a high level of specific glycan species as compared with the wild type Fc domain, a low level of specific glycan species as compared with the wild type Fc domain, or a deglycosylated form. In addition, an aglycosylated Fc domain is included therein. The Fc domain or a variant thereof may be adapted to have an adjusted number of sialic acids, fucosylations, or other types of glycosylations, through modulation of culture conditions or genetic manipulation of a host cell.

In addition, glycosylation of the Fc domain of an immunoglobulin may be modified by conventional methods such as chemical methods, enzymatic methods, and genetic engineering methods using microorganisms. In addition, the Fc domain variant may be in a mixed form of respective Fc regions of immunoglobulin IgG, IgA, IgE, IgD, or IgM. In addition, the Fc domain variant may be a form in which some amino acids of the Fc domain are substituted with other amino acids.

An "amino acid" introduced by the substitution and/or addition may be any one selected from the group consisting of lysine (K), alanine (A), arginine (R), asparagine (N), aspartic acid (D), cysteine (C), glutamine (Q), glutamic acid (E), glycine (G), histidine (H), isoleucine (I), leucine (L), methionine (M), phenylalanine (F), proline (P), serine (S), threonine (T), tryptophan (W), tyrosine (Y), and valine (V).

In one embodiment, the variant of the Fc region may be a form in which amino acids 239 and/or 332 of the CH2 region are substituted with other amino acids (see Kabat numbering system). Specifically, S239 may be substituted with an amino acid other than S, and specifically, S239 may be substituted with S239D. In addition, I332 may be substituted with an amino acid other than I, and specifically, I332 may be substituted with I332E.

In addition, the Fc region may include a variant or structure of a knob or a variant or structure of a hole .

As used herein, the term "knob-into-hole" refers to an Fc heterodimerization strategy for producing antibodies that specifically bind to different regions, such as bispecific antibodies, multispecific antibodies, or heterodimeric antibodies. Generally, this technique involves introducing a knob mutation at the interface of a first polypeptide (e.g., the first CH3 domain of a first antibody heavy chain) and a corresponding hole mutation at the interface of a second polypeptide (e.g., the second CH3 domain of a second antibody heavy chain), such that a knob may be placed within the hole to promote heterodimer formation and prevent homodimer formation.

The "knob" variant is constructed by replacing small amino acid side chains from the interface of the first polypeptide (e.g., the first CH3 domain of the first antibody heavy chain) with larger side chains (e.g., arginine, phenylalanine, tyrosine, or tryptophan). The complementary "hole" variant of the same or similar size to the knob is created by replacing large amino acid side chains at the interface of the second polypeptide (e.g., the second CH3 domain of the second antibody heavy chain) with smaller side chains (e.g., alanine, serine, valine, or threonine). The knob and hole may be created by altering the nucleic acid encoding the polypeptide, for example, by site-directed mutagenesis, or by peptide synthesis.

Examples of variants of the Fc region that promote the formation of a heterodimer may include those described in WO2014084607A1 and WO2018059502A1, etc. The disclosures of WO2014084607A1 and WO2018059502A1 are incorporated herein by reference. WO2014084607A1 describes, for example, mutations in the CH3 domain that may comprise (a-1) tryptophan (W) substituted at Lys409 of one CH3 domain that interacts with valine (V) substituted at Asp399 and threonine (T) substituted at Phe405 of another CH3 domain; and (a-2) serine (S) substituted at Tyr349 of one CH3 domain that interacts with tryptophan (W) substituted at Glu357 of another CH3 domain, and in addition, may further comprise (b-1) glutamic acid (E) substituted at Lys360 of one CH3 domain that interacts with arginine (R) substituted at Gln347 of another CH3 domain; and (b-2) glutamic acid (E) substituted at Gln347 and glutamic acid substituted at Lys360 of one CH3 domain that interact with arginine (R) substituted at Gln347 of another CH3 domain. Here, the position of the amino acid residue follows the EU index. WO2018059502A1, for example, describes mutations in the Fc domain including one or more mutations selected from a) - e), respectively: a) L351G, L351Y, L351V, L351P, L351D, L351E, L351K, or L351W; b) T366L, T366P, T366W, or T366V; c) D399C, D399N, D399I, D399G, D399R, D399T, or D399A; d) Y407L, Y407A, Y407P, Y407F, Y407T, or Y407H; and e) K409C, K409P, K409S, K409F, K409V, K409Q, or K409R. Here, the position of the amino acid residue follows the EU index.

**Structure of fusion protein**

The fusion protein may comprise polypeptide chains represented by the following structural formulas (I), (II), (III), and (IV), respectively:

$$N'\text{-}X\text{-}(L1)n\text{-}A\text{-}C' \qquad (I);$$

$$N'\text{-}Y\text{-}(L2)m\text{-}B\text{-}C' \qquad (II);$$

$$N'\text{-}(Z1)r\text{-}(L5)s\text{-}C\text{-}C' \qquad (III);$$

and

$$N'\text{-}(Z2)t\text{-}(L6)u\text{-}D\text{-}C' \qquad (IV)$$

wherein, in the structural formulas (I), (II), (III), and (IV),
N' is the N-terminus of each polypeptide,
C' is the C-terminus of each polypeptide,

- refers to a linkage,

A, B, C, and D are monomeric polypeptide sequences of an Fc domain each comprising the CH2 and CH3 regions of an immunoglobulin, and optionally further comprising CH4 and/or a hinge sequence, wherein
A forms a dimer with one of C or D to form the first Fc domain (b), and
B forms a dimer with the remaining one of C or D to form the second Fc domain (c);
L1, L2, L5, and L6 are each peptide linker,
n, m, r, s, t, and u are each independently 0 or 1,
X comprises a heavy chain variable region or a light chain variable region of an antibody that specifically binds to an antigen;
Y comprises a light chain variable region or a heavy chain variable region of an antibody that specifically binds to an antigen; and
X and Y pair with each other to form the antigen binding site (a) that specifically binds to an antigen, and
the polypeptides of (I), (II), (III), and (IV) may be assembled into a fusion protein comprising one antigen binding site and two Fc domains.

In addition, Z1 and Z2 may be each independently any one selected from the group consisting of a receptor, a soluble protein, a cytokine, a single-chain Fv fragment (single-chain fragment variable; scFv), an antibody mimetics, a single domain antibody (sdAb), a therapeutic peptide, and a peptide vaccine.
Specifically, X is a first polypeptide sequence of the antigen binding site, which comprises heavy chain CDR1, CDR2, and CDR3 sequences of an antibody that specifically binds to a first antigen, or a heavy chain variable region of an antibody that specifically binds to a first antigen; Y is a second polypeptide sequence of the antigen binding site, which comprises light chain CDR1, CDR2, and CDR3 sequences of an antibody that specifically binds to a first antigen, or a light chain variable region of an antibody that specifically binds to a first antigen; and X and Y pair with each other to form the antigen binding site (a) that specifically binds to an antigen.
According to one embodiment, the CH3 region may be mutated to minimize the interaction between A and B, and between C and D and promote the formation of a heterodimeric Fc between A and C, and between B and D. Specifically, the Fc domain monomer comprises a knob variant or a hole variant that promotes the formation of an Fc heterodimer (heterodimeric Fc); or the Fc domain monomer may comprise a variant that promotes the formation of a heterodimer by electrostatic steering mechanism.
According to one embodiment, X in the structural formula (I) may further comprise a heavy chain CH1 region, and/or Y in the structural formula (II) may further comprise a light chain constant region.
In addition, the fusion protein may comprise polypeptide chains represented by the following structural formulas (I'), (II'), (III), and (IV):

$$N'\text{-}VD1\text{-}(L3)p\text{-}X\text{-}(L1)n\text{-}A\text{-}C' \qquad (I');$$

$$N'\text{-}VD2\text{-}(L4)q\text{-}Y\text{-}(L2)m\text{-}B\text{-}C' \qquad (II');$$

$$N'\text{-}(Z1)r\text{-}(L5)s\text{-}C\text{-}C' \qquad (III);$$

and

$$N'\text{-}(Z2)t\text{-}(L6)u\text{-}D\text{-}C' \qquad (IV)$$

wherein, in the structural formulas (I'), (II'), (III), and (IV),
N' is the N-terminus of the polypeptide chain,
C' is the C-terminus of the polypeptide chain,

- refers to a linkage,

A, B, C, and D are monomeric polypeptide sequences of an Fc domain each comprising the CH2 and CH3 regions of an immunoglobulin, and optionally further comprising CH4 and/or a hinge sequence, wherein A forms a dimer with one of C or D to form the first Fc domain (b), and B forms a dimer with the remaining one of C or D to form the second Fc domain (c);
L1, L2, L3, L4, L5, and L6 are each peptide linker,
n, m, p, q, r, s, t, and u are each 0 or 1,
VD1 consists of a heavy chain or light chain variable region of an antibody or CDR1, CDR2, and CDR3 of an antibody heavy chain or light chain that specifically binds to an antigen;
VD2 consists of a light chain or heavy chain variable region of an antibody or CDR1, CDR2, and CDR3 of an antibody heavy chain or light chain that specifically binds to an antigen;
VD1 and VD2 pair with each other to form a second antibody variable region that specifically binds to a second antigen,
X comprises a heavy chain or light chain variable region of an antibody or CDR1, CDR2, and CDR3 of an antibody heavy chain or light chain that specifically binds to an antigen;
Y comprises a light chain or heavy chain variable region of an antibody or CDR1, CDR2, and CDR3 of an antibody heavy chain or light chain that specifically binds to an antigen; and
X and Y pair with each other to form a first antibody variable region that specifically binds to a first antigen, and
VD1-(L3)p-X forms a first polypeptide sequence of the antigen binding site (a), and VD2-(L4)q-Y forms a second polypeptide sequence of the antigen binding site (a).

In addition, Z1 and Z2 may be each independently any one selected from the group consisting of a receptor, a soluble protein, a cytokine, a single-chain Fv fragment (single-chain fragment variable; scFv), an antibody mimetics, a single domain antibody (sdAb), a therapeutic peptide, and a peptide vaccine.

According to one embodiment, the CH3 region may be mutated to minimize the interaction between A and B, and between C and D and promote the formation of a heterodimeric Fc between A and C, and between B and D. Specifically, the Fc domain monomer comprises a knob variant or a hole variant that promotes the formation of an Fc heterodimer; or the Fc domain monomer may comprise a variant that promotes the formation of a heterodimer by electrostatic steering mechanism.

According to one embodiment, the heavy chain variable region may further comprise a heavy chain CH1 region. In addition, the light chain variable region may further comprise a light chain constant region.

In the structures of the fusion proteins described herein, the binding between X and Y may be achieved i) through a disulfide bond formed by Cys present in CH1 and a light chain constant region, ii) through a disulfide bond formed by Cys present in a heavy chain variable region and a light chain variable region, or iii) through a disulfide bond formed by Cys present in CH1 and a light chain constant region, and a disulfide bond formed by Cys present in a heavy chain variable region and a light chain variable region.

Specifically, the binding between X and Y may be formed by a disulfide bond present between $CH_1 233$ and CL214 based on Kabat numbering. In addition, X and Y may further comprise Cys through amino acid substitution. Examples of such variants may include mutations in the variable region, and specifically may include mutations at 105C of VH and 43C of VL, or mutations at 44C of VH and 100C of VL based on Kabat numbering. In one embodiment, the mutation may be Q105C of VH and A43C of VL. In addition, in one embodiment, the mutation may be G44C of VH and Q100C of VL. In addition, examples of variants in the constant region may include mutations at 122C of CH1 and 121C of CL based on Kabat numbering. In one embodiment, the mutation may be F122C of CH1 and S121C of CL.

**Linker and hinge**

The hinge is a hinge region derived from immunoglobulins. In one embodiment, the antibody hinge region is an IgG hinge region. The IgG hinge region provided herein may be selected, for example, from antibody hinge regions of various IgG subtypes. The table below lists exemplary IgG subtypes with core hinge sequences that may be included in the flexible peptide regions provided herein. In addition, at least one Cys may exist within the hinge. Specifically, 1, 2, or 3 Cys may exist within the hinge.

[Table 5]

| IgG subtype | Core hinge sequence | SEQ ID NO |
|---|---|---|
| IgG1 | EPKSCDKTHTCPPCP | 828 |
| IgG2 | ERKCCVECPPCP | 829 |
| IgG3 | ELKTPLDTTHTCPRCP(EPKSCDTPPPCPRCP)3 | 830 |
| IgG4 | ESKYGPPCPSCP | 831 |

The hinge may be modified to delete disulfide bonds or introduce additional disulfide bonds.
In addition, the linkers L1 and L2 may each comprise about 1 to about 70 amino acids. According to one exemplary embodiment, L1 and L2 may each comprise about 5 to about 60 amino acids, about 10 to about 50 amino acids, about 15 to about 40 amino acids, or about 20 to about 30 amino acids. According to another exemplary embodiment, for example, L1 and L2 may each be a peptide consisting of about 1 to about 70 amino acid residues, about 2 to about 60 amino acid residues, about 2 to about 50 amino acid residues, about 2 to about 40 amino acid residues, about 2 to about 30 amino acid residues, about 3 to about 50 amino acid residues, about 3 to about 40 amino acid residues, about 3 to about 30 amino acid residues, about 2 to about 28 amino acid residues, about 2 to about 26 amino acid residues, about 2 to about 24 amino acid residues, about 2 to about 22 amino acid residues, about 2 to about 20 amino acid residues, about 2 to about 18 amino acid residues, about 2 to about 16 amino acid residues, about 2 to about 14 amino acid residues, about 2 to about 12 amino acid residues, or about 2 to about 10 amino acid residues. Specifically, L1 and L2 may include the amino acid sequence of (G4S)o (where o is an integer of 1 to 5) in Table 6 below, but are not limited thereto. In addition, L1 and L2 may have different amino acid sequences. In addition, here, L1 and L2 may comprise at least one Cys. In addition, a disulfide bond may be formed through Cys present in L1 and L2.

[Table 6]

| Linker sequence | SEQ ID NO |
|---|---|
| GGGGS | 832 |
| GGGGSGGGGS | 833 |
| GGGGSGGGGSGGGGS | G |

In addition, L3 and L4 may each comprise about 1 to about 30 amino acids. According to one exemplary embodiment, L3 and L4 may each comprise about 5 to about 25 amino acids, about 10 to about 20 amino acids, or about 15 amino acids. According to another exemplary embodiment, L3 and L4 may each be a peptide consisting of about 2 to about 30 amino acid residues, about 2 to about 25 amino acid residues, about 2 to about 20 amino acid residues, about 2 to about 15 amino acid residues, about 3 to about 30 amino acid residues, about 2 to about 28 amino acid residues, about 2 to about 26 amino acid residues, about 2 to about 24 amino acid residues, about 2 to about 22 amino acid residues, about 2 to about 20 amino acid residues, about 2 to about 18 amino acid residues, about 2 to about 16 amino acid residues, about 2 to about 14 amino acid residues, about 2 to about 12 amino acid residues, or about 2 to about 10 amino acid residues. Specifically, L3 and L4 may include the amino acid sequence of (G4S)o (where o is an integer of 1 to 5) in Table 6 above, but are not limited thereto. In addition, L3 and L4 may have different amino acid sequences.

**Z protein**

The Z protein may be additionally bound to the fusion protein. Here, the Z protein may be referred to as Z1 protein or Z2 protein. Here, the Z1 protein and the Z2 protein may be the same protein or may be different proteins. Here, the Z1 protein and the Z2 protein may be any one selected from the group consisting of a receptor, a soluble protein, a cytokine, a single-chain Fv fragment (single-chain fragment variable; scFv), an antibody mimetics, a single domain antibody (sdAb), a therapeutic peptide, and a peptide vaccine, as described above.

Here, one embodiment of the Z protein may be the extracellular domain (ECD) of the receptor or a fragment thereof. Specifically, the receptor may be VEGF receptor, GITR (glucocorticoid-induced TNF receptor), SIRP$\alpha$, or CD80.

Here, one embodiment of the Z protein may be a soluble protein. Specifically, the soluble protein may be an IL-1R antagonist or a fragment thereof.

Here, one embodiment of the Z protein may be a cytokine. Specifically, the cytokine may be IL-2, IL-12, IL-15, IL-18, or a variant thereof.

Here, one embodiment of the Z protein may be a single-chain Fv fragment (single-chain fragment variable; scFv). Specifically, the scFv may be an scFv that specifically binds to the above-described antigen. Specifically, the scFv may specifically bind to HER2, EGFR, or c-MET.

Here, one embodiment of the Z protein may be an antibody mimetics. Specifically, the antibody mimetic may be DARPin-E01.

Here, one embodiment of the Z protein may be a single domain antibody (sdAb). Specifically, the single domain antibody may comprise a CDR sequence that specifically binds to the above-described antigen.

Here, one embodiment of the Z protein may be a therapeutic peptide.

Here, one embodiment of the Z protein may be a peptide vaccine. Specifically, the peptide vaccine may be any one selected from the group consisting of a KRAS mutant (G12D, G12C, G12V) derived peptide, a p53 mutant derived peptide, a BRAF mutant derived peptide, an EGFRvIII derived peptide, an LMP1 derived peptide, an LMP2 derived peptide, an HPV E6/E7 derived peptide, a WT1 derived peptide, a MAGE-A3 derived peptide, a NY-ESO-1 derived peptide, a HER2 derived peptide, a gp100 derived peptide, a PAP derived peptide, and an IDO derived peptide.

**Embodiments of fusion proteins**

**Fusion protein comprising one antigen binding site and two Fe domains**

**i) Fusion protein in which antigen binding site is Fab**

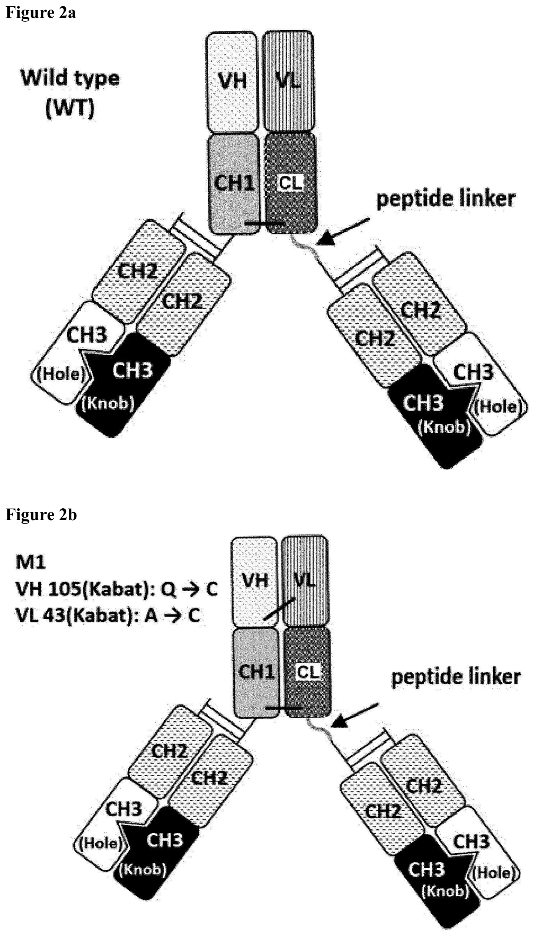

As shown in Figure 2a, the fusion protein comprises polypeptides of the structural formulas (1), (II), (III), and (IV), where X is a heavy chain variable region and further comprises CH1, and Y is a light chain variable region and comprises a light chain constant region. In addition, they are attached to each other by Cys in the CH1 structure and the light chain variable region to form a Fab structure. Here, n is 0, CH1 is directly linked to the hinge, m is 1, and L2 includes a peptide linker. Here, A and C are attached to each other to form the first Fc domain, and B and D are attached to each other to form the second Fc domain. In addition, the CH3 region of A comprises a hole variant, and the CH3 region of C comprises a knob variant. In addition, the CH3 region of B comprises a hole variant, and the CH3 region of D comprises a knob variant. In addition, the antigen binding site, antigen, hinge, linker, and Fc domain are as described above.

As shown in Figure 2b, the fusion protein comprises polypeptides of the structural formulas (I), (II), (III), and (IV), where X is a heavy chain variable region and further comprises CH1, and Y is a light chain variable region and comprises a light chain constant region. In addition, they are attached to each other by Cys in the CH1 structure and the light chain variable region to form a Fab structure. Here, n is 0, CH1 is directly linked to the hinge, m is 1, and L2 includes a peptide linker. Here, A and C are attached to each other to form the first Fc domain, and B and D are attached to each other to form the second Fc domain. In addition, the CH3 region of A comprises a hole variant, and the CH3 region of C comprises a knob variant. In addition, the CH3 region of B comprises a hole variant, and the CH3 region of D comprises a knob variant. In addition, X comprises the mutation of 105C, and Y comprises the mutation of 43C, and a disulfide bond between Cys is formed. In addition, the antigen binding site, antigen, hinge, linker, and Fc domain are as described above.

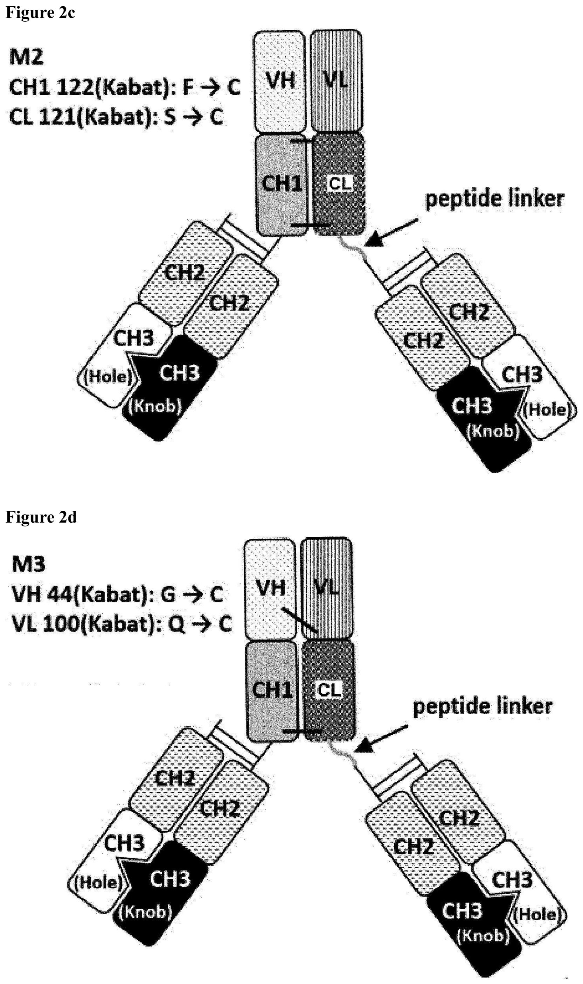

As shown in Figure 2c, the fusion protein comprises polypeptides of the structural formulas (I), (II), (III), and (IV), where X is a heavy chain variable region and further comprises CH1, and Y is a light chain variable region and comprises a light chain constant region. In addition, they are attached to each other by Cys in the CH1 structure and the light chain variable region to form a Fab structure. Here, n is 0, CH1 is directly linked to the hinge, m is 1, and L2 includes a peptide linker. Here, A and C are attached to each other to form the first Fc domain, and B and D are attached to each other to form the second Fc domain. In addition, the CH3 region of A comprises a hole variant, and the CH3 region of C comprises a knob variant. In addition, the CH3 region of B comprises a hole variant, and the CH3 region of D comprises a knob variant. In addition, CH1 comprises the mutation of 122C, and the light chain constant region comprises the mutation of 121C, and a disulfide bond between Cys is formed. In addition, the antigen binding site, antigen, hinge, linker, and Fc domain are as described above.

As shown in Figure 2d, the fusion protein comprises polypeptides of the structural formulas (I), (II), (III), and (IV), where X is a heavy chain variable region and further comprises CH1, and Y is a light chain variable region and comprises a light chain constant region. In addition, they are attached to each other by Cys in the CH1 structure and the light chain variable region to form a Fab structure. Here, n is 0, CH1 is directly linked to the hinge, m is 1, and L2 includes a peptide linker. Here, A and C are attached to each other to form the first Fc domain, and B and D are attached to each other to form the second Fc domain. In addition, the CH3 region of A comprises a hole variant, and the CH3 region of C comprises a knob variant . In addition, the CH3 region of B comprises a hole variant, and the CH3 region of D comprises a knob variant. In addition, X comprises the mutation of 44C, and Y comprises the mutation of 100C, and a disulfide bond between Cys is formed. In addition, the antigen binding site, antigen, hinge, linker, and Fc domain are as described above.

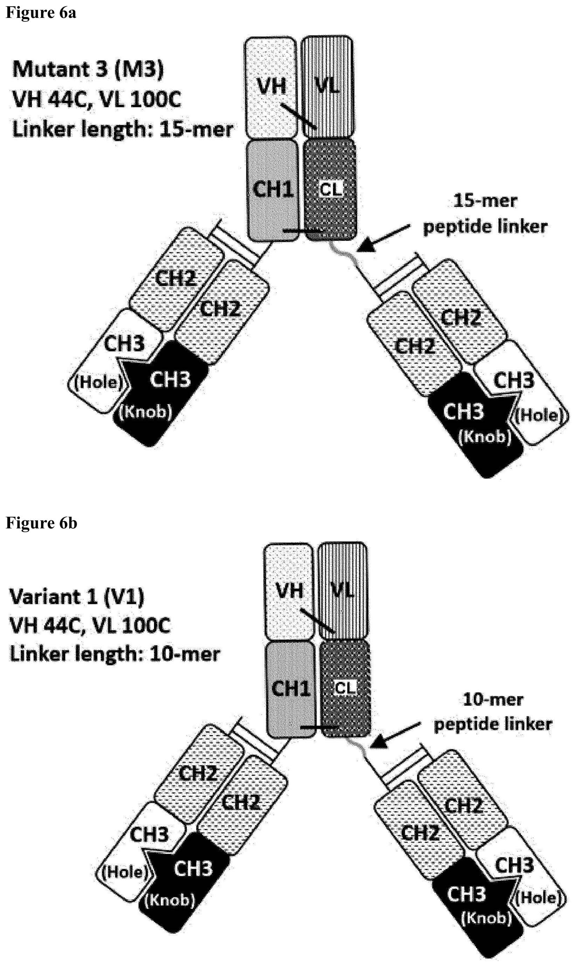

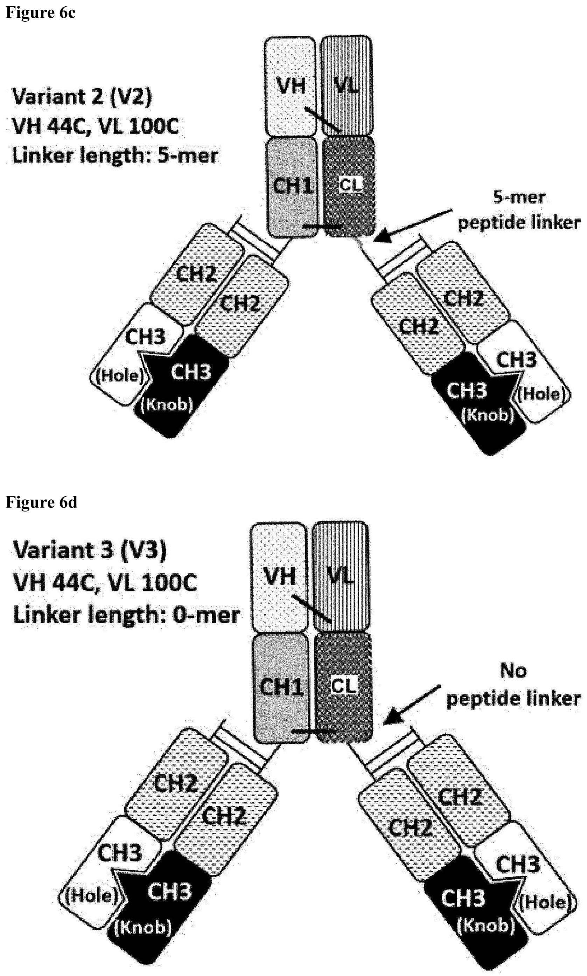

As shown in Figures 6a to 6d, the fusion protein comprises polypeptides of the structural formulas (I), (II), (III), and (IV), where X is a heavy chain variable region and further comprises CH1, and Y is a light chain variable region and comprises a light chain constant region. In addition, they are attached to each other by Cys in the CH1 structure and the light chain variable region to form a Fab structure. Here, n is 0, and CH1 is directly linked to the hinge. Here, A and C are attached to each other to form the first Fc domain, and B and D are attached to each other to form the second Fc domain. In addition, the CH3 region of A comprises a hole structure, and the CH3 region of C comprises a knob structure. In addition, the CH3 region of B comprises a hole structure, and the CH3 region of D comprises a knob structure. In addition, X comprises the mutation of 44C, and Y comprises the mutation of 100C, and a disulfide bond between Cys is formed. Here, m is 0, and the light chain variable region may be directly linked to the hinge (Figure 6d). In addition, m is 1, and L2 may include a 15-mer peptide linker (Figure 6a), a 10-mer peptide linker (Figure 6b), or a 5-mer peptide linker (Figure 6c). In addition, the antigen binding site, antigen, hinge, linker, and Fc domain are as described above.

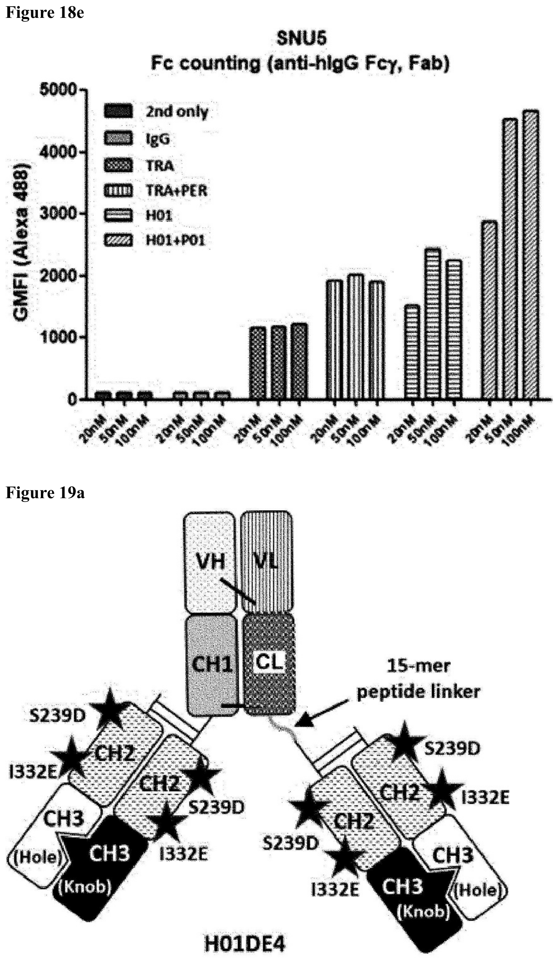

As shown in Figure 19a, the fusion protein comprises polypeptides of the structural formulas (I), (II), (III), and (IV), where X is a heavy chain variable region and further comprises CH1, and Y is a light chain variable region and comprises a light chain constant region. In addition, they are attached to each other by Cys in the CH1 structure and the light chain variable region to form a Fab structure. Here, n is 0, CH1 is directly linked to the hinge, m is 1, and L2 includes a peptide linker. Here, A and C are attached to each other to form the first Fc domain, and B and D are attached to each other to form the second Fc domain. In addition, the CH3 region of A comprises a hole variant and the CH3 region of C comprises a knob variant. In addition, the CH3 region of B comprises a hole variant, and the CH3 region of D comprises a knob variant. In addition, X comprises the mutation of 44C, and Y comprises the mutation of 100C, and a disulfide bond between Cys is formed. Here, all CH2s of A, B, C, and D comprise the 239D and 332E mutations. In addition, the antigen binding site, antigen, hinge, linker, and Fc domain are as described above.

### ii) Fusion protein in which antigen binding site is Fv

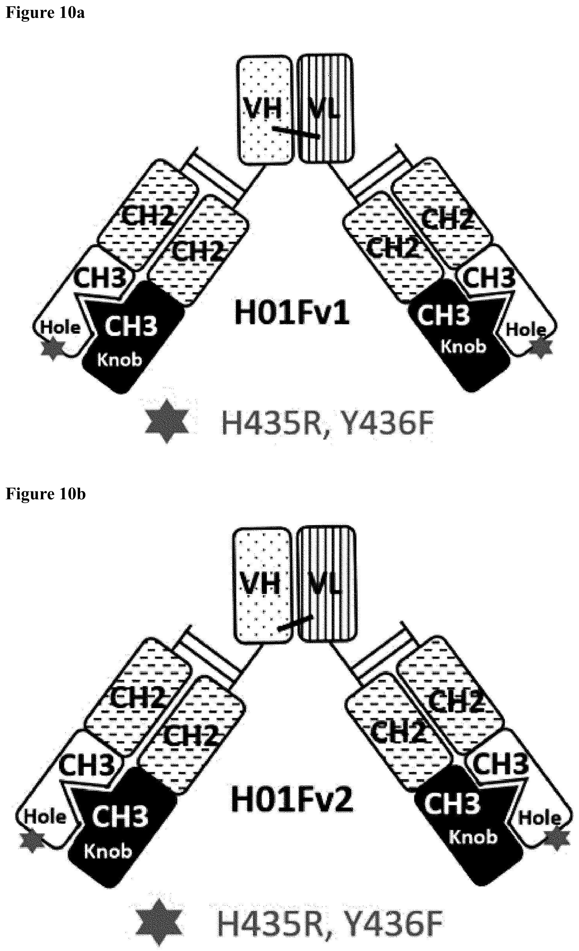

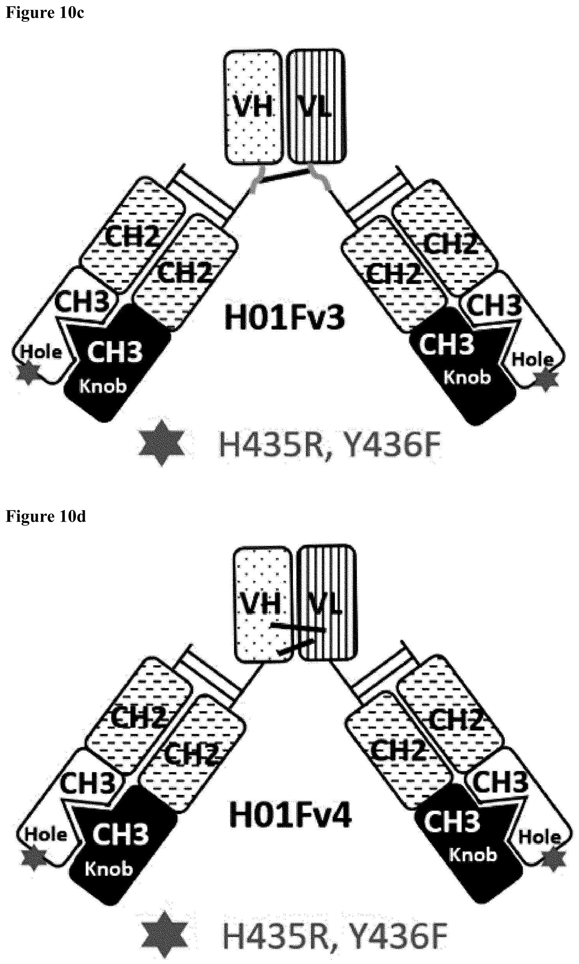

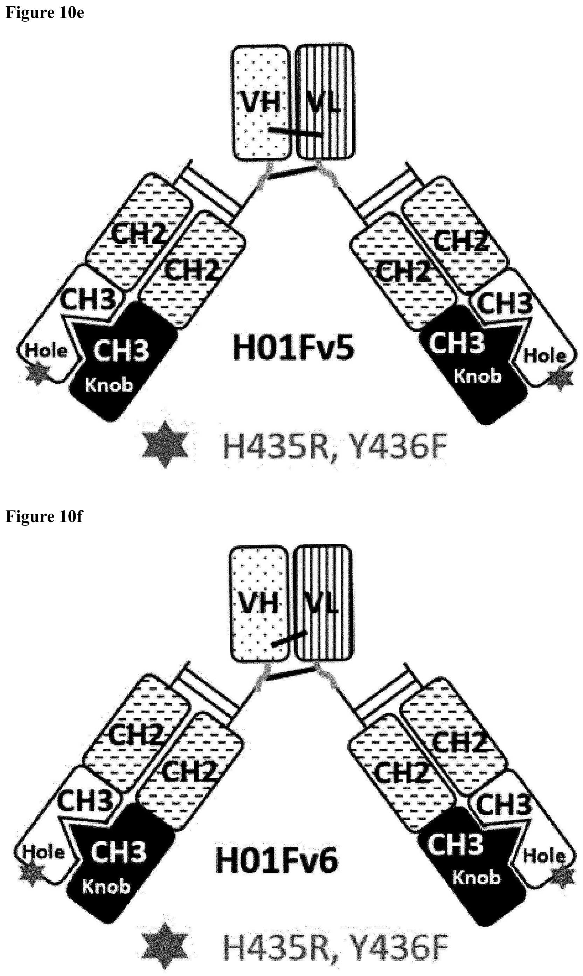

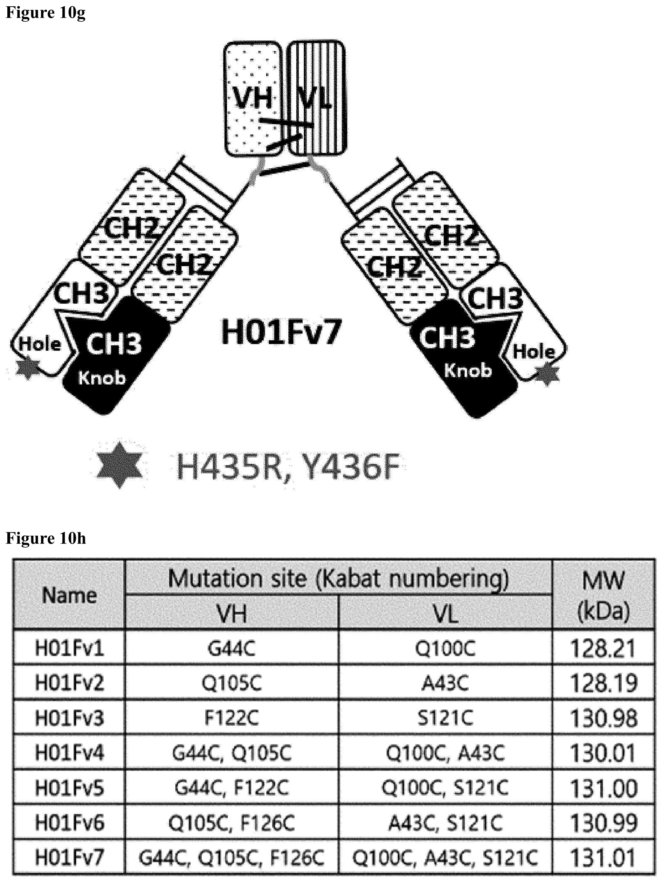

As shown in Figure 10, the fusion protein comprises polypeptides of the structural formulas (I), (II), (III), and (IV), where X and Y are attached to each other by at least one Cys present therein to form a Fv structure. Here, A and C are attached to each other to form the first Fc domain, and B and D are attached to each other to form the second Fc domain. In addition, the CH3 region of A comprises a hole variant and the CH3 region of C comprises a knob variant. In addition, the CH3 region of B comprises a hole variant, and the CH3 region of D comprises a knob variant. X may comprise the mutations of 44C, 105C, 122C, 44C/105C, 44C/122C, 105C/126C or 44C/105C/126C, and Y may comprise the mutations of 100C, 43C, 121C, 100C/43C, 100C/121C, 43C/121C, or 100C/43C/121C. In addition, a disulfide bond may be formed by Cys present in L1 and L2. In addition, the antigen binding site, antigen, hinge, linker, and Fc domain are as described above.

### Fusion protein comprising two antigen binding sites and two Fc domains

### iii) Fusion protein in which antigen binding site is Fab

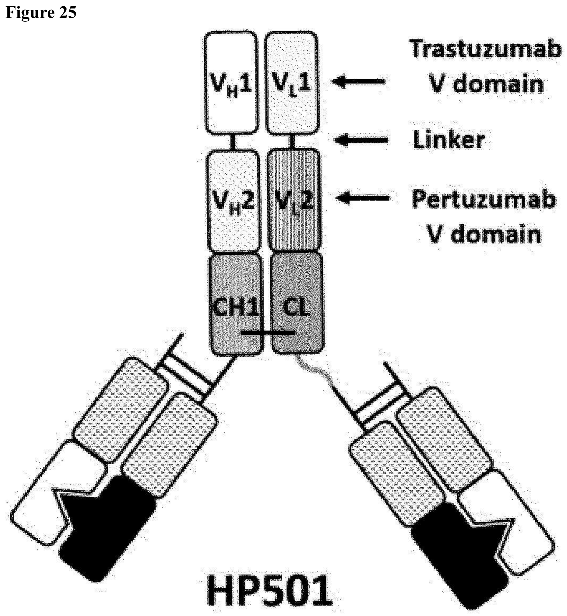

As shown in Figure 25, the fusion protein comprises polypeptides of the structural formulas (I'), (II'), (III), and (IV), where X is a heavy chain variable region and further comprises CH1, and Y is a light chain variable region and comprises a light chain constant region. In addition, they are attached to each other by Cys in the CH1 structure and the light chain variable region to form a Fab structure. Here, n is 0, CH1 is directly linked to the hinge, m is 1, and L2 includes a peptide linker. Here, A and C are attached to each other to form the first Fc domain, and B and D are attached to each other to form the second Fc domain. In addition, the CH3 region of A comprises a hole variant, and the CH3 region of C comprises a knob variant. In addition, the CH3 region of B comprises a hole variant, and the CH3 region of D comprises a knob variant. In addition, VD1 in the structural formula (I') is a heavy chain variable region, and VD2 in the structural formula (II') is a light chain variable region, and VD1 and VD2 pair with each other to form Fv. In addition, p and q are each 1, and L3 and L4 are peptide linkers. As a non-limiting example, X and Y may pair with each other to form the variable region of pertuzumab, and VD1 and VD2 may pair with each other to form the variable region of trastuzumab. In addition, the antigen binding site, antigen, hinge, linker, and Fc domain are as described above.

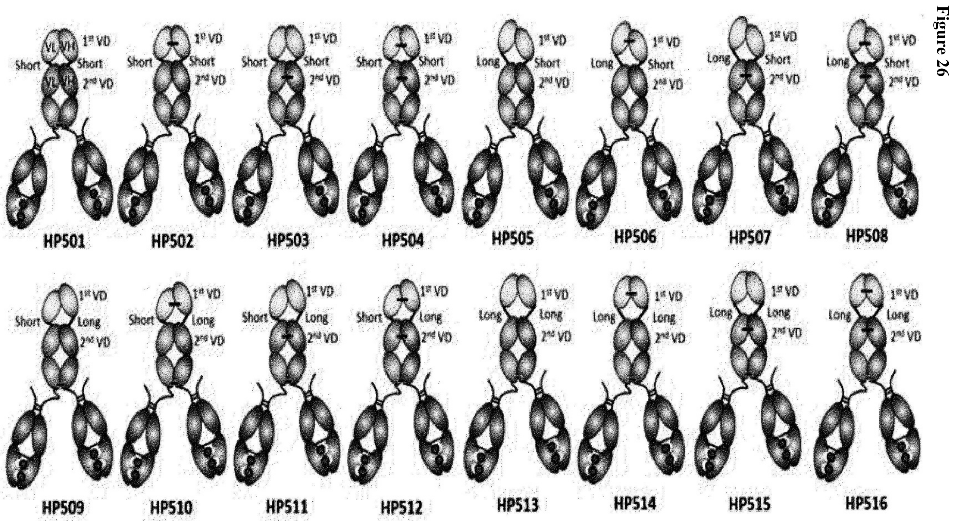

As shown in Figure 26, the peptide linkers of L3 and L4 in the structural formulas (I') and (II') may be of various lengths. In addition, the first antigen binding site formed by pairing between X and Y, and the second antigen binding site formed by pairing between VD1 and VD2 may be the same or different. In addition, L1 and L2 may also comprise various peptide linkers.

**Fusion protein comprising one antigen binding site comprising Z protein and two Fe domains**

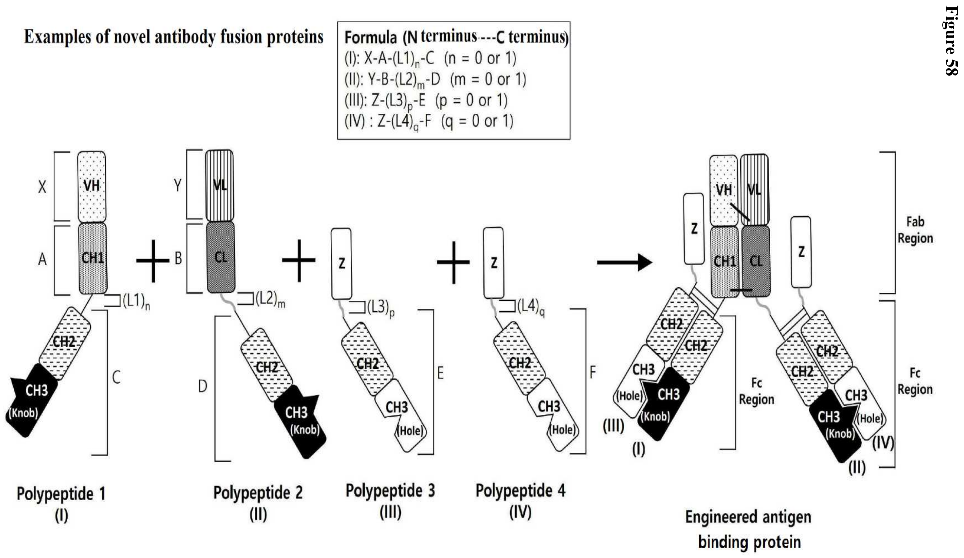

As shown in Figure 58, the fusion protein comprises polypeptides of the structural formulas (I), (II), (III), and (IV), where X is a heavy chain variable region and further comprises CH1, and Y is a light chain variable region and comprises a light chain constant region. In addition, they are attached to each other by Cys in the CH1 structure and the light chain variable region to form a Fab structure. Here, n is 0, CH1 is directly linked to the hinge, m is 1, and L2 includes a peptide linker. Here, A and C are attached to each other to form the first Fc domain, and B and D are attached to each other to form the second Fc domain. In addition, the CH3 region of A comprises a hole structure, and the CH3 region of C comprises a knob structure. In addition, the CH3 region of B comprises a hole structure, and the CH3 region of D comprises a knob structure. In addition, r, s, t, and u are 1. In addition, Z1 and Z2 are the same protein. In addition, the antigen binding site, antigen, hinge, linker, and Fc domain are as described above.

**Polynucleotide encoding fusion protein**

In another aspect of the present invention, there is provided a polynucleotide encoding a polypeptide of the structural formula (I), (II), (III), and/or (IV).
In another aspect of the present invention, there is provided a polynucleotide encoding a polypeptide of the structural formula (I'), (II'), (III), and/or (IV).
The polynucleotide may comprise a nucleic acid sequence having an identity of at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100% to a polynucleotide encoding a polypeptide of the structural formula (I), (II), (III), and/or (IV), and a polynucleotide encoding a polypeptide of the structural formula (I'), (II'), (III), and/or (IV).
The polynucleotide may further comprise a nucleic acid encoding a signal sequence or a leader sequence. As used herein, the term "signal sequence" refers to a signal peptide that directs secretion of a target protein. The signal peptide is translated and then cleaved in a host cell. Specifically, the signal sequence is an amino acid sequence that initiates migration of a protein across the endoplasmic reticulum (ER) membrane.
The signal sequences are well known in the art for their characteristics. Such signal sequences typically comprise about 16 to about 30 amino acid residues, and may comprise more or fewer amino acid residues than such amino acid residues. A typical signal peptide consists of three regions, that is, a basic N-terminal region, a central hydrophobic region, and a more polar C-terminal region. The central hydrophobic region comprises 4 to 12 hydrophobic residues that cause the signal sequence to be immobilized during migration of an immature polypeptide through the membrane lipid bilayer.
After initiation, signal sequences are cleaved in the lumen of ER by cellular enzymes, commonly known as signal peptidases. Here, the signal sequence may be a secretory signal sequence of tPa (tissue plasminogen activator), HSV gDs (signal sequence of Herpes simplex virus glycoprotein D), or a growth hormone. Preferably, a secretory signal sequence used in higher eukaryotic cells including mammals and the like may be used. In addition, as the signal sequence, a wild type signal sequence may be used, or a signal sequence that has been substituted with a codon having high expression frequency in a host cell may be used.

**Vector loaded with polynucleotide**

In another aspect of the present invention, there is provided a vector comprising the polynucleotide. The vector may comprise a polynucleotide encoding a polypeptide of the structural formula (I), (II), (III), and/or (IV). In addition, the vector may comprise a polynucleotide encoding a polypeptide of the structural formula (I'), (II'), (III), and/or (IV).
The vector may be introduced into a host cell to be recombined with and inserted into the genome of the host cell. Alternatively, the vector is understood as nucleic acid means comprising a polynucleotide sequence which is autonomously replicable as an episome. The vectors include linear nucleic acids, plasmids, phagemids, cosmids, RNA vectors, viral vectors, and analogs thereof. Examples of the viral vector include, but are not limited to, retroviruses, adenoviruses, and adeno-associated viruses.
Specifically, the vector may include plasmid DNA, phage DNA, and the like; and commercially developed plasmids (pUC18, pBAD, pIDTSAMRT-AMP, and the like), *E. coli*-derived plasmids (pYG601BR322, pBR325, pUC118, pUC119, and the like), Bacillus subtilis-derived plasmids (pUB110, pTP5, and the like), yeast-derived plasmids (YEp13, YEp24, YCp50, and the like), phage DNA (Charon4A, Charon21A, EMBL3, EMBL4, Agt10, λgt11, λZAP, and the like), animal viral vectors (retroviruses, adenoviruses, vaccinia viruses, and the like), insect viral vectors (baculoviruses and the like). Since the vector exhibits different expression levels and modification of a protein depending on a host cell, it is preferred to select and use a host cell which is most suitable for the purpose.

As used herein, the term "gene expression" or "expression" of a target protein is understood to mean transcription of DNA sequences, translation of mRNA transcripts, and secretion of fusion protein products or fragments thereof. A useful expression vector may be RcCMV (Invitrogen, Carlsbad) or a variant thereof. The expression vector may comprise human cytomegalovirus (CMV) promoter for promoting continuous transcription of a target gene in mammalian cells, and a bovine growth hormone polyadenylation signal sequence for increasing the stability level of RNA after transcription.

**Transformed cell expressing fusion protein**

In another aspect of the present invention, there is provided a transformed cell expressing. Specifically the transformed cell may be one into which the vector has been introduced.

Host cells for the transformed cell may include, but are not limited to, prokaryotic cells, eukaryotic cells, and cells of mammalian, plant, insect, fungal, or cellular origin. As an example of the prokaryotic cells, *E. coli* may be used. In addition, as an example of the eukaryotic cells, yeast may be used. In addition, for the mammalian cells, CHO cells, F2N cells, CSO cells, BHK cells, Bowes melanoma cells, HeLa cells, 911 cells, AT1080 cells, A549 cells, HEK 293 cells, HEK293T cells, or the like may be used. However, the mammalian cells are not limited thereto, and any cells which are known to those of ordinary skill in the art to be usable as mammalian host cells may be used.

In addition, for the introduction of an expression vector into the host cell, $CaCl_2$ precipitation, Hanahan method whose efficiency has been increased efficiency by using a reducing agent such as dimethyl sulfoxide (DMSO) in $CaCl_2$ precipitation, electroporation, calcium phosphate precipitation, protoplast fusion, agitation using silicon carbide fiber, Agrobacteria-mediated transformation, transformation using PEG, dextran sulfate-, Lipofectamine-, and dry/inhibition-mediated transformation, and the like may be used.

As described above, for optimization of properties of a fusion protein as a therapeutic agent or for any other purpose, glycosylation pattern of the fusion protein (for example, sialic acids, fucosylations, glycosylations) may be adjusted by manipulating, through methods known to those of ordinary skill in the art, glycosylation-related genes possessed by host cells.

**Method for producing a fusion protein**

In another aspect of the present invention, there is provided a method for producing a fusion protein comprising an antigen binding site, a first Fc domain or a variant thereof, and a second Fc domain or a variant thereof, the method comprising the steps of: i) culturing the transformed cells; and ii) collecting the produced fusion proteins.

As used herein, the term "culture" refers to a method of growing microorganisms (for example, the transformed cells) under appropriately artificially controlled environmental conditions.

The method of culturing the transformed cells may be carried out using methods well known in the art. Specifically, the culture may be carried out in a batch process, or carried out continuously in a fed batch or repeated fed batch process.

In addition, the step of obtaining the fusion protein from the culture product may be performed by methods known in the art. Here, the obtaining method is not particularly limited as long as the produced fusion protein of the present invention may be obtained. Preferably, the obtaining method may be a method such as centrifugation, filtration, extraction, spraying, drying, evaporation, precipitation, crystallization, electrophoresis, fractional dissolution (for example, ammonium sulfate precipitation), chromatography (for example, ion exchange, affinity, hydrophobicity, and size exclusion).

The method of culturing the transformed cells may be carried out using methods well known in the art. Specifically, the culture may be carried out in a batch process, or carried out continuously in a fed batch or repeated fed batch process.

**Use of fusion protein**

In another aspect of the present invention, there is provided a pharmaceutical composition for preventing or treating cancer, comprising the fusion protein as an active ingredient.

As used herein, the term "cancer" is a general term for a disease caused by cells that have aggressive characteristics in which cells divide and proliferate in defiance of normal growth limits, invasive characteristics that invade surrounding tissues, and metastatic characteristics that spread to other parts of the body, and is used in the same sense as malignant tumor.

The pharmaceutical composition may be used for the prevention or treatment of cancer, such as any one cancer selected from the group consisting of gastric cancer, liver cancer, lung cancer, large intestine cancer, breast cancer, prostate cancer, gallbladder cancer, bladder cancer, kidney cancer, esophageal cancer, skin cancer, rectal cancer, osteosarcoma, multiple myeloma, glioma, ovarian cancer, pancreatic cancer, cervical cancer, endometrial cancer, thyroid cancer, laryngeal cancer, testicular cancer, mesothelioma, acute myeloid leukemia, chronic myeloid leukemia, acute lymphoblastic leukemia, chronic lymphoblastic leukemia, brain tumor, neuroblastoma, retinoblastoma, head and neck cancer, salivary gland cancer, and lymphoma.

A preferred dosage of the pharmaceutical composition varies depending on the patient's condition and body weight, severity of disease, form of drug, route and duration of administration and may be appropriately selected by those of ordinary skill in the art. In the pharmaceutical composition for treating or preventing tumor of the present invention, the active ingredient may be contained in any amount (effective amount) depending on application, dosage form, blending purpose, and the like, as long as the active ingredient may exhibit therapeutic activity against tumor or, in particular, may exhibit a therapeutic effect on cancer. A conventional effective amount thereof will be determined within a range of 0.001% to 20.0% by weight, based on the total weight of the composition. Here, the term "effective amount" refers to an amount of an active ingredient that may induce an effect of improving or treating the condition of a disease, especially an effect of improving or treating the condition of cancer. Such an effective amount may be experimentally determined within the scope of common knowledge of those of ordinary skill in the art.

As used herein, the term "treatment" may be used to mean both therapeutic and prophylactic treatment. Here, prophylaxis may be used to mean that a pathological condition or disease of a subject is alleviated or mitigated. In one embodiment, the term "treatment" includes both application or any form of administration for treating a disease in a mammal, including a human. In addition, the term includes inhibiting or slowing down the progression of a disease; and includes meanings of restoring or repairing impaired or lost function so that a disease is partially or completely alleviated; stimulating inefficient processes; or alleviating a serious disease.

Pharmacokinetic parameters such as bioavailability and underlying parameters such as clearance rate may also affect efficacy. Therefore, "improved efficacy" (for example, improvement in efficacy) may be due to improved pharmacokinetic parameters and improved efficacy, which may be measured by comparing clearance rate in test animals or human subjects and parameters such as tumor treatment or improvement.

As used herein, the term "therapeutically effective amount" or "pharmaceutically effective amount" refers to an amount of a compound or composition effective to prevent or treat the disease in question, which is sufficient to treat the disease at a reasonable benefit/risk ratio applicable to medical treatment and does not cause adverse effects. A level of the effective amount may be determined depending on factors including the patient's health condition, type and severity of disease, activity of drug, the patient's sensitivity to drug, mode of administration, time of administration, route of administration and excretion rate, duration of treatment, combined or simultaneously used drugs, and other factors well known in the medical field. In one embodiment, the therapeutically effective amount means an amount of drug effective to treat cancer.

Here, the pharmaceutical composition may further comprise a pharmaceutically acceptable carrier. The pharmaceutically acceptable carrier may be any carrier as long as the carrier is a non-toxic substance suitable for delivery to a patient. Here, the term "pharmaceutically acceptable" means that the carrier does not have more toxicity than the subject to be applied (prescribed) may adapt while not inhibiting activity of the active ingredient. The carrier is used to include an excipient, diluent, or auxiliary agent. For example, the carrier may be selected from the group consisting of lactose, dextrose, sucrose, sorbitol, mannitol, xylitol, erythritol, maltitol, starch, gum acacia, alginate, gelatin, calcium phosphate, calcium silicate, cellulose, methyl cellulose, polyvinyl pyrrolidone, water, physiological saline, buffer such as PBS, methyl hydroxybenzoate, propyl hydroxybenzoate, talc, magnesium stearate, and mineral oil. The composition may include a filler, an anti-coagulant, a lubricant, a wetting agent, a flavoring agent, an emulsifier, a preservative, or a combination thereof.

Specifically, by including a pharmaceutically acceptable carrier in addition to an active ingredient, the pharmaceutical composition may be prepared into a parenteral formulation depending on its route of administration using conventional methods known in the art.

When the pharmaceutical composition is prepared into a parenteral formulation, it may be made into preparations in the form of injections, transdermal patches, nasal inhalants, or suppositories with suitable carriers according to methods known in the art. In a case of being made into injections, sterile water, ethanol, polyol such as glycerol or propylene glycol, or a mixture thereof may be used as a suitable carrier; and an isotonic solution, such as Ringer's solution, phosphate buffered saline (PBS) containing triethanolamine or sterile water for injection, and 5% dextrose, or the like may preferably be used. Formulation of pharmaceutical compositions is known in the art, and reference may specifically be made to Remington's Pharmaceutical Sciences (19th ed., 1995) and the like. This document is considered part of the present specification.

The fusion protein or a pharmaceutical composition comprising the same may be administered to a patient in a therapeutically effective amount or a pharmaceutically effective amount.

As used herein, the term "administration" means introducing a predetermined substance into a subject by an appropriate method, and the composition may be administered through any general route as long as it may reach the target tissue. the composition may be administered by intraperitoneal administration, intravenous administration, intramuscular administration, subcutaneous administration, intradermal administration, topical administration, intranasal administration, or intrarectal administration, but is not limited thereto.

A preferred dosage of the pharmaceutical composition may range from 0.01 μg/kg to 10 g/kg, or 0.01 mg/kg to 1 g/kg, per day, depending on the patient's condition, body weight, sex, age, severity of the patient, and route of administration. The dosage may be administered once a day or may be divided into several times a day. Specifically, the administration may be performed once a day, 2 to 24 times a day, 1 to 2 times per 3 days, 1 to 6 times a week, 1 to 10 times per 2 weeks, 1 to 15 times per 3 weeks, 1 to 3 times per 4 weeks, or 1 to 12 times per year. However, since it may increase or decrease depending on the route of administration, severity of disease, gender, body weight, age, and the like, the scope of the present invention is not limited thereto. Such a dosage should not be construed as limiting the scope of the present invention in any aspect.

A subject to which the pharmaceutical composition may be applied (prescribed) is a mammal including a dog, cat, human, and the like, and preferably, may be a human. In addition to the active ingredient, the pharmaceutical composition of the present invention may further comprise any compound or natural extract, which is known to have a therapeutic effect on tumor.

## Treatment method using fusion protein

In another aspect of the present invention, there is provided a method for preventing or treating cancer, comprising administering a fusion protein comprising an antigen binding site, a first Fc domain or a variant thereof, and a second Fc domain or a variant thereof, or a pharmaceutical composition comprising the fusion protein as an active ingredient to a subject.

In another aspect of the present invention, there is provided a use of a fusion protein comprising an antigen binding site, a first Fc domain or a variant thereof, and a second Fc domain or a variant thereof, or a pharmaceutical composition comprising the fusion protein as an active ingredient for the treatment of cancer.

The fusion protein, pharmaceutical composition, cancer, prevention, and treatment are as described above.

Here, the subject may be an individual suffering from cancer. In addition, the subject may be a mammal, and preferably, may be a human.

Route of administration, dosage, and frequency of administration of the fusion protein may vary depending on the patient's condition and the presence or absence of side effects, and thus the fusion protein may be administered to a subject in various ways and amounts. The optimal administration method, dosage, and frequency of administration may be selected in an appropriate range by those of ordinary skill in the art. In addition, the fusion protein may be administered in combination with other drugs or physiologically active substances whose therapeutic effect is known with respect to a disease to be treated, or may be formulated in the form of combination preparations with other drugs.

## Mode for Carrying out the Invention

Hereinafter, the present invention will be described in more detail by way of the following examples. However, the following examples are only for illustrating the present invention, and the scope of the present invention is not limited only to these examples.

## Example 1. Design, preparation and analysis of a novel antibody with two Fc domains

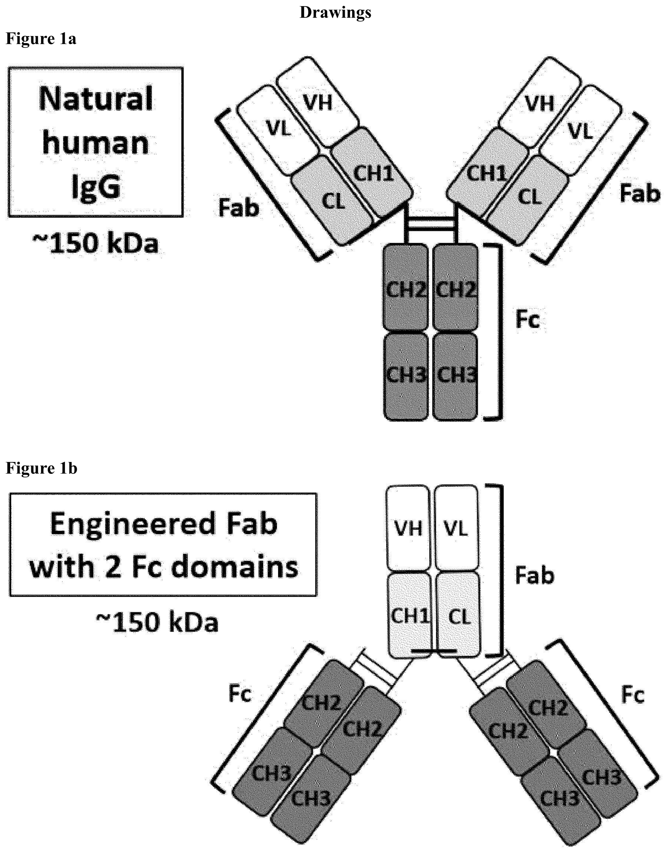

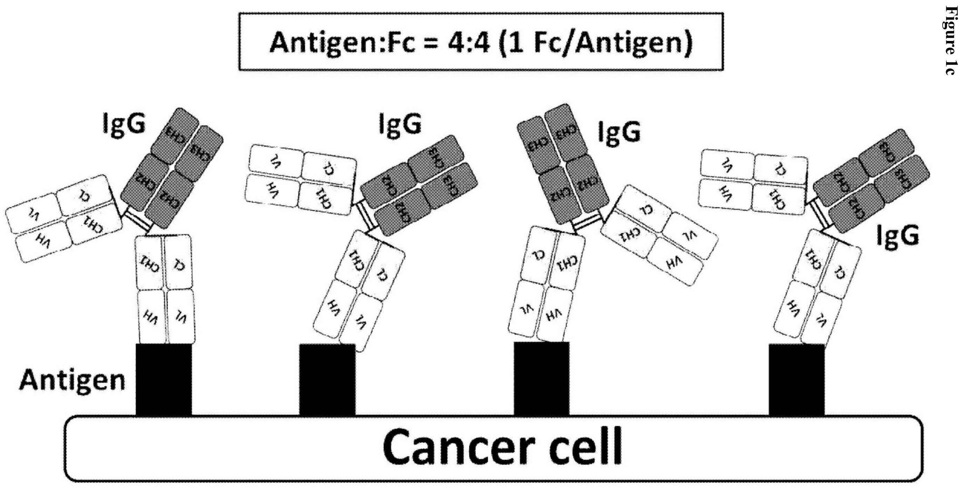

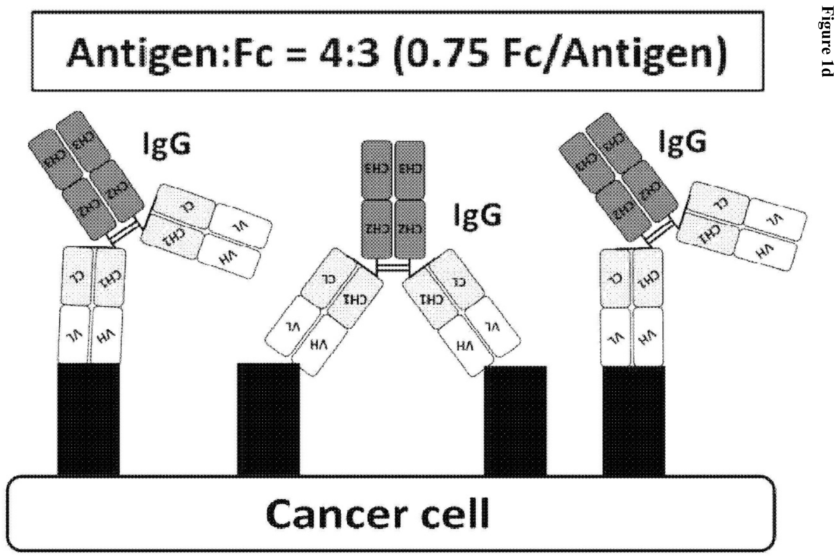

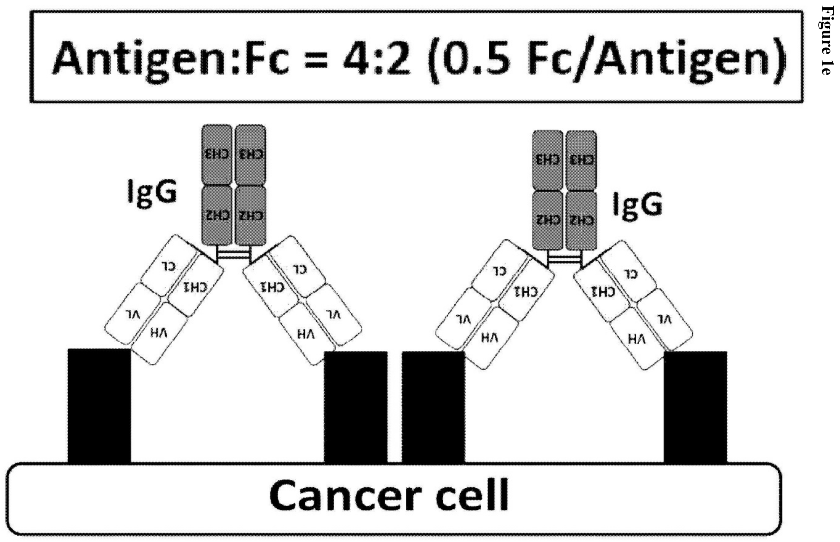

Naturally occurring human immunoglobulin G (IgG) consists of two fragment antigen-binding (Fab) regions and one fragment crystallizable (Fc) domain (Figure 1a). Human IgG binds to the target antigen in a monovalent or bivalent manner, and in some cases has 0.5 to 1 Fc domain per target antigen (Figures 1c to 1e).

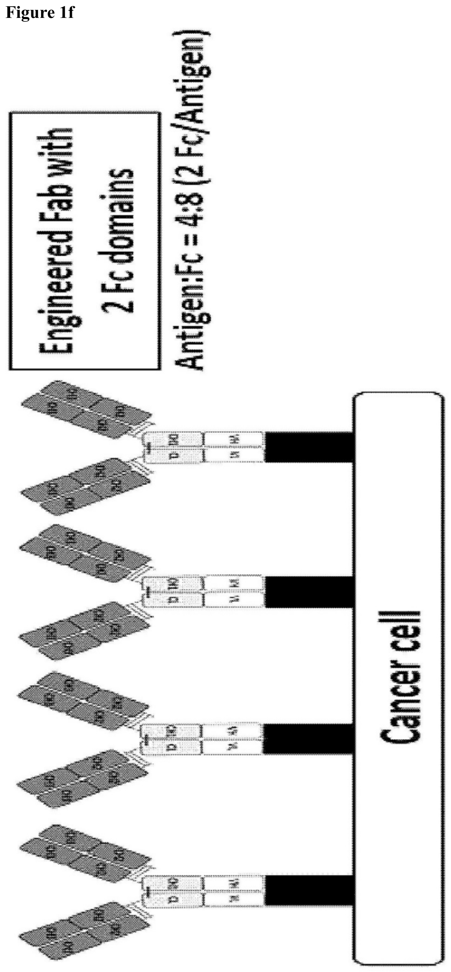

The object of the present invention is to improve effector function by increasing the amount of Fc domain present per antigen while having a molecular weight similar to that of an antibody and having a homogeneous composition. Therefore, we designed a novel antibody format with two Fc domains that has a molecular weight similar to that of a natural human IgG antibody (approximately 150 kDa) (Figure 1b). This form binds to a cancer cell surface antigen and enables Fc domains to be present on a cancer cell surface a maximum of four times compared to conventional antibodies (Figures 1b and 1f).

In order to implement the novel antibody format, trastuzumab was used as a template (Figure 2a). In order to improve pairing between the VH-CH1 region and the VL-CL region of the trastuzumab Fab region, a specific amino acid was substituted with Cys to introduce an artificial disulfide bond (Figures 2b to 2d).

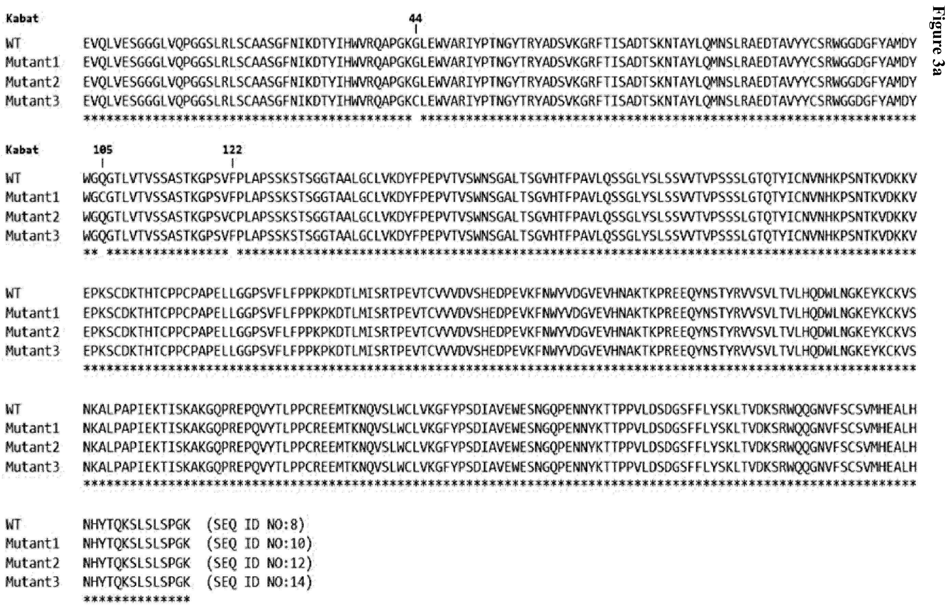

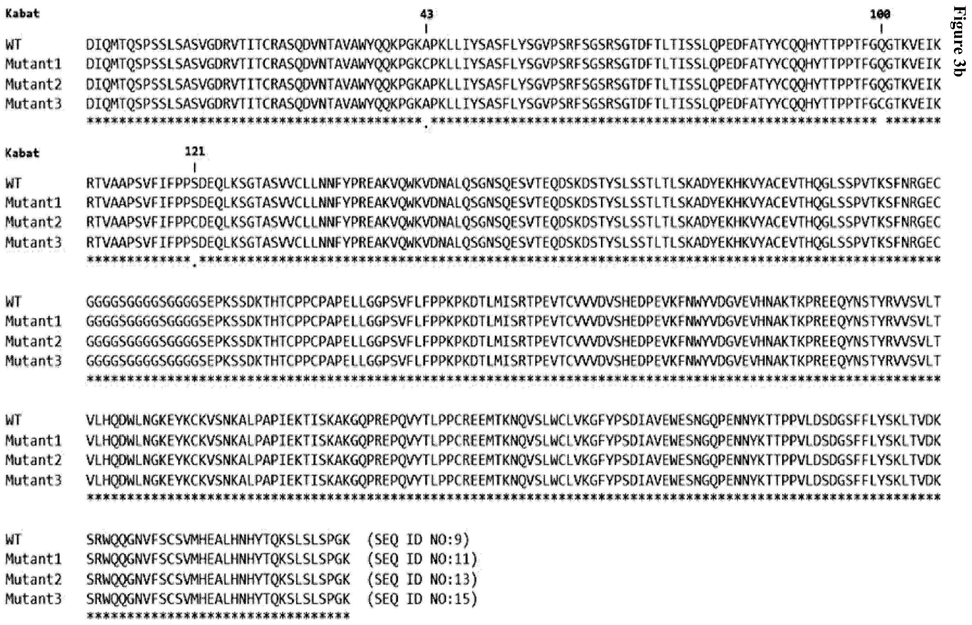

Fab in which glutamine (Q) at number 105 of the heavy chain and alanine (A) at number 43 of the light chain are substituted with cysteines, Fab in which phenylalanine (F) at number 122 of the heavy chain and serine (S) at number 121 of the light chain are substituted with cysteines, and Fab in which glycine (G) at number 44 of the heavy chain and glutamine (Q) at number 100 of the light chain are substituted with cysteines were designed, and they were referred to as Mutant 1, Mutant 2, and Mutant 3, respectively. Based on the above, the Fab-(Fc)$_2$ structure with trastuzumab as a template was referred to as wild type (WT) (Figure 2a), and the Fab-(Fc)$_2$ structures with Fab corresponding to Mutant 1 to Mutant 3 were referred to as M1, M2, and M3, respectively (Figures 2b to 2d, 3a, and 3b).

The notation of the positions of amino acids constituting an antibody follows the Kabat numbering rules. In order to minimize unwanted Fc-related byproducts, knob-into-hole mutation technology (Merchant et al, Nat. Biotechnol. 1998) was applied to the Fc domain (SEQ ID NO: 3) of human immunoglobulin G1 to design polypeptides of Fc with knob mutation (S354C, T366W; SEQ ID NO: 4) and Fc with hole mutation (Y349C, T366S, L368A, Y407V; SEQ ID NO: 5). In order to provide additional flexibility between a CL region and a hinge, a (G$_4$S)$_3$ linker was introduced (SEQ ID NO: 6). The WT consists of the polypeptides of Fc-Hole (SEQ ID NO: 7), TraH-WT-Knob (SEQ ID NO: 8), and TraL-WT-Knob (SEQ ID NO: 9), and was prepared by co-transfection of expression vectors loaded with polynucleotides encoding the polypeptides into the EXPICHO-S™ (Gibco, A29127) cell line.

M1 consists of the polypeptides of Fc-Hole (SEQ ID NO: 7), TraH-Q105C-Knob (SEQ ID NO: 10), and TraL-A43C-Knob (SEQ ID NO: 11), and was prepared by co-transfection of an expression vector loaded with a polynucleotide encoding the polypeptide into the EXPICHO-S™ cell line.

M2 consists of the polypeptides of Fc-Hole (SEQ ID NO: 7), TraH-F122C-Knob (SEQ ID NO: 12), and TraL-S121C-Knob (SEQ ID NO: 13), and was prepared by co-transfection of an expression vector loaded with a polynucleotide encoding the polypeptide into the EXPICHO-S™ cell line.

M3 consists of the polypeptides of Fc-Hole (SEQ ID NO: 7), TraH-G44C-Knob (SEQ ID NO: 14), and TraL-Q100C-Knob (SEQ ID NO: 15), and was prepared by co-transfection of an expression vector loaded with a polynucleotide encoding the polypeptide into the EXPICHO-S™ cell line.

The proteins prepared as described above were purified using an AKTA pure 25 (Cytiva) or AKTA avant 150 (Cytiva) protein isolation purification system equipped with a CAPTURESELECT™ CH1-XL Pre-packed Column (Thermo Scientific, 494346205) purification column. The purified product was further subjected to affinity chromatography using a KappaSelect resin (Cytiva, 17545801), and then concentrated using an Amicon Ultra-15 centrifugal filter unit (Merck millipore). For the final purified product, the absorbance of at 280 nm was measured using a NanoDrop One trace spectrophotometer (Thermo Fisher Scientific), and the concentration was analyzed and quantified based on the sample's intrinsic extinction coefficient and molecular weight.

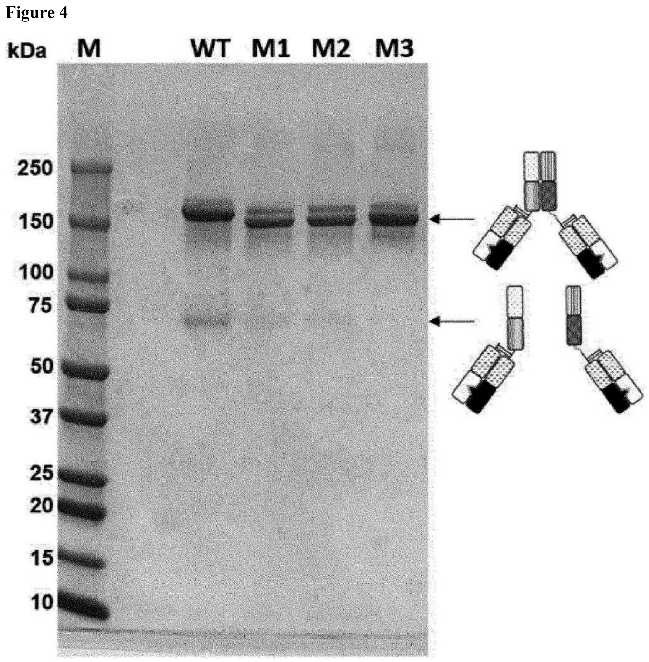

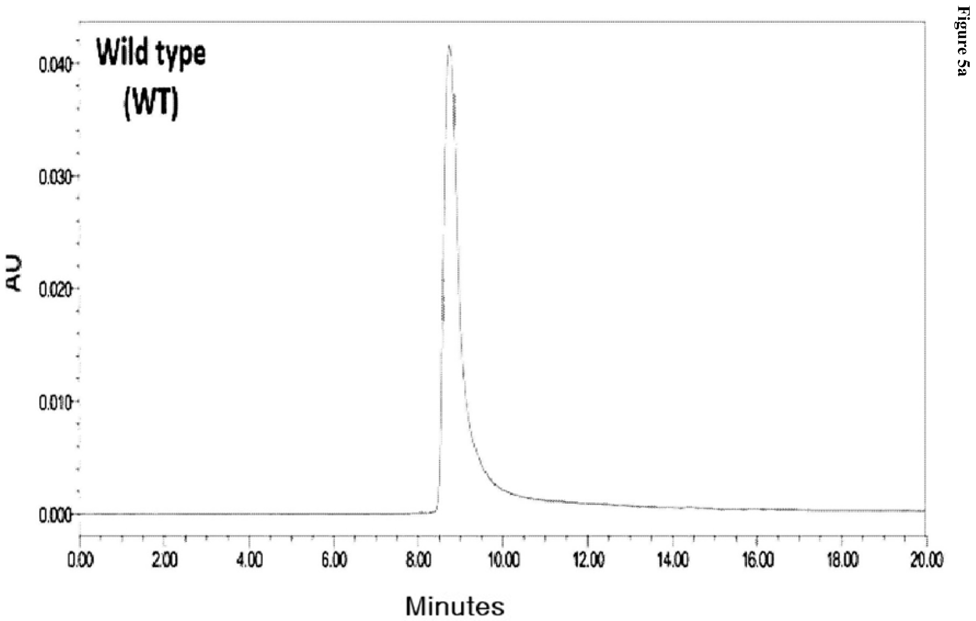

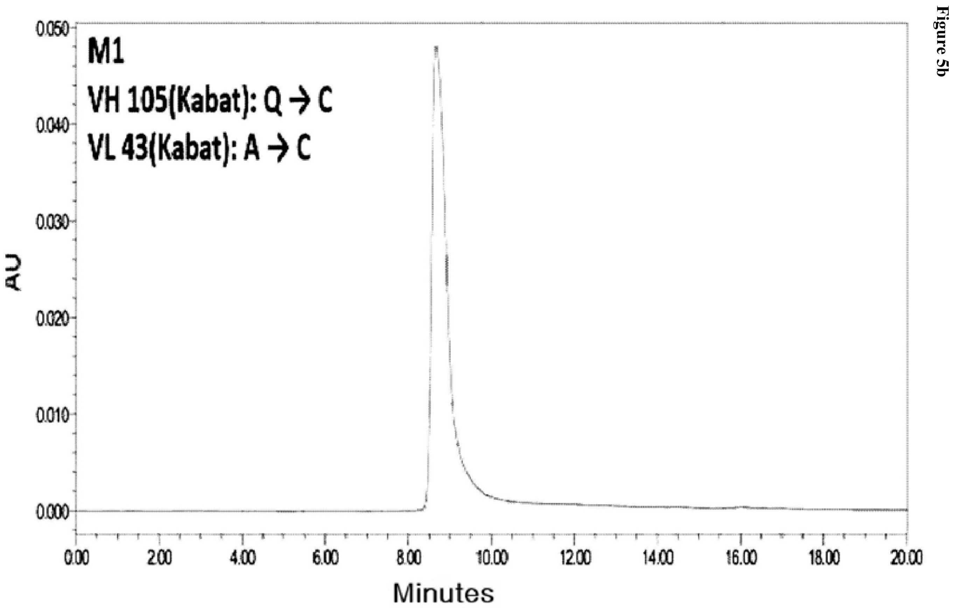

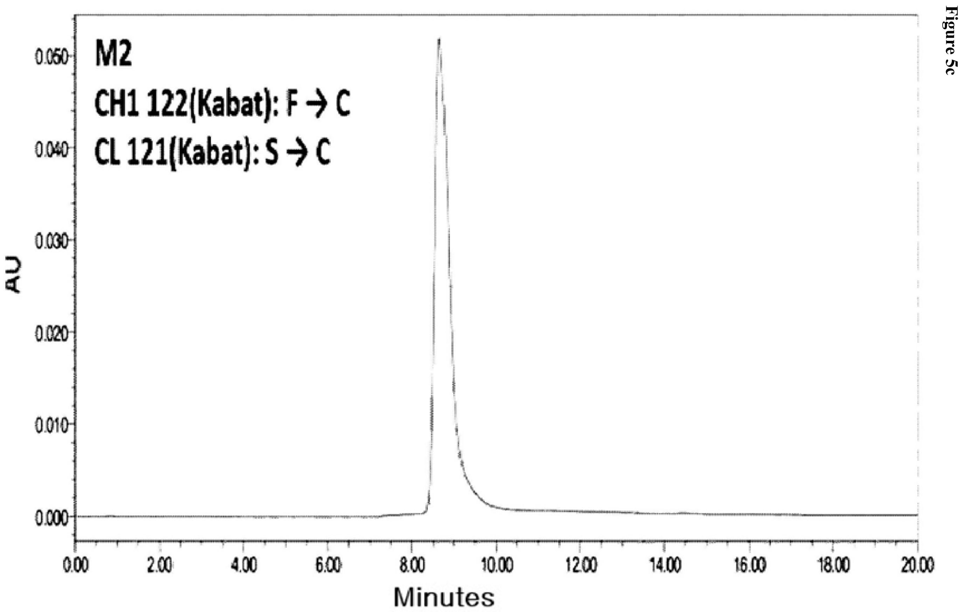

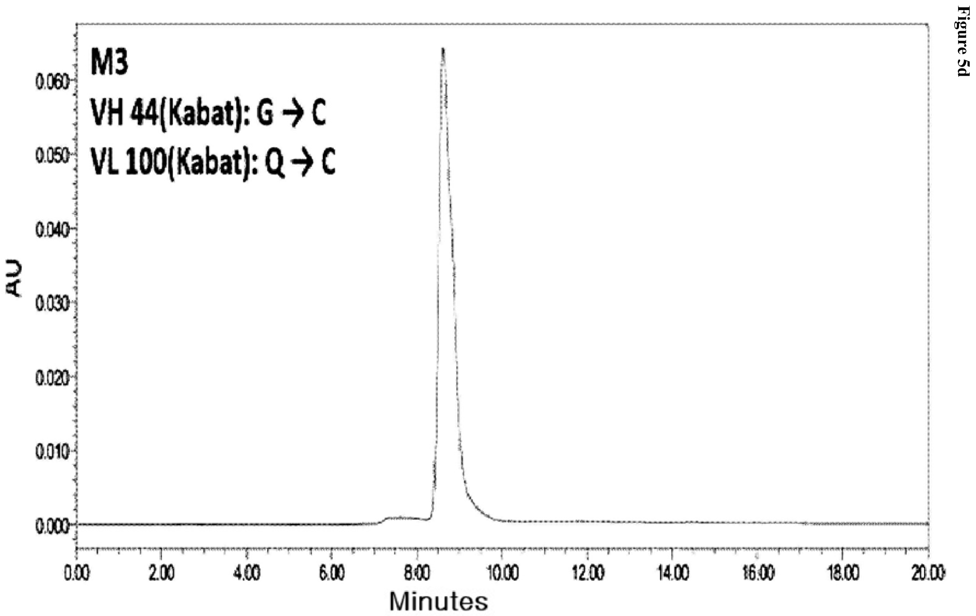

The above purified product was analyzed using sodium dodecyl sulfate polyacrylamide gel electrophoresis (SDS-PAGE) and size exclusion chromatography (SEC) (Figures 4 and 5a to 5d).

Bio-rad's electrophoresis gel and system were used for SDS-PAGE analysis, and the process was performed under non-reducing conditions. After electrophoresis, each size was identified by staining each fusion protein with Coomassie Brilliant Blue.

As a result, as shown in Figure 4, WT, M1, M2, and M3 were identified at about 150 kDa. The monomer bands were identified at about 75 kDa in WT which has no additional disulfide bond introduced into the Fab interface. Similarly, for M1 and M2, a trace amount of the monomer bands was identified at 75 kDa. However, for M3, almost no monomer bands were identified probably because most of the monomers were easily paired through the formation of disulfide bonds.

For size exclusion chromatography analysis, an Alliance® HPLC - e2695 Separations Module (Waters, 2695) equipped with an Agilent Bio SEC-3 HPLC column (Agilent, 5190-2511) was used.

As shown in Figures 5a to 5d, the analysis showed that the main product was identified at a retention time of about 8.6 minutes to about 8.8 minutes.

Based on the M3 structure, the impact of the linker that connects the CL domain with the hinge region on the structural integrity of the antibody were analyzed. M3 has a 15-mer polypeptide linker consisting of (G$_4$S)$_3$, and V1 (SEQ ID NO: 7, SEQ ID NO: 14, SEQ ID NO: 16) and V2 (SEQ ID NO: 7, SEQ ID NO: 14, SEQ ID NO: 17) have polypeptide linkers of (G$_4$S)$_2$ and G$_4$S, respectively, and V3 (SEQ ID NO: 7, 14, 18) is a fusion protein in which the CL domain and the hinge are directly linked without a linker (Figures 6a to 6d).

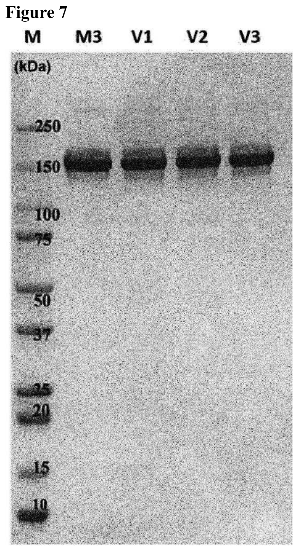

By SDS-PAGE analysis for identifying the size of the fusion protein, as shown in Figure 7, the main product was identified at about 150 kDa, and the byproduct was not identified. The above results showed that the presence or absence of a linker between the CL domain and the hinge had no significant effect on the formation of the byproduct.

Based on the above results, it was found that the Fab-(Fc)2 structure was stably formed when the VH 44 and VL 100 positions of Fab were substituted with Cys.

[Table 7]

| Name | Sequence | SEQ ID NO |
|------|----------|-----------|
| Trastuzumab VH-CH1 domain | EVQLVESGGGLVQPGGSLRLSCAASGFNIKDTYIHWVRQAPGKGLEWVARIYPTNGYTRYA DSVKGRFTISADTSKNTAYLQMNSLRAEDTAVYYCSRWGGDGFYAMDYWGQGTLVTVSSAS TKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKV | 1 |
| Trastuzumab VL-CL domain | DIQMTQSPSSLSASVGDRVTITCRASQDVNTAVAWYQQKPGKAPKLLIYSASFLYSGVPSR FSGSRSGTDFTLTISSLQPEDFATYYCQQHYTTPPTFGQGTKVEIKRTVAAPSVFIFPPSD EQLKSGTASVVCLLNNFYPREAKVQWKVDNALQSGNSQESVTEQDSKDSTYSLSSTLTLSK ADYEKHKVYACEVTHQGLSSPVTKSFNRGEC | 2 |
| IgG1 Fc(CH2-CH3) | APELLGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYTLP PSREEMTKNQVSLTCLVKGFYPSDIAVEWESNGQPENNYKTTPPVLDSDGSFFLYSKLTVD KSRWQQGNVFSCSVMHEALHNHYTQKSLSLSPGK | 3 |
| IgG1 Fc(Knob; S354C, T366W) | APELLGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYTLP PCREEMTKNQVSLWCLVKGFYPSDIAVEWESNGQPENNYKTTPPVLDSDGSFFLYSKLTVD KSRWQQGNVFSCSVMHEALHNHYTQKSLSLSPGK | 4 |
| IgG1 Fc(Hole; Y349C, T366S, L368A, Y407V) | APELLGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVCTLP PSREEMTKNQVSLSCAVKGFYPSDIAVEWESNGQPENNYKTTPPVLDSDGSFFLVSKLTVD KSRWQQGNVFSCSVMHEALHNHYTQKSLSLSPGK | 5 |
| 15-mer Linker | GGGGSGGGGSGGGGS | 6 |
| Fc-Hole | DKTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKGQ PREPQVCTLPPSREEMTKNQVSLSCAVKGFYPSDIAVEWESNGQPENNYKTTPPVLDSDGS FFLYSKLTVDKSRWQQGNVFSCSVMHEALHNHYTQKSLSLSPGK | 7 |
| TraII-WT-Knob | EVQLVESGGGLVQPGGSLRLSCAASGFNIKDTYIHWVRQAPGKGLEWVARIYPTNGYTRYA DSVKGRFTISADTSKNTAYLQMNSLRAEDTAVYYCSRWGGDGFYAMDYWGQGTLVTVSSAS | 8 |

| | | |
|---|---|---|
| | TKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | |
| TraL-WT-Knob | DIQMTQSPSSLSASVGDRVTITCRASQDVNTAVAWYQQKPGKAPKLLIYSASFLYSGVPSRFSGSRSGTDFTLTISSLQPEDFATYYCQQHYTTPPTFGQGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGECGGGGSGGGGSGGGGSEPKSSDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 9 |
| TraH-Q105C-Knob | EVQLVESGGGLVQPGGSLRLSCAASGFNIKDTYIHWVRQAPGKGLEWVARIYPTNGYTRYADSVKGRFTISADTSKNTAYLQMNSLRAEDTAVYYCSRWGGDGFYAMDYWGCGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 10 |
| TraL-A43C-Knob | DIQMTQSPSSLSASVGDRVTITCRASQDVNTAVAWYQQKPGKCPKLLIYSASFLYSGVPSRFSGSRSGTDFTLTISSLQPEDFATYYCQQHYTTPPTFGQGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGECGGGGSGGGGSGGGGSEPKSSDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVD | 11 |

| | | |
|---|---|---|
| | KSRWQQGNVFSCSVMHEALHNHYTQKSLSLSPGK | |
| TraH-F122C-Knob | EVQLVESGGGLVQPGGSLRLSCAASGFNIKDTYIHWVRQAPGKGLEWVARIYPTNGYTRYA DSVKGRFTISADTSKNTAYLQMNSLRAEDTAVYYCSRWGGDGFYAMDYWGQGTLVTVSSAS TKGPSVCPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPELLGGPSVF LFPPKPKDTLMISRTPEVTCVVVDVSHEDPEVKFNWYVDGVEVHNAKTKPREEQYNSTYRV VSVLTVLHQDWLNGKEYKCKVSNKALPAPIEKTISKAKGQPREPQVYTLPPCREEMTKNQV SLWCLVKGFYPSDIAVEWESNGQPENNYKTTPPVLDSDGSFFLYSKLTVDKSRWQQGNVFS CSVMHEALHNHYTQKSLSLSPGK | 12 |
| TraL-S121C-Knob | DIQMTQSPSSLSASVGDRVTITCRASQDVNTAVAWYQQKPGKAPKLLIYSASFLYSGVPSR FSGSRSGTDFTLTISSLQPEDFATYYCQQHYTTPPTFGQGTKVEIKRTVAAPSVFIFPPCD EQLKSGTASVVCLLNNFYPREAKVQWKVDNALQSGNSQESVTEQDSKDSTYSLSSTLTLSK ADYEKHKVYACEVTHQGLSSPVTKSFNRGECGGGGSGGGGSGGGGSEPKSSDKTHTCPPCP APELLGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYTLP PCREEMTKNQVSLWCLVKGFYPSDIAVEWESNGQPENNYKTTPPVLDSDGSFFLYSKLTVD KSRWQQGNVFSCSVMHEALHNRFTQKSLSLSPGK | 13 |
| TraH-G44C-Knob | EVQLVESGGGLVQPGGSLRLSCAASGFNIKDTYIHWVRQAPGKCLEWVARIYPTNGYTRYA DSVKGRFTISADTSKNTAYLQMNSLRAEDTAVYYCSRWGGDGFYAMDYWGQGTLVTVSSAS TKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPELLGGPSVF LFPPKPKDTLMISRTPEVTCVVVDVSHEDPEVKFNWYVDGVEVHNAKTKPREEQYNSTYRV VSVLTVLHQDWLNGKEYKCKVSNKALPAPIEKTISKAKGQPREPQVYTLPPCREEMTKNQV SLWCLVKGFYPSDIAVEWESNGQPENNYKTTPPVLDSDGSFFLYSKLTVDKSRWQQGNVFS CSVMHEALHNHYTQKSLSLSPGK | 14 |
| TraL-Q100C-Knob | DIQMTQSPSSLSASVGDRVTITCRASQDVNTAVAWYQQKPGKAPKLLIYSASFLYSGVPSR FSGSRSGTDFTLTISSLQPEDFATYYCQQHYTTPPTFGCGTKVEIKRTVAAPSVFIFPPSD EQLKSGTASVVCLLNNFYPREAKVQWKVDNALQSGNSQESVTEQDSKDSTYSLSSTLTLSK ADYEKHKVYACEVTHQGLSSPVTKSFNRGECGGGGSGGGGSGGGGSEPKSSDKTHTCPPCP | 15 |

| | | |
|---|---|---|
| | APELLGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYTLP PCREEMTKNQVSLWCLVKGFYPSDIAVEWESNGQPENNYKTTPPVLDSDGSFFLYSKLTVD KSRWQQGNVFSCSVMHEALHNHYTQKSLSLSPGK | |
| TraL-Q100C-Knob1 | DIQMTQSPSSLSASVGDRVTITCRASQDVNTAVAWYQQKPGKAPKLLIYSASFLYSGVPSR FSGSRSGTDFTLTISSLQPEDFATYYCQQHYTTPPTFGCGTKVEIKRTVAAPSVFIFPPSD EQLKSGTASVVCLLNNFYPREAKVQWKVDNALQSGNSQESVTEQDSKDSTYSLSSTLTLSK ADYEKHKVYACEVTHQGLSSPVTKSFNRGECGGGGSGGGGSEPKSSDKTHTCPPCPAPELL GGPSVFLFPPKPKDTLMISRTPEVTCVVVDVSHEDPEVKFNWYVDGVEVHNAKTKPREEQY NSTYRVVSVLTVLHQDWLNGKEYKCKVSNKALPAPIEKTISKAKGQPREPQVYTLPPCREE MTKNQVSLWCLVKGFYPSDIAVEWESNGQPENNYKTTPPVLDSDGSFFLYSKLTVDKSRWQ QGNVFSCSVMHEALHNHYTQKSLSLSPGK | 16 |
| TraL-Q100C-Knob2 | DIQMTQSPSSLSASVGDRVTITCRASQDVNTAVAWYQQKPGKAPKLLIYSASFLYSGVPSR FSGSRSGTDFTLTISSLQPEDFATYYCQQHYTTPPTFGCGTKVEIKRTVAAPSVFIFPPSD EQLKSGTASVVCLLNNFYPREAKVQWKVDNALQSGNSQESVTEQDSKDSTYSLSSTLTLSK ADYEKHKVYACEVTHQGLSSPVTKSFNRGECGGGGSEPKSSDKTHTCPPCPAPELLGGPSV FLFPPKPKDTLMISRTPEVTCVVVDVSHEDPEVKFNWYVDGVEVHNAKTKPREEQYNSTYR VVSVLTVLHQDWLNGKEYKCKVSNKALPAPIEKTISKAKGQPREPQVYTLPPCREEMTKNQ VSLWCLVKGFYPSDIAVEWESNGQPENNYKTTPPVLDSDGSFFLYSKLTVDKSRWQQGNVF SCSVMHEALHNHYTQKSLSLSPGK | 17 |
| TraL-Q100C-Knob3 | DIQMTQSPSSLSASVGDRVTITCRASQDVNTAVAWYQQKPGKAPKLLIYSASFLYSGVPSR FSGSRSGTDFTLTISSLQPEDFATYYCQQHYTTPPTFGCGTKVEIKRTVAAPSVFIFPPSD EQLKSGTASVVCLLNNFYPREAKVQWKVDNALQSGNSQESVTEQDSKDSTYSLSSTLTLSK ADYEKHKVYACEVTHQGLSSPVTKSFNRGECEPKSSDKTHTCPPCPAPELLGGPSVFLFPP KPKDTLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPCREEMTKNQVSLWC LVKGFYPSDIAVEWESNGQPENNYKTTPPVLDSDGSFFLYSKLTVDKSRWQQGNVFSCSVM HEALHNHYTQKSLSLSPGK | 18 |

[Table 8]

| Name | Sequence | SEQ ID NO |
|------|----------|-----------|
| Trastuzumab VH-CH1 domain | GAAGTGCAGCTGGTCGAAAGTGGCGGTGGACTTGTGCAACCTGGCGGTAGCCTCCGTCTCA GCTGCCGCTGCAAGTGGGTTCAACATCAAGGACACTTATATTCATTGGGTCCGACAGGCACC TGCGAAAGGCTTTGGAGTGGGTCGCACGGATCTATCCCACTAATGGTTACACAAGATATGCC GATTCAGTAAAAGGCCGGTTTACAATCAGCGCAGATACTTCAAAAAACACTGCCTATCTTC AAATGAACTCACTTCGAGCAGAAGACACAGCCGTCTATTATTGTAGTCGTTGGGGAGGCGA CGGCTTTTATGCTATGGACTACTGGGGACAAGGAACTCTGGTCACAGTTTCATCAGCTAGC ACCAAAGGACCTAGTGTTTTTCCTCTTGCCCCCTTCCTCAAAGTCTACCTCTGGGGGGACAG CCGCTCTGGGCTGCCTGGTCAAGGATTATTTCCCAGAGCCTGTCACTGTCAGTTGGAACTC TGCAGCCTTGACTTCTGGTGTTCATACATTTCCTGCTGTCCTTCAAAGCAGCGGCTTGTAC TCATTGTCTTCTGTTGTGACAGTACCCTCAAGCAGCCTCGGCACTCAGACATACATCTGCA ATGTCAACCACAAACCCTCAAATACAAAGGTAGATAAAAAGTC | 452 |
| Trastuzumab VL-CL domain | GATATTCAGATGACTCAGAGTCCTAGTTCCCTCAGCGCCTCCGTAGGCGACAGAGTTACAA TAACTTGCCCGAGCAAGCCAAGACGTAAACACTGCAGTCGCCTGGTACCAACAGAAACCAGG CAAAGCTCCAAAACTCTTGATTTACAGTGCTTCCTTCCTTTATAGTGGCGTTCCAAGCCGC TTCAGCGGCAGCCGCTCTGGCACCGACTTCACTCTCACTATTTCTTCCTTGCAACCTGAAG ACTTCGCCACTTATTATTGCCAGCAACACTACACAACACCCCCAACATTCGGACAGGGCAC AAAGGTAGAAATAAAACGTACCGGTGGCAGCTCCCAGCGTTTTTATCTTTTCCCCCATCCGAC GAGCCAGCTCAAGAGTGGCCACTGCCTCTGTAGTTTGTTTGCTGAATAACTTCTATCCACGTG AAGCCAAAAGTACAGTGGAAGGTCGATAATGCCCTTCAGAGCCGGTAACAGCCAAGAAAGTGT TACCGAGCAAGATTCCAAAGATTCCACTTACAGTCTGTCCAGCACATTGACACTGAGTAAG GCTGATTACGAAAAACACAAGGTGTACGCATGCGAGGTGACACACCAAGGTCTTTCATCTC CTGTAACTAAGAGCTTTAACCGGGGAGAATGT | 453 |
| IgG1 Fc(CH2-CH3) | GCACCTGAACTCCTGGGGGGACCGTCAGTCTTCCTCTTCCCCCCAAAACCCAAGGACACCC TCATGATCTCCCGGACCCCTGAGGTCACATGCGTGGTGGTGGACGTGAGCCACGAAGACCC TGAGGTCAAGTTCAACTGGTACGTGGACGGCGTGGAGGTGCATAATGCCAAGACAAAGCCG CGGGAGGAGCAGTACAACAGCACGTACCGTGTGGTCAGCGTCCTCACCGTCCTGCACCAGG ACTGGCTGAATGGCAAGGAGTACAAGTGCAAGGTCTCCAACAAAGCCCTCCCAGCCCCCAT | 454 |

| | | |
|---|---|---|
| | CGAGAAAACCATCTCCAAAGCCAAAGGGCAGCCCCGAGAACCACAGGTGTACACCCTGCCCCCAAGCCGGGAGGAGATGACCAAGAACCAGGTCAGCCTGACGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | |
| IgG1 Fc(Knob; S354C, T366W) | GCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATGCCGGGAGGAGATGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | 455 |
| IgG1 Fc(Hole; Y349C, T366S, L368A, Y407V) | GCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTGCACCCTGCCCCCATCCCGGGAGGAGATGACCAAGAACCAGGTCAGCCTGAGCTGCGCGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCGTGAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | 456 |
| 15-mer Linker | GGTGGTGGGGGCAGCGGGGGCGGAGGTAGTGGAGGCGGCGGTAGT | 457 |

| Fc-Hole | GACAAAACTCACACATGCCCACCGTGCCCAGCACCTGAACTCCTGGGGGGACCGTCAGTCT TCCTCTTCCCCCCAAAACCCAAGGACACCCTCATGATCTCCCGGACCCCTGAGGTCACATG CGTGGTGGTGGACGTGAGCCACGAAGACCCTGAGGTCAAGTTCAACTGGTACGTGGACGGC GTGGAGGTGCATAATGCCAAGACAAAGCCGCGGGAGGAGCAGTACAACAGCACGTACCGTG TGGTCAGCGTCCTCACCGTCCTGCACCAGGACTGGCTGAATGGCAAGGAGTACAAGTGCAA GGTCTCCAACAAAGCCCTCCCAGCCCCCATCGAGAAAACCATCTCCAAAGCCAAAGGGCAG CCCCGAGAACCACAGGTGTGCACCCTGCCCCCATCCCGGGAGGAGATGACCAAGAACCAGG TCAGCCTGAGCTGCGCGGTCAAAGGCTTCTATCCCAGCGACATCGCCGTGGAGTGGGAGAG CAATGGGCAGCCGGAGAACAACTACAAGACCACGCCTCCCGTGCTGGACTCCGACGGCTCC TTCTTCCTCGTGAGCAAGCTCACCGTGGACAAGAGCAGGTGGCAGCAGGGGAACGTCTTCT CATGCTCCGTGATGCATGAGGCTCTGCACAACCACTACACGCAGAAGAGCCTCTCCCTGTC TCCGGGTAAA | 458 |
|---|---|---|
| TraH-WT-Knob | GAAGTTCAACTTGTGGAGTCTGGAGGCGGTTTGGTACAACCTGGAGGTTCACTTCGTTTGT CATGTGCTGCCTCTGGGTTTAATATCAAGGATACTTATATTCATTGGGTTCGCCAGGCACC CGGAAAAGGGCTGGAATGGGTGGCCCGTATATATCCCACTAACGGTTACACTCGATATGCT GACTCTGTAAAGGGTCGATTTACTATATCCGCTGATACTAGCAAAAACACTGCCTACCTCC AGATGAACAGTCTGCGCGCCGAGGATACAGCAGTTTATTATTGTTCCCGATGGGGAGGTGA CGGGTTCTATGCCATGGACTACTGGGGTCAAGGGACACTGGTAACCGTTTCTTCTGCTAGC ACCAAAGGACCTAGTGTTTTTCCTCTTGCCCCCTTCCTCAAAGTCTACCTCTGGGGGGACAG CCGCTCTGGGCTGCCTGGTCAAGGATTATTTCCCAGAGCCTGTCACTGTCAGTTGGAACTC TGGAGCCTTGACTTCTGGTGTTCATACATTTCCTGCTGTCCTTCAAAGCAGCGGCTTGTAC TCATTGTCTTCTGTTGTGACAGTACCCTCAAGCAGCCTCGGCACTCAGACATACATCTGCA ATGTCAACCACAAACCCTCAAATACAAAGGTAGATAAAAAAGTCGAACCAAAGTCTTGTGA CAAAACTCACACGTGCCCACCGTGCCCAGCACCTGAACTCCTGGGGGGACCGTCAGTCTTC CTCTTCCCCCCAAAACCCAAGGACACCCTCATGATCTCCCGGACCCCTGAGGTCACATGCG TGGTGGTGGACGTGAGCCACGAAGACCCTGAGGTCAAGTTCAACTGGTACGTGGACGGCGT GGAGGTGCATAATGCCAAGACAAAGCCGCGGGAGGAGCAGTACAACAGCACGTACCGTGTG GTCAGCGTCCTCACCGTCCTGCACCAGGACTGGCTGAATGGCAAGGAGTACAAGTGCAAGG TCTCCAACAAAGCCCTCCCAGCCCCCATCGAGAAAACCATCTCCAAAGCCAAAGGGCAGCC | 459 |

| | | |
|---|---|---|
| | CCGAGAACCACAGGTGTACACCCTGCCCCCATGCCGGGAGGAGATGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | |
| TraL-WT-Knob | GACATTCAGATGACCCAATCCCCCTCTAGCCTTTCAGCCTCCGTAGGGGACAGAGTTACAATAACCTGTAGGGCTAGTCAGGACGTTAATACAGCAGTCGCATGGTACCAGCAGAAGCCCGGTAAGGCTCCTAAGCTTTTGATCTACAGTGCTTCTTTCCTGTATTCTGGTGTACCTAGCCGATTCTCAGGCTCTCGGAGTGGCACAGACTTCACTCTGACAATTTCAAGTCTCCAGCCAGAAGATTTTGCAACCTACTACTGCCAACAACACTACACTACTCCTCCAACCTTCGGACAGGGCACAAAAGTAGAGATTAAGCGTACGGTGGCAGCTCCCAGCGTTTTTATCTTTCCCCCATCCGACGAGCAGCTCAAGAGTGGCACTGCCTCTGTAGTTTGTTTGCTGAATAACTTCTATCCACGTGAAGCAAAAGTACAGTGGAAGGTCGATAATGCCCTTCAGAGCGGTAACAGCCAAGAAAGTGTTACCGAGCAAGATTCCAAAGATTCCACTTACAGTCTGTCCAGCACATTGACACTGAGTAAGGCTGATTACGAAAAACACAAGGTGTACGCATGCGAGGTGACACACCAAGGTCTTTCATCTCCTGTAACTAAGAGCTTTAACCGGGGAGAATGTGGTGGTGGGGGCAGCGGGGGCGGAGGTAGTGGAGGCGGCGGTAGTGAACCAAAGAGTAGTGACAAAACTCACACGTGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATGCCGGGAGGAGATGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | 460 |

| TraH-Q105C-Knob | GAAGTTCAACTTGTGGAGTCTGGAGGCGGTTTGGTACAACCTGGAGGTTCACTTCGTTTGT CATGTGCTGCCTCTGGGTTTAATATCAAGGATACTTATATTCATTGGGTTCGCCAGGCACC CGGAAAAGGGCTGGAATGGGTGGCCCGTATATATCCCACTAACGGTTACACTCGATATGCT GACTCTGTAAAGGGTCGATTTACTATATCCGCTGATACTAGCAAAAACACTGCCTACCTCC AGATGAACAGTCTGCGCGCCGAGGATACAGCAGTTTATTATTGTTCCCGATGGGGAGGTGA CGGGTTCTATGCCATGGACTACTGGGGTTGTGGGACACTGGTAACCGTTTCTTCTGCTAGC ACCAAAGGACCTAGTGTTTTTCCTCTTGCCCCTTCCTCAAAGTCTACCTCTGGGGGGACAG CCGCTCTGGGCTGCCTGGTCAAGGATTATTTCCCAGAGCCTGTCACTGTCAGTTGGAACTC TGGAGCCTTGACTTCTGGTGTTCATACATTTCCTGCTGTCCTTCAAAGCAGCGGCTTGTAC TCATTGTCTTCTGTTGTGACAGTACCCTCAAGCAGCCTCGGCACTCAGACATACATCTGCA ATGTCAACCACAAACCCTCAAATACAAAGGTAGATAAAAAAGTCGAACCAAAGTCTTGTGA CAAAACTCACACGTGCCCACCGTGCCCAGCACCTGAACTCCTGGGGGGACCGTCAGTCTTC CTCTTCCCCCCAAAACCCAAGGACACCCTCATGATCTCCCGGACCCCTGAGGTCACATGCG TGGTGGTGGACGTGAGCCACGAAGACCCTGAGGTCAAGTTCAACTGGTACGTGGACGGCGT GGAGGTGCATAATGCCAAGACAAAGCCGCGGGAGGAGCAGTACAACAGCACGTACCGTGTG GTCAGCGTCCTCACCGTCCTGCACCAGGACTGGCTGAATGGCAAGGAGTACAAGTGCAAGG TCTCCAACAAAGCCCTCCCAGCCCCCATCGAGAAAACCATCTCCAAAGCCAAAGGGCAGCC CCGAGAACCACAGGTGTACACCCTGCCCCCATGCCGGGAGGAGATGACCAAGAACCAGGTC AGCCTGTGGTGCCTGGTCAAAGGCTTCTATCCCAGCGACATCGCCGTGGAGTGGGAGAGCA ATGGGCAGCCGGAGAACAACTACAAGACCACGCCTCCCGTGCTGGACTCCGACGGCTCCTT CTTCCTCTACAGCAAGCTCACCGTGGACAAGAGCAGGTGGCAGCAGGGGAACGTCTTCTCA TGCTCCGTGATGCATGAGGCTCTGCACAACCACTACACGCAGAAGAGCCTCTCCCTGTCTC CGGGTAAA | 461 |
| TraL-A43C-Knob | GACATTCAGATGACCCAATCCCCCTCTAGCCTTTCAGCCTCCGTAGGGGACAGAGTTACAA TAACCTGTAGGGCTAGTCAGGACGTTAATACAGCAGTCGCATGGTACCAGCAGAAGCCCGG TAAGTGTCCTAAGCTTTTGATCTACAGTGCTTCTTTCCTGTATTCTGGTGTACCTAGCCGA TTCTCAGGCTCTCGGAGTGGCACAGACTTCACTCTGACAATTTCAAGTCTCCAGCCAGAAG ATTTTGCAACCTACTACTGCCAACAACACTACACTACTCCTCCAACCTTCGGACAGGGCAC AAAAGTAGAGATTAAGCGTACGGTGGCAGCTCCCAGCGTTTTTATCTTTCCCCCATCCGAC | 462 |

| | | |
|---|---|---|
| | GAGCAGCTCAAGAGTGGCACTGCCTCTGTAGTTTGTTTGCTGAATAACTTCTATCCACGTGAAGCAAAAGTACAGTGGAAGGTCGATAATGCCCTTCAGAGCGGTAACAGCCAAGAAAGTGTTACCGAGCAAGATTCCAAAGATTCCACTTACAGTCTGTCCAGCACATTGACACTGAGTAAGGCTGATTACGAAAAACACAAGGTGTACGCATGCGAGGTGACACACCAAGGTCTTTCATCTCCTGTAACTAAGAGCTTTAACCGGGGAGAATGTGGTGGTGGGGGCAGCGGGGGCGGAGGTAGTGGAGGCGGCGGTAGTGAACCAAAGAGTAGTGACAAAACTCACACGTGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATGCCGGGAGGAGATGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | |
| TraH-F122C-Knob | GAAGTTCAACTTGTGGAGTCTGGAGGCGGTTTGGTACAACCTGGAGGTTCACTTCGTTTGTCATGTGCTGCCTCTGGGTTTAATATCAAGGATACTTATATTCATTGGGTTCGCCAGGCACCCGGAAAAGGGCTGGAATGGGTGGCCCGTATATATCCCACTAACGGTTACACTCGATATGCTGACTCTGTAAAGGGTCGATTTACTATATCCGCTGATACTAGCAAAAACACTGCCTACCTCCAGATGAACAGTCTGCGCGCCGAGGATACAGCAGTTTATTATTGTTCCCGATGGGGAGGTGACGGGGTTCTATGCCATGGACTACTGGGGTCAAGGGACACTGGTAACCGTTTCTTCTGCTAGCACCAAAGGACCTAGTGTTTGTCCTCTTGCCCCCTTCCTCAAAGTCTACCTCTGGGGGGACAGCCGCTCTGGGCTGCCTGGTCAAGGATTATTTCCCAGAGCCTGTCACTGTCAGTTGGAACTCTGGAGCCTTGACTTCTGGTGTTCATACATTTCCTGCTGTCCTTCAAAGCAGCGGCTTGTACTCATTGTCTTCTGTTGTGACAGTACCCTCAAGCAGCCTCGGCACTCAGACATACATCTGCAATGTCAACCACAAACCCTCAAATACAAAGGTAGATAAAAAAGTCGAACCAAAGTCTTGTGACAAAACTCACACGTGCCCACCGTGCCCAGCACCTGAACTCCTGGGGGGACCGTCAGTCTTC | 463 |

| | | |
|---|---|---|
| | CTCTTCCCCCCCAAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATGCCGGGAGGAGATGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | |
| TraL-S121C-Knob | GACATTCAGATGACCCAATCCCCCCTCTAGCCTTTCAGCCTCCGTAGGGGACAGAGTTACAATAACCTGTAGGGCTAGTCAGGACGTTAATACAGCAGTCGCATGGTACCAGCAGAAGCCCGGTAAGGCTCCTAAGCTTTTGATCTACAGTGCTTCTTTCCTGTATTCTGGTGTACCTAGCCGATTCTCAGGCTCTCGGAGTGGCACAGACTTCACTCTGACAATTTCAAGTCTCCAGCCAGAAGATTTTGCAACCTACTACTGCCAACAACACTACACTACTCCTCCAACCTTCGGACAGGGCACAAAAGTAGAGATTAAGCGTACGGTGGCAGCTCCCAGCGTTTTTATCTTTCCCCCATGTGACGAGCAGCTCAAGAGTGGCACTGCCTCTGTAGTTTGTTTGCTGAATAACTTCTATCCACGTGAAGCAAAAGTACAGTGGAAGGTCGATAATGCCCTTCAGAGCGGTAACAGCCAAGAAAGTGTTACCGAGCAAGATTCCAAAGATTCCACTTACAGTCTGTCCAGCACATTGACACTGAGTAAGGCTGATTACGAAAAACACAAGGTGTACGCATGCGAGGTGACACACCAAGGTCTTTCATCTCCTGTAACTAAGAGCTTTAACCGGGGAGAATGTGGTGGTGGGGGCAGCGGGGGGCGGAGGTAGTGGAGGCGGCGGTAGTGAACCAAAGAGTAGTGACAAAACTCACACGTGCCCACCGTGCCCAGCACCTGAACTCCTGGGGGGACCGTCAGTCTTCCTCTTCCCCCCAAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCC | 464 |

| | | |
|---|---|---|
| | CCATGCCGGGAGGAGATGACCAAGAACCAGGTCAGCCTGTGGTGCCTGGTCAAAGGCTTCT ATCCCAGCGACATCGCCGTGGAGTGGGAGAGCAATGGGCAGCCGGAGAACAACTACAAGAC CACGCCTCCCGTGCTGGACTCCGACGGCTCCTTCTTCCTCTACAGCAAGCTCACCGTGGAC AAGAGCAGGTGGCAGCAGGGGAACGTCTTCTCATGCTCCGTGATGCATGAGGCTCTGCACA ACAGATTTACGCAGAAGAGCCTCTCCCTGTCTCCGGGTAAA | |
| TraH-G44C-Knob | GAAGTTCAACTTGTGGAGTCTGGAGGCGGTTTGGTACAACCTGGAGGTTCACTTCGTTTGT CATGTGCTGCCTCTGGGTTTAATATCAAGGATACTTATATTCATTGGGTTCGCCAGGCACC CGGAAAATGCCTGGAATGGGTGGCCCGTATATATCCCACTAACGGTTACACTCGATATGCT GACTCTGTAAAGGGTCGATTTACTATATCCGCTGATACTAGCAAAAACACTGCCTACCTCC AGATGAACAGTCTGCGCGCCGAGGATACAGCAGTTTATTATTGTTCCCGATGGGGGAGGTGA CGGGTTCTATGCCATGGACTACTGGGGTCAAGGGACACTGGTAACCGTTTCTTCTGCTAGC ACCAAAGGACCTAGTGTTTTTCCTCTTGCCCCTTCCTCAAAGTCTACCTCTGGGGGGACAG CCGCTCTGGGCTGCCTGGTCAAGGATTATTTCCCAGAGCCTGTCACTGTCAGTTGGAACTC TGGAGCCTTGACTTCTGGTGTTCATACATTTCCTGCTGTCCTTCAAAGCAGCGGCTTGTAC TCATTGTCTTCTGTTGTGACAGTACCCTCAAGCAGCCTCGGCACTCAGACATACATCTGCA ATGTCAACCACAAACCCTCAAATACAAAGGTAGATAAAAAAGTCGAACCAAAGTCTTGTGA CAAAACTCACACGTGCCCACCGTGCCCAGCACCTGAACTCCTGGGGGGGACCGTCAGTCTTC CTCTTCCCCCCAAAACCCAAGGACACCCTCATGATCTCCCGGACCCCTGAGGTCACATGCG TGGTGGTGGACGTGAGCCACGAAGACCCTGAGGTCAAGTTCAACTGGTACGTGGACGGCGT GGAGGTGCATAATGCCAAGACAAAGCCGCGGGAGGAGCAGTACAACAGCACGTACCGTGTG GTCAGCGTCCTCACCGTCCTGCACCAGGACTGGCTGAATGGCAAGGAGTACAAGTGCAAGG TCTCCAACAAAGCCCTCCCAGCCCCCATCGAGAAAACCATCTCCAAAGCCAAAGGGCAGCC CCGAGAACCACAGGTGTACACCCTGCCCCCCATGCCGGGAGGAGATGACCAAGAACCAGGTC AGCCTGTGGTGCCTGGTCAAAGGCTTCTATCCCAGCGACATCGCCGTGGAGTGGGAGAGCA ATGGGCAGCCGGAGAACAACTACAAGACCACGCCTCCCGTGCTGGACTCCGACGGCTCCTT CTTCCTCTACAGCAAGCTCACCGTGGACAAGAGCAGGTGGCAGCAGGGGAACGTCTTCTCA TGCTCCGTGATGCATGAGGCTCTGCACAACCACTACACGCAGAAGAGCCTCTCCCTGTCTC CGGGTAAA | 465 |
| TraL-Q100C- | GACATTCAGATGACCCAATCCCCCTCTAGCCTTTCAGCCTCCGTAGGGGACAGAGTTACAA | 466 |

| Knob | TAACCTGTAGGGCTAGTCAGGACGTTAATACAGCAGTCGCATGGTACCAGCAGAAGCCCGG TAAGGCTCCTAAGCTTTTGATCTACAGTGCTTCTTTCCTGTATTCTGGTGTACCTAGCCGA TTCTCAGGCTCTCGGAGTGGCACAGACTTCACTCTGACAATTTCAAGTCTCCAGCCAGAAG ATTTTGCAACCTACTACTGCCAACAACACTACACTACTCCTCCAACCTTCGGATGTGGCAC AAAAGTAGAGATTAAGCGTACGGTGGCAGCTCCCAGCGTTTTTATCTTTCCCCCATCCGAC GAGCAGCTCAAGAGTGGCACTGCCTCTGTAGTTTGTTTGCTGAATAACTTCTATCCACGTG AAGCAAAAGTACAGTGGAAGGTCGATAATGCCCTTCAGAGCGGTAACAGCCAAGAAAGTGT TACCGAGCAAGATTCCAAAGATTCCACTTACAGTCTGTCCAGCACATTGACACTGAGTAAG GCTGATTACGAAAAACACAAGGTGTACGCATGCGAGGTGACACACCAAGGTCTTTCATCTC CTGTAACTAAGAGCTTTAACCGGGGAGAATGTGGTGGTGGGGGCAGCGGGGGCGGAGGTAG TGGAGGCGGCGGTAGTGAACCAAAGAGTAGTGACAAAACTCACACGTGCCCACCGTGCCCA GCACCTGAACTCCTGGGGGGACCGTCAGTCTTCCTCTTCCCCCCAAAACCCAAGGACACCC TCATGATCTCCCGGACCCCTGAGGTCACATGCGTGGTGGTGGACGTGAGCCACGAAGACCC TGAGGTCAAGTTCAACTGGTACGTGGACGGCGTGGAGGTGCATAATGCCAAGACAAAGCCG CGGGAGGAGCAGTACAACAGCACGTACCGTGTGGTCAGCGTCCTCACCGTCCTGCACCAGG ACTGGCTGAATGGCAAGGAGTACAAGTGCAAGGTCTCCAACAAAGCCCTCCCAGCCCCCAT CGAGAAAACCATCTCCAAAGCCAAAGGGCAGCCCCGAGAACCACAGGTGTACACCCTGCCC CCATGCCGGGAGGAGATGACCAAGAACCAGGTCAGCCTGTGGTGCCTGGTCAAAGGCTTCT ATCCCAGCGACATCGCCGTGGAGTGGGAGAGCAATGGGCAGCCGGAGAACAACTACAAGAC CACGCCTCCCGTGCTGGACTCCGACGGCTCCTTCTTCCTCTACAGCAAGCTCACCGTGGAC AAGAGCAGGTGGCAGCAGGGGAACGTCTTCTCATGCTCCGTGATGCATGAGGCTCTGCACA ACCACTACACGCAGAAGAGCCTCTCCCTGTCTCCGGGTAAA | |
| TraL-Q100C-Knob1 | GACATTCAGATGACCCAATCCCCCTCTAGCCTTTCAGCCTCCGTAGGGGACAGAGTTACAA TAACCTGTAGGGCTAGTCAGGACGTTAATACAGCAGTCGCATGGTACCAGCAGAAGCCCGG TAAGGCTCCTAAGCTTTTGATCTACAGTGCTTCTTTCCTGTATTCTGGTGTACCTAGCCGA TTCTCAGGCTCTCGGAGTGGCACAGACTTCACTCTGACAATTTCAAGTCTCCAGCCAGAAG ATTTTGCAACCTACTACTGCCAACAACACTACACTACTCCTCCAACCTTCGGATGTGGCAC AAAAGTAGAGATTAAGCGTACGGTGGCAGCTCCCAGCGTTTTTATCTTTCCCCCATCCGAC GAGCAGCTCAAGAGTGGCACTGCCTCTGTAGTTTGTTTGCTGAATAACTTCTATCCACGTG | 467 |

| | | |
|---|---|---|
| | AAGCAAAAGTACAGTGGAAGGTCGATAATGCCCTTCAGAGCGGTAACAGCCAAGAAAGTGT TACCGAGCAAGATTCCAAAGATTCCACTTACAGTCTGTCCAGCACATTGACACTGAGTAAG GCTGATTACGAAAAACACAAGGTGTACGCATGCGAGGTGACACACCAAGGTCTTTCATCTC CTGTAACTAAGAGCTTTAACCGGGGAGAATGTGGTGGTGGGGGCAGCGGGGGCGGAGGTAG TGAACCAAAGAGTAGTGACAAAACTCACACGTGCCCACCGTGCCCAGCACCTGAACTCCTG GGGGGACCGTCAGTCTTCCTCTTCCCCCCAAAACCCAAGGACACCCTCATGATCTCCCGGA CCCCTGAGGTCACATGCGTGGTGGTGGACGTGAGCCACGAAGACCCTGAGGTCAAGTTCAA CTGGTACGTGGACGGCGTGGAGGTGCATAATGCCAAGACAAAGCCGCGGGAGGAGCAGTAC AACAGCACGTACCGTGTGGTCAGCGTCCTCACCGTCCTGCACCAGGACTGGCTGAATGGCA AGGAGTACAAGTGCAAGGTCTCCAACAAAGCCCTCCCAGCCCCCATCGAGAAAACCATCTC CAAAGCCAAAGGGCAGCCCCGAGAACCACAGGTGTACACCCTGCCCCCATGCCGGGAGGAG ATGACCAAGAACCAGGTCAGCCTGTGGTGCCTGGTCAAAGGCTTCTATCCCAGCGACATCG CCGTGGAGTGGGAGAGCAATGGGCAGCCGGAGAACAACTACAAGACCACGCCTCCCGTGCT GGACTCCGACGGCTCCTTCTTCCTCTACAGCAAGCTCACCGTGGACAAGAGCAGGTGGCAG CAGGGGAACGTCTTCTCATGCTCCGTGATGCATGAGGCTCTGCACAACCACTACACGCAGA AGAGCCTCTCCCTGTCTCCGGGTAAA | |
| TraL-Q100C-Knob2 | GACATTCAGATGACCCAATCCCCCTCTAGCCTTTCAGCCTCCGTAGGGGACAGAGTTACAA TAACCTGTAGGGCTAGTCAGGACGTTAATACAGCAGTCGCATGGTACCAGCAGAAGCCCGG TAAGGCTCCTAAGCTTTTGATCTACAGTGCTTCTTTCCTGTATTCTGGTGTACCTAGCCGA TTCTCAGGCTCTCGGAGTGGCACAGACTTCACTCTGACAATTTCAAGTCTCCAGCCAGAAG ATTTTGCAACCTACTACTGCCAACAACACTACACTACTCCTCCAACCTTCGGATGTGGCAC AAAAGTAGAGATTAAGCGTACGGTGGCAGCTCCCAGCGTTTTTATCTTTCCCCCATCCGAC GAGCAGCTCAAGAGTGGCACTGCCTCTGTAGTTTGTTTGCTGAATAACTTCTATCCACGTG AAGCAAAAGTACAGTGGAAGGTCGATAATGCCCTTCAGAGCGGTAACAGCCAAGAAAGTGT TACCGAGCAAGATTCCAAAGATTCCACTTACAGTCTGTCCAGCACATTGACACTGAGTAAG GCTGATTACGAAAAACACAAGGTGTACGCATGCGAGGTGACACACCAAGGTCTTTCATCTC CTGTAACTAAGAGCTTTAACCGGGGAGAATGTGGTGGTGGGGGCAGCGAACCAAAGAGTAG TGACAAAACTCACACGTGCCCACCGTGCCCAGCACCTGAACTCCTGGGGGGGACCGTCAGTC TTCCTCTTCCCCCCAAAACCCAAGGACACCCTCATGATCTCCCGGACCCCTGAGGTCACAT | 468 |

| | | |
|---|---|---|
| | GCGTGGTGGTGGACGTGAGCCACGAAGACCCTGAGGTCAAGTTCAACTGGTACGTGGACGG CGTGGAGGTGCATAATGCCAAGACAAAGCCGCGGGAGGAGCAGTACAACAGCACGTACCGT GTGGTCAGCGTCCTCACCGTCCTGCACCAGGACTGGCTGAATGGCAAGGAGTACAAGTGCA AGGTCTCCAACAAAGCCCTCCCAGCCCCCATCGAGAAAACCATCTCCAAAGCCAAAGGGCA GCCCCGAGAACCACAGGTGTACACCCTGCCCCCATGCCGGGAGGAGATGACCAAGAACCAG GTCAGCCTGTGGTGCCTGGTCAAAGGCTTCTATCCCAGCGACATCGCCGTGGAGTGGGAGA GCAATGGGCAGCCGGAGAACAACTACAAGACCACGCCTCCCGTGCTGGACTCCGACGGCTC CTTCTTCCTCTACAGCAAGCTCACCGTGGACAAGAGCAGGTGGCAGCAGGGGAACGTCTTC TCATGCTCCGTGATGCATGAGGCTCTGCACAACCACTACACGCAGAAGAGCCTCTCCCTGT CTCCGGGTAAA | |
| TraL-Q100C-Knob3 | GACATTCAGATGACCCAATCCCCCTCTAGCCTTTCAGCCTCCGTAGGGGACAGAGTTACAA TAACCTGTAGGGCTAGTCAGGACGTTAATACAGCAGTCGCATGGTACCAGCAGAAGCCCGG TAAGGCTCCTAAGCTTTTGATCTACAGTGCTTCTTTCCTGTATTCTGGTGTACCTAGCCGA TTCTCAGGCTCTCGGAGTGGCACAGACTTCACTCTGACAATTTCAAGTCTCCAGCCAGAAG ATTTTGCAACCTACTACTGCCAACAACACTACACTACTCCTCCAACCTTCGGATGTGGCAC AAAAGTAGAGATTAAGCGTACGGTGGCAGCTCCCAGCGTTTTTATCTTTCCCCCATCCGAC GAGCAGCTCAAGAGTGGCACTGCCTCTGTAGTTTGTTTGCTGAATAACTTCTATCCACGTG AAGCAAAAGTACAGTGGAAGGTCGATAATGCCCTTCAGAGCGGTAACAGCCAAGAAAGTGT TACCGAGCAAGATTCCAAAGATTCCACTTACAGTCTGTCCAGCACATTGACACTGAGTAAG GCTGATTACGAAAAACACAAGGTGTACGCATGCGAGGTGACACACCAAGGTCTTTCATCTC CTGTAACTAAGAGCTTTAACCGGGGAGAATGTGAACCAAAGAGTAGTGACAAAACTCACAC GTGCCCACCGTGCCCAGCACCTGAACTCCTGGGGGGACCGTCAGTCTTCCTCTTCCCCCCA AAACCCAAGGACACCCTCATGATCTCCCGGACCCCTGAGGTCACATGCGTGGTGGTGGACG TGAGCCACGAAGACCCTGAGGTCAAGTTCAACTGGTACGTGGACGGCGTGGAGGTGCATAA TGCCAAGACAAAGCCGCGGGAGGAGCAGTACAACAGCACGTACCGTGTGGTCAGCGTCCTC ACCGTCCTGCACCAGGACTGGCTGAATGGCAAGGAGTACAAGTGCAAGGTCTCCAACAAAG CCCTCCCAGCCCCCATCGAGAAAACCATCTCCAAAGCCAAAGGGCAGCCCCGAGAACCACA GGTGTACACCCTGCCCCCATGCCGGGAGGAGATGACCAAGAACCAGGTCAGCCTGTGGTGC CTGGTCAAAGGCTTCTATCCCAGCGACATCGCCGTGGAGTGGGAGAGCAATGGGCAGCCGG | 469 |
| | AGAACAACTACAAGACCACGCCTCCCGTGCTGGACTCCGACGGCTCCTTCTTCCTCTACAG CAAGCTCACCGTGGACAAGAGCAGGTGGCAGCAGGGGAACGTCTTCTCATGCTCCGTGATG CATGAGGCTCTGCACAACCACTACACGCAGAAGAGCCTCTCCCTGTCTCCGGGTAAA | |

Table 9 below shows the polypeptide sequences of the heavy chain variable region (VH) and light chain variable region (VL) of H01. Table 10 below shows the polynucleotide sequences encoding the polypeptides of the heavy chain variable region (VH) and light chain variable region (VL) of H01.

[Table 9]

| Name | Sequence | SEQ ID NO |
|---|---|---|
| H01 VH | EVQLVESGGGLVQPGGSLRLSCAASGFNIKDTY IHWVRQAPGKCLEWVARIYPTNGYTRYADS VKGRFTISADTSKNTAYLQMNSLRAEDTAVYYCSRWGGDGFYAMDYWGQGTLVTVSS | 19 |
| H01 VL | DIQMTQSPSSLSASVGDRVTITCRASQDVNTAVAWYQQKPGKAPKLLIYSASFLYSGVPSRFS GSRSGTDFTLTISSLQPEDFATYYCQQHYTTPPTFGCGTKVEIK | 20 |

[Table 10]

| Name | Sequence | SEQ ID NO |
|---|---|---|
| H01 VH | GAAGTTCAACTTGTGGAGTCTGGAGGCGGTTTGGTACAACCTGGAGGTTCACTTCGTTTGTCA TGTGCTGCCTCTGGGTTTAATATCAAGGATACTTATATTCATTGGGTTCGCCAGGCACCCGGA AAATGCCTGGAATGGGTGGCCCGTATATATCCCACTAACGGTTACACTCGATATGCTGACTCT GTAAAGGGTCGATTTACTATATCCGCTGATACTAGCAAAAACACTGCCTACCTCCAGATGAAC AGTCTGCGCGCCGAGGATACAGCAGTTTATTATTGTTCCCGATGGGGAGGTGACGGTTCTAT GCCATGGACTACTGGGGTCAAGGGACACTGGTAACCGTTTCTTCT | 170 |
| H01 VL | GACATTCAGATGACCCAATCCCCCTCTAGCCTTTCAGCCTCCGTAGGGGACAGAGTTACAATA ACCTGTAGGGCTAGTCAGGACGTTAATACAGCAGTCGCATGGTACCAGCAGAAGCCCCGGTAAG GCTCCTAAGCTTTTTGATCTACAGTGCTTCTTTCCTGTATTCTGGTGTACCTAGCCGATTCTCA GGCTCTCGGAGTGGCACAGACTTCACTCTGACAATTTCAAGTCTCCAGCCAGAAGATTTTGCA ACCTACTACTGCCAACAACACTACACTACTCCTCCAACCTTCGGATGTGGCACAAAAGTAGAG ATTAAG | 171 |

Table 11 below shows the CDR sequences of the heavy chain and light chain of H01.

[Table 11]

| Name | CDR region | Sequence | SEQ ID NO |
|---|---|---|---|
| H01 VH | CDR-H1 | DTYIH | 21 |
| | CDR-H2 | RIYPTNGYTRYADSVKG | 22 |
| | CDR-H3 | WGGDGFYAMDY | 23 |
| H01 VL | CDR-L1 | RASQDVNTAVA | 24 |
| | CDR-L2 | SASFLYS | 25 |
| | CDR-L3 | QQHYTTPPT | 26 |

## Example 2. Preparation of a novel antibody with two Fc domains using pertuzumab as a template

M3 is characterized by a (trastuzumab Fab)-(Fc)$_2$ structure with mutations of VH G44C and VL Q100C, hereinafter referred to as "H01". Similarly, based on the VH and VL regions of pertuzumab (SEQ ID NOs: 27 and 28), the (pertuzumab Fab)-(Fc)$_2$ structure with the mutations VH G44C and VL Q100C is hereinafter referred to as "P01".

P01 consists of the polypeptides of Fc-Hole (SEQ ID NO: 7), PerH-G44C-Knob (SEQ ID NO: 29), and PerL-Q 100C-Knob (SEQ ID NO: 30), and was prepared by co-transfection of expression vectors loaded with polynucleotides encoding the polypeptides into the EXPICHO-S™ cell line. The protein prepared as above was purified and analyzed as a fusion protein in the same manner as in Example 1 above.

[Table 12]

| Name | Sequence | SEQ ID NO |
|---|---|---|
| Pertuzumab VH domain | EVQLVESGGGLVQPGGSLRLSCAASGFTFTDYTMDWVRQAPGKGLEWVADVNPNSGGSIYNQRFKGRFTLSVDRSKNTLYLQMNSLRAEDTAVYYCARNLGPSFYFDYWGQGTLVTVSS | 27 |
| Pertuzumab VL domain | DIQMTQSPSSLSASVGDRVTITCKASQDVSIGVAWYQQKPGKAPKLLIYSASYRYTGVPSRFSGSGSGTDFTLTISSLQPEDFATYYCQQYYIYPYTFGQGTKVEIK | 28 |
| PerH-G14C-Knob | EVQLVESGGGLVQPGGSLRLSCAASGFTFTDYTMDWVRQAPGKGLEWVADVNPNSGGSIYNQRFKGRFTLSVDRSKNTLYLQMNSLRAEDTAVYYCARNLGPSFYFDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVTGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 29 |
| PerL-Q100C-Knob | DIQMTQSPSSLSASVGDRVTITCKASQDVSIGVAWYQQKPGKAPKLLIYSASYRYTGVPSRFSGSGSGTDFTLTISSLQPEDFATYYCQQYYIYPYTFGCGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGECGGGGSGGGGSGGGGSEPKSSDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 30 |

[Table 13]

| Name | | Sequence | SEQ ID NO |
|------|---|----------|-----------|
| Pertuzumab domain | VH | GAAGTGCAACTGGTGGAGTCTGGTGGTGGATTGGTGCAGCCAGGCGGTTCTCTGCGACTTAGT TGTGCAGCCTCCGGCTTTACCTTCACTGATTATACAATGGACTGGGTTCGGCAGGCACCCGGT AAGGGGCTTGAGTGGGTCGCCGACGTCAATCCTAATTCAGGGGGAAGTATTTATAACCAAAGG TTCAAGGGTCGATTTACATTGTCCGTAGATCGTAGTAAAAATACCCTCTACCTTCAAATGAAC TCCCTGAGGGCAGAGGATACCGCAGTCTACTACTGCGCTCGTAACCTGGGGCCTAGTTTTTAT TTCGATTATTGGGGCCAAGGCACATTGGTAACTGTGTCTTCA | 414 |
| Pertuzumab domain | VL | GATATACAAATGACACAATCTCCTAGTTCATTGAGTGCCTCAGTCGGCGACCGAGTCACTATA ACTTGTAAGGCAAGCCAAGATGTTAGCATTGGCGTAGCTTGGTATCAGCAGAAACCTGGAAAA GCACCAAAACTGCTTATCTACTCCGCTAGTTACCGTTACACCGGAGTTCCCTCAAGGTTTTCT GGCAGCGGAAGTGGGACTGACTTCACTCTGACTATTTCTTCACTTCAGCCAGAAGACTTCGCT ACTTATTACTGTCAGCAGTACTATATCTATCCCTATACATTTGGACAAGGAACCAAAGTTGAG ATTAAA | 415 |
| PerH-G44C-Knob | | GAAGTACAGTTGGTGGAGTCTGGTGGAGGTCTTGTACAACCTGGCGGGAGTTTGCGGCTTTCC TGCGCTGCAAGCGGGTTTACCTTCACCGATTATACCATGGATTGGGTACGCCAAGCCCCTGGT AAGTGCCTTGAGTGGGTTGCCGATGTAAACCCTAATTCCGGGAGGAAGTATCTATAATCAACGC TTCAAGGGCCGATTCACTCTGAGTGTGGATCGAAGCAAGAACACCTTGTACTTGCAGATGAAT TCCTTGCGGGCTGAAGACACAGCCGTCTATTACTGCGCCCGAAATTTGGGGCCTTCATTCTAT TTTGACTATTGGGGTCAGGGAACTCTGGTAACTGTTTCAAGTGCTAGCACCAAAGGACCTAGT GTTTTTCCTCTTGCCCCTTCCTCAAAGTCTACCTCTGGGGGGACAGCCGCTCTGGGCTGCCTG GTCAAGGATTATTTCCCAGAGCCTGTCACTGTCAGTTGGAACTCTGGAGCCTTGACTTCTGGT GTTCATACATTTCCTGCTGTCCTTCAAAGCAGCGGCTTGTACTCATTGTCTTCTGTTGTGACA GTACCCTCAAGCAGCCTCGGCACTCAGACATACATCTGCAATGTCAACCACAAACCCTCAAAT ACAAAGGTAGATAAAAAAGTCGAACCAAAGTCTTGTGACAAAACTCACACGTGCCCACCGTGC CCAGCACCTGAACTCCTGGGGGGACCGTCAGTCTTCCTCTTCCCCCCAAAACCCAAGGACACC CTCATGATCTCCCGGACCCCTGAGGTCACATGCGTGGTGGTGGACGTGAGCCACGAAGACCCT GAGGTCAAGTTCAACTGGTACGTGGACGGCGTGGAGGTGCATAATGCCAAGACAAAGCCGCGG GAGGAGCAGTACAACAGCACGTACCGTGTGGTCAGCGTCCTCACCGTCCTGCACCAGGACTGG CTGAATGGCAAGGAGTACAAGTGCAAGGTCTCCAACAAAGCCCTCCCAGCCCCCATCGAGAAA ACCATCTCCAAAGCCAAAGGGCAGCCCCGAGAACCACAGGTGTACACCCTGCCCCCATCCCGG GAGGAGATGACCAAGAACCAGGTCAGCCTGTGGTGCCTGGTCAAAGGCTTCTATCCCAGCGAC ATCGCCGTGGAGTGGGAGAGCAATGGGCAGCCGGAGAACAACTACAAGACCACGCCTCCCGTG CTGGACTCCGACGGCTCCTTCTTCCTCTACAGCAAGCTCACCGTGGACAAGAGCAGGTGGCAG CAGGGGAACGTCTTCTCATGCTCCGTGATGCATGAGGCTCTGCACAACCACTACACGCAGAAG AGCCTCTCCCTGTCTCCGGGTAAA | 472 |
| PerL-Q100C-Knob | | GATATTCAAATGACCCAAAGTCCAAGTTCCCTTTCAGCATCTGTCGGTGATCGAGTCACTATA ACTTGTAAGGCCAGCCAAGACGTTAGCATAGGAGTAGCATGGTACCAACAAAAGCCTGGGAAG GCTCCCAAACTTCTCATTTATTCTGCTTCCTACCGATATACTGGTGTCCCAAGTAGATTTTCT GGCAGCGGATCTGGAACTGATTTTACATTGACTATCAGCTCCCTCCAGCCTGAGGACTTCGCT ACTTATTACTGCCAACAGTACTACATTTATCCCTATACATTCGGTTGTGGGACCAAAGTAGAG ATCAAACGTACGGTGGCAGCTCCCAGCCGTTTTTATCTTTCCCCCATCCGACGAGCAGCTCAAG AGTGGCACTGCCTCTGTAGTTTTGTTTGCTGAATAACTTCTATCCACGTGAAGCAAAAGTACAG TGGAAGGTCGATAATGCCCTTCAGAGCGGTAACAGCCAAGAAAGTGTTACCGAGCAAGATTCC AAAGATTCCACTTACAGTCTGTCCAGCACATTGACACTGAGTAAGGCTGATTACGAAAAACAC AAGGTGTACGCATGCGAGGTGACACACCAAGGTCTTTCATCTCCTGTAACTAAGAGCTTTAAC CGGGGAGAATGTCGGTGGTGGGGGCAGCGGGGGCGGAGGTAGTGGAGGCGGCGGTAGTGAACCA AAGAGTAGTGACAAAACTCACACCGTGCCCACCGTGCCCAGCACCTGAACTCCTGGGGGGACCG TCAGTCTTCCTCTTCCCCCCAAAACCCAAGGACACCCTCATGATCTCCCGGACCCCTGAGGTC | 473 |

| | |
|---|---|
| ACATGCGTGGTGGTGGACGTGAGCCACGAAGACCCTGAGGTCAAGTTCAACTGGTACGTGGAC GGCGTGGAGGTGCATAATGCCAAGACAAAGCCGCGGGAGGAGCAGTACAACAGCACGTACCGT GTGGTCAGCGTCCTCACCGTCCTGCACCAGGACTGGCTGAATGGCAAGGAGTACAAGTGCAAG GTCTCCAACAAAGCCCTCCCAGCCCCCATCGAGAAAACCATCTCCAAAGCCAAAGGGCAGCCC CGAGAACCACAGGTGTACACCCTGCCCCCATGCCGGGAGGAGATGACCAAGAACCAGGTCAGC CTGTGGTGCCTGGTCAAAGGCTTCTATCCCAGCGACATCGCCGTGGAGTGGGGAGAGCAATGGG CAGCCGGAGAACAACTACAAGACCACGCCTCCCGTGCTGGACTCCGACGGCTCCTTCTTCCTC TACAGCAAGCTCACCGTGGACAAGAGCAGGTGGCAGCAGGGGAACGTCTTCTCATGCTCCGTG ATGCATGAGGCTCTGCACAACCACTACACGCAGAAGAGCCTCTCCCTGTCTCCGGGTAAA | |

Table 14 below shows the polypeptide sequences of the heavy chain variable region (VH) and light chain variable region (VL) of P01. Table 15 below shows the polynucleotide sequences encoding the polypeptides of the heavy chain variable region (VH) and light chain variable region (VL) of P01.

[Table 14]

| Name | Sequence | SEQ ID NO |
|---|---|---|
| P01 VH | EVQLVESGGGLVQPGGSLRLSCAASGFTFTDYTMDWVRQAPGKCLEWVADVNPNSGGSIYNQRF KGRFTLSVDRSKNTLYLQMNSLRAEDTAVYYCARNLGPSFYFDYWGQGTLVTVSS | 31 |
| P01 VL | DIQMTQSPSSLSASVGDRVTITCKASQDVSIGVAWYQQKPGKAPKLLIYSASYRYTGVPSRFSG SGSGTDFTLTISSLQPEDFATYYCQQYYIYPYTFGQGTKVEIK | 32 |

[Table 15]

| Name | Sequence | SEQ ID NO |
|---|---|---|
| P01 VH | GAAGTACAGTTGGTGGAGTCTGGTGGAGGTCTTGTACAACCTGGCGGGAGTTTGCGGCTTTCCT TCGCGCTGCAAGCGGGTTTAACCTTCACCGATTATACCATGGATTGGGTACGCCAAGCCCCTGGTAA GTGCCTTGAGTGGGTTGCCGATGTAAACCCTAATTCCGGAGGAAGTATCTATAATCAACGCTTC AAGGGCCGATTCACTCTGAGTGTGGATCGAAGCAAGAACACCTTGTACTTGCAGATGAATTCCT TGCGGGCTGAAGACACAGCCGTCTATTACTGCGCCCGAAATTTGGGGCCTTCATTCTATTTTGA CTATTGGGGTCAGGGAACTCTGGTAACTGTTTCAAGT | 174 |
| P01 VL | GATATTCAAATGACCCAAAGTCCAAGTTCCCTTTCAGCATCTGTCGGTGATCGAGTCACTATAA CTTGTAAGGCCAGCCAAGACGTTAGCATAGGAGTAGCATGGTACCAACAAAAGCCTGGGAAGGC TCCCAAACTTCTCATTTATTCTGCTTCCTACCGATATACTGGTGTCCCAAGTAGATTTTCTGGC AGCGGATCTGGAACTGATTTTACATTGACTATCAGCTCCCTCCAGCCTGAGGACTTCGCTACTT ATTACTGCCAACAGTACTACATTTATCCCTATACATTCGGTTGTGGGACCAAGTAGAGATCAA A | 175 |

Table 16 below shows the CDR sequences of the heavy chain and light chain of P01.

[Table 16]

| Name | | Sequence | SEQ ID NO |
|---|---|---|---|
| P01 VH | CDR-H1 | DYTMD | 33 |
| | CDR-H2 | DVNPNSGGSIYNQRFKG | 34 |
| | CDR-H3 | NLGPSFYFDY | 35 |
| P01 VL | CDR-L1 | KASQDVSIGVA | 36 |
| | CDR-L2 | SASYRYT | 37 |
| | CDR-L3 | QQYYIYPYT | 38 |

**Example 3. Analysis of H01 and P01 byproducts through papain digestion**

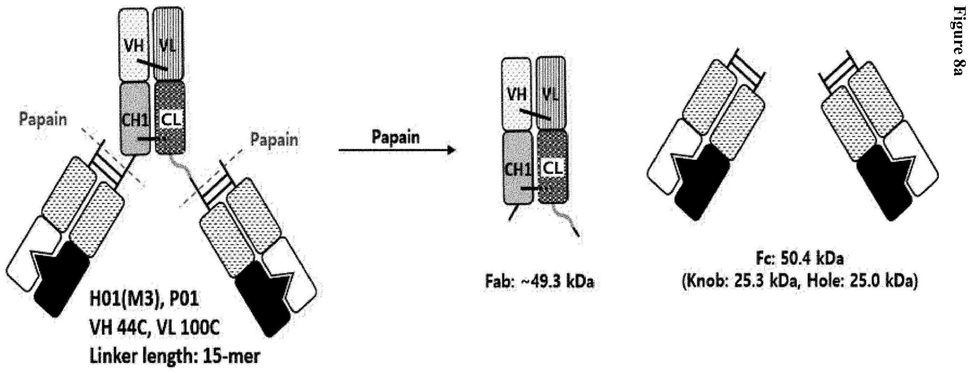

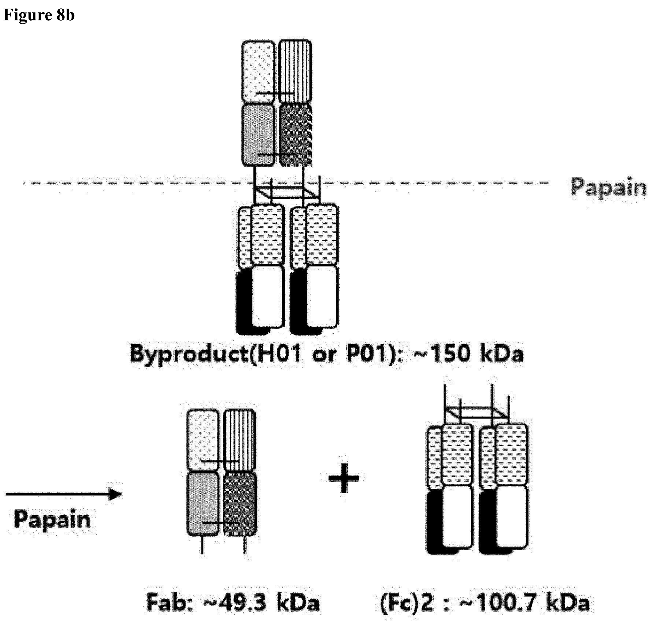

Papain recognizes specific sequences in the hinge region and induces antibody digestion. In the case of the Fab-(Fc)₂ structure, when papain digestion is performed, it is cleaved into a Fab portion of approximately 49.3 kDa and two Fc domains of approximately 50.4 kDa (Figure 8a). However, if abnormal disulfide bonds are formed in the hinge region, unwanted inter-chain disulfide bond byproducts could be observed. In this case, a Fab fragment of approximately 49.3 kDa and an abnormal (Fc)₂ product of approximately 100.7 kDa could be observed (Figure 8b).

In order to verify this, papain digestion of H01 and P01 was performed. Papain (Sigma, P3125) was used by diluting it to 0.1 mg/mL in digestion buffer (20 mM EDTA + 10 mM Cys-HCl in PBS pH 7.4). 200 μg of H01 or P01 was mixed with the Papain and reacted at 37 °C for 2 hours, and then SDS-PAGE was performed.

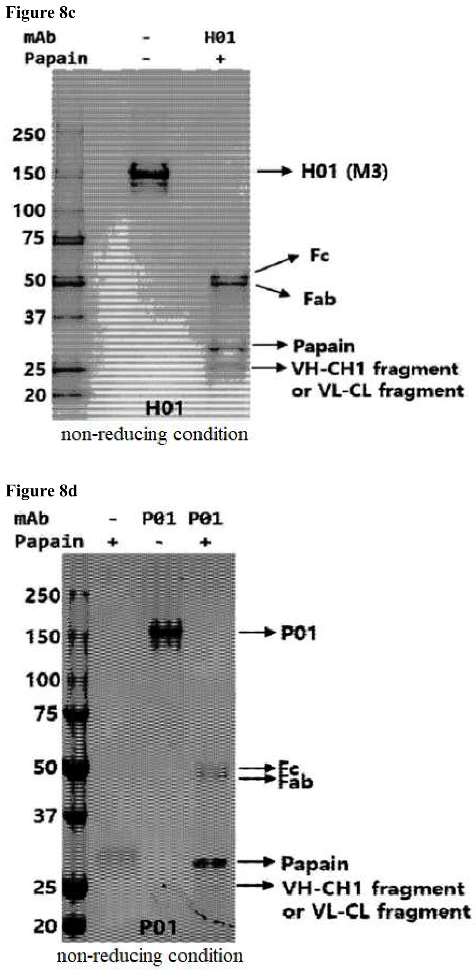

As a result of SDS-PAGE performed under non-reducing conditions, as shown in Figures 8c and 8d, the abnormal (Fc)₂ was not identified.

## Example 4. Analysis of physical properties of H01wt

In H01, four Fc monomers are assembled into two Fc dimers due to a knob-into-hole mutations, resulting in a structure as shown in Figure 6a.

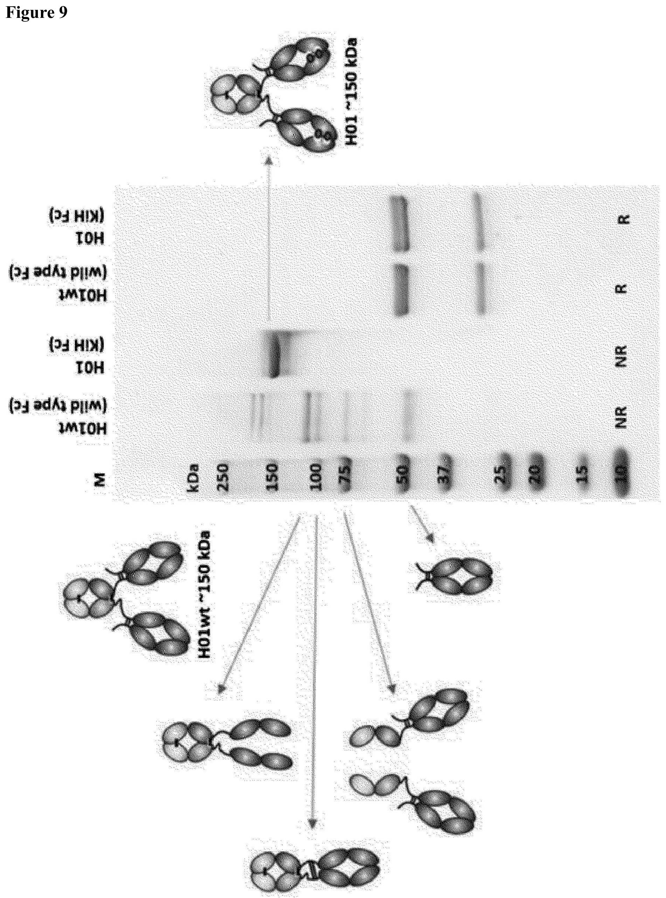

In order to analyze the effect of the knob-into-hole mutations on the formation of H01 structure, the Fc hole monomer polypeptide (SEQ ID NO: 7) was substituted with a polypeptide (SEQ ID NO: 39) corresponding to the wild type IgG1 Fc monomer (Table 17). The two knob polypeptides (SEQ ID NOs: 14 and 15) constituting H01 were also substituted with polypeptides (SEQ ID NOs: 40 and 41) corresponding to the wild type IgG1 Fc monomer (Table 17). This novel antibody format consisting of two wtFc polypeptides (SEQ ID NO: 39), one TraH-G44C-wtFc polypeptide (SEQ ID NO: 40), and one TraL-Q100C-wtFc polypeptide (SEQ ID NO: 41) is referred to as H01wt (Figure 9).

H01wt consists of the polypeptides of wtFc(SEQ ID NO: 39), TraH-G44C-wtFc(SEQ ID NO: 40), and TraL-Q100C-wtFc(SEQ ID NO: 41), and was prepared by co-transfection of expression vectors loaded with polynucleotides encoding the polypeptides into the ExpiCHO-S™ cell line. The protein prepared as above was purified and analyzed in the same manner as in Example 1 above.

As shown in Figure 9, SDS-PAGE analysis under non-reducing conditions identified a small amount of H01wt at about 150 kDa, and most of H01wt were expressed as abnormally structured byproducts. On the other hand, in the case of H01, which has knob-into-hole mutations in the Fc domain, each polypeptide was efficiently assembled into a product that is identified at about 150 kDa.

[Table 17]

| Name | Sequence | SEQ ID NO |
|---|---|---|
| wtFc | DKTHTCPPCPAPELLGGPSVFLFPPKPKDTLMISRTPEVTCVVVDVSHEDPEVKFNWYVDGVEV HNAKTKPREEQYNSTYRVVSVLTVLHQDWLNGKEYKCKVSNKALPAPIEKTISKAKGQPREPQV YTLPPSREEMTKNQVSLTCLVKGFYPSDIAVEWESNGQPENNYKTTPPVLDSDGSFFLYSKLTV DKSRWQQGNVFSCSVMHEALHNHYTQKSLSLSPGK | 39 |
| TraH-G44C-wtFc | EVQLVESGGGLVQPGGSLRLSCAASGFNIKDTYIHWVRQAPGKCLEWVARIYPTNGYTRYADSV KGRFTISADTSKNTAYLQMNSLRAEDTAVYYCSRWGGDGFYAMDYWGQGTLVTVSSASTKGPSV FPLAPSSKSTSGGTAALGCLVKDYFPEPVTVSWNSGALTSGVHTFPAVLQSSGLYSLSSVVTVP SSSLGTQTYICNVNHKPSNTKVDKKVEPKSCDKTHTCPPCPAPELLGGPSVFLFPPKPKDTLMI SRTPEVTCVVVDVSHEDPEVKFNWYVDGVEVHNAKTKPREEQYNSTYRVVSVLTVLHQDWLNGK EYKCKVSNKALPAPIEKTISKAKGQPREPQVYTLPPSREEMTKNQVSLTCLVKGFYPSDIAVEW ESNGQPENNYKTTPPVLDSDGSFFLYSKLTVDKSRWQQGNVFSCSVMHEALHNHYTQKSLSLSP GK | 40 |
| TraL-Q100C-wtFc | DIQMTQSPSSLSASVGDRVTITCRASQDVNTAVAWYQQKPGKAPKLLIYSASFLYSGVPSRFSG SRSGTDFTLTISSLQPEDFATYYCQQHYTTPPTFGCGTKVEIKRTVAAPSVFIFPPSDEQLKSG TASVVCLLNNFYPREAKVQWKVDNALQSGNSQESVTEQDSKDSTYSLSSTLTLSKADYEKHKVY ACEVTHQGLSSPVTKSFNRGECGGGGSGGGGSGGGGSEPKSSDKTHTCPPCPAPELLGGPSVFL FPPKPKDTLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPSREEMTKNQVSLTCLVK GFYPSDIAVEWESNGQPENNYKTTPPVLDSDGSFFLYSKLTVDKSRWQQGNVFSCSVMHEALHN HYTQKSLSLSPGK | 41 |

Table 18 below shows the polynucleotide sequences encoding the polypeptides of wtFc, TraH-G44C-wtFc, and TraL-Q100C-wtFc of HO1wt.

[Table 18]

| Name | Sequence | SEQ ID NO |
|---|---|---|
| wtFc | GACAAAACTCACACATGCCCACCGTGCCCAGCACCTGAACTCCTGGGGGGACCGTCAGTCTTCC TCTTCCCCCCAAAACCCAAGGACACCCTCATGATCTCCCGGACCCCTGAGGTCACATGCGTGGT GGTGGACGTGAGCCACGAAGACCCTGAGGTCAAGTTCAACTGGTACGTGGACGGCGTGGAGGTG CATAATGCCAAGACAAAGCCGCGGGAGGAGCAGTACAACAGCACGTACCGTGTGGTCAGCGTCC TCACCGTCCTGCACCAGGACTGGCTGAATGGCAAGGAGTACAAGTGCAAGGTCTCCAACAAAGC CCTCCCAGCCCCCATCGAGAAAACCATCTCCAAAGCCAAAGGGCAGCCCCGAGAACCACAGGTG TACACCCTGCCCCCATCCCGGGAGGAGATGACCAAGAACCAGGTCAGCCTGACGTGCCTGGTCA AAGGCTTCTATCCCAGCGACATCGCCGTGGAGTGGGAGAGCAATGGGCAGCCGGAGAACAACTA CAAGACCACGCCTCCCGTGCTGGACTCCGACGGCTCCTTCTTCCTCTACAGCAAGCTCACCGTG GACAAGAGCAGGTGGCAGCAGGGGAACGTCTTCTCATGCTCCGTGATGCATGAGGCTCTGCACA ACCACTACACGCAGAAGAGCCTCTCCCTGTCTCCGGGTAAA | 476 |
| TraII-G44C-wtFc | GAAGTTCAACTTGTGGAGTCTGGAGGCGGTTTGGTACAACCTGGAGGTTCACTTGTTTGTCAT GTGCTGCCTCTGGGTTTAATATCAAGGATACTTATATTCATTGGGTTCGCCAGGCACCCGGAAA ATGCCTGGAATGGGTGGCCCGTATATATCCCACTAACGGTTACACTCGATATGCTGACTCTGTA AAGGGTCGATTTACTATATCCGCTGATACTAGCAAAAACACTGCCTACCTCCAGATGAACAGTC TGCGCGCCGAGGATACAGCAGTTTATTATTGTTCCCGATGGGGAGCGTGACCGGTTCTATGCCAT GGACTACTGGGGTCAAGGGACACTGGTAACCGTTTCTTCTGCTAGCACCAAAGGACCTAGTGTT TTTCCTCTTGCCCCTTCCTCAAAGTCTACCTCTGGGGGGACAGCCGGCTCTGGGCTGCCTGGTCA ACGGATTATTTCCCAGAGCCTGTGCACTGTCAGTTGGAACTCTGGAGCCTTGACTTCTGGTGTTCA TACATTTCCTGCTGTGTCCTTCAAAGCAGCGGCTTGTACTCATTGTCTTCTGTTGTGACAGTACCC TCAAGCAGCCTCGGCACTCAGACATACATCTGCAATGTCAACCACAAACCCTCAAATACAAACG TAGATAAAAAAGTCGAACCAAAGTCTTGTGACAAAAACTCACACGTGCCCACCGTGCCCAGCACC TGAACTCCTGGGGGGACCGTCAGTCTTCCTCTTCCCCCCAAAACCCAAGGACACCCTCATGATC TCCCGGACCCCTGAGGTCACATGCGTGGTGGTGGACGTGAGCCACGAAGACCCTGAGGTCAAGT TCAACTGGTACGTGGACGGCGTGGAGGTGCATAATGCCAAGACAAAGCCGCGGGAGGAGCAGTA CAACAGCACGTACCGTGTGGTCAGCGTCCTCACCGTCCTGCACCAGGACTGGCTGAATGGCAAG GAGTACAAGTGCAAGGTCTCCAACAAAGCCCTCCCAGCCCCCATCGAGAAAACCATCTCCAAAG CCAAAGGGCAGCCCCGAGAACCACAGGTGTACACCCTGCCCCCAAGCCGGGAGGAGATGACCAA GAACCAGGTCAGCCTGACGTGCCTGGTCAAAGGCTTCTATCCCAGCGACATCGCCGTGGAGTGG GAGAGCAATGGGCAGCCGGAGAACAACTACAAGACCACGCCTCCCGTGCTGGACTCCGACGGCT CCTTCTTCCTCTACAGCAAGCTCACCGTGGACAAGAGCAGGTGGCAGCAGGGGAACGTCTTCTC ATGCTCCGTGATGCATGAGGCTCTGCACAACCACTACACGCAGAAGAGCCTCTCCCTGTCTCCG GGTAAA | 477 |
| TraL-Q100C-wtFc | GACATTCAGATGACCCAATCCCCCTCTAGCCTTTCAGCCTCCGTAGGGGACAGAGTTACAATAA CCTGTAGGGGCTAGTCAGGACGTTAATACAGCAGTCGCATGGTACCAGCAGAAGCCCGGTAAGGC TCCTAAGCTTTTTGATCTACAGTGCCTTCTTTCCTGTATTCTGGTGTACCTAGCCGATTCTCAGGC TCTCGGAGTGGCACAGACTTCACTCTGACAAATTTCAAGTCTCCAGCCAGAAGATTTTGCAACCT ACTACTGCCAACAACACTACACTACTCCTCCAACCTTCGGATGTGGCACAAAAGTAGAGATTAA GCGTACGGTGGCAGCTCCCAGCGTTTTTTATCTTTCCCCCATCCGACGAGCAGCTCAAGAGTGGC ACTGCCTCTGTAGTTGTTTGCTGAATAACTTCTATCCACGTGAAGCAAAAGTACAGTGGAAGG TCGATAATGCCCTTCAGAGCGGTAACAGCCAAGAAAGTGTTACCGAGCAAGATTCCAAAGATTC CACTTACAGTCTGTCCAGCACATTGACACTGAGTAAGGCTGATTACGAAAAACACAAGGTGTAC GCATGCGAGGTGACACACCAAGGTCTTTCATCTCCTGTAACTAAGAGCTTTAACCGGGGAGAAT GTGGTGGTGGGGGCAGCGGGGGCGGAGGTAGTGGAGGCGGCGGTAGTGAACCAAAGAGTAGTGA CAAAACTCACACGTGCCCACCGTGCCCAGCACCTGAACTCCTGGGGGGACCGTCAGTCTTCCTC TTCCCCCCAAAACCCAAGGACACCCTCATGATCTCCCGGACCCCTGAGGTCACATGCGTGGTGG TGGACGTGAGCCACGAAGACCCTGAGGTCAAGTTCAACTGGTACGTGGACGGCGTGGAGGTGCA | 478 |

TAATGCCAAGACAAAGCCGCGGGAGGAGCAGTACAACAGCACGTACCGTGTGGTCAGCGTCCTC
ACCGTCCTGCACCAGGACTGGCTGAATGGCAAGGAGTACAAGTGCAAGGTCTCCAACAAAGCCC
TCCCAGCCCCCATCGAGAAAACCATCTCCAAAGCCAAAGGGCAGCCCCGAGAACCACAGGTGTA
CACCCTGCCCCCAAGCCGGGAGGAGATGACCAAGAACCAGGTCAGCCTGACGTGCCTGGTCAAA
GGCTTCTATCCCAGCGACATCGCCGTGGAGTGGGAGAGCAATGGGCAGCCGGAGAACAACTACA
AGACCACGCCTCCCGTGCTGGACTCCGACGGCTCCTTCTTCCTCTACAGCAAGCTCACCGTGGA
CAAGAGCAGGTGGCAGCAGGGGAACGTCTTCTCATGCTCCGTGATGCATGAGGCTCTGCACAAC
CACTACACGCAGAAGAGCCTCTCCCTGTCTCCGGGTAAA

### Example 5. Characterization of H01Fv variant

The schematic diagram of Fv-(Fc)$_2$, in which two Fc domains are fused in parallel to an antibody Fv fragment, is shown in Figures 10a to 10g. Here, Fv consists of a VH region and a VL region. In order to improve interaction of the domains at the domain interfaces, an artificial disulfide bond was formed by substituting an amino acid at a specific position with Cys (Figures 10a to 10h and Table 19).

[Table 19]

|  | VH Mutation site (Kabat numbering) | VL Mutation site (Kabat numbering) | MW (kDa) |
|---|---|---|---|
| H01Fv1 | G44C | Q100C | 128.21 |
| H01Fv2 | Q105C | A43C | 128.19 |
| H01Fv3 | F122C | S121C | 130.98 |
| H01Fv4 | G44C, Q105C | Q100C, A43C | 130.01 |
| H01Fv5 | G44C, F122C | Q100C, S121C | 130.00 |
| H01Fv6 | Q105C, F126C | A43C, S121C | 130.99 |
| H01Fv7 | G44C, Q105C, F126C | Q100C, A43C, S121C | 131.01 |

Table 20 shows the polypeptide sequences constituting H01Fv1 to H01Fv7.

[Table 20]

| Name | Sequence | SEQ ID NO |
|------|----------|-----------|
| Fc-Hole-RF | DKTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKGQ PREPQVCTLPPSREEMTKNQVSLSCAVKGFYPSDIAVEWESNGQPENNYKTTPPVLDSDGS FFLVSKLTVDKSRWQQGNVFSCSVMHEALHNRFTQKSLSLSPGK | 789 |
| HO1Fv1-HC | EVQLVESGGGLVQPGGSLRLSCAASGFNIKDTYIHWVRQAPGKCLEWVARIYPTNGYTRYA DSVKGRFTISADTSKNTAYLQMNSLRAEDTAVYYCSRWGGDGFYAMDYWGQGTLVTVSSEP KSCDKTHTCPPCPAPELLGGPSVFLFPPKPKDTLMISRTPEVTCVVVDVSHEDPEVKFNWY VDGVEVHNAKTKPREEQYNSTYRVVSVLTVLHQDWLNGKEYKCKVSNKALPAPIEKTISKA KGQPREPQVYTLPPCREEMTKNQVSLWCLVKGFYPSDIAVEWESNGQPENNYKTTPPVLDS DGSFFLYSKLTVDKSRWQQGNVFSCSVMHEALHNHYTQKSLSLSPGK | 790 |
| HO1Fv1-LC | DIQMTQSPSSLSASVGDRVTITCRASQDVNTAVAWYQQKPGKAPKLLIYSASFLYSGVPSR FSGSRSGTDFTLTISSLQPEDFATYYCQQHYTTPPTFGCGTKVEIKREPKSCDKTHTCPPC PAPELLGGPSVFLFPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISKAKGQPREPQVYTL PPCREEMTKNQVSLWCLVKGFYPSDIAVEWESNGQPENNYKTTPPVLDSDGSFFLYSKLTV DKSRWQQGNVFSCSVMHEALHNHYTQKSLSLSPGK | 791 |
| HO1Fv2-HC | EVQLVESGGGLVQPGGSLRLSCAASGFNIKDTYIHWVRQAPGKGLEWVARIYPTNGYTRYA DSVKGRFTISADTSKNTAYLQMNSLRAEDTAVYYCSRWGGDGFYAMDYWGCGTLVTVSSEP KSCDKTHTCPPCPAPELLGGPSVFLFPPKPKDTLMISRTPEVTCVVVDVSHEDPEVKFNWY VDGVEVHNAKTKPREEQYNSTYRVVSVLTVLHQDWLNGKEYKCKVSNKALPAPIEKTISKA KGQPREPQVYTLPPCREEMTKNQVSLWCLVKGFYPSDIAVEWESNGQPENNYKTTPPVLDS DGSFFLYSKLTVDKSRWQQGNVFSCSVMHEALHNHYTQKSLSLSPGK | 792 |
| HO1Fv2-LC | DIQMTQSPSSLSASVGDRVTITCRASQDVNTAVAWYQQKPGKCPKLLIYSASFLYSGVPSR FSGSRSGTDFTLTISSLQPEDFATYYCQQHYTTPPTFGQGTKVEIKREPKSCDKTHTCPPC PAPELLGGPSVFLFPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISKAKGQPREPQVYTL PPCREEMTKNQVSLWCLVKGFYPSDIAVEWESNGQPENNYKTTPPVLDSDGSFFLYSKLTV | 793 |

| | | |
|---|---|---|
| | DKSRWQQGNVFSCSVMHEALHNHYTQKSLSLSPGK | |
| HO1Fv3-HC | EVQLVESGGGLVQPGGSLRLSCAASGFNIKDTYIHWVRQAPGKGLEWVARIYPTNGYTRYA DSVKGRFTISADTSKNTAYLQMNSLRAEDTAVYYCSRWGGDGFYAMDYWGQGTLVTVSSAS TKGPSVCPLAPEPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQDWLNGKEYKCKVSNK ALPAPIEKTISKAKGQPREPQVYTLPPCREEMTKNQVSLWCLVKGFYPSDIAVEWESNGQP ENNYKTTPPVLDSDGSFFLYSKLTVDKSRWQQGNVFSCSVMHEALHNHYTQKSLSLSPGK | 794 |
| HO1Fv3-LC | DIQMTQSPSSLSASVGDRVTITCRASQDVNTAVAWYQQKPGKAPKLLIYSASFLYSGVPSR FSGSRSGTDFTLTISSLQPEDFATYYCQQHYTTPPTFGQGTKVEIKRTVAAPSVFIFPPCG GGSEPKSCDKTHTCPPCPAPELLGGPSVFLFPPKPKDTLMISRTPEVTCVVVDVSHEDPEV KFNWYVDGVEVHNAKTKPREEQYNSTYRVVSVLTVLHQDWLNGKEYKCKVSNKALPAPIEK TISKAKGQPREPQVYTLPPCREEMTKNQVSLWCLVKGFYPSDIAVEWESNGQPENNYKTTP PVLDSDGSFFLYSKLTVDKSRWQQGNVFSCSVMHEALHNHYTQKSLSLSPGK | 795 |
| HO1Fv4-HC | EVQLVESGGGLVQPGGSLRLSCAASGFNIKDTYIHWVRQAPGKCLEWVARIYPTNGYTRYA DSVKGRFTISADTSKNTAYLQMNSLRAEDTAVYYCSRWGGDGFYAMDYWGCGTLVTVSSAS TKGPSVCPLAPEPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQDWLNGKEYKCKVSNK ALPAPIEKTISKAKGQPREPQVYTLPPCREEMTKNQVSLWCLVKGFYPSDIAVEWESNGQP ENNYKTTPPVLDSDGSFFLYSKLTVDKSRWQQGNVFSCSVMHEALHNHYTQKSLSLSPGK | 796 |
| HO1Fv4-LC | DIQMTQSPSSLSASVGDRVTITCRASQDVNTAVAWYQQKPGKCPKLLIYSASFLYSGVPSR FSGSRSGTDFTLTISSLQPEDFATYYCQQHYTTPPTFGCGTKVEIKREPKSCDKTHTCPPC PAPELLGGPSVFLFPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISKAKGQPREPQVYTL PPCREEMTKNQVSLWCLVKGFYPSDIAVEWESNGQPENNYKTTPPVLDSDGSFFLYSKLTV DKSRWQQGNVFSCSVMHEALHNHYTQKSLSLSPGK | 797 |
| HO1Fv5-HC | EVQLVESGGGLVQPGGSLRLSCAASGFNIKDTYIHWVRQAPGKCLEWVARIYPTNGYTRYA DSVKGRFTISADTSKNTAYLQMNSLRAEDTAVYYCSRWGGDGFYAMDYWGQGTLVTVSSAS TKGPSVCPLAPEPKSCDKTHTCPPCPAPELLGGPSVFLFPPKPKDTLMISRTPEVTCVVVD VSHEDPEVKFNWYVDGVEVHNAKTKPREEQYNSTYRVVSVLTVLHQDWLNGKEYKCKVSNK | 798 |

| | | |
|---|---|---|
| | ALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | |
| H01Fv5-LC | DIQMTQSPSSLSASVGDRVTITCRASQDVNTAVAWYQQKPGKAPKLLIYSASFLYSGVPSRFSGSRSGTDFTLTISSLQPEDFATYYCQQHYTTPPTFGCGTKVEIKRTVAAPSVFIFPPCGGGSEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 799 |
| H01Fv6-HC | EVQLVESGGGLVQPGGSLRLSCAASGFNIKDTYIHWVRQAPGKGLEWVARIYPTNGYTRYADSVKGRFTISADTSKNTAYLQMNSLRAEDTAVYYCSRWGGDGFYAMDYWGCGTLVTVSSASTKGPSVCPLAPEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 800 |
| H01Fv6-LC | DIQMTQSPSSLSASVGDRVTITCRASQDVNTAVAWYQQKPGKCPKLLIYSASFLYSGVPSRFSGSRSGTDFTLTISSLQPEDFATYYCQQHYTTPPTFGQGTKVEIKRTVAAPSVFIFPPCGGGSEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 801 |
| H01Fv7-HC | EVQLVESGGGLVQPGGSLRLSCAASGFNIKDTYIHWVRQAPGKCLEWVARIYPTNGYTRYADSVKGRFTISADTSKNTAYLQMNSLRAEDTAVYYCSRWGGDGFYAMDYWGCGTLVTVSSASTKGPSVCPLAPEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 802 |
| H01Fv7-LC | DIQMTQSPSSLSASVGDRVTITCRASQDVNTAVAWYQQKPGKCPKLLIYSASFLYSGVPSRFSGSRSGTDFTLTISSLQPEDFATYYCQQHYTTPPTFGCGTKVEIKRTVAAPSVFIFPPCGGGSEPKSCDKTHTCPPCPAPELLGGPSVFLFPPKPKDTLMISRTPEVTCVVVDVSHEDPEV | 803 |
| | KFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | |

Table 21 shows the polynucleotide sequences encoding the polypeptides constituting H01Fv1 to H01Fv7.

[Table 21]

| Name | Sequence | SEQ ID NO |
|---|---|---|
| Fc-Hole-RF | GACAAAACTCACACATGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTGCACCCTGCCCCCATCCCGGGAGGAGATGACCAAGAACCAGGTCAGCCTGAGCTGCGCGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCGTGAGCAAGCTCACCGTGGACAAGAGCAGGTGGCAGCAGGGGAACGTCTTCTCATGCTCCGTGATGCATGAGGCTCTGCACAACAGATTTACGCAGAAGAGCCTCTCCCTGTCTCCGGGTAAA | 804 |
| H01Fv1-HC | GAAGTTCAACTTGTGGAGTCTGGAGGCGGTTTGGTACAACCTGGAGGTTCACTTCGTTTGTCATGTGCTGCCTCTGGGTTTAATATCAAGGATACTTATATTCATTGGGTTCGCCAGGCACCCGGAAAATGCCTGGAATGGGTGGCCCGTATATATCCCACTAACGGTTACACTCGATATGCTGACTCTGTAAAGGGTCGATTTACTATATCCGCTGATACTAGCAAAAACACTGCCTACCTCCAGATGAACAGTCTGCGCGCCGAGGATACAGCAGTTTATTATTGTTCCCGATGGGGAGGTGACCGGGTTCTATGCCATGGACTACTGGGGTCAAGGGACACTGGTAACCGTTTCTTCTGAACCAAAGTCTTGTGACAAAACTCACACGTGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTAC | 805 |

| | GTGGACGGCGTGGAGGTGCATAATGCCAAGACAAAGCCGCGGGAGGAGCAGTACAACAGCA CGTACCGTGTGGTCAGCGTCCTCACCGTCCTGCACCAGGACTGGCTGAATGGCAAGGAGTA CAAGTGCAAGGTCTCCAACAAAGCCCTCCCAGCCCCCATCGAGAAAACCATCTCCAAAGCC AAAGGGCAGCCCCGAGAACCACAGGTGTACACCCTGCCCCCATGCCGGGAGGAGATGACCA AGAACCAGGTCAGCCTGTGGTGCCTGGTCAAAGGCTTCTATCCCAGCGACATCGCCGTGGA GTGGGAGAGCAATGGGCAGCCGGAGAACAACTACAAGACCACGCCTCCCGTGCTGGACTCC GACGGCTCCTTCTTCCTCTACAGCAAGCTCACCGTGGACAAGAGCAGGTGGCAGCAGGGGA ACGTCTTCTCATGCTCCGTGATGCATGAGGCTCTGCACAACCACTACACGCAGAAGAGCCT CTCCCTGTCTCCGGGTAAA | |
| HO1Fv1-LC | GACATTCAGATGACCCAATCCCCCTCTAGCCTTTCAGCCTCCGTAGGGGACAGAGTTACAA TAACCTGTAGGGCTAGTCAGGACGTTAATACAGCAGTCGCATGGTACCAGCAGAAGCCCGG TAAGGCTCCTAAGCTTTTGATCTACAGTGCTTCTTTCCTGTATTCTGGTGTACCTAGCCGA TTCTCAGGCTCTCGGAGTGGCACAGACTTCACTCTGACAATTTCAAGTCTCCAGCCAGAAG ATTTTGCAACCTACTACTGCCAACAACACTACACTACTCCTCCAACCTTCGGATGTGGCAC AAAAGTAGAGATTAAGCGTGAACCAAAGTCTTGTGACAAAACTCACACGTGCCCACCGTGC CCAGCACCTGAACTCCTGGGGGGACCGTCAGTCTTCCTCTTCCCCCCAAAACCCAAGGACA CCCTCATGATCTCCCGGACCCCTGAGGTCACATGCGTGGTGGTGGACGTGAGCCACGAAGA CCCTGAGGTCAAGTTCAACTGGTACGTGGACGGCGTGGAGGTGCATAATGCCAAGACAAAG CCGCGGGAGGAGCAGTACAACAGCACGTACCGTGTGGTCAGCGTCCTCACCGTCCTGCACC AGGACTGGCTGAATGGCAAGGAGTACAAGTGCAAGGTCTCCAACAAAGCCCTCCCAGCCCC CATCGAGAAAACCATCTCCAAAGCCAAAGGGCAGCCCCGAGAACCACAGGTGTACACCCTG CCCCCATGCCGGGAGGAGATGACCAAGAACCAGGTCAGCCTGTGGTGCCTGGTCAAAGGCT TCTATCCCAGCGACATCGCCGTGGAGTGGGAGAGCAATGGGCAGCCGGAGAACAACTACAA GACCACGCCTCCCGTGCTGGACTCCGACGGCTCCTTCTTCCTCTACAGCAAGCTCACCGTG GACAAGAGCAGGTGGCAGCAGGGGAACGTCTTCTCATGCTCCGTGATGCATGAGGCTCTGC ACAACCACTACACGCAGAAGAGCCTCTCCCTGTCTCCGGGTAAA | 806 |
| HO1Fv2-HC | GAAGTTCAACTTGTGGAGTCTGGAGGCGGTTTGGTACAACCTGGAGGTTCACTTCGTTTGT CATGTGCTGCCTCTGGGTTTAATATCAAGGATACTTATATTCATTGGGTTCGCCAGGCACC CGGAAAAGGGCTGGAATGGGTGGCCCGTATATATCCCACTAACGGTTACACTCGATATGCT | 807 |

| | | |
|---|---|---|
| | GACTCTGTAAAGGGTCGATTTACTATATCCGCTGATACTAGCAAAAACACTGCCTACCTCC AGATGAACAGTCTGCGCGCCGAGGATACAGCAGTTTATTATTGTTCCCGATGGGGAGGTGA CGGGTTCTATGCCATGGACTACTGGGGTTGTGGGACACTGGTAACCGTTTCTTCTGAACCA AAGTCTTGTGACAAAACTCACACGTGCCCACCGTGCCCAGCACCTGAACTCCTGGGGGGAC CGTCAGTCTTCCTCTTCCCCCCAAAACCCAAGGACACCCTCATGATCTCCCGGACCCCTGA GGTCACATGCGTGGTGGTGGACGTGAGCCACGAAGACCCTGAGGTCAAGTTCAACTGGTAC GTGGACGGCGTGGAGGTGCATAATGCCAAGACAAAGCCGCGGGAGGAGCAGTACAACAGCA CGTACCGTGTGGTCAGCGTCCTCACCGTCCTGCACCAGGACTGGCTGAATGGCAAGGAGTA CAAGTGCAAGGTCTCCAACAAAGCCCTCCCAGCCCCCATCGAGAAAACCATCTCCAAAGCC AAAGGGCAGCCCCGAGAACCACAGGTGTACACCCTGCCCCCATGCCGGGAGGAGATGACCA AGAACCAGGTCAGCCTGTGGTGCCTGGTCAAAGGCTTCTATCCCAGCGACATCGCCGTGGA GTGGGAGAGCAATGGGCAGCCGGAGAACAACTACAAGACCACGCCTCCCGTGCTGGACTCC GACGGCTCCTTCTTCCTCTACAGCAAGCTCACCGTGGACAAGAGCAGGTGGCAGCAGGGGA ACGTCTTCTCATGCTCCGTGATGCATGAGGCTCTGCACAACCACTACACGCAGAAGAGCCT CTCCCTGTCTCCGGGTAAA | |
| H01Fv2-LC | GACATTCAGATGACCCAATCCCCCTCTAGCCTTTCAGCCTCCGTAGGGGACAGAGTTACAA TAACCTGTAGGGCTAGTCAGGACGTTAATACAGCAGTCGCATGGTACCAGCAGAAGCCCGG TAAGTGTCCTAAGCTTTTGATCTACAGTGCTTCTTTCCTGTATTCTGGTGTACCTAGCCGA TTCTCAGGCTCTCGGAGTGGCACAGACTTCACTCTGACAATTTCAAGTCTCCAGCCAGAAG ATTTTGCAACCTACTACTGCCAACAACACTACACTACTCCTCCAACCTTCGGACAGGGCAC AAAAGTAGAGATTAAGCGTGAACCAAAGTCTTGTGACAAAACTCACACGTGCCCACCGTGC CCAGCACCTGAACTCCTGGGGGGACCGTCAGTCTTCCTCTTCCCCCCAAAACCCAAGGACA CCCTCATGATCTCCCGGACCCCTGAGGTCACATGCGTGGTGGTGGACGTGAGCCACGAAGA CCCTGAGGTCAAGTTCAACTGGTACGTGGACGGCGTGGAGGTGCATAATGCCAAGACAAAG CCGCGGGAGGAGCAGTACAACAGCACGTACCGTGTGGTCAGCGTCCTCACCGTCCTGCACC AGGACTGGCTGAATGGCAAGGAGTACAAGTGCAAGGTCTCCAACAAAGCCCTCCCAGCCCC CATCGAGAAAACCATCTCCAAAGCCAAAGGGCAGCCCCGAGAACCACAGGTGTACACCCTG CCCCCATGCCGGGAGGAGATGACCAAGAACCAGGTCAGCCTGTGGTGCCTGGTCAAAGGCT TCTATCCCAGCGACATCGCCGTGGAGTGGGAGAGCAATGGGCAGCCGGAGAACAACTACAA | 808 |

| | | |
|---|---|---|
| | GACCACGCCTCCCGTGCTGGACTCCGACGGCTCCTTCTTCCTCTACAGCAAGCTCACCGTG GACAAGAGCAGGTGGCAGCAGGGGAACGTCTTCTCATGCTCCGTGATGCATGAGGCTCTGC ACAACCACTACACGCAGAAGAGCCTCTCCCTGTCTCCGGGTAAA | |
| HO1Fv3-HC | GAAGTTCAACTTGTGGAGTCTGGAGGCGGTTTGGTACAACCTGGAGGTTCACTTCGTTTGT CATGTGCTGCCTCTGGGTTTAATATCAAGGATACTTATATTCATTGGGTTCGCCAGGCACC CGGAAAAGGGCTGGAATGGGTGGCCCGTATATATCCCACTAACGGTTACACTCGATATGCT GACTCTGTAAAGGGTCGATTTACTATATCCGCTGATACTAGCAAAAACACTGCCTACCTCC AGATGAACAGTCTGCGCGCCGAGGATACAGCAGTTTATTATTGTTCCCGATGGGGAGGTGA CGGGTTCTATGCCATGGACTACTGGGGTCAAGGGACACTGGTAACCGTTTCTTCTGCTAGC ACCAAAGGACCTAGTGTTTGTCCTCTTGCCCCTGAACCAAAGTCTTGTGACAAAACTCACA CGTGCCCACCGTGCCCAGCACCTGAACTCCTGGGGGGACCGTCAGTCTTCCTCTTCCCCCC AAAACCCAAGGACACCCTCATGATCTCCCGGACCCCTGAGGTCACATGCGTGGTGGTGGAC GTGAGCCACGAAGACCCTGAGGTCAAGTTCAACTGGTACGTGGACGGCGTGGAGGTGCATA ATGCCAAGACAAAGCCGCGGGAGGAGCAGTACAACAGCACGTACCGTGTGGTCAGCGTCCT CACCGTCCTGCACCAGGACTGGCTGAATGGCAAGGAGTACAAGTGCAAGGTCTCCAACAAA GCCCTCCCAGCCCCCATCGAGAAAACCATCTCCAAAGCCAAAGGGCAGCCCCGAGAACCAC AGGTGTACACCCTGCCCCCATGCCGGGAGGAGATGACCAAGAACCAGGTCAGCCTGTGGTG CCTGGTCAAAGGCTTCTATCCCAGCGACATCGCCGTGGAGTGGGAGAGCAATGGGCAGCCG GAGAACAACTACAAGACCACGCCTCCCGTGCTGGACTCCGACGGCTCCTTCTTCCTCTACA GCAAGCTCACCGTGGACAAGAGCAGGTGGCAGCAGGGGAACGTCTTCTCATGCTCCGTGAT GCATGAGGCTCTGCACAACCACTACACGCAGAAGAGCCTCTCCCTGTCTCCGGGTAAA | 809 |
| HO1Fv3-LC | GACATTCAGATGACCCAATCCCCCTCTAGCCTTTCAGCCTCCGTAGGGGACAGAGTTACAA TAACCTGTAGGGCTAGTCAGGACGTTAATACAGCAGTCGCATGGTACCAGCAGAAGCCCGG TAAGGCTCCTAAGCTTTTGATCTACAGTGCTTCTTTCCTGTATTCTGGTGTACCTAGCCGA TTCTCAGGCTCTCGGAGTGGCACAGACTTCACTCTGACAATTTCAAGTCTCCAGCCAGAAG ATTTTGCAACCTACTACTGCCAACAACACTACACTACTCCTCCAACCTTCGGACAGGGCAC AAAAGTAGAGATTAAGCGTACGGTGGCAGCTCCCAGCGTTTTTATCTTTCCCCCATGTGGA GGTGGAAGTGAACCAAAGTCTTGTGACAAAACTCACACGTGCCCACCGTGCCCAGCACCTG AACTCCTGGGGGGACCGTCAGTCTTCCTCTTCCCCCCAAAACCCAAGGACACCCTCATGAT | 810 |

| | | |
|---|---|---|
| | CTCCCGGACCCCTGAGGTCACATGCGTGGTGGTGGACGTGAGCCACGAAGACCCTGAGGTC AAGTTCAACTGGTACGTGGACGGCGTGGAGGTGCATAATGCCAAGACAAAGCCGCGGGAGG AGCAGTACAACAGCACGTACCGTGTGGTCAGCGTCCTCACCGTCCTGCACCAGGACTGGCT GAATGGCAAGGAGTACAAGTGCAAGGTCTCCAACAAAGCCCTCCCAGCCCCCATCGAGAAA ACCATCTCCAAAGCCAAAGGGCAGCCCCGAGAACCACAGGTGTACACCCTGCCCCCATGCC GGGAGGAGATGACCAAGAACCAGGTCAGCCTGTGGTGCCTGGTCAAAGGCTTCTATCCCAG CGACATCGCCGTGGAGTGGGAGAGCAATGGGCAGCCGGAGAACAACTACAAGACCACGCCT CCCGTGCTGGACTCCGACGGCTCCTTCTTCCTCTACAGCAAGCTCACCGTGGACAAGAGCA GGTGGCAGCAGGGGAACGTCTTCTCATGCTCCGTGATGCATGAGGCTCTGCACAACCACTA CACGCAGAAGAGCCTCTCCCTGTCTCCGGGTAAA | |
| H01Fv4-HC | GAAGTTCAACTTGTGGAGTCTGGAGGCGGTTTGGTACAACCTGGAGGTTCACTTCGTTTGT CATGTGCTGCCTCTGGGTTTAATATCAAGGATACTTATATTCATTGGGTTCGCCAGGCACC CGGAAAATGCCTGGAATGGGTGGCCCGTATATATCCCACTAACGGTTACACTCGATATGCT GACTCTGTAAAGGGTCGATTTACTATATCCGCTGATACTAGCAAAAACACTGCCTACCTCC AGATGAACAGTCTGCGCGCCGAGGATACAGCAGTTTATTATTGTTCCCGATGGGGAGGTGA CGGGTTCTATGCCATGGACTACTGGGGTTGTGGGACACTGGTAACCGTTTCTTCTGCTAGC ACCAAAGGACCTAGTGTTTGTCCTCTTGCCCCTGAACCAAAGTCTTGTGACAAAACTCACA CGTGCCCACCGTGCCCAGCACCTGAACTCCTGGGGGGACCGTCAGTCTTCCTCTTCCCCCC AAAACCCAAGGACACCCTCATGATCTCCCGGACCCCTGAGGTCACATGCGTGGTGGTGGAC GTGAGCCACGAAGACCCTGAGGTCAAGTTCAACTGGTACGTGGACGGCGTGGAGGTGCATA ATGCCAAGACAAAGCCGCGGGAGGAGCAGTACAACAGCACGTACCGTGTGGTCAGCGTCCT CACCGTCCTGCACCAGGACTGGCTGAATGGCAAGGAGTACAAGTGCAAGGTCTCCAACAAA GCCCTCCCAGCCCCCATCGAGAAAACCATCTCCAAAGCCAAAGGGCAGCCCCGAGAACCAC AGGTGTACACCCTGCCCCCATGCCGGGAGGAGATGACCAAGAACCAGGTCAGCCTGTGGTG CCTGGTCAAAGGCTTCTATCCCAGCGACATCGCCGTGGAGTGGGAGAGCAATGGGCAGCCG GAGAACAACTACAAGACCACGCCTCCCGTGCTGGACTCCGACGGCTCCTTCTTCCTCTACA GCAAGCTCACCGTGGACAAGAGCAGGTGGCAGCAGGGGAACGTCTTCTCATGCTCCGTGAT GCATGAGGCTCTGCACAACCACTACACGCAGAAGAGCCTCTCCCTGTCTCCGGGTAAA | 811 |
| H01Fv4-LC | GACATTCAGATGACCCAATCCCCCTCTAGCCTTTCAGCCTCCGTAGGGGACAGAGTTACAA | 812 |

| | | |
|---|---|---|
| | TAACCTGTAGGGCTAGTCAGGACGTTAATACAGCAGTCGCATGGTACCAGCAGAAGCCCGG TAAGTGTCCTAAGCTTTTGATCTACAGTGCTTCTTTCCTGTATTCTGGTGTACCTAGCCGA TTCTCAGGCTCTCGGAGTGGCACAGACTTCACTCTGACAATTTCAAGTCTCCAGCCAGAAG ATTTTGCAACCTACTACTGCCAACAACACTACACTACTCCTCCAACCTTCGGATGTGGCAC AAAAGTAGAGATTAAGCGTGAACCAAAGTCTTGTGACAAAACTCACACGTGCCCACCGTGC CCAGCACCTGAACTCCTGGGGGGACCGTCAGTCTTCCTCTTCCCCCCAAAACCCAAGGACA CCCTCATGATCTCCCGGACCCCTGAGGTCACATGCGTGGTGGTGGACGTGAGCCACGAAGA CCCTGAGGTCAAGTTCAACTGGTACGTGGACGGCGTGGAGGTGCATAATGCCAAGACAAAG CCGCGGGAGGAGCAGTACAACAGCACGTACCGTGTGGTCAGCGTCCTCACCGTCCTGCACC AGGACTGGCTGAATGGCAAGGAGTACAAGTGCAAGGTCTCCAACAAAGCCCTCCCAGCCCC CATCGAGAAAACCATCTCCAAAGCCAAAGGGCAGCCCCGAGAACCACAGGTGTACACCCTG CCCCCATGCCGGGAGGAGATGACCAAGAACCAGGTCAGCCTGTGGTGCCTGGTCAAAGGCT TCTATCCCAGCGACATCGCCGTGGAGTGGGAGAGCAATGGGCAGCCGGAGAACAACTACAA GACCACGCCTCCCGTGCTGGACTCCGACGGCTCCTTCTTCCTCTACAGCAAGCTCACCGTG GACAAGAGCAGGTGGCAGCAGGGGAACGTCTTCTCATGCTCCGTGATGCATGAGGCTCTGC ACAACCACTACACGCAGAAGAGCCTCTCCCTGTCTCCGGGTAAA | |
| H01Fv5-HC | GAAGTTCAACTTGTGGAGTCTGGAGGCGGTTTGGTACAACCTGGAGGTTCACTTCGTTTGT CATGTGCTGCCTCTGGGTTTAATATCAAGGATACTTATATTCATTGGGTTCGCCAGGCACC CGGAAAATGCCTGGAATGGGTGGCCCGTATATATCCCACTAACGGTTACACTCGATATGCT GACTCTGTAAAGGGTCGATTTACTATATCCGCTGATACTAGCAAAAACACTGCCTACCTCC AGATGAACAGTCTGCGCGCCGAGGATACAGCAGTTTATTATTGTTCCCGATGGGGAGGTGA CGGGTTCTATGCCATGGACTACTGGGGTCAAGGGACACTGGTAACCGTTTCTTCTGCTAGC ACCAAAGGACCTAGTGTTTGTCCTCTTGCCCCTGAACCAAAGTCTTGTGACAAAACTCACA CGTGCCCACCGTGCCCAGCACCTGAACTCCTGGGGGGACCGTCAGTCTTCCTCTTCCCCCC AAAACCCAAGGACACCCTCATGATCTCCCGGACCCCTGAGGTCACATGCGTGGTGGTGGAC GTGAGCCACGAAGACCCTGAGGTCAAGTTCAACTGGTACGTGGACGGCGTGGAGGTGCATA ATGCCAAGACAAAGCCGCGGGAGGAGCAGTACAACAGCACGTACCGTGTGGTCAGCGTCCT CACCGTCCTGCACCAGGACTGGCTGAATGGCAAGGAGTACAAGTGCAAGGTCTCCAACAAA GCCCTCCCAGCCCCCATCGAGAAAACCATCTCCAAAGCCAAAGGGCAGCCCCGAGAACCAC | 813 |

| | | |
|---|---|---|
| | AGGTGTACACCCTGCCCCCATGCCGGGAGGAGATGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | |
| H01Fv5-LC | GACATTCAGATGACCCAATCCCCCTCTAGCCTTTCAGCCTCCGTAGGGGACAGAGTTACAATAACCTGTAGGGCTAGTCAGGACGTTAATACAGCAGTCGCATGGTACCAGCAGAAGCCCGGTAAGGCTCCTAAGCTTTTGATCTACAGTGCTTCTTTCCTGTATTCTGGTGTACCTAGCCGATTCTCAGGCTCTCGGAGTGGCACAGACTTCACTCTGACAATTTCAAGTCTCCAGCCAGAAGATTTTGCAACCTACTACTGCCAACAACACTACACTACTCCTCCAACCTTCGGATGTGGCACAAAAGTAGAGATTAAGCGTACGGTGGCAGCTCCCAGCGTTTTTATCTTTCCCCCATGTGGAGGTGGAAGTGAACCAAAGTCTTGTGACAAAACTCACACGTGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATGCCGGGAGGAGATGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | 814 |
| H01Fv6-HC | GAAGTTCAACTTGTGGAGTCTGGAGGCGGTTTGGTACAACCTGGAGGTTCACTTCGTTTGTCATGTGCTGCCTCTGGGTTTAATATCAAGGATACTTATATTCATTGGGTTCGCCAGGCACCCGGAAAAGGGCTGGAATGGGTGGCCCGTATATATCCCACTAACGGTTACACTCGATATGCTGACTCTGTAAAGGGTCGATTTACTATATCCGCTGATACTAGCAAAAACACTGCCTACCTCCAGATGAACAGTCTGCGCGCCGAGGATACAGCAGTTTATTATTGTTCCCGATGGGGAGGTGACGGGGTTCTATGCCATGGACTACTGGGGTTGTGGGACACTGGTAACCGTTTCTTCTGCTAGC | 815 |

| | | |
|---|---|---|
| | ACCAAAGGACCTAGTGTTTGTCCTCTTGCCCCCTGAACCAAAGTCTTGTGACAAAACTCACACGTGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATGCCGGGAGGAGATGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | |
| HO1Fv6-LC | GACATTCAGATGACCCAATCCCCCTCTAGCCTTTCAGCCTCCGTAGGGGACAGAGTTACAATAACCTGTAGGGCTAGTCAGGACGTTAATACAGCAGTCGCATGGTACCAGCAGAAGCCCGGTAAGTGTCCTAAGCTTTTGATCTACAGTGCTTCTTTCCTGTATTCTGGTGTACCTAGCCGATTCTCAGGCTCTCGGAGTGGCACAGACTTCACTCTGACAATTTCAAGTCTCCAGCCAGAAGATTTTGCAACCTACTACTGCCAACAACACTACACTACTCCTCCAACCTTCGGACAGGGCACAAAAGTAGAGATTAAGCGTACGGTGGCAGCTCCCAGCGTTTTTATCTTTCCCCCATGTGGAGGTGGAAGTGAACCAAAGTCTTGTGACAAAACTCACACGTGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATGCCGGGAGGAGATGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTA | 816 |

| | CACGCAGAAGAGCCTCTCCCTGTCTCCGGGTAAA | |
|---|---|---|
| H01Fv7-HC | GAAGTTCAACTTGTGGAGTCTGGAGGCGGTTTGGTACAACCTGGAGGTTCACTTCGTTTGT CATGTGCTGCCTCTGGGTTTAATATCAAGGATACTTATATTCATTGGGTTCGCCAGGCACC CGGAAAATGCCTGGAATGGGTGGCCCGTATATATCCCACTAACGGTTACACTCGATATGCT GACTCTGTAAAGGGTCGATTTACTATATCCGCTGATACTAGCAAAAACACTGCCTACCTCC AGATGAACAGTCTGCGCGCCGAGGATACAGCAGTTTATTATTGTTCCCGATGGGGAGGTGA CGGGTTCTATGCCATGGACTACTGGGGTTGTGGGACACTGGTAACCGTTTCTTCTGCTAGC ACCAAAGGACCTAGTGTTTGTCCTCTTGCCCCTGAACCAAAGTCTTGTGACAAAACTCACA CGTGCCCACCGTGCCCAGCACCTGAACTCCTGGGGGGACCGTCAGTCTTCCTCTTCCCCCC AAAAACCCAAGGACACCCTCATGATCTCCCGGACCCCTGAGGTCACATGCGTGGTGGTGGAC GTGAGCCACGAAGACCCTGAGGTCAAGTTCAACTGGTACGTGGACGGCGTGGAGGTGCATA ATGCCAAGACAAAGCCGCGGGAGGAGCAGTACAACAGCACGTACCGTGTGGTCAGCGTCCT CACCGTCCTGCACCAGGACTGGCTGAATGGCAAGGAGTACAAGTGCAAGGTCTCCAACAAA GCCCTCCCAGCCCCCATCGAGAAAACCATCTCCAAAGCCAAAGGGCAGCCCCGAGAACCAC AGGTGTACACCCTGCCCCCATGCCGGGAGGAGATGACCAAGAACCAGGTCAGCCTGTGGTG CCTGGTCAAAGGCTTCTATCCCAGCGACATCGCCGTGGAGTGGGAGAGCAATGGGCAGCCG GAGAACAACTACAAGACCACGCCTCCCGTGCTGGACTCCGACGGCTCCTTCTTCCTCTACA GCAAGCTCACCGTGGACAAGAGCAGGTGGCAGCAGGGGAACGTCTTCTCATGCTCCGTGAT GCATGAGGCTCTGCACAACCACTACACGCAGAAGAGCCTCTCCCTGTCTCCGGGTAAA | 817 |
| H01Fv7-LC | GACATTCAGATGACCCAATCCCCCTCTAGCCTTTCAGCCTCCGTAGGGGACAGAGTTACAA TAACCTGTAGGGCTAGTCAGGACGTTAATACAGCAGTCGCATGGTACCAGCAGAAGCCCGG TAAGTGTCCTAAGCTTTTGATCTACAGTGCTTCTTTCCTGTATTCTGGTGTACCTAGCCGA TTCTCAGGCTCTCGGAGTGGCACAGACTTCACTCTGACAATTTCAAGTCTCCAGCCAGAAG ATTTTGCAACCTACTACTGCCAACAACACTACACTACTCCTCCAACCTTCGGATGTGGCAC AAAAGTAGAGATTAAGCGTACGGTGGCAGCTCCCAGCGTTTTTATCTTTCCCCCATGTGGA GGTGGAAGTGAACCAAAGTCTTGTGACAAAACTCACACGTGCCCACCGTGCCCAGCACCTG AACTCCTGGGGGGACCGTCAGTCTTCCTCTTCCCCCCAAAAACCCAAGGACACCCTCATGAT CTCCCGGACCCCTGAGGTCACATGCGTGGTGGTGGACGTGAGCCACGAAGACCCTGAGGTC AAGTTCAACTGGTACGTGGACGGCGTGGAGGTGCATAATGCCAAGACAAAGCCGCGGGAGG | 818 |

| |
|---|
| AGCAGTACAACAGCACGTACCGTGTGGTCAGCGTCCTCACCGTCCTGCACCAGGACTGGCT GAATGGCAAGGAGTACAAGTGCAAGGTCTCCAACAAAGCCCTCCCAGCCCCCATCGAGAAA ACCATCTCCAAAGCCAAAGGGCAGCCCCGAGAACCACAGGTGTACACCCTGCCCCCATGCC GGGAGGAGATGACCAAGAACCAGGTCAGCCTGTGGTGCCTGGTCAAAGGCTTCTATCCCAG CGACATCGCCGTGGAGTGGGAGAGCAATGGGCAGCCGGAGAACAACTACAAGACCACGCCT CCCGTGCTGGACTCCGACGGCTCCTTCTTCCTCTACAGCAAGCTCACCGTGGACAAGAGCA GGTGGCAGCAGGGGAACGTCTTCTCATGCTCCGTGATGCATGAGGCTCTGCACAACCACTA CACGCAGAAGAGCCTCTCCCTGTCTCCGGGTAAA |

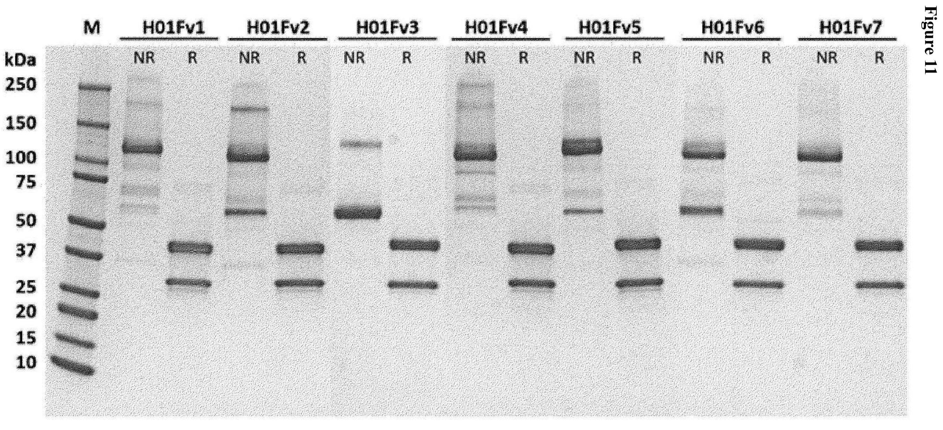

H01Fv1 consists of the polypeptides of Fc-Hole-RF (SEQ ID NO: 789), H01Fv1-HC (SEQ ID NO: 790), and H01Fv1-LC (SEQ ID NO: 791), and was prepared by co-transfection of expression vectors loaded with polynucleotides encoding the polypeptides into the ExpiCHO-S™ cell line (Figure 10, Table 20 and Table 21). Thereafter, it was purified through affinity chromatography using MABSELECT™ PrismA (Cytiva, 17549853). The Fc-Hole polypeptide (SEQ ID NO: 7) can form an Fc-Hole/Fc-Hole dimer. In order to remove this Fc-Hole/Fc-Hole dimer, H435R and Y436F mutations were induced in the Fc-Hole (SEQ ID NO: 7) polypeptide sequence to produce the Fc-Hole-RF polypeptide (SEQ ID NO: 789). This can prevent the Fc-Hole/Fc-Hole dimer from binding to Protein A resin and remove the mispaired Fc-Hole/Fc-Hole dimer (Figures 10 and 11).

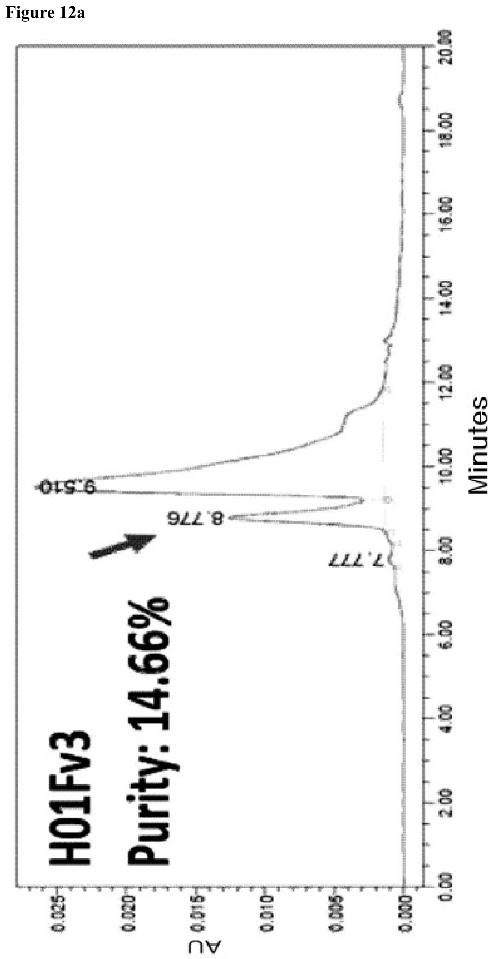

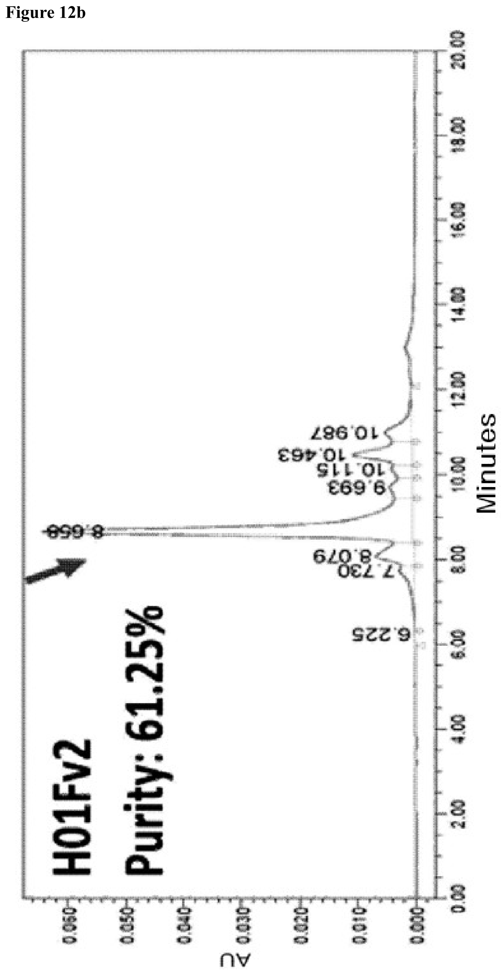

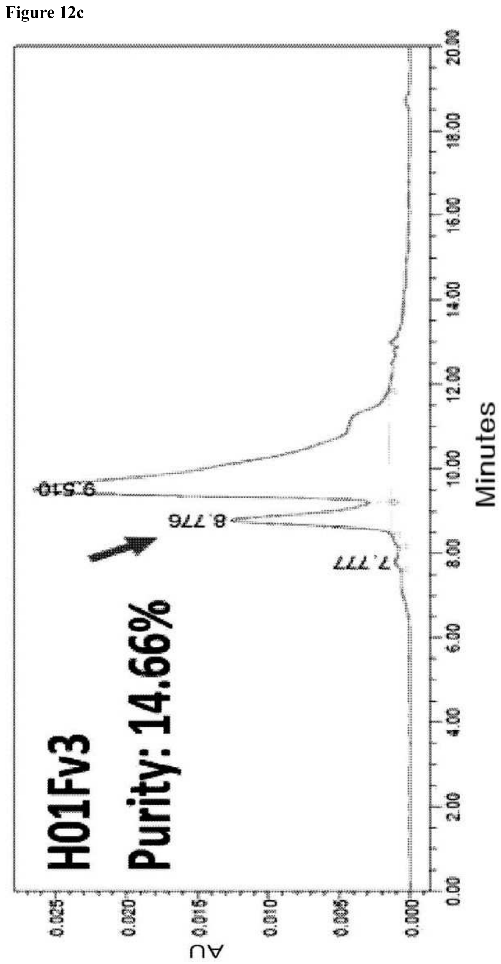

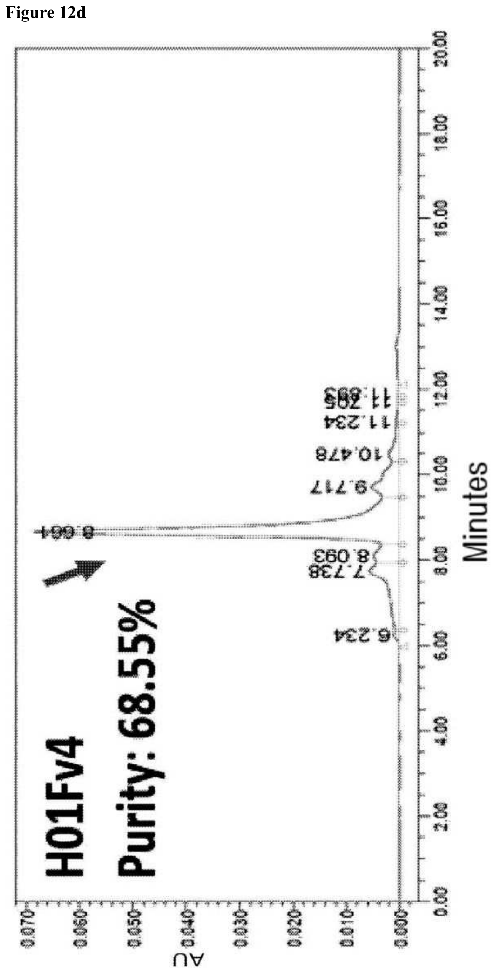

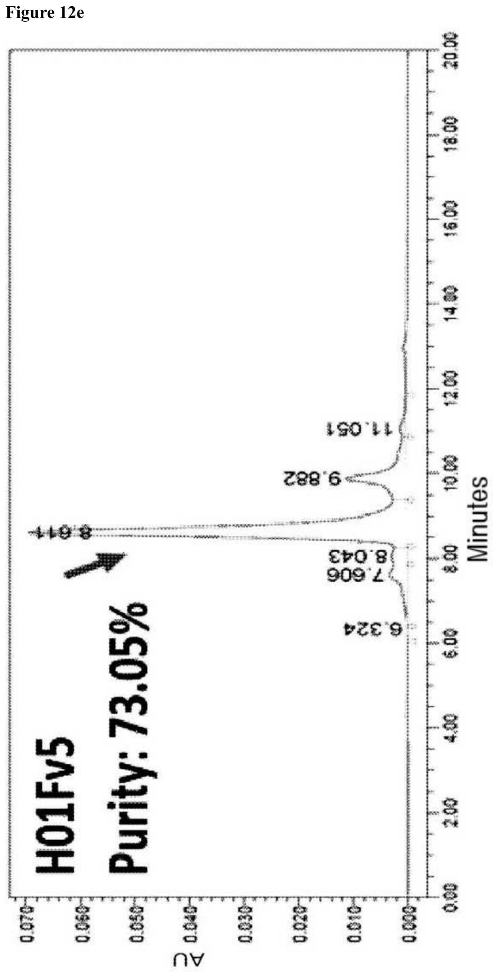

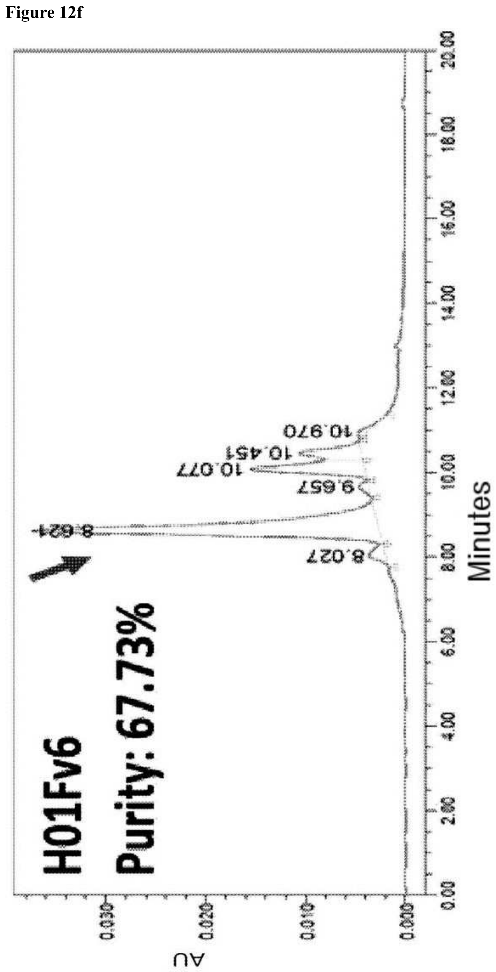

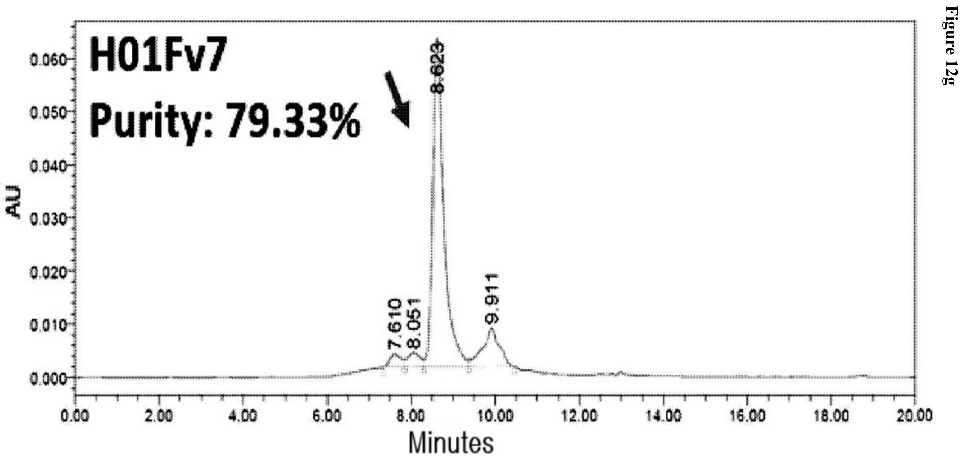

SDS-PAGE analysis under non-reducing conditions identified the main product at about 130 kDa (Figure 11). In addition, monomer purity was determined by SEC analysis (Figures 12a to 12f). In particular, H01Fv3 exists mostly in an unpaired form of about 65 kDa, and the monomer purity of fully assembled form was determined to be 14.66% (Figures 11 and 12c). It was found that H01Fv1, H01Fv2, H01Fv4, H01Fv5, H01Fv6, and H01Fv7 have monomer purities of 71.24%, 61.25%, 68.55%, 73.05%, 67.73%, and 79.33%, respectively.

The binding characteristics of H01Fv1, H01Fv2, H01Fv4, H01Fv5, H01Fv6, and H01Fv7 to the HER2 protein were analyzed using Octet Red96e (Sartorius), a bio-layer interferometry (BLI).

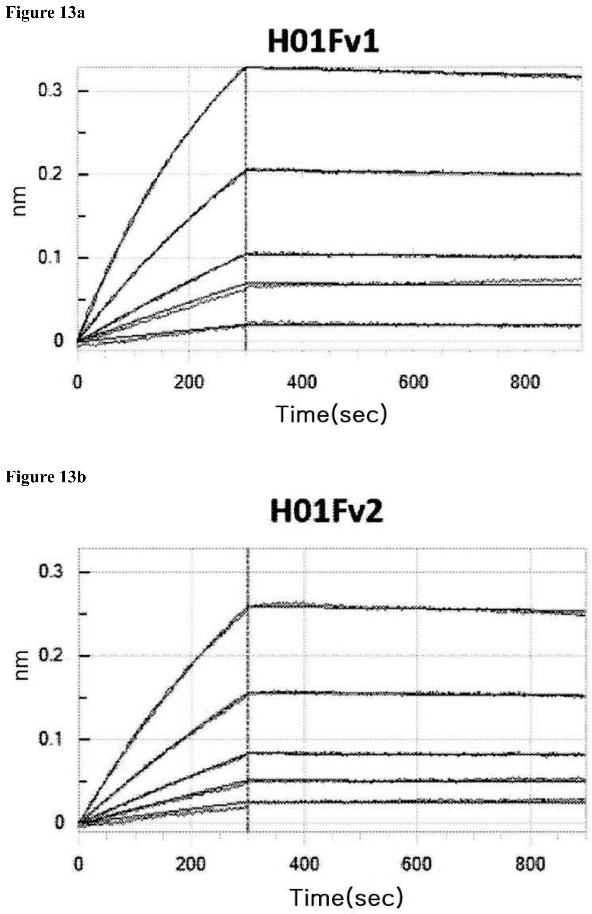

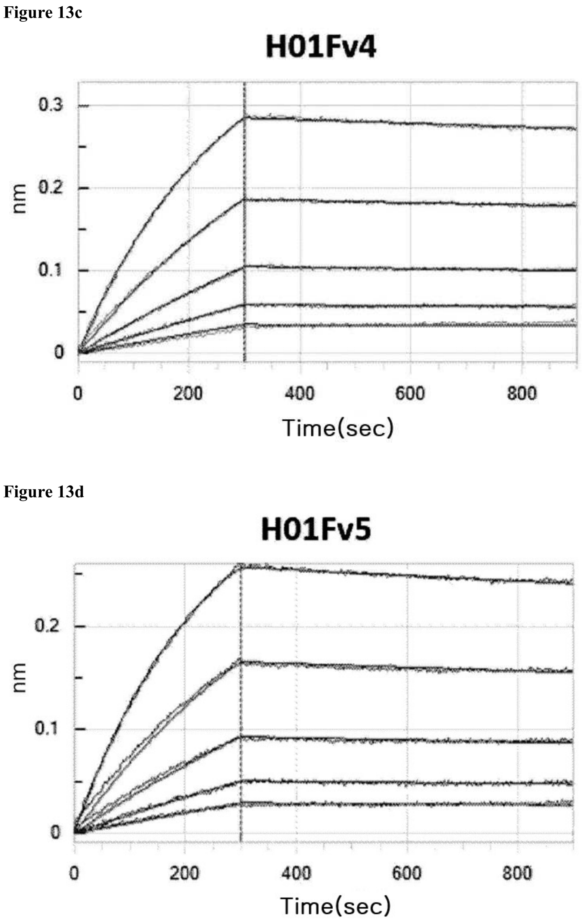

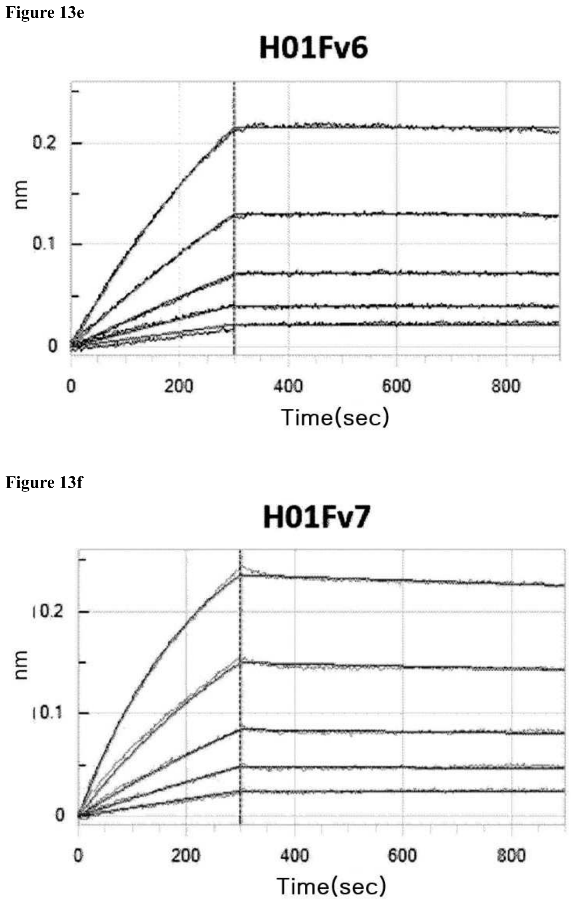

Specifically, the human HER2 recombinant protein (R&D systems, 1129-ER) was loaded onto the Anti-Penta-HIS (HIS1K) biosensor (Sartorius, 18-5120), and then each of H01Fv1, H01Fv2, H01Fv4, H01Fv5, H01Fv6, and H01Fv7 was added in a binding reaction and a dissociation reaction. The binding constants were calculated, and the results are shown in Figures 13a to 13f and Table 22.

[Table 22]

| Clone | KD(M) | kon(1/Ms) | kdis(1/s) |
|---|---|---|---|
| H01Fv1 | 2.44E-10 | 2.10E+05 | 5.11E-05 |
| H01Fv2 | 3.24E-10 | 1.17E+05 | 3.78E-05 |
| H01Fv3 | Not Determined | Not Determined | Not Determined |
| H01Fv4 | 3.26E-10 | 2.37E+05 | 7.71E-05 |
| H01Fv5 | 3.93E-10 | 2.73E+05 | 1.07E-04 |
| H01Fv6 | 4.87E-11 | 1.36E+05 | 6.61E-06 |
| H01Fv7 | 2.79E-10 | 2.76E+05 | 7.70E-05 |

**Example 6. Analysis of thermal stability**

Analysis of thermal stability of the antibody was performed using the PROTEIN THERMAL SHIFT™ Dye Kit (Applied biosystems, 4461146) according to the manufacturer's manual.

Briefly, 5 µL of reaction buffer and 2.5 µL of dye included in the kit were mixed with 5 µg of trastuzumab, pertuzumab, H01, or P01, and the final volume was adjusted to 20 µL by adding PBS. The mixture was reacted at 20 °C for 30 seconds in a C1000 thermal cycler (Bio-Rad, 1841000) equipped with a CFX96 optical reaction module (Bio-Rad, 1845096), respectively, and then the fluorescence intensity of the plate was measured while increasing the temperature from 20 °C to 95 °C at 1 °C/min. Thereafter, the reaction was stopped after reaction at 95 °C for 30 seconds. After the reaction, the median value of relative fluorescence unit (RFU) values was taken, and analysis of melting temperature ($T_m$) was performed.

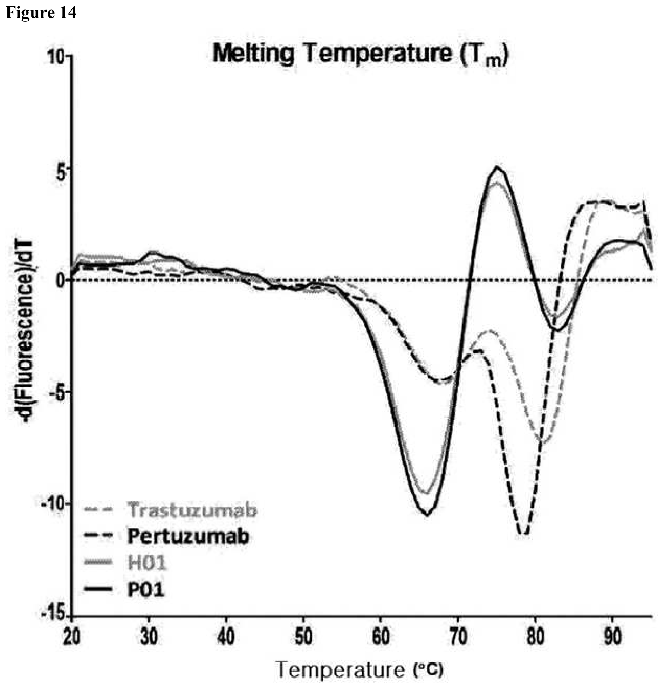

As a result, it was found that the $T_{m1}$ values for trastuzumab, pertuzumab, H01, and P01 were 68 °C, 68 °C, 66 °C, and 66 °C, respectively, and the $T_{m2}$ values for trastuzumab, pertuzumab, H01, and P01 were 81 °C, 79 °C, 83 °C, and 83 °C, respectively. Through the above results, it was found that the fusion proteins according to the present invention have Tm values similar to those of commercialized therapeutic antibodies (Figure 14 and Table 23).

[Table 23]

| Antibody name | $T_{m1}$ | $T_{m2}$ |
|---|---|---|
| Trastuzumab | 68 | 81 |
| Pertuzumab | 68 | 79 |
| H01 | 66 | 83 |
| P01 | 66 | 83 |

**Example 7. Identification of competitive binding of H01 and P01**

In order to identify whether H01 and P01 bind to different epitopes or compete for binding, Octet Red96e, a bio-layer interferometry (BLI), was used.

The human HER2 recombinant protein (R&D systems, 1129-ER) was loaded onto the Anti-Penta-HIS (HIS1K) biosensor, and then 100 nM human IgG1 (Bio X cell, BE0297), H01, or trastuzumab was each loaded and bound. Thereafter, 100 nM human IgG1, P01, or pertuzumab was each loaded and bound to determine the binding of each antibody.

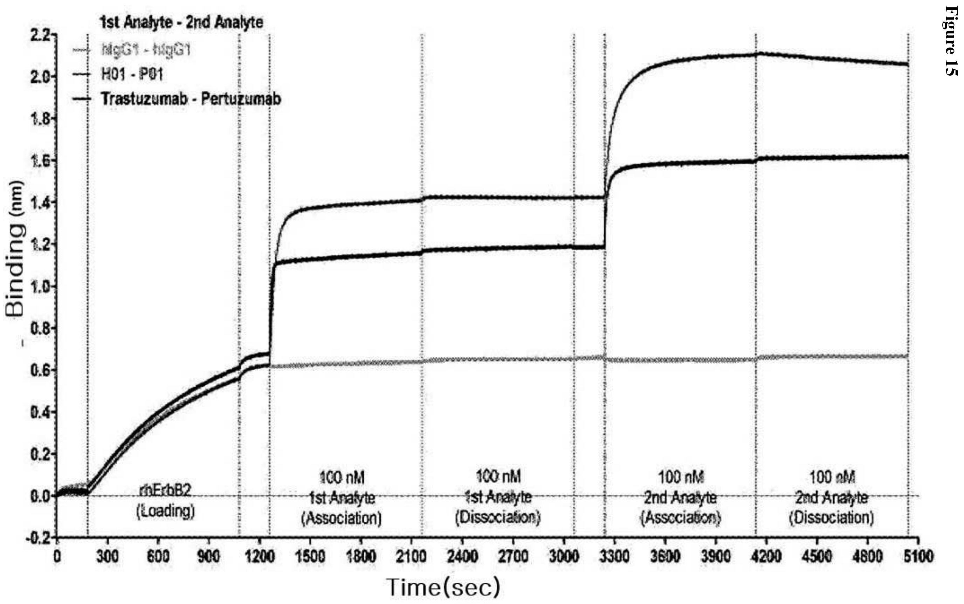

As a result, the binding signals (nm shift from baseline) measured at equilibrium after completion of HER2 recombinant protein loading were 0.620 nm, 0.625 nm, and 0.672 nm, respectively (Figure 15 and Table 24).

The first analyte and the second analyte were sequentially bound with association time and dissociation time of 900 seconds, and then the degree of binding was confirmed. When the human IgG1 antibody was sequentially bound, it did not bind to HER2. On the other hand, sequential binding of H01 and P01 and sequential binding of trastuzumab and pertuzumab confirmed binding to HER2, respectively.

The results indicate that the antibodies bind to different epitopes. The binding signals (nm shift from baseline) of H01 + P01 on the HER2-loaded sensor and the binding signals (nm shift from baseline) of trastuzumab + pertuzumab were measured to be 1.477 nm (y-axis value at 4140 s - y-axis value at 1260 s) and 0.923 nm (y-axis value at 4140 s - y-axis value at 1260 s), respectively. The binding signals (nm shift from baseline) tend to increase as more proteins bind to the surface of the biosensor. Therefore, this indicates that to the same amount of HER2, H01 and P01 cause a greater amount of antibody binding than trastuzumab and pertuzumab (Figure 15 and Table 24).

[Table 24]

| $1^{st}$ analyte − $2^{nd}$ analyte | Baseline(0 s) | HER2 Loading (1260 s) | $1^{st}$ Analyte binding(2160 s) | $2^{nd}$ Analyte binding(4140 s) |
|---|---|---|---|---|
| hIgG1 − hIgG1 | 0 | 0.620 | 0.635 | 0.647 |
| H01− P01 | 0 | 0.625 | 1.407 | 2.102 |
| Trastuzumab − Pertuzumab | 0 | 0.679 | 1.157 | 1.595 |

**Example 8. Quantification of Fc loads**

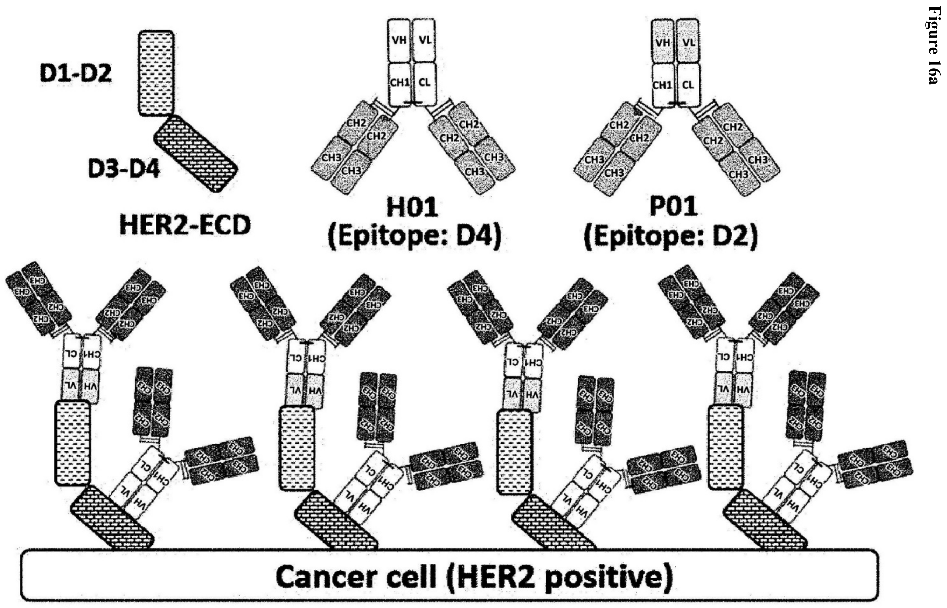

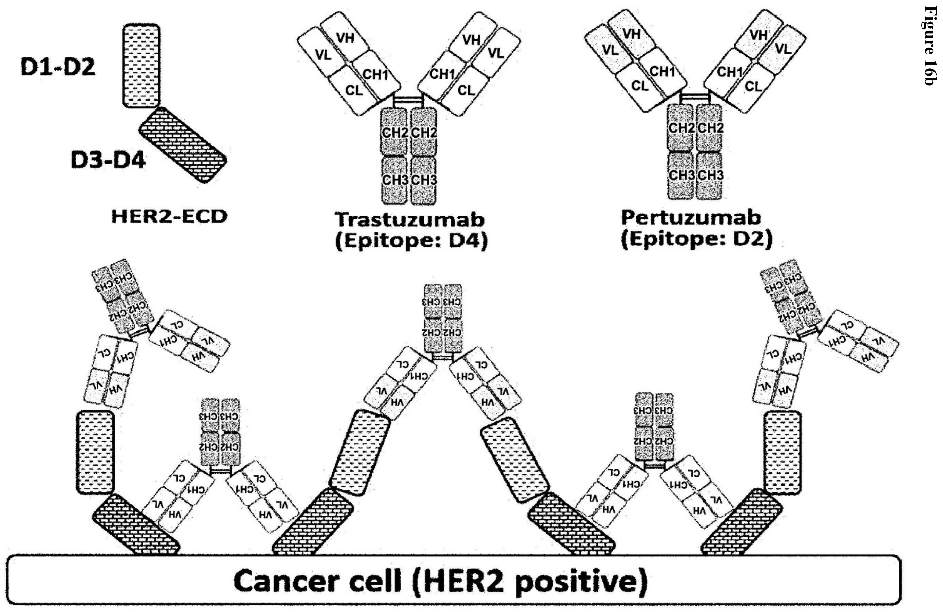

When H01 and P01 are treated in combination, a total of 16 Fc domains bind to four HER2 antigens present on the surface of cancer cells (Figure 16a). When trastuzumab and pertuzumab are treated in combination, eight Fc domains bind to four HER2 antigens present on the surface of cancer cells if the binding is in a monovalent mode, and fewer Fc domains can be bound if the binding is in a bivalent mode (Figure 16b). Therefore, the combination of H01 and P01 should result in increased Fc loads on the surface of HER2-positive cancer cells than the combination of trastuzumab and pertuzumab, and thereby should lead to a stronger effector function.

In order to confirm the above effect, Fc loads of the antibodies on the cell surface was quantified in the HER2-expressing cancer cell lines (NCI-N87 cells, BT474 cells, SK-OV-3 cells, SNU1 cells, and SNU5 cells). Here, all of the cancer cell lines were cultured in RPMI-1640 (containing 10% FBS) medium.

Specifically, the cancer cells were each inoculated into a 96-well plate, and then treated with the human IgG1 (Bio X cell, BE0297) (50 nM), trastuzumab (TRA) (50 nM), trastuzumab (50 nM) + pertuzumab (50 nM) (TRA + PER), H01 (50 nM), or H01 (50 nM) + P01 (50 nM), respectively, and then reacted at 4 °C for 30 minutes. Thereafter, the cells were treated with the Alexa 488 fluorescence-conjugated anti-human IgG Fcγ Fab antibody (Jackson ImmunoResearch, 109-547-008), and the antibody Fc domain bound to the cells was quantified using a flow cytometer (BD biosciences, FACSverse).

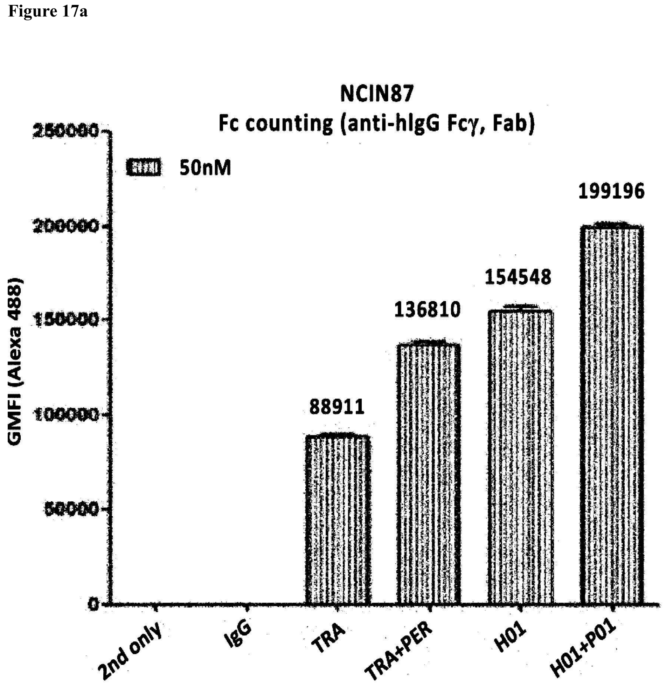

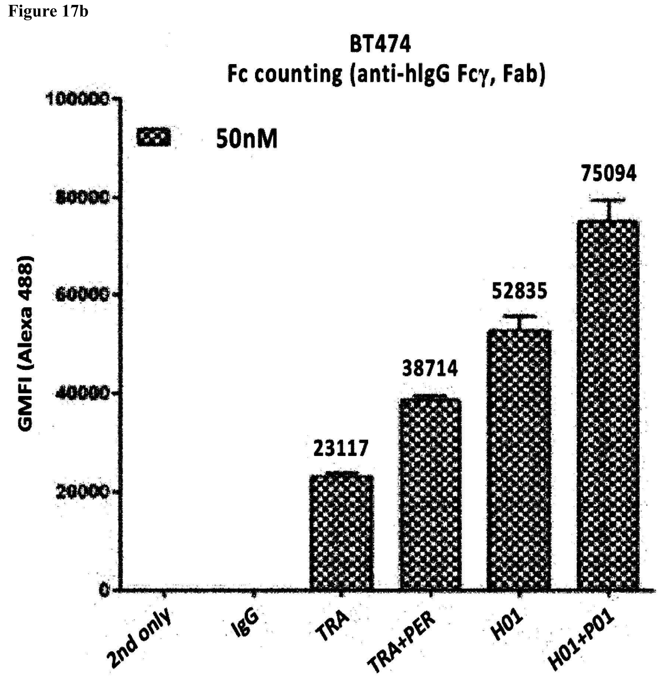

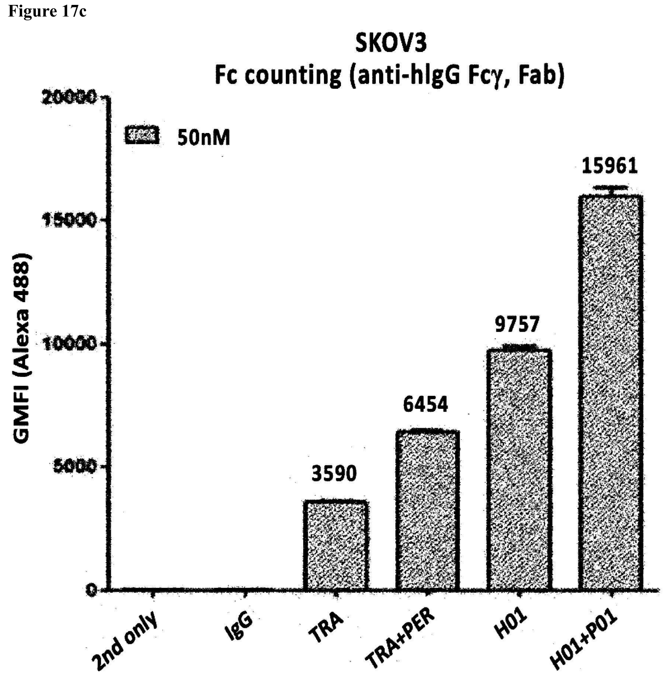

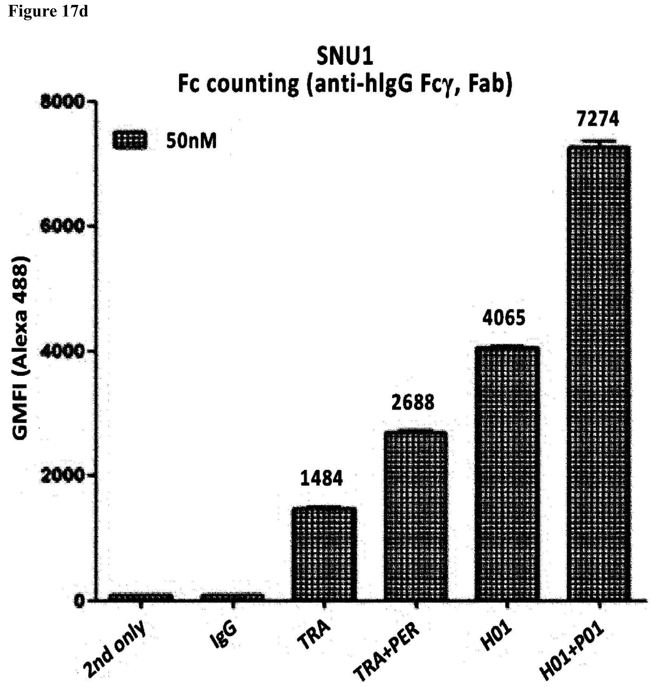

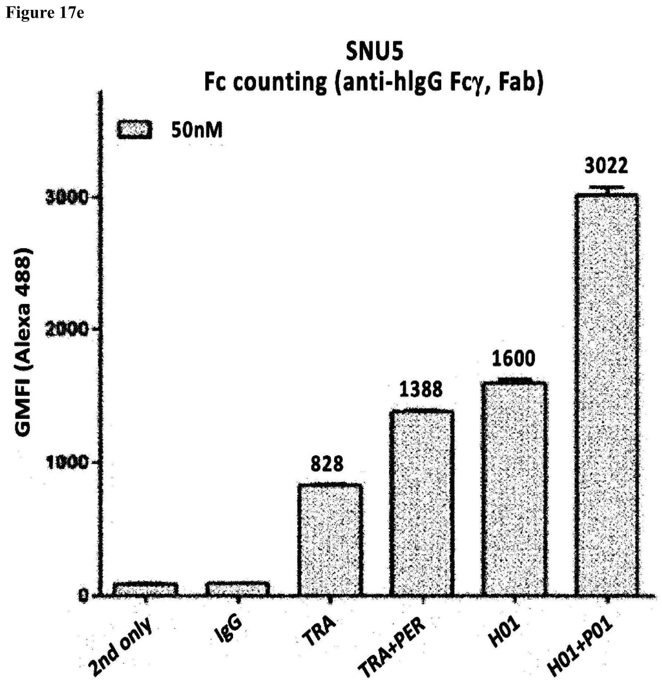

As a result, as shown in Figures 17a to 17e and Table 25, the fluorescence intensities of the group treated with H01 alone were higher compared to the group treated with TRA + PER in all cancer cell lines. In addition, compared to treatment of H01 alone, an increase in the Fc loads on the cancer cell surface was observed in the group treated with H01 + P01.

[Table 25]

| GMFI | NCI-N87 | BT474 | SK-OV-3 | SNU-1 | SNU-5 |
|---|---|---|---|---|---|
| 50 nM Trastuzumab | 88911 | 23117 | 3590 | 1484 | 828 |
| 50 nM Trastuzumab + 50 nM Pertuzumab | 136810 | 38714 | 6454 | 2688 | 1388 |
| 50 nM H01 | 154548 | 52835 | 9757 | 4065 | 1600 |
| 50 nM H01 + 50 nM P01 | 199196 | 75094 | 15961 | 7274 | 3022 |

In order to determine the saturation concentration for each treated group, the cells were treated with the antibodies at various concentrations (final concentrations of 20 nM, 50 nM, and 100 nM) under the same conditions as above, and then the Fc domain was quantified in the same manner as above.

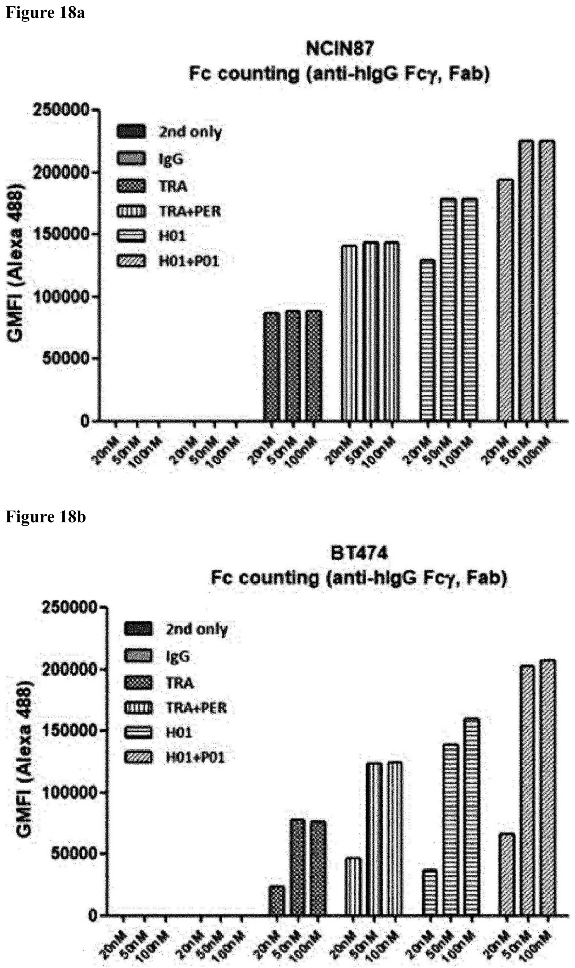

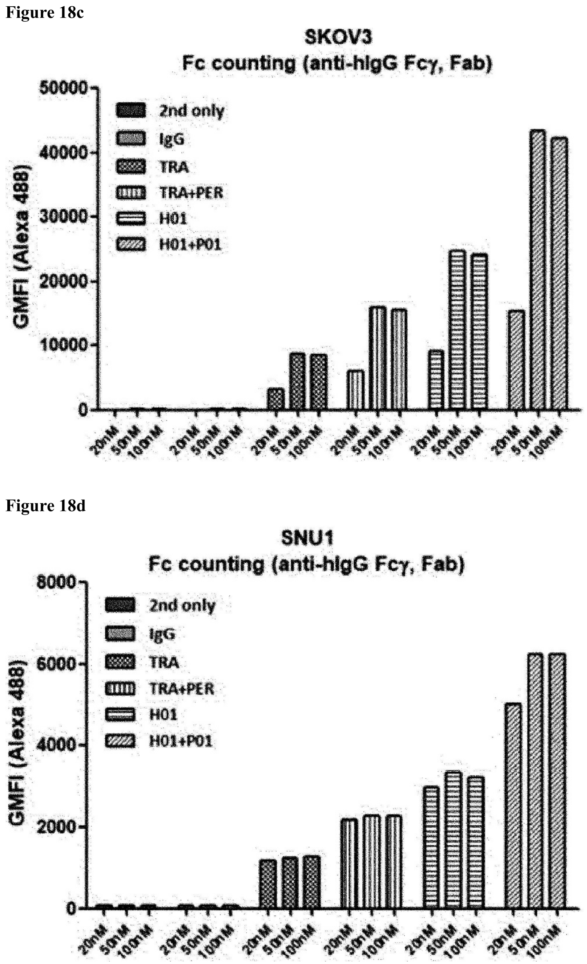

As a result, as shown in Figures 18a to 18e, the saturation concentration of each treated group was 50 nM. Through the above results, it was found that treatment of 50 nM H01 alone results in more Fc loads on the surface of the five cancer cell lines compared to treatment of 50 nM trastuzumab and 50 nM pertuzumab in combination.

**Example 9. Analysis of binding affinity of antibody to HER2**

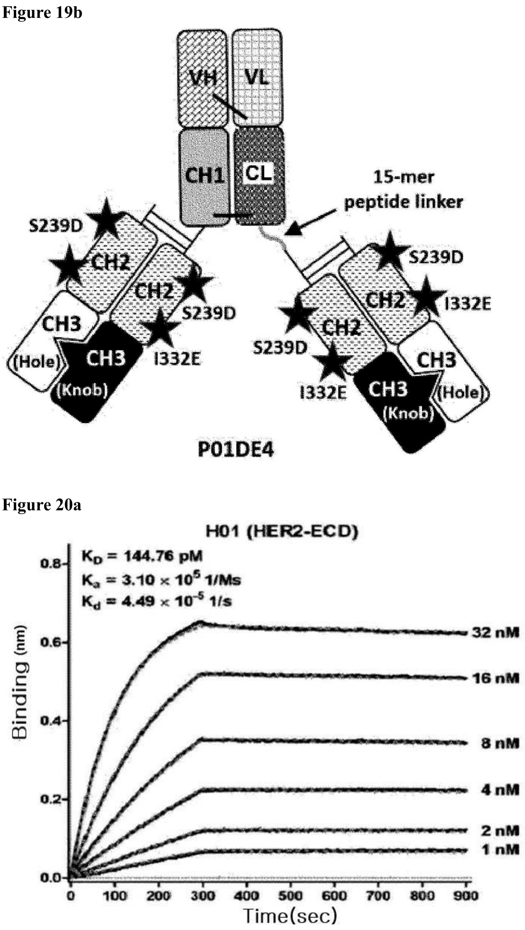

The S239D or 1332E mutation in the Fc domain of the antibody improves the affinity of the antibody for Fcγ receptors, which improve effector function (Greg A. Lazar et al., PNAS, 2006). Therefore, H01DE4 and P01DE4 were designed by introducing the S239D and I332E mutations in the Fc domains of H01 and P01, respectively (Figures 19a and 19b).

H01DE4 consists of the polypeptides of Fc-Hole-S239D-I332E (Table 26, SEQ ID NO: 42), TraH-G44C-Knob-S239D-I332E (Table 26, SEQ ID NO: 43), and TraL-Q100C-KnobS239D-I332E (Table 26, SEQ ID NO: 44), and was prepared by co-transfection of expression vectors loaded with polynucleotides encoding the polypeptides into the ExpiCHO-S™ cell line.

P01DE4 consists of the polypeptides of Fc-Hole-S239D-I332E (Tables 26 and 27, SEQ ID NO: 42), PerH-G44C-Knob-S239D-I332E (Tables 26 and 27, SEQ ID NO: 45), and

PerL-Q100C-Knob-S239D-I332E (Tables 26 and 27, SEQ ID NO: 46), and was prepared by co-transfection of expression vectors loaded with polynucleotides encoding the polypeptides into the ExpiCHO-S™ cell line.

[Table 26]

| Name | Sequence | SEQ ID NO |
|---|---|---|
| Fc-Hole-S239D-I332E | DKTHTCPPCPAPELLGGPDVFLFPPKPKDTLMISRTPEVTCVVVDVSHEDPEVKFNWYVDGV EVHNAKTKPREEQYNSTYRVVSVLTVLHQDWLNGKEYKCKVSNKALPAPEEKTISKAKGQPR EPQVCTLPPSREEMTKNQVSLSCAVKGFYPSDIAVEWESNGQPENNYKTTPPVLDSDGSFFL VSKLTVDKSRWQQGNVFSCSVMHEALHNHYTQKSLSLSPGK | 42 |
| TraH-G44C-Knob-S239D-I332E | EVQLVESGGGLVQPGGSLRLSCAASGFNIKDTYIHWVRQAPGKCLEWVARIYPTNGYTRYAD SVKGRFTISADTSKNTAYLQMNSLRAEDTAVYYCSRWGGDGFYAMDYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCDKTHTCPPCPAPELLGGPDVFLFPP KPKDTLMISRTPEVTCVVVDVSHEDPEVKFNWYVDGVEVHNAKTKPREEQYNSTYRVVSVLT VLHQDWLNGKEYKCKVSNKALPAPEEKTISKAKGQPREPQVYTLPPCREEMTKNQVSLWCLV KGFYPSDIAVEWESNGQPENNYKTTPPVLDSDGSFFLYSKLTVDKSRWQQGNVFSCSVMHEA LHNHYTQKSLSLSPGK | 43 |
| TraL-Q100C-Knob-S239D-I332E | DIQMTQSPSSLSASVGDRVTITCRASQDVNTAVAWYQQKPGKAPKLLIYSASFLYSGVPSRF SGSRSGTDFTLTISSLQPEDFATYYCQQHYTTPPTFGCGTKVEIKRTVAAPSVFIFPPSDEQ LKSGTASVVCLLNNFYPREAKVQWKVDNALQSGNSQESVTEQDSKDSTYSLSSTLTLSKADY EKHKVYACEVTHQGLSSPVTKSFNRGECGGGGSGGGGSGGGGSEPKSSDKTHTCPPCPAPEL LGGPDVFLFPPKPKDTLMISRTPEVTCVVVDVSHEDPEVKFNWYVDGVEVHNAKTKPREEQY NSTYRVVSVLTVLHQDWLNGKEYKCKVSNKALPAPEEKTISKAKGQPREPQVYTLPPCREEM TKNQVSLWCLVKGFYPSDIAVEWESNGQPENNYKTTPPVLDSDGSFFLYSKLTVDKSRWQQG NVFSCSVMHEALHNHYTQKSLSLSPGK | 41 |
| PerH-G44C-Knob-S239D-I332E | EVQLVESGGGLVQPGGSLRLSCAASGFTFTDYTMDWVRQAPGKCLEWVADVNPNSGGSIYNQ RFKGRFTLSVDRSKNTLYLQMNSLRAEDTAVYYCARNLGPSFYFDYWGQGTLVTVSSASTKG PSVFPLAPSSKSTSGGTAALGCLVKDYFPEPVTVSWNSGALTSGVHTFPAVLQSSGLYSLSS VVTVPSSSLGTQTYICNVNHKPSNTKVDKKVEPKSCDKTHTCPPCPAPELLGGPDVFLFPPK PKDTLMISRTPEVTCVVVDVSHEDPEVKFNWYVDGVEVHNAKTKPREEQYNSTYRVVSVLTV LHQDWLNGKEYKCKVSNKALPAPEEKTISKAKGQPREPQVYTLPPCREEMTKNQVSLWCLVK GFYPSDIAVEWESNGQPENNYKTTPPVLDSDGSFFLYSKLTVDKSRWQQGNVFSCSVMHEAL HNHYTQKSLSLSPGK | 45 |
| PerL-Q100C-Knob-S239D-I332E | DIQMTQSPSSLSASVGDRVTITCKASQDVSIGVAWYQQKPGKAPKLLIYSASYRYTGVPSRF SGSGSGTDFTLTISSLQPEDFATYYCQQYYIYPYTFGCGTKVEIKRTVAAPSVFIFPPSDEQ LKSGTASVVCLLNNFYPREAKVQWKVDNALQSGNSQESVTEQDSKDSTYSLSSTLTLSKADY EKHKVYACEVTHQGLSSPVTKSFNRGECGGGGSGGGGSGGGGSEPKSSDKTHTCPPCPAPEL LGGPDVFLFPPKPKDTLMISRTPEVTCVVVDVSHEDPEVKFNWYVDGVEVHNAKTKPREEQY NSTYRVVSVLTVLHQDWLNGKEYKCKVSNKALPAPEEKTISKAKGQPREPQVYTLPPCREEM TKNQVSLWCLVKGFYPSDIAVEWESNGQPENNYKTTPPVLDSDGSFFLYSKLTVDKSRWQQG NVFSCSVMHEALHNHYTQKSLSLSPGK | 46 |

[Table 27]

| Name | Sequence | SEQ ID NO |
|---|---|---|
| Fe-Hole-S239D-I332E | GACAAAACTCACACATCCCCACCGTGCCCAGCACCTGAACTCCTGGGGGGACCGGATGTCTT CCTCTTCCCCCCAAAACCCAAGGACACCCTCATGATCTCCCGGACCCCTGAGGTCACATGCG TGGTGGTGGACGTGAGCCACGAAGACCCTGAGGTCAAGTTCAACTGGTACGTGGACGGCGTG GAGGTGCATAATGCCAAGACAAAGCCGCGGGAGGAGCAGTACAACAGCACGTACCGTGTGGT CAGCGTCCTCACCGTCCTGCACCAGGACTGGCTGAATGGCAAGGAGTACAAGTGCAAGGTCT CCAACAAAGCCCTCCCAGCCCCCGAAGAGAAAACCATCTCCAAAGCCAAAGGGCAGCCCCGA GAACCACAGGTGTGCACCCTGCCCCCATCCCGGGAGGAGATGACCAAGAACCAGGTCAGCCT GAGCTGCGCGGTCAAAGGCTTCTATCCCAGCGACATCGCCGTGGAGTGGGAGAGCAATGGGC AGCCGGAGAACAACTACAAGACCACGCCTCCCGTGCTGGACTCCGACGGCTCCTTCTTCCTC GTGAGCAAGCTCACCGTGGACAAGAGCAGGTGGCAGCAGGGGAACGTCTTCTCATGCTCCGT GATGCATGAGGCTCTGCACAACCACTACACGCAGAAGAGCCTCTCCCTGTCTCCGGGTAAA | 179 |
| TraH-G4IC-Knob-S239D-I332E | GAAGTTCAACTTGTGGAGTCTGGAGGCGGTTTGGTACAACCTGGAGGTTCACTTCGTTTGTC ATGTTGCTGCCTCTGGGTTTAATATCAAGGATACTTATATTCATTGGGTTCGCCAGGCACCCG GAAAATGCCTGGAATGGGTGGGCCCGTATATATCCCACTAACGGTTACACTCGATATGCTGAC TCTGTAAAGGGTCGATTTACTATATCCCGCTGATACTAGCAAAAACACTCCCTACCTCCAGAT GAACAGTCTGCGCGCCGAGGATACAGCAGTTTATTATTGTTCCCGATGGGGAGGTGACGGGT TCTATGCCATGGACTACTGGGGTCAAGGGCACACTGGTAACCGTTTCTTCTGCTAGCACCAAA GGACCTAGTGTGTTTTTCCTCTTGCCCCTTCCTCAAAGTCTACCTCTGGGGGGACAGCCGCTCT GGGCTGCCTGGTCAAGGATTATTTCCCAGAGCCTGTCACTGTCAGTTGGAACTCTGGAGCCT TGACTTCTGGTGTTCATACATTTCCTGCTGTCCTTCAAAGCAGCGGCTTGTACTCATTGTCT TCTGTTGTGACAGTACCCTCAAGCAGCCTCGGCACTCAGACATACATCTGCAATGTCAACCA CAAACCCTCAAATACAAAGGTAGATAAAAAAGTCGAACCAAAGTCTTGTGACAAAACTCACA CGTGCCCACCGTGCCCAGCACCTGAACTCCTGGGGGGACCGGATGTCTTCCTCTTCCCCCCA AAACCCAAGGACACCCTCATGATCTCCCGGACCCCTGAGGTCACATGCCGTGGTGGTGGACGT GAGCCACGAAGACCCTGAGGTCAAGTTCAACTGGTACGTGGACGGCGTGGAGGTGCATAATG CCAAGACAAAGCCGCGGGAGGAGCAGTACAACAGCACGTACCGTGTGGTCAGCGTCCTCACC GTCCTGCACCAGGACTGGCTGAATGGCAAGGAGTACAAGTGCAAGGTCTCCAACAAAGCCCT CCCAGCCCCCGAAGAGAAAACCATCTCCAAAGCCAAAGGGCAGCCCCGAGAACCACAGGTGT ACACCCTGCCCCCATGCCCGGGAGGAGATGACCAAGAACCAGGTCAGCCTGTGGTGCCTGGTC AAAGGCTTCTATCCCAGCGACATCGCCGTGGAGTGGGAGAGCAATGGGCAGCCGGAGAACAA CTACAAGACCACGCCTCCCGTGCTGGACTCCGACGGCTCCTTCTTCCTCTACAGCAAGCTCA CCGTGGACAAGAGCAGGTGGCAGCAGGGGAACGTCTTCTCATGCTCCGTGATGCATGAGGCT CTGCACAACCACTACACGCAGAAGAGCCTCTCCCTGTCTCCGGGTAAA | 180 |
| TraL-Q100C-Knob-S239D-I332E | GACATTCAGATGACCCAATCCCCCTCTAGCCTTTCAGCCTCCGTAGGGGACAGAGTTACAAT AACCTGTAGGGCTAGTCACGACGTTAATACAGCAGTCGCATGGTACCAGCAGAAGCCCGGTA AGGCTCCTAAGCTTTTGATCTACAGTGCTTCTTTCCTGTATTCTGGTGTACCTAGCCGATTC TCAGGCTCTCGGAGTGGCACAGACTTCACTCTGACAATTTCAAGTCTCCAGCCAGAAGATTT TGCAACCTACTACTGCCAACAACACTACACTACTCCTCCAACCTTCGGATGTGGCACAAAAG TAGAGATTAAGCGTACCGTGGCAGCTCCCAGCGTTTTTATCTTTCCCCCATCCGACGAGCAG CTCAAGAGTGGCACTGCCTCTGTAGTTTGTTTGCTGAATAACTTCTATCCACGTGAAGCAAA AGTACAGTGGAAGGTCGATAATGCCCTTCAGAGCGGTAACAGCCAAGAAAGTGTTACCGAGC AAGATTCCAAAGATTCCACTTACAGTCTGTCCAGCACATTGACACTGAGTAAGGCTGATTAC GAAAACACAAGGTGTACGCATGCGAGGTGACACACCAAGGTCTTTCATCTCCTGTAACTAA GAGCTTTAACCGGGGAGAATGTGGTGGTGGGGGCAGCGGCGGCGGAGGTAGTGGAGGCGGCG GTAGTGAACCAAAGAGTAGTGACAAAACTCACACGTGCCCACCGTGCCCAGCACCTGAACTC CTGGGGGGACCGGATGTCTTCCTCTTCCCCCCAAAACCCAAGGACACCCTCATGATCTCCCG GACCCCTGAGGTCACATGCGTGGTGGTGGACGTGAGCCACGAAGACCCTGAGGTCAAGTTCA | 181 |

| | | |
|---|---|---|
| | ACTGGTACGTGGACGGCGTGGAGGTGCATAATGCCAAGACAAAGCCGCGGGAGGAGCAGTAC AACAGCACGTACCGTGTGGTCAGCGTCCTCACCGTCCTGCACCAGGACTGGCTGAATGGCAA GGAGTACAAGTGCAAGGTCTCCAACAAAGCCCTCCCAGCCCCCGAAGAGAAAACCATCTCCA AAGCCAAAGGGCAGCCCCGAGAACCACAGGTGTACACCCTGCCCCCATGCCGGGAGGAGATG ACCAAGAACCAGGTCAGCCTGTGGTGCCTGGTCAAAGGCTTCTATCCCAGCGACATCGCCGT GGAGTGGGAGAGCAATGGGCAGCCGGAGAACAACTACAAGACCACGCCTCCCGTGCTGGACT CCGACGGCTCCTTCTTCCTCTACAGCAAGCTCACCGTGGACAAGAGCAGGTGGCAGCAGGGG AACGTCTTCTCATGCTCCGTGATGCATGAGGCTCTGCACAACCACTACACGCAGAAGAGCCT CTCCCTGTCTCCGGGTAAA | |
| PerH-G44C-Knob-S239D-I332E | GAAGTACAGTTGGTGGAGTCTGGTGGAGGTCTTGTACAACCTGGCGGGAGTTTGCGGCTTTC CTGCGCTGCAAGCGGGTTTACCTTCACCGATTATACCATGGATTGGGTACGCCAAGCCCCTG GTAAGTGCCTTGAGTGGGTTGCCGATGTAAACCCTAATTCCGGAGGAAGTATCTATAATCAA CGCTTCAAGGGCCGATTCACTCTGAGTGTGGATCGAAGCAAGAACACCTTGTACTTGCAGAT GAATTCCTTGCGGGCTGAAGACACAGCCGTCTATTACTGCGCCCCGAAATTTGGGGGCCTTCAT TCTATTTTGACTATTGGGGTCAGGGAACTCTGGTAACTGTTTCAAGTGCTAGCACCAAAGGA CCTAGTGTTTTTCCTCTTGCCCCTTCCTCAAAGTCTACCTCTGGGGGGACAGCCGCTCTGGG CTGCCTGGTCAAGGATTATTTCCCAGAGCCTGTCACTGTCAGTTGGAACTCTGGAGCCTTGA CTTCTGGTGTTCATACATTTCCTGCTGTCCTTCAAAGCAGCGGCTTGTACTCATTGTCTTCT GTTGTGACAGTACCCTCAAGCAGCCTCGGCACTCAGACATACATCTGCAATGTCAACCACAA ACCCTCAAATACAAAGGTAGATAAAAAAGTCGAACCAAAGTCTTGTGACAAAACTCACACGT GCCCACCGTGCCCAGCACCTGAACTCCTGGGGGGACCGGATGTCTTCCTCTTCCCCCCAAAA CCCAAGGACACCCTCATGATCTCCCGGACCCCTGAGGTCACATGCGTGGTGGTGGACGTGAG CCACGAAGACCCTGAGGTCAAGTTCAACTGGTACGTGGACGGCGTGGAGGTGCATAATGCCA AGACAAAGCCGCGGGAGGAGCAGTACAACAGCACGTACCGTGTGGTCAGCGTCCTCACCGTC CTGCACCAGGACTGGCTGAATGGCAAGGAGTACAAGTGCAAGGTCTCCAACAAAGCCCTCCC AGCCCCCGAAGAGAAAACCATCTCCAAAGCCAAAGGGCAGCCCCGAGAACCACAGGTGTACA CCCTGCCCCCATGCCGGGAGGAGATGACCAAGAACCAGGTCAGCCTGTGGTGCCTGGTCAAA GGCTTCTATCCCAGCGACATCGCCGTGGAGTGGGAGAGCAATGGGCAGCCGGAGAACAACTA CAAGACCACGCCTCCCGTGCTGGACTCCGACGGCTCCTTCTTCCTCTACAGCAAGCTCACCG TGGACAAGAGCAGGTGGCAGCAGGGGAACGTCTTCTCATGCTCCGTGATGCATGAGGCTCTG CACAACCACTACACGCAGAAGAGCCTCTCCCTGTCTCCGGGTAAA | 482 |
| PerL-Q100C-Knob-S239D-I332E | GATATTCAAATGACCCAAAGTCCAAGTTCCCCTTTCAGCATCTGTCGGTGATCGAGTCACTAT AACTTGTAAGGCCAGCCAAGACGTTAGCATAGGAGTAGCATGGTACCAACAAAAGCCTGGGA AGGCTCCCAAACTTCTCATTTATTCTGCTTCCTACCGATATACTGGTGTCCCAAGTAGATTT TCTGGCAGCGGATCTGGAACTGATTTTACATTGACTATCAGCTCCCTCCAGCCTGAGGACTT CGCTACTTATTACTGCCAACAGTACTACATTTATCCCTATACATTCGGTTGTGGGACCAAAG TAGAGATCAAACGTACGGTGGCAGCTCCCAGCCGTTTTTTATCTTTCCCCCATCCGACGAGCAG CTCAAGAGTGGCACTGCCTCTGTAGTTTGTTTGCTGAATAACTTCTATCCACGTGAAGCAAA AGTACAGTGGAAGGTCGATAATGCCCTTCAGAGCGGTAACAGCCAAGAAAGTGTTACCGAGC AAGATTCCAAAGATTCCACTTACAGTCTGTCCAGCACATTGACACTGAGTAAGGCTGATTAC GAAAAACACAAGGTGTACGCATGCGAGGTGACACACCAAGGTCTTTCATCTCCTGTAACTAA GAGCTTTAACCGGGGAGAATGTGGTGGTGGGGGGCAGCGGGGGCGGAGGTAGTGGAGGCGGCG GTAGTGAACCAAAGAGTAGTGACAAAACTCACACGTGCCCACCGTGCCCAGCACCTGAACTC CTGGGGGGACCGGATGTCTTCCTCTTCCCCCCAAAACCCAAGGACACCCTCATGATCTCCCG GACCCCTGAGGTCACATGCGTGGTGGTGGACGTGAGCCACGAAGACCCTGAGGTCAAGTTCA ACTGGTACGTGGACGGCGTGGAGGTGCATAATGCCAAGACAAAGCCGCGGGAGGAGCAGTAC AACAGCACGTACCGTGTGGTCAGCGTCCTCACCGTCCTGCACCAGGACTGGCTGAATGGCAA GGAGTACAAGTGCAAGGTCTCCAACAAAGCCCTCCCAGCCCCCGAAGAGAAAACCATCTCCA AAGCCAAAGGGCAGCCCCGAGAACCACAGGTGTACACCCTGCCCCCATGCCGGGAGGAGATG ACCAAGAACCAGGTCAGCCTGTGGTGCCTGGTCAAAGGCTTCTATCCCAGCGACATCGCCGT | 483 |

| | GGAGTGGGAGAGCAATGGGCAGCCGGAGAACAACTACAAGACCACGCCTCCCGTGCTGGACT CCGACGGCTCCTTCTTCCTCTACAGCAAGCTCACCGTGGACAAGAGCAGGTGGCAGCAGGGG AACGTCTTCTCATGCTCCGTGATGCATGAGGCTCTGCACAACCACTACACGCAGAAGAGCCT CTCCCTGTCTCCGGGTAAA |
|---|---|

The binding characteristics of the antibody prepared as above were analyzed at 25 °C using Octet Red96e, a bio-layer interferometry (BLI). The buffer used for analysis was 10X Kinetics buffer (Sartorius, 18-1042) diluted in PBS (pH 7.4, Gibco, 10010), and the analysis plate was agitated at 1,000 rpm.

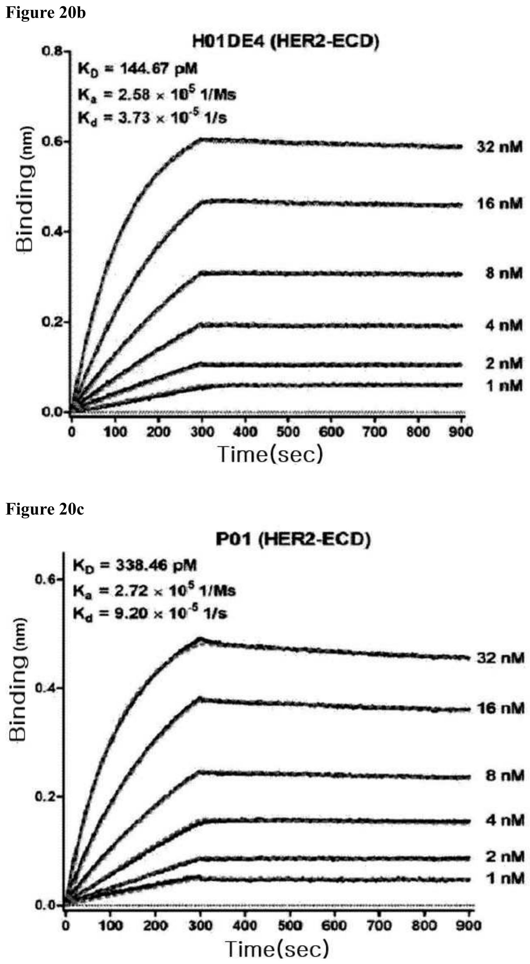

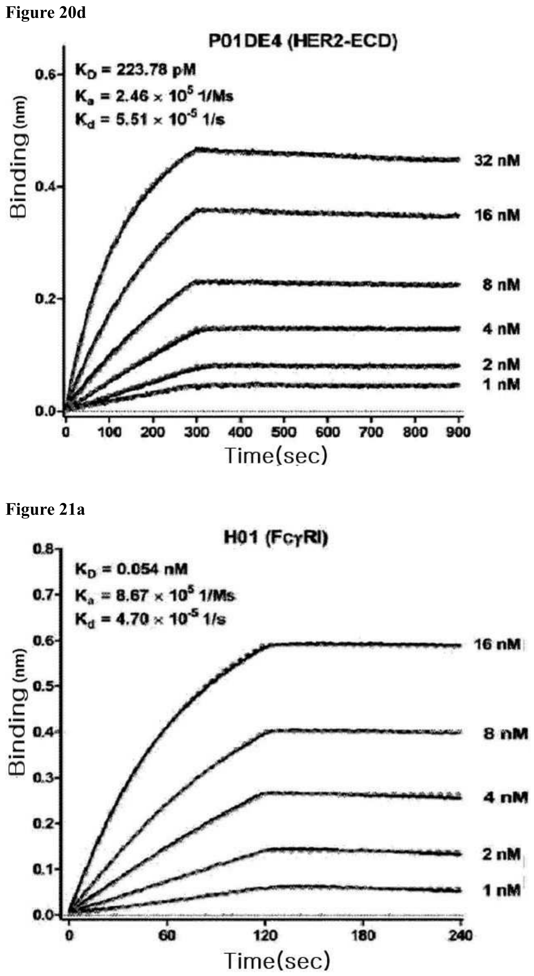

The human HER2 recombinant protein (R&D systems, 1129-ER) was loaded onto the Anti-Penta-HIS (HIS1K) biosensor (Sartorius, 18-5120). Thereafter, in order to measure the association rate constant ($K_a$), H01, H01DE4, P01, or P01DE4 at a concentration of 1 nM to 32 nM was loaded and allowed to bind to the loaded antigen for 300 seconds. Thereafter, the dissociation rate constant ($K_d$) was determined after 600 seconds of dissociation in Kinetics Buffer. The $K_a$ value and the $K_d$ value were analyzed using a 1:1 binding model in Octet analysis software (Sartorius), and the equilibrium dissociation constant ($K_D$) value was determined (Figures 20a to 20d and Table 28).

[Table 28]

| | $K_D$ (pM) | $K_a$ (1/Ms) | $K_d$ (1/s) |
|---|---|---|---|
| H01 | 144.76 | $3.10 \times 10^5$ | $4.49 \times 10^{-5}$ |
| H01DE4 | 144.67 | $2.58 \times 10^5$ | $3.73 \times 10^{-5}$ |
| P01 | 338.46 | $2.72 \times 10^5$ | $9.20 \times 10^{-5}$ |
| P01DE4 | 223.78 | $2.46 \times 10^5$ | $5.51 \times 10^{-5}$ |

**Example 10. Analysis of binding affinity of antibody to Fcγ receptors**

The binding constants of each antibody to the Fc gamma receptors at 25 °C were analyzed using Octet Red96e, a bio-layer interferometry (BLI).

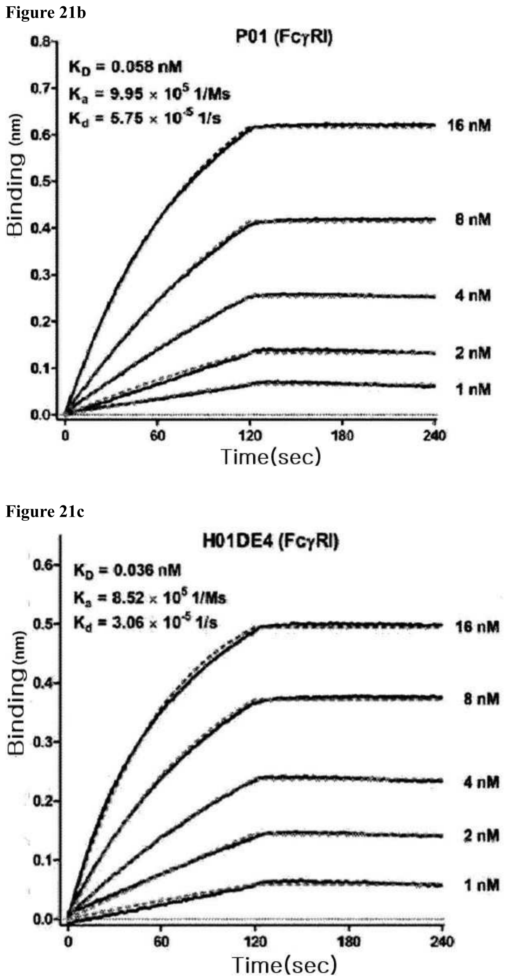

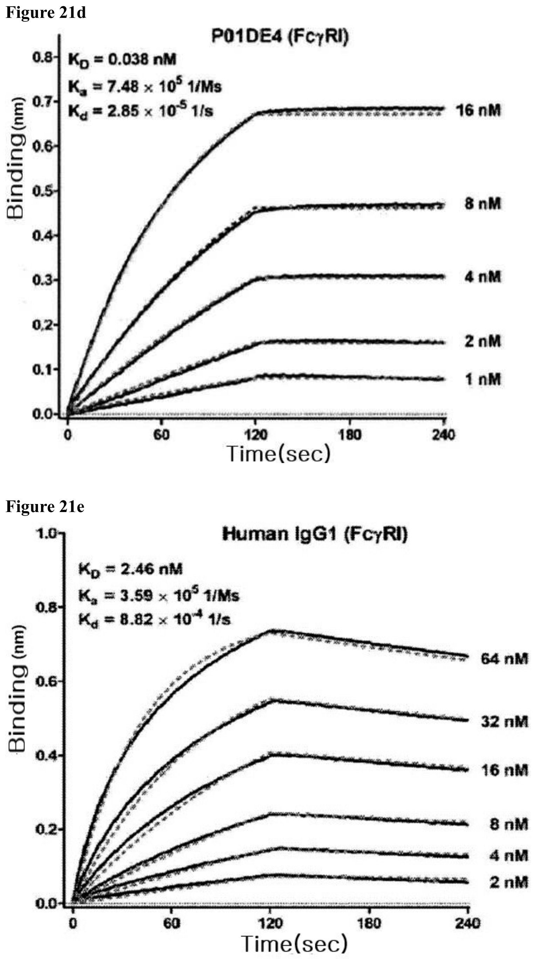

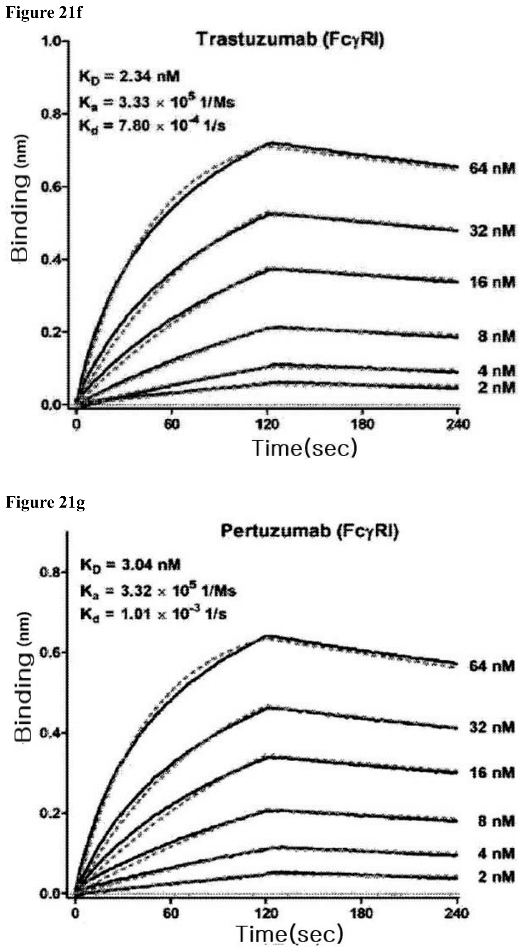

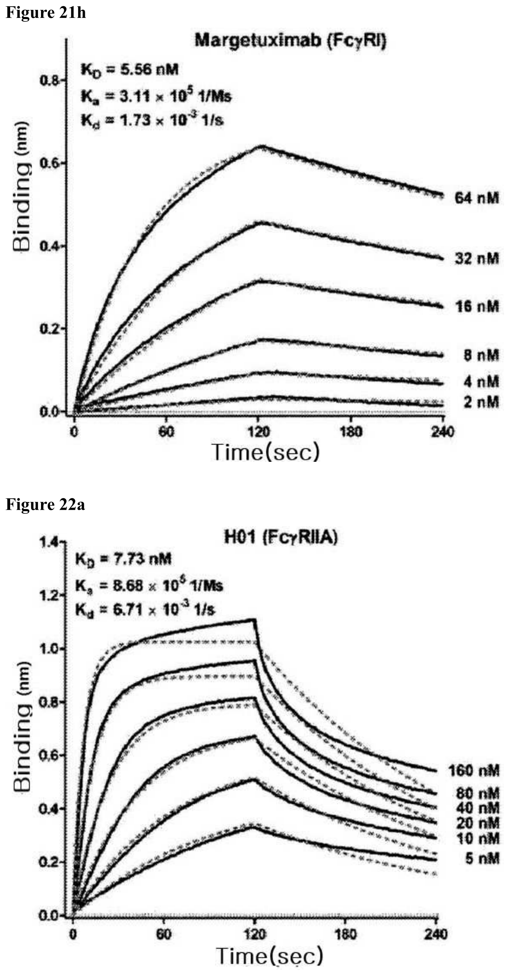

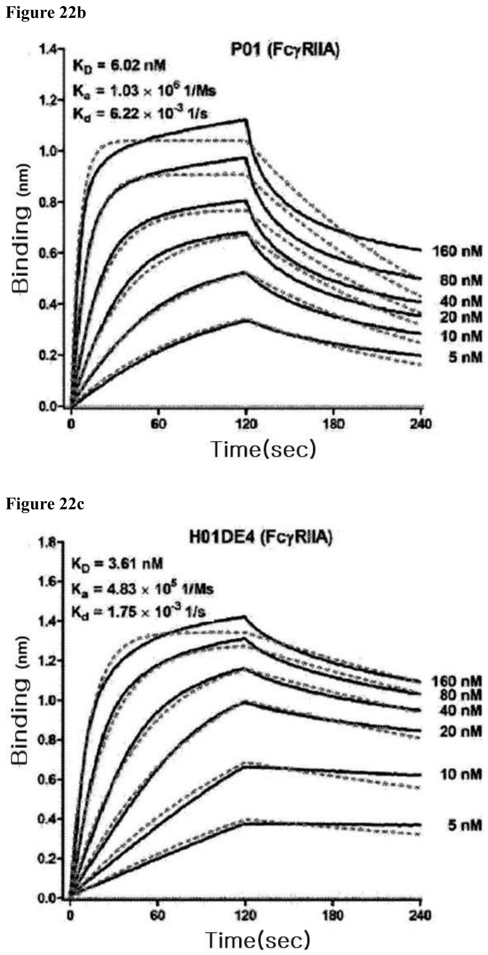

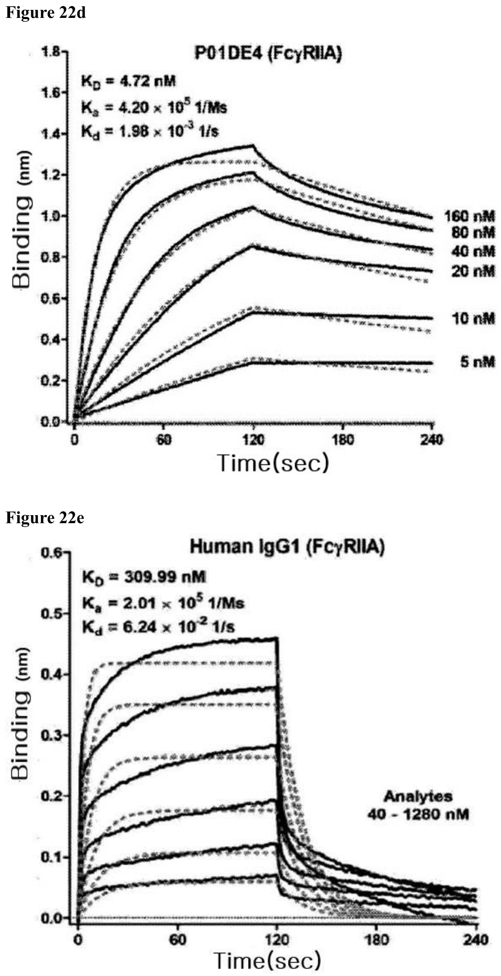

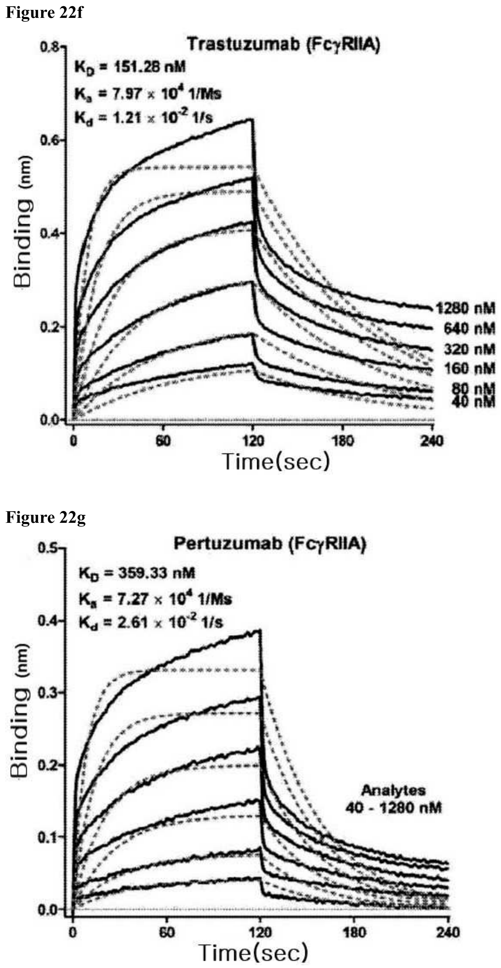

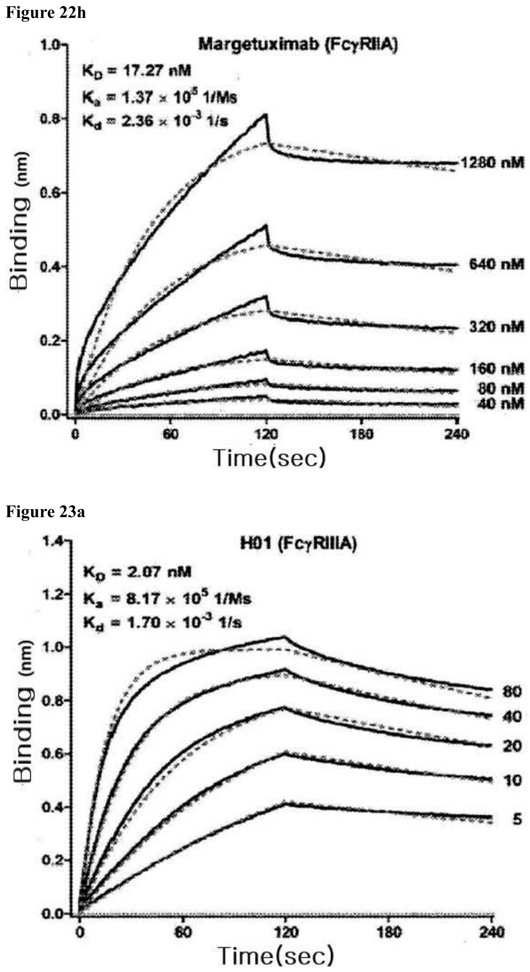

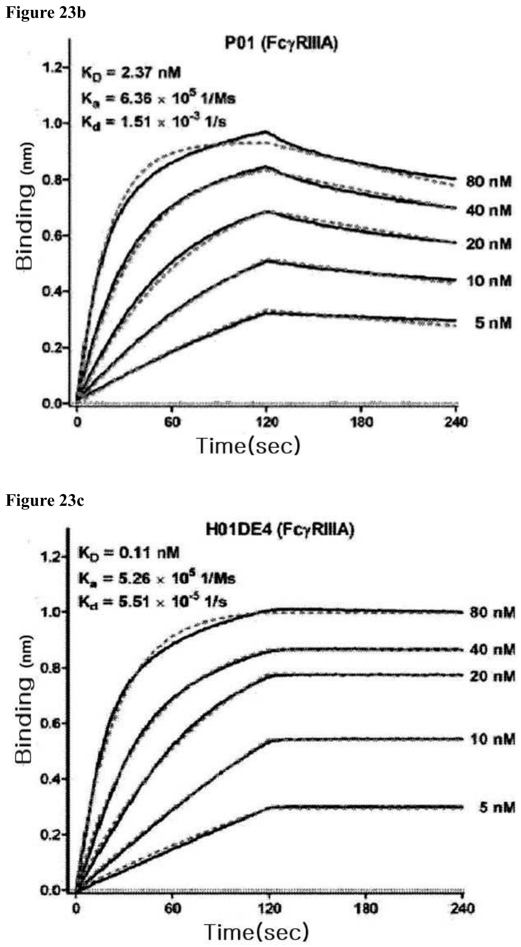

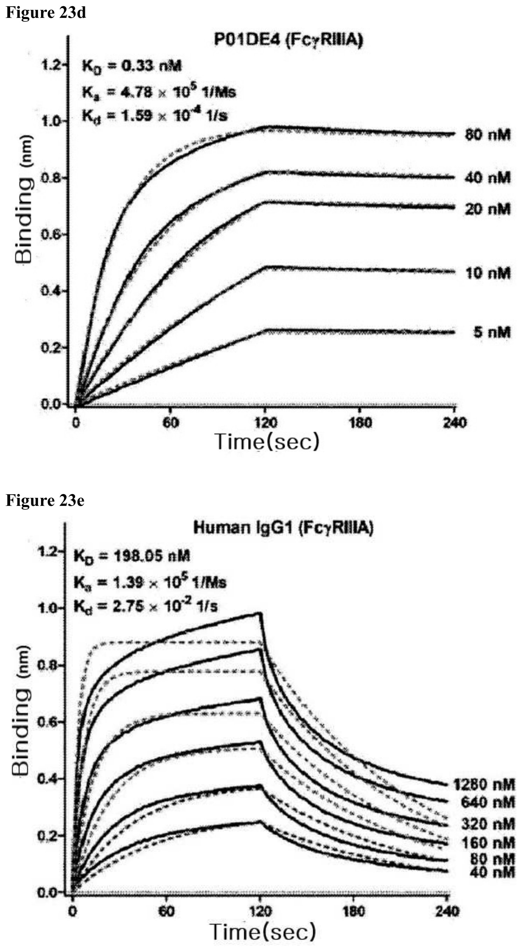

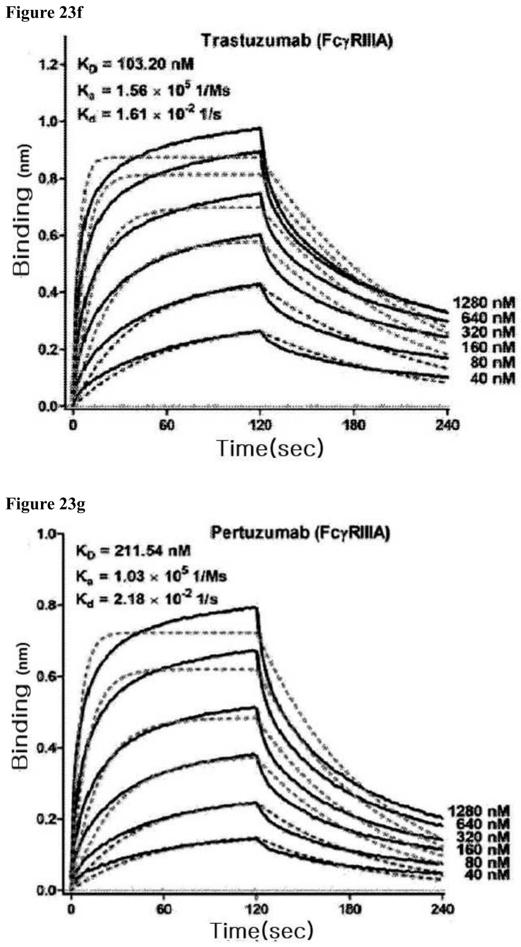

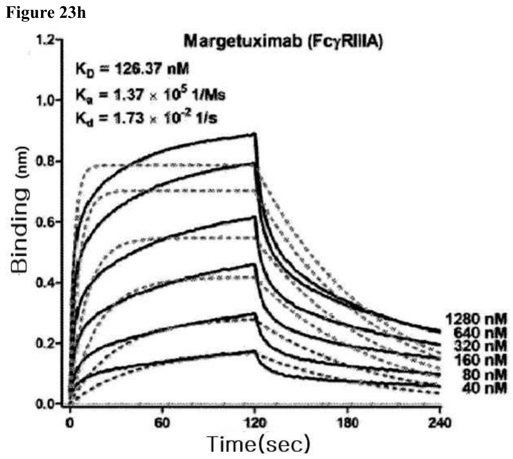

Specifically, each of the human FcγRI (R&D systems, 1257-FC), the human FcγIIA (R&D systems, 1330-CD), or the human FcγRIIIA (158V isoform, R&D systems, 4325-FC), which contains a His tag, was loaded onto the Anti-Penta-HIS (HIS1K) biosensor, and then each H01, P01, H01DE4, P01DE4, human IgG1 (Bio X cell, BE0297), trastuzumab, pertuzumab, or margetuximab was added in a binding reaction and a dissociation reaction in the same manner as above. The association rate constant ($K_a$), dissociation rate constant ($K_d$), and equilibrium dissociation constant ($K_D$) values were determined (Figures 21a to 21h, Figures 22a to 22h, Figures 23a to 23h, and Table 29).

[Table 29]

| Antigen | Fc γ RI(CD64) | | | Fc γ RIIA(CD32A, 131R) | | | Fc γ RIIIA(CD16A,176V) | | |
|---|---|---|---|---|---|---|---|---|---|
| Antibody | KD(nM) | Ka(1/Ms) | Kd(1/s) | KD(nM) | Ka(1/Ms) | Kd(1/s) | KD(nM) | Ka(1/Ms) | Kd(1/s) |
| H01 | 0.054 | 8.67E+05 | 4.70E-05 | 7.73 | 8.68E+05 | 6.71E-03 | 2.08 | 8.17E+05 | 1.70E-03 |
| P01 | 0.058 | 9.95E+05 | 5.75E-05 | 6.04 | 1.03E+06 | 6.22E-03 | 2.37 | 6.36E+05 | 1.51E-03 |
| H01DE4 | 0.036 | 8.52E+05 | 3.06E-05 | 3.62 | 4.83E+05 | 1.75E-03 | 0.10 | 5.26E+05 | 5.51E-05 |
| P01DE4 | 0.038 | 7.48E+05 | 2.85E-05 | 4.71 | 4.20E+05 | 1.98E-03 | 0.33 | 4.78E+05 | 1.59E-04 |
| Human IgG1 | 2.457 | 3.59E+05 | 8.82E-04 | 310.45 | 2.01E+05 | 6.24E-02 | 197.84 | 1.39E+05 | 2.75E-02 |
| Trastuzumab | 2.342 | 3.33E+05 | 7.80E-04 | 151.82 | 7.97E+04 | 1.21E-02 | 103.21 | 1.56E+05 | 1.61E-02 |
| Pertuzumab | 3.042 | 3.32E+05 | 1.01E-03 | 359.01 | 7.27E+04 | 2.61E-02 | 211.65 | 1.03E+05 | 2.18E-02 |
| Margetuximab | 5.563 | 3.11E+05 | 1.73E-03 | 17.23 | 1.37E+05 | 2.36E-03 | 126.28 | 1.37E+05 | 1.73E-02 |

### Example 11. Pharmacokinetic analysis in rats

H01, P01, trastuzumab, or pertuzumab was administered to 7 week old male Sprague-Dawley rats (Orientbio) at a concentration of 10 mg/kg per rat via the intravenous (i.v.) route. 5 minutes, 15 minutes, 30 minutes, 1 hour, 2 hours, 4 hours, 8 hours, 1 day, 2 days, 3 days, 7 days, 10 days, 14 days, 21 days, 28 days, 35 days, and 42 days after administration, blood was obtained, respectively. The serum was separated from the blood for analysis, and then the concentration of the fusion protein in the blood was measured by ELISA.

Specifically, a 96-well ELISA plate (Corning, 3590) was coated with the human HER2 recombinant protein (R&D systems, 1129-ER) and then reacted at 4 °C overnight. Each well of the plate coated as above was appropriately treated with the serum, and then allowed to bind to human HER2. After the binding reaction was completed, each well was treated with peroxidase-conjugated anti-human Fab goat antibody (Invitrogen, 31482), and then treated with a substrate to develop the color, and the absorbance was measured. Standard samples of H01, P01, trastuzumab, and pertuzumab were prepared, and the concentrations of the analytes at each time point were quantified based on a standard curve created from naive rat serum containing different concentrations of the antibody standards.

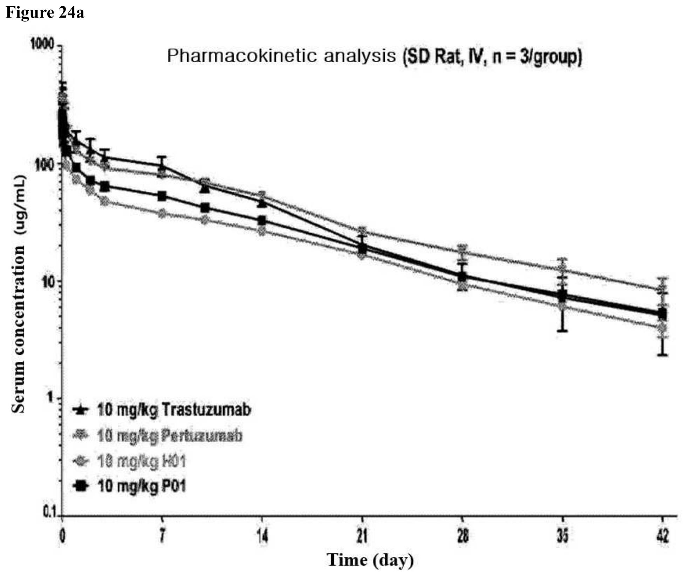

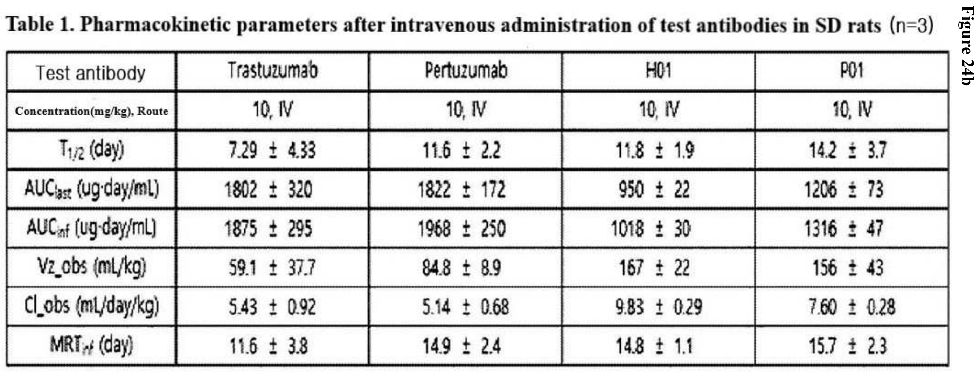

As shown in Figure 24a, Figure 24b, and Table 30, the half-lives of H01, P01, trastuzumab, and pertuzumab were determined to be about 11.8 days, 14.2 days, 7.3 days, and 11.6 days, respectively. Through the above results, the engineered novel antibodies, H01 and P01, were found to have similar levels of pharmacokinetic (PK) parameters to the parental humanized antibodies trastuzumab and pertuzumab.

[Table 30]

| | Trastuzumab | Pertuzumab | H01 | P01 |
|---|---|---|---|---|
| Dose (mg/kg), Route | 10, IV | 10, IV | 10, IV | 10, IV |
| $T_{1/2}$ (day) | 7.3 ± 4.3 | 11.6 ± 2.2 | 11.8 ± 1.9 | 14.2 ± 3.7 |
| $AUC_{last}$ (μg*day/mL) | 1802 ± 320 | 1822 ± 172 | 950 ± 22 | 1206 ± 73 |
| $AUC_{inf}$ (μg*day/mL) | 1875 ± 295 | 1968 ± 250 | 1018 ± 30 | 1316 ± 47 |
| Vz_obs (mL/kg) | 59.1 ± 37.7 | 84.8 ± 8.9 | 167 ± 22 | 156 ± 43 |
| Cl_obs (mL/day/kg) | 5.4 ± 0.9 | 5.1 ± 0.7 | 9.8 ± 0.3 | 7.6 ± 0.3 |
| $MRT_{inf}$ (day) | 11.6 ± 3.8 | 14.9 ± 2.4 | 14.8 ± 1.1 | 15.7 ± 2.3 |

### Example 12. Design, preparation and analysis of a novel antibody that recognizes two epitopes of HER2

In order to construct an antibody that recognizes two epitopes of HER2 protein, a biparatopic antibody HP501 was designed by connecting the V domains of trastuzumab and pertuzumab via a linker (Figure 25). In order to minimize the decrease in binding affinity due to interference between different V domains, linkers with variable lengths connecting the V domains were tested. At the same time, as an attempt to improve the physical integrity of the antibody, 16 variants were designed in which Cys substitution mutations capable of forming a disulfide bond in the V domain were introduced (Figure 26 and Table 31).

Based on Kabat numbering, mutations were introduced only at position 44 for the heavy chain and position 100 for the light chain. The VH linker and the VL linker having 6 amino acid residues were designated as VH-S-linker and VL-L-linker, respectively, and the VH linker and the VL linker having 13 amino acid residues were designated as VH-L-linker and VL-L-linker, respectively (Table 31).

[Table 31]

| Clone name | VH domain | | | VH domain | | |
|---|---|---|---|---|---|---|
| | VH1 44 (Upper) | Linker length | VH2 44 (Lower) | VL1 100 (Upper) | Linker length | VL2 100 (Lower) |
| HP501 | Gly (G) | 6 | Gly (G) | Gln (Q) | 6 | Gln (Q) |
| HP502 | Cys (C) | 6 | Gly (G) | Cys (C) | 6 | Gln (Q) |
| HP503 | Gly (G) | 6 | Cys (C) | Gln (Q) | 6 | Cys (C) |
| HP504 | Cys (C) | 6 | Cys (C) | Cys (C) | 6 | Cys (C) |
| HP505 | Gly (G) | 6 | Gly (G) | Gln (Q) | 13 | Gln (Q) |
| HP506 | Cys (C) | 6 | Gly (G) | Cys (C) | 13 | Gln (Q) |
| HP507 | Gly (G) | 6 | Cys (C) | Gln (Q) | 13 | Cys (C) |
| HP508 | Cys (C) | 6 | Cys (C) | Cys (C) | 13 | Cys (C) |
| HP509 | Gly (G) | 13 | Gly (G) | Gln (Q) | 6 | Gln (Q) |
| HP510 | Cys (C) | 13 | Gly (G) | Cys (C) | 6 | Gln (Q) |
| HP511 | Gly (G) | 13 | Cys (C) | Gln (Q) | 6 | Cys (C) |
| HP512 | Cys (C) | 13 | Cys (C) | Cys (C) | 6 | Cys (C) |
| HP513 | Gly (G) | 13 | Gly (G) | Gln (Q) | 13 | Gln (Q) |
| HP514 | Cys (C) | 13 | Gly (G) | Cys (C) | 13 | Gln (Q) |
| HP515 | Gly (G) | 13 | Cys (C) | Gln (Q) | 13 | Cys (C) |
| HP516 | Cys (C) | 13 | Cys (C) | Cys (C) | 13 | Cys (C) |

HP501 consists of the polypeptides of Fc-Hole (SEQ ID NO: 7), TH-S-PH-Knob (SEQ ID NO: 51), and TL-S-PL-Knob (SEQ ID NO: 59), and was prepared by co-transfection of expression vectors loaded with polynucleotides encoding the polypeptides into the ExpiCHO-S™ cell line (Tables 31 to 34). The antibody prepared as above was purified and analyzed in the same manner as in Example 1 above. In addition, HP502 to HP516 were expressed, purified, and analyzed in the same manner as HP501.

Tables 32 to 34 show the polypeptide sequences constituting the antibodies and the polynucleotide sequences encoding the same.

[Table 32]

| Name | Sequence | SEQ ID NO |
|------|----------|-----------|
| VH-S-Linker | ASTKGP | 47 |
| VH-L-Linker | ASTKGPSVFPLAP | 48 |
| VL-S-Linker | RTVAAP | 49 |
| VL-L-Linker | RTVAAPSVFIFPP | 50 |
| TH-S-PH-Knob | EVQLVESGGGLVQPGGSLRLSCAASGFNIKDTYIHWVRQAPGKGLEWVARIYPTNGYTR YADSVKGRFTISADTSKNTAYLQMNSLRAEDTAVYYCSRWGGDGFYAMDYWGQGTLVTV SSASTKGPEVQLVESGGGLVQPGGSLRLSCAASGFTFTDYTMDWVRQAPGKGLEWVADV KPNSGGSIYNQRFKGRFTLSVDRSKNTLYLQMNSLRAEDTAVYYCARNLGPSFYFDYWG QGTLVTVSSASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKKVEPKSCDKTHTC PPCPAPELLGGPSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPIEKTISKAKGQPR EPQVYTLPPCREEMTKNQVSLWCLVKGFYPSDIAVEWESNGQPENNYKTTPPVLDSDGS FFLYSKLTVDKSRWQQGNVFSCSVMHEALHNHYTQKSLSLSPGK | 51 |
| THC-S-PH-Knob | EVQLVESGGGLVQPGGSLRLSCAASGFNIKDTYIHWVRQAPGKCLEWVARIYPTNGYTR YADSVKGRFTISADTSKNTAYLQMNSLRAEDTAVYYCSRWGGDGFYAMDYWGQGTLVTV SSASTKGPEVQLVESGGGLVQPGGSLRLSCAASGFTFTDYTMDWVRQAPGKGLEWVADV KPNSGGSIYNQRFKGRFTLSVDRSKNTLYLQMNSLRAEDTAVYYCARNLGPSFYFDYWG QGTLVTVSSASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKKVEPKSCDKTHTC PPCPAPELLGGPSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPIEKTISKAKGQPR EPQVYTLPPCREEMTKNQVSLWCLVKGFYPSDIAVEWESNGQPENNYKTTPPVLDSDGS FFLYSKLTVDKSRWQQGNVFSCSVMHEALHNHYTQKSLSLSPGK | 52 |
| TH-S-PHC-Knob | EVQLVESGGGLVQPGGSLRLSCAASGFNIKDTYIHWVRQAPGKGLEWVARIYPTNGYTR YADSVKGRFTISADTSKNTAYLQMNSLRAEDTAVYYCSRWGGDGFYAMDYWGQGTLVTV SSASTKGPEVQLVESGGGLVQPGGSLRLSCAASGFTFTDYTMDWVRQAPGKCLEWVADV | 53 |

| | Sequence | |
|---|---|---|
| | NPNSGGSIYNQRFKGRFTLSVDRSKNTLYLQMNSLRAEDTAVYYCARNLGPSFYFDYWG QGTLVTVSSASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKKVEPKSCDKTHTC PPCPAPELLGGPSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPIEKTISKAKGQPR EPQVYTLPPCREEMTKNQVSLWCLVKGFYPSDIAVEWESNGQPENNYKTTPPVLDSDGS FFLYSKLTVDKSRWQQGNVFSCSVMHEALHNHYTQKSLSLSPGK | |
| THC-S-PHC-Knob | EVQLVESGGGLVQPGGSLRLSCAASGFNIKDTYIHWVRQAPGKCLEWVARIYPTNGYTR YADSVKGRFTISADTSKNTAYLQMNSLRAEDTAVYYCSRWGGDGFYAMDYWGQGTLVTV SSASTKGPEVQLVESGGGLVQPGGSLRLSCAASGFTFTDYTMDWVRQAPGKCLEWVADV NPNSGGSIYNQRFKGRFTLSVDRSKNTLYLQMNSLRAEDTAVYYCARNLGPSFYFDYWG QGTLVTVSSASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKKVEPKSCDKTHTC PPCPAPELLGGPSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPIEKTISKAKGQPR EPQVYTLPPCREEMTKNQVSLWCLVKGFYPSDIAVEWESNGQPENNYKTTPPVLDSDGS FFLYSKLTVDKSRWQQGNVFSCSVMHEALHNHYTQKSLSLSPGK | 54 |
| TH-L-PH-Knob | EVQLVESGGGLVQPGGSLRLSCAASGFNIKDTYIHWVRQAPGKGLEWVARIYPTNGYTR YADSVKGRFTISADTSKNTAYLQMNSLRAEDTAVYYCSRWGGDGFYAMDYWGQGTLVTV SSASTKGPSVFPLAPEVQLVESGGGLVQPGGSLRLSCAASGFTFTDYTMDWVRQAPGKG LEWVADVNPNSGGSIYNQRFKGRFTLSVDRSKNTLYLQMNSLRAEDTAVYYCARNLGPS FYFDYWGQGTLVTVSSASTKGPSVFPLAPSSKSTSGGTAALGCLVKDYFPEPVTVSWNS GALTSGVHTFPAVLQSSGLYSLSSVVTVPSSSLGTQTYICNVNHKPSNTKVDKKVEPKS CDKTHTCPPCPAPELLGGPSVFLFPPKPKDTLMISRTPEVTCVVVDVSHEDPEVKFNWY VDGVEVHNAKTKPREEQYNSTYRVVSVLTVLHQDWLNGKEYKCKVSNKALPAPIEKTIS KAKGQPREPQVYTLPPCREEMTKNQVSLWCLVKGFYPSDIAVEWESNGQPENNYKTTPP VLDSDGSFFLYSKLTVDKSRWQQGNVFSCSVMHEALHNHYTQKSLSLSPGK | 55 |
| THC-L-PH-Knob | EVQLVESGGGLVQPGGSLRLSCAASGFNIKDTYIHWVRQAPGKCLEWVARIYPTNGYTR YADSVKGRFTISADTSKNTAYLQMNSLRAEDTAVYYCSRWGGDGFYAMDYWGQGTLVTV | 56 |

| | | |
|---|---|---|
| | SSASTKGPSVFPLAPEVQLVESGGGLVQPGGSLRLSCAASGFTFTDYTMDWVRQAPGKGLEWVADVNPNSGGSIYNQRFKGRFTLSVDRSKNTLYLQMNSLRAEDTAVYYCARNLGPSFYFDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | |
| TH-L-PHC-Knob | EVQLVESGGGLVQPGGSLRLSCAASGFNIKDTYIHWVRQAPGKGLEWVARIYPTNGYTRYADSVKGRFTISADTSKNTAYLQMNSLRAEDTAVYYCSRWGGDGFYAMDYWGQGTLVTVSSASTKGPSVFPLAPEVQLVESGGGLVQPGGSLRLSCAASGFTFTDYTMDWVRQAPGKCLEWVADVNPNSGGSIYNQRFKGRFTLSVDRSKNTLYLQMNSLRAEDTAVYYCARNLGPSFYFDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 57 |
| THC-L-PHC-Knob | EVQLVESGGGLVQPGGSLRLSCAASGFNIKDTYIHWVRQAPGKCLEWVARIYPTNGYTRYADSVKGRFTISADTSKNTAYLQMNSLRAEDTAVYYCSRWGGDGFYAMDYWGQGTLVTVSSASTKGPSVFPLAPEVQLVESGGGLVQPGGSLRLSCAASGFTFTDYTMDWVRQAPGKCLEWVADVNPNSGGSIYNQRFKGRFTLSVDRSKNTLYLQMNSLRAEDTAVYYCARNLGPSFYFDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 58 |
| TL-S-PL-Knob | DIQMTQSPSSLSASVGDRVTITCRASQDVNTAVAWYQQKPGKAPKLLIYSASFLYSGVP | 59 |

| | | |
|---|---|---|
| | SRFSGSRSGTDFTLTISSLQPEDFATYYCQQHYTTPPTFGQGTKVEIKRTVAAPDIQMT QSPSSLSASVGDRVTITCKASQDVSIGVAWYQQKPGKAPKLLIYSASYRYTGVPSRFSG SGSGTDFTLTISSLQPEDFATYYCQQYYIYPYTFGQGTKVEIKRTVAAPSVFIFPPSDE QLKSGTASVVCLLNNFYPREAKVQWKVDNALQSGNSQESVTEQDSKDSTYSLSSTLTLS KADYEKHKVYACEVTHQGLSSPVTKSFNRGECGGGGSGGGGSGGGGSEPKSSDKTHTCP PCPAPELLGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKAKGQPRE PQVYTLPPCREEMTKNQVSLWCLVKGFYPSDIAVEWESNGQPENNYKTTPPVLDSDGSF FLYSKLTVDKSRWQQGNVFSCSVMHEALHNHYTQKSLSLSPGK | |
| TLC-S-PL-Knob | DIQMTQSPSSLSASVGDRVTITCRASQDVNTAVAWYQQKPGKAPKLLIYSASFLYSGVP SRFSGSRSGTDFTLTISSLQPEDFATYYCQQHYTTPPTFGCGTKVEIKRTVAAPDIQMT QSPSSLSASVGDRVTITCKASQDVSIGVAWYQQKPGKAPKLLIYSASYRYTGVPSRFSG SGSGTDFTLTISSLQPEDFATYYCQQYYIYPYTFGQGTKVEIKRTVAAPSVFIFPPSDE QLKSGTASVVCLLNNFYPREAKVQWKVDNALQSGNSQESVTEQDSKDSTYSLSSTLTLS KADYEKHKVYACEVTHQGLSSPVTKSFNRGECGGGGSGGGGSGGGGSEPKSSDKTHTCP PCPAPELLGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKAKGQPRE PQVYTLPPCREEMTKNQVSLWCLVKGFYPSDIAVEWESNGQPENNYKTTPPVLDSDGSF FLYSKLTVDKSRWQQGNVFSCSVMHEALHNHYTQKSLSLSPGK | 60 |
| TL-S-PLC-Knob | DIQMTQSPSSLSASVGDRVTITCRASQDVNTAVAWYQQKPGKAPKLLIYSASFLYSGVP SRFSGSRSGTDFTLTISSLQPEDFATYYCQQHYTTPPTFGQGTKVEIKRTVAAPDIQMT QSPSSLSASVGDRVTITCKASQDVSIGVAWYQQKPGKAPKLLIYSASYRYTGVPSRFSG SGSGTDFTLTISSLQPEDFATYYCQQYYIYPYTFGCGTKVEIKRTVAAPSVFIFPPSDE QLKSGTASVVCLLNNFYPREAKVQWKVDNALQSGNSQESVTEQDSKDSTYSLSSTLTLS KADYEKHKVYACEVTHQGLSSPVTKSFNRGECGGGGSGGGGSGGGGSEPKSSDKTHTCP PCPAPELLGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKAKGQPRE PQVYTLPPCREEMTKNQVSLWCLVKGFYPSDIAVEWESNGQPENNYKTTPPVLDSDGSF FLYSKLTVDKSRWQQGNVFSCSVMHEALHNHYTQKSLSLSPGK | 61 |

| TLC-S-PLC-Knob | DIQMTQSPSSLSASVGDRVTITCRASQDVNTAVAWYQQKPGKAPKLLIYSASFLYSGVP SRFSGSRSGTDFTLTISSLQPEDFATYYCQQHYTTPPTFGCGTKVEIKRTVAAPDIQMT QSPSSLSASVGDRVTITCKASQDVSIGVAWYQQKPGKAPKLLIYSASYRYTGVPSRFSG SGSGTDFTLTISSLQPEDFATYYCQQYYIYPYTFGCGTKVEIKRTVAAPSVFIFPPSDE QLKSGTASVVCLLNNFYPREAKVQWKVDNALQSGNSQESVTEQDSKDSTYSLSSTLTLS KADYEKHKVYACEVTHQGLSSPVTKSFNRGECGGGGSGGGGSGGGGSEPKSSDKTHTCP PCPAPELLGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKAKGQPRE PQVYTLPPCREEMTKNQVSLWCLVKGFYPSDIAVEWESNGQPENNYKTTPPVLDSDGSF FLYSKLTVDKSRWQQGNVFSCSVMHEALHNHYTQKSLSLSPGK | 62 |
|---|---|---|
| TL-L-PL-Knob | DIQMTQSPSSLSASVGDRVTITCRASQDVNTAVAWYQQKPGKAPKLLIYSASFLYSGVP SRFSGSRSGTDFTLTISSLQPEDFATYYCQQHYTTPPTFGQGTKVEIKRTVAAPSVFIF PPDIQMTQSPSSLSASVGDRVTITCKASQDVSIGVAWYQQKPGKAPKLLIYSASYRYTG VPSRFSGSGSGTDFTLTISSLQPEDFATYYCQQYYIYPYTFGQGTKVEIKRTVAAPSVF IFPPSDEQLKSGTASVVCLLNNFYPREAKVQWKVDNALQSGNSQESVTEQDSKDSTYSL SSTLTLSKADYEKHKVYACEVTHQGLSSPVTKSFNRGECGGGGSGGGGSGGGGSEPKSS DKTHTCPPCPAPELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTISK AKGQPREPQVYTLPPCREEMTKNQVSLWCLVKGFYPSDIAVEWESNGQPENNYKTTPPV LDSDGSFFLYSKLTVDKSRWQQGNVFSCSVMHEALHNHYTQKSLSLSPGK | 63 |
| TLC-L-PL-Knob | DIQMTQSPSSLSASVGDRVTITCRASQDVNTAVAWYQQKPGKAPKLLIYSASFLYSGVP SRFSGSRSGTDFTLTISSLQPEDFATYYCQQHYTTPPTFGCGTKVEIKRTVAAPSVFIF PPDIQMTQSPSSLSASVGDRVTITCKASQDVSIGVAWYQQKPGKAPKLLIYSASYRYTG VPSRFSGSGSGTDFTLTISSLQPEDFATYYCQQYYIYPYTFGQGTKVEIKRTVAAPSVF IFPPSDEQLKSGTASVVCLLNNFYPREAKVQWKVDNALQSGNSQESVTEQDSKDSTYSL SSTLTLSKADYEKHKVYACEVTHQGLSSPVTKSFNRGECGGGGSGGGGSGGGGSEPKSS DKTHTCPPCPAPELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTISK AKGQPREPQVYTLPPCREEMTKNQVSLWCLVKGFYPSDIAVEWESNGQPENNYKTTPPV | 64 |

| | | |
|---|---|---|
| | LDSDGSFFLYSKLTVDKSRWQQGNVFSCSVMHEALHNHYTQKSLSLSPGK | |
| TL-L-PLC-Knob | DIQMTQSPSSLSASVGDRVTITCRASQDVNTAVAWYQQKPGKAPKLLIYSASFLYSGVP SRFSGSRSGTDFTLTISSLQPEDFATYYCQQHYTTPPTFGCGTKVEIKRTVAAPSVFIF PPDIQMTQSPSSLSASVGDRVTITCKASQDVSIGVAWYQQKPGKAPKLLIYSASYRYTG VPSRFSGSGSGTDFTLTISSLQPEDFATYYCQQYYIYPYTFGQGTKVEIKRTVAAPSVF IFPPSDEQLKSGTASVVCLLNNFYPREAKVQWKVDNALQSGNSQESVTEQDSKDSTYSL SSTLTLSKADYEKHKVYACEVTHQGLSSPVTKSFNRGECGGGGSGGGGSGGGGSEPKSS DKTHTCPPCPAPELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTISK AKGQPREPQVYTLPPCREEMTKNQVSLWCLVKGFYPSDIAVEWESNGQPENNYKTTPPV LDSDGSFFLYSKLTVDKSRWQQGNVFSCSVMHEALHNHYTQKSLSLSPGK | 65 |
| TLC-L-PLC-Knob | DIQMTQSPSSLSASVGDRVTITCRASQDVNTAVAWYQQKPGKAPKLLIYSASFLYSGVP SRFSGSRSGTDFTLTISSLQPEDFATYYCQQHYTTPPTFGCGTKVEIKRTVAAPSVFIF PPDIQMTQSPSSLSASVGDRVTITCKASQDVSIGVAWYQQKPGKAPKLLIYSASYRYTG VPSRFSGSGSGTDFTLTISSLQPEDFATYYCQQYYIYPYTFGCGTKVEIKRTVAAPSVF IFPPSDEQLKSGTASVVCLLNNFYPREAKVQWKVDNALQSGNSQESVTEQDSKDSTYSL SSTLTLSKADYEKHKVYACEVTHQGLSSPVTKSFNRGECGGGGSGGGGSGGGGSEPKSS DKTHTCPPCPAPELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTISK AKGQPREPQVYTLPPCREEMTKNQVSLWCLVKGFYPSDIAVEWESNGQPENNYKTTPPV LDSDGSFFLYSKLTVDKSRWQQGNVFSCSVMHEALHNHYTQKSLSLSPGK | 66 |

[Table 33]

| Name | Sequence | SEQ ID NO |
|------|----------|-----------|
| VH-S-Linker | GCTAGCACAAAAGGACCT | 484 |
| VH-L-Linker | GCTAGCACAAAAGGACCTAGTGTTTTCCCACTGGCTCCA | 485 |
| VL-S-Linker | CGTACGGTGGCTGCTCCA | 486 |
| VL-L-Linker | CGTACGGTGGCTGCTCCATCCGTTTTTATCTTTCCCCCA | 487 |
| TH-S-PH-Knob | GAAGTTCAACTTGTGGAGTCTGGAGGCGGTTTGGTACAACCTGGAGGTTCACTTCGTTT GTCATGTGCTGCCTCTGGGTTTAATATCAAGGATACTTATATTCATTGGGTTCGCCAGG CACCCGGAAAAGGGCTGGAATGGGTGGCCCGTATATATCCCACTAACGGTTACACTCGA TATGCTGACTCTGTAAAGGGTCGATTTACTATATCCGCTGATACTAGCAAAAACACTGC CTACCTCCAGATGAACAGTCTGCGCGCCGAGGATACAGCAGTTTATTATTGTTCCCGAT GGGGAGGTGACGGGTTCTATGCCATGGACTACTGGGGTCAAGGGACACTGGTAACCGTT TCTTCTGCTAGCACAAAAGGACCTGAAGTACAGTTGGTGGAGTCTGGTGGAGGTCTTGT ACAACCTGGCGGGAGTTTGCGGCTTTCCTGCGCTGCAAGCGGGTTTACCTTCACCGATT ATACCATGGATTGGGTACGCCAAGCCCCTGGTAAGGGCCTTGAGTGGGTTGCCGATGTA AACCCTAATTCCGGAGGAAGTATCTATAATCAACGCTTCAAGGGCCGATTCACTCTGAG TGTGGATCGAAGCAAGAACACCTTGTACTTGCAGATGAATTCCTTGCGGGCTGAAGACA CAGCCGTCTATTACTGCGCCCGAAATTTGGGGCCTTCATTCTATTTTGACTATTGGGGT CAGGGAACTCTGGTAACTGTTTCAAGTGCTAGCACCAAAGGACCTAGTGTTTTTCCTCT TGCCCCTTCCTCAAAGTCTACCTCTGGGGGGACAGCCGCTCTGGGCTGCCTGGTCAAGG ATTATTTCCCAGAGCCTGTCACTGTCAGTTGGAACTCTGGAGCCTTGACTTCTGGTGTT CATACATTTCCTGCTGTCCTTCAAAGCAGCGGCTTGTACTCATTGTCTTCTGTTGTGAC AGTACCCTCAAGCAGCCTCGGCACTCAGACATACATCTGCAATGTCAACCACAAACCCT CAAATACAAAGGTAGATAAAAAAGTCGAACCAAAGTCTTGTGACAAAACTCACACGTGC CCACCGTGCCCAGCACCTGAACTCCTGGGGGGACCGTCAGTCTTCCTCTTCCCCCCAAA ACCCAAGGACACCCTCATGATCTCCCGGACCCCTGAGGTCACATGCGTGGTGGTGGACG TGAGCCACGAAGACCCTGAGGTCAAGTTCAACTGGTACGTGGACGGCGTGGAGGTGCAT AATGCCAAGACAAAGCCGCGGGAGGAGCAGTACAACAGCACGTACCGTGTGGTCAGCGT CCTCACCGTCCTGCACCAGGACTGGCTGAATGGCAAGGAGTACAAGTGCAAGGTCTCCA | 488 |

| | | |
|---|---|---|
| | ACAAAGCCCTCCCAGCCCCCATCGAGAAAACCATCTCCAAAGCCAAAGGGCAGCCCCGA GAACCACAGGTGTACACCCTGCCCCCATGCCGGGAGGAGATGACCAAGAACCAGGTCAG CCTGTGGTGCCTGGTCAAAGGCTTCTATCCCAGCGACATCGCCGTGGAGTGGGAGAGCA ATGGGCAGCCGGAGAACAACTACAAGACCACGCCTCCCGTGCTGGACTCCGACGGCTCC TTCTTCCTCTACAGCAAGCTCACCGTGGACAAGAGCAGGTGGCAGCAGGGGAACGTCTT CTCATGCTCCGTGATGCATGAGGCTCTGCACAACCACTACACGCAGAAGAGCCTCTCCC TGTCTCCGGGTAAA | |
| THC-S-PH-Knob | GAAGTTCAACTTGTGGAGTCTGGAGGCGGTTTGGTACAACCTGGAGGTTCACTTCGTTT GTCATGTGCTGCCTCTGGGTTTAATATCAAGGATACTTATATTCATTGGGTTCGCCAGG CACCCGGAAAATGCCTGGAATGGGTGGCCCGTATATATCCCACTAACGGTTACACTCGA TATGCTGACTCTGTAAAGGGTCGATTTACTATATCCGCTGATACTAGCAAAAACACTGC CTACCTCCAGATGAACAGTCTGCGCGCCGAGGATACAGCAGTTTATTATTGTTCCCGAT GGGGAGGTGACGGGTTCTATGCCATGGACTACTGGGGTCAAGGGACACTGGTAACCGTT TCTTCTGCTAGCACAAAAGGACCTGAAGTACAGTTGGTGGAGTCTGGTGGAGGTCTTGT ACAACCTGGCGGGAGTTTGCGGCTTTCCTGCGCTGCAAGCGGGTTTACCTTCACCGATT ATACCATGGATTGGGTACGCCAAGCCCCTGGTAAGGGCCTTGAGTGGGTTGCCGATGTA AACCCTAATTCCGGAGGAAGTATCTATAATCAACGCTTCAAGGGCCGATTCACTCTGAG TGTGGATCGAAGCAAGAACACCTTGTACTTGCAGATGAATTCCTTGCGGGCTGAAGACA CAGCCGTCTATTACTGCGCCCGAAATTTGGGGCCTTCATTCTATTTTGACTATTGGGGT CAGGGAACTCTGGTAACTGTTTCAAGTGCTAGCACCAAAGGACCTAGTGTTTTTCCTCT TGCCCCTTCCTCAAAGTCTACCTCTGGGGGGACAGCCGCTCTGGGCTGCCTGGTCAAGG ATTATTTCCCAGAGCCTGTCACTGTCAGTTGGAACTCTGGAGCCTTGACTTCTGGTGTT CATACATTTCCTGCTGTCCTTCAAAGCAGCGGCTTGTACTCATTGTCTTCTGTTGTGAC AGTACCCTCAAGCAGCCTCGGCACTCAGACATACATCTGCAATGTCAACCACAAACCCT CAAATACAAAGGTAGATAAAAAAGTCGAACCAAAGTCTTGTGACAAAACTCACACGTGC CCACCGTGCCCAGCACCTGAACTCCTGGGGGGACCGTCAGTCTTCCTCTTCCCCCCAAA ACCCAAGGACACCCTCATGATCTCCCGGACCCCTGAGGTCACATGCGTGGTGGTGGACG TGAGCCACGAAGACCCTGAGGTCAAGTTCAACTGGTACGTGGACGGCGTGGAGGTGCAT AATGCCAAGACAAAGCCGCGGGAGGAGCAGTACAACAGCACGTACCGTGTGGTCAGCGT | 489 |

| | | |
|---|---|---|
| | CCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATGCCGGGAGGAGATGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | |
| TH-S-PHC-Knob | GAAGTTCAACTTGTGGAGTCTGGAGGCGGTTTGGTACAACCTGGAGGTTCACTTCGTTTGTCATGTGCTGCCTCTGGGTTTAATATCAAGGATACTTATATTCATTGGGTTCGCCAGGCACCCGGAAAAGGGCTGGAATGGGTGGCCCGTATATATCCCACTAACGGTTACACTCGATATGCTGACTCTGTAAAGGGTCGATTTACTATATCCGCTGATACTAGCAAAAACACTGCCTACCTCCAGATGAACAGTCTGCGCGCCGAGGATACAGCAGTTTATTATTGTTCCCGATGGGGAGGTGACGGGTTCTATGCCATGGACTACTGGGGTCAAGGGACACTGGTAACCGTTTCTTCTGCTAGCACAAAAGGACCTGAAGTACAGTTGGTGGAGTCTGGTGGAGGTCTTGTACAACCTGGCGGGAGTTTGCGGCTTTCCTGCGCTGCAAGCGGGTTTACCTTCACCGATTATACCATGGATTGGGTACGCCAAGCCCCTGGTAAGTGCCTTGAGTGGGTTGCCGATGTAAACCCTAATTCCGGAGGAAGTATCTATAATCAACGCTTCAAGGGCCGATTCACTCTGAGTGTGGATCGAAGCAAGAACACCTTGTACTTGCAGATGAATTCCTTGCGGGCTGAAGACACAGCCGTCTATTACTGCGCCCGAAATTTGGGGCCTTCATTCTATTTTGACTATTGGGGTCAGGGAACTCTGGTAACTGTTTCAAGTGCTAGCACCAAAGGACCTAGTGTTTTTCCTCTTGCCCCTTCCTCAAAGTCTACCTCTGGGGGGACAGCCGCTCTGGGCTGCCTGGTCAAGGATTATTTCCCAGAGCCTGTCACTGTCAGTTGGAACTCTGGAGCCTTGACTTCTGGTGTTCATACATTTCCTGCTGTCCTTCAAAGCAGCGGCTTGTACTCATTGTCTTCTGTTGTGACAGTACCCTCAAGCAGCCTCGGCACTCAGACATACATCTGCAATGTCAACCACAAACCCTCAAATACAAAGGTAGATAAAAAAGTCGAACCAAAGTCTTGTGACAAAACTCACACGTGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCAT | 490 |

| | | |
|---|---|---|
| | AATGCCAAGACAAAGCCGCGGGAGGAGCAGTACAACAGCACGTACCGTGTGGTCAGCGT CCTCACCGTCCTGCACCAGGACTGGCTGAATGGCAAGGAGTACAAGTGCAAGGTCTCCA ACAAAGCCCTCCCAGCCCCCATCGAGAAAACCATCTCCAAAGCCAAAGGGCAGCCCCGA GAACCACAGGTGTACACCCTGCCCCCATGCCGGGAGGAGATGACCAAGAACCAGGTCAG CCTGTGGTGCCTGGTCAAAGGCTTCTATCCCAGCGACATCGCCGTGGAGTGGGAGAGCA ATGGGCAGCCGGAGAACAACTACAAGACCACGCCTCCCGTGCTGGACTCCGACGGCTCC TTCTTCCTCTACAGCAAGCTCACCGTGGACAAGAGCAGGTGGCAGCAGGGGAACGTCTT CTCATGCTCCGTGATGCATGAGGCTCTGCACAACCACTACACGCAGAAGAGCCTCTCCC TGTCTCCGGGTAAA | |
| THC-S-PHC-Knob | GAAGTTCAACTTGTGGAGTCTGGAGGCGGTTTGGTACAACCTGGAGGTTCACTTCGTTT GTCATGTGCTGCCTCTGGGTTTAATATCAAGGATACTTATATTCATTGGGTTCGCCAGG CACCCGGAAAATGCCTGGAATGGGTGGCCCGTATATATCCCACTAACGGTTACACTCGA TATGCTGACTCTGTAAAGGGTCGATTTACTATATCCGCTGATACTAGCAAAAACACTGC CTACCTCCAGATGAACAGTCTGCGCGCCGAGGATACAGCAGTTTATTATTGTTCCCGAT GGGGAGGTGACGGGTTCTATGCCATGGACTACTGGGGTCAAGGGACACTGGTAACCGTT TCTTCTGCTAGCACAAAAGGACCTAGTGTTTTCCCACTGGCTCCAGAAGTACAGTTGGT GGAGTCTGGTGGAGGTCTTGTACAACCTGGCGGGAGTTTGCGGCTTTCCTGCGCTGCAA GCGGGTTTACCTTCACCGATTATACCATGGATTGGGTACGCCAAGCCCCTGGTAAGTGC CTTGAGTGGGTTGCCGATGTAAACCCTAATTCCGGAGGAAGTATCTATAATCAACGCTT CAAGGGCCGATTCACTCTGAGTGTGGATCGAAGCAAGAACACCTTGTACTTGCAGATGA ATTCCTTGCGGGCTGAAGACACAGCCGTCTATTACTGCGCCCGAAATTTGGGGCCTTCA TTCTATTTTGACTATTGGGGTCAGGGAACTCTGGTAACTGTTTCAAGTGCTAGCACCAA AGGACCTAGTGTTTTTCCTCTTGCCCCTTCCTCAAAGTCTACCTCTGGGGGGACAGCCG CTCTGGGCTGCCTGGTCAAGGATTATTTCCCAGAGCCTGTCACTGTCAGTTGGAACTCT GGAGCCTTGACTTCTGGTGTTCATACATTTCCTGCTGTCCTTCAAAGCAGCGGCTTGTA CTCATTGTCTTCTGTTGTGACAGTACCCTCAAGCAGCCTCGGCACTCAGACATACATCT GCAATGTCAACCACAAACCCTCAAATACAAAGGTAGATAAAAAAGTCGAACCAAAGTCT TGTGACAAAACTCACACGTGCCCACCGTGCCCAGCACCTGAACTCCTGGGGGGACCGTC AGTCTTCCTCTTCCCCCCAAAACCCAAGGACACCCTCATGATCTCCCGGACCCCTGAGG | 491 |

| | | |
|---|---|---|
| | TCACATGCGTGGTGGTGGACGTGAGCCACGAAGACCCTGAGGTCAAGTTCAACTGGTAC GTGGACGGCGTGGAGGTGCATAATGCCAAGACAAAGCCGCGGGAGGAGCAGTACAACAG CACGTACCGTGTGGTCAGCGTCCTCACCGTCCTGCACCAGGACTGGCTGAATGGCAAGG AGTACAAGTGCAAGGTCTCCAACAAAGCCCTCCCAGCCCCCATCGAGAAAACCATCTCC AAAGCCAAAGGGCAGCCCCGAGAACCACAGGTGTACACCCTGCCCCCATGCCGGGAGGA GATGACCAAGAACCAGGTCAGCCTGTGGTGCCTGGTCAAAGGCTTCTATCCCAGCGACA TCGCCGTGGAGTGGGAGAGCAATGGGCAGCCGGAGAACAACTACAAGACCACGCCTCCC GTGCTGGACTCCGACGGCTCCTTCTTCCTCTACAGCAAGCTCACCGTGGACAAGAGCAG GTGGCAGCAGGGGAACGTCTTCTCATGCTCCGTGATGCATGAGGCTCTGCACAACCACT ACACGCAGAAGAGCCTCTCCCTGTCTCCGGGTAAA | |
| TH-L-PH-Knob | GAAGTTCAACTTGTGGAGTCTGGAGGCGGTTTGGTACAACCTGGAGGTTCACTTCGTTT GTCATGTGCTGCCTCTGGGTTTAATATCAAGGATACTTATATTCATTGGGTTCGCCAGG CACCCGGAAAAGGGCTGGAATGGGTGGCCCGTATATATCCCACTAACGGTTACACTCGA TATGCTGACTCTGTAAAGGGTCGATTTACTATATCCGCTGATACTAGCAAAAACACTGC CTACCTCCAGATGAACAGTCTGCGCGCCGAGGATACAGCAGTTTATTATTGTTCCCGAT GGGGAGGTGACGGGTTCTATGCCATGGACTACTGGGGTCAAGGGACACTGGTAACCGTT TCTTCTGCTAGCACAAAAGGACCTAGTGTTTTCCCACTGGCTCCAGAAGTACAGTTGGT GGAGTCTGGTGGAGGTCTTGTACAACCTGGCGGGAGTTTGCGGCTTTCCTGCGCTGCAA GCGGGTTTACCTTCACCGATTATACCATGGATTGGGTACGCCAAGCCCCTGGTAAGGGC CTTGAGTGGGTTGCCGATGTAAACCCTAATTCCGGAGGAAGTATCTATAATCAACGCTT CAAGGGCCGATTCACTCTGAGTGTGGATCGAAGCAAGAACACCTTGTACTTGCAGATGA ATTCCTTGCGGGCTGAAGACACAGCCGTCTATTACTGCGCCCGAAATTTGGGGCCTTCA TTCTATTTTGACTATTGGGGTCAGGGAACTCTGGTAACTGTTTCAAGTGCTAGCACCAA AGGACCTAGTGTTTTTCCTCTTGCCCCTTCCTCAAAGTCTACCTCTGGGGGGACAGCCG CTCTGGGCTGCCTGGTCAAGGATTATTTCCCAGAGCCTGTCACTGTCAGTTGGAACTCT GGAGCCTTGACTTCTGGTGTTCATACATTTCCTGCTGTCCTTCAAAGCAGCGGCTTGTA CTCATTGTCTTCTGTTGTGACAGTACCCTCAAGCAGCCTCGGCACTCAGACATACATCT GCAATGTCAACCACAAACCCTCAAATACAAAGGTAGATAAAAAAGTCGAACCAAAGTCT TGTGACAAAACTCACACGTGCCCACCGTGCCCAGCACCTGAACTCCTGGGGGGACCGTC | 492 |

| | | |
|---|---|---|
| | AGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATGCCGGGAGGAGATGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | |
| THC-L-PH-Knob | GAAGTTCAACTTGTGGAGTCTGGAGGCGGTTTGGTACAACCTGGAGGTTCACTTCGTTTGTCATGTGCTGCCTCTGGGTTTAATATCAAGGATACTTATATTCATTGGGTTCGCCAGGCACCCGGAAAATGCCTGGAATGGGTGGCCCGTATATATCCCACTAACGGTTACACTCGATATGCTGACTCTGTAAAGGGTCGATTTACTATATCCGCTGATACTAGCAAAAACACTGCCTACCTCCAGATGAACAGTCTGCGCGCCGAGGATACAGCAGTTTATTATTGTTCCCGATGGGGAGGTGACGGGTTCTATGCCATGGACTACTGGGGTCAAGGGACACTGGTAACCGTTTCTTCTGCTAGCACAAAAGGACCTAGTGTTTTCCCACTGGCTCCAGAAGTACAGTTGGTGGAGTCTGGTGGAGGTCTTGTACAACCTGGCGGGAGTTTGCGGCTTTCCTGCGCTGCAAGCGGGTTTACCTTCACCGATTATACCATGGATTGGGTACGCCAAGCCCCTGGTAAGGGCCTTGAGTGGGTTGCCGATGTAAACCCTAATTCCGGAGGAAGTATCTATAATCAACGCTTCAAGGGCCGATTCACTCTGAGTGTGGATCGAAGCAAGAACACCTTGTACTTGCAGATGAATTCCTTGCGGGCTGAAGACACAGCCGTCTATTACTGCGCCCGAAATTTGGGGCCTTCATTCTATTTTGACTATTGGGGTCAGGGAACTCTGGTAACTGTTTCAAGTGCTAGCACCAAAGGACCTAGTGTTTTTCCTCTTGCCCCTTCCTCAAAGTCTACCTCTGGGGGGACAGCCGCTCTGGGCTGCCTGGTCAAGGATTATTTCCCAGAGCCTGTCACTGTCAGTTGGAACTCTGGAGCCTTGACTTCTGGTGTTCATACATTTCCTGCTGTCCTTCAAAGCAGCGGCTTGTACTCATTGTCTTCTGTTGTGACAGTACCCTCAAGCAGCCTCGGCACTCAGACATACATCTGCAATGTCAACCACAAACCCTCAAATACAAAGGTAGATAAAAAAGTCGAACCAAAGTCT | 493 |

| | | |
|---|---|---|
| | TGTGACAAAACTCACACGTGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATGCCGGGAGGAGATGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | |
| TH-L-PHC-Knob | GAAGTTCAACTTGTGGAGTCTGGAGGCGGTTTGGTACAACCTGGAGGTTCACTTCGTTTGTCATGTGCTGCCTCTGGGTTTAATATCAAGGATACTTATATTCATTGGGTTCGCCAGGCACCCGGAAAAGGGCTGGAATGGGTGGCCCGTATATATCCCACTAACGGTTACACTCGATATGCTGACTCTGTAAAGGGTCGATTTACTATATCCGCTGATACTAGCAAAAACACTGCCTACCTCCAGATGAACAGTCTGCGCGCCGAGGATACAGCAGTTTATTATTGTTCCCGATGGGGAGGTGACGGGTTCTATGCCATGGACTACTGGGGTCAAGGGACACTGGTAACCGTTTCTTCTGCTAGCACAAAAGGACCTAGTGTTTTCCCACTGGCTCCAGAAGTACAGTTGGTGGAGTCTGGTGGAGGTCTTGTACAACCTGGCGGGAGTTTGCGGCTTTCCTGCGCTGCAAGCGGGTTTACCTTCACCGATTATACCATGGATTGGGTACGCCAAGCCCCTGGTAAGTGCCTTGAGTGGGTTGCCGATGTAAACCCTAATTCCGGAGGAAGTATCTATAATCAACGCTTCAAGGGCCGATTCACTCTGAGTGTGGATCGAAGCAAGAACACCTTGTACTTGCAGATGAATTCCTTGCGGGCTGAAGACACAGCCGTCTATTACTGCGCCCGAAATTTGGGGGCCTTCATTCTATTTTGACTATTGGGGTCAGGGAACTCTGGTAACTGTTTCAAGTGCTAGCACCAAAGGACCTAGTGTTTTTCCTCTTGCCCCTTCCTCAAAGTCTACCTCTGGGGGGACAGCCGCTCTGGGCTGCCTGGTCAAGGATTATTTCCCAGAGCCTGTCACTGTCAGTTGGAACTCTGGAGCCTTGACTTCTGGTGTTCATACATTTCCTGCTGTCCTTCAAAGCAGCGGCTTGTACTCATTGTCTTCTGTTGTGACAGTACCCTCAAGCAGCCTCGGCACTCAGACATACATCT | 494 |

| | | |
|---|---|---|
| | GCAATGTCAACCACAAACCCTCAAATACAAAGGTAGATAAAAAAGTCGAACCAAAGTCT TGTGACAAAACTCACACGTGCCCACCGTGCCCAGCACCTGAACTCCTGGGGGGACCGTC AGTCTTCCTCTTCCCCCCAAAACCCAAGGACACCCTCATGATCTCCCGGACCCCTGAGG TCACATGCGTGGTGGTGGACGTGAGCCACGAAGACCCTGAGGTCAAGTTCAACTGGTAC GTGGACGGCGTGGAGGTGCATAATGCCAAGACAAAGCCGCGGGAGGAGCAGTACAACAG CACGTACCGTGTGGTCAGCGTCCTCACCGTCCTGCACCAGGACTGGCTGAATGGCAAGG AGTACAAGTGCAAGGTCTCCAACAAAGCCCTCCCAGCCCCCATCGAGAAAACCATCTCC AAAGCCAAAGGGCAGCCCCGAGAACCACAGGTGTACACCCTGCCCCCATGCCGGGAGGA GATGACCAAGAACCAGGTCAGCCTGTGGTGCCTGGTCAAAGGCTTCTATCCCAGCGACA TCGCCGTGGAGTGGGAGAGCAATGGGCAGCCGGAGAACAACTACAAGACCACGCCTCCC GTGCTGGACTCCGACGGCTCCTTCTTCCTCTACAGCAAGCTCACCGTGGACAAGAGCAG GTGGCAGCAGGGGAACGTCTTCTCATGCTCCGTGATGCATGAGGCTCTGCACAACCACT ACACGCAGAAGAGCCTCTCCCTGTCTCCGGGTAAA | |
| THC-L-PHC-Knob | GAAGTTCAACTTGTGGAGTCTGGAGGCGGTTTGGTACAACCTGGAGGTTCACTTCGTTT GTCATGTGCTGCCTCTGGGTTTAATATCAAGGATACTTATATTCATTGGGTTCGCCAGG CACCCGGAAAATGCCTGGAATGGGTGGCCCGTATATATCCCACTAACGGTTACACTCGA TATGCTGACTCTGTAAAGGGTCGATTTACTATATCCGCTGATACTAGCAAAAACACTGC CTACCTCCAGATGAACAGTCTGCGCGCCGAGGATACAGCAGTTTATTATTGTTCCCGAT GGGGAGGTGACGGGTTCTATGCCATGGACTACTGGGGTCAAGGGACACTGGTAACCGTT TCTTCTGCTAGCACAAAAGGACCTAGTGTTTTCCCACTGGCTCCAGAAGTACAGTTGGT GGAGTCTGGTGGAGGTCTTGTACAACCTGGCGGGAGTTTGCGGCTTTCCTGCGCTGCAA GCGGGTTTACCTTCACCGATTATACCATGGATTGGGTACGCCAAGCCCCTGGTAAGTGC CTTGAGTGGGTTGCCGATGTAAACCCTAATTCCGGAGGAAGTATCTATAATCAACGCTT CAAGGGCCGATTCACTCTGAGTGTGGATCGAAGCAAGAACACCTTGTACTTGCAGATGA ATTCCTTGCGGGCTGAAGACACAGCCGTCTATTACTGCGCCCGAAATTTGGGGCCTTCA TTCTATTTTGACTATTGGGGTCAGGGAACTCTGGTAACTGTTTCAAGTGCTAGCACCAA AGGACCTAGTGTTTTTCCTCTTGCCCCTTCCTCAAAGTCTACCTCTGGGGGGACAGCCG CTCTGGGCTGCCTGGTCAAGGATTATTTCCCAGAGCCTGTCACTGTCAGTTGGAACTCT GGAGCCTTGACTTCTGGTGTTCATACATTTCCTGCTGTCCTTCAAAGCAGCGGCTTGTA | 495 |

| | | | |
|---|---|---|---|
| | | CTCATTGTCTTCTGTTGTGACAGTACCCTCAAGCAGCCTCGGCACTCAGACATACATCTGCAATGTCAACCACAAACCCTCAAATACAAAGGTAGATAAAAAAGTCGAACCAAAGTCTTGTGACAAAACTCACACGTGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATGCCGGGAGGAGATGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | |
| TL-S-PL-Knob | GACATTCAGATGACCCAATCCCCCTCTAGCCTTTCAGCCTCCGTAGGGGACAGAGTTACAATAACCTGTAGGGCTAGTCAGGACGTTAATACAGCAGTCGCATGGTACCAGCAGAAGCCCGGTAAGGCTCCTAAGCTTTTGATCTACAGTGCTTCTTTCCTGTATTCTGGTGTACCTAGCCGATTCTCAGGCTCTCGGAGTGGCACAGACTTCACTCTGACAATTTCAAGTCTCCAGCCAGAAGATTTTGCAACCTACTACTGCCAACAACACTACACTACTCCTCCAACCTTCGGACAGGGCACAAAAGTAGAGATTAAGCGTACGGTGGCTGCTCCAGATATTCAAATGACCCAAAGTCCAAGTTCCCTTTCAGCATCTGTCGGTGATCGAGTCACTATAACTTGTAAGGCCAGCCAAGACGTTAGCATAGGAGTAGCATGGTACCAACAAAAGCCTGGGAAGGCTCCCAAACTTCTCATTTATTCTGCTTCCTACCGATATACTGGTGTCCCAAGTAGATTTTCTGGCAGCGGATCTGGAACTGATTTTACATTGACTATCAGCTCCCTCCAGCCTGAGGACTTCGCTACTTATTACTGCCAACAGTACTACATTTATCCCTATACATTCGGTCAAGGGACCAAAGTAGAGATCAAACGTACGGTGGCAGCTCCCAGCGTTTTTATCTTTCCCCCATCCGACGAGCAGCTCAAGAGTGGCACTGCCTCTGTAGTTTGTTTGCTGAATAACTTCTATCCACGTGAAGCAAAAGTACAGTGGAAGGTCGATAATGCCCTTCAGAGCGGTAACAGCCAAGAAAGTGTTACCGAGCAAGATTCCAAAGATTCCACTTACAGTCTGTCCAGCACATTGACACTGAGT | 496 |

| | | |
|---|---|---|
| | AAGGCTGATTACGAAAAACACAAGGTGTACGCATGCGAGGTGACACACCAAGGTCTTTC ATCTCCTGTAACTAAGAGCTTTAACCGGGGGAGAATGTGGTGGTGGGGGGCAGCGGGGGCG GAGGTAGTGGAGGCGGCGGTAGTGAACCAAAGAGTAGTGACAAAACTCACACGTGCCCA CCGTGCCCAGCACCTGAACTCCTGGGGGGACCGTCAGTCTTCCTCTTCCCCCCCAAAACC CAAGGACACCCTCATGATCTCCCGGACCCCTGAGGTCACATGCGTGGTGGTGGACGTGA GCCACGAAGACCCTGAGGTCAAGTTCAACTGGTACGTGGACGGCGTGGAGGTGCATAAT GCCAAGACAAAGCCGCGGGAGGAGCAGTACAACAGCACGTACCGTGTGGTCAGCGTCCT CACCGTCCTGCACCAGGACTGGCTGAATGGCAAGGAGTACAAGTGCAAGGTCTCCAACA AAGCCCTCCCAGCCCCCATCGAGAAAACCATCTCCAAAGCCAAAGGGCAGCCCCGAGAA CCACAGGTGTACACCCTGCCCCCATGCCGGGAGGAGATGACCAAGAACCAGGTCAGCCT GTGGTGCCTGGTCAAAGGCTTCTATCCCAGCGACATCGCCGTGGAGTGGGAGAGCAATG GGCAGCCGGAGAACAACTACAAGACCACGCCTCCCGTGCTGGACTCCGACGGCTCCTTC TTCCTCTACAGCAAGCTCACCGTGGACAAGAGCAGGTGGCAGCAGGGGAACGTCTTCTC ATGCTCCGTGATGCATGAGGCTCTGCACAACCACTACACGCAGAAGAGCCTCTCCCTGT CTCCGGGTAAA | |
| TLC-S-PL-Knob | GACATTCAGATGACCCAATCCCCCTCTAGCCTTTCAGCCTCCGTAGGGGACAGAGTTAC AATAACCTGTAGGGCTAGTCAGGACGTTAATACAGCAGTCGCATGGTACCAGCAGAAGC CCGGTAAGGCTCCTAAGCTTTTGATCTACAGTGCTTCTTTCCTGTATTCTGGTGTACCT AGCCGATTCTCAGGCTCTCGGAGTGGCACAGACTTCACTCTGACAATTTCAAGTCTCCA GCCAGAAGATTTTGCAACCTACTACTGCCAACAACACTACACTACTCCTCCAACCTTCG GATGTGGCACAAAAGTAGAGATTAAGCGTACGGTGGCTGCTCCAGATATTCAAATGACC CAAAGTCCAAGTTCCCTTTCAGCATCTGTCGGTGATCGAGTCACTATAACTTGTAAGGC CAGCCAAGACGTTAGCATAGGAGTAGCATGGTACCAACAAAAGCCTGGGAAGGCTCCCA AACTTCTCATTTATTCTGCTTCCTACCGATATACTGGTGTCCCAAGTAGATTTTCTGGC AGCGGATCTGGAACTGATTTTACATTGACTATCAGCTCCCTCCAGCCTGAGGACTTCGC TACTTATTACTGCCAACAGTACTACATTTATCCCTATACATTCGGTCAAGGGACCAAAG TAGAGATCAAACGTACGGTGGCAGCTCCCAGCGTTTTTATCTTTCCCCCATCCGACGAG CAGCTCAAGAGTGGCACTGCCTCTGTAGTTTGTTTGCTGAATAACTTCTATCCACGTGA AGCAAAAGTACAGTGGAAGGTCGATAATGCCCTTCAGAGCGGTAACAGCCAAGAAAGTG | 497 |

| | | | |
|---|---|---|---|
| | | TTACCGAGCAAGATTCCAAAGATTCCACTTACAGTCTGTCCAGCACATTGACACTGAGT AAGGCTGATTACGAAAAACACAAGGTGTACGCATGCGAGGTGACACACCAAGGTCTTTC ATCTCCTGTAACTAAGAGCTTTAACCGGGGAGAATGTGGTGGTGGGGGCAGCGGGGGCG GAGGTAGTGGAGGCGGCGGTAGTGAACCAAAGAGTAGTGACAAAACTCACACGTGCCCA CCGTGCCCAGCACCTGAACTCCTGGGGGGACCGTCAGTCTTCCTCTTCCCCCCCAAAACC CAAGGACACCCTCATGATCTCCCGGACCCCTGAGGTCACATGCGTGGTGGTGGACGTGA GCCACGAAGACCCTGAGGTCAAGTTCAACTGGTACGTGGACGGCGTGGAGGTGCATAAT GCCAAGACAAAGCCGCGGGAGGAGCAGTACAACAGCACGTACCGTGTGGTCAGCGTCCT CACCGTCCTGCACCAGGACTGGCTGAATGGCAAGGAGTACAAGTGCAAGGTCTCCAACA AAGCCCTCCCAGCCCCCATCGAGAAAACCATCTCCAAAGCCAAAGGGCAGCCCCGAGAA CCACAGGTGTACACCCTGCCCCCATGCCGGGAGGAGATGACCAAGAACCAGGTCAGCCT GTGGTGCCTGGTCAAAGGCTTCTATCCCAGCGACATCGCCGTGGAGTGGGAGAGCAATG GGCAGCCGGAGAACAACTACAAGACCACGCCTCCCGTGCTGGACTCCGACGGCTCCTTC TTCCTCTACAGCAAGCTCACCGTGGACAAGAGCAGGTGGCAGCAGGGGAACGTCTTCTC ATGCTCCGTGATGCATGAGGCTCTGCACAACCACTACACGCAGAAGAGCCTCTCCCTGT CTCCGGGTAAA | |
| TL-S-PLC-Knob | | GACATTCAGATGACCCAATCCCCCTCTAGCCTTTCAGCCTCCGTAGGGGACAGAGTTAC AATAACCTGTAGGGCTAGTCAGGACGTTAATACAGCAGTCGCATGGTACCAGCAGAAGC CCGGTAAGGCTCCTAAGCTTTTGATCTACAGTGCTTCTTTCCTGTATTCTGGTGTACCT AGCCGATTCTCAGGCTCTCGGAGTGGCACAGACTTCACTCTGACAATTTCAAGTCTCCA GCCAGAAGATTTTGCAACCTACTACTGCCAACAACACTACACTACTCCTCCAACCTTCG GACAGGGCACAAAAGTAGAGATTAAGCGTACGGTGGCTGCTCCAGATATTCAAATGACC CAAAGTCCAAGTTCCCTTTCAGCATCTGTCGGTGATCGAGTCACTATAACTTGTAAGGC CAGCCAAGACGTTAGCATAGGAGTAGCATGGTACCAACAAAAGCCTGGGAAGGCTCCCA AACTTCTCATTTATTCTGCTTCCTACCGATATACTGGTGTCCCAAGTAGATTTTCTGGC AGCGGATCTGGAACTGATTTTACATTGACTATCAGCTCCCTCCAGCCTGAGGACTTCGC TACTTATTACTGCCAACAGTACTACATTTATCCCTATACATTCGGTTGTGGGACCAAAG TAGAGATCAAACGTACGGTGGCAGCTCCCAGCGTTTTTATCTTTCCCCCATCCGACGAG CAGCTCAAGAGTGGCACTGCCTCTGTAGTTTGTTTGCTGAATAACTTCTATCCACGTGA | 498 |

| | | | |
|---|---|---|---|
| | | AGCAAAAGTACAGTGGAAGGTCGATAATGCCCTTCAGAGCGGTAACAGCCAAGAAAGTG TTACCGAGCAAGATTCCAAAGATTCCACTTACAGTCTGTCCAGCACATTGACACTGAGT AAGGCTGATTACGAAAAACACAAGGTGTACGCATGCGAGGTGACACACCAAGGTCTTTC ATCTCCTGTAACTAAGAGCTTTAACCGGGGAGAATGTGGTGGTGGGGGCAGCGGGGGCG GAGGTAGTGGAGGCGGCGGTAGTGAACCAAAGAGTAGTGACAAAACTCACACGTGCCCA CCGTGCCCAGCACCTGAACTCCTGGGGGGACCGTCAGTCTTCCTCTTCCCCCCCAAAACC CAAGGACACCCTCATGATCTCCCGGACCCCTGAGGTCACATGCGTGGTGGTGGACGTGA GCCACGAAGACCCTGAGGTCAAGTTCAACTGGTACGTGGACGGCGTGGAGGTGCATAAT GCCAAGACAAAGCCGCGGGAGGAGCAGTACAACAGCACGTACCGTGTGGTCAGCGTCCT CACCGTCCTGCACCAGGACTGGCTGAATGGCAAGGAGTACAAGTGCAAGGTCTCCAACA AAGCCCTCCCAGCCCCCATCGAGAAAACCATCTCCAAAGCCAAAGGGCAGCCCCGAGAA CCACAGGTGTACACCCTGCCCCCATGCCGGGAGGAGATGACCAAGAACCAGGTCAGCCT GTGGTGCCTGGTCAAAGGCTTCTATCCCAGCGACATCGCCGTGGAGTGGGAGAGCAATG GGCAGCCGGAGAACAACTACAAGACCACGCCTCCCGTGCTGGACTCCGACGGCTCCTTC TTCCTCTACAGCAAGCTCACCGTGGACAAGAGCAGGTGGCAGCAGGGGAACGTCTTCTC ATGCTCCGTGATGCATGAGGCTCTGCACAACcactacACGCAGAAGAGCCTCTCCCTGT CTCCGGGTAAA | |
| TLC-S-PLC-Knob | GACATTCAGATGACCCAATCCCCCTCTAGCCTTTCAGCCTCCGTAGGGGACAGAGTTAC AATAACCTGTAGGGCTAGTCAGGACGTTAATACAGCAGTCGCATGGTACCAGCAGAAGC CCGGTAAGGCTCCTAAGCTTTTGATCTACAGTGCTTCTTTCCTGTATTCTGGTGTACCT AGCCGATTCTCAGGCTCTCGGAGTGGCACAGACTTCACTCTGACAATTTCAAGTCTCCA GCCAGAAGATTTTGCAACCTACTACTGCCAACAACACTACACTACTCCTCCAACCTTCG GATGTGGCACAAAAGTAGAGATTAAGCGTACGGTGGCTGCTCCAGATATTCAAATGACC CAAAGTCCAAGTTCCCTTTCAGCATCTGTCGGTGATCGAGTCACTATAACTTGTAAGGC CAGCCAAGACGTTAGCATAGGAGTAGCATGGTACCAACAAAAGCCTGGGAAGGCTCCCA AACTTCTCATTTATTCTGCTTCCTACCGATATACTGGTGTCCCAAGTAGATTTTCTGGC AGCGGATCTGGAACTGATTTTACATTGACTATCAGCTCCCTCCAGCCTGAGGACTTCGC TACTTATTACTGCCAACAGTACTACATTTATCCCTATACATTCGGTTGTGGGACCAAAG TAGAGATCAAACGTACGGTGGCAGCTCCCAGCGTTTTTATCTTTCCCCCATCCGACGAG | 499 |

| | | |
|---|---|---|
| | CAGCTCAAGAGTGGCACTGCCTCTGTAGTTTGTTTGCTGAATAACTTCTATCCACGTGA AGCAAAAGTACAGTGGAAGGTCGATAATGCCCTTCAGAGCGGTAACAGCCAAGAAAGTG TTACCGAGCAAGATTCCAAAGATTCCACTTACAGTCTGTCCAGCACATTGACACTGAGT AAGGCTGATTACGAAAAACACAAGGTGTACGCATGCGAGGTGACACACCAAGGTCTTTC ATCTCCTGTAACTAAGAGCTTTAACCGGGGAGAATGTGGTGGTGGGGGCAGCGGGGGCG GAGGTAGTGGAGGCGGCGGTAGTGAACCAAAGAGTAGTGACAAAACTCACACGTGCCCA CCGTGCCCAGCACCTGAACTCCTGGGGGGACCGTCAGTCTTCCTCTTCCCCCCAAAACC CAAGGACACCCTCATGATCTCCCGGACCCCTGAGGTCACATGCGTGGTGGTGGACGTGA GCCACGAAGACCCTGAGGTCAAGTTCAACTGGTACGTGGACGGCGTGGAGGTGCATAAT GCCAAGACAAAGCCGCGGGAGGAGCAGTACAACAGCACGTACCGTGTGGTCAGCGTCCT CACCGTCCTGCACCAGGACTGGCTGAATGGCAAGGAGTACAAGTGCAAGGTCTCCAACA AAGCCCTCCCAGCCCCCATCGAGAAAACCATCTCCAAAGCCAAAGGGCAGCCCCGAGAA CCACAGGTGTACACCCTGCCCCCCATGCCGGGAGGAGATGACCAAGAACCAGGTCAGCCT GTGGTGCCTGGTCAAAGGCTTCTATCCCAGCGACATCGCCGTGGAGTGGGAGAGCAATG GGCAGCCGGAGAACAACTACAAGACCACGCCTCCCGTGCTGGACTCCGACGGCTCCTTC TTCCTCTACAGCAAGCTCACCGTGGACAAGAGCAGGTGGCAGCAGGGGAACGTCTTCTC ATGCTCCGTGATGCATGAGGCTCTGCACAACCACTACACGCAGAAGAGCCTCTCCCTGT CTCCGGGTAAA | |
| TL-L-PL-Knob | GACATTCAGATGACCCAATCCCCCTCTAGCCTTTCAGCCTCCGTAGGGGACAGAGTTAC AATAACCTGTAGGGCTAGTCAGGACGTTAATACAGCAGTCGCATGGTACCAGCAGAAGC CCGGTAAGGCTCCTAAGCTTTTTGATCTACAGTGCTTCTTTCCTGTATTCTGGTGTACCT AGCCGATTCTCAGGCTCTCGGAGTGGCACAGACTTCACTCTGACAATTTCAAGTCTCCA GCCAGAAGATTTTGCAACCTACTACTGCCAACAACACTACACTACTCCTCCAACCTTCG GACAGGGCACAAAAGTAGAGATTAAGCGTACGGTGGCTGCTCCATCCGTTTTTATCTTT CCCCCAGATATTCAAATGACCCAAAGTCCAAGTTCCCTTTCAGCATCTGTCGGTGATCG AGTCACTATAACTTGTAAGGCCAGCCAAGACGTTAGCATAGGAGTAGCATGGTACCAAC AAAAGCCTGGGAAGGCTCCCAAACTTCTCATTTATTCTGCTTCCTACCGATATACTGGT GTCCCAAGTAGATTTTCTGGCAGCGGATCTGGAACTGATTTTACATTGACTATCAGCTC CCTCCAGCCTGAGGACTTCGCTACTTATTACTGCCAACAGTACTACATTTATCCCTATA | 500 |

| | | |
|---|---|---|
| | CATTCGGTCAAGGGACCAAAGTAGAGATCAAACGTACGGTGGCAGCTCCCAGCGTTTTT ATCTTTCCCCCATCCGACGAGCAGCTCAAGAGTGGCACTGCCTCTGTAGTTTGTTTGCT GAATAACTTCTATCCACGTGAAGCAAAAGTACAGTGGAAGGTCGATAATGCCCTTCAGA GCGGTAACAGCCAAGAAAGTGTTACCGAGCAAGATTCCAAAGATTCCACTTACAGTCTG TCCAGCACATTGACACTGAGTAAGGCTGATTACGAAAAACACAAGGTGTACGCATGCGA GGTGACACACCAAGGTCTTTCATCTCCTGTAACTAAGAGCTTTAACCGGGGAGAATGTG GTGGTGGGGGCAGCGGGGGCGGAGGTAGTGGAGGCGGCGGTAGTGAACCAAAGAGTAGT GACAAAACTCACACGTGCCCACCGTGCCCAGCACCTGAACTCCTGGGGGGACCGTCAGT CTTCCTCTTCCCCCCAAAACCCAAGGACACCCTCATGATCTCCCGGACCCCTGAGGTCA CATGCGTGGTGGTGGACGTGAGCCACGAAGACCCTGAGGTCAAGTTCAACTGGTACGTG GACGGCGTGGAGGTGCATAATGCCAAGACAAAGCCGCGGGAGGAGCAGTACAACAGCAC GTACCGTGTGGTCAGCGTCCTCACCGTCCTGCACCAGGACTGGCTGAATGGCAAGGAGT ACAAGTGCAAGGTCTCCAACAAAGCCCTCCCAGCCCCCATCGAGAAAACCATCTCCAAA GCCAAAGGGCAGCCCCGAGAACCACAGGTGTACACCCTGCCCCCATGCCGGGAGGAGAT GACCAAGAACCAGGTCAGCCTGTGGTGCCTGGTCAAAGGCTTCTATCCCAGCGACATCG CCGTGGAGTGGGAGAGCAATGGGCAGCCGGAGAACAACTACAAGACCACGCCTCCCGTG CTGGACTCCGACGGCTCCTTCTTCCTCTACAGCAAGCTCACCGTGGACAAGAGCAGGTG GCAGCAGGGGAACGTCTTCTCATGCTCCGTGATGCATGAGGCTCTGCACAACCACTACA CGCAGAAGAGCCTCTCCCTGTCTCCGGGTAAA | |
| TLC-L-PL-Knob | GACATTCAGATGACCCAATCCCCCTCTAGCCTTTCAGCCTCCGTAGGGGACAGAGTTAC AATAACCTGTAGGGCTAGTCAGGACGTTAATACAGCAGTCGCATGGTACCAGCAGAAGC CCGGTAAGGCTCCTAAGCTTTTGATCTACAGTGCTTCTTTCCTGTATTCTGGTGTACCT AGCCGATTCTCAGGCTCTCGGAGTGGCACAGACTTCACTCTGACAATTTCAAGTCTCCA GCCAGAAGATTTTGCAACCTACTACTGCCAACAACACTACACTACTCCTCCAACCTTCG GATGTGGCACAAAAGTAGAGATTAAGCGTACGGTGGCTGCTCCATCCGTTTTTATCTTT CCCCCAGATATTCAAATGACCCAAAGTCCAAGTTCCCTTTCAGCATCTGTCGGTGATCG AGTCACTATAACTTGTAAGGCCAGCCAAGACGTTAGCATAGGAGTAGCATGGTACCAAC AAAAGCCTGGGAAGGCTCCCAAACTTCTCATTTATTCTGCTTCCTACCGATATACTGGT GTCCCAAGTAGATTTTCTGGCAGCGGATCTGGAACTGATTTTACATTGACTATCAGCTC | 501 |

| | | |
|---|---|---|
| | CCTCCAGCCTGAGGACTTCGCTACTTATTACTGCCAACAGTACTACATTTATCCCTATA CATTCGGTCAAGGGACCAAAGTAGAGATCAAACGTACGGTGGCAGCTCCCAGCGTTTTT ATCTTTCCCCCATCCGACGAGCAGCTCAAGAGTGGCACTGCCTCTGTAGTTTGTTTGCT GAATAACTTCTATCCACGTGAAGCAAAAGTACAGTGGAAGGTCGATAATGCCCTTCAGA GCGGTAACAGCCAAGAAAGTGTTACCGAGCAAGATTCCAAAGATTCCACTTACAGTCTG TCCAGCACATTGACACTGAGTAAGGCTGATTACGAAAAACACAAGGTGTACGCATGCGA GGTGACACACCAAGGTCTTTCATCTCCTGTAACTAAGAGCTTTAACCGGGGAGAATGTG GTGGTGGGGGCAGCGGGGGCGGAGGTAGTGGAGGCGGCGGTAGTGAACCAAAGAGTAGT GACAAAACTCACACGTGCCCACCGTGCCCAGCACCTGAACTCCTGGGGGGACCGTCAGT CTTCCTCTTCCCCCCAAAACCCAAGGACACCCTCATGATCTCCCGGACCCCTGAGGTCA CATGCGTGGTGGTGGACGTGAGCCACGAAGACCCTGAGGTCAAGTTCAACTGGTACGTG GACGGCGTGGAGGTGCATAATGCCAAGACAAAGCCGCGGGAGGAGCAGTACAACAGCAC GTACCGTGTGGTCAGCGTCCTCACCGTCCTGCACCAGGACTGGCTGAATGGCAAGGAGT ACAAGTGCAAGGTCTCCAACAAAGCCCTCCCAGCCCCCATCGAGAAAACCATCTCCAAA GCCAAAGGGCAGCCCCGAGAACCACAGGTGTACACCCTGCCCCCCATGCCGGGAGGAGAT GACCAAGAACCAGGTCAGCCTGTGGTGCCTGGTCAAAGGCTTCTATCCCAGCGACATCG CCGTGGAGTGGGAGAGCAATGGGCAGCCGGAGAACAACTACAAGACCACGCCTCCCGTG CTGGACTCCGACGGCTCCTTCTTCCTCTACAGCAAGCTCACCGTGGACAAGAGCAGGTG GCAGCAGGGGAACGTCTTCTCATGCTCCGTGATGCATGAGGCTCTGCACAACCACTACA CGCAGAAGAGCCTCTCCCTGTCTCCGGGTAAA | |
| TL-L-PLC-Knob | GACATTCAGATGACCCAATCCCCCTCTAGCCTTTCAGCCTCCGTAGGGGACAGAGTTAC AATAACCTGTAGGGCTAGTCAGGACGTTAATACAGCAGTCGCATGGTACCAGCAGAAGC CCGGTAAGGCTCCTAAGCTTTTGATCTACAGTGCTTCTTTCCTGTATTCTGGTGTACCT AGCCGATTCTCAGGCTCTCGGAGTGGCACAGACTTCACTCTGACAATTTCAAGTCTCCA GCCAGAAGATTTTGCAACCTACTACTGCCAACAACACTACACTACTCCTCCAACCTTCG GACAGGGCACAAAAGTAGAGATTAAGCGTACGGTGGCTGCTCCATCCGTTTTTATCTTT CCCCCAGATATTCAAATGACCCAAAGTCCAAGTTCCCTTTCAGCATCTGTCGGTGATCG AGTCACTATAACTTGTAAGGCCAGCCAAGACGTTAGCATAGGAGTAGCATGGTACCAAC AAAAGCCTGGGAAGGCTCCCAAACTTCTCATTTATTCTGCTTCCTACCGATATACTGGT | 502 |

| | GTCCCAAGTAGATTTTCTGGCAGCGGATCTGGAACTGATTTTACATTGACTATCAGCTC CCTCCAGCCTGAGGACTTCGCTACTTATTACTGCCAACAGTACTACATTTATCCCTATA CATTCGGTTGTGGGACCAAAGTAGAGATCAAACGTACGGTGGCAGCTCCCAGCGTTTTT ATCTTTCCCCCATCCGACGAGCAGCTCAAGAGTGGCACTGCCTCTGTAGTTTGTTTGCT GAATAACTTCTATCCACGTGAAGCAAAAGTACAGTGGAAGGTCGATAATGCCCTTCAGA GCGGTAACAGCCAAGAAAGTGTTACCGAGCAAGATTCCAAAGATTCCACTTACAGTCTG TCCAGCACATTGACACTGAGTAAGGCTGATTACGAAAAACACAAGGTGTACGCATGCGA GGTGACACACCAAGGTCTTTCATCTCCTGTAACTAAGAGCTTTAACCGGGGAGAATGTG GTGGTGGGGGCAGCGGGGGCGGAGGTAGTGGAGGCGGCGGTAGTGAACCAAAGAGTAGT GACAAAACTCACACGTGCCCACCGTGCCCAGCACCTGAACTCCTGGGGGGACCGTCAGT CTTCCTCTTCCCCCCAAAACCCAAGGACACCCTCATGATCTCCCGGACCCCTGAGGTCA CATGCGTGGTGGTGGACGTGAGCCACGAAGACCCTGAGGTCAAGTTCAACTGGTACGTG GACGGCGTGGAGGTGCATAATGCCAAGACAAAGCCGCGGGAGGAGCAGTACAACAGCAC GTACCGTGTGGTCAGCGTCCTCACCGTCCTGCACCAGGACTGGCTGAATGGCAAGGAGT ACAAGTGCAAGGTCTCCAACAAAGCCCTCCCAGCCCCCATCGAGAAAACCATCTCCAAA GCCAAAGGGCAGCCCCGAGAACCACAGGTGTACACCCTGCCCCCCATGCCGGGAGGAGAT GACCAAGAACCAGGTCAGCCTGTGGTGCCTGGTCAAAGGCTTCTATCCCAGCGACATCG CCGTGGAGTGGGAGAGCAATGGGCAGCCGGAGAACAACTACAAGACCACGCCTCCCGTG CTGGACTCCGACGGCTCCTTCTTCCTCTACAGCAAGCTCACCGTGGACAAGAGCAGGTG GCAGCAGGGGAACGTCTTCTCATGCTCCGTGATGCATGAGGCTCTGCACAACCACTACA CGCAGAAGAGCCTCTCCCTGTCTCCGGGTAAA | |
| TLC-L-PLC-Knob | GACATTCAGATGACCCAATCCCCCCTCTAGCCTTTCAGCCTCCGTAGGGGACAGAGTTAC AATAACCTGTAGGGCTAGTCAGGACGTTAATACAGCAGTCGCATGGTACCAGCAGAAGC CCGGTAAGGCTCCTAAGCTTTTTGATCTACAGTGCTTCTTTCCTGTATTCTGGTGTACCT AGCCGATTCTCAGGCTCTCGGAGTGGCACAGACTTCACTCTGACAATTTCAAGTCTCCA GCCAGAAGATTTTGCAACCTACTACTGCCAACAACACTACACTACTCCTCCAACCTTCG GATGTGGCACAAAAGTAGAGATTAAGCGTACGGTGGCTGCTCCATCCGTTTTTATCTTT CCCCCAGATATTCAAATGACCCAAAGTCCAAGTTCCCTTTCAGCATCTGTCGGTGATCG AGTCACTATAACTTGTAAGGCCAGCCAAGACGTTAGCATAGGAGTAGCATGGTACCAAC | 503 |

AAAAGCCTGGGAAGGCTCCCAAACTTCTCATTTATTCTGCTTCCTACCGATATACTGGT
GTCCCAAGTAGATTTTCTGGCAGCGGATCTGGAACTGATTTTACATTGACTATCAGCTC
CCTCCAGCCTGAGGACTTCGCTACTTATTACTGCCAACAGTACTACATTTATCCCTATA
CATTCGGTTGTGGGACCAAAGTAGAGATCAAACGTACGGTGGCAGCTCCCAGCGTTTTT
ATCTTTCCCCCATCCGACGAGCAGCTCAAGAGTGGCACTGCCTCTGTAGTTTGTTTGCT
GAATAACTTCTATCCACGTGAAGCAAAAGTACAGTGGAAGGTCGATAATGCCCTTCAGA
GCGGTAACAGCCAAGAAAGTGTTACCGAGCAAGATTCCAAAGATTCCACTTACAGTCTG
TCCAGCACATTGACACTGAGTAAGGCTGATTACGAAAAACACAAGGTGTACGCATGCGA
GGTGACACACCAAGGTCTTTCATCTCCTGTAACTAAGAGCTTTAACCGGGGAGAATGTG
GTGGTGGGGGCAGCGGGGGCGGAGGTAGTGGAGGCGGCGGTAGTGAACCAAAGAGTAGT
GACAAAACTCACACGTGCCCACCGTGCCCAGCACCTGAACTCCTGGGGGGACCGTCAGT
CTTCCTCTTCCCCCCAAAACCCAAGGACACCCTCATGATCTCCCGGACCCCTGAGGTCA
CATGCGTGGTGGTGGACGTGAGCCACGAAGACCCTGAGGTCAAGTTCAACTGGTACGTG
GACGGCGTGGAGGTGCATAATGCCAAGACAAAGCCGCGGGAGGAGCAGTACAACAGCAC
GTACCGTGTGGTCAGCGTCCTCACCGTCCTGCACCAGGACTGGCTGAATGGCAAGGAGT
ACAAGTGCAAGGTCTCCAACAAAGCCCTCCCAGCCCCCATCGAGAAAACCATCTCCAAA
GCCAAAGGGCAGCCCCGAGAACCACAGGTGTACACCCTGCCCCCATGCCGGGAGGAGAT
GACCAAGAACCAGGTCAGCCTGTGGTGCCTGGTCAAAGGCTTCTATCCCAGCGACATCG
CCGTGGAGTGGGAGAGCAATGGGCAGCCGGAGAACAACTACAAGACCACGCCTCCCGTG
CTGGACTCCGACGGCTCCTTCTTCCTCTACAGCAAGCTCACCGTGGACAAGAGCAGGTG
GCAGCAGGGGAACGTCTTCTCATGCTCCGTGATGCATGAGGCTCTGCACAACCACTACA
CGCAGAAGAGCCTCTCCCTGTCTCCGGGTAAA

[Table 34]

EP 4 700 047 A1

|  | VH1-Linker-VH2-CH1-Fc(Knob) | VL1-Linker-VL2-CL-Linker-Fc(Knob) | Fc(Hole) |
|---|---|---|---|
| HP501 | SEQ ID NO: 51 | SEQ ID NO: 59 | SEQ ID NO: 7 |
| HP502 | SEQ ID NO: 52 | SEQ ID NO: 60 | SEQ ID NO: 7 |
| HP503 | SEQ ID NO: 53 | SEQ ID NO: 61 | SEQ ID NO: 7 |
| HP504 | SEQ ID NO: 51 | SEQ ID NO: 62 | SEQ ID NO: 7 |
| HP505 | SEQ ID NO: 51 | SEQ ID NO: 63 | SEQ ID NO: 7 |
| HP506 | SEQ ID NO: 52 | SEQ ID NO: 64 | SEQ ID NO: 7 |
| HP507 | SEQ ID NO: 53 | SEQ ID NO: 65 | SEQ ID NO: 7 |
| HP508 | SEQ ID NO: 54 | SEQ ID NO: 66 | SEQ ID NO: 7 |
| HP509 | SEQ ID NO: 55 | SEQ ID NO: 59 | SEQ ID NO: 7 |
| HP510 | SEQ ID NO: 56 | SEQ ID NO: 60 | SEQ ID NO: 7 |
| HP511 | SEQ ID NO: 57 | SEQ ID NO: 61 | SEQ ID NO: 7 |
| HP512 | SEQ ID NO: 58 | SEQ ID NO: 62 | SEQ ID NO: 7 |
| HP513 | SEQ ID NO: 55 | SEQ ID NO: 63 | SEQ ID NO: 7 |
| HP514 | SEQ ID NO: 56 | SEQ ID NO: 64 | SEQ ID NO: 7 |
| HP515 | SEQ ID NO: 57 | SEQ ID NO: 65 | SEQ ID NO: 7 |
| HP516 | SEQ ID NO: 58 | SEQ ID NO: 66 | SEQ ID NO: 7 |

The purity of each antibody prepared as above was analyzed by size exclusion chromatography in the same manner as in Example 1 above.

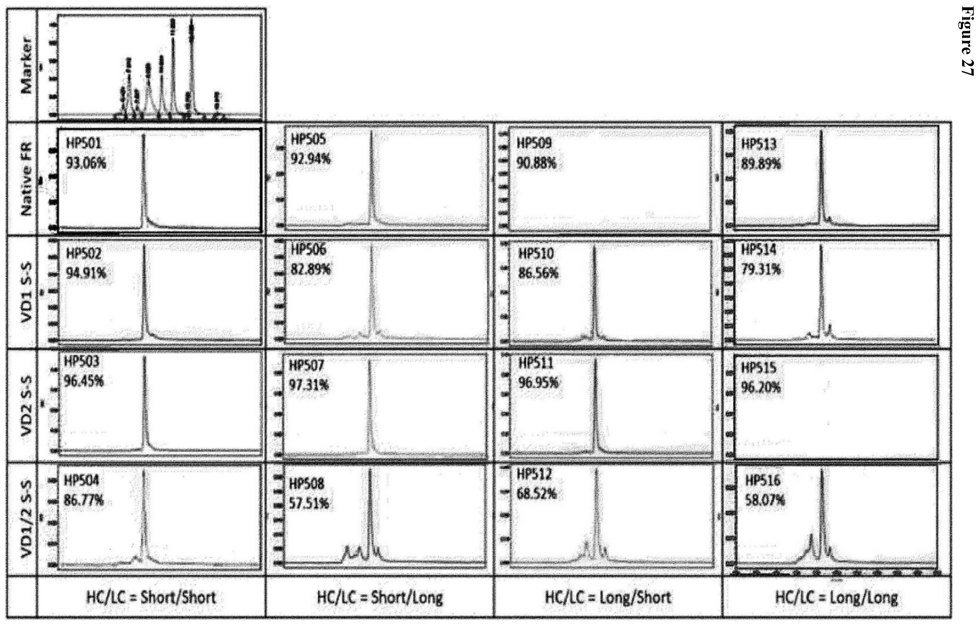

As shown in Figure 27 and Table 35, the analysis showed that HP503, HP507, HP511, and HP515, in which the first V domain is wild type and the second V domain is Cys substituted variant (VH 44C, VL 100C), have an excellent purity.

[Table 35]

| Clone name | Monomer purity (%) |
|---|---|
| HP501 | 93.06 |
| HP502 | 94.91 |
| HP503 | 96.45 |
| HP504 | 86.77 |
| HP505 | 92.94 |
| HP506 | 82.89 |
| HP507 | 97.31 |
| HP508 | 57.51 |
| HP509 | 90.88 |
| HP510 | 86.56 |
| HP511 | 96.95 |
| HP512 | 68.52 |
| HP513 | 89.89 |
| HP514 | 79.31 |
| HP515 | 96.20 |
| HP516 | 58.07 |

The binding constants of each of HP501 to HP516 to the D2 region and the D4 region of the HER2 protein were determined using Octet Red96e.

Specifically, the human HER2 recombinant protein (R&D systems, 1129-ER) was loaded onto the Anti-Penta-HIS (HIS1K) biosensor, and then saturated with trastuzumab (100 nM) which targets the D2 region or pertuzumab which targets the D4 region.

Thereafter, each of the sixteen antibodies was added in a binding reaction (300 seconds) and a dissociation reaction (600 seconds) at a concentration of 100 nM, respectively, and their binding affinities for the D2 region and the D4 region were calculated, respectively.

As shown in Table 36, the binding constants of HP507, HP511, and HP515 to the D2 region were 2.285 nM, 3.267 nM, and 2.012 nM, respectively, showing excellent binding affinities to the D2 region compared to other clones. HP503 has a binding constant of 8.098 nM to the D2 region and shows a relatively low binding affinity to the D2 region compared to HP507, HP511, and HP515. On the other hand, HP503 has a binding constant of 0.181 nM to the D4 region, demonstrating a high binding affinity, compared to HP507 (0.228 nM), HP511 (0.162 nM), and HP515 (0.227 nM).

[Table 36]

| 100 nM Single Kinetics | Binding kinetics of mAbs to D2 epitope | | | Binding kinetics of mAbs to D4 epitope | | |
|---|---|---|---|---|---|---|
| | $K_D$ (nM) | $K_a$ (1/Ms) | $K_d$ (1/s) | $K_D$ (nM) | $K_a$ (1/Ms) | $K_d$ (1/s) |
| HP501 | 13.009 | 8.05E+03 | 1.05E-04 | 0.218 | 2.00E+05 | 4.36E-05 |
| HP502 | 8.433 | 8.97E+03 | 7.56E-05 | 0.531 | 1.47E+05 | 7.82E-05 |
| HP503 | 8.098 | 4.69E+03 | 3.80E-05 | 0.181 | 2.14E+05 | 3.87E-05 |
| HP504 | 7.304 | 2.53E+04 | 1.85E-04 | 0.788 | 1.13E+05 | 8.94E-05 |
| HP505 | 5.882 | 1.41E+04 | 8.30E-05 | 0.586 | 1.50E+05 | 8.79E-05 |
| HP506 | 5.682 | 1.21E+04 | 6.89E-05 | 0.611 | 1.37E+05 | 8.37E-05 |
| HP507 | 2.285 | 1.85E+04 | 4.23E-05 | 0.228 | 1.99E+05 | 4.53E-05 |
| HP508 | 6.490 | 2.36E+04 | 1.53E-04 | 1.219 | 8.44E+04 | 1.03E-04 |
| HP509 | 7.962 | 1.33E+04 | 1.06E-04 | 0.393 | 1.72E+05 | 6.77E-05 |
| HP510 | 10.736 | 9.86E+03 | 1.06E-04 | 0.336 | 1.97E+05 | 6.60E-05 |
| HP511 | 3.267 | 1.47E+04 | 4.81E-05 | 0.162 | 2.27E+05 | 3.67E-05 |
| HP512 | 7.615 | 2.80E+04 | 2.13E-04 | 0.667 | 1.12E+05 | 7.47E-05 |
| HP513 | 4.739 | 2.17E+04 | 1.03E-04 | 0.381 | 1.95E+05 | 7.43E-05 |
| HP514 | 5.920 | 1.78E+04 | 1.06E-04 | 0.451 | 1.74E+05 | 7.82E-05 |
| HP515 | 2.012 | 2.89E+04 | 5.81E-05 | 0.227 | 2.14E+05 | 4.87E-05 |
| HP516 | 5.094 | 3.29E+04 | 1.67E-04 | 0.607 | 1.23E+05 | 7.46E-05 |

The binding constants of each of HP503, HP507, HP511, and HP515 to the HER2 extracellular domain (ECD) were measured using Octet Red96e.

Specifically, the human HER2 recombinant protein was loaded onto the Anti-Penta-HIS (HIS1K) biosensor, and then each of the above antibodies at various concentrations (0.25 nM, 0.5 nM, 1 nM, 2 nM, 4 nM, 8 nM) were added in a binding reaction (600 seconds) and a dissociation reaction (1800 seconds). The binding constants were calculated based on the results obtained through the above binding and dissociation reactions.

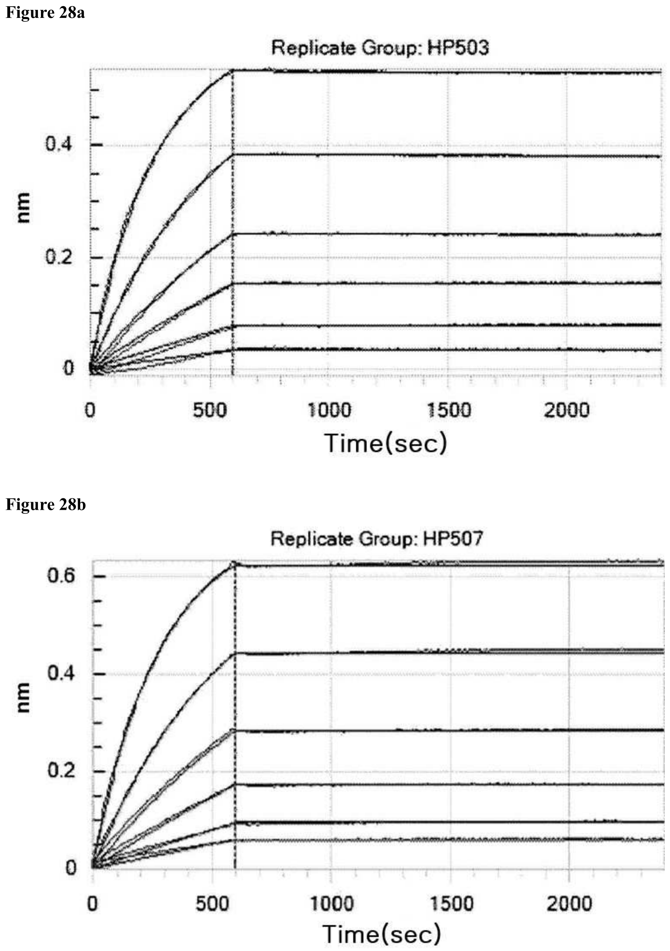

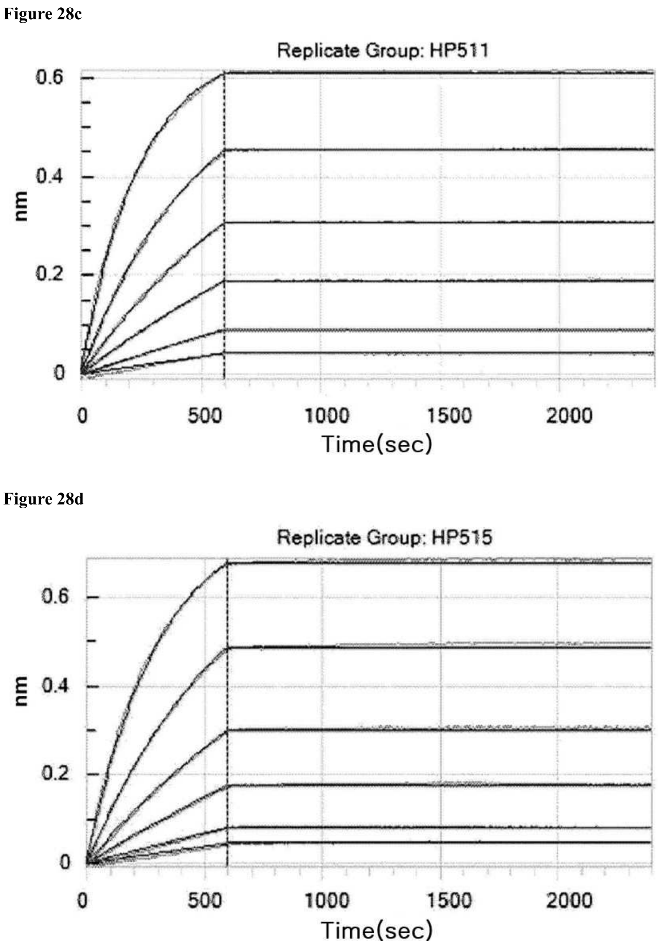

As shown in Figures 28a to 28d and Table 37, under the above conditions, HP503 had a binding constant of 6.57 nM, and HP507, HP511, and HP515 had dissociation constants (<1.0E-07 1/s) that exceeded the measurement limit of the equipment.

[Table 37]

| | $K_D$ (M) | $K_a$ (1/Ms) | $K_d$ (1/s) |
|---|---|---|---|
| HP503 | 6.57E-12 | 4.69E+05 | 3.08E-06 |
| HP507 | <1.0E-12 | 4.58E+05 | <1.0E-07 |
| HP511 | <1.0E-12 | 5.20E+05 | <1.0E-07 |
| HP515 | <1.0E-12 | 4.14E+05 | <1.0E-07 |

The binding constants of HP503, HP507, HP511, and HP515 to the Fc gamma receptors were analyzed using Octet Red96e in the same manner as above.

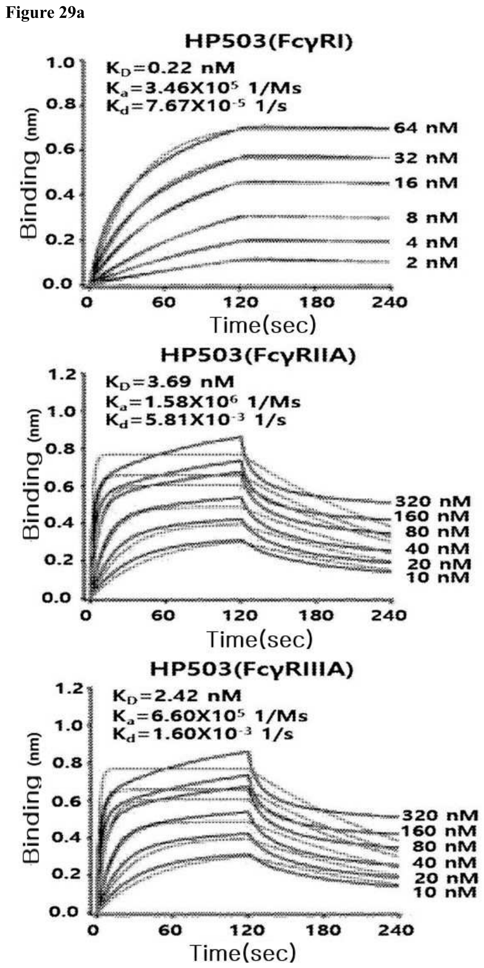

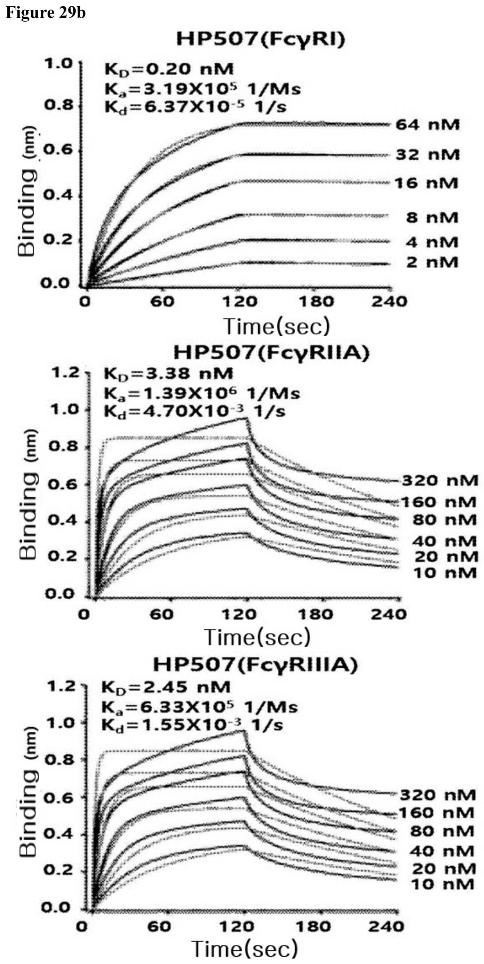

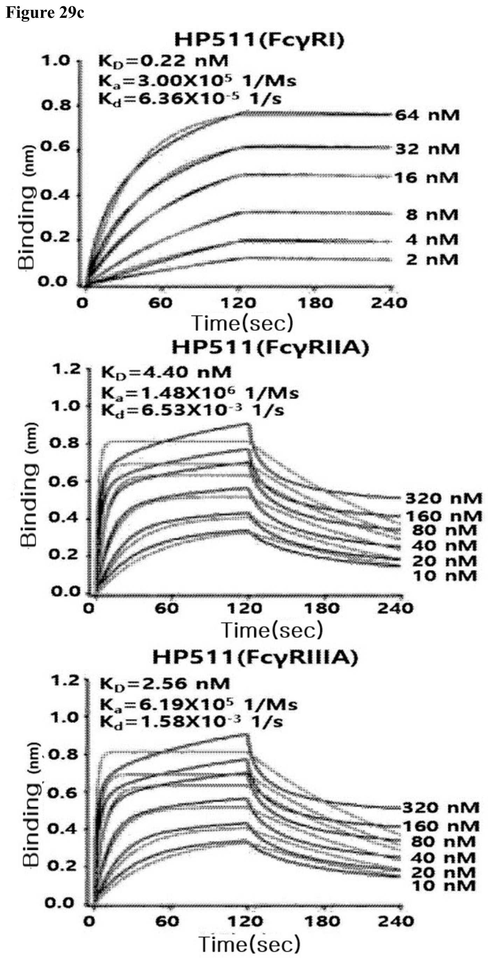

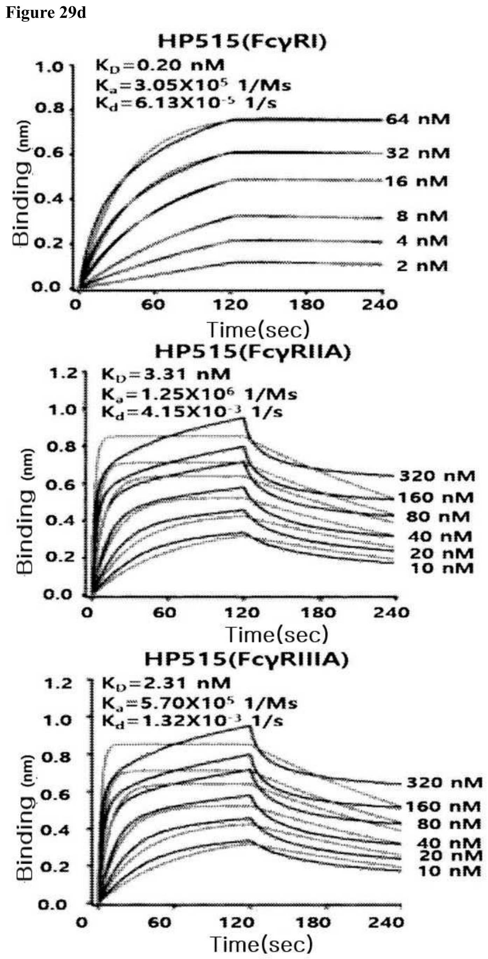

As shown in Figures 29a to 29d and Table 38, the analysis showed that HP503, HP507, HP511, and HP515 have excellent binding affinities to FcγRI (CD64), FcγRIIA (CD32A, 131R), or FcγRIIIA (CD16A, 158V) compared to human IgG1, trastuzumab, pertuzumab, and margetuximab (Figures 21a to 21h, Figures 22a to 22h, Figures 23a to 23h, Figures 29a to 29d, and Tables 29 and 38).

[Table 38]

| Antigen | Fc γ RI (CD64) | | | Fc γ RIIA (CD32A, 131R) | | | Fc γ RIIIA (CD16A,176V) | | |
|---|---|---|---|---|---|---|---|---|---|
| Antibody | KD (nM) | Ka (1/Ms) | Kd (1/s) | KD (nM) | Ka (1/Ms) | Kd (1/s) | KD (nM) | Ka (1/Ms) | Kd (1/s) |
| HP503 | 0.22 | 3.46E+05 | 7.67E-05 | 3.69 | 1.58E+06 | 5.81E-03 | 2.42 | 6.60E+05 | 1.60E-03 |
| HP507 | 0.20 | 3.19E+05 | 6.37E-05 | 3.38 | 1.39E+06 | 4.70E-03 | 2.45 | 6.33E+05 | 1.55E-03 |
| HP511 | 0.22 | 3.00E+05 | 6.63E-05 | 4.40 | 1.48E+06 | 6.53E-03 | 2.56 | 6.19E+05 | 1.58E-03 |
| HP515 | 0.20 | 3.05E+05 | 6.13E-05 | 3.31 | 1.25E+06 | 4.15E-03 | 2.31 | 5.70E+05 | 1.32E-03 |

The binding constants of each of HP503, HP507, HP511, and HP515 to the neonatal Fc receptor (FcRn) were measured using Octet Red96e.

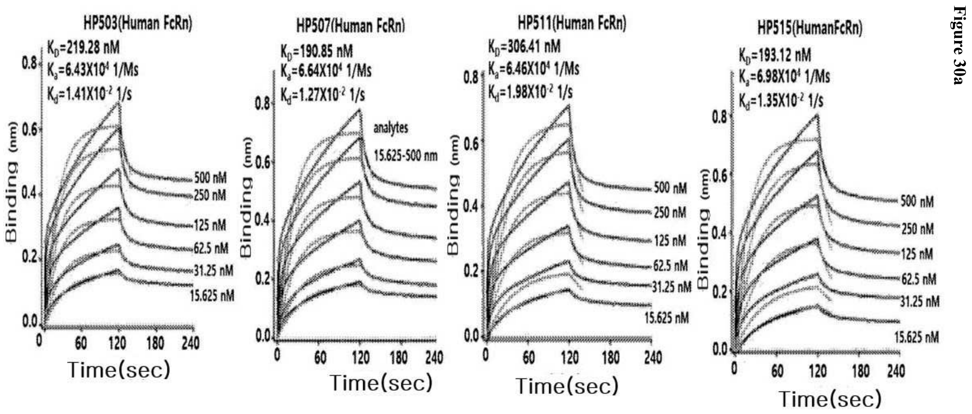

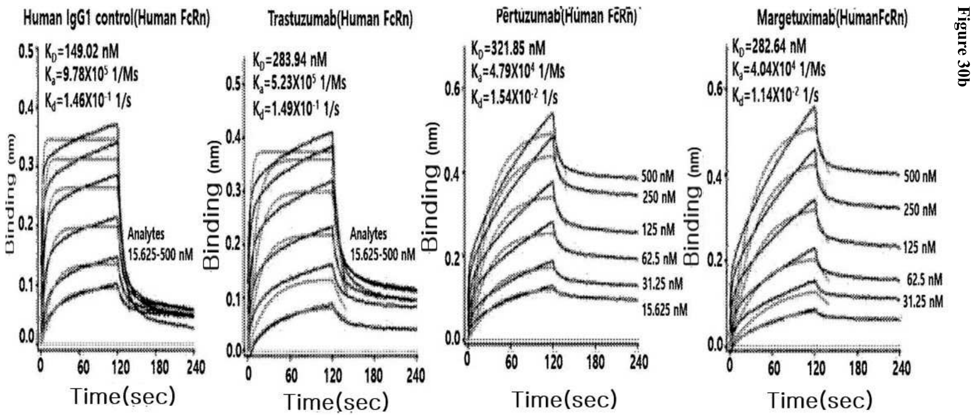

Specifically, HP503, HP507, HP511, HP515, human IgG1 (Bio X cell, BE0297), trastuzumab, pertuzumab, or margetuximab was loaded onto the anti-human Fab-CH1 2nd generation (FAB2G) biosensor, and then a binding reaction (120 seconds) and a dissociation reaction (120 seconds) were performed. For analysis, Kinetics buffer (Sartorius, 18-1105) was used at pH 6.0. The binding constants were calculated from the above analysis results (Figure 30a, Figure 30b, and Table 39).

[Table 39]

| Antibodies | $K_D$ (nM) | $K_a$ (1/Ms) | $K_d$ (1/s) |
|---|---|---|---|
| HP503 | 219.28 | 6.43E+04 | 1.41E-02 |
| HP507 | 190.85 | 6.64E+04 | 1.27E-02 |
| HP511 | 306.41 | 6.46E+04 | 1.98E-02 |
| HP515 | 193.12 | 6.98E+04 | 1.35E-02 |
| Human IgG1 | 149.02 | 9.78E+05 | 1.46E-01 |
| Trastuzumab | 283.94 | 5.23E+05 | 1.49E-01 |
| Pertuzumab | 321.85 | 4.79E+04 | 1.54E-02 |
| Margetuximab | 282.64 | 4.04E+04 | 1.14E-02 |

### Example 13. Analysis of complement dependent cytotoxicity

For the analysis of complement dependent cytotoxicity was performed in the BT474 (HER2 3+; High) breast cancer cell line or the NCI-N87 (HER2 3+; High) gastric cancer cell line.

Specifically, the above cell lines were inoculated into a 96-well plate at 10,000 cells per well, and then the cells, antibodies, and human serum (Sigma, H4522) were treated and reacted at a volume ratio of 1:1:1. Human IgG1, trastuzumab (TRA), trastuzumab + pertuzumab (TRA + PER), H01, or H01 + P01 was used as an antibody, and the antibodies were serially diluted by a factor of two six times from the initial concentration of 1200 nM and used as three-fold concentrates. The human serum was treated at the final concentration of 25%. The cells treated as above were reacted for 5 hours in a humid incubator under 37 °C and 5% (v/v) $CO_2$ conditions.

After completion of the reaction, the cells were treated with Cell Titer Glo-Reagent (Promega, G9243) in the same amount as the cell culture medium, and then the cells were lysed using a plate shaker (Allsheng, MX100-4A) (500 rpm, 2 minutes). In order to stabilize the luminescence signal, the mixture was left at room temperature for 10 minutes, and then luminescence was measured using a plate reader (Envision; PerkinElmer, 2105-0010) equipment, and complement dependent cytotoxicity activity (CDC activity) was calculated using Equation 1 below.

**CDC activity (%) = 100 × [1-(luminescence with experimental antibody/luminescence without antibody)]**              <Equation 1>

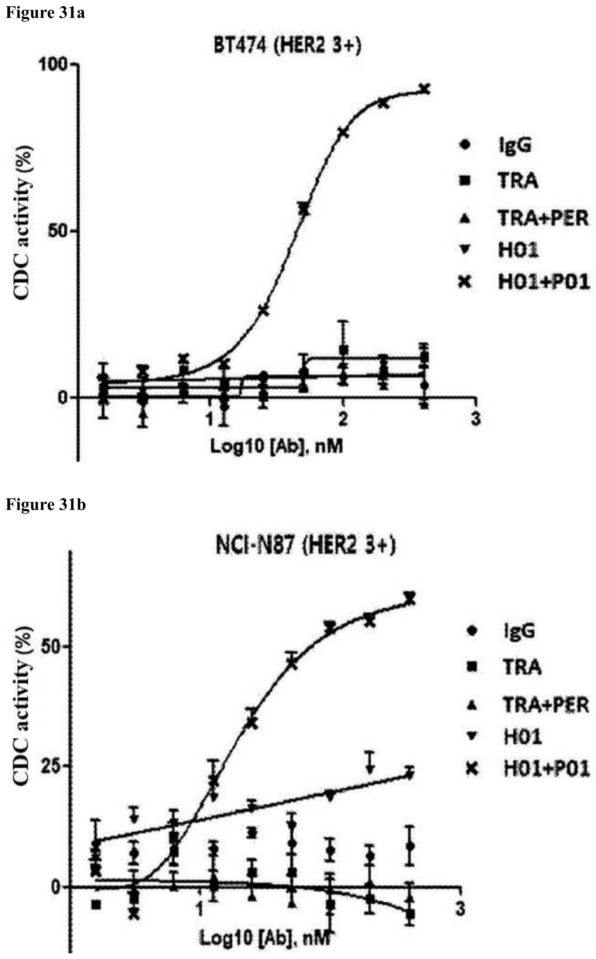

As shown in Figures 31a and 31b, a CDC response was not induced in the BT474 cells when treated with TRA, TRA + PER, or H01. In addition, a CDC response was not induced even in NCI-N87 cells when treated with human IgG1, TRA, or TRA + PER. On the other hand, it was confirmed that when treated with H01 + P01 in combination, a strong CDC response was induced in both BT474 cells and NCI-N87 cells.

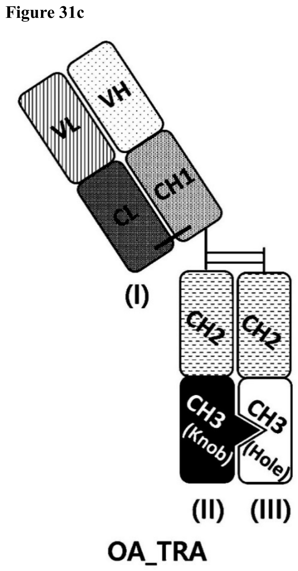

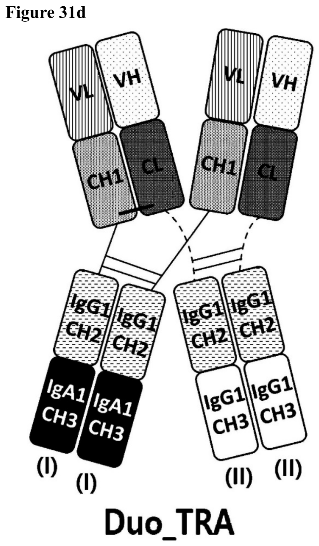

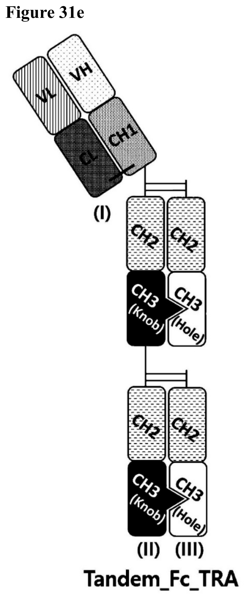

Similar to the present invention, technologies for increasing the Fc loading and effector function of antibodies that bind to the cell surface include Monovalent IgG1, DuoMab, and Tandem Fc antibody technologies. In order to compare the CDC inducing ability of the antibody of the present invention and the antibody to which these technologies were applied, Monovalent IgG1 (OA _TRA), DuoMab (Duo_TRA), and Tandem Fc antibody (Tandem_Fc_TRA) comprising trastuzumab Fab were constructed (Figure 31c to Figure 31e). OA_TRA consists of the polypeptides of OA_TRA_1, OA_TRA_2, and OA_TRA_3 (Figure 31c, Table 40, and Table 41). Duo_TRA consists of the polypeptides of Duo_TRA_1 and Duo_TRA_2 (Figure 31d, Table 40, and Table 41), and Tandem_Fc_TRA consists of the polypeptides of Tandem_Fc_TRA_1, Tandem_Fc_TRA_2, and Tandem_Fc_TRA_3 (Figure 31e, Table 40, and Table 41).

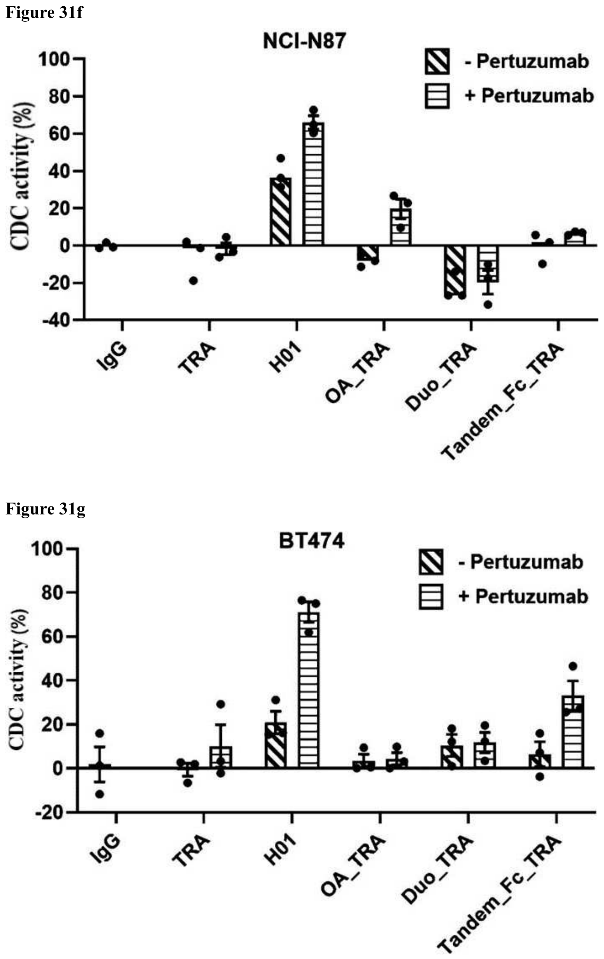

When the NCI-N87 and BT474 cell lines were treated with 200 nM IgG, TRA, H01, OA_TRA, Duo_TRA, or Tandem _Fc_TRA alone or in combination with 200 nM pertuzumab, the highest CDC response was induced in the group treated with H01 alone (Figures 31f and 31g).

Table 40 below shows the polypeptide sequences constituting OA_TRA, Duo_TRA, and Tandem_Fc_TRA.

[Table 40]

| Name | Sequence | SEQ ID NO |
|------|----------|-----------|
| OA_TRA_1 | DIQMTQSPSSLSASVGDRVTITCRASQDVNTAVAWYQQKPGKAPKLLIYSASFLYSGVP SRFSGSRSGTDFTLTISSLQPEDFATYYCQQHYTTPPTFGQGTKVEIKRTVAAPSVFIF PPSDEQLKSGTASVVCLLNNFYPREAKVQWKVDNALQSGNSQESVTEQDSKDSTYSLSS TLTLSKADYEKHKVYACEVTHQGLSSPVTKSFNRGEC | 2 |
| OA_TRA_2 | EVQLVESGGGLVQPGGSLRLSCAASGFNIKDTYIHWVRQAPGKGLEWVARIYPTNGYTR | 8 |

| | | |
|---|---|---|
| | YADSVKGRFTISADTSKNTAYLQMNSLRAEDTAVYYCSRWGGDGFYAMDYWGQGTLVTV SSASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCDKTHTCPPCPAPE LLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTL PPCREEMTKNQVSLWCLVKGFYPSDIAVEWESNGQPENNYKTTPPVLDSDGSFFLYSKL TVDKSRWQQGNVFSCSVMHEALHNHYTQKSLSLSPGK | |
| OA_TRA_3 | DKTHTCPPCPAPELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTISK AKGQPREPQVCTLPPSREEMTKNQVSLSCAVKGFYPSDIAVEWESNGQPENNYKTTPPV LDSDGSFFLVSKLTVDKSRWQQGNVFSCSVMHEALHNHYTQKSLSLSPGK | 7 |
| Duo_TRA_1 | DIQMTQSPSSLSASVGDRVTITCRASQDVNTAVAWYQQKPGKAPKLLIYSASFLYSGVP SRFSGSRSGTDFTLTISSLQPEDFATYYCQQHYTTPPTFGQGTKVEIKSSASTKGPSVF PLAPSSKSTSGGTAALGCLVKDYFPEPVTVSWNSGALTSGVHTFPAVLQSSGLYSLSSV VTVPSSSLGTQTYICNVNHKPSNTKVDKKVEPKSCDKTHTCPPCPAPELLGGPSVFLFP PKPKDTLMISRTPEVTCVVVDVSHEDPEVKFNWYVDGVEVHNAKTKPREEQYNSTYRVV SVLTVLHQDWLNGKEYKCKVSNKALPAPIEKTISKAKGNTFRPEVHLLPPPSEELALNE LVTLTCLARGFSPKDVLVRWLQGSQELPREKYLTWASRQEPSQGTTTFAVTSILRVAAE DWKKGDTFSCMVGHEALPLAFTQKTIDRLAGK | 943 |
| Duo_TRA_2 | EVQLVESGGGLVQPGGSLRLSCAASGFNIKDTYIHWVRQAPGKGLEWVARIYPTNGYTR YADSVKGRFTISADTSKNTAYLQMNSLRAEDTAVYYCSRWGGDGFYAMDYWGQGTLVTV SSASVAAPSVFIFPPSDEQLKSGTASVVCLLNNFYPREAKVQWKVDNALQSGNSQESVT EQDSKDSTYSLSSTLTLSKADYEKHKVYACEVTHQGLSSPVTKSFNRGECDKTHTCPPC PAPELLGGPSVFLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTISKAKGQPREPQ VYTLPPSREEMTKNQVSLTCLVKGFYPSDIAVEWESNGQPENNYKTTPPVLDSDGSFFL YSKLTVDKSRWQQGNVFSCSVMHEALHNHYTQKSLSLSPGK | 944 |
| Tandem_Fc_TRA_1 | DIQMTQSPSSLSASVGDRVTITCRASQDVNTAVAWYQQKPGKAPKLLIYSASFLYSGVP SRFSGSRSGTDFTLTISSLQPEDFATYYCQQHYTTPPTFGQGTKVEIKRTVAAPSVFIF | 2 |

|  | PPSDEQLKSGTASVVCLLNNFYPREAKVQWKVDNALQSGNSQESVTEQDSKDSTYSLSSTLTLSKADYEKHKVYACEVTHQGLSSPVTKSFNRGEC |  |
|---|---|---|
| Tandem_Fc_TRA_2 | EVQLVESGGGLVQPGGSLRLSCAASGFNIKDTYIHWVRQAPGKGLEWVARIYPTNGYTRYADSVKGRFTISADTSKNTAYLQMNSLRAEDTAVYYCSRWGGDGFYAMDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCRDKLTKNQVSLWCLVKGFYPSDIAVEWESNGQPENNYKTTPPVLDSDGSFFLYSKLTVDKSRWQQGNVFSCSVMHEALHNHYTQKSLSLSPGKGGGGGGGGGGGGGGGGGGGGGGGDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCRDKLTKNQVSLWCLVKGFYPSDIAVEWESNGQPENNYKTTPPVLDSDGSFFLYSKLTVDKSRWQQGNVFSCSVMHEALHNHYTQKSLSLSPG | 945 |
| Tandem_Fc_TRA_3 | DKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVCTLPPSREEMTKNQVSLSCAVKGFYPSDIAVEWESNGQPENNYKTTPPVLDSDGSFFLVSKLTVDKSRWQQGNVFSCSVMHEALHNHYTQKSLSLSPGK | 7 |

Table 41 shows the polynucleotide sequences encoding the polypeptides constituting OA_TRA, DUO_TRA, and Tandem_Fc_TRA.

[Table 41]

| Name | Sequence | SEQ ID NO |
|------|----------|-----------|
| OA_TRA_1 | GATATTCAGATGACTCAGAGTCCTAGTTCCCTCAGCGCCTCCGTAGGCGACAGAGTTAC AATAACTTGCCGAGCAAGCCAAGACGTAAACACTGCAGTCGCCTGGTACCAACAGAAAC CAGGCAAAGCTCCAAAACTCTTGATTTACAGTGCTTCCTTCCTTTATAGTGGCGTTCCA AGCCCGCTTCAGCGGCAGCCGCTCTGGCACCGACTTCACTCTCACTATTTCTTCCTTGCA ACCTGAAGACTTCGCCACTTATTATTGCCAGCAACACTACACAACACCCCCAACATTCG GACAGGGCACAAAGGTAGAAATAAAACGTACGGTGGCAGCTCCCAGCGTTTTTATCTTT CCCCCATCCGACGAGCAGCTCAAGAGTGGCACTGCCTCTGTAGTTTGTTTGCTGAATAA CTTCTATCCACGTGAAGCAAAAGTACAGTGGAAGGTCGATAATGCCCTTCAGAGCGGTA ACAGCCAAGAAAGTGTTACCGAGCAAGATTCCAAAGATTCCACTTACAGTCTGTCCAGC ACATTGACACTGAGTAAGGCTGATTACGAAAAACACAAGGTGTACGCATGCGAGGTGAC ACACCAAGGTCTTTCATCTCCTGTAACTAAGAGCTTTAACCGGGGAGAATGT | 453 |
| OA_TRA_2 | GAAGTTCAACTTGTGGAGTCTGGAGGCGGTTTGGTACAACCTGGAGGTTCACTTCGTTT GTCATGTGCTGCCTCTGGGTTTAATATCAAGGATACTTATATTCATTGGGTTCGCCAGG CACCCGGAAAAGGGCTGGAATGGGTGGCCCGTATATATCCCACTAACGGTTACACTCGA TATGCTGACTCTGTAAAGGGTCGATTTACTATATCCGCTGATACTAGCAAAAACACTGC CTACCTCCAGATGAACAGTCTGCGCGCCGAGGATACAGCAGTTTATTATTGTTCCCGAT GGGGAGGTGACGGGTTCTATGCCATGGACTACTGGGGTCAAGGGACACTGGTAACCGTT TCTTCTGCTAGCACCAAAGGACCTAGTGTTTTTCCTCTTGCCCCTTCCTCAAAGTCTAC CTCTGGGGGGACAGCCGCTCTGGGCTGCCTGGTCAAGGATTATTTCCCAGAGCCTGTCA CTGTCAGTTGGAACTCTGGAGCCTTGACTTCTGGTGTTCATACATTTCCTGCTGTCCTT CAAAGCAGCGGCTTGTACTCATTGTCTTCTGTTGTGACAGTACCCTCAAGCAGCCTCGG CACTCAGACATACATCTGCAATGTCAACCACAAACCCTCAAATACAAAGGTAGATAAAA AAGTCGAACCAAAGTCTTGTGACAAAACTCACACGTGCCCACCGTGCCCAGCACCTGAA CTCCTGGGGGGACCGTCAGTCTTCCTCTTCCCCCCAAAACCCAAGGACACCCTCATGAT CTCCCGGACCCCTGAGGTCACATGCGTGGTGGTGGACGTGAGCCACGAAGACCCTGAGG TCAAGTTCAACTGGTACGTGGACGGCGTGGAGGTGCATAATGCCAAGACAAAGCCGCGG GAGGAGCAGTACAACAGCACGTACCGTGTGGTCAGCGTCCTCACCGTCCTGCACCAGGA | 459 |

| | | |
|---|---|---|
| | CTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATGCCGGGAGGAGATGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | |
| OA_TRA_3 | GACAAAACTCACACATGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTGCACCCTGCCCCCCATCCCGGGAGGAGATGACCAAGAACCAGGTCAGCCTGAGCTGCGCGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCGTGAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | 458 |
| Duo_TRA_1 | GACATTCAGATGACCCAATCCCCCTCTAGCCTTTCAGCCTCCGTAGGGGACAGAGTTACAATAACCTGTAGGGCTAGTCAGGACGTTAATACAGCAGTCGCATGGTACCAGCAGAAGCCCGGTAAGGCTCCTAAGCTTTTGATCTACAGTGCTTCTTTCCTGTATTCTGGTGTACCTAGCCGATTCTCAGGCTCTCGGAGTGGCACAGACTTCACTCTGACAATTTCAAGTCTCCAGCCAGAAGATTTTGCAACCTACTACTGCCAACAACACTACACTACTCCTCCAACCTTCGGACAGGGCACAAAAGTAGAGATTAAGTCTTCTGCTAGCACCAAAGGACCTAGTGTTTTTCCTCTTGCCCCTTCCTCAAAGTCTACCTCTGGGGGGACAGCCGCTCTGGGCTGCCTGGTCAAGGATTATTTCCCAGAGCCTGTCACTGTCAGTTGGAACTCTGGAGCCTTGACTTCTGGTGTTCATACATTTCCTGCTGTCCTTCAAAGCAGCGGCTTGTACTCATTGTCTTCTGTTGTGACAGTACCCTCAAGCAGCCTCGGCACTCAGACATACATCTGCAATGTCAACCACAA | 946 |

| | | | |
|---|---|---|---|
| | | ACCCTCAAATACAAAGGTAGATAAAAAAGTCGAACCAAAGTCTTGTGACAAAACTCACA CGTGCCCACCGTGCCCAGCACCTGAACTCCTGGGGGGACCGTCAGTCTTCCTCTTCCCC CCAAAACCCAAGGACACCCTCATGATCTCCCGGACCCCTGAGGTCACATGCGTGGTGGT GGACGTGAGCCACGAAGACCCTGAGGTCAAGTTCAACTGGTACGTGGACGGCGTGGAGG TGCATAATGCCAAGACAAAGCCGCGGGAGGAGCAGTACAACAGCACGTACCGTGTGGTC AGCGTCCTCACCGTCCTGCACCAGGACTGGCTGAATGGCAAGGAGTACAAGTGCAAGGT CTCCAACAAAGCCCTCCCAGCCCCCATCGAGAAAACCATCTCCAAAGCCAAAGGCAACA CATTTCGTCCAGAGGTCCACCTCCTGCCTCCTCCCTCAGAAGAACTTGCACTGAACGAA TTGGTCACACTGACTTGTCTGGCTCGGGGTTTCAGCCCTAAGGATGTGCTCGTTAGATG GCTTCAGGGTTCCCAGGAACTGCCCCGTGAAAAAATATCTCACATGGGCCTCACGACAGG AACCCAGCCAGGGAACCACTACATTTGCCGTCACATCCATATTGCGAGTGGCTGCCGAA GACTGGAAAAAAGGAGATACATTCTCTTGCATGGTGGGACACGAAGCCCTGCCCTTGGC CTTCACCCAGAAGACAATCGATAGATTGGCAGGGAAA | |
| | Duo_TRA_2 | GAAGTTCAACTTGTGGAGTCTGGAGGCGGTTTGGTACAACCTGGAGGTTCACTTCGTTT GTCATGTGCTGCCTCTGGGTTTAATATCAAGGATACTTATATTCATTGGGTTCGCCAGG CACCCGGAAAAGGGCTGGAATGGGTGGCCCGTATATATCCCACTAACGGTTACACTCGA TATGCTGACTCTGTAAAGGGTCGATTTACTATATCCGCTGATACTAGCAAAAACACTGC CTACCTCCAGATGAACAGTCTGCGCGCCGAGGATACAGCAGTTTATTATTGTTCCCGAT GGGGAGGTGACGGGTTCTATGCCATGGACTACTGGGGTCAAGGGACACTGGTAACCGTT TCTTCTGCTAGCGTGGCCGCTCCCTCCGTGTTCATCTTCCCACCCTCCGACGAGCAGCT GAAGTCCGGCACCGCCTCCGTCGTGTGCCTGCTGAACAACTTCTACCCCCGCGAGGCCA AGGTGCAGTGGAAGGTGGACAACGCCCTGCAGTCCGGCAACTCCCAGGAATCCGTCACC GAGCAGGACTCCAAGGACTCTACCTACTCCCTGTCCTCCACCCTGACCCTGAGCAAGGC CGACTACGAGAAGCACAAGGTGTACGCCTGCGAAGTGACCCACCAGGGCCTGTCCTCCC CAGTGACCAAGAGCTTCAACCGGGGCGAGTGTGACAAAACTCACACGTGCCCACCGTGC CCAGCACCTGAACTCCTGGGGGGACCGTCAGTCTTCCTCTTCCCCCCAAAACCCAAGGA CACCCTCATGATCTCCCGGACCCCTGAGGTCACATGCGTGGTGGTGGACGTGAGCCACG AAGACCCTGAGGTCAAGTTCAACTGGTACGTGGACGGCGTGGAGGTGCATAATGCCAAG ACAAAGCCGCGGGAGGAGCAGTACAACAGCACGTACCGTGTGGTCAGCGTCCTCACCGT | 947 |

| | | |
|---|---|---|
| | CCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGCCAGCCACGGGAGCCCCAGGTGTACACCCTGCCACCCTCCAGAGAAGAGATGACCAAGAACCAGGTGTCCCTGACCTGTCTGGTGAAGGGCTTCTACCCCAGCGACATCGCCGTGGAGTGGGAGAGCAACGGCCAGCCCGAGAACAACTACAAGACCACCCCCCCCAGTGCTGGACAGCGACGGCAGCTTCTTCCTGTACAGCAAGCTGACCGTGGACAAGAGCAGGTGGCAGCAGGGCAACGTGTTCAGTTGCAGCGTGATGCACGAAGCTCTCCACAACCACTACACCCAGAAGTCCCTGTCCCTGAGCCCCGGCAAG | |
| Tandem_Fc_TRA_1 | GATATTCAGATGACTCAGAGTCCTAGTTCCCTCAGCGCCTCCGTAGGCGACAGAGTTACAATAACTTGCCGAGCAAGCCAAGACGTAAACACTGCAGTCGCCTGGTACCAACAGAAACCAGGCAAAGCTCCAAAACTCTTGATTTACAGTGCTTCCTTCCTTTATAGTGGCGTTCCAAGCCGCTTCAGCGGCAGCCGCTCTGGCACCGACTTCACTCTCACTATTTCTTCCTTGCAACCTGAAGACTTCGCCACTTATTATTGCCAGCAACACTACACAACACCCCCAACATTCGGACAGGGCACAAAGGTAGAAATAAAACGTACGGTGGCAGCTCCCAGCGTTTTTATCTTTCCCCCATCCGACGAGCAGCTCAAGAGTGGCACTGCCTCTGTAGTTTGTTTGCTGAATAACTTCTATCCACGTGAAGCAAAAGTACAGTGGAAGGTCGATAATGCCCTTCAGAGCGGTAACAGCCAAGAAAGTGTTACCGAGCAAGATTCCAAAGATTCCACTTACAGTCTGTCCAGCACATTGACACTGAGTAAGGCTGATTACGAAAAACACAAGGTGTACGCATGCGAGGTGACACACCAAGGTCTTTCATCTCCTGTAACTAAGAGCTTTAACCGGGGAGAATGT | 453 |
| Tandem_Fc_TRA_2 | GAAGTTCAACTTGTGGAGTCTGGAGGCGGTTTGGTACAACCTGGAGGTTCACTTCGTTTGTCATGTGCTGCCTCTGGGTTTAATATCAAGGATACTTATATTCATTGGGTTCGCCAGGCACCCGGAAAAGGGCTGGAATGGGTGGCCCGTATATATCCCACTAACGGTTACACTCGATATGCTGACTCTGTAAAGGGTCGATTTACTATATCCGCTGATACTAGCAAAAACACTGCCTACCTCCAGATGAACAGTCTGCGCGCCGAGGATACAGCAGTTTATTATTGTTCCCGATGGGGAGGTGACGGGTTCTATGCCATGGACTACTGGGGTCAAGGGACACTGGTAACCGTTTCTTCTGCTAGCACCAAAGGACCTAGTGTTTTTCCTCTTGCCCCTTCCTCAAAGTCTACCTCTGGGGGGACAGCCGCTCTGGGCTGCCTGGTCAAGGATTATTTCCCAGAGCCTGTCACTGTCAGTTGGAACTCTGGAGCCTTGACTTCTGGTGTTCATACATTTCCTGCTGTCCTTCAAAGCAGCGGCTTGTACTCATTGTCTTCTGTTGTGACAGTACCCTCAAGCAGCCTCGG | 948 |

| | CACTCAGACATACATCTGCAATGTCAACCACAAACCCTCAAATACAAAGGTAGATAAAAGAGTCGAACCAAAGTCTTGTGACAAAACTCACACGTGCCCACCGTGCCCAGCACCTGAACTCCTGGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATGCCGGGATAAGTTGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAAGGTGGTGGAGGCGGGGGCGGAGGAGGTGGCGGGGGAGGCGGTGGCGGAGGTGGCGGAGGTGACAAAACTCACACGTGCCCACCGTGCCCAGCACCTGAACTCCTGGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATGCCGGGATAAGTTGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGT | |
| Tandem_Fc_TRA_3 | GACAAAACTCACACATGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTG | 458 |

| | GACGGCGTGGAGGTGCATAATGCCAAGACAAAGCCGCGGGAGGAGCAGTACAACAGCAC GTACCGTGTGGTCAGCGTCCTCACCGTCCTGCACCAGGACTGGCTGAATGGCAAGGAGT ACAAGTGCAAGGTCTCCAACAAAGCCCTCCCAGCCCCCATCGAGAAAACCATCTCCAAA GCCAAAGGGCAGCCCCGAGAACCACAGGTGTGCACCCTGCCCCCCATCCCGGGAGGAGAT GACCAAGAACCAGGTCAGCCTGAGCTGCGCGGTCAAAGGCTTCTATCCCAGCGACATCG CCGTGGAGTGGGAGAGCAATGGGCAGCCGGAGAACAACTACAAGACCACGCCTCCCGTG CTGGACTCCGACGGCTCCTTCTTCCTCGTGAGCAAGCTCACCGTGGACAAGAGCAGGTG GCAGCAGGGGAACGTCTTCTCATGCTCCGTGATGCATGAGGCTCTGCACAACCACTACA CGCAGAAGAGCCTCTCCCTGTCTCCGGGTAAA | |

### Example 14. Analysis of antibody-dependent cytotoxicity

The antibody-dependent cell-mediated cytotoxicity of H01 was analyzed in NCI-N87 (HER2 3+; High) cells, MDA-MB-453 (HER2 2+; Mid) cells, SNU-601 (HER2 1+; Low) cells, or SNU-5 (HER2 1+; Low) cells.

Specifically, each of the above cells was inoculated into a 96-well plate at a concentration of $1.0 \times 10^4$ cells/well and then treated with H01 of the present invention. Thereafter, peripheral blood mononuclear cells (PBMC) isolated on the same day were used as effector cells, and treated at $1.5 \times 10^5$ cells/well to make the number of PBMC 15 times more than the number of the target cells (the above cancer cell line) (E:T ratio = 15:1). The cells were incubated for 18 hours in a humid incubator under 37 °C and 5%(v/v) $CO_2$ conditions. After 18 hours, the cytotoxicity was measured using a cytotoxicity detection kit (LDH) (Roche, 11644793001), and the cytotoxicity was calculated using Equation 2 below.

**Cytotoxicity (%) = [(Test release - spontaneous release)/(Maximum release - spontaneous release)] $\times$ 100**      \<Equation 2\>

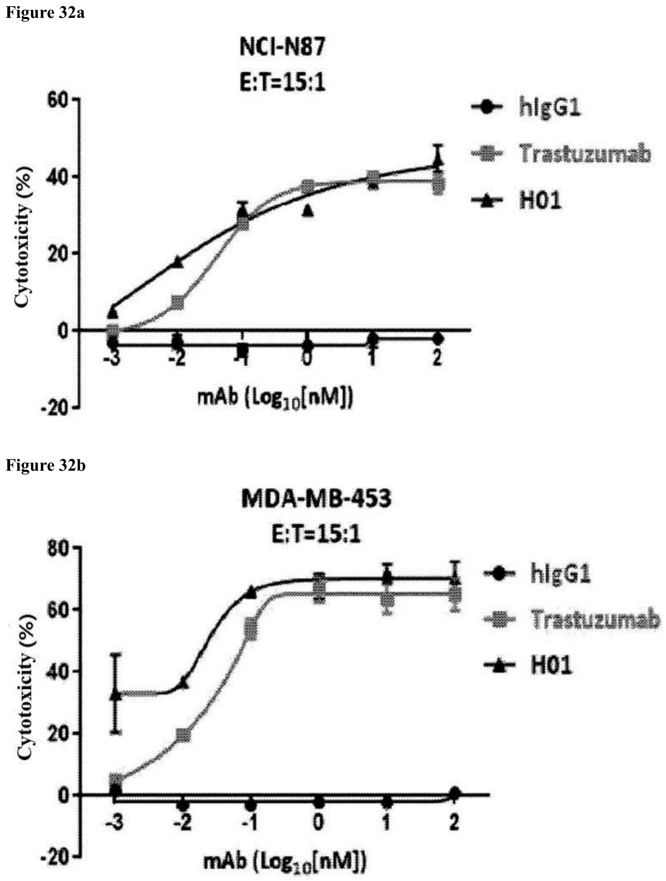

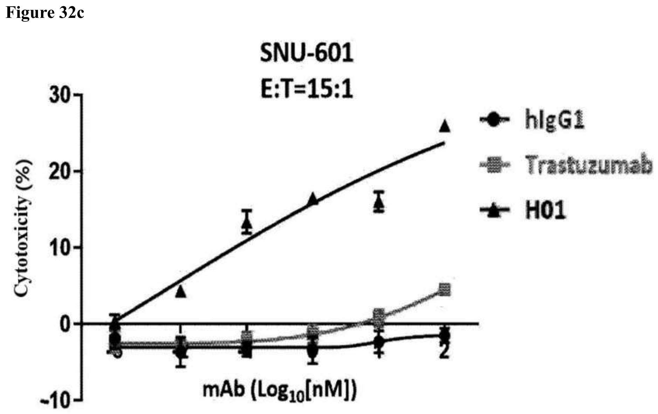

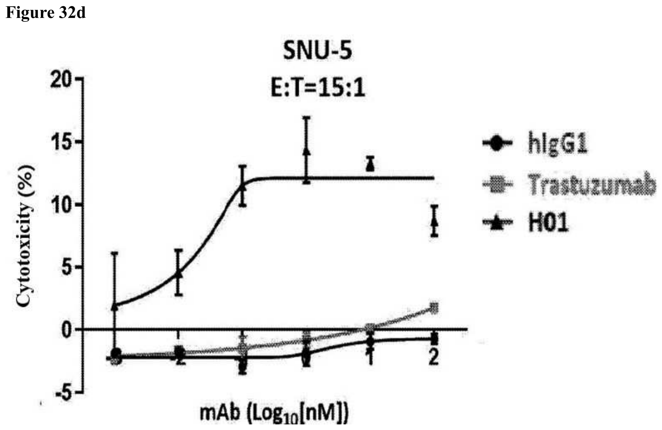

In NCI-N87 (HER2 3+; High) cells and MDA-MB-453 (HER2 2+; Mid) cells, H01 showed excellent cytotoxicity at low concentrations compared to trastuzumab (Figures 32a and 32b). In addition, the cytotoxicity analysis in SNU-601 (HER2 1+; Low) cell line and SNU-5 (HER2 1+; Low) cell line showed H01 has excellent cytotoxicity compared to that of trastuzumab (Figures 32c and 32d).

### Example 15. Evaluation of efficacy in xenograft mouse models

The antitumor activity of the antibody of according to the present invention was confirmed in the SNU-5 (HER2 1+; Low) gastric cancer cell line-derived xenograft model.

First, efficacy was evaluated using 6 week old female SCID mice (C.B-17/NcrKoat-Prkdc[scid], Koatech). The SNU-5 cells was suspended in PBS to a concentration of $1 \times 10^7$ cells/100 μL and then mixed with MATRIGEL® Growth Factor Reduced (GFR) Basement Membrane Matrix (Corning, 354230) at a ratio of 1:1. The mixture (100 μL) was transplanted subcutaneously into the right flank of the mouse, and then tumor growth was monitored. The mice were grouped so that the average tumor volume was approximately 107 mm$^3$, and then administered intravenously once a week for a total of 6 weeks with PBS (vehicle), 5 mg/kg H01, 5 mg/kg H01 + 5 mg/kg P01, 5 mg/kg HP507, 5 mg/kg trastuzumab, or 5 mg/kg trastuzumab + 5 mg/kg pertuzumab.

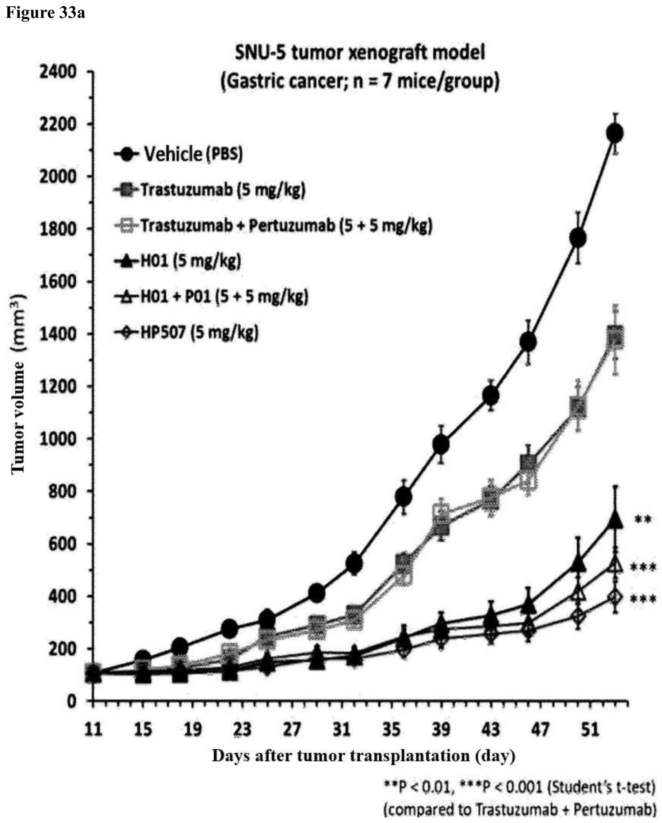

As shown in Figure 33a, H01 alone showed superior antitumor activity compared to trastuzumab and trastuzumab + pertuzumab. It was shown that H01 + P01 induces improved antitumor activity compared to H01 alone, and HP507 induces the strongest antitumor activity.

In addition, the antitumor activity was confirmed in the BALB/c-nu mice (Orientbio) xenografted with SNU-5 gastric cancer cell line. At this time, the SNU-5 cells were suspended in PBS to a concentration of $1 \times 10^7$ cells/100 μL and then transplanted into the mice in the same manner as above. The mice were grouped so that the average tumor volume was approximately 122 mm$^3$, and then administered intraperitoneally (I.P.) twice a week for a total of 6 weeks with PBS (vehicle), 1 mg/kg trastuzumab, 1 mg/kg pertuzumab, 0.5 mg/kg trastuzumab + 0.5 mg/kg pertuzumab, 1 mg/kg H01, 1 mg/kg P01, or 0.5 mg/kg H01 + 0.5 mg/kg P01. In addition, since there are no antibodies present in the blood of the SCID mice, 50 mg/kg intravenous immunoglobulin (IVIG; LIV-r, SK Plasma) was administered to all mice twice a week for 6 weeks to simulate the actual human blood environment.

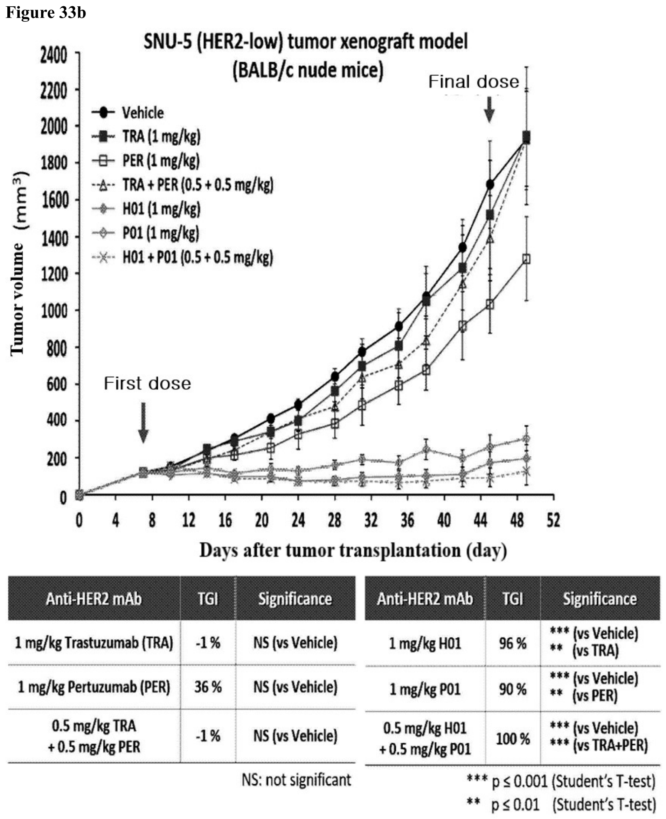

As shown in Figure 33b, H01, P01, or H01 + P01 in combination induced more superior antitumor activity compared to trastuzumab, pertuzumab, or trastuzumab + pertuzumab in combination.

In addition, the antitumor activity was confirmed in the SNU-601 (HER2 1+; Low) gastric cancer cell line-derived xenograft model. 6 week old female SCID mice (C.B-17/NcrKoat-Prkdc$^{scid}$, Koatech) were used. The SNU-601 cell line was suspended in PBS to a concentration of $1 \times 10^7$ cells/100 $\mu$L and then transplanted into the mice in the same manner as above. The mice were grouped so that the average tumor volume was approximately 142 mm$^3$, and administered intraperitoneally twice a week for a total of 6 weeks with PBS (vehicle), 5 mg/kg H01, 5 mg/kg trastuzumab, 5 mg/kg trastuzumab + 5 mg/kg pertuzumab. In addition, 50 mg/kg intravenous immunoglobulin (IVIG; LIV-r, SK Plasma) was administered to all mice twice a week for 6 weeks.

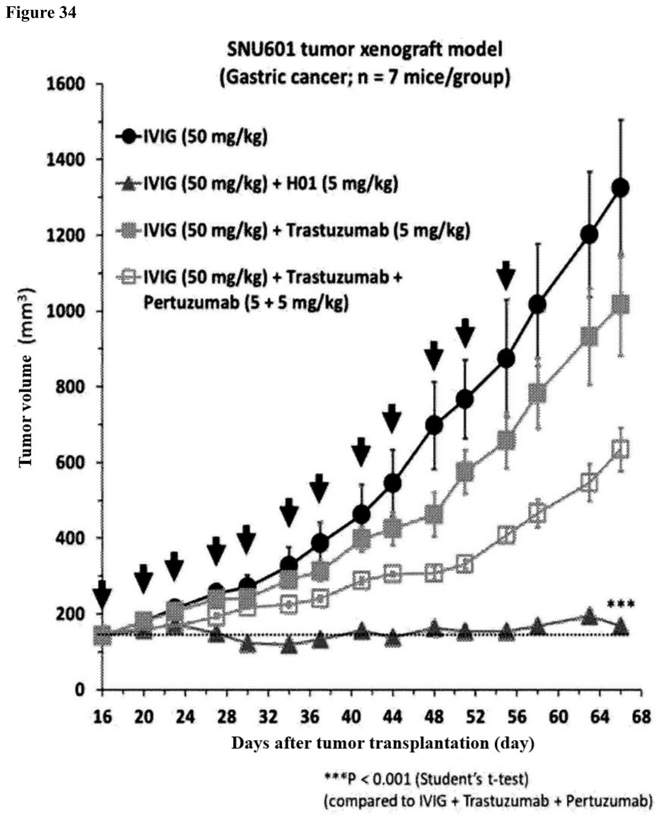

As shown in Figure 34, H01 alone induced most superior antitumor activity in the SNU-601 gastric cancer xenograft model.

Efficacy in the NCI-N87 (HER2 3+; High) gastric cancer cell line-derived xenograft model was evaluated using 6 week old female SCID mice (C.B-17/NcrKoat-Prkdc$^{scid}$, Koatech). The NCI-N87 cells were suspended in PBS to a concentration of $5 \times 10^6$ cells/100 $\mu$L and then transplanted into the mice in the same manner as above. The mice were grouped so that the average tumor volume was approximately 146 mm$^3$, and then administered intraperitoneally twice a week for a total of 6 weeks with PBS (vehicle), 0.2 mg/kg H01, 5 mg/kg H01, 0.2 mg/kg trastuzumab, or 5 mg/kg trastuzumab. In addition, 50 mg/kg intravenous immunoglobulin (IVIG; LIV-r, SK Plasma) was administered to all mice twice a week for 6 weeks.

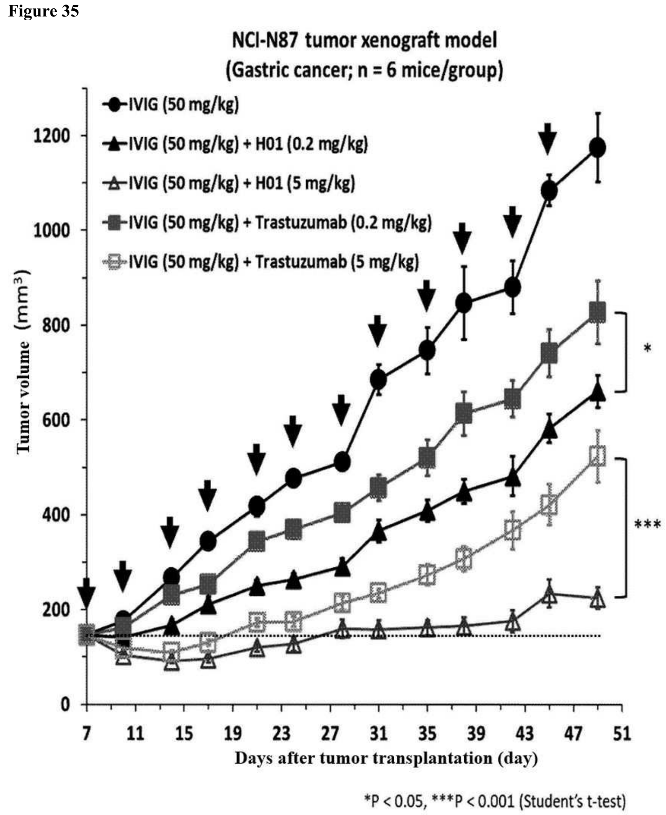

As shown in Figure 35, H01 induced superior antitumor activity compared to trastuzumab at 5 mg/kg and at 0.2 mg/kg.

**Example 16. Evaluation of antitumor activity in CT26-HER2 syngeneic mouse model**

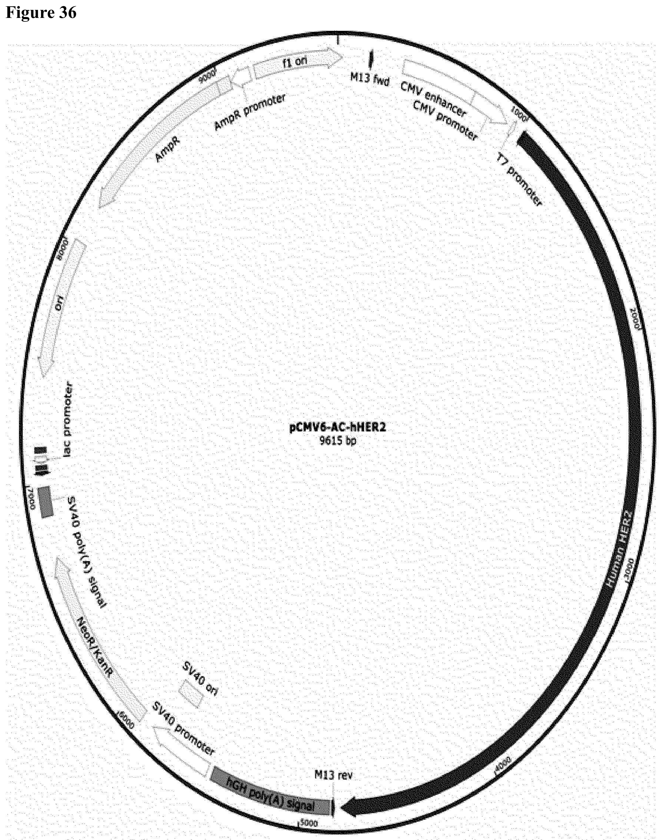

The polynucleotide (SEQ ID NO: 566, Table 42) encoding the human HER2 protein (SEQ ID NO: 567, Table 42) was cloned into a protein expression vector (ORIGENE, PS100020) containing a neomycin resistance gene to construct the human HER2 expression vector pCMV6-AC-hHER2 (Figure 36 and Table 42).

[Table 42]

| Name | Sequence | SEQ ID NO |
|---|---|---|
| Human nucleotide sequence | HER2 ATGGAGCTGGCGGCCTTGTGCCGCTGGGGGCTCCTCCTCGCCCTCTTGGCCCCCCGGAGCCGGC GAGCACCCAAGTGTGCACCGGCACAGACATGAAGCTGCGGCTCCCTGCCAGTCCCGAGACCC ACCTGGACATGCTCCGCCACCTCTACCAGGGCTGCCAGGTGGTGCAGGGAAACCTGGAACTC ACCTACCTGCCCACCAATGCCAGCCTGTCCTTCCTGCAGGATATCCAGGAGGTGCAGGGCTA CGTGCTCATCGCTCACAACCAAGTGAGGCAGGTCCCACTGCAGAGGCTGCGGATTGTGCGAG GCACCCAGCTCTTTGAGGACAACTATGCCCTGGCCGTGCTAGACAATGGAGACCCGCTGAAC AATACCACCCCTGTCACAGGGGCCTCCCCAGGAGGCCTGCGGGAGCTGCAGCTTCGAAGCCT CACAGAGATCTTGAAAGGAGGGGTCTTGATCCAGCGGAACCCCCAGCTCTGCTACCAGGACA CGATTTTGTGGAAGGACATCTTCCACAAGAACAACCAGCTGGCTCTCACACTGATAGACACC AACCGCTCTCGGGCCTGCCACCCCTGTTCTCCGATGTGTAAGGGCTCCCGCTGCTGGGGAGA GAGTTCTGAGGATTGTCAGAGCCTGACGCGCACTGTCTGTGCCGGTGGCTGTGCCCGCTGCA AGGGGCCACTGCCCACTGACTGCTGCCATGAGCAGTGTGCTGCCGGCTGCACGGGCCCCAAG CACTCTGACTGCCTGGCCTGCCTCCACTTCAACCACAGTGGCATCTGTGAGCTGCACTGCCC AGCCCTGGTCACCTACAACACAGACACCGTTTGAGTCCATGCCCAATCCGGAGGGCCGGTATA CATTCGGCGCCAGCTGTGTGACTGCCTGTCCCTACAACTACCTTTCTACGGACGTGGGATCC TGCACCCTCGTCTGCCCCCTGCACAACCAAGAGGTGACAGCAGAGGATGGAACACAGCGGTG TGAGAAGTGCAGCAAGCCCTGTGCCCGAGTGTGCTATGGTCTGGGCATGGAGCACTTGCGAG AGGTGAGGGCAGTTACCAGTGCCAATATCCAGGAGTTTGCTGGCTGCAAGAAGATCTTTGGG AGCCTGGCATTTCTGCCGGAGAGCTTTGATGGGGACCCAGCCTCCAACACTGCCCCGCTCCA GCCAGAGCAGCTCCAAGTGTTTGAGACTCTGGAAGAGATCACAGGTTACCTATACATCTCAG CATGGCCGGACAGCCTGCCTGACCTCAGCGTCTTCCAGAACCTGCAAGTAATCCGGGGACGA ATTCTGCACAATGGCGCCTACTCGCTGACCCTGCAAGGGCTGGGCATCAGCTGGCTGGGGCT GCGCTCACTGAGGGAACTGGGCAGTGGACTGGCCCTCATCCACCATAACACCCACCTCTGCT TCGTGCACACGGTGCCCTGGGACCAGCTCTTTCGGAACCCGCACCAAGCTCTGCTCCACACT GCCAACCGGCCAGAGGACGAGTGTGTGGGCGAGGGCCTGGCCTGCCACCAGCTGTGCGCCCG AGGGCACTGCTGGGGTCCAGGGCCCACCCAGTGTGTGAACTGCAGCCAGTTCCTTCGGGGCC AGGAGTGCGTGGAGGAATGCCGAGTACTGCAGGGGCTCCCCAGGGAGTATGTGAATGCCAGG CACTGTTTGCCGTGCCACCCTGAGTGTCAGCCCCAGAATGGCTCAGTGACCTGTTTTGGACC GGAGGCTGACCAGTGTGTGGCCTGTGCCCACTATAAGGACCCTCCCTTCTGCGTGGCCCGCT GCCCCAGCGGTGTGAAACCTGACCTCTCCTACATGCCCATCTGGAAGTTTCCAGATGAGGAG GGCGCATGCCAGCCTTGCCCCATCAACTGCACCCACTCCTGTGTGGACCTGGATGACAAGGG CTGCCCCGCCGAGCAGAGAGCCAGCCCTCTGACGTCCATCATCTCTGCGGTGGTTGGCATTC TGCTGGTCGTGGTCTTGGGGGTGGTCTTTGGGATCCTCATCAAGCGACGGCAGCAGAAGATC CGGAAGTACACGATGCGGAGACTGCTGCAGGAAACGGAGCTGGTGGAGCCGCTGACACCTAG CGGAGCGATGCCCAACCAGGCGCAGATGCGGATCCTGAAAGAGACGGAGCTGAGGAAGGTGA AGGTGCTTGGATCTGGCGCTTTTGGCACAGTCTACAAGGGCATCTGGATCCCTGATGGGGAG AATGTGAAAATTCCAGTGGCCATCAAAGTGTTGAGGGAAAACACATCCCCCAAAGCCAACAA AGAAATCTTAGACGAAGCATACGTGATGGCTGGTGTGGGCTCCCCATATGTCTCCCGCCTTC TGGGCATCTGCCTGACATCCACGGTGCAGCTGGTGACACAGCTTATGCCCTATGGCTGCCTC TTAGACCATGTCCGGGAAAACCGCGGACGCCTGGGCTCCCAGGACCTGCTGAACTGGTGTAT GCAGATTGCCAAGGGGATGAGCTACCTGGAGGATGTGCGGCTCGTACACAGGGACTTGGCCG CTCGGAACGTGCTGGTCAAGAGTCCCAACCATGTCAAAATTACAGACTTCGGGCTGGCTCGG CTGCTGGACATTGACGAGACAGAGTACCATGCAGATGGGGGCAAGGTGCCCATCAAGTGGAT GGGCGCTGGAGTCCATTCTCCGCCGGCGGTTCACCCACCAGAGTGATGTGTGGAGTTATGGTG TGACTGTGTGGGAGCTGATGACTTTTGGGGCCAAACCTTACGATGGGATCCCAGCCCGGGAG ATCCCTGACCTGCTGGAAAAGGGGGAGCGGCTGCCCCAGCCCCCCATCTGCACCATTGATGT CTACATGATCATGGTCAAATGTTGGATGATTGACTCTGAATGTCGGCCAAGATTCCGGGAGT | 566 |

| | | |
|---|---|---|
| | TGGTGTCTGAATTCTCCCGCATGGCCAGGGACCCCCAGCGCTTTGTGGTCATCCAGAATGAG GACTTGGGCCCAGCCAGTCCCTTGGACAGCACCTTCTACCGCTCACTGCTGGAGGACGATGA CATGGGGGACCTGGTGGATGCTGAGGAGTATCTGGTACCCCAGCAGGGCTTCTTCTGTCCAG ACCCTGCCCCGGGCGCTGGGGGCATGGTCCACCACAGGCACCGCAGCTCATCTACCAGGAGT GGCGGTGGGGACCTGACACTAGGGCTGGAGCCCTCTGAAGAGGAGGCCCCCAGGTCTCCACT GGCACCCTCCGAAGGGGCTGGCTCCGATGTATTTGATGGTGACCTGGGAATGGGGGCAGCCA AGGGGCTGCAAAGCCTCCCCACACATGACCCCAGCCCTCTACAGCGGTACAGTGAGGACCCC ACAGTACCCCTGCCCTCTGAGACTGATGGCTACGTTGCCCCCCTGACCTGCAGCCCCCAGCC TGAATATGTGAACCAGCCAGATGTTCGGCCCCAGCCCCCTTCGCCCCGAGAGGGCCCTCTGC CTGCTGCCCGACCTGCTGGTGCCACTCTGGAAAGGCCCAAGACTCTCTCCCCAGGGAAGAAT GGGGTCGTCAAAGACGTTTTTGCCTTTGGGGGTGCCGTGGAGAACCCCGAGTACTTGACACC CCAGGGAGGAGCTGCCCCTCAGCCCCACCCTCCTCCTGCCTTCAGCCCAGCCTTCGACAACC TCTATTACTGGGACCAGGACCCACCAGAGCGGGGGGCTCCACCCAGCACCTTCAAAGGGACA CCTACGGCAGAGAACCCAGAGTACCTGGGTCTGGACGTGCCAGTG | |
| Human HER2 protein sequence | MELAALCRWGLLLALLPPGAASTQVCTGTDMKLRLPASPETHLDMLRHLYQGCQVVQGNLEL TYLPTNASLSFLQDIQEVQGYVLIAHNQVRQVPLQRLRIVRGTQLFEDNYALAVLDNGDPLN NTTPVTGASPGGLRELQLRSLTEILKGGVLIQRNPQLCYQDTILWKDIFHKNNQLALTLIDT NRSRACHPCSPMCKGSRCWGESSEDCQSLTRTVCAGGCARCKGPLPTDCCHEQCAAGCTGPK HSDCLACLHFNHSGICELHCPALVTYNTDTFESMPNPEGRYTFGASCVTACPYNYLSTDVGS CTLVCPLHNQEVTAEDGTQRCEKCSKPCARVCYGLGMEHLREVRAVTSANIQEFAGCKKIFG SLAFLPESFDGDPASNTAPLQPEQLQVFETLEEITGYLYISAWPDSLPDLSVFQNLQVIRGR ILHNGAYSLTLQGLGISWLGLRSLRELGSGLALIHHNTHLCFVHTVPWDQLFRNPHQALLHT ANRPEDECVGEGLACHQLCARGHCWGPGPTQCVNCSQFLRGQECVEECRVLQGLPREYVNAR HCLPCHPECQPQNGSVTCFGPEADQCVACAHYKDPPFCVARCPSGVKPDLSYMPIWKFPDEE GACQPCPINCTHSCVDLDDKGCPAEQRASPLTSIISAVVGILLVVVLGVVFGILIKRRQQKI RKYTMRRLLQETELVEPLTPSGAMPNQAQMRILKETELRKVKVLGSGAFGTVYKGIWIPDGE NVKIPVAIKVLRENTSPKANKEILDEAYVMAGVGSPYVSRLLGICLTSTVQLVTQLMPYGCL LDHVRENRGRLGSQDLLNWCMQIAKGMSYLEDVRLVHRDLAARNVLVKSPNHVKITDFGLAR LLDIDETEYHADGGKVPIKWMALESILRRRFTHQSDVWSYGVTVWELMTFGAKPYDGIPARE IPDLLEKGERLPQPPICTIDVYMIMVKCWMIDSECRPRFRELVSEFSRMARDPQRFVVIQNE DLGPASPLDSTFYRSLLEDDDMGDLVDAEEYLVPQQGFFCPDPAPGAGGMVHHRHRSSSTRS GGGDLTLGLEPSEEEAPRSPLAPSEGAGSDVFDGDLGMGAAKGLQSLPTHDPSPLQRYSEDP TVPLPSETDGYVAPLTCSPQPEYVNQPDVRPQPPSPREGPLPAARPAGATLERPKTLSPGKN GVVKDVFAFGGAVENPEYLTPQGGAAPQPHPPPAFSPAFDNLYYWDQDPPERGAPPSTFKGT PTAENPEYLGLDVPV | 567 |

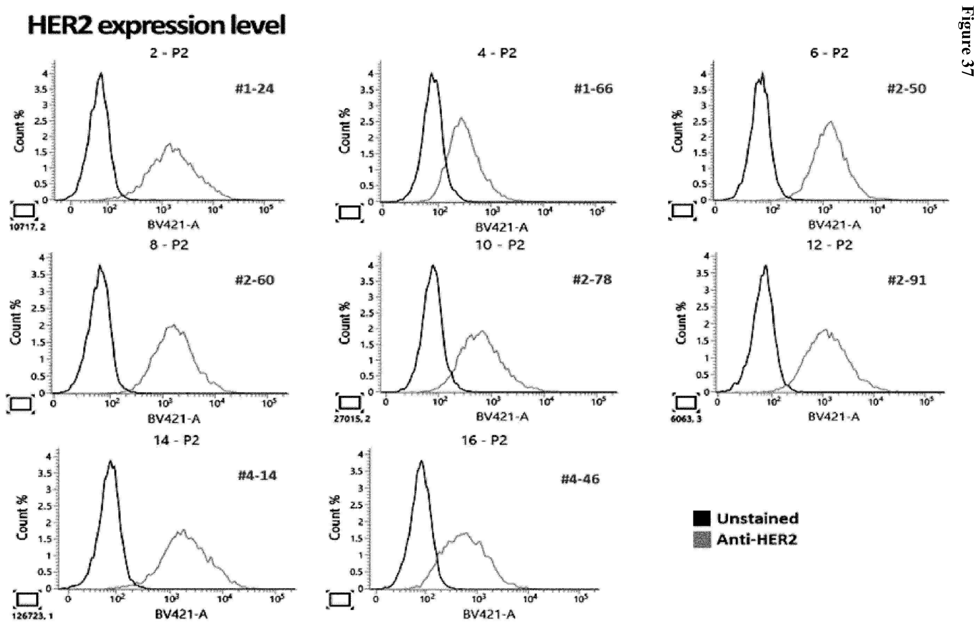

The mouse large intestine-derived CT26 cancer cells were transfected with the pCMV6-AC-hHER2 human HER2 expression vector using the lipofectamine 2000 transfection reagent (Invitrogen, 11668-019). The transformed cells were incubated for 14 days in culture medium containing 1 mg/mL G418 (Invivogen, ant-gn-5), and only cells transfected with the pCMV6-AC-hHER2 human HER2 expression vector were selected. The top 3% clones in terms of HER2 expression was sorted into a 96-well plate (ThermoFisher, 167008) with 1 cell per well using SH800S Cell Sorter (SONY). Cell selection was performed by incubation in G418-containing medium for 21 days, a total of eight CT26 mouse large intestine cancer cell line clones expressing human HER2 were obtained. The expression of human HER2 in the cell clones was monitored using a flow cytometer after staining with the anti-human HER2-BV421 (BD, 744811) (Figure 37 and Table 43).

[Table 43]

| ΔGMFI (of unstained control) | |
|---|---|
| #1-24 | 27.3 |
| #1-66 | 3.7 |
| #2-50 | 19.1 |
| #2-60 | 25.9 |
| #2-78 | 7.5 |
| #2-91 | 17.5 |
| #4-14 | 26.8 |
| #4-46 | 6.5 |

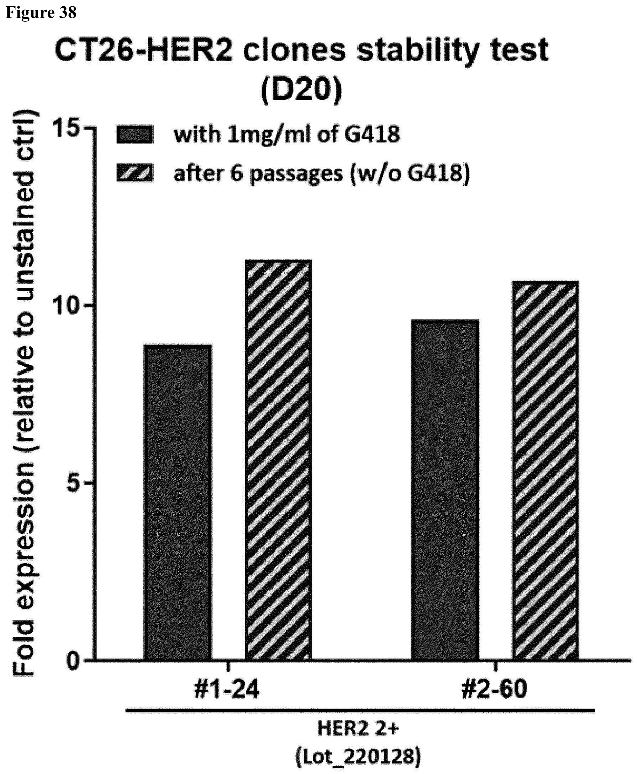

The cell clones were subcultured six times for 20 days in a G418-free environment and then stained with the anti-human HER2-BV421 (BD, 744811), and the level of human HER2 expression in the clones was measured. It was shown that the level of human HER2 expression was not reduced in cell clones grown without G418 compared to cells grown with G418 (Figure 38 and Table 44). Hereinafter, the CT-26 cell clone expressing the human HER2 protein was described as the CT26-HER2 cell line.

[Table 44]

| ΔGMFI (of unstained control) | | |
|---|---|---|
| Name | #1-24 | #2-60 |
| With 1 mg/mL of G418 | 8.9 | 9.6 |
| 6 times passage (w/o G418)_D20 | 11.3 | 10.7 |

The cell surface Fc loads of H01 and trastuzumab were compared among parental CT26, CT26-HER2 cell line (Clone #2-60), or human cancer cell line (SNU5, SNU601, NCI-N87). Specifically, the above cells and 100 nM of human IgG1, trastuzumab (TRA), or H01 were mixed in a 96-well v-bottom plate (Corning, 3363) and allowed to bind at 4 °C for 30 minutes.

Thereafter, the cells were treated with the Alexa 488 fluorescence-conjugated anti-human IgG Fcγ Fab antibody (Jackson ImmunoResearch, 109-547-008), and the Fc loads of the antibody on the cells were quantified using a flow cytometer.

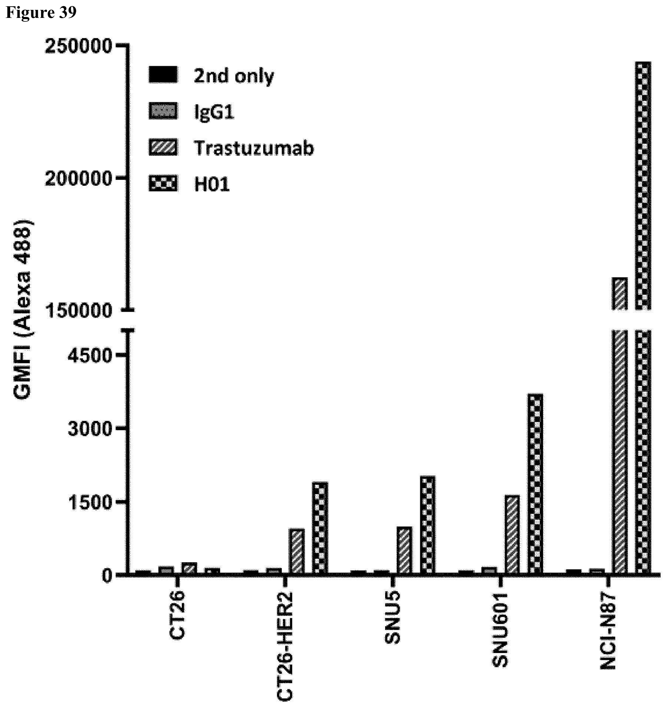

As shown in Figure 39 and Table 45, CT26-HER2 cells (Clone #2-60) was shown to express human HER2 at a similar level to the SNU5 cells. In addition, it was shown that in the CT26-HER2 cells, treatment of H01 results in increased Fc loads on the cell surface compared to that of trastuzumab (TRA).

[Table 45]

| GMFI | CT26 | CT26-HER2 (#2-60) | SNU5 | SNU601 | NCI-N87 |
|---|---|---|---|---|---|
| 2nd only | 99 | 102 | 94 | 97 | 116 |
| 100 nM IgG1 isotype | 176 | 150 | 101 | 173 | 132 |
| 100 nM Trastuzumab | 252 | 959 | 991 | 1642 | 162433 |
| 100 nM H01 | 147 | 1907 | 2028 | 3703 | 243897 |

Next, the antitumor activity of H01 was confirmed in the CT26-HER2 cell line (Clone #2-60) syngeneic mouse model (Syngeneic mouse model). Efficacy was evaluated using 6 week old female Balb/c mice (Orientbio). PBS (vehicle), 5 mg/kg trastuzumab, and 5 mg/kg H01 were administered intraperitoneally twice a week for a total of 2 weeks.

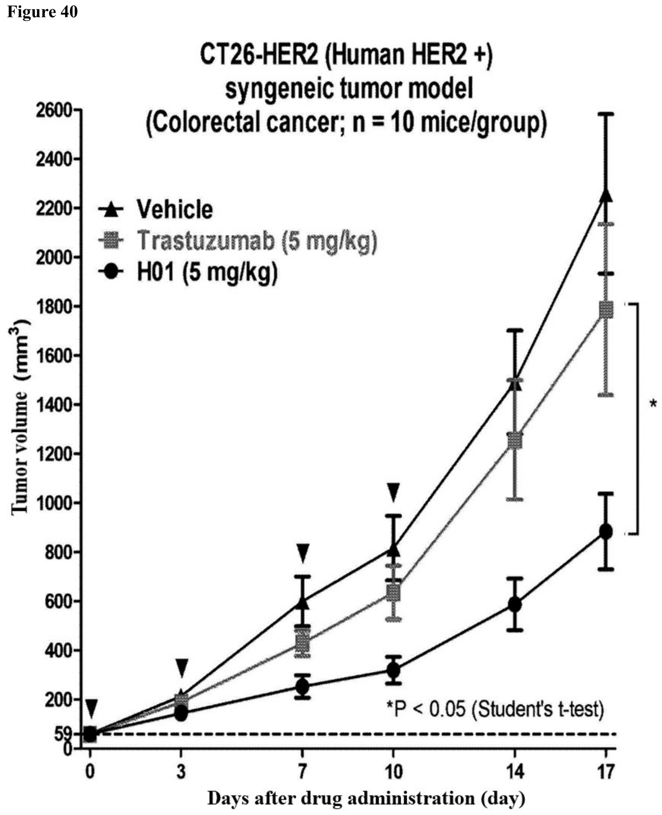

As shown in Figure 40, the analysis showed that H01 induces superior antitumor activity compared to trastuzumab (Figure 40).

**Example 17. Design, preparation and analysis of a novel antibody structure targeting glypican-3**

The polypeptide sequences of the variant light chain and heavy chain of an antibody that specifically recognizes the glypican-3 (GPC-3) protein are shown in Table 46.

GPM01 consists of the polypeptides of Fc-Hole (SEQ ID NO: 7), GPM01 HC (SEQ ID NO: 67), and GPM01 LC (SEQ ID NO: 68), and was prepared by co-transfection of expression vectors loaded with polynucleotides encoding the polypeptides into the EXPICHO-S™ cell line. The protein prepared as above was purified and analyzed in the same manner as in Example 1 above. In addition, GPM02, GPM04, GPB01, GPB03, GPB04, and GPB06 were each prepared, purified, and analyzed in the same manner as above.

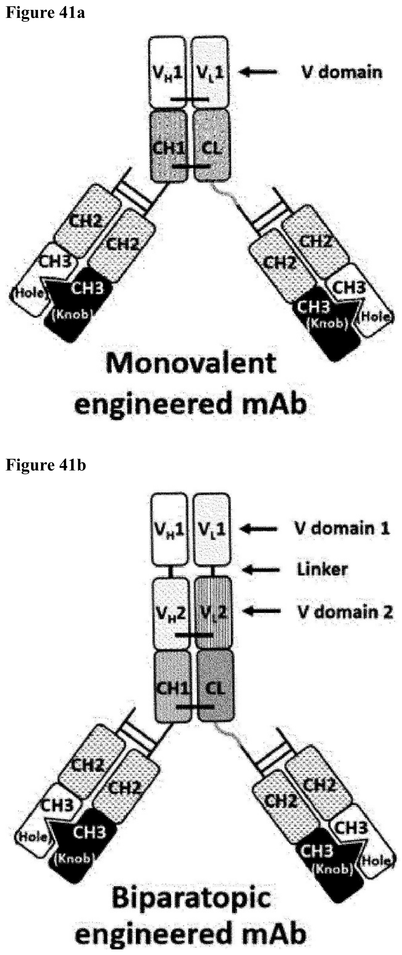

At this time, GPM01, GPM02, and GPM04 bind to different epitopes of the antigen in a monovalent manner and have structures consisting of two Fc domains (Figure 41a). GPB01, GPB03, GPB04, and GPB06 have structures in which the variable regions of GPM01, GPM02, or GPM04 are linked with a polypeptide linker (SEQ ID NO: 48, SEQ ID NO: 50), and bind to GPC-3 in a biparatopic manner, and have two Fc domains (Figure 41b).

[Table 46]

| Name | Sequence | SEQ ID NO |
|---|---|---|
| GPM01 HC | EVQLVESGGGLVKPGGSLKLSCAASGFTFSRYAMSWVRQIPEKCLEWVAAIDSSGGDTY YLDTVKDRFTISRDNANNTLHLQMRSLRSEDTALYYCVRQGGAYWGQGTLVTVSSASTK GPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPELLGGPS VFLFPPKPKDTLMISRTPEVTCVVVDVSHEDPEVKFNWYVTGVEVHNAKTKPREEQYNS TYRVVSVLTVLHQDWLNGKEYKCKVSNKALPAPIEKTISKAKGQPREPQVYTLPPCREE MTKNQVSLWCLVKGFYPSDIAVEWESNGQPENNYKTTPPVLDSDGSFFLYSKLTVDKSR WQQGNVFSCSVMHEALHNHYTQKSLSLSPGK | 67 |
| GPM01 LC | DVVMTQTPLTLSVTIGQPASISCKSSQSLLDSDGKTYLNWLLQRPGQSPKRLIYLVSKL DSGAPDRFTGSGSGTDFTLKISRVEAEDLGIYYCWQGTHFPLTFGCGTKLEIKRTVAAP SVFIFPPSDEQLKSGTASVVCLLNNFYPREAKVQWKVDNALQSGNSQESVTEQDSKDST YSLSSTLTLSKADYEKHKVYACEVTHQGLSSPVTKSFNRGECGGGGSGGGGSGGGGSEP KSSDKTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKT ISKAKGQPREPQVYTLPPCREEMTKNQVSLWCLVKGFYPSDIAVEWESNGQPENNYKTT PPVLDSDGSFFLYSKLTVDKSRWQQGNVFSCSVMHEALHNHYTQKSLSLSPGK | 68 |
| GPM02 HC | QVQLVQSGAEVKKPGASVKVSCKASGYTFTDYEMHWVRQAPGQCLEWMGALDPKTGDTA YSQKFKGRVTLTADKSTSTAYMELSSLTSEDTAVYYCTRFYSYTYWGQGTLVTVSSAST KGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTCPPCPAPELLGGP SVFLFPPKPKDTLMISRTPEVTCVVVDVSHEDPEVKFNWYVDGVEVHNAKTKPREEQYN STYRVVSVLTVLHQDWLNGKEYKCKVSNKALPAPIEKTISKAKGQPREPQVYTLPPCRE EMTKNQVSLWCLVKGFYPSDIAVEWESNGQPENNYKTTPPVLDSDGSFFLYSKLTVDKS RWQQGNVFSCSVMHEALHNHYTQKSLSLSPGK | 69 |
| GPM02 LC | DVVMTQSPLSLPVTPGEPASISCRSSQSLVHSNRNTYLHWYLQKPGQSPQLLIYKVSNR FSGVPDRFSGSGSGTDFTLKISRVEAEDVGVYYCSQNTHVPPTFGCGTKLEIKRTVAAP SVFIFPPSDEQLKSGTASVVCLLNNFYPREAKVQWKVDNALQSGNSQESVTEQDSKDST | 70 |

| | | |
|---|---|---|
| | YSLSSTLTLSKADYEKHKVYACEVTHQGLSSPVTKSFNRGECGGGGSGGGGSGGGGSEP KSSDKTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKT ISKAKGQPREPQVYTLPPCREEMTKNQVSLWCLVKGFYPSDIAVEWESNGQPENNYKTT PPVLDSDGSFFLYSKLTVDKSRWQQGNVFSCSVMHEALHNHYTQKSLSLSPGK | |
| GPM04 HC | EVQLVETGGGLVQPKGSLKLSCAASGFTFNASAMNWVRQAPGKCLEWVARIRSKSNNYA IYYADSVKDRFTISRDDSQSMLYLQMNNLKTEDTAMYYCVRDPGYYGNPWFAYWGQGTL VTVSSASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKKVEPKSCDKTHTCPPCP APELLGGPSVFLFPPKPKDTLMISRTPEVTCVVVDVSHEDPEVKFNWYVDGVEVHNAKT KPREEQYNSTYRVVSVLTVLHQDWLNGKEYKCKVSNKALPAPIEKTISKAKGQPREPQV YTLPPCREEMTKNQVSLWCLVKGFYPSDIAVEWESNGQPENNYKTTPPVLDSDGSFFLY SKLTVDKSRWQQGNVFSCSVMHEALHNHYTQKSLSLSPGK | 71 |
| GPM04 LC | QIVLTQSPAIMSAFPGEKVTMTCSASSSVSYMYWYQQKSGSSPRLLIYDTSNLASGVPV RFSGSGSGTSYSLTISRMEAEDAATYYCQQWSSYPLTFGCGTKLEIKRTVAAPSVFIFP PSDEQLKSGTASVVCLLNNFYPREAKVQWKVDNALQSGNSQESVTEQDSKDSTYSLSST LTLSKADYEKHKVYACEVTHQGLSSPVTKSFNRGECGGGGSGGGGSGGGGSEPKSSDKT HTCPPCPAPELLGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKAKG QPREPQVYTLPPCREEMTKNQVSLWCLVKGFYPSDIAVEWESNGQPENNYKTTPPVLDS DGSFFLYSKLTVDKSRWQQGNVFSCSVMHEALHNHYTQKSLSLSPGK | 72 |
| GPB01 HC | QVQLVQSGAEVKKPGASVKVSCKASGYTFTDYEMHWVRQAPGQGLEWMGALDPKTGDTA YSQKFKGRVTLTADKSTSTAYMELSSLTSEDTAVYYCTRFYSYTYWGQGTLVTVSSAST KGPSVFPLAPEVQLVESGGGLVKPGGSLKLSCAASGFTFSRYAMSWVRQIPEKCLEWVA AIDSSGGDTYYLDTVKDRFTISRDNANNTLHLQMRSLRSEDTALYYCVRQGGAYWGQGT LVTVSSASTKGPSVFPLAPSSKSTSGGTAALGCLVKDYFPEPVTVSWNSGALTSGVHTF PAVLQSSGLYSLSSVVTVPSSSLGTQTYICNVNHKPSNTKVDKKVEPKSCDKTHTCPPC PAPELLGGPSVFLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTISKAKGQPREPQ | 73 |

| | | | |
|---|---|---|---|
| | | VYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | |
| GPB01 LC | | DVVMTQSPLSLPVTPGEPASISCRSSQSLVHSNRNTYLHWYLQKPGQSPQLLIYKVSNRFSGVPDRFSGSGSGTDFTLKISRVEAEDVGVYYCSQNTHVPPTFGQGTKLEIKRTVAAPSVFIFPPDVVMTQTPLTLSVTIGQPASISCKSSQSLLDSDGKTYLNWLLQRPGQSPKRLIYLVSKLDSGAPDRFTGSGSGTDFTLKISRVEAEDLGIYYCWQGTHFPLTFGCGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGECGGGGSGGGGSGGGGSEPKSSDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 74 |
| GPB03 HC | | QVQLVQSGAEVKKPGASVKVSCKASGYTFTDYEMHWVRQAPGQGLEWMGALDPKTGDTAYSQKFKGRVTLTADKSTSTAYMELSSLTSEDTAVYYCTRFYSYTYWGQGTLVTVSSASTKGPSVFPLAPEVQLVETGGGLVQPKGSLKLSCAASGFTFNASAMNWVRQAPGKCLEWVARIRSKSNNYAIYYADSVKDRFTISRDDSQSMLYLQMNNLKTEDTAMYYCVRDPGYYGNPWFAYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 75 |
| GPB03 LC | | DVVMTQSPLSLPVTPGEPASISCRSSQSLVHSNRNTYLHWYLQKPGQSPQLLIYKVSNRFSGVPDRFSGSGSGTDFTLKISRVEAEDVGVYYCSQNTHVPPTFGQGTKLEIKRTVAAPSVFIFPPQIVLTQSPAIMSAFPGEKVTMTCSASSSVSYMYWYQQKSGSSPRLLIYDTSNLASGVPVRFSGSGSGTSYSLTISRMEAEDAATYYCQQWSSYPLTFGCGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGECGGGGSGGGGSGGGGSE | 76 |

| | | |
|---|---|---|
| | PKSSDKTHTCPPCPAPELLGGPSVFLFPPKPKDTLMISRTPEVTCVVVDVSHEDPEVKF NWYVDGVEVHNAKTKPREEQYNSTYRVVSVLTVLHQDWLNGKEYKCKVSNKALPAPIEK TISKAKGQPREPQVYTLPPCREEMTKNQVSLWCLVKGFYPSDIAVEWESNGQPENNYKT TPPVLDSDGSFFLYSKLTVDKSRWQQGNVFSCSVMHEALHNHYTQKSLSLSPGK | |
| GPB04 HC | EVQLVESGGGLVKPGGSLKLSCAASGFTFSRYAMSWVRQIPEKILEWVAAIDSSGGDTY YLDTVKDRFTISRDNANNTLHLQMRSLRSEDTALYYCVRQGGAYWGQGTLVTVSSASTK GPSVFPLAPQVQLVQSGAEVKKPGASVKVSCKASGYTFTDYEMHWVRQAPGQCLEWMGA LDPKTGDTAYSQKFKGRVTLTADKSTSTAYMELSSLTSEDTAVYYCTRFYSYTYWGQGT LVTVSSASTKGPSVFPLAPSSKSTSGGTAALGCLVKDYFPEPVTVSWNSGALTSGVHTF PAVLQSSGLYSLSSVVTVPSSSLGTQTYICNVNHKPSNTKVDKKVEPKSCDKTHTCPPC PAPELLGGPSVFLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTISKAKGQPREPQ VYTLPPCREEMTKNQVSLWCLVKGFYPSDIAVEWESNGQPENNYKTTPPVLDSDGSFFL YSKLTVDKSRWQQGNVFSCSVMHEALHNHYTQKSLSLSPGK | 77 |
| GPB04 LC | DVVMTQTPLTLSVTIGQPASISCKSSQSLLDSDGKTYLNWLLQRPGQSPKRLIYLVSKL DSGAPDRFTGSGSGTDFTLKISRVEAEDLGIYYCWQGTHFPLTFGAGTKLEIKRTVAAP SVFIFPPDVVMTQSPLSLPVTPGEPASISCRSSQSLVHSNRNTYLHWYLQKPGQSPQLL IYKVSNRFSGVPDRFSGSGSGTDFTLKISRVEAEDVGVYYCSQNTHVPPTFGCGTKLEI KRTVAAPSVFIFPPSDEQLKSGTASVVCLLNNFYPREAKVQWKVDNALQSGNSQESVTE QDSKDSTYSLSSTLTLSKADYEKHKVYACEVTHQGLSSPVTKSFNRGECGGGGSGGGGS GGGGSEPKSSDKTHTCPPCPAPELLGGPSVFLFPPKPKDTLMISRTPEVTCVVVDVSHE DPEVKFNWYVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWESNGQP ENNYKTTPPVLDSDGSFFLYSKLTVDKSRWQQGNVFSCSVMHEALHNHYTQKSLSLSPG K | 78 |
| GPB06 HC | EVQLVETGGGLVQPKGSLKLSCAASGFTFNASAMNWVRQAPGKGLEWVARIRSKSNNYA IYYADSVKDRFTISRDDSQSMLYLQMNNLKTEDTAMYYCVRDPGYYGNPWFAYWGQGTL VTVSSASTKGPSVFPLAPQVQLVQSGAEVKKPGASVKVSCKASGYTFTDYEMHWVRQAP GQCLEWMGALDPKTGDTAYSQKFKGRVTLTADKSTSTAYMELSSLTSEDTAVYYCTRFY | 79 |

| | | |
|---|---|---|
| | SYTYWGQGTLVTVSSASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKKVEPKSC DKTHTCPPCPAPELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTISK AKGQPREPQVYTLPPCREEMTKNQVSLWCLVKGFYPSDIAVEWESNGQPENNYKTTPPV LDSDGSFFLYSKLTVDKSRWQQGNVFSCSVMHEALHNHYTQKSLSLSPGK | |
| GPB06 LC | QIVLTQSPAIMSAFPGEKVTMTCSASSSVSYMYWYQQKSGSSPRLLIYDTSNLASGVPV RFSGSGSGTSYSLTISRMEAEDAATYYCQQWSSYPLTFGGGTKLEIKRTVAAPSVFIFP PDVVMTQSPLSLPVTPGEPASISCRSSQSLVHSNRNTYLHWYLQKPGQSPQLLIYKVSN RFSGVPDRFSGSGSGTDFTLKISRVEAEDVGVYYCSQNTHVPPTFGCGTKLEIKRTVAA PSVFIFPPSDEQLKSGTASVVCLLNNFYPREAKVQWKVDNALQSGNSQESVTEQDSKDS TYSLSSTLTLSKADYEKHKVYACEVTHQGLSSPVTKSFNRGECGGGGSGGGGSGGGGSE PKSSDKTHTCPPCPAPELLGGPSVFLFPPKPKDTLMISRTPEVTCVVVDVSHEDPEVKF NWYVDGVEVHNAKTKPREEQYNSTYRVVSVLTVLHQDWLNGKEYKCKVSNKALPAPIEK TISKAKGQPREPQVYTLPPCREEMTKNQVSLWCLVKGFYPSDIAVEWESNGQPENNYKT TPPVLDSDGSFFLYSKLTVDKSRWQQGNVFSCSVMHEALHNHYTQKSLSLSPGK | 80 |

[Table 47]

| Name | Sequence | SEQ ID NO |
|---|---|---|
| GPM01 HC | GAAGTTCAGCTTGTCGAAAGTGGCGGAGGTCTTGTAAAGCCAGGAGGGTCCCTGAAGTT GAGTTGTGCTGCCTCTGGCTTCACCTTTTTCACGGTATGCCATGTCCTGGGTTCGACAGA TACCTGAGAAGTGTCTTGAATGGGTGGCCGCAATAGACAGTAGCGGTGGTGACACCTAT TACCTCGACACAGTCAAAGACCGCTTTACTATCTCACGCGATAACGCCAACAATACCCT GCACTTGCAGATGCGATCACTTCGTTCAGAAGACACTGCTCTTTACTATTGTGTACGCC AAGGGGGAGCATACTGGGGTCAGGGAACACTGGTTACCGTGTCTTCAGCTAGCACCAAA GGACCTAGTGTTTTTCCTCTTGCCCCTTCCTCAAAGTCTACCTCTGGGGGGACAGCCGC TCTGGGCTGCCTGGTCAAGGATTATTTCCCAGAGCCTGTCACTGTCAGTTGGAACTCTG GAGCCTTGACTTCTGGTGTTCATACATTTCCTGCTGTCCTTCAAAGCAGCGGCTTGTAC TCATTGTCTTCTGTTGTGACAGTACCCTCAAGCAGCCTCGGCACTCAGACATACATCTG CAATGTCAACCACAAACCCTCAAATACAAAGGTAGATAAAAAAGTCGAACCAAAGTCTT | 504 |

| | | | |
|---|---|---|---|
| | | GTGACAAAACTCACACGTGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATGCCGGGAGGAGATGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | |
| GPM01 LC | | GACGTGGTTATGACTCAGACTCCTCTGACTCTCTCCGTCACTATTGGTCAGCCCGCTTCCATATCATGTAAATCATCACAATCTCTTCTTGATAGCGATGGCAAGACTTATTTGAACTGGTTGTTGCAACGCCCAGGTCAGAGCCCTAAGAGACTTATCTATTTGGTGAGCAAACTCGACAGCGGTGCACCCGATCGTTTTACCGGAAGCGGCAGCGGCACCGATTTCACACTGAAGATCAGTAGGGTCGAAGCTGAAGACCTGGGAATCTACTACTGCTGGCAAGGTACTCACTTTCCCCTGACTTTCGGCTGCGGTACTAAACTTGAGATCAAACGTACGGTGGCAGCTCCCAGCGTTTTTATCTTTCCCCCATCCGACGAGCAGCTCAAGAGTGGCACTGCCTCTGTAGTTTGTTTGCTGAATAACTTCTATCCACGTGAAGCAAAAGTACAGTGGAAGGTCGATAATGCCCTTCAGAGCGGTAACAGCCAAGAAAGTGTTACCGAGCAAGATTCCAAAGATTCCACTTACAGTCTGTCCAGCACATTGACACTGAGTAAGGCTGATTACGAAAAACACAAGGTGTACGCATGCGAGGTGACACACCAAGGTCTTTCATCTCCTGTAACTAAGAGCTTTAACCGGGGAGAATGTGGTGGTGGGGGCAGCGGGGGCGGAGGTAGTGGAGGCGGCGGTAGTGAACCAAAGAGTAGTGACAAAACTCACACGTGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATG | 505 |

| | | | |
|---|---|---|---|
| | | GCAAGGAGTACAAGTGCAAGGTCTCCAACAAAGCCCTCCCAGCCCCCATCGAGAAAACC ATCTCCAAAGCCAAAGGGCAGCCCCGAGAACCACAGGTGTACACCCTGCCCCCATGCCG GGAGGAGATGACCAAGAACCAGGTCAGCCTGTGGTGCCTGGTCAAAGGCTTCTATCCCA GCGACATCGCCGTGGAGTGGGAGAGCAATGGGCAGCCGGAGAACAACTACAAGACCACG CCTCCCGTGCTGGACTCCGACGGCTCCTTCTTCCTCTACAGCAAGCTCACCGTGGACAA GAGCAGGTGGCAGCAGGGGAACGTCTTCTCATGCTCCGTGATGCATGAGGCTCTGCACA ACCACTACACGCAGAAGAGCCTCTCCCTGTCTCCGGGTAAA | |
| GPMO2 HC | CAGGTTCAGCTTGTGCAATCAGGCGCTGAAGTCAAAAAACCAGGAGCAAGCGTGAAAGT AAGCTGTAAGGCTTCCGGTTACACTTTCACCGATTACGAAATGCACTGGGTACGCCAGG CTCCTGGACAATGTCTGGAATGGATGGGCGCACTTGATCCAAAAACAGGTGACACTGCT TACAGTCAAAAATTCAAAGGTCGTGTCACTCTTACAGCAGATAAGTCCACCAGTACCGC TTATATGGAGCTTAGCTCCCTGACTTCCGAGGACACCGCCGTGTATTATTGTACAAGAT TCTACTCATATACTTACTGGGGCCAAGGAACCCTGGTGACAGTGTCATCTGCTAGCACC AAAGGACCTAGTGTTTTTCCTCTTGCCCCTTCCTCAAAGTCTACCTCTGGGGGGACAGC CGCTCTGGGCTGCCTGGTCAAGGATTATTTCCCAGAGCCTGTCACTGTCAGTTGGAACT CTGGAGCCTTGACTTCTGGTGTTCATACATTTCCTGCTGTCCTTCAAAGCAGCGGCTTG TACTCATTGTCTTCTGTTGTGACAGTACCCTCAAGCAGCCTCGGCACTCAGACATACAT CTGCAATGTCAACCACAAACCCTCAAATACAAAGGTAGATAAAAAAGTCGAACCAAAGT CTTGTGACAAAACTCACACGTGCCCACCGTGCCCAGCACCTGAACTCCTGGGGGGACCG TCAGTCTTCCTCTTCCCCCCAAAACCCAAGGACACCCTCATGATCTCCCGGACCCCTGA GGTCACATGCGTGGTGGTGGACGTGAGCCACGAAGACCCTGAGGTCAAGTTCAACTGGT ACGTGGACGGCGTGGAGGTGCATAATGCCAAGACAAAGCCGCGGGAGGAGCAGTACAAC AGCACGTACCGTGTGGTCAGCGTCCTCACCGTCCTGCACCAGGACTGGCTGAATGGCAA GGAGTACAAGTGCAAGGTCTCCAACAAAGCCCTCCCAGCCCCCATCGAGAAAACCATCT CCAAAGCCAAAGGGCAGCCCCGAGAACCACAGGTGTACACCCTGCCCCCATGCCGGGAG GAGATGACCAAGAACCAGGTCAGCCTGTGGTGCCTGGTCAAAGGCTTCTATCCCAGCGA CATCGCCGTGGAGTGGGAGAGCAATGGGCAGCCGGAGAACAACTACAAGACCACGCCTC CCGTGCTGGACTCCGACGGCTCCTTCTTCCTCTACAGCAAGCTCACCGTGGACAAGAGC AGGTGGCAGCAGGGGAACGTCTTCTCATGCTCCGTGATGCATGAGGCTCTGCACAACCA | 506 |

| | | |
|---|---|---|
| | CTACACGCAGAAGAGCCTCTCCCTGTCTCCGGGTAAA | |
| GPM02 LC | GATGTTGTTATGACTCAGTCACCTCTCTCACTTCCTGTAACCCCTGGTGAACCAGCCTC TATAAGCTGCCGGTCAAGTCAAAGCCTGGTTCATAGCAACCGTAACACTTACCTTCACT GGTACTTGCAAAAACCTGGTCAGTCCCCACAACTCTTGATCTACAAAGTCTCCAATCGC TTCTCTGGAGTCCCTGACAGGTTTTCTGGTAGTGGATCAGGTACAGACTTCACTTTGAA AATCAGCCGCGTTGAGGCCGAGGACGTGGGAGTGTATTATTGCTCTCAGAATACCCATG TACCCCCAACCTTTGGCTGTGGGACTAAACTCGAGATTAAACGTACGGTGGCAGCTCCC AGCGTTTTTATCTTTCCCCCATCCGACGAGCAGCTCAAGAGTGGCACTGCCTCTGTAGT TTGTTTGCTGAATAACTTCTATCCACGTGAAGCAAAAGTACAGTGGAAGGTCGATAATG CCCTTCAGAGCGGTAACAGCCAAGAAAGTGTTACCGAGCAAGATTCCAAAGATTCCACT TACAGTCTGTCCAGCACATTGACACTGAGTAAGGCTGATTACGAAAAACACAAGGTGTA CGCATGCGAGGTGACACACCAAGGTCTTTCATCTCCTGTAACTAAGAGCTTTAACCGGG GAGAATGTGGTGGTGGGGGCAGCGGGGGCGGAGGTAGTGGAGGCGGCGGTAGTGAACCA AAGAGTAGTGACAAAACTCACACGTGCCCACCGTGCCCAGCACCTGAACTCCTGGGGGG ACCGTCAGTCTTCCTCTTCCCCCCAAAACCCAAGGACACCCTCATGATCTCCCGGACCC CTGAGGTCACATGCGTGGTGGTGGACGTGAGCCACGAAGACCCTGAGGTCAAGTTCAAC TGGTACGTGGACGGCGTGGAGGTGCATAATGCCAAGACAAAGCCGCGGGAGGAGCAGTA CAACAGCACGTACCGTGTGGTCAGCGTCCTCACCGTCCTGCACCAGGACTGGCTGAATG GCAAGGAGTACAAGTGCAAGGTCTCCAACAAAGCCCTCCCAGCCCCCATCGAGAAAACC ATCTCCAAAGCCAAAGGGCAGCCCCGAGAACCACAGGTGTACACCCTGCCCCCATGCCG GGAGGAGATGACCAAGAACCAGGTCAGCCTGTGGTGCCTGGTCAAAGGCTTCTATCCCA GCGACATCGCCGTGGAGTGGGAGAGCAATGGGCAGCCGGAGAACAACTACAAGACCACG CCTCCCGTGCTGGACTCCGACGGCTCCTTCTTCCTCTACAGCAAGCTCACCGTGGACAA GAGCAGGTGGCAGCAGGGGAACGTCTTCTCATGCTCCGTGATGCATGAGGCTCTGCACA ACCACTACACGCAGAAGAGCCTCTCCCTGTCTCCGGGTAAA | 507 |
| GPM04 HC | GAAGTGCAACTGGTCGAAACAGGCGGGGGACTGGTACAGCCCAAGGGATCTTTGAAACT TAGTTGTGCTGCTAGTGGGTTTACATTCAATGCCTCCGCAATGAACTGGGTAAGACAAG CTCCTGGCAAGTGCCTGGAATGGGTGGCCCGTATTCGCTCTAAAAGTAATAACTACGCT ATTTATTACGCTGATTCTGTAAAGGATCGGTTTACAATAAGTCGGGACGACAGCCAATC | 508 |

| | CATGCTGTATCTCCAAATGAATAACCTGAAAACAGAGGATACTGCCATGTACTATTGTG TGCGGGACCCAGGCTATTACGGGAATCCCTGGTTCGCCTATTGGGGACAGGGCACTCTG GTTACCGTATCATCAGCTAGCACCAAAGGACCTAGTGTTTTTCCTCTTGCCCCTTCCTC AAAGTCTACCTCTGGGGGGACAGCCGCTCTGGGCTGCCTGGTCAAGGATTATTTCCCAG AGCCTGTCACTGTCAGTTGGAACTCTGGAGCCTTGACTTCTGGTGTTCATACATTTCCT GCTGTCCTTCAAAGCAGCGGCTTGTACTCATTGTCTTCTGTTGTGACAGTACCCTCAAG CAGCCTCGGCACTCAGACATACATCTGCAATGTCAACCACAAACCCTCAAATACAAAGG TAGATAAAAAAGTCGAACCAAAGTCTTGTGACAAAACTCACACGTGCCCACCGTGCCCA GCACCTGAACTCCTGGGGGGACCGTCAGTCTTCCTCTTCCCCCCAAAACCCAAGGACAC CCTCATGATCTCCCGGACCCCTGAGGTCACATGCGTGGTGGTGGACGTGAGCCACGAAG ACCCTGAGGTCAAGTTCAACTGGTACGTGGACGGCGTGGAGGTGCATAATGCCAAGACA AAGCCGCGGGAGGAGCAGTACAACAGCACGTACCGTGTGGTCAGCGTCCTCACCGTCCT GCACCAGGACTGGCTGAATGGCAAGGAGTACAAGTGCAAGGTCTCCAACAAAGCCCTCC CAGCCCCCATCGAGAAAACCATCTCCAAAGCCAAAGGGCAGCCCCGAGAACCACAGGTG TACACCCTGCCCCCATGCCGGGAGGAGATGACCAAGAACCAGGTCAGCCTGTGGTGCCT GGTCAAAGGCTTCTATCCCAGCGACATCGCCGTGGAGTGGGAGAGCAATGGGCAGCCGG AGAACAACTACAAGACCACGCCTCCCGTGCTGGACTCCGACGGCTCCTTCTTCCTCTAC AGCAAGCTCACCGTGGACAAGAGCAGGTGGCAGCAGGGGAACGTCTTCTCATGCTCCGT GATGCATGAGGCTCTGCACAACCACTACACGCAGAAGAGCCTCTCCCTGTCTCCGGGTA AA | |
| GPM04 LC | CAAATTGTCCTCACCCAATCACCAGCTATAATGTCTGCTTTTCCCGGCGAGAAAGTAAC CATGACTTGTAGCGCCTCTAGTAGCGTGTCATATATGTATTGGTATCAACAAAAGAGCG GTAGTTCACCTCGACTCCTTATCTACGACACAAGTAATCTCGCTAGTGGTGTCCCAGTC CGTTTCTCCGGGAGCGGCAGCGGCACATCATACTCCCTGACCATCTCCAGAATGGAGGC CGAGGACGCTGCCACATACTATTGTCAGCAGTGGAGCTCATATCCTTTGACATTCGGTT GCGGTACTAAACTCGAAATCAAGCGTACGGTGGCAGCTCCCAGCGTTTTTATCTTTCCC CCATCCGACGAGCAGCTCAAGAGTGGCACTGCCTCTGTAGTTTGTTTGCTGAATAACTT CTATCCACGTGAAGCAAAAGTACAGTGGAAGGTCGATAATGCCCTTCAGAGCGGTAACA GCCAAGAAAGTGTTACCGAGCAAGATTCCAAAGATTCCACTTACAGTCTGTCCAGCACA | 509 |

| | | |
|---|---|---|
| | TTGACACTGAGTAAGGCTGATTACGAAAAACACAAGGTGTACGCATGCGAGGTGACACACCAAGGTCTTTCATCTCCTGTAACTAAGAGCTTTAACCGGGGAGAATGTGGTGGTGGGGGCAGCGGGGGCGGAGGTAGTGGAGGCGGCGGTAGTGAACCAAAGAGTAGTGACAAAACTCACACGTGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAGGGCAGCCCCGAGAACCACAGGTGTACACCCTGCCCCCATGCCGGGAGGAGATGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | |
| GPB01 HC | CAGGTTCAGCTTGTGCAATCAGGCGCTGAAGTCAAAAAACCAGGAGCAAGCGTGAAAGTAAGCTGTAAGGCTTCCGGTTACACTTTCACCGATTACGAAATGCACTGGGTACGCCAGGCTCCTGGACAAGGCCTGGAATGGATGGGCGCACTTGATCCAAAAACAGGTGACACTGCTTACAGTCAAAAATTCAAAGGTCGTGTCACTCTTACAGCAGATAAGTCCACCAGTACCGCTTATATGGAGCTTAGCTCCCTGACTTCCGAGGACACCGCCGTGTATTATTGTACAAGATTCTACTCATATACTTACTGGGGCCAAGGAACCCTGGTGACAGTGTCATCTGCTAGCACCAAAGGACCTAGTGTTTTTCCTCTTGCCCCTGAAGTTCAGCTTGTCGAAAGTGGCGGAGGTCTTGTAAAGCCAGGAGGGTCCCTGAAGTTGAGTTGTGCTGCCTCTGGCTTCACCTTTTCACGGTATGCCATGTCCTGGGTTCGACAGATACCTGAGAAGTGTCTTGAATGGGTGGCCGCAATAGACAGTAGCGGTGGTGACACCTATTACCTCGACACAGTCAAAGACCGCTTTACTATCTCACGCGATAACGCCAACAATACCCTGCACTTGCAGATGCGATCACTTCGTTCAGAAGACACTGCTCTTTACTATTGTGTACGCCAAGGGGGAGCATACTGGGGTCAGGGAACACTGGTTACCGTGTCTTCAGCTAGCACCAAAGGACCTAGTGTTTTTCCTCTTGCCCCTTCCTCAAAGTCTACCTCTGGGGGGACAGCCGCTCTGGGCTGCCTGGTCAAGGATTATTTCC | 510 |

| | | |
|---|---|---|
| | CAGAGCCTGTCACTGTCAGTTGGAACTCTGGAGCCTTGACTTCTGGTGTTCATACATTT CCTGCTGTCCTTCAAAGCAGCGGCTTGTACTCATTGTCTTCTGTTGTGACAGTACCCTC AAGCAGCCTCGGCACTCAGACATACATCTGCAATGTCAACCACAAACCCTCAAATACAA AGGTAGATAAAAAAGTCGAACCAAAGTCTTGTGACAAAACTCACACGTGCCCACCGTGC CCAGCACCTGAACTCCTGGGGGGACCGTCAGTCTTCCTCTTCCCCCCCAAAACCCAAGGA CACCCTCATGATCTCCCGGACCCCTGAGGTCACATGCGTGGTGGTGGACGTGAGCCACG AAGACCCTGAGGTCAAGTTCAACTGGTACGTGGACGGCGTGGAGGTGCATAATGCCAAG ACAAAGCCGCGGGAGGAGCAGTACAACAGCACGTACCGTGTGGTCAGCGTCCTCACCGT CCTGCACCAGGACTGGCTGAATGGCAAGGAGTACAAGTGCAAGGTCTCCAACAAAGCCC TCCCAGCCCCCATCGAGAAAACCATCTCCAAAGCCAAAGGGCAGCCCCGAGAACCACAG GTGTACACCCTGCCCCCATGCCGGGAGGAGATGACCAAGAACCAGGTCAGCCTGTGGTG CCTGGTCAAAGGCTTCTATCCCAGCGACATCGCCGTGGAGTGGGAGAGCAATGGGCAGC CGGAGAACAACTACAAGACCACGCCTCCCGTGCTGGACTCCGACGGCTCCTTCTTCCTC TACAGCAAGCTCACCGTGGACAAGAGCAGGTGGCAGCAGGGGAACGTCTTCTCATGCTC CGTGATGCATGAGGCTCTGCACAACCACTACACGCAGAAGAGCCTCTCCCTGTCTCCGG GTAAA | |
| GPB01 LC | GATGTTGTTATGACTCAGTCACCTCTCTCACTTCCTGTAACCCCTGGTGAACCAGCCTC TATAAGCTGCCGGTCAAGTCAAAGCCTGGTTCATAGCAACCGTAACACTTACCTTCACT GGTACTTGCAAAAACCTGGTCAGTCCCCACAACTCTTGATCTACAAAGTCTCCAATCGC TTCTCTGGAGTCCCTGACAGGTTTTCTGGTAGTGGATCAGGTACAGACTTCACTTTGAA AATCAGCCGCGTTGAGGCCGAGGACGTGGGAGTGTATTATTGCTCTCAGAATACCCATG TACCCCCAACCTTTGGCCAAGGGACTAAACTCGAGATTAAACGTACGGTGGCCGCTCCC TCCGTGTTCATCTTCCCACCCGACGTGGTTATGACTCAGACTCCTCTGACTCTCTCCGT CACTATTGGTCAGCCCGCTTCCATATCATGTAAATCATCACAATCTCTTCTTGATAGCG ATGGCAAGACTTATTTGAACTGGTTGTTGCAACGCCCAGGTCAGAGCCCTAAGAGACTT ATCTATTTGGTGAGCAAACTCGACAGCGGTGCACCCGATCGTTTTACCGGAAGCGGCAG CGGCACCGATTTCACACTGAAGATCAGTAGGGTCGAAGCTGAAGACCTGGGAATCTACT ACTGCTGGCAAGGTACTCACTTTCCCCTGACTTTCGGCTGCGGTACTAAACTTGAGATC AAACGTACGGTGGCAGCTCCCAGCGTTTTTATCTTTCCCCCATCCGACGAGCAGCTCAA | 511 |

| | | | |
|---|---|---|---|
| | | GAGTGGCACTGCCTCTGTAGTTTGTTTGCTGAATAACTTCTATCCACGTGAAGCAAAAGTACAGTGGAAGGTCGATAATGCCCTTCAGAGCGGTAACAGCCAAGAAAGTGTTACCGAGCAAGATTCCAAAGATTCCACTTACAGTCTGTCCAGCACATTGACACTGAGTAAGGCTGATTACGAAAAACACAAGGTGTACGCATGCGAGGTGACACACCAAGGTCTTTCATCTCCTGTAACTAAGAGCTTTAACCGGGGAGAATGTGGTGGTGGGGGCAGCGGGGGCGGAGGTAGTGGAGGCGGCGGTAGTGAACCAAAGAGTAGTGACAAAACTCACACGTGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATGCCGGGAGGAGATGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | |
| GPB03 HC | | CAGGTTCAGCTTGTGCAATCAGGCGCTGAAGTCAAAAAACCAGGAGCAAGCGTGAAAGTAAGCTGTAAGGCTTCCGGTTACACTTTCACCGATTACGAAATGCACTGGGTACGCCAGGCTCCTGGACAAGGCCTGGAATGGATGGGCGCACTTGATCCAAAAAACAGGTGACACTGCTTACAGTCAAAAATTCAAAGGTCGTGTCACTCTTACAGCAGATAAGTCCACCAGTACCGCTTATATGGAGCTTAGCTCCCTGACTTCCGAGGACACCGCCGTGTATTATTGTACAAGATTCTACTCATATACTTACTGGGGCCAAGGAACCCTGGTGACAGTGTCATCTGCTAGCACCAAAGGACCTAGTGTTTTTCCTCTTGCCCCTGAAGTGCAACTGGTCGAAACAGGCGGGGGACTGGTACAGCCCAAGGGATCTTTGAAACTTAGTTGTGCTGCTAGTGGGTTTACATTCAATGCCTCCGCAATGAACTGGGTAAGACAAGCTCCTGGCAAGTGCCTGGAATGGGTGGCCCGTATTCGCTCTAAAAGTAATAACTACGCTATTTATTACGCTGATTCTGTAAAGGATCGGTTTACAATAAGTCGGGACGACAGCCAATCCATGCTGTATCTCCAAATGAATAACCTGA | 512 |

138

| | | |
|---|---|---|
| | AAACAGAGGATACTGCCATGTACTATTGTGTGCGGGACCCAGGCTATTACGGGAATCCC<br>TGGTTCGCCTATTGGGGACAGGGCACTCTGGTTACCGTATCATCAGCTAGCACCAAAGG<br>ACCTAGTGTTTTTCCTCTTGCCCCTTCCTCAAAGTCTACCTCTGGGGGGACAGCCGCTC<br>TGGGCTGCCTGGTCAAGGATTATTTCCCAGAGCCTGTCACTGTCAGTTGGAACTCTGGA<br>GCCTTGACTTCTGGTGTTCATACATTTCCTGCTGTCCTTCAAAGCAGCGGCTTGTACTC<br>ATTGTCTTCTGTTGTGACAGTACCCTCAAGCAGCCTCGGCACTCAGACATACATCTGCA<br>ATGTCAACCACAAACCCTCAAATACAAAGGTAGATAAAAAAGTCGAACCAAAGTCTTGT<br>GACAAAACTCACACGTGCCCACCGTGCCCAGCACCTGAACTCCTGGGGGGACCGTCAGT<br>CTTCCTCTTCCCCCCAAAACCCAAGGACACCCTCATGATCTCCCGGACCCCTGAGGTCA<br>CATGCGTGGTGGTGGACGTGAGCCACGAAGACCCTGAGGTCAAGTTCAACTGGTACGTG<br>GACGGCGTGGAGGTGCATAATGCCAAGACAAAGCCGCGGGAGGAGCAGTACAACAGCAC<br>GTACCGTGTGGTCAGCGTCCTCACCGTCCTGCACCAGGACTGGCTGAATGGCAAGGAGT<br>ACAAGTGCAAGGTCTCCAACAAAGCCCTCCCAGCCCCCATCGAGAAAACCATCTCCAAA<br>GCCAAAGGGCAGCCCCGAGAACCACAGGTGTACACCCTGCCCCCATGCCGGGAGGAGAT<br>GACCAAGAACCAGGTCAGCCTGTGGTGCCTGGTCAAAGGCTTCTATCCCAGCGACATCG<br>CCGTGGAGTGGGAGAGCAATGGGCAGCCGGAGAACAACTACAAGACCACGCCTCCCGTG<br>CTGGACTCCGACGGCTCCTTCTTCCTCTACAGCAAGCTCACCGTGGACAAGAGCAGGTG<br>GCAGCAGGGGAACGTCTTCTCATGCTCCGTGATGCATGAGGCTCTGCACAACCACTACA<br>CGCAGAAGAGCCTCTCCCTGTCTCCGGGTAAA | |
| GPB03 LC | GATGTTGTTATGACTCAGTCACCTCTCTCACTTCCTGTAACCCCTGGTGAACCAGCCTC<br>TATAAGCTGCCGGTCAAGTCAAAGCCTGGTTCATAGCAACCGTAACACTTACCTTCACT<br>GGTACTTGCAAAAACCTGGTCAGTCCCCACAACTCTTGATCTACAAAGTCTCCAATCGC<br>TTCTCTGGAGTCCCTGACAGGTTTTCTGGTAGTGGATCAGGTACAGACTTCACTTTGAA<br>AATCAGCCGCGTTGAGGCCGAGGACGTGGGAGTGTATTATTGCTCTCAGAATACCCATG<br>TACCCCCAACCTTTGGCCAAGGGACTAAACTCGAGATTAAACGTACGGTGGCCGCTCCC<br>TCCGTGTTCATCTTCCCACCCCAAATTGTCCTCACCCAATCACCAGCTATAATGTCTGC<br>TTTTCCCGGCGAGAAAGTAACCATGACTTGTAGCGCCTCTAGTAGCGTGTCATATATGT<br>ATTGGTATCAACAAAAGAGCGGTAGTTCACCTCGACTCCTTATCTACGACACAAGTAAT<br>CTCGCTAGTGGTGTCCCAGTCCGTTTCTCCGGGAGCGGCAGCGGCACATCATACTCCCT | 513 |

| | | | |
|---|---|---|---|
| | | GACCATCTCCAGAATGGAGGCCGAGGACGCTGCCACATACTATTGTCAGCAGTGGAGCT CATATCCTTTGACATTCGGTTGCGGTACTAAACTCGAAATCAAGCGTACGGTGGCAGCT CCCAGCGTTTTTATCTTTCCCCCATCCGACGAGCAGCTCAAGAGTGGCACTGCCTCTGT AGTTTGTTTGCTGAATAACTTCTATCCACGTGAAGCAAAAGTACAGTGGAAGGTCGATA ATGCCCTTCAGAGCGGTAACAGCCAAGAAAGTGTTACCGAGCAAGATTCCAAAGATTCC ACTTACAGTCTGTCCAGCACATTGACACTGAGTAAGGCTGATTACGAAAAACACAAGGT GTACGCATGCGAGGTGACACACCAAGGTCTTTCATCTCCTGTAACTAAGAGCTTTAACC GGGGAGAATGTGGTGGTGGGGGGCAGCGGGGGCGGAGGTAGTGGAGGCGGCGGTAGTGAA CCAAAGAGTAGTGACAAAACTCACACGTGCCCACCGTGCCCAGCACCTGAACTCCTGGG GGGACCGTCAGTCTTCCTCTTCCCCCCAAAACCCAAGGACACCCTCATGATCTCCCGGA CCCCTGAGGTCACATGCGTGGTGGTGGACGTGAGCCACGAAGACCCTGAGGTCAAGTTC AACTGGTACGTGGACGGCGTGGAGGTGCATAATGCCAAGACAAAGCCGCGGGAGGAGCA GTACAACAGCACGTACCGTGTGGTCAGCGTCCTCACCGTCCTGCACCAGGACTGGCTGA ATGGCAAGGAGTACAAGTGCAAGGTCTCCAACAAAGCCCTCCCAGCCCCCATCGAGAAA ACCATCTCCAAAGCCAAAGGGCAGCCCCGAGAACCACAGGTGTACACCCTGCCCCCCATG CCGGGAGGAGATGACCAAGAACCAGGTCAGCCTGTGGTGCCTGGTCAAAGGCTTCTATC CCAGCGACATCGCCGTGGAGTGGGAGAGCAATGGGCAGCCGGAGAACAACTACAAGACC ACGCCTCCCGTGCTGGACTCCGACGGCTCCTTCTTCCTCTACAGCAAGCTCACCGTGGA CAAGAGCAGGTGGCAGCAGGGGAACGTCTTCTCATGCTCCGTGATGCATGAGGCTCTGC ACAACCACTACACGCAGAAGAGCCTCTCCCTGTCTCCGGGTAAA | |
| GPB04 HC | GAAGTTCAGCTTGTCGAAAGTGGCGGAGGTCTTGTAAAGCCAGGAGGGTCCCTGAAGTT GAGTTGTGCTGCCTCTGGCTTCACCTTTTCACGGTATGCCATGTCCTGGGTTCGACAGA TACCTGAGAAGATTCTTGAATGGGTGGCCGCAATAGACAGTAGCGGTGGTGACACCTAT TACCTCGACACAGTCAAAGACCGCTTTACTATCTCACGCGATAACGCCAACAATACCCT GCACTTGCAGATGCGATCACTTCGTTCAGAAGACACTGCTCTTTACTATTGTGTACGCC AAGGGGGAGCATACTGGGGTCAGGGAACACTGGTTACCGTGTCTTCAGCTAGCACCAAA GGACCTAGTGTTTTTCCTCTTGCCCCTCAGGTTCAGCTTGTGCAATCAGGCGCTGAAGT CAAAAAACCAGGAGCAAGCGTGAAAGTAAGCTGTAAGGCTTCCGGTTACACTTTCACCG ATTACGAAATGCACTGGGTACGCCAGGCTCCTGGACAATGTCTGGAATGGATGGGCGCA | 514 |

| | CTTGATCCAAAAACAGGTGACACTGCTTACAGTCAAAAATTCAAAGGTCGTGTCACTCT TACAGCAGATAAGTCCACCAGTACCGCTTATATGGAGCTTAGCTCCCTGACTTCCGAGG ACACCGCCGTGTATTATTGTACAAGATTCTACTCATATACTTACTGGGGCCAAGGAACC CTGGTGACAGTGTCATCTGCTAGCACCAAAGGACCTAGTGTTTTTCCTCTTGCCCCTTC CTCAAAGTCTACCTCTGGGGGGACAGCCGCTCTGGGCTGCCTGGTCAAGGATTATTTCC CAGAGCCTGTCACTGTCAGTTGGAACTCTGGAGCCTTGACTTCTGGTGTTCATACATTT CCTGCTGTCCTTCAAAGCAGCGGCTTGTACTCATTGTCTTCTGTTGTGACAGTACCCTC AAGCAGCCTCGGCACTCAGACATACATCTGCAATGTCAACCACAAACCCTCAAATACAA AGGTAGATAAAAAAGTCGAACCAAAGTCTTGTGACAAAACTCACACGTGCCCACCGTGC CCAGCACCTGAACTCCTGGGGGGACCGTCAGTCTTCCTCTTCCCCCCCAAAACCCAAGGA CACCCTCATGATCTCCCGGACCCCTGAGGTCACATGCGTGGTGGTGGACGTGAGCCACG AAGACCCTGAGGTCAAGTTCAACTGGTACGTGGACGGCGTGGAGGTGCATAATGCCAAG ACAAAGCCGCGGGAGGAGCAGTACAACAGCACGTACCGTGTGGTCAGCGTCCTCACCGT CCTGCACCAGGACTGGCTGAATGGCAAGGAGTACAAGTGCAAGGTCTCCAACAAAGCCC TCCCAGCCCCCATCGAGAAAACCATCTCCAAAGCCAAAGGGCAGCCCCGAGAACCACAG GTGTACACCCTGCCCCCATGCCGGGAGGAGATGACCAAGAACCAGGTCAGCCTGTGGTG CCTGGTCAAAGGCTTCTATCCCAGCGACATCGCCGTGGAGTGGGAGAGCAATGGGCAGC CGGAGAACAACTACAAGACCACGCCTCCCGTGCTGGACTCCGACGGCTCCTTCTTCCTC TACAGCAAGCTCACCGTGGACAAGAGCAGGTGGCAGCAGGGGAACGTCTTCTCATGCTC CGTGATGCATGAGGCTCTGCACAACCACTACACGCAGAAGAGCCTCTCCCTGTCTCCGG GTAAA | |
| GPB04 LC | GACGTGGTTATGACTCAGACTCCTCTGACTCTCTCCGTCACTATTGGTCAGCCCGCTTC CATATCATGTAAATCATCACAATCTCTTCTTGATAGCGATGGCAAGACTTATTTGAACT GGTTGTTGCAACGCCCAGGTCAGAGCCCTAAGAGACTTATCTATTTGGTGAGCAAACTC GACAGCGGTGCACCCGATCGTTTTACCGGAAGCGGCAGCGGCACCGATTTCACACTGAA GATCAGTAGGGTCGAAGCTGAAGACCTGGGAATCTACTACTGCTGGCAAGGTACTCACT TTCCCCTGACTTTCGGCGCCGGTACTAAACTTGAGATCAAACGTACGGTGGCAGCTCCC AGCGTTTTTATCTTTCCCCCAGATGTTGTTATGACTCAGTCACCTCTCTCACTTCCTGT AACCCCTGGTGAACCAGCCTCTATAAGCTGCCGGTCAAGTCAAAGCCTGGTTCATAGCA | 515 |

| | | | |
|---|---|---|---|
| | | ACCGTAACACTTACCTTCACTGGTACTTGCAAAAACCTGGTCAGTCCCCACAACTCTTGATCTACAAAGTCTCCAATCGCTTCTCTGGAGTCCCTGACAGGTTTTCTGGTAGTGGATCAGGTACAGACTTCACTTTGAAAATCAGCCGCGTTGAGGCCGAGGACGTGGGAGTGTATTATTGCTCTCAGAATACCCATGTACCCCCAACCTTTGGCTGTGGGACTAAACTCGAGATTAAACGTACGGTGGCAGCTCCCAGCGTTTTTATCTTTCCCCCATCCGACGAGCAGCTCAAGAGTGGCACTGCCTCTGTAGTTTGTTTGCTGAATAACTTCTATCCACGTGAAGCAAAAGTACAGTGGAAGGTCGATAATGCCCTTCAGAGCGGTAACAGCCAAGAAAGTGTTACCGAGCAAGATTCCAAAGATTCCACTTACAGTCTGTCCAGCACATTGACACTGAGTAAGGCTGATTACGAAAAACACAAGGTGTACGCATGCGAGGTGACACACCAAGGTCTTTCATCTCCTGTAACTAAGAGCTTTAACCGGGGAGAATGTGGTGGTGGGGGGCAGCGGGGGCGGAGGTAGTGGAGGCGGCGGTAGTGAACCAAAGAGTAGTGACAAAACTCACACGTGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATGCCGGGAGGAGATGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | |
| GPB06 HC | | GAAGTGCAACTGGTCGAAACAGGCGGGGGACTGGTACAGCCCAAGGGATCTTTGAAACTTAGTTGTGCTGCTAGTGGGTTTACATTCAATGCCTCCGCAATGAACTGGGTAAGACAAGCTCCTGGCAAGGGCCTGGAATGGGTGGCCCGTATTCGCTCTAAAAGTAATAACTACGCTATTTATTACGCTGATTCTGTAAAGGATCGGTTTACAATAAGTCGGGACGACAGCCAATCCATGCTGTATCTCCAAATGAATAACCTGAAAACAGAGGATACTGCCATGTACTATTGTGTGCGGGACCCAGGCTATTACGGGAATCCCTGGTTCGCCTATTGGGGACAGGGCACTCTG | 516 |

| | | |
|---|---|---|
| | GTTACCGTATCATCAGCTAGCACCAAAGGACCTAGTGTTTTTCCTCTTGCCCCTCAGGT<br>TCAGCTTGTGCAATCAGGCGCTGAAGTCAAAAAACCAGGAGCAAGCGTGAAAGTAAGCT<br>GTAAGGCTTCCGGTTACACTTTCACCGATTACGAAATGCACTGGGTACGCCAGGCTCCT<br>GGACAATGTCTGGAATGGATGGGCGCACTTGATCCAAAAACAGGTGACACTGCTTACAG<br>TCAAAAATTCAAAGGTCGTGTCACTCTTACAGCAGATAAGTCCACCAGTACCGCTTATA<br>TGGAGCTTAGCTCCCTGACTTCCGAGGACACCGCCGTGTATTATTGTACAAGATTCTAC<br>TCATATACTTACTGGGGCCAAGGAACCCTGGTGACAGTGTCATCTGCTAGCACCAAAGG<br>ACCTAGTGTTTTTCCTCTTGCCCCTTCCTCAAAGTCTACCTCTGGGGGGACAGCCGCTC<br>TGGGCTGCCTGGTCAAGGATTATTTCCCAGAGCCTGTCACTGTCAGTTGGAACTCTGGA<br>GCCTTGACTTCTGGTGTTCATACATTTCCTGCTGTCCTTCAAAGCAGCGGCTTGTACTC<br>ATTGTCTTCTGTTGTGACAGTACCCTCAAGCAGCCTCGGCACTCAGACATACATCTGCA<br>ATGTCAACCACAAACCCTCAAATACAAAGGTAGATAAAAAAGTCGAACCAAAGTCTTGT<br>GACAAAACTCACACGTGCCCACCGTGCCCAGCACCTGAACTCCTGGGGGGACCGTCAGT<br>CTTCCTCTTCCCCCCAAAACCCAAGGACACCCTCATGATCTCCCGGACCCCTGAGGTCA<br>CATGCGTGGTGGTGGACGTGAGCCACGAAGACCCTGAGGTCAAGTTCAACTGGTACGTG<br>GACGGCGTGGAGGTGCATAATGCCAAGACAAAGCCGCGGGAGGAGCAGTACAACAGCAC<br>GTACCGTGTGGTCAGCGTCCTCACCGTCCTGCACCAGGACTGGCTGAATGGCAAGGAGT<br>ACAAGTGCAAGGTCTCCAACAAAGCCCTCCCAGCCCCCATCGAGAAAACCATCTCCAAA<br>GCCAAAGGGCAGCCCCGAGAACCACAGGTGTACACCCTGCCCCCATGCCGGGAGGAGAT<br>GACCAAGAACCAGGTCAGCCTGTGGTGCCTGGTCAAAGGCTTCTATCCCAGCGACATCG<br>CCGTGGAGTGGGAGAGCAATGGGCAGCCGGAGAACAACTACAAGACCACGCCTCCCGTG<br>CTGGACTCCGACGGCTCCTTCTTCCTCTACAGCAAGCTCACCGTGGACAAGAGCAGGTG<br>GCAGCAGGGGAACGTCTTCTCATGCTCCGTGATGCATGAGGCTCTGCACAACCACTACA<br>CGCAGAAGAGCCTCTCCCTGTCTCCGGGTAAA | |
| GPB06 LC | CAAATTGTCCTCACCCAATCACCAGCTATAATGTCTGCTTTTCCCGGCGAGAAAGTAAC<br>CATGACTTGTAGCGCCTCTAGTAGCGTGTCATATATGTATTGGTATCAACAAAAGAGCG<br>GTAGTTCACCTCGACTCCTTATCTACGACACAAGTAATCTCGCTAGTGGTGTCCCAGTC<br>CGTTTCTCCGGGAGCGGCAGCGGCACATCATACTCCCTGACCATCTCCAGAATGGAGGC<br>CGAGGACGCTGCCACATACTATTGTCAGCAGTGGAGCTCATATCCTTTGACATTCGGTG | 517 |

GAGGTACTAAACTCGAAATCAAGCGTACGGTGGCAGCTCCCAGCGTTTTTATCTTTCCC

CCAGATGTTGTTATGACTCAGTCACCTCTCTCACTTCCTGTAACCCCTGGTGAACCAGC

CTCTATAAGCTGCCGGTCAAGTCAAAGCCTGGTTCATAGCAACCGTAACACTTACCTTC

ACTGGTACTTGCAAAAACCTGGTCAGTCCCCACAACTCTTGATCTACAAAGTCTCCAAT

CGCTTCTCTGGAGTCCCTGACAGGTTTTCTGGTAGTGGATCAGGTACAGACTTCACTTT

GAAAATCAGCCGCGTTGAGGCCGAGGACGTGGGAGTGTATTATTGCTCTCAGAATACCC

ATGTACCCCCAACCTTTGGCTGTGGGACTAAACTCGAGATTAAACGTACGGTGGCAGCT

CCCAGCGTTTTTATCTTTCCCCCATCCGACGAGCAGCTCAAGAGTGGCACTGCCTCTGT

AGTTTGTTTGCTGAATAACTTCTATCCACGTGAAGCAAAAGTACAGTGGAAGGTCGATA

ATGCCCTTCAGAGCGGTAACAGCCAAGAAAGTGTTACCGAGCAAGATTCCAAAGATTCC

ACTTACAGTCTGTCCAGCACATTGACACTGAGTAAGGCTGATTACGAAAAACACAAGGT

GTACGCATGCGAGGTGACACACCAAGGTCTTTCATCTCCTGTAACTAAGAGCTTTAACC

GGGGAGAATGTGGTGGTGGGGGCAGCGGGGGCGGAGGTAGTGGAGGCGGCGGTAGTGAA

CCAAAGAGTAGTGACAAAACTCACACGTGCCCACCGTGCCCAGCACCTGAACTCCTGGG

GGGACCGTCAGTCTTCCTCTTCCCCCCAAAACCCAAGGACACCCTCATGATCTCCCGGA

CCCCTGAGGTCACATGCGTGGTGGTGGACGTGAGCCACGAAGACCCTGAGGTCAAGTTC

AACTGGTACGTGGACGGCGTGGAGGTGCATAATGCCAAGACAAAGCCGCGGGAGGAGCA

GTACAACAGCACGTACCGTGTGGTCAGCGTCCTCACCGTCCTGCACCAGGACTGGCTGA

ATGGCAAGGAGTACAAGTGCAAGGTCTCCAACAAAGCCCTCCCAGCCCCCATCGAGAAA

ACCATCTCCAAAGCCAAAGGGCAGCCCCGAGAACCACAGGTGTACACCCTGCCCCCATG

CCGGGAGGAGATGACCAAGAACCAGGTCAGCCTGTGGTGCCTGGTCAAAGGCTTCTATC

CCAGCGACATCGCCGTGGAGTGGGAGAGCAATGGGCAGCCGGAGAACAACTACAAGACC

ACGCCTCCCGTGCTGGACTCCGACGGCTCCTTCTTCCTCTACAGCAAGCTCACCGTGGA

CAAGAGCAGGTGGCAGCAGGGGAACGTCTTCTCATGCTCCGTGATGCATGAGGCTCTGC

ACAACCACTACACGCAGAAGAGCCTCTCCCTGTCTCCGGGTAAA

Table 48 below shows the polypeptide sequences of the heavy chain variable region (VH) and light chain variable region (VL) of the engineered antibody targeting GPC-3. Table 49 below shows the polynucleotide sequences encoding the polypeptides of the heavy chain variable region (VH) and light chain variable region (VL) of the engineered antibody targeting GPC-3.

[Table 48]

| Name | | Sequence | SEQ ID NO |
|---|---|---|---|
| GPM01 | VH | EVQLVESGGGLVKPGGSLKLSCAASGFTFSRYAMSWVRQIPEKCLEWVAAIDSSGGDTYYLDTVKDRFTISRDNANNTLHLQMRSLRSEDTALYYCVRQGGAYWGQGTLVTVSS | 81 |
| | VL | DVVMTQTPLTLSVTIGQPASISCKSSQSLLDSDGKTYLNWLLQRPGQSPKRLIYLVSKLDSGAPDRFTGSGSGTDFTLKISRVEAEDLGIYYCWQGTHFPLTFGCGTKLEIK | 82 |
| GPM02 | VH | QVQLVQSGAEVKKPGASVKVSCKASGYTFTDYEMHWVRQAPGQCLEWMGALDPKTGDTAYSQKFKGRVTLTADKSTSTAYMELSSLTSEDTAVYYCTRFYSYTWGQGTLVTVSS | 83 |
| | VL | DVVMTQSPLSLPVTPGEPASISCRSSQSLVIISNRNTYLIWYLQKPGQSPQLLIYKVSNRFSGVPDRFSGSGSGTDFTLKISRVEAEDVGVYYCSQNTHVPPTFGCGTKLEIK | 84 |
| GPM04 | VH | EVQLVETGGGLVQPKGSLKLSCAASGFTFNASAMNWVRQAPGKCLEWVARIRSKSNNYAIYYADSVKDRFTISRDDSQSMLYLQMNNLKTEDTAMYYCVRDPGYYGNPWFAYWGQGTLVTVSS | 85 |
| | VL | QIVLTQSPAIMSAFPGEKVTMTCSASSSVSYMYWYQQKSGSSPRLLIYDTSNLASGVPVRFSGSGSGTSYSLTISRMEAEDAATYYCQQWSSYPLTFGCGTKLEIK | 86 |
| GPB01 | VH1 | QVQLVQSGAEVKKPGASVKVSCKASGYTFTDYEMHWVRQAPGQGLEWMGALDPKTGDTAYSQKFKGRVTLTADKSTSTAYMELSSLTSEDTAVYYCTRFYSYTWGQGTLVTVSS | 87 |
| | VH2 | EVQLVESGGGLVKPGGSLKLSCAASGFTFSRYAMSWVRQIPEKCLEWVAAIDSSGGDTYYLDTVKDRFTISRDNANNTLHLQMRSLRSEDTALYYCVRQGGAYWGQGTLVTVSS | 81 |
| | VL1 | DVVMTQSPLSLPVTPGEPASISCRSSQSLVIISNRNTYLIWYLQKPGQSPQLLIYKVSNRFSGVPDRFSGSGSGTDFTLKISRVEAEDVGVYYCSQNTHVPPTFGQGTKLEIK | 88 |
| | VL2 | DVVMTQTPLTLSVTIGQPASISCKSSQSLLDSDGKTYLNWLLQRPGQSPKRLIYLVSKLDSGAPDRFTGSGSGTDFTLKISRVEAEDLGIYYCWQGTHFPLTFGCGTKLEIK | 82 |
| GPB03 | VH1 | QVQLVQSGAEVKKPGASVKVSCKASGYTFTDYEMHWVRQAPGQGLEWMGALDPKTGDTAYSQKFKGRVTLTADKSTSTAYMELSSLTSEDTAVYYCTRFYSYTWGQGTLVTVSS | 87 |
| | VH2 | EVQLVETGGGLVQPKGSLKLSCAASGFTFNASAMNWVRQAPGKCLEWVARIRSKSNNYAIYYADSVKDRFTISRDDSQSMLYLQMNNLKTEDTAMYYCVRDPGYYGNPWFAYWGQGTLVTVSS | 85 |
| | VL1 | DVVMTQSPLSLPVTPGEPASISCRSSQSLVIISNRNTYLIWYLQKPGQSPQLLIYKVSNRFSGVPDRFSGSGSGTDFTLKISRVEAEDVGVYYCSQNTHVPPTFGQGTKLEIK | 88 |
| | VL2 | QIVLTQSPAIMSAFPGEKVTMTCSASSSVSYMYWYQQKSGSSPRLLIYDTSNLASGVPVRFSGSGSGTSYSLTISRMEAEDAATYYCQQWSSYPLTFGCGTKLEIK | 86 |
| GPB04 | VH1 | EVQLVESGGGLVKPGGSLKLSCAASGFTFSRYAMSWVRQIPEKILEWVAAIDSSGGDTYYLDTVKDRFTISRDNANNTLHLQMRSLRSEDTALYYCVRQGGAYWGQGTLVTVSS | 89 |
| | VH2 | QVQLVQSGAEVKKPGASVKVSCKASGYTFTDYEMHWVRQAPGQCLEWMGALDPKTGDTAYSQKFKGRVTLTADKSTSTAYMELSSLTSEDTAVYYCTRFYSYTWGQGTLVTVSS | 83 |
| | VL1 | DVVMTQTPLTLSVTIGQPASISCKSSQSLLDSDGKTYLNWLLQRPGQSPKRLIYLVSKLDSGAPDRFTGSGSGTDFTLKISRVEAEDLGIYYCWQGTHFPLTFGAGTKLEIK | 90 |
| | VL2 | DVVMTQSPLSLPVTPGEPASISCRSSQSLVIISNRNTYLIWYLQKPGQSPQLLIYKVSNRFSGVPDRFSGSGSGTDFTLKISRVEAEDVGVYYCSQNTHVPPTFGCGTKLEIK | 84 |
| GPB06 | VH1 | EVQLVETGGGLVQPKGSLKLSCAASGFTFNASAMNWVRQAPGKGLEWVARIRSKSNNYAIYYADSVKDRFTISRDDSQSMLYLQMNNLKTEDTAMYYCVRDPGYYGNPWFAYWGQGTLVTVSS | 91 |
| | VH2 | QVQLVQSGAEVKKPGASVKVSCKASGYTFTDYEMHWVRQAPGQCLEWMGALDPKTGDTAYSQKFKGRVTLTADKSTSTAYMELSSLTSEDTAVYYCTRFYSYTWGQGTLVTVSS | 83 |
| | VL1 | QIVLTQSPAIMSAFPGEKVTMTCSASSSVSYMYWYQQKSGSSPRLLIYDTSNLASGVPVRFSGSGSGTSYSLTISRMEAEDAATYYCQQWSSYPLTFGGGTKLEIK | 92 |
| | VL2 | DVVMTQSPLSLPVTPGEPASISCRSSQSLVIISNRNTYLIWYLQKPGQSPQLLIYKVSNRFSGVPDRFSGSGSGTDFTLKISRVEAEDVGVYYCSQNTHVPPTFGCGTKLEIK | 84 |

[Table 49]

145

| Name | | Sequence | SEQ ID NO |
|---|---|---|---|
| GPM01 | VH | GAAGTTCAGCTTGTCGAAAGTGGCGGAGGTCTTGTAAAGCCAGGAGGGTCCCTGAACTTGAGTT GTGCTGCCTCTCGGCTTCACCTTTTCACGGTATGCCATGTCCTGGGTTCGACAGATACCTGAGAA GTGTCTTGAATGGGTGGCCGCAATAGACAGTAGCGGTGGTGACACCTATTACCTCGACACAGTC AAAGACCGGCTTTACTATCTCACGCGATAACGCCAACAATACCCTGCACTTGCAGATGCGATCAC TTCGTTCAGAAGACACTGCTCTTTACTATTGTGTACGCCAAGGGCGGAGCATACTGGGGTCAGGG AACACTGGTTACCGTGTCTTCA | 518 |
| | VL | GACGTGGTTATGACTCAGACTCCTCTGACTCTCTCCGTCACTATTGGTCAGCCCGCTTCCATAT CATGTAAATCATCACAATCTCTTCTTGATAGCGATGGCAAGACTTATTTGAACTGGTTGTTGCA ACGCCCAGGTCAGAGCCCTAAGAGACTTATCTATTTGGTGAGCAAACTCGACAGCGGTGCACCC GATCGTTTTACCGGAAGCGGCAGCGGCACCGATTTCACACTGAAGATCAGTAGGGTCGAAGCTG AAGACCTGGGAATCTACTACTGCTGGCAAGGTACTCACTTTCCCCTGACTTTCGGCTGCGGTAC TAAACTTGAGATCAAA | 519 |
| GPM02 | VH | CAGGTTCAGCTTGTGCAATCAGGCGCTGAAGTCAAAAAACCAGGAGCAAGCGTGAAAGTAAGCT GTAAGGCTTCCGGTTACACTTTCACCGATTACGAAATGCACTGGGTACGCCAGGCTCCTGGACA ATGTCTGGAATGGATGGGCGCACTTGATCCAAAAACAGGTGACACTGCTTACAGTCAAAAATTC AAAGGTCGTGTCACTCTTACAGCAGATAAGTCCACCAGTACCGCTTATATGGAGCTTAGCTCCC TGACTTCCGAGGACACCGCCGTGTATTATTGTACAAGATTCTACTCATATACTTACTGGGGCCA AGGAACCCTGGTGACAGTGTCATCT | 520 |
| | VL | GATGTTGTTATGACTCAGTCACCTCTCTCACTTCCTGTAACCCCTGGTGAACCAGCCTCTATAA GCTGCCCGTCAAGTCAAAGCCTGGTTCATAGCAACCGTAACACTTACCTTCACTGGTACTTGCA AAAACCTGGTCAGTCCCCACAACTCTTGATCTACAAAGTCTCCAATCGCTTCTCTGGAGTCCCT GACAGGTTTTCTGGTAGTGGATCAGGTACAGACTTCACTTTGAAAATCAGCCGCGTTGAGGCCG AGGACGTGGGAGTGTATTATTGCTCTCAGAATACCCATGTACCCCCAACCTTTGGCTGTGGGAC TAAACTCGAGATTAAA | 521 |
| GPM01 | VH | GAAGTGCAACTGGTCGAAACAGGCGGGGGACTGGTACAGCCCAAGGGATCTTTGAAACTTAGTT GTGCTGCTAGTGGGTTTACATTCAATGCCTCCGCAATGAACTGGGTAAGACAAGCTCCTGGCAA GTGCCTGGAATGGGTGGCCCGTATTCGCTCTAAAGTAATAACTACGCTATTTATTACGCTGAT TCTGTAAAGGATCGGTTTACAATAAGTCGGGACGACAGCCAATCCATGCTGTATCTCCAAATGA ATAACCTGAAAACAGAGGATACTGCCATGTACTATTGTGTGCGGGACCCAGGCTATTACGGGAA TCCCTGGTTCGCCTATTGGGGACAGGGCACTCTGGTTACCGTATCATCA | 522 |
| | VL | CAAATTGTCCTCACCCAATCACCAGCTATAATGTCTGCTTTTCCCGGCGAGAAAGTAACCATGA CTTGTAGCGCCTCTAGTAGCGTGTCATATATGTATTGGTATCAACAAAAGAGCGGTAGTTCACC TCGACTCCTTATCTACGACACAAGTAATCTCGCTAGTGGTGTCCCAGTCCGTTTCTCCGGGAGC GGCAGCGGCACATCATACTCCCTGACCATCTCCAGAATGGAGGCCGAGGACGCTGCCACATACT ATTGTCAGCAGTGGAGCTCATATCCTTTGACATTCGGTTCGGGTACTAAACTCGAAATCAAG | 523 |
| GPB01 | VH1 | CAGGTGCAACTCGTTCAAAGCGGGGCCGAGGTGAAGAAACCAGGGGCCTCAGTTAAGGTTGAGTT GCAAGGCAAGTGGATACACTTTCACCGGATTATGAAATGCATTGGGTGCGTCAGGCCCCAGGACA AGGACTGGAGTGGATGGGCGCTCTCGATCCTAAGACTGGTGATACTGCTTACTCTCAAAAGTTC AAAGGCCGAGTCACCTTGACCGCCGACAAGTCCACATCCACTGCATATATGGAATTGTCAAGTC TGACAAGCGAAGATACAGCCGTCTACTACTGCACCCGCTTTTATAGCTATACATATTGGGGACA GGGGACCTTGGTTACTGTGTCATCT | 434 |
| | VH2 | GAAGTTCAGCTTGTCGAAAGTGGCGGAGGTCTTGTAAAGCCAGGAGGGTCCCTGAACTTGAGTT GTGCTGCCTCTCGGCTTCACCTTTTCACGGTATGCCATGTCCTGGGTTCGACAGATACCTGAGAA GTGTCTTGAATGGGTGGCCGCAATAGACAGTAGCGGTGGTGACACCTATTACCTCGACACAGTC AAAGACCGGCTTTACTATCTCACGCGATAACGCCAACAATACCCTGCACTTGCAGATGCGATCAC | 518 |

| | | | |
|---|---|---|---|
| | | TTCGTTCAGAAGACACTGCTCTTTACTATTGTGTACGCCAAGGGGGAGCATACTGGGGTCAGGGAACACTGGTTACCGTGTCTTCA | |
| | VL1 | GACGTGGTAATGACACAATCACCTTTGTCTCTTCCCGTAACCCCCGGTGAACCAGCCAGCATCTCATGCAGAAGCAGTCAGTCACTGGTACATTCCAACCGTAATACTTATCTTCACTGGTACTTGCAGAAGCCTGGGCAGTCTCCTCAACTTTTGATATATAAAGTGAGCAATCGGTTTAGCGGTGTCCCAGACCGCTTTTCTGGATCTGGAAGTGGAACAGACTTTACTCTGAAAATAAGCAGAGTCGAGGCAGAAGATGTCGGAGTTTACTACTGTAGCCAGAACACACACGTACCCCCAACCTTTGGACAGGGCACAAAGTTGGAAATCAAG | 435 |
| | VL2 | GACGTGGTTATGACTCAGACTCCTCTGACTCTCTCCGTCACTATTGGTCAGCCCGCTTCCATATCATGTAAATCATCACAATCTCTTCTTGATAGCGATGGCAAGACTTATTTGAACTGGTTGTTGCAACGCCCAGGTCAGAGCCCTAAGAGACTTATCTATTTGGTGAGCAAACTCGACAGCGGTGCACCCGATCGTTTTACCGGAAGCGGCAGCGGCACCGATTTCACACTGAAGATCAGTAGGGTCGAAGCTGAAGACCTGGGAATCTACTACTGCTGGCAAGGTACTCACTTTCCCCTGACTTTCGGCTGCGGTACTAAACTTGAGATCAAA | 519 |
| GPB03 | VH1 | CAGGTGCAACTCGTTCAAAGCGGGGCCGAGGTGAAGAAACCAGGGGCCTCAGTTAAGGTGAGTTGCAAGGCAAGTGGATACACTTTCACCGATTATGAAATGCATTGGGTGCGTCAGGCCCCAGGACAAGGACTGGAGTGGATGGGCGCTCTCGATCCTAAGACTGGTGATACTGCTTACTCTCAAAAGTTCAAAGGCCGAGTCACCTTGACCGCCGACAAGTCCACATCCACTGCATATATGGAATTGTCAAGTCTGACAAGCGAAGATACAGCCGTCTACTACTGCACCCGCTTTTATAGCTATACATATTGGGGACAGGGGACCTTGGTTACTGTGTCATCT | 434 |
| | VH2 | GAAGTGCAACTGGTCGAAACAGGCGGGGGACTGGTACAGCCCAAGGGATCTTTGAAACTTAGTTGTGCTGCTAGTGGGTTTACATTCAATGCCTCCGCAATGAACTGGGTAAGACAAGCTCCTGGCAAGTGCCTGGAATGGGTGGCCCGTATTCGCTCTAAAAGTAATAACTACGCTATTTATTACGCTGATTCTGTAAAGGATCGGTTTACAATAAGTCGGGACGACAGCCAATCCATGCTGTATCTCCAAATGAATAACCTGAAAACAGAGGATACTGCCATGTACTATTGTGTGCGGGACCCCAGGCTATTACGGGAATCCCTGGTTCGCCTATTGGGGACAGGGCACTCTGGTTACCGTATCATCA | 522 |
| | VL1 | GACGTGGTAATGACACAATCACCTTTGTCTCTTCCCGTAACCCCCGGTGAACCAGCCAGCATCTCATGCAGAAGCAGTCAGTCACTGGTACATTCCAACCGTAATACTTATCTTCACTGGTACTTGCAGAAGCCTGGGCAGTCTCCTCAACTTTTGATATATAAAGTGAGCAATCGGTTTAGCGGTGTCCCAGACCGCTTTTCTGGATCTGGAAGTGGAACAGACTTTACTCTGAAAATAAGCAGAGTCGAGGCAGAAGATGTCGGAGTTTACTACTGTAGCCAGAACACACACGTACCCCCAACCTTTGGACAGGGCACAAAGTTGGAAATCAAG | 435 |
| | VL2 | CAAATTGTCCTCACCCAATCACCAGCTATAATGTCTGCTTTTCCCGGCGAGAAAGTAACCATGACTTGTAGCGCCTCTAGTAGCGTGTCATATATGTATTGGTATCAACAAAAGAGCGGTAGTTCACCTCGACTCCTTATCTACGACACAAGTAATCTCGCTAGTGGTGTCCCAGTCCGTTTCTCCGGGAGCGGCAGCGGCACATCATACTCCCTGACCATCTCCAGAATGGAGGCCGAGGACGCTGCCACATACTATTGTCAGCAGTGGAGCTCATATCCTTTGACATTCGGTTGCGGTACTAAACTCGAAATCAAG | 523 |
| GPB04 | VH1 | GAAGTTCAGCTTGTCGAAAGTGGCGGAGGTCTTGTAAAGCCAGGAGGGTCCCTGAAGTTGAGTTGTGCTGCCTCTGGCTTCACCTTTTCACGGTATGCCATGTCCTGGGTTCGACAGATACCTGAGAAGATTCTTGAATGGGTGGCCGCAATAGACAGTAGCGGTGGTGACACCTATTACCTCGACACAGTCAAAGACCGCTTTACTATCTCACGCGATAACGCCAACAATACCCTGCACTTGCAGATGCGATCACTTCGTTCAGAAGACACTGCTCTTTACTATTGTGTACGCCAAGGGGGAGCATACTGGGGTCAGGGAACACTGGTTACCGTGTCTTCA | 524 |
| | VH2 | CAGGTTCAGCTTGTGCAATCAGGCGCTGAAGTCAAAAAACCAGGAGCAAGCGTGAAAGTAAGCTGTAAGGCTTCCGGTTACACTTTCACCGATTACGAAATGCACTGGGTACGCCAGGCTCCTGGACAATGTCTGGAATGGATGGGCGCACTTGATCCAAAAACAGGTGACACTGCTTACAGTCAAAAATTCAAAGGTCGTGTCACTCTTACAGCAGATAAGTCCACCAGTACCGCTTATATGGAGCTTAGCTCCCTGACTTCCGAGGACACCGCCGTGTATTATTGTACAAGATTCTACTCATATACTTACTGGGGCCAAGGAACCCTGGTGACAGTGTCATCT | 520 |

| | | | |
|---|---|---|---|
| | VL1 | GACGTGGTTATGACTCAGACTCCTCTGACTCTCTCCGTCACTATTGGTCAGCCCGCTTCCATAT CATGTAAATCATCACAATCTCTTCTTGATAGCGATGGCAAGACTTATTTGAACTGGTTGTTGCA ACGCCCAGGTCAGAGCCCTAAGAGACTTATCTATTTGGTGAGCAAACTCGACAGCGGTGCACCC GATCGTTTTACCGGAAGCGGCAGCGGCACCGATTTCACACTGAAGATCAGTAGGGTCGAAGCTG AAGACCTGGGAATCTACTACTGCTGGCAAGGTACTCACTTTCCCCTGACTTTCGGCGCCGGTAC TAAACTTGAGATCAAA | 525 |
| | VL2 | GATGTTGTTATGACTCAGTCACCTCTCTCACTTCCTGTAACCCCTGGTGAACCAGCCTCTATAA GCTGCCGGTCAAGTCAAAGCCTGGTTCATAGCAACCGTAACACTTACCTTCACTGGTACTTGCA AAAACCTGGTCAGTCCCCACAACTCTTGATCTACAAAGTCTCCAATCGCTTCTCTGGAGTCCCT GACAGGTTTTCTGGTAGTGGATCAGGTACAGACTTCACTTTGAAAAATCAGCCGCGTTGAGGCCG AGGACGTGGGAGTGTATTATTGCTCTCAGAATACCCATGTACCCCCAACCTTTGGCTGTGGGAC TAAACTCGAGATTAAA | 521 |
| GPB06 | VH1 | GAAGTGCAACTGGTCGAAACAGGCGGGGGACTGGTACAGCCCAAGGGATCTTTGAAACTTAGTT GTGCTGCTAGTGGGTTTACATTCAATGCCTCCGCAATGAACTGGGTAAGACAAGCTCCTGGCAA GGGCCTGGAATGGGTGGCCCGTATTCGCTCTAAAAGTAATAACTACGCTATTTATTACGCTGAT TCTGTAAAGGATCGGTTTACAATAAGTCGGGACGACAGCCAATCCATGCTGTATCTCCAAATGA ATAACCTGAAAACAGAGGATACTGCCATGTACTATTGTGTGCGGGACCCAGGCTATTACGGGAA TCCCTGGTTCGCCTATTGGGGACAGGGCACTCTGGTTACCGTATCATCA | 526 |
| | VH2 | CAGGTTCAGCTTGTGCAATCAGGCGCTGAAGTCAAAAAACCAGGAGCAAGCGTGAAAGTAAGCT GTAAGGCTTCCGGTTACACTTTCACCGATTACGAAATGCACTGGGTACGCCAGGCTCCTGGACA ATGTCTGGAATGGATGGGCGCACTTGATCCAAAAACAGGTGACACTGCTTACAGTCAAAAATTC AAAGGTCGTGTCACTCTTACAGCAGATAAGTCCACCAGTACCGCTTATATGGAGCTTAGCTCCC TGACTTCCGAGGACACCGCCGTGTATTATTGTACAAGATTCTACTCATATACTTACTGGGGCCA AGGAACCCTGGTGACAGTGTCATCT | 520 |
| | VL1 | CAAATTGTCCTCACCCAATCACCAGCTATAATGTCTGCTTTTCCCGGCGAGAAAGTAACCATGA CTTGTAGCGCCTCTAGTAGCGTGTCATATATGTATTGGTATCAACAAAAGAGCGGTAGTTCACC TCGACTCCTTATCTACGACACAAGTAATCTCGCTAGTGGTGTCCCAGTCCGTTTCTCCGGGAGC GGCAGCGGCACATCATACTCCCTGACCATCTCCAGAATGGAGGCCGAGGACGCTGCCACATACT ATTGTCAGCAGTGGAGCTCATATCCTTTGACATTCGGTGGAGGTACTAAACTCGAAATCAAG | 527 |
| | VL2 | GATGTTGTTATGACTCAGTCACCTCTCTCACTTCCTGTAACCCCTGGTGAACCAGCCTCTATAA GCTGCCGGTCAAGTCAAAGCCTGGTTCATAGCAACCGTAACACTTACCTTCACTGGTACTTGCA AAAACCTGGTCAGTCCCCACAACTCTTGATCTACAAAGTCTCCAATCGCTTCTCTGGAGTCCCT GACAGGTTTTCTGGTAGTGGATCAGGTACAGACTTCACTTTGAAAAATCAGCCGCGTTGAGGCCG AGGACGTGGGAGTGTATTATTGCTCTCAGAATACCCATGTACCCCCAACCTTTGGCTGTGGGAC TAAACTCGAGATTAAA | 521 |

Table 50 below shows the CDR sequences of the heavy chain and light chain of the engineered antibody targeting GPC-3.

[Table 50]

| Name | CDR | Sequence | SEQ ID NO |
|---|---|---|---|
| GPM01 | CDR-H1 | RYAMS | 93 |
| | CDR-H2 | AIDSSGGDTYYLDTVKD | 94 |
| | CDR-H3 | QGGAY | 95 |
| | CDR-L1 | KSSQSLLDSDGKTYLN | 96 |
| | CDR-L2 | LVSKLDS | 97 |
| | CDR-L3 | WQGTHFPLT | 98 |
| GPM02 | CDR-H1 | DYEMH | 99 |
| | CDR-H2 | ALDPKTGDTAYSQKFKG | 100 |
| | CDR-H3 | FYSYTY | 101 |
| | CDR-L1 | RSSQSLVHSNRNTYLH | 102 |
| | CDR-L2 | KVSNRFS | 103 |
| | CDR-L3 | SQNTHVPPT | 104 |
| GPM04 | CDR-H1 | ASAMN | 105 |
| | CDR-H2 | RIRSKSNNYATYYADSVKD | 106 |
| | CDR-H3 | DPGYYGNPWFAY | 107 |
| | CDR-L1 | SASSSVSYMY | 108 |
| | CDR-L2 | DTSNLAS | 109 |
| | CDR-L3 | QQWSSYPLT | 110 |
| GPB01 | V1 CDR-H1 | DYEMH | 99 |
| | V1 CDR-H2 | ALDPKTGDTAYSQKFKG | 100 |
| | V1 CDR-H3 | FYSYTY | 101 |
| | V1 CDR-L1 | RSSQSLVHSNRNTYLH | 102 |
| | V1 CDR-L2 | KVSNRFS | 103 |
| | V1 CDR-L3 | SQNTHVPPT | 104 |
| | V2 CDR-H1 | RYAMS | 93 |
| | V2 CDR-H2 | AIDSSGGDTYYLDTVKD | 94 |
| | V2 CDR-H3 | QGGAY | 95 |
| | V2 CDR-L1 | KSSQSLLDSDGKTYLN | 96 |
| | V2 CDR-L2 | LVSKLDS | 97 |
| | V2 CDR-L3 | WQGTHFPLT | 98 |
| GPB03 | V1 CDR-H1 | DYEMH | 99 |
| | V1 CDR-H2 | ALDPKTGDTAYSQKFKG | 100 |
| | V1 CDR-H3 | FYSYTY | 101 |
| | V1 CDR-L1 | RSSQSLVHSNRNTYLH | 102 |
| | V1 CDR-L2 | KVSNRFS | 103 |
| | V1 CDR-L3 | SQNTHVPPT | 104 |
| | V2 CDR-H1 | ASAMN | 105 |
| | V2 CDR-H2 | RIRSKSNNYATYYADSVKD | 106 |
| | V2 CDR-H3 | DPGYYGNPWFAY | 107 |
| | V2 CDR-L1 | SASSSVSYMY | 108 |
| | V2 CDR-L2 | DTSNLAS | 109 |
| | V2 CDR-L3 | QQWSSYPLT | 110 |
| GPB04 | V1 CDR-H1 | RYAMS | 93 |
| | V1 CDR-H2 | AIDSSGGDTYYLDTVKD | 94 |

| | V1 CDR-H3 | QGGAY | 95 |
|---|---|---|---|
| | V1 CDR-L1 | KSSQSLLDSDGKTYLN | 96 |
| | V1 CDR-L2 | LVSKLDS | 97 |
| | V1 CDR-L3 | WQGTHFPLT | 98 |
| | V2 CDR-H1 | DYEMH | 99 |
| | V2 CDR-H2 | ALDPKTGDTAYSQKFKG | 100 |
| | V2 CDR-H3 | FYSYTY | 101 |
| | V2 CDR-L1 | RSSQSLVHSNRNTYLH | 102 |
| | V2 CDR-L2 | KVSNRFS | 103 |
| | V2 CDR-L3 | SQNTHVPPT | 104 |
| GPB06 | V1 CDR-H1 | ASAMN | 105 |
| | V1 CDR-H2 | RIRSKSNNYAIYYADSVKD | 106 |
| | V1 CDR-H3 | DPGYYGNPWFAY | 107 |
| | V1 CDR-L1 | SASSSVSYMY | 108 |
| | V1 CDR-L2 | DTSNLAS | 109 |
| | V1 CDR-L3 | QQWSSYPLT | 110 |
| | V2 CDR-H1 | DYEMH | 99 |
| | V2 CDR-H2 | ALDPKTGDTAYSQKFKG | 100 |
| | V2 CDR-H3 | FYSYTY | 101 |
| | V2 CDR-L1 | RSSQSLVHSNRNTYLH | 102 |
| | V2 CDR-L2 | KVSNRFS | 103 |
| | V2 CDR-L3 | SQNTHVPPT | 104 |

The GPC-3 protein binding constants of each of GPM01, GPM02, GPM04, GPB01, GPB03, GPB04, and GPB06 were determined using Octet Red96e. Specifically, the human GPC-3 recombinant protein (Sino Biologicals, 10088-H08H) was loaded onto the Anti-Penta-HIS (HIS1K) biosensor. Then each antibody was added in a binding reaction (300 seconds) and a dissociation reaction (1200 seconds) at various concentrations. Their affinities for GPC-3 were calculated, and the results are shown in Figure 42 and Table 51.

[Table 51]

| Antibodies | Antigen | Binding mode | $K_D$ (nM) | $K_a$ (1/Ms) | $K_d$ (1/s) |
|---|---|---|---|---|---|
| GPM01 | rhGPC3 | Monovalent | 2.7048 | 3.72E+05 | 1.01E-03 |
| GPM02 | rhGPC3 | Monovalent | 2.3799 | 1.92E+05 | 4.57E-04 |
| GPM04 | rhGPC3 | Monovalent | 38.3721 | 2.92E+04 | 1.12E-03 |
| GPB01 | rhGPC3 | Biparatopic | 0.5827 | 2.49E+05 | 1.45E-04 |
| GPB03 | rhGPC3 | Biparatopic | 0.1601 | 2.03E+05 | 3.25E-05 |
| GPB04 | rhGPC3 | Biparatopic | 0.2510 | 3.32E+05 | 8.33E-05 |
| GPB06 | rhGPC3 | Biparatopic | 0.3427 | 6.64E+04 | 2.28E-05 |

In addition, the Fc loads on the surface of GPC-3 expressing cells were quantified in the HepG2 liver cancer cell line.

Specifically, the HepG2 cells were treated with 100 nM of each of human IgG1, GPM02, GPB01, GPB03, and GC3, and then reacted at 4 °C for 30 minutes. Thereafter, the Fc loads were quantified using the Alexa 488 fluorescence-conjugated anti-human IgG Fcγ Fab antibody (Jackson ImmunoResearch, 109-547-008). GC33, which is a humanized antibody targeting GPC-3, was used as a positive control (Nakano et al., US7,919,086 B2).

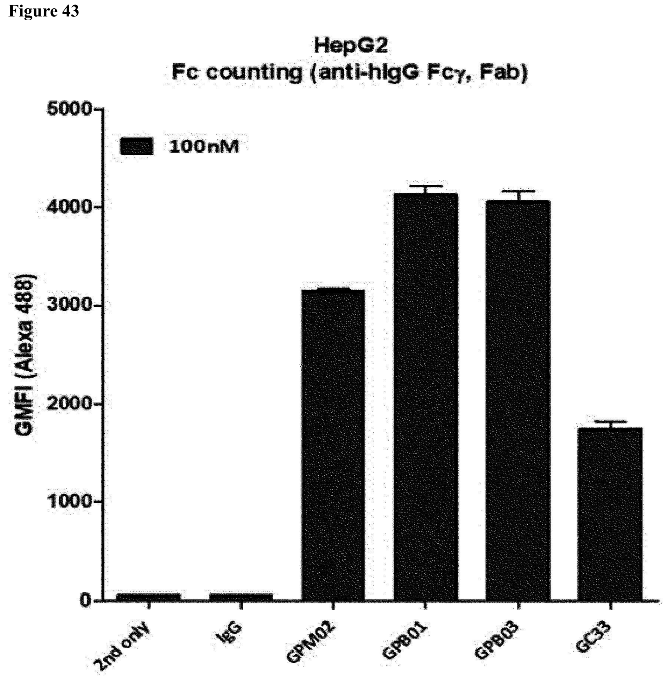

As shown in Figure 43, it was shown that higher Fc loads on the cell surface are induced by treatment of GPM02, GPB01, or GPB03 compared to GC33.

**Example 18. Design, preparation and analysis of antibody structure targeting EPH receptor A2**

The polypeptide sequences of the variant light chain and heavy chain of an antibody that specifically binds to the EPH receptor A2 (EphA2) protein are shown in Table 52.

EPB01 consists of the polypeptides of Fc-Hole (SEQ ID NO: 7), EPB01 HC (SEQ ID NO: 111), and EPB01 LC (SEQ ID NO: 112), and was prepared by co-transfection of expression vectors loaded with polynucleotides encoding the polypeptides into the EXPICHO-S™ cell line. The protein prepared as above was purified and analyzed in the same manner as in Example 1 above. In addition, EPB02, EPB03, EPB04, EPB05, EPB06, EPB07, EPB08, EPB09, EPB10, EPB11, and EPB12 were each prepared, purified, and then analyzed in the same manner as above.

The 12 antibodies have structures in which the variable regions that bind to two different epitopes of EphA2 are linked with a polypeptide linker (SEQ ID NO: 48, SEQ ID NO: 50), and bind to EphA2 in a biparatopic manner, and have two Fc domains (Figure 41b).

[Table 52]

| Name | Sequence | SEQ ID NO |
|---|---|---|
| EPB01 HC | EVQLLESGGGLVQPGGSLRLSCAASGFTFSRYQMMWVRQAPGKGLEWVSSISPSGGVTLYAD SVKGRFTISRDNSKNTLYLQMNSLRAEDTAVYYCTRELLGTVVVPVAWKMRGYFDYWGQLTL VTVSSASTKGPSVFPLAPEVQLLESGGGLVQPGGSLRLSCAASGFTFSHYMMAWVRQAPGKC LEWVSRIGPSGGPTHYADSVKGRFTISRDNSKNTLYLQMNSLRAEDTAVYYCAGYDSGYDYV AVAGPAEYFQHWGQGTLVTVSSASTKGPSVFPLAPSSKSTSGGTAALGCLVKDYFPEPVTVS WNSGALTSGVHTFPAVLQSSGLYSLSSVVTVPSSSLGTQTYICNVNHKPSNTKVDKKVEPKS CDKTHTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAKGQP REPQVYTLPPCREEMTKNQVSLWCLVKGFYPSDIAVEWESNGQPENNYKTTPPVLDSDGSFF LYSKLTVDKSRWQQGNVFSCSVMHEALHNHYTQKSLSLSPGK | 111 |
| EPB01 LC | DIQMTQSPGTLSVSPGERATLSCRASQSVSSNLAWYQQKPGQAPRLLIYGASTRATGIPARF SGSGSGTEFTLTISSMQSEDFAVYYCQQYNNWPPLTFGGGTKVEIKRTVAAPSVFIFPPDIQ MTQSPSSLSASVGDRVTITCRASQSISTWLAWYQQKPGKAPKLLIYKASNLHTGVPSRFSGS GSGTEFSLTISGLQPDDFATYYCQQYNSYSRTFGCGTKVEIKRTVAAPSVFIFPPSDEQLKS GTASVVCLLNNFYPREAKVQWKVDNALQSGNSQESVTEQDSKDSTYSLSSTLTLSKADYEKH KVYACEVTHQGLSSPVTKSFNRGECGGGGSGGGGSGGGGSEPKSSDKTHTCPPCPAPELLGG PSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPCREEMTKN QVSLWCLVKGFYPSDIAVEWESNGQPENNYKTTPPVLDSDGSFFLYSKLTVDKSRWQQGNVF SCSVMHEALHNHYTQKSLSLSPGK | 112 |
| EPB02 HC | EVQLVESGGGVVRPGGSLRLSCAASGFTVSDYSMNWVRQAPGKGLEWIGPIRNKANAYTTEY SASVKGRFTISRDDSKNTLYLQMNSLKTEDTAVYYCTTYPRYHAMDSWGQGTMVTVSSASTK GPSVFPLAPEVQLLESGGGLVQPGGSLRLSCAASGFTFSHYMMAWVRQAPGKCLEWVSRIGP SGGPTHYADSVKGRFTISRDNSKNTLYLQMNSLRAEDTAVYYCAGYDSGYDYVAVAGPAEYF QHWGQGTLVTVSSASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKVEPKSCDKTHTCPP CPAPELLGGPSVFLFPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISKAKGQPREPQVYTLP PCREEMTKNQVSLWCLVKGFYPSDIAVEWESNGQPENNYKTTPPVLDSDGSFFLYSKLTVDK SRWQQGNVFSCSVMHEALHNHYTQKSLSLSPGK | 113 |
| EPB02 LC | AIQLTQSPSSLSASVGDRVTITCRASQSISNNLIWYLQKPGQSPQLLIYYGFQSISGVPSRF SGSGSGTDFTLTISSLQPEDFATYYCQQANSWPLTFGGGTKLEIKRTVAAPSVFIFPPDIQM TQSPSSLSASVGDRVTITCRASQSISTWLAWYQQKPGKAPKLLIYKASNLHTGVPSRFSGSG SGTEFSLTISGLQPDDFATYYCQQYNSYSRTFGCGTKVEIKRTVAAPSVFIFPPSDEQLKSG TASVVCLLNNFYPREAKVQWKVDNALQSGNSQESVTEQDSKDSTYSLSSTLTLSKADYEKHK VYACEVTHQGLSSPVTKSFNRGECGGGGSGGGGSGGGGSEPKSSDKTHTCPPCPAPELLGGP SVFLFPPKPKDTLMISRTPEVTCVVVDVSHEDPEVKFNWYVDGVEVHNAKTKPREEQYNSTY RVVSVLTVLHQDWLNGKEYKCKVSNKALPAPIEKTISKAKGQPREPQVYTLPPCREEMTKNQ VSLWCLVKGFYPSDIAVEWESNGQPENNYKTTPPVLDSDGSFFLYSKLTVDKSRWQQGNVFS CSVMHEALHNHYTQKSLSLSPGK | 114 |
| EPB03 HC | QVQLLESGGGLVQPGGSLRLSCAASGFTFSSYTMSWVRQAPGQALEWMGTISSGGTYTYYPD SVKGRFTISRDNAKNSLYLQMNSLRAEDTAVYYCAREAIPTYWGRGTLVTVSSASTKGPSVF PLAPEVQLLESGGGLVQPGGSLRLSCAASGFTFSHYMMAWVRQAPGKCLEWVSRIGPSGGPT HYADSVKGRFTISRDNSKNTLYLQMNSLRAEDTAVYYCAGYDSGYDYVAVAGPAEYFQHWGQ GTLVTVSSASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKKVEPKSCDKTHTCPPCPAPE LLGGPSVFLFPPKPKDTLMISRTPEVTCVVVDVSHEDPEVKFNWYVDGVEVHNAKTKPREEQ | 115 |

152

| | | |
|---|---|---|
| | YNSTYRVVSVLTVLHQDWLNGKEYKCKVSNKALPAPIEKTISKAKGQPREPQVYTLPPCREE<br>MTKNQVSLWCLVKGFYPSDIAVEWESNGQPENNYKTTPPVLDSDGSFFLYSKLTVDKSRWQQ<br>GNVFSCSVMHEALHNHYTQKSLSLSPGK | |
| EPB03 LC | DIQLTQSPSSLSLSPGERVTLSCKASQDINNYLSWYQQKPDQAPKLLIKRANRLVDGVPDRF<br>SGSGSGTDFTLKISRVEAEDVGVYYCLKYDEFPYTFGQGTRLEIKRTVAAPSVFIFPPPDIQM<br>TQSPSSLSASVGDRVTITCRASQSISTWLAWYQQKPGKAPKLLIYKASNLHTGVPSRFSGSG<br>SGTEFSLTISGLQPDDFATYYCQQYNSYSRTFGCGTKVEIKRTVAAPSVFIFPPPSDEQLKSG<br>TASVVCLLNNFYPREAKVQWKVDNALQSGNSQESVTEQDSKDSTYSLSSTLTLSKADYEKHK<br>VYACEVTHQGLSSPVTKSFNRGECGGGGSGGGGSGGGGSEPKSSDKTHTCPPCPAPELLGGP<br>SVFLFPPKPKDTLMISRTPEVTCVVVDVSHEDPEVKFNWYVDGVEVHNAKTKPREEQYNSTY<br>RVVSVLTVLHQDWLNGKEYKCKVSNKALPAPIEKTISKAKGQPREPQVYTLPPCREEMTKNQ<br>VSLWCLVKGFYPSDIAVEWESNGQPENNYKTTPPVLDSDGSFFLYSKLTVDKSRWQQGNVFS<br>CSVMHEALHNHYTQKSLSLSPGK | 116 |
| EPB04 HC | EVQLLESGGGLVQPGGSLRLSCAASGFTFSHYMMAWVRQAPGKGLEWVSRIGPSGGPTHYAD<br>SVKGRFTISRDNSKNTLYLQMNSLRAEDTAVYYCAGYDSGYDYVAVAGPAEYFQHWGQGTLV<br>TVSSASTKGPSVFPLAPEVQLLESGGGLVQPGGSLRLSCAASGFTFSRYQMMWVRQAPGKCL<br>EWVSSISPSGGVTLYADSVKGRFTISRDNSKNTLYLQMNSLRAEDTAVYYCTRELLGTVVVP<br>VAWKMRGYFDYWGQLTLVTVSSASTKGPSVFPLAPSSKSTSGGTAALGCLVKDYFPEPVTVS<br>WNSGALTSGVHTFPAVLQSSGLYSLSSVVTVPSSSLGTQTYICNVNHKPSNTKVDKKVEPKS<br>CDKTHTCPPCPAPELLGGPSVFLFPPKPKDTLMISRTPEVTCVVVDVSHEDPEVKFNWYVDG<br>VEVHNAKTKPREEQYNSTYRVVSVLTVLHQDWLNGKEYKCKVSNKALPAPIEKTISKAKGQP<br>REPQVYTLPPCREEMTKNQVSLWCLVKGFYPSDIAVEWESNGQPENNYKTTPPVLDSDGSFF<br>LYSKLTVDKSRWQQGNVFSCSVMHEALHNHYTQKSLSLSPGK | 117 |
| EPB04 LC | DIQMTQSPSSLSASVGDRVTITCRASQSISTWLAWYQQKPGKAPKLLIYKASNLHTGVPSRF<br>SGSGSGTEFSLTISGLQPDDFATYYCQQYNSYSRTFGQGTKVEIKRTVAAPSVFIFPPPDIQM<br>TQSPGTLSVSPGERATLSCRASQSVSSNLAWYQQKPGQAPRLLIYGASTRATGIPARFSGSG<br>SGTEFTLTISSMQSEDFAVYYCQQYNNWPPLTFGCGTKVEIKRTVAAPSVFIFPPPSDEQLKS<br>GTASVVCLLNNFYPREAKVQWKVDNALQSGNSQESVTEQDSKDSTYSLSSTLTLSKADYEKH<br>KVYACEVTHQGLSSPVTKSFNRGECGGGGSGGGGSGGGGSEPKSSDKTHTCPPCPAPELLGG<br>PSVFLFPPKPKDTLMISRTPEVTCVVVDVSHEDPEVKFNWYVDGVEVHNAKTKPREEQYNST<br>YRVVSVLTVLHQDWLNGKEYKCKVSNKALPAPIEKTISKAKGQPREPQVYTLPPCREEMTKN<br>QVSLWCLVKGFYPSDIAVEWESNGQPENNYKTTPPVLDSDGSFFLYSKLTVDKSRWQQGNVF<br>SCSVMHEALHNHYTQKSLSLSPGK | 118 |
| EPB05 HC | EVQLLESGGGLVQPGGSLRLSCAASGFTFSHYMMAWVRQAPGKGLEWVSRIGPSGGPTHYAD<br>SVKGRFTISRDNSKNTLYLQMNSLRAEDTAVYYCAGYDSGYDYVAVAGPAEYFQHWGQGTLV<br>TVSSASTKGPSVFPLAPEVQLVESGGGVVRPGGSLRLSCAASGFTVSDYSMNWVRQAPGKCL<br>EWIGFIRNKANAYTTEYSASVKGRFTISRDDSKNTLYLQMNSLKTEDTAVYYCTTYPRYHAM<br>DSWGQGTMVTVSSASTKGPSVFPLAPSSKSTSGGTAALGCLVKDYFPEPVTVSWNSGALTSG<br>VHTFPAVLQSSGLYSLSSVVTVPSSSLGTQTYICNVNHKPSNTKVDKKVEPKSCDKTHTCPP<br>CPAPELLGGPSVFLFPPKPKDTLMISRTPEVTCVVVDVSHEDPEVKFNWYVDGVEVHNAKTK<br>PREEQYNSTYRVVSVLTVLHQDWLNGKEYKCKVSNKALPAPIEKTISKAKGQPREPQVYTLP<br>PCREEMTKNQVSLWCLVKGFYPSDIAVEWESNGQPENNYKTTPPVLDSDGSFFLYSKLTVDK<br>SRWQQGNVFSCSVMHEALHNHYTQKSLSLSPGK | 119 |
| EPB05 LC | DIQMTQSPSSLSASVGDRVTITCRASQSISTWLAWYQQKPGKAPKLLIYKASNLHTGVPSRF<br>SGSGSGTEFSLTISGLQPDDFATYYCQQYNSYSRTFGQGTKVEIKRTVAAPSVFIFPPAIQL<br>TQSPSSLSASVGDRVTITCRASQSISNNLHWYLQKPGQSPQLLIYYGFQSISGVPSRFSGSG<br>SGTDFTLTISSLQPEDFATYYCQQANSWPLTFGCGTKLEIKRTVAAPSVFIFPPPSDEQLKSG<br>TASVVCLLNNFYPREAKVQWKVDNALQSGNSQESVTEQDSKDSTYSLSSTLTLSKADYEKHK<br>VYACEVTHQGLSSPVTKSFNRGECGGGGSGGGGSGGGGSEPKSSDKTHTCPPCPAPELLGGP<br>SVFLFPPKPKDTLMISRTPEVTCVVVDVSHEDPEVKFNWYVDGVEVHNAKTKPREEQYNSTY | 120 |

| | | |
|---|---|---|
| | RVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | |
| EPB06 HC | EVQLLESGGGLVQPGGSLRLSCAASGFTFSHYMMAWVRQAPGKGLEWVSRIGPSGGPTHYADSVKGRFTISRDNSKNTLYLQMNSLRAEDTAVYYCAGYDSGYDYVAVAGPAEYFQHWGQGTLVTVSSASTKGPSVFPLAPQVQLLESGGGLVQPGGSLRLSCAASGFTFSSYTMSWVRQAPGQCLEWMGTISSGGTYTYYPDSVKGRFTISRDNAKNSLYLQMNSLRAEDTAVYYCAREAIFTYWGRGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 121 |
| EPB06 LC | DIQMTQSPSSLSASVGDRVTITCRASQSISTWLAWYQQKPGKAPKLLIYKASNLHTGVPSRFSGSGSGTEFSLTISGLQPDDFATYYCQQYNSYSRTFGQGTKVEIKRTVAAPSVFIFPPDIQLTQSPSSLSLSPGERVTLSCKASQDINNYLSWYQQKPDQAPKLLIKRANRLVDGVPDRFSGSGSGTDFTLKISRVEAEDVGVYYCLKYDEFPYTFGCGTRLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGECGGGGSGGGGSGGGGSSEPKSSDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 122 |
| EPB07 HC | EVQLVESGGGVVRPGGSLRLSCAASGFTVSDYSMNWVRQAPGKGLEWIGFIRNKANAYTTEYSASVKGRFTISRDDSKNTLYLQMNSLKTEDTAVYYCTTYPRYHAMDSWGQGTMVTVSSASTKGPSVFPLAPEVQLLESGGGLVQPGGSLRLSCAASGFTFSRYQMMWVRQAPGKCLEWVSSISPSGGVTLYADSVKGRFTISRDNSKNTLYLQMNSLRAEDTAVYYCTRELLGTVVVPVAWKMRGYFDYWGQLTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 123 |
| EPB07 LC | AIQLTQSPSSLSASVGDRVTITCRASQSISNNLHWYLQKPGQSPQLLIYYGFQSISGVPSRFSGSGSGTDFTLTISSLQPEDFATYYCQQANSWPLTFGGGTKLEIKRTVAAPSVFIFPPDIQMTQSPGTLSVSPGERATLSCRASQSVSSNLAWYQQKPGQAPRLLIYGASTRATGIPARFSGSGSGTEFTLTISSMQSEDFAVYYCQQYNNWPPLTFGCGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGECGGGGSGGGGSGGGGSEPKSSDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 124 |
| EPB08 HC | QVQLLESGGGLVQPGGSLRLSCAASGFTFSSYTMSWVRQAPGQALEWMGTISSGGTYTYYPDSVKGRFTISRDNAKNSLYLQMNSLRAEDTAVYYCAREAIFTYWGRGTLVTVSSASTKGPSVFPLAPEVQLLESGGGLVQPGGSLRLSCAASGFTFSRYQMMWVRQAPGKCLEWVSSISPSGGVTLYADSVKGRFTISRDNSKNTLYLQMNSLRAEDTAVYYCTRELLGTVVVPVAWKMRGYFDYWGQLTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREE | 125 |

| | | |
|---|---|---|
| EPB08 LC | QYNSTYRVVSVLTVLHQDWLNGKEYKCKVSNKALPAPIEKTISKAKGQPREPQVYTLPPCRE EMTKNQVSLWCLVKGFYPSDIAVEWESNGQPENNYKTTPPVLDSDGSFFLYSKLTVDKSRWQ QGNVFSCSVMHEALHNHYTQKSLSLSPGK DIQLTQSPSSLSLSPGERVTLSCKASQDINNYLSWYQQKPDQAPKLLIKRANRLVDGVPDRF SGSGSGTDFTLKISRVEAEDVGVYYCLKYDEFPYTFGQGTRLEIKRTVAAPSVFIFPPDIQM TQSPGTLSVSPGERATLSCRASQSVSSNLAWYQQKPGQAPRLLIYGASTRATGIPARFSGSG SGTEFTLTISSMQSEDFAVYYCQQYNNWPPLTFGCGTKVEIKRTVAAPSVFIFPPSDEQLKS GTASVVCLLNNFYPREAKVQWKVDNALQSGNSQESVTEQDSKDSTYSLSSTLTLSKADYEKH KVYACEVTHQGLSSPVTKSFNRGECGGGGSGGGGSGGGGSEPKSSDKTHTCPPCPAPELLGG PSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPCREEMTKN QVSLWCLVKGFYPSDIAVEWESNGQPENNYKTTPPVLDSDGSFFLYSKLTVDKSRWQQGNVF SCSVMHEALHNHYTQKSLSLSPGK | 126 |
| EPB09 HC | EVQLLESGGGLVQPGGSLRLSCAASGFTFSRYQMMWVRQAPGKGLEWVSSISPSGGVTLYAD SVKGRFTISRDNSKNTLYLQMNSLRAEDTAVYYCTRELLGTVVVPVAWKMRGYFDYWGQLTL VTVSSASTKGPSVFPLAPEVQLVESGGGVVRPGGSLRLSCAASGFTVSDYSMNWVRQAPGKC LEWIGFIRNKANAYTTEYSASVKGRFTISRDDSKNTLYLQMNSLKTEDTAVYYCTTYPRYHA MDSWGQGTMVTVSSASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDKKVEPKSCDKTHTCP PCPAPELLGGPSVFLFPPKPKDTLMISRTPEVTCVVVDVSHEDPEVKFNWYVDGVEVHNAKT KPREEQYNSTYRVVSVLTVLHQDWLNGKEYKCKVSNKALPAPIEKTISKAKGQPREPQVYTL PPCREEMTKNQVSLWCLVKGFYPSDIAVEWESNGQPENNYKTTPPVLDSDGSFFLYSKLTVD KSRWQQGNVFSCSVMHEALHNHYTQKSLSLSPGK | 127 |
| EPB09 LC | DIQMTQSPGTLSVSPGERATLSCRASQSVSSNLAWYQQKPGQAPRLLIYGASTRATGIPARF SGSGSGTEFTLTISSMQSEDFAVYYCQQYNNWPPLTFGGGTKVEIKRTVAAPSVFIFPPAIQ LTQSPSSLSASVGDRVTITCRASQSISNNLIHWYLQKPGQSPQLLIYYGFQSISGVPSRFSGS GSGTDFTLTISSLQPEDFATYYCQQANSWPLTFGCGTKLEIKRTVAAPSVFIFPPSDEQLKS GTASVVCLLNNFYPREAKVQWKVDNALQSGNSQESVTEQDSKDSTYSLSSTLTLSKADYEKH KVYACEVTHQGLSSPVTKSFNRGECGGGGSGGGGSGGGGSEPKSSDKTHTCPPCPAPELLGG PSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPCREEMTKN QVSLWCLVKGFYPSDIAVEWESNGQPENNYKTTPPVLDSDGSFFLYSKLTVDKSRWQQGNVF SCSVMHEALHNHYTQKSLSLSPGK | 128 |
| EPB10 HC | QVQLLESGGGLVQPGGSLRLSCAASGFTFSSYTMSWVRQAPGQALEWMGTISSGGTYTYYPD SVKGRFTISRDNAKNSLYLQMNSLRAEDTAVYYCAREAIFTYWGRGTLVTVSSASTKGPSVF PLAPEVQLVESGGGVVRPGGSLRLSCAASGFTVSDYSMNWVRQAPGKCLEWIGFIRNKANAY TTEYSASVKGRFTISRDDSKNTLYLQMNSLKTEDTAVYYCTTYPRYHAMDSWGQGTMVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTCPPCPAPELLGGPSVF LFPPKPKDTLMISRTPEVTCVVVDVSHEDPEVKFNWYVDGVEVHNAKTKPREEQYNSTYRVV SVLTVLHQDWLNGKEYKCKVSNKALPAPIEKTISKAKGQPREPQVYTLPPCREEMTKNQVSL WCLVKGFYPSDIAVEWESNGQPENNYKTTPPVLDSDGSFFLYSKLTVDKSRWQQGNVFSCSV MHEALHNHYTQKSLSLSPGK | 129 |
| EPB10 LC | DIQLTQSPSSLSLSPGERVTLSCKASQDINNYLSWYQQKPDQAPKLLIKRANRLVDGVPDRF SGSGSGTDFTLKISRVEAEDVGVYYCLKYDEFPYTFGQGTRLEIKRTVAAPSVFIFPPAIQL TQSPSSLSASVGDRVTITCRASQSISNNLHWYLQKPGQSPQLLIYYGFQSISGVPSRFSGSG SGTDFTLTISSLQPEDFATYYCQQANSWPLTFGCGTKLEIKRTVAAPSVFIFPPSDEQLKSG TASVVCLLNNFYPREAKVQWKVDNALQSGNSQESVTEQDSKDSTYSLSSTLTLSKADYEKHK VYACEVTHQGLSSPVTKSFNRGECGGGGSGGGGSGGGGSEPKSSDKTHTCPPCPAPELLGGP SVFLFPPKPKDTLMISRTPEVTCVVVDVSHEDPEVKFNWYVDGVEVHNAKTKPREEQYNSTY | 130 |

| | | |
|---|---|---|
| | RVVSVLTVLHQDWLNGKEYKCKVSNKALPAPIEKTISKAKGQPREPQVYTLPPCREEMTKNQ VSLWCLVKGFYPSDIAVEWESNGQPENNYKTTPPVLDSDGSFFLYSKLTVDKSRWQQGNVFS CSVMHEALHNHYTQKSLSLSPGK | |
| EPB11 HC | EVQLLESGGGLVQPGGSLRLSCAASGFTFSRYQMMWVRQAPGKGLEWVSSISPSGGVTLYAD SVKGRFTISRDNSKNTLYLQMNSLRAEDTAVYYCTRELLGTVVVPVAWKMRGYFDYWGQLTL VTVSSASTKGPSVFPLAPQVQLLESGGGLVQPGGSLRLSCAASGFTFSSYTMSWVRQAPGQC LEWMGTISSGGTYTYYPDSVKGRFTISRDNAKNSLYLQMNSLRAEDTAVYYCAREAIFTYWG RGTLVTVSSASTKGPSVFPLAPSSKSTSGGTAALGCLVKDYFPEPVTVSWNSGALTSGVHTF PAVLQSSGLYSLSSVVTVPSSSLGTQTYICNVNHKPSNTKVDKKVEPKSCDKTHTCPPCPAP ELLGGPSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREPQVYTLPPCRE EMTKNQVSLWCLVKGFYPSDIAVEWESNGQPENNYKTTPPVLDSDGSFFLYSKLTVDKSRWQ QGNVFSCSVMHEALHNHYTQKSLSLSPGK | 131 |
| EPB11 LC | DIQMTQSPGTLSVSPGERATLSCRASQSVSSNLAWYQQKPGQAPRLLIYGASTRATGIPARF SGSGSGTEFTLTISSMQSEDFAVYYCQQYNNWPPLTFGGGTKVEIKRTVAAPSVFIFPPPDIQ LTQSPSSLSLSPGERVTLSCKASQDINNYLSWYQQKPDQAPKLLIKRANRLVDGVPDRFSGS GSGTDFTLKISRVEAEDVGVYYCLKYDEFPYTFGCGTRLEIKRTVAAPSVFIFPPPSDEQLKS GTASVVCLLNNFYPREAKVQWKVDNALQSGNSQESVTEQDSKDSTYSLSSTLTLSKADYEKH KVYACEVTHQGLSSPVTKSFNRGECGGGGSGGGGSGGGGSEPKSSDKTHTCPPCPAPELLGG PSVFLFPPKPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVYTLPPCREEMTKN QVSLWCLVKGFYPSDIAVEWESNGQPENNYKTTPPVLDSDGSFFLYSKLTVDKSRWQQGNVF SCSVMHEALHNHYTQKSLSLSPGK | 132 |
| EPB12 HC | EVQLVESGGGVVRPGGSLRLSCAASGFTVSDYSMNWVRQAPGKGLEWIGFIRNKANAYTTEY SASVKGRFTISRDDSKNTLYLQMNSLKTEDTAVYYCTTYPRYHAMDSWGQGTMVTVSSASTK GPSVFPLAPQVQLLESGGGLVQPGGSLRLSCAASGFTFSSYTMSWVRQAPGQCLEWMGTISS GGTYTYYPDSVKGRFTISRDNAKNSLYLQMNSLRAEDTAVYYCAREAIFTYWGRGTLVTVSS ASTKGPSVFPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSCDKTHTCPPCPAPELLGGPSVF LFPPKPKDTLMISRTPEVTCVVVDVSHEDPEVKFNWYVDGVEVHNAKTKPREEQYNSTYRVV SVLTVLHQDWLNGKEYKCKVSNKALPAPIEKTISKAKGQPREPQVYTLPPCREEMTKNQVSL WCLVKGFYPSDIAVEWESNGQPENNYKTTPPVLDSDGSFFLYSKLTVDKSRWQQGNVFSCSV MHEALHNHYTQKSLSLSPGK | 133 |
| EPB12 LC | AIQLTQSPSSLSASVGDRVTITCRASQSISNNLHWYLQKPGQSPQLLIYYGFQSISGVPSRF SGSGSGTDFTLTISSLQPEDFATYYCQQANSWPLTFGGGTKLEIKRTVAAPSVFIFPPPDIQL TQSPSSLSLSPGERVTLSCKASQDINNYLSWYQQKPDQAPKLLIKRANRLVDGVPDRFSGSG SGTDFTLKISRVEAEDVGVYYCLKYDEFPYTFGCGTRLEIKRTVAAPSVFIFPPPSDEQLKSG TASVVCLLNNFYPREAKVQWKVDNALQSGNSQESVTEQDSKDSTYSLSSTLTLSKADYEKHK VYACEVTHQGLSSPVTKSFNRGECGGGGSGGGGSGGGGSEPKSSDKTHTCPPCPAPELLGGP SVFLFPPKPKDTLMISRTPEVTCVVVDVSHEDPEVKFNWYVDGVEVHNAKTKPREEQYNSTY RVVSVLTVLHQDWLNGKEYKCKVSNKALPAPIEKTISKAKGQPREPQVYTLPPCREEMTKNQ VSLWCLVKGFYPSDIAVEWESNGQPENNYKTTPPVLDSDGSFFLYSKLTVDKSRWQQGNVFS CSVMHEALHNHYTQKSLSLSPGK | 134 |

[Table 53]

EP 4 700 047 A1

| Name | Sequence | SEQ ID NO |
|------|----------|-----------|
| EPB01 HC | GAGGTGCAACTGCTTGAGTCAGGAGGAGGACTGGTTCAACCCGGCGGGAGCCTTCGACTCTC ATGTGCAGCCTCTGGGTTTACATTTAGTCGTTACCAAATGATGTGGGTACGCCAGGCCCCCG GAAAAGGTTTGGAGTGGGTGAGCAGCCATTTCTCCTTCAGGAGGAGTGACACTTTACGCAGAC AGTGTTAAGGGTCGGTTCACAATCAGTCGTGATAATTCCAAGAACACTCTCTATCTCCAAAT GAACTCCCTTCGAGCTGAGGACACTGCTGTTTATTACTGTACTCGGGAACTTCTCGGCACTG TGGTTGTTCCTGTGGCTTGGAAGATGCGTGGATACTTCGACTATTGGGGGCAACTCACTCTT GTTACCGTCTCAAGCGCTAGCACCAAAGGACCTAGTGTTTTTCCTCTTGCCCCTGAGGTTCA ACTCCTTGAATCCGGAGGAGGACTTGTCCAACCAGGCGGCAGCCTTAGGCTGTCCTGCGCTG CCTCAGGCTTTACATTCAGTCATTACATGATGGCCTGGGTTAGACAGGCACCCGGCAAATGT CTGGAATGGGTCAGCCGAATAGGACCATCAGGAGGTCCCACTCACTATGCCGATTCTGTAAA AGGGAGGTTTACAATTTCCAGAGACAATTCAAAGAATACCCTTTACCTCCAGATGAACTCAT TGAGAGCCGAGGACACAGCCGTATATTATTGCGCAGGTTATGATTCCGGTTACGATTACGTT GCAGTCGCAGGCCCAGCCGAATACTTCCAACATTGGGGTCAGGGAACCCTCGTGACCGTGTC CAGTGCTAGCACCAAAGGACCTAGTGTTTTTCCTCTTGCCCCTTCCTCAAAGTCTACCTCTG GGGGGACAGCCGCTCTCGGCTGCCTGGTCAAGGATTATTTCCCAGAGCCTGTCACTGTCAGT TGGAACTCTGGAGCCTTGACTTCTGGTGTTCATACATTTCCTGCTGTCCTTCAAAGCAGCGG CTTGTACTCATTGTCTTCTGTTGTGACAGTACCCTCAAGCAGCCTCGGCACTCAGACATACA TCTGCAATGTCAACCACAAACCCTCAAATACAAAGGTAGATAAAAAAGTCGAACCAAAGTCT TGTGACAAAACTCACACGTGCCCACCGTGCCCAGCACCTGAACTCCTGGGGGGACCGTCAGT CTTCCTCTTCCCCCCAAAACCCAAGGACACCCTCATGATCTCCCGGACCCCTGAGGTCACAT GCGTGGTGGTGGACGTGAGCCACGAAGACCCTGAGGTCAAGTTCAACTGGTACGTGGACGGC GTGGAGGTGCATAATGCCAAGACAAAGCCGCGGGAGGAGCAGTACAACAGCACGTACCGTGT GGTCAGCGTCCTCACCGTCCTGCACCAGGACTGGCTGAATGGCAAGGAGTACAAGTGCAAGG TCTCCAACAAAGCCCTCCCAGCCCCCATCGAGAAAACCATCTCCAAAGCCAAAGGGCAGCCC CGAGAACCACAGGTGTACACCCTGCCCCCATGCCCGGGAGGAGATGACCAAGAACCAGGTCAG CCTGTGGTGCCTGGTCAAAGGCTTCTATCCCAGCGACATCGCCGTGGAGTGGGAGAGCAATG GGCAGCCGGAGAACAACTACAAGACCACGCCTCCCGTGCTGGACTCCGACGGCTCCTTCTTC CTCTACAGCAAGCTCACCGTGGACAAGAGCAGGTGGCAGCAGGGGAACGTCTTCTCATGCTC CGTGATGCATGAGGCTCTGCACAACCACTACACGCAGAAGAGCCTCTCCCTGTCTCCGGGTA AA | 528 |
| EPB01 LC | GACATACAGATGACTCAGAGCCCTGGTACACTGAGTGTATCTCCTGGGGAACGCGCCACTCT GTCTTGCCGAGCCTCTCAAAGCGTTAGTTCCAACCTGGCTTGGTATCAGCAAAAACCCGGTC AAGCACCTCGCTTGCTTATCTACGGGGCCTCCACACGGGCAACCGGCATTCCTGCCACGCTTT AGTGGCTCTGGTAGCGGGACTGAATTCACTTTGACTATATCTTCCATGCAGAGTGAGGATTT CGCCGTTTATTATTGTCAACAGTACAATAACTGGCCTCCACTCACTTTTGGTGGTGGCACCA AAGTGGAGATAAAGCGTACGGTGGCAGCTCCCAGCCGTTTTTATCTTTCCCCCAGACATACAG ATGACACAAAGTCCAAGTAGCCTTTCTGCATCCGTTGGAGACCGCCGTAACCATAACTTGTCG AGCATCACAAAGCATAAGCACTTGGCTTGCTTGGTATCAGCAGAAGCCAGGCAAAGCACCCA AGCTGCTGATTTATAAAGCCTCTAATCTCCATACAGGAGTTCCCAGCCGTTTCTCAGGCAGC GGGTCTGGAACCGAATTCTCTCTGACCATCTCTGGCTTGCAACCTGATGACTTTGCAACATA TTACTGTCAACAATATAATTCATATAGTCGTACTTTCGGGTGTGGCACTAAGGTGGAAATCA AACGTACGGTGGCAGCTCCCAGCCGTTTTTATCTTTCCCCCATCCGACGAGCAGCTCAAGAGT GGCACTGCCTCTGTAGTTTGTTTGCTGAATAACTTCTATCCACGTGAAGCAAAAGTACAGTG GAAGGTCGATAATGCCCTTCAGAGCGGTAACAGCCAAGAAAGTGTTACCGAGCAAGATTCCA AAGATTCCACTTACAGTCTGTCCAGCACATTGACACTGAGTAAGGCTGATTACGAAAAACAC AAGGTGTACGCATGCGAGGTGACACACCAAGGTCTTTCATCTCCTGTAACTAAGAGCTTTAA CCGGGGGAGAATGTGGTGGTGGGGGCAGCGGGGGCGGAGGTAGTGGAGGCGGCGGTAGTGAAC | 529 |

157

| | | | |
|---|---|---|---|
| | | CAAAGAGTAGTGACAAAACTCACACGTGCCCACCGTGCCCAGCACCTGAACTCCTGGGGGGA<br>CCGTCAGTCTTCCTCTTCCCCCCAAAACCCAAGGACACCCTCATGATCTCCCGGACCCCTGA<br>GGTCACATGCGTGGTGGTGGACGTGAGCCACGAAGACCCTGAGGTCAAGTTCAACTGGTACG<br>TGGACGGCGTGGAGGTGCATAATGCCAAGACAAAGCCGCGGGAGGAGCAGTACAACAGCACG<br>TACCGTGTGGTCAGCGTCCTCACCGTCCTGCACCAGGACTGGCTGAATGGCAAGGAGTACAA<br>GTGCAAGGTCTCCAACAAAGCCCTCCCAGCCCCCATCGAGAAAACCATCTCCAAAGCCAAAG<br>GGCAGCCCCGAGAACCACAGGTGTACACCCTGCCCCCATGCCGGGAGGAGATGACCAAGAAC<br>CAGGTCAGCCTGTGGTGCCTGGTCAAAGGCTTCTATCCCAGCGACATCGCCGTGGAGTGGGA<br>GAGCAATGGGCAGCCGGAGAACAACTACAAGACCACGCCTCCCGTGCTGGACTCCGACGGCT<br>CCTTCTTCCTCTACAGCAAGCTCACCGTGGACAAGAGCAGGTGGCAGCAGGGGAACGTCTTC<br>TCATGCTCCGTGATGCATGAGGCTCTGCACAACCACTACACGCAGAAGAGCCTCTCCCTGTC<br>TCCGGGTAAA | |
| EPB02 HC | | GAGGTCCAGCTGGTTGAGTCTGGAGGTGGTGTAGTCAGGCCAGGAGGTTCCCTGCGTCTGTC<br>TTGCGCTGCCTCTGGGTTCACTGTGTCTGACTACAGTATGAACTGGGTTAGACAGGCTCCAG<br>GGAAAGGTTTGGAATGGATAGGGTTTATTCGCAATAAAGCCAATGCCTATACCACCGAATAC<br>AGCGCAAGCGTTAAAGGCAGGTTCACCATCAGTAGGGACGACTCTAAGAATACCCTCTACCT<br>GCAGATGAATAGCTTGAAGACAGAGGATACCGCTGTTTATTACTGCACTACCTACCCCAGGT<br>ATCATGCTATGGACAGTTGGGGTCAAGGAACTATGGTCACCGTCTCCTCTGCTAGCACCAAA<br>GGACCTAGTGTTTTTCCTCTTGCCCCTGAGGTTCAACTCCTTGAATCCGGAGGAGGACTTGT<br>CCAACCAGGCGGCAGCCTTAGGCTGTCCTGCGCTGCCTCAGGCTTTACATTCAGTCATTACA<br>TGATGGCCTGGGTTAGACAGGCACCCGGCAAATGTCTGGAATGGGTCAGCCGAATAGGACCA<br>TCAGGAGGTCCCACTCACTATGCCGATTCTGTAAAAGGGGAGGTTTACAATTTCCAGAGACAA<br>TTCAAAGAATACCCTTTACCTCCAGATGAACTCATTGAGAGCCGAGGACACAGCCGTATATT<br>ATTGCGCAGGTTATGATTCCGGTTACGATTACGTTGCAGTCGCAGGCCCAGCCGAATACTTC<br>CAACATTGGGGTCAGGGAACCCTCGTGACCGTGTCCAGTGCTAGCACCAAAGGACCTAGTGT<br>TTTTCCTCTTGCCCCTTCCTCAAAGTCTACCTCTGGGGGGACAGCCGCTCTGGGCTGCCTGG<br>TCAAGGATTATTTCCCAGAGCCTGTCACTGTCAGTTGGAACTCTGGAGCCTTGACTTCTGGT<br>GTTCATACATTTCCTGCTGTCCTTCAAAGCAGCGGCTTGTACTCATTGTCTTCTGTTGTGAC<br>AGTACCCTCAAGCAGCCTCGGCACTCAGACATACATCTGCAATGTCAACCACAAACCCTCAA<br>ATACAAAGGTAGATAAAAAAGTCGAACCAAAGTCTTGTGACAAAAACTCACACGTGCCCACCG<br>TGCCCAGCACCTGAACTCCTGGGGGGACCGTCAGTCTTCCTCTTCCCCCCAAAACCCAAGGA<br>CACCCTCATGATCTCCCGGACCCCTGAGGTCACATGCGTGGTGGTGGACGTGAGCCACGAAG<br>ACCCTGAGGTCAAGTTCAACTGGTACGTGGACGGCGTGGAGGTGCATAATGCCAAGACAAAG<br>CCGCGGGAGGAGCAGTACAACAGCACGTACCGTGTGGTCAGCGTCCTCACCGTCCTGCACCA<br>GGACTGGCTGAATGGCAAGGAGTACAAGTGCAAGGTCTCCAACAAAGCCCTCCCAGCCCCCA<br>TCGAGAAAACCATCTCCAAAGCCAAAGGGCAGCCCCGAGAACCACAGGTGTACACCCTGCCC<br>CCATGCCGGGAGGAGATGACCAAGAACCAGGTCAGCCTGTGGTGCCTGGTCAAAGGCTTCTA<br>TCCCAGCGACATCGCCGTGGAGTGGGAGAGCAATGGGCAGCCGGAGAACAACTACAAGACCA<br>CGCCTCCCGTGCTGGACTCCGACGGCTCCTTCTTCCTCTACAGCAAGCTCACCGTGGACAAG<br>AGCAGGTGGCAGCAGGGGAACGTCTTCTCATGCTCCGTGATGCATGAGGCTCTGCACAACCA<br>CTACACGCAGAAGAGCCTCTCCCTGTCTCCGGGTAAA | 530 |
| EPB02 LC | | GCCATACAGCTGACTCAATCACCTTCTTCTTTGAGCGCCTCTGTCGGTGATCGCGTTACAAT<br>TACATGCCGAGCTTCTCAGTCTATCTCTAATAACCTCCACTGGTATCTCCAGAAACCTGGAC<br>AGTCACCACAGCTTCTGATATATTACGGCTTTCAGTCTATAAGTGGAGTCCCAAGTAGATTT<br>TCCGGTAGTGGATCTGGCACCGATTTTACTCTTACTATCTCTAGCCTCCAACCTGAAGATTT<br>CGCAACCTATTACTGCCAGCAGGCCAATTCCTGGCCCTTGACCTTCGGAGGTGGGACTAAGC<br>TGGAAATCAAACGTACGGTGGCAGCTCCCAGCGTTTTTATCTTTCCCCCAGACATACAGATG<br>ACACAAAGTCCAAGTAGCCTTTCTGCATCCGTTGGAGACCGCGTAACCATAACTTGTCGAGC<br>ATCACAAAGCATAAGCACTTGGCTTGCTTGGTATCAGCAGAAGCCAGGCAAAGCACCCAAGC<br>TGCTGATTTATAAAGCCTCTAATCTCCATACAGGAGTTCCCAGCCGTTTCTCAGGCAGCGGG | 531 |

| | | |
|---|---|---|
| | TCTGGAACCGAATTCTCTCTGACCATCTCTGGCTTGCAACCTGATGACTTTGCAACATATTA CTGTCAACAATATAATTCATATAGTCGTACTTTCGGGTGTGGCACTAAGGTGGAAATCAAAC GTACGGTGGCAGCTCCCAGCGTTTTTATCTTTCCCCCATCCGACGAGCAGCTCAAGAGTGGC ACTGCCTCTGTAGTTTGTTTGCTGAATAACTTCTATCCACGTGAAGCAAAAGTACAGTGGAA GGTCGATAATGCCCTTCAGAGCGGTAACAGCCAAGAAAGTGTTACCGAGCAAGATTCCAAAG ATTCCACTTACAGTCTGTCCAGCACATTGACACTGAGTAAGGCTGATTACGAAAAACACAAG GTGTACGCATGCGAGGTGACACACCAAGGTCTTTCATCTCCTGTAACTAAGAGCTTTAACCG GGGAGAATGTGGTGGTGGGGGCAGCGGGGGCGGAGGTAGTGGAGGCGGCGGTAGTGAACCAA AGAGTAGTGACAAAACTCACACGTGCCCACCGTGCCCAGCACCTGAACTCCTGGGGGGACCG TCAGTCTTCCTCTTCCCCCCAAAACCCAAGGACACCCTCATGATCTCCCGGACCCCTGAGGT CACATGCGTGGTGGTGGACGTGAGCCACGAAGACCCTGAGGTCAAGTTCAACTGGTACGTGG ACGGCGTGGAGGTGCATAATGCCAAGACAAAGCCGCGGGAGGAGCAGTACAACAGCACGTAC CGTGTGGTCAGCGTCCTCACCGTCCTGCACCAGGACTGGCTGAATGGCAAGGAGTACAAGTG CAAGGTCTCCAACAAAGCCCTCCCAGCCCCCATCGAGAAAACCATCTCCAAAGCCAAAGGGC AGCCCCGAGAACCACAGGTGTACACCCTGCCCCCATGCCGGGAGGAGATGACCAAGAACCAG GTCAGCCTGTGGTGCCTGGTCAAAGGCTTCTATCCCAGCGACATCGCCGTGGAGTGGGAGAG CAATGGGCAGCCGGAGAACAACTACAAGACCACGCCTCCCGTGCTGGACTCCGACGGCTCCT TCTTCCTCTACAGCAAGCTCACCGTGGACAAGAGCAGGTGGCAGCAGGGGAACGTCTTCTCA TGCTCCGTGATGCATGAGGCTCTGCACAACCACTACACGCAGAAGAGCCTCTCCCTGTCTCC GGGTAAA | |
| | CAAGTGCAACTCCTTGAAAGTGGAGGTGGCCTTGTCCAGCCAGGCGGTTCACTGCGTTTGAG CTGTGCCGCATCCGGCTTTACCTTTTCCAGTTACACAATGAGTTGGGTCCGTCAAGCACCCG GGCAAGCTTTGGAATGGATGGGAACTATTTCATCTGGTGGGACTTACACTTACTATCCCGAT AGCGTCAAGGGACGTTTTACCATAAGCCGGGACAACGCTAAAAATTCACTCTATCTTCAGAT GAACAGTCTTAGGGCTGAGGATACCGCTGTTTATTATTGCGCCCGAGAAGCCATTTTCACCT ACTGGGGGCGCGGTACACTTGTCACCGTTAGCAGTGCTAGCACCAAAGGACCTAGTGTTTTT CCTCTTGCCCCTGAGGTTCAACTCCTTGAATCCGGAGGAGGACTTGTCCAACCAGGCGGCAG CCTTAGGCTGTCCTGCGCTGCCTCAGGCTTTACATTCAGTCATTACATGATGGCCTGGGTTA GACAGGCACCCGGCAAATGTCTGGAATGGGTCAGCCGAATAGGACCATCAGGAGGTCCCACT CACTATGCCGATTCTGTAAAAGGGGAGGTTTACAATTTCCAGAGACAATTCAAAGAATACCCT TTACCTCCAGATGAACTCATTGAGAGCCGAGGACACAGCCGTATATTATTGCGCAGGTTATG ATTCCGGTTACGATTACGTTGCAGTCGCAGGCCCAGCCGAATACTTCCAACATTGGGGTCAG GGAACCCTCGTGACCGTGTCCAGTGCTAGCACCAAAGGACCTAGTGTTTTTCCTCTTGCCCC TTCCTCAAAGTCTACCTCTGGGGGGACAGCCGCTCTGGGCTGCCTGGTCAAGGATTATTTCC | |
| EPB03 HC | CAGAGCCTGTCACTGTCAGTTGGAACTCTGGAGCCTTGACTTCTGGTGTTCATACATTTCCT GCTGTCCTTCAAAGCAGCGGCTTGTACTCATTGTCTTCTGTTGTGACAGTACCCTCAAGCAG CCTCGGCACTCAGACATACATCTGCAATGTCAACCACAAACCCTCAAATACAAAGGTAGATA AAAAAGTCGAACCAAAGTCTTGTGACAAAACTCACACGTGCCCACCGTGCCCAGCACCTGAA CTCCTGGGGGGACCGTCAGTCTTCCTCTTCCCCCCAAAACCCAAGGACACCCTCATGATCTC CCGGACCCCTGAGGTCACATGCGTGGTGGTGGACGTGAGCCACGAAGACCCTGAGGTCAAGT TCAACTGGTACGTGGACGGCGTGGAGGTGCATAATGCCAAGACAAAGCCGCGGGAGGAGCAG TACAACAGCACGTACCGTGTGGTCAGCGTCCTCACCGTCCTGCACCAGGACTGGCTGAATGG CAAGGAGTACAAGTGCAAGGTCTCCAACAAAGCCCTCCCAGCCCCCATCGAGAAAACCATCT CCAAAGCCAAAGGGCAGCCCCGAGAACCACAGGTGTACACCCTGCCCCCATGCCGGGAGGAG ATGACCAAGAACCAGGTCAGCCTGTGGTGCCTGGTCAAAGGCTTCTATCCCAGCGACATCGC CGTGGAGTGGGAGAGCAATGGGCAGCCGGAGAACAACTACAAGACCACGCCTCCCGTGCTGG ACTCCGACGGCTCCTTCTTCCTCTACAGCAAGCTCACCGTGGACAAGAGCAGGTGGCAGCAG GGGAACGTCTTCTCATGCTCCGTGATGCATGAGGCTCTGCACAACCACTACACGCAGAAGAG CCTCTCCCTGTCTCCGGGTAAA | 532 |
| EPB03 LC | GACATACAACTCACACAAAGTCCAAGCTCTCTTTCCCTTAGTCCAGGAGAACGCGTAACTCT | 533 |

| | | |
|---|---|---|
| | CTCATGTAAAGCCTCCCAGGATATCAATAACTATCTCTCTTGGTACCAACAAAAGCCTGACC<br>AAGCTCCTAAGCTGCTTATCAAACGCGCCAATCGGTTGGTAGACGGAGTTCCCGACAGGTTC<br>AGTGGGTCAGGCTCAGGTACAGATTTTACCTTGAAGATATCACGCGTGGAAGCCGAAGACGT<br>AGGGGTATATTATTGTCTGAAATATGACGAGTTCCCCTATACTTTTGGGCAAGGAACTCGCC<br>TGGAAATAAAACGTACGGTGGCAGCTCCCAGCGTTTTTATCTTTCCCCCAGACATACAGATG<br>ACACAAAGTCCAAGTAGCCTTTCTGCATCCGTTGGAGACCGCGTAACCATAACTTGTCGAGC<br>ATCACAAAGCATAAGCACTTGGCTTGCTTGGTATCAGCAGAAGCCAGGCAAAGCACCCAAGC<br>TGCTGATTTATAAAGCCTCTAATCTCCATACAGGAGTTCCCAGCCGTTTCTCAGGCAGCGGG<br>TCTGGAACCGAATTCTCTCTGACCATCTCTGGCTTGCAACCTGATGACTTTGCAACATATTA<br>CTGTCAACAATATAATTCATATAGTCGTACTTTCGGGTGTGGCACTAAGGTGGAAATCAAAC<br>GTACGGTGGCAGCTCCCAGCGTTTTTATCTTTCCCCCATCCGACGAGCAGCTCAAGAGTGGC<br>ACTGCCTCTGTAGTTTGTTTGCTGAATAACTTCTATCCACGTGAAGCAAAAGTACAGTGGAA<br>GGTCGATAATGCCCTTCAGAGCGGTAACAGCCAAGAAAGTGTTACCGAGCAAGATTCCAAAG<br>ATTCCACTTACAGTCTGTCCAGCACATTGACACTGAGTAAGGCTGATTACGAAAAACACAAG<br>GTGTACGCATGCGAGGTGACACACCAAGGTCTTTCATCTCCTGTAACTAAGAGCTTTAACCG<br>GGGAGAATGTGGTGGTGGGGGGCAGCGGGGGCGGAGGTAGTGGAGGCGGCGGTAGTGAACCAA<br>AGAGTAGTGACAAAACTCACACGTGCCCACCGTGCCCAGCACCTGAACTCCTGGGGGGACCG<br>TCAGTCTTCCTCTTCCCCCCAAAACCCAAGGACACCCTCATGATCTCCCGGACCCCTGAGGT<br>CACATGCGTGGTGGTGGACGTGAGCCACGAAGACCCTGAGGTCAAGTTCAACTGGTACGTGG<br>ACGGCGTGGAGGTGCATAATGCCAAGACAAAGCCGCGGGAGGAGCAGTACAACAGCACGTAC<br>CGTGTGGTCAGCGTCCTCACCGTCCTGCACCAGGACTGGCTGAATGGCAAGGAGTACAAGTG<br>CAAGGTCTCCAACAAAGCCCTCCCAGCCCCCATCGAGAAAACCATCTCCAAAGCCAAAGGGC<br>AGCCCCGAGAACCACAGGTGTACACCCTGCCCCCATGCCGGGAGGAGATGACCAAGAACCAG<br>GTCAGCCTGTGGTGCCTGGTCAAAGGCTTCTATCCCAGCGACATCGCCGTGGAGTGGGAGAG<br>CAATGGGCAGCCGGAGAACAACTACAAGACCACGCCTCCCGTGCTGGACTCCGACGGCTCCT<br>TCTTCCTCTACAGCAAGCTCACCGTGGACAAGAGCAGGTGGCAGCAGGGGAACGTCTTCTCA<br>TGCTCCGTGATGCATGAGGCTCTGCACAACCACTACACGCAGAAGAGCCTCTCCCTGTCTCC<br>GGGTAAA | |
| EPB04 HC | GAGGTTCAACTCCTTGAATCCGGAGGAGGACTTGTCCAACCAGGCGGCAGCCTTAGGCTGTC<br>CTGCGCTGCCTCAGGCTTTACATTCAGTCATTACATGATGGCCTGGGTTAGACAGGCACCCG<br>GCAAAGGTCTGGAATGGGTCAGCCGAATAGGACCATCAGGAGGTCCCACTCACTATGCCGAT<br>TCTGTAAAAGGGGAGGTTTACAATTTCCAGAGACAATTCAAAGAATACCCTTTACCTCCAGAT<br>GAACTCATTGAGAGCCGAGGACACAGCCGTATATTATTGCGCAGGTTATGATTCCGGTTACG<br>ATTACGTTGCAGTCGCAGGCCCAGCCGAATACTTCCAACATTGGGGTCAGGGAACCCTCGTG<br>ACCGTGTCCAGTGCTAGCACCAAAGGACCTAGTGTTTTTCCTCTTGCCCCTGAGGTGCAACT<br>GCTTGAGTCAGGAGGAGGACTGGTTCAACCCGGCGGGAGCCTTCGACTCTCATGTGCAGCCT<br>CTGGGTTTACATTTAGTCGTTACCAAATGATGTGGGTACGCCAGGCCCCCGGAAAATGTTTG<br>GAGTGGGTGAGCAGCATTTCTCCTTCAGGAGGAGTGACACTTTACGCAGACAGTGTTAAGGG<br>TCGGTTCACAATCAGTCGTGATAATTCCAAGAACACTCTCTATCTCCAAATGAACTCCCTTC<br>GAGCTGAGGACACTGCTGTTTATTACTGTACTCGGGAACTTCTCGGCACTGTGGTTGTTCCT<br>GTGGCTTGGAAGATGCGTGGATACTTCGACTATTGGGGGCAACTCACTCTTGTTACCGTCTC<br>AAGCGCTAGCACCAAAGGACCTAGTGTTTTTCCTCTTGCCCCCTTCCTCAAAGTCTACCTCTG<br>GGGGGACAGCCGCTCTGGGCTGCCTGGTCAAGGATTATTTCCCAGAGCCTGTCACTGTCAGT<br>TGGAACTCTGGAGCCTTGACTTCTGGTGTTCATACATTTCCTGCTGTCCTTCAAAGCAGCGG<br>CTTGTACTCATTGTCTTCTGTTGTGACAGTACCCTCAAGCAGCCTCGGCACTCAGACATACA<br>TCTGCAATGTCAACCACAAACCCTCAAATACAAAGGTAGATAAAAAAGTCGAACCAAAGTCT<br>TGTGACAAAACTCACACGTGCCCACCGTGCCCAGCACCTGAACTCCTGGGGGGACCGTCAGT<br>CTTCCTCTTCCCCCCAAAACCCAAGGACACCCTCATGATCTCCCGGACCCCTGAGGTCACAT<br>GCGTGGTGGTGGACGTGAGCCACGAAGACCCTGAGGTCAAGTTCAACTGGTACGTGGACGGC<br>GTGGAGGTGCATAATGCCAAGACAAAGCCGCGGGAGGAGCAGTACAACAGCACGTACCGTGT | 534 |

| | | |
|---|---|---|
| | GGTCAGCGTCCTCACCGTCCTGCACCAGGACTGGCTGAATGGCAAGGAGTACAAGTGCAAGG TCTCCAACAAAGCCCTCCCAGCCCCCATCGAGAAAACCATCTCCAAAGCCAAAGGGCAGCCC CGAGAACCACAGGTGTACACCCTGCCCCCATGCCGGGAGGAGATGACCAAGAACCAGGTCAG CCTGTGGTGCCTGGTCAAAGGCTTCTATCCCAGCGACATCGCCGTGGAGTGGGAGAGCAATG GGCAGCCGGAGAACAACTACAAGACCACGCCTCCCGTGCTGGACTCCGACGGCTCCTTCTTC CTCTACAGCAAGCTCACCGTGGACAAGAGCAGGTGGCAGCAGGGGAACGTCTTCTCATGCTC CGTGATGCATGAGGCTCTGCACAACCACTACACGCAGAAGAGCCTCTCCCTGTCTCCGGGTA AA | |
| EPB04 LC | GACATACAGATGACACAAAGTCCAAGTAGCCTTTCTGCATCCGTTGGAGACCGCGTAACCAT AACTTGTCGAGCATCACAAAGCATAAGCACTTGGCTTGCTTGGTATCAGCAGAAGCCAGGCA AAGCACCCAAGCTGCTGATTTATAAAGCCTCTAATCTCCATACAGGAGTTCCCAGCCGTTTC TCAGGCAGCGGGTCTGGAACCGAATTCTCTCTGACCATCTCTGGCTTGCAACCTGATGACTT TGCAACATATTACTGTCAACAATATAATTCATATAGTCGTACTTTCGGGCAAGGCACTAAGG TGGAAATCAAACGTACGGTGGCAGCTCCCAGCGTTTTTATCTTTCCCCCAGACATACAGATG ACTCAGAGCCCTGGTACACTGAGTGTATCTCCTGGGGAACGCGCCACTCTGTCTTGCCGAGC CTCTCAAAGCGTTAGTTCCAACCTGGCTTGGTATCAGCAAAAACCCGGTCAAGCACCTCGCT TGCTTATCTACGGGGCCTCCACACGGGCAACCGGCATTCCTGCACGCTTTAGTGGCTCTGGT AGCGGGACTGAATTCACTTTGACTATATCTTCCATGCAGAGTGAGGATTTCGCCGTTTATTA TTGTCAACAGTACAATAACTGGCCTCCACTCACTTTTGGTTGTGGCACCAAAGTGGAGATAA AGCGTACGGTGGCAGCTCCCAGCGTTTTTATCTTTCCCCCATCCGACGAGCAGCTCAAGAGT GGCACTGCCTCTGTAGTTTGTTTGCTGAATAACTTCTATCCACGTGAAGCAAAAGTACAGTG GAAGGTCGATAATGCCCTTCAGAGCGGTAACAGCCAAGAAAGTGTTACCGAGCAAGATTCCA AAGATTCCACTTACAGTCTGTCCAGCACATTGACACTGAGTAAGGCTGATTACGAAAAACAC AAGGTGTACGCATGCGAGGTGACACACCAAGGTCTTTCATCTCCTGTAACTAAGAGCTTTAA CCGGGGGAGAATGTGGTGGTGGGGGCAGCGGGGGCGGAGGTAGTGGAGGCGGCGGTAGTGAAC CAAAGAGTAGTGACAAAACTCACACGTGCCCACCGTGCCCAGCACCTGAACTCCTGGGGGGA CCGTCAGTCTTCCTCTTCCCCCCAAAACCCAAGGACACCCTCATGATCTCCCGGACCCCTGA GGTCACATGCGTGGTGGTGGACGTGAGCCACGAAGACCCTGAGGTCAAGTTCAACTGGTACG TGGACGGCGTGGAGGTGCATAATGCCAAGACAAAGCCGCGGGAGGAGCAGTACAACAGCACG TACCGTGTGGTCAGCGTCCTCACCGTCCTGCACCAGGACTGGCTGAATGGCAAGGAGTACAA GTGCAAGGTCTCCAACAAAGCCCTCCCAGCCCCCATCGAGAAAACCATCTCCAAAGCCAAAG GGCAGCCCCGAGAACCACAGGTGTACACCCTGCCCCCATGCCGGGAGGAGATGACCAAGAAC CAGGTCAGCCTGTGGTGCCTGGTCAAAGGCTTCTATCCCAGCGACATCGCCGTGGAGTGGGA GAGCAATGGGCAGCCGGAGAACAACTACAAGACCACGCCTCCCGTGCTGGACTCCGACGGCT CCTTCTTCCTCTACAGCAAGCTCACCGTGGACAAGAGCAGGTGGCAGCAGGGGAACGTCTTC TCATGCTCCGTGATGCATGAGGCTCTGCACAACCACTACACGCAGAAGAGCCTCTCCCTGTC TCCGGGTAAA | 535 |
| EPB05 HC | GAGGTTCAACTCCTTGAATCCGGAGGAGGACTTGTCCAACCAGGCGGCAGCCTTAGGCTGTC CTGCGCTGCCTCAGGCTTTACATTCAGTCATTACATGATGGCCTGGGTTAGACAGGCACCCG GCAAAGGTCTGGAATGGGTCAGCCGAATAGGACCATCAGGAGGTCCCACTCACTATGCCGAT TCTGTAAAAGGGAGGTTTACAATTTCCAGAGACAATTCAAAGAATACCCTTTACCTCCAGAT GAACTCATTGAGAGCCGAGGACACAGCCGTATATTATTGCGCAGGTTATGATTCCGGTTACG ATTACGTTGCAGTCGCAGGCCCAGCCGAATACTTCCAACATTGGGGTCAGGGAACCCTCGTG ACCGTGTCCAGTGCTAGCACCAAAGGACCTAGTGTTTTTCCTCTTGCCCCTGAGGTCCAGCT GGTTGAGTCTGGAGGTGGTGTAGTCAGGCCAGGAGGTTCCCTGCGTCTGTCTTGCGCTGCCT CTGGGTTCACTGTGTCTGACTACAGTATGAACTGGGTTAGACAGGCTCCAGGGAAATGTTTG GAATGGATAGGGTTTATTCGCAATAAAGCCAATGCCTATACCACCGAATACAGCGCAAGCGT TAAAGGCAGGTTCACCATCAGTAGGGACGACTCTAAGAATACCCTCTACCTGCAGATGAATA GCTTGAAGACAGAGGATACCGCTGTTTATTACTGCACTACCTACCCCAGGTATCATGCTATG GACAGTTGGGGTCAAGGAACTATGGTCACCGTCTCCTCTGCTAGCACCAAAGGACCTAGTGT | 536 |

| | | |
|---|---|---|
| | TTTTCCTCTTGCCCCTTCCTCAAAGTCTACCTCTGGGGGGACAGCCGCTCTGGGCTGCCTGG TCAAGGATTATTTCCCAGAGCCTGTCACTGTCAGTTGGAACTCTGGAGCCTTGACTTCTGGT GTTCATACATTTCCTGCTGTCCTTCAAAGCAGCGGCTTGTACTCATTGTCTTCTGTTGTGAC AGTACCCTCAAGCAGCCTCGGCACTCAGACATACATCTGCAATGTCAACCACAAACCCTCAA ATACAAAGGTAGATAAAAAAGTCGAACCAAAGTCTTGTGACAAAACTCACACGTGCCCACCG TGCCCAGCACCTGAACTCCTGGGGGGACCGTCAGTCTTCCTCTTCCCCCCAAAACCCAAGGA CACCCTCATGATCTCCCGGACCCCTGAGGTCACATGCGTGGTGGTGGACGTGAGCCACGAAG ACCCTGAGGTCAAGTTCAACTGGTACGTGGACGGCGTGGAGGTGCATAATGCCAAGACAAAG CCGCGGGAGGAGCAGTACAACAGCACGTACCGTGTGGTCAGCGTCCTCACCGTCCTGCACCA GGACTGGCTGAATGGCAAGGAGTACAAGTGCAAGGTCTCCAACAAAGCCCTCCCAGCCCCCA TCGAGAAAACCATCTCCAAAGCCAAAGGGCAGCCCCGAGAACCACAGGTGTACACCCTGCCC CCATGCCGGGAGGAGATGACCAAGAACCAGGTCAGCCTGTGGTGCCTGGTCAAAGGCTTCTA TCCCAGCGACATCGCCGTGGAGTGGGAGAGCAATGGGCAGCCGGAGAACAACTACAAGACCA CGCCTCCCGTGCTGGACTCCGACGGCTCCTTCTTCCTCTACAGCAAGCTCACCGTGGACAAG AGCAGGTGGCAGCAGGGGAACGTCTTCTCATGCTCCGTGATGCATGAGGCTCTGCACAACCA CTACACGCAGAAGAGCCTCTCCCTGTCTCCGGGTAAA | |
| EPB05 LC | GACATACAGATGACACAAAGTCCAAGTAGCCTTTCTGCATCCGTTGGAGACCGCGTAACCAT AACTTGTCGAGCATCACAAAGCATAAGCACTTGGCTTGCTTGGTATCAGCAGAAGCCAGGCA AAGCACCCAAGCTGCTGATTTATAAAGCCTCTAATCTCCATACAGGAGTTCCCAGCCGTTTC TCAGGCAGCGGGTCTGGAACCGAATTCTCTCTGACCATCTCTGGCTTGCAACCTGATGACTT TGCAACATATTACTGTCAACAATATAATTCATATAGTCGTACTTTCGGGCAAGGCACTAAGG TGGAAATCAAACGTACGGTGGCAGCTCCCAGCGTTTTTATCTTTCCCCCAGCCATACAGCTG ACTCAATCACCTTCTTCTTTGAGCGCCTCTGTCGGTGATCGCGTTACAATTACATGCCGAGC TTCTCAGTCTATCTCTAATAACCTCCACTGGTATCTCCAGAAACCTGGACAGTCACCACAGC TTCTGATATATTACGGCTTTCAGTCTATAAGTGGAGTCCCAAGTAGATTTTCCGGTAGTGGA TCTGGCACCGATTTTACTCTTACTATCTCTAGCCTCCAACCTGAAGATTTCGCAACCTATTA CTGCCAGCAGGCCAATTCCTGGCCCTTGACCTTCGGATGTGGGACTAAGCTGGAAATCAAAC GTACGGTGGCAGCTCCCAGCGTTTTTATCTTTCCCCCATCCGACGAGCAGCTCAAGAGTGGC ACTGCCTCTGTAGTTTGTTTGCTGAATAACTTCTATCCACGTGAAGCAAAAGTACAGTGGAA GGTCGATAATGCCCTTCAGAGCGGTAACAGCCAAGAAAGTGTTACCGAGCAAGATTCCAAAG ATTCCACTTACAGTCTGTCCAGCACATTGACACTGAGTAAGGCTGATTACGAAAAACACAAG GTGTACGCATGCGAGGTGACACACCAAGGTCTTTCATCTCCTGTAACTAAGAGCTTTAACCG GGGAGAATGTGGTGGTGGGGGCAGCGGGGGCGGAGGTAGTGGAGGCGGCGGTAGTGAACCAA AGAGTAGTGACAAAACTCACACGTGCCCACCGTGCCCAGCACCTGAACTCCTGGGGGGACCG TCAGTCTTCCTCTTCCCCCCAAAACCCAAGGACACCCTCATGATCTCCCGGACCCCTGAGGT CACATGCGTGGTGGTGGACGTGAGCCACGAAGACCCTGAGGTCAAGTTCAACTGGTACGTGG ACGGCGTGGAGGTGCATAATGCCAAGACAAAGCCGCGGGAGGAGCAGTACAACAGCACGTAC CGTGTGGTCAGCGTCCTCACCGTCCTGCACCAGGACTGGCTGAATGGCAAGGAGTACAAGTG CAAGGTCTCCAACAAAGCCCTCCCAGCCCCCATCGAGAAAACCATCTCCAAAGCCAAAGGGC AGCCCCGAGAACCACAGGTGTACACCCTGCCCCCCATGCCGGGAGGAGATGACCAAGAACCAG GTCAGCCTGTGGTGCCTGGTCAAAGGCTTCTATCCCAGCGACATCGCCGTGGAGTGGGAGAG CAATGGGCAGCCGGAGAACAACTACAAGACCACGCCTCCCGTGCTGGACTCCGACGGCTCCT TCTTCCTCTACAGCAAGCTCACCGTGGACAAGAGCAGGTGGCAGCAGGGGAACGTCTTCTCA TGCTCCGTGATGCATGAGGCTCTGCACAACCACTACACGCAGAAGAGCCTCTCCCTGTCTCC GGGTAAA | 537 |
| EPB06 HC | GAGGTTCAACTCCTTGAATCCGGAGGAGGACTTGTCCAACCAGGCGGCAGCCTTAGGCTGTC CTGCGCTGCCTCAGGCTTTACATTCAGTCATTACATGATGGCCTGGGTTAGACAGGCACCCG GCAAAGGTCTGGAATGGGTCAGCCGAATAGGACCATCAGGAGGTCCCACTCACTATGCCGAT TCTGTAAAAGGGAGGTTTACAATTTCCAGAGACAATTCAAAGAATACCCTTTACCTCCAGAT GAACTCATTGAGAGCCGAGGACACAGCCGTATATTATTGCGCAGGTTATGATTCCGGTTACG | 538 |

| | | |
|---|---|---|
| | ATTACGTTGCAGTCGCAGGCCCAGCCGAATACTTCCAACATTGGGGTCAGGGAACCCTCGTGACCGTGTCCAGTGCTAGCACCAAAGGACCTAGTGTTTTTCCTCTTGCCCCTCAAGTGCAACTCCTTGAAAGTGGAGGTGGCCTTGTCCAGCCAGGCGGTTCACTGCGTTTGAGCTGTGCCGCATCCGGCTTTACCTTTTCCAGTTACACAATGAGTTGGGTCCGTCAAGCACCCGGGCAATGTTTGGAATGGATGGGAACTATTTCATCTGGTGGGACTTACACTTACTATCCCGATAGCGTCAAGGGACGTTTTACCATAAGCCGGGACAACGCTAAAAATTCACTCTATCTTCAGATGAACAGTCTTAGGGCTGAGGATACCGCTGTTTATTATTGCGCCCGAGAAGCCATTTTCACCTACTGGGGGCGCGGTACACTTGTCACCGTTAGCAGTGCTAGCACCAAAGGACCTAGTGTTTTTCCTCTTGCCCCTTCCTCAAAGTCTACCTCTGGGGGGACAGCCGCTCTGGGCTGCCTGGTCAAGGATTATTTCCCAGAGCCTGTCACTGTCAGTTGGAACTCTGGAGCCTTGACTTCTGGTGTTCATACATTTCCTGCTGTCCTTCAAAGCAGCGGCTTGTACTCATTGTCTTCTGTTGTGACAGTACCCTCAAGCAGCCTCGGCACTCAGACATACATCTGCAATGTCAACCACAAACCCTCAAATACAAAGGTAGATAAAAAAGTCGAACCAAAGTCTTGTGACAAAACTCACACGTGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATGCCGGGAGGAGATGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | |
| EPBO6 LC | GACATACAGATGACACAAAGTCCAAGTAGCCTTTCTGCATCCGTTGGAGACCGCGTAACCATAACTTGTCGAGCATCACAAAGCATAAGCACTTGGCTTGCTTGGTATCAGCAGAAGCCAGGCAAAGCACCCAAGCTGCTGATTTATAAAGCCTCTAATCTCCATACAGGAGTTCCCAGCCGTTTCTCAGGCAGCGGGTCTGGAACCGAATTCTCTCTGACCATCTCTGGCTTGCAACCTGATGACTTTGCAACATATTACTGTCAACAATATAATTCATATAGTCGTACTTTCGGGCAAGGCACTAAGGTGGAAATCAAACGTACGGTGGCAGCTCCCAGCGTTTTTATCTTTCCCCCAGACATACAACTCACACAAAGTCCAAGCTCTCTTTCCCTTAGTCCAGGAGAACGCGTAACTCTCTCATGTAAAGCCTCCCAGGATATCAATAACTATCTCTCTTGGTACCAACAAAGCCTGACCAAGCTCCTAAGCTGCTTATCAAACGCGCCAATCGGTTGGTAGACGGAGTTCCCGACAGGTTCAGTGGGTCAGGCTCAGGTACAGATTTTACCTTGAAGATATCACGCGTGGAAGCCGAAGACGTAGGGGTATATTATTGTCTGAAATATGACGAGTTCCCCTATACTTTTGGGTGTGGAACTCGCCTGGAAATAAAACGTACGGTGGCAGCTCCCAGCGTTTTTATCTTTCCCCCATCCGACGAGCAGCTCAAGAGTGGCACTGCCTCTGTAGTTTGTTTGCTGAATAACTTCTATCCACGTGAAGCAAAAGTACAGTGGAAGGTCGATAATGCCCTTCAGAGCGGTAACAGCCAAGAAAGTGTTACCGAGCAAGATTCCAAAGATTCCACTTACAGTCTGTCCAGCACATTGACACTGAGTAAGGCTGATTACGAAAAACACAAGGTGTACGCATGCGAGGTGACACACCAAGGTCTTTCATCTCCTGTAACTAAGAGCTTTAACCGGGGAGAATGTGGTGGTGGGGGGCAGCGGGGGCGGAGGTAGTGGAGGCGGCGGTAGTGAACCAAAGAGTAGTGACAAAACTCACACGTGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATGCCGGGAGGAGATGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCT | 539 |

| | | |
|---|---|---|
| | TCTTCCTCTACAGCAAGCTCACCGTGGACAAGAGCAGGTGGCAGCAGGGGAACGTCTTCTCA TGCTCCGTGATGCATGAGGCTCTGCACAACCACTACACGCAGAAGAGCCTCTCCCTGTCTCC GGGTAAA | |
| EPB07 HC | GAGGTCCAGCTGGTTGAGTCTGGAGGTGGTGTAGTCAGGCCAGGAGGTTCCCTGCGTCTGTC TTGCGCTGCCTCTGGGTTCACTGTGTCTGACTACAGTATGAACTGGGTTAGACAGGCTCCAG GGAAAGGTTTGGAATGGATAGGGTTTATTCGCAATAAAGCCAATGCCTATACCACCGAATAC AGCGCAAGCGTTAAAGGCAGGTTCACCATCAGTAGGGACGACTCTAAGAATACCCTCTACCT GCAGATGAATAGCTTGAAGACAGAGGATACCGCTGTTTATTACTGCACTACCTACCCCAGGT ATCATGCTATGGACAGTTGGGGTCAAGGAACTATGGTCACCGTCTCCTCTGCTAGCACCAAA GGACCTAGTGTTTTTCCTCTTGCCCCTGAGGTGCAACTGCTTGAGTCAGGAGGAGGACTGGT TCAACCCGGCGGGAGCCTTCGACTCTCATGTGCAGCCTCTGGGTTTACATTTAGTCGTTACC AAATGATGTGGGTACGCCAGGCCCCCGGAAAATGTTTGGAGTGGGTGAGCAGCATTTCTCCT TCAGGAGGAGTGACACTTTACGCAGACAGTGTTAAGGGTCGGTTCACAATCAGTCGTGATAA TTCCAAGAACACTCTCTATCTCCAAATGAACTCCCTTCGAGCTGAGGACACTGCTGTTTATT ACTGTACTCGGGAACTTCTCGGCACTGTGGTTGTTCCTGTGGCTTGGAAGATGCGTGGGATAC TTCGACTATTGGGGGCAACTCACTCTTGTTACCGTCTCAAGCGCTAGCACCAAAGGACCTAG TGTTTTTCCTCTTGCCCCTTCCTCAAAGTCTACCTCTGGGGGGACAGCCGCTCTGGGCTGCC TGGTCAAGGATTATTTCCCAGAGCCTGTCACTGTCAGTTGGAACTCTGGAGCCTTGACTTCT GGTGTTCATACATTTCCTGCTGTCCTTCAAAGCAGCGGCTTGTACTCATTGTCTTCTGTTGT GACAGTACCCTCAAGCAGCCTCGGCACTCAGACATACATCTGCAATGTCAACCACAAACCCT CAAATACAAAGGTAGATAAAAAAGTCGAACCAAAGTCTTGTGACAAAACTCACACGTGCCCA CCGTGCCCAGCACCTGAACTCCTGGGGGGACCGTCAGTCTTCCTCTTCCCCCCAAAACCCAA GGACACCCTCATGATCTCCCGGACCCCTGAGGTCACATGCGTGGTGGTGGACGTGAGCCACG AAGACCCTGAGGTCAAGTTCAACTGGTACGTGGACGGCGTGGAGGTGCATAATGCCAAGACA AAGCCGCGGGAGGAGCAGTACAACAGCACGTACCGTGTGGTCAGCGTCCTCACCGTCCTGCA CCAGGACTGGCTGAATGGCAAGGAGTACAAGTGCAAGGTCTCCAACAAAGCCCTCCCAGCCC CCATCGAGAAAACCATCTCCAAAGCCAAAGGGCAGCCCCGAGAACCACAGGTGTACACCCTG CCCCCATGCCGGGAGGAGATGACCAAGAACCAGGTCAGCCTGTGGTGCCTGGTCAAAGGCTT CTATCCCAGCGACATCGCCGTGGAGTGGGAGAGCAATGGGCAGCCGGAGAACAACTACAAGA CCACGCCTCCCGTGCTGGACTCCGACGGCTCCTTCTTCCTCTACAGCAAGCTCACCGTGGAC AAGAGCAGGTGGCAGCAGGGGAACGTCTTCTCATGCTCCGTGATGCATGAGGCTCTGCACAA CCACTACACGCAGAAGAGCCTCTCCCTGTCTCCGGGTAAA | 540 |
| EPB07 LC | GCCATACAGCTGACTCAATCACCTTCTTCTTTGAGCGCCTCTGTCGGTGATCGCGTTACAAT TACATGCCGAGCTTCTCAGTCTATCTCTAATAACCTCCACTGGTATCTCCAGAAACCTGGAC AGTCACCACAGCTTCTGATATATTACGGCTTTCAGTCTATAAGTGGAGTCCCAAGTAGATTT TCCGGTAGTGGATCTGGCACCGATTTTACTCTTACTATCTCTAGCCTCCAACCTGAAGATTT CGCAACCTATTACTGCCAGCAGGCCAATTCCTGGCCCTTGACCTTCGGAGGTGGGACTAAGC TGGAAATCAAACGTACGGTGGCAGCTCCCAGCGTTTTTATCTTTCCCCCAGACATACAGATG ACTCAGAGCCCTGGTACACTGAGTGTATCTCCTGGGGAACGCGCCACTCTGTCTTGCCGAGC CTCTCAAAGCGTTAGTTCCAACCTGGCTTGGTATCAGCAAAAACCCGGTCAAGCACCTCGCT TGCTTATCTACGGGGCCTCCACACGGGCAACCGGCATTCCTGCACGCTTTAGTGGCTCTGGT AGCGGGACTGAATTCACTTTGACTATATCTTCCATGCAGAGTGAGGATTTCGCCGTTTATTA TTGTCAACAGTACAATAACTGGCCTCCACTCACTTTTGGTTGTGGCACCAAAGTGGAGATAA AGCGTACGGTGGCAGCTCCCAGCGTTTTTATCTTTCCCCCATCCGACGAGCAGCTCAAGAGT GGCACTGCCTCTGTAGTTTGTTTGCTGAATAACTTCTATCCACGTGAAGCAAAAGTACAGTG GAAGGTCGATAATGCCCTTCAGAGCGGTAACAGCCAAGAAAGTGTTACCGAGCAAGATTCCA AAGATTCCACTTACAGTCTGTCCAGCACATTGACACTGAGTAAGGCTGATTACGAAAAACAC AAGGTGTACGCATGCGAGGTGACACACCAAGGTCTTTCATCTCCTGTAACTAAGAGCTTTAA CCCGGGGGAGAATGTGGTGGTGGGGGCAGCGGGGGCGGAGGTAGTGGAGGCGGCGGTAGTGAAC CAAAGAGTAGTGACAAAACTCACACGTGCCCACCGTGCCCAGCACCTGAACTCCTGGGGGGA | 541 |

EP 4 700 047 A1

| | | |
|---|---|---|
| | CCGTCAGTCTTCCTCTTCCCCCCAAAAACCCAAGGACACCCTCATGATCTCCCGGACCCCTGA<br>GGTCACATGCGTGGTGGTGGACGTGAGCCACGAAGACCCTGAGGTCAAGTTCAACTGGTACG<br>TGGACGGCGTGGAGGTGCATAATGCCAAGACAAAGCCGCGGGAGGAGCAGTACAACAGCACG<br>TACCGTGTGGTCAGCGTCCTCACCGTCCTGCACCAGGACTGGCTGAATGGCAAGGAGTACAA<br>GTGCAAGGTCTCCAACAAAGCCCTCCCAGCCCCCATCGAGAAAACCATCTCCAAAGCCAAAG<br>GGCAGCCCCGAGAACCACAGGTGTACACCCTGCCCCCATGCCGGGAGGAGATGACCAAGAAC<br>CAGGTCAGCCTGTGGTGCCTGGTCAAAGGCTTCTATCCCAGCGACATCGCCGTGGAGTGGGA<br>GAGCAATGGGCAGCCGGAGAACAACTACAAGACCACGCCTCCCGTGCTGGACTCCGACGGCT<br>CCTTCTTCCTCTACAGCAAGCTCACCGTGGACAAGAGCAGGTGGCAGCAGGGGAACGTCTTC<br>TCATGCTCCGTGATGCATGAGGCTCTGCACAACCACTACACGCAGAAGAGCCTCTCCCTGTC<br>TCCGGGTAAA | |
| EPB08 HC | CAAGTGCAACTCCTTGAAAGTGGAGGTGGCCTTGTCCAGCCAGGCGGTTCACTGCGTTTGAG<br>CTGTGCCGCATCCGGCTTTACCTTTTCCAGTTACACAATGAGTTGGGTCCGTCAAGCACCCG<br>GGCAAGCTTTGGAATGGATGGGAACTATTTCATCTGGTGGGACTTACACTTACTATCCCGAT<br>AGCGTCAAGGGACGTTTTACCATAAGCCGGGACAACGCTAAAAATTCACTCTATCTTCAGAT<br>GAACAGTCTTAGGGCTGAGGATACCGCTGTTTATTATTGCGCCCGAGAAGCCATTTTCACCT<br>ACTGGGGGCGCGGTACACTTGTCACCGTTAGCAGTGCTAGCACCAAAGGACCTAGTGTTTTT<br>CCTCTTGCCCCTGAGGTGCAACTGCTTGAGTCAGGAGGAGGACTGGTTCAACCCGGCGGGAG<br>CCTTCGACTCTCATGTGCAGCCTCTGGGTTTACATTTAGTCGTTACCAAATGATGTGGGTAC<br>GCCAGGCCCCCGGAAAATGTTTGGAGTGGGTGAGCAGCATTTCTCCTTCAGGAGGAGTGACA<br>CTTTACGCAGACAGTGTTAAGGGTCGGTTCACAATCAGTCGTGATAATTCCAAGAACACTCT<br>CTATCTCCAAATGAACTCCCTTCGAGCTGAGGACACTGCTGTTTATTACTGTACTCGGGAAC<br>TTCTCGGCACTGTGGTTGTTCCTGTGGCTTGGAAGATGCGTGGATACTTCGACTATTGGGGG<br>CAACTCACTCTTGTTACCGTCTCAAGCGCTAGCACCAAAGGACCTAGTGTTTTTCCTCTTGC<br>CCCTTCCTCAAAGTCTACCTCTGGGGGGACAGCCGCTCTGGGCTGCCTGGTCAAGGATTATT<br>TCCCAGAGCCTGTCACTGTCAGTTGGAACTCTGGAGCCTTGACTTCTGGTGTTCATACATTT<br>CCTGCTGTCCTTCAAAGCAGCGGCTTGTACTCATTGTCTTCTGTTGTGACAGTACCCTCAAG<br>CAGCCTCGGCACTCAGACATACATCTGCAATGTCAACCACAAACCCTCAAATACAAAGGTAG<br>ATAAAAAAGTCGAACCAAAGTCTTGTGACAAAACTCACACGTGCCCACCGTGCCCAGCACCT<br>GAACTCCTGGGGGGACCGTCAGTCTTCCTCTTCCCCCCAAAAACCCAAGGACACCCTCATGAT<br>CTCCCGGACCCCTGAGGTCACATGCGTGGTGGTGGACGTGAGCCACGAAGACCCTGAGGTCA<br>AGTTCAACTGGTACGTGGACGGCGTGGAGGTGCATAATGCCAAGACAAAGCCGCGGGAGGAG<br>CAGTACAACAGCACGTACCGTGTGGTCAGCGTCCTCACCGTCCTGCACCAGGACTGGCTGAA<br>TGGCAAGGAGTACAAGTGCAAGGTCTCCAACAAAGCCCTCCCAGCCCCCATCGAGAAAACCA<br>TCTCCAAAGCCAAAGGGCAGCCCCGAGAACCACAGGTGTACACCCTGCCCCCATGCCGGGAG<br>GAGATGACCAAGAACCAGGTCAGCCTGTGGTGCCTGGTCAAAGGCTTCTATCCCAGCGACAT<br>CGCCGTGGAGTGGGAGAGCAATGGGCAGCCGGAGAACAACTACAAGACCACGCCTCCCGTGC<br>TGGACTCCGACGGCTCCTTCTTCCTCTACAGCAAGCTCACCGTGGACAAGAGCAGGTGGCAG<br>CAGGGGAACGTCTTCTCATGCTCCGTGATGCATGAGGCTCTGCACAACCACTACACGCAGAA<br>GAGCCTCTCCCTGTCTCCGGGTAAA | 542 |
| EPB08 LC | GACATACAACTCACACAAAGTCCAAGCTCTCTTTCCCTTAGTCCAGGAGAACGCGTAACTCT<br>CTCATGTAAAGCCTCCCAGGATATCAATAACTATCTCTCTTGGTACCAACAAAAGCCTGACC<br>AAGCTCCTAAGCTGCTTATCAAACGCGCCAATCGGTTGGTAGACGGAGTTCCCGACAGGTTC<br>AGTGGGTCAGGCTCAGGTACAGATTTTACCTTGAAGATATCACGCGTGGAAGCCGAAGACGT<br>AGGGGTATATTATTGTCTGAAATATGACGAGTTCCCCTATACTTTTGGGCAAGGAACTCGCC<br>TGGAAATAAAACGTACGGTGGCAGCTCCCAGCGTTTTTATCTTTCCCCCAGACATACAGATG<br>ACTCAGAGCCCTGGTACACTGAGTGTATCTCCTGGGGAACGCGCCACTCGTCTTGCCGAGC<br>CTCTCAAAGCGTTAGTTCCAACCTGGCTTGGTATCAGCAAAAACCCGGTCAAGCACCTCGCT<br>TGCTTATCTACGGGGCCTCCACACGGGCAACCGGCATTCCTGCACGCTTTAGTGGCTCTGGT<br>AGCGGGACTGAATTCACTTTGACTATATCTTCCATGCAGAGTGAGGATTTCGCCGTTTATTA | 543 |

165

| | | |
|---|---|---|
| | TTGTCAACAGTACAATAACTGGCCTCCACTCACTTTTGGTTGTGGCACCAAAGTGGAGATAA AGCGTACGGTGGCAGCTCCCAGCGTTTTTATCTTTCCCCCATCCGACGAGCAGCTCAAGAGT GGCACTGCCTCTGTAGTTTGTTTGCTGAATAACTTCTATCCACGTGAAGCAAAAGTACAGTG GAAGGTCGATAATGCCCTTCAGAGCGGTAACAGCCAAGAAAGTGTTACCGAGCAAGATTCCA AAGATTCCACTTACAGTCTGTCCAGCACATTGACACTGAGTAAGGCTGATTACGAAAAACAC AAGGTGTACGCATGCGAGGTGACACACCAAGGTCTTTCATCTCCTGTAACTAAGAGCTTTAA CCGGGGGAGAATGTGGTGGTGGGGGCAGCGGGGGCGGAGGTAGTGGAGGCGGCGGTAGTGAAC CAAAGAGTAGTGACAAAACTCACACGTGCCCACCGTGCCCAGCACCTGAACTCCTGGGGGGA CCGTCAGTCTTCCTCTTCCCCCCAAAACCCAAGGACACCCTCATGATCTCCCGGACCCCTGA GGTCACATGCGTGGTGGTGGACGTGAGCCACGAAGACCCTGAGGTCAAGTTCAACTGGTACG TGGACGGCGTGGAGGTGCATAATGCCAAGACAAAGCCGCGGGAGGAGCAGTACAACAGCACG TACCGTGTGGTCAGCGTCCTCACCGTCCTGCACCAGGACTGGCTGAATGGCAAGGAGTACAA GTGCAAGGTCTCCAACAAAGCCCTCCCAGCCCCCATCGAGAAAACCATCTCCAAAGCCAAAG GGCAGCCCCGAGAACCACAGGTGTACACCCTGCCCCCATGCCGGGAGGAGATGACCAAGAAC CAGGTCAGCCTGTGGTGCCTGGTCAAAGGCTTCTATCCCAGCGACATCGCCGTGGAGTGGGA GAGCAATGGGCAGCCGGAGAACAACTACAAGACCACGCCTCCCGTGCTGGACTCCGACGGCT CCTTCTTCCTCTACAGCAAGCTCACCGTGGACAAGAGCAGGTGGCAGCAGGGGAACGTCTTC TCATGCTCCGTGATGCATGAGGCTCTGCACAACCACTACACGCAGAAGAGCCTCTCCCTGTC TCCGGGTAAA | |
| EPB09 HC | GAGGTGCAACTGCTTGAGTCAGGAGGAGGACTGGTTCAACCCGGCGGGAGCCTTCGACTCTC ATGTGCAGCCTCTGGGTTTACATTTAGTCGTTACCAAATGATGTGGGTACGCCAGGCCCCCG GAAAAGGTTTGGAGTGGGTGAGCAGCATTTCTCCTTCAGGAGGAGTGACACTTTACGCAGAC AGTGTTAAGGGTCGGTTCACAATCAGTCGTGATAATTCCAAGAACACTCTCTATCTCCAAAT GAACTCCCTTCGAGCTGAGGACACTGCTGTTTATTACTGTACTCGGGAACTTCTCGGCACTG TGGTTGTTCCTGTGGCTTGGAAGATGCGTGGATACTTCGACTATTGGGGGCAACTCACTCTT GTTACCGTCTCAAGCGCTAGCACCAAAGGACCTAGTGTTTTTCCTCTTGCCCCTGAGGTCCA GCTGGTTGAGTCTGGAGGTGGTGTAGTCAGGCCAGGAGGTTCCCTGCGTCTGTCTTGCGCTG CCTCTGGGTTCACTGTGTCTGACTACAGTATGAACTGGGTTAGACAGGCTCCAGGGAAATGT TTGGAATGGATAGGGTTTATTCGCAATAAAGCCAATGCCTATACCACCGAATACAGCGCAAG CGTTAAAGGCAGGTTCACCATCAGTAGGGACGACTCTAAGAATACCCTCTACCTGCAGATGA ATAGCTTGAAGACAGAGGATACCGCTGTTTATTACTGCACTACCTACCCCAGGTATCATGCT ATGGACAGTTGGGGTCAAGGAACTATGGTCACCGTCTCCTCTGCTAGCACCAAAGGACCTAG TGTTTTTCCTCTTGCCCCTTCCTCAAAGTCTACCTCTGGGGGGACAGCCGCTCTGGGCTGCC TGGTCAAGGATTATTTCCCAGAGCCTGTCACTGTCAGTTGGAACTCTGGAGCCTTGACTTCT GGTGTTCATACATTTCCTGCTGTCCTTCAAAGCAGCGGCTTGTACTCATTGTCTTCTGTTGT GACAGTACCCTCAAGCAGCCTCGGCACTCAGACATACATCTGCAATGTCAACCACAAACCCT CAAATACAAAGGTAGATAAAAAAGTCGAACCAAAGTCTTGTGACAAAACTCACACGTGCCCA CCGTGCCCAGCACCTGAACTCCTGGGGGGACCGTCAGTCTTCCTCTTCCCCCCAAAACCCAA GGACACCCTCATGATCTCCCGGACCCCTGAGGTCACATGCGTGGTGGTGGACGTGAGCCACG AAGACCCTGAGGTCAAGTTCAACTGGTACGTGGACGGCGTGGAGGTGCATAATGCCAAGACA AAGCCGCGGGAGGAGCAGTACAACAGCACGTACCGTGTGGTCAGCGTCCTCACCGTCCTGCA CCAGGACTGGCTGAATGGCAAGGAGTACAAGTGCAAGGTCTCCAACAAAGCCCTCCCAGCCC CCATCGAGAAAACCATCTCCAAAGCCAAAGGGCAGCCCCGAGAACCACAGGTGTACACCCTG CCCCCATGCCGGGAGGAGATGACCAAGAACCAGGTCAGCCTGTGGTGCCTGGTCAAAGGCTT CTATCCCAGCGACATCGCCGTGGAGTGGGAGAGCAATGGGCAGCCGGAGAACAACTACAAGA CCACGCCTCCCGTGCTGGACTCCGACGGCTCCTTCTTCCTCTACAGCAAGCTCACCGTGGAC AAGAGCAGGTGGCAGCAGGGGAACGTCTTCTCATGCTCCGTGATGCATGAGGCTCTGCACAA CCACTACACGCAGAAGAGCCTCTCCCTGTCTCCGGGTAAA | 544 |
| EPB09 LC | GACATACAGATGACTCAGAGCCCCTGGTACACTGAGTGTATCTCCTGGGGAACGCGCCACTCT GTCTTGCCGAGCCTCTCAAAGCGTTAGTTCCAACCTGGCTTGGTATCAGCAAAAACCCGGTC | 545 |

| | | |
|---|---|---|
| | AAGCACCTCGCTTGCTTATCTACGGGGCCTCCACACGGGCAACCGGCATTCCTGCACGCTTT AGTGGCTCTGGTAGCGGGACTGAATTCACTTTGACTATATCTTCCATGCAGAGTGAGGATTT CGCCGTTTATTATTGTCAACAGTACAATAACTGGCCTCCACTCACTTTTGGTGGTGGCACCA AAGTGGAGATAAAGCGTACGGTGGCAGCTCCCAGCGTTTTTATCTTTCCCCCAGCCATACAG CTGACTCAATCACCTTCTTCTTTGAGCGCCTCTGTCGGTGATCGCGTTACAATTACATGCCG AGCTTCTCAGTCTATCTCTAATAACCTCCACTGGTATCTCCAGAAACCTGGACAGTCACCAC AGCTTCTGATATATTACGGCTTTCAGTCTATAAGTGGAGTCCCAAGTAGATTTTCCGGTAGT GGATCTGGCACCGATTTTACTCTTACTATCTCTAGCCTCCAACCTGAAGATTTCGCAACCTA TTACTGCCAGCAGGCCAATTCCTGGCCCTTGACCTTCGGATGTGGGACTAAGCTGGAAATCA AACGTACGGTGGCAGCTCCCAGCGTTTTTATCTTTCCCCCATCCGACGAGCAGCTCAAGAGT GGCACTGCCTCTGTAGTTTGTTTGCTGAATAACTTCTATCCACGTGAAGCAAAAGTACAGTG GAAGGTCGATAATGCCCTTCAGAGCGGTAACAGCCAAGAAAGTGTTACCGAGCAAGATTCCA AAGATTCCACTTACAGTCTGTCCAGCACATTGACACTGAGTAAGGCTGATTACGAAAAACAC AAGGTGTACGCATGCGAGGTGACACACCAAGGTCTTTCATCTCCTGTAACTAAGAGCTTTAA CCGGGGGAGAATGTGGTGGTGGGGGCAGCGGGGGCGGAGGTAGTGGAGGCGGCGGTAGTGAAC CAAAGAGTAGTGACAAAACTCACACGTGCCCACCGTGCCCAGCACCTGAACTCCTGGGGGGA CCGTCAGTCTTCCTCTTCCCCCCAAAACCCAAGGACACCCTCATGATCTCCCGGACCCCTGA GGTCACATGCGTGGTGGTGGACGTGAGCCACGAAGACCCTGAGGTCAAGTTCAACTGGTACG TGGACGGCGTGGAGGTGCATAATGCCAAGACAAAGCCGCGGGAGGAGCAGTACAACAGCACG TACCGTGTGGTCAGCGTCCTCACCGTCCTGCACCAGGACTGGCTGAATGGCAAGGAGTACAA GTGCAAGGTCTCCAACAAAGCCCTCCCAGCCCCCATCGAGAAAACCATCTCCAAAGCCAAAG GGCAGCCCCGAGAACCACAGGTGTACACCCTGCCCCCATGCCGGGAGGAGATGACCAAGAAC CAGGTCAGCCTGTGGTGCCTGGTCAAAGGCTTCTATCCCAGCGACATCGCCGTGGAGTGGGA GAGCAATGGGCAGCCGGAGAACAACTACAAGACCACGCCTCCCGTGCTGGACTCCGACGGCT CCTTCTTCCTCTACAGCAAGCTCACCGTGGACAAGAGCAGGTGGCAGCAGGGGAACGTCTTC TCATGCTCCGTGATGCATGAGGCTCTGCACAACCACTACACGCAGAAGAGCCTCTCCCTGTC TCCGGGTAAA | |
| EPB10 HC | CAAGTGCAACTCCTTGAAAGTGGAGGTGGCCTTGTCCAGCCAGGCGGTTCACTGCGTTTGAG CTGTGCCGCATCCGGCTTTACCTTTTCCAGTTACACAATGAGTTGGGTCCGTCAAGCACCCG GGCAAGCTTTGGAATGGATGGGAACTATTTCATCTGGTGGGACTTACACTTACTATCCCGAT AGCGTCAAGGGACGTTTTACCATAAGCCGGGACAACGCTAAAAAATTCACTCTATCTTCAGAT GAACAGTCTTAGGGCTGAGGATACCGCTGTTTATTATTGCGCCCGAGAAGCCATTTTCACCT ACTGGGGGCGCGGTACACTTGTCACCGTTAGCAGTGCTAGCACCAAAGGACCTAGTGTTTTT CCTCTTGCCCCTGAGGTCCAGCTGGTTGAGTCTGGAGGTGGTGTAGTCAGGCCAGGAGGTTC CCTGCGTCTGTCTTGCGCTGCCTCTGGGTTCACTGTGTCTGACTACAGTATGAACTGGGTTA GACAGGCTCCAGGGAAATGTTTGGAATGGATAGGGTTTATTCGCAATAAAGCCAATGCCTAT ACCACCGAATACAGCGCAAGCGTTAAAGGCAGGTTCACCATCAGTAGGGACGACTCTAAGAA TACCCTCTACCTGCAGATGAATAGCTTGAAGACAGAGGATACCGCTGTTTATTACTGCACTA CCTACCCCAGGTATCATGCTATGGACAGTTGGGGTCAAGGAACTATGGTCACCGTCTCCTCT GCTAGCACCAAAGGACCTAGTGTTTTTCCTCTTGCCCCTTCCTCAAAGTCTACCTCTGGGGG GACAGCCGCTCTGGGCTGCCTGGTCAAGGATTATTTCCCAGAGCCTGTCACTGTCAGTTGGA ACTCTGGAGCCTTGACTTCTGGTGTTCATACATTTCCTGCTGTCCTTCAAAGCAGCGGCTTG TACTCATTGTCTTCTGTTGTGACAGTACCCTCAAGCAGCCTCGGCACTCAGACATACATCTG CAATGTCAACCACAAACCCTCAAATACAAAGGTAGATAAAAAAGTCGAACCAAAGTCTTGTG ACAAAACTCACACGTGCCCACCGTGCCCAGCACCTGAACTCCTGGGGGGACCGTCAGTCTTC CTCTTCCCCCCAAAACCCAAGGACACCCTCATGATCTCCCGGACCCCTGAGGTCACATGCGT GGTGGTGGACGTGAGCCACGAAGACCCTGAGGTCAAGTTCAACTGGTACGTGGACGGCGTGG AGGTGCATAATGCCAAGACAAAGCCGCGGGAGGAGCAGTACAACAGCACGTACCGTGTGGTC AGCGTCCTCACCGTCCTGCACCAGGACTGGCTGAATGGCAAGGAGTACAAGTGCAAGGTCTC CAACAAAGCCCTCCCAGCCCCCATCGAGAAAACCATCTCCAAAGCCAAAGGGCAGCCCCGAG | 546 |

|  |  |  |
|---|---|---|
|  | AACCACAGGTGTACACCCTGCCCCCATGCCGGGAGGAGATGACCAAGAACCAGGTCAGCCTG TGGTGCCTGGTCAAAGGCTTCTATCCCAGCGACATCGCCGTGGAGTGGGAGAGCAATGGGCA GCCGGAGAACAACTACAAGACCACGCCTCCCGTGCTGGACTCCGACGGCTCCTTCTTCCTCT ACAGCAAGCTCACCGTGGACAAGAGCAGGTGGCAGCAGGGGAACGTCTTCTCATGCTCCGTG ATGCATGAGGCTCTGCACAACCACTACACGCAGAAGAGCCTCTCCCTGTCTCCGGGTAAA |  |
| EPB10 LC | GACATACAACTCACACAAAGTCCAAGCTCTCTTTCCCTTAGTCCAGGAGAACGCGTAACTCT CTCATGTAAAGCCTCCCAGGATATCAATAACTATCTCTCTTGGTACCAACAAAAGCCTGACC AAGCTCCTAAGCTGCTTATCAAACGCGCCAATCGGTTGGTAGACGGAGTTCCCGACAGGTTC AGTGGGTCAGGCTCAGGTACAGATTTTACCTTGAAGATATCACGCGTGGAAGCCGAAGACGT AGGGGTATATTATTGTCTGAAATATGACGAGTTCCCCTATACTTTTGGGCAAGGAACTCGCC TGGAAATAAAACGTACGGTGGCAGCTCCCAGCGTTTTTATCTTTCCCCCAGCCATACAGCTG ACTCAATCACCTTCTTCTTTGAGCGCCTCTGTCGGTGATCGCGTTACAATTACATGCCGAGC TTCTCAGTCTATCTCTAATAACCTCCACTGGTATCTCCAGAAACCTGGACAGTCACCACAGC TTCTGATATATTACGGCTTTCAGTCTATAAGTGGAGTCCCAAGTAGATTTTCCGGTAGTGGA TCTGGCACCGATTTTACTCTTACTATCTCTAGCCTCCAACCTGAAGATTTCGCAACCTATTA CTGCCAGCAGGCCAATTCCTGGCCCTTGACCTTCGGATGTGGGACTAAGCTGGAAATCAAAC GTACGGTGGCAGCTCCCAGCGTTTTTATCTTTCCCCCATCCGACGAGCAGCTCAAGAGTGGC ACTGCCTCTGTAGTTTGTTTGCTGAATAACTTCTATCCACGTGAAGCAAAAGTACAGTGGAA GGTCGATAATGCCCTTCAGAGCGGTAACAGCCAAGAAAGTGTTACCGAGCAAGATTCCAAAG ATTCCACTTACAGTCTGTCCAGCACATTGACACTGAGTAAGGCTGATTACGAAAAACACAAG GTGTACGCATGCGAGGTGACACACCAAGGTCTTTCATCTCCTGTAACTAAGAGCTTTAACCG GGGAGAATGTGGTGGTGGGGGGCAGCGGGGGCGGAGGTAGTGGAGGCGGCGGTAGTGAACCAA AGAGTAGTGACAAAACTCACACGTGCCCACCGTGCCCAGCACCTGAACTCCTGGGGGGACCG TCAGTCTTCCTCTTCCCCCCAAAAACCCAAGGACACCCTCATGATCTCCCGGACCCCTGAGGT CACATGCGTGGTGGTGGACGTGAGCCACGAAGACCCTGAGGTCAAGTTCAACTGGTACGTGG ACGGCGTGGAGGTGCATAATGCCAAGACAAAGCCGCGGGAGGAGCAGTACAACAGCACGTAC CGTGTGGTCAGCGTCCTCACCGTCCTGCACCAGGACTGGCTGAATGGCAAGGAGTACAAGTG CAAGGTCTCCAACAAAGCCCTCCCAGCCCCCATCGAGAAAACCATCTCCAAAGCCAAAGGGC AGCCCCGAGAACCACAGGTGTACACCCTGCCCCCATGCCGGGAGGAGATGACCAAGAACCAG GTCAGCCTGTGGTGCCTGGTCAAAGGCTTCTATCCCAGCGACATCGCCGTGGAGTGGGAGAG CAATGGGCAGCCGGAGAACAACTACAAGACCACGCCTCCCGTGCTGGACTCCGACGGCTCCT TCTTCCTCTACAGCAAGCTCACCGTGGACAAGAGCAGGTGGCAGCAGGGGAACGTCTTCTCA TGCTCCGTGATGCATGAGGCTCTGCACAACCACTACACGCAGAAGAGCCTCTCCCTGTCTCC GGGTAAA | 547 |
| EPB11 HC | GAGGTGCAACTGCTTGAGTCAGGAGGAGGACTGGTTCAACCCGGCGGGAGCCTTCGACTCTC ATGTGCAGCCTCTGGGTTTACATTTAGTCGTTACCAAATGATGTGGGTACGCCAGGCCCCCG GAAAAGGTTTGGAGTGGGTGAGCAGCATTTCTCCTTCAGGAGGAGTGACACTTTACGCAGAC AGTGTTAAGGGTCGGTTCACAATCAGTCGTGATAATTCCAAGAACACTCTCTATCTCCAAAT GAACTCCCTTCGAGCTGAGGACACTGCTGTTTATTACTGTACTCGGGAACTTCTCGGCACTG TGGTTGTTCCTGTGGCTTGGAAGATGCGTGGATACTTCGACTATTGGGGGCAACTCACTCTT GTTACCGTCTCAAGCGCTAGCACCAAAGGACCTAGTGTTTTTCCTCTTGCCCCTCAAGTGCA ACTCCTTGAAAGTGGAGGTGGCCTTGTCCAGCCAGGCGGTTCACTGCGTTTGAGCTGTGCCG CATCCGGCTTTACCTTTTCCAGTTACACAATGAGTTGGGTCCGTCAAGCACCCGGGCAATGT TTGGAATGGATGGGAACTATTTCATCTGGTGGGACTTACACTTACTATCCCGATAGCGTCAA GGGACGTTTTACCATAAGCCGGGACAACGCTAAAAAATTCACTCTATCTTCAGATGAACAGTC TTAGGGCTGAGGATACCGCTGTTTATTATTGCGCCCGAGAAGCCATTTTCACCTACTGGGGG CGCGGTACACTTGTCACCGTTAGCAGTGCTAGCACCAAAGGACCTAGTGTTTTTCCTCTTGC CCCTTCCTCAAAGTCTACCTCTGGGGGGACAGCCGCTCTGGGCTGCCTGGTCAAGGATTATT TCCCAGAGCCTGTCACTGTCAGTTGGAACTCTGGAGCCTTGACTTCTGGTGTTCATACATTT CCTGCTGTCCTTCAAAGCAGCGGCTTGTACTCATTGTCTTCTGTTGTGACAGTACCCTCAAG | 548 |

| | | |
|---|---|---|
| | CAGCCTCGGCACTCAGACATACATCTGCAATGTCAACCACAAACCCTCAAATACAAAGGTAG ATAAAAAAGTCGAACCAAAGTCTTGTGACAAAACTCACACGTGCCCACCGTGCCCAGCACCT GAACTCCTGGGGGGACCGTCAGTCTTCCTCTTCCCCCCAAAACCCAAGGACACCCTCATGAT CTCCCGGACCCCTGAGGTCACATGCGTGGTGGTGGACGTGAGCCACGAAGACCCTGAGGTCA AGTTCAACTGGTACGTGGACGGCGTGGAGGTGCATAATGCCAAGACAAAGCCGCGGGAGGAG CAGTACAACAGCACGTACCGTGTGGTCAGCGTCCTCACCGTCCTGCACCAGGACTGGCTGAA TGGCAAGGAGTACAAGTGCAAGGTCTCCAACAAAGCCCTCCCAGCCCCCATCGAGAAAACCA TCTCCAAAGCCAAAGGGCAGCCCCGAGAACCACAGGTGTACACCCTGCCCCCATGCCGGGAG GAGATGACCAAGAACCAGGTCAGCCTGTGGTGCCTGGTCAAAGGCTTCTATCCCAGCGACAT CGCCGTGGAGTGGGAGAGCAATGGGCAGCCGGAGAACAACTACAAGACCACGCCTCCCGTGC TGGACTCCGACGGCTCCTTCTTCCTCTACAGCAAGCTCACCGTGGACAAGAGCAGGTGGCAG CAGGGGAACGTCTTCTCATGCTCCGTGATGCATGAGGCTCTGCACAACCACTACACGCAGAA GAGCCTCTCCCTGTCTCCGGGTAAA | |
| EPB11 LC | GACATACAGATGACTCAGAGCCCTGGTACACTGAGTGTATCTCCTGGGGAACGCGCCACTCT GTCTTGCCGAGCCTCTCAAAGCGTTAGTTCCAACCTGGCTTGGTATCAGCAAAAACCCGGTC AAGCACCTCGCTTGCTTATCTACGGGGCCTCCACACGGGCAACCGGCATTCCTGCACGCTTT AGTGGCTCTGGTAGCGGGACTGAATTCACTTTGACTATATCTTCCATGCAGAGTGAGGATTT CGCCGTTTATTATTGTCAACAGTACAATAACTGGCCTCCACTCACTTTTGGTGGTGGCACCA AAGTGGAGATAAAGCGTACGGTGGCAGCTCCCAGCGTTTTTATCTTTCCCCCAGACATACAA CTCACACAAAGTCCAAGCTCTCTTTCCCTTAGTCCAGGAGAACGCGTAACTCTCTCATGTAA AGCCTCCCAGGATATCAATAACTATCTCTCTTGGTACCAACAAAAGCCTGACCAAGCTCCTA AGCTGCTTATCAAACGCGCCAATCGGTTGGTAGACGGAGTTCCCGACAGGTTCAGTGGGTCA GGCTCAGGTACAGATTTTACCTTGAAGATATCACGCGTGGAAGCCGAAGACGTAGGGGTATA TTATTGTCTGAAATATGACGAGTTCCCCTATACTTTTGGGTGTGGAACTCGCCTGGAAATAA AACGTACGGTGGCAGCTCCCAGCGTTTTTATCTTTCCCCCATCCGACGAGCAGCTCAAGAGT GGCACTGCCTCTGTAGTTTGTTTGCTGAATAACTTCTATCCACGTGAAGCAAAAGTACAGTG GAAGGTCGATAATGCCCTTCAGAGCGGTAACAGCCAAGAAAGTGTTACCGAGCAAGATTCCA AAGATTCCACTTACAGTCTGTCCAGCACATTGACACTGAGTAAGGCTGATTACGAAAAACAC AAGGTGTACGCATGCGAGGTGACACACCAAGGTCTTTCATCTCCTGTAACTAAGAGCTTTAA CCGGGGGAGAATGTGGTGGTGGGGGGCAGCGGGGGCGGAGGTAGTGGAGGCGGCGGTAGTGAAC CAAAGAGTAGTGACAAAACTCACACGTGCCCCACCGTGCCCAGCACCTGAACTCCTGGGGGGA CCGTCAGTCTTCCTCTTCCCCCCAAAACCCAAGGACACCCTCATGATCTCCCGGACCCCTGA GGTCACATGCGTGGTGGTGGACGTGAGCCACGAAGACCCTGAGGTCAAGTTCAACTGGTACG TGGACGGCGTGGAGGTGCATAATGCCAAGACAAAGCCGCGGGAGGAGCAGTACAACAGCACG TACCGTGTGGTCAGCGTCCTCACCGTCCTGCACCAGGACTGGCTGAATGGCAAGGAGTACAA GTGCAAGGTCTCCAACAAAGCCCTCCCAGCCCCCATCGAGAAAACCATCTCCAAAGCCAAAG GGCAGCCCCGAGAACCACAGGTGTACACCCTGCCCCCATGCCGGGAGGAGATGACCAAGAAC CAGGTCAGCCTGTGGTGCCTGGTCAAAGGCTTCTATCCCAGCGACATCGCCGTGGAGTGGGA GAGCAATGGGCAGCCGGAGAACAACTACAAGACCACGCCTCCCGTGCTGGACTCCGACGGCT CCTTCTTCCTCTACAGCAAGCTCACCGTGGACAAGAGCAGGTGGCAGCAGGGGAACGTCTTC TCATGCTCCGTGATGCATGAGGCTCTGCACAACCACTACACGCAGAAGAGCCTCTCCCTGTC TCCGGGTAAA | 549 |
| EPB12 HC | GAGGTCCAGCTGGTTGAGTCTGGAGGTGGTGTAGTCAGGCCAGGAGGTTCCCTGCGTCTGTC TTGCGCTGCCTCTGGGTTCACTGTGTCTGACTACAGTATGAACTGGGTTAGACAGGCTCCAG GGAAAGGTTTGGAATGGATAGGGTTTATTCGCAATAAAGCCAATGCCTATACCACCGAATAC AGCGCAAGCGTTAAAGGCAGGTTCACCATCAGTAGGGACGACTCTAAGAATACCCTCTACCT GCAGATGAATAGCTTGAAGACAGAGGATACCGCTGTTTATTACTGCACTACCTACCCCAGGT ATCATGCTATGGACAGTTGGGGTCAAGGAACTATGGTCACCGTCTCCTCTGCTAGCACCAAA GGACCTAGTGTTTTTCCTCTTGCCCCTCAAGTGCAACTCCTTGAAAGTGGAGGTGGCCTTGT CCAGCCAGGCGGTTCACTGCGTTTGAGCTGTGCCGCATCCGGCTTTACCTTTTCCAGTTACA | 550 |

| | | |
|---|---|---|
| | CAATGAGTTGGGTCCGTCAAGCACCCGGGCAATGTTTGGAATGGATGGGAACTATTTCATCT GGTGGGACTTACACTTACTATCCCGATAGCGTCAAGGGACGTTTTACCATAAGCCGGGACAA CGCTAAAAATTCACTCTATCTTCAGATGAACAGTCTTAGGGCTGAGGATACCGCTGTTTATT ATTGCGCCCGAGAAGCCATTTTCACCTACTGGGGGCGCGGTACACTTGTCACCGTTAGCAGT GCTAGCACCAAAGGACCTAGTGTTTTTCCTCTTGCCCCTTCCTCAAAGTCTACCTCTGGGGG GACAGCCGCTCTGGGCTGCCTGGTCAAGGATTATTTCCCAGAGCCTGTCACTGTCAGTTGGA ACTCTGGAGCCTTGACTTCTGGTGTTCATACATTTCCTGCTGTCCTTCAAAGCAGCGGCTTG TACTCATTGTCTTCTGTTGTGACAGTACCCTCAAGCAGCCTCGGCACTCAGACATACATCTG CAATGTCAACCACAAACCCTCAAATACAAAGGTAGATAAAAAAGTCGAACCAAAGTCTTGTG ACAAAACTCACACGTGCCCACCGTGCCCAGCACCTGAACTCCTGGGGGGACCGTCAGTCTTC CTCTTCCCCCCAAAACCCAAGGACACCCTCATGATCTCCCGGACCCCTGAGGTCACATGCGT GGTGGTGGACGTGAGCCACGAAGACCCTGAGGTCAAGTTCAACTGGTACGTGGACGGCGTGG AGGTGCATAATGCCAAGACAAAGCCGCGGGAGGAGCAGTACAACAGCACGTACCGTGTGGTC AGCGTCCTCACCGTCCTGCACCAGGACTGGCTGAATGGCAAGGAGTACAAGTGCAAGGTCTC CAACAAAGCCCTCCCAGCCCCCATCGAGAAAACCATCTCCAAAGCCAAAGGGCAGCCCCGAG AACCACAGGTGTACACCCTGCCCCCATGCCGGGAGGAGATGACCAAGAACCAGGTCAGCCTG TGGTGCCTGGTCAAAGGCTTCTATCCCAGCGACATCGCCGTGGAGTGGGAGAGCAATGGGCA GCCGGAGAACAACTACAAGACCACGCCTCCCGTGCTGGACTCCGACGGCTCCTTCTTCCTCT ACAGCAAGCTCACCGTGGACAAGAGCAGGTGGCAGCAGGGGAACGTCTTCTCATGCTCCGTG ATGCATGAGGCTCTGCACAACCACTACACGCAGAAGAGCCTCTCCCTGTCTCCGGGTAAA | |
| EPB12 LC | GCCATACAGCTGACTCAATCACCTTCTTCTTTGAGCGCCTCTGTCGGTGATCGCGTTACAAT TACATGCCGAGCTTCTCAGTCTATCTCTAATAACCTCCACTGGTATCTCCAGAAACCTGGAC AGTCACCACAGCTTCTGATATATTACGGCTTTCAGTCTATAAGTGGAGTCCCAAGTAGATTT TCCGGTAGTGGATCTGGCACCGATTTTACTCTTACTATCTCTAGCCTCCAACCTGAAGATTT CGCAACCTATTACTGCCAGCAGGCCAATTCCTGGCCCTTGACCTTCGGAGGTGGGACTAAGC TGGAAATCAAACGTACGGTGGCAGCTCCCAGCGTTTTTATCTTTCCCCCAGACATACAACTC ACACAAAGTCCAAGCTCTCTTTCCCTTAGTCCAGGAGAACGCGTAACTCTCTCATGTAAAGC CTCCCAGGATATCAATAACTATCTCTCTTGGTACCAACAAAAGCCTGACCAAGCTCCTAAGC TGCTTATCAAACGCGCCAATCGGTTGGTAGACGGAGTTCCCGACAGGTTCAGTGGGTCAGGC TCAGGTACAGATTTTACCTTGAAGATATCACGCGTGGAAGCCGAAGACGTAGGGGTATATTA TTGTCTGAAATATGACGAGTTCCCCTATACTTTTGGGTGTGGAACTCGCCTGGAAATAAAAC GTACGGTGGCAGCTCCCAGCGTTTTTATCTTTCCCCCATCCGACGAGCAGCTCAAGAGTGGC ACTGCCTCTGTAGTTTGTTTGCTGAATAACTTCTATCCACGTGAAGCAAAAGTACAGTGGAA GGTCGATAATGCCCTTCAGAGCGGTAACAGCCAAGAAAGTGTTACCGAGCAAGATTCCAAAG ATTCCACTTACAGTCTGTCCAGCACATTGACACTGAGTAAGGCTGATTACGAAAAACACAAG GTGTACGCATGCGAGGTGACACACCAAGGTCTTTCATCTCCTGTAACTAAGAGCTTTAACCG GGGAGAATGTGGTGGTGGGGGGCAGCGGGGGGCGGAGGTAGTGGAGGCGGCGGTAGTGAACCAA AGAGTAGTGACAAAACTCACACGTGCCCACCGTGCCCAGCACCTGAACTCCTGGGGGGACCG TCAGTCTTCCTCTTCCCCCCAAAACCCAAGGACACCCTCATGATCTCCCGGACCCCTGAGGT CACATGCGTGGTGGTGGACGTGAGCCACGAAGACCCTGAGGTCAAGTTCAACTGGTACGTGG ACGGCGTGGAGGTGCATAATGCCAAGACAAAGCCGCGGGAGGAGCAGTACAACAGCACGTAC CGTGTGGTCAGCGTCCTCACCGTCCTGCACCAGGACTGGCTGAATGGCAAGGAGTACAAGTG CAAGGTCTCCAACAAAGCCCTCCCAGCCCCCATCGAGAAAACCATCTCCAAAGCCAAAGGGC AGCCCCGAGAACCACAGGTGTACACCCTGCCCCCATGCCGGGAGGAGATGACCAAGAACCAG GTCAGCCTGTGGTGCCTGGTCAAAGGCTTCTATCCCAGCGACATCGCCGTGGAGTGGGAGAG CAATGGGCAGCCGGAGAACAACTACAAGACCACGCCTCCCGTGCTGGACTCCGACGGCTCCT TCTTCCTCTACAGCAAGCTCACCGTGGACAAGAGCAGGTGGCAGCAGGGGAACGTCTTCTCA TGCTCCGTGATGCATGAGGCTCTGCACAACCACTACACGCAGAAGAGCCTCTCCCTGTCTCC GGGTAAA | 551 |

Table 54 below shows the polypeptide sequences of the heavy chain variable region (VH) and light chain variable region (VL) of the engineered antibody targeting EphA2. Table 55 below shows the polynucleotide sequences encoding the polypeptides of the heavy chain variable region (VH) and light chain variable region (VL) of the engineered antibody targeting EphA2.

[Table 54]

| Name | | Sequence | SEQ ID NO |
|---|---|---|---|
| EP801 | VH1 | EVQLLESGGGLVQPGGSLRLSCAASGFTFSRYQMWWVRQAPGKGLEWVSSISPSGGVTLYADSVKGRFTISRDNSKNTLYLQMNSLRAEDTAVYYCTRELLGTVVVPVAWKMRGYFDYWGQLTLVTVSS | 135 |
| | VH2 | EVQLLESGGGLVQPGGSLRLSCAASGFTFSHYMMAWVRQAPGKCLEWVSRIGPSGGPTHYADSVKGRFTISRDNSKNTLYLQMNSLRAEDTAVYYCAGYDSGYDYVAVAGPAEYFQHWGQGTLVTVSS | 136 |
| | VL1 | DIQMTQSPGTLSVSPGERATLSCRASQSVSSNLAWYQQKPGQAPRLLIYGASTRATGIPARFSGSGSGTEFTLTISSMQSEDFAVYYCQQYNWPPLTFGGGTKVEIK | 137 |
| | VL2 | DIQMTQSPSSLSASVGDRVTITCRASQSISTWLAWYQQKPGKAPKLLIYKASNLHTGVPSRFSGSGSGTEFSLTISGLQPDDFATYYCQQYNSYSRTFGCGTKVEIK | 138 |
| EP802 | VH1 | EVQLVESGGGVVRPGGSLRLSCAASGFTVSDYSMNWVRQAPGKGLEWIGFIRNKANAYTTEYSASVKGRFTISRDDSKNTLYLQMNSLKTEDTAVYYCTTYPRYHAMDSWGQGTMVTVSS | 139 |
| | VH2 | EVQLLESGGGLVQPGGSLRLSCAASGFTFSHYMMAWVRQAPGKCLEWVSRIGPSGGPTHYADSVKGRFTISRDNSKNTLYLQMNSLRAEDTAVYYCAGYDSGYDYVAVAGPAEYFQHWGQGTLVTVSS | 136 |
| | VL1 | AIQLTQSPSSLSASVGDRVTITCRASQSISNNLHWYLQKPGQSPQLLIYYGFQSISGVPSRFSGSGSGTDFTLTISSLQPEDFATYYCQQANSWPLTFGGGTKLEIK | 140 |
| | VL2 | DIQMTQSPSSLSASVGDRVTITCRASQSISTWLAWYQQKPGKAPKLLIYKASNLHTGVPSRFSGSGSGTEFSLTISGLQPDDFATYYCQQYNSYSRTFGCGTKVEIK | 138 |
| EP803 | VH1 | QVQLLESGGGLVQPGGSLRLSCAASGFTFSSYTMSWVRQAPGQALEWMGTISSGGTYTYYPDSVKGRFTISRDNAKNSLYLQMNSLRAEDTAVYYCAREAIFTYWGRGTLVTVSS | 141 |
| | VH2 | EVQLLESGGGLVQPGGSLRLSCAASGFTFSHYMMAWVRQAPGKCLEWVSRIGPSGGPTHYADSVKGRFTISRDNSKNTLYLQMNSLRAEDTAVYYCAGYDSGYDYVAVAGPAEYFQHWGQGTLVTVSS | 136 |
| | VL1 | DIQLTQSPSSLSLSPGERVTLSCKASQDINNYLSWYQQKPDQAPKLLIKRANRLVDGVPDRFSGSGSGTDFTLKISRVEAEDVGAYYCLKYDEFPYTFGQGTRLEIK | 142 |
| | VL2 | DIQMTQSPSSLSASVGDRVTITCRASQSISTWLAWYQQKPGKAPKLLIYKASNLHTGVPSRFSGSGSGTEFSLTISGLQPDDFATYYCQQYNSYSRTFGCGTKVEIK | 138 |
| EP804 | VH1 | EVQLLESGGGLVQPGGSLRLSCAASGFTFSHYMMAWVRQAPGKGLEWVSRIGPSGGPTHYADSVKGRFTISRDNSKNTLYLQMNSLRAEDTAVYYCAGYDSGYDYVAVAGPAEYFQHWGQGTLVTVSS | 143 |
| | VH2 | EVQLLESGGGLVQPGGSLRLSCAASGFTFSRYQMWWVRQAPGKCLEWVSSISPSGGVTLYADSVKGRFTISRDNSKNTLYLQMNSLRAEDTAVYYCTRELLGTVVVPVAWKMRGYFDYWGQLTLVTVSS | 144 |
| | VL1 | DIQMTQSPSSLSASVGDRVTITCRASQSISTWLAWYQQKPGKAPKLLIYKASNLHTGVPSRFSGSGSGTEFSLTISGLQPDDFATYYCQQYNSYSRTFGQGTKVEIK | 145 |
| | VL2 | DIQMTQSPGTLSVSPGERATLSCRASQSVSSNLAWYQQKPGQAPRLLIYGASTRATGIPARFSGSGSGTEFTLTISSMQSEDFAVYYCQQYNWPPLTFGCGTKVEIK | 146 |
| EP805 | VH1 | EVQLLESGGGLVQPGGSLRLSCAASGFTFSHYMMAWVRQAPGKGLEWVSRIGPSGGTHYADSVKGRFTISRDNSKNTLYLQMNSLRAEDTAVYYCAGYDSGYDYVAVAGPAEYFQHWGQGTLVTVSS | 143 |
| | VH2 | EVQLVESGGGVVRPGGSLRLSCAASGFTVSDYSMNWVRQAPGKCLEWIGFIRNKANAYTTEYSASVKGRFTISRDDSKNTLYLQMNSLKTEDTAVYYCTTYPRYHAMDSWGQGTMVTVSS | 147 |
| | VL1 | DIQMTQSPSSLSASVGDRVTITCRASQSISTWLAWYQQKPGKAPKLLIYKASNLHTGVPSRFSGSGSGTEFSLTISGLQPDDFATYYCQQYNSYSRTFGQGTKVEIK | 145 |
| | VL2 | AIQLTQSPSSLSASVGDRVTITCRASQSISNNLHWYLQKPGQSPQLLIYYGFQSISGVPSRFSGSGSGTDFTLTISSLQPEDFATYYCQQANSWPLTFGCGTKLEIK | 148 |
| EP806 | VH1 | EVQLLESGGGLVQPGGSLRLSCAASGFTFSHYMMAWVRQAPGKGLEWVSRIGPSGGPTHYADSVKGRFTISRDNSKNTLYLQMNSLRAEDTAVYYCAGYDSGYDYVAVAGPAEYFQHWGQGTLVTVSS | 143 |
| | VH2 | QVQLLESGGGLVQPGGSLRLSCAASGFTFSSYTMSWVRQAPGKGLEWMGTISSGGTYTYYPDSVKGRFTISRDNAKNSLYLQMNSLRAEDTAVYYCAREAIFTYWGRGTLVTVSS | 149 |

172

| | | | |
|---|---|---|---|
| | VL1 | DIQMTQSPSSLSASVGDRVTITCRASQSISTWLAWYQQKPGKAPKLLIYKASNLHTGVPSRFSGSGSGTEFSLTISGLQPDDFATYYCQQYNSYSRTFGQGTKVEIK | 145 |
| | VL2 | DIQLTQSPSSLSLSPGERVTLSCKASQDINNYLSWYQQKPDQAPKLLIKRANRLVDGVPDRFSGSGSGTDFTLKISRVEAEDVGVYYCLKYDEFPYTFGCGTRLEIK | 150 |
| EPB07 | VH1 | EVQLVESGGGVVRPGGSLRLSCAASGFTVSDYSMNWVRQAPGKGLEWIGFIRNKANAYTTEYSASVKGRFTISRDDSKNTLYLQMNSLKTEDTAVYYCTTYPRYHAMDSWGQGTMVTVSS | 139 |
| | VH2 | EVQLLESGGGLVQPGGSLRLSCAASGFTFSRYQMMWVRQAPGKCLEWVSSISPSGGVTLYADSVKGRFTISRDNSKNTLYLQMNSLRAEDTAVYYCTRELLGTVVVPVAWKMRGYFDYWGQLTLVTVSS | 144 |
| | VL1 | AIQLTQSPSSLSASVGDRVTITCRASQSISNNLHWYLQKPGQSPQLLIYYGFQSISGVPSRFSGSGSGTDFTLTISSLQPEDFATYYCQQANSWPLTFGGGTKLEIK | 140 |
| | VL2 | DIQMTQSPGTLSVSPGERATLSCRASQSVSSNLAWYQQKPGQAPRLLIYGASTRATGIPARFSGSGSGTEFTLTISSMQSEDFAVYYCQQYNNWPPLTFGCGTKVEIK | 146 |
| EPB08 | VH1 | QVQLLESGGGLVQPGGSLRLSCAASGFTFSSYTMSWVRQAPGQALEWMGTISSGGTYTYYPDSVKGRFTISRDNAKNSLYLQMNSLRAEDTAVYYCAREAIFTYWGRGTLVTVSS | 141 |
| | VH2 | EVQLLESGGGLVQPGGSLRLSCAASGFTFSRYQMMWVRQAPGKCLEWVSSISPSGGVTLYADSVKGRFTISRDNSKNTLYLQMNSLRAEDTAVYYCTRELLGTVVVPVAWKMRGYFDYWGQLTLVTVSS | 144 |
| | VL1 | DIQLTQSPSSLSLSPGERVTLSCKASQDINNYLSWYQQKPDQAPKLLIKRANRLVDGVPDRFSGSGSGTDFTLKISRVEAEDVGVYYCLKYDEFPYTFGQGTRLEIK | 142 |
| | VL2 | DIQMTQSPGTLSVSPGERATLSCRASQSVSSNLAWYQQKPGQAPRLLIYGASTRATGIPARFSGSGSGTEFTLTISSMQSEDFAVYYCQQYNNWPPLTFGCGTKVEIK | 146 |
| EPB09 | VH1 | EVQLLESGGGLVQPGGSLRLSCAASGFTFSRYQMMWVRQAPGKGLEWVSSISPSGGVTLYADSVKGRFTISRDNSKNTLYLQMNSLRAEDTAVYYCTRELLGTVVVPVAWKMRGYFDYWGQLTLVTVSS | 135 |
| | VH2 | EVQLVESGGGVVRPGGSLRLSCAASGFTVSDYSMNWVRQAPGKCLEWIGFIRNKANAYTTEYSASVKGRFTISRDDSKNTLYLQMNSLKTEDTAVYYCTTYPRYHAMDSWGQGTMVTVSS | 147 |
| | VL1 | DIQMTQSPGTLSVSPGERATLSCRASQSVSSNLAWYQQKPGQAPRLLIYGASTRATGIPARFSGSGSGTEFTLTISSMQSEDFAVYYCQQYNNWPPLTFGGGTKVEIK | 137 |
| | VL2 | AIQLTQSPSSLSASVGDRVTITCRASQSISNNLHWYLQKPGQSPQLLIYYGFQSISGVPSRFSGSGSGTDFTLTISSLQPEDFATYYCQQANSWPLTFGCGTKLEIK | 148 |
| EPB10 | VH1 | QVQLLESGGGLVQPGGSLRLSCAASGFTFSSYTMSWVRQAPGQALEWMGTISSGGTYTYYPDSVKGRFTISRDNAKNSLYLQMNSLRAEDTAVYYCAREAIFTYWGRGTLVTVSS | 141 |
| | VH2 | EVQLVESGGGVVRPGGSLRLSCAASGFTVSDYSMNWVRQAPGKCLEWIGFIRNKANAYTTEYSASVKGRFTISRDDSKNTLYLQMNSLKTEDTAVYYCTTYPRYHAMDSWGQGTMVTVSS | 147 |
| | VL1 | DIQLTQSPSSLSLSPGERVTLSCKASQDINNYLSWYQQKPDQAPKLLIKRANRLVDGVPDRFSGSGSGTDFTLKISRVEAEDVGVYYCLKYDEFPYTFGQGTRLEIK | 142 |
| | VL2 | AIQLTQSPSSLSASVGDRVTITCRASQSISNNLHWYLQKPGQSPQLLIYYGFQSISGVPSRFSGSGSGTDFTLTISSLQPEDFATYYCQQANSWPLTFGCGTKLEIK | 148 |
| EPB11 | VH1 | EVQLLESGGGLVQPGGSLRLSCAASGFTFSRYQMMWVRQAPGKGLEWVSSISPSGGVTLYADSVKGRFTISRDNSKNTLYLQMNSLRAEDTAVYYCTRELLGTVVVPVAWKMRGYFDYWGQLTLVTVSS | 135 |
| | VH2 | QVQLLESGGGLVQPGGSLRLSCAASGFTFSSYTMSWVRQAPGQCLEWMGTISSGGTYTYYPDSVKGRFTISRDNAKNSLYLQMNSLRAEDTAVYYCAREAIFTYWGRGTLVTVSS | 149 |
| | VL1 | DIQMTQSPGTLSVSPGERATLSCRASQSVSSNLAWYQQKPGQAPRLLIYGASTRATGIPARFSGSGSGTEFTLTISSMQSEDFAVYYCQQYNNWPPLTFGGGTKVEIK | 137 |
| | VL2 | DIQLTQSPSSLSLSPGERVTLSCKASQDINNYLSWYQQKPDQAPKLLIKRANRLVDGVPDRFSGSGSGTDFTLKISRVEAEDVGVYYCLKYDEFPYTFGCGTRLEIK | 150 |
| EPB12 | VH1 | EVQLVESGGGVVRPGGSLRLSCAASGFTVSDYSMNWVRQAPGKGLEWIGFIRNKANAYTTEYSA | 139 |

| | | | |
|---|---|---|---|
| | | SVKGRFTISRDDSKNTLYLQMNSLKTEDTAVYYCTTYPRYHAMDSWGQGTMVTVSS | |
| | VH2 | QVQLLESGGGLVQPGGSLRLSCAASGFTFSSYTMSWVRQAPGQCLEWMGTISSGGTYTYYPDSV KGRFTISRDNAKNSLYLQMNSLRAEDTAVYYCAREAIFTYWGRGTLVTVSS | 149 |
| | VL1 | AIQLTQSPSSLSASVGDRVTITCRASQSISNNLHWYLQKPGQSPQLLIYYGFQSISGVPSRFSG SGSGTDFTLTISSLQPEDFATYYCQQANSWPLTFGGGTKLEIK | 140 |
| | VL2 | DIQLTQSPSSLSLSPGERVTLSCKASQDINNYLSWYQQKPDQAPKLLIKRANRLVDGVPDRFSG SGSGTDFTLKISRVEAEDVGVYYCLKYDEFPYTFGCGTRLEIK | 150 |

[Table 55]

| Name | | Sequence | SEQ ID NO |
|---|---|---|---|
| EPB01 | VH1 | GAGGTGCAACTGCTTGAGTCAGGAGGAGGACTGGTTCAACCCCGGCGGGAGCCTTCGACTCTCAT GTGCAGCCTCTGGGTTTACATTTAGTCGTTACCAAATGATGTGGGTACGCCAGGCCCCCGGAAA AGGGTTTGGAGTGGGTGAGCAGCATTTCTCCTTCAGGAGGAGTGACACTTTACCGCAGACAGTGTT AAGGGTCGGTTCACAATCAGTCGTGATAATTCCAAGAACACTCTCTATCTCCAAATGAACTCCC TTCGAGCTGAGGACACTGCTGTTTATTACTGTACTCGGGAACTTCTCGGCACTGTGGTTGTTCC TGTGGCTTGGAAGATGCGTGGATACTTCGACTATTGGGGGCAACTCACTCTTGTTACCGTCTCA AGC | 552 |
| | VH2 | GAGGTTCAACTCCTTGAATCCGGAGGAGGACTTGTCCAACCAGTCGGCAGCCTTAGGCTGTCCT GCGCTGCCTCAGGCTTTACATTCAGTCATTACATGATGGCCTGGGTTAGACAGGCACCGGCAA ATGTCTGGAATGGGTCAGCCGAATAGGACCATCAGGAGGTCCCACTCACTATGCCGATTCTGTA AAAGGGAGGTTTACAATTTCCAGAGACAATTCAAAGAATACCCTTTACCTCCAGATGAACTCAT TGAGAGCCGAGGACACAGCCGTATATTATTGCGCAGGTTATGATTCCGGTTACGATTACGTTGC AGTCGCAGGCCCAGCCGAATACTTCCAACATTGGGGTCAGGGAACCCTCGTGACCGTGTCCAGT | 553 |
| | VL1 | GACATACAGATGACTCAGAGCCCTGGTACACTGAGTGTATCTCCTGGGGAACGCGCCACTCTGT CTTGCCGAGCCTCTCAAAGCGTTAGTTCCAACCTGGCTTGGTATCAGCAAAAACCCGGTCAAGC ACCTCGCTTGCTTATCTACGGGGCCTCCACACGGGCAACCGGCATTCCTGCACGCTTTAGTGGC TCTGGTAGCGGGACTGAATTCACTTTGACTATATCTTCCATGCAGAGTGAGGATTTCGCCGTTT ATTATTGTCAACAGTACAATAACTGGCCTCCACTCACTTTTGGTGGTGGCACCAAAGTGGAGAT AAAG | 554 |
| | VL2 | GACATACAGATGACACAAAGTCCAAGTAGCCTTTCTGCATCCGTTGGAGACCGCGTAACCATAA CTTGTCGAGCATCACAAAGCATAAGCACTTGGCTTGCTTGGTATCAGCAGAAGCCAGGCAAAGC ACCCAAGCTGCTGATTTATAAAGCCTCTAATCTCCATACAGGAGTTCCCAGCCCGTTTCTCAGGC AGCGGGTCTGGAACCGAATTCTCTCTGACCATCTCTGGCTTGCAACCTGATGACTTTGCAACAT ATTACTGTCAACAATATAATTCATATAGTCGTACTTTCGGGGTGTGGCACTAAGGTGGAAATCAA A | 555 |
| EPB02 | VH1 | GAGGTCCAGCTGGTTGAGTCTGGAGGTGGTGTAGTCAGGCCAGGAGGTTCCCTGCGTCTGTCTT GCGCTGCCTCTGGGTTCACTGTGTCTGACTACAGTATGAACTGGGTTAGACAGGCTCCAGGGAA AGGTTTGGAATGGATAGGGTTTATTCGCAATAAAGCCAATGCCTATACCACCGAATACAGCGCA AGCGTTAAAGGCAGGTTCACCATCAGTAGGGGACGACTCTAAGAATACCCTCTACCTGCAGATGA ATAGCTTGAAGACAGAGGATACCGCTGTTTATTACTGCACTACCTACCCCAGGTATCATGCTAT GGACAGTTGGGGTCAAGGAACTATGGTCACCGTCTCCTCT | 556 |
| | VH2 | GAGGTTCAACTCCTTGAATCCGGAGGAGGACTTGTCCAACCAGGCGGCAGCCTTAGGCTGTCCT GCGCTGCCTCAGGCTTTACATTCAGTCATTACATGATGGCCTGGGTTAGACAGGCACCCGGCAA ATGTCTGGAATGGGTCAGCCGAATAGGACCATCAGGAGGTCCCACTCACTATGCCGATTCTGTA AAAGGGAGGTTTACAATTTCCAGAGACAATTCAAAGAATACCCTTTACCTCCAGATGAACTCAT TGAGAGCCGAGGACACAGCCGTATATTATTGCGCAGGTTATGATTCCGGTTACGATTACGTTGC AGTCGCAGGCCCAGCCGAATACTTCCAACATTGGGGTCAGGGAACCCTCGTGACCGTGTCCAGT | 553 |
| | VL1 | GCCATACAGCTGACTCAATCACCTTCTTCTTTGAGCGCCTCTGTCGGTGATCGCGTTACAATTA CATGCCGAGCTTCTCAGTCTATCTCTAATAACCTCCACTGGTATCTCCAGAAACCTGGACAGTC ACCACAGCTTCTGATATATTACGGCTTTCAGTCTATAAGTGGAGTCCCAAGTAGATTTTCCGGT AGTGGATCTGGCACCGATTTTACTCTTACTATCTCTAGCCTCCAACCTGAAGATTTCGCAACCT ATTACTGCCAGCAGGCCAATTCCTGGCCCTTGACCTTCGGAGGTGGGACTAAGCTGGAAATCAA A | 557 |
| | VL2 | GACATACAGATGACACAAAGTCCAAGTAGCCTTTCTGCATCCGTTGGAGACCGCGTAACCATAA CTTGTCGAGCATCACAAAGCATAAGCACTTGGCTTGCTTGGTATCAGCAGAAGCCAGGCAAAGC ACCCAAGCTGCTGATTTATAAAGCCTCTAATCTCCATACAGGAGTTCCCAGCCCGTTTCTCAGGC AGCGGGTCTGGAACCGAATTCTCTCTGACCATCTCTGGCTTGCAACCTGATGACTTTGCAACAT ATTACTGTCAACAATATAATTCATATAGTCGTACTTTCGGGGTGTGGCACTAAGGTGGAAATCAA A | 555 |

| | | | |
|---|---|---|---|
| EPB03 | VH1 | CAAGTGCAACTCCTTGAAAGTGGAGGTGGCCTTGTCCAGCCAGGCGGTTCACTGCGTTTGAGCTGTGCCGCATCCGGCTTTACCTTTTCCAGTTACACAATGAGTTGGGTCCGTCAAGCACCCGGGCAAGCTTTGGAATGGATGGGAACTATTTCATCTGGTGGGACTTACACTTACTATCCCGATAGCGTCAAGGGACGTTTTACCATAAGCCGGGACAACGCTAAAAATTCACTCTATCTTCAGATGAACAGTCTTAGGGCTGAGGATACCGCTGTTTATTATTGCGCCCGAGAAGCCATTTTCACCTACTGGGGGCGCGGTACACTTGTCACCGTTAGCAGT | 558 |
| | VH2 | GAGGTTCAACTCCTTGAATCCGGAGGAGGACTTGTCCAACCAGGCGGCAGCCTTAGGCTGTCCTGCGCTGCCTCAGGCTTTACATTCAGTCATTACATGATGGCCTGGGTTAGACAGGCACCCGGCAAATGTCTGGAATGGGTCAGCCGAATAGGACCATCAGGAGGTCCCACTCACTATGCCGATTCTGTAAAAGGGAGGTTTACAATTTCCAGAGACAATTCAAAGAATACCCTTTACCTCCAGATGAACTCATTGAGAGCCGAGGACACAGCCGTATATTATTGCGCAGGTTATGATTCCGGTTACGATTACGTTGCAGTCGCAGGCCCAGCCGAATACTTCCAACATTGGGGTCAGGGAACCCTCGTGACCGTGTCCAGT | 553 |
| | VL1 | GACATACAACTCACACAAAGTCCAAGCTCTCTTTCCCTTAGTCCAGGAGAACGCGTAACTCTCTCATGTAAAGCCTCCCAGGATATCAATAACTATCTCTCTTGGTACCAACAAAAGCCTGACCAAGCTCCTAAGCTGCTTATCAAACGCGCCAATCGGTTGGTAGACGGAGTTCCCGACAGGTTCAGTGGGTCAGGCTCAGGTACAGATTTTACCTTGAAGATATCACGCGTGGAAGCCGAAGACGTAGGGGTATATTATTGTCTGAAATATGACGAGTTCCCCTATACTTTTGGGCAAGGAACTCGCCTGGAAATAAAA | 559 |
| | VL2 | GACATACAGATGACACAAAGTCCAAGTAGCCTTTCTGCATCCGTTGGAGACCGCGTAACCATAACTTGTCGAGCATCACAAAGCATAAGCACTTGGCTTGCTTGGTATCAGCAGAAGCCAGGCAAAGCACCCAAGCTGCTGATTTATAAAGCCTCTAATCTCCATACAGGAGTTCCCAGCCGTTTCTCAGGCAGCGGGTCTGGAACCGAATTCTCTCTGACCATCTCTGGCTTGCAACCTGATGACTTTGCAACATATTACTGTCAACAATATAATTCATATAGTCGTACTTTCGGGTGTGGCACTAAGGTGGAAATCAAA | 555 |
| EPB04 | VH1 | GAGGTACAGTTGCTGGAGTCAGGAGGTGGATTGGTCCAACCCGGAGGATCTCTTCGTCTGTCCTGCGCCGCCTCAGGATTTACCTTCTCTCATTATATGATGGCATGGGTACGTCAGGCTCCAGGCAAAGGTCTGGAATGGGTTAGTCGGATTGGTCCCTCAGGGGGTCCTACCCATTATGCCGATTCTGTAAAGGGCCGTTTTACCATAAGCAGAGACAACTCTAAGAACACCCTTTACCTTCAGATGAATAGCCTGAGGGCTGAGGATACCGCAGTGTATTACTGCGCAGGCTATGACTCTGGGTACGACTATGTCGCCGTAGCAGGACCTGCCGAGTATTTTCAACACTGGGGACAGGGGACCCTTGTCACAGTTTCTAGT | 450 |
| | VH2 | GAGGTGCAACTGCTTGAGTCAGGAGGAGGACTGGTTCAACCCGGCGGGAGCCTTCGACTCTCATGTGCAGCCTCTGGGTTTACATTTAGTCGTTACCAAATGATGTGGGTACGCCAGGCCCCCGGAAAATGTTTGGAGTGGGTGAGCAGCATTTCTCCTTCAGGAGGAGTGACACTTTACGCAGACAGTGTTAAGGGTCGGTTCACAATCAGTCGTGATAATTCCAAGAACACTCTCTATCTCCAAATGAACTCCCTTCGAGCTGAGGACACTGCTGTTTATTACTGTACTCGGGAACTTCTCGGCACTGTGGTTGTTCCTGTGGCTTGGAAGATGCGTGGATACTTCGACTATTGGGGGCAACTCACTCTTGTTACCGTCTCAAGC | 560 |
| | VL1 | GATATTCAAATGACACAAAGCCCAAGTTCCTTGTCCGCCTCAGTTGGTGATCGTGTGACAATAACCTGTCGGGCTTCACAATCCATATCTACATGGCTGGCTTGGTACCAGCAAAAGCCAGGTAAAGCCCCAAAACTCCTGATTTACAAGGCAAGTAACTTGCATACTGGGGTACCCAGCCGTTTCTCTGGGTCAGGCTCTGGGACAGAGTTTAGTCTTACAATTTCTGGTCTGCAACCCGATGACTTCGCTACCTATTACTGTCAACAATATAATAGTTATTCTCGAACATTTGGTCAGGGAACAAAAGTGGAAATCAAA | 451 |
| | VL2 | GACATACAGATGACTCAGAGCCCTGGTACACTGAGTGTATCTCCTGGGGAACGCGCCACTCTGTCTTGCCGAGCCTCTCAAAGCGTTAGTTCCAACCTGGCTTGGTATCAGCAAAAACCCGGTCAAGCACCTCGCTTGCTTATCTACGGGGCCTCCACACGGGCAACCGGCATTCCTGCACGCTTTAGTGGCTCTGGTAGCGGGACTGAATTCACTTTGACTATATCTTCCATGCAGAGTGAGGATTTCGCCGTTTATTATTGTCAACAGTACAATAACTGGCCTCCACTCACTTTTGGTTGTGGCACCAAAGTGGAGATAAAG | 561 |

| | | | |
|---|---|---|---|
| EPB05 | VH1 | GAGGTACAGTTGCTGGAGTCAGGAGGTGGATTGGTCCAACCCGGAGGATCTCTTCGTCTGTCCTGCGCCGCCTCAGGATTTACCTTCTCTCATTATATGATGGCATGGGTACGTCAGGCTCCAGGCAAAGGTCTGGAATGGGTTAGTCGGATTGGTCCCTCAGGGGGTCCTACCCATTATGCCGATTCTGTAAAGGGCCGTTTTACCATAAGCAGAGACAACTCTAAGAACACCCTTTACCTTCAGATGAATAGCCTGAGGGCTGAGGATACCGCAGTGTATTACTGCGCAGGCTATGACTCTGGGTACGACTATGTCGCCGTAGCAGGACCTGCCGAGTATTTTCAACACTGGGGACAGGGGACCCTTGTCACAGTTTCTAGT | 450 |
| | VH2 | GAGGTCCAGCTGGTTGAGTCTGGAGGTGGTGTAGTCAGGCCAGGAGGTTCCCTGCGTCTGTCTTGCGCTGCCTCTGGGTTCACTGTGTCTGACTACAGTATGAACTGGGTTAGACAGGCTCCAGGGAAATGTTTGGAATGGATAGGGTTTATTCGCAATAAAGCCAATGCCTATACCACCGAATACAGCGCAAGCGTTAAAGGCAGGTTCACCATCAGTAGGGACGACTCTAAGAATACCCTCTACCTGCAGATGAATAGCTTGAAGACAGAGGATACCGCTGTTTATTACTGCACTACCTACCCCAGGTATCATGCTATGGACAGTTGGGGTCAAGGAACTATGGTCACCGTCTCCTCT | 562 |
| | VL1 | GATATTCAAATGACACAAAGCCCAAGTTCCTTGTCCGCCTCAGTTGGTGATCGTGTGACAATAACCTGTCGGGCTTCACAATCCATATCTACATGGCTGGCTTGGTACCAGCAAAAGCCAGGTAAAGCCCCAAAACTCCTGATTTACAAGGCAAGTAACTTGCATACTGGGGTACCCAGCCGTTTCTCTGGGTCAGGCTCTGGGACAGAGTTTAGTCTTACAATTTCTGGTCTGCAACCCGATGACTTCGCTACCTATTACTGTCAACAATATAATAGTTATTCTCGAACATTTGGTCAGGGAACAAAAGTGGAAATCAAA | 451 |
| | VL2 | GCCATACAGCTGACTCAATCACCTTCTTCTTTGAGCGCCTCTGTCGGTGATCGCGTTACAATTACATGCCGAGCTTCTCAGTCTATCTCTAATAACCTCCACTGGTATCTCCAGAAACCTGGACAGTCACCACAGCTTCTGATATATTACGGCTTTCAGTCTATAAGTGGAGTCCCAAGTAGATTTTCCGGTAGTGGATCTGGCACCGATTTTACTCTTACTATCTCTAGCCTCCAACCTGAAGATTTCGCAACCTATTACTGCCAGCAGGCCAATTCCTGGCCCTTGACCTTCGGATGTGGGACTAAGCTGGAAATCAAA | 563 |
| EPB06 | VH1 | GAGGTACAGTTGCTGGAGTCAGGAGGTGGATTGGTCCAACCCGGAGGATCTCTTCGTCTGTCCTGCGCCGCCTCAGGATTTACCTTCTCTCATTATATGATGGCATGGGTACGTCAGGCTCCAGGCAAAGGTCTGGAATGGGTTAGTCGGATTGGTCCCTCAGGGGGTCCTACCCATTATGCCGATTCTGTAAAGGGCCGTTTTACCATAAGCAGAGACAACTCTAAGAACACCCTTTACCTTCAGATGAATAGCCTGAGGGCTGAGGATACCGCAGTGTATTACTGCGCAGGCTATGACTCTGGGTACGACTATGTCGCCGTAGCAGGACCTGCCGAGTATTTTCAACACTGGGGACAGGGGACCCTTGTCACAGTTTCTAGT | 450 |
| | VH2 | CAAGTGCAACTCCTTGAAAGTGGAGGTGGCCTTGTCCAGCCAGGCGGTTCACTGCGTTTGAGCTGTGCCGCATCCGGCTTTACCTTTTCCAGTTACACAATGAGTTGGGTCCGTCAAGCACCCGGGCAATGTTTGGAATGGATGGGAACTATTTCATCTGGTGGGACTTACACTTACTATCCCGATAGCGTCAAGGGACGTTTTACCATAAGCCGGGACAACGCTAAAAATTCACTCTATCTTCAGATGAACAGTCTTAGGGCTGAGGATACCGCTGTTTATTATTGCGCCCGAGAAGCCATTTTCACCTACTGGGGGCGCGGTACACTTGTCACCGTTAGCAGT | 564 |
| | VL1 | GATATTCAAATGACACAAAGCCCAAGTTCCTTGTCCGCCTCAGTTGGTGATCGTGTGACAATAACCTGTCGGGCTTCACAATCCATATCTACATGGCTGGCTTGGTACCAGCAAAAGCCAGGTAAAGCCCCAAAACTCCTGATTTACAAGGCAAGTAACTTGCATACTGGGGTACCCAGCCGTTTCTCTGGGTCAGGCTCTGGGACAGAGTTTAGTCTTACAATTTCTGGTCTGCAACCCGATGACTTCGCTACCTATTACTGTCAACAATATAATAGTTATTCTCGAACATTTGGTCAGGGAACAAAAGTGGAAATCAAA | 451 |
| | VL2 | GACATACAACTCACACAAAGTCCAAGCTCTCTTTCCCTTAGTCCAGGAGAACGCGTAACTCTCTCATGTAAAGCCTCCCAGGATATCAATAACTATCTCTCTTGGTACCAACAAAAGCCTGACCAAGCTCCTAAGCTGCTTATCAAACGCGCCAATCGGTTGGTAGACGGAGTTCCCGACAGGTTCAGTGGGTCAGGCTCAGGTACAGATTTTACCTTGAAGATATCACGCGTGGAAGCCGAAGACGTAGGGGTATATTATTGTCTGAAATATGACGAGTTCCCCTATACTTTTGGGTGTGGAACTCGCCTGGAAATAAAA | 565 |
| EPB07 | VH1 | GAGGTCCAGCTGGTTGAGTCTGGAGGTGGTGTAGTCAGGCCAGGAGGTTCCCTGCGTCTGTCTT | 556 |

| | | | |
|---|---|---|---|
| | | GCGCTGCCTCTGGGTTCACTGTGTCTGACTACAGTATGAACTGGGTTAGACAGGCTCCAGGGAA AGGTTTGGAATGGATAGGGTTTATTCGCAATAAAGCCAATGCCTATACCACCGAATACAGCGCA AGCGTTAAAGGCAGGTTCACCATCAGTAGGGACGACTCTAAGAATACCCTCTACCTGCAGATGA ATAGCTTGAAGACAGAGGATACCGCTGTTTATTACTGCACTACCTACCCCAGGTATCATGCTAT GGACAGTTGGGGTCAAGGAACTATGGTCACCGTCTCCTCT | |
| | VH2 | GAGGTGCAACTGCTTGAGTCAGGAGGAGGACTGGTTCAACCCGGCGGGAGCCTTCGACTCTCAT GTGCAGCCTCTGGGTTACATTTAGTCGTTACCAAATGATGTGGGTACGCCAGGCCCCCGGAAA ATGTTTGGAGTGGGTGAGCAGCATTTCTCCTTCAGGAGGAGTGACACTTTACGCAGACAGTGTT AAGGGTCGGTTCACAATCAGTCGTGATAATTCCAAGAACACTCTCTATCTCCAAATGAACTCCC TTCGAGCTGAGGACACTGCTGTTTATTACTGTACTCGGGAACTTCTCGGCACTGTGGTTGTTCC TGTGGCTTGGAAGATGCGTGGATACTTCGACTATTGGGGGCAACTCACTCTTGTTACCGTCTCA AGC | 560 |
| | VL1 | GCCATACAGCTGACTCAATCACCTTCTTCTTTGAGCGCCTCTGTCGGTGATCGCGTTACAATTA CATGCCGAGCTTCTCAGTCTATCTAATAACCTCCACTGGTATCTCCAGAAACCTGGACAGTC ACCACAGCTTCTGATATATTACGGCTTTCAGTCTATAAGTGGAGTCCCAAGTAGATTTTCCGGT AGTGGATCTGGCACCGATTTTACTCTTACTATCTCTAGCCTCCAACCTGAAGATTTCGCAACCT ATTACTGCCAGCAGGCCAATTCCTGGCCCTTGACCTTCGGAGGTGGGACTAAGCTGGAAATCAA A | 557 |
| | VL2 | GACATACAGATGACTCAGAGCCCTGGTACACTGAGTGTATCTCCTGGGGAACGCGCCACTCTGT CTTGCCGAGCCTCTCAAAGCGTTAGTTCCAACCTGGCTTGGTATCAGCAAAAACCCGGTCAAGC ACCTCGCTTGCTTATCTACGGGGCCTCCACACGGGCAACCGGCATTCCTGCACGCTTTAGTGGC TCTGGTAGCGGGACTGAATTCACTTTGACTATATCTTCCATGCAGAGTGAGGATTTCGCCGTTT ATTATTGTCAACAGTACAATAACTGGCCTCCACTCACTTTTGGTTGTGGCACCAAAGTGGAGAT AAAG | 561 |
| EPB08 | VH1 | CAAGTGCAACTCCTTGAAAGTGGAGGTGGCCTTGTCCAGCCAGGCGGTTCACTGCGTTTGAGCT GTGCCGCATCCGGCTTTACCTTTTCCAGTTACACAATGAGTTGGGTCCGTCAAGCACCCGGGCA AGCTTTGGAATGGATGGGAACTATTTCATCTGGTGGGACTTACACTTACTATCCCGATAGCGTC AAGGGACGTTTTACCATAAGCCGGGACAACGCTAAAAATTCACTCTATCTTCAGATGAACAGTC TTAGGGCTGAGGATACCGCTGTTTATTATTGCGCCCGAGAAGCCATTTTCACCTACTGGGGGCG CGGTACACTTGTCACCGTTAGCAGT | 558 |
| | VH2 | GAGGTGCAACTGCTTGAGTCAGGAGGAGGACTGGTTCAACCCGGCGGGAGCCTTCGACTCTCAT GTGCAGCCTCTGGGTTACATTTAGTCGTTACCAAATGATGTGGGTACGCCAGGCCCCCGGAAA ATGTTTGGAGTGGGTGAGCAGCATTTCTCCTTCAGGAGGAGTGACACTTTACGCAGACAGTGTT AAGGGTCGGTTCACAATCAGTCGTGATAATTCCAAGAACACTCTCTATCTCCAAATGAACTCCC TTCGAGCTGAGGACACTGCTGTTTATTACTGTACTCGGGAACTTCTCGGCACTGTGGTTGTTCC TGTGGCTTGGAAGATGCGTGGATACTTCGACTATTGGGGGCAACTCACTCTTGTTACCGTCTCA AGC | 560 |
| | VL1 | GACATACAACTCACACAAAGTCCAAGCTCTCTTTCCCTTAGTCCAGGAGAACGCGTAACTCTCT CATGTAAAGCCTCCCAGGATATCAATAACTATCTCTCTTGGTACCAACAAAAGCCTGACCAAGC TCCTAAGCTGCTTATCAAACGCGCCAATCGGTTGGTAGACGGAGTTCCCGACAGGTTCAGTGGG TCAGGCTCAGGTACAGATTTTACCTTGAAGTATATCACGCGTGGAAGCCGAAGACGTAGGGGTAT ATTATTGTCTGAAATATGACGAGTTCCCCTATACTTTTGGGCAAGGAACTCGCCTGGAAATAAA A | 559 |
| | VL2 | GACATACAGATGACTCAGAGCCCTGGTACACTGAGTGTATCTCCTGGGGAACGCGCCACTCTGT CTTGCCGAGCCTCTCAAAGCGTTAGTTCCAACCTGGCTTGGTATCAGCAAAAACCCGGTCAAGC ACCTCGCTTGCTTATCTACGGGGCCTCCACACGGGCAACCGGCATTCCTGCACGCTTTAGTGGC TCTGGTAGCGGGACTGAATTCACTTTGACTATATCTTCCATGCAGAGTGAGGATTTCGCCGTTT ATTATTGTCAACAGTACAATAACTGGCCTCCACTCACTTTTGGTTGTGGCACCAAAGTGGAGAT AAAG | 561 |

178

| | | | |
|---|---|---|---|
| EPB09 | VH1 | GAGGTGCAACTGCTTGAGTCAGGAGGAGGACTGGTTCAACCCGGCGGGAGCCTTCGACTCTCAT GTGCAGCCTCTGGGTTTACATTTAGTCGTTACCAAATGATGTGGGTACGCCAGGCCCCCGGAAA AGGTTTGGAGTGGGTGAGCAGCATTTCTCCTTCAGGAGGAGTGACACTTTACGCAGACAGTGTT AAGGGTCGGTTCACAATCAGTCGTGATAATTCCAAGAACACTCTCTATCTCCAAATGAACTCCC TTCGAGCTGAGGACACTGCTGTTTATTACTGTACTCGGGAACTTCTCGGCACTGTGGTTGTTCC TGTGGCTTGGAAGATGCGTGGATACTTCGACTATTGGGGGCAACTCACTCTTGTTACCGTCTCA AGC | 552 |
| | VH2 | GAGGTCCAGCTGGTTGAGTCTGGAGGTGGTGTAGTCAGGCCAGGAGGTTCCCTGCGTCTGTCTT GCGCTGCCTCTGGGTTCACTGTGTCTGACTACAGTATGAACTGGGTTAGACAGGCTCCAGGGAA ATGTTTGGAATGGATAGGGTTTATTCGCAATAAAGCCAATGCCTATACCACCGAATACAGCGCA AGCGTTAAAGGCAGGTTCACCATCAGTAGGGACGACTCTAAGAATACCCTCTACCTGCAGATGA ATAGCTTGAAGACAGAGGATACCGCTGTTTATTACTGCACTACCTACCCCAGGTATCATGCTAT GGACAGTTGGGGTCAAGGAACTATGGTCACCGTCTCCTCT | 562 |
| | VL1 | GACATACAGATGACTCAGAGCCCTGGTACACTGAGTGTATCTCCTGGGGAACGCGCCACTCTGT CTTGCCGAGCCTCTCAAAGCGTTAGTTCCAACCTGGCTTGGTATCAGCAAAAACCCGGTCAAGC ACCTCGCTTGCTTATCTACGGGGCCTCCACACGGGCAACCGGCATTCCTGCACGCTTTAGTGGC TCTGGTAGCGGGACTGAATTCACTTTGACTATATCTTCCATGCAGAGTGAGGATTTCGCCGTTT ATTATTGTCAACAGTACAATAACTGGCCTCCACTCACTTTTGGTGGTGGCACCAAAGTGGAGAT AAAG | 554 |
| | VL2 | GCCATACAGCTGACTCAATCACCTTCTTCTTTGAGCGCCTCTGTCGGTGATCGCGTTACAATTA CATGCCGAGCTTCTCAGTCTATCTCTAATAACCTCCACTGGTATCTCCAGAAACCTGGACAGTC ACCACAGCTTCTGATATATTACGGCTTTCAGTCTATAAGTGGAGTCCCAAGTAGATTTTCCGGT AGTGGATCTGGCACCGATTTTACTCTTACTATCTCTAGCCTCCAACCTGAAGATTTCGCAACCT ATTACTGCCAGCAGGCCAATTCCTGGCCCTTGACCTTCGGATGTGGGACTAAGCTGGAAATCAA A | 563 |
| EPB10 | VH1 | CAAGTGCAACTCCTTGAAAGTGGAGGTGGCCTTGTCCAGCCAGGCGGTTCACTGCGTTTGAGCT GTGCCGCATCCGGCTTTACCTTTTCCAGTTACACAATGAGTTGGGTCCGTCAAGCACCCGGGCA AGCTTTGGAATGGATGGGAACTATTTCATCTGGTGGGACTTACACTTACTATCCCGATAGCGTC AAGGGACGTTTTACCATAAGCCGGGACAACGCTAAAAATTCACTCTATCTTCAGATGAACAGTC TTAGGGCTGAGGATACCGCTGTTTATTATTGCGCCCGAGAAGCCATTTTCACCTACTGGGGGCG CGGTACACTTGTCACCGTTAGCAGT | 558 |
| | VH2 | GAGGTCCAGCTGGTTGAGTCTGGAGGTGGTGTAGTCAGGCCAGGAGGTTCCCTGCGTCTGTCTT GCGCTGCCTCTGGGTTCACTGTGTCTGACTACAGTATGAACTGGGTTAGACAGGCTCCAGGGAA ATGTTTGGAATGGATAGGGTTTATTCGCAATAAAGCCAATGCCTATACCACCGAATACAGCGCA AGCGTTAAAGGCAGGTTCACCATCAGTAGGGACGACTCTAAGAATACCCTCTACCTGCAGATGA ATAGCTTGAAGACAGAGGATACCGCTGTTTATTACTGCACTACCTACCCCAGGTATCATGCTAT GGACAGTTGGGGTCAAGGAACTATGGTCACCGTCTCCTCT | 562 |
| | VL1 | GACATACAACTCACACAAAGTCCAAGCTCTCTTTCCCTTAGTCCAGGAGAACGCGTAACTCTCT CATGTAAAGCCTCCCAGGATATCAATAACTATCTCTCTTGGTACCAACAAAAGCCTGACCAAGC TCCTAAGCTGCTTATCAAACGCGCCAATCGGTTGGTAGACGGAGTTCCCGACAGGTTCAGTGGG TCAGGCTCAGGTACAGATTTTACCTTGAAGATATCACGCGTGGAAGCCGAAGACGTAGGGGTAT ATTATTGTCTGAAATATGACGAGTTCCCCTATACTTTTGGGCAAGGAACTCGCCTGGAAATAAA A | 559 |
| | VL2 | GCCATACAGCTGACTCAATCACCTTCTTCTTTGAGCGCCTCTGTCGGTGATCGCGTTACAATTA CATGCCGAGCTTCTCAGTCTATCTCTAATAACCTCCACTGGTATCTCCAGAAACCTGGACAGTC ACCACAGCTTCTGATATATTACGGCTTTCAGTCTATAAGTGGAGTCCCAAGTAGATTTTCCGGT AGTGGATCTGGCACCGATTTTACTCTTACTATCTCTAGCCTCCAACCTGAAGATTTCGCAACCT ATTACTGCCAGCAGGCCAATTCCTGGCCCTTGACCTTCGGATGTGGGACTAAGCTGGAAATCAA A | 563 |

| | | | |
|---|---|---|---|
| EPB11 | VH1 | GAGGTGCAACTGCTTGAGTCAGGAGGAGGACTGGTTCAACCCGGCGGGAGCCTTCGACTCTCAT GTGCAGCCTCTGGGTTTACATTTAGTCGTTACCAAATGATGTGGGTACGCCAGGCCCCCGGAAA AGGTTTGGAGTGGGTGAGCAGCATTTCTCCTTCAGGAGGAGTGACACTTTACGCAGACAGTGTT AAGGGTCGGTTCACAATCAGTCGTGATAATTCCAAGAACACTCTCTATCTCCAAATGAACTCCC TTCGAGCTGAGGACACTGCTGTTTATTACTGTACTCGGGAACTTCTCGGCACTGTGGTTGTTCC TGTGGCTTGGAAGATGCGTGGATACTTCGACTATTGGGGGCAACTCACTCTTGTTACCGTCTCA AGC | 552 |
| | VH2 | CAAGTGCAACTCCTTGAAAGTGGAGGTGGCCTTGTCCAGCCAGGCGGTTCACTGCGTTTGAGCT GTGCCGCATCCGGCTTTACCTTTTCCAGTTACACAATGAGTTGGGTCCGTCAAGCACCCGGGCA ATGTTTGGAATGGATGGGAACTATTTCATCTGGTGGGACTTACACTTACTATCCCGATAGCGTC AAGGGACGTTTTACCATAAGCCGGGACAACGCTAAAAAATTCACTCTATCTTCAGATGAACAGTC TTAGGGCTGAGGATACCGCTGTTTATTATTGCGCCCGAGAAGCCATTTTCACCTACTGGGGGCG CGGTACACTTGTCACCGTTAGCAGT | 564 |
| | VL1 | GACATACAGATGACTCAGAGCCCTGGTACACTGAGTGTATCTCCTGGGGAACGCGCCACTCTGT CTTGCCGAGCCTCTCAAAGCGTTAGTTCCAACCTGGCTTGGTATCAGCAAAAACCCGGTCAAGC ACCTCGCTTGCTTATCTACGGGGCCTCCACACGGGCAACCGGCATTCCTGCACGCTTTAGTGGC TCTGGTAGCGGGACTGAATTCACTTTGACTATATCTTCCATGCAGAGTGAGGATTTCGCCGTTT ATTATTGTCAACAGTACAATAACTGGCCTCCACTCACTTTTGGTGGTGGCACCAAAGTGGAGAT AAAG | 554 |
| | VL2 | GACATACAACTCACACAAAGTCCAAGCTCTCTTTCCCTTAGTCCAGGAGAACGCGTAACTCTCT CATGTAAAGCCTCCCAGGATATCAATAACTATCTCTCTTGGTACCAACAAAAGCCTGACCAAGC TCCTAAGCTGCTTATCAAACGCGCCAATCGGTTGGTAGACGGGAGTTCCCGACAGGTTCAGTGGG TCAGGCTCAGGTACAGATTTTTACCTTGAAGATATCACGCGTGGAAGCCGAAGACGTAGGGGTAT ATTATTGTCTGAAATATGACGAGTTCCCCTATACTTTTGGGTGTGGAACTCGCCTGGAAATAAA A | 565 |
| EPB12 | VH1 | GAGGTCCAGCTGGTTGAGTCTGGAGGTGGTGTAGTCAGGCCAGGAGGTTCCCTGCGTCTGTCTT GCGCTGCCTCTGGGTTCACTGTGTCTGACTACAGTATGAACTGGGTTAGACAGGCTCCAGGGAA AGGTTTGGAATGGATAGGGTTTATTCGCAATAAAGCCAATGCCTATACCACCGAATACAGCGCA AGCGTTAAAGGCAGGTTCACCATCAGTAGGGACGACTCTAAGAATACCCTCTACCTGCAGATGA ATAGCTTGAAGACAGAGGATACCGCTGTTTATTACTGCACTACCTACCCCCAGGTATCATGCTAT GGACAGTTGGGGTCAAGGAACTATGGTCACCGTCTCCTCT | 556 |
| | VH2 | CAAGTGCAACTCCTTGAAAGTGGAGGTGGCCTTGTCCAGCCAGGCGGTTCACTGCGTTTGAGCT GTGCCGCATCCGGCTTTACCTTTTCCAGTTACACAATGAGTTGGGTCCGTCAAGCACCCGGGCA ATGTTTGGAATGGATGGGAACTATTTCATCTGGTGGGACTTACACTTACTATCCCGATAGCGTC AAGGGACGTTTTACCATAAGCCGGGACAACGCTAAAAAATTCACTCTATCTTCAGATGAACAGTC TTAGGGCTGAGGATACCGCTGTTTATTATTGCGCCCGAGAAGCCATTTTCACCTACTGGGGGCG CGGTACACTTGTCACCGTTAGCAGT | 564 |
| | VL1 | GCCATACAGCTGACTCAATCACCTTCTTCTTTGAGCGCCTCTGTCGGTGATCGCGTTACAATTA CATGCCGAGCTTCTCAGTCTATCTCTAATAACCTCCACTGGTATCTCCAGAAACCTGGACAGTC ACCACAGCTTCTGATATATTACGGCTTTCAGTCTATAAGTGGAGTCCCAAGTAGATTTTCCGGT AGTGGATCTGGCACCGATTTTACTCTTACTATCTCTAGCCTCCAACCTGAAGATTTCGCAACCT ATTACTGCCAGCAGGCCAATTCCTGGCCCTTGACCTTCGGAGGTGGGACTAAGCTGGAAATCAA A | 557 |
| | VL2 | GACATACAACTCACACAAAGTCCAAGCTCTCTTTCCCTTAGTCCAGGAGAACGCGTAACTCTCT CATGTAAAGCCTCCCAGGATATCAATAACTATCTCTCTTGGTACCAACAAAAGCCTGACCAAGC TCCTAAGCTGCTTATCAAACGCGCCAATCGGTTGGTAGACGGGAGTTCCCGACAGGTTCAGTGGG TCAGGCTCAGGTACAGATTTTTACCTTGAAGATATCACGCGTGGAAGCCGAAGACGTAGGGGTAT ATTATTGTCTGAAATATGACGAGTTCCCCTATACTTTTGGGTGTGGAACTCGCCTGGAAATAAA A | 565 |

Table 56 below shows the CDR sequences of the heavy chain and light chain of the engineered antibodies targeting EphA2.

[Table 56]

| Name | CDR | Sequence | SEQ ID NO |
|---|---|---|---|
| EPB01 | V1 CDR-H1 | RYQMM | 151 |
| | V1 CDR-H2 | SISPSGGVTLYADSVKG | 152 |
| | V1 CDR-H3 | ELLGTVVVPVAWKMRGYFDY | 153 |
| | V1 CDR-L1 | RASQSVSSNLA | 154 |
| | V1 CDR-L2 | GASTRAT | 155 |
| | V1 CDR-L3 | QQYNMPPLT | 156 |
| | V2 CDR-H1 | HYMMA | 157 |
| | V2 CDR-H2 | RIGPSGGPTHYADSVKG | 158 |
| | V2 CDR-H3 | YDSGYDYVAVAGPAEYFQH | 159 |
| | V2 CDR-L1 | RASQSISTWLA | 160 |
| | V2 CDR-L2 | KASNLHT | 161 |
| | V2 CDR-L3 | QQYNSYSRT | 162 |
| EPB02 | V1 CDR-H1 | DYSMN | 163 |
| | V1 CDR-H2 | FIRNKANAYTTEYSASVKG | 164 |
| | V1 CDR-H3 | YPRYHAMDSW | 165 |
| | V1 CDR-L1 | RASQSISNNLH | 166 |
| | V1 CDR-L2 | YGFQSIS | 167 |
| | V1 CDR-L3 | QQANSWPLT | 168 |
| | V2 CDR-H1 | HYMMA | 157 |
| | V2 CDR-H2 | RIGPSGGPTHYADSVKG | 158 |
| | V2 CDR-H3 | YDSGYDYVAVAGPAEYFQH | 159 |
| | V2 CDR-L1 | RASQSISTWLA | 160 |
| | V2 CDR-L2 | KASNLHT | 161 |
| | V2 CDR-L3 | QQYNSYSRT | 162 |
| EPB03 | V1 CDR-H1 | SYTMS | 169 |
| | V1 CDR-H2 | TISSGGTYTYYPDSVKG | 170 |
| | V1 CDR-H3 | EAIFTYW | 171 |
| | V1 CDR-L1 | KASQDINNYLS | 172 |
| | V1 CDR-L2 | RANRLVD | 173 |
| | V1 CDR-L3 | LKYDEFPYT | 174 |
| | V2 CDR-H1 | HYMMA | 157 |
| | V2 CDR-H2 | RIGPSGGPTHYADSVKG | 158 |
| | V2 CDR-H3 | YDSGYDYVAVAGPAEYFQH | 159 |
| | V2 CDR-L1 | RASQSISTWLA | 160 |
| | V2 CDR-L2 | KASNLHT | 161 |
| | V2 CDR-L3 | QQYNSYSRT | 162 |
| EPB04 | V1 CDR-H1 | HYMMA | 157 |
| | V1 CDR-H2 | RIGPSGGPTHYADSVKG | 158 |
| | V1 CDR-H3 | YDSGYDYVAVAGPAEYFQH | 159 |
| | V1 CDR-L1 | RASQSISTWLA | 160 |
| | V1 CDR-L2 | KASNLHT | 161 |
| | V1 CDR-L3 | QQYNSYSRT | 162 |
| | V2 CDR-H1 | RYQMM | 151 |
| | V2 CDR-H2 | SISPSGGVTLYADSVKG | 152 |

| | V2 CDR-H3 | ELLGTVVVPVAWKMRGYFDY | 153 |
|---|---|---|---|
| | V2 CDR-L1 | RASQSVSSNLA | 154 |
| | V2 CDR-L2 | GASTRAT | 155 |
| | V2 CDR-L3 | QQYNNWPPLT | 156 |
| EPB05 | V1 CDR-H1 | HYMMA | 157 |
| | V1 CDR-H2 | RIGPSGGPTHYADSVKG | 158 |
| | V1 CDR-H3 | YDSGYDYVAVAGPAEYFQH | 159 |
| | V1 CDR-L1 | RASQSISTWLA | 160 |
| | V1 CDR-L2 | KASNLHT | 161 |
| | V1 CDR-L3 | QQYNSYSRT | 162 |
| | V2 CDR-H1 | DYSMN | 163 |
| | V2 CDR-H2 | FIRNKANAYTTEYSASVKG | 164 |
| | V2 CDR-H3 | YPRYHAMDSW | 165 |
| | V2 CDR-L1 | RASQSISNNLH | 166 |
| | V2 CDR-L2 | YGFQSIS | 167 |
| | V2 CDR-L3 | QQANSWPLT | 168 |
| EPB06 | V1 CDR-H1 | HYMMA | 157 |
| | V1 CDR-H2 | RIGPSGGPTHYADSVKG | 158 |
| | V1 CDR-H3 | YDSGYDYVAVAGPAEYFQH | 159 |
| | V1 CDR-L1 | RASQSISTWLA | 160 |
| | V1 CDR-L2 | KASNLHT | 161 |
| | V1 CDR-L3 | QQYNSYSRT | 162 |
| | V2 CDR-H1 | SYTMS | 169 |
| | V2 CDR-H2 | TISSGGTYTYYPDSVKG | 170 |
| | V2 CDR-H3 | EAIFTYW | 171 |
| | V2 CDR-L1 | KASQDINNYLS | 172 |
| | V2 CDR-L2 | RANRLVD | 173 |
| | V2 CDR-L3 | LKYDEFPYT | 174 |
| EPB07 | V1 CDR-H1 | DYSMN | 163 |
| | V1 CDR-H2 | FIRNKANAYTTEYSASVKG | 164 |
| | V1 CDR-H3 | YPRYHAMDSW | 165 |
| | V1 CDR-L1 | RASQSISNNLH | 166 |
| | V1 CDR-L2 | YGFQSIS | 167 |
| | V1 CDR-L3 | QQANSWPLT | 168 |
| | V2 CDR-H1 | RYQMM | 151 |
| | V2 CDR-H2 | SISPSGGVTLYADSVKG | 152 |
| | V2 CDR-H3 | ELLGTVVVPVAWKMRGYFDY | 153 |
| | V2 CDR-L1 | RASQSVSSNLA | 154 |
| | V2 CDR-L2 | GASTRAT | 155 |
| | V2 CDR-L3 | QQYNNWPPLT | 156 |
| EPB08 | V1 CDR-H1 | SYTMS | 169 |
| | V1 CDR-H2 | TISSGGTYTYYPDSVKG | 170 |
| | V1 CDR-H3 | EAIFTYW | 171 |
| | V1 CDR-L1 | KASQDINNYLS | 172 |
| | V1 CDR-L2 | RANRLVD | 173 |
| | V1 CDR-L3 | LKYDEFPYT | 174 |
| | V2 CDR-H1 | RYQMM | 151 |
| | V2 CDR-H2 | SISPSGGVTLYADSVKG | 152 |

| | V2 CDR-H3 | ELLGTVVVPVAWKMRGYFDY | 153 |
|---|---|---|---|
| | V2 CDR-L1 | RASQSVSSNLA | 154 |
| | V2 CDR-L2 | GASTRAT | 155 |
| | V2 CDR-L3 | QQYNNWPPLT | 156 |
| EPB09 | V1 CDR-H1 | RYQMM | 151 |
| | V1 CDR-H2 | SISPSGGVTLYADSVKG | 152 |
| | V1 CDR-H3 | ELLGTVVVPVAWKMRGYFDY | 153 |
| | V1 CDR-L1 | RASQSVSSNLA | 154 |
| | V1 CDR-L2 | GASTRAT | 155 |
| | V1 CDR-L3 | QQYNNWPPLT | 156 |
| | V2 CDR-H1 | DYSMN | 163 |
| | V2 CDR-H2 | FIRNKANAYTTEYSASVKG | 164 |
| | V2 CDR-H3 | YPRYHAMDSW | 165 |
| | V2 CDR-L1 | RASQSISNNLH | 166 |
| | V2 CDR-L2 | YGFQSIS | 167 |
| | V2 CDR-L3 | QQANSWPLT | 168 |
| EPB10 | V1 CDR-H1 | SYTMS | 169 |
| | V1 CDR-H2 | TISSGGTYTYYPDSVKG | 170 |
| | V1 CDR-H3 | EAIFTYW | 171 |
| | V1 CDR-L1 | KASQDINNYLS | 172 |
| | V1 CDR-L2 | RANRLVD | 173 |
| | V1 CDR-L3 | LKYDEFPYT | 174 |
| | V2 CDR-H1 | DYSMN | 163 |
| | V2 CDR-H2 | FIRNKANAYTTEYSASVKG | 164 |
| | V2 CDR-H3 | YPRYHAMDSW | 165 |
| | V2 CDR-L1 | RASQSISNNLH | 166 |
| | V2 CDR-L2 | YGFQSIS | 167 |
| | V2 CDR-L3 | QQANSWPLT | 168 |
| EPB11 | V1 CDR-H1 | RYQMM | 151 |
| | V1 CDR-H2 | SISPSGGVTLYADSVKG | 152 |
| | V1 CDR-H3 | ELLGTVVVPVAWKMRGYFDY | 153 |
| | V1 CDR-L1 | RASQSVSSNLA | 154 |
| | V1 CDR-L2 | GASTRAT | 155 |
| | V1 CDR-L3 | QQYNNWPPLT | 156 |
| | V2 CDR-H1 | SYTMS | 169 |
| | V2 CDR-H2 | TISSGGTYTYYPDSVKG | 170 |
| | V2 CDR-H3 | EAIFTYW | 171 |
| | V2 CDR-L1 | KASQDINNYLS | 172 |
| | V2 CDR-L2 | RANRLVD | 173 |
| | V2 CDR-L3 | LKYDEFPYT | 174 |
| EPB12 | V1 CDR-H1 | DYSMN | 163 |
| | V1 CDR-H2 | FIRNKANAYTTEYSASVKG | 164 |
| | V1 CDR-H3 | YPRYHAMDSW | 165 |
| | V1 CDR-L1 | RASQSISNNLH | 166 |
| | V1 CDR-L2 | YGFQSIS | 167 |
| | V1 CDR-L3 | QQANSWPLT | 168 |
| | V2 CDR-H1 | SYTMS | 169 |
| | V2 CDR-H2 | TISSGGTYTYYPDSVKG | 170 |

| V2 CDR-H3 | EAIFTYW | 171 |
| V2 CDR-L1 | KASQDINNYLS | 172 |
| V2 CDR-L2 | RANRLVD | 173 |
| V2 CDR-L3 | LKYDEFPYT | 174 |

The EphA2 protein binding constants of each of EPB01, EPB02, EPB03, EPB04, EPB05, EPB06, EPB07, EPB08, EPB09, EPB10, EPB11, and EPB12 were determined using Octet Red96e. The human EphA2 recombinant protein (Sino Biologicals, 13926-H08H) was loaded onto the Anti-Penta-HIS (HIS1K) biosensor, and then each antibody was added in a binding reaction (300 seconds) and a dissociation reaction (1200 seconds). Based on the above results, the affinity for the EphA2 protein was calculated, and the results are shown in Table 57.

[Table 57]

| Antibodies | $K_D$ (nM) | $K_a$ (1/Ms) | $K_d$ (1/s) |
| --- | --- | --- | --- |
| EPB01 | 9.8579 | 2.32E+05 | 2.29E-03 |
| EPB02 | <0.001 | 2.15E+05 | <1.0E-07 |
| EPB03 | 0.7944 | 4.91E+05 | 3.90E-04 |
| EPB04 | 2.8644 | 4.95E+05 | 1.42E-03 |
| EPB05 | <0.001 | 3.42E+05 | <1.0E-07 |
| EPB06 | <0.001 | 3.84E+05 | <1.0E-07 |
| EPB07 | 0.0795 | 2.03E+05 | 1.62E-05 |
| EPB08 | 3.2174 | 6.58E+05 | 2.12E-03 |
| EPB09 | 2.3522 | 2.64E+05 | 6.20E-04 |
| EPB10 | 0.1878 | 3.92E+05 | 7.36E-05 |
| EPB11 | 0.5349 | 2.98E+05 | 1.59E-04 |
| EPB12 | 0.8657 | 5.44E+05 | 4.71E-04 |

The ability of antibodies to inhibit the signaling pathway mediated by EphA2 was analyzed using the PC-3 prostate cancer cell line.

Specifically, the PC-3 cells were treated with each antibody at a concentration of 50 nM for 30 minutes, the cells were lysed, and western blot was performed. The 1C1 humanized antibody that targets human EphA2 was used as a positive control and was prepared based on the sequence published in the literature (Kinch et al., US 20090304721 A1). As primary antibodies for analysis, Akt rabbit mAb (Cell Signaling Technology, 9272), Phospho-Akt (Ser473) (D9E) XP® rabbit mAb (Cell Signaling Technology, 4060), and β-Actin (13E5) rabbit mAb (Cell Signaling Technology, 4970) were used. As a secondary antibody, anti-rabbit IgG, HRP-linked antibody (Cell Signaling Technology, 7074) was used.

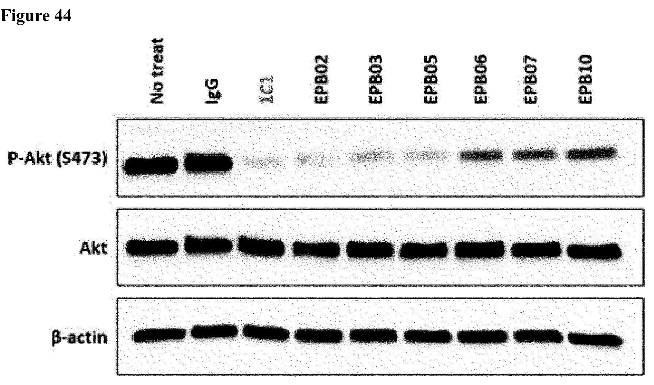

As shown in Figure 44, EPB02, EPB03, and EPB05 inhibited the phosphorylation of AKT at a level similar to that of the positive control (1C1).

Next, the Fc loads on the cell surface was quantified in the PC-3 cells. Each cell was treated with 100 nM of the antibody, and then reacted at 4 °C for 30 minutes, and treated with the Alexa 488 fluorescence-conjugated anti-human IgG Fcy Fab antibody. The Fc loads were quantified.

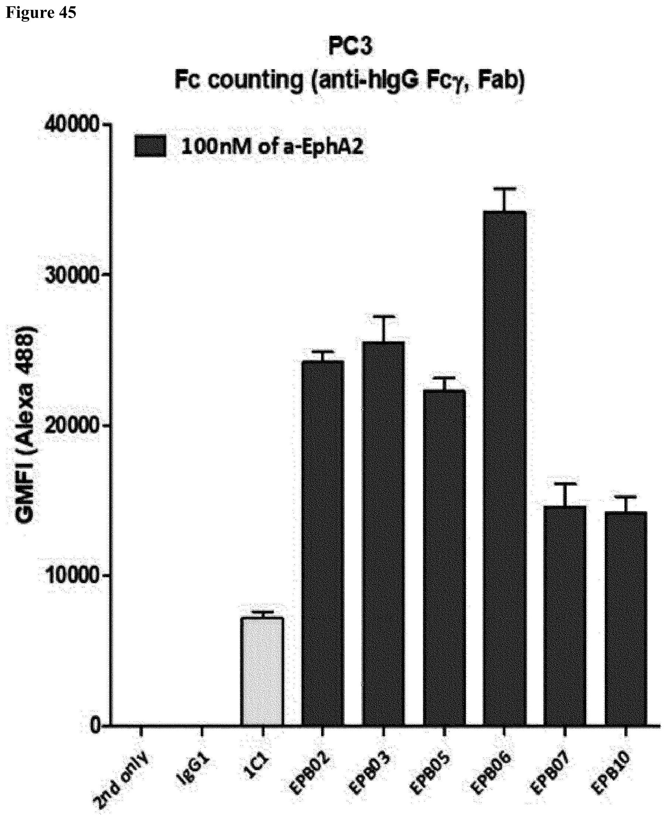

As shown in Figure 45, it was shown that higher Fc loads on the cell surface are induced by treatment of EPB02, EPB03, EPB05, EPB06, EPB07, or EPB 10 compared to the treatment of 1C1 humanized antibody that targets EphA2.

**Example 19. Design, preparation and analysis of antibody structure targeting MET**

The polypeptide sequences of the variant light chain and heavy chain of an antibody that specifically binds to the MET protein are shown in Table 58.

MEM01 consists of the polypeptides of Fc-Hole (SEQ ID NO: 7), MEM01 HC (SEQ ID NO: 568), and MEM01 LC (SEQ ID NO: 569), and was prepared by co-transfection of expression vectors loaded with polynucleotides encoding the polypeptides into the EXPICHO-S™ cell line (Figure 41a). The protein prepared as above was purified and analyzed in the same manner as in Example 1 above. In addition, MEM06 was prepared, purified, and then analyzed in the same manner as above (Table 58 and Table 59).

[Table 58]

| Name | Sequence | SEQ ID NO |
|---|---|---|
| MEMO1 HC | EVQLVESGGGLVQPGGSLRLSCAASGYTFTSYWLHWVRQAPGKGLEWVGMIDPSNSDTRFNPNFKDRFTISADTSKNTAYLQMNSLRAEDTAVYYCATYRSYVTPLDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 568 |
| MEMO1 LC | DIQMTQSPSSLSASVGDRVTITCKSSQSLLYTSSQKNYLAWYQQKPGKAPKLLIYWASTRESGVPSRFSGSGSGTDFTLTISSLQPEDFATYYCQQYYAYPWTFGCGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGECGGGGSGGGGSGGGGSEPKSSDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 569 |
| MEMO6 HC | QVQLVQSGAEVKKPGASVKVSCKASGYTFTSYGFSWVRQAPGQGLEWMGWISASNGNTYYAQKLQGRVTMTTDTSTSTAYMELRSLRSDDTAVYYCARVYADYADYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 570 |
| MEMO6 LC | DIQMTQSPSSVSASVGDRVTITCRASQGINTWLAWYQQKPGKAPKLLIYAASSLKSGVPSRFSGSGSGTDFTLTISSLQPEDFATYYCQQANSFPLTFGCGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGECGGGGSGGGGSGGGGSEPKSSDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 571 |

[Table 59]

| Name | Sequence | SEQ ID NO |
|---|---|---|
| MEM01 HC | GAAGTCCAATTGGTCGAGAGTGGAGGCGGGTTGGTACAGCCAGGAGGTTCACTTAGGTTGTC<br>CTGCGCCGCCAGTGGTTACACTTTCACATCTTATTGTCTGCACTGGGTGCGCCAAGCTCCTG<br>GGAAGTGTCTCGAATGGGTGGGTATGATCGATCCATCTAATTCCGACACACGGTTTAACCCA<br>AATTTTAAGGATAGATTTACAATTAGTGCTGACACTTCAAAAAACACAGCATACCTCCAGAT<br>GAACAGCCTGCGTGCTGAGGATACTGCTGTCTACTACTGTGCAACTTACCGCTCCTATGTCA<br>CACCTTTGGATTATTGGGGCCAGGGGACTCTGGTGACCGTGAGTTCTGCTAGCACCAAAGGA<br>CCTAGTGTTTTTCCTCTTGCCCCTTCCTCAAAGTCTACCTCTGGGGGGACAGCCGGCTCTGGG<br>CTGCCTGGTCAAGGATTATTTCCCAGAGCCTGTCACTGTCAGTTGGAACTCTGGAGCCTTGA<br>CTTCTGGTGTTCATACATTTCCTGCTGTCCTTCAAAGCAGCCGGCTTGTACTCATTGTCTTCT<br>GTTGTGACAGTACCCTCAAGCAGCCTCGGCACTCAGACATACATCTGCAATGTCAACCACAA<br>ACCCTCAAATACAAAGGTAGATAAAAAAGTCGAACCAAAGTCTTGTGACAAAACTCACACGT<br>GCCCACCGTGCCCAGCACCTGAACTCCTGGGGGGACCGTCAGTCTTCCTCTTCCCCCCAAAA<br>CCCAAGGACACCCTCATGATCTCCCGGACCCCTGAGGTCACATGCGTGGTGGTGGACGTGAG<br>CCACGAAGACCCTGAGGTCAAGTTCAACTGGTACGTGGACGGCGTGGAGGTGCATAATGCCA<br>AGACAAAGCCGCGGGAGGAGCAGTACAACAGCACGTACCGTGTGGTCAGCGTCCTCACCGTC<br>CTGCACCAGGACTGGCTGAATGGCAAGGAGTACAAGTGCAAGGTCTCCAACAAAGCCCTCCC<br>AGCCCCCATCGAGAAAACCATCTCCAAAGCCAAAGGGCAGCCCCGAGAACCACAGGTGTACA<br>CCCTGCCCCCATGCCGGGAGGAGATGACCAAGAACCAGGTCAGCCTGTGGTGCCTGGTCAAA<br>GGCTTCTATCCCAGCGACATCGCCGTGGAGTGGGAGAGCAATGGGCAGCCGGAGAACAACTA<br>CAAGACCACGCCTCCCGTGCTGGACTCCGACGGCTCCTTCTTCCTCTACAGCAAGCTCACCG<br>TGGACAAGAGCAGGTGGCAGCAGGGGAACGTCTTCTCATGCTCCGTGATGCATGAGGCTCTG<br>CACAACCACTACACGCAGAAGAGCCTCTCCCTGTCTCCGGGTAAA | 572 |
| MEM01 LC | GATATACAGATGACACAAAGTCCCTCATCACTTTCTGCCTCCGTTGGAGATCGTGTGACCAT<br>TACCTGTAAGAGTTCCCAATCACTGCTTTATACCTCTTCACAAAAAAATTACCTCGCTTGGT<br>ACCAGCAGAAGCCAGGTAAAGCACCTAAGCTGTTGATCTATTGGGCCTCCACTAGAGAGTCA<br>GGCGTGCCCAGCCGTTTCTCCGGTTCAGGGAGTGGGACAGACTTTACCTTGACCATTTCTTC<br>TTTGCAACCTGAAGACTTCGCCACATACTATTGTCAGCAATATTACGGCATATCCATGGACCT<br>TTGGGGTGTGGAACCAAAGTCGAAATAAAACGTACGGTGGCAGCTCCCAGCGTTTTTATCTTT<br>CCCCCATCCGACGAGCAGCTCAAGAGTGGCACTGCCTCTGTAGTTTGTTTGCTGAATAACTT<br>CTATCCACGTGAAGCAAAAGTACAGTGGAAGGTCGATAATGCCCTTCAGAGCGGGTAACAGCC<br>AAGAAAGTGTTACCGAGCAAGATTCCAAAGATTCCACTTACAGTCTGTCCAGCACATTGACA<br>CTGAGTAAGGCTGATTACGAAAAACACAAGGTGTACGGCATGCGAGGTGACACACCAAGGTCT<br>TTCATCTCCTGTAACTAAGAGCTTTAACCGGGGAGAATGTGGTGGTGGGGGCAGCGGGGGCG<br>GAGGTAGTGGAGGCGGCGGTAGTGAACCAAAGAGTAGTGACAAAACTCACACGTGCCCACCG<br>TGCCCAGCACCTGAACTCCTGGGGGGACCGTCAGTCTTCCTCTTCCCCCCAAAACCCAAGGA<br>CACCCTCATGATCTCCCGGACCCCTGAGGTCACATGCGTGGTGGTGGACGTGAGCCACGAAG<br>ACCCTGAGGTCAAGTTCAACTGGTACGTGGACGGCGTGGAGGTGCATAATGCCAAGACAAAG<br>CCGCGGGAGGAGCAGTACAACAGCACGTACCGTGTGGTCAGCGTCCTCACCGTCCTGCACCA<br>GGACTGGCTGAATGGCAAGGAGTACAAGTGCAAGGTCTCCAACAAAGCCCTCCCAGCCCCCA<br>TCGAGAAAACCATCTCCAAAGCCAAAGGGCAGCCCCGAGAACCACAGGTGTACACCCTGCCC<br>CCATGCCGGGAGGAGATGACCAAGAACCAGGTCAGCCTGTGGTGCCTGGTCAAAGGCTTCTA<br>TCCCAGCGACATCGCCGTGGAGTGGGAGAGCAATGGGCAGCCGGAGAACAACTACAAGACCA<br>CGCCTCCCGTGCTGGACTCCGACGGCTCCTTCTTCCTCTACAGCAAGCTCACCGTGGACAAG<br>AGCAGGTGGCAGCAGGGGAACGTCTTCTCATGCTCCGTGATGCATGAGGCTCTGCACAACCA<br>CTACACGCAGAAGAGCCTCTCCCTGTCTCCGGGTAAA | 573 |
| MEM06 HC | CAAGTGCAGCTTGTTCAATCAGGGGCCGAGGTTAAGAAACCAGGTGCTTCCGTCAAGGTCTC<br>CTGCAAGGCTTCCGGCTACACTTTTACCAGTTATGGGTTCAGTTGGGTTAGACAGGCCCCAG | 574 |

| | | |
|---|---|---|
| | GGCAGTGTCTCGAATGGATGGGATGGATTTCCGCATCTAACGGGAATACTTACTATGCCCAGAAACTTCAAGGTAGGGTTACCATGACTACCGATACTTCCACTAGTACAGCCTACATGGAACTCAGATCACTCCGTTCAGATGACACCGCAGTATATTACTGTGCAAGGGTATATGCTGATTATGCCGATTATTGGGGGCAAGGAACACTTGTCACAGTATCCAGCGCTAGCACCAAAGGACCTAGTGTTTTTCCTCTTGCCCCTTCCTCAAAGTCTACCTCTGGGGGGGACAGCCGCTCTGGGCTGCCTGGTCAAGGATTATTTCCCAGAGCCTGTCACTGTCAGTTGGAACTCTGGAGCCTTGACTTCTGGTGTTCATACATTTCCTGCTGTCCTTCAAAGCAGCGGCTTGTACTCATTGTCTTCTGTTGTGACAGTACCCTCAAGCAGCCTCGGCACTCAGACATACATCTGCAATGTCAACCACAAACCCTCAAATACAAAGGTAGATAAAAAAGTCGAACCAAAGTCTTGTGACAAAACTCACACGTGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATGCCGGGAGGAGATGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | |
| MEM06 LC | GATATCCAAATGACACAGTCACCCTCAAGTGTTAGCGCAAGTGTCGGGGACAGGGTGACCATCACATGCAGAGCTTCCCAGGGTATCAATACATGGCTGGCATGGTATCAACAGAAACCCGGAAAAGCACCAAAATTGCTTATTTATGCAGCTTCTAGCTTGAAGAGTGGGGTTCCCTCTCGTTTCTCTGGTTCAGGAAGCGGTACTGACTTTACCTTGACCATCAGTAGCTTGCAGCCCGAAGATTTCGCTACATATTATTGCCAACAGGCCAACTCTTTTCCCCTGACATTCGGTTGTGGCACTAAAGTGGAAATTAAGCGTACGGTGGCAGCTCCCAGCGTTTTTATCTTTCCCCCATCCGACGAGCAGCTCAAGAGTGGCACTGCCTCTGTAGTTTGTTTGCTGAATAACTTCTATCCACGTGAAGCAAAGTACAGTGGAAGGTCGATAATGCCCTTCAGAGCGGTAACAGCCAAGAAAGTGTTACCGAGCAAGATTCCAAAGATTCCACTTACAGTCTGTCCAGCACATTGACACTGAGTAAGGCTGATTACGAAAAACACAAGGTGTACGCATGCGAGGTGACACACCAAGGTCTTTCATCTCCTGTAACTAAGAGCTTTAACCGGGGAGAATGTGGTGGTGGGGGCAGCGGGGGCGGAGGTAGTGGAGGCGGCGGTAGTGAACCAAAGAGTAGTGACAAAACTCACACGTGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATGCCGGGAGGAGATGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | 575 |

Table 60 below shows the polypeptide sequences of the heavy chain variable region (VH) and light chain variable region (VL) of the engineered antibodies targeting MET. Table 61 below shows the polynucleotide sequences encoding the polypeptides of the heavy chain variable region (VH) and light chain variable region (VL) of the engineered antibodies targeting MET.

[Table 60]

| Name | | Sequence | SEQ ID NO |
|---|---|---|---|
| MEMO1 | VH | EVQLVESGGGLVQPGGSLRLSCAASGYTFTSYWLIWVRQAPGKCLEWVGMIDPSNSDTRFNPNF KDRFTISADTSKNTAYLQMNSLRAEDTAVYYCATYRSYVTPLDYWGQGTLVTVSS | 576 |
| | VL | DIQMTQSPSSLSASVGDRVTITCKSSQSLLYTSSQKNYLAWYQQKPGKAPKLLIYWASTRESGV PSRFSGSGSGTDFTLTISSLQPEDFATYYCQQYYAYPWTFGCGTKVEIK | 577 |
| MEMO6 | VH | QVQLVQSGAEVKKPGASVKVSCKASGYTFTSYGFSWVRQAPGQCLEWMGWISASNGNTYYAQKL QGRVTMTTDTSTSTAYMELRSLRSDDTAVYYCARVYADYADYWGQGTLVTVSS | 578 |
| | VL | DIQMTQSPSSVSASVGDRVTITCRASQGINTWLAWYQQKPGKAPKLLIYAASSLKSGVPSRFSG SGSGTDFTLTISSLQPEDFATYYCQQANSFPLTFGCGTKVEIK | 579 |

[Table 61]

| Name | | Sequence | SEQ ID NO |
|---|---|---|---|
| MEMO1 | VH | GAAGTCCAATTGGTCGAGAGTGGAGGCGGGTTGGTACAGCCAGGAGGTTCACTTAGGTTGTCCT GCCGCCGCCAGTGGTTACACTTTCACATCTTATTGGCTGCACTGGGTGCGCCAAGCTCCTGGGAA GTGTCTCGAATGGGTGGGTATGATCGATCCATCTAATTCCGACACACGGTTTAAACCCAAATTTT AAGGATAGATTTACAATTAGTGCTGACACTTCAAAAAACACAGCCATACCTCCAGATGAACAGCC TGCGTGCTGAGGATACTGCTGTCTACTACTGTGCAACTTACCGCTCCTATGTCACACCTTTGGA TTATTGGGGCCAGGGGACTCTGGTGACCGTGAGTTCT | 580 |
| | VL | GATATACAGATGACACAAAGTCCCTCATCACTTTCTGCCTCCGTTGGAGATCGTGTGACCATTA CCTGTAAGAGTTCCCAATCACTGCTTTATACCTCTTCACAAAAAAAATTACCTCGCTTGGTACCA GCAGAAGCCAGGTAAAGCACCTAAGCTGTTGATCTATTGGGCCTCCACTAGAGAGTCAGGCGTG CCCAGCCGTTTCTCCGGTTCAGGGAGTGGGACAGACTTTACCTTGACCATTTCTTCTTTGCAAC CTGAAGACTTCGCCACATACTATTGTCAGCAATATTACGCATATCCATGGACCTTTGGGGTGTGG AACCAAAGTCGAAATAAAA | 581 |
| MEMO6 | VH | CAAGTGCAGCTTGTTCAATCAGGGGCCGAGGTTAAGAAACCAGGTGCTTCCGTCAAGGTCTCCT GCAAGGCTTCCGGCTACACTTTTACCAGTTATGGGTTCAGTTGGGTTAGACAGGCCCCAGGGCA GTGTCTCGAATGGATGGGATGGATTTCCGCCATCTAACGGGAATACTTACTATGCCCAGAAACTT CAAGGTAGGGTTACCATGACTACCGATACTTCCACTAGTACAGCCTACATGGAACTCAGATCAC TCCGTTCAGATGACACCGCAGTATATTACTGTGCAAGGGTATATGCTGATTATGCCGATTATTG GGGGCAAGGAACACTTGTCACAGTATCCAGC | 582 |
| | VL | GATATCCAAATGACACAGTCACCCTCAAGTGTTAGCGCAAGTGTCGGGGACAGGGTGACCATCA CATGCAGAGCTTCCCAGGGTATCAATACATGGCTGGCATGGTATCAACAGAAACCGGGAAAGC ACCAAAATTGCTTATTTATGCAGCTTCTAGCTTGAAGAGTGGGGTTCCCTCTCGTTTCTCTGGT TCAGGAAGCGGTACTGACTTTACCTTGACCATCAGTAGCTTGCAGCCCGAAGATTTCGCTACAT ATTATTGCCAACAGGCCAACTCTTTTCCCCTGACATTCGGTTGTGGCACTAAAGTGGAAATTAA G | 583 |

Table 62 below shows the CDR sequences of the heavy chain and light chain of the engineered antibodies targeting MET.

[Table 62]

188

| Name | CDR | Sequence | SEQ ID NO |
|---|---|---|---|
| MEM01 | CDR-H1 | SYWLH | 236 |
| | CDR-H2 | MIDPSNSDTRFNPNFKD | 237 |
| | CDR-H3 | YRSYVTPLDY | 238 |
| | CDR-L1 | KSSQSLLYTSSQKNYLA | 239 |
| | CDR-L2 | WASTRES | 240 |
| | CDR-L3 | QQYYAYPWT | 241 |
| MEM06 | CDR-H1 | SYGFS | 584 |
| | CDR-H2 | WISASNGNTYYAQKLQG | 585 |
| | CDR-H3 | VYADYADY | 586 |
| | CDR-L1 | QQANSFPLT | 587 |
| | CDR-L2 | AASSLKS | 588 |
| | CDR-L3 | QQANSFPLT | 589 |

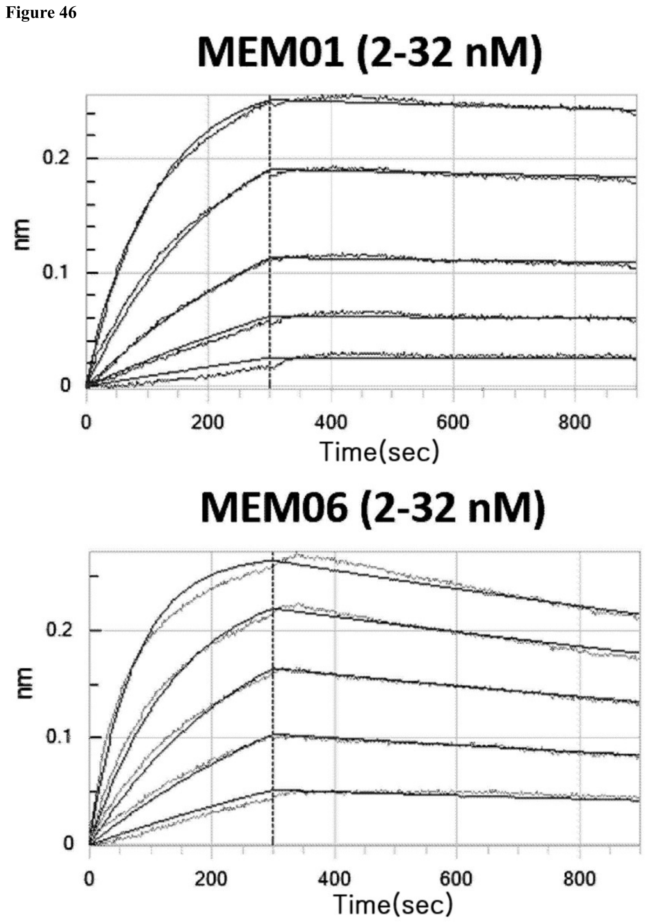

The MET protein binding constants of MEM01 and MEM06 were determined using Octet Red96e. Each antibody was loaded onto the anti-human Fab-CH1 2nd generation (FAB2G) biosensor (Sartorius, 18-5125). Then the human MET recombinant protein (Sino Biologicals, 10692-H08H) was added in a binding reaction (300 seconds) and a dissociation reaction (600 seconds) at various concentrations. The affinities of antibodies for the MET protein were calculated, and the results are shown in Figure 46 and Table 63.

[Table 63]

| Antibodies | $K_D$ (nM) | $K_a$ (1/Ms) | $K_d$ (1/s) |
|---|---|---|---|
| MEM01 | 0.1861 | 3.31E+05 | 6.16E-05 |
| MEM06 | 0.7881 | 4.20E+05 | 3.31E-04 |

The MKN45 and SNU-5 gastric cancer cell lines were used to quantify the Fc loads on the surface of MET expressing cells. Each cell was treated with the human IgG1 control, onartuzumab (produced from CHO cell line), emibetuzumab, MEM01, or MEM06, and then the Fc loads were quantified.

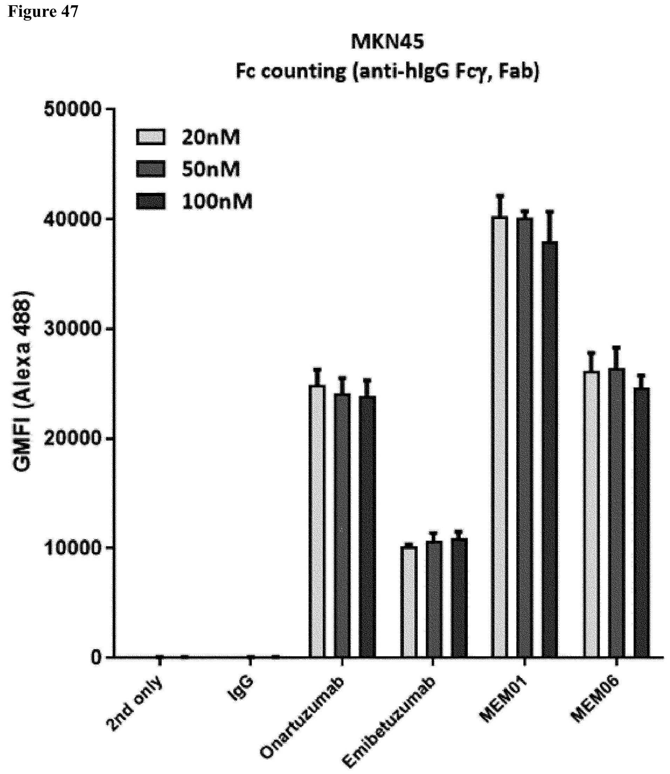

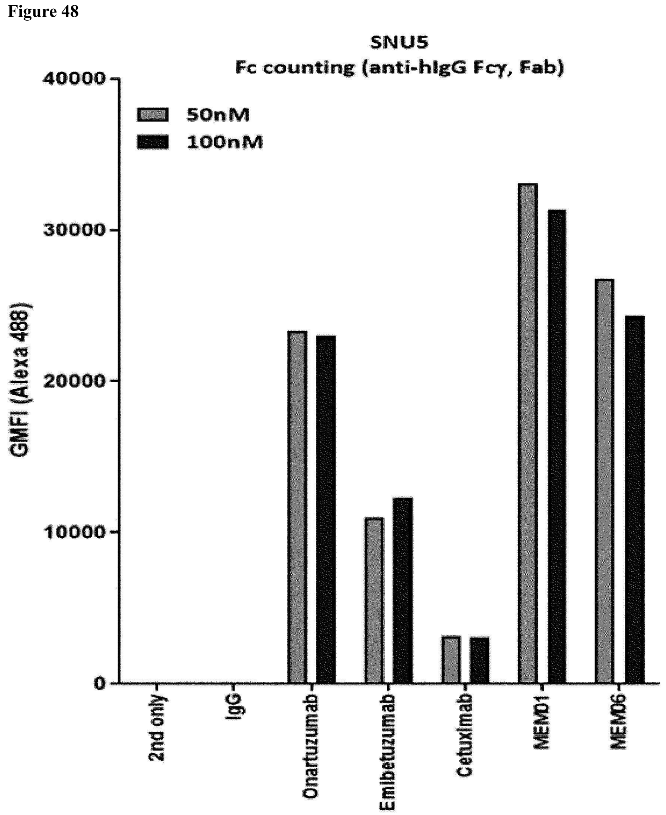

As a result, it was shown that higher Fc loads on the surface of the MET expressing cancer cells are induced by treatment of MEM01 compared to onartuzumab and emibetuzumab, which target MET (Figures 47 and 48). It was shown that higher Fc loads on the surface of the MET expressing cancer cells are induced by treatment of MEM06 compared to emibetuzumab. It was shown that MEM06 induces Fc loads at a level equivalent to that of onartuzumab (Figures 47 and 48).

**Example 20. Design, preparation and analysis of antibody structure targeting EGFR**

The polypeptide sequences of the variant light chain and heavy chain of an antibody that specifically binds to the EGFR protein are shown in Table 64.

EGM01 consists of the polypeptides of Fc-Hole (SEQ ID NO: 7), EGM01 HC (SEQ ID NO: 590), and EGF01 LC (SEQ ID NO: 591), and was prepared by co-transfection of expression vectors loaded with polynucleotides encoding the polypeptides into the EXPICHO-S™ cell line. The protein prepared as above was purified and analyzed in the same manner as in Example 1 above (Figure 41a). In addition, EGM02, EGM03, EGM04, EGM05, and EGM06 were prepared, purified, and then analyzed in the same manner as above.

[Table 64]

| Name | Sequence | SEQ ID NO |
|---|---|---|
| EGM01 HC | QVQLKQSGPGLVQPSQSLSITCTVSGFSLTNYGVHWVRQSPGKCLEWLGVIWSGGNTDY NTPFTSRLSINKDNSKSQVFFKMNSLQSNDTAIYYCARALTYYDYEFAYWGQGTLVTVS AASTKGPSVFPLAPSSKSTSGGTAALGCLVKDYFPEPVTVSWNSGALTSGVHTFPAVLQ SSGLYSLSSVVTVPSSSLGTQTYICNVNHKPSNTKVDKKVEPKSCDKTHTCPPCPAPEL LGGPSVFLFPPKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQPREPQVYTLP PCREEMTKNQVSLWCLVKGFYPSDIAVEWESNGQPENNYKTTPPVLDSDGSFFLYSKLT VDKSRWQQGNVFSCSVMHEALHNHYTQKSLSLSPGK | 590 |
| EGM01 LC | DILLTQSPVILSVSPGERVSFSCRASQSIGTNIHWYQQRTNGSPRLLIKYASESISGIP SRFSGSGSGTDFTLSINSVESEDIADYYCQQNNNWPTTFGCGTKLELKRTVAAPSVFIF PPSDEQLKSGTASVVCLLNNFYPREAKVQWKVDNALQSGNSQESVTEQDSKDSTYSLSS TLTLSKADYEKHKVYACEVTHQGLSSPVTKSFNRGECGGGGSGGGGSGGGGSEPKSSDK THTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAK GQPREPQVYTLPPCREEMTKNQVSLWCLVKGFYPSDIAVEWESNGQPENNYKTTPPVLD SDGSFFLYSKLTVDKSRWQQGNVFSCSVMHEALHNHYTQKSLSLSPGK | 591 |
| EGM02 HC | QVQLQESGPGLVKPSQTLSLTCTVSGGSISSGDYYWSWIRQPPGKCLEWIGYIYYSGST DYNPSLKSRVTMSVDTSKNQFSLKVNSVTAADTAVYYCARVSIFGVGTFDYWGQGTLVT VSSASTKGPSVFPLAPSSKSTSGGTAALGCLVKDYFPEPVTVSWNSGALTSGVHTFPAV LQSSGLYSLSSVVTVPSSSLGTQTYICNVNHKPSNTKVDKKVEPKSCDKTHTCPPCPAP ELLGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYT LPPCREEMTKNQVSLWCLVKGFYPSDIAVEWESNGQPENNYKTTPPVLDSDGSFFLYSK LTVDKSRWQQGNVFSCSVMHEALHNHYTQKSLSLSPGK | 592 |
| EGM02 LC | EIVMTQSPATLSLSPGERATLSCRASQSVSSYLAWYQQKPGQAPRLLIYDASNRATGIP ARFSGSGSGTDFTLTISSLEPEDFAVYYCHQYGSTPLTFGCGTKAEIKRTVAAPSVFIF PPSDEQLKSGTASVVCLLNNFYPREAKVQWKVDNALQSGNSQESVTEQDSKDSTYSLSS | 593 |

| | | |
|---|---|---|
| | TLTLSKADYEKHKVYACEVTHQGLSSPVTKSFNRGECGGGGSGGGGSGGGGSEPKSSDK THTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAK GQPREPQVYTLPPCREEMTKNQVSLWCLVKGFYPSDIAVEWESNGQPENNYKTTPPVLD SDGSFFLYSKLTVDKSRWQQGNVFSCSVMHEALHNHYTQKSLSLSPGK | |
| EGM03 HC | QVQLQESGPGLVKPSETLSLTCTVSGGSVSSGDYYWTWIRQSPGKCLEWIGHIYYSGNT NYNPSLKSRLTISIDTSKTQFSLKLSSVTAADTAIYYCVRDRVTGAFDIWGQGTMVTVS SASTKGPSVFPLAPSSKSTSGGTAALGCLVKDYFPEPVTVSWNSGALTSGVHTFPAVLQ SSGLYSLSSVVTVPSSSLGTQTYICNVNHKPSNTKVDKKVEPKSCDKTHTCPPCPAPEL LGGPSVFLFPPKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQPREPQVYTLP PCREEMTKNQVSLWCLVKGFYPSDIAVEWESNGQPENNYKTTPPVLDSDGSFFLYSKLT VDKSRWQQGNVFSCSVMHEALHNHYTQKSLSLSPGK | 594 |
| EGM03 LC | DIQMTQSPSSLSASVGDRVTITCQASQDISNYLNWYQQKPGKAPKLLIYDASNLETGVP SRFSGSGSGTDFTFTISSLQPEDIATYFCQHFDHLPLAFGCGTKVEIKRTVAAPSVFIF PPSDEQLKSGTASVVCLLNNFYPREAKVQWKVDNALQSGNSQESVTEQDSKDSTYSLSS TLTLSKADYEKHKVYACEVTHQGLSSPVTKSFNRGECGGGGSGGGGSGGGGSEPKSSDK THTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISKAK GQPREPQVYTLPPCREEMTKNQVSLWCLVKGFYPSDIAVEWESNGQPENNYKTTPPVLD SDGSFFLYSKLTVDKSRWQQGNVFSCSVMHEALHNHYTQKSLSLSPGK | 595 |
| EGM04 HC | QVQLVQSGAEVKKPGASVKVSCKASGYTFTSHWMHWVRQAPGQCLEWIGEFNPSNGRTN YNEKFKSKATMTVDTSTNTAYMELSSLRSEDTAVYYCASRDYDYDGRYFDYWGQGTLVT VSSASTKGPSVFPLAPSSKSTSGGTAALGCLVKDYFPEPVTVSWNSGALTSGVHTFPAV LQSSGLYSLSSVVTVPSSSLGTQTYICNVNHKPSNTKVDKKVEPKSCDKTHTCPPCPAP ELLGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYT LPPCREEMTKNQVSLWCLVKGFYPSDIAVEWESNGQPENNYKTTPPVLDSDGSFFLYSK LTVDKSRWQQGNVFSCSVMHEALHNHYTQKSLSLSPGK | 596 |

| | | |
|---|---|---|
| EGM04 LC | DIQMTQSPSSLSASVGDRVTITCSASSSVTYMYWYQQKPGKAPKLLIYDTSNLASGVPS RFSGSGSGTDYTFTISSLQPEDIATYYCQQWSSHIFTFGCGTKVEIKRTVAAPSVFIFP PSDEQLKSGTASVVCLLNNFYPREAKVQWKVDNALQSGNSQESVTEQDSKDSTYSLSST LTLSKADYEKHKVYACEVTHQGLSSPVTKSFNRGECGGGGSGGGGSGGGGSEPKSSDKT HTCPPCPAPELLGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKAKG QPREPQVYTLPPCREEMTKNQVSLWCLVKGFYPSDIAVEWESNGQPENNYKTTPPVLDS DGSFFLYSKLTVDKSRWQQGNVFSCSVMHEALHNHYTQKSLSLSPGK | 597 |
| EGM05 HC | QVQLVQSGAEVKKPGSSVKVSCKASGFTFTDYKIHWVRQAPGQCLEWMGYFNPNSGYST YAQKFQGRVTITADKSTSTAYMELSSLRSEDTAVYYCARLSPGGYYVMDAWGQGTTVTV SSASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCDKTHTCPPCPAPE LLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTL PPCREEMTKNQVSLWCLVKGFYPSDIAVEWESNGQPENNYKTTPPVLDSDGSFFLYSKL TVDKSRWQQGNVFSCSVMHEALHNHYTQKSLSLSPGK | 598 |
| EGM05 LC | DIQMTQSPSSLSASVGDRVTITCRASQGINNYLNWYQQKPGKAPKRLIYNTNNLQTGVP SRFSGSGSGTEFTLTISSLQPEDFATYYCLQHNSFPTFGCGTKLEIKRTVAAPSVFIFP PSDEQLKSGTASVVCLLNNFYPREAKVQWKVDNALQSGNSQESVTEQDSKDSTYSLSST LTLSKADYEKHKVYACEVTHQGLSSPVTKSFNRGECGGGGSGGGGSGGGGSEPKSSDKT HTCPPCPAPELLGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKAKG QPREPQVYTLPPCREEMTKNQVSLWCLVKGFYPSDIAVEWESNGQPENNYKTTPPVLDS DGSFFLYSKLTVDKSRWQQGNVFSCSVMHEALHNHYTQKSLSLSPGK | 599 |

Table 65 below shows the polynucleotide sequences encoding the polypeptides of the heavy chain and light chain of the engineered antibodies targeting EGFR.

[Table 65]

| Name | Sequence | SEQ ID NO |
|---|---|---|
| EGM01 HC | CAAGTACAGTTGAAACAATCAGGGCCTGGTTTGGTGCAGCCTTCCCAATCACTTAGCAT CACCTGCACTGTCTCAGGGTTCAGTCTTACAAACTACGGCGTACACTGGGTACGGCAAA GCCCTGGTAAGTGCCTGGAGTGGCTTGGGGTTATATGGTCTGGAGGGAATACCGACTAT AACACACCCTTTACCAGCAGGCTGTCCATCAATAAAGATAACTCTAAATCCCAGGTCTT CTTTAAAATGAACTCCCTCCAGTCTAATGACACTGCCATATATTACTGTGCTAGAGCAT TGACTTACTACGATTATGAGTTCGCATATTGGGGACAGGGTACTCTGGTCACCGTATCC GCTGCTAGCACCAAAGGACCTAGTGTTTTTCCTCTTGCCCCTTCCTCAAAGTCTACCTC TGGGGGGACAGCCGCTCTGGGCTGCCTGGTCAAGGATTATTTCCCAGAGCCTGTCACTG TCAGTTGGAACTCTGGAGCCTTGACTTCTGGTGTTCATACATTTCCTGCTGTCCTTCAA AGCAGCGGCTTGTACTCATTGTCTTCTGTTGTGACAGTACCCTCAAGCAGCCTCGGCAC TCAGACATACATCTGCAATGTCAACCACAAACCCTCAAATACAAAGGTAGATAAAAAAG TCGAACCAAAGTCTTGTGACAAAACTCACACGTGCCCACCGTGCCCAGCACCTGAACTC CTGGGGGGACCGTCAGTCTTCCTCTTCCCCCCAAAACCCAAGGACACCCTCATGATCTC CCGGACCCCTGAGGTCACATGCGTGGTGGTGGACGTGAGCCACGAAGACCCTGAGGTCA AGTTCAACTGGTACGTGGACGGCGTGGAGGTGCATAATGCCAAGACAAAGCCGCGGGAG GAGCAGTACAACAGCACGTACCGTGTGGTCAGCGTCCTCACCGTCCTGCACCAGGACTG GCTGAATGGCAAGGAGTACAAGTGCAAGGTCTCCAACAAAGCCCTCCCAGCCCCCATCG AGAAAACCATCTCCAAAGCCAAAGGGCAGCCCCGAGAACCACAGGTGTACACCCTGCCC CCATGCCGGGAGGAGATGACCAAGAACCAGGTCAGCCTGTGGTGCCTGGTCAAAGGCTT CTATCCCAGCGACATCGCCGTGGAGTGGGAGAGCAATGGGCAGCCGGAGAACAACTACA AGACCACGCCTCCCGTGCTGGACTCCGACGGCTCCTTCTTCCTCTACAGCAAGCTCACC GTGGACAAGAGCAGGTGGCAGCAGGGGAACGTCTTCTCATGCTCCGTGATGCATGAGGC TCTGCACAACCACTACACGCAGAAGAGCCTCTCCCTGTCTCCGGGTAAA | 600 |
| EGM01 LC | GACATCTTGCTTACTCAATCACCTGTAATACTTTCAGTTTCACCAGGTGAACGCGTTAG CTTCTCTTGTAGAGCCTCCCAATCTATAGGTACTAATATCCATTGGTATCAGCAGAGAA CCAACGGGTCTCCTCGTTTGCTCATTAAATATGCAAGCGAATCAATCTCAGGGATTCCT AGCCGTTTTAGTGGCTCTGGCAGTGGTACTGATTTCACACTCAGCATCAATTCTGTAGA | 601 |

| | | |
|---|---|---|
| | GAGCGAAGATATTGCAGACTACTATTGCCAACAGAACAATAATTGGCCCACAACCTTCG GGTGTGGCACAAAATTGGAACTCAAACGTACGGTGGCAGCTCCCAGCGTTTTTATCTTT CCCCCATCCGACGAGCAGCTCAAGAGTGGCACTGCCTCTGTAGTTTGTTTGCTGAATAA CTTCTATCCACGTGAAGCAAAAGTACAGTGGAAGGTCGATAATGCCCTTCAGAGCGGTA ACAGCCAAGAAAGTGTTACCGAGCAAGATTCCAAAGATTCCACTTACAGTCTGTCCAGC ACATTGACACTGAGTAAGGCTGATTACGAAAAACACAAGGTGTACGCATGCGAGGTGAC ACACCAAGGTCTTTCATCTCCTGTAACTAAGAGCTTTAACCGGGGAGAATGTGGTGGTG GGGGCAGCGGGGGCGGAGGTAGTGGAGGCGGCGGTAGTGAACCAAAGAGTAGTGACAAA ACTCACACGTGCCCACCGTGCCCAGCACCTGAACTCCTGGGGGGACCGTCAGTCTTCCT CTTCCCCCCAAAACCCAAGGACACCCTCATGATCTCCCGGACCCCTGAGGTCACATGCG TGGTGGTGGACGTGAGCCACGAAGACCCTGAGGTCAAGTTCAACTGGTACGTGGACGGC GTGGAGGTGCATAATGCCAAGACAAAGCCGCGGGAGGAGCAGTACAACAGCACGTACCG TGTGGTCAGCGTCCTCACCGTCCTGCACCAGGACTGGCTGAATGGCAAGGAGTACAAGT GCAAGGTCTCCAACAAAGCCCTCCCAGCCCCCATCGAGAAAACCATCTCCAAAGCCAAA GGGCAGCCCCGAGAACCACAGGTGTACACCCTGCCCCCCATGCCGGGAGGAGATGACCAA GAACCAGGTCAGCCTGTGGTGCCTGGTCAAAGGCTTCTATCCCAGCGACATCGCCGTGG AGTGGGAGAGCAATGGGCAGCCGGAGAACAACTACAAGACCACGCCTCCCGTGCTGGAC TCCGACGGCTCCTTCTTCCTCTACAGCAAGCTCACCGTGGACAAGAGCAGGTGGCAGCA GGGGAACGTCTTCTCATGCTCCGTGATGCATGAGGCTCTGCACAACCACTACACGCAGA AGAGCCTCTCCCTGTCTCCGGGTAAA | |
| EGM02 HC | CAAGTGCAGCTTCAAGAGTCTGGACCAGGGCTGGTCAAGCCCTCCCAAACCTTGAGCCT CACCTGTACTGTTTCCGGGGGCAGCATAAGTTCTGGTGATTACTACTGGAGTTGGATAC GCCAACCTCCCGGAAAATGTCTGGAGTGGATTGGGTATATCTATTATAGTGGCTCAACA GACTACAATCCTTCTCTCAAGAGTCGGGTAACTATGAGCGTAGATACAAGTAAAAACCA ATTTTCCCTTAAAGTCAATAGCGTTACAGCCGCTGACACTGCAGTTTACTACTGTGCCC GTGTTTCAATCTTCGGTGTCGGCACTTTCGATTACTGGGGTCAGGGTACTCTGGTTACC GTGTCATCCGCTAGCACCAAAGGACCTAGTGTTTTTCCTCTTGCCCCTTCCTCAAAGTC TACCTCTGGGGGGACAGCCGCTCTGGGCTGCCTGGTCAAGGATTATTTCCCAGAGCCTG TCACTGTCAGTTGGAACTCTGGAGCCTTGACTTCTGGTGTTCATACATTTCCTGCTGTC | 602 |

| | | | |
|---|---|---|---|
| | | CTTCAAAGCAGCGGCTTGTACTCATTGTCTTCTGTTGTGACAGTACCCTCAAGCAGCCT CGGCACTCAGACATACATCTGCAATGTCAACCACAAACCCTCAAATACAAAGGTAGATA AAAAAGTCGAACCAAAGTCTTGTGACAAAACTCACACGTGCCCACCGTGCCCAGCACCT GAACTCCTGGGGGGACCGTCAGTCTTCCTCTTCCCCCCCAAAACCCAAGGACACCCTCAT GATCTCCCGGACCCCTGAGGTCACATGCGTGGTGGTGGACGTGAGCCACGAAGACCCTG AGGTCAAGTTCAACTGGTACGTGGACGGCGTGGAGGTGCATAATGCCAAGACAAAGCCG CGGGAGGAGCAGTACAACAGCACGTACCGTGTGGTCAGCGTCCTCACCGTCCTGCACCA GGACTGGCTGAATGGCAAGGAGTACAAGTGCAAGGTCTCCAACAAAGCCCTCCCAGCCC CCATCGAGAAAACCATCTCCAAAGCCAAAGGGCAGCCCCGAGAACCACAGGTGTACACC CTGCCCCCATGCCGGGAGGAGATGACCAAGAACCAGGTCAGCCTGTGGTGCCTGGTCAA AGGCTTCTATCCCAGCGACATCGCCGTGGAGTGGGAGAGCAATGGGCAGCCGGAGAACA ACTACAAGACCACGCCTCCCGTGCTGGACTCCGACGGCTCCTTCTTCCTCTACAGCAAG CTCACCGTGGACAAGAGCAGGTGGCAGCAGGGGAACGTCTTCTCATGCTCCGTGATGCA TGAGGCTCTGCACAACCACTACACGCAGAAGAGCCTCTCCCTGTCTCCGGGTAAA | |
| EGM02 LC | | GAGATTGTTATGACCCAAAGCCCTGCAACACTTAGTTTGTCTCCAGGAGAGCGCGCCAC CCTTTCTTGTCGTGCATCCCAAAGCGTTAGCAGCTATCTCGCCTGGTATCAGCAGAAAC CCGGACAGGCTCCACGATTGCTGATCTACGACGCAAGTAATAGAGCTACAGGAATACCT GCTCGTTTCTCAGGCTCTGGATCTGGCACTGATTTCACCTTGACCATAAGCAGCCTGGA GCCCGAGGATTTCGCTGTATATTATTGCCATCAATACGGGAGTACCCCCCTCACATTCG GTTGCGGGACTAAGGCCGAAATTAAACGTACGGTGGCAGCTCCCAGCGTTTTTATCTTT CCCCCATCCGACGAGCAGCTCAAGAGTGGCACTGCCTCTGTAGTTTGTTTGCTGAATAA CTTCTATCCACGTGAAGCAAAAGTACAGTGGAAGGTCGATAATGCCCTTCAGAGCGGTA ACAGCCAAGAAAGTGTTACCGAGCAAGATTCCAAAGATTCCACTTACAGTCTGTCCAGC ACATTGACACTGAGTAAGGCTGATTACGAAAAACACAAGGTGTACGCATGCGAGGTGAC ACACCAAGGTCTTTCATCTCCTGTAACTAAGAGCTTTAACCGGGGAGAATGTGGTGGTG GGGGCAGCGGGGGCGGAGGTAGTGGAGGCGGCGGTAGTGAACCAAAGAGTAGTGACAAA ACTCACACGTGCCCACCGTGCCCAGCACCTGAACTCCTGGGGGGACCGTCAGTCTTCCT CTTCCCCCCAAAACCCAAGGACACCCTCATGATCTCCCGGACCCCTGAGGTCACATGCG TGGTGGTGGACGTGAGCCACGAAGACCCTGAGGTCAAGTTCAACTGGTACGTGGACGGC | 603 |

| | | |
|---|---|---|
| | GTGGAGGTGCATAATGCCAAGACAAAGCCGCGGGAGGAGCAGTACAACAGCACGTACCG TGTGGTCAGCGTCCTCACCGTCCTGCACCAGGACTGGCTGAATGGCAAGGAGTACAAGT GCAAGGTCTCCAACAAAGCCCTCCCAGCCCCCATCGAGAAAACCATCTCCAAAGCCAAA GGGCAGCCCCGAGAACCACAGGTGTACACCCTGCCCCCATGCCGGGAGGAGATGACCAA GAACCAGGTCAGCCTGTGGTGCCTGGTCAAAGGCTTCTATCCCAGCGACATCGCCGTGG AGTGGGAGAGCAATGGGCAGCCGGAGAACAACTACAAGACCACGCCTCCCGTGCTGGAC TCCGACGGCTCCTTCTTCCTCTACAGCAAGCTCACCGTGGACAAGAGCAGGTGGCAGCA GGGGAACGTCTTCTCATGCTCCGTGATGCATGAGGCTCTGCACAACCACTACACGCAGA AGAGCCTCTCCCTGTCTCCGGGTAAA | |
| EGM03 HC | CAGGTACAACTTCAAGAAAGTGGTCCAGGTCTTGTAAAACCCTCAGAAACTTTGAGTCT CACTTGTACCGTCAGTGGCGGAAGTGTAAGTTCTGGCGATTACTACTGGACCTGGATAC GCCAGTCTCCAGGCAAATGTCTGGAGTGGATAGGCCACATCTACTACAGCGGGAACACC AATTACAATCCATCTCTTAAATCAAGGTTGACAATTTCAATAGACACCAGTAAGACCCA GTTTTCTCTCAAACTTAGCAGTGTAACAGCAGCAGATACTGCAATCTACTACTGCGTTA GAGACCGTGTTACAGGGGCTTTCGACATCTGGGGGCAGGGAACTATGGTTACCGTCTCT TCTGCTAGCACCAAAGGACCTAGTGTTTTTCCTCTTGCCCCTTCCTCAAAGTCTACCTC TGGGGGGACAGCCGCTCTGGGCTGCCTGGTCAAGGATTATTTCCCAGAGCCTGTCACTG TCAGTTGGAACTCTGGAGCCTTGACTTCTGGTGTTCATACATTTCCTGCTGTCCTTCAA AGCAGCGGCTTGTACTCATTGTCTTCTGTTGTGACAGTACCCTCAAGCAGCCTCGGCAC TCAGACATACATCTGCAATGTCAACCACAAACCCTCAAATACAAAGGTAGATAAAAAAG TCGAACCAAAGTCTTGTGACAAAACTCACACGTGCCCACCGTGCCCAGCACCTGAACTC CTGGGGGGACCGTCAGTCTTCCTCTTCCCCCCAAAACCCAAGGACACCCTCATGATCTC CCGGACCCCTGAGGTCACATGCGTGGTGGTGGACGTGAGCCACGAAGACCCTGAGGTCA AGTTCAACTGGTACGTGGACGGCGTGGAGGTGCATAATGCCAAGACAAAGCCGCGGGAG GAGCAGTACAACAGCACGTACCGTGTGGTCAGCGTCCTCACCGTCCTGCACCAGGACTG GCTGAATGGCAAGGAGTACAAGTGCAAGGTCTCCAACAAAGCCCTCCCAGCCCCCATCG AGAAAACCATCTCCAAAGCCAAAGGGCAGCCCCGAGAACCACAGGTGTACACCCTGCCC CCATGCCGGGAGGAGATGACCAAGAACCAGGTCAGCCTGTGGTGCCTGGTCAAAGGCTT CTATCCCAGCGACATCGCCGTGGAGTGGGAGAGCAATGGGCAGCCGGAGAACAACTACA | 604 |

| | | |
|---|---|---|
| | AGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | |
| EGM03 LC | GACATACAGATGACACAGAGTCCTAGCTCATTGTCAGCCTCTGTCGGTGACCGCGTCACTATCACCTGCCAAGCCAGCCAAGACATATCAAATTATCTTAACTGGTACCAGCAAAAGCCTGGAAAGGCTCCAAAACTGCTGATTTACGACGCCTCTAATTTGGAGACCGGGGTTCCCTCTAGGTTCAGCGGGTCTGGTTCAGGCACCGACTTTACATTCACTATCTCAAGTCTCCAGCCAGAGGACATCGCTACATACTTTTGCCAACATTTTGACCATCTGCCCCTGGCATTTGGGTGTGGCACCAAGGTAGAAATTAAGCGTACGGTGGCAGCTCCCAGCGTTTTTATCTTTCCCCCATCCGACGAGCAGCTCAAGAGTGGCACTGCCTCTGTAGTTTGTTTGCTGAATAACTTCTATCCACGTGAAGCAAAAGTACAGTGGAAGGTCGATAATGCCCTTCAGAGCGGTAACAGCCAAGAAAGTGTTACCGAGCAAGATTCCAAAGATTCCACTTACAGTCTGTCCAGCACATTGACACTGAGTAAGGCTGATTACGAAAAACACAAGGTGTACGCATGCGAGGTGACACACCAAGGTCTTTCATCTCCTGTAACTAAGAGCTTTAACCGGGGGAGAATGTGGTGGTGGGGGCAGCGGGGGCGGAGGTAGTGGAGGCGGCGGTAGTGAACCAAAGAGTAGTGACAAAACTCACACGTGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATGCCGGGAGGAGATGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | 605 |
| EGM04 HC | CAAGTCCAACTGGTCCAATCTGGGGCAGAGGTCAAGAAACCTGGCGCAAGCGTAAAGGTATCCTGTAAAGCATCTGGCTACACATTCACTTCACATTGGATGCACTGGGTTCGGCAGG | 606 |

|  | CACCTGGGCAATGTCTTGAATGGATTGGGGAGTTTAACCCCAGTAACGGGAGGACTAAC | |
|  | TACAATGAAAAGTTCAAGTCCAAAGCAACCATGACCGTCGATACCAGCACAAACACTGC | |
|  | CTACATGGAACTTTCATCATTGCGATCTGAAGACACAGCAGTATATTACTGTGCCAGTA | |
|  | GGGATTACGACTACGACGGTCGCTACTTCGACTATTGGGGGCAAGGTACTTTGGTAACA | |
|  | GTGAGTAGTGCTAGCACCAAAGGACCTAGTGTTTTTCCTCTTGCCCCTTCCTCAAAGTC | |
|  | TACCTCTGGGGGGACAGCCGCTCTGGGCTGCCTGGTCAAGGATTATTTCCCAGAGCCTG | |
|  | TCACTGTCAGTTGGAACTCTGGAGCCTTGACTTCTGGTGTTCATACATTTCCTGCTGTC | |
|  | CTTCAAAGCAGCGGCTTGTACTCATTGTCTTCTGTTGTGACAGTACCCTCAAGCAGCCT | |
|  | CGGCACTCAGACATACATCTGCAATGTCAACCACAAACCCTCAAATACAAAGGTAGATA | |
|  | AAAAAGTCGAACCAAAGTCTTGTGACAAAACTCACACGTGCCCACCGTGCCCAGCACCT | |
|  | GAACTCCTGGGGGGACCGTCAGTCTTCCTCTTCCCCCCAAAACCCAAGGACACCCTCAT | |
|  | GATCTCCCGGACCCCTGAGGTCACATGCGTGGTGGTGGACGTGAGCCACGAAGACCCTG | |
|  | AGGTCAAGTTCAACTGGTACGTGGACGGCGTGGAGGTGCATAATGCCAAGACAAAGCCG | |
|  | CGGGAGGAGCAGTACAACAGCACGTACCGTGTGGTCAGCGTCCTCACCGTCCTGCACCA | |
|  | GGACTGGCTGAATGGCAAGGAGTACAAGTGCAAGGTCTCCAACAAAGCCCTCCCAGCCC | |
|  | CCATCGAGAAAACCATCTCCAAAGCCAAAGGGCAGCCCCGAGAACCACAGGTGTACACC | |
|  | CTGCCCCCATGCCGGGAGGAGATGACCAAGAACCAGGTCAGCCTGTGGTGCCTGGTCAA | |
|  | AGGCTTCTATCCCAGCGACATCGCCGTGGAGTGGGAGAGCAATGGGCAGCCGGAGAACA | |
|  | ACTACAAGACCACGCCTCCCGTGCTGGACTCCGACGGCTCCTTCTTCCTCTACAGCAAG | |
|  | CTCACCGTGGACAAGAGCAGGTGGCAGCAGGGGAACGTCTTCTCATGCTCCGTGATGCA | |
|  | TGAGGCTCTGCACAACCACTACACGCAGAAGAGCCTCTCCCTGTCTCCGGGTAAA | |
| EGM04 LC | GACATACAGATGACACAATCCCCATCTAGCCTGTCCGCAAGTGTTGGGGACCGTGTCAC | 607 |
|  | TATAACATGCTCAGCATCATCATCAGTGACTTATATGTACTGGTACCAGCAAAAGCCCG | |
|  | GAAAGGCACCTAAACTGCTCATTTATGACACCAGCAATCTTGCTTCCGGGGGTTCCTTCT | |
|  | CGATTTTCCGGTTCTGGCAGCGGTACTGACTATACTTTTACTATCAGTTCTCTGCAACC | |
|  | TGAAGACATCGCAACTTACTATTGTCAACAATGGTCCAGCCACATCTTTACTTTTGGAT | |
|  | GTGGGACAAAAGTCGAAATTAAACGTACGGTGGCAGCTCCCAGCGTTTTTATCTTTCCC | |
|  | CCATCCGACGAGCAGCTCAAGAGTGGCACTGCCTCTGTAGTTTGTTTGCTGAATAACTT | |
|  | CTATCCACGTGAAGCAAAAGTACAGTGGAAGGTCGATAATGCCCTTCAGAGCGGTAACA | |

| | | |
|---|---|---|
| | GCCAAGAAAGTGTTACCGAGCAAGATTCCAAAGATTCCACTTACAGTCTGTCCAGCACATTGACACTGAGTAAGGCTGATTACGAAAAACACAAGGTGTACGCATGCGAGGTGACACACCAAGGTCTTTCATCTCCTGTAACTAAGAGCTTTAACCGGGGAGAATGTGGTGGTGGGGGCAGCGGGGGCGGAGGTAGTGGAGGCGGCGGTAGTGAACCAAAGAGTAGTGACAAAACTCACACGTGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATGCCGGGAGGAGATGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | |
| EGM05 HC | CAAGTACAACTTGTTCAATCTGGAGCAGAGGTTAAGAAACCCGGATCTTCCGTTAAGGTTAGTTGCAAGGCCTCCGGGTTTACTTTCACTGACTATAAAATCCATTGGGTCCGGCAGGCCCCTGGGCAGTGTCTTGAATGGATGGGCTACTTCAACCCAAATTCTGGTTATTCCACTTATGCCCAGAAGTTTCAAGGCAGGGTCACCATCACTGCCGACAAGTCTACTTCAACCGCCTATATGGAACTTAGCAGTCTGCGATCAGAGGATACAGCAGTCTACTACTGCGCCAGACTGTCACCCGGCGGTTATTATGTGATGGATGCTTGGGGCCAGGGCACCACTGTTACAGTATCCTCTGCTAGCACCAAAGGACCTAGTGTTTTTCCTCTTGCCCCTTCCTCAAAGTCTACCTCTGGGGGGACAGCCGCTCTGGGCTGCCTGGTCAAGGATTATTTCCCAGAGCCTGTCACTGTCAGTTGGAACTCTGGAGCCTTGACTTCTGGTGTTCATACATTTCCTGCTGTCCTTCAAAGCAGCGGCTTGTACTCATTGTCTTCTGTTGTGACAGTACCCTCAAGCAGCCTCGGCACTCAGACATACATCTGCAATGTCAACCACAAACCCTCAAATACAAAGGTAGATAAAAAGTCGAACCAAAGTCTTGTGACAAAACTCACACGTGCCCACCGTGCCCAGCACCTGAACTCCTGGGGGGACCGTCAGTCTTCCTCTTCCCCCCAAAACCCAAGGACACCCTCATGAT | 608 |

| | | |
|---|---|---|
| | CTCCCGGACCCCTGAGGTCACATGCGTGGTGGTGGACGTGAGCCACGAAGACCCTGAGG TCAAGTTCAACTGGTACGTGGACGGCGTGGAGGTGCATAATGCCAAGACAAAGCCGCGG GAGGAGCAGTACAACAGCACGTACCGTGTGGTCAGCGTCCTCACCGTCCTGCACCAGGA CTGGCTGAATGGCAAGGAGTACAAGTGCAAGGTCTCCAACAAAGCCCTCCCAGCCCCCA TCGAGAAAACCATCTCCAAAGCCAAAGGGCAGCCCCGAGAACCACAGGTGTACACCCTG CCCCCATGCCGGGAGGAGATGACCAAGAACCAGGTCAGCCTGTGGTGCCTGGTCAAAGG CTTCTATCCCAGCGACATCGCCGTGGAGTGGGAGAGCAATGGGCAGCCGGAGAACAACT ACAAGACCACGCCTCCCGTGCTGGACTCCGACGGCTCCTTCTTCCTCTACAGCAAGCTC ACCGTGGACAAGAGCAGGTGGCAGCAGGGGAACGTCTTCTCATGCTCCGTGATGCATGA GGCTCTGCACAACCACTACACGCAGAAGAGCCTCTCCCTGTCTCCGGGTAAA | |
| EGM05 LC | GACATTCAGATGACTCAGTCCCCATCCTCTCTGTCTGCCAGCGTGGGAGATAGGGTCAC CATAACTTGTCGGGCATCCCAAGGGATCAATAACTACCTCAATTGGTATCAACAAAAAC CTGGCAAGGCTCCTAAAAGGCTGATTTATAACACTAACAATCTCCAAACCGGGGTGCCA AGTCGCTTTAGTGGGTCAGGGAGTGGAACAGAGTTTACTCTTACTATCTCCAGCCTCCA GCCCGAGGACTTTGCCACTTACTATTGCCTCCAACACAACTCATTTCCAACATTTGGTT GTGGCACTAAACTTGAAATTAAACGTACGGTGGCAGCTCCCAGCGTTTTTATCTTTCCC CCATCCGACGAGCAGCTCAAGAGTGGCACTGCCTCTGTAGTTTGTTTGCTGAATAACTT CTATCCACGTGAAGCAAAAGTACAGTGGAAGGTCGATAATGCCCTTCAGAGCGGTAACA GCCAAGAAAGTGTTACCGAGCAAGATTCCAAAGATTCCACTTACAGTCTGTCCAGCACA TTGACACTGAGTAAGGCTGATTACGAAAAACACAAGGTGTACGCATGCGAGGTGACACA CCAAGGTCTTTCATCTCCTGTAACTAAGAGCTTTAACCGGGGAGAATGTGGTGGTGGGG GCAGCGGGGGCGGAGGTAGTGGAGGCGGCGGTAGTGAACCAAAGAGTAGTGACAAAACT CACACGTGCCCACCGTGCCCAGCACCTGAACTCCTGGGGGGACCGTCAGTCTTCCTCTT CCCCCCAAAACCCAAGGACACCCTCATGATCTCCCGGACCCCTGAGGTCACATGCGTGG TGGTGGACGTGAGCCACGAAGACCCTGAGGTCAAGTTCAACTGGTACGTGGACGGCGTG GAGGTGCATAATGCCAAGACAAAGCCGCGGGAGGAGCAGTACAACAGCACGTACCGTGT GGTCAGCGTCCTCACCGTCCTGCACCAGGACTGGCTGAATGGCAAGGAGTACAAGTGCA AGGTCTCCAACAAAGCCCTCCCAGCCCCCATCGAGAAAACCATCTCCAAAGCCAAAGGG CAGCCCCGAGAACCACAGGTGTACACCCTGCCCCCATGCCGGGAGGAGATGACCAAGAA | 609 |

| | |
|---|---|
| | CCAGGTCAGCCTGTGGTGCCTGGTCAAAGGCTTCTATCCCAGCGACATCGCCGTGGAGT GGGAGAGCAATGGGCAGCCGGAGAACAACTACAAGACCACGCCTCCCGTGCTGGACTCC GACGGCTCCTTCTTCCTCTACAGCAAGCTCACCGTGGACAAGAGCAGGTGGCAGCAGGG GAACGTCTTCTCATGCTCCGTGATGCATGAGGCTCTGCACAACCACTACACGCAGAAGA GCCTCTCCCTGTCTCCGGGTAAA | |

Table 66 below shows the polypeptide sequences of the heavy chain variable region (VH) and light chain variable region (VL) of the engineered antibodies targeting EGFR. Table 67 below shows the polynucleotide sequences encoding the polypeptides of the heavy chain variable region (VH) and light chain variable region (VL) of the engineered antibodies targeting EGFR.

[Table 66]

| Name | | Sequence | SEQ ID NO |
|---|---|---|---|
| EGM01 | VH | QVQLKQSGPGLVQPSQSLSITCTVSGFSLTNYGVHWVRQSPGKCLEWLGVIWSGGNTDYNTPFT SRLSINKDNSKSQVFFKMNSLQSNDTAIYYCARALTYYDYEFAYWGQGTLVTVSA | 610 |
| | VL | DILLTQSPVILSVSPGERVSFSCRASQSIGTNIHWYQQRTNGSPRLLIKYASESISGIPSRFSG SGSGTDFTLSINSVESEDIADYYCQQNNNWPTTFGCGTKLELK | 611 |
| EGM02 | VH | QVQLQESGPGLVKPSQTLSLTCTVSGGSISSGDYYWSWIRQPPGKCLEWIGYIYYSGSTDYNPS LKSRVTMSVDTSKNQFSLKVNSVTAADTAVYYCARVSIFGVGTFDYWGQGTLVTVSS | 612 |
| | VL | EIVMTQSPATLSLSPGERATLSCRASQSVSSYLAWYQQKPGQAPRLLIYDASNRATGIPARFSG SGSGTDFTLTISSLEPEDFAVYYCHQYGSTPLTFGCGTKAEIK | 613 |
| EGM03 | VH | QVQLQESGPGLVKPSETLSLTCTVSGGSVSSGDYYWTWIRQSPGKCLEWIGHIYYSGNTNYNPS LKSRLTISIDTSKTQFSLKLSSVTAADTAIYYCVRDRVTGAFDIWGQGTMVTVSS | 614 |
| | VL | DIQMTQSPSSLSASVGDRVTITCQASQDISNYLNWYQQKPGKAPKLLIYDASNLETGVPSRFSG SGSGTDFTFTISSLQPEDIATYFCQHFDHLPLAFGCGTKVEIK | 615 |
| EGM04 | VH | QVQLVQSGAEVKKPGASVKVSCKASGYTFTSHWMHWVRQAPGQCLEWIGEFNPSNGRTNYNEKF KSKATMTVDTSTNTAYMELSSLRSEDTAVYYCASRDYDYDGRYFDYWGQGTLVTVSS | 616 |
| | VL | DIQMTQSPSSLSASVGDRVTITCSASSSVTYMYWYQQKPGKAPKLLIYDTSNLASGVPSRFSGS GSGTDYTFTISSLQPEDIATYYCQQWSSHIFTFGCGTKVEIK | 617 |
| EGM05 | VH | QVQLVQSGAEVKKPGSSVKVSCKASGFTFTDYKIHWVRQAPGQCLEWMGYFNPNSGYSTYAQKF QGRVTITADKSTSTAYMELSSLRSEDTAVYYCARLSPGGYVMDAWGQGTTVTVSS | 618 |
| | VL | DIQMTQSPSSLSASVGDRVTITCRASQGINNYLNWYQQKPGKAPKRLIYNTNNLQTGVPSRFSG SGSGTEFTLTISSLQPEDFATYYCLQHNSFPTFGCGTKLEIK | 619 |

[Table 67]

| Name | | Sequence | SEQ ID NO |
|------|---|----------|-----------|
| EGM01 | VH | CAAGTACAGTTGAAACAATCAGGGCCTGGTTTCGTGCAGCCTTCCCAATCACTTAGCATCACCT GCACTGTCTCAGGGTTCAGTCTTACAAACTACGGCGTACACTGGGTACGGCAAAGCCCTGGTAA GTGCCTGGAGTGGCTTGGGGTTATATGGTCTCGGAGGGAATACCGACTATAACACACCCTTTACC AGCAGGCTGTCCATCAATAAAGATAACTCTAAATCCCAGGTCTTCTTTAAAATGAACTCCCTCC AGTCTAATGACACTGCCATATATTACTGTGCTAGAGCATTGACTTACTACGATTATGAGTTCGC ATATTGGGGACAGGGTACTCTGGTCACCGTATCCGCT | G20 |
| | VL | GACATCTTGCTTACTCAATCACCTGTAATACTTTCAGTTTCACCAGGTGAACGCGTTAGCTTCT CTTGTAGAGCCTCCCAATCTATAGGTACTAATATCCATTGGTATCAGCAGAGAACCAACGGGTC TCCTCGTTTGCTCATTAAATATGCAAGCGAATCAATCTCAGGGATTCCTAGCCGTTTTAGTGGC TCTGGCAGTGGTACTGATTTCACACTCAGCATCAATTCTGTAGAGAGCGAAGATATTGCAGACT ACTATTGCCAACAGAACAATAATTGGCCCACAACCTTCGGGTGTGGCACAAAATTGGAACTCAA A | 621 |
| EGM02 | VH | CAAGTGCAGCTTCAAGAGTCTGGACCAGGGCTGGTCAAGCCCTCCCAAACCTTGAGCCTCACCT GTACTGTTTCCGGGGGGCAGCATAAGTTCTGGTGATTACTACTGGAGTTGGATACGCCAACCTCC CGGAAAATGTCTGGAGTGGATTGGGTATATCTATTATAGTGGCTCAACAGACTACAATCCTTCT CTCAAGAGTCGGGTAACTATGAGCGTAGATACAAGTAAAAACCAATTTTTCCCTTAAAGTCAATA GCGTTACAGCCGCTGACACTGCAGTTTACTACTGTGCCCGTGTTTCAATCTTCGGTGTCGGCAC TTTCGATTACTGGGGTCAGGGTACTCTGGTTACCGTGTCATCC | 622 |
| | VL | GAGATTGTTATGACCCAAAGCCCTGCAACACTTAGTTTGTCTCCAGGAGAGCGCGCCACCCTTT CTTGTCGTGCATCCCAAAGCGTTAGCAGCTATCTCGCCTGGTATCAGCAGAAACCCGGACAGGC TCCACGATTGCTGATCTACGACGCAAGTAATAGAGCTACAGGAATACCTGCTCGTTTCTCAGGC | 623 |

| | | | |
|---|---|---|---|
| | | TCTGGATCTGGCACTGATTTCACCTTGACCATAAGCAGCCTGGAGCCCGAGGATTTCGCTGTAT ATTATTGCCATCAATACGGGAGTACCCCCCTCACATTCGGTTGCGGGACTAAGGCCGAAATTAA A | |
| EGM03 | VH | GACATACAGATGACACAGAGTCCTAGCTCATTGTCAGCCTCTGTCGGTGACCGCGTCACTATCA CCTGCCAAGCCAGCCAAGACATATCAAATTATCTTAACTGGTACCAGCAAAAGCCTGGAAAGGC TCCAAAACTGCTGATTTACGACGCCTCTAATTTGGAGACCGGGGTTCCCTCTAGGTTCAGCGGG TCTGGTTCAGGCACCGACTTTACATTCACTATCTCAAGTCTCCAGCCAGAGGACATCGCTACAT ACTTTTGCCAACATTTTGACCATCTGCCCCTGGCATTTGGGTGTGGCACCAAGGTAGAAATTAA G | 624 |
| | VL | CAGGTACAACTTCAAGAAAGTGGTCCAGGTCTTGTAAAACCCTCAGAAACTTTGAGTCTCACTT GTACCGTCAGTGGCGGAAGTGTAAGTTCTGGCGATTACTACTGGACCTGGATACGCCAGTCTCC AGGCAAATGTCTGGAGTGGATAGGCCACATCTACTACAGCGGGAACACCAATTACAATCCATCT CTTAAATCAAGGTTGACAATTTCAATAGACACCAGTAAGACCCAGTTTTCTCTCAAACTTAGCA GTGTAACAGCAGCAGATACTGCAATCTACTACTGCGTTAGAGACCGTGTTACAGGGGCTTTCGA CATCTGGGGGCAGGGAACTATGGTTACCGTCTCTTCT | 625 |
| EGM04 | VH | CAAGTCCAACTGGTCCAATCTGGGGCAGAGGTCAAGAAACCTGGCGCAAGCGTAAAGGTATCCT GTAAAGCATCTGGCTACACATTCACTTCACATTGGATGCACTGGGTTCGGCAGGCACCTGGGCA ATGTCTTGAATGGATTGGGGAGTTTAACCCCAGTAACGGGAGGACTAACTACAATGAAAAGTTC AAGTCCAAAGCAACCATGACCGTCGATACCAGCACAAACACTGCCTACATGGAACTTTCATCAT TGCGATCTGAAGACACAGCAGTATATTACTGTGCCAGTAGGGATTACGACTACGACGGTCGCTA CTTCGACTATTGGGGGCAAGGTACTTTGGTAACAGTGAGTAGT | 626 |
| | VL | GACATACAGATGACACAATCCCCATCTAGCCTGTCCGCAAGTGTTGGGGACCGTGTCACTATAA CATGCTCAGCATCATCATCAGTGACTTATATGTACTGGTACCAGCAAAAGCCCGGAAAGGCACC TAAACTGCTCATTTATGACACCAGCAATCTTGCTTCCGGGGTTCCTTCTCGATTTTCCGGTTCT GGCAGCGGTACTGACTATACTTTTACTATCAGTTCTCTGCAACCTGAAGACATCGCAACTTACT ATTGTCAACAATGGTCCAGCCACATCTTTACTTTTGGATGTGGGACAAAAGTCGAAATTAAA | 627 |
| EGM05 | VH | CAAGTACAACTTGTTCAATCTGGAGCAGAGGTTAAGAAACCCGGATCTTCCGTTAAGGTTAGTT GCAAGGCCTCCGGGTTTACTTTCACTGACTATAAAATCCATTGGGTCCGGCAGGCCCCTGGGCA GTGTCTTGAATGGATGGGCTACTTCAACCCAAATTCTGGTTATTCCACTTATGCCCAGAAGTTT CAAGGCAGGGTCACCATCACTGCCGACAAGTCTACTTCAACCGCCTATATGGAACTTAGCAGTC TGCGATCAGAGGATACAGCAGTCTACTACTGCGCCAGACTGTCACCCGGCGGTTATTATGTGAT GGATGCTTGGGGCCAGGGCACCACTGTTACAGTATCCTCT | 628 |
| | VL | GACATTCAGATGACTCAGTCCCCATCCTCTCTGTCTGCCAGCGTGGGAGATAGGGTCACCATAA CTTGTCGGGCATCCCAAGGGATCAATAACTACCTCAATTGGTATCAACAAAAACCTGGCAAGGC TCCTAAAAGGCTGATTTATAACACTAACAATCTCCAAACCGGGGTGCCAAGTCGCTTTAGTGGG TCAGGGAGTGGAACAGAGTTTACTCTTACTATCTCCAGCCTCCAGCCCGAGGACTTTGCCACTT ACTATTGCCTCCAACACAACTCATTTCCAACATTTGGTTGTGGCACTAAACTTGAAATTAAA | 629 |

Table 68 below shows the CDR sequences of the heavy chain and light chain of the engineered antibodies targeting EGFR.

[Table 68]

| Name | CDR | Sequence | SEQ ID NO |
|---|---|---|---|
| EGM01 | CDR-H1 | NYGVH | 175 |
| | CDR-H2 | VIWSGGNTDYNTPFTS | 176 |
| | CDR-H3 | ALTYYDYEFAY | 177 |
| | CDR-L1 | RASQSIGTNIH | 178 |
| | CDR-L2 | YASESIS | 179 |
| | CDR-L3 | QQNNNWPTT | 180 |
| EGM02 | CDR-H1 | DYWS | 187 |
| | CDR-H2 | YIYYSGSTDYNPSLKS | 188 |
| | CDR-H3 | VSIFGVGTFDY | 189 |
| | CDR-L1 | RASQSVSSYLA | 190 |
| | CDR-L2 | DASNRAT | 191 |
| | CDR-L3 | HQYGSTPLT | 192 |
| EGM03 | CDR-H1 | DYYWT | 181 |
| | CDR-H2 | HIYYSGNTNYNPSLKS | 182 |
| | CDR-H3 | DRVTGAFDI | 183 |
| | CDR-L1 | QASQDISNYLN | 184 |
| | CDR-L2 | DASNLET | 185 |
| | CDR-L3 | QHFDHLPLA | 186 |
| EGM04 | CDR-H1 | SHWMH | 630 |
| | CDR-H2 | EFNPSNGRTNYNEKFKS | 631 |
| | CDR-H3 | RDYDYDGRYFDY | 632 |
| | CDR-L1 | SASSSVTYMY | 633 |
| | CDR-L2 | DTSNLAS | 634 |
| | CDR-L3 | QQWSSHFT | 635 |
| EGM05 | CDR-H1 | DYKIH | 193 |
| | CDR-H2 | YFNPNSGYSTYAQKFQG | 194 |
| | CDR-H3 | LSPGGYVMDA | 195 |
| | CDR-L1 | RASQGINNYLN | 196 |
| | CDR-L2 | NTNNLQT | 197 |
| | CDR-L3 | LQHNSFPT | 198 |

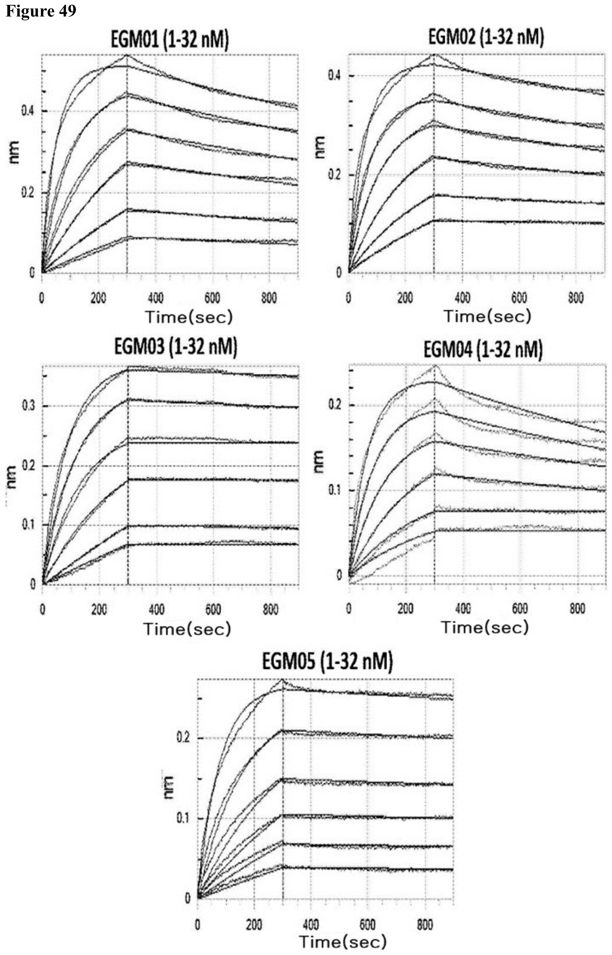

The EGFR protein binding constants of each of EGM01 to EGM05 to the EGFR protein were determined using Octet Red96e. Each antibody was loaded onto the anti-human Fab-CH1 2nd generation (FAB2G) biosensor, and then the human EGFR recombinant protein (Sino Biologicals, 10692-H08H) was added in a binding reaction (300 seconds) and a dissociation reaction (600 seconds) at various concentrations. The affinities of antibodies for the EGFR protein were calculated, and the results are shown in Figure 49 and Table 69.

[Table 69]

| Antibodies | $K_D$ (nM) | $K_a$ (1/Ms) | $K_d$ (1/s) |
|---|---|---|---|
| EGM01 | 0.6446 | 6.00E+05 | 3.87E-04 |
| EGM02 | 0.3946 | 7.30E+05 | 2.88E-04 |
| EGM03 | 0.1518 | 4.28E+05 | 6.51E-05 |
| EGM04 | 0.6421 | 6.31E+05 | 4.05E-04 |
| EGM05 | 0.1989 | 4.08E+05 | 8.11E-05 |

**Example 21. Design, preparation and analysis of a novel antibody structure targeting CD33**

The polypeptide sequences of the variant light chain and heavy chain of an antibody that specifically recognizes the CD33 protein are shown in Table 70.

GPM01 consists of the polypeptides of Fc-Hole (SEQ ID NO: 7), 33-1 HC (SEQ ID NO: 636), and 33-1 LC (SEQ ID NO: 637), and was prepared by co-transfection of expression vectors loaded with polynucleotides encoding the polypeptides into the EXPICHO-S™ cell line (Figure 41a). The protein prepared as above was purified and analyzed in the same manner as in Example 1 above. In addition, 33-2, 33-3, 33-4, 33-5, 33-6 and 33-7 were each prepared, purified, and analyzed in the same manner as above (Figure 41a, Figure 41b, Table 70, and Table 71).

33-1, 33-2, and 33-3 bind to different epitopes of the antigen in a monovalent manner and have structures consisting of two Fc domains (Figure 41a). 33-4, 33-5, 33-6, and 33-7 have structures in which the variable regions of the CD33 antibody are linked with a polypeptide linker (SEQ ID NO: 48, SEQ ID NO: 50), and bind to CD33 in a biparatopic manner, and have two Fc domains (Figure 41b).

[Table 70]

| Name | Sequence | SEQ ID NO |
|---|---|---|
| 33-1 HC | EVQLVQSGAEVKKPGSSVKVSCKASGYTITDSNIIHWVRQAPGQGLEWIGYIYPYNGGTDYNQ KFKNRATLTVDNPTNTAYMELSSLRSEDTAFYYCVNGNPWLAYWGQGTLVTVSSASTKGPSV FPLAPSSKSTSGGTAALGCLVKDYFPEPVTVSWNSGALTSGVHTFPAVLQSSGLYSLSSVVT VPSSSLGTQTYICNVNHKPSNTKVDKKVEPKSCDKTHTCPPCPAPELLGGPSVFLFPPKPKD TLMISRTPEVTCVVVDVSHEDPEVKFNWYVDGVEVHNAKTKPREEQYNSTYRVVSVLTVLHQ DWLNGKEYKCKVSNKALPAPIEKTISKAKGQPREPQVYTLPPCREEMTKNQVSLWCLVKGFY PSDIAVEWESNGQPENNYKTTPPVLDSDGSFFLYSKLTVDKSRWQQGNVFSCSVMHEALHNH YTQKSLSLSPGK | 636 |
| 33-1 LC | DIQLTQSPSTLSASVGDRVTITCRASESLDNYGIRFLTWFQQKPGKAPKLLMYAASNQGSGV PSRFSGSGSGTEFTLTISSLQPDDFATYYCQQTKEVPWSFGCGTKVEVKRTVAAPSVFIFPP SDEQLKSGTASVVCLLNNFYPREAKVQWKVDNALQSGNSQESVTEQDSKDSTYSLSSTLTLS KADYEKHKVYACEVTHQGLSSPVTKSFNRGECGGGGSGGGGSGGGGSEPKSSDKTHTCPPCP APELLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLPPC REEMTKNQVSLWCLVKGFYPSDIAVEWESNGQPENNYKTTPPVLDSDGSFFLYSKLTVDKSR WQQGNVFSCSVMHEALHNHYTQKSLSLSPGK | 637 |
| 33-2 HC | QVQLVQSGAEVKKPGSSVKVSCKASGYTFTDYNMHWVRQAPGQGLEWIGYIYPYNGGTGYNQ KFKSKATITADESTNTAYMELSSLRSEDTAVYYCARGRPAMDYWGQGTLVTVSSASTKGPSV FPLAPSSKSTSGGTAALGCLVKDYFPEPVTVSWNSGALTSGVHTFPAVLQSSGLYSLSSVVT VPSSSLGTQTYICNVNHKPSNTKVDKKVEPKSCDKTHTCPPCPAPELLGGPSVFLFPPKPKD TLMISRTPEVTCVVVDVSHEDPEVKFNWYVDGVEVHNAKTKPREEQYNSTYRVVSVLTVLHQ DWLNGKEYKCKVSNKALPAPIEKTISKAKGQPREPQVYTLPPCREEMTKNQVSLWCLVKGFY PSDIAVEWESNGQPENNYKTTPPVLDSDGSFFLYSKLTVDKSRWQQGNVFSCSVMHEALHNH YTQKSLSLSPGK | 638 |
| 33-2 LC | DIQMTQSPSSLSASVGDRVTITCRASESVDNYGISFMNWFQQKPGKAPKLLIYAASNQGSGV PSRFSGSGSGTDFTLTISSLQPDDFATYYCQQSKEVPWTFGCGTKVEIKRTVAAPSVFIFPP SDEQLKSGTASVVCLLNNFYPREAKVQWKVDNALQSGNSQESVTEQDSKDSTYSLSSTLTLS KADYEKHKVYACEVTHQGLSSPVTKSFNRGECGGGGSGGGGSGGGGSEPKSSDKTHTCPPCP APELLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLPPC REEMTKNQVSLWCLVKGFYPSDIAVEWESNGQPENNYKTTPPVLDSDGSFFLYSKLTVDKSR WQQGNVFSCSVMHEALHNHYTQKSLSLSPGK | 639 |
| 33-3 HC | QVQLQQPGAEVVKPGASVKMSCKASGYTFTSYYIHWIKQTPGQGLEWVGVIYPGNDDISYNQ KFQGKATLTADKSSTTAYMQLSSLTSEDSAVYYCAREVRLRYFDVWGQGTTVTVSSASTKGP SVFPLAPSSKSTSGGTAALGCLVKDYFPEPVTVSWNSGALTSGVHTFPAVLQSSGLYSLSSV VTVPSSSLGTQTYICNVNHKPSNTKVDKKVEPKSCDKTHTCPPCPAPELLGGPSVFLFPPKP KDTLMISRTPEVTCVVVDVSHEDPEVKFNWYVDGVEVHNAKTKPREEQYNSTYRVVSVLTVL HQDWLNGKEYKCKVSNKALPAPIEKTISKAKGQPREPQVYTLPPCREEMTKNQVSLWCLVKG FYPSDIAVEWESNGQPENNYKTTPPVLDSDGSFFLYSKLTVDKSRWQQGNVFSCSVMHEALH NHYTQKSLSLSPGK | 640 |
| 33-3 LC | EIVLTQSPGSLAVSPGERVTMSCKSSQSVFFSSSQKNYLAWYQQIPGQSPRLLIYWASTRES GVPDRFTGSGSGTDFTLTISSVQPEDLAIYYCHQYLSSRTFGCGTKLEIKRTVAAPSVFIFP PSDEQLKSGTASVVCLLNNFYPREAKVQWKVDNALQSGNSQESVTEQDSKDSTYSLSSTLTL SKADYEKHKVYACEVTHQGLSSPVTKSFNRGECGGGGSGGGGSGGGGSEPKSSDKTHTCPPC PAPELLGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYTLPP CREEMTKNQVSLWCLVKGFYPSDIAVEWESNGQPENNYKTTPPVLDSDGSFFLYSKLTVDKS | 641 |

| | | |
|---|---|---|
| | RWQQGNVFSCSVMHEALHNHYTQKSLSLSPGK | |
| 33-4 HC | EVQLVQSGAEVKKPGSSVKVSCKASGYTITDSNIHWVRQAPGQSLEWIGYIYPYNGGTDYNQKFKNRATLTVDNPTNTAYMELSSLRSEDTAFYYCVNGNPWLAYWGQGTLVTVSSASTKGPSVFPLAPQVQLVQSGAEVKKPGSSVKVSCKASGYTFTDYNMHWVRQAPGQCLEWIGYIYPYNGGTGYNQKFKSKATITADESTNTAYMELSSLRSEDTAVYYCARGRPAMDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 642 |
| 33-4 LC | DIQLTQSPSTLSASVGDRVTITCRASESLDNYGIRFLTWFQQKPGKAPKLLMYAASNQGSGVPSRFSGSGSGTEFTLTISSLQPDDFATYYCQQTKEVPWSFGQGTKVEVKRTVAAPSVFIFPPDIQMTQSPSSLSASVGDRVTITCRASESVDNYGISFMNWFQQKPGKAPKLLIYAASNQGSGVPSRFSGSGSGTDFTLTISSLQPDDFATYYCQQSKEVPWTFGCGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGECGGGGSGGGGSGGGGSEPKSSDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 643 |
| 33-5 HC | QVQLQQPGAEVVKPGASVKMSCKASGYTFTSYYIHWIKQTPGQGLEWVGIYPGNDDISYNQKFQGKATLTADKSSTTAYMQLSSLTSEDSAVYYCAREVRLRYFDVWGQGTTVTVSSASTKGPSVFPLAPQVQLVQSGAEVKKPGSSVKVSCKASGYTFTDYNMHWVRQAPGQCLEWIGYIYPYNGGTGYNQKFKSKATITADESTNTAYMELSSLRSEDTAVYYCARGRPAMDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 644 |
| 33-5 LC | EIVLTQSPGSLAVSPGERVTMSCKSSQSVFFSSSQKNYLAWYQQIPGQSPRLLIYWASTRESGVPDRFTGSGSGTDFTLTISSVQPEDLAIYYCHQYLSSRTFGQGTKLEIKRTVAAPSVFIFPPDIQMTQSPSSLSASVGDRVTITCRASESVDNYGISFMNWFQQKPGKAPKLLIYAASNQGSGVPSRFSGSGSGTDFTLTISSLQPDDFATYYCQQSKEVPWTFGCGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGECGGGGSGGGGSGGGGSEPKSSDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 645 |
| 33-6 HC | QVQLVQSGAEVKKPGSSVKVSCKASGYTFTDYNMHWVRQAPGQGLEWIGYIYPYNGGTGYNQKFKSKATITADESTNTAYMELSSLRSEDTAVYYCARGRPAMDYWGQGTLVTVSSASTKGPSVFPLAPEVQLVQSGAEVKKPGSSVKVSCKASGYTITDSNIHWVRQAPGQCLEWIGYIYPYNGGTDYNQKFKNRATLTVDNPTNTAYMELSSLRSEDTAFYYCVNGNPWLAYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHE | 646 |

| | | | |
|---|---|---|---|
| | | ALHNHYTQKSLSLSPGK | |
| | 33-6 LC | DIQMTQSPSSLSASVGDRVTITCRASESVDNYGISFMNWFQQKPGKAPKLLIYAASNQGSGV PSRFSGSGSGTDFTLTISSLQPDDFATYYCQQSKEVPWTFGQGTKVEIKRTVAAPSVFIFPP SDEQLKSGTASVVCLLNNFYPREAKVQWKVDNALQSGNSQESVTEQDSKDSTYSLSSTLTLS KADYEKHKVYACEVTHQGLSSPVTKSFNRGECGGGGSGGGGSGGGGSEPKSSDKTHTCPPCP APELLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLPPC REEMTKNQVSLWCLVKGFYPSDIAVEWESNGQPENNYKTTPPVLDSDGSFFLYSKLTVDKSR WQQGNVFSCSVMHEALHNHYTQKSLSLSPGK | 647 |
| | 33-7 HC | QVQLVQSGAEVKKPGSSVKVSCKASGYTFTDYNMHWVRQAPGQGLEWIGYIYPYNGGTGYNQ KFKSKATITADESTNTAYMELSSLRSEDTAVYYCARGRPAMDYWGQGTLVTVSSASTKGPSV FPLAPQVQLQQPGAEVVKPGASVKMSCKASGYTFTSYYIHWIKQTPGQCLEWVGVIYPGNDD ISYNQKFQGKATLTADKSSTTAYMQLSSLTSEDSAVYYCAREVRLRYFDVWGQGTTVTVSSA STKGPSVFPLAPSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPELLGGPSVFL FPPKPKDTLMISRTPEVTCVVVDVSHEDPEVKFNWYVDGVEVHNAKTKPREEQYNSTYRVVS VLTVLHQDWLNGKEYKCKVSNKALPAPIEKTISKAKGQPREPQVYTLPPCREEMTKNQVSLW CLVKGFYPSDIAVEWESNGQPENNYKTTPPVLDSDGSFFLYSKLTVDKSRWQQGNVFSCSVM HEALHNHYTQKSLSLSPGK | 648 |
| | 33-7 LC | DIQMTQSPSSLSASVGDRVTITCRASESVDNYGISFMNWFQQKPGKAPKLLIYAASNQGSGV PSRFSGSGSGTDFTLTISSLQPDDFATYYCQQSKEVPWTFGQGTKVEIKRTVAAPSVFIFPP EIVLTQSPGSLAVSPGERVTMSCKSSQSVFFSSSQKNYLAWYQQIPGQSPRLLIYWASTRES GVPDRFTGSGSGTDFTLTISSVQPEDLAIYYCHQYLSSRTFGCGTKLEIKRTVAAPSVFIFP PSDEQLKSGTASVVCLLNNFYPREAKVQWKVDNALQSGNSQESVTEQDSKDSTYSLSSTLTL SKADYEKHKVYACEVTHQGLSSPVTKSFNRGECGGGGSGGGGSGGGGSEPKSSDKTHTCPPC PAPELLGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYTLPP CREEMTKNQVSLWCLVKGFYPSDIAVEWESNGQPENNYKTTPPVLDSDGSFFLYSKLTVDKS RWQQGNVFSCSVMHEALHNHYTQKSLSLSPGK | 649 |

[Table 71]

| Name | Sequence | SEQ ID NO |
|---|---|---|
| 33-1 HC | GAGGTTCAGTTGGTTCAGTCAGGAGCAGAGGTCAAAAAACCTGGAAGCTCTGTCAAAGTTAG<br>TTGTAAGGCCAGTGGATACACCATAACCGATTCAAATATACATTGGGTTAGGCAAGCACCAG<br>GACAGTGCTTGGAATGGATCGGGTACATCTATCCATATAATGGGGGCACCGATTACAACCAA<br>AAGTTTAAGAATCGCGCCACACTCACTGTTGATAATCCAACCAATACAGCATACATGGAGTT<br>GAGCAGTCTTCGGTCCGAGGACACTGCTTTTTACTATTGTGTGAACGGTAACCCATGGTTGG<br>CCTATTGGGGCCAAGGTACACTTGTAACAGTTTCATCTGCTAGCACCAAAGGACCTAGTGTT<br>TTTCCTCTTGCCCCTTCCTCAAAGTCTACCTCTGGGGGGACAGCCGCTCTGGGCTGCCTGGT<br>CAAGGATTATTTCCCAGAGCCTGTCACTGTCAGTTGGAACTCTGGAGCCTTGACTTCTGGTG<br>TTCATACATTTCCTGCTGTCCTTCAAAGCAGCGGCTTGTACTCATTGTCTTCTGTTGTGACA<br>GTACCCTCAAGCAGCCTCGGCACTCAGACATACATCTGCAATGTCAACCACAAACCCTCAAA<br>TACAAAGGTAGATAAAAAAGTCGAACCAAAGTCTTGTGACAAAACTCACACGTGCCCACCGT<br>GCCCAGCACCTGAACTCCTGGGGGGACCGTCAGTCTTCCTCTTCCCCCCAAAACCCAAGGAC<br>ACCCTCATGATCTCCCGGACCCCTGAGGTCACATGCCGTGGTGGTGGACGTGAGCCACGAAGA<br>CCCTGAGGTCAAGTTCAACTGGTACGTGGACGGCGTGGAGGTGCATAATGCCAAGACAAAGC<br>CGCGGGAGGAGCAGTACAACAGCACGTACCGTGTGGTCAGCGTCCTCACCGTCCTGCACCAG<br>GACTGGCTGAATGGCAAGGAGTACAAGTGCAAGGTCTCCAACAAAGCCCTCCCAGCCCCCAT<br>CGAGAAAACCATCTCCAAAGCCAAAGGGCAGCCCCGAGAACCACAGGTGTACACCCTGCCCC<br>CATCCCGGGAGGAGATGACCAAGAACCAGGTCAGCCTGTGGTGCCTGGTCAAAGGCTTCTAT<br>CCCAGCGACATCGCCGTGGAGTGGGAGAGCAATGGGCAGCCGGAGAACAACTACAAGACCAC<br>GCCTCCCGTGCTTGACTCCGACGGCTCCTTCTTCCTCTACAGCAAGCTCACCGTTGACAAGA<br>GCAGGTGGCAGCAGGGGAACGTCTTCTCATGCTCCGTGATGCATGAGGCTCTGCACAACCAC<br>TACACGCAGAAGAGCCTCTCCCTGTCTCCGGGTAAA | 650 |
| 33-1 LC | GATATACAACTGACTCAGAGTCCCAGCACTCTCAGTGCAAGTGTAGGCGATAGAGTAACTAT<br>AACCTGTCGCGCCTCAGAATCTCTTGATAATTATGGGATCCGATTTCTTACTTGGTTTCAGC<br>AAAAGCCTGGTAAAGCTCCTAAATTGCTCATGTATGCCGCCAGTAATCAGGGTTCAGGAGTT<br>CCTAGTCGTTTCTCTGGGTCAGGAAGCGGCACAGAATTTACCCTTACAATTTCCAGCCTCCA<br>GCCCGACGATTTCGCCACTTACTATTGCCAACAAACTAAAGAGGTTCCTTGGAGTTTTGGGT<br>GTGGCACCAAGGTAGAAGTAAAAACGTACGGTGGCAGCTCCCATCGTTTTTATCTTTCCCCCA<br>TCCGACGAGCAGCTCAAGAGTGGCACTGCCTCTGTAGTTTGTTTGCTGAATAACTTCTATCC<br>ACGTGAAGCAAAAGTACAGTGGAAGGTCGATAATGCCCTTCAGAGCGGTAACAGCCAAGAAA<br>GTGTTACCGAGCAAGATTCCAAAGATTCCACTTACAGTCTGTCCAGCACATTGACACTGAGT<br>AAGGCTGATTACGAAAAACACAAGGTGTACGCATGCGAGGTGACACACCAAGGTCTTTCATC<br>TCCTGTAACTAAGAGCTTTAACCGGGGAGAATGTGGTGGTGGGGGCAGCGGGGGCGGAGGTA<br>GTGGAGGCGGCGGTAGTGAACCAAAGAGTAGTGACAAAACTCACACGTGCCCACCGTGCCCA<br>GCACCTGAACTCCTGGGGGGACCGTCAGTCTTCCTCTTCCCCCCAAAACCCAAGGACACCCT<br>CATGATCTCCCGGACCCCTGAGGTCACATGCCGTGGTGGTGGACGTGAGCCACGAAGACCCTG<br>AGGTCAAGTTCAACTGGTACGTGGACGGCGTGGAGGTGCATAATGCCAAGACAAAGCCGCGG<br>GAGGAGCAGTACAACAGCACGTACCGTGTGGTCAGCGTCCTCACCGTCCTGCACCAGGACTG<br>GCTGAATGGCAAGGAGTACAAGTGCAAGGTCTCCAACAAAGCCCTCCCAGCCCCCATCGAGA<br>AAACCATCTCCAAAGCCAAAGGGCAGCCCCGAGAACCACAGGTGTACACCCTGCCCCCATCC<br>CGGGAGGAGATGACCAAGAACCAGGTCAGCCTGTGGTGCCTGGTCAAAGGCTTCTATCCCAG<br>CGACATCGCCGTGGAGTGGGAGAGCAATGGGCAGCCGGAGAACAACTACAAGACCACGCCTC<br>CCGTGCTGGACTCCGACGGCTCCTTCTTCCTCTACAGCAAGCTCACCGTGGACAAGAGCAGG<br>TGGCAGCAGGGGAACGTCTTCTCATGCTCCGTGATGCATGAGGCTCTGCACAACCACTACAC<br>GCAGAAGAGCCTCTCCCTGTCTCCGGGTAAA | 651 |
| 33-2 HC | CAGGTACAGCTTGTGCAATCTGGAGCTGAGGTCAAAAAACCAGGAAGTTCTGTAAAGGTGTC | 652 |

| | | |
|---|---|---|
| | TTGTAAGGCATCTGGTTACACATTTACCGATTACAACATGCATTGGGTACGTCAAGCCCCTG GCCAGTGCCTGGAATGGATCGGATATATATACCCCTACAACGGTGGAACAGGTTACAACCAA AAATTCAAAAGCAAGGCTACTATCACTGCCGACGAGAGCACTAACACCGCTTACATGGAACT GTCTTCCTTGCGTTCAGAAGACACTGCCGTGTATTATTGTGCTCGGGGGCGACCCGCAATGG ACTATTGGGGCCAAGGTACATTGGTGACTGTCAGTTCCGCTAGCACCAAAGGACCTAGTGTT TTTCCTCTTGCCCCTTCCTCAAAGTCTACCTCTGGGGGGACAGCCGCTCTGGGCTGCCTGGT CAAGGATTATTTCCCAGAGCCTGTCACTGTCAGTTGGAACTCTGGAGCCTTGACTTCTGGTG TTCATACATTTCCTGCTGTCCTTCAAAGCAGCGGCTTGTACTCATTGTCTTCTGTTGTGACA GTACCCTCAAGCAGCCTCGGCACTCAGACATACATCTGCAATGTCAACCACAAACCCTCAAA TACAAAGGTAGATAAAAAAGTCGAACCAAAGTCTTGTGACAAAACTCACACGTGCCCACCGT GCCCAGCACCTGAACTCCTGGGGGGACCGTCAGTCTTCCTCTTCCCCCCAAAACCCAAGGAC ACCCTCATGATCTCCCGGACCCCTGAGGTCACATGCGTGGTGGTGGACGTGAGCCACGAAGA CCCTGAGGTCAAGTTCAACTGGTACGTGGACGGCGTGGAGGTGCATAATGCCAAGACAAAGC CGCGGGAGGAGCAGTACAACAGCACGTACCGTGTGGTCAGCGTCCTCACCGTCCTGCACCAG GACTGGCTGAATGGCAAGGAGTACAAGTGCAAGGTCTCCAACAAAGCCCTCCCAGCCCCCAT CGAGAAAACCATCTCCAAAGCCAAAGGGCAGCCCCGAGAACCACAGGTGTACACCCTGCCCC CATGCCGGGAGGAGATGACCAAGAACCAGGTCAGCCTGTGGTGCCTGGTCAAAGGCTTCTAT CCCAGCGACATCGCCGTGGAGTGGGAGAGCAATGGGCAGCCGGAGAACAACTACAAGACCAC GCCTCCCGTGCTGGACTCCGACGGCTCCTTCTTCCTCTACAGCAAGCTCACCGTGGACAAGA GCAGGTGGCAGCAGGGGAACGTCTTCTCATGCTCCGTGATGCATGAGGCTCTGCACAACCAC TACACGCAGAAGAGCCTCTCCCTGTCTCCGGGTAAA | |
| 33-2 LC | GACATCCAAATGACACAGTCCCCTAGCTCACTTTCAGCAAGCGTGGGGGACCGAGTTACTAT CACATGCCGCGCAAGTGAGTCTGTGGACAACTATGGAATATCATTCATGAACTGGTTCCAGC AGAAACCTGGGAAAGCACCCAAGCTGCTTATCTACGCAGCAAGTAATCAGGGTAGTGGCGTC CCTTCTCGATTCAGTGGGAGCGGTAGCGGCACCGACTTCACCCTTACCATCTCATCACTTCA ACCCGATGATTTTGCTACCTACTATTGCCAGCAATCCAAGGAAGTTCCTTGGACCTTCGGGT GTGGGACAAAGGTAGAGATTAAACGTACGGTGGCAGCTCCCAGCGTTTTTATCTTTCCCCCA TCCGACGAGCAGCTCAAGAGTGGCACTGCCTCTGTAGTTTGTTTGCTGAATAACTTCTATCC ACGTGAAGCAAAAGTACAGTGGAAGGTCGATAATGCCCTTCAGAGCGGTAACAGCCAAGAAA GTGTTACCGAGCAAGATTCCAAAGATTCCACTTACAGTCTGTCCAGCACATTGACACTGAGT AAGGCTGATTACGAAAAACACAAGGTGTACGCATGCGAGGTGACACACCAAGGTCTTTCATC TCCTGTAACTAAGAGCTTTAACCGGGGAGAATGTGGTGGTGGGGGGCAGCGGGGGCGGAGGTA GTGGAGGCGGCGGTAGTGAACCAAAGAGTAGTGACAAAACTCACACGTGCCCACCGTGCCCA GCACCTGAACTCCTGGGGGGACCGTCAGTCTTCCTCTTCCCCCCAAAACCCAAGGACACCCT CATGATCTCCCGGACCCCTGAGGTCACATGCGTGGTGGTGGACGTGAGCCACGAAGACCCTG AGGTCAAGTTCAACTGGTACGTGGACGGCGTGGAGGTGCATAATGCCAAGACAAAGCCGCGG GAGGAGCAGTACAACAGCACGTACCGTGTGGTCAGCGTCCTCACCGTCCTGCACCAGGACTG GCTGAATGGCAAGGAGTACAAGTGCAAGGTCTCCAACAAAGCCCTCCCAGCCCCCATCGAGA AAACCATCTCCAAAGCCAAAGGGCAGCCCCGAGAACCACAGGTGTACACCCTGCCCCCATGC CGGGAGGAGATGACCAAGAACCAGGTCAGCCTGTGGTGCCTGGTCAAAGGCTTCTATCCCAG CGACATCGCCGTGGAGTGGGAGAGCAATGGGCAGCCGGAGAACAACTACAAGACCACGCCTC CCGTGCTGGACTCCGACGGCTCCTTCTTCCTCTACAGCAAGCTCACCGTGGACAAGAGCAGG TGGCAGCAGGGGAACGTCTTCTCATGCTCCGTGATGCATGAGGCTCTGCACAACCACTACAC GCAGAAGAGCCTCTCCCTGTCTCCGGGTAAA | 653 |
| 33-3 HC | CAAGTCCAACTTCAGCAGCCTGGAGCTGAGGTAGTGAAACCCGGCGCATCTGTAAAAATGAG CTGCAAAGCATCAGGTTACACATTTACATCCTACTACATCCATTGGATCAAGCAAACACCAG GCCAATGTCTTGAGTGGGTTGGCGTCATTTACCCAGGAAACGATGATATATCTTACAATCAG AAATTTCAAGGGAAAGCCACACTTACAGCCGACAAGAGTTCCACAACTGCATATATGCAACT CTCCTCCCTGACATCTGAAGACAGTGCCGTATACTATTGTGCTCGTGAAGTCAGGCTCAGAT ACTTTGACGTGTGGGGTCAAGGCACAACCGTCACCGTCAGTAGCGCTAGCACCAAAGGACCT | 654 |

| | | |
|---|---|---|
| | AGTGTTTTTCCTCTTGCCCCTTCCTCAAAGTCTACCTCTGGGGGGACAGCCGCTCTGGGCTG CCTGGTCAAGGATTATTTCCCAGAGCCTGTCACTGTCAGTTGGAACTCTGGAGCCTTGACTT CTGGTGTTCATACATTTCCTGCTGTCCTTCAAAGCAGCGGCTTGTACTCATTGTCTTCTGTT GTGACAGTACCCTCAAGCAGCCTCGGCACTCAGACATACATCTGCAATGTCAACCACAAACC CTCAAATACAAAGGTAGATAAAAAAAGTCGAACCAAAGTCTTGTGACAAAACTCACACGTGCC CACCGTGCCCAGCACCTGAACTCCTGGGGGGACCGTCAGTCTTCCTCTTCCCCCCAAAACCC AAGGACACCCTCATGATCTCCCGGACCCCTGAGGTCACATGCGTGGTGGTGGACGTGAGCCA CGAAGACCCTGAGGTCAAGTTCAACTGGTACGTGGACGGCGTGGAGGTGCATAATGCCAAGA CAAAGCCGCGGGAGGAGCAGTACAACAGCACGTACCGTGTGGTCAGCGTCCTCACCGTCCTG CACCAGGACTGGCTGAATGGCAAGGAGTACAAGTGCAAGGTCTCCAACAAAGCCCTCCCAGC CCCCATCGAGAAAACCATCTCCAAAGCCAAAGGGCAGCCCCGAGAACCACAGGTGTACACCC TGCCCCCATGCCGGGAGGAGATGACCAAGAACCAGGTCAGCCTGTGGTGCCTGGTCAAAGGC TTCTATCCCAGCGACATCGCCGTGGAGTGGGAGAGCAATGGGCAGCCGGAGAACAACTACAA GACCACGCCTCCCGTGCTGGACTCCGACGGCTCCTTCTTCCTCTACAGCAAGCTCACCGTGG ACAAGAGCAGGTGGCAGCAGGGGAACGTCTTCTCATGCTCCGTGATGCATGAGGCTCTGCAC AACCACTACACGCAGAAGAGCCTCTCCCTGTCTCCGGGTAAA | |
| 33-3 LC | GAGATAGTCCTCACCCAATCACCAGGTAGTCTGGCTGTTAGCCCAGGTGAGCGAGTTACTAT GTCTTGCAAGTCCTCTCAATCTGTGTTCTTTTCATCAAGTCAGAAGAATTATCTTGCCTGGT ATCAGCAGATTCCTGGTCAATCTCCCCGACTGCTTATTTACTGGGCTTCAACTCGGGAGTCA GGGGTTCCCGACCGATTCACAGGGTCTGGGTCAGGAACTGACTTTACCCTTACTATCAGCTC TGTGCAGCCTGAAGACCTCGCAATATATTATTGTCATCAGTACCTCTCTTCTCGCACTTTTG GATGTGGCACCAAATTGGAGATTAAGCGTACGGTGGCAGCTCCCAGCGTTTTTATCTTTCCC CCATCCGACGAGCAGCTCAAGAGTGGCACTGCCTCTGTAGTTTGTTTGCTGAATAACTTCTA TCCACGTGAAGCAAAAGTACAGTGGAAGGTCGATAATGCCCTTCAGAGCGGTAACAGCCAAG AAAGTGTTACCGAGCAAGATTCCAAAGATTCCACTTACAGTCTGTCCAGCACATTGACACTG AGTAAGGCTGATTACGAAAAACACAAGGTGTACGCATGCGAGGTGACACACCAAGGTCTTTC ATCTCCTGTAACTAAGAGCTTTAACCGGGGAGAATGTGGTGGTGGGGGCAGCGGGGGCGGAG GTAGTGGAGGCGGCGGTAGTGAACCAAAGAGTAGTGACAAAACTCACACGTGCCCACCGTGC CCAGCACCTGAACTCCTGGGGGGACCGTCAGTCTTCCTCTTCCCCCCAAAACCCAAGGACAC CCTCATGATCTCCCGGACCCCTGAGGTCACATGCGTGGTGGTGGACGTGAGCCACGAAGACC CTGAGGTCAAGTTCAACTGGTACGTGGACGGCGTGGAGGTGCATAATGCCAAGACAAAGCCG CGGGAGGAGCAGTACAACAGCACGTACCGTGTGGTCAGCGTCCTCACCGTCCTGCACCAGGA CTGGCTGAATGGCAAGGAGTACAAGTGCAAGGTCTCCAACAAAGCCCTCCCAGCCCCCATCG AGAAAACCATCTCCAAAGCCAAAGGGCAGCCCCGAGAACCACAGGTGTACACCCTGCCCCCA TGCCGGGAGGAGATGACCAAGAACCAGGTCAGCCTGTGGTGCCTGGTCAAAGGCTTCTATCC CAGCGACATCGCCGTGGAGTGGGAGAGCAATGGGCAGCCGGAGAACAACTACAAGACCACGC CTCCCGTGCTGGACTCCGACGGCTCCTTCTTCCTCTACAGCAAGCTCACCGTGGACAAGAGC AGGTGGCAGCAGGGGAACGTCTTCTCATGCTCCGTGATGCATGAGGCTCTGCACAACCACTA CACGCAGAAGAGCCTCTCCCTGTCTCCGGGTAAA | 655 |
| 33-4 HC | GAGGTTCAGTTGGTTCAGTCAGGAGCAGAGGTCAAAAAACCTGGAAGCTCTGTCAAAGTTAG TTGTAAGGCCAGTGGATACACCATAACCGATTCAAATATACATTGGGTTAGGCAAGCACCAG GACAGTCCTTGGAATGGATCGGGTACATCTATCCATATAATGGGGGCACCGATTACAACCAA AAGTTTAAGAATCGCGCCACACTCACTGTTGATAATCCAACCAATACAGCATACATGGAGTT GAGCAGTCTTCGGTCCGAGGACACTGCTTTTTACTATTGTGTGAACGGTAACCCATGGTTGG CCTATTGGGGCCAAGGTACACTTGTAACAGTTTCATCCGCTAGCACCAAAGGACCTAGTGTT TTTCCTCTTGCCCCTCAGGTACAGCTTGTGCAATCTGGAGCTGAGGTCAAAAAACCAGGAAG TTCTGTAAAGGTGTCTTGTAAGGCATCTGGTTACACATTTACCGATTACAACATGCATTGGG TACGTCAAGCCCCTGGCCAGTGCCTGGAATGGATCGGATATATATACCCCTACAACGGTGGA ACAGGTTACAACCAAAAAATTCAAAAGCAAGGCTACTATCACTGCCGACGAGAGCACTAACAC CGCTTACATGGAACTGTCTTCCTTGCGTTCAGAAGACACTGCCGTGTATTATTGTGCTCGGG | 656 |

| | | |
|---|---|---|
| | GGCGACCCGCAATGGACTATTGGGGCCAAGGTACATTGGTGACTGTCAGTTCCGCTAGCACC AAAGGACCTAGTGTTTTTCCTCTTGCCCCTTCCTCAAAGTCTACCTCTGGGGGGACAGCCGC TCTGGGCTGCCTGGTCAAGGATTATTTCCCAGAGCCTGTCACTGTCAGTTGGAACTCTGGAG CCTTGACTTCTGGTGTTCATACATTTCCTGCTGTCCTTCAAAGCAGCGGCTTGTACTCATTG TCTTCTGTTGTGACAGTACCCTCAAGCAGCCTCGGCACTCAGACATACATCTGCAATGTCAA CCACAAACCCTCAAATACAAAGGTAGATAAAAAAGTCGAACCAAAGTCTTGTGACAAAACTC ACACGTGCCCACCGTGCCCAGCACCTGAACTCCTGGGGGGACCGTCAGTCTTCCTCTTCCCC CCAAAACCCAAGGACACCCTCATGATCTCCCGGACCCCTGAGGTCACATGCGTGGTGGTGGA CGTGAGCCACGAAGACCCTGAGGTCAAGTTCAACTGGTACGTGGACGGCGTGGAGGTGCATA ATGCCAAGACAAAGCCGCGGGAGGAGCAGTACAACAGCACGTACCGTGTGGTCAGCGTCCTC ACCGTCCTGCACCAGGACTGGCTGAATGGCAAGGAGTACAAGTGCAAGGTCTCCAACAAAGC CCTCCCAGCCCCCATCGAGAAAACCATCTCCAAAGCCAAAGGGCAGCCCCGAGAACCACAGG TGTACACCCTGCCCCCATGCCGGGAGGAGATGACCAAGAACCAGGTCAGCCTGTGGTGCCTG GTCAAAGGCTTCTATCCCAGCGACATCGCCGTGGAGTGGGAGAGCAATGGGCAGCCGGAGAA CAACTACAAGACCACGCCTCCCGTGCTGGACTCCGACGGCTCCTTCTTCCTCTACAGCAAGC TCACCGTGGACAAGAGCAGGTGGCAGCAGGGGAACGTCTTCTCATGCTCCGTGATGCATGAG GCTCTGCACAACCACTACACGCAGAAGAGCCTCTCCCTGTCTCCGGGTAAA | |
| 33-4 LC | GATATACAACTGACTCAGAGTCCCAGCACTCTCAGTGCAAGTGTAGGCGATAGAGTAACTAT AACCTGTCGCGCCTCAGAATCTCTTGATAATTATGGGATCCGATTTCTTACTTGGTTTCAGC AAAAGCCTGGTAAAGCTCCTAAATTGCTCATGTATGCCGCCAGTAATCAGGGTTCAGGAGTT CCTAGTCGTTTCTCTGGGTCAGGAAGCGGCACAGAATTTACCCTTACAATTTCCAGCCTCCA GCCCGACGATTTCGCCACTTACTATTGCCAACAAACTAAAGAGGTTCCTTGGAGTTTTGGGC AAGGCACCAAGGTAGAAGTAAAACGTACGGTGGCCGCTCCCTCCGTTTTTATCTTTCCCCCA GACATCCAAATGACACAGTCCCCTAGCTCACTTTCAGCAAGCGTGGGGGACCGAGTTACTAT CACATGCCGCGCAAGTGAGTCTGTGGACAACTATGGAATATCATTCATGAACTGGTTCCAGC AGAAACCTGGGAAAGCACCCAAGCTGCTTATCTACGCAGCAAGTAATCAGGGTAGTGGCGTC CCTTCTCGATTCAGTGGGAGCGGTAGCGGCACCGACTTCACCCTTACCATCTCATCACTTCA ACCCGATGATTTTGCTACCTACTATTGCCAGCAATCCAAGGAAGTTCCTTGGACCTTCGGGT GTGGGACAAAGGTAGAGATTAAACGTACGGTGGCAGCTCCCAGCGTTTTTATCTTTCCCCCA TCCGACGAGCAGCTCAAGAGTGGCACTGCCTCTGTAGTTTGTTTGCTGAATAACTTCTATCC ACGTGAAGCAAAAGTACAGTGGAAGGTCGATAATGCCCTTCAGAGCGGTAACAGCCAAGAAA GTGTTACCGAGCAAGATTCCAAAGATTCCACTTACAGTCTGTCCAGCACATTGACACTGAGT AAGGCTGATTACGAAAAACACAAGGTGTACGCATGCGAGGTGACACACCAAGGTCTTTCATC TCCTGTAACTAAGAGCTTTAACCGGGGGAGAATGTGGTGGTGGGGGCAGCGGGGGCGGAGGTA GTGGAGGCGGCGGTAGTGAACCAAAGAGTAGTGACAAAACTCACACGTGCCCACCGTGCCCA GCACCTGAACTCCTGGGGGGACCGTCAGTCTTCCTCTTCCCCCCAAAACCCAAGGACACCCT CATGATCTCCCGGACCCCTGAGGTCACATGCGTGGTGGTGGACGTGAGCCACGAAGACCCTG AGGTCAAGTTCAACTGGTACGTGGACGGCGTGGAGGTGCATAATGCCAAGACAAAGCCGCGG GAGGAGCAGTACAACAGCACGTACCGTGTGGTCAGCGTCCTCACCGTCCTGCACCAGGACTG GCTGAATGGCAAGGAGTACAAGTGCAAGGTCTCCAACAAAGCCCTCCCAGCCCCCATCGAGA AAACCATCTCCAAAGCCAAAGGGCAGCCCCGAGAACCACAGGTGTACACCCTGCCCCCATGC CGGGAGGAGATGACCAAGAACCAGGTCAGCCTGTGGTGCCTGGTCAAAGGCTTCTATCCCAG CGACATCGCCGTGGAGTGGGAGAGCAATGGGCAGCCGGAGAACAACTACAAGACCACGCCTC CCGTGCTGGACTCCGACGGCTCCTTCTTCCTCTACAGCAAGCTCACCGTGGACAAGAGCAGG TGGCAGCAGGGGAACGTCTTCTCATGCTCCGTGATGCATGAGGCTCTGCACAACCACTACAC GCAGAAGAGCCTCTCCCTGTCTCCGGGTAAA | 657 |
| 33-5 HC | CAAGTCCAACTTCAGCAGCCTGGAGCTGAGGTAGTGAAACCCGGCGCATCTGTAAAAATGAG CTGCAAAGCATCAGGTTACACATTTACATCCTACTACATCCATTGGATCAAGCAAACACCAG GCCAAGGTCTTGAGTGGGTTGGCGTCATTTACCCAGGAAACGATGATATATCTTACAATCAG AAATTTCAAGGGAAAGCCACACTTACAGCCGACAAGAGTTCCACAACTGCATATATGCAACT | 658 |

| | | |
|---|---|---|
| | CTCCTCCCTGACATCTGAAGACAGTGCCGTATACTATTGTGCTCGTGAAGTCAGGCTCAGAT<br>ACTTTGACGTGTGGGGTCAAGGCACAACCGTCACCGTCAGTAGCGCTAGCACCAAAGGACCT<br>AGTGTTTTTCCTCTTGCCCCTCAGGTACAGCTTGTGCAATCTGGAGCTGAGGTCAAAAAACC<br>AGGAAGTTCTGTAAAGGTGTCTTGTAAGGCATCTGGTTACACATTTACCGATTACAACATGC<br>ATTGGGTACGTCAAGCCCCTGGCCAGTGCCTGGAATGGATCGGATATATATACCCCTACAAC<br>GGTGGAACAGGTTACAACCAAAAATTCAAAAGCAAGGCTACTATCACTGCCGACGAGAGCAC<br>TAACACCGCTTACATGGAACTGTCTTCCTTGCGTTCAGAAGACACTGCCGTGTATTATTGTG<br>CTCGGGGGCGACCCGCAATGGACTATTGGGGCCAAGGTACATTGGTGACTGTCAGTTCCGCT<br>AGCACCAAAGGACCTAGTGTTTTTCCTCTTGCCCCTTCCTCAAAGTCTACCTCTGGGGGGAC<br>AGCCGCTCTGGGCTGCCTGGTCAAGGATTATTTCCCAGAGCCTGTCACTGTCAGTTGGAACT<br>CTGGAGCCTTGACTTCTGGTGTTCATACATTTCCTGCTGTCCTTCAAAGCAGCGGCTTGTAC<br>TCATTGTCTTCTGTTGTGACAGTACCCTCAAGCAGCCTCGGCACTCAGACATACATCTGCAA<br>TGTCAACCACAAACCCTCAAATACAAAGGTAGATAAAAAAGTCGAACCAAAGTCTTGTGACA<br>AAACTCACACGTGCCCACCGTGCCCAGCACCTGAACTCCTGGGGGGACCGTCAGTCTTCCTC<br>TTCCCCCCAAAACCCAAGGACACCCTCATGATCTCCCGGACCCCTGAGGTCACATGCGTGGT<br>GGTGGACGTGAGCCACGAAGACCCTGAGGTCAAGTTCAACTGGTACGTGGACGGCGTGGAGG<br>TGCATAATGCCAAGACAAAGCCGCGGGAGGAGCAGTACAACAGCACGTACCGTGTGGTCAGC<br>GTCCTCACCGTCCTGCACCAGGACTGGCTGAATGGCAAGGAGTACAAGTGCAAGGTCTCCAA<br>CAAAGCCCTCCCAGCCCCCATCGAGAAAACCATCTCCAAAGCCAAAGGGCAGCCCCGAGAAC<br>CACAGGTGTACACCCTGCCCCCATGCCGGGAGGAGATGACCAAGAACCAGGTCAGCCTGTGG<br>TGCCTGGTCAAAGGCTTCTATCCCAGCGACATCGCCGTGGAGTGGGAGAGCAATGGGCAGCC<br>GGAGAACAACTACAAGACCACGCCTCCCGTGCTGGACTCCGACGGCTCCTTCTTCCTCTACA<br>GCAAGCTCACCGTGGACAAGAGCAGGTGGCAGCAGGGGAACGTCTTCTCATGCTCCGTGATG<br>CATGAGGCTCTGCACAACCACTACACGCAGAAGAGCCTCTCCCTGTCTCCGGGTAAA | |
| 33-5 LC | GAGATAGTCCTCACCCAATCACCAGGTAGTCTGGCTGTTAGCCCAGGTGAGCGAGTTACTAT<br>GTCTTGCAAGTCCTCTCAATCTGTGTTCTTTTCATCAAGTCAGAAGAATTATCTTGCCTGGT<br>ATCAGCAGATTCCTGGTCAATCTCCCCGACTGCTTATTTACTGGGCTTCAACTCGGGAGTCA<br>GGGGTTCCCGACCGATTCACAGGGTCTGGGTCAGGAACTGACTTTACCCTTACTATCAGCTC<br>TGTGCAGCCTGAAGACCTCGCAATATATTATTGTCATCAGTACCTCTCTTCTCGCACTTTTG<br>GACAGGGCACCAAATTGGAGATTAAGCGTACGGTGGCCGCTCCCTCCGTTTTTATCTTTCCC<br>CCAGACATCCAAATGACACAGTCCCCTAGCTCACTTTCAGCAAGCGTGGGGGACCGAGTTAC<br>TATCACATGCCGCGCAAGTGAGTCTGTGGACAACTATGGAATATCATTCATGAACTGGTTCC<br>AGCAGAAACCTGGGAAAGCACCCAAGCTGCTTATCTACGCAGCAAGTAATCAGGGTAGTGGC<br>GTCCCTTCTCGATTCAGTGGGAGCGGTAGCGGCACCGACTTCACCCTTACCATCTCATCACT<br>TCAACCCGATGATTTTGCTACCTACTATTGCCAGCAATCCAAGGAAGTTCCTTGGACCTTCG<br>GGTGTGGGACAAAGGTAGAGATTAAACGTACGGTGGCAGCTCCCAGCGTTTTTATCTTTCCC<br>CCATCCGACGAGCAGCTCAAGAGTGGCACTGCCTCTGTAGTTTGTTTGCTGAATAACTTCTA<br>TCCACGTGAAGCAAAAGTACAGTGGAAGGTCGATAATGCCCTTCAGAGCGGTAACAGCCAAG<br>AAAGTGTTACCGAGCAAGATTCCAAAGATTCCACTTACAGTCTGTCCAGCACATTGACACTG<br>AGTAAGGCTGATTACGAAAAACACAAGGTGTACGCATGCGAGGTGACACACCAAGGTCTTTC<br>ATCTCCTGTAACTAAGAGCTTTAACCGGGGGAGAATGTGGTGGTGGGGGCAGCGGGGGCGGAG<br>GTAGTGGAGGCGGCGGTAGTGAACCAAAGAGTAGTGACAAAACTCACACGTGCCCACCGTGC<br>CCAGCACCTGAACTCCTGGGGGGACCGTCAGTCTTCCTCTTCCCCCCAAAACCCAAGGACAC<br>CCTCATGATCTCCCGGACCCCTGAGGTCACATGCGTGGTGGTGGACGTGAGCCACGAAGACC<br>CTGAGGTCAAGTTCAACTGGTACGTGGACGGCGTGGAGGTGCATAATGCCAAGACAAAGCCG<br>CGGGAGGAGCAGTACAACAGCACGTACCGTGTGGTCAGCGTCCTCACCGTCCTGCACCAGGA<br>CTGGCTGAATGGCAAGGAGTACAAGTGCAAGGTCTCCAACAAAGCCCTCCCAGCCCCCATCG<br>AGAAAACCATCTCCAAAGCCAAAGGGCAGCCCCGAGAACCACAGGTGTACACCCTGCCCCCA<br>TGCCGGGAGGAGATGACCAAGAACCAGGTCAGCCTGTGGTGCCTGGTCAAAGGCTTCTATCC<br>CAGCGACATCGCCGTGGAGTGGGAGAGCAATGGGCAGCCGGAGAACAACTACAAGACCACGC | 659 |

| | | |
|---|---|---|
| | CTCCCGTGCTGGACTCCGACGGCTCCTTCTTCCTCTACAGCAAGCTCACCGTGGACAAGAGC<br>AGGTGGCAGCAGGGGAACGTCTTCTCATGCTCCGTGATGCATGAGGCTCTGCACAACCACTA<br>CACGCAGAAGAGCCTCTCCCTGTCTCCGGGTAAA | |
| 33-6 HC | CAGGTACAGCTTGTGCAATCTGGAGCTGAGGTCAAAAAACCAGGAAGTTCTGTAAAGGTGTC<br>TTGTAAGGCATCTGGTTACACATTTACCGATTACAACATGCATTGGGTACGTCAAGCCCCTG<br>GCCAGGGGCCTGGAATGGATCGGATATATATACCCCTACAACGGTGGAACAGGTTACAACCAA<br>AAATTCAAAAGCAAGGCTACTATCACTGCCGACGAGAGCACTAACACCGCTTACATGGAACT<br>GTCTTCCTTGCGTTCAGAAGACACTGCCGTGTATTATTGTGCTCGGGGGCGACCCGCAATGG<br>ACTATTGGGGCCAAGGTACATTGGTGACTGTCAGTTCCGCTAGCACCAAAGGACCTAGTGTT<br>TTTCCTCTTGCCCCTGAGGTTCAGTTGGTTCAGTCAGGAGCAGAGGTCAAAAAACCTGGAAG<br>CTCTGTCAAAGTTAGTTGTAAGGCCAGTGGATACACCATAACCGATTCAAATATACATTGGG<br>TTAGGCAAGCACCAGGACAGTGCTTGGAATGGATCGGGTACATCTATCCATATAATGGGGGC<br>ACCGATTACAACCAAAAGTTTAAGAATCGCGCCACACTCACTGTTGATAATCCAACCAATAC<br>AGCATACATGGAGTTGAGCAGTCTTCGGTCCGAGGACACTGCTTTTTACTATTGTGTGAACG<br>GTAACCCATGGTTGGCCTATTGGGGCCAAGGTACACTTGTAACAGTTTCATCTGCTAGCACC<br>AAAGGACCTAGTGTTTTTCCTCTTGCCCCTTCCTCAAAGTCTACCTCTGGGGGGACAGCCGC<br>TCTGGGCTGCCTGGTCAAGGATTATTTCCCAGAGCCTGTCACTGTCAGTTGGAACTCTGGAG<br>CCTTGACTTCTGGTGTTCATACATTTCCTGCTGTCCTTCAAAGCAGCGGCTTGTACTCATTG<br>TCTTCTGTTGTGACAGTACCCTCAAGCAGCCTCGGCACTCAGACATACATCTGCAATGTCAA<br>CCACAAACCCTCAAATACAAAGGTAGATAAAAAAGTCGAACCAAAGTCTTGTGACAAAACTC<br>ACACGTGCCCACCGTGCCCAGCACCTGAACTCCTGGGGGGACCGTCAGTCTTCCTCTTCCCC<br>CCAAAACCCAAGGACACCCTCATGATCTCCCGGACCCCTGAGGTCACATGCGTGGTGGTGGA<br>CGTGAGCCACGAAGACCCTGAGGTCAAGTTCAACTGGTACGTGGACGGCGTGGAGGTGCATA<br>ATGCCAAGACAAAGCCGCGGGAGGAGCAGTACAACAGCACGTACCGTGTGGTCAGCGTCCTC<br>ACCGTCCTGCACCAGGACTGGCTGAATGGCAAGGAGTACAAGTGCAAGGTCTCCAACAAAGC<br>CCTCCCAGCCCCCATCGAGAAAACCATCTCCAAAGCCAAAGGGCAGCCCCGAGAACCACAGG<br>TGTACACCCTGCCCCCATGCCGGGAGGAGATGACCAAGAACCAGGTCAGCCTGTGGTGCCTG<br>GTCAAAGGCTTCTATCCCAGCGACATCGCCGTGGAGTGGGAGAGCAATGGGCAGCCGGAGAA<br>CAACTACAAGACCACGCCTCCCGTGCTGGACTCCGACGGCTCCTTCTTCCTCTACAGCAAGC<br>TCACCGTGGACAAGAGCAGGTGGCAGCAGGGGAACGTCTTCTCATGCTCCGTGATGCATGAG<br>GCTCTGCACAACCACTACACGCAGAAGAGCCTCTCCCTGTCTCCGGGTAAA | 660 |
| 33-6 LC | GACATCCAAATGACACAGTCCCCTAGCTCACTTTCAGCAAGCGTGGGGGACCGAGTTACTAT<br>CACATGCCGCGCAAGTGAGTCTGTGGACAACTATGGAATATCATTCATGAACTGGTTCCAGC<br>AGAAACCTGGGAAAGCACCCAAGCTGCTTATCTACGCAGCAAGTAATCAGGGTAGTGGCGTC<br>CCTTCTCGATTCAGTGGGAGCGGTAGCGGCACCGACTTCACCCCTTACCATCTCATCACTTCA<br>ACCCGATGATTTTGCTACCTACTATTGCCAGCAATCCAAGGAAGTTCCTTGGACCTTCGGGC<br>AAGGGACAAAGGTAGAGATTAAACGTACGGTGGCAGCTCCCAGCGTTTTTATCTTTCCCCCA<br>TCCGACGAGCAGCTCAAGAGTGGCACTGCCTCTGTAGTTTGTTTGCTGAATAACTTCTATCC<br>ACGTGAAGCAAAAGTACAGTGGAAGGTCGATAATGCCCTTCAGAGCGGTAACAGCCAAGAAA<br>GTGTTACCGAGCAAGATTCCAAAGATTCCACTTACAGTCTGTCCAGCACATTGACACTGAGT<br>AAGGCTGATTACGAAAAACACAAGGTGTACGCATGCGAGGTGACACACCAAGGTCTTTCATC<br>TCCTGTAACTAAGAGCTTTAACCGGGGAGAATGTGGTGGTGGGGGCAGCGGGGGCGGAGGTA<br>GTGGAGGCGGCGGTAGTGAACCAAAGAGTAGTGACAAAACTCACACGTGCCCACCGTGCCCA<br>GCACCTGAACTCCTGGGGGGACCGTCAGTCTTCCTCTTCCCCCCAAAACCCAAGGACACCCT<br>CATGATCTCCCGGACCCCTGAGGTCACATGCGTGGTGGTGGACGTGAGCCACGAAGACCCTG<br>AGGTCAAGTTCAACTGGTACGTGGACGGCGTGGAGGTGCATAATGCCAAGACAAAGCCGCGG<br>GAGGAGCAGTACAACAGCACGTACCGTGTGGTCAGCGTCCTCACCGTCCTGCACCAGGACTG<br>GCTGAATGGCAAGGAGTACAAGTGCAAGGTCTCCAACAAAGCCCTCCCAGCCCCCATCGAGA<br>AAACCATCTCCAAAGCCAAAGGGCAGCCCCGAGAACCACAGGTGTACACCCTGCCCCCATGC<br>CGGGAGGAGATGACCAAGAACCAGGTCAGCCTGTGGTGCCTGGTCAAAGGCTTCTATCCCAG | 661 |

| | | |
|---|---|---|
| | CGACATCGCCGTGGAGTGGGAGAGCAATGGGCAGCCGGAGAACAACTACAAGACCACGCCTC CCGTGCTGGACTCCGACGGCTCCTTCTTCCTCTACAGCAAGCTCACCGTGGACAAGAGCAGG TGGCAGCAGGGGAACGTCTTCTCATGCTCCGTGATGCATGAGGCTCTGCACAACCACTACAC GCAGAAGAGCCTCTCCCTGTCTCCGGGTAAA | |
| 33-7 HC | CAGGTACAGCTTGTGCAATCTGGAGCTGAGGTCAAAAAACCAGGAAGTTCTGTAAAGGTGTC TTGTAAGGCATCTGGTTACACATTTACCGATTACAACATGCATTGGGTACGTCAAGCCCCTG GCCAGGGGCCTGGAATGGATCGGATATATATACCCCTACAACGGTGGAACAGGTTACAACCAA AAATTCAAAAGCAAGGCTACTATCACTGCCGACGAGAGCACTAACACCGCTTACATGGAACT GTCTTCCTTGCGTTCAGAAGACACTGCCGTGTATTATTGTGCTCGGGGGCGACCCGCAATGG ACTATTGGGGCCAAGGTACATTGGTGACTGTCAGTTCCGCTAGCACCAAAGGACCTAGTGTT TTTCCTCTTGCCCCTCAAGTCCAACTTCAGCAGCCTGGAGCTGAGGTAGTGAAACCCGGCGC ATCTGTAAAAATGAGCTGCAAAGCATCAGGTTACACATTTACATCCTACTACATCCATTGGA TCAAGCAAACACCAGGCCAATGTCTTGAGTGGGTTGGCGTCATTTACCCAGGAAACGATGAT ATATCTTACAATCAGAAATTTCAAGGGAAAGCCACACTTACAGCCGACAAGAGTTCCACAAC TGCATATATGCAACTCTCCTCCCTGACATCTGAAGACAGTGCCGTATACTATTGTGCTCGTG AAGTCAGGCTCAGATACTTTGACGTGTGGGGTCAAGGCACAACCGTCACCGTCAGTAGCGCT AGCACCAAAGGACCTAGTGTTTTTCCTCTTGCCCCTTCCTCAAAGTCTACCTCTGGGGGGAC AGCCGCTCTGGGCTGCCTGGTCAAGGATTATTTCCCAGAGCCTGTCACTGTCAGTTGGAACT CTGGAGCCTTGACTTCTGGTGTTCATACATTTCCTGCTGTCCTTCAAAGCAGCGGCTTGTAC TCATTGTCTTCTGTTGTGACAGTACCCTCAAGCAGCCTCGGCACTCAGACATACATCTGCAA TGTCAACCACAAACCCTCAAATACAAAGGTAGATAAAAAAGTCGAACCAAAGTCTTGTGACA AAACTCACACGTGCCCACCGTGCCCAGCACCTGAACTCCTGGGGGGACCGTCAGTCTTCCTC TTCCCCCCAAAACCCAAGGACACCCTCATGATCTCCCGGACCCCTGAGGTCACATGCGTGGT GGTGGACGTGAGCCACGAAGACCCTGAGGTCAAGTTCAACTGGTACGTGGACGGCGTGGAGG TGCATAATGCCAAGACAAAGCCGCGGGAGGAGCAGTACAACAGCACGTACCGTGTGGTCAGC GTCCTCACCGTCCTGCACCAGGACTGGCTGAATGGCAAGGAGTACAAGTGCAAGGTCTCCAA CAAAGCCCTCCCAGCCCCCATCGAGAAAACCATCTCCAAAGCCAAAGGGCAGCCCCGAGAAC CACAGGTGTACACCCTGCCCCCATGCCGGGAGGAGATGACCAAGAACCAGGTCAGCCTGTGG TGCCTGGTCAAAGGCTTCTATCCCAGCGACATCGCCGTGGAGTGGGAGAGCAATGGGCAGCC GGAGAACAACTACAAGACCACGCCTCCCGTGCTGGACTCCGACGGCTCCTTCTTCCTCTACA GCAAGCTCACCGTGGACAAGAGCAGGTGGCAGCAGGGGAACGTCTTCTCATGCTCCGTGATG CATGAGGCTCTGCACAACCACTACACGCAGAAGAGCCTCTCCCTGTCTCCGGGTAAA | 662 |
| 33-7 LC | GACATCCAAATGACACAGTCCCCTAGCTCACTTTCAGCAAGCGTGGGGGACCGAGTTACTAT CACATGCCGCGCAAGTGAGTCTGTGGACAACTATGGAATATCATTCATGAACTGGTTCCAGC AGAAACCTGGGAAAGCACCCAAGCTGCTTATCTACGCAGCAAGTAATCAGGGTAGTGGCGTC CCTTCTCGATTCAGTGGGAGCGGTAGCGGCACCGACTTCACCCTTACCATCTCATCACTTCA ACCCGATGATTTTGCTACCTACTATTGCCAGCAATCCAAGGAAGTTCCTTGGACCTTCGGGC AAGGGACAAAGGTAGAGATTAAACGTACGGTGGCCGCTCCCTCCGTTTTTATCTTTCCCCCA GAGATAGTCCTCACCCAATCACCAGGTAGTCTGGCTGTTAGCCCAGGTGAGCGAGTTACTAT GTCTTGCAAGTCCTCTCAATCTGTGTTCTTTTCATCAAGTCAGAAGAATTATCTTGCCTGGT ATCAGCAGATTCCTGGTCAATCTCCCCGACTGCTTATTTACTGGGCTTCAACTCGGGAGTCA GGGGTTCCCGACCGATTCACAGGGTCTGGGTCAGGAACTGACTTTACCCTTACTATCAGCTC TGTGCAGCCTGAAGACCTCGCAATATATTATTGTCATCAGTACCTCTCTTCTCGCACTTTTG GATGTGGCACCAAATTGGAGATTAAGCGTACGGTGGCAGCTCCCAGCGTTTTTATCTTTCCC CCATCCGACGAGCAGCTCAAGAGTGGCACTGCCTCTGTAGTTTGTTTGCTGAATAACTTCTA TCCACGTGAAGCAAAAGTACAGTGGAAGGTCGATAATGCCCTTCAGAGCGGTAACAGCCAAG AAAGTGTTACCGAGCAAGATTCCAAAGATTCCACTTACAGTCTGTCCAGCACATTGACACTG AGTAAGGCTGATTACGAAAAACACAAAGGTGTACGCATGCGAGGTGACACACCAAGGTCTTTC ATCTCCTGTAACTAAGAGCTTTAACCGGGGGAGAATGTGGTGGTGGGGGGCAGCGGGGGCGGAG GTAGTGGAGGCGGCGGTAGTGAACCAAAGAGTAGTGACAAAACTCACACGTGCCCACCGTGC | 663 |

CCAGCACCTGAACTCCTGGGGGGACCGTCAGTCTTCCTCTTCCCCCCCAAAACCCAAGGACAC
CCTCATGATCTCCCGGACCCCTGAGGTCACATGCGTGGTGGTGGACGTGAGCCACGAAGACC
CTGAGGTCAAGTTCAACTGGTACGTGGACGGCGTGGAGGTGCATAATGCCAAGACAAAGCCG
CGGGAGGAGCAGTACAACAGCACGTACCGTGTGGTCAGCGTCCTCACCGTCCTGCACCAGGA
CTGGCTGAATGGCAAGGAGTACAAGTGCAAGGTCTCCAACAAAGCCCTCCCAGCCCCCATCG
AGAAAACCATCTCCAAAGCCAAAGGGCAGCCCCGAGAACCACAGGTGTACACCCTGCCCCCA
TGCCCGGAGGAGATGACCAAGAACCAGGTCAGCCTGTGGTGCCTGGTCAAAGGCTTCTATCC
CAGCGACATCGCCGTGGAGTGGGAGAGCAATGGGCAGCCGGAGAACAACTACAAGACCACGC
CTCCCGTGCTGGACTCCGACGGCTCCTTCTTCCTCTACAGCAAGCTCACCGTGGACAAGAGC
AGGTGGCAGCAGGGGAACGTCTTCTCATGCTCCGTGATGCATGAGGCTCTGCACAACCACTA
CACGCAGAAGAGCCTCTCCCTGTCTCCGGGTAAA

Table 72 below shows the polypeptide sequences of the heavy chain variable region (VH) and light chain variable region (VL) of the engineered antibodies targeting CD33. Table 73 below shows the polynucleotide sequences encoding the polypeptides of the heavy chain variable region (VH) and light chain variable region (VL) of the engineered antibodies targeting CD33.

[Table 72]

| Name | | Sequence | SEQ ID NO |
|---|---|---|---|
| 33-1 | VH | EVQLVQSGAEVKKPGSSVKVSCKASGYTITDSNIHWVRQAPGQGLEWIGYIYPYNGGTDYNQKF KNRATLTVDNPTNTAYMELSSLRSEDTAFYYCVNGXPWLAYWGQGTLVTVSS | 664 |
| | VL | DIQLTQSPSTLSASVGDRVTITCRASESLDNYGIRFLTWFQQKPGKAPKLLMYAASNQGSGVPS RFSGSGSGTEFTLTISSLQPDDFATYYCQQTKEVPWSFGCGTKVEVK | 665 |
| 33-2 | VH | QVQLVQSGAEVKKPGSSVKVSCKASGYTFTDYNMHWVRQAPGQCLEWIGYIYPYNGGTGYNQKF KSKATITADESTNTAYMELSSLRSEDTAVYYCARGRPAMDYWGQGTLVTVSS | 666 |
| | VL | DIQMTQSPSSLSASVGDRVTITCRASESVDNYGISFMNWFQQKPGKAPKLLIYAASNQGSGVPS RFSGSGSGTDFTLTISSLQPDDFATYYCQQSKEVPWTFGCGTKVEIK | 667 |
| 33-3 | VH | QVQLQQPGAEVVKPGASVKMSCKASGYTFTSYYIHWIKQTPGQGLEWVGYIYPGNDDISYNQKF QGKATLTADKSSTTAYMQLSSLTSEDSAVYYCAREVRLRYFDVWGQGTTVTVSS | 668 |
| | VL | EIVLTQSPGSLAVSPGERVTMSCKSSQSVFFSSSQKNYLAWYQQIPGQSPRLLIYWASTRESGV PDRFTGSGSGTDFTLTISSVQPEDLAIYYCHQYLSSRTFGCGTKLEIK | 669 |
| 33-4 | VH1 | EVQLVQSGAEVKKPGSSVKVSCKASGYTITDSNIHWVRQAPGQSLEWIGYIYPYNGGTDYNQKF KNRATLTVDNPTNTAYMELSSLRSEDTAFYYCVNGXPWLAYWGQGTLVTVSS | 670 |
| | VH2 | QVQLVQSGAEVKKPGSSVKVSCKASGYTFTDYNMHWVRQAPGQCLEWIGYIYPYNGGTGYNQKF KSKATITADESTNTAYMELSSLRSEDTAVYYCARGRPAMDYWGQGTLVTVSS | 666 |
| | VL1 | DIQLTQSPSTLSASVGDRVTITCRASESLDNYGIRFLTWFQQKPGKAPKLLMYAASNQGSGVPS RFSGSGSGTEFTLTISSLQPDDFATYYCQQTKEVPWSFGQGTKVEVK | 671 |
| | VL2 | DIQMTQSPSSLSASVGDRVTITCRASESVDNYGISFMNWFQQKPGKAPKLLIYAASNQGSGVPS RFSGSGSGTDFTLTISSLQPDDFATYYCQQSKEVPWTFGCGTKVEIK | 667 |
| 33-5 | VH1 | QVQLQQPGAEVVKPGASVKMSCKASGYTFTSYYIHWIKQTPGQGLEWVGYIYPGNDDISYNQKF QGKATLTADKSSTTAYMQLSSLTSEDSAVYYCAREVRLRYFDVWGQGTTVTVSS | 672 |
| | VH2 | QVQLVQSGAEVKKPGSSVKVSCKASGYTFTDYNMHWVRQAPGQCLEWIGYIYPYNGGTGYNQKF KSKATITADESTNTAYMELSSLRSEDTAVYYCARGRPAMDYWGQGTLVTVSS | 666 |
| | VL1 | EIVLTQSPGSLAVSPGERVTMSCKSSQSVFFSSSQKNYLAWYQQIPGQSPRLLIYWASTRESGV PDRFTGSGSGTDFTLTISSVQPEDLAIYYCHQYLSSRTFGQGTKLEIK | 673 |
| | VL2 | DIQMTQSPSSLSASVGDRVTITCRASESVDNYGISFMNWFQQKPGKAPKLLIYAASNQGSGVPS RFSGSGSGTDFTLTISSLQPDDFATYYCQQSKEVPWTFGCGTKVEIK | 667 |
| 33-6 | VH1 | QVQLVQSGAEVKKPGSSVKVSCKASGYTFTDYNMHWVRQAPGQGLEWIGYIYPYNGGTGYNQKF KSKATITADESTNTAYMELSSLRSEDTAVYYCARGRPAMDYWGQGTLVTVSS | 674 |
| | VH2 | EVQLVQSGAEVKKPGSSVKVSCKASGYTITDSNIHWVRQAPGQCLEWIGYIYPYNGGTDYNQKF KNRATLTVDNPTNTAYMELSSLRSEDTAFYYCVNGXPWLAYWGQGTLVTVSS | 664 |
| | VL1 | DIQMTQSPSSLSASVGDRVTITCRASESVDNYGISFMNWFQQKPGKAPKLLIYAASNQGSGVPS RFSGSGSGTDFTLTISSLQPDDFATYYCQQSKEVPWTFGQGTKVEIK | 675 |
| | VL2 | DIQLTQSPSTLSASVGDRVTITCRASESLDNYGIRFLTWFQQKPGKAPKLLMYAASNQGSGVPS RFSGSGSGTEFTLTISSLQPDDFATYYCQQTKEVPWSFGCGTKVEVK | 665 |
| 33-7 | VH1 | QVQLVQSGAEVKKPGSSVKVSCKASGYTFTDYNMHWVRQAPGQGLEWIGYIYPYNGGTGYNQKF KSKATITADESTNTAYMELSSLRSEDTAVYYCARGRPAMDYWGQGTLVTVSS | 674 |
| | VH2 | QVQLQQPGAEVVKPGASVKMSCKASGYTFTSYYIHWIKQTPGQCLEWVGVIYPGNDDISYNQKF QGKATLTADKSSTTAYMQLSSLTSEDSAVYYCAREVRLRYFDVWGQGTTVTVSS | 668 |
| | VL1 | DIQMTQSPSSLSASVGDRVTITCRASESVDNYGISFMNWFQQKPGKAPKLLIYAASNQGSGVPS RFSGSGSGTDFTLTISSLQPDDFATYYCQQSKEVPWTFGQGTKVEIK | 675 |
| | VL2 | EIVLTQSPGSLAVSPGERVTMSCKSSQSVFFSSSQKNYLAWYQQIPGQSPRLLIYWASTRESGV PDRFTGSGSGTDFTLTISSVQPEDLAIYYCHQYLSSRTFGCGTKLEIK | 669 |

[Table 73]

| Name | | Sequence | SEQ ID NO |
|---|---|---|---|
| 33-1 | VH | GAGGTTCAGTTGGTTCAGTCAGGAGCAGAGGTCAAAAAACCTGGAAGCTCTGTCAAAGTTAGTT GTAAGGCCAGTGGATACACCATAACCGATTCAAATATACATTGGGTTAGGCAAGCACCAGGACA GTGCTTGGAATGGATCGGGTACATCTATCCATATAATGGGGGCACCGATTACAACCAAAAGTTT AAGAATCGCGCCACACTCACTGTTGATAATCCAACCAATACAGCATACATGGAGTTGAGCAGTC TTCGGTCCGAGGACACTGCTTTTTACTATTGTGTGAACGGTAACCCATGGTTGGCCTATTGGGG CCAAGGTACACTTGTAACAGTTTCATCT | 676 |
| | VL | GATATACAACTGACTCAGAGTCCCAGCACTCTCAGTGCAAGTGTAGGCGATAGAGTAACTATAA CCTGTCGCGCCTCAGAATCTCTTGATAATTATGGGATCCGATTTCTTACTTGGTTTCAGCAAAA GCCTGGTAAAGCTCCTAAATTGCTCATGTATGCCGCCAGTAATCAGGGTTCAGGAGTTCCTAGT CGTTTCTCTGGGTCAGGAAGCGGCACAGAATTTACCCCTTACAATTTCCAGCCTCCAGCCCGACG ATTTCGCCACTTACTATTGCCAACAAACTAAAGAGGTTCCTTGGAGTTTTTGGGTGTGGCACCAA GGTAGAAGTAAAA | 677 |
| 33-2 | VH | CAGGTACAGCTTGTGCAATCTGGAGCTGAGGTCAAAAAACCAGGAAGTTCTGTAAAGGTGTCTT GTAAGGCATCTGGTTACACATTTACCGATTACAACATGCATTXXGGTACGTCAAGCCCCTGGCCA GTGCCTGGAATGGATCGGATATATATACCCCTACAACGGTGGAACAGGTTACAACCAAAAATTC AAAAGCCAAGGCTACTATCACTGCCGACGAGAGCACTAACACCGCTTACATGGAACTGTCTTCCT TGCGTTCAGAAGACACTGCCGTGTATTATTGTGCTCGGGGGCGACCCGCAATGGACTATTGGGG CCAAGGTACATTGGTGACTGTCAGTTCC | 678 |
| | VL | GACATCCAAATGACACAGTCCCCTAGCTCACTTTCAGCAAGCGTGGCGGACCGAGTTACTATCA CATGCCGCGCAAGTGAGTCTGTGGACAACTATGGAATATCATTCATGAACTGGTTCCAGCAGAA ACCTGGGAAAGCACCCAAGCTGCTTATCTACGCAGCAAGTAATCAGGGTAGTGGCGTCCCTTCT CGATTCAGTGGGAGCGGTAGCGGCACCGACTTCACCCTTACCATCTCATCACTTCAACCCGATG ATTTTGCTACCTACTATTGCCAGCAATCCAAGGAAGTTCCTTGGACCTTCGGGTGTGGGACAAA GGTAGAGATTAAA | 679 |
| 33-3 | VH | CAAGTCCAACTTCAGCAGCCTGGAGCTGAGGTAGTGAAACCCGGCGCATCTGTAAAAATGAGCT GCAAAGCATCAGGTTACACATTTACATCCTACTACATCCATTGGATCAAGCAAACACCAGGCCA ATGTCTTGAGTGGGTTGGCGTCATTTACCCAGGAAACGATGATATATCTTACAATCAGAAATTT CAAGGGAAAGCCACACTTACAGCCGACAAGAGTTCCACAACTGCATATATGCAACTCTCCTCCC TGACATCTGAAGACAGTGCCGTATACTATTGTGCTCGTGAAGTCAGGCTCAGATACTTTGACGT GTGGGGTCAAGGCACAACCGTCACCGTCAGTAGC | 680 |
| | VL | GAGATAGTCCTCACCCAATCACCAGGTAGTCTGGCTGTTAGCCCAGGTGAGCGAGTTACTATGT CTTGCAAGTCCTCTCAATCTGTGTTCTTTTCATCAAGTCAGAAGAATTATCTTGCCTGGTATCA GCAGATTCCTGGTCAATCTCCCCGACTGCTTATTTACTGGGCTTCAACTCGGGAGTCAGGGGTT CCCGACCGATTCACAGGGTCTGGGTCAGGAACTGACTTTACCCTTACTATCAGCTCTGTGCAGC CTGAAGACCTCGCAATATATTATTGTCATCAGTACCTCTCTTCTCGCACTTTTGGATGTGGCAC CAAATTGGAGATTAAG | 681 |
| 33-4 | VH1 | GAGGTTCAGTTGGTTCAGTCAGGAGCAGAGGTCAAAAAACCTGGAAGCTCTGTCAAAGTTAGTT GTAAGGCCAGTGGATACACCATAACCGATTCAAATATACATTGGGTTAGGCAAGCACCAGGACA GTCCTTGGAATGGATCGGGTACATCTATCCATATAATGGGGGCACCGATTACAACCAAAAGTTT AAGAATCGCGCCACACTCACTGTTGATAATCCAACCAATACAGCATACATGGAGTTGAGCAGTC TTCGGTCCGAGGACACTGCTTTTTACTATTGTGTGAACGGTAACCCATGGTTGGCCTATTGGGG CCAAGGTACACTTGTAACAGTTTCATCC | 682 |
| | VH2 | CAGGTACAGCTTGTGCAATCTGGAGCTGAGGTCAAAAAACCAGGAAGTTCTGTAAAGGTGTCTT GTAAGGCATCTGGTTACACATTTACCGATTACAACATGCATTGGGTACGTCAAGCCCCTGGCCA GTGCCTGGAATGGATCGGATATATATACCCCTACAACGGTGGAACAGGTTACAACCAAAAATTC AAAAGCAAGGCTACTATCACTGCCGACGAGAGCACTAACACCGCTTACATGGAACTGTCTTCCT TGCGTTCAGAAGACACTGCCGTGTATTATTGTGCTCGGGGGCGACCCGCAATGGACTATTGGGG CCAAGGTACATTGGTGACTGTCAGTTCC | 678 |

| | | | |
|---|---|---|---|
| | VL1 | GATATACAACTGACTCAGAGTCCCAGCACTCTCAGTGCAAGTGTAGGCGATAGAGTAACTATAA CCTGTCGCGCCTCAGAATCTCTTGATAATTATGGGATCCGATTTCTTACTTGGTTTCAGCAAAA GCCTGGTAAAGCTCCTAAATTGCTCATGTATGCCGCCAGTAATCAGGGTTCAGGAGTTCCTAGT CGTTTCTCTGGGTCAGGAAGCGGCACAGAATTTACCCTTACAATTTCCAGCCTCCAGCCCGACG ATTTCGCCACTTACTATTGCCAACAAACTAAAGAGGTTCCTTGGAGTTTTGGGCAAGGCACCAA GGTAGAAGTAAAA | 683 |
| | VL2 | GACATCCAAATGACACAGTCCCCTAGCTCACTTTCAGCAAGCGTGGGGGACCGAGTTACTATCA CATGCCGCGCAAGTGAGTCTGTGGACAACTATGGAATATCATTCATGAACTGGTTCCAGCAGAA ACCTGGGAAAGCACCCAAGCTGCTTATCTACGCAGCAAGTAATCAGGGTAGTGGCGTCCCTTCT CGATTCAGTGGGAGCGGTAGCGGCACCGACTTCACCCTTACCATCTCATCACTTCAACCCGATG ATTTTGCTACCTACTATTGCCAGCAATCCAAGGAAGTTCCTTGGACCTTCGGGTGTGGGACAAA GGTAGAGATTAAA | 679 |
| 33-5 | VH1 | CAAGTCCAACTTCAGCAGCCTGGAGCTGAGGTAGTGAAACCCGGCGCATCTGTAAAAATGAGCT GCAAAGCATCAGGTTACACATTTACATCCTACTACATCCATTGGATCAAGCAAACACCAGGCCA AGGTCTTGAGTGGGTTGGCGTCATTTACCCAGGAAACGATGATATATCTTACAATCAGAAATTT CAAGGGAAAGCCACACTTACAGCCGACAAGAGTTCCACAACTGCATATATGCAACTCTCCTCCC TGACATCTGAAGACAGTGCCGTATACTATTGTGCTCGTGAAGTCAGGCTCAGATACTTTGACGT GTGGGGTCAAGGCACAACCGTCACCGTCAGTAGC | 684 |
| | VH2 | CAGGTACAGCTTGTGCAATCTGGAGCTGAGGTCAAAAAACCAGGAAGTTCTGTAAAGGTGTCTT GTAAGGCATCTGGTTACACATTTACCGATTACAACATGCATTGGGTACGTCAAGCCCCTGGCCA GTGCCTGGAATGGATCGGATATATATACCCCTACAACGGTGGAACAGGTTACAACCAAAAATTC AAAAGCAAGGCTACTATCACTGCCGACGAGAGCACTAACACCGCTTACATGGAACTGTCTTCCT TGCGTTCAGAAGACACTGCCGTGTATTATTGTGCTCGGGGGCGACCCGCAATGGACTATTGGGG CCAAGGTACATTGGTGACTGTCAGTTCC | 678 |
| | VL1 | GAGATAGTCCTCACCCAATCACCAGGTAGTCTGGCTGTTAGCCCAGGTGAGCGAGTTACTATGT CTTGCAAGTCCTCTCAATCTGTGTTCTTTTCATCAAGTCAGAAGAATTATCTTGCCTGGTATCA GCAGATTCCTGGTCAATCTCCCCGACTGCTTATTTACTGGGCTTCAACTCGGGAGTCAGGGGTT CCCGACCGATTCACAGGGTCTGGGTCAGGAACTGACTTTACCCTTACTATCAGCTCTGTGCAGC CTGAAGACCTCGCAATATATTATTGTCATCAGTACCTCTCTTCTCGCACTTTTGGACAGGGCAC CAAATTGGAGATTAAG | 685 |
| | VL2 | GACATCCAAATGACACAGTCCCCTAGCTCACTTTCAGCAAGCGTGGGGGACCGAGTTACTATCA CATGCCGCGCAAGTGAGTCTGTGGACAACTATGGAATATCATTCATGAACTGGTTCCAGCAGAA ACCTGGGAAAGCACCCAAGCTGCTTATCTACGCAGCAAGTAATCAGGGTAGTGGCGTCCCTTCT CGATTCAGTGGGAGCGGTAGCGGCACCGACTTCACCCTTACCATCTCATCACTTCAACCCGATG ATTTTGCTACCTACTATTGCCAGCAATCCAAGGAAGTTCCTTGGACCTTCGGGTGTGGGACAAA GGTAGAGATTAAA | 679 |
| 33-6 | VH1 | CAGGTACAGCTTGTGCAATCTGGAGCTGAGGTCAAAAAACCAGGAAGTTCTGTAAAGGTGTCTT GTAAGGCATCTGGTTACACATTTACCGATTACAACATGCATTGGGTACGTCAAGCCCCTGGCCA GGGCCTGGAATGGATCGGATATATATACCCCTACAACGGTGGAACAGGTTACAACCAAAAATTC AAAAGCAAGGCTACTATCACTGCCGACGAGAGCACTAACACCGCTTACATGGAACTGTCTTCCT TGCGTTCAGAAGACACTGCCGTGTATTATTGTGCTCGGGGGCGACCCGCAATGGACTATTGGGG CCAAGGTACATTGGTGACTGTCAGTTCC | 686 |
| | VH2 | GAGGTTCAGTTGGTTCAGTCAGGAGCAGAGGTCAAAAAACCTGGAAGCTCTGTCAAAGTTAGTT GTAAGGCCAGTGGATACACCATAACCGATTCAAATATACATTGGGTTAGGCAAGCACCAGGACA GTGCTTGGAATGGATCGGGTACATCTATCCATATAATGGGGGCACCGATTACAACCAAAAGTTT AAGAATCGCGCCACACTCACTGTTGATAATCCAACCAATACAGCATACATGGAGTTGAGCAGTC TTCGGTCCGAGGACACTGCTTTTTACTATTGTGTGAACGGTAACCCATGGTTGGCCTATTGGGG CCAAGGTACACTTGTAACAGTTTCATCT | 676 |
| | VL1 | GACATCCAAATGACACAGTCCCCTAGCTCACTTTCAGCAAGCGTGGGGGACCGAGTTACTATCA | 687 |

| | | | |
|---|---|---|---|
| | | CATGCCGCGCAAGTGAGTCTGTGGACAACTATGGAATATCATTCATGAACTGGTTCCAGCAGAA ACCTGGGAAAGCACCCAAGCTGCTTATCTACGCAGCAAGTAATCAGGGTAGTGGCGTCCCTTCT CGATTCAGTGGGAGCGGTAGCGGCACCGACTTCACCCTTACCATCTCATCACTTCAACCCGATG ATTTTGCTACCTACTATTGCCAGCAATCCAAGGAAGTTCCTTGGACCTTCGGGCAAGGGACAAA GGTAGAGATTAAA | |
| | VL2 | GATATACAACTGACTCAGAGTCCCAGCACTCTCAGTGCAAGTGTAGGCGATAGAGTAACTATAA CCTGTCGCGCCTCAGAATCTCTTGATAATTATGGGATCCGATTTCTTACTTGGTTTCAGCAAAA GCCTGGTAAAGCTCCTAAATTGCTCATGTATGCCGCCAGTAATCAGGGTTCAGGAGTTCCTAGT CGTTTCTCTGGGTCAGGAAGCGGCACAGAATTTACCCTTACAATTTCCAGCCTCCAGCCCGACG ATTTCGCCACTTACTATTGCCAACAAACTAAAGAGGTTCCTTGGAGTTTTGGGTGTGGCACCAA GGTAGAAGTAAAA | 677 |
| 33-7 | VH1 | CAGGTACAGCTTGTGCAATCTGGAGCTGAGGTCAAAAAACCAGGAAGTTCTGTAAAGGTGTCTT GTAAGGCATCTGGTTACACATTTACCGATTACAACATGCATTGGGTACGTCAAGCCCCTGGCCA GGGCCTGGAATGGATCGGATATATATACCCCTACAACGGTGGAACAGGTTACAACCAAAAATTC AAAAGCAAGGCTACTATCACTGCCGACGAGAGCACTAACACCGCTTACATGGAACTGTCTTCCT TGCGTTCAGAAGACACTGCCGTGTATTATTGTGCTCGGGGGCGACCCGCAATGGACTATTGGGG CCAAGGTACATTGGTGACTGTCAGTTCC | 686 |
| | VH2 | CAAGTCCAACTTCAGCAGCCTGGAGCTGAGGTAGTGAAACCCGGCGCATCTGTAAAAATGAGCT GCAAAGCATCAGGTTACACATTTACATCCTACTACATCCATTGGATCAAGCAAACACCAGGCCA ATGTCTTGAGTGGGTTGGCGTCATTTACCCAGGAAACGATGATATATCTTACAATCAGAAATTT CAAGGGAAAGCCACACTTACAGCCGACAAGAGTTCCACAACTGCATATATGCAACTCTCCTCCC TGACATCTGAAGACAGTGCCGTATACTATTGTGCTCGTGAAGTCAGGCTCAGATACTTTGACGT GTGGGGTCAAGGCACAACCGTCACCGTCAGTAGC | 680 |
| | VL1 | GACATCCAAATGACACAGTCCCCTAGCTCACTTTCAGCAAGCGTGGGGGACCGAGTTACTATCA CATGCCGCGCAAGTGAGTCTGTGGACAACTATGGAATATCATTCATGAACTGGTTCCAGCAGAA ACCTGGGAAAGCACCCAAGCTGCTTATCTACGCAGCAAGTAATCAGGGTAGTGGCGTCCCTTCT CGATTCAGTGGGAGCGGTAGCGGCACCGACTTCACCCTTACCATCTCATCACTTCAACCCGATG ATTTTGCTACCTACTATTGCCAGCAATCCAAGGAAGTTCCTTGGACCTTCGGGCAAGGGACAAA GGTAGAGATTAAA | 687 |
| | VL2 | GAGATAGTCCTCACCCAATCACCAGGTAGTCTGGCTGTTAGCCCAGGTGAGCGAGTTACTATGT CTTGCAAGTCCTCTCAATCTGTGTTCTTTTCATCAAGTCAGAAGAATTATCTTGCCTGGTATCA GCAGATTCCTGGTCAATCTCCCCGACTGCTTATTTACTGGGCTTCAACTCGGGAGTCAGGGGTT CCCGACCGATTCACAGGGTCTGGGTCAGGAACTGACTTTACCCTTACTATCAGCTCTGTGCAGC CTGAAGACCTCGCAATATATTATTGTCATCAGTACCTCTCTTCTCGCACTTTTGGATGTGGCAC CAAATTGGAGATTAAG | 681 |

[0311] Table 74 below shows the CDR sequences of the heavy chain and light chain of the engineered antibodies targeting CD33.

[Table 74]

| Name | CDR | Sequence | SEQ ID NO |
|---|---|---|---|
| 33-1 | CDR-H1 | DSNIH | 688 |
| | CDR-H2 | YIYPYNGGTDYNQKFKN | 689 |
| | CDR-H3 | GNPWLAY | 690 |
| | CDR-L1 | RASESLDNYGIRFLT | 691 |
| | CDR-L2 | AASNQGS | 692 |
| | CDR-L3 | QQTKEVPWS | 693 |
| 33-2 | CDR-H1 | DYNMH | 694 |
| | CDR-H2 | YIYPYNGGTGYNQKFKS | 695 |
| | CDR-H3 | GRPAMDY | 696 |
| | CDR-L1 | RASESVDNYGISFMN | 697 |
| | CDR-L2 | AASNQGS | 698 |
| | CDR-L3 | QQSKEVPWT | 699 |
| 33-3 | CDR-H1 | SYYIH | 700 |
| | CDR-H2 | VIYTGNDDISYNQKFQG | 701 |
| | CDR-H3 | EVRLRYFDV | 702 |
| | CDR-L1 | KSSQSVFFSSSQKNYLA | 703 |
| | CDR-L2 | WASTRES | 704 |
| | CDR-L3 | HQYLSSRT | 705 |
| 33-4 | V1 CDR-H1 | DSNIH | 688 |
| | V1 CDR-H2 | YIYPYNGGTDYNQKFKN | 689 |
| | V1 CDR-H3 | GNPWLAY | 690 |
| | V1 CDR-L1 | RASESLDNYGIRFLT | 691 |
| | V1 CDR-L2 | AASNQGS | 692 |
| | V1 CDR-L3 | QQTKEVPWS | 693 |
| | V2 CDR-H1 | DYNMH | 694 |
| | V2 CDR-H2 | YIYPYNGGTGYNQKFKS | 695 |
| | V2 CDR-H3 | GRPAMDY | 696 |
| | V2 CDR-L1 | RASESVDNYGISFMN | 697 |
| | V2 CDR-L2 | AASNQGS | 698 |
| | V2 CDR-L3 | QQSKEVPWT | 699 |
| 33-5 | V1 CDR-H1 | SYYIH | 700 |
| | V1 CDR-H2 | VIYTGNDDISYNQKFQG | 701 |
| | V1 CDR-H3 | EVRLRYFDV | 702 |
| | V1 CDR-L1 | KSSQSVFFSSSQKNYLA | 703 |
| | V1 CDR-L2 | WASTRES | 704 |
| | V1 CDR-L3 | HQYLSSRT | 705 |
| | V2 CDR-H1 | DYNMH | 694 |
| | V2 CDR-H2 | YIYPYNGGTGYNQKFKS | 695 |
| | V2 CDR-H3 | GRPAMDY | 696 |
| | V2 CDR-L1 | RASESVDNYGISFMN | 697 |
| | V2 CDR-L2 | AASNQGS | 698 |
| | V2 CDR-L3 | QQSKEVPWT | 699 |
| 33-6 | V1 CDR-H1 | DYNMH | 694 |
| | V1 CDR-H2 | YIYPYNGGTGYNQKFKS | 695 |
| | V1 CDR-H3 | GRPAMDY | 696 |

| | V1 CDR-L1 | RASESVDNYGISFMN | 697 |
|---|---|---|---|
| | V1 CDR-L2 | AASNQGS | 698 |
| | V1 CDR-L3 | QQSKEVPWT | 699 |
| | V2 CDR-H1 | DSNIH | 688 |
| | V2 CDR-H2 | YIYPYNGGTDYNQKFKN | 689 |
| | V2 CDR-H3 | GNPWLAY | 690 |
| | V2 CDR-L1 | RASESLDNYGIRFLT | 691 |
| | V2 CDR-L2 | AASNQGS | 692 |
| | V2 CDR-L3 | QQTKEVPWS | 693 |
| 33-7 | V1 CDR-H1 | DYNMH | 694 |
| | V1 CDR-H2 | YIYPYNGGTGYNQKFKS | 695 |
| | V1 CDR-H3 | GRPAMDY | 696 |
| | V1 CDR-L1 | RASESVDNYGISFMN | 697 |
| | V1 CDR-L2 | AASNQGS | 698 |
| | V1 CDR-L3 | QQSKEVPWT | 699 |
| | V2 CDR-H1 | DYNMH | 694 |
| | V2 CDR-H2 | YIYPYNGGTGYNQKFKS | 695 |
| | V2 CDR-H3 | GRPAMDY | 696 |
| | V2 CDR-L1 | RASESVDNYGISFMN | 697 |
| | V2 CDR-L2 | AASNQGS | 698 |
| | V2 CDR-L3 | QQSKEVPWT | 699 |

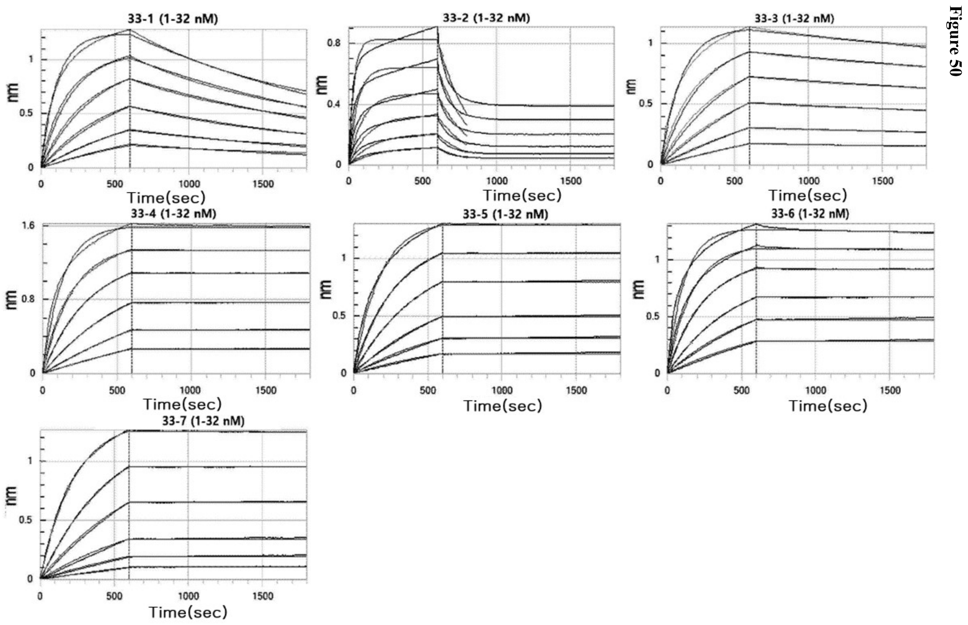

The CD33 protein binding constants of each of 33-1, 33-2, 33-3, 33-4, 33-5, 33-6, and 33-7 to the CD33 protein were determined using Octet Red96e. The human CD33 recombinant protein (Sino Biologicals, 12238-H08H) was loaded onto the Anti-Penta-HIS (HIS1K) biosensor, and then each antibody was added in a binding reaction (600 seconds) and a dissociation reaction (1200 seconds) at various concentrations. Their affinities for the CD33 protein were calculated, and the results are shown in Figure 50 and Table 75.

[Table 75]

| Antibodies | Antigen | Binding mode | $K_D$ (nM) | $K_a$ (1/Ms) | $K_d$ (1/s) |
|---|---|---|---|---|---|
| 33-1 | rhCD33 | Monovalent | 1.7835 | 2.80E+05 | 4.99E-04 |
| 33-2 | rhCD33 | Monovalent | 5.5540 | 8.01E+05 | 4.45E-03 |
| 33-3 | rhCD33 | Monovalent | 0.5163 | 2.24E+05 | 1.16E-04 |
| 33-4 | rhCD33 | Biparatopic | 0.0851 | 2.79E+05 | 8.11E+02 |
| 33-5 | rhCD33 | Biparatopic | 0.0641 | 2.01E+05 | 4.07E+02 |
| 33-6 | rhCD33 | Biparatopic | 0.0772 | 3.60E+05 | 1.08E+03 |
| 33-7 | rhCD33 | Biparatopic | 0.1290 | 1.37E+05 | 1.24E+02 |

**Example 22. Design, preparation and analysis of antibody structure targeting CEACAMS**

The polypeptide sequences of the variant light chain and heavy chain of an antibody that specifically binds to the CEACAM5 protein are shown in Table 76.

CEA01 consists of the polypeptides of Fc-Hole (SEQ ID NO: 7), CEA01 HC (SEQ ID NO: 590), and CEA01 LC (SEQ ID NO: 591), and was prepared by co-transfection of expression vectors loaded with polynucleotides encoding the polypeptides into the EXPICHO-S™ cell line (Figure 41a). The protein prepared as above was purified and analyzed in the same manner as in Example 1 above. In addition, CEA02, CEA03, and CEA04 were prepared, purified, and analyzed in the same manner as above.

[Table 76]

| Name | Sequence | SEQ ID NO |
|---|---|---|
| CEA01 HC | EVQLVESGGGVVQPGRSLRLSCSASGFDFTTYWMSWVRQAPGKGLEWIGEIHPDSSTINYAP SLKDRFTISRDNAKNTLFLQMDSLRPEDTGVYFCASLYFGFPWFAYWGQGTPVTVSSASTKG PSVFPLAPSSKSTSGGTAALGCLVKDYFPEPVTVSWNSGALTSGVHTFPAVLQSSGLYSLSS VVTVPSSSLGTQTYICNVNHKPSNTKVDKKVEPKSCDKTHTCPPCPAPELLGGPSVFLFPPK PKDTLMISRTPEVTCVVVDVSHEDPEVKFNWYVDGVEVHNAKTKPREEQYNSTYRVVSVLTV LHQDWLNGKEYKCKVSNKALPAPIEKTISKAKGQPREPQVYTLPPCREEMTKNQVSLWCLVK GFYPSDIAVEWESNGQPENNYKTTPPVLDSDGSFFLYSKLTVDKSRWQQGNVFSCSVMHEAL HNHYTQKSLSLSPGK | 706 |
| CEA01 LC | DIQLTQSPSSLSASVGDRVTITCKASQDVGTSVAWYQQKPGKAPKLLIYWTSTRHTGVPSRF SGSGSGTDFTFTISSLQPEDIATYYCQQYSLYRSFGQGTKVEIKRTVAAPSVFIFPPSDEQL KSGTASVVCLLNNFYPREAKVQWKVDNALQSGNSQESVTEQDSKDSTYSLSSTLTLSKADYE KHKVYACEVTHQGLSSPVTKSFNRGECGGGGSGGGGSGGGGSEPKSSDKTHTCPPCPAPELL GGPSVFLFPPKPKDTLMISRTPEVTCVVVDVSHEDPEVKFNWYVDGVEVHNAKTKPREEQYN STYRVVSVLTVLHQDWLNGKEYKCKVSNKALPAPIEKTISKAKGQPREPQVYTLPPCREEMT KNQVSLWCLVKGFYPSDIAVEWESNGQPENNYKTTPPVLDSDGSFFLYSKLTVDKSRWQQGN VFSCSVMHEALHNHYTQKSLSLSPGK | 707 |
| CEA02 HC | EVQLVESGGGVVQPGRSLRLSCAASGFTVSSYWMHWVRQAPGKGLEWVGFILNKANGGTTEY AASVKGRFTISRDDSKNTLYLQMNSLRAEDTAVYYCARDRGLRFYFDVWGQGTTVTVSSAST KGPSVFPLAPSSKSTSGGTAALGCLVKDYFPEPVTVSWNSGALTSGVHTFPAVLQSSGLYSL SSVVTVPSSSLGTQTYICNVNHKPSNTKVDKKVEPKSCDKTHTCPPCPAPELLGGPSVFLFPP PKPKDTLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPCREEMTKNQVSLWCL VKGFYPSDIAVEWESNGQPENNYKTTPPVLDSDGSFFLYSKLTVDKSRWQQGNVFSCSVMHE ALHNHYTQKSLSLSPGK | 708 |
| CEA02 LC | QAVLTQPASLSASPGASASLTCTLRRGINVGAYSIYWYQQKPGSPPQYLLRYKSDSDKQQGS GVSSRFSASKDASANAGILLTSGLQSEDEADYYCMIWHSGASAVFGCGTKLTVLGQPKAAPS VTLFPPSSEELQANKATLVCLISDFYPGAVTVAWKADSSPVKAGVETTTPSKQSNNKYAASS YLSLTPEQWKSHRSYSCQVTHEGSTVEKTVAPTECSGGGGSGGGGSGGGGSEPKSSDKTHTC PPCPAPELLGGPSVFLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTISKAKGQPREPQVYT LPPCREEMTKNQVSLWCLVKGFYPSDIAVEWESNGQPENNYKTTPPVLDSDGSFFLYSKLTV DKSRWQQGNVFSCSVMHEALHNHYTQKSLSLSPGK | 709 |
| CEA03 HC | EVQLQQSGPGLVRPSQTLSLTCTASGFNIKDNYMHWVRQPPGRGLEWIGWIDPENGDTEYAP KFRGRVTMLADTSKNQFSLRLSSVTAADTAVYYCHVLIYAGYLAMDYWGQGTLVTVSSASTK GPSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCDKTHTCPPCPAPELLGGPSVFLFPP KPKDTLMISRTPEVTCVVVDVSHEDPEVKFNWYVDGVEVHNAKTKPREEQYNSTYRVVSVLT VLHQDWLNGKEYKCKVSNKALPAPIEKTISKAKGQPREPQVYTLPPCREEMTKNQVSLWCLV KGFYPSDIAVEWESNGQPENNYKTTPPVLDSDGSFFLYSKLTVDKSRWQQGNVFSCSVMHEA LHNHYTQKSLSLSPGK | 710 |
| CEA03 LC | DIQMTQSPSSLSASVGDRVTITCSASSSVTYMHWYQQKPGKAPKLWIYSTSNLASGVPSRFS GSGSGTDYTFTISSLQPEDIATYYCQQRSTYPLTFGCGTKLEIKRTVAAPSVFIFPPSDEQL KSGTASVVCLLNNFYPREAKVQWKVDNALQSGNSQESVTEQDSKDSTYSLSSTLTLSKADYE KHKVYACEVTHQGLSSPVTKSFNRGECGGGGSGGGGSGGGGSEPKSSDKTHTCPPCPAPELL GGPSVFLFPPKPKDTLMISRTPEVTCVVVDVSHEDPEVKFNWYVDGVEVHNAKTKPREEQYN STYRVVSVLTVLHQDWLNGKEYKCKVSNKALPAPIEKTISKAKGQPREPQVYTLPPCREEMT KNQVSLWCLVKGFYPSDIAVEWESNGQPENNYKTTPPVLDSDGSFFLYSKLTVDKSRWQQGN | 711 |

| | | | |
|---|---|---|---|
| | | VFSCSVMHEALHNHYTQKSLSLSPGK | |
| | CEA04 HC | EVQLVESGGGLVQPGGSLRLSCAASGFNIKDTYMHWVRQAPGKCLEWVARIDPANGNSKYAD SVKGRFTISADTSKNTAYLQMNSLRAEDTAVYYCAPFGYYVSDYAMAYWGQGTLVTVSSAST KGPSVFPLAPSSKSTSGGTAALGCLVKDYFPEPVTVSWNSGALTSGVHTFPAVLQSSGLYSL SSVVTVPSSSLGTQTYICNVNHKPSNTKVDKKVEPKSCDKTHTCPPCPAPELLGGPSVFLFP PKPKDTLMISRTPEVTCVVVDVSHEDPEVKFNWYVDGVEVHNAKTKPREEQYNSTYRVVSVL TVLHQDWLNGKEYKCKVSNKALPAPIEKTISKAKGQPREPQVYTLPPCREEMTKNQVSLWCL VKGFYPSDIAVEWESNGQPENNYKTTPPVLDSDGSFFLYSKLTVDKSRWQQGNVFSCSVMHE ALHNHYTQKSLSLSPGK | 712 |
| | CEA04 LC | DIQLTQSPSSLSASVGDRVTITCRAGESVDIFGVGFLHWYQQKPGKAPKLLIYRASNLESGV PSRFSGSGSRTDFTLTISSLQPEDFATYYCQQTNEDPYTFGCGTKVEIKRTVAAPSVFIFPP SDEQLKSGTASVVCLLNNFYPREAKVQWKVDNALQSGNSQESVTEQDSKDSTYSLSSTLTLS KADYEKHKVYACEVTHQGLSSPVTKSFNRGECGGGGSGGGGSGGGGSEPKSSDKTHTCPPCP APELLGGPSVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREPQVYTLPPC REEMTKNQVSLWCLVKGFYPSDIAVEWESNGQPENNYKTTPPVLDSDGSFFLYSKLTVDKSR WQQGNVFSCSVMHEALHNHYTQKSLSLSPGK | 713 |

Table 77 below shows the polynucleotide sequences encoding the polypeptides of the heavy chain and light chain of the engineered antibodies targeting CEACAM5.

[Table 77]

| Name | Sequence | SEQ ID NO |
|------|----------|-----------|
| CEA01 HC | GAAGTGCAACTGGTAGAATCCGGCGGGGGCGTCGTACAGCCCGGCCGTTCTTTGAGACTTAG CTGTAGTGCTTCTGGGTTTGACTTCACTACATACTGGATGTCATGGGTAAGACAGGCACCTG GCAAGTGCCTTGAATGGATCGGTGAAATCCATCCCGACAGCTCCACAATCAACTACGCCCCA AGTTTGAAAGACCGGTTCACCATATCTCGTGACAACGCCAAGAATACATTGTTCCTTCAGAT GGATAGTCTTCGTCCAGAGGATACTGGGGTATATTTTTGTGCCAGCTTGTATTTTGGCTTCC CCTGGTTCGCTTATTGGGGCCAAGGTACACCCGTCACTGTCTCTTCTGCTAGCACCAAAGGA CCTAGTGTTTTTCCTCTTGCCCCTTCCTCAAAGTCTACCTCTGGGGGGACAGCCGCTCTGGG CTGCCTGGTCAAGGATTATTTCCCAGAGCCTGTCACTGTCAGTTGGAACTCTGGAGCCTTGA CTTCTGGTGTTCATACATTTCCTGCTGTCCTTCAAAGCAGCGGCTTGTACTCATTGTCTTCT GTTGTGACAGTACCCTCAAGCAGCCTCGGCACTCAGACATACATCTGCAATGTCAACCACAA ACCCTCAAATACAAAGGGTAGATAAAAAAGTCGAACCAAAGTCTTGTGACAAAACTCACACGT GCCCACCGTGCCCAGCACCTGAACTCCTGGGGGGACCGTCAGTCTTCCTCTTCCCCCCAAAA CCCAAGGACACCCTCATGATCTCCCGGACCCCTGAGGTCACATGCGTGGTGGTGGACGTGAG CCACGAAGACCCTGAGGTCAAGTTCAACTGGTACGTGGACGGCGTGGAGGTGCATAATGCCA AGACAAAGCCGCGGGAGGAGCAGTACAACAGCACGTACCGTGTGGTCAGCGTCCTCACCGTC CTGCACCAGGACTGGCTGAATGGCAAGGAGTACAAGTGCAAGGTCTCCAACAAAGCCCTCCC AGCCCCCATCGAGAAAACCATCTCCAAAGCCAAAGGGCAGCCCCGAGAACCACAGGTGTACA CCCTGCCCCCATGCCGGGAGGAGATGACCAAGAACCAGGTCAGCCTGTGGTGCCTGGTCAAA GGCTTCTATCCCAGCGACATCGCCGTGGAGTGGGAGAGCAATGGGCAGCCGGAGAACAACTA CAAGACCACGCCTCCCGTGCTGGACTCCGACGGCTCCTTCTTCCTCTACAGCAAGCTCACCG TGGACAAGAGCAGGTGGCAGCAGGGGAACGTCTTCTCATGCTCCGTGATGCATGAGGCTCTG CACAACCACTACACGCAGAAGAGCCTCTCCCTGTCTCCGGGTAAA | 714 |
| CEA01 LC | GACATCCAACTTACCCAGTCACCCTCATCTCTTAGTGCCTCTGTAGGGGACCGAGTTACTAT TACATGTAAAGCCAGTCAAGATGTTGGCACCTCAGTAGCATGGTATCAACAAAAGCCTGGTA AAGCCCCAAAACTGCTGATCTATTGGACAAGCACACGACATACAGGAGTGCCAAGTCGCTTC AGCGGTTCAGGTTCAGGCACAGATTTTACATTCACTATATCAAGCCTGCAACCCGAGGACAT TGCCACATATTACTGCCAGCAATATAGTCTGTATCGTAGCTTCGGATGTGGCACCAAGGTTG AAATTAAGCGTACGGTGGCAGCTCCCAGCGTTTTTATCTTTCCCCCATCCGACGAGCAGCTC AAGAGTGGCACTGCCTCTGTAGTTTGTTTGCTGAATAACTTCTATCCACGTGAAGCAAAAGT ACAGTGGAAGGTCGATAATGCCCTTCAGAGCGGTAACAGCCAAGAAAGTGTTACCGAGCAAG ATTCCAAAGATTCCACTTACAGTCTGTCCAGCACATTGACACTGAGTAAGGCTGATTACGAA AAACACAAGGTGTACGCATGCGAGGTGACACACCAAGGTCTTTCATCTCCTGTAACTAAGAG CTTTAACCGGGGAGAATGTGGTGGTGGGGGCACCGGGGGCGGAGGTAGTGGAGGCGGCGGTA GTGAACCAAAGAGTAGTGACAAAACTCACACGTGCCCACCGTGCCCAGCACCTGAACTCCTG GGGGGACCGTCAGTCTTCCTCTTCCCCCCAAAACCCAAGGACACCCTCATGATCTCCCGGAC CCCTGAGGTCACATGCGTGGTGGTGGACGTGAGCCACGAAGACCCTGAGGTCAAGTTCAACT GGTACGTGGACGGCGTGGAGGTGCATAATGCCAAGACAAAGCCGCGGGAGGAGCAGTACAAC AGCACGTACCGTGTGGTCAGCGTCCTCACCGTCCTGCACCAGGACTGGCTGAATGGCAAGGA GTACAAGTGCAAGGTCTCCAACAAAGCCCTCCCAGCCCCCATCGAGAAAACCATCTCCAAAG CCAAAGGGCAGCCCCGAGAACCACAGGTGTACACCCTGCCCCCATGCCGGGAGGAGATGACC AAGAACCAGGTCAGCCTGTGGTGCCTGGTCAAAGGCTTCTATCCCAGCGACATCGCCGTGGA GTGGGAGAGCAATGGGCAGCCGGAGAACAACTACAAGACCACGCCTCCCGTGCTGGACTCCG ACGGCTCCTTCTTCCTCTACAGCAAGCTCACCGTGGACAAGAGCAGGTGGCAGCAGGGGAAC GTCTTCTCATGCTCCGTGATGCATGAGGCTCTGCACAACCACTACACGCAGAAGAGCCTCTC CCTGTCTCCGGGTAAA | 715 |
| CEA02 HC | GAAGTTCAACTGGTGGAGTCTGGCGGCGGACTTGTCCAGCCAGGGCGAAGCCTGCGTCTCTC ATGCGCTGCCTCCGGTTTCACTGTTTCTTCATACTGGATGCACTGGGTAAGACAGGCTCCTG | 716 |

| | | |
|---|---|---|
| | GCAAGTGTCTTGAATGGGTGGGCTTCATTTTGAACAAGGCAAACGGCGGGACTACCGAATAC<br>GCTGCCAGCGTTAAGGGTCGATTCACCATCTCAAGGGATGATTCTAAAAACACATTGTACCT<br>TCAGATGAACTCTCTGAGGGCCGAGGACACAGCAGTCTACTATTGCGCTAGAGATAGGGGGC<br>TTAGATTCTATTTTGACTACTGGGGGCAGGGAACAACTGTCACCGTTTCCAGTGCTAGCACC<br>AAAGGACCTAGTGTTTTTCCTCTTGCCCCTTCCTCAAAGTCTACCTCTGGGGGGACAGCCGC<br>TCTGGGCTGCCTGGTCAAGGATTATTTCCCAGAGCCTGTCACTGTCAGTTGGAACTCTGGAG<br>CCTTGACTTCTGGTGTTCATACATTTCCTGCTGTCCTTCAAAGCAGCGGCTTGTACTCATTG<br>TCTTCTGTTGTGACAGTACCCTCAAGCAGCCTCGGCACTCAGACATACATCTGCAATGTCAA<br>CCACAAACCCTCAAATACAAAGGTAGATAAAAAAGTCGAACCAAAGTCTTGTGACAAAACTC<br>ACACGTGCCCACCGTGCCCAGCACCTGAACTCCTGGGGGGACCGTCAGTCTTCCTCTTCCCC<br>CCAAAACCCAAGGACACCCTCATGATCTCCCGGACCCCTGAGGTCACATGCGTGGTGGTGGA<br>CGTGAGCCACGAAGACCCTGAGGTCAAGTTCAACTGGTACGTGGACGGCGTGGAGGTGCATA<br>ATGCCAAGACAAAGCCGCGGGAGGAGCAGTACAACAGCACGTACCGTGTGGTCAGCGTCCTC<br>ACCGTCCTGCACCAGGACTGGCTGAATGGCAAGGAGTACAAGTGCAAGGTCTCCAACAAAGC<br>CCTCCCAGCCCCCATCGAGAAAACCATCTCCAAAGCCAAAGGGCAGCCCCGAGAACCACAGG<br>TGTACACCCTGCCCCCATGCCGGGAGGAGATGACCAAGAACCAGGTCAGCCTGTGGTGCCTG<br>GTCAAAGGCTTCTATCCCAGCGACATCGCCGTGGAGTGGGAGAGCAATGGGCAGCCGGAGAA<br>CAACTACAAGACCACGCCTCCCGTGCTGGACTCCGACGGCTCCTTCTTCCTCTACAGCAAGC<br>TCACCGTGGACAAGAGCAGGTGGCAGCAGGGGAACGTCTTCTCATGCTCCGTGATGCATGAG<br>GCTCTGCACAACCACTACACGCAGAAGAGCCTCTCCCTGTCTCCGGGTAAA | |
| CEA02 LC | CAGGCAGTGCTTACTCAGCCTGCTTCACTCTCTGCCTCACCAGGAGCATCTGCAAGCCTCAC<br>CTGCACATTGCGTCGAGGTATAAACGTAGGCGCTTACTCAATTTACTGGTACCAGCAGAAAC<br>CTGGGAGCCCACCTCAATACCTCCTCCGATACAAGAGCGATTCTGATAAGCAACAGGGCAGT<br>GGTGTATCCAGCAGATTTTCCGCCAGCAAGGATGCAAGCGCTAATGCAGGTATTCTTCTCAT<br>TTCAGGCTTGCAAAGCGAGGACGAAGCAGACTACTACTGCATGATTTGGCACTCCGGTGCCT<br>CCGCAGTTTTTGGCTGCGGAACAAAGCTTACAGTCCTTGGTCAGCCCAAGGCTGCCCCCTCG<br>GTCACTCTGTTCCCGCCCTCCTCTGAGGAGCTTCAAGCCAACAAGGCCACACTGGTGTGTCT<br>CATAAGTGACTTCTACCCGGGAGCCGTGACAGTGGCCTGGAAGGCAGATAGCAGCCCCGTCA<br>AGGCGGGAGTGGAGACCACCACACCCTCCAAACAAAGCAACAACAAGTACGCGGCCAGCAGC<br>TATCTGAGCCTGACGCCTGAGCAGTGGAAGTCCCACAGAAGCTACAGCTGCCAGGTCACGCA<br>TGAAGGGAGCACCGTGGAGAAGACAGTGGCCCCTACAGAATGTTCAGGTGGTGGGGGCAGCG<br>GGGGCGGAGGTAGTGGAGGCGGCGGTAGTGAACCAAAGAGTAGTGACAAAACTCACACGTGC<br>CCACCGTGCCCAGCACCTGAACTCCTGGGGGGACCGTCAGTCTTCCTCTTCCCCCCAAAACC<br>CAAGGACACCCTCATGATCTCCCGGACCCCTGAGGTCACATGCGTGGTGGTGGACGTGAGCC<br>ACGAAGACCCTGAGGTCAAGTTCAACTGGTACGTGGACGGCGTGGAGGTGCATAATGCCAAG<br>ACAAAGCCGCGGGAGGAGCAGTACAACAGCACGTACCGTGTGGTCAGCGTCCTCACCGTCCT<br>GCACCAGGACTGGCTGAATGGCAAGGAGTACAAGTGCAAGGTCTCCAACAAAGCCCTCCCAG<br>CCCCCATCGAGAAAACCATCTCCAAAGCCAAAGGGCAGCCCCGAGAACCACAGGTGTACACC<br>CTGCCCCCATGCCGGGAGGAGATGACCAAGAACCAGGTCAGCCTGTGGTGCCTGGTCAAAGG<br>CTTCTATCCCAGCGACATCGCCGTGGAGTGGGAGAGCAATGGGCAGCCGGAGAACAACTACA<br>AGACCACGCCTCCCGTGCTGGACTCCGACGGCTCCTTCTTCCTCTACAGCAAGCTCACCGTG<br>GACAAGAGCAGGTGGCAGCAGGGGAACGTCTTCTCATGCTCCGTGATGCATGAGGCTCTGCA<br>CAACCACTACACGCAGAAGAGCCTCTCCCTGTCTCCGGGTAAA | 717 |
| CEA03 HC | GAGGTACAACTGCAGCAAAGCGGTCCAGGCCTTGTTCGGCCTAGCCAGACTCTGTCACTGAC<br>CTGTACCGCAAGCGGTTTCAATATCAAGGACAACTATATGCACTGGGTCCGCCAGCCTCCAG<br>GACGCTGTTTGGAGTGGATAGGATGGATAGACCCAGAAAATGGGGATACCGAATACGCTCCA<br>AAATTTCGTGGACGAGTTACCATGCTGGCCGATACATCCAAAAACCAGTTTTCTCTGAGGCT<br>CTCCAGCGTGACAGCCGCAGATACCGCCGTATATTACTGTCATGTGTTGATCTATGCCGGGT<br>ATCTTGCAATGGATTACTGGGGACAGGGCACTCTCGTAACAGTCTCTTCAGCTAGCACCAAA<br>GGACCTAGTGTTTTTCCTCTTGCCCCTTCCTCAAAGTCTACCTCTGGGGGGACAGCCGCTCT | 718 |

| | | |
|---|---|---|
| | GGGCTGCCTGGTCAAGGATTATTTCCCAGAGCCTGTCACTGTCAGTTGGAACTCTGGAGCCT TGACTTCTGGTGTTCATACATTTCCTGCTGTCCTTCAAAGCAGCGGCTTGTACTCATTGTCT TCTGTTGTGACAGTACCCTCAAGCAGCCTCGGCACTCAGACATACATCTGCAATGTCAACCA CAAACCCTCAAATACAAAGGTAGATAAAAAAGTCGAACCAAAGTCTTGTGACAAAACTCACA CGTGCCCACCGTGCCCAGCACCTGAACTCCTGGGGGGACCGTCAGTCTTCCTCTTCCCCCCA AAACCCAAGGACACCCTCATGATCTCCCGGACCCCTGAGGTCACATGCGTGGTGGTGGACGT GAGCCACGAAGACCCTGAGGTCAAGTTCAACTGGTACGTGGACGGCGTGGAGGTGCATAATG CCAAGACAAAGCCGCGGGAGGAGCAGTACAACAGCACGTACCGTGTGGTCAGCGTCCTCACC GTCCTGCACCAGGACTGGCTGAATGGCAAGGAGTACAAGTGCAAGGTCTCCAACAAAGCCCT CCCAGCCCCCATCGAGAAAACCATCTCCAAAGCCAAAGGGCAGCCCCGAGAACCACAGGTGT ACACCCTGCCCCCATGCCGGGAGGAGATGACCAAGAACCAGGTCAGCCTGTGGTGCCTGGTC AAAGGCTTCTATCCCAGCGACATCGCCGTGGAGTGGGAGAGCAATGGGCAGCCGGAGAACAA CTACAAGACCACGCCTCCCGTGCTGGACTCCGACGGCTCCTTCTTCCTCTACAGCAAGCTCA CCGTGGACAAGAGCAGGTGGCAGCAGGGGAACGTCTTCTCATGCTCCGTGATGCATGAGGCT CTGCACAACCACTACACGCAGAAGAGCCTCTCCCTGTCTCCGGGTAAA | |
| CEA03 LC | GACATACAAATGACCCAAAGCCCAAGCTCATTGAGCGCCTCCGTAGGAGATCGCGTAACAAT AACCTGCAGCGCCAGCAGTTCAGTAACTTATATGCACTGGTATCAGCAGAAGCCAGGTAAGG CTCCCAAACTGTGGATATATATAGCACCAGCAACCTGGCATCTGGTGTACCCTCTCGATTTAGT GGCAGTGGTTCTGGAACAGACTATACCTTCACTATATCTTCTCTTCAGCCTGAGGATATAGC CACTTACTATTGCCAGCAACGTTCCACTTACCCCCTGACTTTTGGGTGTGGTACAAAGTTGG AAATAAAGCGTACGGTGGCAGCTCCCAGCGTTTTTATCTTTCCCCCATCCGACGAGCAGCTC AAGAGTGGCACTGCCTCTGTAGTTTGTTTGCTGAATAACTTCTATCCACGTGAAGCAAAAGT ACAGTGGAAGGTCGATAATGCCCTTCAGAGCGGTAACAGCCAAGAAAGTGTTACCGAGCAAG ATTCCAAAGATTCCACTTACAGTCTGTCCAGCACATTGACACTGAGTAAGGCTGATTACGAA AAACACAAGGTGTACGCATGCGAGGTGACACACCAAGGTCTTTCATCTCCTGTAACTAAGAG CTTTAACCGGGGAGAATGTGGTGGTGGGGGGCAGCGGGGGCGGAGGTAGTGGAGGCGGCGGTA GTGAACCAAAGAGTAGTGACAAAACTCACACGTGCCCACCGTGCCCAGCACCTGAACTCCTG GGGGGACCGTCAGTCTTCCTCTTCCCCCCAAAACCCAAGGACACCCTCATGATCTCCCGGAC CCCTGAGGTCACATGCGTGGTGGTGGACGTGAGCCACGAAGACCCTGAGGTCAAGTTCAACT GGTACGTGGACGGCGTGGAGGTGCATAATGCCAAGACAAAGCCGCGGGAGGAGCAGTACAAC AGCACGTACCGTGTGGTCAGCGTCCTCACCGTCCTGCACCAGGACTGGCTGAATGGCAAGGA GTACAAGTGCAAGGTCTCCAACAAAGCCCTCCCAGCCCCCATCGAGAAAACCATCTCCAAAG CCAAAGGGCAGCCCCGAGAACCACAGGTGTACACCCTGCCCCCATGCCGGGAGGAGATGACC AAGAACCAGGTCAGCCTGTGGTGCCTGGTCAAAGGCTTCTATCCCAGCGACATCGCCGTGGA GTGGGAGAGCAATGGGCAGCCGGAGAACAACTACAAGACCACGCCTCCCGTGCTGGACTCCG ACGGCTCCTTCTTCCTCTACAGCAAGCTCACCGTGGACAAGAGCAGGTGGCAGCAGGGGAAC GTCTTCTCATGCTCCGTGATGCATGAGGCTCTGCACAACCACTACACGCAGAAGAGCCTCTC CCTGTCTCCGGGTAAA | 719 |
| CEA04 HC | GAAGTGCAGTTGGTGGAGTCTGGGGGCGGGTTGGTCCAACCAGGAGGCTCTCTGCGACTCTC TTGCGCAGCATCAGGCTTCAACATAAAGGACACATATATGCACTGGGTTCGGCAGGCTCCCG GAAAATGTCTTGAATGGGTCGCCCGAATTGATCCTGCAAATGGGAACAGTAAATACGCAGAT TCAGTTAAAGGCCGCTTCACTATCAGCGCAGATACATCCAAAAACACCGCATACCTTCAGAT GAACTCACTCCGTGCAGAAGACACTGCAGTCTACTATTGCGCTCCCTTCGGTTACTACGTCT CTGACTATGCAATGGCTTACTGGGGCCAAGGAACCTTGGTGACTGTATCTTCTGCTAGCACC AAAGGACCTAGTGTTTTTCCTCTTGCCCCTTCCTCAAAGTCTACCTCTGGGGGGACAGCCGC TCTGGGCTGCCTGGTCAAGGATTATTTCCCAGAGCCTGTCACTGTCAGTTGGAACTCTGGAG CCTTGACTTCTGGTGTTCATACATTTCCTGCTGTCCTTCAAAGCAGCGGCTTGTACTCATTG TCTTCTGTTGTGACAGTACCCTCAAGCAGCCTCGGCACTCAGACATACATCTGCAATGTCAA CCACAAACCCTCAAATACAAAGGTAGATAAAAAAGTCGAACCAAAGTCTTGTGACAAAACTC ACGTGCCCACCGTGCCCAGCACCTGAACTCCTGGGGGGACCGTCAGTCTTCCTCTTCCCC | 720 |

| | | |
|---|---|---|
| | CCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATGCCGGGAGGAGATGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | |
| CEA04 LC | GATATACAGCTTACACAATCACCCTCCTCCCTGTCTGCCTCCGTAGGAGATAGAGTAACAATCACATGCAGAGCTGGCGAGAGTGTTGATATATTCGGTGTTGGATTTTTTGCACTGGTACCAACAGAAACCAGGGAAAGCACCTAAGCTCTTGATTTATAGAGCTTCTAACCTTGAGAGCGGGGTGCCTAGTAGGTTTTCTGGGTCAGGAAGTCGGACCGATTTTACTCTCACAATTTCATCCCTTCAGCCCGAAGACTTTGCAACCTACTACTGTCAGCAGACAAACGAAGACCCCTATACATTCGGATGTGGTACAAAGGTGGAGATTAAACGTACGGTGGCAGCTCCCAGCGTTTTTATCTTTCCCCCATCCGACGAGCAGCTCAAGAGTGGCACTGCCTCTGTAGTTTGTTTGCTGAATAACTTCTATCCACGTGAAGCAAAAGTACAGTGGAAGGTCGATAATGCCCTTCAGAGCGGTAACAGCCAAGAAAGTGTTACCGAGCAAGATTCCAAAGATTCCACTTACAGTCTGTCCAGCACATTGACACTGAGTAAGGCTGATTACGAAAAACACAAGGTGTACGCATGCGAGGTGACACACCAAGGTCTTTCATCTCCTGTAACTAAGAGCTTTAACCGGGGAGAATGTGGTGGTGGGGGCAGCGGGGGCGGAGGTAGTGGAGGCGGCGGTAGTGAACCAAAGAGTAGTGACAAAACTCACACGTGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATGCCGGGAGGAGATGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | 721 |

Table 78 below shows the polypeptide sequences of the heavy chain variable region (VH) and light chain variable region (VL) of the engineered antibodies targeting CEACAM5. Table 79 below shows the polynucleotide sequences encoding the polypeptides of the heavy chain variable region (VH) and light chain variable region (VL) of the engineered antibodies targeting CEACAM5.

[Table 78]

228

| Name | | Sequence | SEQ ID NO |
|---|---|---|---|
| CEA01 | VH | EVQLVESGGGVVQPGRSLRLSCSASGFDFTTYWMSWVRQAPGKCLEWIGEIHPDSSTINYAPSLKDRFTISRDNAKNTLFLQMDSLRPEDTGVYFCASLYFGFPWFAYWGQGTPVTVSS | 819 |
| | VL | DIQLTQSPSSLSASVGDRVTITCKASQDVGTSVAWYQQKPGKAPKLLIYWTSTRHTGAPSRFSGSGSGTDFTFTISSLQPEDIATYYCQQYSLARSFGCGTKVEIK | 820 |
| CEA02 | VH | EVQLVESGGGLVQPGRSLRLSCAASGFTVSSYWMHWVRQAPGKCLEWVGFILNKANGGTTEYAASVKGRFTISRDDSKNTLYLQMNSLRAEDTAVYYCARDRGLRFYFDYWGQGTTVTVSS | 821 |
| | VL | QAVLTQPASLSASPGASASLTCTLRRGINVGAYSIYWYQQKPGSPPQYLLRYKSDSDKQQGSGVSSRFSASKDASANAGILLISGLQSEDEADYYCMIWISGASAVFGCGTKLTVL | 822 |
| CEA03 | VH | EVQLQQSGPGLVRPSQTLSLTCTASGFNIKDNYMIHWVRQPPGRCLEWIGWIDPENGDTEYAPKFRGRVTMLADTSKNQFSLRLSSVTAADTAVYYCHVLIYAGYLAMDYWGQGTLVTVSS | 823 |
| | VL | DIQMTQSPSSLSASVGDRVTITCSASSSVTYMHWYQQKPGKAPKLWIYSTSNLASGVPSRFSGSGSGTDYTFTISSLQPEDIATYYCQQRSTYPLTFGCGTKLEIK | 824 |
| CEA04 | VH | EVQLVESGGGLVQPGGSLRLSCAASGFNIKDTYMIHWVRQAPGKCLEWVARIDPANGNSKYADSVKGRFTISADTSKNTAYLQMNSLRAEDTAVYYCAPFGYYVSDYAMAYWGQGTLVTVSS | 825 |
| | VL | DIQLTQSPSSLSASVGDRVTITCRAGESVDIFGVGFLIDFYQQKPGKAPKLLIYRASNLESGVPSRFSGSGSRTDFTLTISSLQPEDFATYYCQQTNEDPYTFGCGTKVEIK | 826 |

[Table 79]

229

| Name | | Sequence | SEQ ID NO |
|---|---|---|---|
| CEA01 | VH | GAAGTGCAACTCGTAGAATCCGGCGGCGGCGTCGTACAGCCCGGCCGTTCTTTGAGACTTAGCTGTAGTGCTTCTGGGTTTGACTTCACTACATACTGGATGTCATGGGTAAGACAGGCACCTGGCAAGTGCCTTGAATGGATCGGTGAAATCCATCCCGACAGCTCCACAATCAACTACGCCCCAAGTTTGAAAGACCGGTTCACCATATCTCGTGACAACGCCAAGAATACATTGTTCCTTCAGATGGATAGTCTTCGTCCAGAGGATACTGGGGTATATTTTTGTGCCAGCTTGTATTTTGGCTTCCCCTGGTTCGCTTATTGGGGCCAAGGTACACCCGTCACTGTCTCTTCT | 827 |
| | VL | GACATCCAACTTACCCAGTCACCCTCATCTCTTAGTGCCTCTGTAGGGGACCGAGTTACTATTACATGTAAAGCCAGTCAAGATGTTGGCACCTCAGTAGCATGGTATCAACAAAAGCCTGGTAAAGCCCCAAAACTGCTGATCTATTGGACAAGCACACGACATACAGGAGTGCCAAGTCGCTTCAGCGGTTCAGGTTCAGGCACAGATTTTACATTCACTATATCAAGCCTGCAACCCGAGGACATTGCCACATATTACTGCCAGCAATATAGTCTGTATCGTAGCTTCGGATGTGGCACCAAGGTTGAAATTAAG | 722 |
| CEA02 | VH | GAAGTTCAACTGGTGGAGTCTGGCGGCGGACTTGTCCAGCCAGGGCGAAGCCTGCGTCTCTCATGCGCTGCCTCCGGTTTCACTGTTTCTTCATACTGGATGCACTGGGTAAGACAGGCTCCTGGCAAGTGTCTTGAATGGGTGGGCTTCATTTTTGAACAAGGCAAACGGCGGGACTACCGAATACGCTGCCAGCGTTAAGGGTCGATTCACCATCTCAAGGGATGATTCTAAAAACACATTGTACCTTCAGATGAACTCTCTGAGGGCCGAGGACACAGCAGTCTACTATTGCGCTAGAGATAGGGGGCTTAGATTCTATTTTGACTACTGGGGGCAGGGAACAACTGTCACCGTTTCCAGT | 723 |
| | VL | CAGGCAGTGCTTACTCAGCCTGCTTCACTCTCTGCCTCACCAGGAGCCATCTGCAAGCCTCACCTGCACATTGCGTCGAGGTATAAACGTAGGCGCTTACTCAATTTACTGGTACCAGCAGAAACCTGGGAGCCCACCTCAATACCTCCTCCGATACAAGAGCGATTCTGATAAGCAACAGGGCAGTGGTGTATCCAGCAGATTTTCCGCCAGCAAGGATGCAAGCGCTAATGCAGGTATTCTTTCATTTCAGGCTTGCAAAGCGAGGACGAAGCAGACTACTACTGCATGATTTGGCACTCCGGTGCCTCCGCAGTTTTTTGGCTGCGGAACAAAGCTTACAGTCCTT | 724 |
| CEA03 | VH | GAGGTACAACTGCAGCAAAGCGGTCCAGGCCTTGTTCGGCCTAGCCAGACTCTGTCACTGACCTGTACCGCAAGCCGGTTTCAATATCAAGGACAACTATATGCACTGGGTCCGCCAGCCTCCAGGACGCTGTTTGGAGTCGATAGGATCGATAGACCCAGAAAAATGGGGATACCGAATACCCT | 725 |

| | | | |
|---|---|---|---|
| | | CCAAAATTTCGTGGACGAGTTACCATGCTGGCCGATACATCCAAAAACCAGTTTTCTCTGA GGCTCTCCAGCGTGACAGCCGCAGATACCGCCGTATATTACTGTCATGTGTTGATCTATGC CGGGTATCTTGCAATGGATTACTGGGGACAGGGCACTCTCGTAACAGTCTCTTCA | |
| | VL | GACATACAAATGACCCAAAGCCCAAGCTCATTGAGCGCCTCCGTAGGAGATCGCGTAACAA TAACCTGCAGCGCCAGCAGTTCAGTAACTTATATGCACTGGTATCAGCAGAAGCCAGGTAA GGCTCCCAAACTGTGGATATATAGCACCAGCAACCTGGCATCTGGTGTACCCTCTCGATTT AGTGGCAGTGGTTCTGGAACAGACTATACCTTCACTATATCTTCTCTTCAGCCTGAGGATA TAGCCACTTACTATTGCCAGCAACGTTCCACTTACCCCCTGACTTTTGGGTGTGGTACAAA GTTGGAAATAAAG | 726 |
| CEA04 | VH | GAAGTGCAGTTGGTGGAGTCTGGGGGCGGGTTGGTCCAACCAGGAGGCTCTCTGCGACTCT CTTGCGCAGCATCAGGCTTCAACATAAAGGACACATATATGCACTGGGTTCGGCAGGCTCC CGGAAAATGTCTTGAATGGGTCGCCCGAATTGATCCTGCAAATGGGAACAGTAAATACGCA GATTCAGTTAAAGGCCGCTTCACTATCAGCGCAGATACATCCAAAAACACCGCATACCTTC AGATGAACTCACTCCGTGCAGAAGACACTGCAGTCTACTATTGCGCTCCCTTCGGTTACTA CGTCTCTGACTATGCAATGGCTTACTGGGGCCAAGGAACCTTGGTGACTGTATCTTCT | 727 |
| | VL | GATATACAGCTTACACAATCACCCTCCTCCCTGTCTGCCTCCGTAGGAGATAGAGTAACAA TCACATGCAGAGCTGGCGAGAGTGTTGATATATTCGGTGTTGGATTTTTGCACTGGTACCA ACAGAAACCAGGGAAAGCACCTAAGCTCTTGATTTATAGAGCTTCTAACCTTGAGAGCGGG GTGCCTAGTAGGTTTTCTGGGTCAGGAAGTCGGACCGATTTTACTCTCACAATTTCATCCC TTCAGCCCGAAGACTTTGCAACCTACTACTGTCAGCAGACAAACGAAGACCCCTATACATT CGGATGTGGTACAAAGGTGGAGATTAAA | 728 |

Table 80 below shows the CDR sequences of the heavy chain and light chain of the engineered antibodies targeting CEACAM5.

[Table 80]

| Name | CDR | Sequence | SEQ ID NO |
|---|---|---|---|
| CEA01 | CDR-H1 | TYWMS | 729 |
| | CDR-H2 | EIHPDSSTINYAPSLKD | 730 |
| | CDR-H3 | LYFGFPWFAY | 731 |
| | CDR-L1 | KASQDVGTSVA | 732 |
| | CDR-L2 | WTSTRHT | 733 |
| | CDR-L3 | QQYSLYRS | 734 |
| CEA02 | CDR-H1 | SYWMH | 735 |
| | CDR-H2 | FILSKANGGTTEYAASVKG | 736 |
| | CDR-H3 | DRGLRFYFDY | 737 |
| | CDR-L1 | TLRRGINVGAYSIY | 738 |
| | CDR-L2 | SDKQQGS | 739 |
| | CDR-L3 | MIWHSGASAV | 740 |
| CEA03 | CDR-H1 | DXYMH | 741 |
| | CDR-H2 | WIDPENGDTEYAPKFRG | 742 |
| | CDR-H3 | LIYAGYLAMDY | 743 |
| | CDR-L1 | SASSSVTYMH | 744 |
| | CDR-L2 | STSNLAS | 745 |
| | CDR-L3 | QQRSTYPLT | 746 |
| CEA04 | CDR-H1 | DTYMH | 747 |
| | CDR-H2 | RIDPANGNSKYADSVKG | 748 |
| | CDR-H3 | FGYYVSDYAMAY | 749 |
| | CDR-L1 | RAGESVDIFGVGFLH | 750 |
| | CDR-L2 | RASNLES | 751 |
| | CDR-L3 | QQTNEDPYT | 752 |

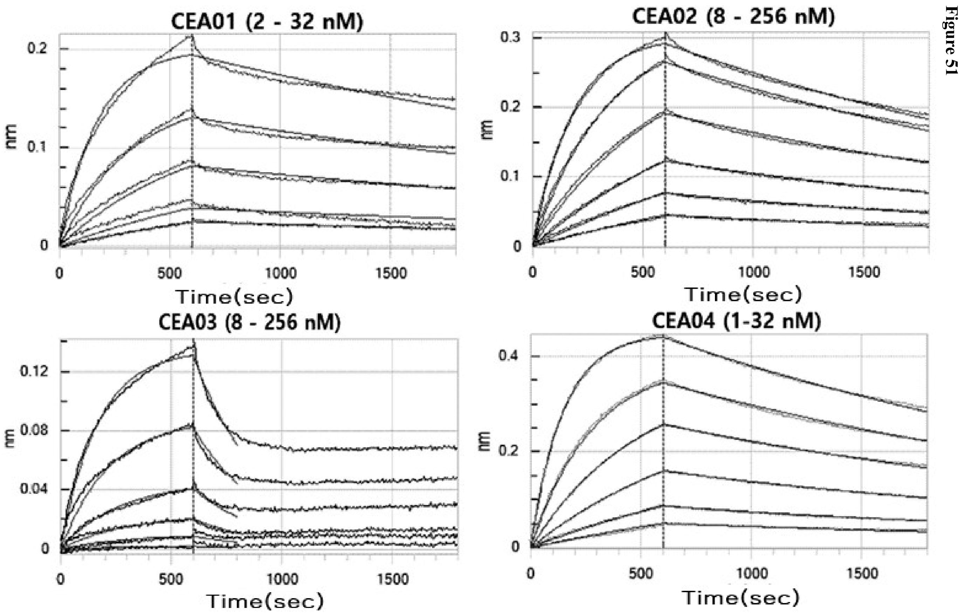

The CEACAM5 protein binding constants of each of CEA01 to CEA04 were determined using Octet Red96e. The human CEACAM5 recombinant protein (Sino Biologicals, 11077-H08H) was loaded onto the Anti-Penta-HIS (HIS1K) biosensor, and then each antibody was added in a binding reaction (600 seconds) and a dissociation reaction (1200 seconds) at various concentrations. Their affinity for the human CEACAM5 protein was calculated, and the results are shown in Figure 51 and Table 81.

[Table 81]

| Antibodies | $K_D$ (nM) | $K_a$ (1/Ms) | $K_d$ (1/s) |
|---|---|---|---|
| CEA01 | 1.5740 | 1.76E+05 | 2.78E-04 |
| CEA02 | 19.2395 | 2.03E+04 | 3.90E-04 |
| CEA03 | 274.7780 | 1.13E+04 | 3.09E-03 |
| CEA04 | 1.7856 | 2.05E+05 | 3.66E-04 |

## Example 23. Design, preparation and analysis of antibody structure targeting TROP2, mesothelin or LIV-1

The polypeptide sequences of the variant light chain and heavy chain of the antibody T01 that specifically binds to the TROP2 protein are shown in Table 82.

T01 consists of the polypeptides of Fc-Hole (SEQ ID NO: 7), T01 HC (SEQ ID NO: 753), and T01 LC (SEQ ID NO: 754), and was prepared by co-transfection of expression vectors loaded with polynucleotides encoding the polypeptides into the ExpiCHO-S™ cell line (Figure 41a). The protein prepared as above was purified and analyzed in the same manner as in Example 1 above.

The polypeptide sequences of the variant light chain and heavy chain of the antibody MSM01 that specifically binds to the mesothelin protein are shown in Table 82.

MSM01 consists of the polypeptides of Fc-Hole (SEQ ID NO: 7), MSM01 HC (SEQ ID NO: 755), and MSM01 LC (SEQ ID NO: 756), and was prepared by co-transfection of expression vectors loaded with polynucleotides encoding the polypeptides into the ExpiCHO-S™ cell line (Figure 41a). The protein prepared as above was purified and analyzed in the same manner as in Example 1 above.

The polypeptide sequences of the variant light chain and heavy chain of the antibody LIM01 that specifically binds to the LIV-1 protein are shown in Table 82.

LIM01 consists of the polypeptides of Fc-Hole (SEQ ID NO: 7), LIM01 HC (SEQ ID NO: 757), and LIM01 LC (SEQ ID NO: 758), and was prepared by co-transfection of expression vectors loaded with polynucleotides encoding the polypeptides into the ExpiCHO-S™ cell line (Figure 41a). The protein prepared as above was purified and analyzed in the same manner as in Example 1 above.

[Table 82]

| Target Ag | Name | Sequence | SEQ ID NO |
|---|---|---|---|
| Human TROP2 | T01 HC | QVQLQQSGSELKKPGASVKVSCKASGYTFTNYGMNWVKQAPGQCLKWMGWINTYTGEPTYTDDFKGRFAFSLDTSVSTAYLQISSLKADDTAVYFCARGGFGSSYWYFDVWGQGSLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 753 |
| | T01 LC | DIQLTQSPSSLSASVGDRVSITCKASQDVSIAVAWYQQKPGKAPKLLIYSASYRYTGVPDRFSGSGSGTDFTLTISSLQPEDFAVYYCQQHYITPLTFGCGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGECGGGGSGGGGSGGGGSEPKSSDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNWVSLWCLVKGFYPSDIAVEWESNGQPENNYKTTPPVLDSDGSFFLYSKLTVDKSRWQQGNVFSCSVMHEALHNHYTQKSLSLSPGK | 754 |
| Human Mesothelin | MSM01 HC | QVQLQQSGPELEKPGASVKISCKASGYSFTGYTMNWVKQSHGKCLEWIGLITPYNGASSYNQKFRGKATLTVDKSSSTAYMDLLSLTSEDSAVYFCARGGYDGRGFDYWGQGTTVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 755 |
| | MSM01 LC | DIELTQSPAIMSASPGEKVTMTCSASSSVSYMHWYQQKSGTSPKRWIYDTSKLASGVPGRFSGSGSGNSYSLTISSVEAEDDATYYCQQWSKHPLTFGCGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGECGGGGSGGGGSGGGGSEPKSSDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 756 |
| Human LIV-1 | LIV01 HC | EIQLQQSGPELMKPGASVKISCKASTYSFTRYFMHWVKQSHGECLEWIGYIDPFNGGTGYNQKFRGKATLTVDKSSSTAYMILSSLTSEDSAVYYCVTYGSDAFDYWGQGTTLTVSSASTKGPSVFPLAPSSKSTSGGTAALGCLVRDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 757 |
| | LIV01 LC | DIVMTQTQKFMSTSVGDRVSVTCKASQNVETDVYWYQQKPGQPPKMLIYSASYRHSGVPDRFTGSGSGTNFTLTISTVQSEDLAEYFCQQYNNYPFTFGCGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGECGGGGSGGGGSGGGGSEPKSSDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 758 |

Table 83 below shows the polynucleotide sequences encoding the polypeptides of the heavy chain and light chain of the engineered antibodies targeting TROP2, mesothelin, or LIV-1.

234

[Table 83]

| Target Ag | Name | Sequence | SEQ ID NO |
|---|---|---|---|
| Human TROP2 | TO1 HC | CAGGTGCAGCTCCAGCAGTCTGGTTCCGAGCTGAAGAAACCTGGGGCTTCAGTCAAGTCTCT TGCAAGGCCTTCAGGTTACACTTTCACCAATTATGGTATGAACTGGGTCAAGCAAGCTCCTGGT CAGTGTTTGAAGTGGATGGGGTGGATAAACACATATACTGGCGAACCTACATACACCGACGAC TTCAAGGGACGGCTTCGCCTTCTCTCTTGACACAAGTGTCTCAACAGCATATCTCCAAATCAGT AGCCTTAAGGCCGACGACACAGCAGTTTATTTTTGCGCTAGGGGTGGATTCGGATCTTCTTAC TGGTATTTCGATGTCTGGGGACAAGGAAGTCTGGTCACAGTTTCCAGCGGCTAGCACCAAAGGA CCTAGTGTTTTTCCTCTTGCCCCTTCCTCAAAGTCTACCTCTGGGGAGGACAGCCCGCTCTGGC TGCCTGGTCAAGGATTATTTCCCAGAGCCTGTCACTGTCAGTTGGAACTCTGGAGCCTTGACT TCTGGTGTTCATACATTTCCTGCTGTCCTTCAAAGCAGCCGGCTTGTACTCATTGTCTTCTGTT GTGACAGTACCCTCAAGCAGCCTCGGCACTCAGACATACATCTGCAATGTCAACCACAAACCC TCAAATACAAAGGTAGATAAAAAAGTCGAACCAAAGTCTTGTGACAAAACTCACACGTGCCCA CCGTGCCCAGCACCTGAACTCCTGGGGGGACCGTCAGTCTTCCTCTTCCCCCCAAAACCCAAG GACACCCTCATGATCTCCCGGACCCCTGAGGTCACATGCGTGGTGGTGGACGTGAGCCACGAA GACCCTGAGGTCAAGTTCAACTGGTACGTGGACGGCGTGGAGGTGCATAATGCCAAGACAAAG CCGCGGGAGGAGCAGTACAACAGCACGTACCGTGTGGTCAGCGTCCTCACCGTCCTGCACCAG GACTGGCTGAATGGCAAGGAGTACAAGTGCAAGGTCTCCAACAAAGCCCTCCCAGCCCCCATC GAGAAAACCATCTCCAAAGCCAAAGGGCAGCCCCGAGAACCACAGGTGTACACCCTGCCCCCA TGCCCGGAGGAGATGACCAAGAACCAGGTCAGCCTGTCGGTGCCTGGGTCAAAGGCTTCTATCCC AGCGACATCGCCGTGGAGTGGGAGAGCAATGGGCAGCCGGAGAACAACTACAAGACCACGCCT CCCGTGCTGGACTCCGACGGCTCCTTCTTCCTCTACAGCAAGCTCACCGTGGACAAGAGCAGG TGGCAGCAGGGGAACGTCTTCTCATGCTCCGTGATGCATGAGGCTCTGCACAACCACTACACG CAGAAGAGCCTCTCCCTGTCTCCGGGTAAA | 759 |
| | TO1 LC | GACATACAACTCACACAATCTCCCTCTTCTTTGTCAGCTTCCGTTGGGGACAGGGTGTCAATT ACTTGCAAAGCCTCTCAAGATGTTTCTATAGCTGTAGCCTGGTATCAACAGAAACCCGGAAAA GCTCCCAAGTTGTTGATTTATAGTGCTAGTTATAGGTACACTGGCGTGCCAGATAGATTCAGT GGTAGCGGTTCTGGGACCGACTTTACCTTGACTATTTCTTCCCTGCAACCTGACGAATTTTGCC GTTTACTATTGCCAACAACATTATATTACTCCCCTTACTTTTGGGTGTGGGACCAAGGTAGAA ATCAAGCGTACGGTGGCAGCTCCCAGCGGTTTTATCTTTCCCCCATCCGACGAGCCAGCTCAAG AGTGGCACTTCCTCTGTAGTTGTTGCTGAATAACTTCTATCCACGTGAAGCAAAAGTACAG TGGAAGGTCGATAATGCCCTTCAGAGCGGTAACAGCCAAGAAAGTGTTACCGAGCAAGATTCC AAAGATTCCACTTACAGTCTGTCCAGCACATTGACACTGAGTAAGGCTGATTACGAAAAACAC AAGGTGTACGCCATGCGAGGTGACACACCAAGGTCTTTCATCTCCTGTAACTAAGAGCTTTAAC CGGGGAGAATGTGTGGTGGTGGGGGGCAGCGGGGGCGGAGGTAGTGGAGGCGGCGGTAGTGAACCA AAGAGTAGTGACAAAACTCACACGTGCCCACCGTGCCCAGCACCTGAACTCCTGGGGGGACCG TCAGTCTTCCTCTTCCCCCCAAAACCCAAGGACACCCTCATGATCTCCCGGACCCCTGAGGTC ACATGCGTGGTGGTGGACGTGAGCCACGAAGACCCTGAGGTCAAGTTCAACTGGTACGTGGAC GGCGTGGACGTGCCATAATGCCAAGACAAACCGCGGGAGGAGCAGTACAACAGCACGTACCGT GTGGTCAGCGTCCTCACCGTCCTGCACCAGGACTGGCTGAATGGCAAGGAGTACAAGTGCAAG GTCTCCAACAAAGCCCTCCCAGCCCCCATCGAGAAAACCATCTCCAAAGCCAAAGGGCAGCCC CGAGAACCACAGGTGTACACCCTGCCCCCATCCCGGGAGGAGATGACCAAGAACCAGGTCAGC CTGTGGTGCCTGGTCAAAGGCTTCTATCCCAGCGACATCGCCGTGGAGTGGGAGAGCAATGGG CAGCCGGAGAACAACTACAAGACCACGCCTCCCGTGCTGGACTCCGACGGCTCCTTCTTCCTC TACAGCAAGCTCACCGTGGACAAGAGCAGGTGGCAGCAGGGGAACGTCTTCTCATGCTCCGTG ATGCATGAGGCTCTGCACAACCACTACACGCAGAAGAGCCTCTCCCTGTCTCCGGGTAAA | 760 |
| Human Mesothelin | MSM01 HC | CAGGTTCAATTGCAGCAGTCTGGACCAGAATTCGAGAAGCCTGGCGCAAGCGGTCAAAATATCT TGCAAGGCTTCAGGTTACAGTTTCACCGGATACACTATGAACTGGGTCAAGCAAAGCCACGGC AAGTGTCTTGAATGGATAGGATTGATTACTCCATACAATGGCGCTTCATCATACAATCAGAAG | 761 |

| | | | |
|---|---|---|---|
| | | TTTAGGGGCAAGGCAACTTTGACCGTGGATAAGTCATCATCTACCGCATATATGGACCTCTTG AGCCTCACAAGTGAAGACTCAGCTGTTTACTTTTGTGCCAGAGGAGGGTATGATGGGCGAGGA TTCGACTATTGGGGTCAAGGAACCACCGTGACAGTAAGCTCTGCTAGCACCAAAGGACCTAGT GTTTTTCCTCTTGCCCCTTCCTCAAAGTCTACCTCTGGGGGGACAGCCGCTCTGGGCTGCCTG GTCAAGGATTATTTCCCAGAGCCTGTCACTGTCAGTTGGAACTCTGGAGCCTTGACTTCTGGT GTTCATACATTTCCTGCTGTCCTTCAAAGCAGCGGCTTGTACTCATTGTCTTCTGTTGTGACA GTACCCTCAAGCAGCCTCGGCACTCAGACATACATCTGCAATGTCAACCACAAACCCTCAAAT ACAAAGGTAGATAAAAAAGTCGAACCAAAGTCTTGTGACAAAACTCACACGTGCCCACCGTGC CCAGCACCTGAACTCCTGGGGGGACCGTCAGTCTTCCTCTTCCCCCCAAAACCCAAGGACACC CTCATGATCTCCCGGACCCCTGAGGTCACATGCGTGGTGGTGGACGTGAGCCACGAAGACCCT GAGGTCAAGTTCAACTGGTACGTGGACGGCGTGGAGGTGCATAATGCCAAGACAAAGCCGCGG GAGGAGCAGTACAACAGCACGTACCGTGTGGTCAGCGTCCTCACCGTCCTGCACCAGGACTGG CTGAATGGCAAGGAGTACAAGTGCAAGGTCTCCAACAAAGCCCTCCCAGCCCCCATCGAGAAA ACCATCTCCAAAGCCAAAGGGCAGCCCCGAGAACCACAGGTGTACACCCTGCCCCCATGCCGG GAGGAGATGACCAAGAACCAGGTCAGCCTGTGGTGCCTGGTCAAAGGCTTCTATCCCAGCGAC ATCGCCGTGGAGTGGGAGAGCAATGGGCAGCCGGAGAACAACTACAAGACCACGCCTCCCGTG CTGGACTCCGACGGCTCCTTCTTCCTCTACAGCAAGCTCACCGTGGACAAGAGCAGGTGGCAG CAGGGGAACGTCTTCTCATGCTCCGTGATGCATGAGGCTCTGCACAACCACTACACGCAGAAG AGCCTCTCCCTGTCTCCGGGTAAA | |
| | MSM01 LC | GACATAGAGTTGACTCAATCTCCAGCAATCATGTCAGCCTCACCCGGAGAAAAGGTCACCATG ACTTGTTCTGCAAGTTCCAGCGTTTCTTATATGCATTGGTACCAGCAGAAGTCAGGGACTAGC CCTAAGAGATGGATTTACGATACCTCCAAACTGGCCTCCGGGGTGCCAGGCCGGTTTAGTGGC AGTGGAAGCGGTAACAGCTACTCTTTGACCATATCTAGCGTGGAAGCAGAGGACGACGCTACT TATTACTGTCAACAGTGGTCTAAGCACCCACTGACCTTCGGCTGTGGTACAAAGCTCGAAATA AAACGTACGGTGGCAGCTCCCAGCGTTTTTATCTTTCCCCCATCCGACGAGCAGCTCAAGAGT GGCACTGCCTCTGTAGTTTGTTTGCTGAATAACTTCTATCCACGTGAAGCAAAAGTACAGTGG AAGGTCGATAATGCCCTTCAGAGCGGTAACAGCCAAGAAAGTGTTACCGAGCAAGATTCCAAA GATTCCACTTACAGTCTGTCCAGCACATTGACACTGAGTAAGGCTGATTACGAAAAACACAAG GTGTACGCATGCGAGGTGACACACCAAGGTCTTTCATCTCCTGTAACTAAGAGCTTTAACCGG GGAGAATGTGGTGGTGGGGGCAGCGGGGGCGGAGGTAGTGGAGGCGGCGGTAGTGAACCAAAG AGTAGTGACAAAACTCACACGTGCCCACCGTGCCCAGCACCTGAACTCCTGGGGGGACCGTCA GTCTTCCTCTTCCCCCCAAAACCCAAGGACACCCTCATGATCTCCCGGACCCCTGAGGTCACA TGCGTGGTGGTGGACGTGAGCCACGAAGACCCTGAGGTCAAGTTCAACTGGTACGTGGACGGC GTGGAGGTGCATAATGCCAAGACAAAGCCGCGGGAGGAGCAGTACAACAGCACGTACCGTGTG GTCAGCGTCCTCACCGTCCTGCACCAGGACTGGCTGAATGGCAAGGAGTACAAGTGCAAGGTC TCCAACAAAGCCCTCCCAGCCCCCATCGAGAAAACCATCTCCAAAGCCAAAGGGCAGCCCCGA GAACCACAGGTGTACACCCTGCCCCCATGCCGGGAGGAGATGACCAAGAACCAGGTCAGCCTG TGGTGCCTGGTCAAAGGCTTCTATCCCAGCGACATCGCCGTGGAGTGGGAGAGCAATGGGCAG CCGGAGAACAACTACAAGACCACGCCTCCCGTGCTGGACTCCGACGGCTCCTTCTTCCTCTAC AGCAAGCTCACCGTGGACAAGAGCAGGTGGCAGCAGGGGAACGTCTTCTCATGCTCCGTGATG CATGAGGCTCTGCACAACCACTACACGCAGAAGAGCCTCTCCCTGTCTCCGGGTAAA | 762 |
| Human LIV-1 | LIM01 HC | GAGATACAGTTGCAGCAAAGCGGACCCGAGCTTATGAAACCAGGAGCTAGTGTGAAAATTAGC TGCAAGGCTAGCACCTACTCTTTTACTCGCTATTTTATGCATTGGGTTAAACAGTCACATGGT GAGTGTTTGGAATGGATCGGGTACATTGATCCCTTTAATGGAGGGACTGGCTACAACCAGAAG TTTAAAGGAAAAGCCACTCTCACTGTTGACAAAAGTAGTAGTACAGCATATATGCACCTCAGT TCCCTTACCAGTGAAGATAGCGCAGTTTACTATTGTGTCACTTACGGATCAGACTACTTCGAC TATTGGGGACAGGGTACAACCCCTTACAGTCTCCAGTGCTAGCACCAAAGGACCTAGTGTTTTT CCTCTTGCCCCTTCCTCAAAGTCTACCTCTGGGGGGACAGCCGCTCTGGGCTGCCTGGTCAAG GATTATTTCCCAGAGCCTGTCACTGTCAGTTGGAACTCTGGAGCCTTGACTTCTGGTGTTCAT ACATTTCCTGCTGTCCTTCAAAGCAGCGGCTTGTACTCATTGTCTTCTGTTGTGACAGTACCC | 763 |

| | | | | |
|---|---|---|---|---|
| | | TCAAGCAGCCTCGGCACTCAGACATACATCTGCAATGTCAACCACAAACCCTCAAATACAAAG GTAGATAAAAAAGTCGAACCAAAGTCTTGTGACAAAACTCACACGTGCCCACCGTGCCCAGCA CCTGAACTCCTGGGGGGACCGTCAGTCTTCCTCTTCCCCCCAAAACCCAAGGACACCCTCATG ATCTCCCGGACCCCTGAGGTCACATGCGTGGTGGTGGACGTGAGCCACGAAGACCCTGAGGTC AAGTTCAACTGGTACGTGGACGGCGTGGAGGTGCATAATGCCAAGACAAAGCCGCGGGAGGAG CAGTACAACAGCACGTACCGTGTGGTCAGCGTCCTCACCGTCCTGCACCAGGACTGGCTGAAT GGCAAGGAGTACAAGTGCAAGGTCTCCAACAAAGCCCTCCCAGCCCCCATCGAGAAAACCATC TCCAAAGCCAAAGGGCAGCCCCGAGAACCACAGGTGTACACCCTGCCCCCATGCCGGGAGGAG ATGACCAAGAACCAGGTCAGCCTGTGGTGCCTGGTCAAAGGCTTCTATCCCAGCGACATCGCC GTGGAGTGGGAGAGCAATGGGCAGCCGGAGAACAACTACAAGACCACGCCTCCCGTGCTGGAC TCCGACGGCTCCTTCTTCCTCTACAGCAAGCTCACCGTGGACAAGAGCAGGTGGCAGCAGGGG AACGTCTTCTCATGCTCCGTGATGCATGAGGCTCTGCACAACCACTACACGCAGAAGAGCCTC TCCCTGTCTCCGGGTAAA | |
| | LIMO1 LC | GACATAGTGATGACCCAACCACAAAAGTTTATGTCAACCTCCGTTGGAGATCGTGTTTCTGTC ACTTGTAAGGCATCACAGAATGTAGAGACAGACGTAGTCTGGTATCAGCAGAAACCTGGTCAG CCACCTAAAGCACTCATCTACAGTGCAAGTTACCGACATTCTGGCGTACCCGACAGATTTACT GGGTCTGGTTCTGGTACAAATTTCACTCTCACCATCTCAACCGTCCAATCAGAAGACTTGGCC GAGTATTTTTGCCAGCAATATAACAATTATCCCTTTACATTCGGATGTGGGACCAAACTCGAA ATCAAACGTACGGTGGCAGCTCCCAGCGTTTTTATCTTTCCCCCATCCGACGAGCAGCTCAAG AGTGGCACTGCCTCTGTAGTTTGTTTGCTGAATAACTTCTATCCACGTGAAGCAAAAGTACAG TGGAAGGTCGATAATGCCCTTCAGAGCGGTAACAGCCAAGAAAGTGTTACCGAGCAAGATTCC AAAGATTCCACTTACAGTCTGTCCAGCACATTGACACTGAGTAAGGCTGATTACGAAAAACAC AAGGTGTACGCATGCGAGGTGACACACCAAGGTCTTTCATCTCCTGTAACTAAGAGCTTTAAC CGGGGAGAATGTGGTGGTGGGGGCAGCGGGGGCGGAGGTAGTGGAGGCGGCGGTAGTGAACCA AAGAGTAGTGACAAAACTCACACGTGCCCACCGTGCCCAGCACCTGAACTCCTGGGGGGACCG TCAGTCTTCCTCTTCCCCCCAAAACCCAAGGACACCCTCATGATCTCCCGGACCCCTGAGGTC ACATGCGTGGTGGTGGACGTGAGCCACGAAGACCCTGAGGTCAAGTTCAACTGGTACGTGGAC GGCGTGGAGGTGCATAATGCCAAGACAAAGCCGCGGGAGGAGCAGTACAACAGCACGTACCGT GTGGTCAGCGTCCTCACCGTCCTGCACCAGGACTGGCTGAATGGCAAGGAGTACAAGTGCAAG GTCTCCAACAAAGCCCTCCCAGCCCCCATCGAGAAAACCATCTCCAAAGCCAAAGGGCAGCCC CGAGAACCACAGGTGTACACCCTGCCCCCATGCCGGGAGGAGATGACCAAGAACCAGGTCAGC CTGTGGTGCCTGGTCAAAGGCTTCTATCCCAGCGACATCGCCGTGGAGTGGGAGAGCAATGGG CAGCCGGAGAACAACTACAAGACCACGCCTCCCGTGCTGGACTCCGACGGCTCCTTCTTCCTC TACAGCAAGCTCACCGTGGACAAGAGCAGGTGGCAGCAGGGGAACGTCTTCTCATGCTCCGTG ATGCATGAGGCTCTGCACAACCACTACACGCAGAAGAGCCTCTCCCTGTCTCCGGGTAAA | 764 |

Table 84 below shows the polypeptide sequences of the heavy chain variable region (VH) and light chain variable region (VL) of the engineered antibodies targeting TROP2, mesothelin, or LIV-1. Table 85 below shows the polynucleotide sequences encoding the polypeptides of the heavy chain variable region (VH) and light chain variable region (VL) of the engineered antibodies targeting TROP2, mesothelin, or LIV-1.

[Table 84]

| Ag | Name | | Sequence | SEQIDNO |
|---|---|---|---|---|
| Human TROP2 | T01 | VH | QVQLQQSGSELKKPGASVKVSCKASGYTFTNYGMNWVKQAPGQCLKWMGWINTYTGEPTYT DDFKGRFAFSLDTSVSTAYLQISSLKAIIDTAVYFCARGGFGSSYWYFDVWGQGSLVTVSS | 765 |
| | | VL | DIQLTQSPSSLSASVGDRVSITCKASQDVSIAVAWYQQKPGKAPKLLIYSASYRYTGVPDR FSGSGSGTDFTLTISSLQPEDFAVYYCQQHYTTPLTFGCGTKVEIK | 766 |
| Human Mesothelin | MSM01 | VH | QVQLQQSGPELKKPGASVKISCKASGYSFTGYTMNWVKQSHGKCLEWIGLITIANGASSYN QKFRGKATLTVDKSSSTAYMDLLSLTSEDSAVYFCARGGYDGRGFDYWGQGTTVTVSS | 767 |
| | | VL | DIELTQSPAIMSASPGEKVTMTCSASSSVSYMBWYQQKSGTSPKRWIYDTSKLASGVPGRF SGSGSGNSYSLTISSVEMEDDATYYCQQWSKHPLTFGCGTKLEIK | 768 |
| Human LIV-1 | LIM01 | VH | EIQLQQSGPELMKPGASVKISCKASTYSFTRYFMHWVKQSHGECLEWIGYIDPFNGGTGYN QKFKGKATLTVDKSSSTAYMHLSSLTSEDSAVYYCVTYGSDVFDYWGQGTTLTVSS | 769 |
| | | VL | DIVMTQFQKFMSTSVGDRVSVTCKASQNVETDVVWYQQKPGQPTKALIYSASYRHSGVPDR FTGSGSGTNFTLTISTVQSEDLAEYFCQQYNNYPFTFGCGTKLEIK | 770 |

[Table 85]

| Ag | Name | | Sequence | SEQIDNO |
|---|---|---|---|---|
| Human TROP2 | T01 | VH | CAGGTGCAGCTCCAGCAGTCTGGTTCCGAGCTGAAGAAACCTGGGGCTTCAGTCAAAGTCT CTTGCAAGGCTTCAGGTTACACTTTCACCAATTATGGTATGAACTGGGTCAAGCAAGCTCC TGGTCAGTGTTTGAAGTGGATGGGGTGGATAAACACATATACTGGCGAACCTACATACACC GACGACTTCAAGGGACGGCTTCGCCTTCTCTCTTGACACAAAGTGTCTCAACAGCATATCTCC AAATCAGTAGCCCTTAAGGCCGACGACACAGCAGTTTATTTTTGCGCTAGGGGTGGATTCGG ATCTTCTTACTGGTATTTCGATGTCTGGGGACAAGGAAGTCTGGTCACAGTTTCCAGC | 771 |
| | | VL | GACATACAACTCACACAATCTCCCTCTTCTTTGTCAGCTTCCGTTGGGGACAGGGTGTCAA TTACTTGCAAAGCCTCTCAAGATGTTTCTATAGCTGTAGCCTGGTATCAACAGAAACCCGG AAAAGCTCCCAAGTTGTTGATTTATAGTGCTAGTTATAGGTACACTGGCGTGCCAGATAGA TTCAGTGGTAGCGGTTCTGGGACCGACTTTACCTTGACTATTTCTTCCCTGCAACCTGAGG ATTTTGCCGTTTACTATTGCCAACAACATTATATTACTCCCCTTACTTTTGGGTGTGGGAC CAAGGTAGAAATCAAG | 772 |
| Human Mesothelin | MSM01 | VH | CAGGTTCAATTGCAGCAGTCTGGACCAGAATTGGAGAAGCCTGGCGCAAGCGTCAAAATAT CTTGCAAGGCTTCAGGTTACAGTTTCACCGGATACACTATGAACTGGGTCAAGCAAAGCCA CGGCAAGTGTCTTGAATGGATAGGATTGATTACTCCATACAATGGCGCTTCATCATACAAT CAGAAGTTTAGGGGCAAGGCAACTTTGACCGTGGATAAGTCATCATCTACCGCATATATGG ACCTCTTGAGCCTCACAAGTGAAGACTCAGCTGTTTACTTTTGTGCCAGAGGAGGGTATGA TTGGCGGAGGATTCGACTATTGGGGGTCAAGGAACCACCGTGACAGTAAGCTCT | 773 |
| | | VL | GACATAGAGTTGACTCAATCTCCAGCAATCATGTCAGCCTCACCCGGAGAAAAGGTCACCA TGACTTGTTCTGCAAGTTCCAGCGTTTCTTATATGCATTGGTACCAGCAGAAGTCAGGGAC TAGCCCTAAGAGATGGATTTACGATACCTCCAAACTGGCCTCCGGGGGTGCCAGGCCGGTTT AGTGGCAGTGGAAGCGGTAACAGCTACTCTTTGACCATATCTAGCGTGGAAGCAGAGGACG ACGCTACTTATTACTGTCAACAGTGGTCTAAGCACCCACTGACCTTCGGCTGTGGTACAAA GCTCGAAATAAAA | 774 |
| Human LIV-1 | LIM01 | VH | GAGATACAGTTGCAGCAAAGCGGACCCGAGCTTATGAAACCAGGAGCTAGTGTGAAAATTA GCTGCAAGGCTAGCACCTACTCTTTTACTCGCTATTTTATGCATTGGGTTAAACAGTCACA TGGTGAGTGTTTGGAATGGATCGGGTACATTGATCCCTTTAATGGAGGGACTGGCTACAAC CAGAAGTTTAAAGGAAAAGCCACTCTCACTGTTGACAAAAGTAGTAGTACAGCATATATGC ACCTCAGTTCCCTTACCAGTGAAGATAGCGCAGTTTACTATTGTGTCACTTACGGATCAGA CTACTTCGACTATTGGGGACAGGGTACAACCCTTACAGTCTCCAGT | 775 |
| | | VL | GACATAGTGATGACCCAACCACAAAGTTTATGTCAACCTCCGTTGGAGATCGTGTTTCTG TCACTTGTAAGGCATCACAGAATGTAGAGACAGACGTAGTCTGGTATCAGCAGAAACCTGG TCAGCCACCTAAAGCACTCATCTACAGTGCAAGTTACCGACATTCTGGCGTACCCGACAGA TTTACTGGGTCTGGTTCTGGTACAAATTTCACTCTCACCATCTCAACCGTCCAATCAGAAG ACTTGCCCGAGTATTTTTGCCAGCAATATAACAATTATCCCTTTACATTCGGATGTGGGAC CAAACTCGAAATCAAA | 776 |

Table 86 below shows the CDR sequences of the heavy chain and light chain of the engineered antibodies targeting TROP2, mesothelin, or LIV-1.

[Table 86]

| Ag | Name | CDR | Sequence | SEQ ID NO |
|---|---|---|---|---|
| human TROP2 | CEA01 | CDR-H1 | NYGMN | 260 |
| | | CDR-H2 | WINTYTGEPTYTDDFKG | 261 |
| | | CDR-H3 | GGFGSSYWYFDV | 262 |
| | | CDR-L1 | KASQDVSIAVA | 263 |
| | | CDR-L2 | SASYRYT | 264 |
| | | CDR-L3 | QQHYITPLT | 265 |
| human Mesothelin | MSM01 | CDR-H1 | GYTMN | 777 |
| | | CDR-H2 | LITPYNGASSYNQKFKG | 778 |
| | | CDR-H3 | GGYDGRGFDY | 779 |
| | | CDR-L1 | SASSSVSYMH | 780 |
| | | CDR-L2 | DTSKLAS | 781 |
| | | CDR-L3 | QQWSKHPLT | 782 |
| Human LIV-1 | LIM01 | CDR-H1 | RYFMH | 783 |
| | | CDR-H2 | YIDPFNGGTGYNQKFKG | 784 |
| | | CDR-H3 | YGSDYFDY | 785 |
| | | CDR-L1 | KASQNVETDVV | 786 |
| | | CDR-L2 | SASYRHS | 787 |
| | | CDR-L3 | QQYNNYP | 788 |

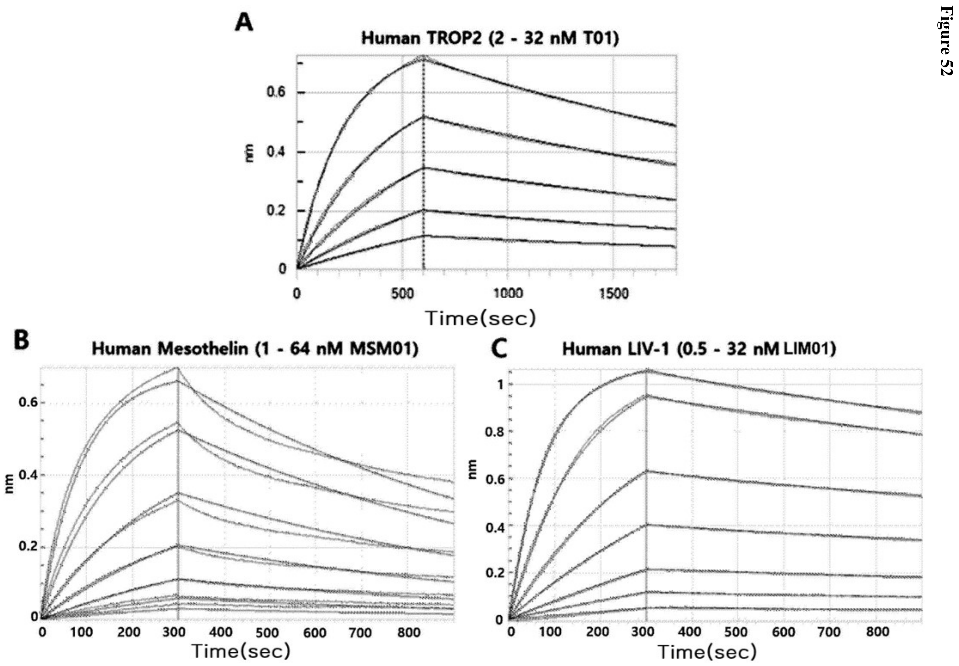

The binding constants of each antibody to TROP2, mesothelin or LIV-1 were determined using Octet Red96e. Each of the human TROP2 recombinant protein (Sino Biologicals, 10428-H08H), the human mesothelin recombinant protein (Sino Biologicals, 13128-H08H), or the human LIV-1 recombinant protein (Acro biosystems, LV1-H5223) was loaded onto the Anti-Penta-HIS (HIS1K) biosensor. Then, each antibody was added in a binding reaction (600 seconds) and a dissociation reaction (1200 seconds) at various concentrations. The affinities of each antibody for the TROP2, mesothelin, or LIV-1 protein was calculated, and the results are shown in Figure 52 and Table 87.

[Table 87]

| Antigens | Antibodies | $K_D$ (nM) | $K_a$ (1/Ms) | $K_d$ (1/s) |
|---|---|---|---|---|
| Human TROP2 | T01 | 1.2008 | 2.67E+05 | 3.21E−04 |
| Human Mesothelin | MSM01 | 7.1771 | 1.59E+05 | 1.14E−03 |
| Human LIV-1 | LIM01 | 0.8072 | 3.84E+05 | 3.10E−04 |

**Example 24. Design, preparation and analysis of novel antibody format with two Fc domains**

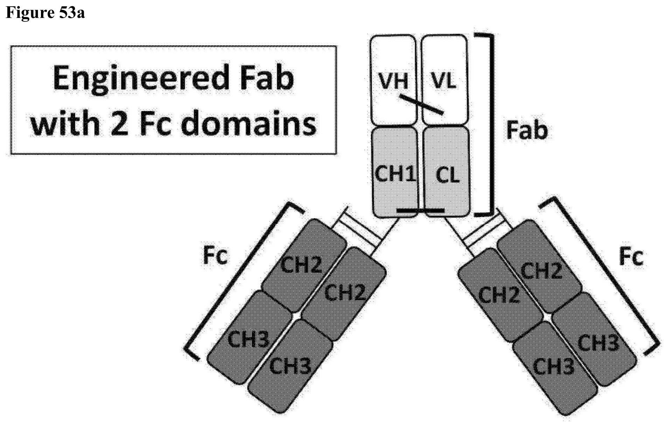

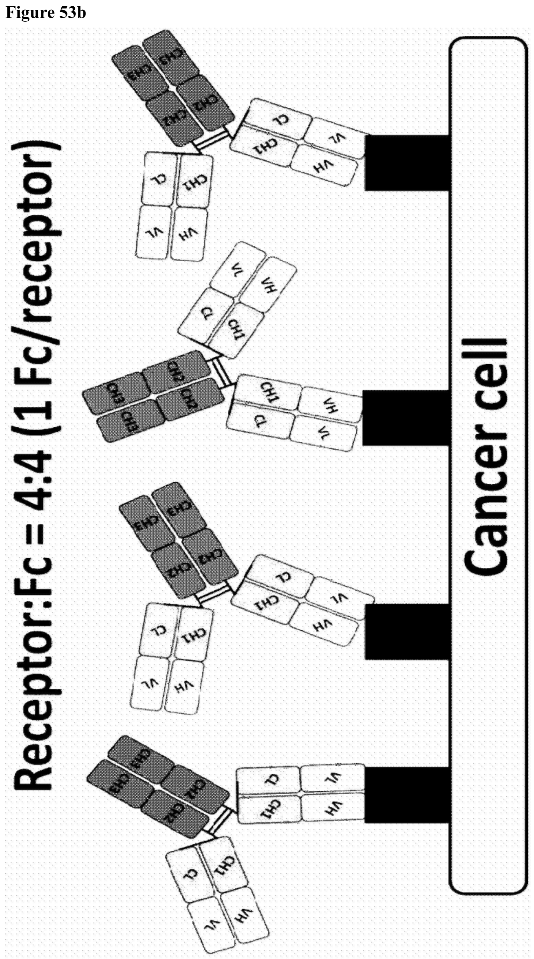

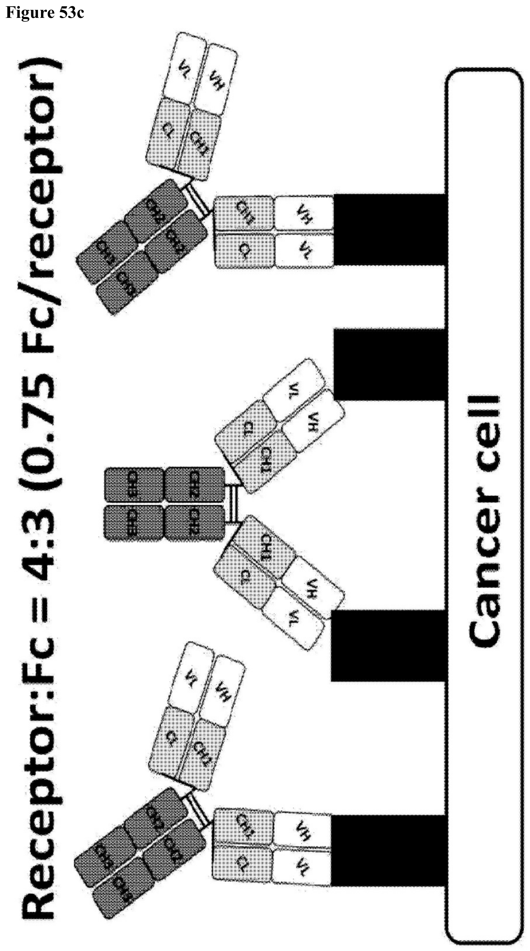

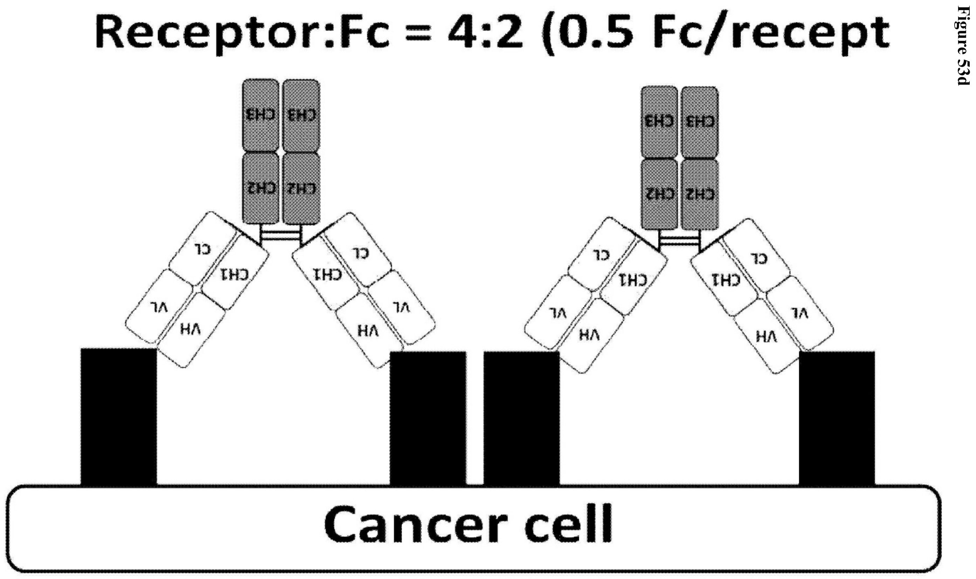

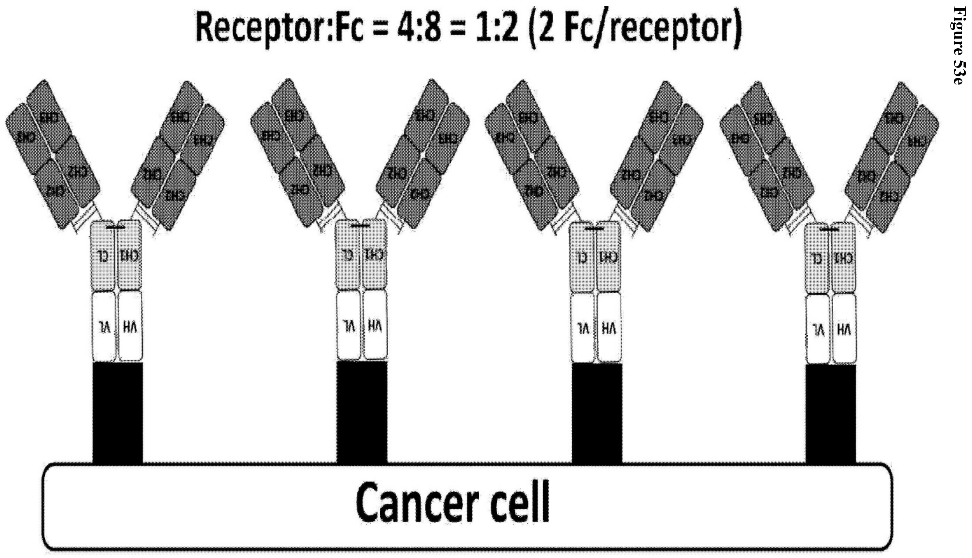

Natural human immunoglobulin G1 consists of two fragment antigen-binding (Fab) regions and one fragment crystallizable (Fc) domain (Figure 1a). Human IgG1 binds to the target antigen in a monovalent or bivalent manner, and in some cases has 0.5 to 1 Fc region per target antigen (Figures 53b to 53d). The object of the present invention is to improve effector function by increasing the Fc loads per antigen while having a molecular weight similar to that of an antibody and having a homogeneous composition. Therefore, we designed a novel antibody format with two Fc domains that has a molecular weight similar to that of a natural human IgG1 antibody (approximately 150 kDa) (Figure 53a). This form of antibody binds to a cancer cell surface antigen and can have Fc loads on a cancer cell surface a maximum of four times compared to existing antibodies (Figure 53e).

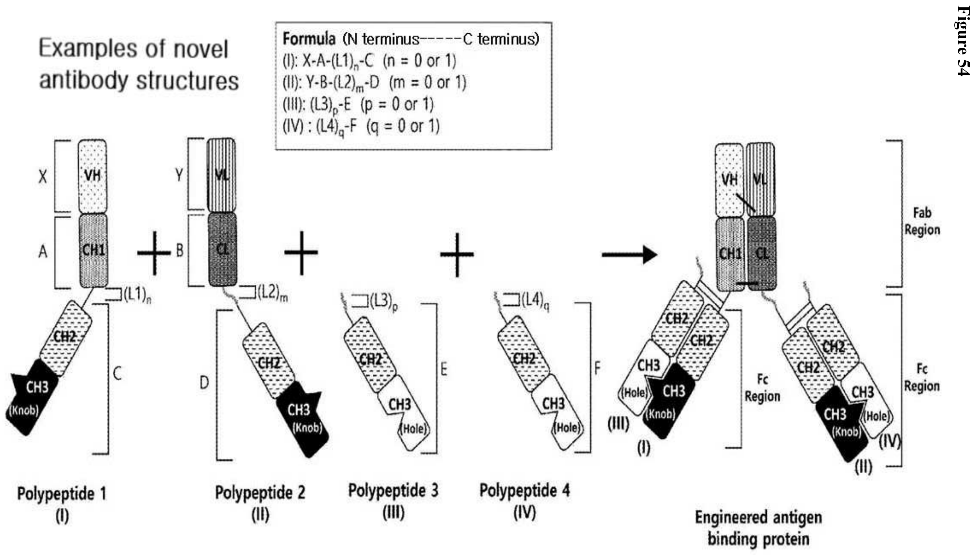

Polypeptide 1 of the novel antibody format according to the present invention (Figure 54) consists of a heavy chain variable region (X), a heavy chain CH1 region (A), a linker (L1), and a hinge-CH2 region-CH3 with Knob mutation (C); Polypeptide 2 consists of a light chain variable region (Y), a light chain CL region (B), a linker (L2), and a hinge-CH2 region-CH3 with Knob mutation (D); Polypeptide 3 consists of a linker (L3) and a hinge-CH2 region-CH3 with Hole mutation (E); and Polypeptide 4 consists of a linker (L4) and a hinge-CH2 region-CH3 with Hole mutation (F). In addition, the antibody format may comprise Cys mutations in a heavy chain variable region and a light chain variable region, a CH1 and a light chain constant region, or a heavy chain variable region and a light chain variable region to induce additional interchain disulfide bonds in addition to the naturally occurring disulfide bonds connecting the heavy and light chains of the Fab region. H01 is a novel antibody format consisting of two Fc regions and one trastuzumab Fab region.

**Example 25. Analysis of binding affinity of novel antibody format with two Fc domains to Fcγ receptors**

A novel antibody format with two Fc domains may have improved binding affinities due to an increased avidity effect compared to a human IgG1 antibody for the Fcγ receptors that mediates the effector functions (ADCC, ADCP, and CDC) of an antibody. In order to compare the binding affinities of trastuzumab and H01 to the Fcγ receptors, the binding constants of each antibody to the Fcγ receptors were determined using Octet Red96e. The human FcγRI, the human FcγIIA (131H isoform) or the human FcγRIIIA (158V isoform) was loaded onto the Anti-Penta-HIS (HIS1K) biosensor, and each of trastuzumab and H01 was added in a binding reaction and a dissociation reaction. The association rate constant ($K_a$) and the dissociation rate constant ($K_d$) values were calculated through a 1:1 binding model in Octet analysis software (Sartorius), and the equilibrium dissociation constant ($K_D$) value was calculated.

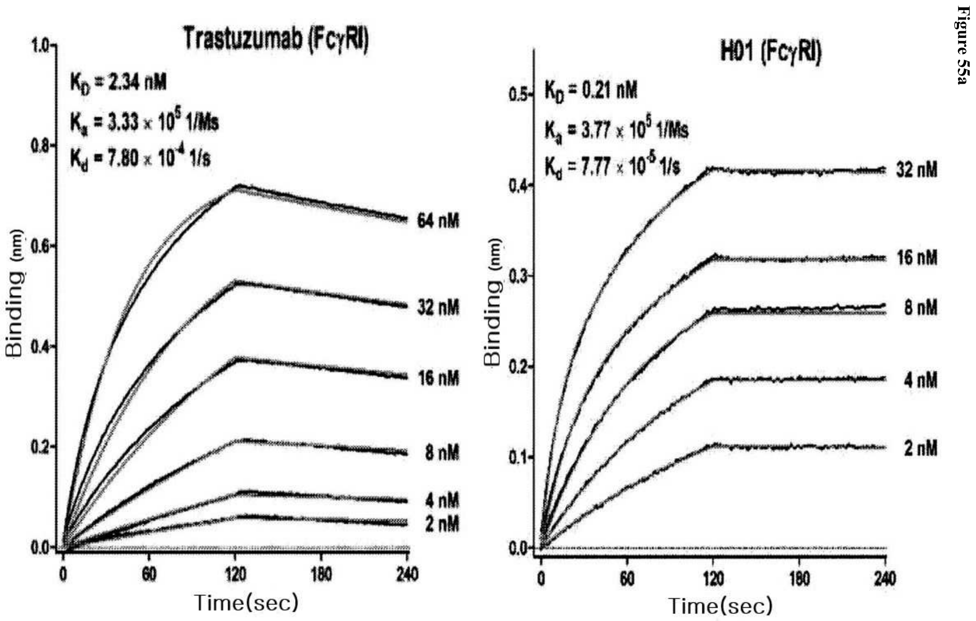

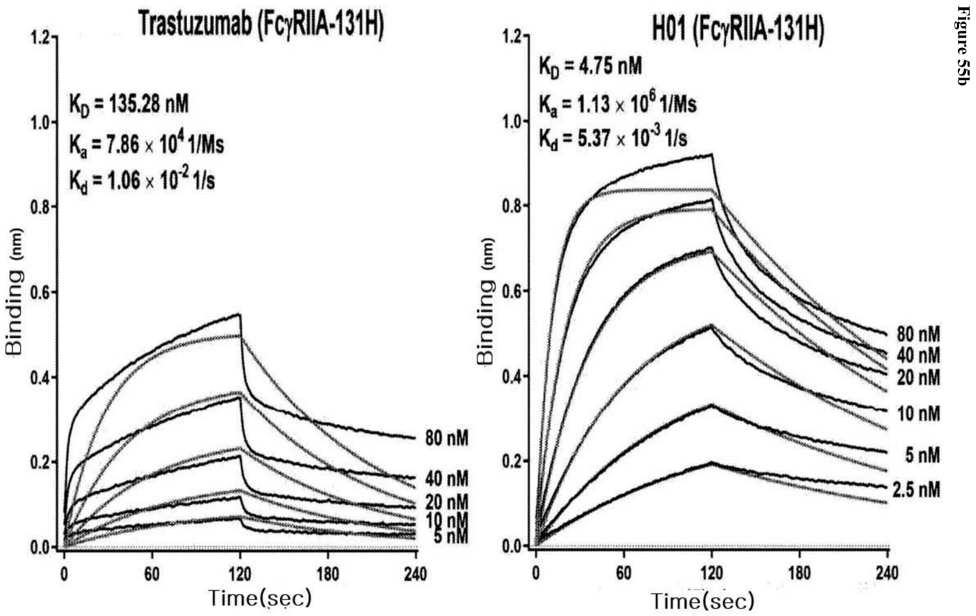

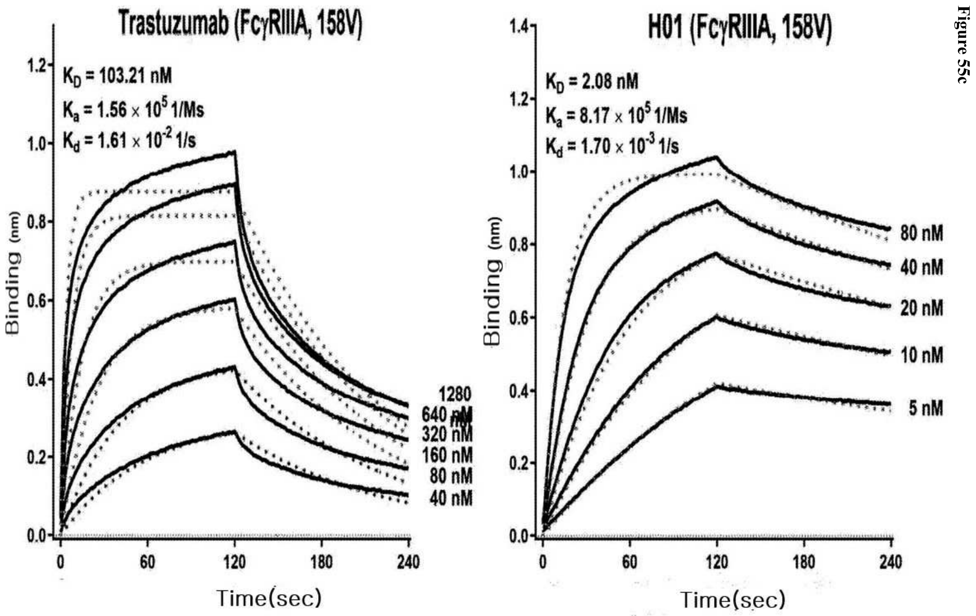

As shown in Figures 55a to 55d and Table 88, H01 showed higher binding affinities to each of FcγRI (CD64), FcγRIIA (CD32A, 131H), and FcγRIIIA (CD16A,158V) compared to trastuzumab.

[Table 88]

| Fcγ receptor | Antibodies | Relative affinity | $K_D$ (nM) | $K_a$ (1/Ms) | $K_d$ (1/s) |
|---|---|---|---|---|---|
| FcγRI | Trastuzumab | 1 | 2.3 | 3.33E+05 | 7.80E-04 |
| | H01 | 11.4 | 0.2 | 3.77E+05 | 7.77E-05 |
| FcγRIIA (131H) | Trastuzumab | 1 | 135.3 | 7.86E+04 | 1.06E-02 |
| | H01 | 28.5 | 4.8 | 1.13E+06 | 5.37E-03 |
| FcγRIIIA (158V) | Trastuzumab | 1 | 103.2 | 1.56E+05 | 1.61E-02 |
| | H01 | 49.1 | 2.1 | 8.17E+05 | 1.70E-03 |

**Example 26. Analysis of binding affinity of novel antibody format with two Fc domains to immune cells**

A novel antibody format with two Fc domains may have improved binding affinities to human immune cells expressing the Fcγ receptors due to an improved Fcγ receptor avidity.

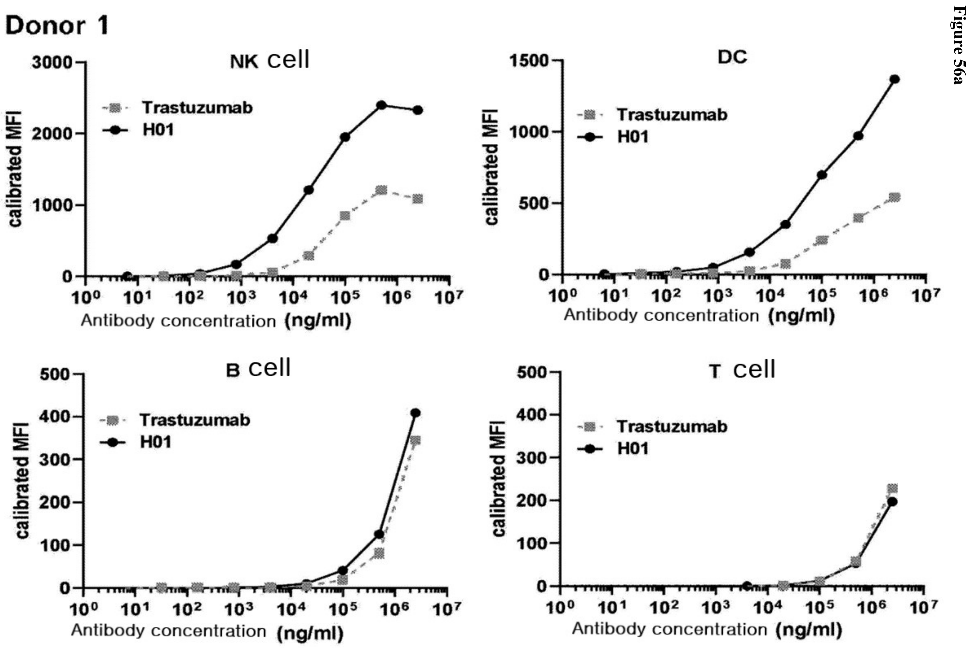

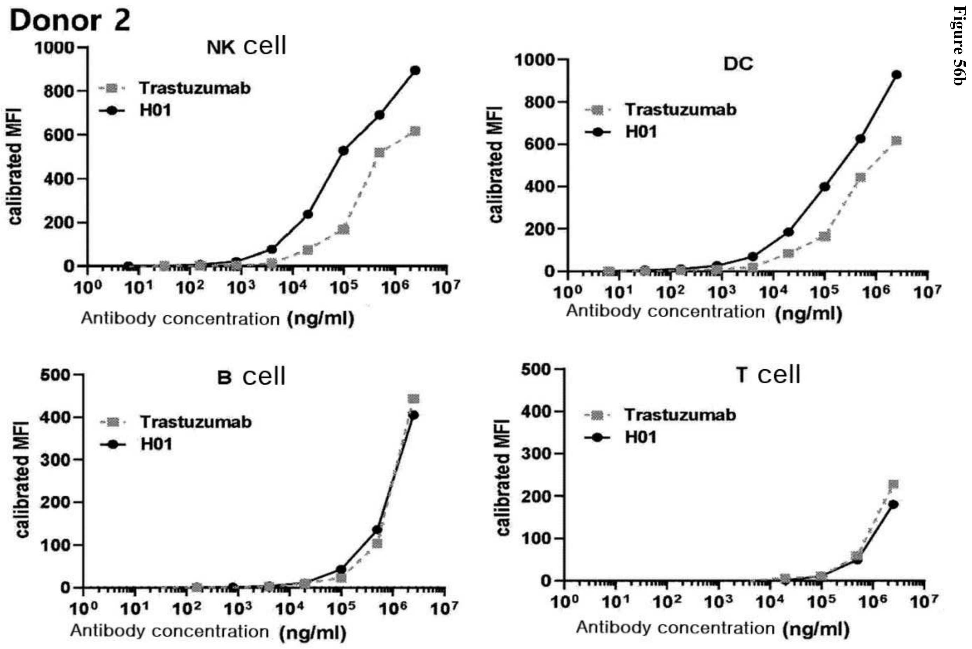

In order to compare the binding affinity of human trastuzumab and H01 to human immune cells, each antibody was labeled with Vivotag 645 (Perkin Elmer), and the protein-to-dye ratio was measured. The peripheral blood mononuclear cells (PBMC) ($2 \times 10^5$ cells/well) isolated from the blood of Donor 1 (Figure 56a) and the blood of Donor 2 (Figure 56b) were treated with each of the above antibodies at different concentrations and then reacted at 37 °C for 45 minutes. Thereafter, the antibody-bound cells were washed, fixed with 2% paraformaldehyde, permeabilized, and then the binding affinities of each of the above antibodies to immune cells were determined using a flow cytometer. The binding affinities to T cells that do not express Fcγ receptors was used as a negative control.

As shown in Figures 56a and 56b, the analysis showed that H01 binds more to natural killer (NK) cells and dendritic (DC) cells than human trastuzumab.

**Example 27. Analysis of antibody-dependent cytotoxicity of novel antibody format with two Fc domains**

The ADCC activity of trastuzumab and H01 was compared in SNU-5 gastric cancer cell line (HER2 1+; Low) and ZR-75-1 breast cancer cell line (HER2 1+; Low).

Specifically, each of the above cancer cell lines was inoculated into a 96-well plate at a concentration of $1 \times 10^4$ cells/well and then treated with the antibody at each concentration. Thereafter, peripheral blood mononuclear cells (PBMC) isolated on the same day were used as effector cells, and treated at $1.5 \times 10^5$ cells/well to make the number of PBMC 25 times more than the number of the target cells (the above cancer cell line) (E:T ratio = 15:1) (). The cells were incubated for 18 hours in a humid incubator under 37 °C and 5% (v/v) $CO_2$ conditions. The cytotoxicity was measured using a cytotoxicity detection kit (LDH) and calculated using Equation 2 above.

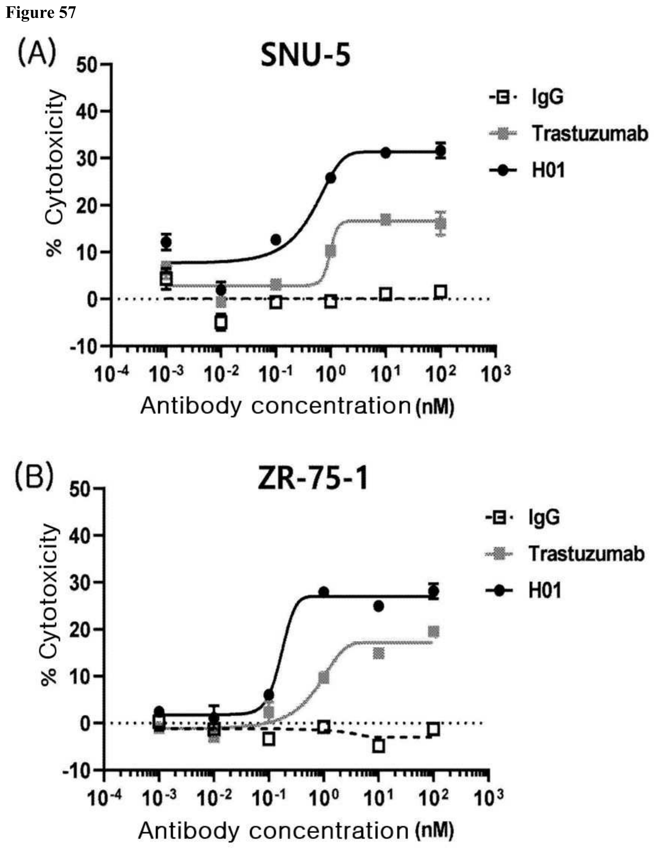

As shown in Figure 57, the cytotoxicity analysis in the SNU-5 gastric cancer cell line (HER2 1+; Low) and the ZR-75-1 breast cancer cell line (HER2 1+; Low) showed H01 has excellent cytotoxicity compared to that of trastuzumab.

**Example 28. Design, preparation and analysis of novel antibody fusion protein with two Fc domains**

An additional specific protein can be fused to the N-terminus of the hinge of the novel antibody format of Example 24 above to possess a new therapeutic function.

Polypeptide 1 of the antibody consists of a heavy chain variable region (X), a heavy chain CH1 region (A), a linker (L1), and a hinge-CH2 region-CH3 with Knob mutation (C); Polypeptide 2 consists of a light chain variable region (Y), a light chain CL region (B), a linker (L2), and a hinge-CH2 region-CH3 with Knob mutation (D); Polypeptide 3 consists of a specific additional protein (Z), a linker (L3), and a hinge-CH2 region-CH3 with Hole mutation (E); and Polypeptide 4 consists of a specific additional protein (Z), a linker (L4), and a hinge-CH2 region-CH3 with Hole mutation (F). The four polypeptides are assembled into an engineered antigen binding protein having two Fc domains and one Fab in which two Z proteins are fused. The Z protein includes a receptor, a soluble protein, a cytokine, a single-chain Fv fragment (single-chain fragment variable; scFv), an antibody mimetics, a single domain antibody (sdAb), a therapeutic peptide, a peptide vaccine, and the like, which can be fused to the novel antibody format (Figure 58).

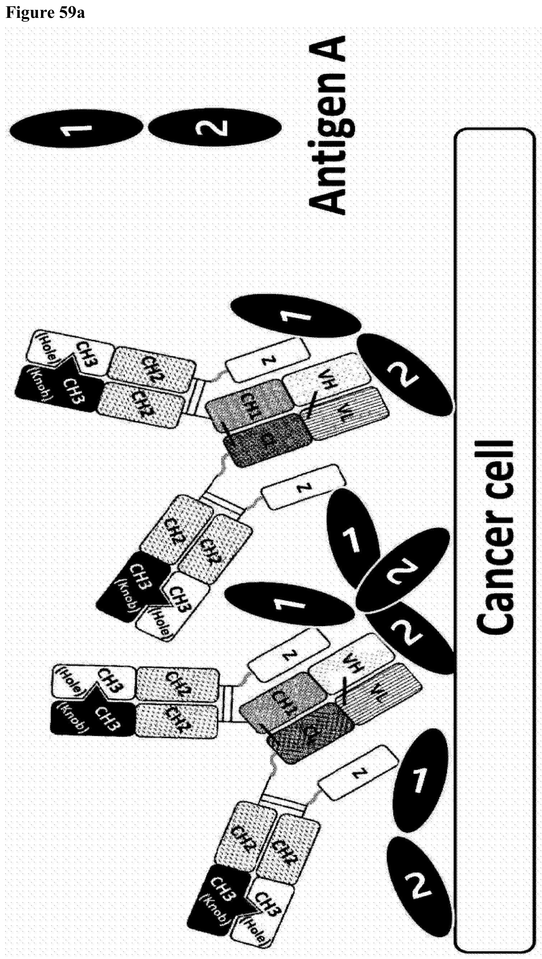

For example, trastuzumab and pertuzumab bind to different domains of the HER2 antigen, and therefore a novel antibody format consisting of two Fc domains, a trastuzumab Fab region, and a pertuzumab scFv (Z) can function like a biparatopic antibody that binds to two different sites on one antigen (Figure 59a).

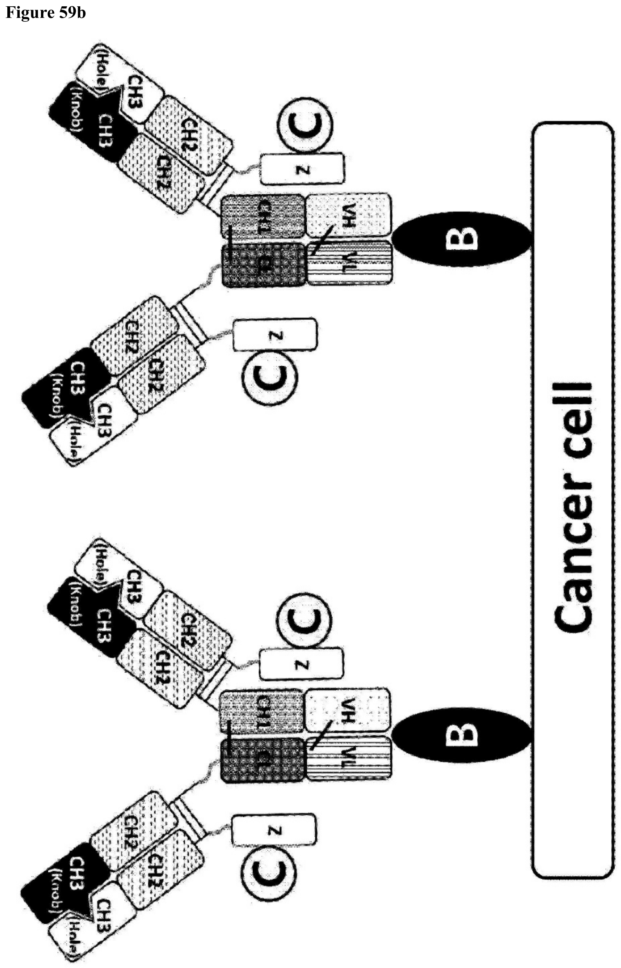

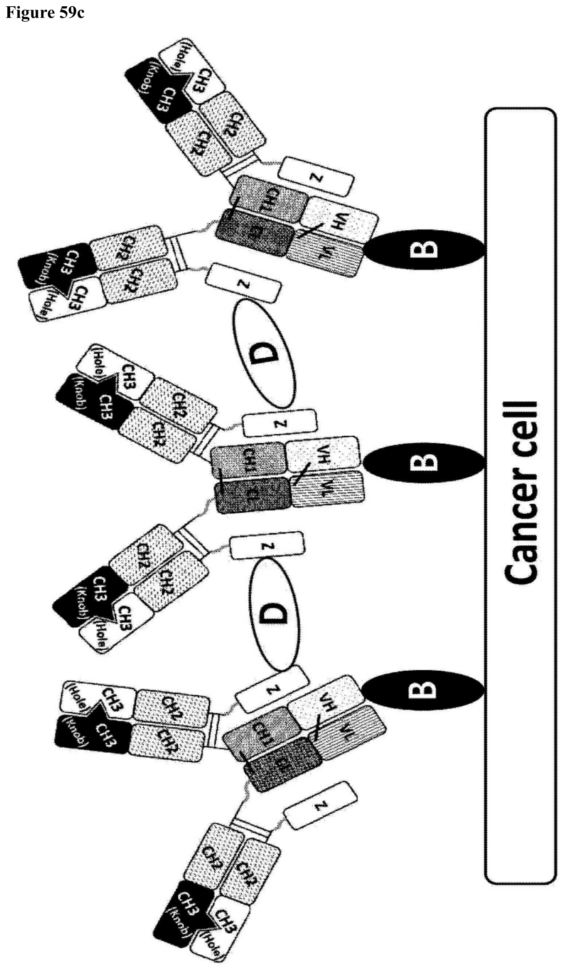

For example, a novel antibody format consisting of two Fc domains and an anti-antigen B Fab region and a receptor (Z) can target antigen B on the cell surface and simultaneously neutralize soluble antigen C or D (Figures 59b and 59c).

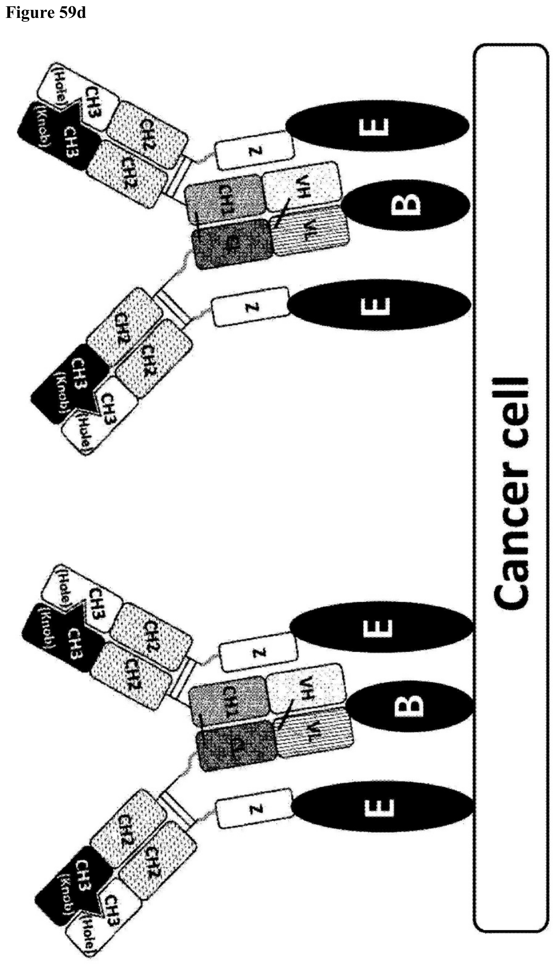

For example, a novel antibody format consisting of two Fc domains and an anti-antigen B Fab region and an anti-antigen E protein (Z) can simultaneously bind to antigen B and antigen E on the cell surface to exert a therapeutic effect (Figure 59d).

H01 according to the present invention is a novel antibody format that consists of two Fc domains and one trastuzumab Fab region and does not comprise a Z region (Figure 54 and Table 89).

H04, H05, H06, H07, and H08 were prepared by fusing each of VEGFR1 domain 2-VEGFR2 domain 3, GITR, IL-1R antagonist, IL2, an antibody mimetics (E01 DARPin), and a single domain mAb (VHH domain 9G8) to the H01 antibody as a template (Tables 89 to 93).

Specifically, H01, H04, H05, H06, H07, H08, and H09 were prepared by co-transfecting polynucleotides encoding each of the four polypeptides into the EXPICHO-S™ (Gibco, A29127) cell line. The transfected cells were incubated for 12 days, and then the culture solution was filtered and purified using an AKTA pure 25 or AKTA avant 25 protein isolation purification system equipped with a CaptureSelect™ CH1-XL Pre-packed Column purification column. The purified product was buffer exchanged and concentrated using an Amicon Ultra-15 Centrifugal Filter Unit, and then quantified using a NanoDrop One trace spectrophotometer. The final product purified as above was identified through SDS-PAGE in the same manner as in Example 1 above.

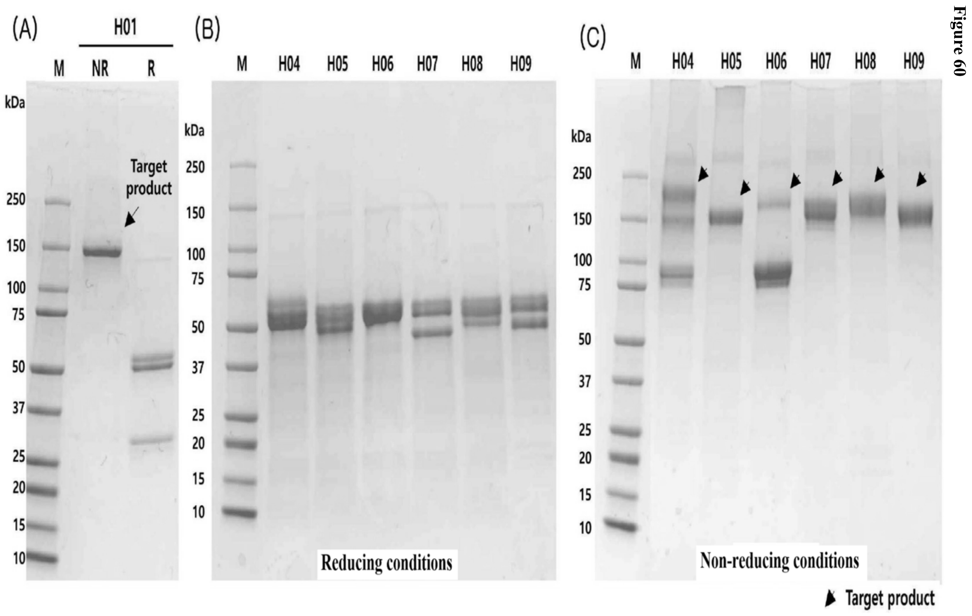

As shown in Figure 60, four polypeptides of appropriate sizes were identified under reducing conditions in all H01, H04, H05, H06, H07, H08, and H09 (A in Figure 60 and B in Figure 60). The analysis showed that a significant number of mispaired impurities of H04 were identified under non-reducing conditions, but H01, H05, H06, H07, H08, and H09 were assembled well into the intact form (C in Figure 60).

Table 89 below shows the information on compositions of the novel antibody format of the present invention.

[Table 89]

| Name | Region | Composition |
|------|--------|-------------|
| H01 | Fab region | Anti-HER2 Fab (Fab of Trastuzumab) |
| | Z protein | None |
| H04 | Fab region | Anti-HER2 Fab (Fab of Trastuzumab) |
| | Z protein | Receptor (VEGFR1 Domain 2-VEGFR2 Domain 3) |
| H05 | Fab region | Anti-HER2 Fab (Fab of Trastuzumab) |
| | Z protein | Receptor (GITR) |
| H06 | Fab region | Anti-HER2 Fab (Fab of Trastuzumab) |
| | Z protein | Soluble protein (IL-1R antagonist) |
| H07 | Fab region | Anti-HER2 Fab (Fab of Trastuzumab) |
| | Z protein | Cytokine (IL2) |
| H08 | Fab region | Anti-HER2 Fab (Fab of Trastuzumab) |
| | Z protein | Antibody mimetics (E01 DARPin) |
| H09 | Fab region | Anti-HER2 Fab (Fab of Trastuzumab) |
| | Z protein | Single domain mAb (VHH domain 9G8) |

Table 90 below shows the polypeptide sequence of each region constituting the novel antibody format.

[Table 90]

| Name | Polypeptide | SEQ ID NO | SEQ ID NO |
|---|---|---|---|
| H01 | Fab region (VH-CH1) | EVQLVESGGGLVQPGGSLRLSCAASGFNIKDTYIHWVRQAPGKCLEWVAR IYPTNGYTRYADSVKGRFTISADTSKNTAYLQMNSLRAEDTAVYYCSRWG GDGFYAMDYWGQGTLVTVSSASTKGPSVFPLAPSSKSTSGGTAALGCLVK DYFPEPVTVSWNSGALTSGVHTFPAVLQSSGLYSLSSVVTVPSSSLGTQT YICNVNHKPSNTKVDKKV | 834 |
| | Fab region (VL-CL) | DIQMTQSPSSLSASVGDRVTITCRASQDVNTAVAWYQQKPGKAPKLLIYS ASFLYSGVPSRFSGSRSGTDFTLTISSLQPEDFATYYCQQHYTTPPTFGC GTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGEC | 835 |
| | L1 linker | EPKSC | 836 |
| | L2 linker | GGGGSGGGGSGGGGSEPKSS | 837 |
| | Hinge-Fc (Knob) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVYTLPPCREEMTKNQVSLWCLVK GFYPSDIAVEWESNGQPENNYKTTPPVLDSDGSFFLYSKLTVDKSRWQQG NVFSCSVMHEALHNHYTQKSLSLSPGK | 839 |
| | Hinge-Fc (Hole) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVCTLPPSREEMTKNQVSLSCAVK GFYPSDIAVEWESNGQPENNYKTTPPVLDSDGSFFLVSKLTVDKSRWQQG NVFSCSVMHEALHNHYTQKSLSLSPGK | 7 |
| H04 | Fab region (VH-CH1) | EVQLVESGGGLVQPGGSLRLSCAASGFNIKDTYIHWVRQAPGKCLEWVAR IYPTNGYTRYADSVKGRFTISADTSKNTAYLQMNSLRAEDTAVYYCSRWG GDGFYAMDYWGQGTLVTVSSASTKGPSVFPLAPSSKSTSGGTAALGCLVK DYFPEPVTVSWNSGALTSGVHTFPAVLQSSGLYSLSSVVTVPSSSLGTQT YICNVNHKPSNTKVDKKV | 834 |

| | Fab region (VL-CL) | DIQMTQSPSSLSASVGDRVTITCRASQDVNTAVAWYQQKPGKAPKLLIYS ASFLYSGVPSRFSGSRSGTDFTLTISSLQPEDFATYYCQQIIYTTPPTFGC GTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGEC | 835 |
|---|---|---|---|
| | L1 linker | EPKSC | 836 |
| | L2 linker | GGGGSGGGGSGGGGSEPKSS | 837 |
| | L3 linker | GGGGS | 838 |
| | L4 linker | GGGGS | 838 |
| | Hinge-Fc (Knob) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVYTLPPCREEMTKNQVSLWCLVK GFYPSDIAVEWESNGQPENNYKTTPPVLDSDGSFFLYSKLTVDKSRWQQG NVFSCSVMHEALHNHYTQKSLSLSPGK | 839 |
| | Hinge-Fc (Hole) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVCTLPPSREEMTKNQVSLSCAVK GFYPSDIAVEWESNGQPENNYKTTPPVLDSDGSFFLYSKLTVDKSRWQQG NVFSCSVMHEALHNHYTQKSLSLSPGK | 7 |
| | Z protein(Recepto r; VEGFR1 Domain 2-VEGFR2 Domain 3) | SDTGRPFVEMYSEIPEIIHMTEGRELVIPCRVTSPNITVTLKKFPLDTLI PDGKRIIWDSRKGFIISNATYKEIGLLTCEATVNGHLYKTNYLTHRQTNT IIDVVLSPSHGIELSVGEKLVLKCTARTELNVGIDFNWEYPSSKHQHKKL VNRDLKTQSGSEMKKFLSTLTIDGVTRSDQGLYTCAASSGLMTKKNSTFV RVHEK | 840 |
| 1105 | Fab region (VH-CH1) | EVQLVESGGGLVQPGGSLRLSCAASGFNIKDTYIHWVRQAPGKCLEWVAR IYPTNGYTRYADSVKGRFTISADTSKNTAYLQMNSLRAEDTAVYYCSRWG GDGFYAMDYWGQGTLVTVSSASTKGPSVFPLAPSSKSTSGGTAALGCLVK DYFPEPVTVSWNSGALTSGVHTFPAVLQSSGLYSLSSVVTVPSSSLGTQT YICNVNHKPSNTKVDKKV | 834 |

| | | | |
|---|---|---|---|
| | Fab region (VL-CL) | DIQMTQSPSSLSASVGDRVTITCRASQDVNTAVAWYQQKPGKAPKLLIYS ASFLYSGVPSRFSGSRSGTDFTLTISSLQPEDFATYYCQQHYTTPPTFGC GTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGEC | 835 |
| | L1 linker | EPKSC | 836 |
| | L2 linker | GGGGSGGGGSGGGGSEPKSS | 837 |
| | L3 linker | GGGGS | 838 |
| | L4 linker | GGGGS | 838 |
| | Hinge-Fc (Knob) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVYTLPPCREEMTKNQVSLWCLVK GFYPSDIAVEWESNGQPENNYKTTPPVLDSDGSFFLYSKLTVDKSRWQQG NVFSCSVMHEALHNHYTQKSLSLSPGK | 839 |
| | Hinge-Fc (Hole) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVCTLPPSREEMTKNQVSLSCAVK GFYPSDIAVEWESNGQPENNYKTTPPVLDSDGSFFLVSKLTVDKSRWQQG NVFSCSVMHEALHNHYTQKSLSLSPGK | 7 |
| | Z protein(Recepto r; GITR) | QRPTGGPGCGPGRLLLGTGTDARCCRVHTTRCCRDYPGEECCSEWDCMCV QPEFHCGDPCCTTCRIHHPCPPGQGVQSQGKFSFGFQCIDCASGTFSGGHE GHCKPWTDCTQFGFLTVFPGNKTHNAVCVPGSPPAEP | 841 |
| HO6 | Fab region (VH-CH1) | EVQLVESGGGLVQPGGSLRLSCAASGFNIKDTYIHWVRQAPGKCLEWVAR IYPTNGYTRYADSVKGRFTISADTSKNTAYLQMNSLRAEDTAVYYCSRWG GDGFYAMDYWGQGTLVTVSSASTKGPSVFPLAPSSKSTSGGTAALGCLVK DYFPEPVTVSWNSGALTSGVHTFPAVLQSSGLYSLSSVVTVPSSSLGTQT YICNVNHKPSNTKVDKKV | 834 |
| | Fab region (VL-CL) | DIQMTQSPSSLSASVGDRVTITCRASQDVNTAVAWYQQKPGKAPKLLIYS ASFLYSGVPSRFSGSRSGTDFTLTISSLQPEDFATYYCQQHYTTPPTFGC | 835 |

| | | | |
|---|---|---|---|
| | | GTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGEC | |
| | L1 linker | EPKSC | 836 |
| | L2 linker | GGGGSGGGGSGGGGSEPKSS | 837 |
| | L3 linker | GGGGS | 838 |
| | L4 linker | GGGGS | 838 |
| | Hinge-Fc (Knob) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVYTLPPCREEMTKNQVSLWCLVK GFYPSDIAVEWESNGQPENNYKTTPPVLDSDGSFFLYSKLTVDKSRWQQG NVFSCSVMHEALHNHYTQKSLSLSPGK | 839 |
| | Hinge-Fc (Hole) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVCTLPPSREEMTKNQVSLSCAVK GFYPSDIAVEWESNGQPENNYKTTPPVLDSDGSFFLVSKLTVDKSRWQQG NVFSCSVMHEALHNHYTQKSLSLSPGK | 7 |
| | Z protein(Soluble protein: IL-1R antagonist) | RPSGRKSSKMQAFRIWDVNQKTFYLRNNQLVAGYLQGPNVNLEEKIDVVP IEPHALFLGIHGGKMCLSCVKSGDETRLQLEAVNITDLSENRKQDKRFAF IRSDSGPTTSFESAACPGWFLCTAMEADQPVSLTNMPDEGVMVTKFYFQE DE | 842 |
| H07 | Fab region (VH-CH1) | EVQLVESGGGLVQPGGSLRLSCAASGFNIKDTYIHWVRQAPGKCLEWVAR IYPTNGYTRYADSVKGRFTISADTSKNTAYLQMNSLRAEDTAVYYCSRWG GDGFYAMDYWGQGTLVTVSSASTKGPSVFPLAPSSKSTSGGTAALGCLVK DYFPEPVTVSWNSGALTSGVHTFPAVLQSSGLYSLSSVVTVPSSSLGTQT YICNVNHKPSNTKVDKKV | 834 |
| | Fab region (VL-CL) | DIQMTQSPSSLSASVGDRVTITCRASQDVNTAVAWYQQKPGKAPKLLIYS ASFLYSGVPSRFSGSRSGTDFTLTISSLQPEDFATYYCQQHYTTPPTFGC GTKVEIKRTVAAPSVFIFPPSDEQLKSGTASVVCLLNNFYPREAKVQWKV | 835 |

| | | | |
|---|---|---|---|
| | | DNALQSGNSQESVTEQDSKDSTYSLSSTLTLSKADYEKHKVYACEVTHQG LSSPVTKSFNRGEC | |
| | L1 linker | EPKSC | 836 |
| | L2 linker | GGGGSGGGGSGGGGSEPKSS | 837 |
| | L3 linker | GGGGS | 838 |
| | L4 linker | GGGGS | 838 |
| | Hinge-Fc (Knob) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVYTLPPCREEMTKNQVSLWCLVK GFYPSDIAVEWESNGQPENNYKTTPPVLDSDGSFFLYSKLTVDKSRWQQG NVFSCSVMHEALHNHYTQKSLSLSPGK | 839 |
| | Hinge-Fc (Hole) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVCTLPPSREEMTKNQVSLSCAVK GFYPSDIAVEWESNGQPENNYKTTPPVLDSDGSFFLVSKLTVDKSRWQQG NVFSCSVMHEALHNHYTQKSLSLSPGK | 7 |
| | Z protein(Cytokin e: IL2) | APTSSSTKKTQLQLEHLLLDLQMILNGINNYKNPKLTRMLTFKFYMPKKA TELKHLQCLEEELKPLEEVLNLAQSKNFHLRPRDLISNINVIVLELKGSE TTFMCEYADETATIVEFLNRWITFCQSIISTLT | 843 |
| HO8 | Fab region (VH-CH1) | EVQLVESGGGLVQPGGSLRLSCAASGFNIKDTYIHWVRQAPGKCLEWVAR IYPTNGYTRYADSVKGRFTISADTSKNTAYLQMNSLRAEDTAVYYCSRWG GDGFYAMDYWGQGTLVTVSSASTKGPSVFPLAPSSKSTSGGTAALGCLVK DYFPEPVTVSWNSGALTSGVHTFPAVLQSSGLYSLSSVVTVPSSSLGTQT YICNVNHKPSNTKVDKKV | 834 |
| | Fab region (VL-CL) | DIQMTQSPSSLSASVGDRVTITCRASQDVNTAVAWYQQKPGKAPKLLIYS ASFLYSGVPSRFSGSRSGTDFTLTISSLQPEDFATYYCQQHYTTPPTFGC GTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGEC | 835 |

| | | | |
|---|---|---|---|
| | L1 linker | EPKSC | 836 |
| | L2 linker | GGGGSGGGGSGGGGSEPKSS | 837 |
| | L3 linker | GGGGS | 838 |
| | L4 linker | GGGGS | 838 |
| | Hinge-Fc (Knob) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVYTLPPCREEMTKNQVSLWCLVK GFYPSDIAVEWESNGQPENNYKTTPPVLDSDGSFFLYSKLTVDKSRWQQG NVFSCSVMHEALHNHYTQKSLSLSPGK | 839 |
| | Hinge-Fc (Hole) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVCTLPPSREEMTKNQVSLSCAVK GFYPSDIAVEWESNGQPENNYKTTPPVLDSDGSFFLVSKLTVDKSRWQQG NVFSCSVMHEALHNHYTQKSLSLSPGK | 7 |
| | Z protein(Antibod y mimetics: E01 DARPin) | DLGKKLLEAARAGQDDEVRILMANGADVNADDTWGWTPLHLAAYQGHLEI VEVLLKNGADVNAYDYIGWTPLHLAADGHLEIVEVLLKNGADVNASDYIG DTPLHLAAHNGHLEIVEVLLKHGADVNAQDKFGKTAFDISIDNGNEDLAE ILQ | 844 |
| H09 | Fab region (VH-CH1) | EVQLVESGGGLVQPGGSLRLSCAASGFNIKDTYIHWVRQAPGKCLEWVAR IYPTNGYTRYADSVKGRFTISADTSKNTAYLQMNSLRAEDTAVYYCSRWG GDGFYAMDYWGQGTLVTVSSASTKGPSVFPLAPSSKSTSGGTAALGCLVK DYFPEPVTVSWNSGALTSGVHTFPAVLQSSGLYSLSSVVTVPSSSLGTQT YICNVNHKPSNTKVDKKV | 834 |
| | Fab region (VL-CL) | DIQMTQSPSSLSASVGDRVTITCRASQDVNTAVAWYQQKPGKAPKLLIYS ASFLYSGVPSRFSGSRSGTDFTLTISSLQPEDFATYYCQQHYTTPPTFGC GTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGEC | 835 |
| | L1 linker | EPKSC | 836 |

| L2 linker | GGGGSGGGGSGGGGSEPKSS | 837 |
|---|---|---|
| L3 linker | GGGGS | 838 |
| L4 linker | GGGGS | 838 |
| Hinge-Fc (Knob) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVYTLPPCREEMTKNQVSLWCLVK GFYPSDIAVEWESNGQPENNYKTTPPVLDSDGSFFLYSKLTVDKSRWQQG NVFSCSVMHEALHNHYTQKSLSLSPGK | 839 |
| Hinge-Fc (Hole) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVCTLPPSREEMTKNQVSLSCAVK GFYPSDIAVEWESNGQPENNYKTTPPVLDSDGSFFLVSKLTVDKSRWQQG NVFSCSVMHEALHNHYTQKSLSLSPGK | 7 |
| Z protein(Single domain mAb; VHH domain 9G8) | EVQLVESGGGLVQAGGSLRLSCAASGRTFSSYAMGWFRQAPGKEREFVVA INWSSGSTYYADSVKGRFTISRDNAKNTMYLQMNSLKPEDTAVYYCAAGY QINSGNYNFKDYEYDYWGQGTQVTVSSALE | 845 |

Table 91 below shows the polynucleotide sequences encoding the polypeptides of each region constituting the novel antibody format.

[Table 91]

| Name | Polypeptide | Sequence | SEC ID NO |
|------|-------------|----------|-----------|
| | Fab region (VH-CH1) | GAAGTTCAACTTGTGGAGTCTGGAGGCGGTTTGGTACAACCTGGAGGTTCA CTTCGTTTGTCATGTGCTGCCTCTGGGTTTAATATCAAGGATACTTATATT CATTGGGTTCGCCAGGCACCCGGAAAATGCCTGGAATGGGTGGCCCGTATA TATCCCACTAACCGGTTACACTCGATATGCTGACTCTGTAAAGGGTCGATTT ACTATATCCGCTGATACTAGCAAAAACACTGCCTACCTCCAGATGAACAGT CTGCGCGCCGAGGATACAGCAGTTTATTATTGTTCCCGATGGGGAGGTGAC GGGTTCTATGCCATCGACTACTGGGGTCAAGGGACACTGGTAACCGTTTCT TCTGCTAGCACCAAAGGACCTAGTGTTTTTCCTCTTGCCCCTTCCTCAAAG TCTACCTCTGGGGCCGACAGCCGCTCTGGGCTGCCTGGTCAACGGATTATTTC CCAGAGCCTGTCACTGTCAGTTGGAACTCTGGAGCCTTGACTTCTGGTGTT CATACATTTCCTGCTGTCCTTCAAAGCAGCGGCTTGTACTCATTGTCTTCT GTTGTGACAGTACCCTCAAGCAGCCTCGGCACTCAGACATACATCTGCAAT GTCAACCACAAACCCTCAAATACAAAGGTAGATAAAAAAGTC | 846 |
| HO1 | Fab region (VL-CL) | GACATTCAGATGACCCAATCCCCCTCTAGCCTTTCAGCCTCCGTAGGGGAC AGAGTTACAATAACCTGTAGGGCTAGTCAGGACGTTAATACAGCAGTCGCA TGGTACCAGCAGAAGCCCGGTAAGGCTCCTAAGCTTTTGATCTACAGTGCT TCTTTCCTGTATTCTGGTGTACCTAGCCGATTCTCAGGCTCTCGGAGTGGC ACAGACTTCACTCTGACAATTTCAAGTCTCCAGCCAGAAGATTTTGCAACC TACTACTGCCAACAACACTACACTACTCCTCCAACCTTCGGATGTGGCACA AAAGTAGAGATTAAGCGTACGGTGGCAGCTCCCAGCGTTTTTATCTTTCCC CCATCCGACGAGCAGCTCAAGAGTGGCACTGCCTCTGTAGTTTGTTTGCTG AATAACTTCTATCCACGTGAAGCAAAAGTACAGTGGAAGGTCGATAATGCC CTTCAGAGCGGTAACAGCCAAGAAAGTGTTACCGAGCAAGATTCCAAAGAT TCCACTTACAGTCTGTCCAGCACATTGACACTGAGTAAGGCTGATTACGAA AAACACAAGGTGTACGCATGCGAGGTGACACACCAAGGTCTTTCATCTCCT GTAACTAAGAGCTTTAACCGGGGAGAATGT | 847 |
| | L1 linker | GAACCAAAGTCTTGT | 848 |

| | | | |
|---|---|---|---|
| | L2 linker | GGTGGTGGGGGCAGCGGGGGCGGAGGTAGTGGAGGCGGCGGTAGTGAACCA AAGAGTAGT | 849 |
| | Hinge-Fc (Knob) | GACAAAACTCACACGTGCCCACCGTGCCCAGCACCTGAACTCCTGGGGGGA CCGTCAGTCTTCCTCTTCCCCCCAAAACCCAAGGACACCCTCATGATCTCC CGGACCCCTGAGGTCACATGCGTGGTGGTGGACGTGAGCCACGAAGACCCT GAGGTCAAGTTCAACTGGTACGTGGACGGCGTGGAGGTGCATAATGCCAAG ACAAAGCCGCGGGAGGAGCAGTACAACAGCACGTACCGTGTGGTCAGCGTC CTCACCGTCCTGCACCAGGACTGGCTGAATGGCAAGGAGTACAAGTGCAAG GTCTCCAACAAAGCCCTCCCAGCCCCCATCGAGAAAACCATCTCCAAAGCC AAAGGGCAGCCCCGAGAACCACAGGTGTACACCCTGCCCCCCATGCCGGGAG GAGATGACCAAGAACCAGGTCAGCCTGTGGTGCCTGGTCAAAGGCTTCTAT CCCAGCGACATCGCCGTGGAGTGGGAGAGCAATGGGCAGCCGGAGAACAAC TACAAGACCACGCCTCCCGTGCTGGACTCCGACGGCTCCTTCTTCCTCTAC AGCAAGCTCACCGTGGACAAGAGCAGGTGGCAGCAGGGGAACGTCTTCTCA TGCTCCGTGATGCATGAGGCTCTGCACAACCACTACACGCAGAAGAGCCTC TCCCTGTCTCCGGGTAAA | 851 |
| | Hinge-Fc (Hole) | GACAAAACTCACACGTGCCCACCGTGCCCAGCACCTGAACTCCTGGGGGGA CCGTCAGTCTTCCTCTTCCCCCCAAAACCCAAGGACACCCTCATGATCTCC CGGACCCCTGAGGTCACATGCGTGGTGGTGGACGTGAGCCACGAAGACCCT GAGGTCAAGTTCAACTGGTACGTGGACGGCGTGGAGGTGCATAATGCCAAG ACAAAGCCGCGGGAGGAGCAGTACAACAGCACGTACCGTGTGGTCAGCGTC CTCACCGTCCTGCACCAGGACTGGCTGAATGGCAAGGAGTACAAGTGCAAG GTCTCCAACAAAGCCCTCCCAGCCCCCATCGAGAAAACCATCTCCAAAGCC AAAGGGCAGCCCCGAGAACCACAGGTGTGCACCCTGCCCCCCATCCCGGGAG GAGATGACCAAGAACCAGGTCAGCCTGAGCTGCGCGGTCAAAGGCTTCTAT CCCAGCGACATCGCCGTGGAGTGGGAGAGCAATGGGCAGCCGGAGAACAAC TACAAGACCACGCCTCCCGTGCTGGACTCCGACGGCTCCTTCTTCCTCGTG AGCAAGCTCACCGTGGACAAGAGCAGGTGGCAGCAGGGGAACGTCTTCTCA TGCTCCGTGATGCATGAGGCTCTGCACAACCACTACACGCAGAAGAGCCTC | 458 |

| | | TCCCTGTCTCCGGGTAAA | |
|---|---|---|---|
| H04 | Fab region (VH-CH1) | GAAGTTCAACTTGTGGAGTCTGGAGGCGGTTTGGTACAACCTGGAGGTTCA CTTCGTTTGTCATGTGCTGCCTCTGGGTTTAATATCAAGGATACTTATATT CATTGGGTTCGCCAGGCACCCGGAAAATGCCTCGAATGGGTGGCCCGTATA TATCCCACTAACGGTTACACTCGATATGCTGACTCTGTAAAGGGTCGATTT ACTATATCCGCTGATACTAGCAAAAACACTGCCTACCTCCAGATGAACAGT CTGCGCGCCGAGGATACAGCAGTTTATTATTGTTCCCGATGGGGACGGTGAC GGGTTCTATGCCATGGACTACTGGGGTCAAGGGACACTGGTAACCGTTTCT TCTGCTAGCACCAAAGGACCTAGTGTTTTTCCTCTTGCCCCTTCCTCAAAG TCTACCTCTGGGGGGACAGCCGCTCTGGGCTGCCTGGTCAAGGATTATTTC CCAGAGCCTGTCACTGTCAGTTGGAACTCTGGAGCCTTGACTTCTGGTGTT CATACATTTCCTGCTGTCCTTCAAAGCAGCGGCTTGTACTCATTGTCTTCT GTTGTGACAGTACCCTCAAGCAGCCTCGGCACTCAGACATACATCTGCAAT GTCAACCACAAACCCTCAAATACAAAGGTAGATAAAAAAGTC | 846 |
| | Fab region (VL-CL) | GACATTCAGATGACCCAATCCCCCTCTAGCCTTTCAGCCTCCGTAGGGGAC AGAGTTACAATAACCTGTAGGGCTAGTCAGGACGTTAATACAGCAGTCGCA TGGTACCAGCAGAAGCCCGGTAAGGCTCCTAAGCTTTTTGATCTACAGTGCT TCTTTCCTGTATTCTGGTGTACCTAGCCGATTCTCAGGCTCTCGGAGTGGC ACAGACTTCACTCTGACAATTTCAAGTCTCCAGCCAGAAGATTTTGCAACC TACTACTGCCAACAACACTACACTACTCCTCCAACCTTCGGATGTGGCACA AAAGTAGAGATTAAGCGTACGGTGGCAGCTCCCAGCCGTTTTTATCTTTCCC CCATCCGACGAGCAGCTCAAGAGTGGCACTGCCTCTGTAGTTTGTTTGCTG AATAACTTCTATCCACGTGAAGCAAAAGTACAGTGGAAGGTCGATAATGCC CTTCAGAGCGGTAACAGCCAAGAAAGTGTTACCGAGCAAGATTCCAAAGAT TCCACTTACAGTCTGTCCAGCACATTGACACTGAGTAAGGCTGATTACGAA AAACACAAGGTGTACGCATGCGAGGTGACACACCAAGGTCTTTCATCTCCT GTAACTAAGAGCTTTAACCGGGGACAATGT | 847 |
| | L1 linker | GAACCAAAGTCTTCT | 848 |
| | L2 linker | GGTGGTGGGGGCAGCCCGGGGCGGAGGTAGTGGAGGCGGCGGTAGTGAACCA | 849 |

| | | AAGAGTAGT | |
|---|---|---|---|
| | L3 linker | GGAGGCGGCGGCTCT | 850 |
| | L4 linker | GGAGGCGGCGGCTCT | 850 |
| | Hinge-Fc (Knob) | GACAAAACTCACACGTGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATGCCGGGAGGAGATGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | 851 |
| | Hinge-Fc (Hole) | GACAAAACTCACACGTGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTGCACCCTGCCCCCATCCCGGGAGGAGATGACCAAGAACCAGGTCAGCCTGAGCTGCGCGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCGTGAGCAAGCTCACCGTGGACAAGAGCAGGTGGCAGCAGGGGAACGTCTTCTCA | 458 |

| | | | |
|---|---|---|---|
| | | TGCTCCGTGATGCATGAGGCTCTGCACAACCACTACACGCAGAAGAGCCTC TCCCTGTCTCCCGCGTAAA | |
| | Z protein(Recept or: VEGFR1 Domain 2-VEGFR2 Domain 3) | AGCGATACAGGGAGACCTTTCGTGGAGATGTACTCCGAAATACCAGAGATC ATACACATGACCGAGGGTAGAGAGCTCGTTATACCCTGTCGGGTAACCTCC CCAAACATAACAGTGACTCTCAAAAAATTCCCACTCGATACCCTTATACCA GACGGAAAGCGGATAATATGGGACTCACGAAAGGGTTTCATTATTTCTAAT GCAACTTATAAAGAAATCGGCATTGCTTACATGCGAACCCACAGTGAACGGT CATTTGTATAAGACCAATTATCTTACCCACCGTCAAACCAATACAATCATT GACGTGGTCCTGTCCCCCTCTCACGGTATCGAGTTGAGTGTGGAGAAAAG TTGGTACTCAACTGTACCGCCCGTACTGAACTCAACGTAGGGATAGATTTT AACTGGGAATATCCTAGCAGTAAACACCAGCACAAGAAACTTGTTAATCGA GACTTGAAAACCCAATCAGGCTCTGAAATGAAAAAGTTCCTCTCCACTTTG ACCATCGATGGAGTGACACGCTCCGATCAAGCGGTTGTACACTTGTGCCGCC AGCTCCGGTTTGATGACTAAAAAAAAATTCTACCTTTGTACGTGTCCACGAA AAA | 852 |
| 1105 | Fab region (VH-CH1) | GAAGTTCAACTTGTGGAGTCTGGAGGCGGTTTGGTACAACCTGGAGGTTCA CTTCGTTTGTCATGTGCTGCCTCTGGGTTTAATATCAAGGATACTTATATT CATTGGGTTCGCCAGGCACCCGGAAAATGCCTGGAATGGGTGGCCCGTATA TATCCCACTAACGGTTACACTCGATATGCTGACTCTGTAAAGGGTCGATTT ACTATATCCGCTGATACTAGCAAAAACACTGCCTACCTCCAGATGAACAGT CTGCGCGCCGAGGATACAGCAGTTTATTATTGTTCCCGATGGGGAGGTGAC GGGTTCTATGCCATGGACTACTGGGGTCAAGGGACACTGGTAACCGTTTCT TCTGCTAGCACCAAAGGACCTAGTGTTTTTCCTCTTGCCCCCTTCCTCAAAG TCTACCTCTGGCGGGACAGCCCCTCTGGCCTGCCTGGTCAAGGATTATTTC CCAGAGCCTGTCACTGTCAGTTGGAACTCTGGAGCCTTGACTTCTGGTGTT CATACATTTCCTGCTGTCCTTCAAAGCAGCGGCTTGTACTCATTGTCTTCT GTTGTGACAGTACCCTCAAGCAGCCTCGGCACTCAGACATACATCTGCAAT GTCAACCACAAACCCTCAAATACAAAGGTAGATAAAAAGTC | 846 |
| | Fab region | GACATTCAGATGACCCAATCCCCCTCTAGCCTTTCAGCCTCCGTAGGGGAC | 847 |

| | (VL-CL) | AGAGTTACAATAACCTGTAGGGCTAGTCAGGACGTTAATACAGCAGTCGCA TGGTACCAGCAGAAGCCCGGTAAGGCTCCTAAGCTTTTGATCTACAGTGCT TCTTTCCTGTATTCTGGTGTACCTAGCCGATTCTCAGGCTCTCGGAGTGGC ACAGACTTCACTCTGACAATTTCAAGTCTCCAGCCAGAAGATTTTGCAACC TACTACTGCCAACAACACTACACTACTCCTCCAACCTTCGGATGTGGCACA AAAGTAGAGATTAAGCGTACGGTGGCAGCTCCCAGCGTTTTTATCTTTCCC CCATCCGACGAGCAGCTCAAGAGTGGCACTGCCTCTGTAGTTTGTTTGCTG AATAACTTCTATCCACGTGAAGCAAAAGTACAGTGGAAGGTCGATAATGCC CTTCAGAGCGGTAACAGCCAAGAAAGTGTTACCGAGCAAGATTCCAAAGAT TCCACTTACAGTCTGTCCAGCACATTGACACTGAGTAAGGCTGATTACGAA AAACACAAGGTGTACGCATGCGAGGTGACACACCAAGGTCTTTCATCTCCT GTAACTAAGAGCTTTAACCGGGGAGAATGT | |
| L1 linker | GAACCAAAGTCTTGT | 848 |
| L2 linker | GGTGGTGGGGGCAGCGGGGGCGGAGGTAGTGGAGGCGGCGGTAGTGAACCA AAGAGTAGT | 849 |
| L3 linker | GGAGGCGGCGGCTCT | 850 |
| L4 linker | GGAGGCGGCGGCTCT | 850 |
| Hinge-Fc (Knob) | GACAAAACTCACACGTGCCCACCGTGCCCAGCACCTGAACTCCTGGGGGGA CCGTCAGTCTTCCTCTTCCCCCCAAAACCCAAGGACACCCTCATGATCTCC CGGACCCCTGAGGTCACATGCGTGGTGGTGGACGTGAGCCACGAAGACCCT GAGGTCAAGTTCAACTGGTACGTGGACGGCGTGGAGGTGCATAATGCCAAG ACAAAGCCGCGGGAGGAGCAGTACAACAGCACGTACCGTGTGGTCAGCGTC CTCACCGTCCTGCACCAGGACTGGCTGAATGGCAAGGAGTACAAGTGCAAG GTCTCCAACAAAGCCCTCCCAGCCCCCATCGAGAAAACCATCTCCAAAGCC AAAGGGCAGCCCCGAGAACCACAGGTGTACACCCTGCCCCCATGCCGGGAG GAGATGACCAAGAACCAGGTCAGCCTGTGGTGCCTGGTCAAAGGCTTCTAT CCCAGCGACATCGCCGTGGAGTGGGAGAGCAATGGGCAGCCGGAGAACAAC TACAAGACCACGCCTCCCGTGCTGGACTCCGACGGCTCCTTCTTCCTCTAC AGCAAGCTCACCGTGGACAAGAGCAGGTGGCAGCAGGGGAACGTCTTCTCA | 851 |

| | | | |
|---|---|---|---|
| | | TGCTCCGTGATGCATGAGGCTCTGCACAACCACTACACGCAGAAGAGCCTC TCCCTGTCTCCGGGTAAA | |
| | Hinge-Fc (Hole) | GACAAAACTCACACGTGCCCACCGTGCCCAGCACCTGAACTCCTGGGGGGA CCGTCAGTCTTCCTCTTCCCCCCAAAACCCAAGGACACCCTCATGATCTCC CGGACCCCTGAGGTCACATGCCGTGGTGGTGGACGTGAGCCACGAAGACCCT GAGGTCAAGTTCAACTGGTACGTGGACGGCGTGGAGGTGCATAATGCCAAG ACAAAGCCGCGGGAGGAGCAGTACAACAGCACGTACCGTGTGGTCAGCGTC CTCACCGTCCTGCACCAGGACTGGCTGAATGGCAAGGAGTACAAGTGCAAG GTCTCCAACAAAGCCCTCCCAGCCCCCATCGAGAAAACCATCTCCAAAGCC AAAGGGCAGCCCCGAGAACCACAGGTGTGCACCCTGCCCCCCATCCCGGGAG GAGATGACCAAGAACCAGGTCAGCCTGAGCTGCGCGGTCAAAGGCTTCTAT CCCAGCGACATCGCCGTGGAGTGGGAGAGCAATGGGCAGCCGGAGAACAAC TACAAGACCACGCCTCCCGTGCTGGACTCCGACGGCTCCTTCTTCCTCGTG AGCAAGCTCACCGTGGACAAGAGCAGGTGGCAGCAGGGGAACGTCTTCTCA TGCTCCGTGATGCATGAGGCTCTGCACAACCACTACACGCAGAAGAGCCTC TCCCTGTCTCCGGGTAAA | 158 |
| | Z protein(Recept or: GITR) | CAAAGGCCAACTGGCGGACCCGGATGCGGTCCTGGCAGGCTTCTGCTCGGG ACCGGTACTGATGCCCGTTGTTGCCGTGTACATACAACCCGTTGTTGTAGA GACTACCCAGGCGAGGAATGTTGTAGTGAATGGGACTGCATGTGTGTTCAG CCTGAGTTTCATTGCGGAGATCCATGCTGCACAACTTGTCGTCATCACCCC TGCCCACCTGGGCAAGGAGTACAGAGCCAGGGTAAATTTTCATTCGGCTTT CAGTGCATTGATTGCGCATCTGGAACATTCTCCGGGGGGTCACGAGGGCCAT TGCAAACCATGGACTGATTGTACACAATTCGGTTTCCTGACAGTCTTTCCA GGGAACAAAACACACAATGCTGTCTGCGTGCCTCGAAGCCCACCTGCTGAA CCT | 853 |
| 1106 | Fab region (VH-CH1) | GAAGTTCAACTTGTGGAGTCTGGAGGCGGTTTGGTACAACCTGGACGGTTCA CTTCGTTTGTCATGTGCTGCCTCTGGGTTTAATATCAAGGATACTTATATT CATTGGCGTTCGCCAGGCACCGGAAAAATGCCTCGAATGGGTGGCCCGTATA TATCCCACTAACGGTTACACTCGATATGCTGACTCTGTAAAGGGTCGATTT | 846 |

| | | | |
|---|---|---|---|
| | | ACTATATCCGCTGATACTAGCAAAAACACTGCCTACCTCCAGATGAACAGT CTGCGCGCCCGAGCATACAGCAGTTTATTATTGTTCCCGATGGGGAGGTGAC GGGTTCTATGCCATGGACTACTGGGGTCAAGGGACACTGGTAACCGTTTCT TCTGCTAGCACCAAAGGACCTAGTGTTTTTCCTCTTGCCCCTTCCTCAAAG TCTACCTCTGGGGGGACAGCCGCTCTGGGCTGCCTGGTCAAGGATTATTTC CCAGAGCCTGTCACTGTCAGTTGGAACTCTGGAGCCTTGACTTCTGGTGTT CATACATTTCCTGCTGTCCTTCAAAGCAGCGGCTTGTACTCATTGTCTTCT GTTGTGACAGTACCCTCAAGCAGCCTCGGCACTCAGACATACATCTGCAAT GTCAACCACAAACCCTCAAATACAAAGGTAGATAAAAAGTC | |
| | Fab region (VL-CL) | GACATTCAGATGACCCAATCCCCCTCTAGCCTTTCAGCCTCCGTAGGGGAC AGAGTTACAATAAOCTGTAGGGCTAGTCAGGACGTTAATACAGCAGTCGCA TGGTACCAGCAGAAGCCCGGTAAGGCTCCTAAGCTTTTGATCTACAGTGCT TCTTTCCTGTATTCTGGTGTACCTAGCCGATTCTCAGGCTCTCGGAGTGGC ACAGACTTCACTCTGACAATTTCAAGTCTCCAGCCAGAAGATTTTGCAACC TACTACTGCCAACAACACTACACTACTCCTCCAACCTTCGGATGTGGCACA AAAGTAGAGATTAAGCGTACGGTGGCAGCTCCCAGCGTTTTTATCTTTCCC CCATCCGACGAGCAGCTCAAGAGTGGCACTGCCTCTGTAGTTTGTTTGCTG AATAACTTCTATCCACGTGAAGCAAAAGTACAGTGGAAGGTCGATAATGCC CTTCAGAGCGGTAACAGCCAAGAAAGTGTTACCGAGCAAGATTCCAAAGAT TCCACTTACAGTCTGTCCAGCACATTGACACTGAGTAAGGCTGATTACGAA AAACACAAGGTGTACGCATGCGAGGTGACACACCAAGGTCTTTCATCTCCT GTAACTAAGAGCTTTAACCGGGGAGAATGT | 847 |
| | L1 linker | GAACCAAAGTCTTGT | 848 |
| | L2 linker | GGTGGTGGGGGCAGCGGGGGCGGAGGTAGTGGAGGCGGCGGTAGTGAACCA AAGAGTAGT | 849 |
| | L3 linker | GGAGGCGGCGGCTCT | 850 |
| | L4 linker | GGAGGCGGCGGCTCT | 850 |
| | Hinge-Fc (Knob) | GACAAAACTCACACGTGCCCACCGTGCCCAGCACCTGAACTCCTGGGGGGA CCGTCAGTCTTCCTCTTCCCCCCAAAACCCAAGGACACCCTCATGATCTCC | 851 |

| | | CGGACCCCTGAGGTCACATGCGTGGTGGTGGACGTGAGCCACGAAGACCCT GAGGTCAAGTTCAACTGGTACGTGGACGGCGTGGAGGTGCATAATGCCAAG ACAAAGCCGCGGGAGGAGCAGTACAACAGCACGTACCGTGTGGTCAGCGTC CTCACCGTCCTGCACCAGGACTGGCTGAATGGCAAGGAGTACAAGTGCAAG GTCTCCAACAAAGCCCTCCCAGCCCCCATCGAGAAAACCATCTCCAAAGCC AAAGGGCAGCCCCGAGAACCACAGGTGTACACCCTGCCCCCCATGCCGGGAG GAGATGACCAAGAACCAGGTCAGCCTGTGGTGCCTGGTCAAAGGCTTCTAT CCCAGCGACATCGCCGTGGAGTGGGAGAGCAATGGGCAGCCGGAGAACAAC TACAAGACCACGCCTCCCGTGCTGGACTCCGACGGCTCCTTCTTCCTCTAC AGCAAGCTCACCGTGGACAAGAGCAGGTGGCAGCAGGGGAACGTCTTCTCA TGCTCCGTGATGCATGAGGCTCTGCACAACCACTACACGCAGAAGAGCCTC TCCCTGTCTCCGGGTAAA | |
| Hinge-Fc (Hole) | GACAAAACTCACACGTGCCCACCGTGCCCAGCACCTGAACTCCTGGGGGGA CCGTCAGTCTTCCTCTTCCCCCCAAAAACCCAAGGACACCCTCATGATCTCC CGGACCCCTGAGGTCACATGCGTGGTGGTGGACGTGAGCCACGAAGACCCT GAGGTCAAGTTCAACTGGTACGTGGACGGCGTGGAGGTGCATAATGCCAAG ACAAAGCCGCGGGAGGAGCAGTACAACAGCACGTACCGTGTGGTCAGCGTC CTCACCGTCCTGCACCAGGACTGGCTGAATGGCAAGGAGTACAAGTGCAAG GTCTCCAACAAAGCCCTCCCAGCCCCCATCGAGAAAACCATCTCCAAAGCC AAAGGGCAGCCCCGAGAACCACAGGTGTGCACCCTGCCCCCCATCCCGGGAG GAGATGACCAAGAACCAGGTCAGCCTGAGCTGCGCGGTCAAAGGCTTCTAT CCCAGCGACATCGCCGTGGAGTGGGAGAGCAATGGGCAGCCGGAGAACAAC TACAAGACCACGCCTCCCGTGCTGGACTCCGACGGCTCCTTCTTCCTCGTG AGCAAGCTCACCGTGGACAAGAGCAGGTGGCAGCAGGGGAACGTCTTCTCA TGCTCCGTGATGCATGAGGCTCTGCACAACCACTACACGCAGAAGAGCCTC TCCCTGTCTCCGGGTAAA | 458 |
| Z protein(Solubl e protein; IL- | AGGCCAAGCGGACGCAAGTCAAGCAAGATGCAGGCCTTCAGAATTTGGGAT GTGAACCAAAAGACTTTTTATCTCCGAAATAACCAACTCGTGGCTGGCTAC TTGCAAGGACCTAACGTCAACTTGGAGGAGAAAATCGACGTTGTGCCTATT | 854 |

| | | | |
|---|---|---|---|
| | 1R antagonist) | GAACCCCATGCTCTCTTTCTGGGTATACACGGGGGGAAGATGTGTCTGTCT TGTGTTAAGTCCGGTGATGAGACACGCTTGCAACTGGAGGCAGTGAATATA ACAGACTTGAGTGAGAATAGAAAACAGGATAAGCGGTTCGCTTTCATCAGA TCAGATTCTGGACCAACAACCTCTTTTGAGAGCGCAGCTTGTCCTGGCTGG TTCTTGTGCACCGCTATGGAAGCAGACCAACCCGTCTCCCTTACAAACATG CCAGACGAGGGAGTAATGGTCACAAAGTTCTATTTTCAGGAGGATGAG | |
| 1107 | Fab region (VH-CH1) | GAAGTTCAACTTGTGGAGTCTGGAGGCGGTTTGGTACAACCTGGAGGTTCA CTTCGTTTGTCATGTGCTGCCTCTGGGTTTAATATCAAGGATACTTATATT CATTGGGTTCGCCAGGCACCCGGAAAAATGCCTGGAATGGGTGGCCCGTATA TATCCCACTAACGGTTACACTCGATATGCTGACTCTGTAAAGGGTCGATTT ACTATATCCGCTGATACTAGCAAAAACACTGCCTACCTCCAGATGAACAGT CTGCGCGCCGAGGATACAGCAGTTTATTATTGTTCCCGATGGGGAGGTGAC GGGTTCTATGCCATGGACTACTGGGGTCAAGGGACACTGGTAACCGTTTCT TCTGCTAGCACCAAAGGACCTAGTGTTTTTCCTCTTGCCCCTTCCTCAAAG TCTACCTCTGGGGGGACAGCCGCTCTGGGCTGCCTGGTCAAGGATTATTTC CCAGAGCCTGTCACTGTCAGTTGGAACTCTGGAGCCTTGACTTCTGGTGTT CATACATTTCCTGCTGTCCTTCAAAGCAGCGGCTTGTACTCATTGTCTTCT GTTGTGACAGTACCCTCAAGCAGCCTCGGCACTCAGACATACATCTGCAAT GTCAACCACAAACCCTCAAATACAAAGGTAGATAAAAAAGTC | 846 |
| | Fab region (VL-CL) | GACATTCAGATGACCCAATCCCCCTCTAGCCTTTCAGCCTCCGTAGGCGAC AGAGTTACAATAACCTGTAGGGCTAGTCAGGACGTTAATACAGCAGTCGCA TGGTACCAGCAGAAGCCCGGTAAGGCTCCTAAGCTTTTGATCTACAGTGCT TCTTTCCTGTATTCTGGTGTACCTAGCCGATTCTCAGGCTCTCGGAGTGGC ACAGACTTCACTCTGACAATTTCAAGTCTCCAGCCAGAAGATTTTGCAACC TACTACTGCCAACAACACTACACTACTCCTCCAACCTTCGGATGTGGCACA AAAGTAGAGATTAAGCGTACGGTGGCAGCTCCCAGCCGTTTTTATCTTTCCC CCATCCGACGAGCAGCTCAAGAGTGGCACTGCCTCTGTAGTTTGTTTGCTG AATAACTTCTATCCACGTGAAGCAAAAGTACAGTGGAAGGTCGATAATGCC CTTCAGAGCGGTAACAGCCAAGAAAGTGTTACCGAGCAAGATTCCAAAGAT | 847 |

| | | TCCACTTACAGTCTGTCCAGCACATTGACACTGAGTAAGGCTGATTACGAA AAACACAAGGTGTACGCATGCGAGGTGACACACCAAGGTCTTTCATCTCCT GTAACTAAGAGCTTTAACCGGGGAGAATGT | |
| L1 linker | GAACCAAAGTCTTGT | 848 |
| L2 linker | GGTGGTGGGGGCAGCGGGGGCGGAGGTAGTGGAGGCGGCGGTAGTGAACCA AAGAGTAGT | 849 |
| L3 linker | GGAGGCGGCGGCTCT | 850 |
| L4 linker | GGAGGCGGCGGCTCT | 850 |
| Hinge-Fc (Knob) | GACAAAACTCACACGTGCCCACCGTGCCCAGCACCTGAACTCCTGGGGGGA CCGTCAGTCTTCCTCTTCCCCCCAAAAACCCAAGGACACCCTCATGATCTCC CGGACCCCTGAGGTCACATGCGTGGTGGTGGACGTGAGCCACGAAGACCCT GAGGTCAAGTTCAACTGGTACGTGGACGGCGTGGAGGTGCATAATGCCAAG ACAAAGCCGCGGGAGGAGCAGTACAACAGCACGTACCGTGTGGTCAGCGTC CTCACCGTCCTGCACCAGGACTGGCTGAATGGCAAGGAGTACAAGTGCAAG GTCTCCAACAAAGCCCTCCCAGCCCCCATCGAGAAAACCATCTCCAAAGCC AAAGGGCAGCCCCGAGAACCACAGGTGTACACCCTGCCCCCATGCCGGGAG GAGATGACCAAGAACCAGGTCAGCCTGTGGTGCCTGGTCAAAGGCTTCTAT CCCAGCGACATCGCCGTGGAGTGGGAGAGCAATGGGCAGCCGGAGAACAAC TACAAGACCACGCCTCCCGTGCTGGACTCCGACGGCTCCTTCTTCCTCTAC AGCAAGCTCACCGTGGACAAGAGCAGGTGGCAGCAGGGGAACGTCTTCTCA TGCTCCGTGATGCATGAGGCTCTGCACAACCACTACACGCAGAAGAGCCTC TCCCTGTCTCCGGGTAAA | 851 |
| Hinge-Fc (Hole) | GACAAAACTCACACGTGCCCACCGTGCCCAGCACCTGAACTCCTGGGGGGA CCGTCAGTCTTCCTCTTCCCCCCAAAAACCCAAGGACACCCTCATGATCTCC CGGACCCCTGAGGTCACATGCGTGGTGGTGGACGTGAGCCACGAAGACCCT GAGGTCAAGTTCAACTGGTACGTGGACGGCGTGGAGGTGCATAATGCCAAG ACAAAGCCGCGGGAGGAGCAGTACAACAGCACGTACCGTGTGGTCAGCGTC CTCACCGTCCTGCACCAGGACTGGCTGAATGGCAAGGAGTACAAGTGCAAG GTCTCCAACAAAGCCCTCCCAGCCCCCATCGAGAAAACCATCTCCAAAGCC | 458 |

| | | | |
|---|---|---|---|
| | | AAAGGGCAGCCCCGAGAACCACAGGTGTGCACCCTGCCCCCATCCCGGGAG GAGATGACCAAGAACCACGGTCAGCCTGAGCTGCGCCGTCAAAGGCTTCTAT CCCAGCGACATCGCCGTCGAGTGGGAGAGCAATGGGCAGCCGGAGAACAAC TACAAGACCACGCCTCCCGTGCTGGACTCCGACGGCTCCTTCTTCCTCGTG AGCAAGCTCACCGTGGACAAGAGCAGGTGGCAGCAGGGGAACGTCTTCTCA TGCTCCGTGATGCATGAGGCTCTGCACAACCACTACACGCAGAAGAGCCTC TCCCTGTCTCCGGGTAAA | |
| | Z protein(Cytoki ne; IL2) | GCTCCTACCAGTTCTAGCACTAAGAAAACCCAGCTTCAATTGGAGCACCTG CTGCTCGACCTTCAGATGATACTGAATGGAATAAACAACTACAAAAATCCA AAATTGACAAGGATGCTGACTTTTAAATTCTACATGCCTAAGAAGGCAACA GAGTTGAAACATTTGCAGTGCTTGGAGGACGAGCTGAAGCCTTTGGAAGAA GTATTGAACTTGGCCCAGTCTAAGAACTTTCACCTCAGACCCAGAGACCTT ATTTCAAACATTAACGTCATCGTATTGGAACTTAAAGGAAGTGAAACCACT TTCATGTGCGAGTATGCTGATGAGACCGCTACTATCGTGGAGTTTTTGAAC AGATGGATAACATTTTGCCAGAGTATTATTTCCACTCTTACCGGGGGAGGA GGCTCT | 855 |
| II08 | Fab region (VH-CH1) | GAAGTTCAACTTGTGGAGTCTGGAGGCGGTTTGGTACAACCTGGAGGTTCA CTTCGTTTGTCATGTGCTGCCTCTGGGTTTAATATCAAGGATACTTATATT CATTGGGTTCGCCAGGCACCCGGAAAATGCCTGGAATGGGTGGCCCGTATA TATCCCACTAACGGTTACACTCGATATGCTGACTCTGTAAAGGGTCGATTT ACTATATCCGCTGATACTAGCAAAAACACTGCCTACCTCCAGATGAACAGT CTGCGCGCCGAGGATACAGCAGTTTATTATTGTTCCCGATGGGGAGGTGAC GGGTTCTATGCCATGGACTACTGGGGTCAAGGGACACTGGTAACCGTTTCT TCTGCTAGCACCAAAGGACCTAGTGTTTTTTCCTCTTGCCCCCTTCCTCAAAG TCTACCTCTGGGGGGACAGCCGCTCTGGGCTGCCTGGTCAAGGATTATTTC CCAGAGCCTGTCACTGTCAGTTCGAACTCTGGAGCCTTGACTTCTGGTGTT CATACATTTCCTGCTGTCCTTCAAAGCAGCGGCTTGTACTCATTGTCTTCT GTTGTGACAGTACCCTCAAGCAGCCTCGGCACTCAGACATACATCTGCAAT GTCAACCACAAACCCTCAAATACAAAGGTAGATAAAAAAGTC | 846 |

| | Fab region (VL-CL) | GACATTCAGATGACCCAATCCCCCTCTAGCCTTTCAGCCTCCGTAGGGGAC AGAGTTACAATAACCTGTAGGGGCTAGTCAGGACGTTAATACAGCAGTCGCA TGGTACCAGCAGAAGCCCGGTAAGGCTCCTAAGCTTTTTGATCTACAGTGCT TCTTTCCTGTATTCTGGTGTACCTAGCCGATTCTCAGGCTCTCGGAGTGGC ACAGACTTCACTCTGACAATTTCAAGTCTCCAGCCAGAAGATTTTGCAACC TACTACTGCCAACAACACTACACTACTCCTCCAACCTTCGGATGTGGCACA AAAGTAGAGATTAAGCGTACGGTGGCAGCTCCCAGCGTTTTTATCTTTCCC CCATCCGACGAGCAGCTCAAGAGTGGCACTGCCTCTGTAGTTTGTTTGCTG AATAACTTCTATCCACGTGAAGCAAAAGTACAGTGGAAGGTCGATAATGCC CTTCAGAGCGGTAACAGCCAAGAAAGTGTTACCGAGCAAGATTCCAAAGAT TCCACTTACAGTCTGTCCAGCACATTGACACTGAGTAAGGCTGATTACGAA AAACACAAGGTGTACGCATGCGAGGTGACACACCAAGGTCTTTCATCTCCT GTAACTAAGAGCTTTAACCGGGGGAGAATGT | 847 |
| | L1 linker | GAACCAAAGTCTTGT | 848 |
| | L2 linker | GGTGGTGGGGGCAGCGGGGGCGGAGGTAGTGGAGGCGGCGGGTAGTGAACCA AAGAGTAGT | 849 |
| | L3 linker | GGAGGCGGCGGCTCT | 850 |
| | L4 linker | GGAGGCGGCGGCTCT | 850 |
| | Hinge-Fc (Knob) | GACAAAACTCACACGTGCCCACCGTGCCCAGCACCTGAACTCCTGGGGGGA CCGTCAGTCTTCCTCTTCCCCCCAAAACCCAAGGACACCCTCATGATCTCC CGGACCCCTGAGGTCACATGCGTGGTGGTGGACGTGAGCCACGAAGACCCT GAGGTCAAGTTCAACTGGTACGTGGACGGCGTGGAGGTGCATAATGCCAAG ACAAAGCCGCGGGAGGAGCAGTACAACAGCACGTACCGTGTGGTCAGCGTC CTCACCGTCCTGCACCAGGACTGGCTGAATGGCAAGGAGTACAACTGCAAG GTCTCCAACAAAGCCCTCCCAGCCCCCATCGAGAAAACCATCTCCAAAGCC AAAGGGCAGCCCCGAGAACCACAGGTGTACACCCTGCCCCCATGCCGGGAG GAGATGACCAAGAACCACGGTCAGCCTGTGGTGCCTGGTCAAAGGCTTCTAT CCCAGCGACATCGCCGTGGAGTGGGAGAGCAATGGGCAGCCGGAGAACAAC TACAAGACCACGCCTCCCGTGCTGGACTCCGACGGCTCCTTCTTCCTCTAC | 851 |

| | | | |
|---|---|---|---|
| | | AGCAAGCTCACCGTGGACAAGAGCAGGTGGCAGCAGGGGAACGTCTTCTCA TGCTCCGTGATGCATGAGGCTCTGCACAACCACTACACGCAGAAGAGCCTC TCCCTGTCTCCGGGTAAA | |
| | Hinge-Fc (Hole) | GACAAAACTCACACGTGCCCACCGTGCCCAGCACCTGAACTCCTGGGGGGA CCGTCAGTCTTCCTCTTCCCCCCAAAACCCAAGGACACCCTCATGATCTCC CGGACCCCTGAGGTCACATGCGTGGTGGTGGACGTGAGCCACGAAGACCCT GAGGTCAAGTTCAACTGGTACGTGGACGGCGTGGAGGTGCATAATGCCAAG ACAAAGCCGCGGGAGGAGCAGTACAACAGCACGTACCGTGTGGTCAGCGTC CTCACCGTCCTGCACCAGGACTGGCTGAATGGCAAGGAGTACAAGTGCAAG GTCTCCAACAAAGCCCTCCCAGCCCCCATCGAGAAAACCATCTCCAAAGCC AAAGGGCAGCCCCGAGAACCACAGGTGTGCACCCTGCCCCCATCCCGGGAG GAGATGACCAAGAACCAGGTCAGCCTGAGCTGCGCGGTCAAAGGCTTCTAT CCCAGCGACATCGCCGTGGAGTGGGAGAGCAATGGGCAGCCGGAGAACAAC TACAAGACCACGCCTCCCGTGCTGGACTCCGACGGCTCCTTCTTCCTCGTG AGCAAGCTCACCGTGGACAAGAGCAGGTGGCAGCAGGGGAACGTCTTCTCA TGCTCCGTGATGCATGAGGCTCTGCACAACCACTACACGCAGAAGAGCCTC TCCCTGTCTCCGGGTAAA | 458 |
| | Z protein(Antibody mimetics: E01 DARPin) | GATCTCGGCAAGAAACTTCTGGAAGCAGCCAGGGCTGGCCAAGACGACGAG GTGCGTATTCTCATGGCCAATGGCGGCAGATGTCAATGCCGACGACACTTGG GGGTGGACACCCCTGCATTTGGCCGCATACCAAGGCCATCTGGAAATAGTA GAGGTTTTGTTGAAGAACGGAGCCGATGTAAATGCATATGATTACATTGGA TGGACTCCTCTGCACCTCGCCAGCCGACGGCCACTTGGAGATAGTCGACGTC CTCTTGAAGAATGGGGCTGATGTAAATGCATCTGATTACATCGGGGACACA CCCCTCCACTTGGCCGCTCATAATGGGCACCTCGAAATTGTGGAGGTTCTC TTGAAGCACGGAGCCGACGTTAACGCCTCAAGATAAGTTCGGTAAAACCGCT TTTCACATATCAATCGACAACGGAAATGAGGATCTGGCAGAAATCCTTCAA | 856 |
| H09 | Fab region (VH-CH1) | GAAGTTCAACTTGTGGAGTCTGGAGGCGGTTTCGTACAACCTGGAGGTTCA CTTCGTTTGTCATGTGCTGCCTCTGGGTTTAATATCAAGGATACTTATATT CATTGCGTTCGCCAGGCACCCCGGAAAATGCCTGGAATGGGTGGCCGGTATA | 846 |

| | | TATCCCACTAACGGTTACACTCGATATGCTGACTCTGTAAAGGGTCGATTT ACTATATCCGCTGATACTAGCAAAAACACTGCCTACCTCCAGATGAACAGT CTGCGCGCCGAGGATACAGCAGTTTATTATTGTTCCCGATGGGGAGGTGAC GGGTTCTATGCCATGGACTACTGGGGTCAAGGGACACTGGTAACCGTTTCT TCTGCTAGCACCAAAGGACCTAGTGTTTTTCCTCTTGCCCCTTCCTCAAAG TCTACCTCTGGGGGGACAGCCGCTCTGGGCTGCCTGGTCAAGGATTATTTC CCAGAGCCTGTCACTGTCAGTTGGAACTCTGGAGCCTTGACTTCTGGTGTT CATACATTTCCTGCTGTCCTTCAAAGCAGCGGCTTGTACTCATTGTCTTCT GTTGTGACAGTACCCTCAAGCAGCCTCGGCACTCAGACATACATCTGCAAT GTCAACCACAAACCCTCAAATACAAAGGTAGATAAAAAAGTC | |
| Fab region (VL-CL) | GACATTCAGATGACCCAATCCCCCTCTAGCCTTTCAGCCTCCGTAGGGGAC AGAGTTACAATAACCTGTAGGGCTAGTCAGGACGTTAATACAGCAGTCGCA TGGTACCAGCAGAAGCCCGGTAAGGCTCCTAAGCTTTTGATCTACAGTGCT TCTTTCCTGTATTCTGGTGTACCTAGCCGATTCTCAGGCTCTCGGAGTGGC ACAGACTTCACTCTGACAATTTCAAGTCTCCAGCCAGAAGATTTTGCAACC TACTACTGCCAACAACACTACACTACTCCTCCAACCTTCGGATGTGGCACA AAAGTAGAGATTAAGCGTACGGTGGCAGCTCCCAGCGTTTTTATCTTTCCC CCATCCGACGAGCAGCTCAAGAGTGGCACTGCCTCTGTAGTTTGTTTGCTG AATAACTTCTATCCACGTGAAGCAAAAGTACAGTGGAAGGTCGATAATGCC CTTCAGAGCGGTAACAGCCAAGAAAGTGTTACCGAGCAAGATTCCAAAGAT TCCACTTACAGTCTGTCCAGCACATTGACACTGAGTAAGGCTGATTACGAA AAACACAAGGTGTACGCATGCGAGGTGACACACCAAGGTCTTTCATCTCCT GTAACTAAGAGCTTTAACCGGGGAGAATGT | 847 |
| L1 linker | GAACCAAAGTCTTGT | 848 |
| L2 linker | GGTGGTGGGGGCAGCGGGGGCGGAGGTAGTGGAGGCGGCGGTAGTGAACCA AAGAGTAGT | 849 |
| L3 linker | GGAGGCGGCGGCTCT | 850 |
| L4 linker | GGAGGCGGCGGCTCT | 850 |
| Hinge-Fc (Knob) | GACAAAACTCACACGTGCCCACCGTGCCCAGCACCTGAACTCCTGGGGGGA | 851 |

| | | CCGTCAGTCTTCCTCTTCCCCCCAAAAACCCAAGGACACCCTCATGATCTCC CGGACCCCTGAGGTCACATGCGTGGTGGTGGACGTGAGCCACGAAGACCCT GAGGTCAAGTTCAACTGGTACGTGGACGGCGTGGAGGTGCATAATGCCAAG ACAAAGCCGCGGGAGGAGCAGTACAACAGCACGTACCGTGTGGTCAGCGTC CTCACCGTCCTGCACCAGGACTGGCTGAATGGCAAGGAGTACAAGTGCAAG GTCTCCAACAAAGCCCTCCCAGCCCCCATCGAGAAAACCATCTCCAAAGCC AAAGGGCAGCCCCGAGAACCACAGGTGTACACCCTGCCCCCATGCCGGGAG GAGATGACCAAGAACCAGGTCAGCCTGTGGTGCCTGGTCAAAGGCTTCTAT CCCAGCGACATCGCCGTGGAGTGGGAGAGCAATGGGCAGCCGGAGAACAAC TACAAGACCACGCCTCCCGTGCTGGACTCCGACGGCTCCTTCTTCCTCTAC AGCAAGCTCACCGTGGACAAGAGCAGGTGGCAGCAGGGGAACGTCTTCTCA TGCTCCGTGATGCATGAGGCTCTGCACAACCACTACACGCAGAAGAGCCTC TCCCTGTCTCCGGGTAAA | |
| Hinge-Fc (Hole) | GACAAAACTCACACGTGCCCACCGTGCCCAGCACCTGAACTCCTGGGGGGA CCGTCAGTCTTCCTCTTCCCCCCAAAAACCCAAGGACACCCTCATGATCTCC CGGACCCCTGAGGTCACATGCGTGGTGGTGGACGTGAGCCACGAAGACCCT GAGGTCAAGTTCAACTGGTACGTGGACGGCGTGGAGGTGCATAATGCCAAG ACAAAGCCGCGGGAGGAGCAGTACAACAGCACGTACCGTGTGGTCAGCGTC CTCACCGTCCTGCACCAGGACTGGCTGAATGGCAAGGAGTACAAGTGCAAG GTCTCCAACAAAGCCCTCCCAGCCCCCATCGAGAAAACCATCTCCAAAGCC AAAGGGCAGCCCCGAGAACCACAGGTGTGCACCCTGCCCCCATCCCGGGAG GAGATGACCAAGAACCAGGTCAGCCTGAGCTGCGCGGTCAAAGGCTTCTAT CCCAGCGACATCGCCGTGGAGTGGGAGAGCAATGGGCAGCCGGAGAACAAC TACAAGACCACGCCTCCCGTGCTGGACTCCGACGGCTCCTTCTTCCTCGTG AGCAAGCTCACCGTGGACAAGAGCAGGTGGCAGCAGGGGAACGTCTTCTCA TGCTCCGTGATGCATGAGGCTCTGCACAACCACTACACGCAGAAGAGCCTC TCCCTGTCTCCGGGTAAA | 458 |
| Z protein(Single | GAGGTGCAACTTGTGGAATCTGGAGGAGGCCTGGTTCAAGCCGGAGGATCT CTCCGACTTTCTTGTGCTGCTTCTGGCCGTACCTTCTCTTCCTATGCTATG | 857 |

| domain mAb; VHH domain 9G8) | GGCTGGTTCCGGCAAGCACCTGGGAAGGAGAGGGAGTTTGTAGTTGCAATT AACTGGTCCAGCGGGTCAACATATTACGCAGATTCCGTCAAAGGGCGGTTC ACTATTTCACGGGATAATGCTAAAAACACCATGTATCTCCAAATGAACAGC CTTAAGCCCGAAGACACCGCAGTCTATTACTGCGCCGCAGGCTATCAGATC AACTCCGGTAACTATAACTTTAAAGACTATGAGTATGATTATTGGGGCCAA GGCACCCAAGTTACCGTGTCAAGCGCCTTGGAA | |

Table 92 below shows each polypeptide sequence constituting the novel antibody format.

[Table 92]

| Name | Polypeptide | Sequence | SEC ID NO |
|------|-------------|----------|-----------|
| HD1 | Polypeptide 1 | EVQLVESGGGLVQPGGSLRLSCAASGFNIKDTYIHWVRQAPGKCLEWVARIYPTNGYTRYADSVKGRFTISADTSKNTAYLQMNSLRAEDTAVYYCSRWGGDGFYAMDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESSGQPENNYKTTPPVLDSDGSFFLYSKLTVDKSRWQQGNVFSCSVMHEALHNHYTQKSLSLSPGK | 14 |
| HD1 | Polypeptide 2 | DIQMTQSPSSLSASVGDRVTITCRASQDVNTAVAWYQQKPGKAPKLLIYSASFLYSGVPSRFSGSRSGTDFTLTISSLQPEDFATYYCQQHYTTPPTFGCGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGECGGGGSGGGGSGGGGSEPKSSDKTHTCPPCPAPELLGGPSYFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 15 |
| HD1 | Polypeptide 3 | DKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVCTLPPSREEMTKNQVSLSCAVKGFYPSDIAVEWESNGQPENNYKTTPPVLDSDGSFFLVSKLTVDKSRWQQGNVFSCSVMHEALHNHYTQKSLSLSPGK | 7 |
| HD1 | Polypeptide 4 | DKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVCTLPPSREEMTKNQVSLSCAVKGFYPSDIAVEWESNGQPENNYKTTPPVLDSDGSFFLVSKLTVDKSRWQQGNVFSCSVMHEALHNHYTQKS | 7 |

| | | | LSLSPGK | |
|---|---|---|---|---|
| H04 | Polypeptide 1 | | EVQLVESGGGLVQPGGSLRLSCAASGFNIKDTYIHWVRQAPGKCLEWVARIYPTN GYTRYADSVKGRFTISADTSKNTAYLQMNSLRAEDTAVYYCSRWGGDGFYAMDYW GQGTLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVEP KSCDKTHTCPPCPAPELLGGPSVFLFPPKPKDTLMISRTPEVTCVVVDVSHEDPE VKFNWYVDGVEVHNAKTKPREEQYNSTYRVVSVLTVLHQDWLNGKEYKCKVSNKA LPAPIEKTISKAKGQPREPQVYTLPPCREEMTKNQVSLWCLVKGFYPSDIAVEWE SNGQPENNYKTTPPVLDSDGSFFLYSKLTVDKSRWQQGNVFSCSVMHEALHNHYT QKSLSLSPGK | 14 |
| | Polypeptide 2 | | DIQMTQSPSSLSASVGDRVTITCRASQDVNTAVAWYQQKPGKAPKLLIYSASFLY SGVPSRFSGSRSGTDFTLTISSLQPEDFATYYCQQHYTTPPTFGCGTKVEIKRTV AAPSVFIFPPSDEQLKSGTASVVCLLNNFYPREAKVQWKVDNALQSGNSQESVTE QDSKDSTYSLSSTLTLSKADYEKHKVYACEVTHQGLSSPVTKSFNRGECGGGGSG GGGSGGGGSEPKSSDKTHTCPPCPAPELLGGPSVFLFPPKPKDTLMISRTPEVTC VVVDVSHEDPEVKFNWYVDGVEVHNAKTKPREEQYNSTYRVVSVLTVLHQDWLNG KEYKCKVSNKALPAPIEKTISKAKGQPREPQVYTLPPCREEMTKNQVSLWCLVKG FYPSDIAVEWESNGQPENNYKTTPPVLDSDGSFFLYSKLTVDKSRWQQGNVFSCS VMHEALHNHYTQKSLSLSPGK | 15 |
| | Polypeptide 3 | | SDTGRPFVEMYSEIPEIIHMTEGRELVIPCRVTSPNITVTLKKFPLDTLIPDGKR IIWDSRKGFIISNATYKEIGLLTCEATVNGHLYKTNYLTHRQTNTIIDVVLSPSH GIELSVGEKLVLNCTARTELNVGIDFNWEYPSSKHQHKKLVNRDLKTQSGSEMKK FLSTLTIDGVTRSDQGLYTCAASSGLMTKKNSTFVRVHEKGGGGSDKTHTCPPCP APELLGGPSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPIEKTISKAK GQPREPQVCTLPPSREEMTKNQVSLSCAVKGFYPSDIAVEWESNGQPENNYKTTP PVLDSDGSFFLVSKLTVDKSRWQQGNVFSCSVMHEALHNHYTQKSLSLSPGK | 858 |
| | Polypeptide 4 | | SDTGRPFVEMYSEIPEIIHMTEGRELVIPCRVTSPNITVTLKKFPLDTLIPDGKR IIWDSRKGFIISNATYKEIGLLTCEATVNGHLYKTNYLTHRQTNTIIDVVLSPSH | 858 |

| | | | |
|---|---|---|---|
| | | GIELSVGEKLVLNCTARTELNVGIDFNWEYPSSKHQHKKLVNRDLKTQSGSEMKK FLSTLTIDGVTRSDQGLYTCAASSGLMTKKNSTFVRVHEKGGGGSDKTHTCPPCP APELLGGPSVFLFPPKPKDTLMISRTPEVTCVVVDVSHEDPEVKFNWYVDGVEVH NAKTKPREEQYNSTYRVVSVLTVLHQDWLNGKEYKCKVSNKALPAPIEKTISKAK GQPREPQVCTLPPSREEMTKNQVSLSCAVKGFYPSDIAVEWESNGQPENNYKTTP PVLDSDGSFFLVSKLTVDKSRWQQGNVFSCSVMHEALHNHYTQKSLSLSPGK | |
| HO5 | Polypeptide 1 | EVQLVESGGGLVQPGGSLRLSCAASGFNIKDTYIHWVRQAPGKCLEWVARIYPTN GYTRYADSVKGRFTISADTSKNTAYLQMNSLRAEDTAVYYCSRWGGDGFYAMDYW GQGTLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVEP KSCDKTHTCPPCPAPELLGGPSVFLFPPKPKDTLMISRTPEVTCVVVDVSHEDPE VKFNWYVDGVEVHNAKTKPREEQYNSTYRVVSVLTVLHQDWLNGKEYKCKVSNKA LPAPIEKTISKAKGQPREPQVYTLPPCREEMTKNQVSLWCLVKGFYPSDIAVEWE SNGQPENNYKTTPPVLDSDGSFFLYSKLTVDKSRWQQGNVFSCSVMHEALHNHYT QKSLSLSPGK | 14 |
| | Polypeptide 2 | DIQMTQSPSSLSASVGDRVTITCRASQDVNTAVAWYQQKPGKAPKLLIYSASFLY SGVPSRFSGSRSGTDFTLTISSLQPEDFATYYCQQHYTTPPTFGCGTKVEIKRTV AAPSVFIFPPSDEQLKSGTASVVCLLNNFYPREAKVQWKVDNALQSGNSQESVTE QDSKDSTYSLSSTLTLSKADYEKHKVYACEVTHQGLSSPVTKSFNRGECGGGGSG GGGSGGGGSEPKSSDKTHTCPPCPAPELLGGPSVFLFPPKPKDTLMISRTPEVTC VVVDVSHEDPEVKFNWYVDGVEVHNAKTKPREEQYNSTYRVVSVLTVLHQDWLNG KEYKCKVSNKALPAPIEKTISKAKGQPREPQVYTLPPCREEMTKNQVSLWCLVKG FYPSDIAVEWESNGQPENNYKTTPPVLDSDGSFFLYSKLTVDKSRWQQGNVFSCS VMHEALHNHYTQKSLSLSPGK | 15 |
| | Polypeptide 3 | QRPTGGPGCGPGRLLLGTGTDARCCRVHTTRCCRDYPGEECCSEWDCMCVQPEFH CGDPCCTTCRHHPCPPGQGVQSQGKFSFGFQCIDCASGTFSGGHEGHCKPWTDCT QFGFLTVFPGNKTHNAVCVPGSPPAEPGGGGSDKTHTCPPCPAPELLGGPSVFLF PPKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVCTLPP | 859 |

| | | | |
|---|---|---|---|
| | | SREEMTKNQVSLSCAVKGFYPSDIAVEWESNGQPENNYKTTPPVLDSDGSFFLVS KLTVDKSRWQQGNVFSCSVMHEALHNHYTQKSLSLSPGK | |
| | Polypeptid e 4 | QRPTGGPGCGPGRLLLGTGTDARCCRVHTTRCCRDYPGEECCSEWDCMCVQPEFH CGDPCCTTCRHHPCPPGQGVQSQGKFSFGFQCIDCASGTFSGGHEGHCKPWTDCT QFGFLTVFPGNKTHNAVCVPGSPPAEPGGGGSDKTHTCPPCPAPELLGGPSVFLF PPKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVCTLPP SREEMTKNQVSLSCAVKGFYPSDIAVEWESNGQPENNYKTTPPVLDSDGSFFLVS KLTVDKSRWQQGNVFSCSVMHEALHNHYTQKSLSLSPGK | 859 |
| H06 | Polypeptid e 1 | EVQLVESGGGLVQPGGSLRLSCAASGFNIKDTYIHWVRQAPGKCLEWVARIYPTN GYTRYADSVKGRFTISADTSKNTAYLQMNSLRAEDTAVYYCSRWGGDGFYAMDYW GQGTLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVEP KSCDKTHTCPPCPAPELLGGPSVFLFPPKPKDTLMISRTPEVTCVVVDVSHEDPE VKFNWYVDGVEVHNAKTKPREEQYNSTYRVVSVLTVLHQDWLNGKEYKCKVSNKA LPAPIEKTISKAKGQPREPQVYTLPPCREEMTKNQVSLWCLVKGFYPSDIAVEWE SNGQPENNYKTTPPVLDSDGSFFLYSKLTVDKSRWQQGNVFSCSVMHEALHNHYT QKSLSLSPGK | 14 |
| | Polypeptid e 2 | DIQMTQSPSSLSASVGDRVTITCRASQDVNTAVAWYQQKPGKAPKLLIYSASFLY SGVPSRFSGSRSGTDFTLTISSLQPEDFATYYCQQHYTTPPTFGCGTKVEIKRTV AAPSVFIFPPSDEQLKSGTASVVCLLNNFYPREAKVQWKVDNALQSGNSQESVTE QDSKDSTYSLSSTLTLSKADYEKHKVYACEVTHQGLSSPVTKSFNRGECGGGGSG GGGSGGGGSEPKSSDKTHTCPPCPAPELLGGPSVFLFPPKPKDTLMISRTPEVTC VVVDVSHEDPEVKFNWYVDGVEVHNAKTKPREEQYNSTYRVVSVLTVLHQDWLNG KEYKCKVSNKALPAPIEKTISKAKGQPREPQVYTLPPCREEMTKNQVSLWCLVKG FYPSDIAVEWESNGQPENNYKTTPPVLDSDGSFFLYSKLTVDKSRWQQGNVFSCS VMHEALHNHYTQKSLSLSPGK | 15 |
| | Polypeptid e 3 | RPSGRKSSKMQAFRIWDVNQKTFYLRNNQLVAGYLQGPNVNLEEKIDVVPIEPHA LFLGIHGGKMCLSCVKSGDETRLQLEAVNITDLSENRKQDKRFAFIRSDSGPTTS | 860 |

| | | | |
|---|---|---|---|
| | | FESAACPGWFLCTAMEADQPVSLTNMPDEGVMVTKFYFQEDEGGGGSDKTHTCPP CPAPELLGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEKTISK AKGQPREPQVCTLPPSREEMTKNQVSLSCAVKGFYPSDIAVEWESNGQPENNYKT TPPVLDSDGSFFLVSKLTVDKSRWQQGNVFSCSVMHEALHNHYTQKSLSLSPGK | |
| | Polypeptide 4 | RPSGRKSSKMQAFRIWDVNQKTFYLRNNQLVAGYLQGPNVNLEEKIDVVPIEPHA LFLGIHGGKMCLSCVKSGDETRLQLEAVNITDLSENRKQDKRFAFIRSDSGPTTS FESAACPGWFLCTAMEADQPVSLTNMPDEGVMVTKFYFQEDEGGGGSDKTHTCPP CPAPELLGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEKTISK AKGQPREPQVCTLPPSREEMTKNQVSLSCAVKGFYPSDIAVEWESNGQPENNYKT TPPVLDSDGSFFLVSKLTVDKSRWQQGNVFSCSVMHEALHNHYTQKSLSLSPGK | 860 |
| H07 | Polypeptide 1 | EVQLVESGGGLVQPGGSLRLSCAASGFNIKDTYIHWVRQAPGKCLEWVARIYPTN GYTRYADSVKGRFTISADTSKNTAYLQMNSLRAEDTAVYYCSRWGGDGFYAMDYW GQGTLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVEP KSCDKTHTCPPCPAPELLGGPSVFLFPPKPKDTLMISRTPEVTCVVVDVSHEDPE VKFNWYVDGVEVHNAKTKPREEQYNSTYRVVSVLTVLHQDWLNGKEYKCKVSNKA LPAPIEKTISKAKGQPREPQVYTLPPCREEMTKNQVSLWCLVKGFYPSDIAVEWE SNGQPENNYKTTPPVLDSDGSFFLYSKLTVDKSRWQQGNVFSCSVMHEALHNHYT QKSLSLSPGK | 14 |
| | Polypeptide 2 | DIQMTQSPSSLSASVGDRVTITCRASQDVNTAVAWYQQKPGKAPKLLIYSASFLY SGVPSRFSGSRSGTDFTLTISSLQPEDFATYYCQQHYTTPPTFGCGTKVEIKRTV AAPSVFIFPPSDEQLKSGTASVVCLLNNFYPREAKVQWKVDNALQSGNSQESVTE QDSKDSTYSLSSTLTLSKADYEKHKVYACEVTHQGLSSPVTKSFNRGECGGGGSG GGGSGGGGSEPKSSDKTHTCPPCPAPELLGGPSVFLFPPKPKDTLMISRTPEVTC VVVDVSHEDPEVKFNWYVDGVEVHNAKTKPREEQYNSTYRVVSVLTVLHQDWLNG KEYKCKVSNKALPAPIEKTISKAKGQPREPQVYTLPPCREEMTKNQVSLWCLVKG FYPSDIAVEWESNGQPENNYKTTPPVLDSDGSFFLYSKLTVDKSRWQQGNVFSCS | 15 |

| | | | |
|---|---|---|---|
| | | VMHEALHNHYTQKSLSLSPGK | |
| | Polypeptide 3 | APTSSSTKKTQLQLEHLLLDLQMILNGINNYKNPKLTRMLTFKFYMPKKATELKH LQCLEEELKPLEEVLNLAQSKNFHLRPRDLISNINVIVLELKGSETTFMCEYADE TATIVEFLNRWITFCQSIISTLTGGGGSDKTHTCPPCPAPELLGGPSVFLFPPKP KDTLMISRTPEVTCVVVDVSHEDPEVKFNWYVDGVEVHNAKTKPREEQYNSTYRV VSVLTVLHQDWLNGKEYKCKVSNKALPAPIEKTISKAKGQPREPQVCTLPPSREE MTKNQVSLSCAVKGFYPSDIAVEWESNGQPENNYKTTPPVLDSDGSFFLVSKLTV DKSRWQQGNVFSCSVMHEALHNHYTQKSLSLSPGK | 861 |
| | Polypeptide 4 | APTSSSTKKTQLQLEHLLLDLQMILNGINNYKNPKLTRMLTFKFYMPKKATELKH LQCLEEELKPLEEVLNLAQSKNFHLRPRDLISNINVIVLELKGSETTFMCEYADE TATIVEFLNRWITFCQSIISTLTGGGGSDKTHTCPPCPAPELLGGPSVFLFPPKP KDTLMISRTPEVTCVVVDVSHEDPEVKFNWYVDGVEVHNAKTKPREEQYNSTYRV VSVLTVLHQDWLNGKEYKCKVSNKALPAPIEKTISKAKGQPREPQVCTLPPSREE MTKNQVSLSCAVKGFYPSDIAVEWESNGQPENNYKTTPPVLDSDGSFFLVSKLTV DKSRWQQGNVFSCSVMHEALHNHYTQKSLSLSPGK | 861 |
| H08 | Polypeptide 1 | EVQLVESGGGLVQPGGSLRLSCAASGFNIKDTYIHWVRQAPGKCLEWVARIYPTN GYTRYADSVKGRFTISADTSKNTAYLQMNSLRAEDTAVYYCSRWGGDGFYAMDYW GQGTLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVEP KSCDKTHTCPPCPAPELLGGPSVFLFPPKPKDTLMISRTPEVTCVVVDVSHEDPE VKFNWYVDGVEVHNAKTKPREEQYNSTYRVVSVLTVLHQDWLNGKEYKCKVSNKA LPAPIEKTISKAKGQPREPQVYTLPPCREEMTKNQVSLWCLVKGFYPSDIAVEWE SNGQPENNYKTTPPVLDSDGSFFLYSKLTVDKSRWQQGNVFSCSVMHEALHNHYT QKSLSLSPGK | 14 |
| | Polypeptide 2 | DIQMTQSPSSLSASVGDRVTITCRASQDVNTAVAWYQQKPGKAPKLLIYSASFLY SGVPSRFSGSRSGTDFTLTISSLQPEDFATYYCQQHYTTPPTFGCGTKVEIKRTV AAPSVFIFPPSDEQLKSGTASVVCLLNNFYPREAKVQWKVDNALQSGNSQESVTE QDSKDSTYSLSSTLTLSKADYEKHKVYACEVTHQGLSSPVTKSFNRGECGGGGSG GGGSGGGGSEPKSSDKTHTCPPCPAPELLGGPSVFLFPPKPKDTLMISRTPEVTC | 15 |

| | | | |
|---|---|---|---|
| | | VVVDVSHEDPEVKFNWYVDGVEVHNAKTKPREEQYNSTYRVVSVLTVLHQDWLNG KEYKCKVSNKALPAPIEKTISKAKGQPREPQVYTLPPCREEMTKNQVSLWCLVKG FYPSDIAVEWESNGQPENNYKTTPPVLDSDGSFFLYSKLTVDKSRWQQGNVFSCS VMHEALHNHYTQKSLSLSPGK | |
| | Polypeptide 3 | DLGKKLLEAARAGQDDEVRILMANGADVNADDTWGWTPLHLAAYQGHLEIVEVLL KNGADVNAYDYIGWTPLHLAADGHLEIVEVLLKNGADVNASDYIGDTPLHLAAHN GHLEIVEVLLKHGADVNAQDKFGKTAFDISIDNGNEDLAEILQGGGGSDKTHTCP PCPAPELLGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTIS KAKGQPREPQVCTLPPSREEMTKNQVSLSCAVKGFYPSDIAVEWESNGQPENNYK TTPPVLDSDGSFFLVSKLTVDKSRWQQGNVFSCSVMHEALHNIYTQKSLSLSPGK | 862 |
| | Polypeptide 4 | DLGKKLLEAARAGQDDEVRILMANGADVNADDTWGWTPLHLAAYQGHLEIVEVLL KNGADVNAYDYIGWTPLHLAADGHLEIVEVLLKNGADVNASDYIGDTPLHLAAHN GHLEIVEVLLKHGADVNAQDKFGKTAFDISIDNGNEDLAEILQGGGGSDKTHTCP PCPAPELLGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTIS KAKGQPREPQVCTLPPSREEMTKNQVSLSCAVKGFYPSDIAVEWESNGQPENNYK TTPPVLDSDGSFFLVSKLTVDKSRWQQGNVFSCSVMHEALHNHYTQKSLSLSPGK | 862 |
| H09 | Polypeptide 1 | EVQLVESGGGLVQPGGSLRLSCAASGFNIKDTYIHWVRQAPGKCLEWVARIYPTN GYTRYADSVKGRFTISADTSKNTAYLQMNSLRAEDTAVYYCSRWGGDGFYAMDYW GQGTLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVEP KSCDKTHTCPPCPAPELLGGPSVFLFPPKPKDTLMISRTPEVTCVVVDVSHEDPE VKFNWYVDGVEVHNAKTKPREEQYNSTYRVVSVLTVLHQDWLNGKEYKCKVSNKA LPAPIEKTISKAKGQPREPQVYTLPPCREEMTKNQVSLWCLVKGFYPSDIAVEWE SNGQPENNYKTTPPVLDSDGSFFLYSKLTVDKSRWQQGNVFSCSVMHEALHNHYT QKSLSLSPGK | 14 |
| | Polypeptide 2 | DIQMTQSPSSLSASVGDRVTITCRASQDVNTAVAWYQQKPGKAPKLLIYSASFLY SGVPSRFSGSRSGTDFTLTISSLQPEDFATYYCQQHYTTPPTFGCGTKVEIKRTV | 15 |

| | | | |
|---|---|---|---|
| | | AAPSVFIFPPSDEQLKSGTASVVCLLNNFYPREAKVQWKVDNALQSGNSQESVTE QDSKDSTYSLSSTLTLSKADYEKHKVYACEVTHQGLSSPVTKSFNRGECGGGGSG GGGSGGGGSEPKSSDKTHTCPPCPAPELLGGPSVFLFPPKPKDTLMISRTPEVTC VVVDVSHEDPEVKFNWYVDGVEVHNAKTKPREEQYNSTYRVVSVLTVLHQDWLNG KEYKCKVSNKALPAPIEKTISKAKGQPREPQVYTLPPCREEMTKNQVSLWCLVKG FYPSDIAVEWESNGQPENNYKTTPPVLDSDGSFFLYSKLTVDKSRWQQGNVFSCS VMHEALHNHYTQKSLSLSPGK | |
| | Polypeptide 3 | EVQLVESGGGLVQAGGSLRLSCAASGRTFSSYAMGWFRQAPGKEREFVVAINWSS GSTYYADSVKGRFTISRDNAKNTMYLQMNSLKPEDTAVYYCAAGYQINSGNYNFK DYEYDYWGQGTQVTVSSALEGGGGSDKTHTCPPCPAPELLGGPSVFLFPPKPKDT LMISRTPEVTCVVVDVSHEDPEVKFNWYVDGVEVIINAKTKPREEQYNSTYRVVSV LTVLHQDWLNGKEYKCKVSNKALPAPIEKTISKAKGQPREPQVCTLPPSREEMTK NQVSLSCAVKGFYPSDIAVEWESNGQPENNYKTTPPVLDSDGSFFLVSKLTVDKS RWQQGNVFSCSVMHEALHNHYTQKSLSLSPGK | 863 |
| | Polypeptide 4 | EVQLVESGGGLVQAGGSLRLSCAASGRTFSSYAMGWFRQAPGKEREFVVAINWSS GSTYYADSVKGRFTISRDNAKNTMYLQMNSLKPEDTAVYYCAAGYQINSGNYNFK DYEYDYWGQGTQVTVSSALEGGGGSDKTHTCPPCPAPELLGGPSVFLFPPKPKDT LMISRTPEVTCVVVDVSHEDPEVKFNWYVDGVEVHNAKTKPREEQYNSTYRVVSV LTVLHQDWLNGKEYKCKVSNKALPAPIEKTISKAKGQPREPQVCTLPPSREEMTK NQVSLSCAVKGFYPSDIAVEWESNGQPENNYKTTPPVLDSDGSFFLVSKLTVDKS RWQQGNVFSCSVMHEALHNHYTQKSLSLSPGK | 863 |

Table 93 below shows the polynucleotide sequences encoding each polypeptide constituting the novel antibody format.

[Table 93]

| Name | Polypeptide | Sequnece | SEC ID NO |
|---|---|---|---|
| H01 | Polypeptide 1 | GAAGTTCAACTTGTGGAGTCTGGAGGCGGTTTGGTACAACCTGGAGGTTCACTTC GTTTGTCATGTGCTGCCTCTGGGTTTAATATCAAGGATACTTATATTCATTGGGT TCGCCAGGCACCCGGAAAATGCCTGGAATGGGTGGCCCGTATATATCCCACTAAC GGTTACACTCGATATGCTGACTCTGTAAAGGGTCGATTTACTATATCCGCTGATA CTAGCAAAAACACTGCCTACCTCCAGATGAACAGTCTGCGCGCCGAGGATACAGC AGTTTATTATTGTTCCCGATGGGGAGGTGACGGGTTCTATGCCATGGACTACTGG GGTCAAGGGACACTGGTAACCGTTTCTTCTGCTAGCACCAAAGGACCTAGTGTTT TTCCTCTTGCCCCTTCCTCAAAGTCTACCTCTGGCGGGACAGCCGCTCTGGGCTG CCTGGTCAAGGATTATTTCCCAGAGCCTGTCACTGTCAGTTGGAACTCTGGAGCC TTGACTTCTGGTGTTCATACATTTCCTGCTGTCCTTCAAAGCAGCGGCTTGTACT CATTGTCTTCTGTTGTGACAGTACCCTCAAGCAGCCTCGGCACTCAGACATACAT CTGCAATGTCAACCACAAACCCTCAAATACAAAGGTAGATAAAAAAGTCGAACCA AAGTCTTGTGACAAAACTCACACGTGCCCACCGTGCCCAGCACCTGAACTCCTGG GGGGACCGTCAGTCTTCCTCTTCCCCCCAAAACCCAAGGACACCCTCATGATCTC CCGGACCCCTGAGGTCACATGCGTGGTGGTGGACGTGAGCCACGAAGACCCTGAG GTCAAGTTCAACTGGTACGTGGACGGCGTGGAGGTGCATAATGCCAAGACAAAGC CGCGGGAGGAGCAGTACAACAGCACGTACCGTGTGGTCAGCGTCCTCACCGTCCT GCACCAGGACTGGCTGAATGGCAAGGAGTACAAGTGCAAGGTCTCCAACAAAGCC CTCCCAGCCCCCATCGAGAAAACCATCTCCAAAGCCAAAGGGCAGCCCCGAGAAC CACAGGTGTACACCCTGCCCCCATGCCGGGAGGAGATGACCAAGAACCAGGTCAG CCTGTGGTGCCTGGTCAAAGGCTTCTATCCCAGCGACATCGCCGTGGAGTGGGAG AGCAATGGGCAGCCGGAGAACAACTACAAGACCACGCCTCCCGTGCTGGACTCCG ACGGCTCCTTCTTCCTCTACAGCAAGCTCACCGTGGACAAGAGCAGGTGGCAGCA GGGGAACGTCTTCTCATGCTCCGTGATGCATGAGGCTCTGCACAACCACTACACG CAGAAGAGCCTCTCCCTGTCTCCGGGTAAA | 465 |
| | Polypeptide | GACATTCAGATGACCCAATCCCCCTCTAGCCTTTCAGCCTCCGTAGGGGACAGAG | 466 |

| e 2 | TTACAATAACCTGTAGGGCTAGTCAGGACGTTAATACAGCAGTCGCATGGTACCA GCAGAAGCCCGGTAAGGCTCCTAAGCTTTTGATCTACAGTGCTTCTTTCCTGTAT TCTGGTGTACCTAGCCGATTCTCAGGCTCTCGGAGTGGCACAGACTTCACTCTGA CAATTTCAAGTCTCCAGCCAGAAGATTTTGCAACCTACTACTGCCAACAACACTA CACTACTCCTCCAACCTTCGGATGTGGCACAAAAGTAGAGATTAAGCGTACGGTG GCAGCTCCCAGCGTTTTTATCTTTCCCCCATCCGACGAGCAGCTCAAGAGTGGCA CTGCCTCTGTAGTTTGTTTGCTGAATAACTTCTATCCACGTGAAGCAAAAGTACA GTGGAAGGTCGATAATGCCCTTCAGAGCGGTAACAGCCAAGAAAGTGTTACCGAG CAAGATTCCAAAGATTCCACTTACAGTCTGTCCAGCACATTGACACTGAGTAAGG CTGATTACGAAAAACACAAGGTGTACGCATGCGAGGTGACACACCAAGGTCTTTC ATCTCCTGTAACTAAGAGCTTTAACCGGGGAGAATGTGGTGGTGGGGGCAGCGGG GGCGGAGGTAGTGGAGGCGGCGGTAGTGAACCAAAGAGTAGTGACAAAACTCACA CGTGCCCACCGTGCCCAGCACCTGAACTCCTGGGGGGACCGTCAGTCTTCCTCTT CCCCCCAAAAACCCAAGGACACCCTCATGATCTCCCGGACCCCTGAGGTCACATGC GTGGTGGTGGACGTGAGCCACGAAGACCCTGAGGTCAAGTTCAACTGGTACGTGG ACGGCGTGGAGGTGCATAATGCCAAGACAAAGCCGCGGGAGGAGCAGTACAACAG CACGTACCGTGTGGTCAGCGTCCTCACCGTCCTGCACCAGGACTGGCTGAATGGC AAGGAGTACAAGTGCAAGGTCTCCAACAAAGCCCTCCCAGCCCCCATCGAGAAAA CCATCTCCAAAGCCAAAGGGCAGCCCCGAGAACCACAGGTGTACACCCTGCCCCC ATGCCGGGAGGAGATGACCAAGAACCAGGTCAGCCTGTGGTGCCTGGTCAAAGGC TTCTATCCCAGCGACATCGCCGTGGAGTGGGAGAGCAATGGGCAGCCGGAGAACA ACTACAAGACCACGCCTCCCGTGCTGGACTCCGACGGCTCCTTCTTCCTCTACAG CAAGCTCACCGTGGACAAGAGCAGGTGGCAGCAGGGGAACGTCTTCTCATGCTCC GTGATGCATGAGGCTCTGCACAACCACTACACGCAGAAGAGCCTCTCCCTGTCTC CGGGTAAA | |
| Polypeptide 3 | GACAAAACTCACACATGCCCACCGTGCCCAGCACCTGAACTCCTGGGGGGACCGT CAGTCTTCCTCTTCCCCCCAAAAACCCAAGGACACCCTCATGATCTCCCGGACCCC TGAGGTCACATGCGTGGTGGTGGACGTGAGCCACGAAGACCCTGAGGTCAAGTTC AACTGGTACGTGGACGGCGTGGAGGTGCATAATGCCAAGACAAAGCCGCGGGAGG | 458 |

| | | | |
|---|---|---|---|
| | | AGCAGTACAACAGCACGTACCGTGTGGTCAGCGTCCTCACCGTCCTGCACCAGGA CTGGCTGAATGGCAAGGAGTACAAGTGCAAGGTCTCCAACAAAGCCCTCCCAGCC CCCATCGAGAAAACCATCTCCAAAGCCAAAGGGCAGCCCCGAGAACCACAGGTGT GCACCCTGCCCCCATCCCGGGAGGAGATGACCAAGAACCAGGTCAGCCTGAGCTG CGCGGTCAAAGGCTTCTATCCCAGCGACATCGCCGTGGAGTGGGAGAGCAATGGG CAGCCGGAGAACAACTACAAGACCACGCCTCCCGTGCTGGACTCCGACGGCTCCT TCTTCCTCGTGAGCAAGCTCACCGTGGACAAGAGCAGGTGGCAGCAGGGGAACGT CTTCTCATGCTCCGTGATGCATGAGGCTCTGCACAACCACTACACGCAGAAGAGC CTCTCCCTGTCTCCGGGTAAA | |
| | Polypeptide 4 | GACAAAACTCACACATGCCCACCGTGCCCAGCACCTGAACTCCTGGGGGGACCGT CAGTCTTCCTCTTCCCCCCAAAACCCAAGGACACCCTCATGATCTCCCGGACCCC TGAGGTCACATGCGTGGTGGTGGACGTGAGCCACGAAGACCCTGAGGTCAAGTTC AACTGGTACGTGGACGGCGTGGAGGTGCATAATGCCAAGACAAAGCCGCGGGAGG AGCAGTACAACAGCACGTACCGTGTGGTCAGCGTCCTCACCGTCCTGCACCAGGA CTGGCTGAATGGCAAGGAGTACAAGTGCAAGGTCTCCAACAAAGCCCTCCCAGCC CCCATCGAGAAAACCATCTCCAAAGCCAAAGGGCAGCCCCGAGAACCACAGGTGT GCACCCTGCCCCCATCCCGGGAGGAGATGACCAAGAACCAGGTCAGCCTGAGCTG CGCGGTCAAAGGCTTCTATCCCAGCGACATCGCCGTGGAGTGGGAGAGCAATGGG CAGCCGGAGAACAACTACAAGACCACGCCTCCCGTGCTGGACTCCGACGGCTCCT TCTTCCTCGTGAGCAAGCTCACCGTGGACAAGAGCAGGTGGCAGCAGGGGAACGT CTTCTCATGCTCCGTGATGCATGAGGCTCTGCACAACCACTACACGCAGAAGAGC CTCTCCCTGTCTCCGGGTAAA | 458 |
| H04 | Polypeptide 1 | GAAGTTCAACTTGTGGAGTCTGGAGGCGGTTTGGTACAACCTGGAGGTTCACTTC GTTTGTCATGTGCTGCCTCTGGGTTTAATATCAAGGATACTTATATTCATTGGGT TCGCCAGGCACCCGGAAAATGCCTGGAATGGGTGGCCCGTATATATCCCACTAAC GGTTACACTCGATATGCTGACTCTGTAAAGGGTCGATTTACTATATCCGCTGATA CTAGCAAAAACACTGCCTACCTCCAGATGAACAGTCTGCGCGCCGAGGATACAGC AGTTTATTATTGTTCCCGATGGGGAGGTGACGGGTTCTATGCCATGGACTACTGG GGTCAAGGGACACTGGTAACCGTTTCTTCTGCTAGCACCAAAGGACCTAGTGTTT | 465 |

| | | | |
|---|---|---|---|
| | | TTCCTCTTGCCCCCTTCCTCAAAGTCTACCTCTGGGGGGACAGCCGCTCTGGGCTG CCTGGTCAAGGATTATTTCCCAGAGCCTGTCACTGTCAGTTGGAACTCTGGAGCC TTGACTTCTGGTGTTCATACATTTCCTGCTGTCCTTCAAAGCAGCGGCTTGTACT CATTGTCTTCTGTTGTGACAGTACCCTCAAGCAGCCTCGGCACTCAGACATACAT CTGCAATGTCAACCACAAACCCTCAAATACAAAGGTAGATAAAAAAGTCGAACCA AAGTCTTGTGACAAAACTCACACGTGCCCACCGTGCCCAGCACCTGAACTCCTGG GGGGACCGTCAGTCTTCCTCTTCCCCCCAAAACCCAAGGACACCCTCATGATCTC CCGGACCCCTGAGGTCACATGCGTGGTGGTGGACGTGAGCCACGAAGACCCTGAG GTCAAGTTCAACTGGTACGTGGACGGCGTGGAGGTGCATAATGCCAAGACAAAGC CGCGGGAGGAGCAGTACAACAGCACGTACCGTGTGGTCAGCGTCCTCACCGTCCT GCACCAGGACTGGCTGAATGGCAAGGAGTACAAGTGCAAGGTCTCCAACAAAGCC CTCCCAGCCCCCATCGAGAAAACCATCTCCAAAGCCAAAGGGCAGCCCCGAGAAC CACAGGTGTACACCCTGCCCCCATGCCGGGAGGAGATGACCAAGAACCAGGTCAG CCTGTGGTGCCTGGTCAAAGGCTTCTATCCCAGCGACATCGCCGTGGAGTGGGAG AGCAATGGGCAGCCGGAGAACAACTACAAGACCACGCCTCCCGTGCTGGACTCCG ACGGCTCCTTCTTCCTCTACAGCAAGCTCACCGTGGACAAGAGCAGGTGGCAGCA GGGGAACGTCTTCTCATGCTCCGTGATGCATGAGGCTCTGCACAACCACTACACG CAGAAGAGCCTCTCCCTGTCTCCGGGTAAA | |
| | Polypeptide 2 | GACATTCAGATGACCCAATCCCCCTCTAGCCTTTCAGCCTCCGTAGGGGACAGAG TTACAATAACCTGTAGGGCTAGTCAGGACGTTAATACAGCAGTCGCATGGTACCA GCAGAAGCCCGGTAAGGCTCCTAAGCTTTTGATCTACAGTGCTTCTTTCCTGTAT TCTGGTGTACCTAGCCGATTCTCAGGCTCTCGGAGTGGCACAGACTTCACTCTGA CAATTTCAAGTCTCCAGCCAGAAGATTTTGCAACCTACTACTGCCAACAACACTA CACTACTCCTCCAACCTTCGGATGTGGCACAAAAGTAGAGATTAAGCGTACGGTG GCAGCTCCCAGCGTTTTTATCTTTCCCCCATCCGACGAGCAGCTCAAGAGTGGCA CTGCCTCTGTAGTTTGTTTGCTGAATAACTTCTATCCACGTGAAGCAAAAGTACA GTGGAAGGTCGATAATGCCCTTCAGAGCGGTAACAGCCAAGAAAGTGTTACCGAG CAAGATTCCAAAGATTCCACTTACAGTCTGTCCAGCACATTGACACTGAGTAAGG CTGATTACGAAAAACACAAGGTGTACGCATGCGAGGTGACACACCAAGGTCTTTC | 466 |

| | | ATCTCCTGTAACTAAGAGCTTTAACCGGGGAGAATGTGGTGGTGGGGGCAGCGGG GGCGGAGGTAGTGGAGGCGGCGGTAGTGAACCAAAGAGTAGTGACAAAACTCACA CGTGCCCACCGTGCCCAGCACCTGAACTCCTGGGGGGACCGTCAGTCTTCCTCTT CCCCCCAAAACCCAAGGACACCCTCATGATCTCCCGGACCCCTGAGGTCACATGC GTGGTGGTGGACGTGAGCCACGAAGACCCTGAGGTCAAGTTCAACTGGTACGTGG ACGGCGTGGAGGTGCATAATGCCAAGACAAAGCCGCGGGAGGAGCAGTACAACAG CACGTACCGTGTGGTCAGCGTCCTCACCGTCCTGCACCAGGACTGGCTGAATGGC AAGGAGTACAAGTGCAAGGTCTCCAACAAAGCCCTCCCAGCCCCCATCGAGAAAA CCATCTCCAAAGCCAAAGGGCAGCCCCGAGAACCACAGGTGTACACCCTGCCCCC ATGCCGGGAGGAGATGACCAAGAACCAGGTCAGCCTGTGGTGCCTGGTCAAAGGC TTCTATCCCAGCGACATCGCCGTGGAGTGGGAGAGCAATGGGCAGCCGGAGAACA ACTACAAGACCACGCCTCCCGTGCTGGACTCCGACGGCTCCTTCTTCCTCTACAG CAAGCTCACCGTGGACAAGAGCAGGTGGCAGCAGGGGAACGTCTTCTCATGCTCC GTGATGCATGAGGCTCTGCACAACCACTACACGCAGAAGAGCCTCTCCCTGTCTC CGGGTAAA | |
| Polypeptide 3 | AGCGATACAGGGAGACCTTTCGTGGAGATGTACTCCGAAATACCAGAGATCATAC ACATGACCGAGGGTAGAGAGCTCGTTATACCCTGTCGGGTAACCTCCCCAAACAT AACAGTGACTCTCAAAAAATTCCCACTCGATACCCTTATACCAGACGGAAAGCGG ATAATATGGGACTCACGAAAGGGTTTCATTATTTCTAATGCAACTTATAAAGAAA TCGGATTGCTTACATGCGAAGCCACAGTGAACGGTCATTTGTATAAGACCAATTA TCTTACCCACCGTCAAACCAATACAATCATTGACGTGGTCCTGTCCCCCTCTCAC GGTATCGAGTTGAGTGTGGGAGAAAAGTTGGTACTCAACTGTACCGCCCGTACTG AACTCAACGTAGGGATAGATTTTAACTGGGAATATCCTAGCAGTAAACACCAGCA CAAGAAACTTGTTAATCGAGACTTGAAAACCCAATCAGGCTCTGAAATGAAAAAG TTCCTCTCCACTTTGACCATCGATGGAGTGACACGCTCCGATCAAGGGTTGTACA CTTGTGCCGCCAGCTCCGGTTTGATGACTAAAAAAAAATTCTACCTTTGTACGTGT CCACGAAAAAGGAGGCGGCGGCTCTGACAAAACTCACACGTGCCCACCGTGCCCA GCACCTGAACTCCTGGGGGGACCGTCAGTCTTCCTCTTCCCCCCAAAACCCAAGG ACACCCTCATGATCTCCCGGACCCCTGAGGTCACATGCGTGGTGGTGGACGTGAG | 864 |

| | | | |
|---|---|---|---|
| | | CCACGAAGACCCTGAGGTCAAGTTCAACTGGTACGTGGACGGCGTGGAGGTGCAT AATGCCAAGACAAAGCCGCGGGAGGAGCAGTACAACAGCACGTACCGTGTGGTCA GCGTCCTCACCGTCCTGCACCAGGACTGGCTGAATGGCAAGGAGTACAAGTGCAA GGTCTCCAACAAAGCCCTCCCAGCCCCCATCGAGAAAACCATCTCCAAAGCCAAA GGGCAGCCCCGAGAACCACAGGTGTGCACCCTGCCCCCATCCCGGGAGGAGATGA CCAAGAACCAGGTCAGCCTGAGCTGCGCGGTCAAAGGCTTCTATCCCAGCGACAT CGCCGTGGAGTGGGAGAGCAATGGGCAGCCGGAGAACAACTACAAGACCACGCCT CCCGTGCTGGACTCCGACGGCTCCTTCTTCCTCGTGAGCAAGCTCACCGTGGACA AGAGCAGGTGGCAGCAGGGGAACGTCTTCTCATGCTCCGTGATGCATGAGGCTCT GCACAACCACTACACGCAGAAGAGCCTCTCCCTGTCTCCGGGTAAA | |
| | Polypeptid e 4 | AGCGATACAGGGAGACCTTTCGTGGAGATGTACTCCGAAATACCAGAGATCATAC ACATGACCGAGGGTAGAGAGCTCGTTATACCCTGTCGGGTAACCTCCCCAAACAT AACAGTGACTCTCAAAAAATTCCCACTCGATACCCTTATACCAGACGGAAAGCGG ATAATATGGGACTCACGAAAGGGTTTCATTATTTCTAATGCAACTTATAAAGAAA TCGGATTGCTTACATGCGAAGCCACAGTGAACGGTCATTTGTATAAGACCAATTA TCTTACCCACCGTCAAACCAATACAATCATTGACGTGGTCCTGTCCCCCTCTCAC GGTATCGAGTTGAGTGTGGGAGAAAAGTTGGTACTCAACTGTACCGCCCGTACTG AACTCAACGTAGGGATAGATTTTAACTGGGAATATCCTAGCAGTAAACACCAGCA CAAGAAACTTGTTAATCGAGACTTGAAAACCCAATCAGGCTCTGAAATGAAAAAG TTCCTCTCCACTTTGACCATCGATGGAGTGACACGCTCCGATCAAGGGTTGTACA CTTGTGCCGCCAGCTCCGGTTTGATGACTAAAAAAAAATTCTACCTTTGTACGTGT CCACGAAAAAGGAGGCGGCGGCTCTGACAAAACTCACACGTGCCCACCGTGCCCA GCACCTGAACTCCTGGGGGGACCGTCAGTCTTCCTCTTCCCCCCCAAAACCCAAGG ACACCCTCATGATCTCCCGGACCCCTGAGGTCACATGCGTGGTGGTGGACGTGAG CCACGAAGACCCTGAGGTCAAGTTCAACTGGTACGTGGACGGCGTGGAGGTGCAT AATGCCAAGACAAAGCCGCGGGAGGAGCAGTACAACAGCACGTACCGTGTGGTCA GCGTCCTCACCGTCCTGCACCAGGACTGGCTGAATGGCAAGGAGTACAAGTGCAA GGTCTCCAACAAAGCCCTCCCAGCCCCCATCGAGAAAACCATCTCCAAAGCCAAA GGGCAGCCCCGAGAACCACAGGTGTGCACCCTGCCCCCATCCCGGGAGGAGATGA | 864 |

| | | | |
|---|---|---|---|
| | | CCAAGAACCAGGTCAGCCTGAGCTGCGCGGTCAAAGGCTTCTATCCCAGCGACAT CGCCGTGGAGTGGGAGAGCAATGGGCAGCCGGAGAACAACTACAAGACCACGCCT CCCGTGCTGGACTCCGACGGCTCCTTCTTCCTCGTGAGCAAGCTCACCGTGGACA AGAGCAGGTGGCAGCAGGGGAACGTCTTCTCATGCTCCGTGATGCATGAGGCTCT GCACAACCACTACACGCAGAAGAGCCTCTCCCTGTCTCCGGGTAAA | |
| H05 | Polypeptide 1 | GAAGTTCAACTTGTGGAGTCTGGAGGCGGTTTGGTACAACCTGGAGGTTCACTTC GTTTGTCATGTGCTGCCTCTGGGTTTAATATCAAGGATACTTATATTCATTGGGT TCGCCAGGCACCCGGAAAATGCCTGGAATGGGTGGCCCGTATATATCCCACTAAC GGTTACACTCGATATGCTGACTCTGTAAAGGGTCGATTTACTATATCCGCTGATA CTAGCAAAAACACTGCCTACCTCCAGATGAACAGTCTGCGCGCCGAGGATACAGC AGTTTATTATTGTTCCCGATGGGGAGGTGACGGGTTCTATGCCATGGACTACTGG GGTCAAGGGACACTGGTAACCGTTTCTTCTGCTAGCACCAAAGGACCTAGTGTTT TTCCTCTTGCCCCTTCCTCAAAGTCTACCTCTGGGGGGACAGCCGCTCTGGGCTG CCTGGTCAAGGATTATTTCCCAGAGCCTGTCACTGTCAGTTGGAACTCTGGAGCC TTGACTTCTGGTGTTCATACATTTCCTGCTGTCCTTCAAAGCAGCGGCTTGTACT CATTGTCTTCTGTTGTGACAGTACCCTCAAGCAGCCTCGGCACTCAGACATACAT CTGCAATGTCAACCACAAACCCTCAAATACAAAGGTAGATAAAAAAGTCGAACCA AAGTCTTGTGACAAAACTCACACGTGCCCACCGTGCCCAGCACCTGAACTCCTGG GGGGACCGTCAGTCTTCCTCTTCCCCCCCAAAAACCCAAGGACACCCTCATGATCTC CCGGACCCCTGAGGTCACATGCGTGGTGGTGGACGTGAGCCACGAAGACCCTGAG GTCAAGTTCAACTGGTACGTGGACGGCGTGGAGGTGCATAATGCCAAGACAAAGC CGCGGGAGGAGCAGTACAACAGCACGTACCGTGTGGTCAGCGTCCTCACCGTCCT GCACCAGGACTGGCTGAATGGCAAGGAGTACAAGTGCAAGGTCTCCAACAAAGCC CTCCCAGCCCCCATCGAGAAAACCATCTCCAAAGCCAAAGGGCAGCCCCGAGAAC CACAGGTGTACACCCTGCCCCCATGCCGGGAGGAGATGACCAAGAACCAGGTCAG CCTGTGGTGCCTGGTCAAAGGCTTCTATCCCAGCGACATCGCCGTGGAGTGGGAG AGCAATGGGCAGCCGGAGAACAACTACAAGACCACGCCTCCCGTGCTGGACTCCG ACGGCTCCTTCTTCCTCTACAGCAAGCTCACCGTGGACAAGAGCAGGTGGCAGCA GGGGAACGTCTTCTCATGCTCCGTGATGCATGAGGCTCTGCACAACCACTACACG | 465 |

| | | CAGAAGAGCCTCTCCCTGTCTCCGGGTAAA | |
|---|---|---|---|
| | Polypeptide 2 | GACATTCAGATGACCCAATCCCCCTCTAGCCTTTCAGCCTCCGTAGGGGACAGAG TTACAATAACCTGTAGGGCTAGTCAGGACGTTAATACAGCAGTCGCATGGTACCA GCAGAAGCCCGGTAAGGCTCCTAAGCTTTTGATCTACAGTGCTTCTTTCCTGTAT TCTGGTGTACCTAGCCGATTCTCAGGCTCTCGGAGTGGCACAGACTTCACTCTGA CAATTTCAAGTCTCCAGCCAGAAGATTTTGCAACCTACTACTGCCAACAACACTA CACTACTCCTCCAACCTTCGGATGTGGCACAAAAGTAGAGATTAAGCGTACGGTG GCAGCTCCCAGCGTTTTTATCTTTCCCCCATCCGACGAGCAGCTCAAGAGTGGCA CTGCCTCTGTAGTTTGTTTGCTGAATAACTTCTATCCACGTGAAGCAAAAGTACA GTGGAAGGTCGATAATGCCCTTCAGAGCGGTAACAGCCAAGAAAGTGTTACCGAG CAAGATTCCAAAGATTCCACTTACAGTCTGTCCAGCACATTGACACTGAGTAAGG CTGATTACGAAAAACACAAGGTGTACGCATGCGAGGTGACACACCAAGGTCTTTC ATCTCCTGTAACTAAGAGCTTTAACCGGGGAGAATGTGGTGGTGGGGGCAGCGGG GGCGGAGGTAGTGGAGGCGGCGGTAGTGAACCAAAGAGTAGTGACAAAACTCACA CGTGCCCACCGTGCCCAGCACCTGAACTCCTGGGGGGACCGTCAGTCTTCCTCTT CCCCCCAAAACCCAAGGACACCCTCATGATCTCCCGGACCCCTGAGGTCACATGC GTGGTGGTGGACGTGAGCCACGAAGACCCTGAGGTCAAGTTCAACTGGTACGTGG ACGGCGTGGAGGTGCATAATGCCAAGACAAAGCCGCGGGAGGAGCAGTACAACAG CACGTACCGTGTGGTCAGCGTCCTCACCGTCCTGCACCAGGACTGGCTGAATGGC AAGGAGTACAAGTGCAAGGTCTCCAACAAAGCCCTCCCAGCCCCCATCGAGAAAA CCATCTCCAAAGCCAAAGGGCAGCCCCGAGAACCACAGGTGTACACCCTGCCCCC ATGCCGGGAGGAGATGACCAAGAACCAGGTCAGCCTGTGGTGCCTGGTCAAAGGC TTCTATCCCAGCGACATCGCCGTGGAGTGGGAGAGCAATGGGCAGCCGGAGAACA ACTACAAGACCACGCCTCCCGTGCTGGACTCCGACGGCTCCTTCTTCCTCTACAG CAAGCTCACCGTGGACAAGAGCAGGTGGCAGCAGGGGAACGTCTTCTCATGCTCC GTGATGCATGAGGCTCTGCACAACCACTACACGCAGAAGAGCCTCTCCCTGTCTC CGGGTAAA | 466 |
| | Polypeptide 3 | CAAAGGCCAACTGGCGGACCCGGATGCGGTCCTGGCAGGCTTCTGCTCGGGACCG GTACTGATGCCCGTTGTTGCCGTGTACATACAACCCGTTGTTGTAGAGACTACCC | 865 |

| | | AGGCGAGGAATGTTGTAGTGAATGGGACTGCATGTGTGTTCAGCCTGAGTTTCAT TGCGGAGATCCATGCTGCACAACTTGTCGTCATCACCCCTGCCCACCTGGGCAAG GAGTACAGAGCCAGGGTAAATTTTCATTCGGCTTTCAGTGCATTGATTGCGCATC TGGAACATTCTCCGGGGGGTCACGAGGGCCATTGCAAACCATGGACTGATTGTACA CAATTCGGTTTCCTGACAGTCTTTCCAGGGAACAAAACACACAATGCTGTCTGCG TGCCTGGAAGCCCACCTGCTGAACCTGGAGGCGGCGGCTCTGACAAAACTCACAC GTGCCCACCGTGCCCAGCACCTGAACTCCTGGGGGGACCGTCAGTCTTCCTCTTC CCCCCAAAACCCAAGGACACCCTCATGATCTCCCGGACCCCTGAGGTCACATGCG TGGTGGTGGACGTGAGCCACGAAGACCCTGAGGTCAAGTTCAACTGGTACGTGGA CGGCGTGGAGGTGCATAATGCCAAGACAAAGCCGCGGGAGGAGCAGTACAACAGC ACGTACCGTGTGGTCAGCGTCCTCACCGTCCTGCACCAGGACTGGCTGAATGGCA AGGAGTACAAGTGCAAGGTCTCCAACAAAGCCCTCCCAGCCCCCATCGAGAAAAC CATCTCCAAAGCCAAAGGGCAGCCCCGAGAACCACAGGTGTGCACCCTGCCCCCA TCCCGGGAGGAGATGACCAAGAACCAGGTCAGCCTGAGCTGCGCGGTCAAAGGCT TCTATCCCAGCGACATCGCCGTGGAGTGGGAGAGCAATGGGCAGCCGGAGAACAA CTACAAGACCACGCCTCCCGTGCTGGACTCCGACGGCTCCTTCTTCCTCGTGAGC AAGCTCACCGTGGACAAGAGCAGGTGGCAGCAGGGGAACGTCTTCTCATGCTCCG TGATGCATGAGGCTCTGCACAACCACTACACGCAGAAGAGCCTCTCCCTGTCTCC GGGTAAA | |
| | Polypeptide 4 | CAAAGGCCAACTGGCGGACCCGGATGCGGTCCTGGCAGGCTTCTGCTCGGGACCG GTACTGATGCCCGTTGTTGCCGTGTACATACAACCCGTTGTTGTAGAGACTACCC AGGCGAGGAATGTTGTAGTGAATGGGACTGCATGTGTGTTCAGCCTGAGTTTCAT TGCGGAGATCCATGCTGCACAACTTGTCGTCATCACCCCTGCCCACCTGGGCAAG GAGTACAGAGCCAGGGTAAATTTTCATTCGGCTTTCAGTGCATTGATTGCGCATC TGGAACATTCTCCGGGGGGTCACGAGGGCCATTGCAAACCATGGACTGATTGTACA CAATTCGGTTTCCTGACAGTCTTTCCAGGGAACAAAACACACAATGCTGTCTGCG TGCCTGGAAGCCCACCTGCTGAACCTGGAGGCGGCGGCTCTGACAAAACTCACAC GTGCCCACCGTGCCCAGCACCTGAACTCCTGGGGGGGACCGTCAGTCTTCCTCTTC CCCCCAAAACCCAAGGACACCCTCATGATCTCCCGGACCCCTGAGGTCACATGCG | 865 |

| | | | | |
|---|---|---|---|---|
| | | | TGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTGCACCCTGCCCCCATCCCGGGGAGGAGATGACCAAGAACCAGGTCAGCCTGAGCTGCGCGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCGTGAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | |
| H06 | Polypeptide 1 | GAAGTTCAACTTGTGGAGTCTGGAGGCGGTTTGGTACAACCTGGAGGTTCACTTCGTTTGTCATGTGCTGCCTCTGGGTTTAATATCAAGGATACTTATATTCATTGGGTTCGCCAGGCACCCGGAAAATGCCTGGAATGGGTGGCCCGTATATATCCCACTAACGGTTACACTCGATATGCTGACTCTGTAAAGGGTCGATTTACTATATCCGCTGATACTAGCAAAAACACTGCCTACCTCCAGATGAACAGTCTGCGCGCCGAGGATACAGCAGTTTATTATTGTTCCCGATGGGGGAGGTGACGGGTTCTATGCCATGGACTACTGGGGTCAAGGGACACTGGTAACCGTTTCTTCTGCTAGCACCAAAGGACCTAGTGTTTTTCCTCTTGCCCCTTCCTCAAAGTCTACCTCTGGGGGGACAGCCGCTCTGGGCTGCCTGGTCAAGGATTATTTCCCAGAGCCTGTCACTGTCAGTTGGAACTCTGGAGCCTTGACTTCTGGTGTTCATACATTTCCTGCTGTCCTTCAAAGCAGCGGCTTGTACTCATTGTCTTCTGTTGTGACAGTACCCTCAAGCAGCCTCGGCACTCAGACATACATCTGCAATGTCAACCACAAACCCTCAAATACAAAGGTAGATAAAAAAGTCGAACCAAAGTCTTGTGACAAAACTCACACGTGCCCACCGTGCCCAGCACCTGAACTCCTGGGGGGACCGTCAGTCTTCCTCTTCCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCC | 465 |

| | | CTCCCAGCCCCCATCGAGAAAACCATCTCCAAAGCCAAAGGGCAGCCCCGAGAAC CACAGGTGTACACCCTGCCCCCATGCCGGGAGGAGATGACCAAGAACCAGGTCAG CCTGTGGTGCCTGGTCAAAGGCTTCTATCCCAGCGACATCGCCGTGGAGTGGGAG AGCAATGGGCAGCCGGAGAACAACTACAAGACCACGCCTCCCGTGCTGGACTCCG ACGGCTCCTTCTTCCTCTACAGCAAGCTCACCGTGGACAAGAGCAGGTGGCAGCA GGGGAACGTCTTCTCATGCTCCGTGATGCATGAGGCTCTGCACAACCACTACACG CAGAAGAGCCTCTCCCTGTCTCCGGGTAAA | |
|---|---|---|---|
| | Polypeptid e 2 | GACATTCAGATGACCCAATCCCCCTCTAGCCTTTCAGCCTCCGTAGGGGACAGAG TTACAATAACCTGTAGGGCTAGTCAGGACGTTAATACAGCAGTCGCATGGTACCA GCAGAAGCCCGGTAAGGCTCCTAAGCTTTTGATCTACAGTGCTTCTTTCCTGTAT TCTGGTGTACCTAGCCGATTCTCAGGCTCTCGGAGTGGCACAGACTTCACTCTGA CAATTTCAAGTCTCCAGCCAGAAGATTTTGCAACCTACTACTGCCAACAACACTA CACTACTCCTCCAACCTTCGGATGTGGCACAAAAGTAGAGATTAAGCGTACGGTG GCAGCTCCCAGCGTTTTTATCTTTCCCCCATCCGACGAGCAGCTCAAGAGTGGCA CTGCCTCTGTAGTTTGTTTGCTGAATAACTTCTATCCACGTGAAGCAAAAGTACA GTGGAAGGTCGATAATGCCCTTCAGAGCGGTAACAGCCAAGAAAGTGTTACCGAG CAAGATTCCAAAGATTCCACTTACAGTCTGTCCAGCACATTGACACTGAGTAAGG CTGATTACGAAAAACACAAGGTGTACGCATGCGAGGTGACACACCAAGGTCTTTC ATCTCCTGTAACTAAGAGCTTTAACCGGGGAGAATGTGGTGGTGGGGGCAGCGGG GGCGGAGGTAGTGGAGGCGGCGGTAGTGAACCAAAGAGTAGTGACAAAACTCACA CGTGCCCACCGTGCCCAGCACCTGAACTCCTGGGGGGACCGTCAGTCTTCCTCTT CCCCCCAAAACCCAAGGACACCCTCATGATCTCCCGGACCCCTGAGGTCACATGC GTGGTGGTGGACGTGAGCCACGAAGACCCTGAGGTCAAGTTCAACTGGTACGTGG ACGGCGTGGAGGTGCATAATGCCAAGACAAAGCCGCGGGAGGAGCAGTACAACAG CACGTACCGTGTGGTCAGCGTCCTCACCGTCCTGCACCAGGACTGGCTGAATGGC AAGGAGTACAAGTGCAAGGTCTCCAACAAAGCCCTCCCAGCCCCCATCGAGAAAA CCATCTCCAAAGCCAAAGGGCAGCCCCGAGAACCACAGGTGTACACCCTGCCCCC ATGCCGGGAGGAGATGACCAAGAACCAGGTCAGCCTGTGGTGCCTGGTCAAAGGC TTCTATCCCAGCGACATCGCCGTGGAGTGGGAGAGCAATGGGCAGCCGGAGAACA | 466 |

| | | | |
|---|---|---|---|
| | | ACTACAAGACCACGCCTCCCGTGCTGGACTCCGACGGCTCCTTCTTCCTCTACAG CAAGCTCACCGTGGACAAGAGCAGGTGGCAGCAGGGGAACGTCTTCTCATGCTCC GTGATGCATGAGGCTCTGCACAACCACTACACGCAGAAGAGCCTCTCCCTGTCTC CGGGTAAA | |
| | Polypeptide 3 | AGGCCAAGCGGACGCAAGTCAAGCAAGATGCAGGCCTTCAGAATTTGGGATGTGA ACCAAAAGACTTTTTATCTCCGAAATAACCAACTCGTGGCTGGCTACTTGCAAGG ACCTAACGTCAACTTGGAGGAGAAAATCGACGTTGTGCCTATTGAACCCCATGCT CTCTTTCTGGGTATACACGGGGGGAAGATGTGTCTGTCTTGTGTTAAGTCCGGTG ATGAGACACGCTTGCAACTGGAGGCAGTGAATATAACAGACTTGAGTGAGAATAG AAAACAGGATAAGCGGTTCGCTTTCATCAGATCAGATTCTGGACCAACAACCTCT TTTGAGAGCGCAGCTTGTCCTGGCTGGTTCTTGTGCACCGCTATGGAAGCAGACC AACCCGTCTCCCTTACAAACATGCCAGACGAGGGAGTAATGGTCACAAAGTTCTA TTTTCAGGAGGATGAGGGAGGCGGCGGCTCTGACAAAACTCACACGTGCCCACCG TGCCCAGCACCTGAACTCCTGGGGGGACCGTCAGTCTTCCTCTTCCCCCCCAAAAC CCAAGGACACCCTCATGATCTCCCGGACCCCTGAGGTCACATGCGTGGTGGTGGA CGTGAGCCACGAAGACCCTGAGGTCAAGTTCAACTGGTACGTGGACGGCGTGGAG GTGCATAATGCCAAGACAAAGCCGCGGGAGGAGCAGTACAACAGCACGTACCGTG TGGTCAGCGTCCTCACCGTCCTGCACCAGGACTGGCTGAATGGCAAGGAGTACAA GTGCAAGGTCTCCAACAAAGCCCTCCCAGCCCCCATCGAGAAAACCATCTCCAAA GCCAAAGGGCAGCCCCGAGAACCACAGGTGTGCACCCTGCCCCCCATCCCGGGAGG AGATGACCAAGAACCAGGTCAGCCTGAGCTGCGCGGTCAAAGGCTTCTATCCCAG CGACATCGCCGTGGAGTGGGAGAGCAATGGGCAGCCGGAGAACAACTACAAGACC ACGCCTCCCGTGCTGGACTCCGACGGCTCCTTCTTCCTCGTGAGCAAGCTCACCG TGGACAAGAGCAGGTGGCAGCAGGGGAACGTCTTCTCATGCTCCGTGATGCATGA GGCTCTGCACAACCACTACACGCAGAAGAGCCTCTCCCTGTCTCCGGGTAAA | 866 |
| | Polypeptide 4 | AGGCCAAGCGGACGCAAGTCAAGCAAGATGCAGGCCTTCAGAATTTGGGATGTGA ACCAAAAGACTTTTTATCTCCGAAATAACCAACTCGTGGCTGGCTACTTGCAAGG ACCTAACGTCAACTTGGAGGAGAAAATCGACGTTGTGCCTATTGAACCCCATGCT CTCTTTCTGGGTATACACGGGGGGAAGATGTGTCTGTCTTGTGTTAAGTCCGGTG | 866 |

| | | | |
|---|---|---|---|
| | | ATGAGACACGCTTGCAACTGGAGGCAGTGAATATAACAGACTTGAGTGAGAATAG AAAACAGGATAAGCGGTTCGCTTTCATCAGATCAGATTCTGGACCAACAACCTCT TTTGAGAGCGCAGCTTGTCCTGGCTGGTTCTTGTGCACCGCTATGGAAGCAGACC AACCCGTCTCCCTTACAAACATGCCAGACGAGGGAGTAATGGTCACAAAGTTCTA TTTTCAGGAGGATGAGGGAGGCGGCGGCTCTGACAAAACTCACACGTGCCCACCG TGCCCAGCACCTGAACTCCTGGGGGGACCGTCAGTCTTCCTCTTCCCCCCAAAAC CCAAGGACACCCTCATGATCTCCCGGACCCCTGAGGTCACATGCGTGGTGGTGGA CGTGAGCCACGAAGACCCTGAGGTCAAGTTCAACTGGTACGTGGACGGCGTGGAG GTGCATAATGCCAAGACAAAGCCGCGGGAGGAGCAGTACAACAGCACGTACCGTG TGGTCAGCGTCCTCACCGTCCTGCACCAGGACTGGCTGAATGGCAAGGAGTACAA GTGCAAGGTCTCCAACAAAGCCCTCCCAGCCCCCATCGAGAAAACCATCTCCAAA GCCAAAGGGCAGCCCCGAGAACCACAGGTGTGCACCCTGCCCCCATCCCGGGAGG AGATGACCAAGAACCAGGTCAGCCTGAGCTGCGCGGTCAAAGGCTTCTATCCCAG CGACATCGCCGTGGAGTGGGAGAGCAATGGGCAGCCGGAGAACAACTACAAGACC ACGCCTCCCGTGCTGGACTCCGACGGCTCCTTCTTCCTCGTGAGCAAGCTCACCG TGGACAAGAGCAGGTGGCAGCAGGGGAACGTCTTCTCATGCTCCGTGATGCATGA GGCTCTGCACAACCACTACACGCAGAAGAGCCTCTCCCTGTCTCCGGGTAAA | |
| H07 | Polypeptid e 1 | GAAGTTCAACTTGTGGAGTCTGGAGGCGGTTTGGTACAACCTGGAGGTTCACTTC GTTTGTCATGTGCTGCCTCTGGGTTTAATATCAAGGATACTTATATTCATTGGGT TCGCCAGGCACCCGGAAAATGCCTGGAATGGGTGGCCCGTATATATCCCACTAAC GGTTACACTCGATATGCTGACTCTGTAAAGGGTCGATTTACTATATCCGCTGATA CTAGCAAAAACACTGCCTACCTCCAGATGAACAGTCTGCGCGCCGAGGATACAGC AGTTTATTATTGTTCCCGATGGGGAGGTGACGGGTTCTATGCCATGGACTACTGG GGTCAAGGGACACTGGTAACCGTTTCTTCTGCTAGCACCAAAGGACCTAGTGTTT TTCCTCTTGCCCCTTCCTCAAAGTCTACCTCTGGGGGGACAGCCGCTCTGGGCTG CCTGGTCAAGGATTATTTCCCAGAGCCTGTCACTGTCAGTTGGAACTCTGGAGCC TTGACTTCTGGTGTTCATACATTTCCTGCTGTCCTTCAAAGCAGCGGCTTGTACT CATTGTCTTCTGTTGTGACAGTACCCTCAAGCAGCCTCGGCACTCAGACATACAT CTGCAATGTCAACCACAAACCCTCAAATACAAAGGTAGATAAAAAAGTCGAACCA | 465 |

| | | | |
|---|---|---|---|
| | | AAGTCTTGTGACAAAACTCACACGTGCCCACCGTGCCCAGCACCTGAACTCCTGG GGGGACCGTCAGTCTTCCTCTTCCCCCCAAAACCCAAGGACACCCTCATGATCTC CCGGACCCCTGAGGTCACATGCGTGGTGGTGGACGTGAGCCACGAAGACCCTGAG GTCAAGTTCAACTGGTACGTGGACGGCGTGGAGGTGCATAATGCCAAGACAAAGC CGCGGGAGGAGCAGTACAACAGCACGTACCGTGTGGTCAGCGTCCTCACCGTCCT GCACCAGGACTGGCTGAATGGCAAGGAGTACAAGTGCAAGGTCTCCAACAAAGCC CTCCCAGCCCCCATCGAGAAAACCATCTCCAAAGCCAAAGGGCAGCCCCGAGAAC CACAGGTGTACACCCTGCCCCCATGCCGGGAGGAGATGACCAAGAACCAGGTCAG CCTGTGGTGCCTGGTCAAAGGCTTCTATCCCAGCGACATCGCCGTGGAGTGGGAG AGCAATGGGCAGCCGGAGAACAACTACAAGACCACGCCTCCCGTGCTGGACTCCG ACGGCTCCTTCTTCCTCTACAGCAAGCTCACCGTGGACAAGAGCAGGTGGCAGCA GGGGAACGTCTTCTCATGCTCCGTGATGCATGAGGCTCTGCACAACCACTACACG CAGAAGAGCCTCTCCCTGTCTCCGGGTAAA | |
| | Polypeptide 2 | GACATTCAGATGACCCAATCCCCCTCTAGCCTTTCAGCCTCCGTAGGGGACAGAG TTACAATAACCTGTAGGGCTAGTCAGGACGTTAATACAGCAGTCGCATGGTACCA GCAGAAGCCCGGTAAGGCTCCTAAGCTTTTGATCTACAGTGCTTCTTTCCTGTAT TCTGGTGTACCTAGCCGATTCTCAGGCTCTCGGAGTGGCACAGACTTCACTCTGA CAATTTCAAGTCTCCAGCCAGAAGATTTTGCAACCTACTACTGCCAACAACACTA CACTACTCCTCCAACCTTCGGATGTGGCACAAAAGTAGAGATTAAGCGTACGGTG GCAGCTCCCAGCGTTTTTATCTTTCCCCCATCCGACGAGCAGCTCAAGAGTGGCA CTGCCTCTGTAGTTTGTTTGCTGAATAACTTCTATCCACGTGAAGCAAAAGTACA GTGGAAGGTCGATAATGCCCTTCAGAGCGGTAACAGCCAAGAAAGTGTTACCGAG CAAGATTCCAAAGATTCCACTTACAGTCTGTCCAGCACATTGACACTGAGTAAGG CTGATTACGAAAAACACAAGGTGTACGCATGCGAGGTGACACACCAAGGTCTTTC ATCTCCTGTAACTAAGAGCTTTAACCGGGGAGAATGTGGTGGTGGGGGCAGCGGG GGCGGAGGTAGTGGAGGCGGCGGTAGTGAACCAAAGAGTAGTGACAAAACTCACA CGTGCCCACCGTGCCCAGCACCTGAACTCCTGGGGGGGACCGTCAGTCTTCCTCTT CCCCCCAAAACCCAAGGACACCCTCATGATCTCCCGGACCCCTGAGGTCACATGC GTGGTGGTGGACGTGAGCCACGAAGACCCTGAGGTCAAGTTCAACTGGTACGTGG | 466 |

| | | | |
|---|---|---|---|
| | | ACGGCGTGGAGGTGCATAATGCCAAGACAAAGCCGCGGGAGGAGCAGTACAACAG CACGTACCGTGTGGTCAGCGTCCTCACCGTCCTGCACCAGGACTGGCTGAATGGC AAGGAGTACAAGTGCAAGGTCTCCAACAAAGCCCTCCCAGCCCCCATCGAGAAAA CCATCTCCAAAGCCAAAGGGCAGCCCCGAGAACCACAGGTGTACACCCTGCCCCC ATGCCGGGAGGAGATGACCAAGAACCAGGTCAGCCTGTGGTGCCTGGTCAAAGGC TTCTATCCCAGCGACATCGCCGTGGAGTGGGAGAGCAATGGGCAGCCGGAGAACA ACTACAAGACCACGCCTCCCGTGCTGGACTCCGACGGCTCCTTCTTCCTCTACAG CAAGCTCACCGTGGACAAGAGCAGGTGGCAGCAGGGGAACGTCTTCTCATGCTCC GTGATGCATGAGGCTCTGCACAACCACTACACGCAGAAGAGCCTCTCCCTGTCTC CGGGTAAA | |
| | Polypeptid e 3 | GCTCCTACCAGTTCTAGCACTAAGAAAACCCAGCTTCAATTGGAGCACCTGCTGC TCGACCTTCAGATGATACTGAATGGAATAAACAACTACAAAAATCCAAAATTGAC AAGGATGCTGACTTTTAAATTCTACATGCCTAAGAAGGCAACAGAGTTGAAACAT TTGCAGTGCTTGGAGGAGGAGCTGAAGCCTTTGGAAGAAGTATTGAACTTGGCCC AGTCTAAGAACTTTCACCTCAGACCCAGAGACCTTATTTCAAACATTAACGTCAT CGTATTGGAACTTAAAGGAAGTGAAACCACTTTCATGTGCGAGTATGCTGATGAG ACCGCTACTATCGTGGAGTTTTTGAACAGATGGATAACATTTTGCCAGAGTATTA TTTCCACTCTTACCGGAGGCGGCGGCTCTGACAAAACTCACACGTGCCCACCGTG CCCAGCACCTGAACTCCTGGGGGGACCGTCAGTCTTCCTCTTCCCCCCAAAACCC AAGGACACCCTCATGATCTCCCGGACCCCTGAGGTCACATGCGTGGTGGTGGACG TGAGCCACGAAGACCCTGAGGTCAAGTTCAACTGGTACGTGGACGGCGTGGAGGT GCATAATGCCAAGACAAAGCCGCGGGAGGAGCAGTACAACAGCACGTACCGTGTG GTCAGCGTCCTCACCGTCCTGCACCAGGACTGGCTGAATGGCAAGGAGTACAAGT GCAAGGTCTCCAACAAAGCCCTCCCAGCCCCCATCGAGAAAACCATCTCCAAAGC CAAAGGGCAGCCCCGAGAACCACAGGTGTGCACCCTGCCCCCCATCCCGGGAGGAG ATGACCAAGAACCAGGTCAGCCTGAGCTGCGCGGTCAAAGGCTTCTATCCCAGCG ACATCGCCGTGGAGTGGGAGAGCAATGGGCAGCCGGAGAACAACTACAAGACCAC GCCTCCCGTGCTGGACTCCGACGGCTCCTTCTTCCTCGTGAGCAAGCTCACCGTG GACAAGAGCAGGTGGCAGCAGGGGAACGTCTTCTCATGCTCCGTGATGCATGAGG | 867 |

| | | CTCTGCACAACCACTACACGCAGAAGAGCCTCTCCCTGTCTCCGGGTAAA | |
|---|---|---|---|
| | Polypeptide 4 | GCTCCTACCAGTTCTAGCACTAAGAAAACCCAGCTTCAATTGGAGCACCTGCTGC TCGACCTTCAGATGATACTGAATGGAATAAACAACTACAAAAATCCAAAATTGAC AAGGATGCTGACTTTTAAATTCTACATGCCTAAGAAGGCAACAGAGTTGAAACAT TTGCAGTGCTTGGAGGAGGAGCTGAAGCCTTTGGAAGAAGTATTGAACTTGGCCC AGTCTAAGAACTTTCACCTCAGACCCAGAGACCTTATTTCAAACATTAACGTCAT CGTATTGGAACTTAAAGGAAGTGAAACCACTTTCATGTGCGAGTATGCTGATGAG ACCGCTACTATCGTGGAGTTTTTGAACAGATGGATAACATTTTGCCAGAGTATTA TTTCCACTCTTACCGGAGGCGGCGGCTCTGACAAAACTCACACGTGCCCACCGTG CCCAGCACCTGAACTCCTGGGGGGACCGTCAGTCTTCCTCTTCCCCCCAAAACCC AAGGACACCCTCATGATCTCCCGGACCCCTGAGGTCACATGCGTGGTGGTGGACG TGAGCCACGAAGACCCTGAGGTCAAGTTCAACTGGTACGTGGACGGCGTGGAGGT GCATAATGCCAAGACAAAGCCGCGGGAGGAGCAGTACAACAGCACGTACCGTGTG GTCAGCGTCCTCACCGTCCTGCACCAGGACTGGCTGAATGGCAAGGAGTACAAGT GCAAGGTCTCCAACAAAGCCCTCCCAGCCCCCATCGAGAAAACCATCTCCAAAGC CAAAGGGCAGCCCCGAGAACCACAGGTGTGCACCCTGCCCCCATCCCGGGAGGAG ATGACCAAGAACCAGGTCAGCCTGAGCTGCGCGGTCAAAGGCTTCTATCCCAGCG ACATCGCCGTGGAGTGGGAGAGCAATGGGCAGCCGGAGAACAACTACAAGACCAC GCCTCCCGTGCTGGACTCCGACGGCTCCTTCTTCCTCGTGAGCAAGCTCACCGTG GACAAGAGCAGGTGGCAGCAGGGGAACGTCTTCTCATGCTCCGTGATGCATGAGG CTCTGCACAACCACTACACGCAGAAGAGCCTCTCCCTGTCTCCGGGTAAA | 867 |
| H08 | Polypeptide 1 | GAAGTTCAACTTGTGGAGTCTGGAGGCGGTTTGGTACAACCTGGAGGTTCACTTC GTTTGTCATGTGCTGCCTCTGGGTTTAATATCAAGGATACTTATATTCATTGGGT TCGCCAGGCACCCGGAAAATGCCTGGAATGGGTGGCCCGTATATATCCCACTAAC GGTTACACTCGATATGCTGACTCTGTAAAGGGTCGATTTACTATATCCGCTGATA CTAGCAAAAACACTGCCTACCTCCAGATGAACAGTCTGCGCGCCGAGGATACAGC AGTTTATTATTGTTCCCGATGGGGAGGTGACGGGTTCTATGCCATGGACTACTGG GGTCAAGGGACACTGGTAACCGTTTCTTCTGCTAGCACCAAAGGACCTAGTGTTT TTCCTCTTGCCCCTTCCTCAAAGTCTACCTCTGGGGGGACAGCCGCTCTGGGCTG | 465 |

| | | CCTGGTCAAGGATTATTTCCCAGAGCCTGTCACTGTCAGTTGGAACTCTGGAGCC | |
|---|---|---|---|
| | | TTGACTTCTGGTGTTCATACATTTCCTGCTGTCCTTCAAAGCAGCGGCTTGTACT | |
| | | CATTGTCTTCTGTTGTGACAGTACCCTCAAGCAGCCTCGGCACTCAGACATACAT | |
| | | CTGCAATGTCAACCACAAACCCTCAAATACAAAGGTAGATAAAAAAGTCGAACCA | |
| | | AAGTCTTGTGACAAAACTCACACGTGCCCACCGTGCCCAGCACCTGAACTCCTGG | |
| | | GGGGACCGTCAGTCTTCCTCTTCCCCCCAAAACCCAAGGACACCCTCATGATCTC | |
| | | CCGGACCCCTGAGGTCACATGCGTGGTGGTGGACGTGAGCCACGAAGACCCTGAG | |
| | | GTCAAGTTCAACTGGTACGTGGACGGCGTGGAGGTGCATAATGCCAAGACAAAGC | |
| | | CGCGGGAGGAGCAGTACAACAGCACGTACCGTGTGGTCAGCGTCCTCACCGTCCT | |
| | | GCACCAGGACTGGCTGAATGGCAAGGAGTACAAGTGCAAGGTCTCCAACAAAGCC | |
| | | CTCCCAGCCCCCATCGAGAAAACCATCTCCAAAGCCAAAGGGCAGCCCCGAGAAC | |
| | | CACAGGTGTACACCCTGCCCCCATGCCGGGAGGAGATGACCAAGAACCAGGTCAG | |
| | | CCTGTGGTGCCTGGTCAAAGGCTTCTATCCCAGCGACATCGCCGTGGAGTGGGAG | |
| | | AGCAATGGGCAGCCGGAGAACAACTACAAGACCACGCCTCCCGTGCTGGACTCCG | |
| | | ACGGCTCCTTCTTCCTCTACAGCAAGCTCACCGTGGACAAGAGCAGGTGGCAGCA | |
| | | GGGGAACGTCTTCTCATGCTCCGTGATGCATGAGGCTCTGCACAACCACTACACG | |
| | | CAGAAGAGCCTCTCCCTGTCTCCGGGTAAA | |
| | Polypeptide 2 | GACATTCAGATGACCCAATCCCCCTCTAGCCTTTCAGCCTCCGTAGGGGACAGAG | 466 |
| | | TTACAATAACCTGTAGGGCTAGTCAGGACGTTAATACAGCAGTCGCATGGTACCA | |
| | | GCAGAAGCCCGGTAAGGCTCCTAAGCTTTTGATCTACAGTGCTTCTTTCCTGTAT | |
| | | TCTGGTGTACCTAGCCGATTCTCAGGCTCTCGGAGTGGCACAGACTTCACTCTGA | |
| | | CAATTTCAAGTCTCCAGCCAGAAGATTTTGCAACCTACTACTGCCAACAACACTA | |
| | | CACTACTCCTCCAACCTTCGGATGTGGCACAAAAGTAGAGATTAAGCGTACGGTG | |
| | | GCAGCTCCCAGCGTTTTTATCTTTCCCCCATCCGACGAGCAGCTCAAGAGTGGCA | |
| | | CTGCCTCTGTAGTTTGTTTGCTGAATAACTTCTATCCACGTGAAGCAAAAGTACA | |
| | | GTGGAAGGTCGATAATGCCCTTCAGAGCGGTAACAGCCAAGAAAGTGTTACCGAG | |
| | | CAAGATTCCAAAGATTCCACTTACAGTCTGTCCAGCACATTGACACTGAGTAAGG | |
| | | CTGATTACGAAAAACACAAGGTGTACGCATGCGAGGTGACACACCAAGGTCTTTC | |
| | | ATCTCCTGTAACTAAGAGCTTTAACCGGGGAGAATGTGGTGGTGGGGGCAGCGGG | |

| | | GGCGGAGGTAGTGGAGGCGGCGGTAGTGAACCAAAGAGTAGTGACAAAACTCACACGTGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATGCCGGGAGGAGATGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | |
| | Polypeptide 3 | GATCTCGGCAAGAAACTTCTGGAAGCAGCCAGGGCTGGCCAAGACGACGAGGTGCGTATTCTCATGGCCAATGGGGCAGATGTCAATGCCGACGACACTTGGGGGTGGACACCCCTGCATTTGGCCGCATACCAAGGCCATCTGGAAATAGTAGAGGTTTTGTTGAAGAACGGAGCCGATGTAAATGCATATGATTACATTGGATGGACTCCTCTGCACCTCGCAGCCGACGGCCACTTGGAGATAGTCGAGGTCCTCTTGAAGAATGGGGCTGATGTAAATGCATCTGATTACATCGGGGACACACCCCTCCACTTGGCCGCTCATAATGGGCACCTCGAAATTGTGGAGGTTCTCTTGAAGCACGGAGCCGACGTTAACGCTCAAGATAAGTTCGGTAAAACCGCTTTTGACATATCAATCGACAACGGAAATGAGGATCTGGCAGAAATCCTTCAAGGAGGCGGCGGCTCTGACAAAACTCACACGTGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCC | 868 |

| | | | |
|---|---|---|---|
| | | AAAGCCAAAGGGCAGCCCCGAGAACCACAGGTGTGCACCCTGCCCCCATCCCGGG AGGAGATGACCAAGAACCAGGTCAGCCTGAGCTGCGCGGTCAAAGGCTTCTATCC CAGCGACATCGCCGTGGAGTGGGAGAGCAATGGGCAGCCGGAGAACAACTACAAG ACCACGCCTCCCGTGCTGGACTCCGACGGCTCCTTCTTCCTCGTGAGCAAGCTCA CCGTGGACAAGAGCAGGTGGCAGCAGGGGAACGTCTTCTCATGCTCCGTGATGCA TGAGGCTCTGCACAACCACTACACGCAGAAGAGCCTCTCCCTGTCTCCGGGTAAA | |
| | Polypeptide 4 | GATCTCGGCAAGAAACTTCTGGAAGCAGCCAGGGCTGGCCAAGACGACGAGGTGC GTATTCTCATGGCCAATGGGGCAGATGTCAATGCCGACGACACTTGGGGGTGGAC ACCCCTGCATTTGGCCGCATACCAAGGCCATCTGGAAATAGTAGAGGTTTTGTTG AAGAACGGAGCCGATGTAAATGCATATGATTACATTGGATGGACTCCTCTGCACC TCGCAGCCGACGGCCACTTGGAGATAGTCGAGGTCCTCTTGAAGAATGGGGCTGA TGTAAATGCATCTGATTACATCGGGGACACACCCCTCCACTTGGCCGCTCATAAT GGGCACCTCGAAATTGTGGAGGTTCTCTTGAAGCACGGAGCCGACGTTAACGCTC AAGATAAGTTCGGTAAAACCGCTTTTGACATATCAATCGACAACGGAAATGAGGA TCTGGCAGAAATCCTTCAAGGAGGCGGCGGCTCTGACAAAACTCACACGTGCCCA CCGTGCCCAGCACCTGAACTCCTGGGGGGGACCGTCAGTCTTCCTCTTCCCCCCAA AACCCAAGGACACCCTCATGATCTCCCGGACCCCTGAGGTCACATGCGTGGTGGT GGACGTGAGCCACGAAGACCCTGAGGTCAAGTTCAACTGGTACGTGGACGGCGTG GAGGTGCATAATGCCAAGACAAAGCCGCGGGAGGAGCAGTACAACAGCACGTACC GTGTGGTCAGCGTCCTCACCGTCCTGCACCAGGACTGGCTGAATGGCAAGGAGTA CAAGTGCAAGGTCTCCAACAAAGCCCTCCCAGCCCCCATCGAGAAAACCATCTCC AAAGCCAAAGGGCAGCCCCGAGAACCACAGGTGTGCACCCTGCCCCCATCCCGGG AGGAGATGACCAAGAACCAGGTCAGCCTGAGCTGCGCGGTCAAAGGCTTCTATCC CAGCGACATCGCCGTGGAGTGGGAGAGCAATGGGCAGCCGGAGAACAACTACAAG ACCACGCCTCCCGTGCTGGACTCCGACGGCTCCTTCTTCCTCGTGAGCAAGCTCA CCGTGGACAAGAGCAGGTGGCAGCAGGGGAACGTCTTCTCATGCTCCGTGATGCA TGAGGCTCTGCACAACCACTACACGCAGAAGAGCCTCTCCCTGTCTCCGGGTAAA | 868 |
| H09 | Polypeptide 1 | GAAGTTCAACTTGTGGAGTCTGGAGGCGGTTTGGTACAACCTGGAGGTTCACTTC GTTTGTCATGTGCTGCCTCTGGGTTTAATATCAAGGATACTTATATTCATTGGGT | 465 |

| | | | |
|---|---|---|---|
| | | TCGCCAGGCACCCGGAAAATGCCTGGAATGGGTGGCCCGTATATATCCCACTAAC<br>GGTTACACTCGATATGCTGACTCTGTAAAGGGTCGATTTACTATATCCGCTGATA<br>CTAGCAAAAACACTGCCTACCTCCAGATGAACAGTCTGCGCGCCGAGGATACAGC<br>AGTTTATTATTGTTCCCGATGGGGAGGTGACGGGTTCTATGCCATGGACTACTGG<br>GGTCAAGGGACACTGGTAACCGTTTCTTCTGCTAGCACCAAAGGACCTAGTGTTT<br>TTCCTCTTGCCCCTTCCTCAAAGTCTACCTCTGGGGGGACAGCCGCTCTGGGCTG<br>CCTGGTCAAGGATTATTTCCCAGAGCCTGTCACTGTCAGTTGGAACTCTGGAGCC<br>TTGACTTCTGGTGTTCATACATTTCCTGCTGTCCTTCAAAGCAGCGGCTTGTACT<br>CATTGTCTTCTGTTGTGACAGTACCCTCAAGCAGCCTCGGCACTCAGACATACAT<br>CTGCAATGTCAACCACAAACCCTCAAATACAAAGGTAGATAAAAAAGTCGAACCA<br>AAGTCTTGTGACAAAACTCACACGTGCCCACCGTGCCCAGCACCTGAACTCCTGG<br>GGGGACCGTCAGTCTTCCTCTTCCCCCCCAAAACCCAAGGACACCCTCATGATCTC<br>CCGGACCCCTGAGGTCACATGCGTGGTGGTGGACGTGAGCCACGAAGACCCTGAG<br>GTCAAGTTCAACTGGTACGTGGACGGCGTGGAGGTGCATAATGCCAAGACAAAGC<br>CGCGGGAGGAGCAGTACAACAGCACGTACCGTGTGGTCAGCGTCCTCACCGTCCT<br>GCACCAGGACTGGCTGAATGGCAAGGAGTACAAGTGCAAGGTCTCCAACAAAGCC<br>CTCCCAGCCCCCATCGAGAAAACCATCTCCAAAGCCAAAGGGCAGCCCCGAGAAC<br>CACAGGTGTACACCCTGCCCCCATGCCGGGAGGAGATGACCAAGAACCAGGTCAG<br>CCTGTGGTGCCTGGTCAAAGGCTTCTATCCCAGCGACATCGCCGTGGAGTGGGAG<br>AGCAATGGGCAGCCGGAGAACAACTACAAGACCACGCCTCCCGTGCTGGACTCCG<br>ACGGCTCCTTCTTCCTCTACAGCAAGCTCACCGTGGACAAGAGCAGGTGGCAGCA<br>GGGGAACGTCTTCTCATGCTCCGTGATGCATGAGGCTCTGCACAACCACTACACG<br>CAGAAGAGCCTCTCCCTGTCTCCGGGTAAA | |
| | Polypeptid<br>e 2 | GACATTCAGATGACCCAATCCCCCTCTAGCCTTTCAGCCTCCGTAGGGGACAGAG<br>TTACAATAACCTGTAGGGCTAGTCAGGACGTTAATACAGCAGTCGCATGGTACCA<br>GCAGAAGCCCGGTAAGGCTCCTAAGCTTTTGATCTACAGTGCTTCTTTCCTGTAT<br>TCTGGTGTACCTAGCCGATTCTCAGGCTCTCGGAGTGGCACAGACTTCACTCTGA<br>CAATTTCAAGTCTCCAGCCAGAAGATTTTGCAACCTACTACTGCCAACAACACTA<br>CACTACTCCTCCAACCTTCGGATGTGGCACAAAAGTAGAGATTAAGCGTACGGTG | 466 |

| | | | |
|---|---|---|---|
| | | GCAGCTCCCAGCGTTTTTATCTTTCCCCCATCCGACGAGCAGCTCAAGAGTGGCACTGCCTCTGTAGTTTGTTTGCTGAATAACTTCTATCCACGTGAAGCAAAAGTACAGTGGAAGGTCGATAATGCCCTTCAGAGCGGTAACAGCCAAGAAAGTGTTACCGAGCAAGATTCCAAAGATTCCACTTACAGTCTGTCCAGCACATTGACACTGAGTAAGGCTGATTACGAAAAACACAAGGTGTACGCATGCGAGGTGACACACCAAGGTCTTTCATCTCCTGTAACTAAGAGCTTTAACCGGGGAGAATGTGGTGGTGGGGGCAGCGGGGGCGGAGGTAGTGGAGGCGGCGGTAGTGAACCAAAGAGTAGTGACAAAACTCACACGTGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATGCCCGGGAGGAGATGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | |
| | Polypeptide 3 | GAGGTGCAACTTGTGGAATCTGGAGGAGGCCTGGTTCAAGCCGGAGGATCTCTCCGACTTTCTTGTGCTGCTTCTGGCCGTACCTTCTCTTCCTATGCTATGGGCTGGTTCCGGCAAGCACCTGGGAAGGAGAGGGAGTTTGTAGTTGCAATTAACTGGTCCAGCGGGTCAACATATTACGCAGATTCCGTCAAAGGGCGGTTCACTATTTCACGGGATAATGCTAAAAACACCATGTATCTCCAAATGAACAGCCTTAAGCCCGAAGACACCGCAGTCTATTACTGCGCCGCAGGCTATCAGATCAACTCCGGTAACTATAACTTTAAAGACTATGAGTATGATTATTGGGGCCAAGGCACCCAAGTTACCGTGTCAAGCGCCTTGGAAGGAGGCGGCGGCTCTGACAAAACTCACACGTGCCCACCGTGCCCAGCACCTGAACTCCTGGGGGGACCGTCAGTCTTCCTCTTCCCCCCAAAACCCAAGGACACC | 869 |

| | | | |
|---|---|---|---|
| | | CTCATGATCTCCCGGACCCCTGAGGTCACATGCGTGGTGGTGGACGTGAGCCACG AAGACCCTGAGGTCAAGTTCAACTGGTACGTGGACGGCGTGGAGGTGCATAATGC CAAGACAAAGCCGCGGGAGGAGCAGTACAACAGCACGTACCGTGTGGTCAGCGTC CTCACCGTCCTGCACCAGGACTGGCTGAATGGCAAGGAGTACAAGTGCAAGGTCT CCAACAAAGCCCTCCCAGCCCCCATCGAGAAAACCATCTCCAAAGCCAAAGGGCA GCCCCGAGAACCACAGGTGTGCACCCTGCCCCCATCCCGGGAGGAGATGACCAAG AACCAGGTCAGCCTGAGCTGCGCGGTCAAAGGCTTCTATCCCAGCGACATCGCCG TGGAGTGGGAGAGCAATGGGCAGCCGGAGAACAACTACAAGACCACGCCTCCCGT GCTGGACTCCGACGGCTCCTTCTTCCTCGTGAGCAAGCTCACCGTGGACAAGAGC AGGTGGCAGCAGGGGAACGTCTTCTCATGCTCCGTGATGCATGAGGCTCTGCACA ACCACTACACGCAGAAGAGCCTCTCCCTGTCTCCGGGTAAA | |
| | Polypeptide 4 | GAGGTGCAACTTGTGGAATCTGGAGGAGGCCTGGTTCAAGCCGGAGGATCTCTCC GACTTTCTTGTGCTGCTTCTGGCCGTACCTTCTCTTCCTATGCTATGGGCTGGTT CCGGCAAGCACCTGGGAAGGAGAGGGAGTTTGTAGTTGCAATTAACTGGTCCAGC GGGTCAACATATTACGCAGATTCCGTCAAAGGGCGGTTCACTATTTCACGGGATA ATGCTAAAAACACCATGTATCTCCAAATGAACAGCCTTAAGCCCGAAGACACCGC AGTCTATTACTGCGCCGCAGGCTATCAGATCAACTCCGGTAACTATAACTTTAAA GACTATGAGTATGATTATTGGGGCCAAGGCACCCAAGTTACCGTGTCAAGCGCCT TGGAAGGAGGCGGCGGCTCTGACAAAACTCACACGTGCCCACCGTGCCCAGCACC TGAACTCCTGGGGGGACCGTCAGTCTTCCTCTTCCCCCCAAAACCCAAGGACACC CTCATGATCTCCCGGACCCCTGAGGTCACATGCGTGGTGGTGGACGTGAGCCACG AAGACCCTGAGGTCAAGTTCAACTGGTACGTGGACGGCGTGGAGGTGCATAATGC CAAGACAAAGCCGCGGGAGGAGCAGTACAACAGCACGTACCGTGTGGTCAGCGTC CTCACCGTCCTGCACCAGGACTGGCTGAATGGCAAGGAGTACAAGTGCAAGGTCT CCAACAAAGCCCTCCCAGCCCCCATCGAGAAAACCATCTCCAAAGCCAAAGGGCA GCCCCGAGAACCACAGGTGTGCACCCTGCCCCCATCCCGGGAGGAGATGACCAAG AACCAGGTCAGCCTGAGCTGCGCGGTCAAAGGCTTCTATCCCAGCGACATCGCCG TGGAGTGGGAGAGCAATGGGCAGCCGGAGAACAACTACAAGACCACGCCTCCCGT GCTGGACTCCGACGGCTCCTTCTTCCTCGTGAGCAAGCTCACCGTGGACAAGAGC | 869 |

| | | | |
|---|---|---|---|
| | | AGGTGGCAGCAGGGGAACGTCTTCTCATGCTCCGTGATGCATGAGGCTCTGCACA ACCACTACACGCAGAAGAGCCTCTCCCTGTCTCCGGGTAAA | |

## Example 29. Design, preparation and analysis of novel scFv fusion antibody format with two Fc domains

The engineered antibody format consisting of two Fc domains and one Fab region of Example 24 above can form a biparatopic engineered antibody format or a bispecific engineered antibody format in which a single-chain Fv fragment (single-chain fragment variable; scFv) is fused to the N-terminus of the hinge region, thereby possessing a new therapeutic function (Figure 54).

Polypeptide 1 of the antibody consists of a heavy chain variable region (X), a heavy chain CH1 region (A), a linker (L1), and a hinge-CH2 region-CH3 with Knob mutation (C); Polypeptide 2 consists of a light chain variable region (Y), a light chain CL region (B), a linker (L2), and a hinge-CH2 region-CH3 with Knob mutation (D); Polypeptide 3 consists of a single-chain Fv fragment (Z), a linker (L3), and a hinge-CH2 region-CH3 with Hole mutation (E); and Polypeptide 4 consists of a single-chain Fv fragment (Z), a linker (L4), and a hinge-CH2 region-CH3 with Hole mutation (F) (Figure 54). The four polypeptides are assembled into an engineered antigen binding protein having two Fc domains and one Fab in which two single-chain Fv fragments are fused (Figure 58).

H01P and PO1H are biparatopic antigen binding proteins with two Fc domains and one Fab region in which two single-chain Fv fragments are fused, and they specifically recognize HER2 and bind to cancer cells overexpressing HER2 in the same manner as in Figure 55A, thereby exhibiting antitumor activity (Figure 58, Figure 59a, and Table 94).

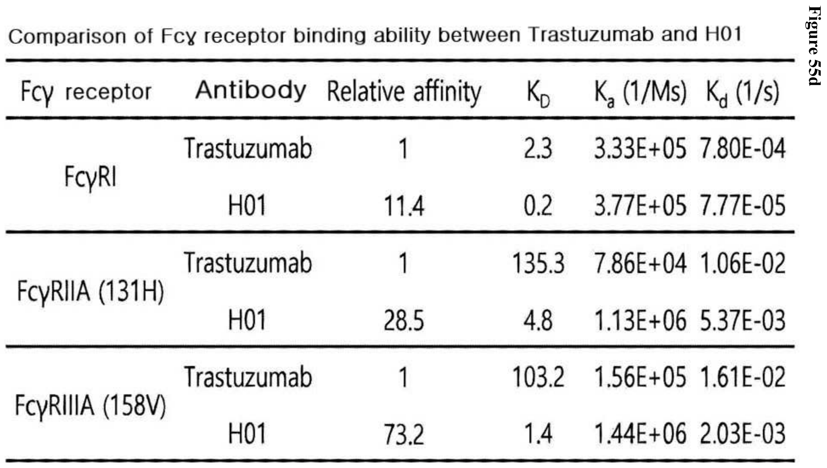

ME13, ME14, ME15, and ME16 are bispecific antigen binding proteins with two Fc domains that simultaneously bind to MET and EGFR, and they bind in the same manner as in Figure 55d in cancer cells in which MET and EGFR are simultaneously overexpressed, thereby exhibiting antitumor activity (Figure 58, Figure 59d, and Table 94).

CE05 is a bispecific antigen binding protein with two Fc domains that simultaneously bind to CEACAM5 and EGFR, and it binds in the same manner as in Figure 55d in cancer cells in which CEACAM5 and EGFR are simultaneously overexpressed, thereby exhibiting antitumor activity (Figure 58, Figure 59d, and Table 94).

The H01P, P01H, ME13, ME14, ME15, ME16, and CE05 were prepared in the same manner as in Example 28 above by co-transfecting polynucleotides encoding each of the four polypeptides into the EXPICHO-S™ cell line, purified, and analyzed.

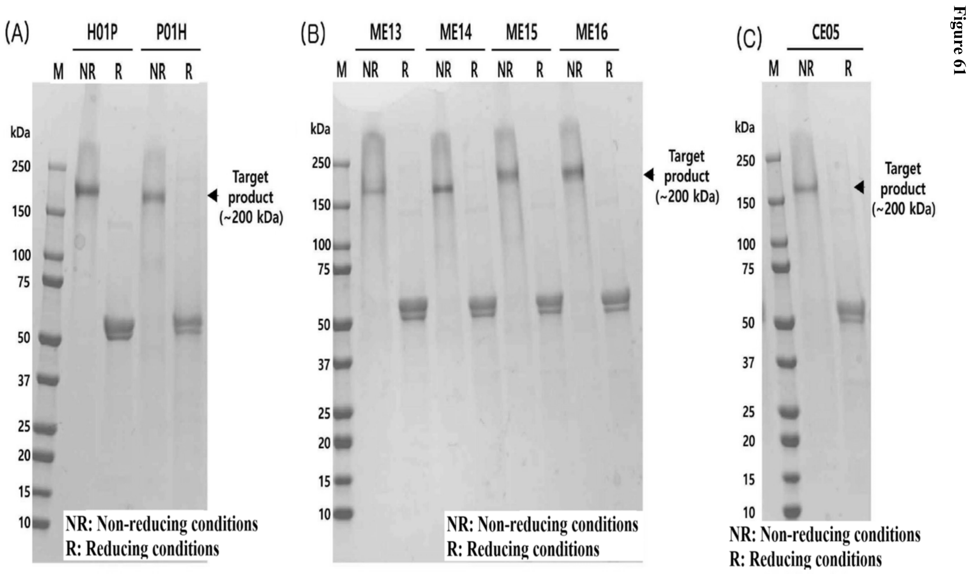

As shown in Figure 61, four polypeptides of appropriate sizes were identified under reducing conditions in all H01P, PO1H, ME13, ME14, ME15, ME16, and CE05. The analysis showed that under non-reducing conditions, H01P, P01H, ME13, ME14, ME15, ME16, and CE05 were all assembled well into the intact form.

Table 94 below shows the compositions of the biparatopic engineered antibody format or the bispecific engineered antibody format.

[Table 94]

| Name | Region | Composition | Epitope |
|---|---|---|---|
| H01P | Fab region | Anti-HER2 Fab (Fab of Trastuzumab) | Domain 4 of HER2 |

| | Z protein | Anti-HER2 scFv (scFv of Pertuzumab) | Domain 2 of HER2 |
|---|---|---|---|
| P01H | Fab region | Anti-HER2 Fab (Fab of Pertuzumab) | Domain 2 of HER2 |
| | Z protein | Anti-HER2 scFv (scFv of Trastuzumab) | Domain 4 of HER2 |
| ME13 | Fab region | Anti-EGFR Fab (Fab of Panitumumab) | Domain 3 of EGFR |
| | Z protein | Anti-MET scFv (scFv of Onartuzumab) | Sema domain of MET |
| ME14 | Fab region | Anti-EGFR Fab (Fab of Panitumumab) | Domain 3 of EGFR |
| | Z protein | Anti-MET scFv (scFv of CE-355621) | Sema domain of MET |
| ME15 | Fab region | Anti-MET Fab (Fab of Onartuzumab) | Sema domain of MET |
| | Z protein | Anti-EGFR scFv (scFv of Panitumumab) | Domain 3 of EGFR |
| ME16 | Fab region | Anti-MET Fab (Fab of CE-355621) | Sema domain of MET |
| | Z protein | Anti-EGFR scFv (scFv of Panitumumab) | Domain 3 of EGFR |
| CE05 | Fab region | Anti-EGFR Fab (Fab of Panitumumab) | Domain 3 of EGFR |
| | Z protein | Anti-CEACAM5 scFv (scFv of Labetuzumab) | A3-B3 domain of CEACAM5 |

Table 95 below shows the polypeptide sequence of each domain of the biparatopic engineered antibody format or the bispecific engineered antibody format.

[Table 95]

| Name | Polypeptide | Sequence | SEC ID NO |
|---|---|---|---|
| I01P | Fab region (VH-CH1) | EVQLVESGGGLVQPGGSLRLSCAASGFNIKDTYIHWVRQAPGKCLEWVARI YPTNGYTRYADSVKGRFTISADTSKNTAYLQMNSLRAEDTAVYYCSRWGGD GFYAMDYWGQGTLVTVSSASTKGPSVFPLAPSSKSTSGGTAALGCLVKDYF PEPVTVSWNSGALTSGVHTFPAVLQSSGLYSLSSVVTVPSSSLGTQTYICN VNHKPSNTKVDKKV | 834 |
| | Fab region (VL-CL) | DIQMTQSPSSLSASVGDRVTITCRASQDVNTAVAWYQQKPGKAPKLLIYSA SFLYSGVPSRFSGSRSGTDFTLTISSLQPEDFATYYCQQHYTTPPTFGCGT KVEIKRTVAAPSVFIFPPSDEQLKSGTASVVCLLNNFYPREAKVQWKVDNA LQSGNSQESVTEQDSKDSTYSLSSTLTLSKADYEKHKVYACEVTHQGLSSP VTKSFNRGEC | 835 |
| | L1 linker | EPKSC | 836 |
| | L2 linker | GGGGSGGGGSGGGGSEPKSS | 837 |
| | L3 linker | GGGGS | 838 |
| | L4 linker | GGGGS | 838 |
| | Hinge-Fc (Knob) | DKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCK VSNKALPAPIEKTISKAKGQPREPQVYTLPPCREEMTKNQVSLWCLVKGFY PSDIAVEWESNGQPENNYKTTPPVLDSDGSFFLYSKLTVDKSRWQQGNVFS CSVMHEALHNHYTQKSLSLSPGK | 839 |
| | Hinge-Fc (Hole) | DKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCK VSNKALPAPIEKTISKAKGQPREPQVCTLPPSREEMTKNQVSLSCAVKGFY PSDIAVEWESNGQPENNYKTTPPVLDSDGSFFLVSKLTVDKSRWQQGNVFS CSVMHEALHNHYTQKSLSLSPGK | 7 |
| | Z protein(scFv of | EVQLVESGGGLVQPGGSLRLSCAASGFTFTDYTMDWVRQAPGKCLEWVADV NPNSGGSIYNQRFKGRFTLSVDRSKNTLYLQMNSLRAEDTAVYYCARNLGP SFYFDYWGQGTLVTVSSGGGGSGGGGSGGGGSDIQMTQSPSSLSASVGDRV | 870 |

298

| | Per(uzumab) | TITCKASQDVSIGVAWYQQKPGKAPKLLIYSASYRYTGVPSRFSGSGSGTD FTLTISSLQPEDFATYYCQQYYIYPYTFGCGTKVEIK | |
|---|---|---|---|
| POIH | Fab region (VH-CH1) | EVQLVESGGGLVQPGGSLRLSCAASGFTFTDYTMDWVRQAPGKCLEWVADV NPNSGGSIYNQRFKGRFTLSVDRSKNTLYLQMNSLRAEDTAVYYCARNLGP SFYFDYWGQGTLVTVSSASTKGPSVFPLAPSSKSTSGGTAALGCLVKDYFP EPVTVSWNSGALTSGVHTFPAVLQSSGLYSLSSVVTVPSSSLGTQTYICNV NHKPSNTKVDKKV | 834 |
| | Fab region (VL-CL) | DIQMTQSPSSLSASVGDRVTITCKASQDVSIGVAWYQQKPGKAPKLLIYSA SYRYTGVPSRFSGSGSGTDFTLTISSLQPEDFATYYCQQYYIYPYTFGCGT KVEIKRTVAAPSVFIFPPSDEQLKSGTASVVCLLNNFYPREAKVQWKVDNA LQSGNSQESVTEQDSKDSTYSLSSTLTLSKADYEKHKVYACEVTHQGLSSP VTKSFNRGEC | 835 |
| | L1 linker | EPKSC | 836 |
| | L2 linker | GGGGSGGGGSGGGGSEPKSS | 837 |
| | L3 linker | GGGGS | 838 |
| | L4 linker | GGGGS | 838 |
| | Hinge-Fc (Knob) | DKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCK VSNKALPAPIEKTISKAKGQPREPQVYTLPPCREEMTKNQVSLWCLVKGFY PSDIAVEWESNGQPENNYKTTPPVLDSDGSFFLYSKLTVDKSRWQQGNVFS CSVMHEALHNHYTQKSLSLSPGK | 839 |
| | Hinge-Fc (Hole) | DKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCK VSNKALPAPIEKTISKAKGQPREPQVCTLPPSREEMTKNQVSLSCAVKGFY PSDIAVEWESNGQPENNYKTTPPVLDSDGSFFLVSKLTVDKSRWQQGNVFS CSVMHEALHNHYTQKSLSLSPGK | 7 |
| | Z protein(scFv of | EVQLVESGGGLVQPGGSLRLSCAASGFNIKDTYIHWVRQAPGKCLEWVARI YPTNGYTRYADSVKGRFTISADTSKNTAYLQMNSLRAEDTAVYYCSRWGGD GFYAMDYWGQGTLVTVSSGGGGSGGGGSGGGGSDIQMTQSPSSLSASVGDR | 871 |

| | | | |
|---|---|---|---|
| | Trastuzumab) | VTITCRASQDVNTAVAWYQQKPGKAPKLLIYSASFLYSGVPSRFSGSRSGT DFTLTISSLQPEDFATYYCQQHYTTPPTFGCGTKVEIK | |
| | Fab region (VH-CH1) | QVQLQESGPGLVKPSETLSLTCTVSGGSVSSGDYYWTWIRQSPGKCLEWIG HIYYSGNTNYNPSLKSRLTISIDTSKTQFSLKLSSVTAADTAIYYCVRDRV TGAFDIWGQGTMVTVSSASTKGPSVFPLAPSSKSTSGGTAALGCLVKDYFP EPVTVSWNSGALTSGVHTFPAVLQSSGLYSLSSVVTVPSSSLGTQTYICNV NHKPSNTKVDKKV | 872 |
| | Fab region (VL-CL) | DIQMTQSPSSLSASVGDRVTITCQASQDISNYLNWYQQKPGKAPKLLIYDA SNLETGVPSRFSGSGSGTDFTFTISSLQPEDIATYFCQHFDHLPLAFGCGT KVEIKRTVAAPSVFIFPPSDEQLKSGTASVVCLLNNFYPREAKVQWKVDNA LQSGNSQESVTEQDSKDSTYSLSSTLTLSKADYEKHKVYACEVTHQGLSSP VTKSFNRGEC | 873 |
| | L1 linker | EPKSC | 836 |
| | L2 linker | GGGGSGGGGSGGGGSEPKSS | 837 |
| | L3 linker | GGGGS | 838 |
| ME13 | L4 linker | GGGGS | 838 |
| | Hinge-Fc (Knob) | DKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCK VSNKALPAPIEKTISKAKGQPREPQVYTLPPCREEMTKNQVSLWCLVKGFY PSDIAVEWESNGQPENNYKTTPPVLDSDGSFFLYSKLTVDKSRWQQGNVFS CSVMHEALHNHYTQKSLSLSPGK | 839 |
| | Hinge-Fc (Hole) | DKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCK VSNKALPAPIEKTISKAKGQPREPQVCTLPPSREEMTKNQVSLSCAVKGFY PSDIAVEWESNGQPENNYKTTPPVLDSDGSFFLVSKLTVDKSRWQQGNVFS CSVMHEALHNHYTQKSLSLSPGK | 7 |
| | Z protein(scFv of | EVQLVESGGGLVQPGGSLRLSCAASGYTFTSYWLHWVRQAPGKCLEWVGMI DPSNSDTRFNPNFKDRFTISADTSKNTAYLQMNSLRAEDTAVYYCATYRSY VTPLDYWGQGTLVTVSSGGGGSGGGGSGGGGSDIQMTQSPSSLSASVGDRV | 874 |

| | Onartuzumab) | TITCKSSQSLLYTSSQKNYLAWYQQKPGKAPKLLIYWASTRESGVPSRFSG SGSGTDFTLTISSLQPEDFATYYCQQYYAYPWTFGCGTKVEIK | |
|---|---|---|---|
| ME14 | Fab region (VH-CH1) | QVQLQESGPGLVKPSETLSLTCTVSGGSVSSGDYYWTWIRQSPGKGLEWIG IIYYSGNTNYNPSLKSRLTISIDTSKTQFSLKLSSVTAADTAIYYCVRDRV TGAFDIWGQGTMVTVSSASTKGPSVFPLAPSSKSTSGGTAALGCLVKDYFP EPVTVSWNSGALTSGVHTFPAVLQSSGLYSLSSVVTVPSSSLGTQTYICNV NHKPSNTKVDKKV | 872 |
| | Fab region (VL-CL) | DIQMTQSPSSLSASVGDRVTITCQASQDISNYLNWYQQKPGKAPKLLIYDA SNLETGVPSRFSGSGSGTDFTFTISSLQPEDIATYFCQHFDHLPLAFGCGT KVEIKRTVAAPSVFIFPPSDEQLKSGTASVVCLLNNFYPREAKVQWKVDNA LQSGNSQESVTEQDSKDSTYSLSSTLTLSKADYEKHKVYACEVTHQGLSSP VTKSFNRGEC | 873 |
| | L1 linker | EPKSC | 836 |
| | L2 linker | GGGGSGGGGSGGGGSEPKSS | 837 |
| | L3 linker | GGGGS | 838 |
| | L4 linker | GGGGS | 838 |
| | Hinge-Fc (Knob) | DKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCK VSNKALPAPIEKTISKAKGQPREPQVYTLPPCREEMTKNQVSLWCLVKGFY PSDIAVEWESNGQPENNYKTTPPVLDSDGSFFLYSKLTVDKSRWQQGNVFS CSVMHEALHNHYTQKSLSLSPGK | 839 |
| | Hinge-Fc (Hole) | DKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCK VSNKALPAPIEKTISKAKGQPREPQVCTLPPSREEMTKNQVSLSCAVKGFY PSDIAVEWESNGQPENNYKTTPPVLDSDGSFFLVSKLTVDKSRWQQGNVFS CSVMHEALHNHYTQKSLSLSPGK | 7 |
| | Z protein(scFv of CE-355621) | QVQLVQSGAEVKKPGASVKVSCKASGYTFTSYGFSWVRQAPGQGLEWMGWI SASNGNTYYAQKLQGRVTMTTDTSTSTAYMELRSLRSDDTAVYYCARVYAD YADYWGQGTLVTVSSGGGGSGGGGSGGGGSDIQMTQSPSSVSASVGDRVTI | 875 |

| | | | |
|---|---|---|---|
| | | TCRASQGINTWLAWYQQKPGKAPKLLIYAASSLKSGVPSRFSGSGSGTDFT LTISSLQPEDFATYYCQQANSFPLTFGCGTKVEIK | |
| ME15: | Fab region (VH-CH1) | EVQLVESGGGLVQPGGSLRLSCAASGYTFTSYWLHWVRQAPGKCLEWVGMI DPSNSDTRFNPNFKDRFTISADTSKNTAYLQMNSLRAEDTAVYYCATYRSY VTPLDYWGQGTLVTVSSASTKGPSVFPLAPSSKSTSGGTAALGCLVKDYFP EPVTVSWNSGALTSGVHTFPAVLQSSGLYSLSSVVTVPSSSLGTQTYICNV NHKPSNTKVDKKV | 876 |
| | Fab region (VL-CL) | DIQMTQSPSSLSASVGDRVTITCKSSQSLLYTSSQKNYLAWYQQKPGKAPK LLIYWASTRESGVPSRFSGSGSGTDFTLTISSLQPEDFATYYCQQYYAYPW TFGCGTKVEIKRTVAAPSVFIFPPSDEQLKSGTASVVCLLNNFYPREAKVQ WKVDNALQSGNSQESVTEQDSKDSTYSLSSTLTLSKADYEKHKVYACEVTH QGLSSPVTKSFNRGEC | 877 |
| | L1 linker | EPKSC | 836 |
| | L2 linker | GGGGSGGGGSGGGGSEPKSS | 837 |
| | L3 linker | GGGGS | 838 |
| | L4 linker | GGGGS | 838 |
| | Hinge-Fc (Knob) | DKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCK VSNKALPAPIEKTISKAKGQPREPQVYTLPPCREEMTKNQVSLWCLVKGFY PSDIAVEWESNGQPENNYKTTPPVLDSDGSFFLYSKLTVDKSRWQQGNVFS CSVMHEALHNHYTQKSLSLSPGK | 839 |
| | Hinge-Fc (Hole) | DKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCK VSNKALPAPIEKTISKAKGQPREPQVCTLPPSREEMTKNQVSLSCAVKGFY PSDIAVEWESNGQPENNYKTTPPVLDSDGSFFLVSKLTVDKSRWQQGNVFS CSVMHEALHNHYTQKSLSLSPGK | 7 |
| | Z protein(scFv of | QVQLQESGPGLVKPSETLSLTCTVSGGSVSSGDYYWTWIRQSPGKCLEWIG HIYYSGNTNYNPSLKSRLTISIDTSKTQFSLKLSSVTAADTAIYYCVRDRV TGAFDIWGQGTMVTVSSGGGGSGGGGSGGGGSDIQMTQSPSSLSASVGDRV | 878 |

| | | | |
|---|---|---|---|
| | Panitumumab) | TITCQASQDISNYLNWYQQKPGKAPKLLIYDASNLETGVPSRFSGSGSGTD FTFTISSLQPEDIATYFCQIFDHLPLAFGCGTRVEIK | |
| ME16 | Fab region (VH-CH1) | QVQLVQSGAEVKKPGASVKVSCKASGYTFTSYGFSWVRQAPGQCLEWMGWI SASNGNTYYAQKLQGRVTMTTDTSTSTAYMELRSLRSDDTAVYYCARVYAD YADYWGQGTLVTVSSASTKGPSVFPLAPSSKSTSGGTAALGCLVKDYFPEP VTVSWNSGALTSGVHTFPAVLQSSGLYSLSSVVTVPSSSLGTQTYICNVNH KPSNTKVDKKV | 879 |
| | Fab region (VL-CL) | DIQMTQSPSSVSASVGDRVTITCRASQGINTWLAWYQQKPGKAPKLLIYAA SSLKSGVPSRFSGSGSGTDFTLTISSLQPEDFATYYCQQANSFPLTFGCGT KVEIKRTVAAPSVFIFPPSDEQLKSGTASVVCLLNNFYPREAKVQWKVDNA LQSGNSQESVTEQDSKDSTYSLSSTLTLSKADYEKHKVYACEVTHQGLSSP VTKSFNRGEC | 880 |
| | L1 linker | EPKSC | 836 |
| | L2 linker | GGGGSGGGGSGGGGSEPKSS | 837 |
| | L3 linker | GGGGS | 838 |
| | L4 linker | GGGGS | 838 |
| | Hinge-Fc (Knob) | DKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCK VSNKALPAPIEKTISKAKGQPREPQVYTLPPCREEMTKNQVSLWCLVKGFY PSDIAVEWESNGQPENNYKTTPPVLDSDGSFFLYSKLTVDKSRWQQGNVFS CSVMHEALHNHYTQKSLSLSPGK | 839 |
| | Hinge-Fc (Hole) | DKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCK VSNKALPAPIEKTISKAKGQPREPQVCTLPPSREEMTKNQVSLSCAVKGFY PSDIAVEWESNGQPENNYKTTPPVLDSDGSFFLVSKLTVDKSRWQQGNVFS CSVMHEALHNHYTQKSLSLSPGK | 7 |
| | Z protein(scFv of | QVQLQESGPGLVKPSETLSLTCTVSGGSVSSGDYYWTWIRQSPGKCLEWIG HIYYSGNTNYNPSLKSRLTISIDTSKTQFSLKLSSVTAADTAIYYCVRDRV TGAFDIWGQGTMVTVSSGGGGSGGGGSGGGGSDIQMTQSPSSLSASVGDRV | 878 |

| | Panitumumab) | TITCQASQDISNYLNWYQQKPGKAPKLLIYDASNLETGVPSRFSGSGSGTD FTFTISSLQPEDIATYFCQHFDHLPLAFGCGTKVEIK | |
|---|---|---|---|
| CE05 | Fab region (VH-CH1) | QVQLQESGPGLVKPSETLSLTCTVSGGSYSSGDYYWTWIRQSPGKCLEWIG HIYYSGNTNYNPSLKSRLTISIDTSKTQFSLKLSSVTAADTAIYYCVRDRV TGAFDIWGQGTMVTVSSASTKGPSVFPLAPSSKSTSGGTAALGCLVKDYFP EPVTVSWNSGALTSGVHTFPAVLQSSGLYSLSSVVTVPSSSLGTQTYICNV NHKPSNTKVDKKV | 872 |
| | Fab region (VL-CL) | DIQMTQSPSSLSASVGDRVTITCQASQDISNYLNWYQQKPGKAPKLLIYDA SNLETGVPSRFSGSGSGTDFTFTISSLQPEDIATYFCQHFDHLPLAFGCGT KVEIKRTVAAPSVFIFPPSDEQLKSGTASVVCLLNNFYPREAKVQWKVDNA LQSGNSQESVTEQDSKDSTYSLSSTLTLSKADYEKHKVYACEVTHQGLSSP VTKSFNRGEC | 873 |
| | L1 linker | EPKSC | 836 |
| | L2 linker | GGGGSGGGGSGGGGSEPKSS | 837 |
| | L3 linker | GGGGS | 838 |
| | L4 linker | GGGGS | 838 |
| | Hinge-Fc (Knob) | DKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCK VSNKALPAPIEKTISKAKGQPREPQVYTLPPCREEMTKNQVSLWCLVKGFY PSDIAVEWESNGQPENNYKTTPPVLDSDGSFFLYSKLTVDKSRWQQGNVFS CSVMHEALHNHYTQKSLSLSPGK | 839 |
| | Hinge-Fc (Hole) | DKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCK VSNKALPAPIEKTISKAKGQPREPQVCTLPPSREEMTKNQVSLSCAVKGFY PSDIAVEWESNGQPENNYKTTPPVLDSDGSFFLVSKLTVDKSRWQQGNVFS CSVMHEALHNHYTQKSLSLSPGK | 7 |
| | Z protein(scFv of | EVQLVESGGGVVQPGRSLRLSCSASGFDFTTYNMSWVRQAPGKCLEWIGEI HPDSSTINYAPSLKDRFTISRDNAKNTLFLQMDSLRPEDTGVYFCASLYFG FPWFAYWGQGTPVTVSSGGGGSGGGGSGGGGSDIQLTQSPSSLSASVGDRV | 881 |

| | Labetuzumab) | TITCKASQDVGTSVAWYQQKPGKAPKLLIYWTSTRHTGVPSRFSGSGSGTD FTFTISSLQPEDIATYYCQQYSLYRSFGCGTKVEIK | |
|---|---|---|---|

Table 96 below shows the polynucleotide sequences encoding the polypeptides of each domain of the biparatopic engineered antibody format or the bispecific engineered antibody format.

[Table 96]

| Name | polypeptide | Sequence | SEQ ID NO |
|---|---|---|---|
| H01P | Fab region (VH-CH1) | GAAGTTCAACTTGTGGAGTCTCGGAGGCGGTTTGGTACAACCTGGAGGTTC ACTTCGTTTGTCATGTGCTGCCTCTGGGTTTAATATCAAGGATACTTATA TTCATTGGGTTCGCCAGGCACCCGGAAAATGCCTGGAATGGGTGGCCCGT ATATATCCCACTAACGGTTACACTCGATATGCTGACTCTGTAAAGGGTCG ATTTACTATATCCGCTGATACTAGCAAAAACACTGCCTACCTCCAGATGA ACAGTCTGCGCGCCGAGGATACAGCAGTTTATTATTGTTCCCGATGGGGA GGTGACGGGTTCTATGCCATGGACTACTGGGGTCAACGGACACTGGTAAC CGTTTCTTCTGCTAGCACCAAAGGACCTAGTGTTTTTTCCTCTTGCCCCTT CCTCAAAGTCTACCTCTGGGGGGACAGCCGCTCTGGGCTGCCTGGTCAAG GATTATTTCCCAGAGCCTGTCACTGTCAGTTGGAACTCTGGAGCCTTGAC TTCTGGTGTTCATACATTTCCTGCTGTCCTTCAAAGCAGCGGCTTGTACT CATTGTCTTCTGTTGTGACAGTACCCTCAAGCAGCCTCGGCACTCAGACA TACATCTGCAATGTCAACCACAAACCCTCAAATACAAAGGTAGATAAAAA AGTC | 846 |
| | Fab region (VL-CL) | GACATTCAGATGACCCAATCCCCTCTAGCCTTTCAGCCTCCGTAGGGGA CAGAGTTACAATAACCTGTAGGGCTAGTCAGGACGTTAATACAGCAGTCG CATGGTACCAGCAGAAGCCCGGTAAGGCTCCTAAGCTTTTGATCTACAGT GCTTCTTTCCTGTATTCTGGTGTACCTAGCCGATTCTCAGGCTCTCGGAG TGGCACAGACTTCACTCTGACAATTTCAAGTCTCCAGCCAGAAGATTTTG CAACCTACTACTGCCAACAACACTACACTACTCCTCCAACCTTCGGATGT GGCACAAAAGTAGAGATTAAGCGTACGGTGGCAGCTCCCAGCGTTTTTAT CTTTCCCCCATCCGACGAGCAGCTCAAGAGTGGCACTGCCTCTGTAGTTT GTTTGCTGAATAACTTCTATCCACGTGAAGCAAAAGTACAGTGGAAGGTC GATAATGCCCTTCAGAGCGGTAACAGCCAAGAAAGTGTTACCGAGCAAGA TTCCAAAGATTCCACTTACAGTCTGTCCAGCACACATTGACACTGAGTAAGG CTGATTACGAAAAACACAAGGTGTACGCATGCGAGGTGACACACCAAGGT CTTTCATCTCCTGTAACTAAGAGCTTTAACCGCGGGAGAATGT | 847 |

| L1 linker | GAACCAAAGTCTTGT | 848 |
|---|---|---|
| L2 linker | GGTGGTGGGGGCAGCGGGGGCGGAGGTAGTGGAGGCGGCGGTAGTGAACC AAAGAGTAGT | 849 |
| L3 linker | GGAGGCGGCGGCTCT | 850 |
| L4 linker | GGAGGCGGCGGCTCT | 850 |
| Hinge-Fc (Knob) | GACAAAACTCACACGTGCCCACCGTGCCCAGCACCTGAACTCCTGGGGGG ACCGTCAGTCTTCCTCTTCCCCCCAAAACCCAAGGACACCCTCATGATCT CCCGGACCCCTGAGGTCACATGCGTGGTGGTGGACGTGAGCCACGAAGAC CCTGAGGTCAAGTTCAACTGGTACGTGGACGGCGTGGAGGTGCATAATGC CAAGACAAAGCCGCGGGAGGAGCAGTACAACAGCACGTACCGTGTGGTCA GCGTCCTCACCGTCCTGCACCAGGACTGGCTGAATGGCAAGGAGTACAAG TGCAAGGTCTCCAACAAAGCCCTCCCAGCCCCCATCGAGAAAACCATCTC CAAAGCCAAAGGGCAGCCCCGAGAACCACAGGTGTACACCCTGCCCCCAT GCCGGGAGGAGATGACCAAGAACCAGGTCAGCCTGTGGTGCCTGGTCAAA GGCTTCTATCCCAGCGACATCGCCGTGGAGTGGGAGAGCAATGGGCAGCC GGAGAACAACTACAAGACCACGCCTCCCGTGCTGGACTCCGACGGCTCCT TCTTCCTCTACAGCAAGCTCACCGTGGACAAGAGCAGGTGGCAGCAGGGG AACGTCTTCTCATGCTCCGTGATGCATGAGGCTCTGCACAACCACTACAC GCAGAAGAGCCTCTCCCTGTCTCCGGGTAAA | 851 |
| Hinge-Fc (Hole) | GACAAAACTCACACGTGCCCACCGTGCCCAGCACCTGAACTCCTGGGGGG ACCGTCAGTCTTCCTCTTCCCCCCAAAACCCAAGGACACCCTCATGATCT CCCGGACCCCTGAGGTCACATGCGTGGTGGTGGACGTGAGCCACGAAGAC CCTGAGGTCAAGTTCAACTGGTACGTGGACGGCGTGGAGGTGCATAATGC CAAGACAAAGCCGCGGGAGGAGCAGTACAACAGCACGTACCGTGTGGTCA GCGTCCTCACCGTCCTGCACCAGGACTGGCTGAATGGCAAGGAGTACAAG TGCAAGGTCTCCAACAAAGCCCTCCCAGCCCCCATCGAGAAAACCATCTC CAAAGCCAAAGGGCAGCCCCGAGAACCACAGGTGTGCACCCTGCCCCCAT CCCGGGAGGAGATGACCAAGAACCAGGTCAGCCTGAGCTGCGCGGTCAAA GGCTTCTATCCCAGCGACATCGCCGTGGAGTGGGAGAGCAATGGGCAGCC | 458 |

| | | | |
|---|---|---|---|
| | | GGAGAACAACTACAAGACCACGCCTCCCGTGCTGGACTCCGACGGCTCCT TCTTCCTCGTGAGCAAGCTCACCGTGGACAAGAGCAGGTGGCAGCAGGGG AACGTCTTCTCATGCTCCGTGATGCATGAGGCTCTGCACAACCACTACAC GCAGAAGAGCCTCTCCCTGTCTCCGGGTAAA | |
| | Z protein(scFv of Pertuzumab) | GAAGTACAGTTGGTGGAGTCTGGTGGAGGTCTTGTACAACCTGGCGGGAG TTTGCGGCTTTCCTGCGCTGCAAGCGGGTTTACCTTCACCGATTATACCA TGGATTGGGTACGCCAAGCCCCTGGTAAGTGCCTTGAGTGGGTTGCCGAT GTAAACCCTAATTCCGGAGGAAGTATCTATAATCAACGCTTCAAGGGCCG ATTCACTCTGAGTGTGGATCGAAGCAAGAACACCTTGTACTTGCAGATGA ATTCCTTGCGGGCTGAAGACACAGCCGTCTATTACTGCGCCCGAAATTTG GGGCCTTCATTCTATTTTGACTATTGGGGTCAGGGAACTCTGGTAACTGT TTCAAGTGGCGGAGGGGGTTCTGGAGGCGGAGGCAGCGGGGGCGGAGGGT CCGATATTCAAATGACCCAAAGTCCAAGTTCCCTTTCAGCATCTGTCGGT GATCGAGTCACTATAACTTGTAAGGCCAGCCAAGACGTTAGCATAGGAGT AGCATGGTACCAACAAAAGCCTGGGAAGGCTCCCAAACTTCTCATTTATT CTGCTTCCTACCGATATACTGGTGTCCCAAGTAGATTTTCTGGCAGCGGA TCTGGAACTGATTTTACATTGACTATCAGCTCCCTCCAGCCTGAGGACTT CGCTACTTATTACTGCCAACAGTACTACATTTATCCCTATACATTCGGTT GTGGGACCAAAGTAGAGATCAAA | 882 |
| PO1H | Fab region (VH-CH1) | GAAGTACAGTTGGTGGAGTCTGGTGGAGGTCTTGTACAACCTGGCGGGAG TTTGCGGCTTTCCTGCGCTGCAAGCGGGTTTACCTTCACCGATTATACCA TGGATTGGGTACGCCAAGCCCCTGGTAAGTGCCTTGAGTGGGTTGCCGAT GTAAACCCTAATTCCGGAGGAAGTATCTATAATCAACGCTTCAAGGGCCG ATTCACTCTGAGTGTGGATCGAAGCAAGAACACCTTGTACTTGCAGATGA ATTCCTTGCGGGCTGAAGACACAGCCGTCTATTACTGCGCCCGAAATTTG GGGCCTTCATTCTATTTTGACTATTGGGGTCAGGGAACTCTGGTAACTGT TTCAAGTGCTAGCACCAAAGGACCTAGTGTTTTTCCTCTTGCCCCTTCCT CAAAGTCTACCTCTGGGGCGGACAGCCGCTCTGCGCTGCCTGGTCAAGGAT TATTTCCCAGAGCCTGTCACTGTCAGTTCGAACTCTGGAGCCTTGACTTC | 883 |

|  |  |  |  |
|---|---|---|---|
|  |  | TGGTGTTCATACATTTCCTGCTGTCCTTCAAAGCAGCGGCTTGTACTCAT TGTCTTCTGTTGTGACAGTACCCTCAAGCAGCCTCGGCACTCAGACATAC ATCTGCAATGTCAACCACAAACCCTCAAATACAAAGGTAGATAAAAAAGT C |  |
|  | Fab region (VL-CL) | GATATTCAAATGACCCAAAGTCCAAGTTCCCTTTCAGCATCTGTCGGTGA TCGAGTCACTATAACTTGTAAGGCCAGCCAAGACGTTAGCATAGGAGTAG CATGGTACCAACAAAAGCCTGGGAAGGCTCCCAAACTTCTCATTTATTCT GCTTCCTACCGATATACTGGTGTCCCAAGTAGATTTTCTGGCAGCGGATC TGGAACTGATTTTACATTGACTATCAGCTCCCTCCAGCCTGAGGACTTCG CTACTTATTACTGCCAACAGTACTACATTTATCCCTATACATTCGGTTGT GGGACCAAAGTAGAGATCAAACGTACGGTGGCAGCTCCCAGCCGTTTTTAT CTTTCCCCCATCCGACGAGCAGCTCAAGAGTGGCACTGCCTCTGTAGTTT GTTTGCTGAATAACTTCTATCCACGTGAAGCAAAAGTACAGTGGAAGGTC GATAATGCCCTTCAGAGCGGTAACAGCCAAGAAAGTGTTACCGAGCAAGA TTCCAAAGATTCCACTTACAGTCTGTCCAGCACATTGACACTGAGTAAGG CTGATTACGAAAAACACAAGGTGTACGCATGCGAGGTGACACACCAAGGT CTTTCATCTCCTGTAACTAAGAGCTTTAACCGGGGAGAATGT | 884 |
|  | L1 linker | GAACCAAAGTCTTGT | 848 |
|  | L2 linker | GGTGGTGGGGGCAGCGGGGGCGGAGGTAGTGGAGGCGGCGGGTAGTGAACC AAAGAGTAGT | 849 |
|  | L3 linker | GGAGGCGGCGGCTCT | 850 |
|  | L4 linker | GGAGGCGGCGGCTCT | 850 |
|  | Hinge-Fc (Knob) | GACAAAACTCACACGTGCCCACCGTGCCCAGCACCTGAACTCCTGGGCGG ACCGTCAGTCTTCCTCTTCCCCCCAAAACCCAAGGACACCCTCATGATCT CCCGGACCCCTGAGGTCACATGCGTGGTGGTGGACGTGAGCCACGAAGAC CCTGAGGTCAAGTTCAACTGGTACGTGGACGGCGTGGAGGTGCATAATGC CAAGACAAAGCCGCGGGAGGAGCAGTACAACAGCACGTACCGTGTGGTCA GCGTCCTCACCGTCCTGCACCAGGACTGGCTGAATGGCAAGGAGTACAAG TGCAAGGTCTCCAACAAAGCCCTCCCAGCCCCCATCGAGAAAACCATCTC | 851 |

| | | CAAAGCCAAAGGGCAGCCCCGAGAACCACAGGTGTACACCCTGCCCCCAT GCCGGGAGGAGATGACCAAGAACCAGGTCAGCCTGTGGTGCCTGGTCAAA GGCTTCTATCCCAGCGACATCGCCGTGGAGTGGGAGAGCAATGGGCAGCC GGAGAACAACTACAAGACCACGCCTCCCGTGCTGGACTCCGACGGCTCCT TCTTCCTCTACAGCAAGCTCACCGTGGACAAGAGCAGGTGGCAGCAGGGG AACGTCTTCTCATGCTCCGTGATGCATGAGGCTCTGCACAACCACTACAC GCAGAAGAGCCTCTCCCTGTCTCCGGGTAAA | |
| Hinge-Fc (Hole) | GACAAAACTCACACGTGCCCACCGTGCCCAGCACCTGAACTCCTGGGGGG ACCGTCAGTCTTCCTCTTCCCCCCAAAACCCAAGGACACCCTCATGATCT CCCGGACCCCTGAGGTCACATGCGTGGTGGTGGACGTGAGCCACGAAGAC CCTGAGGTCAAGTTCAACTGGTACGTGGACGGCGTGGAGGTGCATAATGC CAAGACAAAGCCGCGGGAGGAGCAGTACAACAGCACGTACCGTGTGGTCA GCGTCCTCACCGTCCTGCACCAGGACTGGCTGAATGGCAAGGAGTACAAG TGCAAGGTCTCCAACAAAGCCCTCCCAGCCCCCATCGAGAAAACCATCTC CAAAGCCAAAGGGCAGCCCCGAGAACCACAGGTGTGCACCCTGCCCCCAT CCCGGGAGGAGATGACCAAGAACCAGGTCAGCCTGAGCTGCGCGGTCAAA GGCTTCTATCCCAGCGACATCGCCGTGGAGTGGGAGAGCAATGGGCAGCC GGAGAACAACTACAAGACCACGCCTCCCGTGCTGGACTCCGACGGCTCCT TCTTCCTCGTGAGCAAGCTCACCGTGGACAAGAGCAGGTGGCAGCAGGGG AACGTCTTCTCATGCTCCGTGATGCATGAGGCTCTGCACAACCACTACAC GCAGAAGAGCCTCTCCCTGTCTCCGGGTAAA | 458 |
| Z protein(scFv of Trastuzumab) | GAAGTTCAACTTGTGGAGTCTGGAGGCGGTTTGGTACAACCTGGAGGTTC ACTTCGTTTGTCATGTGCTGCCTCTGGGTTTAATATCAAGGATACTTATA TTCATTGGGTTCGCCAGGCACCCGGAAAATGCCTGGAATGGGTGGCCCGT ATATATCCCACTAACGGTTACACTCGATATGCTGACTCTGTAAAGGGTCG ATTTACTATATCCGCTGATACTAGCAAAAACACTGCCTACCTCCAGATGA ACAGTCTGCGCGCCGAGGATACAGCAGTTTATTATTGTTCCCGATGGGGA GGTGACGGGTTCTATGCCATGGACTACTGGGGTCAAGGGACACTGGTAAC CGTTTCTTCTGGCGGAGGGGGGTTCTGGAGGCGGAGGCAGCGGGGGCGGAG | 885 |

| | | | |
|---|---|---|---|
| | | GGTCCGACATTCAGATGACCCAATCCCCCTCTAGCCTTTCAGCCTCCGTA GGGGACAGAGTTACAATAACCTGTAGGGGCTAGTCAGGACGTTAATACAGC AGTCGCATGGTACCAGCAGAAGCCCGGTAAGGCTCCTAAGCTTTTGATCT ACAGTGCTTCTTTCCTGTATTCTGGTGTACCTAGCCGATTCTCAGGGCTCT CGGAGTGGCACAGACTTCACTCTGACAATTTCAAGTCTCCAGCCAGAAGA TTTTGCAACCTACTACTGCCAACAACACTACACTACTCCTCCAACCTTCG GATGTGGCACAAAAGTAGAGATTAAG | |
| ME13 | Fab region (VH-CH1) | CAGGTACAACTTCAAGAAAGTGGTCCAGGTCTTGTAAAACCCTCAGAAAC TTTGAGTCTCACTTGTACCGTCAGTGGCGGAAGTGTAAGTTCTGGCGATT ACTACTGGACCTGGATACGCCAGTCTCCAGGCAAATGTCTGGAGTGGATA GGCCACATCTACTACAGCGGGGAACACCAATTACAATCCATCTCTTAAATC AAGGTTGACAATTTCAATAGACACCAGTAAGACCCAGTTTTCTCTCAAAC TTAGCAGTGTAACAGCAGCAGATACTGCAATCTACTACTGCGTTAGAGAC CGTGTTACAGGGGCTTTCGACATCTGGGGCAGGGAACTATGGTTACCGT CTCTTCTGCTAGCACCAAAGGACCTAGTGTTTTTCCTCTTGCCCCCTTCCT CAAAGTCTACCTCTGGGGGGACAGCCGCTCTGGGCTGCCTGGTCAAGGAT TATTTCCCAGAGCCTGTCACTGTCAGTTGGAACTCTGGAGCCTTGACTTC TGGTGTTCATACATTTCCTGCTGTCCTTCAAAGCAGCGGCTTGTACTCAT TGTCTTCTGTTGTGACAGTACCCTCAAGCAGCCTCGGCACTCAGACATAC ATCTGCAATGTCAACCACAAACCCTCAAATACAAAGGTAGATAAAAAAGT C | 886 |
| | Fab region (VL-CL) | GACATACAGATGACACAGAGTCCTAGCTCATTGTCAGCCTCTGTCGGTGA CCGCGTCACTATCACCTGCCAAGCCAGCCAAGACATATCAAATTATCTTA ACTGGTACCAGCAAAAGCCTGGAAAGGCTCCAAAACTGCTGATTTACGAC GCCTCTAATTTGGAGACCGGGGTTCCCTCTAGGTTCAGCGGGGTCTGGTTC AGGCACCGACTTTACATTCACTATCTCAAGTCTCCAGCCAGAGGACATCG CTACATACTTTTGCCAACATTTTGACCATCTGCCCCTGGCATTTGGGTGT GGCACCAAGGTAGAAATTAAGCGTACGGTGGCAGCTCCCAGCGTTTTTAT CTTTCCCCCATCCGACGAGCAGCTCAAGAGTGGCACTGCCTCTGTAGTTT | 887 |

| | | GTTTGCTGAATAACTTCTATCCACGTGAAGCAAAAGTACAGTGGAAGGTCGATAATGCCCTTCAGAGCGGTAACAGCCAAGAAAGTGTTACCGAGCAAGATTCCAAAGATTCCACTTACAGTCTGTCCAGCACATTGACACTGAGTAAGGCTGATTACGAAAAACACAAGGTGTACGCATGCGAGGTGACACACCAAGGTCTTTCATCTCCTGTAACTAAGAGCTTTAACCGGGGAGAATGT | |
|---|---|---|---|
| L1 linker | GAACCAAAGTCTTGT | 848 |
| L2 linker | GGTGGTGGGGGCAGCGGGGGCGGAGGTAGTGGAGGCGGCGGTAGTGAACCAAAGAGTAGT | 849 |
| L3 linker | GGAGGCGGCGGCTCT | 850 |
| L4 linker | GGAGGCGGCGGCTCT | 850 |
| Hinge-Fc (Knob) | GACAAAACTCACACGTGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCAGCGTCCTCACCGTCCTGCACCAGGACTGGCTGAATGGCAAGGAGTACAAGTGCAAGGTCTCCAACAAAGCCCTCCCAGCCCCCATCGAGAAAACCATCTCCAAAGCCAAAGGGCAGCCCCGAGAACCACAGGTGTACACCCTGCCCCCATGCCGGGAGGAGATGACCAAGAACCAGGTCAGCCTGTGGTGCCTGGTCAAAGGCTTCTATCCCAGCGACATCGCCGTGGAGTGGGAGAGCAATGGGCAGCCGGAGAACAACTACAAGACCACGCCTCCCGTGCTGGACTCCGACGGCTCCTTCTTCCTCTACAGCAAGCTCACCGTGGACAAGAGCAGGTGGCAGCAGGGGAACGTCTTCTCATGCTCCGTGATGCATGAGGCTCTGCACAACCACTACACGCAGAAGAGCCTCTCCCTGTCTCCGGGTAAA | 851 |
| Hinge-Fc (Hole) | GACAAAACTCACACGTGCCCACCGTGCCCAGCACCTGAACTCCTGGGGGGACCGTCAGTCTTCCTCTTCCCCCCAAAACCCAAGGACACCCTCATGATCTCCCGGACCCCTGAGGTCACATGCGTGGTGGTGGACGTGAGCCACGAAGACCCTGAGGTCAAGTTCAACTGGTACGTGGACGGCGTGGAGGTGCATAATGCCAAGACAAAGCCGCGGGAGGAGCAGTACAACAGCACGTACCGTGTGGTCA | 458 |

| | | | |
|---|---|---|---|
| | | GCGTCCTCACCGTCCTGCACCAGGACTGGCTGAATGGCAAGGAGTACAAG TGCAAGGTCTCCAACAAAGCCCTCCCAGCCCCCATCGAGAAAACCATCTC CAAAGCCAAAGGGCAGCCCCGAGAACCACAGGTGTGCACCCTGCCCCCCAT CCCGGGAGGAGATGACCAAGAACCAGGTCAGCCTGAGCTGCGCGGTCAAA GGCTTCTATCCCAGCGACATCGCCGTGGAGTGGGAGAGCAATGGGCAGCC GGAGAACAACTACAAGACCACGCCTCCCGTGCTGGACTCCGACGGCTCCT TCTTCCTCGTGAGCAAGCTCACCGTGGACAAGAGCAGGTGGCAGCAGGGG AACGTCTTCTCATGCTCCGTGATGCATGAGGCTCTGCACAACCACTACAC GCAGAAGAGCCTCTCCCTGTCTCCGGGTAAA | |
| | Z protein(scFv of Onartuzumab) | GAAGTCCAATTGGTCGAGAGTGGAGGCGGGTTGGTACAGCCAGGAGGTTC ACTTAGGTTGTCCTGCGCCGCCAGTGGTTACACTTTCACATCTTATTGGC TGCACTGGGTGCGCCAAGCTCCTGGGAAGTGTCTCGAATGGGTGGGTATG ATCGATCCATCTAATTCCGACACACGGTTTAACCCAAATTTTAAGGATAG ATTTACAATTAGTGCTGACACTTCAAAAAACACAGCATACCTCCAGATGA ACAGCCTGCGTGCTGAGGATACTGCTGTCTACTACTGTGCAACTTACCGC TCCTATGTCACACCTTTGGATTATTGGGGCCAGGGGACTCTGGTGACCGT GAGTTCTGGCGGAGGGGGTTCTGGAGGCGGAGGCAGCGGGGGCGGAGGGT CCGATATACAGATGACACAAAGTCCCTCATCACTTTCTGCCTCCGTTGGA GATCGTGTGACCATTACCTGTAAGAGTTCCCAATCACTGCTTTATACCTC TTCACAAAAAAATTACCTCGCTTCGTACCAGCAGAAGCCAGGTAAAGCAC CTAAGCTGTTGATCTATTGGGCCTCCACTAGAGAGTCAGGCGTGCCCAGC CGTTTCTCCGGTTCAGGGAGTGGGACAGACTTTACCTTGACCATTCTTC TTTGCAACCTGAAGACTTCGCCACATACTATTGTCAGCAATATTACGCAT ATCCATGGACCTTTGGGTGTGGAACCAAAGTCGAAATAAAA | 888 |
| ME14 | Fab region (VH-CH1) | CAGGTACAACTTCAAGAAAGTGGTCCACGTCTTGTAAAACCCTCAGAAAC TTTGAGTCTCACTTGTACCGTCAGTGGCGGAAGTGTAAGTTCTGGCGATT ACTACTGGACCTGGATACGCCAGTCTCCAGGCAAATGTCTGGAGTGGATA GGCCACATCTACTACAGCGGGAACACCAATTACAATCCATCTCTTAAATC AAGGTTGACAATTTCAATAGACACCAGTAAGACCCAGTTTTTCTCTCAAAC | 886 |

| | | | |
|---|---|---|---|
| | | TTAGCAGTGTAACAGCAGCAGATACTGCAATCTACTACTGCGTTAGAGAC CGTGTTACAGGGGCTTTCGACATCTCGCGGCAGGGAACTATGGTTACCGT CTCTTCTGCTAGCACCAAAGGACCTAGTGTTTTTTCCTCTTGCCCCTTCCT CAAAGTCTACCTCTCGGGGGACAGCCGCTCTGGGCTGCCTGGTCAAGGAT TATTTCCCAGAGCCTGTCACTGTCAGTTGGAACTCTGGAGCCTTGACTTC TGGTGTTCATACATTTCCTGCTGTCCTTCAAAGCAGCGGCTTGTACTCAT TGTCTTCTGTTGTGACAGTACCCTCAAGCAGCCTCGGCACTCAGACATAC ATCTGCAATGTCAACCACAAACCCTCAAATACAAAGGTAGATAAAAAAGT C | |
| Fab region (VL-CL) | GACATACAGATGACACAGAGTCCTAGCTCATTGTCAGCCTCTGTCGGTGA CCGCGTCACTATCACCTGCCAAGCCAGCCAAGACATATCAAATTATCTTA ACTGGTACCAGCAAAAGCCTGGAAAGGCTCCAAAACTGCTGATTTACGAC GCCTCTAATTTGGAGACCGGGGTTCCCTCTAGGTTCAGCGGGTCTGGTTC AGGCACCGACTTTACATTCACTATCTCAAGTCTCCAGCCAGAGGACATCG CTACATACTTTTGCCAACATTTTGACCATCTGCCCCTGGCATTTGGGTGT GGCACCAAGGTAGAAATTAAGCGTACGGTGGCAGCTCCCAGCGTTTTTAT CTTTCCCCCATCCGACGAGCAGCTCAAGAGTGGCACTGCCTCTGTAGTTT GTTTGCTGAATAACTTCTATCCACGTGAAGCAAAAGTACAGTGGAAGGTC GATAATGCCCTTCAGAGCGGTAACAGCCAAGAAAGTGTTACCGAGCAAGA TTCCAAAGATTCCACTTACAGTCTGTCCAGCACATTGACACTGAGTAAGG CTGATTACGAAAAACACAAGGTGTACGCATGCGAGGTGACACACCAAGGT CTTTCATCTCCTGTAACTAAGAGCTTTAACCGGGGAGAATGT | 887 |
| L1 linker | GAACCAAAGTCTTGT | 848 |
| L2 linker | GGTGGTGGCGGCAGCGGGGGCGGCAGGTAGTGGAGGCGGCGGTAGTGAACC AAAGAGTAGT | 849 |
| L3 linker | GGAGGCGGCGGCTCT | 850 |
| L4 linker | GGAGGCGGCGGCTCT | 850 |
| Hinge-Fc (Knob) | GACAAAACTCACACGTGCCCACCGTGCCCAGCACCTGAACTCCTGGGGGG ACCGTCAGTCTTCCTCTTCCCCCCAAAACCCAAGGACACCCTCATGATCT | 851 |

| | | CCCGGACCCCTGAGGTCACATGCGTGGTGGTGGACGTGAGCCACGAAGAC CCTGAGGTCAAGTTCAACTGGTACGTGGACGGCGTGGAGGTGCATAATGC CAAGACAAAGCCGCGGGAGGAGCAGTACAACAGCACGTACCGTGTGGTCA GCGTCCTCACCGTCCTGCACCAGGACTGGCTGAATGGCAAGGAGTACAAG TGCAAGGTCTCCAACAAAGCCCTCCCAGCCCCCATCGAGAAAACCATCTC CAAAGCCAAAGGGCAGCCCCGAGAACCACAGGTGTACACCCTGCCCCCAT GCCGGGAGGAGATGACCAAGAACCAGGTCAGCCTGTGGTGCCTGGTCAAA GGCTTCTATCCCAGCGACATCGCCGTGGAGTGGGAGAGCAATGGGCAGCC GGAGAACAACTACAAGACCACGCCTCCCGTGCTGGACTCCGACGGCTCCT TCTTCCTCTACAGCAAGCTCACCGTGGACAAGAGCAGGTGGCAGCAGGGG AACGTCTTCTCATGCTCCGTGATGCATGAGGCTCTGCACAACCACTACAC GCAGAAGAGCCTCTCCCTGTCTCCGGGTAAA | |
| Hinge-Fc (Hole) | GACAAAACTCACACGTGCCCACCGTGCCCAGCACCTGAACTCCTGGGGGG ACCGTCAGTCTTCCTCTTCCCCCCAAAACCCAAGGACACCCTCATGATCT CCCGGACCCCTGAGGTCACATGCGTGGTGGTGGACGTGAGCCACGAAGAC CCTGAGGTCAAGTTCAACTGGTACGTGGACGGCGTGGAGGTGCATAATGC CAAGACAAAGCCGCGGGAGGAGCAGTACAACAGCACGTACCGTGTGGTCA GCGTCCTCACCGTCCTGCACCAGGACTGGCTGAATGGCAAGGAGTACAAG TGCAAGGTCTCCAACAAAGCCCTCCCAGCCCCCATCGAGAAAACCATCTC CAAAGCCAAAGGGCAGCCCCGAGAACCACAGGTGTGCACCCTGCCCCCAT CCCGGGAGGAGATGACCAAGAACCAGGTCAGCCTGAGCTGCGCGGTCAAA GGCTTCTATCCCAGCGACATCGCCGTGGAGTGGGAGAGCAATGGGCAGCC GGAGAACAACTACAAGACCACGCCTCCCGTGCTGGACTCCGACGGCTCCT TCTTCCTCGTGAGCAAGCTCACCGTGGACAAGAGCAGGTGGCAGCAGGGG AACGTCTTCTCATGCTCCGTGATGCATGAGGCTCTGCACAACCACTACAC GCAGAAGAGCCTCTCCCTGTCTCCGGGTAAA | 458 |
| Z protein(scFv of CE-355621) | CAAGTGCAGCTTGTTCAATCAGGGGCCGAGGTTAAGAAACCAGGTGCTTC CGTCAAGGTCTCCTGCAAGGCTTCCGGCTACACTTTTACCAGTTATGGGT TCAGTTGGGGTTAGACAGGCCCCAGGGCAGTGTCTCGAATGGATGGGATGG | 889 |

| | | | |
|---|---|---|---|
| | | ATTTCCGCATCTAACGGGAATACTTACTATGCCCAGAAACTTCAAGGTAG GGTTACCATGACTACCGATACTTCCACTAGTACAGCCTACATGGAACTCA GATCACTCCGTTCAGATGACACCCGCAGTATATTACTGTGCAAGGGTATAT GCTGATTATGCCGATTATTGGGGGCAAGCAACACTTGTCACAGTATCCAG CCGCCGGAGGCGGTTCTGCAGCCGGCACGCAGCCGGGGGCGGAGGGTCCGATA TCCAAATGACACAGTCACCCTCAAGTGTTAGCGCAAGTGTGGGGGACAGG GTGACCATCACATGCAGAGCTTCCCAGGGTATCAATACAATGGCTGGCATG GTATCAACAGAAACCCGGAAAAGCACCAAAATTGCTTATTTATGCAGCTT CTAGCTTGAAGAGTGGGGTTCCCTCTCGTTTCTCTGGTTCAGGAAGCGGT ACTGACTTTACCTTGACCATCAGTAGCTTGCAGCCCGAAGATTTCGCTAC ATATTATTGCCAACAGGCCAACTCTTTTCCCCTGACATTCGGTTCTGGCA CTAAAGTGGAAATTAAG | |
| ME15 | Fab region (VH-CH1) | GAAGTCCAATTGGTCGAGAGTGGAGGCGGGTTGGTACAGCCAGGAGGTTC ACTTAGGTTGTCCTGCGCCGCCAGTGGTTACACTTTCACATCTTATTGGC TGCACTGGGTGCGCCAAGCTCCTGGGAAGTGTCTCGAATGGGTGGGTATG ATCGATCCATCTAATTCCGACACACGGTTTAACCCAAATTTTAACGGATAG ATTTACAATTAGTGCTGACACTTCAAAAAACACAGCATACCTCCAGATGA ACAGCCTGCGTGCTGAGGATACTGCTGTCTACTACTGTGCAACTTACCGC TCCTATGTCACACCTTTGGATTATTGGGGCCAGGGGACTCTGGTGACCGT GAGTTCTGCTAGCACCAAAGGACCTAGTGTTTTTCCTCTTGCCCCTTCCT CAAAGTCTACCTCTGGGGGGACAGCCGCTCTGGGCTGCCTGGTCAAGGAT TATTTCCCAGAGCCTGTCACTGTCAGTTGGAACTCTGGAGCCTTGACTTC TGGTGTTCATACATTTCCTGCTGTCCTTCAAAGCAGCGGCTTGTACTCAT TGTCTTCTGTTGTGACAGTACCCTCAAGCAGCCTCGGCACTCAGACATAC ATCTGCAATGTCAACCACAAACCCTCAAATACAAAGGTAGATAAAAAAGT C | 890 |
| | Fab region (VL-CL) | GATATACAGATGACACAAAGTCCCTCATCACTTTCTGCCTCCGTTCGAGA TCGTGTGACCATTACCTGTAAGAGTTCCCAATCACTGCTTTATACCTCTT CACAAAAAAAATTACCTCGCTTGGTACCAGCAGAAGCCAGGTAAAGCACCT | 891 |

| | | | |
|---|---|---|---|
| | | AAGCTGTTGATCTATTGGGCCTCCACTAGAGAGTCAGGCGTGCCCAGCCG TTTCTCCGGTTCAGGGAGTGGGACAGACTTTACCTTGACCATTTCTTCTT TGCAACCTGAAGACTTCGCCACATACTATTGTCAGCAATATTACGCATAT CCATGGACCTTTGGGTGTGGAACCAAAGTCGAAATAAAACGTACGGTGGC AGCTCCCAGCGTTTTTATCTTTCCCCCATCCGACGAGCAGCTCAAGAGTG GCACTGCCTCTGTAGTTTGTTTGCTGAATAACTTCTATCCACGTGAAGCA AAAGTACAGTGGAAGGTCGATAATGCCCTTCAGAGCGGTAACAGCCAAGA AAGTGTTACCGAGCAAGATTCCAAAGATTCCACTTACAGTCTGTCCAGCA CATTGACACTGAGTAAGGCTGATTACGAAAAACACAAGGTGTACGCATGC GAGGTGACACACCAAGGTCTTTCATCTCCTGTAACTAAGAGCTTTAACCG GGGAGAATGT | |
| L1 linker | GAACCAAAGTCTTGT | 848 |
| L2 linker | GGTGGTGGGGGCAGCGGGGGCGGAGGTAGTGGAGGCGGCGGTAGTGAACC AAAGAGTAGT | 849 |
| L3 linker | GGAGGCGGCGGCTCT | 850 |
| L4 linker | GGAGGCGGCGGCTCT | 850 |
| Hinge-Fc (Knob) | GACAAAACTCACACGTGCCCACCGTGCCCAGCACCTGAACTCCTGGGGGG ACCGTCAGTCTTCCTCTTCCCCCCAAAACCCAAGGACACCCTCATGATCT CCCGGACCCCTGAGGTCACATGCGTGGTGGTGGACGTGAGCCACGAAGAC CCTGAGGTCAAGTTCAACTGGTACGTGGACGGCGTGGAGGTGCATAATGC CAAGACAAAGCCGCGGGAGGAGCAGTACAACAGCACGTACCGTGTGGTCA GCGTCCTCACCGTCCTGCACCAGGACTGGCTGAATGGCAAGGAGTACAAG TGCAAGGTCTCCAACAAAGCCCTCCCAGCCCCCATCGAGAAAACCATCTC CAAAGCCAAAGGGCAGCCCCGAGAACCACAGGTGTACACCCTGCCCCCAT GCCGGGAGGAGATGACCAAGAACCAGGTCAGCCTGTGGTGCCTGGTCAAA GGCTTCTATCCCAGCGACATCGCCGTGGAGTGGGAGAGCAATGGGCAGCC GGAGAACAACTACAAGACCACGCCTCCCGTGCTGGACTCCGACGGCTCCT TCTTCCTCTACAGCAAGCTCACCGTGGACAAGAGCAGGTGGCAGCAGGGG AACGTCTTCTCATGCTCCGTGATGCATGAGGCTCTGCACAACCACTACAC | 851 |

| | | | | |
|---|---|---|---|---|
| | | | GCAGAAGAGCCTCTCCCTGTCTCCGGGTAAA | |
| | | Hinge-Fc (Hole) | GACAAAACTCACACGTGCCCACCGTGCCCAGCACCTGAACTCCTGGGGGG ACCGTCAGTCTTCCTCTTCCCCCCAAAACCCAAGGACACCCTCATGATCT CCCGGACCCCTGAGGTCACATGCGTGGTGGTGGACGTGAGCCACGAAGAC CCTGAGGTCAAGTTCAACTGGTACGTGGACGGCGTGGAGGTGCATAATGC CAAGACAAAGCCGCGGGAGGAGCAGTACAACAGCACGTACCGTGTGGTCA GCGTCCTCACCGTCCTGCACCAGGACTGGCTGAATGGCAAGGAGTACAAG TGCAAGGTCTCCAACAAAGCCCTCCCAGCCCCCATCGAGAAAACCATCTC CAAAGCCAAAGGGCAGCCCCGAGAACCACAGGTGTGCACCCTGCCCCCAT CCCGGGAGGAGATGACCAAGAACCAGGTCAGCCTGAGCTGCGCGGTCAAA GGCTTCTATCCCAGCGACATCGCCGTGGAGTGGGAGAGCAATGGGCAGCC GGAGAACAACTACAAGACCACGCCTCCCGTGCTGGACTCCGACGGCTCCT TCTTCCTCGTGAGCAAGCTCACCGTGGACAAGAGCAGGTGGCAGCAGGGG AACGTCTTCTCATGCTCCGTGATGCATGAGGCTCTGCACAACCACTACAC GCAGAAGAGCCTCTCCCTGTCTCCGGGTAAA | 458 | |
| | | Z protein(scFv of Panitumumab) | CAGGTACAACTTCAAGAAAGTGGTCCAGGTCTTGTAAAACCCTCAGAAAC TTTGAGTCTCACTTGTACCGTCAGTGGCGGAAGTGTAAGTTCTGGCGATT ACTACTGGACCTGGATACGCCAGTCTCCAGGCAAATGTCTGGAGTGGATA GGCCACATCTACTACAGCGGGAACACCAATTACAATCCATCTCTTAAATC AAGGTTGACAATTTCAATAGACACCAGTAAGACCCAGTTTTCTCTCAAAC TTAGCAGTGTAACAGCAGCAGATACTGCAATCTACTACTGCGTTAGAGAC CGTGTTACAGGGGCTTTCGACATCTGGGGGCAGGGAACTATGGTTACCGT CTCTTCTGGCGGAGGGGGGTTCTGGAGGCGGAGGCAGCGGGGGCGGAGGGT CCGACATACAGATGACACAGAGTCCTAGCTCATTGTCAGCCTCTGTCGGT GACCGCGTCACTATCACCTGCCAAGCCAGCCAAGACATATCAAATTATCT TAACTGGTACCAGCAAAAGCCTGGAAAGGCTCCAAAACTGCTGATTTACG ACGCCTCTAATTTGGAGACCGGGGTTCCCTCTAGGTTCAGCGGGTCTGGT TCAGGCACCGACTTTACATTCACTATCTCAAGTCTCCAGCCAGAGGACAT CGCTACATACTTTTGCCAACATTTTGACCATCTGCCCCTGGCATTTGGGT | 892 | |

| | | | |
|---|---|---|---|
| | | GTGGCACCAAGGTAGAAATTAAG | |
| ME16 | Fab region (VH-CH1) | CAAGTGCAGCTTGTTCAATCAGGGGGCCGAGGTTAAGAAACCAGGTGCTTC CGTCAAGGTCTCCTGCAAGGCTTCCGGCTACACTTTTACCAGTTATGGGT TCAGTTGGGTTAGACAGGCCCCAGGGCAGTGTCTCGAATGGATGGGATGG ATTTCCGCATCTAACGGGAATACTTACTATGCCCAGAAACTTCAAGGTAG GGTTACCATGACTACCGATACTTCCACTAGTACAGCCTACATGGAACTCA GATCACTCCGTTCAGATGACACCGCAGTATATTACTGTGCAAGGGTATAT GCTGATTATGCCGATTATTGGGGGCAAGGAACACTTGTCACAGTATCCAG CGCTAGCACCAAAGGACCTAGTGTTTTTCCTCTTGCCCCTTCCTCAAAGT CTACCTCTGGGGGGACAGCCGCTCTGGGCTGCCTGGTCAAGGATTATTTC CCAGAGCCTGTCACTGTCAGTTGGAACTCTGGAGCCTTGACTTCTGGTGT TCATACATTTCCTGCTGTCCTTCAAAGCAGCGGCTTGTACTCATTGTCTT CTGTTGTGACAGTACCCTCAAGCAGCCTCGGCACTCAGACATACATCTGC AATGTCAACCACAAACCCTCAAATACAAAGGTAGATAAAAAAGTC | 893 |
| | Fab region (VL-CL) | GATATCCAAATGACACAGTCACCCTCAAGTGTTAGCGCAAGTGTCGGCGA CACGGGTGACCATCACATGCAGAGCTTCCCAGGGTATCAATACATCGCTGG CATGGTATCAACAGAAACCCGGAAAAGCACCAAAATTGCTTATTTATGCA GCTTCTAGCTTGAAGAGTGGGGTTCCCTCTCGTTTCTCTGGTTCAGGAAG CGGTACTGACTTTACCTTGACCATCAGTAGCTTGCAGCCCGAAGATTTCG CTACATATTATTGCCAACACGCCAACTCTTTTCCCCTGACATTCGGTTGT GGCACTAAAGTGGAAATTAAGCGTACGGTGGCAGCTCCCAGCCGTTTTTAT CTTTCCCCCATCCGACGAGCAGCTCAAGAGTGGCACTGCCTCTGTAGTTT GTTTGCTGAATAACTTCTATCCACGTGAAGCAAAAGTACAGTGGAAGGTC GATAATGCCCTTCAGAGCGGTAACAGCCAAGAAAGTGTTACCGAGCAAGA TTCCAAAGATTCCACTTACAGTCTGTCCAGCACATTGACACTGAGTAAGG CTGATTACGAAAAACACAAGGTGTACGCATGCGAGGTGACACACCAACGT CTTTCATCTCCTGTAACTAAGAGCTTTAACCGGGGAGAATGT | 894 |
| | L1 linker | GAACCAAAGTCTTGT | 848 |
| | L2 linker | GGTGGTGGGGGCAGCGGGGGCCGGAGGTAGTGGACGGCGGCGGTAGTGAACC | 849 |

| | | | |
|---|---|---|---|
| | | AAAGAGTAGT | |
| | L3 linker | GGAGGCGGCGGCTCT | 850 |
| | L4 linker | GGAGGCGGCGGCTCT | 850 |
| | Hinge-Fc (Knob) | GACAAAACTCACACGTGCCCACCGTGCCCAGCACCTGAACTCCTGGGGGG ACCGTCAGTCTTCCTCTTCCCCCCAAAACCCAAGGACACCCTCATGATCT CCCGGACCCCTGAGGTCACATGCGTGGTGGTGGACGTGAGCCACGAAGAC CCTGAGGTCAAGTTCAACTGGTACGTGGACGGCGTGGAGGTGCATAATGC CAAGACAAAGCCGCGGGAGGAGCAGTACAACAGCACGTACCGTGTGGTCA GCGTCCTCACCGTCCTGCACCAGGACTGGCTGAATGGCAAGGAGTACAAG TGCAAGGTCTCCAACAAAGCCCTCCCAGCCCCCATCGAGAAAACCATCTC CAAAGCCAAAGGGCAGCCCCGAGAACCACAGGTGTACACCCTGCCCCCAT GCCGGGAGGAGATGACCAAGAACCAGGTCAGCCTGTGGTGCCTGGTCAAA GGCTTCTATCCCAGCGACATCGCCGTGGAGTGGGAGAGCAATGGGCAGCC GGAGAACAACTACAAGACCACGCCTCCCGTGCTGGACTCCGACGGCTCCT TCTTCCTCTACAGCAAGCTCACCGTGGACAAGAGCAGGTGGCAGCAGGGG AACGTCTTCTCATGCTCCGTGATGCATGAGGCTCTGCACAACCACTACAC GCAGAAGAGCCTCTCCCTGTCTCCGGGTAAA | 851 |
| | Hinge-Fc (Hole) | GACAAAACTCACACGTGCCCACCGTGCCCAGCACCTGAACTCCTGGGGGG ACCGTCAGTCTTCCTCTTCCCCCCAAAACCCAAGGACACCCTCATGATCT CCCGGACCCCTGAGGTCACATGCGTGGTGGTGGACGTGAGCCACGAAGAC CCTGAGGTCAAGTTCAACTGGTACGTGGACGGCGTGGAGGTGCATAATGC CAAGACAAAGCCGCGGGAGGAGCAGTACAACAGCACGTACCGTGTGGTCA GCGTCCTCACCGTCCTGCACCAGGACTGGCTGAATGGCAAGGAGTACAAG TGCAAGGTCTCCAACAAAGCCCTCCCAGCCCCCATCGAGAAAACCATCTC CAAAGCCAAAGGGCAGCCCCGAGAACCACAGGTGTGCACCCTGCCCCCAT CCCGGGAGGAGATGACCAAGAACCAGGTCAGCCTGAGCTGCGCGGTCAAA GGCTTCTATCCCAGCGACATCGCCGTGGAGTGGGAGAGCAATGGGCAGCC GGAGAACAACTACAAGACCACGCCTCCCGTGCTGGACTCCGACGGCTCCT TCTTCCTCGTGAGCAAGCTCACCGTGGACAAGAGCAGGTGGCAGCAGGGG | 458 |

| | | | |
|---|---|---|---|
| | | AACGTCTTCTCATGCTCCGTGATGCATGAGGCTCTGCACAACCACTACAC GCAGAAGAGCCTCTCCCTGTCTCCGGGTAAA | |
| | Z protein(scFv of Panitumumab) | CAGGTACAACTTCAAGAAAGTGGTCCAGGTCTTGTAAAACCCTCAGAAAC TTTGAGTCTCACTTGTACCGTCAGTGGCGGAAGTGTAAGTTCTGGCGATT ACTACTGGACCTGGATACGCCAGTCTCCAGGCAAATGTCTGGAGTGGATA GGCCACATCTACTACAGCGGGAACACCAATTACAATCCATCTCTTAAATC AAGGTTGACAATTTCAATAGACACCAGTAAGACCCAGTTTTCTCTCAAAC TTAGCAGTGTAACAGCAGCAGATACTGCAATCTACTACTGCGTTAGAGAC CGTGTTACAGGGGGCTTTGACATCTGGGGGCAGGGAACTATGGTTACCGT CTCTTCTGGCGGAGGGGGTTCTGGAGGCGGAGGCAGCGGGGGCGGAGGGT CCGACATACAGATGACACAGAGTCCTAGCTCATTGTCAGCCTCTGTCGGT GACCGCGTCACTATCACCTGCCAAGCCAGCCAAGACATATCAAATTATCT TAACTGGTACCAGCAAAAGCCTGGAAAGGCTCCAAAACTGCTGATTTACG ACGCCTCTAATTTGGAGACCGGGGTTCCCTCTAGGTTCAGCGGGTCTGGT TCAGGCACCGACTTTACATTCACTATCTCAAGTCTCCAGCCAGAGGACAT CGCTACATACTTTTGCCAACATTTTGACCATCTGCCCCTGGCATTTGGGT GTGGCACCAAGGTAGAAATTAAG | 892 |
| CE05 | Fab region (VH-CH1) | CAGGTACAACTTCAAGAAAGTGGTCCAGGTCTTGTAAAACCCTCAGAAAC TTTGAGTCTCACTTGTACCGTCAGTGGCGGAAGTGTAAGTTCTGGCGATT ACTACTGGACCTGGATACGCCAGTCTCCAGGCAAATGTCTGGAGTGGATA GGCCACATCTACTACAGCGGGAACACCAATTACAATCCATCTCTTAAATC AAGGTTGACAATTTCAATAGACACCAGTAAGACCCAGTTTTCTCTCAAAC TTAGCAGTGTAACAGCAGCAGATACTGCAATCTACTACTGCGTTAGAGAC CGTGTTACAGGGGGCTTTGACATCTGGGGGCAGGGAACTATGGTTACCGT CTCTTCTGCTAGCACCAAAGGACCTAGTGTTTTTCCTCTTGCCCCCTTCCT CAAAGTCTACCTCTGGGGGGACAGCCGCTCTGGGCTGCCTGGTCAAGGAT TATTTCCCAGAGCCTGTCACTGTCAGTTGGAACTCTGGAGCCTTGACTTC TGGTGTTCATACATTTCCTGCTGTCCTTCAAAGCAGCGGCTTGTACTCAT TGTCTTCTGTTGTGACAGTACCCTCAAGCAGCCTCGGCACTCAGACATAC | 886 |

| | | ATCTGCAATGTCAACCACAAACCCTCAAATACAAAGGTAGATAAAAAAGT C | |
|---|---|---|---|
| Fab region (VL-CL) | | GACATACAGATGACACAGAGTCCTAGCTCATTGTCAGCCTCTGTCCGTGA CCGCCGTCACTATCACCTGCCAAGCCAGCCAAGACATATCAAATTATCTTA ACTGGTACCAGCAAAAGCCTGGAAAGGCTCCAAAACTGCTGATTTACGAC GCCTCTAATTTTGGAGACCGGGGTTCCCTCTAGGTTCAGCGGGTCTGGTTC AGGCACCGACTTTACATTCACTATCTCAAGTCTCCAGCCAGAGGACATCG CTACATACTTTTGCCAACATTTTGACCATCTGCCCCTGGCATTTCGGTGT CGGCACCAAGGTAGAAATTAAGCCGTACGGTGGCAGCTCCCAGCCGTTTTTAT CTTTCCCCCATCCGACGAGCAGCTCAAGAGTGGCACTGCCTCTGTAGTTT GTTTGCTGAATAACTTCTATCCACGTGAAGCAAAAGTACAGTGGAAGGTC GATAATGCCCTTCAGACACGGTAACAGCCAAGAAAGTGTTACCGAGCAAGA TTCCAAAGATTCCACTTACAGTCTGTCCAGCACATTGACACTGAGTAAGG CTGATTACGAAAAACACAAGGTGTACGCATGCGAGGTGACACACCAAGGT CTTTCATCTCCTGTAACTAAGAGCTTTAACCGGGGAGAATGT | 887 |
| L1 linker | | GAACCAAAGTCTTGT | 848 |
| L2 linker | | CGTCGTCGCCGCAGCCGCGGCCGGAGGTAGTGGAGGCCGCCGTAGTGAACC AAAGAGTAGT | 849 |
| L3 linker | | GGAGGCCGCCGCCTCT | 850 |
| L4 linker | | CGAGGCCGCCGCCTCT | 850 |
| Hinge-Fc (Knob) | | GACAAAACTCACACCTGCCCACCGTGCCCAGCACCTGAACTCCTCGGGGG ACCGTCAGTCTTCCTCTTCCCCCCAAAACCCAAGGACACCCTCATCGATCT CCCCGGACCCCTGAGGTCACATGCGTGGTGGTGGACGTGACCCACGAAGAC CCTGACGTCAAGTTCAACTCGTACGTGCACGGCGTGGAGGTGCATAATGC CAAGACAAAGCCGCGGGAGGAGCAGTACAACAGCACGTACCGTGTGGTCA GCGTCCTCACCGTCCTGCACCAGGACTGGCTGAATGGCAAGGAGTACAAG TGCAAGGTCTCCAACAAAGCCCTCCCAGCCCCCATCGAGAAAACCATCTC CAAAGCCAAAGGGCAGCCCCGAGAACCACAGGTGTACACCCTGCCCCCAT GCCCGGAGGAGATGACCAAGAACCAGGTCAGCCTGTCGTGCCTGGTCAAA | 851 |

| | | GGCTTCTATCCCAGCGACATCGCCGTGGAGTGGGAGAGCAATGGGCAGCC GGAGAACAACTACAAGACCACGCCTCCCGTGCTGGACTCCGACGGCTCCT TCTTCCTCTACAGCAAGCTCACCGTGGACAAGAGCAGGTGGCAGCAGGGG AACGTCTTCTCATGCTCCGTGATGCATGAGGCTCTGCACAACCACTACAC GCAGAAGAGCCTCTCCCTGTCTCCGGGTAAA | |
|---|---|---|---|
| | Hinge-Fc (Hole) | GACAAAACTCACACGTGCCCACCGTGCCCAGCACCTGAACTCCTGGGGGG ACCGTCAGTCTTCCTCTTCCCCCCAAAACCCAAGGACACCCTCATGATCT CCCGGACCCCTGAGGTCACATGCGTGGTGGTGGACGTGAGCCACGAAGAC CCTGAGGTCAAGTTCAACTGGTACGTGGACGGCGTGGAGGTGCATAATGC CAAGACAAAGCCGCGGGAGGAGCAGTACAACAGCACGTACCGTGTGGTCA GCGTCCTCACCGTCCTGCACCAGGACTGGCTGAATGGCAAGGAGTACAAG TGCAAGGTCTCCAACAAAGCCCTCCCAGCCCCCATCGAGAAAACCATCTC CAAAGCCAAAGGGCAGCCCCGAGAACCACAGGTGTGCACCCTGCCCCCAT CCCGGGAGGAGATGACCAAGAACCAGGTCAGCCTGAGCTGCGCGGTCAAA GGCTTCTATCCCAGCGACATCGCCGTGGAGTGGGAGAGCAATGGGCAGCC GGAGAACAACTACAAGACCACGCCTCCCGTGCTGGACTCCGACGGCTCCT TCTTCCTCGTGAGCAAGCTCACCGTGGACAAGAGCAGGTGGCAGCAGGGG AACGTCTTCTCATGCTCCGTGATGCATGAGGCTCTGCACAACCACTACAC GCAGAAGAGCCTCTCCCTGTCTCCGGGTAAA | 458 |
| | Z protein(scFv of Labetuzumab) | GAAGTGCAACTCGTAGAATCCGGCGGGGGCGTCGTACAGCCCGGCCGTTC TTTGAGACTTAGCTGTAGTGCTTCTGGGTTTGACTTCACTACATACTGGA TGTCATGGGTAAGACAGGCACCTGGCAAGTGCCTTGAATGGATCGGTGAA ATCCATCCCGACAGCTCCACAATCAACTACGCCCCAAGTTTGAAAGACCG GTTCACCATATCTCGTGACAACGCCAAGAATACATTGTTCCTTCAGATGG ATAGTCTTCGTCCAGAGGATACTGGGGTATATTTTTGTGCCAGCTTGTAT TTTGGCTTCCCCTGGTTCGCTTATTGGGGCCAAGGTACACCCGTCACTGT CTCTTCTGGCGGAGGGGGTTCTGGAGGCGGAGGCAGCGGGGGCGGAGGGT CCGACATCCAACTTACCCAGTCACCCTCATCTCTTAGTGCCTCTGTAGGG GACCGAGTTACTATTACATGTAAAGCCAGTCAAGATGTTGGCACCTCAGT | 895 |

|  |  | AGCATGGTATCAACAAAAGCCTGGTAAAGCCCCAAAACTGCTGATCTATT GGACAAGCACACGACATACAGGAGTGCCAAGTCGCTTCAGCGGTTCAGGT TCAGGCACAGATTTTACATTCACTATATCAAGCCTGCAACCCGAGGACAT TGCCACATATTACTGCCAGCAATATAGTCTGTATCGTAGCTTCGGATGTG GCACCAAGGTTGAAATTAAG |  |

Table 97 below shows each polypeptide sequence constituting the novel antibody format.

[Table 97]

| Name | Polypeptide | Sequence | SEQ ID NO |
|---|---|---|---|
| HO1P | Polypeptide 1 | EVQLVESGGGLVQPGGSLRLSCAASGFNIKDTYIHWVRQAPGKCLEWVARIYPTNGYTRYADSVKGRFTISADTSKNTAYLQMNSLRAEDTAVYYCSRWGGDGFYAMDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 14 |
| | Polypeptide 2 | DIQMTQSPSSLSASVGDRVTITCRASQDVNTAVAWYQQKPGKAPKLLIYSASFLYSGVPSRFSGSRSGTDFTLTISSLQPEDFATYYCQQHYTTPPTFGQGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGECGGGGSGGGGSGGGGSEPKSSDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 15 |
| | Polypeptide 3 | EVQLVESGGGLVQPGGSLRLSCAASGFTFTDYTMDWVRQAPGKCLEWVADVNPNSGGSIYNQRFKGRFTLSVDRSKNTLYLQMNSLRAEDTAVYYCARNLGPSFYFDYWGQGTLVTVSSGGGGSGGGGSGGGGSDIQMTQSPSSLSASVGDRVTITCKASQDVSIGVAWYQQKPGKAPKLLIYSASYRYTGVPSRFSGSGSGTDFTLTISSLQPEDFATYYCQQYYIYPYTFGCGTKVEIKGGGGSDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVCTLPPSREEMTKNQVSLSCAVKGFYPSDIAVEWESNGQPENNYKTTPPVLDSDGSFFLVSKLTVDKSRWQQGNVFSCSVMHEALHNHYTQKSLSLSPGK | 896 |

| | Polypeptide 4 | EVQLVESGGGLVQPGGSLRLSCAASGFTFTDYTMDWVRQAPGKCLEWVADVNPN SGGSIYNQRFKGRFTLSVDRSKNTLYLQMNSLRAEDTAVYYCARNLGPSFYFDY WGQGTLVTVSSGGGGSGGGGSGGGGSDIQMTQSPSSLSASVGDRVTITCKASQD VSIGVAWYQQKPGKAPKLLIYSASYRYTGVPSRFSGSGSGTDFTLTISSLQPED FATYYCQQYYIYPYTFGCGTKVEIKGGGGSDKTHTCPPCPAPELLGGPSVFLFP PKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVCTLP PSREEMTKNQVSLSCAVKGFYPSDIAVEWESNGQPENNYKTTPPVLDSDGSFFL VSKLTVDKSRWQQGNVFSCSVMHEALHNHYTQKSLSLSPGK | 896 |
|---|---|---|---|
| P01H | Polypeptide 1 | EVQLVESGGGLVQPGGSLRLSCAASGFTFTDYTMDWVRQAPGKCLEWVADVNPN SGGSIYNQRFKGRFTLSVDRSKNTLYLQMNSLRAEDTAVYYCARNLGPSFYFDY WGQGTLVTVSSASTKGPSVFPLAPSSKSTSGGTAALGCLVKDYFPEPVTVSWNS GALTSGVHTFPAVLQSSGLYSLSSVVTVPSSSLGTQTYICNVNHKPSNTKVDKK VEPKSCDKTHTCPPCPAPELLGGPSVFLFPPKPKDTLMISRTPEVTCVVVDVSH EDPEVKFNWYVDGVEVHNAKTKPREEQYNSTYRVVSVLTVLHQDWLNGKEYKCK VSNKALPAPIEKTISKAKGQPREPQVYTLPPCREEMTKNQVSLWCLVKGFYPSD IAVEWESNGQPENNYKTTPPVLDSDGSFFLYSKLTVDKSRWQQGNVFSCSVMHE ALHNHYTQKSLSLSPGK | 29 |
| | Polypeptide 2 | DIQMTQSPSSLSASVGDRVTITCKASQDVSIGVAWYQQKPGKAPKLLIYSASYR YTGVPSRFSGSGSGTDFTLTISSLQPEDFATYYCQQYYIYPYTFGCGTKVEIKR TVAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGECGG GGSGGGGSGGGGSEPKSSDKTHTCPPCPAPELLGGPSVFLFPPKPKDTLMISRT PEVTCVVVDVSHEDPEVKFNWYVDGVEVHNAKTKPREEQYNSTYRVVSVLTVLH QDWLNGKEYKCKVSNKALPAPIEKTISKAKGQPREPQVYTLPPCREEMTKNQVS LWCLVKGFYPSDIAVEWESNGQPENNYKTTPPVLDSDGSFFLYSKLTVDKSRWQ QGNVFSCSVMHEALHNHYTQKSLSLSPGK | 30 |
| | Polypeptide 3 | EVQLVESGGGLVQPGGSLRLSCAASGFNIKDTYIHWVRQAPGKCLEWVARIYPT NGYTRYADSVKGRFTISADTSKNTAYLQMNSLRAEDTAVYYCSRWGGDGFYAMD | 897 |

| | | | |
|---|---|---|---|
| | | YWGQGTLVTVSSGGGGSGGGGSGGGGSDIQMTQSPSSLSASVGDRVTITCRASQ DVNTAVAWYQQKPGKAPKLLIYSASFLYSGVPSRFSGSRSGTDFTLTISSLQPE DFATYYCQQHYTTPPTFGCGTKVEIKGGGGSDKTHTCPPCPAPELLGGPSVFLF PPKPKDTLMISRTPEVTCVVVDVSHEDPEVKFNWYVDGVEVHNAKTKPREEQYN STYRVVSVLTVLHQDWLNGKEYKCKVSNKALPAPIEKTISKAKGQPREPQVCTL PPSREEMTKNQVSLSCAVKGFYPSDIAVEWESNGQPENNYKTTPPVLDSDGSFF LVSKLTVDKSRWQQGNVFSCSVMHEALHNHYTQKSLSLSPGK | |
| | Polypeptid e 4 | EVQLVESGGGLVQPGGSLRLSCAASGFNIKDTYIHWVRQAPGKCLEWVARIYPT NGYTRYADSVKGRFTISADTSKNTAYLQMNSLRAEDTAVYYCSRWGGDGFYAMD YWGQGTLVTVSSGGGGSGGGGSGGGGSDIQMTQSPSSLSASVGDRVTITCRASQ DVNTAVAWYQQKPGKAPKLLIYSASFLYSGVPSRFSGSRSGTDFTLTISSLQPE DFATYYCQQHYTTPPTFGCGTKVEIKGGGGSDKTHTCPPCPAPELLGGPSVFLF PPKPKDTLMISRTPEVTCVVVDVSHEDPEVKFNWYVDGVEVHNAKTKPREEQYN STYRVVSVLTVLHQDWLNGKEYKCKVSNKALPAPIEKTISKAKGQPREPQVCTL PPSREEMTKNQVSLSCAVKGFYPSDIAVEWESNGQPENNYKTTPPVLDSDGSFF LVSKLTVDKSRWQQGNVFSCSVMHEALHNHYTQKSLSLSPGK | 897 |
| ME13 | Polypeptid e 1 | QVQLQESGPGLVKPSETLSLTCTVSGGSVSSGDYYWTWIRQSPGKCLEWIGHIY YSGNTNYNPSLKSRLTISIDTSKTQFSLKLSSVTAADTAIYYCVRDRVTGAFDI WGQGTMVTVSSASTKGPSVFPLAPSSKSTSGGTAALGCLVKDYFPEPVTVSWNS GALTSGVHTFPAVLQSSGLYSLSSVVTVPSSSLGTQTYICNVNHKPSNTKVDKK VEPKSCDKTHTCPPCPAPELLGGPSVFLFPPKPKDTLMISRTPEVTCVVVDVSH EDPEVKFNWYVDGVEVHNAKTKPREEQYNSTYRVVSVLTVLHQDWLNGKEYKCK VSNKALPAPIEKTISKAKGQPREPQVYTLPPCREEMTKNQVSLWCLVKGFYPSD IAVEWESNGQPENNYKTTPPVLDSDGSFFLYSKLTVDKSRWQQGNVFSCSVMHE ALHNHYTQKSLSLSPGK | 594 |
| | Polypeptid e 2 | DIQMTQSPSSLSASVGDRVTITCQASQDISNYLNWYQQKPGKAPKLLIYDASNL ETGVPSRFSGSGSGTDFTFTISSLQPEDIATYFCQHFDHLPLAFGCGTKVEIKR TVAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGECGG | 595 |

| | | | |
|---|---|---|---|
| | | GGSGGGGSGGGGSEPKSSDKTHTCPPCPAPELLGGPSVFLFPPKPKDTLMISRT PEVTCVVVDVSHEDPEVKFNWYVDGVEVHNAKTKPREEQYNSTYRVVSVLTVLH QDWLNGKEYKCKVSNKALPAPIEKTISKAKGQPREPQVYTLPPCREEMTKNQVS LWCLVKGFYPSDIAVEWESNGQPENNYKTTPPVLDSDGSFFLYSKLTVDKSRWQ QGNVFSCSVMHEALHNHYTQKSLSLSPGK | |
| | Polypeptid e 3 | EVQLVESGGGLVQPGGSLRLSCAASGYTFTSYWLHWVRQAPGKCLEWVGMIDPS NSDTRFNPNFKDRFTISADTSKNTAYLQMNSLRAEDTAVYYCATYRSYVTPLDY WGQGTLVTVSSGGGGSGGGGSGGGGSDIQMTQSPSSLSASVGDRVTITCKSSQS LLYTSSQKNYLAWYQQKPGKAPKLLIYWASTRESGVPSRFSGSGSGTDFTLTIS SLQPEDFATYYCQQYYAYPWTFGCGTKVEIKGGGGSDKTHTCPPCPAPELLGGP SVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREP QVCTLPPSREEMTKNQVSLSCAVKGFYPSDIAVEWESNGQPENNYKTTPPVLDS DGSFFLVSKLTVDKSRWQQGNVFSCSVMHEALHNHYTQKSLSLSPGK | 898 |
| | Polypeptid e 4 | EVQLVESGGGLVQPGGSLRLSCAASGYTFTSYWLHWVRQAPGKCLEWVGMIDPS NSDTRFNPNFKDRFTISADTSKNTAYLQMNSLRAEDTAVYYCATYRSYVTPLDY WGQGTLVTVSSGGGGSGGGGSGGGGSDIQMTQSPSSLSASVGDRVTITCKSSQS LLYTSSQKNYLAWYQQKPGKAPKLLIYWASTRESGVPSRFSGSGSGTDFTLTIS SLQPEDFATYYCQQYYAYPWTFGCGTKVEIKGGGGSDKTHTCPPCPAPELLGGP SVFLFPPKPKDTLMISRTPEVTCVVVDVSHEDPEVKFNWYVDGVEVHNAKTKPR EEQYNSTYRVVSVLTVLHQDWLNGKEYKCKVSNKALPAPIEKTISKAKGQPREP QVCTLPPSREEMTKNQVSLSCAVKGFYPSDIAVEWESNGQPENNYKTTPPVLDS DGSFFLVSKLTVDKSRWQQGNVFSCSVMHEALHNHYTQKSLSLSPGK | 898 |
| ME14 | Polypeptid e 1 | QVQLQESGPGLVKPSETLSLTCTVSGGSVSSGDYYWTWIRQSPGKCLEWIGHIY YSGNTNYNPSLKSRLTISIDTSKTQFSLKLSSVTAADTAIYYCVRDRVTGAFDI WGQGTMVTVSSASTKGPSVFPLAPSSKSTSGGTAALGCLVKDYFPEPVTVSWNS GALTSGVHTFPAVLQSSGLYSLSSVVTVPSSSLGTQTYICNVNHKPSNTKVDKK VEPKSCDKTHTCPPCPAPELLGGPSVFLFPPKPKDTLMISRTPEVTCVVVDVSH EDPEVKFNWYVDGVEVHNAKTKPREEQYNSTYRVVSVLTVLHQDWLNGKEYKCK | 594 |

| | | | |
|---|---|---|---|
| | | VSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | |
| | Polypeptide 2 | DIQMTQSPSSLSASVGDRVTITCQASQDISNYLNWYQQKPGKAPKLLIYDASNLETGVPSRFSGSGSGTDFTFTISSLQPEDIATYFCQHFDHLPLAFGCGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGECGGGGSGGGGSGGGGSEPKSSDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 595 |
| | Polypeptide 3 | QVQLVQSGAEVKKPGASVKVSCKASGYTFTSYGFSWVRQAPGQCLEWMGWISASNGNTYYAQKLQGRVTMTTDTSTSTAYMELRSLRSDDTAVYYCARVYADYADYWGQGTLVTVSSGGGGSGGGGSGGGGSDIQMTQSPSSVSASVGDRVTITCRASQGINTWLAWYQQKPGKAPKLLIYAASSLKSGVPSRFSGSGSGTDFTLTISSLQPEDFATYYCQQANSFPLTFGCGTKVEIKGGGGSDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVCTLPPSREEMTKNQVSLSCAVKGFYPSDIAVEWESNGQPENNYKTTPPVLDSDGSFFLVSKLTVDKSRWQQGNVFSCSVMHEALHNHYTQKSLSLSPGK | 899 |
| | Polypeptide 4 | QVQLVQSGAEVKKPGASVKVSCKASGYTFTSYGFSWVRQAPGQCLEWMGWISASNGNTYYAQKLQGRVTMTTDTSTSTAYMELRSLRSDDTAVYYCARVYADYADYWGQGTLVTVSSGGGGSGGGGSGGGGSDIQMTQSPSSVSASVGDRVTITCRASQGINTWLAWYQQKPGKAPKLLIYAASSLKSGVPSRFSGSGSGTDFTLTISSLQPEDFATYYCQQANSFPLTFGCGTKVEIKGGGGSDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVCTLPPSREEMTKNQVSLSCAVKGFYPSDIAVEWESNGQPENNYKTTPPVLDSDGSFFLVS | 899 |

| | | | | |
|---|---|---|---|---|
| | | | KLTVDKSRWQQGNVFSCSVMHEALHNHYTQKSLSLSPGK | |
| | ME15 | Polypeptide 1 | EVQLVESGGGLVQPGGSLRLSCAASGYTFTSYWLHWVRQAPGKCLEWVGMIDPS NSDTRFNPNFKDRFTISADTSKNTAYLQMNSLRAEDTAVYYCATYRSYVTPLDY WGQGTLVTVSSASTKGPSVFPLAPSSKSTSGGTAALGCLVKDYFPEPVTVSWNS GALTSGVHTFPAVLQSSGLYSLSSVVTVPSSSLGTQTYICNVNHKPSNTKVDKK VEPKSCDKTHTCPPCPAPELLGGPSVFLFPPKPKDTLMISRTPEVTCVVVDVSH EDPEVKFNWYVDGVEVHNAKTKPREEQYNSTYRVVSVLTVLHQDWLNGKEYKCK VSNKALPAPIEKTISKAKGQPREPQVYTLPPCREEMTKNQVSLWCLVKGFYPSD IAVEWESNGQPENNYKTTPPVLDSDGSFFLYSKLTVDKSRWQQGNVFSCSVMHE ALHNHYTQKSLSLSPGK | 568 |
| | | Polypeptide 2 | DIQMTQSPSSLSASVGDRVTITCKSSQSLLYTSSQKNYLAWYQQKPGKAPKLLI YWASTRESGVPSRFSGSGSGTDFTLTISSLQPEDFATYYCQQYYAYPWTFGCGT KVEIKRTVAAPSVFIFPPSDEQLKSGTASVVCLLNNFYPREAKVQWKVDNALQS GNSQESVTEQDSKDSTYSLSSTLTLSKADYEKHKVYACEVTHQGLSSPVTKSFN RGECGGGGSGGGGSGGGGSEPKSSDKTHTCPPCPAPELLGGPSVFLFPPKPKDT LMISRTPEVTCVVVDVSHEDPEVKFNWYVDGVEVHNAKTKPREEQYNSTYRVVS VLTVLHQDWLNGKEYKCKVSNKALPAPIEKTISKAKGQPREPQVYTLPPCREEM TKNQVSLWCLVKGFYPSDIAVEWESNGQPENNYKTTPPVLDSDGSFFLYSKLTV DKSRWQQGNVFSCSVMHEALHNHYTQKSLSLSPGK | 569 |
| | | Polypeptide 3 | QVQLQESGPGLVKPSETLSLTCTVSGGSVSSGDYYWTWIRQSPGKCLEWIGHIY YSGNTNYNPSLKSRLTISIDTSKTQFSLKLSSVTAADTAIYYCVRDRVTGAFDI WGQGTMVTVSSGGGGSGGGGSGGGGSDIQMTQSPSSLSASVGDRVTITCQASQD ISNYLNWYQQKPGKAPKLLIYDASNLETGVPSRFSGSGSGTDFTFTISSLQPED IATYFCQHFDHLPLAFGCGTKVEIKGGGGSDKTHTCPPCPAPELLGGPSVFLFP PKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVCTLP PSREEMTKNQVSLSCAVKGFYPSDIAVEWESNGQPENNYKTTPPVLDSDGSFFL VSKLTVDKSRWQQGNVFSCSVMHEALHNHYTQKSLSLSPGK | 900 |
| | | Polypeptide | QVQLQESGPGLVKPSETLSLTCTVSGGSVSSGDYYWTWIRQSPGKCLEWIGHIY | 900 |

| | e 4 | YSGNTNYNPSLKSRLTISIDTSKTQFSLKLSSVTAADTAIYYCVRDRVTGAFDI WGQGTMVTVSSGGGGSGGGGSGGGGSDIQMTQSPSSLSASVGDRVTITCQASQD ISNYLNWYQQKPGKAPKLLIYDASNLETGVPSRFSGSGSGTDFTFTISSLQPED IATYFCQHFDHLPLAFGCGTKVEIKGGGGSDKTHTCPPCPAPELLGGPSVFLFP PKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVCTLP PSREEMTKNQVSLSCAVKGFYPSDIAVEWESNGQPENNYKTTPPVLDSDGSFFL VSKLTVDKSRWQQGNVFSCSVMHEALHNHYTQKSLSLSPGK | |
| ME16 | Polypeptide 1 | QVQLVQSGAEVKKPGASVKVSCKASGYTFTSYGFSWVRQAPGQCLEWMGWISAS NGNTYYAQKLQGRVTMTTDTSTSTAYMELRSLRSDDTAVYYCARVYADYADYWG QGTLVTVSSASTKGPSVFPLAPSSKSTSGGTAALGCLVKDYFPEPVTVSWNSGA LTSGVHTFPAVLQSSGLYSLSSVVTVPSSSLGTQTYICNVNHKPSNTKVDKKVE PKSCDKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYKCKVS NKALPAPIEKTISKAKGQPREPQVYTLPPCREEMTKNQVSLWCLVKGFYPSDIA VEWESNGQPENNYKTTPPVLDSDGSFFLYSKLTVDKSRWQQGNVFSCSVMHEAL HNHYTQKSLSLSPGK | 570 |
| | Polypeptide 2 | DIQMTQSPSSVSASVGDRVTITCRASQGINTWLAWYQQKPGKAPKLLIYAASSL KSGVPSRFSGSGSGTDFTLTISSLQPEDFATYYCQQANSFPLTFGCGTKVEIKR TVAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGECGG GGSGGGGSGGGGSEPKSSDKTHTCPPCPAPELLGGPSVFLFPPKPKDTLMISRT PEVTCVVVDVSHEDPEVKFNWYVDGVEVHNAKTKPREEQYNSTYRVVSVLTVLH QDWLNGKEYKCKVSNKALPAPIEKTISKAKGQPREPQVYTLPPCREEMTKNQVS LWCLVKGFYPSDIAVEWESNGQPENNYKTTPPVLDSDGSFFLYSKLTVDKSRWQ QGNVFSCSVMHEALHNHYTQKSLSLSPGK | 571 |
| | Polypeptide 3 | QVQLQESGPGLVKPSETLSLTCTVSGGSVSSGDYYWTWIRQSPGKCLEWIGHIY YSGNTNYNPSLKSRLTISIDTSKTQFSLKLSSVTAADTAIYYCVRDRVTGAFDI WGQGTMVTVSSGGGGSGGGGSGGGGSDIQMTQSPSSLSASVGDRVTITCQASQD | 900 |

| | | | |
|---|---|---|---|
| | | ISNYLNWYQQKPGKAPKLLIYDASNLETGVPSRFSGSGSGTDFTFTISSLQPED IATYFCQHFDHLPLAFGCGTKVEIKGGGGSDKTHTCPPCPAPELLGGPSVFLFP PKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVCTLP PSREEMTKNQVSLSCAVKGFYPSDIAVEWESNGQPENNYKTTPPVLDSDGSFFL VSKLTVDKSRWQQGNVFSCSVMHEALHNHYTQKSLSLSPGK | |
| | Polypeptide 4 | QVQLQESGPGLVKPSETLSLTCTVSGGSVSSGDYYWTWIRQSPGKCLEWIGHIY YSGNTNYNPSLKSRLTISIDTSKTQFSLKLSSVTAADTAIYYCVRDRVTGAFDI WGQGTMVTVSSGGGGSGGGGSGGGGSDIQMTQSPSSLSASVGDRVTITCQASQD ISNYLNWYQQKPGKAPKLLIYDASNLETGVPSRFSGSGSGTDFTFTISSLQPED IATYFCQHFDHLPLAFGCGTKVEIKGGGGSDKTHTCPPCPAPELLGGPSVFLFP PKPKDTLMISRTPEVTCVVVDVSHEDPEVKFNWYVDGVEVHNAKTKPREEQYNS TYRVVSVLTVLHQDWLNGKEYKCKVSNKALPAPIEKTISKAKGQPREPQVCTLP PSREEMTKNQVSLSCAVKGFYPSDIAVEWESNGQPENNYKTTPPVLDSDGSFFL VSKLTVDKSRWQQGNVFSCSVMHEALHNHYTQKSLSLSPGK | 900 |
| CE05 | Polypeptide 1 | QVQLQESGPGLVKPSETLSLTCTVSGGSVSSGDYYWTWIRQSPGKCLEWIGHIY YSGNTNYNPSLKSRLTISIDTSKTQFSLKLSSVTAADTAIYYCVRDRVTGAFDI WGQGTMVTVSSASTKGPSVFPLAPSSKSTSGGTAALGCLVKDYFPEPVTVSWNS GALTSGVHTFPAVLQSSGLYSLSSVVTVPSSSLGTQTYICNVNHKPSNTKVDKK VEPKSCDKTHTCPPCPAPELLGGPSVFLFPPKPKDTLMISRTPEVTCVVVDVSH EDPEVKFNWYVDGVEVHNAKTKPREEQYNSTYRVVSVLTVLHQDWLNGKEYKCK VSNKALPAPIEKTISKAKGQPREPQVYTLPPCREEMTKNQVSLWCLVKGFYPSD IAVEWESNGQPENNYKTTPPVLDSDGSFFLYSKLTVDKSRWQQGNVFSCSVMHE ALHNHYTQKSLSLSPGK | 594 |
| | Polypeptide 2 | DIQMTQSPSSLSASVGDRVTITCQASQDISNYLNWYQQKPGKAPKLLIYDASNL ETGVPSRFSGSGSGTDFTFTISSLQPEDIATYFCQHFDHLPLAFGCGTKVEIKR TVAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGECGG GGSGGGGSGGGGSEPKSSDKTHTCPPCPAPELLGGPSVFLFPPKPKDTLMISRT | 595 |

| | | | |
|---|---|---|---|
| | | PEVTCVVVDVSHEDPEVKFNWYVDGVEVHNAKTKPREEQYNSTYRVVSVLTVLH QDWLNGKEYKCKVSNKALPAPIEKTISKAKGQPREPQVYTLPPCREEMTKNQVS LWCLVKGFYPSDIAVEWESNGQPENNYKTTPPVLDSDGSFFLYSKLTVDKSRWQ QGNVFSCSVMHEALHNHYTQKSLSLSPGK | |
| | Polypeptide 3 | EVQLVESGGGVVQPGRSLRLSCSASGFDFTTYWMSWVRQAPGKCLEWIGEIHPD SSTINYAPSLKDRFTISRDNAKNTLFLQMDSLRPEDTGVYFCASLYFGFPWFAY WGQGTPVTVSSGGGGSGGGGSGGGGSDIQLTQSPSSLSASVGDRVTITCKASQD VGTSVAWYQQKPGKAPKLLIYWTSTRHTGVPSRFSGSGSGTDFTFTISSLQPED IATYYCQQYSLYRSFGCGTKVEIKGGGGSDKTHTCPPCPAPELLGGPSVFLFPP KPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVCTLPP SREEMTKNQVSLSCAVKGFYPSDIAVEWESNGQPENNYKTTPPVLDSDGSFFLV SKLTVDKSRWQQGNVFSCSVMHEALHNHYTQKSLSLSPGK | 901 |
| | Polypeptide 4 | EVQLVESGGGVVQPGRSLRLSCSASGFDFTTYWMSWVRQAPGKCLEWIGEIHPD SSTINYAPSLKDRFTISRDNAKNTLFLQMDSLRPEDTGVYFCASLYFGFPWFAY WGQGTPVTVSSGGGGSGGGGSGGGGSDIQLTQSPSSLSASVGDRVTITCKASQD VGTSVAWYQQKPGKAPKLLIYWTSTRHTGVPSRFSGSGSGTDFTFTISSLQPED IATYYCQQYSLYRSFGCGTKVEIKGGGGSDKTHTCPPCPAPELLGGPSVFLFPP KPKDTLMISRTPEVTCVVVDVSHEDPEVKFNWYVDGVEVHNAKTKPREEQYNST YRVVSVLTVLHQDWLNGKEYKCKVSNKALPAPIEKTISKAKGQPREPQVCTLPP SREEMTKNQVSLSCAVKGFYPSDIAVEWESNGQPENNYKTTPPVLDSDGSFFLV SKLTVDKSRWQQGNVFSCSVMHEALHNHYTQKSLSLSPGK | 901 |

Table 98 below shows the polynucleotide sequences encoding each polypeptide constituting the novel antibody format.

[Table 98]

| Name | Polypeptid | Sequence | SEQ ID NO |
|------|------------|----------|-----------|
| H01P | Polypeptide 1 | GAAGTTCAACTTGTGGAGTCTGGAGGCGGTTTCGTACAACCTGGAGGTTCACTT CGTTTGTCATGTGCTGCCTCTGGGTTTAATATCAAGGATACTTATATTCATTGG GTTCGCCAGGCACCCGGAAAATGCCTGGAATGGGTGGCCCGTATATATCCCACT AACGGTTACACTCGATATGCTGACTCTGTAAAGGGTCGATTTACTATATCCGCT GATACTAGCAAAAACACTGCCTACCTCCAGATGAACAGTCTGCGCGCCGAGGAT ACAGCCAGTTTATTATTGTTCCCGATGGGGAGGTGACGGGTTCTATGCCATGGAC TACTGGGGTCAAGGGACACTGGTAACCGTTTCTTCTGCTAGCACCAAAGGACCT AGTGTTTTTCCTCTTGCCCCTTCCTCAAAGTCTACCTCTGGGGGGACAGCCGCT CTGGGCTGCCTGGTCAAGGATTATTTCCCAGAGCCTGTCACTGTCAGTTGGAAC TCTGGAGCCTTGACTTCTGGTGTTCATACATTTCCTGCTGTCCTTCAAAGCAGC GGCTTGTACTCATTGTCTTCTGTTGTGACAGTACCCTCAAGCAGCCTCGGCACT CAGACATACATCTGCAATGTCAACCACAAACCCTCAAATACAAAGGTAGATAAA AAAGTCGAACCAAAGTCTTGTGACAAAACTCACACGTGCCCACCGTGCCCAGCA CCTGAACTCCTGGGGGGACCGTCAGTCTTCCTCTTCCCCCCAAAACCCAAGGAC ACCCTCATGATCTCCCGGACCCCTGAGGTCACATGCGTGGTGGTGGACGTGAGC CACGAAGACCCTGAGGTCAAGTTCAACTGGTACGTGGACGGCGTGGAGGTGCAT AATGCCAAGACAAAGCCGCGGGAGGAGCAGTACAACAGCACGTACCGTGTGGTC AGCGTCCTCACCGTCCTGCACCAGGACTGGCTGAATGGCAAGGAGTACAAGTGC AAGGTCTCCAACAAAGCCCTCCCAGCCCCCATCGAGAAAACCATCTCCAAAGCC AAAGGGCAGCCCCGAGAACCACAGGTGTACACCCTGCCCCCATGCCGGGAGGAG ATGACCAAGAACCAGGTCAGCCTGTGGTGCCTGGTCAAAGGCTTCTATCCCAGC GACATCGCCGTGGAGTGGGAGAGCAATGGGCAGCCGGAGAACAACTACAAGACC ACGCCTCCCGTGCTGGACTCCGACGGCTCCTTCTTCCTCTACAGCAAGCTCACC GTGGACAAGAGCAGGTGGCAGCAGGGGAACGTCTTCTCATGCTCCGTGATGCAT GAGGCTCTGCACAACCACTACACGCAGAAGAGCCTCTCCCTGTCTCCGGGTAAA | 465 |
| | Polypeptide 2 | GACATTCAGATGACCCAATCCCCCTCTAGCCTTTCAGCCTCCGTAGGGGACAGA GTTACAATAACCTGTAGGGCTAGTCATGACGTTAATACAGCAGTCGCATGGTAC | 466 |

| | | CAGCAGAAGCCCGGTAAGGCTCCTAAGCTTTTGATCTACAGTGCTTCTTTCCTG TATTCTGGTGTACCTAGCCGATTCTCAGGCTCTCGGAGTGGCACAGACTTCACT CTGACAATTTCAAGTCTCCAGCCAGAAGATTTTGCAACCTACTACTGCCAACAA CACTACACTACTCCTCCAACCTTCGGATGTGGCACAAAAGTAGAGATTAAGCGT ACGGTGGCAGCTCCCAGCGTTTTTATCTTTCCCCCATCCGACGAGCAGCTCAAG AGTGGCACTGCCTCTGTAGTTTGTTTGCTGAATAACTTCTATCCACGTGAAGCA AAAGTACAGTGGAAGGTCGATAATGCCCTTCAGAGCGGTAACAGCCAAGAAAGT GTTACCGAGCAAGATTCCAAAGATTCCACTTACAGTCTGTCCAGCACATTGACA CTGAGTAAGGCTGATTACGAAAAACACAAGGTGTACGCATGCGAGGTGACACAC CAAGGTCTTTCATCTCCTGTAACTAAGAGCTTTAACCGGGGAGAATGTGGTGGT GGGGGCAGCGGGGGCGGAGGTAGTGGAGGCGGCGGTAGTGAACCAAAGAGTAGT GACAAAACTCACACGTGCCCACCGTGCCCAGCACCTGAACTCCTGGGGGGACCG TCAGTCTTCCTCTTCCCCCCAAAAACCCAAGGACACCCTCATGATCTCCCGGACC CCTGAGGTCACATGCGTGGTGGTGGACGTGAGCCACGAAGACCCTGAGGTCAAG TTCAACTGGTACGTGGACGGCGTGGAGGTGCATAATGCCAAGACAAAGCCGCGG GAGGAGCAGTACAACAGCACGTACCGTGTGGTCAGCGTCCTCACCGTCCTGCAC CAGGACTGGCTGAATGGCAAGGAGTACAAGTGCAAGGTCTCCAACAAAGCCCTC CCAGCCCCCATCGAGAAAACCATCTCCAAAGCCAAAGGGCAGCCCCGAGAACCA CAGGTGTACACCCTGCCCCCATGCCGGGAGGAGATGACCAAGAACCAGGTCAGC CTGTGGTGCCTGGTCAAAGGCTTCTATCCCAGCGACATCGCCGTGGAGTGGGAG AGCAATGGGCAGCCGGAGAACAACTACAAGACCACGCCTCCCGTGCTGGACTCC GACGGCTCCTTCTTCCTCTACAGCAAGCTCACCGTGGACAAGAGCAGGTGGCAG CAGGGGAACGTCTTCTCATGCTCCGTGATGCATGAGGCTCTGCACAACCACTAC ACGCAGAAGAGCCTCTCCCTGTCTCCGGGTAAA | |
| Polypeptide 3 | GAAGTACAGTTGGTGGAGTCTGGTGGAGGTCTTGTACAACCTGGCGGGAGTTTG CGGCTTTCCTGCGCTGCAAGCGGGTTTACCTTCACCGATTATACCATGGATTGG GTACGCCAAGCCCCTGGTAAGTGCCTTGAGTGGGTTGCCGATGTAAACCCTAAT TCCGGAGGAAGTATCTATAATCAACGCTTCAAGGGCCGATTCACTCTGAGTGTG GATCGAAGCAAGAACACCTTGTACTTGCAGATGAATTCCTTGCGGGCTGAAGAC | 902 |

| | | ACAGCCGTCTATTACTGCGCCCGAAATTTGGGGCCTTCATTCTATTTTGACTAT TGGGGTCAGGGAACTCTGGTAACTGTTTCAAGTGGCGGAGGGGGTTCTGGAGGC GGAGGCAGCGGGGGCGGAGGGTCCGATATTCAAATGACCCAAAGTCCAAGTTCC CTTTCAGCATCTGTCGGTGATCGAGTCACTATAACTTGTAAGGCCAGCCAAGAC GTTAGCATAGGAGTAGCATGGTACCAACAAAAGCCTGGGAAGGCTCCCAAACTT CTCATTTATTCTGCTTCCTACCGATATACTGGTGTCCCAAGTAGATTTTCTGGC AGCGGATCTGGAACTGATTTTACATTGACTATCAGCTCCCTCCAGCCTGAGGAC TTCGCTACTTATTACTGCCAACAGTACTACATTTATCCCTATACATTCGGTTGT GGGACCAAAGTAGAGATCAAAGGAGGCGGCGGCTCTGACAAAACTCACACGTGC CCACCGTGCCCAGCACCTGAACTCCTGGGGGGACCGTCAGTCTTCCTCTTCCCC CCAAAACCCAAGGACACCCTCATGATCTCCCGGACCCCTGAGGTCACATGCGTG GTGGTGGACGTGAGCCACGAAGACCCTGAGGTCAAGTTCAACTGGTACGTGGAC GGCGTGGAGGTGCATAATGCCAAGACAAAGCCGCGGGAGGAGCAGTACAACAGC ACGTACCGTGTGGTCAGCGTCCTCACCGTCCTGCACCAGGACTGGCTGAATGGC AAGGAGTACAAGTGCAAGGTCTCCAACAAAGCCCTCCCAGCCCCCATCGAGAAA ACCATCTCCAAAGCCAAAGGGCAGCCCCGAGAACCACAGGTGTGCACCCTGCCC CCATCCCGGGAGGAGATGACCAAGAACCAGGTCAGCCTGAGCTGCGCGGTCAAA GGCTTCTATCCCAGCGACATCGCCGTGGAGTGGGAGAGCAATGGGCAGCCGGAG AACAACTACAAGACCACGCCTCCCGTGCTGGACTCCGACGGCTCCTTCTTCCTC GTGAGCAAGCTCACCGTGGACAAGAGCAGGTGGCAGCAGGGGAACGTCTTCTCA TGCTCCGTGATGCATGAGGCTCTGCACAACCACTACACGCAGAAGAGCCTCTCC CTGTCTCCGGGTAAA | |
| Polypeptide 4 | GAAGTACAGTTGGTGGAGTCTGGTGGAGGTCTTGTACAACCTGGCGGGAGTTTG CGGCTTTCCTGCGCTGCAAGCGGGTTTACCTTCACCGATTATACCATGGATTGG GTACGCCAAGCCCCTGGTAAGTGCCTTGAGTGGGTTGCCGATGTAAACCCTAAT TCCGGAGGAAGTATCTATAATCAACGCTTCAAGGGCCGATTCACTCTGAGTGTG GATCGAAGCAAGAACACCTTGTACTTGCAGATGAATTCCTTGCGGGCTGAAGAC ACAGCCGTCTATTACTGCGCCCGAAATTTGGGGCCTTCATTCTATTTTGACTAT TGGGGTCAGGGAACTCTGGTAACTGTTTCAAGTGGCGGAGGGGGTTCTGGAGGC | 902 |

| | | | |
|---|---|---|---|
| | | GGAGGCAGCGGGGGCGGAGGGTCCGATATTCAAATGACCCAAAGTCCAAGTTCC CTTTCAGCATCTGTCGGTGATCGAGTCACTATAACTTGTAAGGCCAGCCAAGAC GTTAGCATAGGAGTAGCATGGTACCAACAAAAGCCTGGGAAGGCTCCCAAACTT CTCATTTATTCTGCTTCCTACCGATATACTGGTGTCCCAAGTAGATTTTCTGGC AGCGGATCTGGAACTGATTTTACATTGACTATCAGCTCCCTCCAGCCTGAGGAC TTCGCTACTTATTACTGCCAACAGTACTACATTTATCCCTATACATTCGGTTGT GGGACCAAAGTAGAGATCAAAGGAGGCGGCGGCTCTGACAAAACTCACACGTGC CCACCGTGCCCAGCACCTGAACTCCTGGGGGGACCGTCAGTCTTCCTCTTCCCC CCAAAACCCAAGGACACCCTCATGATCTCCCGGACCCCTGAGGTCACATGCGTG GTGGTGGACGTGAGCCACGAAGACCCTGAGGTCAAGTTCAACTGGTACGTGGAC GGCGTGGAGGTGCATAATGCCAAGACAAAGCCGCGGGAGGAGCAGTACAACAGC ACGTACCGTGTGGTCAGCGTCCTCACCGTCCTGCACCAGGACTGGCTGAATGGC AAGGAGTACAAGTGCAAGGTCTCCAACAAAGCCCTCCCAGCCCCCATCGAGAAA ACCATCTCCAAAGCCAAAGGGCAGCCCCGAGAACCACAGGTGTGCACCCTGCCC CCATCCCGGGAGGAGATGACCAAGAACCAGGTCAGCCTGAGCTGCGCGGTCAAA GGCTTCTATCCCAGCGACATCGCCGTGGAGTGGGAGAGCAATGGGCAGCCGGAG AACAACTACAAGACCACGCCTCCCGTGCTGGACTCCGACGGCTCCTTCTTCCTC GTGAGCAAGCTCACCGTGGACAAGAGCAGGTGGCAGCAGGGGAACGTCTTCTCA TGCTCCGTGATGCATGAGGCTCTGCACAACCACTACACGCAGAAGAGCCTCTCC CTGTCTCCGGGTAAA | |
| POIH | Polypeptide 1 | GAAGTACAGTTGGTGGAGTCTGGTGGAGGTCTTGTACAACCTGGCGGGAGTTTG CGGCTTTCCTGCGCTGCAAGCGGGTTTACCTTCACCGATTATACCATGGATTGG GTACGCCAAGCCCCTGGTAAGTGCCTTGAGTGGGTTGCCGATGTAAACCCTAAT TCCGGAGGAAGTATCTATAATCAACGCTTCAAGGGCCGATTCACTCTGAGTGTG GATCGAAGCAAGAACACCTTGTACTTGCAGATGAATTCCTTGCGGGCTGAAGAC ACAGCCGTCTATTACTGCGCCCGAAATTTGGGGGCCTTCATTCTATTTTGACTAT TGGGGTCAGGGAACTCTGGTAACTGTTTCAAGTGCTAGCACCAAAGGACCTAGT GTTTTTCCTCTTGCCCCTTCCTCAAAGTCTACCTCTGGGGGGACAGCCGCTCTG GGCTGCCTGGTCAAGGATTATTTCCCAGAGCCTGTCACTGTCAGTTGGAACTCT | 472 |

| | | | |
|---|---|---|---|
| | | GGAGCCTTGACTTCTGGTGTTCATACATTTCCTGCTGTCCTTCAAAGCAGCGGC TTGTACTCATTGTCTTCTGTTGTGACAGTACCCTCAAGCAGCCTCGGCACTCAG ACATACATCTGCAATGTCAACCACAAACCCTCAAATACAAAGGTAGATAAAAAA GTCGAACCAAAGTCTTGTGACAAAACTCACACGTGCCCACCGTGCCCAGCACCT GAACTCCTGGGGGGACCGTCAGTCTTCCTCTTCCCCCCAAAACCCAAGGACACC CTCATGATCTCCCGGACCCCTGAGGTCACATGCGTGGTGGTGGACGTGAGCCAC GAAGACCCTGAGGTCAAGTTCAACTGGTACGTGGACGGCGTGGAGGTGCATAAT GCCAAGACAAAGCCGCGGGAGGAGCAGTACAACAGCACGTACCGTGTGGTCAGC GTCCTCACCGTCCTGCACCAGGACTGGCTGAATGGCAAGGAGTACAAGTGCAAG GTCTCCAACAAAGCCCTCCCAGCCCCCATCGAGAAAACCATCTCCAAAGCCAAA GGGCAGCCCCGAGAACCACAGGTGTACACCCTGCCCCCATGCCGGGAGGAGATG ACCAAGAACCAGGTCAGCCTGTGGTGCCTGGTCAAAGGCTTCTATCCCAGCGAC ATCGCCGTGGAGTGGGAGAGCAATGGGCAGCCGGAGAACAACTACAAGACCACG CCTCCCGTGCTGGACTCCGACGGCTCCTTCTTCCTCTACAGCAAGCTCACCGTG GACAAGAGCAGGTGGCAGCAGGGGAACGTCTTCTCATGCTCCGTGATGCATGAG GCTCTGCACAACCACTACACGCAGAAGAGCCTCTCCCTGTCTCCGGGTAAA | |
| | Polypeptide 2 | GATATTCAAATGACCCAAAGTCCAAGTTCCCTTTCAGCATCTGTCGGTGATCGA GTCACTATAACTTGTAAGGCCAGCCAAGACGTTAGCATAGGAGTAGCATGGTAC CAACAAAAGCCTGGGAAGGCTCCCAAACTTCTCATTTATTCTGCTTCCTACCGA TATACTGGTGTCCCAAGTAGATTTTCTGGCAGCGGATCTGGAACTGATTTTACA TTGACTATCAGCTCCCTCCAGCCTGAGGACTTCGCTACTTATTACTGCCAACAG TACTACATTTATCCCTATACATTCGGTTGTGGGACCAAAGTAGAGATCAAACGT ACGGTGGCAGCTCCCAGCGTTTTTATCTTTCCCCCATCCGACGAGCAGCTCAAG AGTGGCACTGCCTCTGTAGTTTGTTTGCTGAATAACTTCTATCCACGTGAAGCA AAAGTACAGTGGAAGGTCGATAATGCCCTTCAGAGCGGTAACAGCCAAGAAAGT GTTACCGAGCAAGATTCCAAAGATTCCACTTACAGTCTGTCCAGCACATTGACA CTGAGTAAGGCTGATTACGAAAAACACAAGGTGTACGCATGCGAGGTGACACAC CAAGGTCTTTCATCTCCTGTAACTAAGAGCTTTAACCGGGGAGAATGTGGTGGT GGGGGCAGCGGGGGCGGAGGTAGTGGAGGCGGCGGTAGTGAACCAAAGAGTAGT | 473 |

| | | | |
|---|---|---|---|
| | | GACAAAACTCACACGTGCCCACCGTGCCCAGCACCTGAACTCCTGGGGGGACCG TCAGTCTTCCTCTTCCCCCCAAAACCCAAGGACACCCTCATGATCTCCCGGACC CCTGAGGTCACATGCGTGGTGGTGGACGTGAGCCACGAAGACCCTGAGGTCAAG TTCAACTGGTACGTGGACGGCGTGGAGGTGCATAATGCCAAGACAAAGCCGCGG GAGGAGCAGTACAACAGCACGTACCGTGTGGTCAGCGTCCTCACCGTCCTGCAC CAGGACTGGCTGAATGGCAAGGAGTACAAGTGCAAGGTCTCCAACAAAGCCCTC CCAGCCCCCATCGAGAAAACCATCTCCAAAGCCAAAGGGCAGCCCCGAGAACCA CAGGTGTACACCCTGCCCCCATGCCGGGAGGAGATGACCAAGAACCAGGTCAGC CTGTGGTGCCTGGTCAAAGGCTTCTATCCCAGCGACATCGCCGTGGAGTGGGAG AGCAATGGGCAGCCGGAGAACAACTACAAGACCACGCCTCCCGTGCTGGACTCC GACGGCTCCTTCTTCCTCTACAGCAAGCTCACCGTGGACAAGAGCAGGTGGCAG CAGGGGAACGTCTTCTCATGCTCCGTGATGCATGAGGCTCTGCACAACCACTAC ACGCAGAAGAGCCTCTCCCTGTCTCCGGGTAAA | |
| | Polypeptide 3 | GAAGTTCAACTTGTGGAGTCTGGAGGCGGTTTGGTACAACCTGGAGGTTCACTT CGTTTGTCATGTGCTGCCTCTGGGTTTAATATCAAGGATACTTATATTCATTGG GTTCGCCAGGCACCCGGAAAATGCCTGGAATGGGTGGCCCGTATATATCCCACT AACGGTTACACTCGATATGCTGACTCTGTAAAGGGTCGATTTACTATATCCGCT GATACTAGCAAAAACACTGCCTACCTCCAGATGAACAGTCTGCGCGCCGAGGAT ACAGCAGTTTATTATTGTTCCCGATGGGGAGGTGACGGGTTCTATGCCATGGAC TACTGGGGTCAAGGGACACTGGTAACCGTTTCTTCTGGCGGAGGGGGTTCTGGA GGCGGAGGCAGCGGGGGCGGAGGGTCCGACATTCAGATGACCCAATCCCCCTCT AGCCTTTCAGCCTCCGTAGGGGACAGAGTTACAATAACCTGTAGGGCTAGTCAG GACGTTAATACAGCAGTCGCATGGTACCAGCAGAAGCCCGGTAAGGCTCCTAAG CTTTTGATCTACAGTGCTTCTTTCCTGTATTCTGGTGTACCTAGCCGATTCTCA GGCTCTCGGAGTGGCACAGACTTCACTCTGACAATTTCAAGTCTCCAGCCAGAA GATTTTGCAACCTACTACTGCCAACAACACTACACTACTCCTCCAACCTTCGGA TGTGGCACAAAAGTAGAGATTAAGGGAGGCGGCGGCTCTGACAAAACTCACACG TGCCCACCGTGCCCAGCACCTGAACTCCTGGGGGGACCGTCAGTCTTCCTCTTC CCCCCAAAACCCAAGGACACCCTCATGATCTCCCGGACCCCTGAGGTCACATGC | 903 |

| | | | |
|---|---|---|---|
| | | GTGGTGGTGGACGTGAGCCACGAAGACCCTGAGGTCAAGTTCAACTGGTACGTG GACGGCGTGGAGGTGCATAATGCCAAGACAAAGCCGCGGGAGGAGCAGTACAAC AGCACGTACCGTGTGGTCAGCGTCCTCACCGTCCTGCACCAGGACTGGCTGAAT GGCAAGGAGTACAAGTGCAAGGTCTCCAACAAAGCCCTCCCAGCCCCCATCGAG AAAACCATCTCCAAAGCCAAAGGGCAGCCCCGAGAACCACAGGTGTGCACCCTG CCCCCATCCCGGGAGGAGATGACCAAGAACCAGGTCAGCCTGAGCTGCGCGGTC AAAGGCTTCTATCCCAGCGACATCGCCGTGGAGTGGGAGAGCAATGGGCAGCCG GAGAACAACTACAAGACCACGCCTCCCGTGCTGGACTCCGACGGCTCCTTCTTC CTCGTGAGCAAGCTCACCGTGGACAAGAGCAGGTGGCAGCAGGGGAACGTCTTC TCATGCTCCGTGATGCATGAGGCTCTGCACAACCACTACACGCAGAAGAGCCTC TCCCTGTCTCCGGGTAAA | |
| | Polypeptide 4 | GAAGTTCAACTTGTGGAGTCTGGAGGCGGTTTGGTACAACCTGGAGGTTCACTT CGTTTGTCATGTGCTGCCTCTGGGTTTAATATCAAGGATACTTATATTCATTGG GTTCGCCAGGCACCCGGAAAATGCCTGGAATGGGTGGCCCGTATATATCCCACT AACGGTTACACTCGATATGCTGACTCTGTAAAGGGTCGATTTACTATATCCGCT GATACTAGCAAAAACACTGCCTACCTCCAGATGAACAGTCTGCGCGCCGAGGAT ACAGCAGTTTATTATTGTTCCCGATGGGGAGGTGACGGGTTCTATGCCATGGAC TACTGGGGTCAAGGGACACTGGTAACCGTTTCTTCTGGCGGAGGGGGGTTCTGGA GGCGGAGGCAGCGGGGGCGGAGGGTCCGACATTCAGATGACCCAATCCCCCTCT AGCCTTTCAGCCTCCGTAGGGGACAGAGTTACAATAACCTGTAGGGCTAGTCAG GACGTTAATACAGCAGTCGCATGGTACCAGCAGAAGCCCGGTAAGGCTCCTAAG CTTTTGATCTACAGTGCTTCTTTCCTGTATTCTGGTGTACCTAGCCGATTCTCA GGCTCTCGGAGTGGCACAGACTTCACTCTGACAATTTCAAGTCTCCAGCCAGAA GATTTTGCAACCTACTACTGCCAACAACACTACACTACTCCTCCAACCTTCGGA TGTGGCACAAAAGTAGAGATTAAGGGAGGCGGCGGCTCTGACAAAACTCACACG TGCCCACCGTGCCCAGCACCTGAACTCCTGGGGGGACCGTCAGTCTTCCTCTTC CCCCCAAAACCCAAGGACACCCTCATGATCTCCCGGACCCCTGAGGTCACATGC GTGGTGGTGGACGTGAGCCACGAAGACCCTGAGGTCAAGTTCAACTGGTACGTG GACGGCGTGGAGGTGCATAATGCCAAGACAAAGCCGCGGGAGGAGCAGTACAAC | 903 |

| | | | | |
|---|---|---|---|---|
| | | AGCACGTACCGTGTGGTCAGCGTCCTCACCGTCCTGCACCAGGACTGGCTGAAT GGCAAGGAGTACAAGTGCAAGGTCTCCAACAAAGCCCTCCCAGCCCCCATCGAG AAAACCATCTCCAAAGCCAAAGGGCAGCCCCGAGAACCACAGGTGTGCACCCTG CCCCCATCCCGGGAGGAGATGACCAAGAACCAGGTCAGCCTGAGCTGCGCGGTC AAAGGCTTCTATCCCAGCGACATCGCCGTGGAGTGGGAGAGCAATGGGCAGCCG GAGAACAACTACAAGACCACGCCTCCCGTGCTGGACTCCGACGGCTCCTTCTTC CTCGTGAGCAAGCTCACCGTGGACAAGAGCAGGTGGCAGCAGGGGAACGTCTTC TCATGCTCCGTGATGCATGAGGCTCTGCACAACCACTACACGCAGAAGAGCCTC TCCCTGTCTCCGGGTAAA | |
| ME13 | Polypeptide 1 | CAGGTACAACTTCAAGAAAGTGGTCCAGGTCTTGTAAAACCCTCAGAAACTTTG AGTCTCACTTGTACCGTCAGTGGCGGAAGTGTAAGTTCTGGCGATTACTACTGG ACCTGGATACGCCAGTCTCCAGGCAAATGTCTGGAGTGGATAGGCCACATCTAC TACAGCGGGAACACCAATTACAATCCATCTCTTAAATCAAGGTTGACAATTTCA ATAGACACCAGTAAGACCCAGTTTTTCTCTCAAACTTAGCAGTGTAACAGCAGCA GATACTGCAATCTACTACTGCGTTAGAGACCGTGTTACAGGGGCTTTCGACATC TGGGGGCAGGGAACTATGGTTACCGTCTCTTCTGCTAGCACCAAAGGACCTAGT GTTTTTCCTCTTGCCCCTTCCTCAAAGTCTACCTCTGGGGGGACAGCCGCTCTG GGCTGCCTGGTCAAGGATTATTTCCCAGAGCCTGTCACTGTCAGTTGGAACTCT GGAGCCTTGACTTCTGGTGTTCATACATTTCCTGCTGTCCTTCAAAGCAGCGGC TTGTACTCATTGTCTTCTGTTGTGACAGTACCCTCAAGCAGCCTCGGCACTCAG ACATACATCTGCAATGTCAACCACAAACCCTCAAATACAAAGGTAGATAAAAAA GTCGAACCAAAGTCTTGTGACAAAACTCACACGTGCCCACCGTGCCCAGCACCT GAACTCCTGGGGGGACCGTCAGTCTTCCTCTTCCCCCCAAAACCCAAGGACACC CTCATGATCTCCCGGACCCCTGAGGTCACATGCGTGGTGGTGGACGTGAGCCAC GAAGACCCTGAGGTCAAGTTCAACTGGTACGTGGACGGCGTGGAGGTGCATAAT GCCAAGACAAAGCCGCGGGAGGAGCAGTACAACAGCACGTACCGTGTGGTCAGC GTCCTCACCGTCCTGCACCAGGACTGGCTGAATGGCAAGGAGTACAAGTGCAAG GTCTCCAACAAAGCCCTCCCAGCCCCCATCGAGAAAACCATCTCCAAAGCCAAA GGGCAGCCCCGAGAACCACAGGTGTACACCCTGCCCCCCATGCCGGGAGGAGATG | 604 |

| | | | |
|---|---|---|---|
| | | ACCAAGAACCAGGTCAGCCTGTGGTGCCTGGTCAAAGGCTTCTATCCCAGCGAC ATCGCCGTGGAGTGGGAGAGCAATGGGCAGCCGGAGAACAACTACAAGACCACG CCTCCCGTGCTGGACTCCGACGGCTCCTTCTTCCTCTACAGCAAGCTCACCGTG GACAAGAGCAGGTGGCAGCAGGGGAACGTCTTCTCATGCTCCGTGATGCATGAG GCTCTGCACAACCACTACACGCAGAAGAGCCTCTCCCTGTCTCCGGGTAAA | |
| | Polypeptide 2 | GACATACAGATGACACAGAGTCCTAGCTCATTGTCAGCCTCTGTCGGTGACCGC GTCACTATCACCTGCCAAGCCAGCCAAGACATATCAAATTATCTTAACTGGTAC CAGCAAAAGCCTGGAAAGGCTCCAAAACTGCTGATTTACGACGCCTCTAATTTG GAGACCGGGGTTCCCTCTAGGTTCAGCGGGTCTGGTTCAGGCACCGACTTTACA TTCACTATCTCAAGTCTCCAGCCAGAGGACATCGCTACATACTTTTGCCAACAT TTTGACCATCTGCCCCTGGCATTTGGGTGTGGCACCAAGGTAGAAATTAAGCGT ACGGTGGCAGCTCCCAGCGTTTTTATCTTTCCCCCATCCGACGAGCAGCTCAAG AGTGGCACTGCCTCTGTAGTTTGTTTGCTGAATAACTTCTATCCACGTGAAGCA AAAGTACAGTGGAAGGTCGATAATGCCCTTCAGAGCGGTAACAGCCAAGAAAGT GTTACCGAGCAAGATTCCAAAGATTCCACTTACAGTCTGTCCAGCACATTGACA CTGAGTAAGGCTGATTACGAAAAACACAAGGTGTACGCATGCGAGGTGACACAC CAAGGTCTTTCATCTCCTGTAACTAAGAGCTTTAACCGGGGAGAATGTGGTGGT GGGGGCAGCGGGGGCGGAGGTAGTGGAGGCGGCGGTAGTGAACCAAAGAGTAGT GACAAAACTCACACGTGCCCACCGTGCCCAGCACCTGAACTCCTGGGGGGACCG TCAGTCTTCCTCTTCCCCCCAAAAACCCAAGGACACCCTCATGATCTCCCGGACC CCTGAGGTCACATGCGTGGTGGTGGACGTGAGCCACGAAGACCCTGAGGTCAAG TTCAACTGGTACGTGGACGGCGTGGAGGTGCATAATGCCAAGACAAAGCCGCGG GAGGAGCAGTACAACAGCACGTACCGTGTGGTCAGCGTCCTCACCGTCCTGCAC CAGGACTGGCTGAATGGCAAGGAGTACAAGTGCAAGGTCTCCAACAAAGCCCTC CCAGCCCCCATCGAGAAAACCATCTCCAAAGCCAAAGGGCAGCCCCGAGAACCA CAGGTGTACACCCTGCCCCCCATGCCGGGAGGAGATGACCAAGAACCAGGTCAGC CTGTGGTGCCTGGTCAAAGGCTTCTATCCCAGCGACATCGCCGTGGAGTGGGAG AGCAATGGGCAGCCGGAGAACAACTACAAGACCACGCCTCCCGTGCTGGACTCC GACGGCTCCTTCTTCCTCTACAGCAAGCTCACCGTGGACAAGAGCAGGTGGCAG | 605 |

| | | CAGGGGAACGTCTTCTCATGCTCCGTGATGCATGAGGCTCTGCACAACCACTAC ACGCAGAAGAGCCTCTCCCTGTCTCCGGGTAAA | |
| --- | --- | --- | --- |
| | Polypeptide 3 | GAAGTCCAATTGGTCGAGAGTGGAGGCGGGTTGGTACAGCCAGGAGGTTCACTT AGGTTGTCCTGCGCCGCCAGTGGTTACACTTTCACATCTTATTGGCTGCACTGG GTGCGCCAAGCTCCTGGGAAGTGTCTCGAATGGGTGGGTATGATCGATCCATCT AATTCCGACACACGGTTTAACCCAAATTTTAAGGATAGATTTACAATTAGTGCT GACACTTCAAAAAACACAGCATACCTCCAGATGAACAGCCTGCGTGCTGAGGAT ACTGCTGTCTACTACTGTGCAACTTACCGCTCCTATGTCACACCTTTGGATTAT TGGGGCCAGGGGACTCTGGTGACCGTGAGTTCTGGCGGAGGGGGGTTCTGGAGGC GGAGGCAGCGGGGGCGGAGGGTCCGATATACAGATGACACAAAGTCCCTCATCA CTTTCTGCCTCCGTTGGAGATCGTGTGACCATTACCTGTAAGAGTTCCCAATCA CTGCTTTATACCTCTTCACAAAAAAATTACCTCGCTTGGTACCAGCAGAAGCCA GGTAAAGCACCTAAGCTGTTGATCTATTGGGCCTCCACTAGAGAGTCAGGCGTG CCCAGCCGTTTCTCCGGTTCAGGGAGTGGGACAGACTTTACCTTGACCATTTCT TCTTTGCAACCTGAAGACTTCGCCACATACTATTGTCAGCAATATTACGCATAT CCATGGACCTTTGGGTGTGGAACCAAAGTCGAAATAAAAGGAGGCGGCGGCTCT GACAAAACTCACACGTGCCCACCGTGCCCAGCACCTGAACTCCTGGGGGGACCG TCAGTCTTCCTCTTCCCCCCAAAACCCAAGGACACCCTCATGATCTCCCGGACC CCTGAGGTCACATGCGTGGTGGTGGACGTGAGCCACGAAGACCCTGAGGTCAAG TTCAACTGGTACGTGGACGGCGTGGAGGTGCATAATGCCAAGACAAAGCCGCGG GAGGAGCAGTACAACAGCACGTACCGTGTGGTCAGCGTCCTCACCGTCCTGCAC CAGGACTGGCTGAATGGCAAGGAGTACAAGTGCAAGGTCTCCAACAAAGCCCTC CCAGCCCCCATCGAGAAAACCATCTCCAAAGCCAAAGGGCAGCCCCGAGAACCA CAGGTGTGCACCCTGCCCCCATCCCGGGAGGAGATGACCAAGAACCAGGTCAGC CTGAGCTGCGCGGTCAAAGGCTTCTATCCCAGCGACATCGCCGTGGAGTGGGAG AGCAATGGGCAGCCGGAGAACAACTACAAGACCACGCCTCCCGTGCTGGACTCC GACGGCTCCTTCTTCCTCGTGAGCAAGCTCACCGTGGACAAGAGCAGGTGGCAG CAGGGGAACGTCTTCTCATGCTCCGTGATGCATGAGGCTCTGCACAACCACTAC ACGCAGAAGAGCCTCTCCCTGTCTCCGGGTAAA | 904 |

| | Polypeptide 4 | GAAGTCCAATTGGTCGAGAGTGGAGGCGGGTTGGTACAGCCAGGAGGTTCACTT AGGTTGTCCTGCGCCGCCAGTGGTTACACTTTCACATCTTATTGGCTGCACTGG GTGCGCCAAGCTCCTGGGAAGTGTCTCGAATGGGTGGGTATGATCGATCCATCT AATTCCGACACACGGTTTAACCCAAATTTTAAGGATAGATTTACAATTAGTGCT GACACTTCAAAAAACACAGCATACCTCCAGATGAACAGCCTGCGTGCTGAGGAT ACTGCTGTCTACTACTGTGCAACTTACCGCTCCTATGTCACACCTTTGGATTAT TGGGGCCAGGGGACTCTGGTGACCGTGAGTTCTGGCGGAGGGGGTTCTGGAGGC GGAGGCAGCGGGGGCGGAGGGTCCGATATACAGATGACACAAAGTCCCTCATCA CTTTCTGCCTCCGTTGGAGATCGTGTGACCATTACCTGTAAGAGTTCCCAATCA CTGCTTTATACCTCTTCACAAAAAAATTACCTCGCTTGGTACCAGCAGAAGCCA GGTAAAGCACCTAAGCTGTTGATCTATTGGGCCTCCACTAGAGAGTCAGGCGTG CCCAGCCGTTTCTCCGGTTCAGGGAGTGGGACAGACTTTACCTTGACCATTTCT TCTTTGCAACCTGAAGACTTCGCCACATACTATTGTCAGCAATATTACGCATAT CCATGGACCTTTGGGTGTGGAACCAAAGTCGAAATAAAAGGAGGCGGCGGCTCT GACAAAACTCACACGTGCCCACCGTGCCCAGCACCTGAACTCCTGGGGGGACCG TCAGTCTTCCTCTTCCCCCCAAAACCCAAGGACACCCTCATGATCTCCCGGACC CCTGAGGTCACATGCGTGGTGGTGGACGTGAGCCACGAAGACCCTGAGGTCAAG TTCAACTGGTACGTGGACGGCGTGGAGGTGCATAATGCCAAGACAAAGCCGCGG GAGGAGCAGTACAACAGCACGTACCGTGTGGTCAGCGTCCTCACCGTCCTGCAC CAGGACTGGCTGAATGGCAAGGAGTACAAGTGCAAGGTCTCCAACAAAGCCCTC CCAGCCCCCATCGAGAAAACCATCTCCAAAGCCAAAGGGCAGCCCCGAGAACCA CAGGTGTGCACCCTGCCCCCCATCCCGGGAGGAGATGACCAAGAACCAGGTCAGC CTGAGCTGCGCGGTCAAAGGCTTCTATCCCAGCGACATCGCCGTGGAGTGGGAG AGCAATGGGCAGCCGGAGAACAACTACAAGACCACGCCTCCCGTGCTGGACTCC GACGGCTCCTTCTTCCTCGTGAGCAAGCTCACCGTGGACAAGAGCAGGTGGCAG CAGGGGAACGTCTTCTCATGCTCCGTGATGCATGAGGCTCTGCACAACCACTAC ACGCAGAAGAGCCTCTCCCTGTCTCCGGGTAAA | 904 |
| ME14 | Polypeptide 1 | CAGGTACAACTTCAAGAAAGTGGTCCAGGTCTTGTAAAACCCTCAGAAACTTTG AGTCTCACTTGTACCGTCAGTGGCGGAAGTGTAAGTTCTGGCGATTACTACTGG | 604 |

| | | | |
|---|---|---|---|
| | | ACCTGGATACGCCAGTCTCCAGGCAAATGTCTGGAGTGGATAGGCCACATCTAC TACAGCGGGAACACCAATTACAATCCATCTCTTAAATCAAGGTTGACAATTTCA ATAGACACCAGTAAGACCCAGTTTTCTCTCAAACTTAGCAGTGTAACAGCAGCA GATACTGCAATCTACTACTGCGTTAGAGACCGTGTTACAGGGGCTTTCGACATC TGGGGGCAGGGAACTATGGTTACCGTCTCTTCTGCTAGCACCAAAGGACCTAGT GTTTTTCCTCTTGCCCCTTCCTCAAAGTCTACCTCTGGGGGGACAGCCGCTCTG GGCTGCCTGGTCAAGGATTATTTCCCAGAGCCTGTCACTGTCAGTTGGAACTCT GGAGCCTTGACTTCTGGTGTTCATACATTTCCTGCTGTCCTTCAAAGCAGCGGC TTGTACTCATTGTCTTCTGTTGTGACAGTACCCTCAAGCAGCCTCGGCACTCAG ACATACATCTGCAATGTCAACCACAAACCCTCAAATACAAAGGTAGATAAAAAA GTCGAACCAAAGTCTTGTGACAAAACTCACACGTGCCCACCGTGCCCAGCACCT GAACTCCTGGGGGGACCGTCAGTCTTCCTCTTCCCCCCCAAAACCCAAGGACACC CTCATGATCTCCCGGACCCCTGAGGTCACATGCGTGGTGGTGGACGTGAGCCAC GAAGACCCTGAGGTCAAGTTCAACTGGTACGTGGACGGCGTGGAGGTGCATAAT GCCAAGACAAAGCCGCGGGAGGAGCAGTACAACAGCACGTACCGTGTGGTCAGC GTCCTCACCGTCCTGCACCAGGACTGGCTGAATGGCAAGGAGTACAAGTGCAAG GTCTCCAACAAAGCCCTCCCAGCCCCCATCGAGAAAACCATCTCCAAAGCCAAA GGGCAGCCCCGAGAACCACAGGTGTACACCCTGCCCCCCATGCCGGGAGGAGATG ACCAAGAACCAGGTCAGCCTGTGGTGCCTGGTCAAAGGCTTCTATCCCAGCGAC ATCGCCGTGGAGTGGGAGAGCAATGGGCAGCCGGAGAACAACTACAAGACCACG CCTCCCGTGCTGGACTCCGACGGCTCCTTCTTCCTCTACAGCAAGCTCACCGTG GACAAGAGCAGGTGGCAGCAGGGGAACGTCTTCTCATGCTCCGTGATGCATGAG GCTCTGCACAACCACTACACGCAGAAGAGCCTCTCCCTGTCTCCGGGTAAA | |
| | Polypeptide 2 | GACATACAGATGACACAGAGTCCTAGCTCATTGTCAGCCTCTGTCGGTGACCGC GTCACTATCACCTGCCAAGCCAGCCAAGACATATCAAATTATCTTAACTGGTAC CAGCAAAAGCCTGGAAAGGCTCCAAAACTGCTGATTTACGACGCCTCTAATTTG GAGACCGGGGTTCCCTCTAGGTTCAGCGGGTCTGGTTCAGGCACCGACTTTACA TTCACTATCTCAAGTCTCCAGCCAGAGGACATCGCTACATACTTTTGCCAACAT TTTGACCATCTGCCCCTGGCATTTGGGTGTGGCACCAAGGTAGAAATTAAGCGT | 605 |

| | | ACGGTGGCAGCTCCCAGCGTTTTTATCTTTCCCCCATCCGACGAGCAGCTCAAG AGTGGCACTGCCTCTGTAGTTTGTTTGCTGAATAACTTCTATCCACGTGAAGCA AAAGTACAGTGGAAGGTCGATAATGCCCTTCAGAGCGGTAACAGCCAAGAAAGT GTTACCGAGCAAGATTCCAAAGATTCCACTTACAGTCTGTCCAGCACATTGACA CTGAGTAAGGCTGATTACGAAAAACACAAGGTGTACGCATGCGAGGTGACACAC CAAGGTCTTTCATCTCCTGTAACTAAGAGCTTTAACCGGGGGAGAATGTGGTGGT GGGGGCAGCGGGGGCGGAGGTAGTGGAGGCGGCGGTAGTGAACCAAAGAGTAGT GACAAAACTCACACGTGCCCACCGTGCCCAGCACCTGAACTCCTGGGGGGACCG TCAGTCTTCCTCTTCCCCCCAAAACCCAAGGACACCCTCATGATCTCCCGGACC CCTGAGGTCACATGCGTGGTGGTGGACGTGAGCCACGAAGACCCTGAGGTCAAG TTCAACTGGTACGTGGACGGCGTGGAGGTGCATAATGCCAAGACAAAGCCGCGG GAGGAGCAGTACAACAGCACGTACCGTGTGGTCAGCGTCCTCACCGTCCTGCAC CAGGACTGGCTGAATGGCAAGGAGTACAAGTGCAAGGTCTCCAACAAAGCCCTC CCAGCCCCCATCGAGAAAACCATCTCCAAAGCCAAAGGGCAGCCCCGAGAACCA CAGGTGTACACCCTGCCCCCATGCCGGGAGGAGATGACCAAGAACCAGGTCAGC CTGTGGTGCCTGGTCAAAGGCTTCTATCCCAGCGACATCGCCGTGGAGTGGGAG AGCAATGGGCAGCCGGAGAACAACTACAAGACCACGCCTCCCGTGCTGGACTCC GACGGCTCCTTCTTCCTCTACAGCAAGCTCACCGTGGACAAGAGCAGGTGGCAG CAGGGGAACGTCTTCTCATGCTCCGTGATGCATGAGGCTCTGCACAACCACTAC ACGCAGAAGAGCCTCTCCCTGTCTCCGGGTAAA | |
| Polypeptide 3 | CAAGTGCAGCTTGTTCAATCAGGGGCCGAGGTTAAGAAACCAGGTGCTTCCGTC AAGGTCTCCTGCAAGGCTTCCGGCTACACTTTTACCAGTTATGGGTTCAGTTGG GTTAGACAGGCCCCAGGGCAGTGTCTCGAATGGATGGGATGGATTTCCGCATCT AACGGGAATACTTACTATGCCCAGAAACTTCAAGGTAGGGTTACCATGACTACC GATACTTCCACTAGTACAGCCTACATGGAACTCAGATCACTCCGTTCAGATGAC ACCGCAGTATATTACTGTGCAAGGGTATATGCTGATTATGCCGATTATTGGGGG CAAGGAACACTTGTCACAGTATCCAGCGGCGGAGGGGGTTCTGGAGGCGGAGGC AGCGGGGGCGGAGGGTCCGATATCCAAATGACACAGTCACCCTCAAGTGTTAGC GCAAGTGTCGGGGACAGGGTGACCATCACATGCAGAGCTTCCCAGGGTATCAAT | 905 |

| | | | |
|---|---|---|---|
| | | ACATGGCTGGCATGGTATCAACAGAAACCCGGAAAAGCACCAAAATTGCTTATT TATGCAGCTTCTAGCTTGAAGAGTGGGGTTCCCTCTCGTTTCTCTGGTTCAGGA AGCGGTACTGACTTTACCTTGACCATCAGTAGCTTGCAGCCCGAAGATTTCGCT ACATATTATTGCCAACAGGCCAACTCTTTTCCCCTGACATTCGGTTGTGGCACT AAAGTGGAAATTAAGGGAGGCGGCGGCTCTGACAAAACTCACACGTGCCCACCG TGCCCAGCACCTGAACTCCTGGGGGGACCGTCAGTCTTCCTCTTCCCCCCAAAA CCCAAGGACACCCTCATGATCTCCCGGACCCCTGAGGTCACATGCGTGGTGGTG GACGTGAGCCACGAAGACCCTGAGGTCAAGTTCAACTGGTACGTGGACGGCGTG GAGGTGCATAATGCCAAGACAAAGCCGCGGGAGGAGCAGTACAACAGCACGTAC CGTGTGGTCAGCGTCCTCACCGTCCTGCACCAGGACTGGCTGAATGGCAAGGAG TACAAGTGCAAGGTCTCCAACAAAGCCCTCCCAGCCCCCATCGAGAAAACCATC TCCAAAGCCAAAGGGCAGCCCCGAGAACCACAGGTGTGCACCCTGCCCCCATCC CGGGAGGAGATGACCAAGAACCAGGTCAGCCTGAGCTGCGCGGTCAAAGGCTTC TATCCCAGCGACATCGCCGTGGAGTGGGAGAGCAATGGGCAGCCGGAGAACAAC TACAAGACCACGCCTCCCGTGCTGGACTCCGACGGCTCCTTCTTCCTCGTGAGC AAGCTCACCGTGGACAAGAGCAGGTGGCAGCAGGGGAACGTCTTCTCATGCTCC GTGATGCATGAGGCTCTGCACAACCACTACACGCAGAAGAGCCTCTCCCTGTCT CCGGGTAAA | |
| | Polypeptide 4 | CAAGTGCAGCTTGTTCAATCAGGGGCCGAGGTTAAGAAACCAGGTGCTTCCGTC AAGGTCTCCTGCAAGGCTTCCGGCTACACTTTTACCAGTTATGGGTTCAGTTGG GTTAGACAGGCCCCAGGGCAGTGTCTCGAATGGATGGGATGGATTTCCGCATCT AACGGGAATACTTACTATGCCCAGAAACTTCAAGGTAGGGTTACCATGACTACC GATACTTCCACTAGTACAGCCTACATGGAACTCAGATCACTCCGTTCAGATGAC ACCGCAGTATATTACTGTGCAAGGGTATATGCTGATTATGCCGATTATTGGGGG CAAGGAACACTTGTCACAGTATCCAGCGGCGGAGGGGGTTCTGGAGGCGGAGGC AGCGGGGGCGGAGGGTCCGATATCCAAATGACACAGTCACCCTCAAGTGTTAGC GCAAGTGTCGGGGACAGGGTGACCATCACATGCAGAGCTTCCCAGGGTATCAAT ACATGGCTGGCATGGTATCAACAGAAACCCGGAAAAGCACCAAAATTGCTTATT TATGCAGCTTCTAGCTTGAAGAGTGGGGTTCCCTCTCGTTTCTCTGGTTCAGGA | 905 |

| | | | |
|---|---|---|---|
| | | AGCGGTACTGACTTTACCTTGACCATCAGTAGCTTGCAGCCCGAAGATTTCGCT ACATATTATTGCCAACAGGCCAACTCTTTTCCCCTGACATTCGGTTGTGGCACT AAAGTGGAAATTAAGGGAGGCGGCGGCTCTGACAAAACTCACACGTGCCCACCG TGCCCAGCACCTGAACTCCTGGGGGGACCGTCAGTCTTCCTCTTCCCCCCAAAA CCCAAGGACACCCTCATGATCTCCCGGACCCCTGAGGTCACATGCGTGGTGGTG GACGTGAGCCACGAAGACCCTGAGGTCAAGTTCAACTGGTACGTGGACGGCGTG GAGGTGCATAATGCCAAGACAAAGCCGCGGGAGGAGCAGTACAACAGCACGTAC CGTGTGGTCAGCGTCCTCACCGTCCTGCACCAGGACTGGCTGAATGGCAAGGAG TACAAGTGCAAGGTCTCCAACAAAGCCCTCCCAGCCCCCATCGAGAAAACCATC TCCAAAGCCAAAGGGCAGCCCCGAGAACCACAGGTGTGCACCCTGCCCCCATCC CGGGAGGAGATGACCAAGAACCAGGTCAGCCTGAGCTGCGCGGTCAAAGGCTTC TATCCCAGCGACATCGCCGTGGAGTGGGAGAGCAATGGGCAGCCGGAGAACAAC TACAAGACCACGCCTCCCGTGCTGGACTCCGACGGCTCCTTCTTCCTCGTGAGC AAGCTCACCGTGGACAAGAGCAGGTGGCAGCAGGGGAACGTCTTCTCATGCTCC GTGATGCATGAGGCTCTGCACAACCACTACACGCAGAAGAGCCTCTCCCTGTCT CCGGGTAAA | |
| ME15 | Polypeptide 1 | GAAGTCCAATTGGTCGAGAGTGGAGGCGGGTTGGTACAGCCAGGAGGTTCACTT AGGTTGTCCTGCGCCGCCAGTGGTTACACTTTCACATCTTATTGGCTGCACTGG GTGCGCCAAGCTCCTGGGAAGTGTCTCGAATGGGTGGGTATGATCGATCCATCT AATTCCGACACACGGTTTAACCCAAATTTTAAGGATAGATTTACAATTAGTGCT GACACTTCAAAAAAACACAGCATACCTCCAGATGAACAGCCTGCGTGCTGAGGAT ACTGCTGTCTACTACTGTGCAACTTACCGCTCCTATGTCACACCTTTGGATTAT TGGGGCCAGGGGACTCTGGTGACCGTGAGTTCTGCTAGCACCAAAGGACCTAGT GTTTTTCCTCTTGCCCCTTCCTCAAAGTCTACCTCTGGGGGGACAGCCGCTCTG GGCTGCCTGGTCAAGGATTATTTCCCAGAGCCTGTCACTGTCAGTTGGAACTCT GGAGCCTTGACTTCTGGTGTTCATACATTTCCTGCTGTCCTTCAAAGCAGCGGC TTGTACTCATTGTCTTCTGTTGTGACAGTACCCTCAAGCAGCCTCGGCACTCAG ACATACATCTGCAATGTCAACCACAAACCCTCAAATACAAAGGTAGATAAAAAA GTCGAACCAAAGTCTTGTGACAAAACTCACACGTGCCCACCGTGCCCAGCACCT | 572 |

| | | GAACTCCTGGGGGGACCGTCAGTCTTCCTCTTCCCCCCCAAAACCCAAGGACACC | |
| --- | --- | --- | --- |
| | | CTCATGATCTCCCGGACCCCTGAGGTCACATGCGTGGTGGTGGACGTGAGCCAC | |
| | | GAAGACCCTGAGGTCAAGTTCAACTGGTACGTGGACGGCGTGGAGGTGCATAAT | |
| | | GCCAAGACAAAGCCGCGGGAGGAGCAGTACAACAGCACGTACCGTGTGGTCAGC | |
| | | GTCCTCACCGTCCTGCACCAGGACTGGCTGAATGGCAAGGAGTACAAGTGCAAG | |
| | | GTCTCCAACAAAGCCCTCCCAGCCCCCATCGAGAAAACCATCTCCAAAGCCAAA | |
| | | GGGCAGCCCCGAGAACCACAGGTGTACACCCTGCCCCCATGCCGGGAGGAGATG | |
| | | ACCAAGAACCAGGTCAGCCTGTGGTGCCTGGTCAAAGGCTTCTATCCCAGCGAC | |
| | | ATCGCCGTGGAGTGGGAGAGCAATGGGCAGCCGGAGAACAACTACAAGACCACG | |
| | | CCTCCCGTGCTGGACTCCGACGGCTCCTTCTTCCTCTACAGCAAGCTCACCGTG | |
| | | GACAAGAGCAGGTGGCAGCAGGGGAACGTCTTCTCATGCTCCGTGATGCATGAG | |
| | | GCTCTGCACAACCACTACACGCAGAAGAGCCTCTCCCTGTCTCCGGGTAAA | |
| | Polypeptide 2 | GATATACAGATGACACAAAGTCCCTCATCACTTTCTGCCTCCGTTGGAGATCGT | |
| | | GTGACCATTACCTGTAAGAGTTCCCAATCACTGCTTTATACCTCTTCACAAAAA | |
| | | AATTACCTCGCTTGGTACCAGCAGAAGCCAGGTAAAGCACCTAAGCTGTTGATC | |
| | | TATTGGGCCTCCACTAGAGAGTCAGGCGTGCCCAGCCGTTTCTCCGGTTCAGGG | |
| | | AGTGGGACAGACTTTACCTTGACCATTTCTTCTTTGCAACCTGAAGACTTCGCC | |
| | | ACATACTATTGTCAGCAATATTACGCATATCCATGGACCTTTGGGTGTGGAACC | |
| | | AAAGTCGAAATAAAACGTACGGTGGCAGCTCCCAGCGTTTTTATCTTTCCCCCA | |
| | | TCCGACGAGCAGCTCAAGAGTGGCACTGCCTCTGTAGTTTGTTTGCTGAATAAC | |
| | | TTCTATCCACGTGAAGCAAAAGTACAGTGGAAGGTCGATAATGCCCTTCAGAGC | 573 |
| | | GGTAACAGCCAAGAAAGTGTTACCGAGCAAGATTCCAAAGATTCCACTTACAGT | |
| | | CTGTCCAGCACATTGACACTGAGTAAGGCTGATTACGAAAAACACAAGGTGTAC | |
| | | GCATGCGAGGTGACACACCAAGGTCTTTCATCTCCTGTAACTAAGAGCTTTAAC | |
| | | CGGGGAGAATGTGGTGGTGGGGGCAGCGGGGGCGGAGGTAGTGGAGGCGGCGGT | |
| | | AGTGAACCAAAGAGTAGTGACAAAACTCACACGTGCCCACCGTGCCCAGCACCT | |
| | | GAACTCCTGGGGGGACCGTCAGTCTTCCTCTTCCCCCCCAAAACCCAAGGACACC | |
| | | CTCATGATCTCCCGGACCCCTGAGGTCACATGCGTGGTGGTGGACGTGAGCCAC | |
| | | GAAGACCCTGAGGTCAAGTTCAACTGGTACGTGGACGGCGTGGAGGTGCATAAT | |

| | | GCCAAGACAAAGCCGCGGGAGGAGCAGTACAACAGCACGTACCGTGTGGTCAGC GTCCTCACCGTCCTGCACCAGGACTGGCTGAATGGCAAGGAGTACAAGTGCAAG GTCTCCAACAAAGCCCTCCCAGCCCCCATCGAGAAAACCATCTCCAAAGCCAAA GGGCAGCCCCGAGAACCACAGGTGTACACCCTGCCCCCATGCCGGGAGGAGATG ACCAAGAACCAGGTCAGCCTGTGGTGCCTGGTCAAAGGCTTCTATCCCAGCGAC ATCGCCGTGGAGTGGGAGAGCAATGGGCAGCCGGAGAACAACTACAAGACCACG CCTCCCGTGCTGGACTCCGACGGCTCCTTCTTCCTCTACAGCAAGCTCACCGTG GACAAGAGCAGGTGGCAGCAGGGGAACGTCTTCTCATGCTCCGTGATGCATGAG GCTCTGCACAACCACTACACGCAGAAGAGCCTCTCCCTGTCTCCGGGTAAA | |
|---|---|---|---|
| | Polypeptide 3 | CAGGTACAACTTCAAGAAAGTGGTCCAGGTCTTGTAAAACCCTCAGAAACTTTG AGTCTCACTTGTACCGTCAGTGGCGGAAGTGTAAGTTCTGGCGATTACTACTGG ACCTGGATACGCCAGTCTCCAGGCAAATGTCTGGAGTGGATAGGCCACATCTAC TACAGCGGGAACACCAATTACAATCCATCTCTTAAATCAAGGTTGACAATTTCA ATAGACACCAGTAAGACCCAGTTTTCTCTCAAACTTAGCAGTGTAACAGCAGCA GATACTGCAATCTACTACTGCGTTAGAGACCGTGTTACAGGGGCTTTCGACATC TGGGGGCAGGGAACTATGGTTACCGTCTCTTCTGGCGGAGGGGGTTCTGGAGGC GGAGGCAGCGGGGGCGGAGGGTCCGACATACAGATGACACAGAGTCCTAGCTCA TTGTCAGCCTCTGTCGGTGACCGCGTCACTATCACCTGCCAAGCCAGCCAAGAC ATATCAAATTATCTTAACTGGTACCAGCAAAAGCCTGGAAAGGCTCCAAAACTG CTGATTTACGACGCCTCTAATTTGGAGACCGGGGTTCCCTCTAGGTTCAGCGGG TCTGGTTCAGGCACCGACTTTACATTCACTATCTCAAGTCTCCAGCCAGAGGAC ATCGCTACATACTTTTGCCAACATTTTGACCATCTGCCCCCTGGCATTTGGGTGT GGCACCAAGGTAGAAATTAAGGGAGGCGGCGGCTCTGACAAAACTCACACGTGC CCACCGTGCCCAGCACCTGAACTCCTGGGGGGACCGTCAGTCTTCCTCTTCCCC CCAAAACCCAAGGACACCCTCATGATCTCCCGGACCCCTGAGGTCACATGCGTG GTGGTGGACGTGAGCCACGAAGACCCTGAGGTCAAGTTCAACTGGTACGTGGAC GGCGTGGAGGTGCATAATGCCAAGACAAAGCCGCGGGAGGAGCAGTACAACAGC ACGTACCGTGTGGTCAGCGTCCTCACCGTCCTGCACCAGGACTGGCTGAATGGC AAGGAGTACAAGTGCAAGGTCTCCAACAAAGCCCTCCCAGCCCCCATCGAGAAA | 906 |

| | | | | |
|---|---|---|---|---|
| | | ACCATCTCCAAAGCCAAAGGGCAGCCCCGAGAACCACAGGTGTGCACCCTGCCC CCATCCCGGGAGGAGATGACCAAGAACCAGGTCAGCCTGAGCTGCGCGGTCAAA GGCTTCTATCCCAGCGACATCGCCGTGGAGTGGGAGAGCAATGGGCAGCCGGAG AACAACTACAAGACCACGCCTCCCGTGCTGGACTCCGACGGCTCCTTCTTCCTC GTGAGCAAGCTCACCGTGGACAAGAGCAGGTGGCAGCAGGGGAACGTCTTCTCA TGCTCCGTGATGCATGAGGCTCTGCACAACCACTACACGCAGAAGAGCCTCTCC CTGTCTCCGGGTAAA | |
| | Polypeptide 4 | CAGGTACAACTTCAAGAAAGTGGTCCAGGTCTTGTAAAACCCTCAGAAACTTTG AGTCTCACTTGTACCGTCAGTGGCGGAAGTGTAAGTTCTGGCGATTACTACTGG ACCTGGATACGCCAGTCTCCAGGCAAATGTCTGGAGTGGATAGGCCACATCTAC TACAGCGGGAACACCAATTACAATCCATCTCTTAAATCAAGGTTGACAATTTCA ATAGACACCAGTAAGACCCAGTTTTCTCTCAAACTTAGCAGTGTAACAGCAGCA GATACTGCAATCTACTACTGCGTTAGAGACCGTGTTACAGGGGGCTTTCGACATC TGGGGGCAGGGAACTATGGTTACCGTCTCTTCTGGCGGAGGGGGTTCTGGAGGC GGAGGCAGCGGGGGCGGAGGGTCCGACATACAGATGACACAGAGTCCTAGCTCA TTGTCAGCCTCTGTCGGTGACCGCGTCACTATCACCTGCCAAGCCAGCCAAGAC ATATCAAATTATCTTAACTGGTACCAGCAAAAGCCTGGAAAGGCTCCAAAACTG CTGATTTACGACGCCTCTAATTTGGAGACCGGGGTTCCCTCTAGGTTCAGCGGG TCTGGTTCAGGCACCGACTTTACATTCACTATCTCAAGTCTCCAGCCAGAGGAC ATCGCTACATACTTTTGCCAACATTTTGACCATCTGCCCCCTGGCATTTGGGTGT GGCACCAAGGTAGAAATTAAGGGAGGCGGCGGCTCTGACAAAACTCACACGTGC CCACCGTGCCCAGCACCTGAACTCCTGGGGGGACCGTCAGTCTTCCTCTTCCCC CCAAAACCCAAGGACACCCTCATGATCTCCCGGACCCCTGAGGTCACATGCGTG GTGGTGGACGTGAGCCACGAAGACCCTGAGGTCAAGTTCAACTGGTACGTGGAC GGCGTGGAGGTGCATAATGCCAAGACAAAGCCGCGGGAGGAGCAGTACAACAGC ACGTACCGTGTGGTCAGCGTCCTCACCGTCCTGCACCAGGACTGGCTGAATGGC AAGGAGTACAAGTGCAAGGTCTCCAACAAAGCCCTCCCAGCCCCCATCGAGAAA ACCATCTCCAAAGCCAAAGGGCAGCCCCGAGAACCACAGGTGTGCACCCTGCCC CCATCCCGGGAGGAGATGACCAAGAACCAGGTCAGCCTGAGCTGCGCGGTCAAA | 906 |

| | | GGCTTCTATCCCAGCGACATCGCCGTGGAGTGGGAGAGCAATGGGCAGCCGGAG AACAACTACAAGACCACGCCTCCCGTGCTGGACTCCGACGGCTCCTTCTTCCTC GTGAGCAAGCTCACCGTGGACAAGAGCAGGTGGCAGCAGGGGAACGTCTTCTCA TGCTCCGTGATGCATGAGGCTCTGCACAACCACTACACGCAGAAGAGCCTCTCC CTGTCTCCGGGTAAA | |
|---|---|---|---|
| ME16 | Polypeptide 1 | CAAGTGCAGCTTGTTCAATCAGGGGCCGAGGTTAAGAAACCAGGTGCTTCCGTC AAGGTCTCCTGCAAGGCTTCCGGCTACACTTTTACCAGTTATGGGTTCAGTTGG GTTAGACAGGCCCCAGGGCAGTGTCTCGAATGGATGGGATGGATTTCCGCATCT AACGGGAATACTTACTATGCCCAGAAACTTCAAGGTAGGGTTACCATGACTACC GATACTTCCACTAGTACAGCCTACATGGAACTCAGATCACTCCGTTCAGATGAC ACCGCAGTATATTACTGTGCAAGGGTATATGCTGATTATGCCGATTATTGGGGG CAAGGAACACTTGTCACAGTATCCAGCGCTAGCACCAAAGGACCTAGTGTTTTT CCTCTTGCCCCCTTCCTCAAAGTCTACCTCTGGGGGGACAGCCGCTCTGGGCTGC CTGGTCAAGGATTATTTCCCAGAGCCTGTCACTGTCAGTTGGAACTCTGGAGCC TTGACTTCTGGTGTTCATACATTTCCTGCTGTCCTTCAAAGCAGCGGCTTGTAC TCATTGTCTTCTGTTGTGACAGTACCCTCAAGCAGCCTCGGCACTCAGACATAC ATCTGCAATGTCAACCACAAACCCTCAAATACAAAGGTAGATAAAAAAGTCGAA CCAAAGTCTTGTGACAAAACTCACACGTGCCCACCGTGCCCAGCACCTGAACTC CTGGGGGGACCGTCAGTCTTCCTCTTCCCCCCAAAACCCAAGGACACCCTCATG ATCTCCCGGACCCCTGAGGTCACATGCGTGGTGGTGGACGTGAGCCACGAAGAC CCTGAGGTCAAGTTCAACTGGTACGTGGACGGCGTGGAGGTGCATAATGCCAAG ACAAAGCCGCGGGAGGAGCAGTACAACAGCACGTACCGTGTGGTCAGCGTCCTC ACCGTCCTGCACCAGGACTGGCTGAATGGCAAGGAGTACAAGTGCAAGGTCTCC AACAAAGCCCTCCCAGCCCCCATCGAGAAAACCATCTCCAAAGCCAAAGGGCAG CCCCGAGAACCACAGGTGTACACCCTGCCCCCCATGCCGGGAGGAGATGACCAAG AACCAGGTCAGCCTGTGGTGCCTGGTCAAAGGCTTCTATCCCAGCGACATCGCC GTGGAGTGGGAGAGCAATGGGCAGCCGGAGAACAACTACAAGACCACGCCTCCC GTGCTGGACTCCGACGGCTCCTTCTTCCTCTACAGCAAGCTCACCGTGGACAAG AGCAGGTGGCAGCAGGGGAACGTCTTCTCATGCTCCGTGATGCATGAGGCTCTG | 574 |

| | | | |
|---|---|---|---|
| | | CACAACCACTACACGCAGAAGAGCCTCTCCCTGTCTCCGGGTAAA | |
| | Polypeptide 2 | GATATCCAAATGACACAGTCACCCTCAAGTGTTAGCGCAAGTGTCGGGGACAGG GTGACCATCACATGCAGAGCTTCCCAGGGTATCAATACATGGCTGGCATGGTAT CAACAGAAACCCGGAAAAGCACCAAAATTGCTTATTTATGCAGCTTCTAGCTTG AAGAGTGGGGTTCCCTCTCGTTTCTCTGGTTCAGGAAGCGGTACTGACTTTACC TTGACCATCAGTAGCTTGCAGCCCGAAGATTTCGCTACATATTATTGCCAACAG GCCAACTCTTTTCCCCTGACATTCGGTTGTGGCACTAAAGTGGAAATTAAGCGT ACGGTGGCAGCTCCCAGCGTTTTTATCTTTCCCCCATCCGACGAGCAGCTCAAG AGTGGCACTGCCTCTGTAGTTTGTTTGCTGAATAACTTCTATCCACGTGAAGCA AAAGTACAGTGGAAGGTCGATAATGCCCTTCAGAGCGGTAACAGCCAAGAAAGT GTTACCGAGCAAGATTCCAAAGATTCCACTTACAGTCTGTCCAGCACATTGACA CTGAGTAAGGCTGATTACGAAAAACACAAGGTGTACGCATGCGAGGTGACACAC CAAGGTCTTTCATCTCCTGTAACTAAGAGCTTTAACCGGGGAGAATGTGGTGGT GGGGGCAGCGGGGGCGGAGGTAGTGGAGGCGGCGGTAGTGAACCAAAGAGTAGT GACAAAACTCACACGTGCCCACCGTGCCCAGCACCTGAACTCCTGGGGGGACCG TCAGTCTTCCTCTTCCCCCCAAAACCCAAGGACACCCTCATGATCTCCCGGACC CCTGAGGTCACATGCGTGGTGGTGGACGTGAGCCACGAAGACCCTGAGGTCAAG TTCAACTGGTACGTGGACGGCGTGGAGGTGCATAATGCCAAGACAAAGCCGCGG GAGGAGCAGTACAACAGCACGTACCGTGTGGTCAGCGTCCTCACCGTCCTGCAC CAGGACTGGCTGAATGGCAAGGAGTACAAGTGCAAGGTCTCCAACAAAGCCCTC CCAGCCCCCATCGAGAAAACCATCTCCAAAGCCAAAGGGCAGCCCCGAGAACCA CAGGTGTACACCCTGCCCCCATGCCGGGAGGAGATGACCAAGAACCAGGTCAGC CTGTGGTGCCTGGTCAAAGGCTTCTATCCCAGCGACATCGCCGTGGAGTGGGAG AGCAATGGGCAGCCGGAGAACAACTACAAGACCACGCCTCCCGTGCTGGACTCC GACGGCTCCTTCTTCCTCTACAGCAAGCTCACCGTGGACAAGAGCAGGTGGCAG CAGGGGAACGTCTTCTCATGCTCCGTGATGCATGAGGCTCTGCACAACCACTAC ACGCAGAAGAGCCTCTCCCTGTCTCCGGGTAAA | 575 | |
| | Polypeptide 3 | CAGGTACAACTTCAAGAAAGTGGTCCAGGTCTTGTAAAACCCTCAGAAACTTTG AGTCTCACTTGTACCGTCAGTGGCGGAAGTGTAAGTTCTGGCGATTACTACTGG | 906 | |

| | | ACCTGGATACGCCAGTCTCCAGGCAAATGTCTGGAGTGGATAGGCCACATCTAC TACAGCGGGAACACCAATTACAATCCATCTCTTAAATCAAGGTTGACAATTTCA ATAGACACCAGTAAGACCCAGTTTTCTCTCAAACTTAGCAGTGTAACAGCAGCA GATACTGCAATCTACTACTGCGTTAGAGACCGTGTTACAGGGGCTTTCGACATC TGGGGGCAGGGAACTATGGTTACCGTCTCTTCTGGCGGAGGGGGTTCTGGAGGC GGAGGCAGCGGGGGCGGAGGGTCCGACATACAGATGACACAGAGTCCTAGCTCA TTGTCAGCCTCTGTCGGTGACCGCGTCACTATCACCTGCCAAGCCAGCCAAGAC ATATCAAATTATCTTAACTGGTACCAGCAAAAGCCTGGAAAGGCTCCAAAACTG CTGATTTACGACGCCTCTAATTTGGAGACCGGGGTTCCCTCTAGGTTCAGCGGG TCTGGTTCAGGCACCGACTTTACATTCACTATCTCAAGTCTCCAGCCAGAGGAC ATCGCTACATACTTTTGCCAACATTTTGACCATCTGCCCCTGGCATTTGGGTGT GGCACCAAGGTAGAAATTAAGGGAGGCGGCGGCTCTGACAAAACTCACACGTGC CCACCGTGCCCAGCACCTGAACTCCTGGGGGGACCGTCAGTCTTCCTCTTCCCC CCAAAACCCAAGGACACCCTCATGATCTCCCGGACCCCTGAGGTCACATGCGTG GTGGTGGACGTGAGCCACGAAGACCCTGAGGTCAAGTTCAACTGGTACGTGGAC GGCGTGGAGGTGCATAATGCCAAGACAAAGCCGCGGGAGGAGCAGTACAACAGC ACGTACCGTGTGGTCAGCGTCCTCACCGTCCTGCACCAGGACTGGCTGAATGGC AAGGAGTACAAGTGCAAGGTCTCCAACAAAGCCCTCCCAGCCCCCATCGAGAAA ACCATCTCCAAAGCCAAAGGGCAGCCCCGAGAACCACAGGTGTGCACCCTGCCC CCATCCCGGGAGGAGATGACCAAGAACCAGGTCAGCCTGAGCTGCGCGGTCAAA GGCTTCTATCCCAGCGACATCGCCGTGGAGTGGGAGAGCAATGGGCAGCCGGAG AACAACTACAAGACCACGCCTCCCGTGCTGGACTCCGACGGCTCCTTCTTCCTC GTGAGCAAGCTCACCGTGGACAAGAGCAGGTGGCAGCAGGGGAACGTCTTCTCA TGCTCCGTGATGCATGAGGCTCTGCACAACCACTACACGCAGAAGAGCCTCTCC CTGTCTCCGGGTAAA | |
| Polypeptide 4 | CAGGTACAACTTCAAGAAAGTGGTCCAGGTCTTGTAAAACCCTCAGAAACTTTG AGTCTCACTTGTACCGTCAGTGGCGGAAGTGTAAGTTCTGGCGATTACTACTGG ACCTGGATACGCCAGTCTCCAGGCAAATGTCTGGAGTGGATAGGCCACATCTAC TACAGCGGGAACACCAATTACAATCCATCTCTTAAATCAAGGTTGACAATTTCA | 906 |

| | | | |
|---|---|---|---|
| | | ATAGACACCAGTAAGACCCAGTTTTCTCTCAAACTTAGCAGTGTAACAGCAGCA GATACTGCAATCTACTACTGCGTTAGAGACCGTGTTACAGGGGCTTTCGACATC TGGGGGCAGGGAACTATGGTTACCGTCTCTTCTGGCGGAGGGGGTTCTGGAGGC GGAGGCAGCGGGGGCGGAGGGTCCGACATACAGATGACACAGAGTCCTAGCTCA TTGTCAGCCTCTGTCGGTGACCGCGTCACTATCACCTGCCAAGCCAGCCAAGAC ATATCAAATTATCTTAACTGGTACCAGCAAAAGCCTGGAAAGGCTCCAAAACTG CTGATTTACGACGCCTCTAATTTGGAGACCGGGGTTCCCTCTAGGTTCAGCGGG TCTGGTTCAGGCACCGACTTTACATTCACTATCTCAAGTCTCCAGCCAGAGGAC ATCGCTACATACTTTTGCCAACATTTTGACCATCTGCCCCTGGCATTTGGGTGT GGCACCAAGGTAGAAATTAAGGGAGGCGGCGGCTCTGACAAAACTCACACGTGC CCACCGTGCCCAGCACCTGAACTCCTGGGGGGACCGTCAGTCTTCCTCTTCCCC CCAAAACCCAAGGACACCCTCATGATCTCCCGGACCCCTGAGGTCACATGCGTG GTGGTGGACGTGAGCCACGAAGACCCTGAGGTCAAGTTCAACTGGTACGTGGAC GGCGTGGAGGTGCATAATGCCAAGACAAAGCCGCGGGAGGAGCAGTACAACAGC ACGTACCGTGTGGTCAGCGTCCTCACCGTCCTGCACCAGGACTGGCTGAATGGC AAGGAGTACAAGTGCAAGGTCTCCAACAAAGCCCTCCCAGCCCCCATCGAGAAA ACCATCTCCAAAGCCAAAGGGCAGCCCCGAGAACCACAGGTGTGCACCCTGCCC CCATCCCGGGAGGAGATGACCAAGAACCAGGTCAGCCTGAGCTGCGCGGTCAAA GGCTTCTATCCCAGCGACATCGCCGTGGAGTGGGAGAGCAATGGGCAGCCGGAG AACAACTACAAGACCACGCCTCCCGTGCTGGACTCCGACGGCTCCTTCTTCCTC GTGAGCAAGCTCACCGTGGACAAGAGCAGGTGGCAGCAGGGGAACGTCTTCTCA TGCTCCGTGATGCATGAGGCTCTGCACAACCACTACACGCAGAAGAGCCTCTCC CTGTCTCCGGGTAAA | |
| CE05 | Polypeptide 1 | CAGGTACAACTTCAAGAAAGTGGTCCAGGTCTTGTAAAACCCTCAGAAACTTTG AGTCTCACTTGTACCGTCAGTGGCGGAAGTGTAAGTTCTGGCGATTACTACTGG ACCTGGATACGCCAGTCTCCAGGCAAATGTCTGGAGTGGATAGGCCACATCTAC TACAGCGGGAACACCAATTACAATCCATCTCTTAAATCAAGGTTGACAATTTCA ATAGACACCAGTAAGACCCAGTTTTCTCTCAAACTTAGCAGTGTAACAGCAGCA GATACTGCAATCTACTACTGCGTTAGAGACCGTGTTACAGGGGCTTTCGACATC | 604 |

| | | | |
|---|---|---|---|
| | | TGGGGGCAGGGAACTATGGTTACCGTCTCTTCTGCTAGCACCAAAGGACCTAGT<br>GTTTTTCCTCTTGCCCCTTCCTCAAAGTCTACCTCTGGGGGGACAGCCGCTCTG<br>GGCTGCCTGGTCAAGGATTATTTCCCAGAGCCTGTCACTGTCAGTTGGAACTCT<br>GGAGCCTTGACTTCTGGTGTTCATACATTTCCTGCTGTCCTTCAAAGCAGCGGC<br>TTGTACTCATTGTCTTCTGTTGTGACAGTACCCTCAAGCAGCCTCGGCACTCAG<br>ACATACATCTGCAATGTCAACCACAAACCCTCAAATACAAAGGTAGATAAAAAA<br>GTCGAACCAAAGTCTTGTGACAAAACTCACACGTGCCCACCGTGCCCAGCACCT<br>GAACTCCTGGGGGGACCGTCAGTCTTCCTCTTCCCCCCAAAACCCAAGGACACC<br>CTCATGATCTCCCGGACCCCTGAGGTCACATGCGTGGTGGTGGACGTGAGCCAC<br>GAAGACCCTGAGGTCAAGTTCAACTGGTACGTGGACGGCGTGGAGGTGCATAAT<br>GCCAAGACAAAGCCGCGGGAGGAGCAGTACAACAGCACGTACCGTGTGGTCAGC<br>GTCCTCACCGTCCTGCACCAGGACTGGCTGAATGGCAAGGAGTACAAGTGCAAG<br>GTCTCCAACAAAGCCCTCCCAGCCCCCATCGAGAAAACCATCTCCAAAGCCAAA<br>GGGCAGCCCCGAGAACCACAGGTGTACACCCTGCCCCCATGCCGGGAGGAGATG<br>ACCAAGAACCAGGTCAGCCTGTGGTGCCTGGTCAAAGGCTTCTATCCCAGCGAC<br>ATCGCCGTGGAGTGGGAGAGCAATGGGCAGCCGGAGAACAACTACAAGACCACG<br>CCTCCCGTGCTGGACTCCGACGGCTCCTTCTTCCTCTACAGCAAGCTCACCGTG<br>GACAAGAGCAGGTGGCAGCAGGGGAACGTCTTCTCATGCTCCGTGATGCATGAG<br>GCTCTGCACAACCACTACACGCAGAAGAGCCTCTCCCTGTCTCCGGGTAAA | |
| | Polypeptide 2 | GACATACAGATGACACAGAGTCCTAGCTCATTGTCAGCCTCTGTCGGTGACCGC<br>GTCACTATCACCTGCCAAGCCAGCCAAGACATATCAAATTATCTTAACTGGTAC<br>CAGCAAAAGCCTGGAAAGGCTCCAAAACTGCTGATTTACGACGCCTCTAATTTG<br>GAGACCGGGGTTCCCTCTAGGTTCAGCGGGTCTGGTTCAGGCACCGACTTTACA<br>TTCACTATCTCAAGTCTCCAGCCAGAGGACATCGCTACATACTTTTGCCAACAT<br>TTTGACCATCTGCCCCTGGCATTTGGGTGTGGCACCAAGGTAGAAATTAAGCGT<br>ACGGTGGCAGCTCCCAGCGTTTTTATCTTTCCCCCATCCGACGAGCAGCTCAAG<br>AGTGGCACTGCCTCTGTAGTTTGTTTGCTGAATAACTTCTATCCACGTGAAGCA<br>AAAGTACAGTGGAAGGTCGATAATGCCCTTCAGAGCGGTAACAGCCAAGAAAGT<br>GTTACCGAGCAAGATTCCAAAGATTCCACTTACAGTCTGTCCAGCACATTGACA | 605 |

| | | | |
|---|---|---|---|
| | | CTGAGTAAGGCTGATTACGAAAAACACAAGGTGTACGCATGCGAGGTGACACAC CAAGGTCTTTCATCTCCTGTAACTAAGAGCTTTAACCGGGGAGAATGTGGTGGT GGGGGCAGCGGGGGCGGAGGTAGTGGAGGCGGCGGTAGTGAACCAAAGAGTAGT GACAAAACTCACACGTGCCCACCGTGCCCAGCACCTGAACTCCTGGGGGGACCG TCAGTCTTCCTCTTCCCCCCAAAAACCCAAGGACACCCTCATGATCTCCCGGACC CCTGAGGTCACATGCGTGGTGGTGGACGTGAGCCACGAAGACCCTGAGGTCAAG TTCAACTGGTACGTGGACGGCGTGGAGGTGCATAATGCCAAGACAAAGCCGCGG GAGGAGCAGTACAACAGCACGTACCGTGTGGTCAGCGTCCTCACCGTCCTGCAC CAGGACTGGCTGAATGGCAAGGAGTACAAGTGCAAGGTCTCCAACAAAGCCCTC CCAGCCCCCATCGAGAAAACCATCTCCAAAGCCAAAGGGCAGCCCCGAGAACCA CAGGTGTACACCCTGCCCCCATGCCGGGAGGAGATGACCAAGAACCAGGTCAGC CTGTGGTGCCTGGTCAAAGGCTTCTATCCCAGCGACATCGCCGTGGAGTGGGAG AGCAATGGGCAGCCGGAGAACAACTACAAGACCACGCCTCCCGTGCTGGACTCC GACGGCTCCTTCTTCCTCTACAGCAAGCTCACCGTGGACAAGAGCAGGTGGCAG CAGGGGAACGTCTTCTCATGCTCCGTGATGCATGAGGCTCTGCACAACCACTAC ACGCAGAAGAGCCTCTCCCTGTCTCCGGGTAAA | |
| | Polypeptide 3 | GAAGTGCAACTCGTAGAATCCGGCGGGGGCGTCGTACAGCCCGGCCGTTCTTTG AGACTTAGCTGTAGTGCTTCTGGGTTTGACTTCACTACATACTGGATGTCATGG GTAAGACAGGCACCTGGCAAGTGCCTTGAATGGATCGGTGAAATCCATCCCGAC AGCTCCACAATCAACTACGCCCCAAGTTTGAAAGACCGGTTCACCATATCTCGT GACAACGCCAAGAATACATTGTTCCTTCAGATGGATAGTCTTCGTCCAGAGGAT ACTGGGGTATATTTTTGTGCCAGCTTGTATTTTGGCTTCCCCTGGTTCGCTTAT TGGGGCCAAGGTACACCCGTCACTGTCTCTTCTGGCGGAGGGGGTTCTGGAGGC GGAGGCAGCGGGGGCGGAGGGTCCGACATCCAACTTACCCAGTCACCCTCATCT CTTAGTGCCTCTGTAGGGGACCGAGTTACTATTACATGTAAAGCCAGTCAAGAT GTTGGCACCTCAGTAGCATGGTATCAACAAAAGCCTGGTAAAGCCCCAAAACTG CTGATCTATTGGACAAGCACACGACATACAGGAGTGCCAAGTCGCTTCAGCGGT TCAGGTTCAGGCACAGATTTTACATTCACTATATCAAGCCTGCAACCCGAGGAC ATTGCCACATATTACTGCCAGCAATATAGTCTGTATCGTAGCTTCGGATGTGGC | 907 |

| | | ACCAAGGTTGAAATTAAGGGAGGCGGCGGCTCTGACAAAACTCACACGTGCCCA CCGTGCCCAGCACCTGAACTCCTGGGGGGACCGTCAGTCTTCCTCTTCCCCCCA AAACCCAAGGACACCCTCATGATCTCCCGGACCCCTGAGGTCACATGCGTGGTG GTGGACGTGAGCCACGAAGACCCTGAGGTCAAGTTCAACTGGTACGTGGACGGC GTGGAGGTGCATAATGCCAAGACAAAGCCGCGGGAGGAGCAGTACAACAGCACG TACCGTGTGGTCAGCGTCCTCACCGTCCTGCACCAGGACTGGCTGAATGGCAAG GAGTACAAGTGCAAGGTCTCCAACAAAGCCCTCCCAGCCCCCATCGAGAAAACC ATCTCCAAAGCCAAAGGGCAGCCCCGAGAACCACAGGTGTGCACCCTGCCCCCA TCCCGGGAGGAGATGACCAAGAACCAGGTCAGCCTGAGCTGCGCGGTCAAAGGC TTCTATCCCAGCGACATCGCCGTGGAGTGGGAGAGCAATGGGCAGCCGGAGAAC AACTACAAGACCACGCCTCCCGTGCTGGACTCCGACGGCTCCTTCTTCCTCGTG AGCAAGCTCACCGTGGACAAGAGCAGGTGGCAGCAGGGGAACGTCTTCTCATGC TCCGTGATGCATGAGGCTCTGCACAACCACTACACGCAGAAGAGCCTCTCCCTG TCTCCGGGTAAA | |
| Polypeptide 4 | GAAGTGCAACTCGTAGAATCCGGCGGGGGCGTCGTACAGCCCGGCCGTTCTTTG AGACTTAGCTGTAGTGCTTCTGGGTTTGACTTCACTACATACTGGATGTCATGG GTAAGACAGGCACCTGGCAAGTGCCTTGAATGGATCGGTGAAATCCATCCCGAC AGCTCCACAATCAACTACGCCCCAAGTTTGAAAGACCGGTTCACCATATCTCGT GACAACGCCAAGAATACATTGTTCCTTCAGATGGATAGTCTTCGTCCAGAGGAT ACTGGGGTATATTTTTGTGCCAGCTTGTATTTTGGCTTCCCCTGGTTCGCTTAT TGGGGCCAAGGTACACCCGTCACTGTCTCTTCTGGCGGAGGGGGTTCTGGAGGC GGAGGCAGCGGGGGCGGAGGGTCCGACATCCAACTTACCCAGTCACCCTCATCT CTTAGTGCCTCTGTAGGGGACCGAGTTACTATTACATGTAAAGCCAGTCAAGAT GTTGGCACCTCAGTAGCATGGTATCAACAAAAGCCTGGTAAAGCCCCAAAACTG CTGATCTATTGGACAAGCACACGACATACAGGAGTGCCAAGTCGCTTCAGCGGT TCAGGTTCAGGCACAGATTTTACATTCACTATATCAAGCCTGCAACCCGAGGAC ATTGCCACATATTACTGCCAGCAATATAGTCTGTATCGTAGCTTCGGATGTGGC ACCAAGGTTGAAATTAAGGGAGGCGGCGGCTCTGACAAAACTCACACGTGCCCA CCGTGCCCAGCACCTGAACTCCTGGGGGGACCGTCAGTCTTCCTCTTCCCCCCA | 907 |

|  |  | AAACCCAAGGACACCCTCATGATCTCCCGGACCCCTGAGGTCACATGCGTGGTG<br><br>GTGGACGTGAGCCACGAAGACCCTGAGGTCAAGTTCAACTGGTACGTGGACGGC<br><br>GTGGAGGTGCATAATGCCAAGACAAAGCCGCGGGAGGAGCAGTACAACAGCACG<br><br>TACCGTGTGGTCAGCGTCCTCACCGTCCTGCACCAGGACTGGCTGAATGGCAAG<br><br>GAGTACAAGTGCAAGGTCTCCAACAAAGCCCTCCCAGCCCCCATCGAGAAAACC<br><br>ATCTCCAAAGCCAAAGGGCAGCCCCGAGAACCACAGGTGTGCACCCTGCCCCCA<br><br>TCCCGGGAGGAGATGACCAAGAACCAGGTCAGCCTGAGCTGCGCGGTCAAAGGC<br><br>TTCTATCCCAGCGACATCGCCGTGGAGTGGGAGAGCAATGGGCAGCCGGAGAAC<br><br>AACTACAAGACCACGCCTCCCGTGCTGGACTCCGACGGCTCCTTCTTCCTCGTG<br><br>AGCAAGCTCACCGTGGACAAGAGCAGGTGGCAGCAGGGGAACGTCTTCTCATGC<br><br>TCCGTGATGCATGAGGCTCTGCACAACCACTACACGCAGAAGAGCCTCTCCCTG<br><br>TCTCCGGGTAAA |  |

## Example 30. Design, preparation and analysis of novel peptide vaccine fusion antibody format with two Fc domains

The engineered antibody format consisting of two Fc domains and one Fab region of Example 24 above can fused with a peptide vaccine at the N-terminus of its hinge, thereby providing an anticancer vaccine antibody format, which can possess a new therapeutic function (Figure 58).

Polypeptide 1 of the antibody consists of a heavy chain variable region (X), a heavy chain CH1 region (A), a linker (L1), and a hinge-CH2 region-CH3 with Knob mutation (C); Polypeptide 2 consists of a light chain variable region (Y), a light chain CL region (B), a linker (L2), and a hinge-CH2 region-CH3 with Knob mutation (D); Polypeptide 3 consists of a peptide vaccine (Z), a linker (L3), and a hinge-CH2 region-CH3 with Hole mutation (E); and Polypeptide 4 consists of a peptide vaccine (Z), a linker (L4), and a hinge-CH2 region-CH3 with Hole mutation (F) (Figure 58). The four polypeptides are assembled into an engineered antigen binding protein having two Fc domains and one Fab region in which two or four peptide vaccines (Z) are fused (Figure 58).

EAVC-P1, EAVC-P2, EAVC-P3, EAVC-P4, EAVC-P5, EAVC-P6, EAVC-P7, EAVC-P8, EAVC-C3, EAVC-I3, EAVC-L3, EAVC-M3, and EAVC-N3, which specifically bind to EGFR and comprise the KRAS protein as a Z protein, were each prepared by co-transfection of polynucleotides encoding four polypeptides into the EXPICHO-S™ cell line (Tables 99 to 101). The proteins prepared as above were purified, quantified, and then identified by SDS-PAGE in the same manner as in Example 28 above.

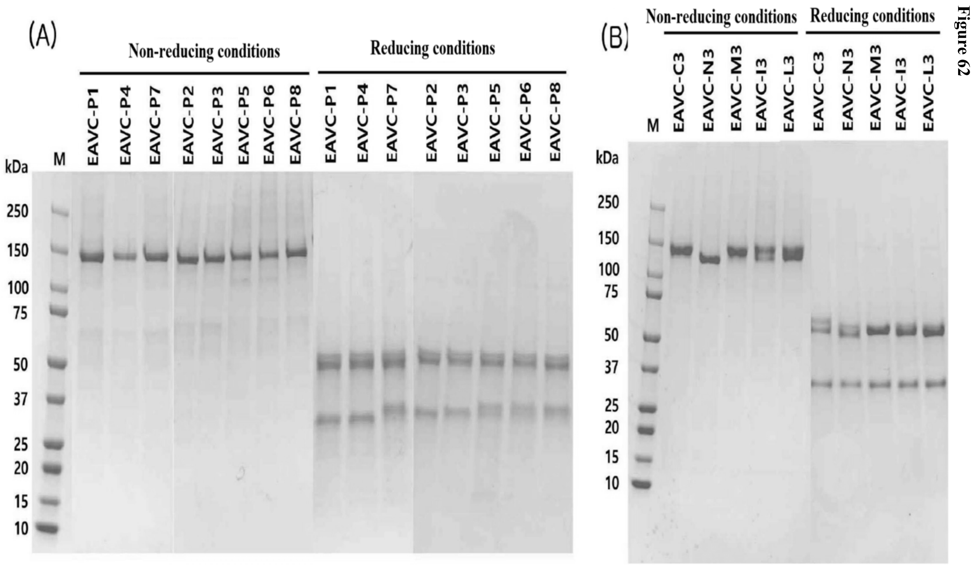

As shown in Figure 62, four polypeptides of appropriate sizes were identified under reducing conditions in all EAVC-P1, EAVC-P2, EAVC-P3, EAVC-P4, EAVC-P5, EAVC-P6, EAVC-P7, EAVC-P8, EAVC-C3, EAVC-I3, EAVC-L3, EAVC-M3, and EAVC-N3. The analysis showed that under non-reducing conditions, EAVC-P1, EAVC-P2, EAVC-P3, EAVC-P4, EAVC-P5, EAVC-P6, EAVC-P7, EAVC-P8, EAVC-C3, EAVC-I3, EAVC-L3, EAVC-M3, and EAVC-N3 were all assembled well into the intact form.

In addition, HAVCO1, HAVC02, HAVC03, HAVC04, HAVC05, HAVC06, HAVC07, and HAVC08, which specifically bind to HER2 and comprise the KRAS protein as a Z protein, were prepared and analyzed in the same manner as above (Tables 99 to 101).

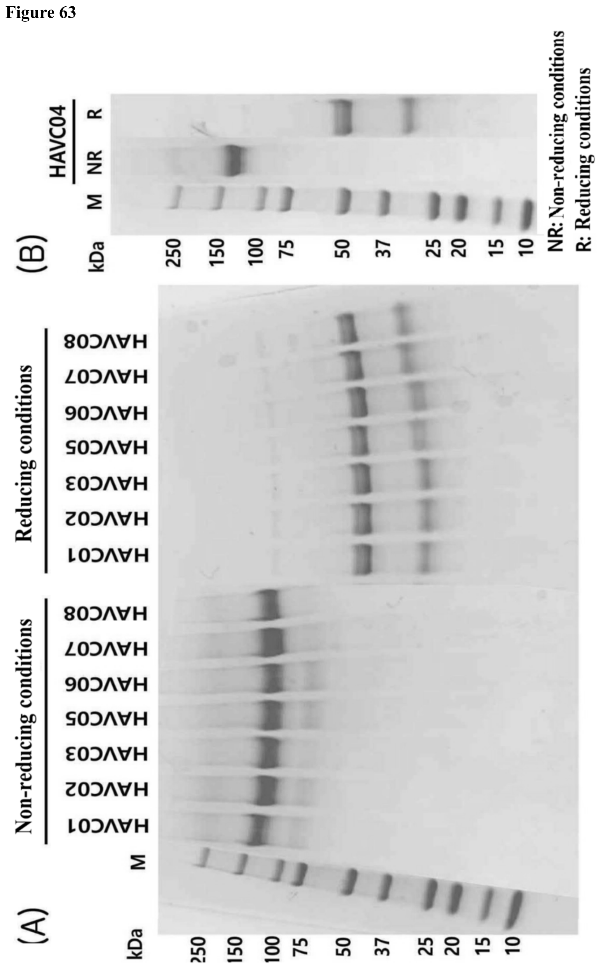

As shown in Figure 63, four polypeptides of appropriate sizes were identified under reducing conditions in all HAVC01, HAVC02, HAVC03, HAVC04, HAVC05, HAVC06, HAVC07, and HAVC08. The analysis showed that under non-reducing conditions, HAVC01, HAVC02, HAVC03, HAVC04, HAVC05, HAVC06, HAVC07, and HAVC08 were all assembled well into the intact form (Figure 63).

In addition, size exclusion chromatography (SEC) analysis was performed on HAVC01, HAVC02, HAVC03, HAVC04, HAVC05, HAVC06, HAVC07, and HAVC08. An Arc™ Premier UHPLC system (Waters, 176019001, 176017033) equipped with an XBridge ProBEH200 SEC Guard Column (Waters, 176004334) and an XBridge ProBEH200 SEC Column (Waters, 176004335) was used, and as a mobile phase, 0.15 M NaCl (in PBS pH 6.8) was used at a flow rate of 0.38 mL/min. A photodiode array (PDA) detector (Waters, 176019010) was used as a detector for analysis.

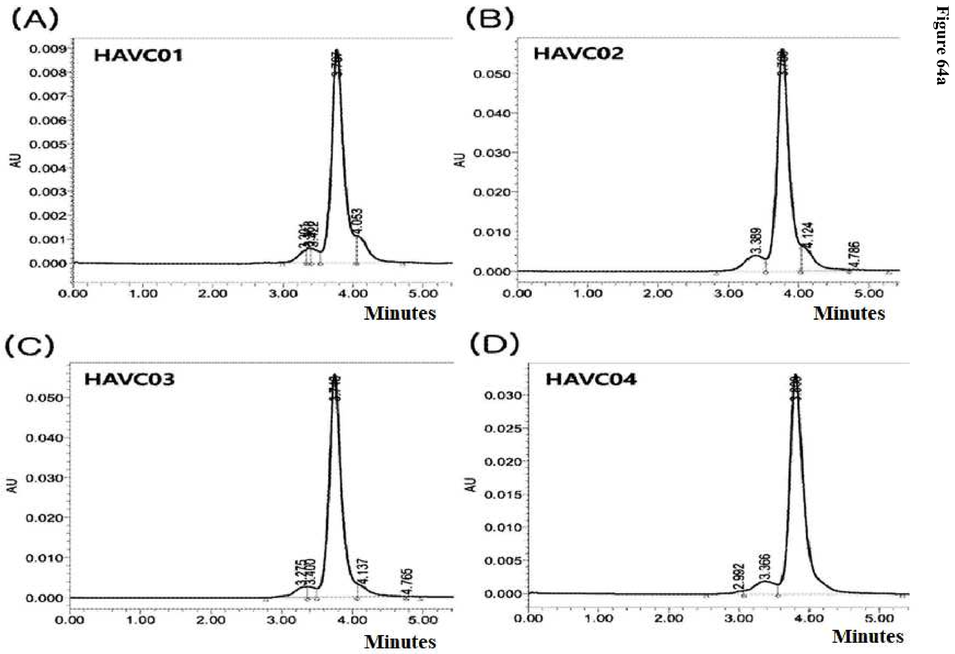

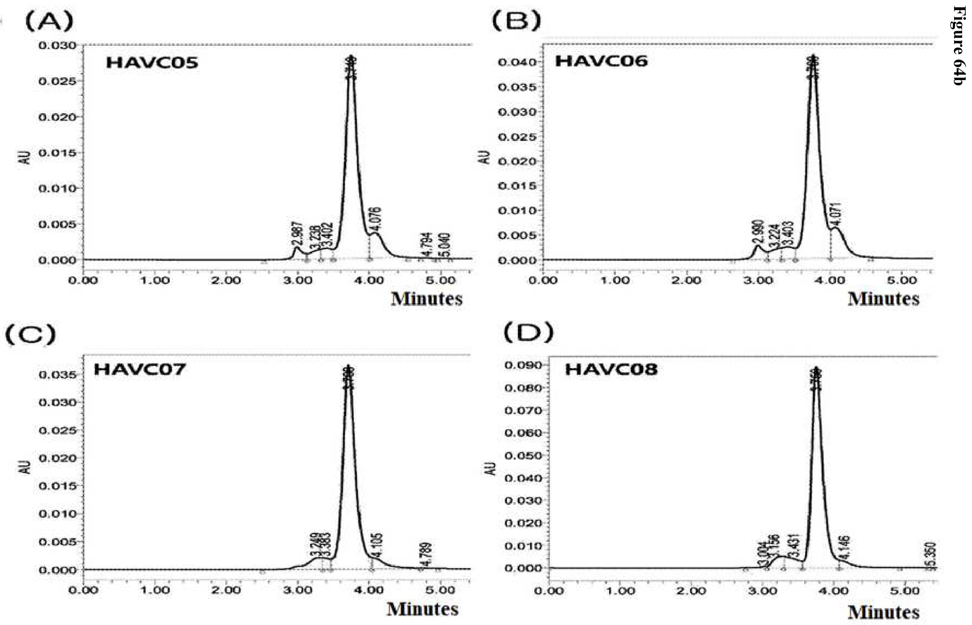

As shown in Figure 64a, Figure 64b, and Table 102, the main product was identified at a retention time of about 3.7 minutes to about 3.8 minutes.

Table 99 below shows the compositions of the novel engineered antibody format conjugated with the peptide vaccine.

[Table 99]

| Name | Region | Composition |
|---|---|---|
| EAVC-P1 | Fab region | Anti-EGFR Fab (Fab of Panitumumab) |
| | Z protein | KRAS$^{WT}$ Peptide-Linker(5-mer) |
| EAVC-P2 | Fab region | Anti-EGFR Fab (Fab of Panitumumab) |
| | Z protein | KRAS$^{WT}$ Peptide-Linker(2-mer) |
| EAVC-P3 | Fab region | Anti-EGFR Fab (Fab of Panitumumab) |
| | Z protein | KRAS$^{G12D}$ Peptide-Linker(5-mer) |
| EAVC-P4 | Fab region | Anti-EGFR Fab (Fab of Panitumumab) |
| | Z protein | KRAS$^{G12D}$ Peptide-Linker(2-mer) |
| EAVC-P5 | Fab region | Anti-EGFR Fab (Fab of Panitumumab) |
| | Z protein | KRAS$^{WT}$ Peptide-Linker(5-mer)-KRAS$^{WT}$ Peptide-Linker(5-mer) |
| EAVC-P6 | Fab region | Anti-EGFR Fab (Fab of Panitumumab) |
| | Z protein | KRAS$^{WT}$ Peptide-Linker(2-mer)-KRAS$^{WT}$ Peptide-Linker(2-mer) |
| EAVC-P7 | Fab region | Anti-EGFR Fab (Fab of Panitumumab) |
| | Z protein | KRAS$^{G12D}$ Peptide-Linker(5-mer)-KRAS$^{G12D}$ Peptide-Linker(5-mer) |
| EAVC-P8 | Fab region | Anti-EGFR Fab (Fab of Panitumumab) |
| | Z protein | KRAS$^{G12D}$ Peptide-Linker(2-mer)-KRAS$^{G12D}$ Peptide-Linker(2-mer) |

| Name | Region | Composition |
|---|---|---|
| EAVC-C3 | Fab region | Anti-EGFR Fab (Fab of Cetuximab) |
| | Z protein | KRAS$^{G12D}$ Peptide-Linker(5-mer) |
| EAVC-I3 | Fab region | Anti-EGFR Fab (Fab of Imgatuzumab) |
| | Z protein | KRAS$^{G12D}$ Peptide-Linker(5-mer) |
| EAVC-L3 | Fab region | Anti-EGFR Fab (Fab of Losatuxizumab) |
| | Z protein | KRAS$^{G12D}$ Peptide-Linker(5-mer) |
| EAVC-M3 | Fab region | Anti-EGFR Fab (Fab of Matuzumab) |
| | Z protein | KRAS$^{G12D}$ Peptide-Linker(5-mer) |
| EAVC-N3 | Fab region | Anti-EGFR Fab (Fab of Necitumumab) |
| | Z protein | KRAS$^{G12D}$ Peptide-Linker(5-mer) |
| HAVC01 | Fab region | Anti-HER2 Fab (Fab of Trastuzumab) |
| | Z protein | KRAS$^{WT}$ Peptide-Linker(5-mer) |
| HAVC02 | Fab region | Anti-HER2 Fab (Fab of Trastuzumab) |
| | Z protein | KRAS$^{WT}$ Peptide-Linker(2-mer) |
| HAVC03 | Fab region | Anti-HER2 Fab (Fab of Trastuzumab) |
| | Z protein | KRAS$^{G12D}$ Peptide-Linker(5-mer) |
| HAVC04 | Fab region | Anti-HER2 Fab (Fab of Trastuzumab) |
| | Z protein | KRAS$^{G12D}$ Peptide-Linker(2-mer) |
| HAVC05 | Fab region | Anti-HER2 Fab (Fab of Trastuzumab) |
| | Z protein | KRAS$^{WT}$ Peptide-Linker(5-mer)-KRAS$^{WT}$ Peptide-Linker(5-mer) |
| HAVC06 | Fab region | Anti-HER2 Fab (Fab of Trastuzumab) |
| | Z protein | KRAS$^{WT}$ Peptide-Linker(2-mer)-KRAS$^{WT}$ Peptide-Linker(2-mer) |
| HAVC07 | Fab region | Anti-HER2 Fab (Fab of Trastuzumab) |
| | Z protein | KRAS$^{G12D}$ Peptide-Linker(5-mer)-KRAS$^{G12D}$ Peptide-Linker(5-mer) |
| HAVC08 | Fab region | Anti-HER2 Fab (Fab of Trastuzumab) |
| | Z protein | KRAS$^{G12D}$ Peptide-Linker(2-mer)-KRAS$^{G12D}$ Peptide-Linker(2-mer) |

Table 100 below shows the polypeptide sequence of each domain of the novel engineered antibody format conjugated with the peptide vaccine.

[Table 100]

| Name | | Sequence | SEQ ID NO |
|---|---|---|---|
| EAVC-P1 | Fab region (VH-CH1) | QVQLQESGPGLVKPSETLSLTCTVSGGSVSSGDYYWTWIRQSPGKGLEW1 GHIYYSGNTNYNPSLKSRLTISIDTSKTQFSLKLSSVTAADTAIYYCVRD RVTGAFDIWGQGTWTVSSASTKGPSVFPLAPSSKSTSGGTAALGCLVKD YFPEPVTVSWNSGALTSGVHTFPAVLQSSGLYSLSSVVTVPSSSLGTQTY ICNVNHKPSNTKVDKKV | 872 |
| | Fab region (VL-CL) | DIQMTQSPSSLSASVGDRVTITCQASQDISNYLNWYQQKPGKAPKLLIYD ASNLETGVPSRFSGSGSGTDFTFTISSLQPEDIATYFCQHFDHLPLAFGC GTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGEC | 873 |
| | L1 linker | EPKSC | 836 |
| | L2 linker | GGGGSGGGGSGGGGSEPKSS | 837 |
| | L3 linker | GGGGS | 838 |
| | L4 linker | GGGGS | 838 |
| | Hinge-Fc (Knob) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVYTLPPCREEMTKNQVSLWCLVK GFYPSDIAVEWESNGQPENNYKTTPPVLDSDGSFFLYSKLTVDKSRWQQG NVFSCSVMHEALHNHYTQKSLSLSPGK | 839 |
| | Hinge-Fc (Hole) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVCTLPPSREEMTKNQVSLSCAVK GFYPSDIAVEWESNGQPENNYKTTPPVLDSDGSFFLVSKLTVDKSRWQQG NVFSCSVMHEALHNHYTQKSLSLSPGK | 7 |
| | Z protein(KRAS⁺ Peptide) | KLVVVGAGGVGKSALTI | 908 |

| | | | |
|---|---|---|---|
| | Fab region (VH-CH1) | QVQLQESGPGLVKPSETLSLTCTVSGGSVSSGDYYWTWIRQSPGKCLEWI GHIYYSGNTNYNPSLKSRLTISIDTSKTQFSLKLSSVTAADTAIYYCVRD RVTGAFDIWGQGTMVTVSSASTKGPSVFPLAPSSKSTSGGTAALGCLVKD YFPEPVTVSWNSGALTSGVHTFPAVLQSSGLYSLSSVVTVPSSSLGTQTY ICNVNHKPSNTKVDKKV | 872 |
| | Fab region (VL-CL) | DIQMTQSPSSLSASVGDRVTITCQASQDISNYLNWYQQKPGKAPKLLIYD ASNLETGVPSRFSGSGSGTDFTFTISSLQPEDIATYFCQHFDHLPLAFGC GTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGEC | 873 |
| | L1 linker | EPKSC | 836 |
| | L2 linker | GGGGSGGGGSGGGGSEPKSS | 837 |
| | L3 linker | GG | 909 |
| EAVC-P2 | L4 linker | GG | 909 |
| | Hinge-Fc (Knob) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVYTLPPCREEMTKNQVSLWCLVK GFYPSDIAVEWESNGQPENNYKTTPPVLDSDGSFFLYSKLTVDKSRWQQG NVFSCSVMHEALHNHYTQKSLSLSPGK | 839 |
| | Hinge-Fc (Hole) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVCTLPPSREEMTKNQVSLSCAVK GFYPSDIAVEWESNGQPENNYKTTPPVLDSDGSFFLVSKLTVDKSRWQQG NVFSCSVMHEALHNHYTQKSLSLSPGK | 7 |
| | Z protein(KRAS<sup>G</sup> Peptide) | KLVVVGAGGVGKSALTI | 908 |
| EAVC-P3 | Fab region (VH-CH1) | QVQLQESGPGLVKPSETLSLTCTVSGGSVSSGDYYWTWIRQSPGKCLEWI GHIYYSGNTNYNPSLKSRLTISIDTSKTQFSLKLSSVTAADTAIYYCVRD | 872 |

| | | | |
|---|---|---|---|
| | | RVTGAFDIWGQGTMVTVSSASTKGPSVFPLAPSSKSTSGGTAALGCLVKD YFPEPVTVSWNSGALTSGVHTFPAVLQSSGLYSLSSVVTVPSSSLGTQTY ICNVNHKPSNTKVDKKV | |
| | Fab region (VL-CL) | DIQMTQSPSSLSASVGDRVTITCQASQDISNYLNWYQQKPGKAPKLLIYD ASNLETGVPSRFSGSGSGTDFTFTISSLQPEDIATYFCQHFDNLPLAFGC GTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGEC | 873 |
| | L1 linker | EPKSC | 836 |
| | L2 linker | GGGGSGGGGSGGGGSEPKSS | 837 |
| | L3 linker | GGGGS | 838 |
| | L4 linker | GGGGS | 838 |
| | Hinge-Fc (Knob) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVYTLPPCREEMTKNQVSLWCLVK GFYPSDIAVEWESNGQPENNYKTTPPVLDSDGSFFLYSKLTVDKSRWQQG NVFSCSVMHEALHNHYTQKSLSLSPGK | 839 |
| | Hinge-Fc (Hole) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVCTLPPSREEMTKNQVSLSCAVK GFYPSDIAVEWESNGQPENNYKTTPPVLDSDGSFFLVSKLTVDKSRWQQG NVFSCSVMHEALHNHYTQKSLSLSPGK | 7 |
| | Z protein(KRAS^C ^12D Peptide) | KLVVVGADGVGKSALTI | 910 |
| EAVC-P4 | Fab region (VH-CH1) | QVQLQESGPGLVKPSETLSLTCTVSGGSVSSGDYYWTWIRQSPGKGLEWI GHIYYSGNTNYNPSLKSRLTISIDTSKTQFSLKLSSVTAADTAIYYCVRD RVTGAFDIWGQGTMVTVSSASTKGPSVFPLAPSSKSTSGGTAALGCLVKD YFPEPVTVSWNSGALTSGVHTFPAVLQSSGLYSLSSVVTVPSSSLGTQTY | 872 |

| | | ICNVNHKPSNTKVDKKV | |
|---|---|---|---|
| | Fab region (VL-CL) | DIQMTQSPSSLSASVGDRVTITCQASQDISNYLNWYQQKPGKAPKLLIYD ASNLETGVPSRFSGSGSGTDFTFTISSLQPEDIATYFCQHFDHLPLAFGC GTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGEC | 873 |
| | L1 linker | EPKSC | 836 |
| | L2 linker | GGGGSGGGGSGGGGSEPKSS | 837 |
| | L3 linker | GG | 909 |
| | L4 linker | GG | 909 |
| | Hinge-Fc (Knob) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVYTLPPCREEMTKNQVSLWCLVK GFYPSDIAVEWESNGQPENNYKTTPPVLDSDGSFFLYSKLTVDKSRWQQG NVFSCSVMHEALHNHYTQKSLSLSPGK | 839 |
| | Hinge-Fc (Hole) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVCTLPPSRFEMTKNQVSLSCAVK GFYPSDIAVEWESNGQPENNYKTTPPVLDSDGSFFLVSKLTVDKSRWQQG NVFSCSVMHEALHNHYTQKSLSLSPGK | 7 |
| | Z protein(KRAS[G12D] Peptide) | KLVVVGADGVGKSALTI | 910 |
| EAVC-P5 | Fab 영역 (VH-CH1) | QVQLQESGPGLVKPSETLSLTCTVSGGSVSSGDYYWTWIRQSPGKCLEWI GHIYYSGNTNYNPSLKSRLTISIDTSKTQFSLKLSSVTAADTAIYYCVRD RVTGAFDIWGQGTMVTVSSASTKGPSVFPLAPSSKSTSGGTAALGCLVKD YFPEPVTVSWNSGALTSGVHTFPAVLQSSGLYSLSSVVTVPSSSLGTQTY ICNVNHKPSNTKVDKKV | 872 |
| | Fab region | DIQMTQSPSSLSASVGDRVTITCQASQDISNYLNWYQQKPGKAPKLLIYD | 873 |

| | (VL-CL) | ASXLETGVPSRFSGSGSGTDFTFTISSLQPEDIATYFCQHFDHLPLAFGC GTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGEC | |
| | L1 linker | EPKSC | 836 |
| | L2 linker | GGGGSGGGGSGGGGSEPKSS | 837 |
| | L3 linker | GGGGS | 838 |
| | L4 linker | GGGGS | 838 |
| | Hinge-Fc (Knob) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVYTLPPCREEMTKNQVSLWCLVK GFYPSDIAVEWESNGQPENNYKTTPPVLDSDGSFFLYSKLTVDKSRWQQG NVFSCSVMHEALHNHYTQKSLSLSPGK | 839 |
| | Hinge-Fc (Hole) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVCTLPPSREEMTKNQVSLSCAVK GFYPSDIAVEWESNGQPENNYKTTPPVLDSDGSFFLVSKLTVDKSRWQQG NVFSCSVMHEALHNHYTQKSLSLSPGK | 7 |
| | Z protein(KRAS[WT] Peptide-Linker(5-mer)-KRAS[WT] Peptide) | KLVVVGAGGVGKSALTIGGGGSKLVVVGAGGVGKSALTI | 911 |
| EAVC-P6 | Fab region (VH-CH1) | QVQLQESGPGLVKPSETLSLTCTVSGGSVSSGDYYWTWIRQSPGKCLEWI GHIYYSGNTNYNPSLKSRLTISIDTSKTQFSLKLSSVTAADTAIYYCVRD RVTGAFDIWGQGTMVTVSSASTKGPSVFPLAPSSKSTSGGTAALGCLVKD YFPEPVTVSWNSGALTSGVHTFPAVLQSSGLYSLSSVVTVPSSSLGTQTY ICNVNHKPSNTKVDKKV | 872 |

| | Fab region (VL-CL) | DIQMTQSPSSLSASVGDRVTITCQASQDISNYLNWYQQKPGKAPKLLIYD ASNLETGVPSRFSGSGSGTDFTFTISSLQPEDIATYFCQHFDHLPLAFGC GTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGEC | 873 |
|---|---|---|---|
| | L1 linker | EPKSC | 836 |
| | L2 linker | GGGGSGGGGSGGGGSEPKSS | 837 |
| | L3 linker | GG | 909 |
| | L4 linker | GG | 909 |
| | Hinge-Fc (Knob) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVYTLPPCREEMTKNQVSLWCLVK GFYPSDIAVEWESNGQPENNYKTTPPVLDSDGSFFLYSKLTVDKSRWQQG NVFSCSVMHEALHNHYTQKSLSLSPGK | 839 |
| | Hinge-Fc (Hole) | DKTHTCPPCPAPELLGGPSVFLFPPKPKDTLMISRTPEVTCVVVDVSHED PEVKFNWYVDGVEVIINAKTKPREEQYNSTYRVVSVLTVLHQDWLNGKEYK CKVSNKALPAPIEKTISKAKGQPREPQVCTLPPSREEMTKNQVSLSCAVK GFYPSDIAVEWESNGQPENNYKTTPPVLDSDGSFFLVSKLTVDKSRWQQG NVFSCSVMHEALHNHYTQKSLSLSPGK | 7 |
| | Z protein(KRAS^T^T Peptide-Linker(2-mer)-KRAS^rt Peptide) | KLVVVGAGGVGKSALTIGGKLVVVGAGGVGKSALTI | 912 |
| EAVC-P7 | Fab region (VH-CH1) | QVQLQESGPGLVKPSETLSLTCTVSGGSVSSGDYYWTWIRQSPGKCLEWI GHIYYSGNTNYNPSLKSRLTISIDTSKTQFSLKLSSVTAADTAIYYCVRD RVTGAFDIWGQGTMVTVSSASTKGPSVFPLAPSSKSTSGGTAALGCLVKD YFPEPVTVSWNSGALTSGVHTFPAVLQSSGLYSLSSVVTVPSSSLGTQTY | 872 |

| | | ICYVNHKPSNTKVDKKV | |
|---|---|---|---|
| | Fab region (VL-CL) | DIQMTQSPSSLSASVGDRVTITCQASQDISNYLNWYQQKPGKAPKLLIYD ASNLETGVPSRFSGSGSGTDFTFTISSLQPEDIATYFCQHFDHLPLAFGC GTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGEC        region | 873 |
| | L1 linker | EPKSC | 836 |
| | L2 linker | GGGGSGGGGSGGGGSEPKSS | 837 |
| | L3 linker | GGGGS | 838 |
| | L4 linker | GGGGS | 838 |
| | Hinge-Fc (Knob) | DKTHTCPPCPAPELLGGPSVFLFPPKPKDTLMISRTPEVTCVVVDVSHED PEVKFNWYVDGVEVHNAKTKPREEQYKSTYRVVSVLTVLHQDWLNGKEYK CKVSNKALPAPIEKTISKAKGQPREPQVYTLPPCREEMTKNQVSLWCLVK GFYPSDIAVEWESNGQPENNYKTTPPVLDSDGSFFLYSKLTVDKSRWQQG NVFSCSVMHEALHNHYTQKSLSLSPGK | 839 |
| | Hinge-Fc (Hole) | DKTHTCPPCPAPELLGGPSVFLFPPKPKDTLMISRTPEVTCVVVDVSHED PEVKFNWYVDGVEVHNAKTKPREEQYKSTYRVVSVLTVLHQDWLNGKEYK CKVSNKALPAPIEKTISKAKGQPREPQVCTLPPSREEMTKNQVSLSCAVK GFYPSDIAVEWESNGQPENNYKTTPPVLDSDGSFFLVSKLTVDKSRWQQG NVFSCSVMHEALHNHYTQKSLSLSPGK | 7 |
| | Z protein(KRAS$^{G12D}$ Peptide-Linker(5-mer)-KRAS$^{G12D}$ Peptide) | KLVVVGADGVGKSALTIGGGGSKLVVVGADGVGKSALTI | 913 |
| EAVC-P8 | Fab region (VH-CH1) | QVQLQESGPGLVKPSETLSLTCTVSGGSVSSGDYYWTWIRQSPGKCLEWI GHIYYSGNTNYNPSLKSRLTISIDTSKTQFSLKLSSVTAADTAIYYCVRD RVTGAFDIWGQGTMVTVSSASTKGPSVFPLAPSSKSTSGGTAALGCLVKD | 872 |

| | | | |
|---|---|---|---|
| | | YFPEPVTVSWNSGALTSGVHTFPAVLQSSGLYSLSSVVTVPSSSLGTQTY ICNVNHKPSNTKVDKKV | |
| | Fab region (VL-CL) | DIQMTQSPSSLSASVGDRVTITCQASQDISNYLNWYQQKPGKAPKLLIYD ASNLETGVPSRFSGSGSGTDFTFTISSLQPEDIATYFCQHFDHLPLAFGC GTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGEC | 873 |
| | L1 linker | EPKSC | 836 |
| | L2 linker | GGGGSGGGGSGGGGSEPKSS | 837 |
| | L3 linker | GG | 909 |
| | L4 linker | GG | 909 |
| | Hinge-Fc (Knob) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVYTLPPCREEMTKNQVSLWCLVK GFYPSDIAVEWESNGQPENNYKTTPPVLDSDGSFFLYSKLTVDKSRWQQG NVFSCSVMHEALHNHYTQKSLSLSPGK | 839 |
| | Hinge-Fc (Hole) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVCTLPPSREEMTKNQVSLSCAVK GFYPSDIAVEWESNGQPENNYKTTPPVLDSDGSFFLVSKLTVDKSRWQQG NVFSCSVMHEALHNHYTQKSLSLSPGK | 7 |
| | Z protein(KRAS$^G$ $^{12D}$ Peptide-Linker(2-mer)-KRAS$^{G12D}$ Peptide) | KLVVVGADGVGKSALTIGGKLVVVGADGVGKSALTI | 914 |
| EAVC-C3 | Fab region (VH-CH1) | QVQLKQSGPGLVQPSQSLSITCTVSGFSLTNYGVHWVRQSPGKCLEWLGV IWSGGNTDYNTPFTSRLSINKDNSKSQVFFKMNSLQSNDTAIYYCARALT | 915 |

| | | | |
|---|---|---|---|
| | | YYDYEFAYWGQGTLVTVSAASTKGPSVFPLAPSSKSTSGGTAALGCLVKD YFPEPVTVSWNSGALTSGVHTFPAVLQSSGLYSLSSVVTVPSSSLGTQTY ICNVNHKPSNTKVDKKV | |
| | Fab region (VL-CL) | DILLTQSPVILSVSPGERVSFSCRASQSIGTNIHWYQQRTNGSPRLLIKY ASESISGIPSRFSGSGSGTDFTLSINSVESEDIADYYCQQNNNWPTTFGC GTKLELKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGEC | 916 |
| L1 linker | EPKSC | 836 |
| L2 linker | GGGGSGGGGSGGGGSEPKSS | 837 |
| L3 linker | GGGGS | 838 |
| L4 linker | GGGGS | 838 |
| Hinge-Fc (Knob) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVYTLPPCREEMTKNQVSLWCLVK GFYPSDIAVEWESNGQPENNYKTTPPVLDSDGSFFLYSKLTVDKSRWQQG NVFSCSVMHEALHNHYTQKSLSLSPGK | 839 |
| Hinge-Fc (Hole) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVCTLPPSREEMTKNQVSLSCAVK GFYPSDIAVEWESNGQPENNYKTTPPVLDSDGSFFLVSKLTVDKSRWQQG NVFSCSVMHEALHNHYTQKSLSLSPGK | 7 |
| Z protein(KRASᴳ ¹²ᴰ Peptide) | KLVVVGADGVGKSALTI | 910 |
| EAVC-13 | Fab region (VH-CH1) | QVQLVQSGAEVKKPGSSVKVSCKASGFTFTDYKIHWVRQAHGQCLEWMGY FNPNSGYSTYAQKFQGRVTITADKSTSTAYMELSSLRSEDTAVYYCARLS PGGYYVMDAWGQGTTVTVSSASTKGPSVFPLAPSSKSTSGGTAALGCLVK DYFPEPVTVSWNSGALTSGVHTFPAVLQSSGLYSLSSVVTVPSSSLGTQT | 917 |

| | | | |
|---|---|---|---|
| | | YICNVKHKPSNTKVDKKV | |
| | Fab region (VL-CL) | DIQMTQSPSSLSASVGDRVTITCRASQGIRNYLNWYQQKPGKAPKRLIYN TNNLQTGVPSRFSGSGSGTEFTLTISSLQPEDFATYYCLQHNSFPTFGCG TKLEIKRTVAAPSVFIFPPSDEQLKSGTASVVCLLNNFYPREAKVQWKVD NALQSGNSQESVTEQDSKDSTYSLSSTLTLSKADYEKHKVYACEVTHQGL SSPVTKSFNRGEC | 918 |
| | L1 linker | EPKSC | 836 |
| | L2 linker | GGGGSGGGGSGGGGSEPKSS | 837 |
| | L3 linker | GGGGS | 838 |
| | L4 linker | GGGGS | 838 |
| | Hinge-Fc (Knob) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVYTLPPCREEMTKNQVSLWCLVK GFYPSDIAVEWESNGQPENNYKTTPPVLDSDGSFFLYSKLTVDKSRWQQG NVFSCSVMHEALHNHYTQKSLSLSPGK | 839 |
| | Hinge-Fc (Hole) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVCTLPPSREEMTKNQVSLSCAVK GFYPSDIAVEWESNGQPENNYKTTPPVLDSDGSFFLVSKLTVDKSRWQQG NVFSCSVMHEALHNHYTQKSLSLSPGK | 7 |
| | Z protein(KRAS<sup>G12D</sup> Peptide) | KLVVVGADGVGKSALTI | 910 |
| EAVC-L3 | Fab region (VH-CH1) | EVQLQESGPGLVKPSQTLSLTCTVSGYSISRDFAWNWIRQPPGKCLEWMG YISYNGNTRYQPSLKSRITISRDTSKNQFFLKLNSVTAADTATYYCVTAS RGFPYWGQGTLVTVSSASTKGPSVFPLAPSSKSTSGGTAALGCLVKDYFP EPVTVSWNSGALTSGVHTFPAVLQSSGLYSLSSVVTVPSSSLGTQTYICN VNHKPSNTKVDKKV | 919 |
| | Fab region | DIQMTQSPSSMSVSVGDRVTITCHSSQDINSNIGWLQQKPGKSFKGLIYH | 920 |

| | (VL-CL) | GTNLDXGVPSRFSGSGSGTDYTLTISSLQPEDFATYYCVQYAQFPWTFGC GTKLEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGEC | |
|---|---|---|---|
| | L1 linker | EPKSC | 836 |
| | L2 linker | GGGGSGGGGSGGGGSEPKSS | 837 |
| | L3 linker | GGGGS | 838 |
| | L4 linker | GGGGS | 838 |
| | Hinge-Fc (Knob) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVYTLPPCREEMTKNQVSLWCLVK GFYPSDIAVEWESNGQPENNYKTTPPVLDSDGSFFLYSKLTVDKSRWQQG NVFSCSVMHEALHNHYTQKSLSLSPGK | 839 |
| | Hinge-Fc (Hole) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVCTLPPSREEMTKNQVSLSCAVK GFYPSDIAVEWESNGQPENNYKTTPPVLDSDGSFFLVSKLTVDKSRWQQG NVFSCSVMHEALHNHYTQKSLSLSPGK | 7 |
| | Z protein(KRAS[6] [120] Peptide) | KLVVVGADGVGKSALTI | 910 |
| EAVC-M3 | Fab region (VH-CH1) | QVQLVQSGAEVKKPGASVKVSCKASGYTFTSHWMHWVRQAPGQCLEWIGE FNPSNGRTNYNEKFKSKATMTVDTSTNTAYMELSSLRSEDTAVYYCASRD YDYDGRYFDYWGQGTLVTVSSASTKGPSVFPLAPSSKSTSGGTAALGCLV KDYFPEPVTVSWNSGALTSGVHTFPAVLQSSGLYSLSSVVTVPSSSLGTQ TYICNVNHKPSNTKVDKKV | 921 |
| | Fab region (VL-CL) | DIQMTQSPSSLSASVGDRVTITCSASSSVTYMYWYQQKPGKAPKLLIYDT SNLASGVPSRFSGSGSGTDYTFTISSLQPEDIATYYCQQWSSHIPTFGCG TKVEIKRTVAAPSVFIFPPSDEQLKSGTASVVCLLNNFYPREAKVQWKVD | 922 |

| | | | |
|---|---|---|---|
| | | NALQSGNSQESVTEQDSKDSTYSLSSTLTLSKADYEKHKVYACEVTHQGLSSPVTKSFNRGEC | |
| | L1 linker | EPKSC | 836 |
| | L2 linker | GGGGSGGGGSGGGGSEPKSS | 837 |
| | L3 linker | GGGGS | 838 |
| | L4 linker | GGGGS | 838 |
| | Hinge-Fc (Knob) | DKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 839 |
| | Hinge-Fc (Hole) | DKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVCTLPPSREEMTKNQVSLSCAVKGFYPSDIAVEWESNGQPENNYKTTPPVLDSDGSFFLVSKLTVDKSRWQQGNVFSCSVMHEALHNHYTQKSLSLSPGK | 7 |
| | Z protein(KRAS$^{G12D}$ Peptide) | KLVVVGADGVGKSALTI | 910 |
| EAYC-N3 | Fab region (VH-CH1) | QVQLQESGPGLVKPSQTLSLTCTVSGGSISSGDYYWSWIRQPPGKCLEWIGYIYYSGSTDYNPSLKSRVTMSVDTSKNQFSLKVNSVTAADTAVYYCARVSIFGVGTFDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKV | 923 |
| | Fab region (VL-CL) | EIVMTQSPATLSLSPGERATLSCRASQSVSSYLAWYQQKPGQAPRLLIYDASNRATGIPARFSGSGSGTDFTLTISSLEPEDFAVYYCHQYGSTPLTFGCGTKAEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC | 924 |

|  | L1 linker | EPKSC | 836 |
|---|---|---|---|
|  | L2 linker | GGGGSGGGGSGGGGSEPKSS | 837 |
|  | L3 linker | GGGGS | 838 |
|  | L4 linker | GGGGS | 838 |
|  | Hinge-Fc (Knob) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVYTLPPCREEMTKNQVSLWCLVK GFYPSDIAVEWESNGQPENNYKTTPPVLDSDGSFFLYSKLTVDKSRWQQG NVFSCSVMHEALHNHYTQKSLSLSPGK | 839 |
|  | Hinge-Fc (Hole) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVCTLPPSREEMTKNQVSLSCAVK GFYPSDIAVEWESNGQPENNYKTTPPVLDSDGSFFLVSKLTVDKSRWQQG NVFSCSVMHEALHNHYTQKSLSLSPGK | 7 |
|  | Z protein(KRAS[G12D] Peptide) | KLVVVGADGVGKSALTI | 910 |
| HAVCO1 | Fab region (VH-CH1) | EVQLVESGGGLVQPGGSLRLSCAASGFNIKDTYIHWVRQAPGKCLEWVAR IYPTNGYTRYADSVKGRFTISADTSKNTAYLQMNSLRAEDTAVYYCSRWG GDGFYAMDYWGQGTLVTVSSASTKGPSVFPLAPSSKSTSGGTAALGCLVK DYFPEPVTVSWNSGALTSGVHTFPAVLQSSGLYSLSSVVTVPSSSLGTQT YICNVNHKPSNTKVDKKV | 834 |
| | Fab region (VL-CL) | DIQMTQSPSSLSASVGDRVTIICRASQDVNTAVAWYQQKPGKAPKLLIYS ASFLYSGVPSRFSGSRSGTDFTLTISSLQPEDFATYYCQQHYTTPPTFGC GTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGEC | 835 |
| | L1 linker | EPKSC | 836 |
| | L2 linker | GGGGSGGGGSGGGGSEPKSS | 837 |

| | L3 linker | GGGGS | 838 |
|---|---|---|---|
| | L4 linker | GGGGS | 838 |
| | Hinge-Fc (Knob) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVYTLPPCREEMTKNQVSLWCLVK GFYPSDIAVEWESNGQPENNYKTTPPVLDSDGSFFLYSKLTVDKSRWQQG NVFSCSVMHEALHNHYTQKSLSLSPGK | 839 |
| | Hinge-Fc (Hole) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVCTLPPSREEMTKNQVSLSCAVK GFYPSDIAVEWESNGQPENNYKTTPPVLDSDGSFFLVSKLTVDKSRWQQG NVFSCSVMHEALHNHYTQKSLSLSPGK | 7 |
| | Z protein(KRAS^r Peptide) | KLVVVGAGGVGKSALTI | 908 |
| HAVC02 | Fab region (VH-CH1) | EVQLVESGGGLVQPGGSLRLSCAASGFNIKDTYIHWVRQAPGKGLEWVAR IYPTNGYTRYADSVKGRFTISADTSKNTAYLQMNSLRAEDTAVYYCSRWG GDGFYAMDYWGQGTLVTVSSASTKGPSVFPLAPSSKSTSGGTAALGCLVK DYFPEPVTVSWNSGALTSGVHTFPAVLQSSGLYSLSSVVTVPSSSLGTQT YICNVNHKPSNTKVDKKV | 834 |
| | Fab region (VL-CL) | DIQMTQSPSSLSASVGDRVTITCRASQDVNTAVAWYQQKPGKAPKLLIYS ASFLYSGVPSRFSGSRSGTDFTLTISSLQPEDFATYYCQQHYTTPPTFGC GTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGEC | 835 |
| | L1 linker | EPKSC | 836 |
| | L2 linker | GGGGSGGGGSGGGGSEPKSS | 837 |
| | L3 linker | GG | 909 |
| | L4 linker | GG | 909 |

| | Hinge-Fc (Knob) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVYTLPPCREEMTKNQVSLWCLVK GFYPSDIAVEWESNGQPENNYKTTPPVLDSDGSFFLYSKLTVDKSRWQQG NVFSCSVMHEALHNHYTQKSLSLSPGK | 839 |
|---|---|---|---|
| | Hinge-Fc (Hole) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVCTLPPSREEMTKNQVSLSCAVK GFYPSDIAVEWESNGQPENNYKTTPPVLDSDGSFFLYSKLTVDKSRWQQG NVFSCSVMHEALHNHYTQKSLSLSPGK | 7 |
| | Z. protein(KRAS* T Peptide) | KLVVVGAGGVGKSALTI | 908 |
| IIAVC03 | Fab region (VH-CH1) | EVQLVESGGGLVQPGGSLRLSCAASGFNIKDTYIHWVRQAPGKCLEWVAR IYPTNGYTRYADSVKGRFTISADTSKNTAYLQMNSLRAEDTAVYYCSRWG GDGFYAMDYWGQGTLVTVSSASTKGPSVFPLAPSSKSTSGGTAALGCLVK DYFPEPVTVSWNSGALTSGVHTFPAVLQSSGLYSLSSVVTVPSSSLGTQT YICNVNHKPSNTKVDKKV | 834 |
| | Fab region (VL-CL) | DIQMTQSPSSLSASVGDRVTITCRASQDVNTAVAWYQQKPGKAPKLLIYS ASFLYSGVPSRFSGSRSGTDFTLTISSLQPEDFATYYCQQHYTTPPTFGC GTKVEIKRTVAAPSVFIFPPSDEQLKSGTASVVCLLNNFYPREAKVQWKV DNALQSGNSQESVTEQDSKDSTYSLSSTLTLSKADYEKIKVYACEVTHQG LSSPVTKSFNRGEC | 835 |
| | L1 linker | EPKSC | 836 |
| | L2 linker | GGGGSGGGGSGGGGSEPKSS | 837 |
| | L3 linker | GGGGS | 838 |
| | L4 linker | GGGGS | 838 |
| | Hinge-Fc (Knob) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK | 839 |

|  |  |  |  |
|---|---|---|---|
|  |  | CKVSNKALPAPIEKTISKAKGQPREPQVYTLPPCREEMTKNQVSLWCLVK GFYPSDIAVEWESNGQPENNYKTTPPVLDSDGSFFLYSKLTVDKSRWQQG NVFSCSVMHEALIDHYTQKSLSLSPGK |  |
|  | Hinge-Fc (Hole) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVCTLPPSREEMTKNQVSLSCAVK GFYPSDIAVEWESNGQPENNYKTTPPVLDSDGSFFLVSKLTVDKSRWQQG NVFSCSVMHEALHNHYTQKSLSLSPGK | 7 |
|  | Z protein(KRAS[G12D] Peptide) | KLVVVGADGVGKSALTI | 910 |
| HAVC04 | Fab region (VH-CH1) | EVQLVESGGGLVQPGGSLRLSCAASGFNIKDTYIHWVRQAPGKCLEWVAR IYPTNGYTRYADSVKGRFTISADTSKNTAYLQMNSLRAEDTAVYYCSRWG GDGFYAMDYWGQGTLVTVSSASTKGPSVFPLAPSSKSTSGGTAALGCLVK DYFPEPVTVSWNSGALTSGVHTFPAVLQSSGLYSLSSVVTVPSSSLGTQT YICNVNHKPSNTKVDKKV | 834 |
|  | Fab region (VL-CL) | DIQMTQSPSSLSASVGDRVTITCRASQDVNTAVAWYQQKPGKAPKLLIYS ASFLYSGVPSRFSGSRSGTDFTLTISSLQPEDFATYYCQQHYTTPPTFGC GTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGEC | 835 |
|  | L1 linker | EPKSC | 836 |
|  | L2 linker | GGGGSGGGGSGGGGSEPKSS | 837 |
|  | L3 linker | GG | 909 |
|  | L4 linker | GG | 909 |
|  | Hinge-Fc (Knob) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVYTLPPCREEMTKNQVSLWCLVK GFYPSDIAVEWESNGQPENNYKTTPPVLDSDGSFFLYSKLTVDKSRWQQG | 839 |

| | | | |
|---|---|---|---|
| | | KVFSCSVMHEALHNHYTQKSLSLSPGK | |
| | Hinge-Fc (Hole) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVCTLPPSREEMTKNQVSLSCAVK GFYPSDIAVEWESNGQPENNYKTTPPVLDSDGSFFLVSKLTVDKSRWQQG KVFSCSVMHEALHNHYTQKSLSLSPGK | 7 |
| | Z protein(KRAS[G12D] Peptide) | KLVVVGADGVGKSALTI | 910 |
| HAVC05 | Fab region (VH-CH1) | EVQLVESGGGLVQPGGSLRLSCAASGFNIKDTYIHWVRQAPGKCLEWVAR IYPTNGYTRYADSVKGRFTISADTSKNTAYLQMNSLRAEDTAVYYCSRWG GDGFYAMDYWGQGTLVTVSSASTKGPSVFPLAPSSKSTSGGTAALGCLVK DYFPEPVTVSWNSGALTSGVHTFPAVLQSSGLYSLSSVVTVPSSSLGTQT YICNVNHKPSNTKVDKKV | 834 |
| | Fab region (VL-CL) | DIQMTQSPSSLSASVGDRVTITCRASQDVNTAVAWYQQKPGKAPKLLIYS ASFLYSGVPSRFSGSRSGTDFTLTISSLQPEDFATYYCQQHYTTPPTFGC GTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGEC | 835 |
| | L1 linker | EPKSC | 836 |
| | L2 linker | GGGGSGGGGSGGGGSEPKSS | 837 |
| | L3 linker | GGGGS | 838 |
| | L4 linker | GGGGS | 838 |
| | Hinge-Fc (Knob) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVYTLPPCREEMTKNQVSLWCLVK GFYPSDIAVEWESNGQPENNYKTTPPVLDSDGSFFLYSKLTVDKSRWQQG KVFSCSVMHEALHNHYTQKSLSLSPGK | 839 |
| | Hinge-Fc | DKTHTCPPCPAPELLGGPSVFLFPPKPKDTLMISRTPEVTCVVVDVSHED | 7 |

| | | | |
|---|---|---|---|
| | (Hole) | PEVKFNWYVDGVEVHNAKTKPREEQYNSTYRVVSVLTVLHQDWLNGKEYK CKVSNKALPAPIEKTISKAKGQPREPQVCTLPPSREEMTKNQVSLSCAVK GFYPSDIAVEWESNGQPENNYKTTPPVLDSDGSFFLVSKLTVDKSRWQQG NVFSCSVMHEALHNHYTQKSLSLSPGK | |
| | Z protein(KRAS^T^T Peptide-Linker(5-mer)-KRAS^T^T Peptide) | KLVVVGAGGVGKSALTIGGGGSKLVVVGAGGVGKSALTI | 911 |
| HAVCQG | Fab region (VH-CH1) | EVQLVESGGGLVQPGGSLRLSCAASGFNIKDTYIHWVRQAPGKCLEWVAR IYPTNGYTRYADSVKGRFTISADTSKNTAYLQMNSLRAEDTAVYYCSRWG GDGFYAMDYWGQGTLVTVSSASTKGPSVFPLAPSSKSTSGGTAALGCLVK DYFPEPVTVSWNSGALTSGVHTFPAVLQSSGLYSLSSVVTVPSSSLGTQT YICNVNHKPSNTKVDKKV | 834 |
| | Fab region (VL-CL) | DIQMTQSPSSLSASVGDRVTITCRASQDVNTAVAWYQQKPGKAPKLLIYS ASFLYSGVPSRFSGSRSGTDFTLTISSLQPEDFATYYCQQHYTTPPTFGC GTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGEC | 835 |
| | L1 linker | EPKSC | 836 |
| | L2 linker | GGGGSGGGGSGGGGSEPKSS | 837 |
| | L3 linker | GG | 909 |
| | L4 linker | GG | 909 |
| | Hinge-Fc (Knob) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVYTLPPCREEMTKNQVSLWCLVK GFYPSDIAVEWESNGQPENNYKTTPPVLDSDGSFFLYSKLTVDKSRWQQG NVFSCSVMHEALHNHYTQKSLSLSPGK | 839 |

| | | Hinge-Fc (Hole) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVCTLPPSREEMTKNQVSLSCAVK GFYPSDIAVEWESNGQPENNYKTTPPVLDSDGSFFLVSKLTVDKSRWQQG NVFSCSVMHEALHNHYTQKSLSLSPGK | 7 |
|---|---|---|---|---|
| | | Z protein(KRAS¹ ⁷ Peptide-Linker(2-mer)-KRAS⁶⁷ Peptide) | KLVVVGAGGVGKSALT IGGKLVVVGAGGVGKSALT I | 912 |
| HAVC07 | | Fab region (VH-CH1) | EVQLVESGGGLVQPGGSLRLSCAASGFXIKDTYIHWVRQAPGKCLEWVAR IYPTNGYTRYADSVKGRFTISADTSKNTAYLQMNSLRAEDTAVYYCSRWG GDGFYAMDYWGQGTLVTVSSASTKGPSVFPLAPSSKSTSGGTAALGCLVK DYFPEPVTVSWNSGALTSGVHTFPAVLQSSGLYSLSSVVTVPSSSLGTQT YICNVNHKPSNTKVDKKV | 834 |
| | | Fab region (VL-CL) | DIQMTQSPSSLSASVGDRVTITCRASQDVNTAVAWYQQKPGKAPKLLIYS ASFLYSGVPSRFSGSRSGTDFTLTISSLQPEDFATYYCQQHYTTPPTFGC GTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGEC | 835 |
| | | L1 linker | EPKSC | 836 |
| | | L2 linker | GGGGSGGGGSGGGGSEPKSS | 837 |
| | | L3 linker | GGGGS | 838 |
| | | L4 linker | GGGGS | 838 |
| | | Hinge-Fc (Knob) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVYTLPPCREEMTKNQVSLWCLVK GFYPSDIAVEWESNGQPENNYKTTPPVLDSDGSFFLYSKLTVDKSRWQQG | 839 |

| | | | |
|---|---|---|---|
| | | NVFSCSVMHEALHNHYTQKSLSLSPGK | |
| | Hinge-Fc (Hole) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVCTLPPSREEMTKNQVSLSCAVK GFYPSDIAVEWESNGQPENNYKTTPPVLDSDGSFFLVSKLTVDKSRWQQG NVFSCSVMHEALHNHYTQKSLSLSPGK | 7 |
| | Z protein(KRAS$^G$ $^{12D}$ Peptide-Linker(5-mer)-KRAS$^{G12D}$ Peptide) | KLVVVGADGVGKSALTIGGGGSKLVVVGADGVGKSALTI | 913 |
| HAVCO8 | Fab region (VH-CH1) | EVQLVESGGGLVQPGGSLRLSCAASGFNIKDTYIHWVRQAPGKCLEWVAR IYPTNGYTRYADSVKGRFTISADTSKNTAYLQMNSLRAEDTAVYYCSRWG GDGFYAMDYWGQGTLVTVSSASTKGPSVFPLAPSSKSTSGGTAALGCLVK DYFPEPVTVSWNSGALTSGVHTFPAVLQSSGLYSLSSVVTVPSSSLGTQT YICNVNHKPSNTKVDKKV | 834 |
| | Fab region (VL-CL) | DIQMTQSPSSLSASVGDRVTITCRASQDVNTAVAWYQQKPGKAPKLLIYS ASFLYSGVPSRFSGSRSGTDFTLTISSLQPEDFATYYCQQHYTTPPTFGC GTKVEIKRTVAAPSVFIFPPSDEQLKSGTASVVCLLNNFYPREAKVQWKV DNALQSGNSQESVTEQDSKDSTYSLSSTLTLSKADYEKHKVYACEVTHQG LSSPVTKSFNRGEC | 835 |
| | L1 linker | EPKSC | 836 |
| | L2 linker | GGGGSGGGGSGGGGSEPKSS | 837 |
| | L3 linker | GG | 909 |
| | L4 linker | GG | 909 |
| | Hinge-Fc (Knob) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVYTLPPCREEMTKNQVSLWCLVK | 839 |

| | | | |
|---|---|---|---|
| | | GFYPSDIAVEWESNGQPENNYKTTPPVLDSDGSFFLYSKLTVDKSRWQQG NVFSCSVMHEALHNHYTQKSLSLSPGK | |
| | Hinge-Fc (Hole) | DKTHTCPPCPAPELLGGPSVFLFPPKPKDTLMISRTPEVTCVVVDVSHED PEVKFNWYVDGVEVHNAKTKPREEQYNSTYRVVSVLTVLHQDWLNGKEYK CKVSNKALPAPIEKTISKAKGQPREPQVCTLPPSREEMTKNQVSLSCAVK GFYPSDIAVEWESNGQPENNYKTTPPVLDSDGSFFLVSKLTVDKSRWQQG NVFSCSVMHEALHNHYTQKSLSLSPGK | 7 |
| | Z protein(KRAS$^{G12D}$ Peptide-Linker(2-mer)-KRAS$^{G12D}$ Peptide) | KLVVVGADGVGKSALTIGGKLVVVGADGVGKSALTI | 914 |

Table 101 below shows each polypeptide sequence constituting the novel antibody structure.

[Table 101]

| Name | | Sequence | SEQ ID NO |
|---|---|---|---|
| EAVC-P1 | Polypeptide 1 | QVQLQESGPGLVKPSETLSLTCTVSGGSVSSGDYYWTWIRQSPGKCLEWIGHIYYSG NTNYNPSLKSRLTISIDTSKTQFSLKLSSVTAADTAIYYCVRDRVTGAFDIWGQGTM VTVSSASTKGPSVFPLAPSSKSTSGGTAALGCLVKDYFPEPVTVSWNSGALTSGVHT FPAVLQSSGLYSLSSVVTVPSSSLGTQTYICNVNHKPSNTKVDKKVEPKSCDKTHTC PPCPAPELLGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEKTISKAK GQPREPQVYTLPPCREEMTKNQVSLWCLVKGFYPSDIAVEWESNGQPENNYKTTPPV LDSDGSFFLYSKLTVDKSRWQQGNVFSCSVMHEALINIIYTQKSLSLSPGK | 594 |
| | Polypeptide 2 | DIQMTQSPSSLSASVGDRVTITCQASQDISNYLNWYQQKPGKAPKLLIYDASNLETG VPSRFSGSGSGTDFTFTISSLQPEDIATYFCQHFDHLPLAFGCGTKVEIKRTVAAPS VFIFPPSDEQLKSGTASVVCLLNNFYPREAKVQWKVDNALQSGNSQESVTEQDSKDS TYSLSSTLTLSKADYEKHKVYACEVTHQGLSSPVTKSFNRGECGGGGSGGGGSGGGG | 595 |

| | | | |
|---|---|---|---|
| | | SEPKSSDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWESNG QPENNYKTTPPVLDSDGSFFLYSKLTVDKSRWQQGNVFSCSVMHEALHNHYTQKSLS LSPGK | |
| | Polype ptide 3 | KLVVVGAGGVGKSALTIGGGGSDKTHTCPPCPAPELLGGPSVFLFPPKPKDTLMISR TPEVTCVVVDVSHEDPEVKFNWYVDGVEVHNAKTKPREEQYNSTYRVVSVLTVLHQD WLNGKEYKCKVSNKALPAPIEKTISKAKGQPREPQVCTLPPSREEMTKNQVSLSCAV KGFYPSDIAVEWESNGQPENNYKTTPPVLDSDGSFFLVSKLTVDKSRWQQGNVFSCS VMHEALHNHYTQKSLSLSPGK | 925 |
| | Polype ptide 4 | KLVVVGAGGVGKSALTIGGGGSDKTHTCPPCPAPELLGGPSVFLFPPKPKDTLMISR TPEVTCVVVDVSHEDPEVKFNWYVDGVEVHNAKTKPREEQYNSTYRVVSVLTVLHQD WLNGKEYKCKVSNKALPAPIEKTISKAKGQPREPQVCTLPPSREEMTKNQVSLSCAV KGFYPSDIAVEWESNGQPENNYKTTPPVLDSDGSFFLVSKLTVDKSRWQQGNVFSCS VMHEALHNHYTQKSLSLSPGK | 925 |
| EAVC-P2 | Polype ptide 1 | QVQLQESGPGLVKPSETLSLTCTVSGGSVSSGDYYWTWIRQSPGKCLEWIGHIYYSG NTNYNPSLKSRLTISIDTSKTQFSLKLSSVTAADTAIYYCVRDRVTGAFDIWGQGTM VTVSSASTKGPSVFPLAPSSKSTSGGTAALGCLVKDYFPEPVTVSWNSGALTSGVHT FPAVLQSSGLYSLSSVVTVPSSSLGTQTYICNVNHKPSNTKVDKKVEPKSCDKTHTC PPCPAPELLGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEKTISKAK GQPREPQVYTLPPCREEMTKNQVSLWCLVKGFYPSDIAVEWESNGQPENNYKTTPPV LDSDGSFFLYSKLTVDKSRWQQGNVFSCSVMHEALHNHYTQKSLSLSPGK | 594 |
| | Polype ptide 2 | DIQMTQSPSSLSASVGDRVTITCQASQDISNYLNWYQQKPGKAPKLLIYDASNLETG VPSRFSGSGSGTDFTFTISSLQPEDIATYFCQHFDHLPLAFGCGTKVEIKRTVAAPS VFIFPPSDEQLKSGTASVVCLLNNFYPREAKVQWKVDNALQSGNSQESVTEQDSKDS TYSLSSTLTLSKADYEKHKVYACEVTHQGLSSPVTKSFNRGECGGGGSGGGGSGGGG SEPKSSDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCKVSNKAL | 595 |

| | | | | |
|---|---|---|---|---|
| | | PAPIEKTISKAKGQPREPQVYTLPPCREEMTKNQVSLWCLVKGFYPSDIAVEWESNG QPENNYKTTPPVLDSDGSFFLYSKLTVDKSRWQQGNVFSCSVMHEALHNHYTQKSLS LSPGK | |
| | Polype ptide 3 | KLVVVGAGGVGKSALTIGGDKTHTCPPCPAPELLGGPSVFLFPPKPKDTLMISRTPE VTCVVVDVSHEDPEVKFNWYVDGVEVHNAKTKPREEQYNSTYRVVSVLTVLHQDWLN GKEYKCKVSNKALPAPIEKTISKAKGQPREPQVCTLPPSREEMTKNQVSLSCAVKGF YPSDIAVEWESNGQPENNYKTTPPVLDSDGSFFLVSKLTVDKSRWQQGNVFSCSVMH EALHNHYTQKSLSLSPGK | 926 |
| | Polype ptide 4 | KLVVVGAGGVGKSALTIGGDKTHTCPPCPAPELLGGPSVFLFPPKPKDTLMISRTPE VTCVVVDVSHEDPEVKFNWYVDGVEVHNAKTKPREEQYNSTYRVVSVLTVLHQDWLN GKEYKCKVSNKALPAPIEKTISKAKGQPREPQVCTLPPSREEMTKNQVSLSCAVKGF YPSDIAVEWESNGQPENNYKTTPPVLDSDGSFFLVSKLTVDKSRWQQGNVFSCSVMH EALHNHYTQKSLSLSPGK | 926 |
| EAVC- P3 | Polype ptide 1 | QVQLQESGPGLVKPSETLSLTCTVSGGSVSSGDYYWTWIRQSPGKCLEWIGHIYYSG NTNYNPSLKSRLTISIDTSKTQFSLKLSSVTAADTAIYYCVRDRVTGAFDIWGQGTM VTVSSASTKGPSVFPLAPSSKSTSGGTAALGCLVKDYFPEPVTVSWNSGALTSGVHT FPAVLQSSGLYSLSSVVTVPSSSLGTQTYICNVNHKPSNTKVDKKVEPKSCDKTHTC PPCPAPELLGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEKTISKAK GQPREPQVYTLPPCREEMTKNQVSLWCLVKGFYPSDIAVEWESNGQPENNYKTTPPV LDSDGSFFLYSKLTVDKSRWQQGNVFSCSVMHEALHNHYTQKSLSLSPGK | 594 |
| | Polype ptide 2 | DIQMTQSPSSLSASVGDRVTITCQASQDISNYLNWYQQKPGKAPKLLIYDASNLETG VPSRFSGSGSGTDFTFTISSLQPEDIATYFCQHFDHLPLAFGCGTKVEIKRTVAAPS VFIFPPSDEQLKSGTASVVCLLNNFYPREAKVQWKVDNALQSGNSQESVTEQDSKDS TYSLSSTLTLSKADYEKHKVYACEVTHQGLSSPVTKSFNRGECGGGGSGGGGSGGGG SEPKSSDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWESNG QPENNYKTTPPVLDSDGSFFLYSKLTVDKSRWQQGNVFSCSVMHEALHNHYTQKSLS | 595 |

| | | | | |
|---|---|---|---|---|
| | | | LSPGK | |
| | Polypeptide 3 | KLVVVGADGVGKSALTIGGGGSDKTHTCPPCPAPELLGGPSVFLFPPKPKDTLMISR TPEVTCVVVDVSHEDPEVKFNWYVDGVEVHNAKTKPREEQYNSTYRVVSVLTVLHQD WLNGKEYKCKVSNKALPAPIEKTISKAKGQPREPQVCTLPPSREEMTKNQVSLSCAV KGFYPSDIAVEWESNGQPENNYKTTPPVLDSDGSFFLVSKLTVDKSRWQQGNVFSCS VMHEALHNHYTQKSLSLSPGK | 927 | |
| | Polypeptide 4 | KLVVVGADGVGKSALTIGGGGSDKTHTCPPCPAPELLGGPSVFLFPPKPKDTLMISR TPEVTCVVVDVSHEDPEVKFNWYVDGVEVHNAKTKPREEQYNSTYRVVSVLTVLHQD WLNGKEYKCKVSNKALPAPIEKTISKAKGQPREPQVCTLPPSREEMTKNQVSLSCAV KGFYPSDIAVEWESNGQPENNYKTTPPVLDSDGSFFLVSKLTVDKSRWQQGNVFSCS VMHEALHNHYTQKSLSLSPGK | 927 | |
| EAVC-P4 | Polypeptide 1 | QVQLQESGPGLVKPSETLSLTCTVSGGSVSSGDYYWTWIRQSPGKCLEWIGHIYYSG NTNYNPSLKSRLTISIDTSKTQFSLKLSSVTAADTAIYYCVRDRVTGAFDIWGQGTM VTVSSASTKGPSVFPLAPSSKSTSGGTAALGCLVKDYFPEPVTVSWNSGALTSGVHT FPAVLQSSGLYSLSSVVTVPSSSLGTQTYICNVNHKPSNTKVDKKVEPKSCDKTHTC PPCPAPELLGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEKTISKAK GQPREPQVYTLPPCREEMTKNQVSLWCLVKGFYPSDIAVEWESNGQPENNYKTTPPV LDSDGSFFLYSKLTVDKSRWQQGNVFSCSVMHEALHNHYTQKSLSLSPGK | 594 | |
| | Polypeptide 2 | DIQMTQSPSSLSASVGDRVTITCQASQDISNYLNWYQQKPGKAPKLLIYDASNLETG VPSRFSGSGSGTDFTFTISSLQPEDIATYFCQHFDHLPLAFGCGTKVEIKRTVAAPS VFIFPPSDEQLKSGTASVVCLLNNFYPREAKVQWKVDNALQSGNSQESVTEQDSKDS TYSLSSTLTLSKADYEKHKVYACEVTHQGLSSPVTKSFNRGECGGGGSGGGGSGGGG SEPKSSDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWESNG QPENNYKTTPPVLDSDGSFFLYSKLTVDKSRWQQGNVFSCSVMHEALHNHYTQKSLS LSPGK | 595 | |
| | Polype | KLVVVGADGVGKSALTIGGDKTHTCPPCPAPELLGGPSVFLFPPKPKDTLMISRTPE | 928 | |

| | | | |
|---|---|---|---|
| | ptide 3 | VTCVVVDVSHEDPEVKFNWYVDGVEVHNAKTKPREEQYNSTYRVVSVLTVLHQDWLN GKEYKCKVSNKALPAPIEKTISKAKGQPREPQVCTLPPSREEMTKNQVSLSCAVKGF YPSDIAVEWESNGQPENNYKTTPPVLDSDGSFFLVSKLTVDKSRWQQGNVFSCSVMH EALHNHYTQKSLSLSPGK | |
| | Polype ptide 4 | KLVVVGADGVGKSALTIGGDKTHTCPPCPAPELLGGPSVFLFPPKPKDTLMISRTPE VTCVVVDVSHEDPEVKFNWYVDGVEVHNAKTKPREEQYNSTYRVVSVLTVLHQDWLN GKEYKCKVSNKALPAPIEKTISKAKGQPREPQVCTLPPSREEMTKNQVSLSCAVKGF YPSDIAVEWESNGQPENNYKTTPPVLDSDGSFFLVSKLTVDKSRWQQGNVFSCSVMH EALHNHYTQKSLSLSPGK | 928 |
| EAVC-P5 | Polype ptide 1 | QVQLQESGPGLVKPSETLSLTCTVSGGSVSSGDYYWTWIRQSPGKCLEWIGHIYYSG NTNYNPSLKSRLTISIDTSKTQFSLKLSSVTAADTAIYYCVRDRVTGAFDIWGQGTM VTVSSASTKGPSVFPLAPSSKSTSGGTAALGCLVKDYFPEPVTVSWNSGALTSGVHT FPAVLQSSGLYSLSSVVTVPSSSLGTQTYICNVNHKPSNTKVDKKVEPKSCDKTHTC PPCPAPELLGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEKTISKAK GQPREPQVYTLPPCREEMTKNQVSLWCLVKGFYPSDIAVEWESNGQPENNYKTTPPV LDSDGSFFLYSKLTVDKSRWQQGNVFSCSVMHEALHNHYTQKSLSLSPGK | 594 |
| | Polype ptide 2 | DIQMTQSPSSLSASVGDRVTITCQASQDISNYLNWYQQKPGKAPKLLIYDASNLETG VPSRFSGSGSGTDFTFTISSLQPEDIATYFCQHFDHLPLAFGCGTKVEIKRTVAAPS VFIFPPSDEQLKSGTASVVCLLNNFYPREAKVQWKVDNALQSGNSQESVTEQDSKDS TYSLSSTLTLSKADYEKHKVYACEVTHQGLSSPVTKSFNRGECGGGGSGGGGSGGGG SEPKSSDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWESNG QPENNYKTTPPVLDSDGSFFLYSKLTVDKSRWQQGNVFSCSVMHEALHNHYTQKSLS LSPGK | 595 |
| | Polype ptide 3 | KLVVVGAGGVGKSALTIGGGGSKLVVVGAGGVGKSALTIGGGGSDKTHTCPPCPAPE LLGGPSVFLFPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISKAKGQPREPQ | 929 |

| | | | |
|---|---|---|---|
| | | VCTLPPSREEMTKNQVSLSCAVKGFYPSDIAVEWESNGQPENNYKTTPPVLDSDGSF FLVSKLTVDKSRWQQGNVFSCSVMHEALHNHYTQKSLSLSPGK | |
| | Polypeptide 4 | KLVVVGAGGVGKSALTIGGGGSKLVVVGAGGVGKSALTIGGGGSDKTHTCPPCPAPE LLGGPSVFLFPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISKAKGQPREPQ VCTLPPSREEMTKNQVSLSCAVKGFYPSDIAVEWESNGQPENNYKTTPPVLDSDGSF FLVSKLTVDKSRWQQGNVFSCSVMHEALHNHYTQKSLSLSPGK | 929 |
| EAVC-P6 | Polypeptide 1 | QVQLQESGPGLVKPSETLSLTCTVSGGSVSSGDYYWTWIRQSPGKCLEWIGHIYYSG NTNYNPSLKSRLTISIDTSKTQFSLKLSSVTAADTAIYYCVRDRVTGAFDIWGQGTM VTVSSASTKGPSVFPLAPSSKSTSGGTAALGCLVKDYFPEPVTVSWNSGALTSGVHT FPAVLQSSGLYSLSSVVTVPSSSLGTQTYICNVNHKPSNTKVDKKVEPKSCDKTHTC PPCPAPELLGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEKTISKAK GQPREPQVYTLPPCREEMTKNQVSLWCLVKGFYPSDIAVEWESNGQPENNYKTTPPV LDSDGSFFLYSKLTVDKSRWQQGNVFSCSVMHEALHNHYTQKSLSLSPGK | 594 |
| | Polypeptide 2 | DIQMTQSPSSLSASVGDRVTITCQASQDISNYLNWYQQKPGKAPKLLIYDASNLETG VPSRFSGSGSGTDFTFTISSLQPEDIATYFCQHFDHLPLAFGCGTKVEIKRTVAAPS VFIFPPSDEQLKSGTASVVCLLNNFYPREAKVQWKVDNALQSGNSQESVTEQDSKDS TYSLSSTLTLSKADYEKHKVYACEVTHQGLSSPVTKSFNRGECGGGGSGGGGSGGGG SEPKSSDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWESNG QPENNYKTTPPVLDSDGSFFLYSKLTVDKSRWQQGNVFSCSVMHEALHNHYTQKSLS LSPGK | 595 |
| | Polypeptide 3 | KLVVVGAGGVGKSALTIGGKLVVVGAGGVGKSALTIGGDKTHTCPPCPAPELLGGPS VFLFPPKPKDTLMISRTPEVTCVVVDVSHEDPEVKFNWYVDGVEVHNAKTKPREEQY NSTYRVVSVLTVLHQDWLNGKEYKCKVSNKALPAPIEKTISKAKGQPREPQVCTLPP SREEMTKNQVSLSCAVKGFYPSDIAVEWESNGQPENNYKTTPPVLDSDGSFFLVSKL TVDKSRWQQGNVFSCSVMHEALHNHYTQKSLSLSPGK | 930 |

| | Polype ptide 4 | KLVVVGAGGVGKSALTIGGKLVVVGAGGVGKSALTIGGDKTHTCPPCPAPELLGGPS VFLFPPKPKDTLMISRTPEVTCVVVDVSHEDPEVKFNWYVDGVEVHNAKTKPREEQY NSTYRVVSVLTVLHQDWLNGKEYKCKVSNKALPAPIEKTISKAKGQPREPQVCTLPP SREEMTKNQVSLSCAVKGFYPSDIAVEWESNGQPENNYKTTPPVLDSDGSFFLVSKL TVDKSRWQQGNVFSCSVMHEALHNHYTQKSLSLSPGK | 930 |
|---|---|---|---|
| EAVC-P7 | Polype ptide 1 | QVQLQESGPGLVKPSETLSLTCTVSGGSVSSGDYYWTWIRQSPGKCLEWIGHIYYSG NTNYNPSLKSRLTISIDTSKTQFSLKLSSVTAADTAIYYCVRDRVTGAFDIWGQGTM VTVSSASTKGPSVFPLAPSSKSTSGGTAALGCLVKDYFPEPVTVSWNSGALTSGVHT FPAVLQSSGLYSLSSVVTVPSSSLGTQTYICNVNHKPSNTKVDKKVEPKSCDKTHTC PPCPAPELLGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEKTISKAK GQPREPQVYTLPPCREEMTKNQVSLWCLVKGFYPSDIAVEWESNGQPENNYKTTPPV LDSDGSFFLYSKLTVDKSRWQQGNVFSCSVMHEALHNHYTQKSLSLSPGK | 594 |
| | Polype ptide 2 | DIQMTQSPSSLSASVGDRVTITCQASQDISNYLNWYQQKPGKAPKLLIYDASNLETG VPSRFSGSGSGTDFTFTISSLQPEDIATYFCQHFDHLPLAFGCGTKVEIKRTVAAPS VFIFPPSDEQLKSGTASVVCLLNNFYPREAKVQWKVDNALQSGNSQESVTEQDSKDS TYSLSSTLTLSKADYEKHKVYACEVTHQGLSSPVTKSFNRGECGGGGSGGGGSGGGG SEPKSSDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWESNG QPENNYKTTPPVLDSDGSFFLYSKLTVDKSRWQQGNVFSCSVMHEALHNHYTQKSLS LSPGK | 595 |
| | Polype ptide 3 | KLVVVGADGVGKSALTIGGGGSKLVVVGADGVGKSALTIGGGGSDKTHTCPPCPAPE LLGGPSVFLFPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISKAKGQPREPQ VCTLPPSREEMTKNQVSLSCAVKGFYPSDIAVEWESNGQPENNYKTTPPVLDSDGSF FLVSKLTVDKSRWQQGNVFSCSVMHEALHNHYTQKSLSLSPGK | 931 |
| | Polype ptide 4 | KLVVVGADGVGKSALTIGGGGSKLVVVGADGVGKSALTIGGGGSDKTHTCPPCPAPE LLGGPSVFLFPPKPKDTLMISRTPEVTCVVVDVSHEDPEVKFNWYVDGVEVHNAKTK | 931 |

| | | | | |
|---|---|---|---|---|
| | | | PREEQYNSTYRVVSVLTVLHQDWLNGKEYKCKVSNKALPAPIEKTISKAKGQPREPQ VCTLPPSREEMTKNQVSLSCAVKGFYPSDIAVEWESNGQPENNYKTTPPVLDSDGSF FLVSKLTVDKSRWQQGNVFSCSVMHEALHNHYTQKSLSLSPGK | |
| EAVC-P8 | Polypeptide 1 | QVQLQESGPGLVKPSETLSLTCTVSGGSVSSGDYYWTWIRQSPGKCLEWIGHIYYSG NTNYNPSLKSRLTISIDTSKTQFSLKLSSVTAADTAIYYCVRDRVTGAFDIWGQGTM VTVSSASTKGPSVFPLAPSSKSTSGGTAALGCLVKDYFPEPVTVSWNSGALTSGVHT FPAVLQSSGLYSLSSVVTVPSSSLGTQTYICNVNHKPSNTKVDKKVEPKSCDKTHTC PPCPAPELLGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEKTISKAK GQPREPQVYTLPPCREEMTKNQVSLWCLVKGFYPSDIAVEWESNGQPENNYKTTPPV LDSDGSFFLYSKLTVDKSRWQQGNVFSCSVMHEALHNHYTQKSLSLSPGK | 594 |
| | Polypeptide 2 | DIQMTQSPSSLSASVGDRVTITCQASQDISNYLNWYQQKPGKAPKLLIYDASNLETG VPSRFSGSGSGTDFTFTISSLQPEDIATYFCQHFDHLPLAFGCGTKVEIKRTVAAPS VFIFPPSDEQLKSGTASVVCLLNNFYPREAKVQWKVDNALQSGNSQESVTEQDSKDS TYSLSSTLTLSKADYEKHKVYACEVTHQGLSSPVTKSFNRGECGGGGSGGGGSGGGG SEPKSSDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWESNG QPENNYKTTPPVLDSDGSFFLYSKLTVDKSRWQQGNVFSCSVMHEALHNHYTQKSLS LSPGK | 595 |
| | Polypeptide 3 | KLVVVGADGVGKSALTIGGKLVVVGADGVGKSALTIGGDKTHTCPPCPAPELLGGPS VFLFPPKPKDTLMISRTPEVTCVVVDVSHEDPEVKFNWYVDGVEVHNAKTKPREEQY NSTYRVVSVLTVLHQDWLNGKEYKCKVSNKALPAPIEKTISKAKGQPREPQVCTLPP SREEMTKNQVSLSCAVKGFYPSDIAVEWESNGQPENNYKTTPPVLDSDGSFFLVSKL TVDKSRWQQGNVFSCSVMHEALHNHYTQKSLSLSPGK | 932 |
| | Polypeptide 4 | KLVVVGADGVGKSALTIGGKLVVVGADGVGKSALTIGGDKTHTCPPCPAPELLGGPS VFLFPPKPKDTLMISRTPEVTCVVVDVSHEDPEVKFNWYVDGVEVHNAKTKPREEQY NSTYRVVSVLTVLHQDWLNGKEYKCKVSNKALPAPIEKTISKAKGQPREPQVCTLPP SREEMTKNQVSLSCAVKGFYPSDIAVEWESNGQPENNYKTTPPVLDSDGSFFLVSKL | 932 |

| | | | |
|---|---|---|---|
| | | | TVDKSRWQQGNVFSCSVMHEALHNHYTQKSLSLSPGK | |
| EAVC-C3 | Polype ptide 1 | QVQLKQSGPGLVQPSQSLSITCTVSGFSLTNYGVHWVRQSPGKCLEWLGVIWSGGNT DYNTPFTSRLSINKDNSKSQVFFKMNSLQSNDTAIYYCARALTYYDYEFAYWGQGTL VTVSAASTKGPSVFPLAPSSKSTSGGTAALGCLVKDYFPEPVTVSWNSGALTSGVHT FPAVLQSSGLYSLSSVVTVPSSSLGTQTYICNVNHKPSNTKVDKKVEPKSCDKTHTC PPCPAPELLGGPSVFLFPPKPKDTLMISRTPEVTCVVVDVSHEDPEVKFNWYVDGVE VHNAKTKPREEQYNSTYRVVSVLTVLHQDWLNGKEYKCKVSNKALPAPIEKTISKAK GQPREPQVYTLPPCREEMTKNQVSLWCLVKGFYPSDIAVEWESNGQPENNYKTTPPV LDSDGSFFLYSKLTVDKSRWQQGNVFSCSVMHEALHNHYTQKSLSLSPGK | 933 |
| | Polype ptide 2 | DILLTQSPVILSVSPGERVSFSCRASQSIGTNIHWYQQRTNGSPRLLIKYASESISG IPSRFSGSGSGTDFTLSINSVESEDIADYYCQQNNNWPTTFGCGTKLELKRTVAAPS VFIFPPSDEQLKSGTASVVCLLNNFYPREAKVQWKVDNALQSGNSQESVTEQDSKDS TYSLSSTLTLSKADYEKHKVYACEVTHQGLSSPVTKSFNRGECGGGGSGGGGSGGGG SEPKSSDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWESNG QPENNYKTTPPVLDSDGSFFLYSKLTVDKSRWQQGNVFSCSVMHEALHNHYTQKSLS LSPGK | 934 |
| | Polype ptide 3 | KLVVVGADGVGKSALTIGGGGSDKTHTCPPCPAPELLGGPSVFLFPPKPKDTLMISR TPEVTCVVVDVSHEDPEVKFNWYVDGVEVHNAKTKPREEQYNSTYRVVSVLTVLHQD WLNGKEYKCKVSNKALPAPIEKTISKAKGQPREPQVCTLPPSREEMTKNQVSLSCAV KGFYPSDIAVEWESNGQPENNYKTTPPVLDSDGSFFLVSKLTVDKSRWQQGNVFSCS VMHEALHNHYTQKSLSLSPGK | 927 |
| | Polype ptide 4 | KLVVVGADGVGKSALTIGGGGSDKTHTCPPCPAPELLGGPSVFLFPPKPKDTLMISR TPEVTCVVVDVSHEDPEVKFNWYVDGVEVHNAKTKPREEQYNSTYRVVSVLTVLHQD WLNGKEYKCKVSNKALPAPIEKTISKAKGQPREPQVCTLPPSREEMTKNQVSLSCAV KGFYPSDIAVEWESNGQPENNYKTTPPVLDSDGSFFLVSKLTVDKSRWQQGNVFSCS VMHEALHNHYTQKSLSLSPGK | 927 |
| EAVC- | Polype | QVQLVQSGAEVKKPGSSVKVSCKASGFTFTDYKIHWVRQAPGQCLEWMGYFNPNSGY | 935 |

| I3 | ptide 1 | STYAQKFQGRVTITADKSTSTAYMELSSLRSEDTAVYYCARLSPGGYYVMDAWGQGT TVTVSSASTKGPSVFPLAPSSKSTSGGTAALGCLVKDYFPEPVTVSWNSGALTSGVH TFPAVLQSSGLYSLSSVVTVPSSSLGTQTYICNVNHKPSNTKVDKKVEPKSCDKTHT CPPCPAPELLGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKA KGQPREPQVYTLPPCREEMTKNQVSLWCLVKGFYPSDIAVEWESNGQPENNYKTTPP VLDSDGSFFLYSKLTVDKSRWQQGNVFSCSVMHEALHNHYTQKSLSLSPGK | |
| | Polype ptide 2 | DIQMTQSPSSLSASVGDRVTITCRASQGINNYLNWYQQKPGKAPKRLIYNTNNLQTG VPSRFSGSGSGTEFTLTISSLQPEDFATYYCLQHNSFPTFGCGTKLEIKRTVAAPSV FIFPPSDEQLKSGTASVVCLLNNFYPREAKVQWKVDNALQSGNSQESVTEQDSKDST YSLSSTLTLSKADYEKHKVYACEVTHQGLSSPVTKSFNRGECGGGGSGGGGSGGGGS EPKSSDKTHTCPPCPAPELLGGPSVFLFPPKPKDTLMISRTPEVTCVVVDVSHEDPE VKFNWYVDGVEVHNAKTKPREEQYNSTYRVVSVLTVLHQDWLNGKEYKCKVSNKALP APIEKTISKAKGQPREPQVYTLPPCREEMTKNQVSLWCLVKGFYPSDIAVEWESNGQ PENNYKTTPPVLDSDGSFFLYSKLTVDKSRWQQGNVFSCSVMHEALHNHYTQKSLSL SPGK | 936 |
| | Polype ptide 3 | KLVVVGADGVGKSALTIGGGGSDKTHTCPPCPAPELLGGPSVFLFPPKPKDTLMISR TPEVTCVVVDVSHEDPEVKFNWYVDGVEVHNAKTKPREEQYNSTYRVVSVLTVLHQD WLNGKEYKCKVSNKALPAPIEKTISKAKGQPREPQVCTLPPSREEMTKNQVSLSCAV KGFYPSDIAVEWESNGQPENNYKTTPPVLDSDGSFFLVSKLTVDKSRWQQGNVFSCS VMHEALHNHYTQKSLSLSPGK | 927 |
| | Polype ptide 4 | KLVVVGADGVGKSALTIGGGGSDKTHTCPPCPAPELLGGPSVFLFPPKPKDTLMISR TPEVTCVVVDVSHEDPEVKFNWYVDGVEVHNAKTKPREEQYNSTYRVVSVLTVLHQD WLNGKEYKCKVSNKALPAPIEKTISKAKGQPREPQVCTLPPSREEMTKNQVSLSCAV KGFYPSDIAVEWESNGQPENNYKTTPPVLDSDGSFFLVSKLTVDKSRWQQGNVFSCS VMHEALHNHYTQKSLSLSPGK | 927 |
| EAVC-L3 | Polype ptide 1 | EVQLQESGPGLVKPSQTLSLTCTVSGYSISRDFAWNWIRQPPGKCLEWMGYISYNGN TRYQPSLKSRITISRDTSKNQFFLKLNSVTAADTATYYCVTASRGFPYWGQGTLVTV SSASTKGPSVFPLAPSSKSTSGGTAALGCLVKDYFPEPVTVSWNSGALTSGVHTFPA | 937 |

| | | | |
|---|---|---|---|
| | | VLQSSGLYSLSSVVTVPSSSLGTQTYICNVNHKPSNTKVDKKVEPKSCDKTHTCPPC PAPELLGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKAKGQP REPQVYTLPPCREEMTKNQVSLWCLVKGFYPSDIAVEWESNGQPENNYKTTPPVLDS DGSFFLYSKLTVDKSRWQQGNVFSCSVMHEALHNHYTQKSLSLSPGK | |
| | Polype ptide 2 | DIQMTQSPSSMSVSVGDRVTITCHSSQDINSNIGWLQQKPGKSFKGLIYHGTNLDDG VPSRFSGSGSGTDYTLTISSLQPEDFATYYCVQYAQFPWTFGCGTKLEIKRTVAAPS VFIFPPSDEQLKSGTASVVCLLNNFYPREAKVQWKVDNALQSGNSQESVTEQDSKDS TYSLSSTLTLSKADYEKHKVYACEVTHQGLSSPVTKSFNRGECGGGGSGGGGSGGGG SEPKSSDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWESNG QPENNYKTTPPVLDSDGSFFLYSKLTVDKSRWQQGNVFSCSVMHEALHNHYTQKSLS LSPGK | 938 |
| | Polype ptide 3 | KLVVVGADGVGKSALTIGGGGSDKTHTCPPCPAPELLGGPSVFLFPPKPKDTLMISR TPEVTCVVVDVSHEDPEVKFNWYVDGVEVHNAKTKPREEQYNSTYRVVSVLTVLHQD WLNGKEYKCKVSNKALPAPIEKTISKAKGQPREPQVCTLPPSREEMTKNQVSLSCAV KGFYPSDIAVEWESNGQPENNYKTTPPVLDSDGSFFLVSKLTVDKSRWQQGNVFSCS VMHEALHNHYTQKSLSLSPGK | 927 |
| | Polype ptide 4 | KLVVVGADGVGKSALTIGGGGSDKTHTCPPCPAPELLGGPSVFLFPPKPKDTLMISR TPEVTCVVVDVSHEDPEVKFNWYVDGVEVHNAKTKPREEQYNSTYRVVSVLTVLHQD WLNGKEYKCKVSNKALPAPIEKTISKAKGQPREPQVCTLPPSREEMTKNQVSLSCAV KGFYPSDIAVEWESNGQPENNYKTTPPVLDSDGSFFLVSKLTVDKSRWQQGNVFSCS VMHEALHNHYTQKSLSLSPGK | 927 |
| EAVC-M3 | Polype ptide 1 | QVQLVQSGAEVKKPGASVKVSCKASGYTFTSHWMHWVRQAPGQCLEWIGEFNPSNGR TNYNEKFKSKATMTVDTSTNTAYMELSSLRSEDTAVYYCASRDYDYDGRYFDYWGQG TLVTVSSASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKKVEPKSCDKTH TCPPCPAPELLGGPSVFLFPPKPKDTLMISRTPEVTCVVVDVSHEDPEVKFNWYVDG | 939 |

| | | | |
|---|---|---|---|
| | | VEVHNAKTKPREEQYNSTYRVVSVLTVLHQDWLNGKEYKCKVSNKALPAPIEKTISK AKGQPREPQVYTLPPCREEMTKNQVSLWCLVKGFYPSDIAVEWESNGQPENNYKTTP PVLDSDGSFFLYSKLTVDKSRWQQGNVFSCSVMHEALHNHYTQKSLSLSPGK | |
| | Polype ptide 2 | DIQMTQSPSSLSASVGDRVTITCSASSSVTYMYWYQQKPGKAPKLLIYDTSNLASGV PSRFSGSGSGTDYTFTISSLQPEDIATYYCQQWSSHIFTFGCGTKVEIKRTVAAPSV FIFPPSDEQLKSGTASVVCLLNNFYPREAKVQWKVDNALQSGNSQESVTEQDSKDST YSLSSTLTLSKADYEKHKVYACEVTHQGLSSPVTKSFNRGECGGGGSGGGGSGGGGS EPKSSDKTHTCPPCPAPELLGGPSVFLFPPKPKDTLMISRTPEVTCVVVDVSHEDPE VKFNWYVDGVEVHNAKTKPREEQYNSTYRVVSVLTVLHQDWLNGKEYKCKVSNKALP APIEKTISKAKGQPREPQVYTLPPCREEMTKNQVSLWCLVKGFYPSDIAVEWESNGQ PENNYKTTPPVLDSDGSFFLYSKLTVDKSRWQQGNVFSCSVMHEALHNHYTQKSLSL SPGK | 940 |
| | Polype ptide 3 | KLVVVGADGVGKSALTIGGGGSDKTHTCPPCPAPELLGGPSVFLFPPKPKDTLMISR TPEVTCVVVDVSHEDPEVKFNWYVDGVEVHNAKTKPREEQYNSTYRVVSVLTVLHQD WLNGKEYKCKVSNKALPAPIEKTISKAKGQPREPQVCTLPPSREEMTKNQVSLSCAV KGFYPSDIAVEWESNGQPENNYKTTPPVLDSDGSFFLVSKLTVDKSRWQQGNVFSCS VMHEALHNHYTQKSLSLSPGK | 927 |
| | Polype ptide 4 | KLVVVGADGVGKSALTIGGGGSDKTHTCPPCPAPELLGGPSVFLFPPKPKDTLMISR TPEVTCVVVDVSHEDPEVKFNWYVDGVEVHNAKTKPREEQYNSTYRVVSVLTVLHQD WLNGKEYKCKVSNKALPAPIEKTISKAKGQPREPQVCTLPPSREEMTKNQVSLSCAV KGFYPSDIAVEWESNGQPENNYKTTPPVLDSDGSFFLVSKLTVDKSRWQQGNVFSCS VMHEALHNHYTQKSLSLSPGK | 927 |
| EAVC- N3 | Polype ptide 1 | QVQLQESGPGLVKPSQTLSLTCTVSGGSISSGDYYWSWIRQPPGKCLEWIGYIYYSG STDYNPSLKSRVTMSVDTSKNQFSLKVNSVTAADTAVYYCARVSIFGVGTFDYWGQG TLVTVSSASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKKVEPKSCDKTH TCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISK AKGQPREPQVYTLPPCREEMTKNQVSLWCLVKGFYPSDIAVEWESNGQPENNYKTTP | 941 |

| | | | |
|---|---|---|---|
| | | PVLDSDGSFFLYSKLTVDKSRWQQGNVFSCSVMHEALHNHYTQKSLSLSPGK | |
| | Polypeptide 2 | EIVMTQSPATLSLSPGERATLSCRASQSVSSYLAWYQQKPGQAPRLLIYDASNRATG IPARFSGSGSGTDFTLTISSLEPEDFAVYYCHQYGSTPLTFGCGTKAEIKRTVAAPS VFIFPPSDEQLKSGTASVVCLLNNFYPREAKVQWKVDNALQSGNSQESVTEQDSKDS TYSLSSTLTLSKADYEKHKVYACEVTHQGLSSPVTKSFNRGECGGGGSGGGGSGGGG SEPKSSDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWESNG QPENNYKTTPPVLDSDGSFFLYSKLTVDKSRWQQGNVFSCSVMHEALHNHYTQKSLS LSPGK | 942 |
| | Polypeptide 3 | KLVVVGADGVGKSALTIGGGGSDKTHTCPPCPAPELLGGPSVFLFPPKPKDTLMISR TPEVTCVVVDVSHEDPEVKFNWYVDGVEVHNAKTKPREEQYNSTYRVVSVLTVLHQD WLNGKEYKCKVSNKALPAPIEKTISKAKGQPREPQVCTLPPSREEMTKNQVSLSCAV KGFYPSDIAVEWESNGQPENNYKTTPPVLDSDGSFFLVSKLTVDKSRWQQGNVFSCS VMHEALHNHYTQKSLSLSPGK | 927 |
| | Polypeptide 4 | KLVVVGADGVGKSALTIGGGGSDKTHTCPPCPAPELLGGPSVFLFPPKPKDTLMISR TPEVTCVVVDVSHEDPEVKFNWYVDGVEVHNAKTKPREEQYNSTYRVVSVLTVLHQD WLNGKEYKCKVSNKALPAPIEKTISKAKGQPREPQVCTLPPSREEMTKNQVSLSCAV KGFYPSDIAVEWESNGQPENNYKTTPPVLDSDGSFFLVSKLTVDKSRWQQGNVFSCS VMHEALHNHYTQKSLSLSPGK | 927 |
| HAVCO1 | Polypeptide 1 | EVQLVESGGGLVQPGGSLRLSCAASGFNIKDTYIHWVRQAPGKCLEWVARIYPTNGY TRYADSVKGRFTISADTSKNTAYLQMNSLRAEDTAVYYCSRWGGDGFYAMDYWGQGT LVTVSSASTKGPSVFPLAPSSKSTSGGTAALGCLVKDYFPEPVTVSWNSGALTSGVH TFPAVLQSSGLYSLSSVVTVPSSSLGTQTYICNVNHKPSNTKVDKKVEPKSCDKTHT CPPCPAPELLGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKA KGQPREPQVYTLPPCREEMTKNQVSLWCLVKGFYPSDIAVEWESNGQPENNYKTTPP VLDSDGSFFLYSKLTVDKSRWQQGNVFSCSVMHEALHNHYTQKSLSLSPGK | 14 |
| | Polype | DIQMTQSPSSLSASVGDRVTITCRASQDVNTAVAWYQQKPGKAPKLLIYSASFLYSG | 15 |

| | | | |
|---|---|---|---|
| | pt ide 2 | VPSRFSGSRSGTDFTLTISSLQPEDFATYYCQQHYTTPPTFGCGTKVEIKRTVAAPS VFIFPPSDEQLKSGTASVVCLLNNFYPREAKVQWKVDNALQSGNSQESVTEQDSKDS TYSLSSTLTLSKADYEKHKVYACEVTHQGLSSPVTKSFNRGECGGGGSGGGGSGGGG SEPKSSDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWESNG QPENNYKTTPPVLDSDGSFFLYSKLTVDKSRWQQGNVFSCSVMHEALHNHYTQKSLS LSPGK | |
| | Polype pt ide 3 | KLVVVGAGGVGKSALTIGGGGSDKTHTCPPCPAPELLGGPSVFLFPPKPKDTLMISR TPEVTCVVVDVSHEDPEVKFNWYVDGVEVHNAKTKPREEQYNSTYRVVSVLTVLHQD WLNGKEYKCKVSNKALPAPIEKTISKAKGQPREPQVCTLPPSREEMTKNQVSLSCAV KGFYPSDIAVEWESNGQPENNYKTTPPVLDSDGSFFLVSKLTVDKSRWQQGNVFSCS VMHEALHNHYTQKSLSLSPGK | 925 |
| | Polype pt ide 4 | KLVVVGAGGVGKSALTIGGGGSDKTHTCPPCPAPELLGGPSVFLFPPKPKDTLMISR TPEVTCVVVDVSHEDPEVKFNWYVDGVEVHNAKTKPREEQYNSTYRVVSVLTVLHQD WLNGKEYKCKVSNKALPAPIEKTISKAKGQPREPQVCTLPPSREEMTKNQVSLSCAV KGFYPSDIAVEWESNGQPENNYKTTPPVLDSDGSFFLVSKLTVDKSRWQQGNVFSCS VMHEALHNHYTQKSLSLSPGK | 925 |
| HAVCO2 | Polype pt ide 1 | EVQLVESGGGLVQPGGSLRLSCAASGFNIKDTYIHWVRQAPGKCLEWVARIYPTNGY TRYADSVKGRFTISADTSKNTAYLQMNSLRAEDTAVYYCSRWGGDGFYAMDYWGQGT LVTVSSASTKGPSVFPLAPSSKSTSGGTAALGCLVKDYFPEPVTVSWNSGALTSGVH TFPAVLQSSGLYSLSSVVTVPSSSLGTQTYICNVNHKPSNTKVDKKVEPKSCDKTHT CPPCPAPELLGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKA KGQPREPQVYTLPPCREEMTKNQVSLWCLVKGFYPSDIAVEWESNGQPENNYKTTPP VLDSDGSFFLYSKLTVDKSRWQQGNVFSCSVMHEALHNHYTQKSLSLSPGK | 14 |
| | Polype pt ide 2 | DIQMTQSPSSLSASVGDRVTITCRASQDVNTAVAWYQQKPGKAPKLLIYSASFLYSG VPSRFSGSRSGTDFTLTISSLQPEDFATYYCQQHYTTPPTFGCGTKVEIKRTVAAPS VFIFPPSDEQLKSGTASVVCLLNNFYPREAKVQWKVDNALQSGNSQESVTEQDSKDS | 15 |

| | | | |
|---|---|---|---|
| | | TYSLSSTLTLSKADYEKHKVYACEVTHQGLSSPVTKSFNRGECGGGGSGGGGSGGGG SEPKSSDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWESNG QPENNYKTTPPVLDSDGSFFLYSKLTVDKSRWQQGNVFSCSVMHEALHNHYTQKSLS LSPGK | |
| | Polype ptide 3 | KLVVVGAGGVGKSALTIGGDKTHTCPPCPAPELLGGPSVFLFPPKPKDTLMISRTPE VTCVVVDVSHEDPEVKFNWYVDGVEVHNAKTKPREEQYNSTYRVVSVLTVLHQDWLN GKEYKCKVSNKALPAPIEKTISKAKGQPREPQVCTLPPSREEMTKNQVSLSCAVKGF YPSDIAVEWESNGQPENNYKTTPPVLDSDGSFFLVSKLTVDKSRWQQGNVFSCSVMH EALHNHYTQKSLSLSPGK | 926 |
| | Polype ptide 4 | KLVVVGAGGVGKSALTIGGDKTHTCPPCPAPELLGGPSVFLFPPKPKDTLMISRTPE VTCVVVDVSHEDPEVKFNWYVDGVEVHNAKTKPREEQYNSTYRVVSVLTVLHQDWLN GKEYKCKVSNKALPAPIEKTISKAKGQPREPQVCTLPPSREEMTKNQVSLSCAVKGF YPSDIAVEWESNGQPENNYKTTPPVLDSDGSFFLVSKLTVDKSRWQQGNVFSCSVMH EALHNHYTQKSLSLSPGK | 926 |
| HAVCO3 | Polype ptide 1 | EVQLVESGGGLVQPGGSLRLSCAASGFNIKDTYIHWVRQAPGKCLEWVARIYPTNGY TRYADSVKGRFTISADTSKNTAYLQMNSLRAEDTAVYYCSRWGGDGFYAMDYWGQGT LVTVSSASTKGPSVFPLAPSSKSTSGGTAALGCLVKDYFPEPVTVSWNSGALTSGVH TFPAVLQSSGLYSLSSVVTVPSSSLGTQTYICNVNHKPSNTKVDKKVEPKSCDKTHT CPPCPAPELLGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKA KGQPREPQVYTLPPCREEMTKNQVSLWCLVKGFYPSDIAVEWESNGQPENNYKTTPP VLDSDGSFFLYSKLTVDKSRWQQGNVFSCSVMHEALHNHYTQKSLSLSPGK | 14 |
| | Polype ptide 2 | DIQMTQSPSSLSASVGDRVTITCRASQDVNTAVAWYQQKPGKAPKLLIYSASFLYSG VPSRFSGSRSGTDFTLTISSLQPEDFATYYCQQHYTTPPTFGCGTKVEIKRTVAAPS VFIFPPSDEQLKSGTASVVCLLNNFYPREAKVQWKVDNALQSGNSQESVTEQDSKDS TYSLSSTLTLSKADYEKHKVYACEVTHQGLSSPVTKSFNRGECGGGGSGGGGSGGGG SEPKSSDKTHTCPPCPAPELLGGPSVFLFPPKPKDTLMISRTPEVTCVVVDVSHEDP | 15 |

| | | | |
|---|---|---|---|
| | | EVKFNWYVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWESNG QPENNYKTTPPVLDSDGSFFLYSKLTVDKSRWQQGNVFSCSVMHEALHNHYTQKSLS LSPGK | |
| | Polypeptide 3 | KLVVVGADGVGKSALTIGGGGSDKTHTCPPCPAPELLGGPSVFLFPPKPKDTLMISR TPEVTCVVVDVSHEDPEVKFNWYVDGVEVHNAKTKPREEQYNSTYRVVSVLTVLHQD WLNGKEYKCKVSNKALPAPIEKTISKAKGQPREPQVCTLPPSREEMTKNQVSLSCAV KGFYPSDIAVEWESNGQPENNYKTTPPVLDSDGSFFLVSKLTVDKSRWQQGNVFSCS VMHEALHNHYTQKSLSLSPGK | 927 |
| | Polypeptide 4 | KLVVVGADGVGKSALTIGGGGSDKTHTCPPCPAPELLGGPSVFLFPPKPKDTLMISR TPEVTCVVVDVSHEDPEVKFNWYVDGVEVHNAKTKPREEQYNSTYRVVSVLTVLHQD WLNGKEYKCKVSNKALPAPIEKTISKAKGQPREPQVCTLPPSREEMTKNQVSLSCAV KGFYPSDIAVEWESNGQPENNYKTTPPVLDSDGSFFLVSKLTVDKSRWQQGNVFSCS VMHEALHNHYTQKSLSLSPGK | 927 |
| HAVCO4 | Polypeptide 1 | EVQLVESGGGLVQPGGSLRLSCAASGFNIKDTYIHWVRQAPGKCLEWVARIYPTNGY TRYADSVKGRFTISADTSKNTAYLQMNSLRAEDTAVYYCSRWGGDGFYAMDYWGQGT LVTVSSASTKGPSVFPLAPSSKSTSGGTAALGCLVKDYFPEPVTVSWNSGALTSGVH TFPAVLQSSGLYSLSSVVTVPSSSLGTQTYICNVNHKPSNTKVDKKVEPKSCDKTHT CPPCPAPELLGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKA KGQPREPQVYTLPPCREEMTKNQVSLWCLVKGFYPSDIAVEWESNGQPENNYKTTPP VLDSDGSFFLYSKLTVDKSRWQQGNVFSCSVMHEALHNHYTQKSLSLSPGK | 14 |
| | Polypeptide 2 | DIQMTQSPSSLSASVGDRVTITCRASQDVNTAVAWYQQKPGKAPKLLIYSASFLYSG VPSRFSGSRSGTDFTLTISSLQPEDFATYYCQQHYTTPPTFGCGTKVEIKRTVAAPS VFIFPPSDEQLKSGTASVVCLLNNFYPREAKVQWKVDNALQSGNSQESVTEQDSKDS TYSLSSTLTLSKADYEKHKVYACEVTHQGLSSPVTKSFNRGECGGGGSGGGGSGGGG SEPKSSDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWESNG | 15 |

| | | | |
|---|---|---|---|
| | | QPENNYKTTPPVLDSDGSFFLYSKLTVDKSRWQQGNVFSCSVMHEALHNHYTQKSLSLSPGK | |
| | Polypeptide 3 | KLVVVGADGVGKSALTIGGDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVCTLPPSREEMTKNQVSLSCAVKGFYPSDIAVEWESNGQPENNYKTTPPVLDSDGSFFLVSKLTVDKSRWQQGNVFSCSVMHEALHNHYTQKSLSLSPGK | 928 |
| | Polypeptide 4 | KLVVVGADGVGKSALTIGGDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVCTLPPSREEMTKNQVSLSCAVKGFYPSDIAVEWESNGQPENNYKTTPPVLDSDGSFFLVSKLTVDKSRWQQGNVFSCSVMHEALHNHYTQKSLSLSPGK | 928 |
| | Polypeptide 1 | EVQLVESGGGLVQPGGSLRLSCAASGFNIKDTYIHWVRQAPGKCLEWVARIYPTNGYTRYADSVKGRFTISADTSKNTAYLQMNSLRAEDTAVYYCSRWGGDGFYAMDYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 14 |
| HAVC05 | Polypeptide 2 | DIQMTQSPSSLSASVGDRVTITCRASQDVNTAVAWYQQKPGKAPKLLIYSASFLYSGVPSRFSGSRSGTDFTLTISSLQPEDFATYYCQQHYTTPPTFGCGTKVEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGECGGGGSGGGGSGGGGSEPKSSDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALHNHYTQKSLSLSPGK | 15 |

| | Polype ptide 3 | KLVVVGAGGVGKSALTIGGGGSKLVVVGAGGVGKSALTIGGGGSDKTHTCPPCPAPE LLGGPSVFLFPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISKAKGQPREPQ VCTLPPSREEMTKNQVSLSCAVKGFYPSDIAVEWESNGQPENNYKTTPPVLDSDGSF FLVSKLTVDKSRWQQGNVFSCSVMHEALHNHYTQKSLSLSPGK | 929 |
|---|---|---|---|
| | Polype ptide 4 | KLVVVGAGGVGKSALTIGGGGSKLVVVGAGGVGKSALTIGGGGSDKTHTCPPCPAPE LLGGPSVFLFPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISKAKGQPREPQ VCTLPPSREEMTKNQVSLSCAVKGFYPSDIAVEWESNGQPENNYKTTPPVLDSDGSF FLVSKLTVDKSRWQQGNVFSCSVMHEALHNHYTQKSLSLSPGK | 929 |
| HAVCO6 | Polype ptide 1 | EVQLVESGGGLVQPGGSLRLSCAASGFNIKDTYIHWVRQAPGKCLEWVARIYPTNGY TRYADSVKGRFTISADTSKNTAYLQMNSLRAEDTAVYYCSRWGGDGFYAMDYWGQGT LVTVSSASTKGPSVFPLAPSSKSTSGGTAALGCLVKDYFPEPVTVSWNSGALTSGVH TFPAVLQSSGLYSLSSVVTVPSSSLGTQTYICNVNHKPSNTKVDKKVEPKSCDKTHT CPPCPAPELLGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKA KGQPREPQVYTLPPCREEMTKNQVSLWCLVKGFYPSDIAVEWESNGQPENNYKTTPP VLDSDGSFFLYSKLTVDKSRWQQGNVFSCSVMHEALHNHYTQKSLSLSPGK | 14 |
| | Polype ptide 2 | DIQMTQSPSSLSASVGDRVTITCRASQDVNTAVAWYQQKPGKAPKLLIYSASFLYSG VPSRFSGSRSGTDFTLTISSLQPEDFATYYCQQHYTTPPTFGCGTKVEIKRTVAAPS VFIFPPSDEQLKSGTASVVCLLNNFYPREAKVQWKVDNALQSGNSQESVTEQDSKDS TYSLSSTLTLSKADYEKHKVYACEVTHQGLSSPVTKSFNRGECGGGGSGGGGSGGGG SEPKSSDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWESNG QPENNYKTTPPVLDSDGSFFLYSKLTVDKSRWQQGNVFSCSVMHEALHNHYTQKSLS LSPGK | 15 |
| | Polype ptide 3 | KLVVVGAGGVGKSALTIGGKLVVVGAGGVGKSALTIGGDKTHTCPPCPAPELLGGPS VFLFPPKPKDTLMISRTPEVTCVVVDVSHEDPEVKFNWYVDGVEVHNAKTKPREEQY | 930 |

| | | | |
|---|---|---|---|
| | | NSTYRVVSVLTVLHQDWLNGKEYKCKVSNKALPAPIEKTISKAKGQPREPQVCTLPP SREEMTKNQVSLSCAVKGFYPSDIAVEWESNGQPENNYKTTPPVLDSDGSFFLVSKL TVDKSRWQQGNVFSCSVMHEALHNHYTQKSLSLSPGK | |
| | Polypeptide 4 | KLVVVGAGGVGKSALTIGGKLVVVGAGGVGKSALTIGGDKTHTCPPCPAPELLGGPS VFLFPPKPKDTLMISRTPEVTCVVVDVSHEDPEVKFNWYVDGVEVHNAKTKPREEQY NSTYRVVSVLTVLHQDWLNGKEYKCKVSNKALPAPIEKTISKAKGQPREPQVCTLPP SREEMTKNQVSLSCAVKGFYPSDIAVEWESNGQPENNYKTTPPVLDSDGSFFLVSKL TVDKSRWQQGNVFSCSVMHEALHNHYTQKSLSLSPGK | 930 |
| HAVCO7 | Polypeptide 1 | EVQLVESGGGLVQPGGSLRLSCAASGFNIKDTYIHWVRQAPGKCLEWVARIYPTNGY TRYADSVKGRFTISADTSKNTAYLQMNSLRAEDTAVYYCSRWGGDGFYAMDYWGQGT LVTVSSASTKGPSVFPLAPSSKSTSGGTAALGCLVKDYFPEPVTVSWNSGALTSGVH TFPAVLQSSGLYSLSSVVTVPSSSLGTQTYICNVNHKPSNTKVDKKVEPKSCDKTHT CPPCPAPELLGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKA KGQPREPQVYTLPPCREEMTKNQVSLWCLVKGFYPSDIAVEWESNGQPENNYKTTPP VLDSDGSFFLYSKLTVDKSRWQQGNVFSCSVMHEALHNHYTQKSLSLSPGK | 14 |
| | Polypeptide 2 | DIQMTQSPSSLSASVGDRVTITCRASQDVNTAVAWYQQKPGKAPKLLIYSASFLYSG VPSRFSGSRSGTDFTLTISSLQPEDFATYYCQQHYTTPPTFGCGTKVEIKRTVAAPS VFIFPPSDEQLKSGTASVVCLLNNFYPREAKVQWKVDNALQSGNSQESVTEQDSKDS TYSLSSTLTLSKADYEKHKVYACEVTHQGLSSPVTKSFNRGECGGGGSGGGGSGGGG SEPKSSDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWESNG QPENNYKTTPPVLDSDGSFFLYSKLTVDKSRWQQGNVFSCSVMHEALHNHYTQKSLS LSPGK | 15 |
| | Polypeptide 3 | KLVVVGADGVGKSALTIGGGGSKLVVVGADGVGKSALTIGGGGSDKTHTCPPCPAPE LLGGPSVFLFPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISKAKGQPREPQ VCTLPPSREEMTKNQVSLSCAVKGFYPSDIAVEWESNGQPENNYKTTPPVLDSDGSF | 931 |

| | | | |
|---|---|---|---|
| | | FLVSKLTVDKSRWQQGNVFSCSVMHEALHNHYTQKSLSLSPGK | |
| | Polype ptide 4 | KLVVVGADGVGKSALTIGGGGSKLVVVGADGVGKSALTIGGGGSDKTHTCPPCPAPE LLGGPSVFLFPPKPKDTLMISRTPEVTCVVVDVSHEDPEVKFNWYVDGVEVHNAKTK PREEQYNSTYRVVSVLTVLHQDWLNGKEYKCKVSNKALPAPIEKTISKAKGQPREPQ VCTLPPSREEMTKNQVSLSCAVKGFYPSDIAVEWESNGQPENNYKTTPPVLDSDGSF FLVSKLTVDKSRWQQGNVFSCSVMHEALHNHYTQKSLSLSPGK | 931 |
| HAVCO8 | Polype ptide 1 | EVQLVESGGGLVQPGGSLRLSCAASGFNIKDTYIHWVRQAPGKCLEWVARIYPTNGY TRYADSVKGRFTISADTSKNTAYLQMNSLRAEDTAVYYCSRWGGDGFYAMDYWGQGT LVTVSSASTKGPSVFPLAPSSKSTSGGTAALGCLVKDYFPEPVTVSWNSGALTSGVH TFPAVLQSSGLYSLSSVVTVPSSSLGTQTYICNVNHKPSNTKVDKKVEPKSCDKTHT CPPCPAPELLGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKA KGQPREPQVYTLPPCREEMTKNQVSLWCLVKGFYPSDIAVEWESNGQPENNYKTTPP VLDSDGSFFLYSKLTVDKSRWQQGNVFSCSVMHEALHNHYTQKSLSLSPGK | 14 |
| | Polype ptide 2 | DIQMTQSPSSLSASVGDRVTITCRASQDVNTAVAWYQQKPGKAPKLLIYSASFLYSG VPSRFSGSRSGTDFTLTISSLQPEDFATYYCQQHYTTPPTFGCGTKVEIKRTVAAPS VFIFPPSDEQLKSGTASVVCLLNNFYPREAKVQWKVDNALQSGNSQESVTEQDSKDS TYSLSSTLTLSKADYEKHKVYACEVTHQGLSSPVTKSFNRGECGGGGSGGGGSGGGG SEPKSSDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCKVSNKAL PAPIEKTISKAKGQPREPQVYTLPPCREEMTKNQVSLWCLVKGFYPSDIAVEWESNG QPENNYKTTPPVLDSDGSFFLYSKLTVDKSRWQQGNVFSCSVMHEALHNHYTQKSLS LSPGK | 15 |
| | Polype ptide 3 | KLVVVGADGVGKSALTIGGKLVVVGADGVGKSALTIGGDKTHTCPPCPAPELLGGPS VFLFPPKPKDTLMISRTPEVTCVVVDVSHEDPEVKFNWYVDGVEVHNAKTKPREEQY NSTYRVVSVLTVLHQDWLNGKEYKCKVSNKALPAPIEKTISKAKGQPREPQVCTLPP SREEMTKNQVSLSCAVKGFYPSDIAVEWESNGQPENNYKTTPPVLDSDGSFFLVSKL TVDKSRWQQGNVFSCSVMHEALHNHYTQKSLSLSPGK | 932 |
| | Polype | KLVVVGADGVGKSALTIGGKLVVVGADGVGKSALTIGGDKTHTCPPCPAPELLGGPS | 932 |

| | | | |
|---|---|---|---|
| | ptide 4 | VFLFPPKPKDTLMISRTPEVTCVVVDVSHEDPEVKFNWYVDGVEVHNAKTKPREEQY NSTYRVVSVLTVLHQDWLNGKEYKCKVSNKALPAPIEKTISKAKGQPREPQVCTLPP SREEMTKNQVSLSCAVKGFYPSDIAVEWESNGQPENNYKTTPPVLDSDGSFFLVSKL TVDKSRWQQGNVFSCSVMHEALHNHYTQKSLSLSPGK | |

Table 102 below shows the results obtained by purity analysis of HAVCO1, HAVC02, HAVC03, HAVC04, HAVC05, HAVC06, HAVC07, and HAVC08, which are novel engineered antibody formats conjugated with peptide vaccine.

[Table 102]

| Name | Purity (%) | Retention Time (min) |
|---|---|---|
| HAVC01 | 83.11 | 3.767 |
| HAVC02 | 81.25 | 3.768 |
| HAVC03 | 88.00 | 3.746 |
| HAVC04 | 91.18 | 3.803 |
| HAVC05 | 79.95 | 3.743 |
| HAVC06 | 76.43 | 3.760 |
| HAVC07 | 86.37 | 3.708 |
| HAVC08 | 86.44 | 3.750 |

**Claims**

1. A fusion protein comprising:

   (a) an antigen binding site consisting of a first polypeptide comprising at least one complementarity-determining region (CDR) sequence and a second polypeptide comprising at least one complementarity-determining region (CDR) sequence, wherein the first polypeptide and the second polypeptide form a dimer and the antigen binding site is capable of specifically binding to a target antigen,
   (b) a first Fc domain or a variant thereof that is a dimer consisting of two polypeptide sequences, one of which is joined to the first polypeptide of the antigen binding site, and
   (c) a second Fc domain or a variant thereof that is a dimer consisting of two polypeptide sequences, one of which is joined to the second polypeptide of the antigen binding site.

2. The fusion protein according to claim 1, wherein the first polypeptide of the antigen binding site comprises CDR1, CDR2, and CDR3 of an antibody heavy chain, and the second polypeptide of the antigen binding site comprises CDR1, CDR2, and CDR3 of an antibody light chain.

3. The fusion protein according to claim 2, wherein the first polypeptide of the antigen binding site further comprises a CH1 region of an antibody heavy chain, and/or the second polypeptide of the antigen binding site further comprises a constant region of an antibody light chain.

4. The fusion protein according to claim 1, wherein the antigen binding site specifically binds to a protein expressed on the cell surface.

5. The fusion protein according to claim 1, wherein the antigen binding site specifically binds to any one selected from the group consisting of PD-L1, EGFR, EGFRvIII, BCMA, CD22, CD25, CD30, CD33, CD37, CD38, CD52, CD56, CD123, c-Met, DLL3, DR4, DR5, GD2, nectin-4, RANKL, SLAMF7, Trop-2, LIV-1, claudin 18.2, IL13$\alpha$2, CD3, HER2, HER3, FGFR2, FGFR3, GPC3, ROR1, Fol$\alpha$, CD20, CD19, CTLA-4, VEGFR, NCAM1, ICAM-1, ICAM-2, CEACAM5, CEACAM6, carcinoembryonic antigen (CEA), CA-125, alphafetoprotein (AFP), MUC-1, MUC-16, PSMA, PSCA, epithelial tumor antigen (ETA), melanoma-associated antigen (MAGE), immature laminin receptor, TAG-72, HPV E6/E7, BING-4, calcium-activated chloride channel 2, cyclin-B1, 9D7, Ep-CAM, EphA2, EphA3, mesothelin, SAP-1, survivin, and virus-derived antigens.

6. The fusion protein according to claim 1, wherein the fusion protein further comprises a Z protein.

7. The fusion protein according to claim 6, wherein the Z protein binds to the N-terminus of the first Fc domain and the second Fc domain.

8. The fusion protein according to claim 6, wherein the Z protein is bound to the first Fc domain and the second Fc domain through a peptide linker.

9. The fusion protein according to claim 8, wherein the peptide linker comprises a hinge.

10. The fusion protein according to claim 6, wherein the Z protein is any one selected from the group consisting of a receptor, a soluble protein, a cytokine, a single-chain Fv fragment (single-chain fragment variable; scFv), an antibody mimetics, a single domain antibody (sdAb), a therapeutic peptide, and a peptide vaccine.

11. The fusion protein according to claim 1, wherein the fusion protein comprises polypeptides of the following structural formulas (I), (II), (III), and (IV):

$$N'-X-(L1)n-A-C' \qquad (I);$$

$$N'-Y-(L2)m-B-C' \qquad (II);$$

$$N'-(Z1)r-(L5)s-C-C' \qquad (III);$$

and

$$N'-(Z2)t-(L6)u-D-C' \qquad (IV)$$

wherein, in the structural formulas (I), (II), (III), and (IV),
N' is the N-terminus of each polypeptide,
C' is the C-terminus of each polypeptide,

   - refers to a linkage,

A, B, C, and D are monomeric polypeptide sequences of an Fc domain each comprising the CH2 and CH3 regions of an immunoglobulin, and optionally further comprising CH4 and/or a hinge sequence, wherein A forms a dimer with one of C or D to form the first Fc domain (b), and B forms a dimer with the remaining one of C or D to form the second Fc domain (c);
L1, L2, L5, and L6 are each peptide linkers,
n, m, r, s, t, and u are each independently 0 or 1,
X is a first polypeptide sequence of the antigen binding site, which comprises heavy chain CDR1, CDR2, CDR3 sequences of an antibody that specifically binds to a first antigen, or a heavy chain variable region of an antibody that specifically binds to a first antigen;
Y is a second polypeptide sequence of the antigen binding site, which comprises light chain CDR1, CDR2, CDR3 sequences of an antibody that specifically binds to a first antigen, or a light chain variable region of an antibody that specifically binds to a first antigen; and
X and Y pair with each other to form the antigen binding site (a) that specifically binds to an antigen, and
Z1 and Z2 are each independently any one selected from the group consisting of a receptor, a soluble protein, a cytokine, a single-chain Fv fragment (single-chain fragment variable; scFv), an antibody mimetics, a single domain antibody (sdAb), a therapeutic peptide, and a peptide vaccine.

12. The fusion protein according to claim 11, wherein

X in the structural formula (I) further comprises a heavy chain CH1 region, and/or
Y in the structural formula (II) further comprises a light chain constant region.

13. The fusion protein according to claim 1, wherein the fusion protein comprises polypeptides of the following structural formulas (I'), (II'), (III), and (IV):

$$N'-VD1-(L3)p-X-(L1)n-A-C' \qquad (I');$$

N'-VD2-(L4)q-Y-(L2)m-B-C'     (II');

N'-(Z1)r-(L5)s-C-C'     (III);

and

N'-(Z2)t-(L6)u-D-C'     (IV)

wherein, in the structural formulas (I'), (II'), (III), and (IV),
N' is the N-terminus of each polypeptide,
C' is the C-terminus of each polypeptide,

- refers to a linkage,

A, B, C, and D are monomeric polypeptide sequences of an Fc domain each comprising the CH2 and CH3 regions of an immunoglobulin, and optionally further comprising CH4 and/or a hinge sequence, wherein A forms a dimer with one of C or D to form the first Fc domain (b), and B forms a dimer with the remaining one of C or D to form the second Fc domain (c);
L1, L2, L3, L4, L5, and L6 are each peptide linkers,
n, m, p, q, r, s, t, and u are each 0 or 1,
VD1 consists of a heavy chain or light chain variable region of an antibody or CDR1, CDR2, and CDR3 of an antibody heavy chain or light chain that specifically binds to an antigen;
VD2 consists of a light chain or heavy chain variable region of an antibody or CDR1, CDR2, and CDR3 of an antibody heavy chain or light chain that specifically binds to an antigen;
VD1 and VD2 pair with each other to form a second antibody variable region that specifically binds to a second antigen,
X comprises a heavy chain or light chain variable region of an antibody or CDR1, CDR2, and CDR3 of an antibody heavy chain or light chain that specifically binds to an antigen;
Y comprises a light chain or heavy chain variable region of an antibody or CDR1, CDR2, and CDR3 of an antibody heavy chain or light chain that specifically binds to an antigen; and
X and Y pair with each other to form a first antibody variable region that specifically binds to a first antigen,
VD1-(L3)p-X forms a first polypeptide sequence of the antigen binding site (a), and VD2-(L4)q-Y forms a second polypeptide sequence of the antigen binding site (a), and
Z1 and Z2 are each independently any one selected from the group consisting of a receptor, a soluble protein, a cytokine, a single-chain Fv fragment (single-chain fragment variable; scFv), an antibody mimetics, a single domain antibody (sdAb), a therapeutic peptide, and a peptide vaccine.

14. The fusion protein according to claim 13, wherein

the heavy chain variable region further comprises a heavy chain CH1 region, and
the light chain variable region further comprises a light chain constant region.

15. The fusion protein according to claim 11 or 13, wherein

the Fc domain monomer comprises a knob variant or a hole variant that promotes the formation of an Fc heterodimer (heterodimeric Fc); or
the Fc domain monomer comprises a variant that promotes the formation of a heterodimer by electrostatic steering mechanism.

16. The fusion protein according to claim 12 or 14, wherein the binding between X and Y is achieved

i) through a disulfide bond formed by Cys present in CH1 and a light chain constant region,
ii) through a disulfide bond formed by Cys present in a heavy chain variable region and a light chain variable region, or
iii) through a disulfide bond formed by Cys present in CH1 and a light chain constant region, and a disulfide bond formed by Cys present in a heavy chain variable region and a light chain variable region.

17. The fusion protein according to claim 14, wherein the binding between X and Y further comprises, in addition to a disulfide bond present between $CH_1233$ and $CL214$ based on Kabat numbering,

    i) a disulfide bond present between VH105 and VL43;
    ii) a disulfide bond present between VH44 and VL100; or
    iii) a disulfide bond present between $CH_1122$ and $CL121$.

18. A pharmaceutical composition for preventing or treating cancer, comprising the fusion protein according to any one of claims 1 to 17 as an active ingredient.

19. The pharmaceutical composition according to claim 18, wherein the cancer is any one selected from the group consisting of gastric cancer, liver cancer, lung cancer, large intestine cancer, breast cancer, prostate cancer, gallbladder cancer, bladder cancer, kidney cancer, esophageal cancer, skin cancer, rectal cancer, osteosarcoma, multiple myeloma, glioma, ovarian cancer, pancreatic cancer, cervical cancer, endometrial cancer, thyroid cancer, laryngeal cancer, testicular cancer, mesothelioma, acute myeloid leukemia, chronic myeloid leukemia, acute lymphoblastic leukemia, chronic lymphoblastic leukemia, brain tumor, neuroblastoma, retinoblastoma, head and neck cancer, salivary gland cancer, and lymphoma.

20. A transformed cell expressing the fusion protein according to any one of claims 1 to 17.

21. A method for preventing or treating cancer, comprising administering the fusion protein according to claim 1 to a subject.

22. A use of the fusion protein according to claim 1.

23. A use of the fusion protein according to claim 1 for use in the manufacture of a medicament for treating cancer.

EP 4 700 047 A1
Drawings

**Figure 1a**

**Figure 1b**

404

**Figure 1c**

**Figure 1d**

**Figure 1e**

**Figure 1f**

**Figure 2a**

Wild type (WT)

peptide linker

**Figure 2b**

M1
VH 105(Kabat): Q → C
VL 43(Kabat): A → C

peptide linker

**Figure 2c**

**M2**
**CH1 122(Kabat): F → C**
**CL 121(Kabat): S → C**

**Figure 2d**

**M3**
**VH 44(Kabat): G → C**
**VL 100(Kabat): Q → C**

Figure 3a

EP 4 700 047 A1

```
Kabat                                                    44
                                                          |
WT       EVQLVESGGGLVQPGGSLRLSCAASGFNIKDTYIHWVRQAPGKGLEWVARIYPTNGYTRYADSVKGRFTISADTSKNTAYLQMNSLRAEDTAVYYCSRWGGDGFYAMDY
Mutant1  EVQLVESGGGLVQPGGSLRLSCAASGFNIKDTYIHWVRQAPGKGLEWVARIYPTNGYTRYADSVKGRFTISADTSKNTAYLQMNSLRAEDTAVYYCSRWGGDGFYAMDY
Mutant2  EVQLVESGGGLVQPGGSLRLSCAASGFNIKDTYIHWVRQAPGKGLEWVARIYPTNGYTRYADSVKGRFTISADTSKNTAYLQMNSLRAEDTAVYYCSRWGGDGFYAMDY
Mutant3  EVQLVESGGGLVQPGGSLRLSCAASGFNIKDTYIHWVRQAPGKCLEWVARIYPTNGYTRYADSVKGRFTISADTSKNTAYLQMNSLRAEDTAVYYCSRWGGDGFYAMDY
         ********************************************  **********************************************************************

Kabat        105              122
              |                |
WT       WGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKV
Mutant1  WGCGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKV
Mutant2  WGQGTLVTVSSASTKGPSVCPLAPSSKSTSGGTAALGCLVKDYFPEPVTVSWNSGALTSGVHTFPAVLQSSGLYSLSSVVTVPSSSLGTQTYICNVNHKPSNTKVDKKV
Mutant3  WGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKV
         **  *************** ****************************************************************************************

WT       EPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVS
Mutant1  EPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVS
Mutant2  EPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVS
Mutant3  EPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVS
         ********************************************************************************************************

WT       NKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALH
Mutant1  NKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALH
Mutant2  NKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALH
Mutant3  NKALPAPIEKTISKAKGQPREPQVYTLPPCREEMTKNQVSLWCLVKGFYPSDIAVEWESNGQPENNYKTTPPVLDSDGSFFLYSKLTVDKSRWQQGNVFSCSVMHEALH
         ********************************************************************************************************

WT       NHYTQKSLSLSPGK  (SEQ ID NO:8)
Mutant1  NHYTQKSLSLSPGK  (SEQ ID NO:10)
Mutant2  NHYTQKSLSLSPGK  (SEQ ID NO:12)
Mutant3  NHYTQKSLSLSPGK  (SEQ ID NO:14)
         **************
```

**Figure 3b**

```
Kabat                                           43                                                                         100
                                                 |                                                                          |
WT       DIQMTQSPSSLSASVGDRVTITCRASQDVNTAVAWYQQKPGKAPKLLIYSASFLYSGVPSRFSGSRSGTDFTLTISSLQPEDFATYYCQQHYTTPPTFGQGTKVEIK
Mutant1  DIQMTQSPSSLSASVGDRVTITCRASQDVNTAVAWYQQKPGKCPKLLIYSASFLYSGVPSRFSGSRSGTDFTLTISSLQPEDFATYYCQQHYTTPPTFGQGTKVEIK
Mutant2  DIQMTQSPSSLSASVGDRVTITCRASQDVNTAVAWYQQKPGKAPKLLIYSASFLYSGVPSRFSGSRSGTDFTLTISSLQPEDFATYYCQQHYTTPPTFGQGTKVEIK
Mutant3  DIQMTQSPSSLSASVGDRVTITCRASQDVNTAVAWYQQKPGKAPKLLIYSASFLYSGVPSRFSGSRSGTDFTLTISSLQPEDFATYYCQQHYTTPPTFGCGTKVEIK
         *********************************************.****************************************************** *******

Kabat           121
                 |
WT       RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC
Mutant1  RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC
Mutant2  RTVAAPSVFIFPPCDEQLKSGTASVVCLLNNFYPREAKVQWKVDNALQSGNSQESVTEQDSKDSTYSLSSTLTLSKADYEKHKVYACEVTHQGLSSPVTKSFNRGEC
Mutant3  RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC
         *************.******************************************************************************************

WT       GGGGSGGGGSGGGGSEPKSSDKTHTCPPCPAPELLGGPSVFLFPPKPKDTLMISRTPEVTCVVVDVSHEDPEVKFNWYVDGVEVHNAKTKPREEQYNSTYRVVSVLT
Mutant1  GGGGSGGGGSGGGGSEPKSSDKTHTCPPCPAPELLGGPSVFLFPPKPKDTLMISRTPEVTCVVVDVSHEDPEVKFNWYVDGVEVHNAKTKPREEQYNSTYRVVSVLT
Mutant2  GGGGSGGGGSGGGGSEPKSSDKTHTCPPCPAPELLGGPSVFLFPPKPKDTLMISRTPEVTCVVVDVSHEDPEVKFNWYVDGVEVHNAKTKPREEQYNSTYRVVSVLT
Mutant3  GGGGSGGGGSGGGGSEPKSSDKTHTCPPCPAPELLGGPSVFLFPPKPKDTLMISRTPEVTCVVVDVSHEDPEVKFNWYVDGVEVHNAKTKPREEQYNSTYRVVSVLT
         *******************************************************************************************************

WT       VLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDK
Mutant1  VLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDK
Mutant2  VLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDK
Mutant3  VLHQDWLNGKEYKCKVSNKALPAPIEKTISKAKGQPREPQVYTLPPCREEMTKNQVSLWCLVKGFYPSDIAVEWESNGQPENNYKTTPPVLDSDGSFFLYSKLTVDK
         *********************************************************************************************************

WT       SRWQQGNVFSCSVMHEALHNHYTQKSLSLSPGK    (SEQ ID NO:9)
Mutant1  SRWQQGNVFSCSVMHEALHNHYTQKSLSLSPGK    (SEQ ID NO:11)
Mutant2  SRWQQGNVFSCSVMHEALHNHYTQKSLSLSPGK    (SEQ ID NO:13)
Mutant3  SRWQQGNVFSCSVMHEALHNHYTQKSLSLSPGK    (SEQ ID NO:15)
         *********************************
```

**Figure 4**

**Figure 5a**

**Figure 5b**

**Figure 5c**

**Figure 5d**

**Figure 6a**

Mutant 3 (M3)
VH 44C, VL 100C
Linker length: 15-mer

**Figure 6b**

Variant 1 (V1)
VH 44C, VL 100C
Linker length: 10-mer

**Figure 6c**

Variant 2 (V2)
VH 44C, VL 100C
Linker length: 5-mer

5-mer
peptide linker

**Figure 6d**

Variant 3 (V3)
VH 44C, VL 100C
Linker length: 0-mer

No
peptide linker

**Figure 7**

**Figure 8a**

**Figure 8b**

Byproduct(H01 or P01): ~150 kDa

Papain →

Fab: ~49.3 kDa + (Fc)2 : ~100.7 kDa

**Figure 8c**

non-reducing condition

**Figure 8d**

non-reducing condition

**Figure 9**

**Figure 10a**

**Figure 10b**

**Figure 10c**

H01Fv3

H435R, Y436F

**Figure 10d**

H01Fv4

H435R, Y436F

**Figure 10e**

H01Fv5

H435R, Y436F

**Figure 10f**

H01Fv6

H435R, Y436F

**Figure 10g**

**Figure 10h**

| Name | Mutation site (Kabat numbering) | | MW (kDa) |
|---|---|---|---|
| | VH | VL | |
| H01Fv1 | G44C | Q100C | 128.21 |
| H01Fv2 | Q105C | A43C | 128.19 |
| H01Fv3 | F122C | S121C | 130.98 |
| H01Fv4 | G44C, Q105C | Q100C, A43C | 130.01 |
| H01Fv5 | G44C, F122C | Q100C, S121C | 131.00 |
| H01Fv6 | Q105C, F126C | A43C, S121C | 130.99 |
| H01Fv7 | G44C, Q105C, F126C | Q100C, A43C, S121C | 131.01 |

**Figure 11**

**Figure 12a**

H01Fv3
Purity: 14.66%

9.510

8.776

7.777

Minutes

AU

**Figure 12b**

**Figure 12c**

**Figure 12d**

**Figure 12e**

**Figure 12f**

**Figure 12g**

**Figure 13a**

H01Fv1

**Figure 13b**

H01Fv2

**Figure 13c**

H01Fv4

**Figure 13d**

H01Fv5

**Figure 13e**

H01Fv6

**Figure 13f**

H01Fv7

**Figure 14**

# Figure 15

Figure 15

**Figure 16a**

**Figure 16b**

**Figure 17a**

NCIN87
Fc counting (anti-hIgG Fcγ, Fab)

**Figure 17b**

BT474
Fc counting (anti-hIgG Fcγ, Fab)

**Figure 17c**

SKOV3
Fc counting (anti-hIgG Fcγ, Fab)

Figure 17d

SNU1
Fc counting (anti-hIgG Fcγ, Fab)

**Figure 17e**

SNU5
Fc counting (anti-hIgG Fcγ, Fab)

**Figure 18a**

NCIN87
Fc counting (anti-hIgG Fcγ, Fab)

**Figure 18b**

BT474
Fc counting (anti-hIgG Fcγ, Fab)

**Figure 18c**

SKOV3
Fc counting (anti-hIgG Fcγ, Fab)

**Figure 18d**

SNU1
Fc counting (anti-hIgG Fcγ, Fab)

**Figure 18e**

SNU5
Fc counting (anti-hIgG Fcγ, Fab)

**Figure 19a**

**Figure 19b**

**Figure 20a**

**Figure 20b**

H01DE4 (HER2-ECD)

$K_D = 144.67$ pM
$K_a = 2.58 \times 10^5$ 1/Ms
$K_d = 3.73 \times 10^{-5}$ 1/s

**Figure 20c**

P01 (HER2-ECD)

$K_D = 338.46$ pM
$K_a = 2.72 \times 10^5$ 1/Ms
$K_d = 9.20 \times 10^{-5}$ 1/s

**Figure 20d**

P01DE4 (HER2-ECD)

$K_D = 223.78$ pM
$K_a = 2.46 \times 10^5$ 1/Ms
$K_d = 5.51 \times 10^{-5}$ 1/s

**Figure 21a**

H01 (FcγRI)

$K_D = 0.054$ nM
$K_a = 8.67 \times 10^5$ 1/Ms
$K_d = 4.70 \times 10^{-5}$ 1/s

**Figure 21b**

**Figure 21c**

**Figure 21d**

P01DE4 (FcγRI)

$K_D = 0.038$ nM
$K_a = 7.48 \times 10^5$ 1/Ms
$K_d = 2.85 \times 10^{-5}$ 1/s

16 nM
8 nM
4 nM
2 nM
1 nM

**Figure 21e**

Human IgG1 (FcγRI)

$K_D = 2.46$ nM
$K_a = 3.59 \times 10^5$ 1/Ms
$K_d = 8.82 \times 10^{-4}$ 1/s

64 nM
32 nM
16 nM
8 nM
4 nM
2 nM

**Figure 21f**

Trastuzumab (FcγRI)

$K_D = 2.34$ nM
$K_a = 3.33 \times 10^5$ 1/Ms
$K_d = 7.80 \times 10^{-4}$ 1/s

**Figure 21g**

Pertuzumab (FcγRI)

$K_D = 3.04$ nM
$K_a = 3.32 \times 10^5$ 1/Ms
$K_d = 1.01 \times 10^{-3}$ 1/s

**Figure 21h**

**Figure 22a**

**Figure 22b**

**Figure 22c**

**Figure 22d**

**Figure 22e**

**Figure 22f**

Trastuzumab (FcγRIIA)

$K_D = 151.28$ nM
$K_a = 7.97 \times 10^4$ 1/Ms
$K_d = 1.21 \times 10^{-2}$ 1/s

1280 nM
640 nM
320 nM
160 nM
80 nM
40 nM

**Figure 22g**

Pertuzumab (FcγRIIA)

$K_D = 359.33$ nM
$K_a = 7.27 \times 10^4$ 1/Ms
$K_d = 2.61 \times 10^{-2}$ 1/s

Analytes
40 - 1280 nM

**Figure 22h**

**Figure 23a**

**Figure 23b**

P01 (FcγRIIIA)

$K_D$ = 2.37 nM
$K_a$ = 6.36 × 10^5 1/Ms
$K_d$ = 1.51 × 10^{-3} 1/s

80 nM
40 nM
20 nM
10 nM
5 nM

**Figure 23c**

H01DE4 (FcγRIIIA)

$K_D$ = 0.11 nM
$K_a$ = 5.26 × 10^5 1/Ms
$K_d$ = 5.51 × 10^{-5} 1/s

80 nM
40 nM
20 nM
10 nM
5 nM

**Figure 23d**

**Figure 23e**

**Figure 23f**

**Figure 23g**

**Figure 23h**

**Figure 24a**

Pharmacokinetic analysis (SD Rat, IV, n = 3/group)

- 10 mg/kg Trastuzumab
- 10 mg/kg Pertuzumab
- 10 mg/kg H01
- 10 mg/kg P01

Table 1. Pharmacokinetic parameters after intravenous administration of test antibodies in SD rats (n=3)

| Test antibody | Trastuzumab | Pertuzumab | H01 | P01 |
|---|---|---|---|---|
| Concentration(mg/kg), Route | 10, IV | 10, IV | 10, IV | 10, IV |
| $T_{1/2}$ (day) | 7.29 ± 4.33 | 11.6 ± 2.2 | 11.8 ± 1.9 | 14.2 ± 3.7 |
| $AUC_{last}$ (ug·day/mL) | 1802 ± 320 | 1822 ± 172 | 950 ± 22 | 1206 ± 73 |
| $AUC_{inf}$ (ug·day/mL) | 1875 ± 295 | 1968 ± 250 | 1018 ± 30 | 1316 ± 47 |
| Vz_obs (mL/kg) | 59.1 ± 37.7 | 84.8 ± 8.9 | 167 ± 22 | 156 ± 43 |
| Cl_obs (mL/day/kg) | 5.43 ± 0.92 | 5.14 ± 0.68 | 9.83 ± 0.29 | 7.60 ± 0.28 |
| $MRT_{inf}$ (day) | 11.6 ± 3.8 | 14.9 ± 2.4 | 14.8 ± 1.1 | 15.7 ± 2.3 |

Figure 24b

EP 4 700 047 A1

**Figure 25**

EP 4 700 047 A1

**Figure 26**

470

**Figure 27**

**Figure 28a**

Replicate Group: HP503

**Figure 28b**

Replicate Group: HP507

**Figure 28c**

Replicate Group: HP511

**Figure 28d**

Replicate Group: HP515

**Figure 29a**

HP503(FcγRI)

$K_D = 0.22$ nM
$K_a = 3.46 \times 10^5$ 1/Ms
$K_d = 7.67 \times 10^{-5}$ 1/s

64 nM
32 nM
16 nM
8 nM
4 nM
2 nM

HP503(FcγRIIA)

$K_D = 3.69$ nM
$K_a = 1.58 \times 10^6$ 1/Ms
$K_d = 5.81 \times 10^{-3}$ 1/s

320 nM
160 nM
80 nM
40 nM
20 nM
10 nM

HP503(FcγRIIIA)

$K_D = 2.42$ nM
$K_a = 6.60 \times 10^5$ 1/Ms
$K_d = 1.60 \times 10^{-3}$ 1/s

320 nM
160 nM
80 nM
40 nM
20 nM
10 nM

**Figure 29b**

**Figure 29c**

HP511(FcγRI)

$K_D = 0.22$ nM
$K_a = 3.00 \times 10^5$ 1/Ms
$K_d = 6.36 \times 10^{-5}$ 1/s

64 nM
32 nM
16 nM
8 nM
4 nM
2 nM

HP511(FcγRIIA)

$K_D = 4.40$ nM
$K_a = 1.48 \times 10^6$ 1/Ms
$K_d = 6.53 \times 10^{-3}$ 1/s

320 nM
160 nM
80 nM
40 nM
20 nM
10 nM

HP511(FcγRIIIA)

$K_D = 2.56$ nM
$K_a = 6.19 \times 10^5$ 1/Ms
$K_d = 1.58 \times 10^{-3}$ 1/s

320 nM
160 nM
80 nM
40 nM
20 nM
10 nM

**Figure 29d**

**Figure 30a**

Figure 30b

**Figure 31a**

BT474 (HER2 3+)

**Figure 31b**

NCI-N87 (HER2 3+)

**Figure 31c**

**Figure 31d**

Duo_TRA

**Figure 31e**

Tandem_Fc_TRA

**Figure 31f**

**Figure 31g**

**Figure 32a**

NCI-N87
E:T=15:1

- hIgG1
- Trastuzumab
- H01

**Figure 32b**

MDA-MB-453
E:T=15:1

- hIgG1
- Trastuzumab
- H01

**Figure 32c**

**Figure 32d**

**Figure 33a**

SNU-5 tumor xenograft model
(Gastric cancer; n = 7 mice/group)

Legend:
- Vehicle (PBS)
- Trastuzumab (5 mg/kg)
- Trastuzumab + Pertuzumab (5 + 5 mg/kg)
- H01 (5 mg/kg)
- H01 + P01 (5 + 5 mg/kg)
- HP507 (5 mg/kg)

Y-axis: Tumor volume (mm$^3$)
X-axis: Days after tumor transplantation (day)

**P < 0.01, ***P < 0.001 (Student's t-test)
(compared to Trastuzumab + Pertuzumab)

**Figure 33b**

SNU-5 (HER2-low) tumor xenograft model (BALB/c nude mice)

| Anti-HER2 mAb | TGI | Significance |
|---|---|---|
| 1 mg/kg Trastuzumab (TRA) | -1 % | NS (vs Vehicle) |
| 1 mg/kg Pertuzumab (PER) | 36 % | NS (vs Vehicle) |
| 0.5 mg/kg TRA + 0.5 mg/kg PER | -1 % | NS (vs Vehicle) |

NS: not significant

| Anti-HER2 mAb | TGI | Significance |
|---|---|---|
| 1 mg/kg H01 | 96 % | *** (vs Vehicle) ** (vs TRA) |
| 1 mg/kg P01 | 90 % | *** (vs Vehicle) ** (vs PER) |
| 0.5 mg/kg H01 + 0.5 mg/kg P01 | 100 % | *** (vs Vehicle) *** (vs TRA+PER) |

*** $p \leq 0.001$ (Student's T-test)
** $p \leq 0.01$ (Student's T-test)

**Figure 34**

SNU601 tumor xenograft model
(Gastric cancer; n = 7 mice/group)

***P < 0.001 (Student's t-test)
(compared to IVIG + Trastuzumab + Pertuzumab)

**Figure 35**

NCI-N87 tumor xenograft model
(Gastric cancer; n = 6 mice/group)

*P < 0.05, ***P < 0.001 (Student's t-test)

Figure 36

**Figure 37**

**Figure 38**

CT26-HER2 clones stability test (D20)

Figure 39

**Figure 40**

CT26-HER2 (Human HER2 +)
syngeneic tumor model
(Colorectal cancer; n = 10 mice/group)

Vehicle
Trastuzumab (5 mg/kg)
H01 (5 mg/kg)

*P < 0.05 (Student's t-test)

Tumor volume (mm³)

Days after drug administration (day)

**Figure 41a**

Monovalent
engineered mAb

**Figure 41b**

Biparatopic
engineered mAb

**Figure 42**

**Figure 43**

HepG2
Fc counting (anti-hIgG Fcγ, Fab)

**Figure 44**

**Figure 45**

PC3
Fc counting (anti-hIgG Fcγ, Fab)

Legend: 100nM of a-EphA2

Y-axis: GMFI (Alexa 488)

X-axis categories: 2nd only, IgG1, 1C1, EPB02, EPB03, EPB05, EPB06, EPB07, EPB10

**Figure 46**

MEM01 (2-32 nM)

MEM06 (2-32 nM)

**Figure 47**

MKN45
Fc counting (anti-hIgG Fcγ, Fab)

Figure 48

SNU5
Fc counting (anti-hIgG Fcγ, Fab)

**Figure 49**

EGM01 (1-32 nM)

EGM02 (1-32 nM)

EGM03 (1-32 nM)

EGM04 (1-32 nM)

EGM05 (1-32 nM)

**Figure 50**

**Figure 51**

**Figure 52**

**Figure 53a**

**Figure 53b**

**Figure 53c**

Receptor:Fc = 4:3 (0.75 Fc/receptor)

**Figure 53d**

Figure 53e

Examples of novel antibody structures

**Formula** (N terminus-----C terminus)
(I): $X\text{-}A\text{-}(L1)_n\text{-}C$ (n = 0 or 1)
(II): $Y\text{-}B\text{-}(L2)_m\text{-}D$ (m = 0 or 1)
(III): $(L3)_p\text{-}E$ (p = 0 or 1)
(IV): $(L4)_q\text{-}F$ (q = 0 or 1)

Polypeptide 1 (I)

Polypeptide 2 (II)

Polypeptide 3 (III)

Polypeptide 4 (IV)

Engineered antigen binding protein

EP 4 700 047 A1

Figure 54

**Figure 55a**

H01 (FcγRI)

$K_D = 0.21$ nM

$K_a = 3.77 \times 10^5$ 1/Ms

$K_d = 7.77 \times 10^{-5}$ 1/s

32 nM
16 nM
8 nM
4 nM
2 nM

Binding (nm)

Time(sec)

Trastuzumab (FcγRI)

$K_D = 2.34$ nM

$K_a = 3.33 \times 10^5$ 1/Ms

$K_d = 7.80 \times 10^{-4}$ 1/s

64 nM
32 nM
16 nM
8 nM
4 nM
2 nM

Binding (nm)

Time(sec)

**Figure 55b**

**Figure 55c**

Comparison of Fcγ receptor binding ability between Trastuzumab and H01

| Fcγ receptor | Antibody | Relative affinity | $K_D$ | $K_a$ (1/Ms) | $K_d$ (1/s) |
|---|---|---|---|---|---|
| FcγRI | Trastuzumab | 1 | 2.3 | 3.33E+05 | 7.80E-04 |
| | H01 | 11.4 | 0.2 | 3.77E+05 | 7.77E-05 |
| FcγRIIA (131H) | Trastuzumab | 1 | 135.3 | 7.86E+04 | 1.06E-02 |
| | H01 | 28.5 | 4.8 | 1.13E+06 | 5.37E-03 |
| FcγRIIIA (158V) | Trastuzumab | 1 | 103.2 | 1.56E+05 | 1.61E-02 |
| | H01 | 73.2 | 1.4 | 1.44E+06 | 2.03E-03 |

Figure 55d

EP 4 700 047 A1

**Figure 56a**

Donor 2

Figure 56b

EP 4 700 047 A1

**Figure 57**

Examples of novel antibody fusion proteins

**Formula (N terminus ---C terminus)**
(I): X-A-$(L1)_n$-C  (n = 0 or 1)
(II): Y-B-$(L2)_m$-D  (m = 0 or 1)
(III): Z-$(L3)_p$-E  (p = 0 or 1)
(IV): Z-$(L4)_q$-F  (q = 0 or 1)

Polypeptide 1
(I)

Polypeptide 2
(II)

Polypeptide 3
(III)

Polypeptide 4
(IV)

Engineered antigen
binding protein

Figure 58

EP 4 700 047 A1

522

**Figure 59a**

**Figure 59b**

**Figure 59c**

**Figure 59d**

Figure 60

**Figure 61**

**Figure 62**

**Figure 63**

**Figure 64a**

**Figure 64b**

## INTERNATIONAL SEARCH REPORT

| International application No. |
| --- |
| **PCT/KR2024/005350** |

| A. CLASSIFICATION OF SUBJECT MATTER |
| --- |
| **C07K 16/46**(2006.01)i; **C07K 16/32**(2006.01)i; **A61P 35/00**(2006.01)i; **A61K 39/00**(2006.01)i |
| According to International Patent Classification (IPC) or to both national classification and IPC |

| B. FIELDS SEARCHED |
| --- |
| Minimum documentation searched (classification system followed by classification symbols) |
| C07K 16/46(2006.01); A61K 38/00(2006.01); A61K 39/00(2006.01); A61K 39/395(2006.01); C07K 16/00(2006.01); C07K 16/28(2006.01) |
| Documentation searched other than minimum documentation to the extent that such documents are included in the fields searched |
| Korean utility models and applications for utility models: IPC as above <br> Japanese utility models and applications for utility models: IPC as above |
| Electronic data base consulted during the international search (name of data base and, where practicable, search terms used) |
| eKOMPASS (KIPO internal) & keywords: 상보성 결정 영역 (Complementarity-determining region, CDR), Fc 도메인 (crystallizable fragment domain), 융합단백질 (fusion protein), 이량체 (dimer), 펩타이드 링커 (peptide linker), 이중 Fc 항체 (dual Fc antibody), 정전기 조향 (electrostatic steering), 면역글로불린 (immunoglobulin), 암 항원 (cancer antigen) |

| C. DOCUMENTS CONSIDERED TO BE RELEVANT | | |
| --- | --- | --- |
| Category* | Citation of document, with indication, where appropriate, of the relevant passages | Relevant to claim No. |
| X | KR 10-2021-0044782 A (MOMENTA PHARMACEUTICALS, INC.) 23 April 2021 (2021-04-23) <br> See abstract; claims 2, 51 and 56; paragraphs [0001], [0125], [0141], [0145], [0423] and [0424]; and figure 1. | 1-5,22,23 |
| Y | | 6-17 |
| Y | KR 10-2019-0060821 A (XENCOR INC.) 03 June 2019 (2019-06-03) <br> See claim 1; and paragraphs [0007] and [0042]. | 6-17 |
| A | GOULET, D. R. et al. Design and characterization of novel dual Fc antibody with enhanced avidity for Fc receptors. Proteins. 2020, vol. 88, pp. 689-697. <br> See entire document. | 1-17,22,23 |
| A | KR 10-2019-0038617 A (JANSSEN BIOTECH, INC.) 08 April 2019 (2019-04-08) <br> See entire document. | 1-17,22,23 |

| ☑ Further documents are listed in the continuation of Box C. | ☑ See patent family annex. |
| --- | --- |

| * Special categories of cited documents: <br> "A" document defining the general state of the art which is not considered to be of particular relevance <br> "D" document cited by the applicant in the international application <br> "E" earlier application or patent but published on or after the international filing date <br> "L" document which may throw doubts on priority claim(s) or which is cited to establish the publication date of another citation or other special reason (as specified) <br> "O" document referring to an oral disclosure, use, exhibition or other means <br> "P" document published prior to the international filing date but later than the priority date claimed | "T" later document published after the international filing date or priority date and not in conflict with the application but cited to understand the principle or theory underlying the invention <br> "X" document of particular relevance; the claimed invention cannot be considered novel or cannot be considered to involve an inventive step when the document is taken alone <br> "Y" document of particular relevance; the claimed invention cannot be considered to involve an inventive step when the document is combined with one or more other such documents, such combination being obvious to a person skilled in the art <br> "&" document member of the same patent family |
| --- | --- |

| Date of the actual completion of the international search | Date of mailing of the international search report |
| --- | --- |
| **22 July 2024** | **22 July 2024** |

| Name and mailing address of the ISA/KR <br><br> **Korean Intellectual Property Office** <br> **Government Complex-Daejeon Building 4, 189 Cheongsa-ro, Seo-gu, Daejeon 35208** | Authorized officer |
| --- | --- |
| Facsimile No. **+82-42-481-8578** | Telephone No. |

Form PCT/ISA/210 (second sheet) (July 2022)

## INTERNATIONAL SEARCH REPORT

| | International application No. |
|---|---|
| | **PCT/KR2024/005350** |

### C.    DOCUMENTS CONSIDERED TO BE RELEVANT

| Category* | Citation of document, with indication, where appropriate, of the relevant passages | Relevant to claim No. |
|---|---|---|
| A | KR 10-2015-0058236 A (GLIKNIK INC.) 28 May 2015 (2015-05-28)<br>See entire document. | 1-17,22,23 |
| PX | KR 10-2023-0060546 A (CENTENAIRE BIOSCIENCES, INC.) 04 May 2023 (2023-05-04)<br>See claims 1-23. | 1-17,22,23 |

Form PCT/ISA/210 (second sheet) (July 2022)

## INTERNATIONAL SEARCH REPORT

| International application No. |
|---|
| **PCT/KR2024/005350** |

**Box No. I      Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT

| International application No. |
| --- |
| **PCT/KR2024/005350** |

**Box No. II**      **Observations where certain claims were found unsearchable (Continuation of item 2 of first sheet)**

This international search report has not been established in respect of certain claims under Article 17(2)(a) for the following reasons:

1. ☑   Claims Nos.: **21**
   because they relate to subject matter not required to be searched by this Authority, namely:

   Claim 21 pertains to a method for treatment of the human body by surgery or therapy, as well as a diagnostic method (PCT Article 17(2)(a)(i) and PCT Rule 39.1(iv)).

2. ☑   Claims Nos.: **19**
   because they relate to parts of the international application that do not comply with the prescribed requirements to such an extent that no meaningful international search can be carried out, specifically:

   Claim 19 refers to claim 18 which violates the manner of referring to dependent claims (PCT Rule 6.4(a)), and thus is unclear.

3. ☑   Claims Nos.: **18,20**
   because they are dependent claims and are not drafted in accordance with the second and third sentences of Rule 6.4(a).

Form PCT/ISA/210 (continuation of first sheet) (July 2022)

EP 4 700 047 A1

## INTERNATIONAL SEARCH REPORT
### Information on patent family members

| | International application No. |
|---|---|
| | **PCT/KR2024/005350** |

| Patent document cited in search report | | | Publication date (day/month/year) | Patent family member(s) | | | Publication date (day/month/year) |
|---|---|---|---|---|---|---|---|
| KR | 10-2021-0044782 | A | 23 April 2021 | CA | 3106108 | A1 | 16 January 2020 |
| | | | | CN | 112996910 | A | 18 June 2021 |
| | | | | EP | 3820998 | A2 | 19 May 2021 |
| | | | | IL | 280038 | A | 01 March 2021 |
| | | | | JP | 2021-530989 | A | 18 November 2021 |
| | | | | MX | 2021-000281 | A | 12 November 2021 |
| | | | | US | 2021-147549 | A1 | 20 May 2021 |
| | | | | WO | 2020-014419 | A2 | 16 January 2020 |
| KR | 10-2019-0060821 | A | 03 June 2019 | EP | 3526241 | A1 | 21 August 2019 |
| | | | | IL | 265997 | A | 30 June 2019 |
| | | | | JP | 2022-180450 | A | 06 December 2022 |
| | | | | JP | 2023-106405 | A | 01 August 2023 |
| | | | | JP | 7142630 | B2 | 27 September 2022 |
| | | | | JP | 7273453 | B2 | 15 May 2023 |
| | | | | KR | 10-2024-0038176 | A | 22 March 2024 |
| | | | | KR | 10-2649972 | B1 | 22 March 2024 |
| | | | | US | 10550185 | B2 | 04 February 2020 |
| | | | | US | 11584794 | B2 | 21 February 2023 |
| | | | | US | 2018-118805 | A1 | 03 May 2018 |
| | | | | WO | 2018-071918 | A1 | 19 April 2018 |
| | | | | ZA | 201902380 | B | 26 August 2020 |
| KR | 10-2019-0038617 | A | 08 April 2019 | AU | 2017-308590 | A1 | 14 February 2019 |
| | | | | AU | 2017-308590 | A1 | 15 February 2018 |
| | | | | AU | 2023-200719 | A1 | 09 March 2023 |
| | | | | CA | 3033661 | A1 | 15 February 2018 |
| | | | | CN | 109863170 | A | 07 June 2019 |
| | | | | EP | 3497120 | A1 | 19 June 2019 |
| | | | | IL | 264512 | A | 28 February 2019 |
| | | | | IL | 264512 | D0 | 28 February 2019 |
| | | | | JP | 2019-533427 | A | 21 November 2019 |
| | | | | JP | 7178342 | B2 | 25 November 2022 |
| | | | | MA | 45919 | A | 19 June 2019 |
| | | | | SG | 11201900746 | A | 27 February 2019 |
| | | | | US | 10669344 | B2 | 02 June 2020 |
| | | | | US | 2018-044427 | A1 | 15 February 2018 |
| | | | | WO | 2018-031258 | A1 | 15 February 2018 |
| KR | 10-2015-0058236 | A | 28 May 2015 | CN | 104718223 | A | 17 June 2015 |
| | | | | EP | 2885320 | A2 | 24 June 2015 |
| | | | | HK | 1210792 | A1 | 06 May 2016 |
| | | | | IL | 237307 | A | 30 April 2015 |
| | | | | IL | 237307 | D0 | 30 April 2015 |
| | | | | US | 2014-0072582 | A1 | 13 March 2014 |
| | | | | US | 2018-094061 | A1 | 05 April 2018 |
| | | | | US | 9683044 | B2 | 20 June 2017 |
| | | | | WO | 2014-031646 | A2 | 27 February 2014 |
| | | | | WO | 2014-031646 | A3 | 01 May 2014 |
| KR | 10-2023-0060546 | A | 04 May 2023 | AU | 2022-372527 | A1 | 27 April 2023 |
| | | | | WO | 2023-068818 | A1 | 27 April 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20200040084 A1
- WO 2014084607 A1
- WO 2018059502 A1
- US 7919086 B2
- US 20090304721 A1, Kinch

### Non-patent literature cited in the description

- **CLAUDIO SUSTMANN et al.** *MAbs*, 2019
- **DENNIS R GOULET et al.** *Proteins*, 2020
- Remington's Pharmaceutical Sciences. 1995
- **MERCHANT et al.** *Nat. Biotechnol.*, 1998
- **GREG A. LAZAR et al.** *PNAS*, 2006